(19) Europäisches Patentamt
European Patent Office
Office européen des brevets

(11) **EP 2 204 376 A2**

## (12) EUROPEAN PATENT APPLICATION

(43) Date of publication:
**07.07.2010 Bulletin 2010/27**

(21) Application number: **10075078.5**

(22) Date of filing: **20.07.2005**

(51) Int Cl.:
*C07H 21/00* (2006.01)     *C12Q 1/68* (2006.01)
*C07K 14/53* (2006.01)     *C07K 14/715* (2006.01)
*G01N 33/68* (2006.01)

(84) Designated Contracting States:
**AT BE BG CH CY CZ DE DK EE ES FI FR GB GR HU IE IS IT LI LT LU LV MC NL PL PT RO SE SI SK TR**

(30) Priority: **20.07.2004 US 895974**

(62) Document number(s) of the earlier application(s) in accordance with Art. 76 EPC:
**05791780.9 / 1 778 873**

(71) Applicant: **Sagres Discovery, Inc.**
**Emeryille CA 94608-2916 (US)**

(72) Inventors:
• **Morris, David W.**
  **Emeryville, CA 94662-8097 (US)**
• **Malandro, Marc S.**
  **Emeryville, CA 94662-8097 (US)**

• **Lai, Albert**
  **Emeryville, CA 94662-8097 (US)**
• **Tse, Christin**
  **Emeryville, CA 94662-8097 (US)**
• **Fattaey, Ali R.**
  **Emeryville, CA 94662-8097 (US)**

(74) Representative: **Goodfellow, Hugh Robin et al**
**Carpmaels & Ransford**
**43-45 Bloomsbury Square**
**London**
**WC1A 2RA (GB)**

Remarks:
• The complete document including Reference Tables and the Sequence Listing can be downloaded from the EPO website
• This application was filed on 22-02-2010 as a divisional application to the application mentioned under INID code 62.

## (54) Novel therapeutic targets in cancer

(57)    The present invention relates to novel sequences for use in detection, diagnosis and treatment of cancers, especially lymphomas. The invention provides cancer-associated (CA) polynucleotide sequences whose expression is associated with cancer. The present invention provides CA polypeptides associated with cancer that are present on the cell surface and present novel therapeutic targets against cancer. The present invention further provides diagnostic composition and methods for the detection of cancer. The present invention provides monoclonal and polyclonal antibodies specific for the CA polypeptides. The present invention also provides diagnostic tools and therapeutic compositions and methods for screening, prevention and treatment of cancer.

EP 2 204 376 A2

**Description**

CROSS-REFERENCE TO RELATED APPLICATIONS

This application is related to the following U.S. Applications: U.S. Ser. No. 10/034,650, filed December 20, 2001; U.S. Ser. No. 10/035,832, filed December 26, 2001; U.S. Ser. No. 10/004,113, filed October 23, 2001; U.S. Ser. No. 09/997,722, filed November 30, 2001; U.S. Ser. No. 10/085,117, filed February 27, 2002; U.S. Ser. No. 10/087,192, filed March 1, 2002; U.S. Ser. No. 10/322,281, filed December 17, 2002; U.S. Ser. No. 10/322,696, filed December 17, 2002, U.S. Ser. No. 10/331,053, filed December 26, 2002; U.S. Ser. No. 10/330,773, filed December 27, 2002; U.S. Ser. No. 10/367,094, filed February 14, 2003; U.S. Ser. No. 10/388,838, filed March 14, 2003, U.S. Ser. No. 10/417,375 filed April 15, 2003, U.S. Ser. No. 10/461,862, filed June 13, 2003, U.S. Ser. No. 10/663,431 filed September 15, 2003, U.S. Ser. No. 10/669,920 filed September 23, 2003, U.S. Ser. No. 10/670,914 filed September 24, 2003, U.S. Ser. No. 10/674,575, filed September 29, 2003, U.S. Ser. No. 10/676,684 filed September 30, 2003, U.S. Ser. No. 10/692,382 filed October 22, 2003, U.S. Serial No. 10/833,833, filed April 27, 2004, U.S. Serial No. 10/836,956, filed April 30, 2004, and U.S. Serial No. 10/836,956, filed April 30, 2004 all of which are expressly incorporated herein by reference in their entirety.

TECHNICAL FIELD OF THE INVENTION

This invention relates generally to the field of cancer-associated genes. Specifically, it relates to novel sequences for use in diagnosis and treatment of cancer and tumors, as well as the use of the novel compositions in screening methods. The present invention provides methods of using cancer associated polynucleotides, their corresponding gene products and antibodies specific for the gene products in the detection, diagnosis, prevention and/or treatment of associated cancers.

BACKGROUND OF THE INVENTION

Oncogenes are genes that can cause cancer. Carcinogenesis can occur by a wide variety of mechanisms, including infection of cells by viruses containing oncogenes, activation of protooncogenes in the host genome, and mutations of protooncogenes and tumor suppressor genes. Carcinogenesis is fundamentally driven by somatic cell evolution (i.e. mutation and natural selection of variants with progressive loss of growth control). The genes that serve as targets for these somatic mutations are classified as either protooncogenes or tumor suppressor genes, depending on whether their mutant phenotypes are dominant or recessive, respectively.

There are a number of viruses known to be involved in human cancer as well as in animal cancer. Of particular interest here are viruses that do not contain oncogenes themselves; these are slow-transforming retroviruses. They induce tumors by integrating into the host genome and affecting neighboring protooncogenes in a variety of ways. Provirus insertion mutation is a normal consequence of the retroviral life cycle. In infected cells, a DNA copy of the retrovirus genome (called a provirus) is integrated into the host genome. A newly integrated provirus can affect gene expression in *cis* at or near the integration site by one of two mechanisms. Type I insertion mutations up-regulate transcription of proximal genes as a consequence of regulatory sequences (enhancers and/or promoters) within the proviral long terminal repeats (LTRs). Type II insertion mutations cause truncation of coding regions due to either integration directly within an open reading frame or integration within an intron flanked on both sides by coding sequences. The analysis of sequences at or near the insertion sites has led to the identification of a number of new protooncogenes.

With respect to lymphoma and leukemia, retroviruses such as AKV murine leukemia virus (MLV) or SL3-3 MLV, are potent inducers of tumors when inoculated into susceptible newborn mice, or when carried in the germline. A number of sequences have been identified as relevant in the induction of lymphoma and leukemia by analyzing the insertion sites; see Sorensen et al., J. of Virology 74:2161 (2000); Hansen et al., Genome Res. 10(2):237-43 (2000); Sorensen et al., J. Virology 70:4063 (1996); Sorensen et al., J. Virology 67:7118 (1993); Joosten et al., Virology 268: 308 (2000); and Li et al., Nature Genetics 23:348 (1999); all of which are expressly incorporated by reference herein. With respect to cancers, especially breast cancer, prostate cancer and cancers with epithelial origin, the mammalian retrovirus, mouse mammary tumor virus (MMTV) is a potent inducer of tumors when inoculated into susceptible newborn mice, or when carried in the germ line. Mammary Tumors in the Mouse, edited by J. Hilgers and M. Sluyser; Elsevier/ North-Holland Biomedical Press; New York, N.Y.

The pattern of gene expression in a particular living cell is characteristic of its current state. Nearly all differences in the state or type of a cell are reflected in the differences in RNA levels of one or more genes. Comparing expression patterns of uncharacterized genes may provide clues to their function. High throughput analysis of expression of hundreds or thousands of genes can help in (a) identification of complex genetic diseases, (b) analysis of differential gene expression over time, between tissues and disease states, and (c) drug discovery and toxicology studies. Increase or decrease in the levels of expression of certain genes correlate with cancer biology. For example, oncogenes are positive regulators of tumorigenesis, while tumor suppressor genes are negative regulators of tumorigenesis. (Marshall, Cell, 64: 313-326 (1991); Weinberg, Science, 254: 1138-1146 (1991)).

Accordingly, it is an object of the invention to provide polynucleotide and polypeptide sequences involved in cancer and, in particular, in oncogenesis.

Immunotherapy, or the use of antibodies for therapeutic purposes has been used in recent years to treat cancer. Passive immunotherapy involves the use of monoclonal antibodies in cancer treatments. See for example, Cancer: Principles and Practice of Oncology, 6th Edition (2001) Chapt. 20 pp. 495-508. Inherent therapeutic biological activity of these antibodies include direct inhibition of tumor cell growth or survival, and the ability to recruit the natural cell killing activity of the body's immune system. These agents are administered alone or in conjunction with radiation or chemotherapeutic agents. Rituxan® and Herceptin®, approved for treatment of lymphoma and breast cancer, respectively, are two examples of such therapeutics. Alternatively, antibodies are used to make antibody conjugates where the antibody is linked to a toxic agent and directs that agent to the tumor by specifically binding to the tumor. Mylotarg® is an example of an approved antibody conjugate used for the treatment of leukemia.

Accordingly, it is another object of this invention to provide antigens (cancer-associated polypeptides) associated with a variety of cancers as targets for diagnostic and/or therapeutic antibodies. These antigens are also useful for drug discovery (*e.g.*, small molecules) and for further characterization of cellular regulation, growth, and differentiation.

SUMMARY OF THE INVENTION

In accordance with the objects outlined above, the present invention provides methods for screening for compositions that modulate cancer, especially lymphoma and leukemia. The present invention also provides methods for screening for compositions which modulate carcinomas, especially mammary adenocarcinomas. Also provided herein are methods of inhibiting proliferation of a cell, preferably a lymphoma cell or a breast cancer cell. Methods of treatment of cancer, including diagnosis, are also provided herein.

In one aspect, a method of screening drug candidates comprises providing a cell that expresses a cancer-associated (CA) gene or fragments thereof. Preferred embodiments of CA genes are genes that are differentially expressed in cancer cells, preferably lymphatic, breast, prostate or epithelial cells, compared to other cells. Preferred embodiments of CA genes used in the methods herein include, but are not limited to the nucleic acids selected from Tables 1-7 (human genomic sequences of SEQ ID NOS: 6, 16, 26, 40, 52, 60 and 66, and sequences of SEQ ID NOS: 7, 9, 17, 19, 21, 27, 29, 41, 43, 45, 47, 53, 61, 67, 69, 71 and 73 corresponding to the human mRNAs generated therefrom). The methods further include adding a drug candidate to the cell and determining the effect of the drug candidate on the expression of the CA gene.

In one embodiment, the method of screening drug candidates includes comparing the level of expression in the absence of the drug candidate to the level of expression in the presence of the drug candidate.

Also provided herein is a method of screening for a bioactive agent capable of binding to a CA protein (CAP), the method comprising combining the CAP and a candidate bioactive agent, and determining the binding of the candidate agent to the CAP.

Further provided herein is a method for screening for a bioactive agent capable of modulating the activity of a CAP. In one embodiment, the method comprises combining the CAP and a candidate bioactive agent, and determining the effect of the candidate agent on the bioactivity of the CAP.

Also provided is a method of evaluating the effect of a candidate cancer drug comprising administering the drug to a patient and removing a cell sample from the patient. The expression profile of the cell is then determined. This method may further comprise comparing the expression profile of the patient to an expression profile of a healthy individual.

In a further aspect, a method for inhibiting the activity of a CA protein is provided. In one embodiment, the method comprises administering to a patient an inhibitor of a CA protein preferably selected from the group consisting of the sequences outlined in Tables 1-7 (SEQ ID NOS: 8, 10, 18, 20, 22, 28, 30, 42, 44, 46, 48, 54, 62, 68, 70, 72 and 74).

A method of neutralizing the effect of a CA protein, preferably a protein encoded by a nucleic acid selected from the group of sequences outlined in Tables 1-7 (human genomic sequences of SEQ ID NOS: 6, 16, 26, 40, 52, 60 and 66, and sequences of SEQ ID NOS: 7, 9, 17, 19, 21, 27, 29, 41, 43, 45, 47, 53, 61, 67, 69, 71 and 73 corresponding to the human mRNAs generated therefrom), is also provided. Preferably, the method comprises contacting an agent specific for said protein with said protein in an amount sufficient to effect neutralization.

Moreover, provided herein is a biochip comprising a nucleic acid segment which encodes a CA protein, preferably selected from the sequences outlined in Tables 1-7 (SEQ ID NOS: 7, 9, 17, 19, 21, 27, 29, 41, 43, 45, 47, 53, 61, 67, 69, 71 and 73).

Also provided herein is a method for diagnosing or determining the propensity to cancers, especially lymphoma or leukemia or carcinoma by sequencing at least one carcinoma or lymphoma gene of an individual. In yet another aspect of the invention, a method is provided for determining cancer including lymphoma and leukemia gene copy numbers in an individual.

The invention provides an isolated nucleic acid comprising at least 10, 12, 15, 20 or 30 contiguous nucleotides of a sequence selected from the group consisting of the polynucleotide sequences SEQ ID NOS: 7, 9, 17, 19, 21, 27, 29, 41, 43, 45, 47, 53, 61, 67, 69, 71 and 73 shown in Tables 1-7, or its complement, or an expression vector comprising the isolated nucleic acids and host cells comprising them.

In some embodiments, the polynucleotide, or its complement or a fragment thereof, further comprises a detectable label, is attached to a solid support, is prepared at least in part by chemical synthesis, is an antisense fragment, is single stranded, is double stranded or comprises a microarray.

The invention provides an isolated polypeptide, encoded within an open reading frame of a CA sequence selected from the group consisting of the polynucleotide sequences of SEQ ID NOS: 6, 16, 26, 40, 52, 60 and 66 shown in Tables 1-7, or its complement. The invention provides an isolated polypeptide, wherein said polypeptide comprises the amino acid sequence encoded by a polynucleotide selected from the group consisting of SEQ ID NOS: 7, 9, 17, 19, 21, 27, 29, 41, 43, 45, 47, 53, 61, 67, 69, 71 and 73 shown in Tables 1-7. The invention provides an isolated polypeptide, wherein said polypeptide comprises the amino acid sequence encoded by a polypeptide selected from the group consisting of SEQ ID NOS: 8, 10,18, 20, 22, 28, 30, 42, 44, 46, 48, 54, 62, 68, 70, 72 and 74 shown in Tables 1-7.

The invention further provides an isolated polypeptide, comprising the amino acid sequence of an epitope of the amino acid sequence of a CA polypeptide selected from the group consisting of SEQ ID NOS: 8, 10, 18, 20, 22, 28, 30, 42, 44, 46, 48, 54, 62, 68, 70, 72 and 74 shown in Tables 1-7, wherein the polypeptide or fragment thereof may be attached to a solid support. In one embodiment the invention provides an isolated antibody (monoclonal or polyclonal) or antigen binding fragment thereof, that binds to such a polypeptide. The isolated antibody or antigen binding fragment thereof may be attached to a solid support, or further comprises a detectable label.

In one embodiment, the invention provides a kit for diagnosing the presence of cancer in a test sample, said kit comprising at least one polynucleotide that selectively hybridizes to a CA polynucleotide sequence shown in Tables 1-7, or its complement. In another embodiment. the invention provides an electronic library comprising a CA polynucleotide, a CA polypeptide, or fragment thereof, shown in Tables 1-7.

In one embodiment, the invention provides a method of screening for anticancer activity comprising: (a) providing a cell that expresses a cancer associated (CA) gene encoded by a nucleic acid sequence selected from the group consisting of the CA sequences shown in Tables 1-7, or fragment thereof; (b) contacting a tissue sample derived from a cancer cell with an anticancer drug candidate; (c) monitoring an effect of the anticancer drug candidate on an expression of the CA polynucleotide in the tissue sample, and optionally (d) comparing the level of expression in the absence of said drug candidate to the level of expression in the presence of the drug candidate.

In one embodiment, the invention provides a method for detecting cancer associated with expression of a polypeptide in a test cell sample, comprising the steps of: (i) detecting a level of expression of at least one polypeptide selected from the group consisting of SEQ ID NOS: 8, 10, 18, 20, 22, 28, 30, 42, 44, 46, 48, 54, 62, 68, 70, 72 and 74 shown in Tables 1-7, or a fragment thereof; and (ii) comparing the level of expression of the polypeptide in the test sample with a level of expression of polypeptide in a normal cell sample, wherein an altered level of expression of the polypeptide in the test cell sample relative to the level of polypeptide expression in the normal cell sample is indicative of the presence of cancer in the test cell sample.

In another embodiment, the invention provides a method for detecting cancer associated with expression of a polypeptide in a test cell sample, comprising the steps of: (i) detecting a level of activity of at least one polypeptide selected from the group consisting of SEQ ID NOS: 8, 10, 18, 20, 22, 28, 30, 42, 44, 46, 48, 54, 62, 68, 70, 72 and 74 shown in Tables 1-7, or a fragment thereof, wherein said activity corresponds to at least one activity for the polypeptide listed in Table 9; and (ii) comparing the level of activity of the polypeptide in the test sample with a level of activity of polypeptide in a normal cell sample, wherein an altered level of activity of the polypeptide in the test cell sample relative to the level of polypeptide activity in the normal cell sample is indicative of the presence of cancer in the test cell sample.

In another embodiment, the invention provides a method for detecting cancer associated with the presence of an antibody in a test serum sample, comprising the steps of: (i) detecting a level of an antibody against an antigenic polypeptide selected from the group consisting of SEQ ID NOS: 8, 10, 18, 20, 22, 28, 30, 42, 44, 46, 48, 54, 62, 68, 70, 72 and 74 shown in Tables 1-7, or antigenic fragment thereof; and (ii) comparing said level of said antibody in the test sample with a level of said antibody in the control sample, wherein an altered level of antibody in said test sample relative to the level of antibody in the control sample is indicative of the presence of cancer in the test serum sample.

The invention provides a method for screening for a bioactive agent capable of modulating the activity of a CA protein (CAP), wherein said CAP is encoded by a nucleic acid comprising a nucleic acid sequence selected from the group consisting of the polynucleotide sequences SEQ ID NOS: 7, 9, 17, 19, 21, 27, 29, 41, 43, 45, 47, 53, 61, 67, 69, 71 and 73 shown in Tables 1-7, said method comprising: a) combining said CAP and a candidate bioactive agent; and b) determining the effect of the candidate agent on the bioactivity of said CAP. According to the method the bioactive agent: affects the expression of the CA protein (CAP); affects the activity of the CA protein (CAP), wherein such activity is selected from the activities listed in Table 9.

In one embodiment, the invention provides a method for diagnosing cancer comprising: a) determining the expression of one or more genes comprising a nucleic acid sequence selected from the group consisting of the human genomic and mRNA sequences outlined in Tables 1-7, in a first tissue type of a first individual; and b) comparing said expression of said gene(s) from a second normal tissue type from said first individual or a second unaffected individual; wherein a difference in said expression indicates that the first individual has cancer.

In another embodiment the invention provides a method for treating cancers comprising administering to a patient a bioactive agent modulating the activity of a CA protein (CAP), wherein said CAP is encoded by a nucleic acid comprising a nucleic acid sequence selected from the group consisting of the human nucleic acid sequences in Tables 1-7 and further wherein the bioactive agent binds to the CA protein, wherein the CA protein: is a signalling protein wherein the CAP sequence is selected from the group consisting of SEQ ID NOS: 6, 12, 14, 96, 130, 172 and 174 shown in Tables 1-7; is involved in signal transduction wherein the CAP sequence is selected from the group consisting of SEQ ID NOS: 20, 84, 90, 92, 94, 96, 98, 100, 102, 104, 106, and 108; is a cell adhesion protein wherein the CAP sequence is selected from the group consisting of SEQ ID NOS: 36, 38, 40, 84, 90, 92, 94, 96, 98, 100, 102, 104, 106, 108, 124, 172, and 174; is involved in inflammatory response wherein the CAP sequence is selected from the group consisting of SEQ ID NOS: 62, 64, 66, 68, 72, 74, and 76; is involved in pheromone response wherein the CAP sequence is selected from the group consisting of SEQ ID NOS: 138, 140, 142, 144, 146, 148, and 150; is an ion transport protein wherein the CAP sequence is selected from the group consisting of SEQ ID NOS: 28, 30, 114, and 118 as shown in Tables 1-7.

The invention provides monoclonal antibodies that preferentially binds to a CA protein (CAP) that is expressed on a cell surface, wherein the CA protein selected from the group consisting of SEQ ID NOS: 8, 10, 18, 20, 22, 28, 30, 42, 44, 46, 48, 54, 62, 68, 70, 72 and 74; preferably to the extracellular domain of the CA protein; preferably to a CA protein differentially expressed on a cancer cell surface relative to a normal cell surface or preferably to at least one human cancer cell line; preferably linked to a therapeutic agent; or preferably humanized. Kits and pharmaceutical compositions for detecting a presence or an absence of cancer cells in an individual, and comprising such antibodies are also provided.

The invention also provides a method for detecting a presence or an absence of cancer cells in an individual, the method comprising: contacting cells from the individual with the antibody according to the invention; and detecting a complex of a CAP from the cancer cells and the antibody, wherein detection of the complex correlates with the presence of cancer cells in the individual. In one embodiment the invention provides a method for inhibiting growth of cancer cells in an individual, the method comprising: administering to the individual an effective amount of a pharmaceutical composition according to the invention. In another embodiment the invention provides a method for delivering a therapeutic agent to cancer cells in an individual, the method comprising: administering to the individual an effective amount of a pharmaceutical composition according to according to the invention.

Novel sequences associated with cancer are also provided herein. Other aspects of the invention will become apparent to the skilled artisan by the following description of the invention.

BRIEF DESCRIPTION OF THE FIGURES

**Figure 1** depicts PCR amplification of host-provirus junction fragments.

**Figure 2** shows an example of average threshold cycle ($C_T$) values for a housekeeper gene and target gene.

**Figure 3** shows an example of the calculated difference ($\Delta\Delta C_T$) between the $C_T$ values of target and housekeeper genes ($\Delta C_T$) for various samples.

**Figure 4** shows the $\Delta\Delta C_T$ and comparative expression level for each sample from Figure 3.

DETAILED DESCRIPTION OF PREFERRED EMBODIMENTS

The present invention is directed to a number of sequences associated with cancers, especially lymphoma, breast cancer or prostate cancer. The relatively tight linkage between clonally-integrated proviruses and protooncogenes forms "provirus tagging", in which slow-transforming retroviruses that act by an insertion mutation mechanism are used to isolate protooncogenes. In some models, uninfected animals have low cancer rates, and infected animals have high cancer rates. It is known that many of the retroviruses involved do not carry transduced host protooncogenes or pathogenic *trans*-acting viral genes, and thus the cancer incidence must therefore be a direct consequence of proviral integration effects into host protooncogenes. Since proviral integration is random, rare integrants will "activate" host protooncogenes that provide a selective growth advantage, and these rare events result in new proviruses at clonal stoichiometries in tumors. In contrast to mutations caused by chemicals, radiation, or spontaneous errors, protooncogene insertion mutations can be easily located by virtue of the fact that a convenient-sized genetic marker of known sequence (the provirus) is present at the site of mutation. Host sequences that flank clonally integrated proviruses can be cloned using a variety of strategies. Once these sequences are in hand, the tagged protooncogenes can be subsequently identified. The presence of provirus at the same locus in two or more independent tumors is *prima facie* evidence that a protooncogene is present at or very near the provirus integration sites. This is because the genome is too large for random integrations to result in observable clustering. Any clustering that is detected is unequivocal evidence for biological selection (i.e. the tumor phenotype). Moreover, the pattern of proviral integrants (including orientations) provides compelling positional information that makes localization of the target gene at each cluster relatively simple. The three mammalian retroviruses that are known to cause cancer by an insertion mutation mechanism are FeLV (leukemia/lymphoma in cats), MLV (leukemia/lymphoma in mice and rats), and MMTV (mammary cancer in mice).

Thus, the use of oncogenic retroviruses, whose sequences insert into the genome of the host organism resulting in cancer, allows the identification of host sequences involved in cancer. These sequences may then be used in a number of different ways, including diagnosis, prognosis, screening for modulators (including both agonists and antagonists), antibody generation (for immunotherapy and imaging), etc. However, as will be appreciated by those in the art, oncogenes that are identified in one type of cancer such as lymphoma or leukemia have a strong likelihood of being involved in other types of cancers as well. Thus, while the sequences outlined herein are initially identified as correlated with lymphoma, they can also be found in other types of cancers as well, outlined below.

**Definitions**

Accordingly, the present invention provides nucleic acid and protein sequences that are associated with cancer, herein termed "cancer associated" or "CA" sequences. In one embodiment, the present invention provides nucleic acid and protein sequences that are associated with cancers that originate in lymphatic tissue, herein termed "lymphoma associated," "leukemia associated" or "LA" sequences. In another embodiment, the present invention provides nucleic acid and protein sequences that are associated with carcinomas which originate in breast tissue, herein termed "breast cancer associated" or "BC" sequences.

Suitable cancers that can be diagnosed or screened for using the methods of the present invention include cancers classified by site or by histological type. Cancers classified by site include cancer of the oral cavity and pharynx (lip, tongue, salivary gland, floor of mouth, gum and other mouth, nasopharynx, tonsil, oropharynx, hypopharynx, other oral/pharynx); cancers of the digestive system (esophagus; stomach; small intestine; colon and rectum; anus, anal canal, and anorectum; liver; intrahepatic bile duct; gallbladder; other biliary; pancreas; retroperitoneum; peritoneum, omentum, and mesentery; other digestive); cancers of the respiratory system (nasal cavity, middle ear, and sinuses; larynx; lung and bronchus; pleura; trachea, mediastinum, and other respiratory); cancers of the mesothelioma; bones and joints; and soft tissue, including heart; skin cancers, including melanomas and other non-epithelial skin cancers; Kaposi's sarcoma and breast cancer; cancer of the female genital system (cervix uteri; corpus uteri; uterus, nos; ovary; vagina; vulva; and other female genital); cancers of the male genital system (prostate gland; testis; penis; and other male genital); cancers of the urinary system (urinary bladder; kidney and renal pelvis; ureter; and other urinary); cancers of the eye and orbit; cancers of the brain and nervous system (brain; and other nervous system); cancers of the endocrine system (thyroid gland and other endocrine, including thymus); lymphomas (Hodgkin's disease and non-Hodgkin's lymphoma), multiple myeloma, and leukemias (lymphocytic leukemia; myeloid leukemia; monocytic leukemia; and other leukemias).

Other cancers, classified by histological type, that may be associated with the sequences of the invention include, but are not limited to, Neoplasm, malignant; Carcinoma, NOS; Carcinoma, undifferentiated, NOS; Giant and spindle cell carcinoma; Small cell carcinoma, NOS; Papillary carcinoma, NOS; Squamous cell carcinoma, NOS; Lymphoepithelial carcinoma; Basal cell carcinoma, NOS; Pilomatrix carcinoma; Transitional cell carcinoma, NOS; Papillary transitional cell carcinoma; Adenocarcinoma, NOS; Gastrinoma, malignant; Cholangiocarcinoma; Hepatocellular carcinoma, NOS; Combined hepatocellular carcinoma and cholangiocarcinoma; Trabecular adenocarcinoma; Adenoid cystic carcinoma; Adenocarcinoma in adenomatous polyp; Adenocarcinoma, familial polyposis coli; Solid carcinoma, NOS; Carcinoid tumor, malignant; Bronchiolo-alveolar adenocarcinoma; Papillary adenocarcinoma, NOS; Chromophobe carcinoma; Acidophil carcinoma; Oxyphilic adenocarcinoma; Basophil carcinoma; Clear cell adenocarcinoma, NOS; Granular cell carcinoma; Follicular adenocarcinoma, NOS; Papillary and follicular adenocarcinoma; Nonencapsulating sclerosing carcinoma; Adrenal cortical carcinoma; Endometroid carcinoma; Skin appendage carcinoma; Apocrine adenocarcinoma; Sebaceous adenocarcinoma; Ceruminous adenocarcinoma; Mucoepidermoid carcinoma; Cystadenocarcinoma, NOS; Papillary cystadenocarcinoma, NOS; Papillary serous cystadenocarcinoma; Mucinous cystadenocarcinoma, NOS; Mucinous adenocarcinoma; Signet ring cell carcinoma; Infiltrating duct carcinoma; Medullary carcinoma, NOS; Lobular carcinoma; Inflammatory carcinoma; Paget's disease, mammary; Acinar cell carcinoma; Adenosquamous carcinoma; Adenocarcinoma w/ squamous metaplasia; Thymoma, malignant; Ovarian stromal tumor, malignant; Thecoma, malignant; Granulosa cell tumor, malignant; Androblastoma, malignant; Sertoli cell carcinoma; Leydig cell tumor, malignant; Lipid cell tumor, malignant; Paraganglioma, malignant; Extra-mammary paraganglioma, malignant; Pheochromocytoma; Glomangiosarcoma; Malignant melanoma, NOS; Amelanotic melanoma; Superficial spreading melanoma; Malig melanoma in giant pigmented nevus; Epithelioid cell melanoma; Blue nevus, malignant; Sarcoma, NOS; Fibrosarcoma, NOS; Fibrous histiocytoma, malignant; Myxosarcoma; Liposarcoma, NOS; Leiomyosarcoma, NOS; Rhabdomyosarcoma, NOS; Embryonal rhabdomyosarcoma; Alveolar rhabdomyosarcoma; Stromal sarcoma, NOS; Mixed tumor, malignant, NOS; Mullerian mixed tumor; Nephroblastoma; Hepatoblastoma; Carcinosarcoma, NOS; Mesenchymoma, malignant; Brenner tumor, malignant; Phyllodes tumor, malignant; Synovial sarcoma, NOS; Mesothelioma, malignant; Dysgerminoma; Embryonal carcinoma, NOS; Teratoma, malignant, NOS; Struma ovarii, malignant; Choriocarcinoma; Mesonephroma, malignant; Hemangiosarcoma; Hemangioendothelioma, malignant; Kaposi's sarcoma; Hemangiopericytoma, malignant; Lymphangiosarcoma; Osteosarcoma, NOS; Juxtacortical osteosarcoma; Chondrosarcoma, NOS; Chondroblastoma, malignant; Mesenchymal chondrosarcoma; Giant cell tumor of bone; Ewing's sarcoma; Odontogenic tumor, malignant; Ameloblastic odontosarcoma; Ameloblastoma, malignant; Ameloblastic fibrosarcoma; Pinealoma, malignant; Chordoma; Glioma, malignant; Ependymoma, NOS; Astrocytoma, NOS; Protoplasmic astrocytoma; Fibrillary astrocytoma; Astroblastoma; Glioblastoma, NOS; Oligodendroglioma, NOS; Oligodendroblastoma; Primitive neuroectodermal; Cerebellar sarcoma, NOS; Ganglioneuroblastoma; Neuroblastoma, NOS; Retinoblastoma, NOS; Olfactory neurogenic tumor; Meningioma, malignant; Neurofibrosarcoma; Neurilemmoma, malignant; Granular cell tumor, malignant; Malignant lymphoma, NOS; Hodgkin's disease, NOS; Hodgkin's; paragranuloma, NOS; Malignant lymphoma, small lymphocytic; Malignant lymphoma, large cell, diffuse; Malignant lymphoma, follicular, NOS; Mycosis fungoides; Other specified non-Hodgkin's lymphomas; Malignant histiocytosis; Multiple myeloma; Mast cell sarcoma; Immunoproliferative small intestinal disease; Leukemia, NOS; Lymphoid leukemia, NOS; Plasma cell leukemia; Erythroleukemia; Lymphosarcoma cell leukemia; Myeloid leukemia, NOS; Basophilic leukemia; Eosinophilic leukemia; Monocytic leukemia, NOS; Mast cell leukemia; Megakaryoblastic leukemia; Myeloid sarcoma; and Hairy cell leukemia.

In addition, the CA genes may be involved in other diseases such as, but not limited to, diseases associated with aging or neurodegeneration.

"Association" in this context means that the nucleotide or protein sequences are either differentially expressed, activated, inactivated or altered in cancers as compared to normal tissue. As outlined below, CA sequences include those that are up-regulated (i.e. expressed at a higher level), as well as those that are down-regulated (i.e. expressed at a lower level), in cancers. CA sequences also include sequences that have been altered (i.e., truncated sequences or sequences with substitutions, deletions or insertions, including point mutations) and show either the same expression profile or an altered profile. In a preferred embodiment, the CA sequences are from humans; however, as will be appreciated by those in the art, CA sequences from other organisms may be useful in animal models of disease and drug evaluation; thus, other CA sequences are provided, from vertebrates, including mammals, including rodents (rats, mice, hamsters, guinea pigs, etc.), primates, and farm animals (including sheep, goats, pigs, cows, horses, etc). In some cases, prokaryotic CA sequences may be useful. CA sequences from other organisms may be obtained using the techniques outlined below.

CA sequences include both nucleic acid and amino acid sequences. In a preferred embodiment, the CA sequences are recombinant nucleic acids. By the term "recombinant nucleic acid" herein is meant nucleic acid, originally formed in vitro, in general, by the manipulation of nucleic acid by polymerases and endonucleases, in a form not normally found in nature. Thus a recombinant nucleic acid is also an isolated nucleic acid, in a linear form, or cloned in a vector formed in vitro by ligating DNA molecules that are not normally joined, are both considered recombinant for the purposes of this invention. It is understood that once a recombinant nucleic acid is made and reintroduced into a host cell or organism, it will replicate using the in vivo cellular machinery of the host cell rather than in vitro manipulations; however, such nucleic acids, once produced recombinantly, although subsequently replicated in vivo, are still considered recombinant or isolated for the purposes of the invention. As used herein a "polynucleotide" or "nucleic acid" is a polymeric form of nucleotides of any length, either ribonucleotides or deoxyribonucleotides. This term refers only to the primary structure of the molecule. Thus, this term includes double- and single-stranded DNA and RNA. It also includes known types of modifications, for example, labels which are known in the art, methylation, "caps", substitution of one or more of the naturally occurring nucleotides with an analog, internucleotide modifications such as, for example, those with uncharged linkages (e.g., phosphorothioates, phosphorodithioates, etc.), those containing pendant moieties, such as, for example proteins (including e.g., nucleases, toxins, antibodies, signal peptides, poly-L-lysine, etc.),those with intercalators (e.g., acridine, psoralen, etc.), those containing chelators (e.g., metals, radioactive metals, etc.), those containing alkylators, those with modified linkages (e.g., alpha anomeric nucleic acids, etc.), as well as unmodified forms of the polynucleotide.

As used herein, a polynucleotide "derived from" a designated sequence refers to a polynucleotide sequence which is comprised of a sequence of approximately at least about 6 nucleotides, preferably at least about 8 nucleotides, more preferably at least about 10-12 nucleotides, and even more preferably at least about 15-20 nucleotides corresponding to a region of the designated nucleotide sequence. "Corresponding" means homologous to or complementary to the designated sequence. Preferably, the sequence of the region from which the polynucleotide is derived is homologous to or complementary to a sequence that is unique to a CA gene.

Similarly, a "recombinant protein" is a protein made using recombinant techniques, i.e. through the expression of a recombinant nucleic acid as depicted above. A recombinant protein is distinguished from naturally occurring protein by at least one or more characteristics. For example, the protein may be isolated or purified away from some or all of the proteins and compounds with which it is normally associated in its wild type host, and thus may be substantially pure. For example, an isolated protein is unaccompanied by at least some of the material with which it is normally associated in its natural state, preferably constituting at least about 0.5%, more preferably at least about 5% by weight of the total protein in a given sample. A substantially pure protein comprises about 50-75% by weight of the total protein, with about 80% being preferred, and about 90% being particularly preferred. The definition includes the production of a CA protein from one organism in a different organism or host cell. Alternatively, the protein may be made at a significantly higher concentration than is normally seen, through the use of an inducible promoter or high expression promoter, such that the protein is made at increased concentration levels. Alternatively, the protein may be in a form not normally found in nature, as in the addition of an epitope tag or amino acid substitutions, insertions and deletions, as discussed below.

In a preferred embodiment, the CA sequences are nucleic acids. As will be appreciated by those in the art and is more fully outlined below, CA sequences are useful in a variety of applications, including diagnostic applications, which will detect naturally occurring nucleic acids, as well as screening applications; for example, biochips comprising nucleic acid probes to the CA sequences can be generated. In the broadest sense, use of "nucleic acid," "polynucleotide" or "oligonucleotide" or equivalents herein means at least two nucleotides covalently linked together. In some embodiments, an oligonucleotide is an oligomer of 6, 8, 10, 12, 20, 30 or up to 100 nucleotides. A "polynucleotide" or "oligonucleotide" may comprise DNA, RNA, PNA or a polymer of nucleotides linked by phosphodiester and/or any alternate bonds.

A nucleic acid of the present invention generally contains phosphodiester bonds, although in some cases, as outlined below (for example, in antisense applications or when a nucleic acid is a candidate drug agent), nucleic acid analogs may have alternate backbones, comprising, for example, phosphoramidate (Beaucage et al., Tetrahedron 49 (10):1925 (1993) and references therein; Letsinger, J. Org. Chem. 35:3800 (1970); Sprinzl et al., Eur. J. Biochem. 81: 579 (1977); Letsinger et al., Nucl. Acids Res. 14:3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Patent No. 5,644,048), phosphorodithioate (Briu et al., J. Am. Chem. Soc. 111:2321 (1989), O-methylphosphoroamidite linkages (see Eckstein, Oligonucleotides and Analogues: A Practical Approach, Oxford University Press), and peptide nucleic acid backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996), all of which are incorporated by reference). Other analog nucleic acids include those with positive backbones (Denpcy et al., Proc. Natl. Acad. Sci. USA 92:6097 (1995); non-ionic backbones (U.S. Patent Nos. 5,386,023, 5,637,684,5,602,240,5,216,141 and 4,469,863; Kiedrowski et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y.S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett. 37:743 (1996)) and non-ribose backbones, including those described in U.S. Patent Nos. 5,235,033 and 5,034,506, and Chapters 6 and 7, ASC Symposium Series 580, "Carbohydrate Modifications in Antisense Research", Ed. Y.S. Sanghui and P. Dan Cook. Nucleic acids containing one or more carbocyclic sugars are also included within one definition of nucleic acids (see Jenkins et al., Chem. Soc. Rev. (1995) pp169-176). Several nucleic acid analogs are described in Rawls, C & E News June 2, 1997 page 35. All of these references are hereby expressly incorporated by reference. These modifications of the ribose-phosphate backbone may be done for a variety of reasons, for example to increase the stability and half-life of such molecules in physiological environments for use in anti-sense applications or as probes on a biochip.

As will be appreciated by those in the art, all of these nucleic acid analogs may find use in the present invention. In addition, mixtures of naturally occurring nucleic acids and analogs can be made; alternatively, mixtures of different nucleic acid analogs, and mixtures of naturally occurring nucleic acids and analogs may be made.

The nucleic acids may be single stranded or double stranded, as specified, or contain portions of both double stranded or single stranded sequence. As will be appreciated by those in the art, the depiction of a single strand "Watson" also defines the sequence of the other strand "Crick"; thus the sequences described herein also includes the complement of the sequence. The nucleic acid may be DNA, both genomic and cDNA, RNA, or a hybrid, where the nucleic acid contains any combination of deoxyribo- and ribo-nucleotides, and any combination of bases, including uracil, adenine, thymine, cytosine, guanine, inosine, xanthine, hypoxanthine, isocytosine, isoguanine, etc. As used herein, the term "nucleoside" includes nucleotides and nucleoside and nucleotide analogs, and modified nucleosides such as amino modified nucleosides. In addition, "nucleoside" includes non-naturally occurring analog structures. Thus for example the individual units of a peptide nucleic acid, each containing a base, are referred to herein as a nucleoside.

As used herein, the term "tag," "sequence tag" or "primer tag sequence" refers to an oligonucleotide with specific nucleic acid sequence that serves to identify a batch of polynucleotides bearing such tags therein. Polynucleotides from the same biological source are covalently tagged with a specific sequence tag so that in subsequent analysis the polynucleotide can be identified according to its source of origin. The sequence tags also serve as primers for nucleic acid amplification reactions.

A "microarray" is a linear or two-dimensional array of preferably discrete regions, each having a defined area, formed on the surface of a solid support. The density of the discrete regions on a microarray is determined by the total numbers of target polynucleotides to be detected on the surface of a single solid phase support, preferably at least about 50/cm$^2$, more preferably at least about 100/cm$^2$, even more preferably at least about 500/cm$^2$, and still more preferably at least about 1,000/cm$^2$. As used herein, a DNA microarray is an array of oligonucleotide primers placed on a chip or other surfaces used to amplify or clone target polynucleotides. Since the position of each particular group of primers in the array is known, the identities of the target polynucleotides can be determined based on their binding to a particular position in the microarray.

A "linker" is a synthetic oligodeoxyribonucleotide that contains a restriction site. A linker may be blunt end-ligated onto the ends of DNA fragments to create restriction sites that can be used in the subsequent cloning of the fragment into a vector molecule.

The term "label" refers to a composition capable of producing a detectable signal indicative of the presence of the target polynucleotide in an assay sample. Suitable labels include radioisotopes, nucleotide chromophores, enzymes, substrates, fluorescent molecules, chemiluminescent moieties, magnetic particles, bioluminescent moieties, and the like. As such, a label is any composition detectable by spectroscopic, photochemical, biochemical, immunochemical, electrical, optical, chemical, or any other appropriate means. The term "label" is used to refer to any chemical group or moiety having a detectable physical property or any compound capable of causing a chemical group or moiety to exhibit a detectable physical property, such as an enzyme that catalyzes conversion of a substrate into a detectable product. The term "label" also encompasses compounds that inhibit the expression of a particular physical property. The label may also be a compound that is a member of a binding pair, the other member of which bears a detectable physical property.

The term "support" refers to conventional supports such as beads, particles, dipsticks, fibers, filters, membranes, and silane or silicate supports such as glass slides.

The term "amplify" is used in the broad sense to mean creating an amplification product which may include, for example, additional target molecules, or target-like molecules or molecules complementary to the target molecule, which molecules are created by virtue of the presence of the target molecule in the sample. In the situation where the target is a nucleic acid, an amplification product can be made enzymatically with DNA or RNA polymerases or reverse transcriptases.

As used herein, a "biological sample" refers to a sample of tissue or fluid isolated from an individual, including but not limited to, for example, blood, plasma, serum, spinal fluid, lymph fluid, skin, respiratory, intestinal and genitourinary tracts, tears, saliva, milk, cells (including but not limited to blood cells), tumors, organs, and also samples of *in vitro* cell culture constituents.

The term "biological sources" as used herein refers to the sources from which the target polynucleotides are derived. The source can be of any form of "sample" as described above, including but not limited to, cell, tissue or fluid. "Different biological sources" can refer to different cells/tissues/organs of the same individual, or cells/tissues/organs from different individuals of the same species, or cells/tissues/organs from different species.

**Cancer-associated Sequences**

The CA sequences of the invention were initially identified by infection of mice with a retrovirus such as murine leukemia virus (MLV) resulting in lymphoma. Retroviruses have a genome that is made out of RNA. After a retrovirus infects a host cell, a double stranded DNA copy of the retrovirus genome (a "provirus") is inserted into the genomic DNA of the host cell. The integrated provirus may affect the expression of host genes at or near the site of integration - a phenomenon known as retroviral insertional mutagenesis. Possible changes in the expression of host cell genes include: (i) increased expression of genes near the site of integration resulting from the proximity of elements in the provirus that act as transcriptional promoters and enhancers, (ii) functional inactivation of a gene caused by the integration of a provirus into the gene itself thus preventing the synthesis of a functional gene product, or (iii) expression of a mutated protein that has a different activity to the normal protein. Typically such a protein would be prematurely truncated and lack a regulatory domain near the C terminus. Such a protein might be constitutively active, or act as a dominant negative inhibitor of the normal protein. For example, retrovirus enhancers, including that of SL3-3, are known to act on genes up to approximately 200 kilobases from the insertion site. Moreover, many of these sequences are also involved in other cancers and disease states. Sequences of mouse genes according to this invention, that are identified in this manner are shown as mDxx-yyy in Tables 1-7.

A CA sequence can be initially identified by substantial nucleic acid and/or amino acid sequence homology to the CA sequences outlined herein. Such homology can be based upon the overall nucleic acid or amino acid sequence, and is generally determined as outlined below, using either homology programs or hybridization conditions.

In one embodiment, CA sequences are those that are up-regulated in cancers; that is, the expression of these genes is higher in cancer tissue as compared to normal tissue of the same differentiation stage. "Up-regulation" as used herein means increased expression by about 50%, preferably about 100%, more preferably about 150% to about 200%, with up-regulation from 300% to 1000% being preferred.

In another embodiment, CA sequences are those that are down-regulated in cancers; that is, the expression of these genes is lower in cancer tissue as compared to normal tissue of the same differentiation stage. "Down-regulation" as used herein means decreased expression by about 50%, preferably about 100%, more preferably about 150% to about 200%, with down-regulation from 300% to 1000% to no expression being preferred.

In yet another embodiment, CA sequences are those that have altered sequences but show either the same or an altered expression profile as compared to normal lymphoid tissue of the same differentiation stage. "Altered CA sequences" as used herein also refers to sequences that are truncated, contain insertions or contain point mutations.

CA proteins of the present invention may be classified as secreted proteins, transmembrane proteins or intracellular proteins. In a preferred embodiment the CA protein is an intracellular protein. Intracellular proteins may be found in the cytoplasm and/or in the nucleus. Intracellular proteins are involved in all aspects of cellular function and replication (including, for example, signaling pathways); aberrant expression of such proteins results in unregulated or disregulated cellular processes. For example, many intracellular proteins have enzymatic activity such as protein kinase activity, protein phosphatase activity, protease activity, nucleotide cyclase activity, polymerase activity and the like. Intracellular proteins also serve as docking proteins that are involved in organizing complexes of proteins, or targeting proteins to various subcellular localizations, and are involved in maintaining the structural integrity of organelles.

An increasingly appreciated concept in characterizing intracellular proteins is the presence in the proteins of one or more motifs for which defined functions have been attributed. In addition to the highly conserved sequences found in the enzymatic domain of proteins, highly conserved sequences have been identified in proteins that are involved in protein-protein interaction. For example, Src-homology-2 (SH2) domains bind tyrosine-phosphorylated targets in a sequence dependent manner. PTB domains, which are distinct from SH2 domains, also bind tyrosine phosphorylated targets. SH3 domains bind to proline-rich targets. In addition, PH domains, tetratricopeptide repeats and WD domains to name only a few, have been shown to mediate protein-protein interactions. Some of these may also be involved in binding to phospholipids or other second messengers. As will be appreciated by one of ordinary skill in the art, these motifs can be identified on the basis of primary sequence; thus, an analysis of the sequence of proteins may provide insight into both the enzymatic potential of the molecule and/or molecules with which the protein may associate.

In a preferred embodiment, the CA sequences are transmembrane proteins. Transmembrane proteins are molecules that span the phospholipid bilayer of a cell. They may have an intracellular domain, an extracellular domain, or both. The intracellular domains of such proteins may have a number of functions including those already described for intracellular proteins. For example, the intracellular domain may have enzymatic activity and/or may serve as a binding site for additional proteins. Frequently the intracellular domain of transmembrane proteins serves both roles. For example certain receptor tyrosine kinases have both protein kinase activity and SH2 domains. In addition, autophosphorylation of tyrosines on the receptor molecule itself creates binding sites for additional SH2 domain containing proteins.

Transmembrane proteins may contain from one to many transmembrane domains. For example, receptor tyrosine kinases, certain cytokine receptors, receptor guanylyl cyclases and receptor serine/threonine protein kinases contain a single transmembrane domain. However, various other proteins including channels and adenylyl cyclases contain numerous transmembrane domains. Many important cell surface receptors are classified as "seven transmembrane domain" proteins, as they contain 7 membrane spanning regions. Important transmembrane protein receptors include, but are not limited to insulin receptor, insulin-like growth factor receptor, human growth hormone receptor, glucose transporters, transferrin receptor, epidermal growth factor receptor, low density lipoprotein receptor, leptin receptor, interleukin receptors, e.g. IL-1 receptor, IL-2 receptor, etc. CA proteins may be derived from genes that regulate apoptosis (IL-3, GM-CSF and Bcl-x) or are shown to have a role in the regulation of apoptosis.

Characteristics of transmembrane domains include approximately 20 consecutive hydrophobic amino acids that may be followed by charged amino acids. Therefore, upon analysis of the amino acid sequence of a particular protein, the localization and number of transmembrane domains within the protein may be predicted.

The extracellular domains of transmembrane proteins are diverse; however, conserved motifs are found repeatedly among various extracellular domains. Conserved structure and/or functions have been ascribed to different extracellular motifs. For example, cytokine receptors are characterized by a cluster of cysteines and a WSXWS (W= tryptophan, S= serine, X=any amino acid) motif. Immunoglobulin-like domains are highly conserved. Mucin-like domains may be involved in cell adhesion and leucine-rich repeats participate in protein-protein interactions.

Many extracellular domains are involved in binding to other molecules. In one aspect, extracellular domains are receptors. Factors that bind the receptor domain include circulating ligands, which may be peptides, proteins, or small molecules such as adenosine and the like. For example, growth factors such as EGF, FGF and PDGF are circulating growth factors that bind to their cognate receptors to initiate a variety of cellular responses. Other factors include cytokines, mitogenic factors, neurotrophic factors and the like. Extracellular domains also bind to cell-associated molecules. In this respect, they mediate cell-cell interactions. Cell-associated ligands can be tethered to the cell for example via a glycosylphosphatidylinositol (GPI) anchor, or may themselves be transmembrane proteins. Extracellular domains also associate with the extracellular matrix and contribute to the maintenance of the cell structure.

CA proteins that are transmembrane are particularly preferred in the present invention as they are good targets for immunotherapeutics, as are described herein. In addition, as outlined below, transmembrane proteins can be also useful in imaging modalities.

It will also be appreciated by those in the art that a transmembrane protein can be made soluble by removing transmembrane sequences, for example through recombinant methods. Furthermore, transmembrane proteins that have been made soluble can be made to be secreted through recombinant means by adding an appropriate signal sequence.

In a preferred embodiment, the CA proteins are secreted proteins; the secretion of which can be either constitutive or regulated. These proteins have a signal peptide or signal sequence that targets the molecule to the secretory pathway. Secreted proteins are involved in numerous physiological events; by virtue of their circulating nature, they serve to transmit signals to various other cell types. The secreted protein may function in an autocrine manner (acting on the cell that secreted the factor), a paracrine manner (acting on cells in close proximity to the cell that secreted the factor) or an endocrine manner (acting on cells at a distance). Thus secreted molecules find use in modulating or altering numerous aspects of physiology. CA proteins that are secreted proteins are particularly preferred in the present invention as they serve as good targets for diagnostic markers, for example for blood tests.

**CA sequences and homologs**

A CA sequence is initially identified by substantial nucleic acid and/or amino acid sequence homology to the CA sequences outlined herein. Such homology can be based upon the overall nucleic acid or amino acid sequence, and is generally determined as outlined below, using either homology programs or hybridization conditions.

As used herein, a nucleic acid is a "CA nucleic acid" if the overall homology of the nucleic acid sequence to one of the nucleic acids of Tables 1-7 is preferably greater than about 75%, more preferably greater than about 80%, even more preferably greater than about 85% and most preferably greater than 90%. In some embodiments the homology will be as high as about 93 to 95 or 98%. In a preferred embodiment, the sequences that are used to determine sequence identity or similarity are selected from those of the nucleic acids of Tables 1-7. In another embodiment, the sequences are naturally occurring allelic variants of the sequences of the nucleic acids of Tables 1-7. In another embodiment, the sequences are sequence variants as further described herein.

Homology in this context means sequence similarity or identity, with identity being preferred. A preferred comparison for homology purposes is to compare the sequence containing sequencing errors to the correct sequence. This homology will be determined using standard techniques known in the art, including, but not limited to, the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, PNAS USA 85:2444 (1988), by computerized implementations of these algorithms (GAP, BESTFIT, FASTA, and TFASTA in the Wisconsin Genetics Software Package, Genetics Computer Group, 575 Science Drive, Madison, WI), the Best Fit sequence program described by Devereux et al., Nucl. Acid Res. 12:387-395 (1984), preferably using the default settings, or by inspection.

One example of a useful algorithm is PILEUP. PILEUP creates a multiple sequence alignment from a group of related sequences using progressive, pairwise alignments. It can also plot a tree showing the clustering relationships used to create the alignment. PILEUP uses a simplification of the progressive alignment method of Feng & Doolittle, J. Mol. Evol. 35:351-360 (1987); the method is similar to that described by Higgins & Sharp CABIOS 5:151-153 (1989). Useful PILEUP parameters include a default gap weight of 3.00, a default gap length weight of 0.10, and weighted end gaps.

Another example of a useful algorithm is the BLAST (Basic Local Alignment Search Tool) algorithm, described in Altschul et al., J. Mol. Biol. 215, 403-410, (1990) and Karlin et al., PNAS USA 90:5873-5787 (1993). A particularly useful BLAST program is the WU-BLAST-2 program which was obtained from Altschul et al., Methods in Enzymology, 266: 460-480 (1996); http://blast.wustl.edu/]. WU-BLAST-2 uses several search parameters, most of which are set to the default values. The adjustable parameters are set with the following values: overlap span =1, overlap fraction = 0.125, word threshold (T) = 11. The HSP S and HSP S2 parameters are dynamic values and are established by the program itself depending upon the composition of the particular sequence and composition of the particular database against which the sequence of interest is being searched; however, the values may be adjusted to increase sensitivity. A percent amino acid sequence identity value is determined by the number of matching identical residues divided by the total number of residues of the "longer" sequence in the aligned region. The "longer" sequence is the one having the most actual residues in the aligned region (gaps introduced by WU-Blast-2 to maximize the alignment score are ignored).

Thus, "percent (%) nucleic acid sequence identity" is defined as the percentage of nucleotide residues in a candidate sequence that are identical with the nucleotide residues of the nucleic acids of Tables 1-7. A preferred method utilizes the BLASTN module of WU-BLAST-2 set to the default parameters, with overlap span and overlap fraction set to 1 and 0.125, respectively.

The alignment may include the introduction of gaps in the sequences to be aligned. In addition, for sequences which contain either more or fewer nucleotides than those of the nucleic acids of Tables 1-7, it is understood that the percentage of homology will be determined based on the number of homologous nucleosides in relation to the total number of nucleosides. Thus homology of sequences shorter than those of the sequences identified herein will be determined using the number of nucleosides in the shorter sequence.

In another embodiment of the invention, polynucleotide compositions are provided that are capable of hybridizing under moderate to high stringency conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5x SSC ("saline sodium citrate"; 9 mM NaCl, 0.9 mM sodium citrate), 0.5% SDS, 1.0 mM EDTA (pH 8.0); hybridizing at 50-60° C, 5x SSC, overnight; followed by washing twice at 65° C for 20 minutes with each of 2x, 0.5x and 0.2x SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, e.g., to 60-65° C, or 65-70° C. Stringent conditions may also be achieved with the addition of destabilizing agents such as formamide.

Thus nucleic acids that hybridize under high stringency to the nucleic acids identified in the figures, or their complements, are considered CA sequences. High stringency conditions are known in the art; see for example Maniatis et al., Molecular Cloning: A Laboratory Manual, 2d Edition, 1989, and Short Protocols in Molecular Biology, ed. Ausubel, et al., both of which are hereby incorporated by reference. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Acid Probes, "Overview of principles of hybridization and the strategy of nucleic acid assays" (1993). Generally, stringent conditions are selected to be about 5-10°C lower than the thermal melting point ($T_m$) for the specific sequence at a defined ionic strength pH. The $T_m$ is the temperature (under defined ionic strength, pH and nucleic acid concentration) at which 50% of the probes complementary to the target hybridize to the target sequence at equilibrium (as the target sequences are present in excess, at $T_m$, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g. 10 to 50 nucleotides) and at least about 60°C for longer probes (e.g. greater than 50 nucleotides). In another embodiment, less stringent hybridization conditions are used; for example, moderate or low stringency conditions may be used, as are known in the art; see Maniatis and Ausubel, supra, and Tijssen, supra.

In addition, the CA nucleic acid sequences of the invention are fragments of larger genes, i.e. they are nucleic acid segments. Alternatively, the CA nucleic acid sequences can serve as indicators of oncogene position, for example, the CA sequence may be an enhancer that activates a protooncogene. "Genes" in this context includes coding regions, non-coding regions, and mixtures of coding and non-coding regions. Accordingly, as will be appreciated by those in the art, using the sequences provided herein, additional sequences of the CA genes can be obtained, using techniques well known in the art for cloning either longer sequences or the full-length sequences; see Maniatis et al., and Ausubel, et al., supra, hereby expressly incorporated by reference. In general, this is done using PCR, for example, kinetic PCR.

**Detection of CA Expression**

Once the CA nucleic acid is identified, it can be cloned and, if necessary, its constituent parts recombined to form the entire CA nucleic acid. Once isolated from its natural source, e.g., contained within a plasmid or other vector or excised therefrom as a linear nucleic acid segment, the recombinant CA nucleic acid can be further used as a probe to identify and isolate other CA nucleic acids, for example additional coding regions. It can also be used as a "precursor" nucleic acid to make modified or variant CA nucleic acids and proteins. In a preferred embodiment, once a CA gene is identified its nucleotide sequence is used to design probes specific for the CA gene.

The CA nucleic acids of the present invention are used in several ways. In a first embodiment, nucleic acid probes hybridizable to CA nucleic acids are made and attached to biochips to be used in screening and diagnostic methods, or for gene therapy and/or antisense applications. Alternatively, the CA nucleic acids that include coding regions of CA proteins can be put into expression vectors for the expression of CA proteins, again either for screening purposes or for administration to a patient.

Recent developments in DNA microarray technology make it possible to conduct a large scale assay of a plurality of target CA nucleic acid molecules on a single solid phase support. U.S. Pat. No. 5,837,832 (Chee et al.) and related patent applications describe immobilizing an array of oligonucleotide probes for hybridization and detection of specific nucleic acid sequences in a sample. Target polynucleotides of interest isolated from a tissue of interest are hybridized to the DNA chip and the specific sequences detected based on the target polynucleotides' preference and degree of hybridization at discrete probe locations. One important use of arrays is in the analysis of differential gene expression, where the profile of expression of genes in different cells, often a cell of interest and a control cell, is compared and any differences in gene expression among the respective cells are identified. Such information is useful for the identification of the types of genes expressed in a particular cell or tissue type and diagnosis of cancer conditions based on the expression profile.

Typically, RNA from the sample of interest is subjected to reverse transcription to obtain labeled cDNA. *See* U.S. Pat. No. 6,410,229 (Lockhart et al.) The cDNA is then hybridized to oligonucleotides or cDNAs of known sequence arrayed on a chip or other surface in a known order. The location of the oligonucleotide to which the labeled cDNA hybridizes provides sequence information on the cDNA, while the amount of labeled hybridized RNA or cDNA provides an estimate of the relative representation of the RNA or cDNA of interest. See Schena, et al. Science 270:467-470 (1995). For example, use of a cDNA microarray to analyze gene expression patterns in human cancer is described by DeRisi, et al. (Nature Genetics 14:457-460 (1996)).

In a preferred embodiment, nucleic acid probes corresponding to CA nucleic acids (both the nucleic acid sequences outlined in the figures and/or the complements thereof) are made. Typically, these probes are synthesized based on the disclosed sequences of this invention. The nucleic acid probes attached to the biochip are designed to be substantially complementary to the CA nucleic acids, i.e. the target sequence (either the target sequence of the sample or to other probe sequences, for example in sandwich assays), such that specific hybridization of the target sequence and the probes of the present invention occurs. As outlined below, this complementarity need not be perfect, in that there may be any number of base pair mismatches that will interfere with hybridization between the target sequence and the single stranded nucleic acids of the present invention. It is expected that the overall homology of the genes at the nucleotide level probably will be about 40% or greater, probably about 60% or greater, and even more probably about 80% or greater; and in addition that there will be corresponding contiguous sequences of about 8-12 nucleotides or longer. However, if the number of mutations is so great that no hybridization can occur under even the least stringent of hybridization conditions, the sequence is not a complementary target sequence. Thus, by "substantially complementary" herein is meant that the probes are sufficiently complementary to the target sequences to hybridize under normal reaction conditions, particularly high stringency conditions, as outlined herein. Whether or not a sequence is unique to a CA gene according to this invention can be determined by techniques known to those of skill in the art. For example, the sequence can be compared to sequences in databanks, e.g., GeneBank, to determine whether it is present in the uninfected host or other organisms. The sequence can also be compared to the known sequences of other viral agents, including those that are known to induce cancer.

A nucleic acid probe is generally single stranded but can be partly single and partly double stranded. The strandedness of the probe is dictated by the structure, composition, and properties of the target sequence. In general, the oligonucleotide probes range from about 6, 8, 10, 12, 15, 20, 30 to about 100 bases long, with from about 10 to about 80 bases being preferred, and from about 30 to about 50 bases being particularly preferred. That is, generally entire genes are rarely used as probes. In some embodiments, much longer nucleic acids can be used, up to hundreds of bases. The probes are sufficiently specific to hybridize to complementary template sequence under conditions known by those of skill in the art. The number of mismatches between the probes sequences and their complementary template (target) sequences to which they hybridize during hybridization generally do not exceed 15%, usually do not exceed 10% and preferably do not exceed 5%, as determined by FASTA (default settings).

Oligonucleotide probes can include the naturally-occurring heterocyclic bases normally found in nucleic acids (uracil, cytosine, thymine, adenine and guanine), as well as modified bases and base analogues. Any modified base or base analogue compatible with hybridization of the probe to a target sequence is useful in the practice of the invention. The sugar or glycoside portion of the probe can comprise deoxyribose, ribose, and/or modified forms of these sugars, such as, for example, 2'-O-alkyl ribose. In a preferred embodiment, the sugar moiety is 2'-deoxyribose; however, any sugar moiety that is compatible with the ability of the probe to hybridize to a target sequence can be used.

In one embodiment, the nucleoside units of the probe are linked by a phosphodiester backbone, as is well known in the art. In additional embodiments, internucleotide linkages can include any linkage known to one of skill in the art that is compatible with specific hybridization of the probe including, but not limited to phosphorothioate, methylphosphonate, sulfamate (*e.g.,* U.S. Patent No. 5,470,967) and polyamide (*i.e.*, peptide nucleic acids). Peptide nucleic acids are described in Nielsen et al. (1991) Science 254: 1497-1500, U.S. Patent No. 5,714,331, and Nielsen (1999) Curr. Opin. Biotechnol. 10:71-75.

In certain embodiments, the probe can be a chimeric molecule; *i.e.,* can comprise more than one type of base or sugar subunit, and/or the linkages can be of more than one type within the same primer. The probe can comprise a moiety to facilitate hybridization to its target sequence, as are known in the art, for example, intercalators and/or minor groove binders. Variations of the bases, sugars, and internucleoside backbone, as well as the presence of any pendant group on the probe, will be compatible with the ability of the probe to bind, in a sequence-specific fashion, with its target sequence. A large number of structural modifications, both known and to be developed, are possible within these bounds. Advantageously, the probes according to the present invention may have structural characteristics such that they allow the signal amplification, such structural characteristics being, for example, branched DNA probes as those described by Urdea et al. (Nucleic Acids Symp. Ser., 24:197-200 (1991)) or in the European Patent No. EP-0225,807. Moreover, synthetic methods for preparing the various heterocyclic bases, sugars, nucleosides and nucleotides that form the probe, and preparation of oligonucleotides of specific predetermined sequence, are well-developed and known in the art. A preferred method for oligonucleotide synthesis incorporates the teaching of U.S. Patent No. 5,419,966.

Multiple probes may be designed for a particular target nucleic acid to account for polymorphism and/or secondary structure in the target nucleic acid, redundancy of data and the like. In some embodiments, where more than one probe per sequence is used, either overlapping probes or probes to different sections of a single target CA gene are used. That is, two, three, four or more probes, with three being preferred, are used to build in a redundancy for a particular target. The probes can be overlapping (i.e. have some sequence in common), or specific for distinct sequences of a CA gene. When multiple target polynucleotides are to be detected according to the present invention, each probe or probe group corresponding to a particular target polynucleotide is situated in a discrete area of the microarray.

Probes may be in solution, such as in wells or on the surface of a microarray, or attached to a solid support. Examples of solid support materials that can be used include a plastic, a ceramic, a metal, a resin, a gel and a membrane. Useful types of solid supports include plates, beads, magnetic material, microbeads, hybridization chips, membranes, crystals, ceramics and self-assembling monolayers. A preferred embodiment comprises a two-dimensional or three-dimensional matrix, such as a gel or hybridization chip with multiple probe binding sites (Pevzner et al., J. Biomol. Struc. & Dyn. 9:399-410, 1991; Maskos and Southern, Nuc. Acids Res. 20:1679-84, 1992). Hybridization chips can be used to construct very large probe arrays that are subsequently hybridized with a target nucleic acid. Analysis of the hybridization pattern of the chip can assist in the identification of the target nucleotide sequence. Patterns can be manually or computer analyzed, but it is clear that positional sequencing by hybridization lends itself to computer analysis and automation. Algorithms and software, which have been developed for sequence reconstruction, are applicable to the methods described herein (R. Drmanac et al., J. Biomol. Struc. & Dyn. 5:1085-1102, 1991; P. A. Pevzner, J. Biomol. Struc. & Dyn. 7:63-73, 1989).

As will be appreciated by those in the art, nucleic acids can be attached or immobilized to a solid support in a wide variety of ways. By "immobilized" herein is meant the association or binding between the nucleic acid probe and the solid support is sufficient to be stable under the conditions of binding, washing, analysis, and removal as outlined below. The binding can be covalent or non-covalent. By "non-covalent binding" and grammatical equivalents herein is meant one or more of either electrostatic, hydrophilic, and hydrophobic interactions. Included in non-covalent binding is the covalent attachment of a molecule, such as streptavidin, to the support and the non-covalent binding of the biotinylated probe to the streptavidin. By "covalent binding" and grammatical equivalents herein is meant that the two moieties, the solid support and the probe, are attached by at least one bond, including sigma bonds, pi bonds and coordination bonds. Covalent bonds can be formed directly between the probe and the solid support or can be formed by a cross linker or by inclusion of a specific reactive group on either the solid support or the probe or both molecules. Immobilization may also involve a combination of covalent and non-covalent interactions.

Nucleic acid probes may be attached to the solid support by covalent binding such as by conjugation with a coupling agent or by, covalent or non-covalent binding such as electrostatic interactions, hydrogen bonds or antibody-antigen coupling, or by combinations thereof. Typical coupling agents include biotin/avidin, biotin/streptavidin, Staphylococcus aureus protein A/IgG antibody $F_c$ fragment, and streptavidin/protein A chimeras (T. Sano and C. R. Cantor, Bio/Technology 9:1378-81 (1991)), or derivatives or combinations of these agents. Nucleic acids may be attached to the solid support by a photocleavable bond, an electrostatic bond, a disulfide bond, a peptide bond, a diester bond or a combination of these sorts of bonds. The array may also be attached to the solid support by a selectively releasable bond such as 4,4'-dimethoxytrityl or its derivative. Derivatives which have been found to be useful include 3 or 4 [bis-(4-methoxyphenyl)]-methyl-benzoic acid, N-succinimidyl-3 or 4 [bis-(4-methoxyphenyl)]-methyl-benzoic acid, N-succinimidyl-3 or 4 [bis-(4-methoxyphenyl)]-hydroxymethyl-benzoic acid, N-succinimidyl-3 or 4 [bis-(4-methoxyphenyl)]-chloromethyl-benzoic acid, and salts of these acids.

In general, the probes are attached to the biochip in a wide variety of ways, as will be appreciated by those in the art. As described herein, the nucleic acids can either be synthesized first, with subsequent attachment to the biochip, or can be directly synthesized on the biochip.

The biochip comprises a suitable solid substrate. By "substrate" or "solid support" or other grammatical equivalents herein is meant any material that can be modified to contain discrete individual sites appropriate for the attachment or association of the nucleic acid probes and is amenable to at least one detection method. The solid phase support of the present invention can be of any solid materials and structures suitable for supporting nucleotide hybridization and synthesis. Preferably, the solid phase support comprises at least one substantially rigid surface on which the primers can be immobilized and the reverse transcriptase reaction performed. The substrates with which the polynucleotide microarray elements are stably associated may be fabricated from a variety of materials, including plastics, ceramics, metals, acrylamide, cellulose, nitrocellulose, glass, polystyrene, polyethylene vinyl acetate, polypropylene, polymethacrylate, polyethylene, polyethylene oxide, polysilicates, polycarbonates, Teflon@, fluorocarbons, nylon, silicon rubber, polyanhydrides, polyglycolic acid, polylactic acid, polyorthoesters, polypropylfumerate, collagen, glycosaminoglycans, and polyamino acids. Substrates may be two-dimensional or three-dimensional in form, such as gels, membranes, thin films, glasses, plates, cylinders, beads, magnetic beads, optical fibers, woven fibers, etc. A preferred form of array is a three-dimensional array. A preferred three-dimensional array is a collection of tagged beads. Each tagged bead has different primers attached to it. Tags are detectable by signaling means such as color (Luminex, Illumina) and electromagnetic field (Pharmaseq) and signals on tagged beads can even be remotely detected (e.g., using optical fibers). The size of the solid support can be any of the standard microarray sizes, useful for DNA microarray technology, and the size may be tailored to fit the particular machine being used to conduct a reaction of the invention. In general, the substrates allow optical detection and do not appreciably fluoresce.

In a preferred embodiment, the surface of the biochip and the probe may be derivatized with chemical functional groups for subsequent attachment of the two. Thus, for example, the biochip is derivatized with a chemical functional group including, but not limited to, amino groups, carboxy groups, oxo groups and thiol groups, with amino groups being particularly preferred. Using these functional groups, the probes can be attached using functional groups on the probes. For example, nucleic acids containing amino groups can be attached to surfaces comprising amino groups, for example using linkers as are known in the art; for example, homo-or hetero-bifunctional linkers as are well known (see 1994 Pierce Chemical Company catalog, technical section on cross-linkers, pages 155-200, incorporated herein by reference). In addition, in some cases, additional linkers, such as alkyl groups (including substituted and heteroalkyl groups) may be used.

In this embodiment, the oligonucleotides are synthesized as is known in the art, and then attached to the surface of the solid support. As will be appreciated by those skilled in the art, either the 5' or 3' terminus may be attached to the solid support, or attachment may be via an internal nucleoside. In an additional embodiment, the immobilization to the solid support may be very strong, yet non-covalent. For example, biotinylated oligonucleotides can be made, which bind to surfaces covalently coated with streptavidin, resulting in attachment.

The arrays may be produced according to any convenient methodology, such as preforming the polynucleotide microarray elements and then stably associating them with the surface. Alternatively, the oligonucleotides may be synthesized on the surface, as is known in the art. A number of different array configurations and methods for their production are known to those of skill in the art and disclosed in WO 95/25116 and WO 95/35505 (photolithographic techniques), U.S. Pat. No. 5,445,934 (in situ synthesis by photolithography), U.S. Pat. No. 5,384,261 (in situ synthesis by mechanically directed flow paths); and U.S. Pat. No. 5,700,637 (synthesis by spotting, printing or coupling); the disclosure of which are herein incorporated in their entirety by reference. Another method for coupling DNA to beads uses specific ligands attached to the end of the DNA to link to ligand-binding molecules attached to a bead. Possible ligand-binding partner pairs include biotin-avidin/streptavidin, or various antibody/antigen pairs such as digoxygenin-antidigoxygenin antibody (Smith et al., "Direct Mechanical Measurements of the Elasticity of Single DNA Molecules by Using Magnetic Beads," Science 258:1122-1126 (1992)). Covalent chemical attachment of DNA to the support can be accomplished by using standard coupling agents to link the 5'-phosphate on the DNA to coated microspheres through a phosphoamidate bond. Methods for immobilization of oligonucleotides to solid-state substrates are well established. See Pease et al., Proc. Natl. Acad. Sci. USA 91(11):5022-5026 (1994). A preferred method of attaching oligonucleotides to solid-state substrates is described by Guo et al., Nucleic Acids Res. 22:5456-5465 (1994). Immobilization can be accomplished either by in situ DNA synthesis (Maskos and Southern, Nucleic Acids Research, 20:1679-1684 (1992) or by covalent attachment of chemically synthesized oligonucleotides (Guo et al., supra) in combination with robotic arraying technologies.

In addition to the solid-phase technology represented by biochip arrays, gene expression can also be quantified using liquid-phase arrays. One such system is kinetic polymerase chain reaction (PCR). Kinetic PCR allows for the simultaneous amplification and quantification of specific nucleic acid sequences. The specificity is derived from synthetic oligonucleotide primers designed to preferentially adhere to single-stranded nucleic acid sequences bracketing the target site. This pair of oligonucleotide primers form specific, non-covalently bound complexes on each strand of the target sequence. These complexes facilitate in vitro transcription of double-stranded DNA in opposite orientations. Temperature cycling of the reaction mixture creates a continuous cycle of primer binding, transcription, and re-melting of the nucleic acid to individual strands. The result is an exponential increase of the target dsDNA product. This product can be quantified in real time either through the use of an intercalating dye or a sequence specific probe. SYBR® Greene I, is an example of an intercalating dye, that preferentially binds to dsDNA resulting in a concomitant increase in the fluorescent signal. Sequence specific probes, such as used with TaqMan® technology, consist of a fluorochrome and a quenching molecule covalently bound to opposite ends of an oligonucleotide. The probe is designed to selectively bind the target DNA sequence between the two primers. When the DNA strands are synthesized during the PCR reaction, the fluorochrome is cleaved from the probe by the exonuclease activity of the polymerase resulting in signal dequenching. The probe signaling method can be more specific than the intercalating dye method, but in each case, signal strength is proportional to the dsDNA product produced. Each type of quantification method can be used in multi-well liquid phase arrays with each well representing primers and/or probes specific to nucleic acid sequences of interest. When used with messenger RNA preparations of tissues or cell lines, an array of probe/primer reactions can simultaneously quantify the expression of multiple gene products of interest. See Germer, S., et al., Genome Res. 10:258-266 (2000); Heid, C. A., et al., Genome Res. 6, 986-994 (1996).

**Expression of CA proteins**

In a preferred embodiment, CA nucleic acids encoding CA proteins are used to make a variety of expression vectors to express CA proteins which can then be used in screening assays, as described below. The expression vectors may be either self-replicating extrachromosomal vectors or vectors which integrate into a host genome. Generally, these expression vectors include transcriptional and translational regulatory nucleic acid operably linked to the nucleic acid encoding the CA protein. The term "control sequences" refers to DNA sequences necessary for the expression of an operably linked coding sequence in a particular host organism. The control sequences that are suitable for prokaryotes, for example, include a promoter, optionally an operator sequence, and a ribosome binding site. Eukaryotic cells are known to utilize promoters, polyadenylation signals, and enhancers.

Nucleic acid is "operably linked" when it is placed into a functional relationship with another nucleic acid sequence. For example, DNA for a presequence or secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" means that the DNA sequences being linked are contiguous, and, in the case of a secretory leader, contiguous and in reading phase. However, enhancers do not have to be contiguous. Linking is accomplished by ligation at convenient restriction sites. If such sites do not exist, synthetic oligonucleotide adaptors or linkers are used in accordance with conventional practice. The transcriptional and translational regulatory nucleic acid will generally be appropriate to the host cell used to express the CA protein; for example, transcriptional and translational regulatory nucleic acid sequences from *Bacillus* are preferably used to express the CA protein in *Bacillus*. Numerous types of appropriate expression vectors, and suitable regulatory sequences are known in the art for a variety of host cells.

In general, the transcriptional and translational regulatory sequences may include, but are not limited to, promoter sequences, ribosomal binding sites, transcriptional start and stop sequences, translational start and stop sequences, and enhancer or activator sequences. In a preferred embodiment, the regulatory sequences include a promoter and transcriptional start and stop sequences.

Promoter sequences encode either constitutive or inducible promoters. The promoters may be either naturally occurring promoters or hybrid promoters. Hybrid promoters, which combine elements of more than one promoter, are also known in the art, and are useful in the present invention.

In addition, the expression vector may comprise additional elements. For example, the expression vector may have two replication systems, thus allowing it to be maintained in two organisms, for example in mammalian or insect cells for expression and in a prokaryotic host for cloning and amplification. Furthermore, for integrating expression vectors, the expression vector contains at least one sequence homologous to the host cell genome, and preferably two homologous sequences that flank the expression construct. The integrating vector may be directed to a specific locus in the host cell by selecting the appropriate homologous sequence for inclusion in the vector. Constructs for integrating vectors are well known in the art.

In addition, in a preferred embodiment, the expression vector contains a selectable marker gene to allow the selection of transformed host cells. Selection genes are well known in the art and will vary with the host cell used.

The CA proteins of the present invention are produced by culturing a host cell transformed with an expression vector containing nucleic acid encoding a CA protein, under the appropriate conditions to induce or cause expression of the CA protein. The conditions appropriate for CA protein expression will vary with the choice of the expression vector and the host cell, and will be easily ascertained by one skilled in the art through routine experimentation. For example, the use of constitutive promoters in the expression vector will require optimizing the growth and proliferation of the host cell, while the use of an inducible promoter requires the appropriate growth conditions for induction. In addition, in some embodiments, the timing of the harvest is important. For example, the baculoviral systems used in insect cell expression are lytic viruses, and thus harvest time selection can be crucial for product yield.

Appropriate host cells include yeast, bacteria, archaebacteria, fungi, and insect, plant and animal cells, including mammalian cells. Of particular interest are *Drosophila melanogaster* cells, *Saccharomyces cerevisiae* and other yeasts, *E. coli, Bacillus subtilis,* Sf9 cells, C129 cells, 293 cells, *Neurospora*, BHK, CHO, COS, HeLa cells, THP1 cell line (a macrophage cell line) and human cells and cell lines.

In a preferred embodiment, the CA proteins are expressed in mammalian cells. Mammalian expression systems are also known in the art, and include retroviral systems. A preferred expression vector system is a retroviral vector system such as is generally described in PCT/US97/01019 and PCT/US97/01048, both of which are hereby expressly incorporated by reference. Of particular use as mammalian promoters are the promoters from mammalian viral genes, since the viral genes are often highly expressed and have a broad host range. Examples include the SV40 early promoter, mouse mammary tumor virus LTR promoter, adenovirus major late promoter, herpes simplex virus promoter, and the CMV promoter. Typically, transcription termination and polyadenylation sequences recognized by mammalian cells are regulatory regions located 3' to the translation stop codon and thus, together with the promoter elements, flank the coding sequence. Examples of transcription terminator and polyadenylation signals include those derived form SV40.

The methods of introducing exogenous nucleic acid into mammalian hosts, as well as other hosts, are well known in the art, and will vary with the host cell used. Techniques include dextran-mediated transfection, calcium phosphate precipitation, polybrene mediated transfection, protoplast fusion, electroporation, viral infection, encapsulation of the polynucleotide(s) in liposomes, and direct microinjection of the DNA into nuclei.

In a preferred embodiment, CA proteins are expressed in bacterial systems. Bacterial expression systems are well known in the art. Promoters from bacteriophage may also be used and are known in the art. In addition, synthetic promoters and hybrid promoters are also useful; for example, the tac promoter is a hybrid of the trp and lac promoter sequences. Furthermore, a bacterial promoter can include naturally occurring promoters of non-bacterial origin that have the ability to bind bacterial RNA polymerase and initiate transcription. In addition to a functioning promoter sequence, an efficient ribosome binding site is desirable. The expression vector may also include a signal peptide sequence that provides for secretion of the CA protein in bacteria. The protein is either secreted into the growth media (gram-positive bacteria) or into the periplasmic space, located between the inner and outer membrane of the cell (gram-negative bacteria). The bacterial expression vector may also include a selectable marker gene to allow for the selection of bacterial strains that have been transformed. Suitable selection genes include genes that render the bacteria resistant to drugs such as ampicillin, chloramphenicol, erythromycin, kanamycin, neomycin and tetracycline. Selectable markers also include biosynthetic genes, such as those in the histidine, tryptophan and leucine biosynthetic pathways. These components are assembled into expression vectors. Expression vectors for bacteria are well known in the art, and include vectors for *Bacillus subtilis, E. coli, Streptococcus cremoris*, and *Streptococcus lividans,* among others. The bacterial expression vectors are transformed into bacterial host cells using techniques well known in the art, such as calcium chloride treatment, electroporation, and others.

In one embodiment, CA proteins are produced in insect cells. Expression vectors for the transformation of insect cells, and in particular, baculovirus-based expression vectors, are well known in the art.

In a preferred embodiment, CA protein is produced in yeast cells. Yeast expression systems are well known in the art, and include expression vectors for *Saccharomyces cerevisiae, Candida albicans* and *C. maltosa, Hansenula polymorpha, Kluyveromyces fragilis* and *K. lactis, Pichia guillerimondii* and *P. pastoris, Schizosaccharomyces pombe,* and *Yarrowia lipolytica.*

The CA protein may also be made as a fusion protein, using techniques well known in the art. Thus, for example, for the creation of monoclonal antibodies. If the desired epitope is small, the CA protein may be fused to a carrier protein to form an immunogen. Alternatively, the CA protein may be made as a fusion protein to increase expression, or for other reasons. For example, when the CA protein is a CA peptide, the nucleic acid encoding the peptide may be linked to other nucleic acid for expression purposes.

In one embodiment, the CA nucleic acids, proteins and antibodies of the invention are labeled. By "labeled" herein is meant that a compound has at least one element, isotope or chemical compound attached to enable the detection of the compound. In general, labels fall into three classes: a) isotopic labels, which may be radioactive or heavy isotopes; b) immune labels, which may be antibodies or antigens; and c) colored or fluorescent dyes. The labels may be incorporated into the CA nucleic acids, proteins and antibodies at any position. For example, the label should be capable of producing, either directly or indirectly, a detectable signal. The detectable moiety may be a radioisotope, such as $^3$H, $^{14}$C, $^{32}$P, $^{35}$S, or $^{125}$I, a fluorescent or chemiluminescent compound, such as fluorescein isothiocyanate, rhodamine, or luciferin, or an enzyme, such as alkaline phosphatase, beta-galactosidase or horseradish peroxidase. Any method known in the art for conjugating the antibody to the label may be employed, including those methods described by Hunter et al., Nature, 144:945 (1962); David et al., Biochemistry, 13:1014 (1974); Pain et al., J. Immunol. Meth., 40:219 (1981); and Nygren, J. Histochem. and Cytochem., 30:407 (1982).

Accordingly, the present invention also provides CA protein sequences. A CA protein of the present invention may be identified in several ways. "Protein" in this sense includes proteins, polypeptides, and peptides. As will be appreciated by those in the art, the nucleic acid sequences of the invention can be used to generate protein sequences. There are a variety of ways to do this, including cloning the entire gene and verifying its frame and amino acid sequence, or by comparing it to known sequences to search for homology to provide a frame, assuming the CA protein has homology to some protein in the database being used. Generally, the nucleic acid sequences are input into a program that will search all three frames for homology. This is done in a preferred embodiment using the following NCBI Advanced BLAST parameters. The program is blastx or blastn. The database is nr. The input data is as "Sequence in FASTA format". The organism list is "none". The "expect" is 10; the filter is default. The "descriptions" is 500, the "alignments" is 500, and the "alignment view" is pairwise. The "query Genetic Codes" is standard (1). The matrix is BLOSUM 62; gap existence cost is 11, per residue gap cost is 1; and the lambda ratio is .85 default. This results in the generation of a putative protein sequence.

In general, the term "polypeptide" as used herein refers to both the full-length polypeptide encoded by the recited polynucleotide, the polypeptide encoded by the gene represented by the recited polynucleotide, as well as portions or fragments thereof. The present invention encompasses variants of the naturally occurring proteins, wherein such variants are homologous or substantially similar to the naturally occurring protein, and can be of an origin of the same or different species as the naturally occurring protein (*e.g.*, human, murine, or some other species that naturally expresses the recited polypeptide, usually a mammalian species). In general, variant polypeptides have a sequence that has at least about 80%, at least about 81 %, at least about 82%, at least about 83%, at least about 84%, at least about 85%, at least about 86%, at least about 87%, at least about 88%, at least about 89%, usually at least about 90%, at least 91%, at least 92%, at least 93%, at least 94%, at least 95%, at least 96%, at least 97%, at least 98% and more usually at least about 99% sequence identity with a differentially expressed polypeptide described herein, as determined by the Smith-Waterman homology search algorithm using an affine gap search with a gap open penalty of 12 and a gap extension penalty of 2, BLOSUM matrix of 62. The Smith-Waterman homology search algorithm is taught in Smith and Waterman, Adv. Appl. Math. (1981) 2: 482-489. The variant polypeptides can be naturally or non-naturally glycosylated, *i.e.*, the polypeptide has a glycosylation pattern that differs from the glycosylation pattern found in the corresponding naturally occurring protein.

Also within the scope of the invention are variants. Variants of polypeptides include mutants, fragments, and fusions. Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge, hydrophobicity/ hydrophilicity, and/or steric bulk of the amino acid substituted. Variants can be designed so as to retain or have enhanced biological activity of a particular region of the protein (*e.g.*, a functional domain and/or, where the polypeptide is a member of a protein family, a region associated with a consensus sequence). Selection of amino acid alterations for production of variants can be based upon the accessibility (interior vs. exterior) of the amino acid (see, e.g., Go et al, Int. J. Peptide Protein Res. (1980) 15:211), the thermostability of the variant polypeptide (see, e.g., Querol et al., Prot. Eng. (1996) 9:265), desired glycosylation sites (see, e.g., Olsen and Thomsen, J. Gen. Microbiol. (1991) 137:579), desired disulfide bridges (see, e.g., Clarke et al., Biochemistry (1993) 32:4322; and Wakarchuk et al., Protein Eng. (1994) 7:1379), desired metal binding sites (see, e.g., Toma et al., Biochemistry (1991) 30:97, and Haezerbrouck et al., Protein Eng. (1993) 6:643), and desired substitutions within proline loops (see, e.g., Masul et al., Appl. Env. Microbiol. (1994) 60:3579). Cysteine-depleted muteins can be produced as disclosed in USPN 4,959,314.

Variants also include fragments of the polypeptides disclosed herein, particularly biologically active fragments and/or fragments corresponding to functional domains. Fragments of interest will typically be at least about 8 amino acids (aa) 10 aa, 15 aa, 20 aa, 25 aa, 30 aa, 35 aa, 40 aa, to at least about 45 aa in length, usually at least about 50 aa in length, at least about 75 aa, at least about 100 aa, at least about 125 aa, at least about 150 aa in length, at least about 200 aa, at least about 300 aa, at least about 400 aa and can be as long as 500 aa in length or longer, but will usually not exceed about 1000 aa in length, where the fragment will have a stretch of amino acids that is identical to a polypeptide encoded by a polynucleotide having a sequence of any one of the polynucleotide sequences provided herein, or a homolog thereof. The protein variants described herein are encoded by polynucleotides that are within the scope of the invention. The genetic code can be used to select the appropriate codons to construct the corresponding variants.

While altered expression of the polynucleotides associated with cancer is observed, altered levels of expression of the polypeptides encoded by these polynucleotides may likely play a role in cancers.

Also included within one embodiment of CA proteins are amino acid variants of the naturally occurring sequences, as determined herein. Preferably, the variants are preferably greater than about 75% homologous to the wild-type sequence, more preferably greater than about 80%, even more preferably greater than about 85% and most preferably greater than 90%. The present application is also directed to proteins containing polypeptides at least 80%, 85%, 90%, 95%, 96%, 97%, 98% or 99% identical to a CA polypeptide sequence set forth herein. As for nucleic acids, homology in this context means sequence similarity or identity, with identity being preferred. This homology will be determined using standard techniques known in the art as are outlined above for the nucleic acid homologies.

CA proteins of the present invention may be shorter or longer than the wild type amino acid sequences. Thus, in a preferred embodiment, included within the definition of CA proteins are portions or fragments of the wild type sequences herein. In addition, as outlined above, the CA nucleic acids of the invention may be used to obtain additional coding regions, and thus additional protein sequence, using techniques known in the art.

In a preferred embodiment, the CA proteins are derivative or variant CA proteins as compared to the wild-type sequence. That is, as outlined more fully below, the derivative CA peptide will contain at least one amino acid substitution, deletion or insertion, with amino acid substitutions being particularly preferred. The amino acid substitution, insertion or deletion may occur at any residue within the CA peptide.

Also included in an embodiment of CA proteins of the present invention are amino acid sequence variants. These variants fall into one or more of three classes: substitutional, insertional or deletional variants. These variants ordinarily are prepared by site-specific mutagenesis of nucleotides in the DNA encoding the CA protein, using cassette or PCR mutagenesis or other techniques well known in the art, to produce DNA encoding the variant, and thereafter expressing the DNA in recombinant cell culture as outlined above. However, variant CA protein fragments having up to about 100-150 residues may be prepared by *in vitro* synthesis using established techniques. Amino acid sequence variants are characterized by the predetermined nature of the variation, a feature that sets them apart from naturally occurring allelic or interspecies variation of the CA protein amino acid sequence. The variants typically exhibit the same qualitative biological activity as the naturally occurring analogue, although variants can also be selected which have modified characteristics as will be more fully outlined below.

While the site or region for introducing an amino acid sequence variation is predetermined, the mutation per se need not be predetermined. For example, in order to optimize the performance of a mutation at a given site, random mutagenesis may be conducted at the target codon or region and the expressed CA variants screened for the optimal combination of desired activity. Techniques for making substitution mutations at predetermined sites in DNA having a known sequence are well known, for example, M13 primer mutagenesis and LAR mutagenesis. Screening of the mutants is done using assays of CA protein activities.

Amino acid substitutions are typically of single residues; insertions usually will be on the order of from about 1 to 20 amino acids, although considerably larger insertions may be tolerated. Deletions range from about 1 to about 20 residues, although in some cases deletions may be much larger.

Substitutions, deletions, insertions or any combination thereof may be used to arrive at a final derivative. Generally these changes are done on a few amino acids to minimize the alteration of the molecule. However, larger changes may be tolerated in certain circumstances. When small alterations in the characteristics of the CA protein are desired, substitutions are generally made in accordance with the following chart:

**Chart 1**

| Original Residue | Exemplary Substitutions |
| --- | --- |
| Ala | Ser |
| Arg | Lys |
| Asn | Gln, His |
| Asp | Glu |
| Cys | Ser |
| Gln | Asn |
| Glu | Asp |
| Gly | Pro |
| His | Asn, Gln |
| Ile | Leu, Val |
| Leu | Ile, Val |
| Lys | Arg, Gln, Glu |
| Met | Leu, Ile |
| Phe | Met, Leu, Tyr |
| Ser | Thr |
| Thr | Ser |
| Trp | Tyr |
| Tyr | Trp, Phe |
| Val | Ile, Leu |

Substantial changes in function or immunological identity are made by selecting substitutions that are less conservative than those shown in Chart I. For example, substitutions may be made full length to more significantly affect one or more of the following: the structure of the polypeptide backbone in the area of the alteration (e.g., the alpha-helical or beta-sheet structure); the charge or hydrophobicity of the molecule at the target site; and the bulk of the side chain. The substitutions which in general are expected to produce the greatest changes in the polypeptide's properties are those in which (a) a hydrophilic residue, e.g. seryl or threonyl is substituted for (or by) a hydrophobic residue, e.g. leucyl, isoleucyl, phenylalanyl, valyl or alanyl; (b) a cysteine or proline is substituted for (or by) any other residue; (c) a residue having an electropositive side chain, e.g. lysyl, arginyl, or histidyl, is substituted for (or by) an electronegative residue, e.g. glutamyl or aspartyl; or (d) a residue having a bulky side chain, e.g. phenylalanine, is substituted for (or by) one not having a side chain, e.g. glycine.

The variants typically exhibit the same qualitative biological activity and will elicit the same immune response as the naturally-occurring analogue, although variants also are selected to modify the characteristics of the CA proteins as needed. Alternatively, the variant may be designed such that the biological activity of the CA protein is altered. For example, glycosylation sites may be altered or removed, dominant negative mutations created, etc.

Covalent modifications of CA polypeptides are included within the scope of this invention, for example for use in screening. One type of covalent modification includes reacting targeted amino acid residues of a CA polypeptide with an organic derivatizing agent that is capable of reacting with selected side chains or the N-or C-terminal residues of a CA polypeptide. Derivatization with bifunctional agents is useful, for instance, for crosslinking CA polypeptides to a water-insoluble support matrix or surface for use in the method for purifying anti-CA antibodies or screening assays, as is more fully described below. Commonly used crosslinking agents include, e.g., 1,1-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'-dithiobis(succinimidylpropionate), bifunctional maleimides such as bis-N-maleimido-1,8-octane and agents such as methyl-3-[(p-azidophenyl)dithio]propioimidate.

Other modifications include deamidation of glutaminyl and asparaginyl residues to the corresponding glutamyl and aspartyl residues, respectively, hydroxylation of proline and lysine, phosphorylation of hydroxyl groups of seryl, threonyl or tyrosyl residues, methylation of the a-amino groups of lysine, arginine, and histidine side chains [T.E. Creighton, Proteins: Structure and Molecular Properties, W.H. Freeman & Co., San Francisco, pp. 79-86 (1983)], acetylation of the N-terminal amine, and amidation of any C-terminal carboxyl group.

Another type of covalent modification of the CA polypeptide included within the scope of this invention comprises altering the native glycosylation pattern of the polypeptide. "Altering the native glycosylation pattern" is intended for purposes herein to mean deleting one or more carbohydrate moieties found in native sequence CA polypeptide, and/or adding one or more glycosylation sites that are not present in the native sequence CA polypeptide.

Addition of glycosylation sites to CA polypeptides may be accomplished by altering the amino acid sequence thereof. The alteration may be made, for example, by the addition of, or substitution by, one or more serine or threonine residues to the native sequence CA polypeptide (for O-linked glycosylation sites). The CA amino acid sequence may optionally be altered through changes at the DNA level, particularly by mutating the DNA encoding the CA polypeptide at preselected bases such that codons are generated that will translate into the desired amino acids.

Another means of increasing the number of carbohydrate moieties on the CA polypeptide is by chemical or enzymatic coupling of glycosides to the polypeptide. Such methods are described in the art, e.g., in WO 87/05330 published 11 September 1987, and in Aplin and Wriston, LA Crit. Rev. Biochem., pp. 259-306 (1981).

Removal of carbohydrate moieties present on the CA polypeptide may be accomplished chemically or enzymatically or by mutational substitution of codons encoding for amino acid residues that serve as targets for glycosylation. Chemical deglycosylation techniques are known in the art and described, for instance, by Hakimuddin, et al., Arch. Biochem. Biophys., 259:52 (1987) and by Edge et al., Anal. Biochem., 118:131 (1981). Enzymatic cleavage of carbohydrate moieties on polypeptides can be achieved by the use of a variety of endo-and exo-glycosidases as described by Thotakura et al., Meth. Enzymol., 138:350 (1987).

Another type of covalent modification of CA comprises linking the CA polypeptide to one of a variety of nonproteinaceous polymers, e.g., polyethylene glycol, polypropylene glycol, or polyoxyalkylenes, in the manner set forth in U.S. Patent Nos. 4,640,835; 4,496,689; 4,301,144; 4,670,417; 4,791,192 or 4,179,337.

CA polypeptides of the present invention may also be modified in a way to form chimeric molecules comprising a CA polypeptide fused to another, heterologous polypeptide or amino acid sequence. In one embodiment, such a chimeric molecule comprises a fusion of a CA polypeptide with a tag polypeptide that provides an epitope to which an anti-tag antibody can selectively bind. The epitope tag is generally placed at the amino-or carboxyl-terminus of the CA polypeptide, although internal fusions may also be tolerated in some instances. The presence of such epitope-tagged forms of a CA polypeptide can be detected using an antibody against the tag polypeptide. Also, provision of the epitope tag enables the CA polypeptide to be readily purified by affinity purification using an anti-tag antibody or another type of affinity matrix that binds to the epitope tag. In an alternative embodiment, the chimeric molecule may comprise a fusion of a CA polypeptide with an immunoglobulin or a particular region of an immunoglobulin. For a bivalent form of the chimeric molecule, such a fusion could be to the Fc region of an IgG molecule.

Various tag polypeptides and their respective antibodies are well known in the art. Examples include poly-histidine (poly-his) or poly-histidine-glycine (poly-his-gly) tags; the flu HA tag polypeptide and its antibody 12CA5 [Field et al., Mol. Cell. Biol., 8:2159-2165 (1988)]; the c-myc tag and the 8F9, 3C7, 6E10, G4, B7 and 9E10 antibodies thereto [Evan et al., Molecular and Cellular Biology, 5:3610-3616 (1985)]; and the Herpes Simplex virus glycoprotein D (gD) tag and its antibody [Paborsky et al., Protein Engineering, 3(6):547-553 (1990)]. Other tag polypeptides include the Flag-peptide [Hopp et al., BioTechnology, 6:1204-1210 (1988)]; the KT3 epitope peptide [Martin et al., Science, 255:192-194 (1992)]; tubulin epitope peptide [Skinner et al., J. Biol. Chem., 266:15163-15166 (1991)]; and the T7 gene 10 protein peptide tag [Lutz-Freyermuth et al., Proc. Natl. Acad. Sci. USA, 87:6393-6397 (1990)].

Also included with the definition of CA protein in one embodiment are other CA proteins of the CA family, and CA proteins from other organisms, which are cloned and expressed as outlined below. Thus, probe or degenerate polymerase chain reaction (PCR) primer sequences may be used to find other related CA proteins from humans or other organisms. As will be appreciated by those in the art, particularly useful probe and/or PCR primer sequences include the unique areas of the CA nucleic acid sequence. As is generally known in the art, preferred PCR primers are from about 15 to about 35 nucleotides in length, with from about 20 to about 30 being preferred, and may contain inosine as needed. The conditions for the PCR reaction are well known in the art.

In addition, as is outlined herein, CA proteins can be made that are longer than those encoded by the nucleic acids of the figures, for example, by the elucidation of additional sequences, the addition of epitope or purification tags, the addition of other fusion sequences, etc.

CA proteins may also be identified as being encoded by CA nucleic acids. Thus, CA proteins are encoded by nucleic acids that will hybridize to the sequences of the sequence listings, or their complements, as outlined herein.

**CA antigens and antibodies thereto**

In one embodiment, the invention provides CA specific antibodies. In a preferred embodiment, when the CA protein is to be used to generate antibodies, for example for immunotherapy, the CA protein should share at least one epitope or determinant with the full-length protein. By "epitope" or "determinant" herein is meant a portion of a protein that will generate and/or bind an antibody or T-cell receptor in the context of MHC. Thus, in most instances, antibodies made to a smaller CA protein will be able to bind to the full-length protein. In a preferred embodiment, the epitope is unique; that is, antibodies generated to a unique epitope show little or no cross-reactivity.

Any polypeptide sequence encoded by the CA polynucleotide sequences may be analyzed to determine certain preferred regions of the polypeptide. Regions of high antigenicity are determined from data by DNASTAR analysis by choosing values that represent regions of the polypeptide that are likely to be exposed on the surface of the polypeptide in an environment in which antigen recognition may occur in the process of initiation of an immune response. For example, the amino acid sequence of a polypeptide encoded by a CA polynucleotide sequence may be analyzed using the default parameters of the DNASTAR computer algorithm (DNASTAR, Inc., Madison, Wis.; http://www.dnastar.com/).

Polypeptide features that may be routinely obtained using the DNASTAR computer algorithm include, but are not limited to, Garnier-Robson alpha-regions, beta-regions, turn-regions, and coil-regions (Garnier et al. J. Mol. Biol., 120: 97 (1978)); Chou-Fasman alpha-regions, beta-regions, and turn-regions (Adv. in Enzymol., 47:45-148 (1978)); Kyte-Doolittle hydrophilic regions and hydrophobic regions (J. Mol. Biol., 157:105-132 (1982)); Eisenberg alpha- and beta-amphipathic regions; Karplus-Schulz flexible regions; Emini surface-forming regions (J. Virol., 55(3):836-839 (1985)); and Jameson-Wolf regions of high antigenic index (CABIOS, 4(1):181-186 (1988)). Kyte-Doolittle hydrophilic regions and hydrophobic regions, Emini surface-forming regions, and Jameson-Wolf regions of high antigenic index (i.e., containing four or more contiguous amino acids having an antigenic index of greater than or equal to 1.5, as identified using the default parameters of the Jameson-Wolf program) can routinely be used to determine polypeptide regions that exhibit a high degree of potential for antigenicity. One approach for preparing antibodies to a protein is the selection and preparation of an amino acid sequence of all or part of the protein, chemically synthesizing the sequence and injecting it into an appropriate animal, typically a rabbit, hamster or a mouse. Oligopeptides can be selected as candidates for the production of an antibody to the CA protein based upon the oligopeptides lying in hydrophilic regions, which are thus likely to be exposed in the mature protein. Additional oligopeptides can be determined using, for example, the Antigenicity Index, Welling, G.W. et al., FEBS Lett. 188:215-218 (1985), incorporated herein by reference.

In one embodiment, the term "antibody" includes antibody fragments, as are known in the art, including Fab, Fab$_2$, single chain antibodies (Fv for example), chimeric antibodies, etc., either produced by the modification of whole antibodies or those synthesized de novo using recombinant DNA technologies.

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include a protein encoded by a nucleic acid of the figures or fragment thereof or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants that may be employed include Freund's complete adjuvant and MPL-TDM adjuvant (monophosphoryl Lipid A, synthetic trehalose dicorynomycolate). The immunization protocol may be selected by one skilled in the art without undue experimentation.

The antibodies may, alternatively, be monoclonal antibodies. Monoclonal antibodies may be prepared using hybridoma methods, such as those described by Kohler and Milstein, Nature, 256:495 (1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.* The immunizing agent will typically include a polypeptide encoded by a nucleic acid of Tables 1-7, or fragment thereof or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Monoclonal antibody technology is used in implementing research, diagnosis and therapy. Monoclonal antibodies are used in radioimmunoassays, enzyme-linked immunosorbent assays, immunocytopathology, and flow cytometry for *in vitro* diagnosis, and *in vivo* for diagnosis and immunotherapy of human disease. Waldmann, T. A. (1991) Science 252:1657-1662. In particular, monoclonal antibodies have been widely applied to the diagnosis and therapy of cancer, wherein it is desirable to target malignant lesions while avoiding normal tissue. See, e.g., U.S. Pat. Nos. 4,753,894 to Frankel, et al.; 4,938,948 to Ring et al.; and 4,956,453 to Bjorn et al.

In one embodiment, the antibodies are bispecific antibodies. Bispecific antibodies are monoclonal, preferably human or humanized, antibodies that have binding specificities for at least two different antigens. A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al. (1989) Proc. Nat. Acad. Sci. USA 86:4220-4224; Shaw et al. (1987) J Immunol. 138:4534-4538; and Brown et al. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients. In the present case, one of the binding specificities is for a protein encoded by a nucleic acid of Tables 1-7, or a fragment thereof, the other one is for any other antigen, and preferably for a cell-surface protein or receptor or receptor subunit, preferably one that is tumor specific.

In a preferred embodiment, the antibodies to CA are capable of reducing or eliminating the biological function of CA, as is described below. That is, the addition of anti-CA antibodies (either polyclonal or preferably monoclonal) to CA (or cells containing CA) may reduce or eliminate the CA activity. Generally, at least a 25% decrease in activity is preferred, with at least about 50% being particularly preferred and about a 95-100% decrease being especially preferred.

In a preferred embodiment the antibodies to the CA proteins are humanized antibodies. "Humanized" antibodies refer to a molecule having an antigen binding site that is substantially derived from an immunoglobulin from a non-human species and the remaining immunoglobulin structure of the molecule based upon the structure and/or sequence of a human immunoglobulin. The antigen binding site may comprise either complete variable domains fused onto constant domains or only the complementarity determining regions (CDRs) grafted onto appropriate framework regions in the variable domains. Antigen binding sites may be wild type or modified by one or more amino acid substitutions, e.g., modified to resemble human immunoglobulin more closely. Alternatively, a humanized antibody may be derived from a chimeric antibody that retains or substantially retains the antigen-binding properties of the parental, non-human, antibody but which exhibits diminished immunogenicity as compared to the parental antibody when administered to humans. The phrase "chimeric antibody," as used herein, refers to an antibody containing sequence derived from two different antibodies (*see, e.g.,* U.S. Patent No. 4,816,567) that typically originate from different species. Typically, in these chimeric antibodies, the variable region of both light and heavy chains mimics the variable regions of antibodies derived from one species of mammals, while the constant portions are homologous to the sequences in antibodies derived from another. Most typically, chimeric antibodies comprise human and murine antibody fragments, generally human constant and mouse variable regions. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues that are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the framework residues (FR) regions are those of a human immunoglobulin consensus sequence. The humanized antibody optimally also will comprise at least a portion of an immunoglobulin constant region (Fc), typically that of a human immunoglobulin (Jones et al., Nature, 321:522-525 (1986); Riechmann et al., Nature, 332:323-329 (1988); and Presta, Curr. Op. Struct. Biol., 2:593-596 (1992)). One clear advantage to such chimeric forms is that, for example, the variable regions can conveniently be derived from presently known sources using readily available hybridomas or B cells from non human host organisms in combination with constant regions derived from, for example, human cell preparations. While the variable region has the advantage of ease of preparation, and the specificity is not affected by its source, the constant region being human, is less likely to elicit an immune response from a human subject when the antibodies are injected than would the constant region from a non-human source. However, the definition is not limited to this particular example.

Because humanized antibodies are far less immunogenic in humans than the parental mouse monoclonal antibodies, they can be used for the treatment of humans with far less risk of anaphylaxis. Thus, these antibodies may be preferred in therapeutic applications that involve *in vivo* administration to a human such as, *e.g.*, use as radiation sensitizers for the treatment of neoplastic disease or use in methods to reduce the side effects of, *e.g.,* cancer therapy. Methods for humanizing non-human antibodies are well known in the art. Generally, a humanized antibody has one or more amino acid residues introduced into it from a source that is non-human. These non-human amino acid residues are often referred to as import residues, which are typically taken from an import variable domain. Humanization can be essentially performed following the method of Winter and co-workers (Jones et al., Nature 321:522-525 (1986); Riechmann et al., Nature 332:323-327 (1988); Verhoeyen et al., Science 239:1534-1536 (1988)), by substituting rodent CDRs or CDR sequences for the corresponding sequences of a human antibody. Accordingly, such humanized antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies [Cole et al., Monoclonal Antibodies and Cancer Therapy, Alan R. Liss, p. 77 (1985) and Boerner et al., J. Immunol., 147(1):86-95 (1991)]. Humanized antibodies may be achieved by a variety of methods including, for example: (1) grafting the non-human complementarity determining regions (CDRs) onto a human framework and constant region (a process referred to in the art as "humanizing"), or, alternatively, (2) transplanting the entire non-human variable domains, but "cloaking" them with a human-like surface by replacement of surface residues (a process referred to in the art as "veneering"). In the present invention, humanized antibodies will include both "humanized" and "veneered" antibodies. Similarly, human antibodies can be made by introducing human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al., Bio/Technology 10, 779-783 (1992); Lonberg et al., Nature 368 856-859 (1994); Morrison, Nature 368, 812-13 (1994); Fishwild et al., Nature Biotechnology 14, 845-51 (1996); Neuberger, Nature Biotechnology 14, 826 (1996); Lonberg and Huszar, Intern. Rev. Immunol. 13 65-93 (1995); Jones et al., Nature 321:522-525 (1986); Morrison et al., Proc. Natl. Acad. Sci, US.A., 81:6851-6855 (1984); Morrison and Oi, Adv. Immunol., 44:65-92 (1988); Verhoeyer et al., Science 239:1534-1536 (1988); Padlan, Molec. Immun. 28:489-498 (1991); Padlan, Molec. Immunol. 31(3):169-217 (1994); and Kettleborough, C.A. et al., Protein Eng. 4(7):773-83 (1991) each of which is incorporated herein by reference.

The phrase "complementarity determining region" refers to amino acid sequences which together define the binding affinity and specificity of the natural Fv region of a native immunoglobulin binding site. *See, e.g.*, Chothia et al., J. Mol. Biol. 196:901-917 (1987); Kabat et al., U.S. Dept. of Health and Human Services NIH Publication No. 91-3242 (1991). The phrase "constant region" refers to the portion of the antibody molecule that confers effector functions. In the present invention, mouse constant regions are substituted by human constant regions. The constant regions of the subject humanized antibodies are derived from human immunoglobulins. The heavy chain constant region can be selected from any of the five isotypes: alpha, delta, epsilon, gamma or mu. One method of humanizing antibodies comprises aligning the non-human heavy and light chain sequences to human heavy and light chain sequences, selecting and replacing the non-human framework with a human framework based on such alignment, molecular modeling to predict the conformation of the humanized sequence and comparing to the conformation of the parent antibody. This process is followed by repeated back mutation of residues in the CDR region that disturb the structure of the CDRs until the predicted conformation of the humanized sequence model closely approximates the conformation of the non-human CDRs of the parent non-human antibody. Such humanized antibodies may be further derivatized to facilitate uptake and clearance, *e.g*, via Ashwell receptors. *See, e.g.*, U.S. Patent Nos. 5,530,101 and 5,585,089 which are incorporated herein by reference.

Humanized antibodies to CA polypeptides can also be produced using transgenic animals that are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci. WO 91/10741 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. WO 96/30498 discloses the use of the Cre/Lox system to modify the immunoglobulin locus in a mammal, such as to replace all or a portion of the constant or variable region to form a modified antibody molecule. WO 94/02602 discloses non-human mammalian hosts having inactivated endogenous Ig loci and functional human Ig loci. U.S. Patent No. 5,939,598 discloses methods of making transgenic mice in which the mice lack endogenous heavy chains, and express an exogenous immunoglobulin locus comprising one or more xenogeneic constant regions.

Using a transgenic animal described above, an immune response can be produced to a selected antigenic molecule, and antibody-producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. The monoclonal antibodies can be tested for the ability to inhibit or neutralize the biological activity or physiological effect of the corresponding protein.

In the present invention, CA polypeptides of the invention and variants thereof are used to immunize a transgenic animal as described above. Monoclonal antibodies are made using methods known in the art, and the specificity of the antibodies is tested using isolated CA polypeptides. Methods for preparation of the human or primate CA or an epitope thereof include, but are not limited to chemical synthesis, recombinant DNA techniques or isolation from biological samples. Chemical synthesis of a peptide can be performed, for example, by the classical Merrifeld method of solid phase peptide synthesis (Merrifeld, J. Am. Chem. Soc. 85:2149, 1963 which is incorporated by reference) or the FMOC strategy on a Rapid Automated Multiple Peptide Synthesis system (E. I. du Pont de Nemours Company, Wilmington, DE) (Caprino and Han, J. Org. Chem. 37:3404, 1972 which is incorporated by reference).

Polyclonal antibodies can be prepared by immunizing rabbits or other animals by injecting antigen followed by subsequent boosts at appropriate intervals. The animals are bled and sera assayed against purified CA proteins usually by ELISA or by bioassay based upon the ability to block the action of CA proteins. When using avian species, e.g., chicken, turkey and the like, the antibody can be isolated from the yolk of the egg. Monoclonal antibodies can be prepared after the method of Milstein and Kohler by fusing splenocytes from immunized mice with continuously replicating tumor cells such as myeloma or lymphoma cells. (Milstein and Kohler, Nature 256:495-497, 1975; Gulfre and Milstein, Methods in Enzymology: Immunochemical Techniques 73:1-46, Langone and Banatis eds., Academic Press, 1981 which are incorporated by reference). The hybridoma cells so formed are then cloned by limiting dilution methods and supernates assayed for antibody production by ELISA, RIA or bioassay.

The unique ability of antibodies to recognize and specifically bind to target proteins provides an approach for treating an overexpression of the protein. Thus, another aspect of the present invention provides for a method for preventing or treating diseases involving overexpression of a CA polypeptide by treatment of a patient with specific antibodies to the CA protein.

Specific antibodies, either polyclonal or monoclonal, to the CA proteins can be produced by any suitable method known in the art as discussed above. For example, murine or human monoclonal antibodies can be produced by hybridoma technology or, alternatively, the CA proteins, or an immunologically active fragment thereof, or an anti-idiotypic antibody, or fragment thereof can be administered to an animal to elicit the production of antibodies capable of recognizing and binding to the CA proteins. Such antibodies can be from any class of antibodies including, but not limited to IgG, IgA, IgM, IgD, and IgE or in the case of avian species, IgY and from any subclass of antibodies.

By immunotherapy is meant treatment of a cancer with an antibody raised against a CA protein. As used herein, immunotherapy can be passive or active. Passive immunotherapy as defined herein is the passive transfer of antibody to a recipient (patient). Active immunization is the induction of antibody and/or T-cell responses in a recipient (patient). Induction of an immune response is the result of providing the recipient with an antigen to which antibodies are raised. As appreciated by one of ordinary skill in the art, the antigen may be provided by injecting a polypeptide against which antibodies are desired to be raised into a recipient, or contacting the recipient with a nucleic acid capable of expressing the antigen and under conditions for expression of the antigen.

In a preferred embodiment, oncogenes which encode secreted growth factors may be inhibited by raising antibodies against CA proteins that are secreted proteins as described above. Without being bound by theory, antibodies used for treatment, bind and prevent the secreted protein from binding to its receptor, thereby inactivating the secreted CA protein.

In another preferred embodiment, the CA protein to which antibodies are raised is a transmembrane protein. Without being bound by theory, antibodies used for treatment, bind the extracellular domain of the CA protein and prevent it from binding to other proteins, such as circulating ligands or cell-associated molecules. The antibody may cause down-regulation of the transmembrane CA protein. As will be appreciated by one of ordinary skill in the art, the antibody may be a competitive, non-competitive or uncompetitive inhibitor of protein binding to the extracellular domain of the CA protein. The antibody is also an antagonist of the CA protein. Further, the antibody prevents activation of the transmembrane CA protein. In one aspect, when the antibody prevents the binding of other molecules to the CA protein, the antibody prevents growth of the cell. The antibody may also sensitize the cell to cytotoxic agents, including, but not limited to TNF-$\alpha$, TNF-$\beta$, IL-1, INF-$\gamma$ and IL-2, or chemotherapeutic agents including 5FU, vinblastine, actinomycin D, cisplatin, methotrexate, and the like. In some instances the antibody belongs to a sub-type that activates serum complement when complexed with the transmembrane protein thereby mediating cytotoxicity. Thus, cancers may be treated by administering to a patient antibodies directed against the transmembrane CA protein.

In another preferred embodiment, the antibody is conjugated to a therapeutic moiety. In one aspect the therapeutic moiety is a small molecule that modulates the activity of the CA protein. In another aspect the therapeutic moiety modulates the activity of molecules associated with or in close proximity to the CA protein. The therapeutic moiety may inhibit enzymatic activity such as protease or protein kinase activity associated with cancer.

In a preferred embodiment, the therapeutic moiety may also be a cytotoxic agent. In this method, radioisotopes, natural toxins, chemotherapy agents, or other substances (such as biological response modifiers) are chemically linked or conjugated to a monoclonal antibody to form "immunoconjugates" and "immunotoxins" which target the cytotoxic agent to tumor tissue or cells resulting in a reduction in the number of afflicted cells, thereby reducing symptoms associated with cancers, including lymphoma. Cytotoxic agents are numerous and varied and include, but are not limited to, cytotoxic drugs or toxins or active fragments of such toxins. Suitable toxins and their corresponding fragments include diphtheria A chain, exotoxin A chain, ricin A chain, abrin A chain, curcin, crotin, phenomycin, enomycin and the like. Cytotoxic agents also include radiochemicals made by conjugating radioisotopes to antibodies raised against CA proteins, or binding of a radionuclide to a chelating agent that has been covalently attached to the antibody. Targeting the therapeutic moiety to transmembrane CA proteins not only serves to increase the local concentration of therapeutic moiety in the cancer of interest, i.e., lymphoma, but also serves to reduce deleterious side effects that may be associated with the therapeutic moiety. A number of investigators have used monoclonal antibodies as carriers of cytotoxic substances in attempts to selectively direct those agents to malignant tissue. More particularly, a number of monoclonal antibodies have been conjugated to toxins such as ricin, abrin, diphtheria toxin and Pseudomonas exotoxin or to enzymatically active portions (A chains) thereof via heterobifunctional agents. See, e.g., U.S. Pat. No. 4,753,894 to Frankel et al.; Nevelle, et al. (1982) Immunol Rev 62:75-91; Ross et al. (1980) Eur. J Biochem 104; Vitteta et al. (1982) Immunol Rev 62:158-183; Raso et al. (1982) Cancer Res 42:457-464, and Trowbridge et al. (1981) Nature 294:171-173.

In another preferred embodiment, the CA protein against which the antibodies are raised is an intracellular protein. In this case, the antibody may be conjugated to a protein that facilitates entry into the cell. In one case, the antibody enters the cell by endocytosis. In another embodiment, a nucleic acid encoding the antibody is administered to the individual or cell. Moreover, wherein the CA protein can be targeted within a cell, e.g., the nucleus, an antibody thereto contains a signal for that target localization, e.g., a nuclear localization signal.

The CA antibodies of the invention specifically bind to CA proteins. By "specifically bind" herein is meant that the antibodies bind to the protein with a binding constant in the range of $10^{-4}$-$10^{-6}$ M$^{-1}$, with a preferred range being $10^{-7}$-$10^{-9}$ M$^{-1}$.

In a preferred embodiment, the CA protein is purified or isolated after expression. CA proteins may be isolated or purified in a variety of ways known to those skilled in the art depending on what other components are present in the sample. Standard purification methods include electrophoretic, molecular, immunological and chromatographic techniques, including ion exchange, hydrophobic, affinity, and reverse-phase HPLC chromatography, and chromatofocusing. For example, the CA protein may be purified using a standard anti-CA antibody column. Ultrafiltration and diafiltration techniques, in conjunction with protein concentration, are also useful. For general guidance in suitable purification techniques, see Scopes, R., Protein Purification, Springer-Verlag, NY (1982). The degree of purification necessary will vary depending on the use of the CA protein. In some instances no purification will be necessary.

## Detection of cancer phenotype

Once expressed and purified if necessary, the CA proteins and nucleic acids are useful in a number of applications. In one aspect, the expression levels of genes are determined for different cellular states in the cancer phenotype; that is, the expression levels of genes in normal tissue and in cancer tissue (and in some cases, for varying severities of lymphoma that relate to prognosis, as outlined below) are evaluated to provide expression profiles. An expression profile of a particular cell state or point of development is essentially a "fingerprint" of the state; while two states may have any particular gene similarly expressed, the evaluation of a number of genes simultaneously allows the generation of a gene expression profile that is unique to the state of the cell. By comparing expression profiles of cells in different states, information regarding which genes are important (including both up- and down-regulation of genes) in each of these states is obtained. Then, diagnosis may be done or confirmed: does tissue from a particular patient have the gene expression profile of normal or cancer tissue.

"Differential expression," or equivalents used herein, refers to both qualitative as well as quantitative differences in the temporal and/or cellular expression patterns of genes, within and among the cells. Thus, a differentially expressed gene can qualitatively have its expression altered, including an activation or inactivation, in, for example, normal versus cancer tissue. That is, genes may be turned on or turned off in a particular state, relative to another state. As is apparent to the skilled artisan, any comparison of two or more states can be made. Such a qualitatively regulated gene will exhibit an expression pattern within a state or cell type which is detectable by standard techniques in one such state or cell type, but is not detectable in both. Alternatively, the determination is quantitative in that expression is increased or decreased; that is, the expression of the gene is either up-regulated, resulting in an increased amount of transcript, or down-regulated, resulting in a decreased amount of transcript. The degree to which expression differs need only be large enough to quantify via standard characterization techniques as outlined below, such as by use of Affymetrix GeneChip® expression arrays, Lockhart, Nature Biotechnology, 14:1675-1680 (1996), hereby expressly incorporated by reference. Other techniques include, but are not limited to, quantitative reverse transcriptase PCR, Northern analysis and RNase protection. As outlined above, preferably the change in expression (i.e. upregulation or downregulation) is at least about 50%, more preferably at least about 100%, more preferably at least about 150%, more preferably, at least about 200%, with from 300 to at least 1000% being especially preferred.

As will be appreciated by those in the art, this may be done by evaluation at either the gene transcript, or the protein level; that is, the amount of gene expression may be monitored using nucleic acid probes to the DNA or RNA equivalent of the gene transcript, and the quantification of gene expression levels, or, alternatively, the final gene product itself (protein) can be monitored, for example through the use of antibodies to the CA protein and standard immunoassays (ELISAs, etc.) or other techniques, including mass spectroscopy assays, 2D gel electrophoresis assays, etc. Thus, the proteins corresponding to CA genes, i.e. those identified as being important in a particular cancer phenotype, i.e., lymphoma, can be evaluated in a diagnostic test specific for that cancer.

In a preferred embodiment, gene expression monitoring is done and a number of genes, i.e. an expression profile, is monitored simultaneously, although multiple protein expression monitoring can be done as well. Similarly, these assays may be done on an individual basis as well.

In this embodiment, the CA nucleic acid probes may be attached to biochips as outlined herein for the detection and quantification of CA sequences in a particular cell. The assays are done as is known in the art. As will be appreciated by those in the art, any number of different CA sequences may be used as probes, with single sequence assays being used in some cases, and a plurality of the sequences described herein being used in other embodiments. In addition, while solid-phase assays are described, any number of solution based assays may be done as well.

In a preferred embodiment, both solid and solution based assays may be used to detect CA sequences that are up-regulated or down-regulated in cancers as compared to normal tissue. In instances where the CA sequence has been altered but shows the same expression profile or an altered expression profile, the protein will be detected as outlined herein.

In a preferred embodiment nucleic acids encoding the CA protein are detected. Although DNA or RNA encoding the CA protein may be detected, of particular interest are methods wherein the mRNA encoding a CA protein is detected. The presence of mRNA in a sample is an indication that the CA gene has been transcribed to form the mRNA, and suggests that the protein is expressed. Probes to detect the mRNA can be any nucleotide/deoxynucleotide probe that is complementary to and base pairs with the mRNA and includes but is not limited to oligonucleotides, cDNA or RNA. Probes also should contain a detectable label, as defined herein. In one method the mRNA is detected after immobilizing the nucleic acid to be examined on a solid support such as nylon membranes and hybridizing the probe with the sample. Following washing to remove the non-specifically bound probe, the label is detected. In another method detection of the mRNA is performed *in situ*. In this method permeabilized cells or tissue samples are contacted with a detectably labeled nucleic acid probe for sufficient time to allow the probe to hybridize with the target mRNA. Following washing to remove the non-specifically bound probe, the label is detected. For example a digoxygenin labeled riboprobe (RNA probe) that is complementary to the mRNA encoding a CA protein is detected by binding the digoxygenin with an anti-digoxygenin secondary antibody and developed with nitro blue tetrazolium and 5-bromo-4-chloro-3-indoyl phosphate.

In a preferred embodiment, any of the three classes of proteins as described herein (secreted, transmembrane or intracellular proteins) are used in diagnostic assays. The CA proteins, antibodies, nucleic acids, modified proteins and cells containing CA sequences are used in diagnostic assays. This can be done on an individual gene or corresponding polypeptide level, or as sets of assays.

As described and defined herein, CA proteins find use as markers of cancers, including lymphomas such as, but not limited to, Hodgkin's and non-Hodgkin's lymphoma. Detection of these proteins in putative cancer tissue or patients allows for a determination or diagnosis of the type of cancer. Numerous methods known to those of ordinary skill in the art find use in detecting cancers. In one embodiment, antibodies are used to detect CA proteins. A preferred method separates proteins from a sample or patient by electrophoresis on a gel (typically a denaturing and reducing protein gel, but may be any other type of gel including isoelectric focusing gels and the like). Following separation of proteins, the CA protein is detected by immunoblotting with antibodies raised against the CA protein. Methods of immunoblotting are well known to those of ordinary skill in the art.

In another preferred method, antibodies to the CA protein find use in *in situ* imaging techniques. In this method cells are contacted with from one to many antibodies to the CA protein(s). Following washing to remove non-specific antibody binding, the presence of the antibody or antibodies is detected. In one embodiment the antibody is detected by incubating with a secondary antibody that contains a detectable label. In another method the primary antibody to the CA protein(s) contains a detectable label. In another preferred embodiment each one of multiple primary antibodies contains a distinct and detectable label. This method finds particular use in simultaneous screening for a plurality of CA proteins. As will be appreciated by one of ordinary skill in the art, numerous other histological imaging techniques are useful in the invention.

In a preferred embodiment the label is detected in a fluorometer that has the ability to detect and distinguish emissions of different wavelengths. In addition, a fluorescence activated cell sorter (FACS) can be used in the method.

In another preferred embodiment, antibodies find use in diagnosing cancers from blood samples. As previously described, certain CA proteins are secreted/circulating molecules. Blood samples, therefore, are useful as samples to be probed or tested for the presence of secreted CA proteins. Antibodies can be used to detect the CA proteins by any of the previously described immunoassay techniques including ELISA, immunoblotting (Western blotting), immunoprecipitation, BIACORE technology and the like, as will be appreciated by one of ordinary skill in the art.

In a preferred embodiment, *in situ* hybridization of labeled CA nucleic acid probes to tissue arrays is done. For example, arrays of tissue samples, including CA tissue and/or normal tissue, are made. *In situ* hybridization as is known in the art can then be done.

It is understood that when comparing the expression fingerprints between an individual and a standard, the skilled artisan can make a diagnosis as well as a prognosis. It is further understood that the genes that indicate diagnosis may differ from those that indicate prognosis.

In a preferred embodiment, the CA proteins, antibodies, nucleic acids, modified proteins and cells containing CA sequences are used in prognosis assays. As above, gene expression profiles can be generated that correlate to cancer, especially lymphoma, severity, in terms of long term prognosis. Again, this may be done on either a protein or gene level, with the use of genes being preferred. As above, the CA probes are attached to biochips for the detection and quantification of CA sequences in a tissue or patient. The assays proceed as outlined for diagnosis.

**Screening for CA-Targeted Drugs**

In one embodiment, any of the CA sequences as described herein are used in drug screening assays. The CA proteins, antibodies, nucleic acids, modified proteins and cells containing CA sequences are used in drug screening assays or by evaluating the effect of drug candidates on a "gene expression profile" or expression profile of polypeptides. In one embodiment, the expression profiles are used, preferably in conjunction with high throughput screening techniques to allow monitoring for expression profile genes after treatment with a candidate agent, Zlokarnik, et al., Science 279, 84-8 (1998), Heid, et al., Genome Res., 6:986-994 (1996).

In another embodiment, the CA proteins, antibodies, nucleic acids, modified proteins and cells containing the native or modified CA proteins are used in screening assays. That is, the present invention provides novel methods for screening for compositions that modulate the cancer phenotype. As above, this can be done by screening for modulators of gene expression or for modulators of protein activity. Similarly, this may be done on an individual gene or protein level or by evaluating the effect of drug candidates on a "gene expression profile". In a preferred embodiment, the expression profiles are used, preferably in conjunction with high throughput screening techniques to allow monitoring for expression profile genes after treatment with a candidate agent, see Zlokarnik, supra.

Having identified the CA genes herein, a variety of assays to evaluate the effects of agents on gene expression may be executed. In a preferred embodiment, assays may be run on an individual gene or protein level. That is, having identified a particular gene as aberrantly regulated in cancer, candidate bioactive agents may be screened to modulate the gene's regulation. "Modulation" thus includes both an increase and a decrease in gene expression or activity. The preferred amount of modulation will depend on the original change of the gene expression in normal versus tumor tissue, with changes of at least 10%, preferably 50%, more preferably 100-300%, and in some embodiments 300-1000% or greater. Thus, if a gene exhibits a 4 fold increase in tumor compared to normal tissue, a decrease of about four fold is desired; a 10 fold decrease in tumor compared to normal tissue gives a 10 fold increase in expression for a candidate agent is desired, etc. Alternatively, where the CA sequence has been altered but shows the same expression profile or an altered expression profile, the protein will be detected as outlined herein.

As will be appreciated by those in the art, this may be done by evaluation at either the gene or the protein level; that is, the amount of gene expression may be monitored using nucleic acid probes and the quantification of gene expression levels, or, alternatively, the level of the gene product itself can be monitored, for example through the use of antibodies to the CA protein and standard immunoassays. Alternatively, binding and bioactivity assays with the protein may be done as outlined below.

In a preferred embodiment, gene expression monitoring is done and a number of genes, i.e. an expression profile, is monitored simultaneously, although multiple protein expression monitoring can be done as well.

In this embodiment, the CA nucleic acid probes are attached to biochips as outlined herein for the detection and quantification of CA sequences in a particular cell. The assays are further described below.

Generally, in a preferred embodiment, a candidate bioactive agent is added to the cells prior to analysis. Moreover, screens are provided to identify a candidate bioactive agent that modulates a particular type of cancer, modulates CA proteins, binds to a CA protein, or interferes between the binding of a CA protein and an antibody.

The term "candidate bioactive agent" or "drug candidate" or grammatical equivalents as used herein describes any molecule, e.g., protein, oligopeptide, small organic or inorganic molecule, polysaccharide, polynucleotide, etc., to be tested for bioactive agents that are capable of directly or indirectly altering either the cancer phenotype, binding to and/or modulating the bioactivity of a CA protein, or the expression of a CA sequence, including both nucleic acid sequences and protein sequences. In a particularly preferred embodiment, the candidate agent suppresses a CA phenotype, for example to a normal tissue fingerprint. Similarly, the candidate agent preferably suppresses a severe CA phenotype. Generally a plurality of assay mixtures are run in parallel with different agent concentrations to obtain a differential response to the various concentrations. Typically, one of these concentrations serves as a negative control, i.e., at zero concentration or below the level of detection.

In one aspect, a candidate agent will neutralize the effect of a CA protein. By "neutralize" is meant that activity of a protein is either inhibited or counter acted against so as to have substantially no effect on a cell.

Candidate agents encompass numerous chemical classes, though typically they are organic or inorganic molecules, preferably small organic compounds having a molecular weight of more than 100 and less than about 2,500 Daltons. Preferred small molecules are less than 2000, or less than 1500 or less than 1000 or less than 500 D. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including peptides, saccharides, fatty acids, steroids, purines, pyrimidines, derivatives, structural analogs or combinations thereof. Particularly preferred are peptides.

Candidate agents are obtained from a wide variety of sources including libraries of synthetic or natural compounds. For example, numerous means are available for random and directed synthesis of a wide variety of organic compounds and biomolecules, including expression of randomized oligonucleotides. Alternatively, libraries of natural compounds in the form of bacterial, fungal, plant and animal extracts are available or readily produced. Additionally, natural or synthetically produced libraries and compounds are readily modified through conventional chemical, physical and biochemical means. Known pharmacological agents may be subjected to directed or random chemical modifications, such as acylation, alkylation, esterification, or amidification to produce structural analogs.

In one embodiment, the candidate bioactive agents are proteins. By "protein" herein is meant at least two covalently attached amino acids, which includes proteins, polypeptides, oligopeptides and peptides. The protein may be made up of naturally occurring amino acids and peptide bonds, or synthetic peptidomimetic structures. Thus "amino acid", or "peptide residue", as used herein means both naturally occurring and synthetic amino acids. For example, homo-phenylalanine, citrulline and norleucine are considered amino acids for the purposes of the invention. "Amino acid" also includes imino acid residues such as proline and hydroxyproline. The side chains may be in either the (R) or the (S) configuration. In the preferred embodiment, the amino acids are in the (S) or L-configuration. If non-naturally occurring side chains are used, non-amino acid substituents may be used, for example to prevent or retard in vivo degradations.

In a preferred embodiment, the candidate bioactive agents are naturally occurring proteins or fragments of naturally occurring proteins. Thus, for example, cellular extracts containing proteins, or random or directed digests of proteinaceous cellular extracts, may be used. In this way libraries of prokaryotic and eukaryotic proteins may be made for screening in the methods of the invention. Particularly preferred in this embodiment are libraries of bacterial, fungal, viral, and mammalian proteins, with the latter being preferred, and human proteins being especially preferred.

In another preferred embodiment, the candidate bioactive agents are peptides of from about 5 to about 30 amino acids, with from about 5 to about 20 amino acids being preferred, and from about 7 to about 15 being particularly preferred. The peptides may be digests of naturally occurring proteins as is outlined above, random peptides, or "biased" random peptides. By "randomized" or grammatical equivalents herein is meant that each nucleic acid and peptide consists of essentially random nucleotides and amino acids, respectively. Since generally these random peptides (or nucleic acids, discussed below) are chemically synthesized, they may incorporate any nucleotide or amino acid at any position. The synthetic process can be designed to generate randomized proteins or nucleic acids, to allow the formation of all or most of the possible combinations over the length of the sequence, thus forming a library of randomized candidate bioactive proteinaceous agents.

In one embodiment, the library is fully randomized, with no sequence preferences or constants at any position.

In a preferred embodiment, the library is biased. That is, some positions within the sequence are either held constant, or are selected from a limited number of possibilities. For example, in a preferred embodiment, the nucleotides or amino acid residues are randomized within a defined class, for example, of hydrophobic amino acids, hydrophilic residues, sterically biased (either small or large) residues, towards the creation of nucleic acid binding domains, the creation of cysteines, for cross-linking, prolines for SH-3 domains, serines, threonines, tyrosines or histidines for phosphorylation sites, etc., or to purines, etc.

In one embodiment, the candidate bioactive agents are nucleic acids. As described generally for proteins, nucleic acid candidate bioactive agents may be naturally occurring nucleic acids, random nucleic acids, or "biased" random nucleic acids. In another embodiment, the candidate bioactive agents are organic chemical moieties, a wide variety of which are available in the literature.

In assays for testing alteration of the expression profile of one or more CA genes, after the candidate agent has been added and the cells allowed to incubate for some period of time, a nucleic acid sample containing the target sequences to be analyzed is prepared. The target sequence is prepared using known techniques (e.g., converted from RNA to labeled cDNA, as described above) and added to a suitable microarray. For example, an *in vitro* reverse transcription with labels covalently attached to the nucleosides is performed. Generally, the nucleic acids are labeled with a label as defined herein, especially with biotin-FITC or PE, Cy3 and Cy5.

As will be appreciated by those in the art, these assays can be direct hybridization assays or can comprise "sandwich assays", which include the use of multiple probes, as is generally outlined in U.S. Patent Nos. 5,681,702, 5,597,909, 5,545,730, 5,594,117, 5,591,584, 5,571,670, 5,580,731, 5,571,670, 5,591,584, 5,624,802, 5,635,352, 5,594,118, 5,359,100, 5,124,246 and 5,681,697, all of which are hereby incorporated by reference. In this embodiment, in general, the target nucleic acid is prepared as outlined above, and then added to the biochip comprising a plurality of nucleic acid probes, under conditions that allow the formation of a hybridization complex.

A variety of hybridization conditions may be used in the present invention, including high, moderate and low stringency conditions as outlined above. The assays are generally run under stringency conditions that allow formation of the label probe hybridization complex only in the presence of target. Stringency can be controlled by altering a step parameter that is a thermodynamic variable, including, but not limited to, temperature, formamide concentration, salt concentration, chaotropic salt concentration, pH, organic solvent concentration, etc. These parameters may also be used to control non-specific binding, as is generally outlined in U.S. Patent No. 5,681,697. Thus it may be desirable to perform certain steps at higher stringency conditions to reduce non-specific binding.

The reactions outlined herein may be accomplished in a variety of ways, as will be appreciated by those in the art. Components of the reaction may be added simultaneously, or sequentially, in any order, with preferred embodiments outlined below. In addition, the reaction may include a variety of other reagents in the assays. These include reagents like salts, buffers, neutral proteins, e.g. albumin, detergents, etc which may be used to facilitate optimal hybridization and detection, and/or reduce non-specific or background interactions. Also reagents that otherwise improve the efficiency of the assay, such as protease inhibitors, nuclease inhibitors, anti-microbial agents, etc., may be used, depending on the sample preparation methods and purity of the target. In addition, either solid phase or solution based (i.e., kinetic PCR) assays may be used..

Once the assay is run, the data are analyzed to determine the expression levels, and changes in expression levels as between states, of individual genes, forming a gene expression profile.

In a preferred embodiment, as for the diagnosis and prognosis applications, having identified the differentially expressed gene(s) or mutated gene(s) important in any one state, screens can be run to test for alteration of the expression of the CA genes individually. That is, screening for modulation of regulation of expression of a single gene can be done. Thus, for example, in the case of target genes whose presence or absence is unique between two states, screening is done for modulators of the target gene expression.

In addition, screens can be done for novel genes that are induced in response to a candidate agent. After identifying a candidate agent based upon its ability to suppress a CA expression pattern leading to a normal expression pattern, or modulate a single CA gene expression profile so as to mimic the expression of the gene from normal tissue, a screen as described above can be performed to identify genes that are specifically modulated in response to the agent. Comparing expression profiles between normal tissue and agent treated CA tissue reveals genes that are not expressed in normal tissue or CA tissue, but are expressed in agent treated tissue. These agent specific sequences can be identified and used by any of the methods described herein for CA genes or proteins. In particular these sequences and the proteins they encode find use in marking or identifying agent-treated cells. In addition, antibodies can be raised against the agent-induced proteins and used to target novel therapeutics to the treated CA tissue sample.

Thus, in one embodiment, a candidate agent is administered to a population of CA cells, that thus has an associated CA expression profile. By "administration" or "contacting" herein is meant that the candidate agent is added to the cells in such a manner as to allow the agent to act upon the cell, whether by uptake and intracellular action, or by action at the cell surface. In some embodiments, nucleic acid encoding a proteinaceous candidate agent (i.e. a peptide) may be put into a viral construct such as a retroviral construct and added to the cell, such that expression of the peptide agent is accomplished; see PCT US97/01019, hereby expressly incorporated by reference.

Once the candidate agent has been administered to the cells, the cells can be washed if desired and are allowed to incubate under preferably physiological conditions for some period of time. The cells are then harvested and a new gene expression profile is generated, as outlined herein.

Thus, for example, CA tissue may be screened for agents that reduce or suppress the CA phenotype. A change in at least one gene of the expression profile indicates that the agent has an effect on CA activity. By defining such a signature for the CA phenotype, screens for new drugs that alter the phenotype can be devised. With this approach, the drug target need not be known and need not be represented in the original expression screening platform, nor does the level of transcript for the target protein need to change.

In a preferred embodiment, as outlined above, screens may be done on individual genes and gene products (proteins). That is, having identified a particular differentially expressed gene as important in a particular state, screening of modulators of either the expression of the gene or the gene product itself can be done. The gene products of differentially expressed genes are sometimes referred to herein as "CA proteins" or "CAP". The CAP may be a fragment, or alternatively, be the full-length protein to the fragment encoded by the nucleic acids of Tables 1-7 (human genomic sequences of SEQ ID NOS: 6, 16, 26, 40, 52, 60 and 66, and sequences of SEQ ID NOS: 7, 9, 17, 19, 21, 27, 29, 41, 43, 45, 47, 53, 61, 67, 69, 71 and 73 corresponding to the human mRNAs generated therefrom). In a preferred embodiment, the CAP is selected from the human protein sequences shown in Tables 1-7 (of SEQ ID NOS: 8, 10, 18, 20, 22, 28, 30, 42, 44, 46, 48, 54, 62, 68, 70, 72 and 74). In another embodiment, the sequences are sequence variants as further described herein.

Preferably, the CAP is a fragment approximately 14 to 24 amino acids in length. More preferably the fragment is a soluble fragment. Preferably, the fragment includes a non-transmembrane region. In a preferred embodiment, the fragment has an N-terminal Cys to aid in solubility. In one embodiment, the C-terminus of the fragment is kept as a free acid and the N-terminus is a free amine to aid in coupling, e.g., to a cysteine.

In one embodiment the CA proteins are conjugated to an immunogenic agent as discussed herein. In one embodiment the CA protein is conjugated to BSA.

In a preferred embodiment, screening is done to alter the biological function of the expression product of the CA gene. Again, having identified the importance of a gene in a particular state, screening for agents that bind and/or modulate the biological activity of the gene product can be run as is more fully outlined below.

In a preferred embodiment, screens are designed to first find candidate agents that can bind to CA proteins, and then these agents may be used in assays that evaluate the ability of the candidate agent to modulate the CAP activity and the cancer phenotype. Thus, as will be appreciated by those in the art, there are a number of different assays that may be run; binding assays and activity assays.

In a preferred embodiment, binding assays are done. In general, purified or isolated gene product is used; that is, the gene products of one or more CA nucleic acids are made. In general, this is done as is known in the art. For example, antibodies are generated to the protein gene products, and standard immunoassays are run to determine the amount of protein present. Alternatively, cells comprising the CA proteins can be used in the assays.

Thus, in a preferred embodiment, the methods comprise combining a CA protein and a candidate bioactive agent, and determining the binding of the candidate agent to the CA protein. Preferred embodiments utilize the human or mouse CA protein, although other mammalian proteins may also be used, for example for the development of animal models of human disease. In some embodiments, as outlined herein, variant or derivative CA proteins may be used.

Generally, in a preferred embodiment of the methods herein, the CA protein or the candidate agent is non-diffusably bound to an insoluble support having isolated sample receiving areas (e.g. a microtiter plate, an array, etc.). The insoluble support may be made of any composition to which the compositions can be bound, is readily separated from soluble material, and is otherwise compatible with the overall method of screening. The surface of such supports may be solid or porous and of any convenient shape. Examples of suitable insoluble supports include microtiter plates, arrays, membranes and beads. These are typically made of glass, plastic (e.g., polystyrene), polysaccharides, nylon or nitrocellulose, Teflon®, etc. Microtiter plates and arrays are especially convenient because a large number of assays can be carried out simultaneously, using small amounts of reagents and samples.

The particular manner of binding of the composition is not crucial so long as it is compatible with the reagents and overall methods of the invention, maintains the activity of the composition and is nondiffusable. Preferred methods of binding include the use of antibodies (which do not sterically block either the ligand binding site or activation sequence when the protein is bound to the support), direct binding to "sticky" or ionic supports, chemical crosslinking, the synthesis of the protein or agent on the surface, etc. Following binding of the protein or agent, excess unbound material is removed by washing. The sample receiving areas may then be blocked through incubation with bovine serum albumin (BSA), casein or other innocuous protein or other moiety.

In a preferred embodiment, the CA protein is bound to the support, and a candidate bioactive agent is added to the assay. Alternatively, the candidate agent is bound to the support and the CA protein is added. Novel binding agents include specific antibodies, non-natural binding agents identified in screens of chemical libraries, peptide analogs, etc. Of particular interest are screening assays for agents that have a low toxicity for human cells. A wide variety of assays may be used for this purpose, including labeled *in vitro* protein-protein binding assays, electrophoretic mobility shift assays, immunoassays for protein binding, functional assays (phosphorylation assays, etc.) and the like.

The determination of the binding of the candidate bioactive agent to the CA protein may be done in a number of ways. In a preferred embodiment, the candidate bioactive agent is labeled, and binding determined directly. For example, this may be done by attaching all or a portion of the CA protein to a solid support, adding a labeled candidate agent (for example a fluorescent label), washing off excess reagent, and determining whether the label is present on the solid support. Various blocking and washing steps may be utilized as is known in the art.

By "labeled" herein is meant that the compound is either directly or indirectly labeled with a label which provides a detectable signal, e.g. radioisotope, fluorescers, enzyme, antibodies, particles such as magnetic particles, chemiluminescers, or specific binding molecules, etc. Specific binding molecules include pairs, such as biotin and streptavidin, digoxin and antidigoxin etc. For the specific binding members, the complementary member would normally be labeled with a molecule which provides for detection, in accordance with known procedures, as outlined above. The label can directly or indirectly provide a detectable signal.

In some embodiments, only one of the components is labeled. For example, the proteins (or proteinaceous candidate agents) may be labeled at tyrosine positions using $^{125}$I, or with fluorophores. Alternatively, more than one component may be labeled with different labels; using $^{125}$I for the proteins, for example, and a fluorophore for the candidate agents.

In a preferred embodiment, the binding of the candidate bioactive agent is determined through the use of competitive binding assays. In this embodiment, the competitor is a binding moiety known to bind to the target molecule (i.e. CA protein), such as an antibody, peptide, binding partner, ligand, etc. Under certain circumstances, there may be competitive binding as between the bioactive agent and the binding moiety, with the binding moiety displacing the bioactive agent.

In one embodiment, the candidate bioactive agent is labeled. Either the candidate bioactive agent, or the competitor, or both, is added first to the protein for a time sufficient to allow binding, if present. Incubations may be performed at any temperature which facilitates optimal activity, typically between 4 and 40° C. Incubation periods are selected for optimum activity, but may also be optimized to facilitate rapid high throughput screening. Typically between 0.1 and 1 hour will be sufficient. Excess reagent is generally removed or washed away. The second component is then added, and the presence or absence of the labeled component is followed, to indicate binding.

In a preferred embodiment, the competitor is added first, followed by the candidate bioactive agent. Displacement of the competitor is an indication that the candidate bioactive agent is binding to the CA protein and thus is capable of binding to, and potentially modulating, the activity of the CA protein. In this embodiment, either component can be labeled. Thus, for example, if the competitor is labeled, the presence of label in the wash solution indicates displacement by the agent. Alternatively, if the candidate bioactive agent is labeled, the presence of the label on the support indicates displacement.

In an alternative embodiment, the candidate bioactive agent is added first, with incubation and washing, followed by the competitor. The absence of binding by the competitor may indicate that the bioactive agent is bound to the CA protein with a higher affinity. Thus, if the candidate bioactive agent is labeled, the presence of the label on the support, coupled with a lack of competitor binding, may indicate that the candidate agent is capable of binding to the CA protein.

In a preferred embodiment, the methods comprise differential screening to identity bioactive agents that are capable of modulating the activity of the CA proteins. In this embodiment, the methods comprise combining a CA protein and a competitor in a first sample. A second sample comprises a candidate bioactive agent, a CA protein and a competitor. The binding of the competitor is determined for both samples, and a change, or difference in binding between the two samples indicates the presence of an agent capable of binding to the CA protein and potentially modulating its activity. That is, if the binding of the competitor is different in the second sample relative to the first sample, the agent is capable of binding to the CA protein.

Alternatively, a preferred embodiment utilizes differential screening to identify drug candidates that bind to the native CA protein, but cannot bind to modified CA proteins. The structure of the CA protein may be modeled, and used in rational drug design to synthesize agents that interact with that site. Drug candidates that affect CA bioactivity are also identified by screening drugs for the ability to either enhance or reduce the activity of the protein.

Positive controls and negative controls may be used in the assays. Preferably all control and test samples are performed in at least triplicate to obtain statistically significant results. Incubation of all samples is for a time sufficient for the binding of the agent to the protein. Following incubation, all samples are washed free of non-specifically bound material and the amount of bound, generally labeled agent determined. For example, where a radiolabel is employed, the samples may be counted in a scintillation counter to determine the amount of bound compound.

A variety of other reagents may be included in the screening assays. These include reagents like salts, neutral proteins, e.g. albumin, detergents, etc which may be used to facilitate optimal protein-protein binding and/or reduce non-specific or background interactions. Also reagents that otherwise improve the efficiency of the assay, such as protease inhibitors, nuclease inhibitors, anti-microbial agents, etc., may be used. The mixture of components may be added in any order that provides for the requisite binding.

Screening for agents that modulate the activity of CA proteins may also be done. In a preferred embodiment, methods for screening for a bioactive agent capable of modulating the activity of CA proteins comprise the steps of adding a candidate bioactive agent to a sample of CA proteins, as above, and determining an alteration in the biological activity of CA proteins. "Modulating the activity of a CA protein" includes an increase in activity, a decrease in activity, or a change in the type or kind of activity present. Thus, in this embodiment, the candidate agent should both bind to CA proteins (although this may not be necessary), and alter its biological or biochemical activity as defined herein. The methods include both *in vitro* screening methods, as are generally outlined above, and in vivo screening of cells for alterations in the presence, distribution, activity or amount of CA proteins.

Thus, in this embodiment, the methods comprise combining a CA sample and a candidate bioactive agent, and evaluating the effect on CA activity. By "CA activity" or grammatical equivalents herein is meant one of the CA protein's biological activities, including, but not limited to, its role in tumorigenesis, including cell division, preferably in lymphatic tissue, cell proliferation, tumor growth and transformation of cells. In one embodiment, CA activity includes activation of or by a protein encoded by a nucleic acid of Tables 1-7. An inhibitor of CA activity is the inhibition of any one or more CA activities.

In a preferred embodiment, the activity of the CA protein is increased; in another preferred embodiment, the activity of the CA protein is decreased. Thus, bioactive agents that are antagonists are preferred in some embodiments, and bioactive agents that are agonists may be preferred in other embodiments.

In a preferred embodiment, the invention provides methods for screening for bioactive agents capable of modulating the activity of a CA protein. The methods comprise adding a candidate bioactive agent, as defined above, to a cell comprising CA proteins. Preferred cell types include almost any cell. The cells contain a recombinant nucleic acid that encodes a CA protein. In a preferred embodiment, a library of candidate agents is tested on a plurality of cells.

In one aspect, the assays are evaluated in the presence or absence or previous or subsequent exposure of physiological signals, for example hormones, antibodies, peptides, antigens, cytokines, growth factors, action potentials, pharmacological agents including chemotherapeutics, radiation, carcinogenics, or other cells (i.e. cell-cell contacts). In another example, the determinations are determined at different stages of the cell cycle process.

In this way, bioactive agents are identified. Compounds with pharmacological activity are able to enhance or interfere with the activity of the CA protein.

**Applications of the invention**

In one embodiment, a method of inhibiting cancer cell division is provided. In another embodiment, a method of inhibiting tumor growth is provided. In a further embodiment, methods of treating cells or individuals with cancer are provided.

The method comprises administration of a cancer inhibitor. In particular embodiments, the cancer inhibitor is an antisense molecule, a pharmaceutical composition, a therapeutic agent or small molecule, or a monoclonal, polyclonal, chimeric or humanized antibody. In particular embodiments, a therapeutic agent is coupled with a an antibody, preferable a monoclonal antobody.

In other embodiments, methods for detection or diagnosis of cancer cells in an individual are provided. In particular embodiments, the diagnostic/detection agent is a small molecule that pereferentially binds to a CAP according to the invention. In one embodiment, the diagnostic/detection agent is an antibody, preferably a monoclonal antobody, preferably linked to a detectable agent.

In other embodiments of the invention, animal models and transgenic animals are provided, which find use in generating animal models of cancers, particularly lymphomas and carcinomas.

**(a) Antisense molecules**

In one embodiment, the cancer inhibitor is an antisense molecule. Antisense molecules as used herein include antisense or sense oligonucleotides comprising a single-stranded nucleic acid sequence (either RNA or DNA) capable of binding to target mRNA (sense) or DNA (antisense) sequences for cancer molecules. Antisense or sense oligonucleotides, according to the present invention, comprise a fragment generally at least about 14 nucleotides, preferably from about 14 to 30 nucleotides. The ability to derive an antisense or a sense oligonucleotide, based upon a cDNA sequence encoding a given protein is described in, for example, Stein and Cohen, Cancer Res. 48:2659, (1988) and van der Krol et al., BioTechniques 6:958, (1988).

Antisense molecules can be modified or unmodified RNA, DNA, or mixed polymer oligonucleotides. These molecules function by specifically binding to matching sequences resulting in inhibition of peptide synthesis (Wu-Pong, Nov 1994, BioPharm, 20-33) either by steric blocking or by activating an RNase H enzyme. Antisense molecules can also alter protein synthesis by interfering with RNA processing or transport from the nucleus into the cytoplasm (Mukhopadhyay & Roth, 1996, Crit. Rev. in Oncogenesis 7, 151-190). In addition, binding of single stranded DNA to RNA can result in nuclease-mediated degradation of the heteroduplex (Wu-Pong, supra). Backbone modified DNA chemistry which have thus far been shown to act as substrates for RNase H are phosphorothioates, phosphorodithioates, borontrifluoridates, and 2'-arabino and 2'-fluoro arabino-containing oligonucleotides.

Antisense molecules may be introduced into a cell containing the target nucleotide sequence by formation of a conjugate with a ligand binding molecule, as described in WO 91/04753. Suitable ligand binding molecules include, but are not limited to, cell surface receptors, growth factors, other cytokines, or other ligands that bind to cell surface receptors. Preferably, conjugation of the ligand binding molecule does not substantially interfere with the ability of the ligand binding molecule to bind to its corresponding molecule or receptor, or block entry of the sense or antisense oligonucleotide or its conjugated version into the cell. Alternatively, a sense or an antisense oligonucleotide may be introduced into a cell containing the target nucleic acid sequence by formation of an oligonucleotide-lipid complex, as described in WO 90/10448. It is understood that the use of antisense molecules or knock out and knock in models may also be used in screening assays as discussed above, in addition to methods of treatment.

**(b) RNA Interference**

RNA interference refers to the process of sequence-specific post transcriptional gene silencing in animals mediated by short interfering RNAs (siRNA) (Fire et al., Nature, 391, 806 (1998)). The corresponding process in plants is referred to as post transcriptional gene silencing or RNA silencing and is also referred to as quelling in fungi. The presence of dsRNA in cells triggers the RNAi response though a mechanism that has yet to be fully characterized. This mechanism appears to be different from the interferon response that results from dsRNA mediated activation of protein kinase PKR and 2',5'-oligoadenylate synthetase resulting in non-specific cleavage of mRNA by ribonuclease L. (reviewed in Sharp, P.A., RNA interference - 2001, Genes & Development 15:485-490 (2001)).

Small interfering RNAs (siRNAs) are powerful sequence-specific reagents designed to suppress the expression of genes in cultured mammalian cells through a process known as RNA interference (RNAi). Elbashir, S.M. et al. Nature 411:494-498 (2001); Caplen, N.J. et al. Proc. Natl. Acad. Sci. USA 98:9742-9747 (2001); Harborth, J. et al. J. Cell Sci. 114:4557-4565 (2001). The term "short interfering RNA" or "siRNA" refers to a double stranded nucleic acid molecule capable of RNA interference "RNAi", (*see* Kreutzer et al., WO 00/44895; Zernicka-Goetz et al. WO 01/36646; Fire, WO 99/32619; Mello and Fire, WO 01/29058). As used herein, siRNA molecules are limited to RNA molecules but further encompasses chemically modified nucleotides and non-nucleotides. siRNA gene-targeting experiments have been carried out by transient siRNA transfer into cells (achieved by such classic methods as liposome-mediated transfection, electroporation, or microinjection).

Molecules of siRNA are 21- to 23-nucleotide RNAs, with characteristic 2-to 3-nucleotide 3'-overhanging ends resembling the RNase III processing products of long double-stranded RNAs (dsRNAs) that normally initiate RNAi. When introduced into a cell, they assemble with yet-to-be-identified proteins of an endonuclease complex (RNA-induced silencing complex), which then guides target mRNA cleavage. As a consequence of degradation of the targeted mRNA, cells with a specific phenotype characteristic of suppression of the corresponding protein product are obtained. The small size of siRNAs, compared with traditional antisense molecules, prevents activation of the dsRNA-inducible interferon system present in mammalian cells. This avoids the nonspecific phenotypes normally produced by dsRNA larger than 30 base pairs in somatic cells.

Intracellular transcription of small RNA molecules is achieved by cloning the siRNA templates into RNA polymerase III (Pol III) transcription units, which normally encode the small nuclear RNA (snRNA) U6 or the human RNase P RNA H1. Two approaches have been developed for expressing siRNAs: in the first, sense and antisense strands constituting the siRNA duplex are transcribed by individual promoters (Lee, N.S. et al. Nat. Biotechnol. 20, 500-505 (2002). Miyagishi, M. & Taira, K. Nat. Biotechnol. 20, 497-500 (2002).); in the second, siRNAs are expressed as fold-back stem-loop structures that give rise to siRNAs after intracellular processing (Paul, C.P. et al. Nat. Biotechnol. 20:505-508 (2002)). The endogenous expression of siRNAs from introduced DNA templates is thought to overcome some limitations of exogenous siRNA delivery, in particular the transient loss of phenotype. U6 and H1 RNA promoters are members of the type III class of Pol III promoters. (Paule, M.R. & White, R.J. Nucleic Acids Res. 28, 1283-1298 (2000)).

Co-expression of sense and antisense siRNAs mediate silencing of target genes, whereas expression of sense or antisense siRNA alone do not greatly affect target gene expression. Transfection of plasmid DNA, rather than synthetic siRNAs, may appear advantageous, considering the danger of RNase contamination and the costs of chemically synthesized siRNAs or siRNA transcription kits. Stable expression of siRNAs allows new gene therapy applications, such as treatment of persistent viral infections. Considering the high specificity of siRNAs, the approach also allows the targeting of disease-derived transcripts with point mutations, such as *RAS* or *TP53* oncogene transcripts, without alteration of the remaining wild-type allele. Finally, by high-throughput sequence analysis of the various genomes, the DNA-based methodology may also be a cost-effective alternative for automated genome-wide loss-of-function phenotypic analysis, especially when combined with miniaturized array-based phenotypic screens. (Ziauddin, J. & Sabatini, D.M. Nature 411:107-110 (2001)).

The presence of long dsRNAs in cells stimulates the activity of a ribonuclease III enzyme referred to as dicer. Dicer is involved in the processing of the dsRNA into short pieces of dsRNA known as short interfering RNAs (siRNA) (Berstein et al., 2001, Nature, 409:363 (2001)). Short interfering RNAs derived from dicer activity are typically about 21-23 nucleotides in length and comprise about 19 base pair duplexes. Dicer has also been implicated in the excision of 21 and 22 nucleotide small temporal RNAs (stRNA) from precursor RNA of conserved structure that are implicated in translational control (Hutvagner et al., Science, 293, 834 (2001)). The RNAi response also features an endonuclease complex containing a siRNA, commonly referred to as an RNA-induced silencing complex (RISC), which mediates cleavage of single stranded RNA having sequence homologous to the siRNA. Cleavage of the target RNA takes place in the middle of the region complementary to the guide sequence of the siRNA duplex (Elbashir et al., Genes Dev., 15, 188 (2001)).

This invention provides an expression system comprising an isolated nucleic acid molecule comprising a sequence capable of specifically hybridizing to the CA sequences. In an embodiment, the nucleic acid molecule is capable of inhibiting the expression of the CA protein. A method of inhibiting expression of CA inside a cell by a vector-directed expression of a short RNA which short RNA can fold in itself and create a double strand RNA having CA mRNA sequence identity and able to trigger posttranscriptional gene silencing, or RNA interference (RNAi), of the CA gene inside the cell. In another method a short double strand RNA having CA mRNA sequence identity is delivered inside the cell to trigger posttranscriptional gene silencing, or RNAi, of the CA gene. In various embodiments, the nucleic acid molecule is at least a 7 mer, at least a 10 mer, or at least a 20 mer. In a further embodiment, the sequence is unique.

## (c) Pharmaceutical Compositions

Pharmaceutical compositions encompassed by the present invention include as active agent, the polypeptides, polynucleotides, antisense oligonucleotides, or antibodies of the invention disclosed herein in a therapeutically effective amount. An "effective amount" is an amount sufficient to effect beneficial or desired results, including clinical results. An effective amount can be administered in one or more administrations. For purposes of this invention, an effective amount of an adenoviral vector is an amount that is sufficient to palliate, ameliorate, stabilize, reverse, slow or delay the progression of the disease state.

The compositions can be used to treat cancer as well as metastases of primary cancer. In addition, the pharmaceutical compositions can be used in conjunction with conventional methods of cancer treatment, *e.g.*, to sensitize tumors to radiation or conventional chemotherapy. The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

Where the pharmaceutical composition comprises an antibody that specifically binds to a gene product encoded by a differentially expressed polynucleotide, the antibody can be coupled to a drug for delivery to a treatment site or coupled to a detectable label to facilitate imaging of a site comprising cancer cells, such as prostate cancer cells. Methods for coupling antibodies to drugs and detectable labels are well known in the art, as are methods for imaging using detectable labels.

A "patient" for the purposes of the present invention includes both humans and other animals, particularly mammals, and organisms. Thus the methods are applicable to both human therapy and veterinary applications. In the preferred embodiment the patient is a mammal, and in the most preferred embodiment the patient is human.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. The effective amount for a given situation is determined by routine experimentation and is within the judgment of the clinician. For purposes of the present invention, an effective dose will generally be from about 0.01 mg/kg to about 5 mg/kg, or about 0.01 mg/kg to about 50 mg/kg or about 0.05 mg/kg to about 10 mg/kg of the compositions of the present invention in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles. Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g., mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington: The Science and Practice of Pharmacy (1995) Alfonso Gennaro, Lippincott, Williams, & Wilkins.

The pharmaceutical compositions can be prepared in various forms, such as granules, tablets, pills, suppositories, capsules, suspensions, salves, lotions and the like. Pharmaceutical grade organic or inorganic carriers and/or diluents suitable for oral and topical use can be used to make up compositions containing the therapeutically-active compounds. Diluents known to the art include aqueous media, vegetable and animal oils and fats. Stabilizing agents, wetting and emulsifying agents, salts for varying the osmotic pressure or buffers for securing an adequate pH value, and skin penetration enhancers can be used as auxiliary agents.

The pharmaceutical compositions of the present invention comprise a CA protein in a form suitable for administration to a patient. In the preferred embodiment, the pharmaceutical compositions are in a water soluble form, such as being present as pharmaceutically acceptable salts, which is meant to include both acid and base addition salts. "Pharmaceutically acceptable acid addition salt" refers to those salts that retain the biological effectiveness of the free bases and that are not biologically or otherwise undesirable, formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid and the like. "Pharmaceutically acceptable base addition salts" include those derived from inorganic bases such as sodium, potassium, lithium, ammonium, calcium, magnesium, iron, zinc, copper, manganese, aluminum salts and the like. Particularly preferred are the ammonium, potassium, sodium, calcium, and magnesium salts. Salts derived from pharmaceutically acceptable organic non-toxic bases include salts of primary, secondary, and tertiary amines, substituted amines including naturally occurring substituted amines, cyclic amines and basic ion exchange resins, such as isopropylamine, trimethylamine, diethylamine, triethylamine, tripropylamine, and ethanolamine.

The pharmaceutical compositions may also include one or more of the following: carrier proteins such as serum albumin; buffers; fillers such as microcrystalline cellulose, lactose, corn and other starches; binding agents; sweeteners and other flavoring agents; coloring agents; and polyethylene glycol. Additives are well known in the art, and are used in a variety of formulations.

The compounds having the desired pharmacological activity may be administered in a physiologically acceptable carrier to a host, as previously described. The agents may be administered in a variety of ways, orally, parenterally e.g., subcutaneously, intraperitoneally, intravascularly, etc. Depending upon the manner of introduction, the compounds may be formulated in a variety of ways. The concentration of therapeutically active compound in the formulation may vary from about 0.1-100% wgt/vol. Once formulated, the compositions contemplated by the invention can be (1) administered directly to the subject (*e.g.*, as polynucleotide, polypeptides, small molecule agonists or antagonists, and the like); or (2) delivered ex vivo, to cells derived from the subject (*e.g.*, as in *ex vivo* gene therapy). Direct delivery of the compositions will generally be accomplished by parenteral injection, e.g., subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the ex vivo delivery and reimplantation of transformed cells into a subject are known in the art and described in *e.g.*, International Publication No. WO 93/14778. Examples of cells useful in ex vivo applications include, for example, stem cells, particularly hematopoetic, lymph cells, macrophages, dendritic cells, or tumor cells. Generally, delivery of nucleic acids for both ex vivo and in vitro applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once differential expression of a gene corresponding to a CA polynucleotide described herein has been found to correlate with a proliferative disorder, such as neoplasia, dysplasia, and hyperplasia, the disorder can be amenable to treatment by administration of a therapeutic agent based on the provided polynucleotide, corresponding polypeptide or other corresponding molecule (e.g., antisense, ribozyme, etc.). In other embodiments, the disorder can be amenable to treatment by administration of a small molecule drug that, for example, serves as an inhibitor (antagonist) of the function of the encoded gene product of a gene having increased expression in cancerous cells relative to normal cells or as an agonist for gene products that are decreased in expression in cancerous cells (*e.g.*, to promote the activity of gene products that act as tumor suppressors).

The dose and the means of administration of the inventive pharmaceutical compositions are determined based on the specific qualities of the therapeutic composition, the condition, age, and weight of the patient, the progression of the disease, and other relevant factors. For example, administration of polynucleotide therapeutic compositions agents includes local or systemic administration, including injection, oral administration, particle gun or catheterized administration, and topical administration. Preferably, the therapeutic polynucleotide composition contains an expression construct comprising a promoter operably linked to a polynucleotide of at least 12, 22, 25, 30, or 35 contiguous nt of the polynucleotide disclosed herein. Various methods can be used to administer the therapeutic composition directly to a specific site in the body. For example, a small metastatic lesion is located and the therapeutic composition injected several times in several different locations within the body of tumor. Alternatively, arteries that serve a tumor are identified, and the therapeutic composition injected into such an artery, in order to deliver the composition directly into the tumor. A tumor that has a necrotic center is aspirated and the composition injected directly into the now empty center of the tumor. An antisense composition is directly administered to the surface of the tumor, for example, by topical application of the composition. X-ray imaging is used to assist in certain of the above delivery methods.

Targeted delivery of therapeutic compositions containing an antisense polynucleotide, subgenomic polynucleotides, or antibodies to specific tissues can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad Sci. (USA) (1990) 87:3655; Wu et al., J. Biol. Chem. (1991) 266: 338. Therapeutic compositions containing a polynucleotide are administered in a range of about 100 ng to about 200 mg of DNA for local administration in a gene therapy protocol. Concentration ranges of about 500 ng to about 50 mg, about 1 $\mu$g to about 2 mg, about 5 $\mu$g to about 500 $\mu$g, and about 20 $\mu$g to about 100 $\mu$g of DNA can also be used during a gene therapy protocol. Factors such as method of action (e.g., for enhancing or inhibiting levels of the encoded gene product) and efficacy of transformation and expression are considerations that will affect the dosage required for ultimate efficacy of the antisense subgenomic polynucleotides. Where greater expression is desired over a larger area of tissue, larger amounts of antisense subgenomic polynucleotides or the same amounts re-administered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect.

The therapeutic polynucleotides and polypeptides of the present invention can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, e.g., WO 90/07936; WO 94/03622; WO 93/25698; WO 93/25234; USPN 5, 219,740; WO 93/11230; WO 93/10218; USPN 4,777,127; GB Patent No. 2,200,651; EP 0 345 242; and WO 91/02805), alphavirus-based vectors (e.g., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532)), and adeno-associated virus (AAV) vectors (see, e.g., WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, e.g., Curiel, Hum. Gene Ther. (1992) 3:147); ligand-linked DNA (see, e.g., Wu, J. Biol. Chem. (1989) 264:16985); eukaryotic cell delivery vehicles cells (see, e.g., USPN 5,814,482; WO 95/07994; WO 96/17072;

WO 95/30763; and WO 97/42338) and nucleic charge neutralization or fusion with cell membranes. Naked DNA can also be employed. Exemplary naked DNA introduction methods are described in WO 90/11092 and USPN 5,580,859. Liposomes that can act as gene delivery vehicles are described in USPN 5,422,120; WO 95/13796; WO 94/23697; WO 91/14445; and EP 0524968. Additional approaches are described in Philip, Mol. Cell Biol. (1994) 14:2411, and in Woffendin, Proc. Natl. Acad. Sci. (1994) 91:1581.

Further non-viral delivery suitable for use includes mechanical delivery systems such as the approach described in Woffendin et al., Proc. Natl. Acad. Sci. USA (1994) 91(24):11581. Moreover, the coding sequence and the product of expression of such can be delivered through deposition of photopolymerized hydrogel materials or use of ionizing radiation (see, e.g., USPN 5,206,152 and WO 92/11033). Other conventional methods for gene delivery that can be used for delivery of the coding sequence include, for example, use of hand-held gene transfer particle gun (see, e.g., USPN 5,149,655); use of ionizing radiation for activating transferred gene (see, e.g., USPN 5,206,152 and WO 92/11033).

The administration of the CA proteins and modulators of the present invention can be done in a variety of ways as discussed above, including, but not limited to, orally, subcutaneously, intravenously, intranasally, transdermally, intraperitoneally, intramuscularly, intrapulmonary, vaginally, rectally, or intraocularly. In some instances, for example, in the treatment of wounds and inflammation, the CA proteins and modulators may be directly applied as a solution or spray.

In a preferred embodiment, CA proteins and modulators are administered as therapeutic agents, and can be formulated as outlined above. Similarly, CA genes (including both the full-length sequence, partial sequences, or regulatory sequences of the CA coding regions) can be administered in gene therapy applications, as is known in the art. These CA genes can include antisense applications, either as gene therapy (i.e. for incorporation into the genome) or as antisense compositions, as will be appreciated by those in the art.

Thus, in one embodiment, methods of modulating CA gene activity in cells or organisms are provided. In one embodiment, the methods comprise administering to a cell an anti-CA antibody that reduces or eliminates the biological activity of an endogenous CA protein. Alternatively, the methods comprise administering to a cell or organism a recombinant nucleic acid encoding a CA protein. As will be appreciated by those in the art, this may be accomplished in any number of ways. In a preferred embodiment, for example when the CA sequence is down-regulated in cancer, the activity of the CA gene product is increased by increasing the amount of CA expression in the cell, for example by overexpressing the endogenous CA gene or by administering a gene encoding the CA sequence, using known gene-therapy techniques. In a preferred embodiment, the gene therapy techniques include the incorporation of the exogenous gene using enhanced homologous recombination (EHR), for example as described in PCT/US93/03868, hereby incorporated by reference in its entirety. Alternatively, for example when the CA sequence is up-regulated in cancer, the activity of the endogenous CA gene is decreased, for example by the administration of a CA antisense nucleic acid.

### (d) Vaccines

In a preferred embodiment, CA genes are administered as DNA vaccines, either single genes or combinations of CA genes. Naked DNA vaccines are generally known in the art. Brower, Nature Biotechnology, 16:1304-1305 (1998).

In one embodiment, CA genes of the present invention are used as DNA vaccines. Methods for the use of genes as DNA vaccines are well known to one of ordinary skill in the art, and include placing a CA gene or portion of a CA gene under the control of a promoter for expression in a patient with cancer. The CA gene used for DNA vaccines can encode full-length CA proteins, but more preferably encodes portions of the CA proteins including peptides derived from the CA protein. In a preferred embodiment a patient is immunized with a DNA vaccine comprising a plurality of nucleotide sequences derived from a CA gene. Similarly, it is possible to immunize a patient with a plurality of CA genes or portions thereof. Without being bound by theory, expression of the polypeptide encoded by the DNA vaccine, cytotoxic T-cells, helper T-cells and antibodies are induced that recognize and destroy or eliminate cells expressing CA proteins.

In a preferred embodiment, the DNA vaccines include a gene encoding an adjuvant molecule with the DNA vaccine. Such adjuvant molecules include cytokines that increase the immunogenic response to the CA polypeptide encoded by the DNA vaccine. Additional or alternative adjuvants are known to those of ordinary skill in the art and find use in the invention.

### (e) Antibodies

In one embodiment, a cancer inhibitor is an antibody as discussed above. In one embodiment, the CA proteins of the present invention may be used to generate polyclonal and monoclonal antibodies to CA proteins, which are useful as described herein. Similarly, the CA proteins can be coupled, using standard technology, to affinity chromatography columns. These columns may then be used to purify CA antibodies. In a preferred embodiment, the antibodies are generated to epitopes unique to a CA protein; that is, the antibodies show little or no cross-reactivity to other proteins. These antibodies find use in a number of applications. For example, the CA antibodies may be coupled to standard affinity chromatography columns and used to purify CA proteins. The antibodies may also be used therapeutically as blocking polypeptides, as outlined above, since they will specifically bind to the CA protein.

The present invention further provides methods for detecting the presence of and/or measuring a level of a polypeptide in a biological sample, which CA polypeptide is encoded by a CA polynucleotide that is differentially expressed in a cancer cell, using an antibody specific for the encoded polypeptide. The methods generally comprise: a) contacting the sample with an antibody specific for a polypeptide encoded by a CA polynucleotide that is differentially expressed in a prostate cancer cell; and b) detecting binding between the antibody and molecules of the sample.

Detection of specific binding of the antibody specific for the encoded cancer-associated polypeptide, when compared to a suitable control is an indication that encoded polypeptide is present in the sample. Suitable controls include a sample known not to contain the encoded CA polypeptide or known not to contain elevated levels of the polypeptide; such as normal tissue, and a sample contacted with an antibody not specific for the encoded polypeptide, e.g., an anti-idiotype antibody. A variety of methods to detect specific antibody-antigen interactions are known in the art and can be used in the method, including, but not limited to, standard immunohistological methods, immunoprecipitation, an enzyme immunoassay, and a radioimmunoassay. In general, the specific antibody will be detectably labeled, either directly or indirectly. Direct labels include radioisotopes; enzymes whose products are detectable (e.g., luciferase, β-galactosidase, and the like); fluorescent labels (e.g., fluorescein isothiocyanate, rhodamine, phycoerythrin, and the like); fluorescence emitting metals, e.g., $^{152}$Eu, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the like. The antibody may be attached (coupled) to an insoluble support, such as a polystyrene plate or a bead. Indirect labels include second antibodies specific for antibodies specific for the encoded polypeptide ("first specific antibody"), wherein the second antibody is labeled as described above; and members of specific binding pairs, e.g., biotin-avidin, and the like. The biological sample may be brought into contact with and immobilized on a solid support or carrier, such as nitrocellulose, that is capable of immobilizing cells, cell particles, or soluble proteins. The support may then be washed with suitable buffers, followed by contacting with a detectably-labeled first specific antibody. Detection methods are known in the art and will be chosen as appropriate to the signal emitted by the detectable label. Detection is generally accomplished in comparison to suitable controls, and to appropriate standards.

In some embodiments, the methods are adapted for use *in vivo*, e.g., to locate or identify sites where cancer cells are present. In these embodiments, a detectably-labeled moiety, e.g., an antibody, which is specific for a cancer-associated polypeptide is administered to an individual (e.g., by injection), and labeled cells are located using standard imaging techniques, including, but not limited to, magnetic resonance imaging, computed tomography scanning, and the like. In this manner, cancer cells are differentially labeled.

**(f) Detection and Diagnosis of Cancers**

Without being bound by theory, it appears that the various CA sequences are important in cancers. Accordingly, disorders based on mutant or variant CA genes may be determined. In one embodiment, the invention provides methods for identifying cells containing variant CA genes comprising determining all or part of the sequence of at least one endogenous CA genes in a cell. As will be appreciated by those in the art, this may be done using any number of sequencing techniques. In a preferred embodiment, the invention provides methods of identifying the CA genotype of an individual comprising determining all or part of the sequence of at least one CA gene of the individual. This is generally done in at least one tissue of the individual, and may include the evaluation of a number of tissues or different samples of the same tissue. The method may include comparing the sequence of the sequenced CA gene to a known CA gene, i.e., a wild-type gene. As will be appreciated by those in the art, alterations in the sequence of some CA genes can be an indication of either the presence of the disease, or propensity to develop the disease, or prognosis evaluations.

The sequence of all or part of the CA gene can then be compared to the sequence of a known CA gene to determine if any differences exist. This can be done using any number of known homology programs, such as Bestfit, etc. In a preferred embodiment, the presence of a difference in the sequence between the CA gene of the patient and the known CA gene is indicative of a disease state or a propensity for a disease state, as outlined herein.

In a preferred embodiment, the CA genes are used as probes to determine the number of copies of the CA gene in the genome. For example, some cancers exhibit chromosomal deletions or insertions, resulting in an alteration in the copy number of a gene.

In another preferred embodiment CA genes are used as probes to determine the chromosomal location of the CA genes. Information such as chromosomal location finds use in providing a diagnosis or prognosis in particular when chromosomal abnormalities such as translocations, and the like are identified in CA gene loci.

The present invention provides methods of using the polynucleotides described herein for detecting cancer cells, facilitating diagnosis of cancer and the severity of a cancer (*e.g.*, tumor grade, tumor burden, and the like) in a subject, facilitating a determination of the prognosis of a subject, and assessing the responsiveness of the subject to therapy (*e.g.*, by providing a measure of therapeutic effect through, for example, assessing tumor burden during or following a chemotherapeutic regimen). Detection can be based on detection of a polynucleotide that is differentially expressed in a cancer cell, and/or detection of a polypeptide encoded by a polynucleotide that is differentially expressed in a cancer cell. The detection methods of the invention can be conducted *in vitro* or *in vivo*, on isolated cells, or in whole tissues or a bodily fluid *e.g.*, blood, plasma, serum, urine, and the like).

In some embodiments, methods are provided for detecting a cancer cell by detecting expression in the cell of a transcript that is differentially expressed in a cancer cell. Any of a variety of known methods can be used for detection, including, but not limited to, detection of a transcript by hybridization with a polynucleotide that hybridizes to a polynucleotide that is differentially expressed in a prostate cancer cell; detection of a transcript by a polymerase chain reaction using specific oligonucleotide primers; *in situ* hybridization of a cell using as a probe a polynucleotide that hybridizes to a gene that is differentially expressed in a prostate cancer cell. The methods can be used to detect and/or measure mRNA levels of a gene that is differentially expressed in a cancer cell. In some embodiments, the methods comprise: a) contacting a sample with a polynucleotide that corresponds to a differentially expressed gene described herein under conditions that allow hybridization; and b) detecting hybridization, if any.

Detection of differential hybridization, when compared to a suitable control, is an indication of the presence in the sample of a polynucleotide that is differentially expressed in a cancer cell. Appropriate controls include, for example, a sample that is known not to contain a polynucleotide that is differentially expressed in a cancer cell, and use of a labeled polynucleotide of the same "sense" as the polynucleotide that is differentially expressed in the cancer cell. Conditions that allow hybridization are known in the art, and have been described in more detail above. Detection can also be accomplished by any known method, including, but not limited to, *in situ* hybridization, PCR (polymerase chain reaction), RT-PCR (reverse transcription-PCR), TMA, bDNA, and Nasbau and "Northern" or RNA blotting, or combinations of such techniques, using a suitably labeled polynucleotide. A variety of labels and labeling methods for polynucleotides are known in the art and can be used in the assay methods of the invention. Specificity of hybridization can be determined by comparison to appropriate controls.

Polynucleotides generally comprising at least 10 nt, at least 12nt or at least 15 contiguous nucleotides of a polynucleotide provided herein, such as, for example, those having the sequence as depicted in Tables 1-7, are used for a variety of purposes, such as probes for detection of and/or measurement of, transcription levels of a polynucleotide that is differentially expressed in a prostate cancer cell. As will be readily appreciated by the ordinarily skilled artisan, the probe can be detectably labeled and contacted with, for example, an array comprising immobilized polynucleotides obtained from a test sample (*e.g.*, mRNA). Alternatively, the probe can be immobilized on an array and the test sample detectably labeled. These and other variations of the methods of the invention are well within the skill in the art and are within the scope of the invention.

Nucleotide probes are used to detect expression of a gene corresponding to the provided polynucleotide. In Northern blots, mRNA is separated electrophoretically and contacted with a probe. A probe is detected as hybridizing to an mRNA species of a particular size. The amount of hybridization can be quantitated to determine relative amounts of expression, for example under a particular condition. Probes are used for in situ hybridization to cells to detect expression. Probes can also be used *in vivo* for diagnostic detection of hybridizing sequences. Probes are typically labeled with a radioactive isotope. Other types of detectable labels can be used such as chromophores, fluorophores, and enzymes. Other examples of nucleotide hybridization assays are described in WO92/02526 and USPN 5,124,246.

PCR is another means for detecting small amounts of target nucleic acids (see, *e.g.*, Mullis et al., Meth. Enzymol. (1987) 155:335; USPN 4,683,195; and USPN 4,683,202). Two primer oligonucleotides that hybridize with the target nucleic acids are used to prime the reaction. The primers can be composed of sequence within or 3' and 5' to the CA polynucleotides disclosed herein. Alternatively, if the primers are 3' and 5' to these polynucleotides, they need not hybridize to them or the complements. After amplification of the target with a thermostable polymerase, the amplified target nucleic acids can be detected by methods known in the art, e.g., Southern blot. mRNA or cDNA can also be detected by traditional blotting techniques (e.g., Southern blot, Northern blot, etc.) described in Sambrook et al., "Molecular Cloning: A Laboratory Manual" (New York, Cold Spring Harbor Laboratory, 1989) (e.g., without PCR amplification). In general, mRNA or cDNA generated from mRNA using a polymerase enzyme can be purified and separated using gel electrophoresis, and transferred to a solid support, such as nitrocellulose. The solid support is exposed to a labeled probe, washed to remove any unhybridized probe, and duplexes containing the labeled probe are detected.

Methods using PCR amplification can be performed on the DNA from a single cell, although it is convenient to use at least about $10^5$ cells. The use of the polymerase chain reaction is described in Saiki et al. (1985) Science 239: 487, and a review of current techniques may be found in Sambrook, et al. Molecular Cloning: A Laboratory Manual, CSH Press 1989, pp.14.2-14.33. A detectable label may be included in the amplification reaction. Suitable detectable labels include fluorochromes,(*e.g.* fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein, 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA)), radioactive labels, (*e.g.* $^{32}$P, $^{35}$S, $^{3}$H, *etc.*), and the like. The label may be a two stage system, where the polynucleotides is conjugated to biotin, haptens, *etc.* having a high affinity binding partner, *e.g.* avidin, specific antibodies, etc., where the binding partner is conjugated to a detectable label. The label may be conjugated to one or both of the primers. Alternatively, the pool of nucleotides used in the amplification is labeled, so as to incorporate the label into the amplification product.

The detection methods can be provided as part of a kit. Thus, the invention further provides kits for detecting the presence and/or a level of a polynucleotide that is differentially expressed in a cancer cell (*e.g.*, by detection of an mRNA encoded by the differentially expressed gene of interest), and/or a polypeptide encoded thereby, in a biological sample. Procedures using these kits can be performed by clinical laboratories, experimental laboratories, medical practitioners, or private individuals. The kits of the invention for detecting a polypeptide encoded by a polynucleotide that is differentially expressed in a cancer cell may comprise a moiety that specifically binds the polypeptide, which may be an antibody that binds the polypeptide or fragment thereof. The kits of the invention used for detecting a polynucleotide that is differentially expressed in a prostate cancer cell may comprise a moiety that specifically hybridizes to such a polynucleotide. The kit may optionally provide additional components that are useful in the procedure, including, but not limited to, buffers, developing reagents, labels, reacting surfaces, means for detection, control samples, standards, instructions, and interpretive information. Accordingly, the present invention provides kits for detecting prostate cancer comprising at least one of polynucleotides having the sequence as shown in Tables 1-7 or fragments thereof.

The present invention further relates to methods of detecting/diagnosing a neoplastic or preneoplastic condition in a mammal (for example, a human). "Diagnosis" as used herein generally includes determination of a subject's susceptibility to a disease or disorder, determination as to whether a subject is presently affected by a disease or disorder, prognosis of a subject affected by a disease or disorder (*e.g.*, identification of pre-metastatic or metastatic cancerous states, stages of cancer, or responsiveness of cancer to therapy), and therametrics (*e.g.*, monitoring a subject's condition to provide information as to the effect or efficacy of therapy).

The terms "treatment", "treating", "treat" and the like are used herein to generally refer to obtaining a desired pharmacologic and/or physiologic effect. The effect may be prophylactic in terms of completely or partially preventing a disease or symptom thereof and/or may be therapeutic in terms of a partial or complete stabilization or cure for a disease and/or adverse effect attributable to the disease. "Treatment" as used herein covers any treatment of a disease in a mammal, particularly a human, and includes: (a) preventing the disease or symptom from occurring in a subject which may be predisposed to the disease or symptom but has not yet been diagnosed as having it; (b) inhibiting the disease symptom, i.e., arresting its development; or (c) relieving the disease symptom, i.e., causing regression of the disease or symptom.

An "effective amount" is an amount sufficient to effect beneficial or desired results, including clinical results. An effective amount can be administered in one or more administrations.

A "cell sample" encompasses a variety of sample types obtained from an individual and can be used in a diagnostic or monitoring assay. The definition encompasses blood and other liquid samples of biological origin, solid tissue samples such as a biopsy specimen or tissue cultures or cells derived therefrom, and the progeny thereof. The definition also includes samples that have been manipulated in any way after their procurement, such as by treatment with reagents, solubilization, or enrichment for certain components, such as proteins or polynucleotides. The term "cell sample" encompasses a clinical sample, and also includes cells in culture, cell supernatants, cell lysates, serum, plasma, biological fluid, and tissue samples.

As used herein, the terms "neoplastic cells", "neoplasia", "tumor", "tumor cells", "cancer" and "cancer cells", (used interchangeably) refer to cells which exhibit relatively autonomous growth, so that they exhibit an aberrant growth phenotype characterized by a significant loss of control of cell proliferation (i.e., de-regulated cell division). Neoplastic cells can be malignant or benign.

The terms "individual," "subject," "host," and "patient," are used interchangeably herein and refer to any mammalian subject for whom diagnosis, treatment, or therapy is desired, particularly humans. Other subjects may include cattle, dogs, cats, guinea pigs, rabbits, rats, mice, horses, and so on. Examples of conditions that can be detected/diagnosed in accordance with these methods include cancers. Polynucleotides corresponding to genes that exhibit the appropriate expression pattern can be used to detect cancer in a subject. For a review of markers of cancer, see, *e.g.*, Hanahan et al. Cell 100:57-70 (2000).

One detection/diagnostic method comprises: (a) obtaining from a mammal (e.g., a human) a biological sample, (b) detecting the presence in the sample of a CA protein and (c) comparing the amount of product present with that in a control sample. In accordance with this method, the presence in the sample of elevated levels of a CA gene product indicates that the subject has a neoplastic or preneoplastic condition.

Biological samples suitable for use in this method include biological fluids such as serum, plasma, pleural effusions, urine and cerebro-spinal fluid, CSF, tissue samples (e.g., mammary tumor or prostate tissue slices) can also be used in the method of the invention, including samples derived from biopsies. Cell cultures or cell extracts derived, for example, from tissue biopsies can also be used.

The compound is preferably a binding protein, e.g., an antibody, polyclonal or monoclonal, or antigen binding fragment thereof, which can be labeled with a detectable marker (e.g., fluorophore, chromophore or isotope, etc). Where appropriate, the compound can be attached to a solid support such as a bead, plate, filter, resin, etc. Determination of formation of the complex can be effected by contacting the complex with a further compound (e.g., an antibody) that specifically binds to the first compound (or complex). Like the first compound, the further compound can be attached to a solid support and/or can be labeled with a detectable marker.

The identification of elevated levels of CA protein in accordance with the present invention makes possible the identification of subjects (patients) that are likely to benefit from adjuvant therapy. For example, a biological sample from a post primary therapy subject (e.g., subject having undergone surgery) can be screened for the presence of circulating CA protein, the presence of elevated levels of the protein, determined by studies of normal populations, being indicative of residual tumor tissue. Similarly, tissue from the cut site of a surgically removed tumor can be examined (e.g., by immunofluorescence), the presence of elevated levels of product (relative to the surrounding tissue) being indicative of incomplete removal of the tumor. The ability to identify such subjects makes it possible to tailor therapy to the needs of the particular subject. Subjects undergoing non-surgical therapy, e.g., chemotherapy or radiation therapy, can also be monitored, the presence in samples from such subjects of elevated levels of CA protein being indicative of the need for continued treatment. Staging of the disease (for example, for purposes of optimizing treatment regimens) can also be effected, for example, by biopsy e.g.,. with antibody specific for a CA protein.

## (g) Animal Models and Transgenics

In another preferred embodiment CA genes find use in generating animal models of cancers, particularly lymphomas and carcinomas. As is appreciated by one of ordinary skill in the art, when the CA gene identified is repressed or diminished in CA tissue, gene therapy technology wherein antisense RNA directed to the CA gene will also diminish or repress expression of the gene. An animal generated as such serves as an animal model of CA that finds use in screening bioactive drug candidates. Similarly, gene knockout technology, for example as a result of homologous recombination with an appropriate gene targeting vector, will result in the absence of the CA protein. When desired, tissue-specific expression or knockout of the CA protein may be necessary.

It is also possible that the CA protein is overexpressed in cancer. As such, transgenic animals can be generated that overexpress the CA protein. Depending on the desired expression level, promoters of various strengths can be employed to express the transgene. Also, the number of copies of the integrated transgene can be determined and compared for a determination of the expression level of the transgene. Animals generated by such methods find use as animal models of CA and are additionally useful in screening for bioactive molecules to treat cancer.

## Characterization of CA sequences

The CA nucleic acid sequences of the invention are depicted in Tables 1-7. The sequences in each Table include genomic DNA sequence (mouse genomic sequences mDxx-yyy; human genomic sequences hDxx-yyy), sequence corresponding to the mRNA(s) generated therefrom (mRxx-yyy; hRxx-yyy) and amino acid sequences of the proteins (mPxx-yyy; hPxx-yyy) encoded by the mRNA for both mouse and human genes. N/A indicates a gene that has been identified, but for which there has not been a name ascribed.

## Table 1

```
MOUSE GENOMIC SEQUENCE : mD22-022 (Seq ID No: 1)
CGCCCTCGCTCGCAAACCCAAACACTCCCGGCTGCTGGTGCATGTGATCTCCCAGTAGTCGCTCGGCAGAGATGTTGCTGTTGGCCG
CTGCCGGCCTCGTGGCCTTCGTGCTGCTCCTCTACATGGTGTGTGCCGCTGTCATCAGTCCCAAGCCCCTCGCGCTGCCCGGCGCGCACG
TAGTGGTGAGTGGCCTCCTGTTGCTGAGCTTGCCCAGGACTGCTTGGCCGGCTTGGGCCAACGAGACCACGGTCCCGGAAAGGAACG
GTTGAGTGGGGCGGGGTTACCTACTGCCTTAGCCCCGGCATTGCCCTGCCCTGTCGGACCTCAGGAACCCGCCCTCCAAGGCGCCGGG
CTACAGGGCAGTGTCGTGGAGAAGGCGCCAGCCTAGTTTCCAGGACTCAGGGGCTCTGCTGCTCGCTGATCCCCGGAGCATCCCTGT
CTGCCACTGTTTGGATCTTCAGTTTCCCCAATGAGTCAACAGAGAGACTGAGAAACTTTGCCAAGGACACGGCAGTCGGTGACTAGC
AGGATAAGGGCGCAAACCCGGATGGGAGTAGCCACAGCCAGGGAATCCTAAGGAGCCCACAGCAAGGAGGGGGTCTGGGCCTCTGGG
GCTGGCCCAGGCAGCTGAGACTGTAGGGTGTCGTGTGCAAGTGACAAAGAGCCCCCAGAGGCTTCTAGGAAGTGATACACCTGAGGG
GTGGCCTGGCAGGTTTGGCCAGCAGGCTTGTCTCTGAACTCAGCCAGGGTCTGCTGAGTACTGCTCAAGTGCAGTCCTTTCTGGCGT
CAGAAGGAAGAGCTAACCAGCTTGATTAGAAGCTTAAGATGGAAGGAGATATGAGTGGTTTGGTTTGTTAAAGTCCGGAAGCTCGCA
CGTTCCTATGTGCAGGCTTGGCTATGCCTCCACCCCGCCCCCACAGCTCAGAGCCTCAACTCTGGCATTTCTCCTCGGTGTCCTATA
TAGACCCAAAACCCGAGGCACAGTGAGGCGAGGAGTTGCGAATAAGGACAGCTAGTATCCAAACCGGCAGCACGAATCTCCTTGGTG
CCCTTAGGTGACTTACATGAGTGCATCCAAGTAAGTCCCCACTGCGTTGTTACAGGATAGAGTGGTTATCTTAGTATGATTTATCCC
AAACAAGAATTTCTGGATGACAACATGTGTTTCCATTAATTTGTCTTTTGTAAAATTACTGATTTATTCCCAGCTGCTGCTATGCAT
GATGTGGAAAGAGTTATTAGAAAAAGCCAAAGTTTACAATCATGCCAAGTTGCTAAAGTTTTACATCCACAAAATCGTGACTAAAGC
AGTTGAACTTGCCGGCTGAGGAAATGGCTCAGTTGGTAAAGTGCTTGCCGTGCATCATGCCCTGAGTTAGGGTCCCTAGCACCCACA
TGCTCACTGGGCCTATGCCTCTAACTTCAGCACTGGGGCCACAACCTGTGGATCCCTGGAGCTCACTGGATAGTTTGCCTAGACGCA
CGAATGATCTTCAGGTTCAGGGAGAAGCCTTGTCGCAAAAATTAAGGTGCAGGGTGACTGAAATGCCAGACACTGCCAGCCTCTGCA
CAGGTGCACCTACATACACAACGCTGAATCAACTTGTGCTTGTATAGCTCAGCTGTGGGGCAAGGGAGGAGAGCCAAAGTCAGAATG
AAAACTAATTCGACAACTACAAGGTACTCACAACTACCCTTGGTACTTGTTCTTGTGTCCCCAGTCCCGGCTCTTTCACAGAGGAG
GTTAGGGCTGTTTCAATAGACGCATTCTTTTTTCCCAACATATTTTTATTGATTCTTTGGGAACTTTTACATAATGCACTCCAAAACAC
TCACTTCCCAGCCCTCCCAGGATCCTGCTTTAATACAATAAAAAATAATTACCGCTATGCATGTATGTATGTTTACTGGTATAAACA
ACATAGCATGGCTTATTCATATGGTGTCCCTATCTGCCTTTTTTAATATCTGAAGAGTAAAATGTGATTTAGTTGTGATCTGGGATC
AGGAGAGTGAGCCAAGCTCTTTGTTTTTAGAGCAGTTCACATACGCTGGTGCTGTGGTGTCTGATCACAGCAGCAAAGCTTTGCTGG
TCTTCTCATCCAGTAGATGATTGTTCAGTGTCCCTCCTTCTGATGACTGAGAGTAGTGCTCCTAGCAGCAGGAAAGTTGCTGGTGC
CCTTAGTAGGGCCTGTATGCTGCGCTTAAAGAGACAGTATCGTTTAAATGGTATTTGTAGTGATCAGTAACAAGGAGTGGTCAAGGA
AGGGAAAGTAAATGGTTATCTTCGGGGCCCTCTGTTTAGATATAGTATACATTTAACGTTTTCTCGGCCATTGTGAAAAACCCACGC
CAGTTCTTAGAGCATCCAGAAGAACATATTCCAGAATTAGCCTAACTGTATTCTTATATGCCACTCAAGACTTAGCTGCGTTGGCTA
GATCCCTTTGAATGCACCTTGATCTGAAAACCAGGTCTCACTCCCAATGTGAGAAAGTGACACTGTTTGCTTTCCTGAAACCCGCCC
TGGCTTTTGTTCACATAATACATCAGAGCATAAAAGATGTCTCAGTAACCTCATTCCTAACTCCATCAGCTTTCTGAACAATAAATA
```

```
GGGAGACTTTTATTTGTGAGCTTTGACGTTTTTGTTTTGTTTTGGTTTTTTTGTTTTCATGGAAATGACATAGGAGAACATTCTGCAAA
CTGAGGAAAGTATTTATTGAGTGCCTGCTTGTGCAGGGGCCTGGCATGGTCTGTGATGAGTGAGACAGGCTGGCTTGGTATTGGTCC
ATAGGAGCCATGAGTTCAGCCAGGGAGCCAAGATATATCCAAAGTGAGTGCAGACTACATGGTGACCAGCCAGGAGGACTGACTGAC
TGATGTGTTCAGGAAAGGTTCCCATAGGGCGTGGGGGATGTGTGGGGGGATGTGTGATCCGGGGAAGATAAACAGCTTAAGGCAGCA
TTATAAAGCATGTGAGGAAATGTTTTAGAGGCTCTGACAAGTACTATGAGGAGTAAAAGTTCATAAACCAGAACAGCCCGTAAGATA
GCCTGATATCAGAAGCCACGGAGATAACACTCAGCCAAGGCAACGTGTATCCTTCTATAGTCATCCTCAGCAGACATTTGGTTAATG
ATCTCCCAAGCAAACCTCTCCATTAGATCCAAGGTGAGACTACAGGGAAGTGACATATTCTCAAGAACACAGAGAGACACATCTATG
CTTGGAGGGTTTCATGGCAATACGAACATTACAGTGTGGGAGATGTTCTTTCTGCATGGCTTTAGAGTAGCAAAATATTCAAAGTAA
TCCAAATGTCTGACTGGAAAGGAGCTAGGTTTTGACAGGAGAGATTTTTGTTTTGCTTTTGATACAGGGTCTTACCGTATAGCCATG
CTGGCTTGGAATTTACTGTGTGGGCCAGAATGGCTTGGATTCGGACAGATAGCCTGCCTCTGCCTCCTGAGTTCTAATATTAAGGTG
TATGCCATCACTACTATGCCTGGCCTGGATGGAGCTCTTTAAATACAGTATTGTGAAGCCTTGTAGTTTTCAACGTTGAAAGGATAT
TCTTTCTTTCTCTCTGCTCACGTTTTTACATAGACTTAGACTTACATAGATGTGCTGGAAGGAAATGAGATTCGTTTGTGTTTGTAG
TCAGCAGCACATAATGTATTATTTCTTAGCTAAAATTGTAGGGCACGTTTCCTTTGGTGACCTGAAACGATTTTAGATGAATAGTTT
ATAGCTCAGCACGGGGGGGGGGGGGGGCTAGGAGGCTAGGAGGCTCTGCTGAGAGTGTAATCTTTGATGCTTACACTGCATTCCAG
GTGACAAATGTGGTATGAGGAGGTTGTGGTTCTTGGAGACGCTTTGTTCCTGAGACAGAGCCCCATGTAGTCCTAGCCAATACCATG
AAACTGTCCGTGAGATTGAACGACTTCAACCTTGACCTCCTCATCCCCTGCAGACACGCTCCTGGGGAATTTTTAAAACTCAGAATT
ATGTCATAGTCCTATGCAGTACTGGAGATCCAACCTGGGGCCTCATGAATCCTAGGCAGGTACCTGCACTGAGCTACACCCTTACTA
CTCCCAACACAAGTATTTCAAGAAGTCTCTGTACAGAGGAGATATGGCAAGTGCTAAGATGGTCTAAGATGGTCTTCCTGATGTTCT
TCATAGGTCACAGGAGGCTCCAGTGGCATTGGGAAGTGCATTGCTATTGAGTGCTACAAACAAGGAGCATTTATAACTCTGGTTGCA
CGAAATGAGGTAAGCGCTCTAGACTCCTCATGACAAACTCACTAACGCGGGAAGTCCAAGAGACGGGTCTTATCTGTCATTTCTCTC
TGTAACTTGATGATGACATGATAGGCATCTTTGGCAGAGTCCCCATGCCCCCAGGAGAGTTTCATTCTTTCCAGTATGTGTAGCAAAG
TAGTTTTTTCCTGTTCTAAGCAATCAGGGGTCTGTTTTTCATTGTATTGGGGTCTGCCGTCTTGCCCAGAATTGCTTTCGGAGCATGAA
GCTATGACTGAAGATGCCCCTTGTTCCTCTTCAGGGTCTGGAGCAGAGACCCCGGCTTCATGGTCAAAAGGTTGGCCTTGCTTGCTG
TGGAGCAGATAGATACTCTGTTGAGTGATGAAATGAAAGAAGAGGGGCTTGGGGTGTGCTCAGTGGGCTCAGCACTTACCCAGGGTG
AATGAATCAAATCCACAAAGCACTCCAAGAAGGTGCATGGGAACCCTAGCCTTAGGGAGGTGGACGCAGAAGGATCAGAAGTTCAAA
GTCTCCCCTGCTATACAGCAAAGGAGAGTCTTGCCTGACTTTCACGATGACCCTGTAATTCAGGGACAGGGAAGAAAAAGAAATGAG
ATGTGCTTGAGGGGTTAACTGTCGACATAGAGCGGTTGGTTGAAAAGGGGCTAGTGGAGAAGACAGGCTAGAAAGGATCTCTGGAGA
AAATACAAAACAATAATGATCCAGAACTAGGTTAAAAATAGCCATCATTTCCTTAAAGAACAAGTAAATGGTTTTTAAAAGAAATTAT
ACAGCCAGGCGTGGTGGTGCACGCCTTTAATCCCAGCACTTGGGAGGCAGAGGCAGGTGGATTTCTGAGTTCGAGGCCAGCCTGGTC
TACAAAGTGAGTTCCAGGACAGCCAGGGCTATACAGAGAAACCCTGTCTCAAAAAAACTCAAAAAAAAATTATATATATATTATATATA
TACATATATATATATATATACACACACATACATACACACACACACACACACACATATATATATATTATATTATGGCTTGGTTGG
ACACACTTTAAGGTCACCATACATGAACAGAACACAGATTTAGGAAAAGGCCACTTTTGACTTTTTAGCAGGGCTAGAATTTGACA
GTGAGTGAGTGGGCGGGGACTGGTGTCAGAGCTGGAAGGAACTTGCAGGACCGTGTTTGGGCTGGCGTCACAGCTAGTGGGTAGGAA
CTCATGCACAGCCTGGTTGGAGTGAGTATCCAGAGAGTTTTCTAGCCTGAAGATGTTCTTTAGTTTGTCATGTAACTTTTCATTGCA
GTCTTGAATTCATTTCCCATCATTCGATAGCATTGTACTATTTTGTCATGAGCATTCATGTTTGTAAATTCGATTTGTTCTTTGAAG
ATACCTACTGGCCCACCCCCACCCCTCTATGAGACAAGGTCTCGAGTATCATGGGCTAACCTGGAACTCTGTAGCAGAGGATATCCT
TGCCAGATACCTAGGTTCCTCCAGGCTTGGTTTTAGCCTTTCCAGGGATTGACCTCAGGGTGCCCATGCATGCTGGGACCTTCTTTGT
TCTTCTACAGTTCTCAAATTTTATTTAAATTTATGAAAAATTAAGGTTCTGTAAACTCATCTGTTTTTATGAGAATAAGCAAAACAA
AATGGAGGACATGGGAAAACTTTGCAATTATCTGGGTAGGTTTGTAAGTCTGAGATATATTTTTGAAAGTAACCAGATGTAGATTTT
TCTTTCTCCTCTTTGTAAGAAGTGATTGATTTTTACTCGATGTTGCATTGAAAGGAGTAAACTGCTGATATTATCTAATAAAGCCCT
CCTGGTGTGGAGTCAGCTGCATGCCTTTAGCCTTCCAGAGCATTTGTGGTCGGTCATGTGCTTATGCTGTGTCCCCGCTCAGTGGTG
GGAATGAAAATATCTGTTGGCGTGGCAGGCGTGACCAGCAACAATGCTGCTGAGAGGATTTGAGCTTGCTTTCTTCTTCTTCC
CATTCTCTGCAATGGACTTAAATGAACCCTGCTGTGACTTACCTGGGCTCTTAGGTGTTTTGCCTCTTTCTTCCTCAAGCCCACAGC
TCTGATTTATAGAATGCAGTGTCTGTTCAAATGTTGCTTTTGCTTTTCTTTTTAACAAGTGCAGACAGACATAATTTTTTTCTGTAT
TGCTTTTAGGACAAGCTACTGCAGGCGAAGAAAGACATTGAAAAGCACTCTATTAATGACAAACAGGTAAAGTGGGGCCGAAGGAGG
AATTTTTCTTTCCTGATAGACAGTTGTTTAAAGGGGAGGAAAGATGTTACTTCCACTGCAACTGGTTCACTGAAGGCATTTATGGTTT
TTCCCAATTAAAATACATTTTAGGCCGCTTTCTAAAAGATGGAATTATTTGAAATGACTAGAATTCAGGTCTCTGTACAGAAACCAG
ATGGTATAGACTATCACACATACCTGAAAATAGCCAAGCAGCGCTGTATGAGAAAATGGTTCGTTGCAATTACTCAATTTACACAA
CGACTTAGATAGAAAGGATGTTTGAAAAGATGTTGTATGATTTGGGGTACTAAACTGATACAGACACTGTAATGTATATTTTCATGT
GCAAGTCAGTGACTGGTTGACTACTACAGATCCACCATCTAATCTAAAGAAATGGAAATTAAAGGACTTTCTGAACCCTCTCTCCTG
TTAAGATTTTACCGTCTCTGGTAGTGTGTATAGTCTTTTGTAGTGAGTGTGTGTGCACCTGTGTCACAACGGTCCTGATATGCAT
GCACATACGTGCACATGCTCGAGAAGCCACGGGCCAACATTGGGTATCTTCTGTAATCTCTCCATATTTTTTTTTTTAAATAGGCT
CTTTTTGGTGAGCCTGAAGCTATCCTTGCTGGGGATCTAAACGCAGGGCCTCCTCACGCGTAGCACCTTGCCAGCTAGTCATCTTAGCA
CCTAAATAGTGCGTGTGCGTGTGCGTGTGTGCGTGTGTGTTTGTGTGTTTTCTGTATCCTTTTGAGCTTTGGCAGCATTCCTATGTT
TCTGAACTGCCTTAATGAGACCTCGCACACAGTATGTAGTTATGCTTAAGACACCCACTTCCTGAGTTTTCACAAAGTTGTGCAATC
ATAATCACAATCAATTTGGAAATTGTCCCAACCCTGAAGTCTTCAGGACTGCCATTAGCACGCCACACCCATTCCTGCCTGCCTCTCC
TAGCCCTAGCCAAGTACTAATAACCTTTATAATTTGATAGACTGATGCATTCTTATGCAGTGTCCTGCAACCTTATTTCTTTATT
CTATATTATTTTTAAAATTCATCTGTGTTGCTGAATAAAATAGCTCATGTGGTCTTTTCTTTTATTTATTTATTTATTTATTT
TTCTTTTTGAGATTCTTTTATGGGTGTGCTGTCCAGCTAGACTCAAATCCCAGGACTGTTTTAGAAAGTTTTAATCAAAATCCGGGA
TTTCTACCCTGCCTTTGATCATTCAGTTCCCAGATAAAAGACACACTTTTATTATTTACAACAAGCTTTAAACAACACAAGAGCTGG
GCAGATATCTGCCTTCCCTGCTATAAGAATCTACTTTCCTATTTATAACCCCGAGTTATTACTTACTGTGCTTTATCTGGGCTGCTC
CCAGCTTCAGTTGGCCAGCCTTATAGCTGTTTTCTTGACTCCTAACCCATAGTGTCTTTCTCTTCTCTTTCTCCTTCACTTCTTCTT
CCCATCAGAGGTTCTCCTCCAACCCCCAAGCCTTGGAACCCTAAACTCCACCTGAGTCTCTTCTGCCCAGCTGTTGGCTGTCAGCAT
CTATATTTACCAATCAGAAATAACTTGGGGATAAGGTCACATTAGCATCACTTGGGTCTGAATGTGGACTCCTCTGGGTCAACCAGG
TCTTGAGGAACTAGCAGTATTAGCATTAGAATACAAGCAACATCAGACCAACCCCTACACAGGACTCAAATGATTCTCCTGTCTCAG
CTTGTTAACAGGATTGCTATAGGCATACATCATTTTCCTACATTTACTTAAGTTTATTATTGATATATATCATGTAGTTGTATGTAT
AAAGTACTTTCACTTACCTGTTCTGTCATTGATAGCTATTTAATTTGCTCCCATATTTTTGTGTTTATTGTGGTTGTTGTCATGGC
AGCTTTTCTTTGTGCACATGATCAAAATGATTCTGTTGCTTTGGGACCTTTGACAAGGGAAAGCAAAGTTGCTCAACTCTTAACGGC
TGGGAAATGGGGACGGGGGTTGGGGAACTTTCCTCAGTATCTTCTGTGGCCTTGAATGGCATACCTTAGGGACCTAAATTCTCTTTA
TTATGCCCTGCCTTTTAAAGGTTTCACTGCCTTCCAATAATGCCAATGTACTAGCCCTTTAACATGTTGTCATTGGGAATACAAATT
TTAGTACGAGACATTCCAGATGCGTGCCTGTTTGCTCTGGCACCCATCACCATGTCCCTTGCCACAACCCCATGTAGGCATTCTGCT
TTGTGATATAAGCTGTTTTTTGGCTGCCAGTGAAAGAACCCATTTAAGCTAGATTAGAGTGTATTAAAGGCAGGTTTATTGGGAAGCTG
CATCCAGAGTGGGAGAAGGAGAAGGGGAGAGAACATGCAAAATGTCTAGATTATATAGAGAGGAGCCTCTAGGAGAAGGGCAGCCCA
GCTCCTGGGTTGAAAAATTCAAGGTTGTGGGCAGGGTATGTCAGGTAGGGACTGAGGGATGCTGGGAGAACCTAGAGGCCAGGTCTG
CTGTGCTATGTAAAATATGCACCTTAGTCTCTGTTCCAGGGTCCAGAACCAAACATCTCAGTCTTTTGTTGCTTATAAAAGAATAAG
```

43

```
AGACTCCATAATCATACATGACTGCTTCCTGCTGACACTGGGGTGCAGTTGTGAATGTGAGAAAATGCTGGAATGTGGGGCAAATCC
AGGAAGAGCTGATTGTCTCACTGCCGCCCAGGTTATGGGACCATCTCAGGGTAGGAGTGGGTAGGCCCAGTTGAAACAGTCTGTGAG
GCTGGGGGCAGAGCACCTGTGAGTAACTGTTTTGGAGCTCTCGTGGATGCAGATTCTGAGAGAACACCTGGATGTTGGAGACAGAAGA
TAAAGTTTAGGGAGAAAAACTTCTGGGGGGGGGGTTATACGTTTGAAACTCTTAGGCTCATGGTTGTGGGAGTTGGAGAAGGGGTGG
GGTGGGGGCAGGACCAGGGATAGGGCCGGTGGAGAAGGAAACAGGCTGTTAATTGACAGTACTTATCTGGAGTCCATTTGGCTATGG
AAGGTGGATTTGAGTGGATTCCCTGAAGCTTACAGAGAATCTTTTTAAGTTTACAAAAAGATAAATCCTACAACTTCATTTATCCA
CTTCAATACCCCTTCCTAGACCTTCAAACAGTTTCTTAGAAGCCTTATATATCCTCAGATCTTACGTAAATGTTTCCTATCCAGTTA
TTCACCATGAGACATGGATGGTTGATCACTAGATTTAAACCCCTCAGAAATCTGAGAATGGTGCATCTAAATGTCCTATGTGTCAGT
TAGAATGACAGAGTTACCCATTACCCATTATACCACTAGGTTCCTTTTACGTCGATGGCTTTGTTGAGGACACAGGTCCAGGGCAGG
ATTCATCTTAGGCTGCCAGGCCCAAAAAATCTGGAAGGCCTTCCTGTGGAGGAGAAACTTGGGAAACTGACCTTGCTTTGACTTAGC
CAACATGAGACATGTCTGGTGTCCCTCTTTGTACACAGTTCAGGTCAGACCTCAGCAGTTTAGGCAATGGAGCAGCCTTTCTCAATG
GTTATTGTACAATTCAGGTGGAGTCCTCTTGTGCCTGGATCTTCTTGGAGACCTTGAGGGTGTCACTACTAGGATTGGGGAGTCTCT
ATCATTGTAAGCATTAAGTTTAAGGTTTTAAATGTCATATTCAGCAGGTCTCTGACAGGTTTGAGGCCGGTACCTACTAAGCATATC
TGCGTTAGTCAGTCTATGTCTGTTTCTAGTTATCTGTACACCTAGCAAACATAAAAAAGGAGGCTAAATAAAGCTTAAATAATTAAC
TGCATCATTTCTCAACATGACTTTAGTGATGCCCTTAAGAAAGATGGTGGTGAAGCCATAATTCAACCCTCTTTATTACAAAGTCAA
GATGGTGACCAGTTGTGCTGTTTACCCAATGTGTGTACATTGTTAGTTCACCATCACTGTTGTAATCAGTATTGCTGCTGCTGCTGC
TGCTGCTGCTGCTGCTGCTACAGGAAGGAGCGGGGGAGAGGTGGGGAAGTCTGAGCTAGGTGGCACCTGGAGCCTTGTGCTCAGTGTG
GTGAAGGGAGAGTGGGAGAACACAGCCTCCAAAAAGCTGGACACAGGGAATTCAGACCAATTTGAATAAATTTTTTTTTTTAGATCC
CATGGCACCCATCATCCCAGCGTTCACTGGGAGATCCCAACACTATTGAATTAGCATCCCAATTCATAGTCTGAGATCAAATGGTTT
ATGCAGAAGTGTAGTAACCTCAGATAAGGCATGTCTGTATCAAAGGTTCCCCCAAAACAAAGACCTGGCCTTTGTTCAGATGGGGGG
AACTCTAACGTAGCTGTGACTCAGCAGAGAAAGCTCCGAGGATCCTAGACCCAGATAGGAAATAGCTGGGGATGGGTAGCCACAGAG
TCAGAAGAAATGATGTATTTCAGTGGAGAGGGCTAGGAAGAACAGATTGGTTCCCAGATGGCCAGGAGAAATACCAGGTCCTTCGAACTG
GGGACTCAACTTCTCTCCAGTTCAGCGACATCTGATAGGTTATTTTTCCACCACCGGTGAAATGAGCCAATTTAAGCCAGATTAGAT
TAGATTAAAGGCTGGTTTATTGGGAAGCTGCTCTCAGGTGGGTGAGTTCACTGGCCCGGAGGACCAAGGCCAGGGAAATCGCCATAA
GGACAAGGGAAGAGAGGGACAGGAGGGGAGGGAGAGAGAAAGAGAAGCTGCACACACAGAGAGGGAAGAGAAGAGGGAGCCTCTGGGG
AAGGGCAGCCCAGCTCCTGGGCTAGAAAGTTCAGGGTTTGGGGCAGGGTATGCCAGGTAGGGACTGAGGGGATGCTAGGAGAACCTGG
AAGCCAGATCTGCTTTGGTATGTAAAATTTGTATCTCCACCCCCGATCCTGGGGTCAGAAATGAAACACTTCGTAGGTGTACGTGGC
TAAATTGGCCATTATTAGAAAGACTTGGTTTTACTATTTTTTTTTTAAATACCGTTACAAATAGTTTTGGTAGACCAACATTTTGGC
ATGTGCTTATAATCTAAGTAATCTCAGTATTTGGAAGGCTGAAACCGGAGGAGAGCTGTGAGCCCAAGTTATTCTAGACAGAAGTCC
AAGACCGTGTGTGTAGGTTAAAATAGCCCGAAGATGTCATTCTGTCCTTCCACCATGTGAGTCCCAGGTGATGAGCACCCTTACCCG
CTGAGCTGTCTCTCAGGTCCTGATCCCATTTCTCCTGTTTTCTTCCTTTGAGCAGGTGGTGCTGTGTATCTCAGTTGATGTGTCTCA
AGACTATAACCAAGTGGAGAATGTCATAAAGCAGGTAAGACGGAGGGTGCTTTCAGTTTCACTGCCTGACTCTGCTCAGCTCTCGCC
AGGAGTTGACAGCTCTTCTCTATAGGCTTAGTGGTTCCATGGACTTGATTGACCTTATCTTTCTGAATGATGAATCTTCCATTCTCT
GATGTAGTGTTTGGAATGATATGTAATGTTATGGAATATTCCTCCAGGCACAAGGAAGCTGGGTCCTGTGGACATGCTGGTCAACT
GTGCAGGCACCTCTATGTCAGGAAAGTTTGAAGAGCTTGAAGTTAGTAGTTTTGAGGTGAGTCACAGTGGTATCAAGATGATGTCTT
CTATTTTAAAACAGCCCTGCCAAGCCAAGAACTCAGCTACTAATGTACTTCAACACTGAGGAAATGGGCATTAGGTGTGAACTCTTA
AGTGCAAGGCCTTTTGACCTTGGCAATACGTCATTCTTTGGTGTTCCTCTTTCACCAGGGTGATAGGGACTCTGAAGTTAAAC
TGCTCTGCTATCTATCTTATAGGAGGTGTGACTGCTGGACCAGTGTCAGAACTATCTTCTTCACAGGCTGTAGTCAACCCCATATACT
CAGAGATACACACAAGGCCTACAGGAGGATCAGAGGGTGGTAAAAGGTAGAATGGTAGGACCTGAGTGTCTGAGTTTGTCTGAGACG
CCCTGTCACTGTGCACATCACGCTGGCCCAGCCTTCTACCTCAGCTTCCTGAGCACTGGGATCACACATCTCTGTCACTGTGCCTAG
TGTCACCGTGCCTAGTGTCACCGTGCCTAGTGTCACTACACCTGGCACAATTGTTTGTTTTACAGTGAATAATTCAGAGGCACTTAG
CAAATTGTGATGTTGTGTAACTATCTAATTCTAAAACATTTTCATCTCTCCAAAAGAATACCCTATACTTTAGTCAGTATTTCTCAT
TAACCCAAACCCTTATTCTAGCCAATGGCTACCCCTAATCTGTGTCTGTCTTGCTGAATAATGGAGTCGTGTGCAGCGTGCGTGCCC
TCCTGTGCCTGGCTTTCTCCCATGCTGAACGCCCTAAAGGTTCCTCCTTCACACTGACGTGTACACCGGTGCTTTGTTTTTCTGAGT
TGGTAACAGTCCAATTGTGTGAAGAGACTGCAGTGTGCTTGCTTGCTTATCACCCCTGAATATTTGGACCATTCTTACCCACCCGGA
CCAGCCCAGAAGCTCCAGGTATCATTTGAGCCCTTGGAGGTTGTGATGCACAACCCTGTGTTGTAGTTTCCCTGTCACCAGTCAACA
CTCAGGTTTCCATGTCTTGCCAAGTGGTGGTACAACTTGTAGTGTGTTCTCCAGCTTTAGGCTGTTGTTGCTTTTCTTTTCCCTCTGT
TGTGTTCTCTCGGGGCTCTTAGCTGGATGAAGTGGCTCATGCGCATAATCCCAGCTCTTGGGAAACTGAGGCAGGAGACTAAGGTTG
AGTTTAAGGCCAGCCTGAGCTATGAAGTAAGTTTCAGGCCCTCGTGAGATACAATATGAGACTCTATCTCCAAAACAGAGAGACAAA
TGTACTCCTTACTGGTGTTTTATTGAGTTCTCTCAAAAGAACTGGGGTAAGCATGGAGTTTTTGTTCATTCACAGGTTCTTTTGGGGG
GTTCTTACTACTAAAGCAATGTCATGAGGGGCTGGAGAGATGGCTCAGCGGCTGACAGTGTTTGCTGCTCTTGTAGAGGACTGAGGT
TCAGTTCCCAACACTCTGAGAGGTCACATCTGTAATTCCAGTACCAAAGGATCCAGTGCCAGCCAGGCACACATACATGCACGCA
AGACACTCAAATGCACATAATAAAAATCAAAGTTTTAAAAGGCTAAAACAAGGCAGTGATATTAAAACATTCTAGGGGAAAACAAAAA
CAAAAAACAAGCAAGAATCCTGGCTTCCAAACACAATCAATATTTTCAGTTCTCTTTGCTTTTACAAAATAGAGTGGTAAGAATTAC
TATCTGCACATATCCTGTCTTCTACTTTTTGAAAACTCATAGCATAAATTTTTTCCCTCACTCTTGTGTTTTCAGTTCTCTTTCCTT
AAAGTTTCTTAGTATTCTGCCACACTGCTATCATGGTTGTGTATGCTCTGACTGGGCTGTGTAGCTTCTCAGGGTAAGGAGAATAGC
CTTCCGGTATATAAACCCGTCACTGTGTGGCCTTCCGGTGTATAAAACCCATCACTGTGTGCCCTTCCGGTGTATAAACCCATCACTG
TGTGCCCTTCCGGTGTATAAACCCGTCAGTCACTGTGCATCCTCAGGTTCTCAGGGTGAACGTGGGGTTCTGGGATGAGAAGCTGGG
ACTGTCTCTGCGGGCACTTTCCAGCAAATACCGAGTTCTTACTGTGCAGGGCAGCAGGATCTGTGCTTGTACTTAGCTTGTGTTTTC
CCGTGTAATCCCTGAAGCATCCTGAGGTCATGCTGAGATTAACCCCATTTTCACCTGAGGAAATGCCCAGCTGAGAAAGTCTCACA
AGATCAGGGGTGGGTAGTGGCATGAAGGCACAGCTCTACTGTTAACTGGTCCCACGTTCTCTGTATAGTCCTTGTCTAGCTTAGAAC
TAACAGGGCCAAAGAAATATTTTGAAGCTTTCTCCTAAAACTGTCCTTCAAGAATTATTACCTATAAGAACTTAAAAAGATAGCTTA
GGGATTAAGAGTCCTGGCCACTCTGGCAGAGGACCCAGGTTTAGTCACCAGCAACTTACAGCTGTCTTTAATTCCAGTTCAGAAGAT
CCAGCGCCCCATTCTGGCCTCTGGGCACTGCACACACATGGTACACAGAAATATTTTCAGGCAAAACCCTATACACATAAAATATCG
TTATAATGCCATTACCTACAGTAAGTACTTTTCTAGTGAAACACTGATTTTGACCATTTGTGTGACCATTTGTGCATGGTTGGAGCAG
AGGTTAATGTTGGCTGTTCGTTCTCGTTCGTTTTCTCTCTCTCTCTCTCTCTCTCTCTCTCTCTCTCTCTGTCACTATTTTGG
TTACACTGGCTGAGACTGTCCAGTAAGCTGCTGGGATCTGCCTGTTTCTGCCTTCTTGCTCCCGGTGCCTGGGTTAGAGGTGTGTTC
CCTTATGCCCCGCAGAGTTTTTGATGGGGATCCAAACTCAAGTCCATATGCTTGCCACACCAAAGGACTTTCCTAACTCAACCTTGTGC
CTAGCCCTAATTTTGACATTTATATCAAAAATGGAACTGCTGTCACTATGAGAATTGTCTTCAAACTAGTCTCCAGAGGCTAGGTCC
TGAGTGTCATAGTATCTACTACGTTTGTTCACTTCCCATACAATATTCAGCAAATCTGCTGCACCCTGGCTCCCAGTCCCCTGGGT
TTTTTTTTTTTTAATTTAGTTGTTATTCTATTGCTGTGAAGAGACACTGACCAATGCAACTCTTATAAAGGAAAGCATTTAATTGGA
GCATGCTTACAGGTTCAGAGGCTCAGTCCATTATGATCGTGGTGGGAAGCAGACAGGCATAATGCTAGAGCAGTAGCTGAGAGCTTT
ATATCCTGGTCACGAGAGAGAGAAGAGGGAGGGGAGGAAGGAGGGAGGGGAGGATGGGATGGGATGAGAAGACGAGAGAAT
GAGAACACGAACACAGACAGACACTGGGTCTACTGTGGGACTTTTGAAACCTCAGCATACCCCCCAGAAACACACCTTCTCCAACAAGG
CCCCCCTACCCTAATCCTTCCAAACTATTAAACCAAATAGGAACCATGCCTACAAATATATATGCAAACTACCATAGGGTCTCTCTTA
AATAAGTTAGTCCTGGGAAGAGACAAACTCCCCTTAGCAGGGCTTAGGCTTTTGTCCTTGTTCGTCCCACCTCATCTGGCCTCTTTG
```

EP 2 204 376 A2

```
TTTCTTGCAGAAATTAATGAGCATAAATTACCTGGGCAGCGTGTACCCCAGCAGGGCAGTAATCACTACCATGAAGGAGCGACGGGT
GGGCAGGATCGTGTTTGTGTCCTCTCAGGCAGGACAGCTGGACTGTTTGGTTTCACGGCCTACTCTTCATCCAAGTTTGCCATAAG
AGGATTGGCAGAAGCTCTGCAGATGGAGGTAAAAAATACATTTATTTCTGTATTTCTTCTTCTTAGATCAGCAGGGAGCTCTGTCAT
TTTTTAGCTCCTTAGCCTACATCACTTTGTAAGCTAAAGGCTTACAGAGGTGATATCTGTAAAAACAAAACAAAACTAACTCAGTTG
TAAGATCTGTGTTTTAATACTTCTTATTGTATAAAATTATACTTAGGACAGCGGCAAAATGACTCTGAGGGTGGAGGGATTGTCTAC
TGTATAAAGTACTTGAAATCCCTTCCTGGTGTTGGTCTGCTTCTGCTTAAGTGAACAGGGATATCATTATAGACAACTTTCTGAAAG
CTCTGGATGTGGTTCTGGGGAAAATGCTTGTTTTACAGGTTGGGTAACAGAGTTTGACTCTCCAGAACTCACATACATGTTGGGCCA
GTGTAGCAGTCTGCCTGAAATTCTAGTGCTCAGAAGGTGAAGATGGGAGCCCAAAGCCGCGTGGCTAACGAGCTAGACCAGTCCTCT
CAGTATGAGTCAAATTTGCGAGGCTTACGTCAAAGAATAAGGTAGAGGATCATTAAGACAAACTCAATATTAGGCTTAGGCCTCTGC
ACACACATGCTCAGTTAAGCTGTGAAGTTGCAAGAGAGGATCCTGGTGATTCTGTGCAAGGATGCTTTGCAAATAACCATGTTCGTG
TGTGCATAGGTCTGTATGTGTGAAAATATGTGCACAAACTTCTTCATACCACTTTCTGGAAAGGGGAATTCAGCTTTTAGTGAATTCA
GTGAGTTACTTAGAGTCACATAGTCACTTGGTGACAGGAATAACAAGGCCTAGCCCCGTAACTGCAAGCTCCACTGGTCTTTTGCAG
CAGAGGATGTATCTGTAATTTGGAATGTGCATGTGATGTGCATCTGGTAGCTGCTTTATCTGGTTTGGGATTTTGGGCTTAGAATCC
ATTCGGGTGCAGAGTTTGAGAGATAAAAATGTATGCCCATTAAGGCCTTTCCTGAAGTCTGACAGTATGATCCACAAATTAAGGGCA
AAGTGTATCTCTGGGAGCCCTGGTTTTCATTGCAGAGGGAACAGTGACCCAGTGGCTGCATCAGAGTCGCTCAGCTTCTTGATGTCA
AGAGTGATCTTTCACACTGCCCAAAGCTGACTGGGCTAAGCTGCCTTGACTTCTGAGCATTAAAATTGTTGTTACTTAAATAAACAA
ACACACATACCATCTAAAAGACTGCTATTTTATTTGGATTAAGTAAATTCAGACTTTTGAATCCAACTTGTTTGTAGCAAAATTTGC
CTCAAACCAAGATTTTCTTATCAACTAATAATTGTTTATTGAAATCTAGATAAATTTGGTCAGTTTTTTATGAGTTAAACCAACATAG
TTGCTTAAATGCTTGATGCTTTGGGTAAACATTTTGGTTTAATTAACTTTTTAAGTTTTTCTAGTTACTATCCTGCTTCCTCCGACA
AGGTATAAACTCTGAAAATGTGGGCACAGTGTGTACTTGAATGCTTTGTTGATATTTTGGATTTGATTTTCTTTTTTCTTCTTTGGT
CTTTTAAATACAAAATTAATATTTAAAGAATTTTTTTCAAAAAGTTGATCTCTTCGAAAGTGTATTTCTGAAGTCAGTTATAGCCA
GAGATTCAAGAATCAACCATAACAAAACAGGAATCCCCAGCCAGGATTCCTCCTGTTTTCTCACCCAGACCATGCCTGGCCCCTCCC
CTCATCTCTTCCCACCATGGTAGACCAGTAAGCTGGAGCTGGGATTCTTTAGTTTGATATAAGCAGCTGAGGTTCCTTCTCCAGCCC
CAGTGTTCTTGTTTTAATAGAACAGCTCTTAAGTGGCACTGTTTTTTTATGATAAACTCATCCTTCGCAAGCCATCCATTGTCTTAC
CTGTGCGAGGCTGGTGCCAAGTAGAAGGGAGAGTAAGAGGACAGGTGAAGAAAGGCAGAGCAGGAGAGTAGAAAACGGCCGTTTCCC
AGGGCCAGGGAGAAACAGGTCAGCGAGGCATGAGTCAGTGAGATGAGTGTAACACTCGTCGTAACCCTCGTCGTAACCTAGAACAGG
CGAATGTAAGCTAAGCCGTCCCTTTTTGGTTGGGTTTATAGTGTTAACAGTTAGAGAGGTGTAAGAATAATTTTGTATGTCCTGGTTT
TTGCAAATTATGTGAACAAGAGTATCAGGAGACCAGGAACTGGGTTATGGACAGAGAGTTGTCCGTTTAGTTCATTCCTGTACGAA
GTCTATGTTGGCATACAGTAGTAATTTAGAAAGTGTTTACTGGAAGCATCCACCCAGATGCTAGCAGAATAATTGTGCTTGATGATT
GCAAAAACAGGGTTGGCTCCAAAGGTGATGGACTGGTTCCTCTCCACCTTGCTGTTTCCAGTCCCTCCCTGTGAGAGAGCGGTTGCT
AACCCAGTCATGACCTTGAGTTTGAGAGACTTTGAAACATGGTTTTGATGATCATAAACGGCACAGCCCCCACTGTGGGGCAGCGCA
GTGGGGAGGCAGTTCAGAGTGGGCCAGAGAAGCCAGCCTGTGTGACTTCTGTGGCTAGTTAACACTCAGGCACTTTTTTTTTTAAAGCCA
TTTTTTCTCTGGTCCAATACAGGACCAGACAAGACTACAAGAGACATTTTATCAATTTTCACCATCTCAAAAGCAAGTGTGAGAGAAG
TCACTTGATCTTTGTTCCAGCTGGCTCACCTGATTGGCCTGGGAGACAGCAAGCACTCCCTGGCAAGGCCAAGCCATGAACCCTCAT
GACTTGGATGCCTGGAATGGAACCAGCTTTCATTCTTGTGATTACTTGGATGGGTGCACAGTACCTACCTACTGCTTTGTTCTCTTG
ACCCATGTTGCTGTCCCGAGCCTTCATAGCTGTGTCACTAAAAGAGTCTGGGAGCTAAGGGGCTGGCGCCCTGGTAGACGGGTGGGG
CTGAGAAAACCTGTCGGATTTCTTCTTTAAGATTCTCAACACTGTTGTGCCGTCCCTCCCAAACACCCAGATGTCTTTATGAG
GACAGAGACACAAGCCAGCAGTACATAAAAAAGCATGCCAGAGTGGACTTGGTGGTCTGAGCCTTTGGATCGCTGATGTCCAGGCAA
GAAAGGAACAGGCGACAGCGACCGGACTGCTGTTGGACAGTTTTGGGAGACTGACCCTTGAAAGAAGGGCCAGTGGGGTTGTGGGG
TGTCTTCATCAGTAGAGGAGTTGACTGGGCAGAGAAAGTGGGAGGCAGACAGTGTTGGAAGAACTAGAGGTTATAATAGGCCTAGTA
CCTATAAAGAAAGCCAGGGAGAACTGGGCATAGTGGTGCACACTTGTATCCCAGGTCTTCGGGAGACCATGGCAGACAGATCACCAG
GTCAGACTAGCCTGGGCTAGGAGAGATCTTACTGATAAAAAGGGCAGAGGGAAGAGCTTCTCGTATAAATAGTGGTAGGGGCTGTTT
ACAGGGGAGTTGCTTGGCTGGTCATACTTTGGTGGTCTACCCAGGATGAAGATGCTTTCTTCCTGATGCTATGTGGGTGGTTATGCA
GGGACTTCTACAGGGATGGATAGAGTTTCCAAATCTGGATTCAGACACAGTAATGTGCATCCGGGTAGGCACTGAGAGACGGTGTTT
TGCAATTGAAATGAACATTGCTGTATTATAGCAGCCTGAAAGGCATAGACCTGCCTTCTGCCTTGAGCTGTGCAGTTCGTGGGCAGA
GTCTGTGATGAAGGAGCATACTGTCATTCCACAGCATATGCAGTTCTCCCTGGGCAGCTCTGAGCCTCTGCTTGGTCAGCCGCAGGA
AGAACTCTACTAGGAGGGGGCTTGACACACTTTAGAGCCAGGCTATTTCTGACATAGGTCAGGGGTACTCTTGCTACTGTTTCTAAG
TCATCTTCATGGAGAGGGGTCCCCAGGACATTTGGTGCCTGTTTACTGATAGCCACTTCATGAACCTGACAAGCAACACCTTCTAAG
GCAGTGCTCTCAGCCTTCCTAGTGCTGTGACCCATTAATACACCTTGTGTTGTGGTGACCCCCAACCATAAGGTGGTTAGTTGCTAT
TTCATAACTGTAATGTTGCTACTATTATGAATCAGTGTAAATATCTGATATATAACACCCCCCCCCAAAAGGGCTGTGGCCCACAGG
TTGGAAGCACTGTTCTAAGAATCAAGATAGCTGAGACAAGTTACAGGATGACTACAAAGTGCTCTGTTCACTGAGAGTGCGTACCTT
GTTTGTGTGACCAGGTGAAGCCGTACAATGTGTACGTCACTGTGCCTACCCACCAGACACCGACACGCCGGGGGTGGCTGAGGAAA
ACAAAACGAAGGTCAGTATTCCTACATAATGCCCTCTGTGAGGAGTGTGTGATGGCGTGTGTGTGTGTGTGTGTGTGTGTGTGTGTG
TGTGCTGTGTGAAGCGTTTTGTGTGTGTAATATGTGTTGTGTGTGTGAAATACATGTGTGTGATATGCATGTTGCGTGTGAAATGTG
TGTATGATCTTCTGTGTGTGCTGTGGGGTGTGATGCATATATTTACACAGATTCATAATAGTAGCAACATTACAGTTATGAAATAAC
AACTAACAACCTTATGGGTGGGGGTCACCTCAACACGATGTGTATTGTCTGTGTTATGTATTGTATGTGTGTGATGGATGATGTGTT
TGATACAGTGTTAAGTGTTCTGAGTGAGGTGTGTATGTGATGTGTGTGCACGTGTCAGGTGGTGTGTATATAGTTTGTA
TTTGTGACTGGAGGAAGTTGTGCATGTTTGGGTGTGTGTGTGTAGTGTGATGTGTATATGTGATATATGTGGGGTGTTATACATATTT
GGGTTTGTGTGGTATGTATGTACATGTTTGGGTGTATATGTGCGTGTGCATGTGTGTGTGTGTGTTTGGGAGTGTGCAGGTGTGTAT
GGAGACCAGAGAACACCCTTGGGTATTCTTTTTCAGGTGGTGTCCTTGGTCTTGAGACAGGGTCTCCTACTGTCCTGGAACTATCAA
GTAGGCCCGGGAGTTCTCTGGTCTCTACCTCCTCCTGGCTGGCTTTGCATGTGCGGGTGCTCAGAGGCCAGACAGGCACTTTATCTC
CCTAGCTCTGTTCTGAATCTTCAGCGTAATTTTTATGAATGTATCCTACTTTAGGTCCTGGCTGTTTATCAGTTAGTGGGTCTGTTA
GGTGCAGGGTGCTGAGCCAAGACTTGACCTTCCAGGCAGAGCTTCCTCCTCCCCTTTTCTGCTCTCCAGTCCCCTTTTCTTAATGTT
GAACTTACTTGTTTCCAACTTTTAGCTCAGTATTTTCTCATAGGACGCAGTTGGCTGCCTAATGCTACCTGTGGCCTTATCACTCCC
ACTTCTGACCCCTGACCCCTAGAGGGAGCCATTTCACCTATGTGTCTAAACCAATCCATACATACATACCTACATACATACAGTG
AATGCACCTACATGCATGCATAACATGCATACATATGCACATACATTCATATGCATCTACGTACAAACTCGTGTTTTAACTAGGG
AAGCTAAAATTTGAGCCTCAGGTCTACTTTGTTCTGCCTGTGTGACCTCTGTTGGAGTCAGATTCTGTGTTTATGAGTGTTGATAAA
GTTGAACCTCTATAATGGCATTTCCTTTCCTGTGTGTTGTTTAACTGAGAGCATTTGTAGGTCATTCACTGGGGAGAACTGGAC
GTCTTTTGAGCATCTGAACCACTGACCTTTCTGTTACTTTCATTCTTCTTCCTCCAAGCCCCTTGTCATGGGGCTCCACCTTCTGCC
AGGACACAGGCCCTGGGGGTGACATCCTCTGCACTCCCCTGTGCTGCGCTTCCCTTCTCTGTCCCTTGCTGGGGTCTGTGCCTTCCA
CTGCTGTCATTTCTCTGCTCACCTTGGTGGCCCTTCAGTCATTCCAGAGAAAGGCACGTGAGAGGCACCATTTGTTTAGAGTCTGGT
TCACAAGAAGGCATCTTCCTTTTCCTATTTTTCGTTGCTGTGTGTGGTAGGGTGTTTTACTGGGTGACAGTTGAAAATGACGGTCCT
CACGGCTTTGCGGGCATTTCTTACTGAGTTCTAGCTTCTAGGGGTTCCTGGGGACCCCACTGACCTGAGATGCCTGAGAAAAGCAAC
TCGTGGCTTGTGGTTCAGACGACACCCGTCCATCAGGACAGGGAGAACTGGCCCGGCAGTCAGTGCTCCTTACAGGAAGGAGAAAAA
GGGGGGACAGCAGAGAGCCAGCTGAGCCATAGCCTCAGCAACACATTCCTCCAGCTGGGCTTCAGCCCCTACCTTTCCCCACCTTCC
ACTAATGCTCAGGGGCCAAGCAAGCATCCGTGACTGAGCAGCTTCCTCATACACATGGGAGGTGTGTACTCCTGGTGGGGTTTAGTT
```

```
GTAGACACTCTTTTCCTTTTTATCTTTTAGAATGCCGTTTGGTGTGAAGTGTTTTATATGCTAGATACTCAGGACCGCCCCTTTGTT
CTCTCATGCACTCAGGTCTGGAACATTGAGGTCATTGTTTCCCATCCAGAACACTTGGAAGCATTCTTTGTGCAGTTGTCGCTCAGG
AGCCCCACTTTCCGGCTGACTTCACCACCTCATCTCTTACTCACTATTGGTTTCTTCTTCAACTTTATTTAATTTAACTTCAATTTT
TATTTCTACTGATTTTTTTAAGCTTAGATCTATCTGTCATGGATTTGTCTTTAGTTACTGTTCTGTTGTTGTGAAGAGAAACCAAGA
CCAATATAACATAAAAGAGAGCATCTACTTGGCTCTGTCTTACAGTTTCATAGGGCTAACCATTATCATCATGGCAGGGATCATGGT
GGCTCTCAGACAGATGTGGTGCTGAAGAAGGAAATCAGAGCTAAATCCTGATCCACAGGCCACAGGCAGGTAGACAGACAGACAGAC
ACACACACACACTGCACCTGGTGTGGACTTTTGAAGCCTCCACCCCGTTCCCACTGAGTTTTCTTCTATTTCTAAGGAGCTTCAGTC
AGAATGTTGCTTGGTTGTATTTTTGTTCTGTTTCTTTTCTTGGTTTTGAGTCTAGGTGTTCTCGACTCTGCTCATGTTGGTGATTGC
ATTCAGCAAGGCAGTGGAGCACAGGTTGTAGAGCCATGTAAAGCCCTTCCGCCACCTTCTGCTTTACATATGGAGGATGGGGTGGCA
GTGGCGCCTTCGTTTCCTTAGCGTCTCGTGTGTGGACAGATGGCATGGGACTGGTCATTTCCGCCCCAGTGGCATCTATCTCCCTTG
CATGTGTGCAGGTCATACAGAATGACTGGGTAGTCCTGAGCTGAGAGTAGGCTCTATTCATCTGGTTCCCCATCTTTCCCTCTGCCA
TGACACAAACAAACAAAACAGATAAACAGGTAAAGTGGGGCTGAAGGAAACCCACGAAAGCTTACCACCTCACACTGAAGGTAACCT
AGTTTTAGACTTAATTATTTTTATGTGGTTTTTCCTCTTAAAGTGTATGTGGATCTCTGTAGCATAAATTTGGGGTGTTTATGTAGTC
TATATTCGGTTTTTGTTTGCCAAAAAAAAAATGGGTTATTTATGTAGAAACAACCTCTTCATTTTCTTTTGAGTTCAGTCAATTTTAT
AATTTCTTTTTGCTTTAAAAACATAACTTGGAACCAAACCTCCTGCTTCAAACCTGCTAAGCAAGCATTAATATTTGCTGCATACTC
TTTAAGTCTGAAGCCAGTGAAGTAGTGACTTCCCTGACGTTGTTTTGTTTTTGTTTAGCCTACAGTAAAATTTTAGACAGAT
TTCTAGTGACTGGAACCTGCATTGATCTGTGGGCCAATAGGGGGGCAGCAGACACTCTTCTAACTCAGTGCATCTTGCCTGTCCTTA
TCCACCTGGTGTGGAGGGAGAGCAAATCGGTGAGATGTGTGTCCTCTCAGATTCTAAAAAAAAGAAAACCTGACTTTTGTGAAACAT
GAGCTATGTTTCTATAATCACAGTATTCAGGAGGTCGCAAGCCTGCTTGGGCTACATAGTCAGACCCTGACTCACAAAACCAGACAT
GGAGGAAAAAGTGTATTCTAATCTTTCCCCTCGAGCCTACATAGTCACATTTAGTCTCCCGTGGCTTAAGGCTTCAGTTCGAAACTT
TTCTTTTGAAGAATGTTTGGATGTGTGTGTAAAATGCCACTCAGAAAAGCCAATGGGAACAGACAAAAACGTTGCCGCTCACAAGTC
AATTAGGACAGAAAGTGTGAATACAGAAGTAGCTAGAACGTGAATTAATGCAGAAATGAAGGAGGAGAAGGAGAGAGAAGGAGGG
GGGAGGAGAGAGAGAAACAGACACAAGAAGCCCGGAATGAGGAAAGGTGTTCTGAGCTAAGTGCGAGCCGGACAGACCTTTATCTGT
CTCTTGAGGAAGAGAAGCCTAGGATTTGGGAATTGGGCCCTGAGTCCTGTAACTACCTCTCAGCCAAACTAGGCAGGAGGCAGTGTT
GCTACTCAAATGGCTAGTGTCTTGGCCTCCTCTGCTCGTCCTTCAGCAAATAGATACAGTTCTTCAATGGGGGCTAGGCCCAGACTG
TCCTCAATCTGTCCCCAGCATCATTCATCTGCTCAATGGGGACTCTATTCAGAATGGAGGCACGGTCCTACGATGGATCACGTTTGC
TCAGTCACAGGGACGAGCCTCTATGCACCATACTGTCTGGCAGTTGTCTGTCCAGTAAGAGAAGGGCCCTTCCTCACTGTGACTGAC
AGACTGATCTACTAGCTCCGGTTCTGGAAGGCCTCACTAGAGCCGTGCTTGCACCACCCAGAACAGCAAGTACCTCCTTAGCCCTGC
CAATGGAGATAAAATCCCACACGCAGAGACTGACTTAGAATCTAGCTTGATCTTTGTCTTGAACAAACGTATTTGTTCGAAGGTAGA
AAAATGATAATTATTTGTGATCCATCTATAAGATGTTTATTTTAATTCAGTGGAATTATTTTAATTTGTATATTTCTCCCACTTGCA
GCCCCTGGAGCCCGGCTTATCTCAGAGACCACAGCTATTTGCAAACCAGAGCAGGTGGCCAAACAAATTGTCAAAGATGCCATAGT
AAGTAAACGCCCTTGTGTGGCTCTCTGTGTATTTATACTGTGTTCCTGAAAGGCACATAAGGACTGACTTCAGACGCAGAG
CGTTCGGCTTTGCGTGGGGTTTGTCTGACGTCAGCCTCCACAACCTCCTCCCCACTTCCTGTGGATTCAGCATTAACCAGAGGGATAG
CGTCACGGTATTGTAAGAGAGCTGAAGCTCTCGGAACGCAAATGACCACTACGTGAGGAGAGGCAACTTCTTTTAATTTTTATTTGT
AATTTTACTTATTGGGTGGGTGTGAATGTTGCCTTCTTACAAGAAAATTTCTCTCATCTACCACGAGAGTCCAGGGCATTGAACTCG
GGTCATTAGGCTTGGTGTCTGTGCTTACCCACTGAGCCTGGCCTTGTCCTTTACCAGGGTTTCAGACCATCTAAGGTATTTGATTGC
CAGGCTCAGGATGGGTTTTTCTTTCCCTTCCTGTTTCCTGTTTACTACTTTTCTTTCCTCATGGTTCACAACAAAGCTGCTGGCCCCAC
CGTTCCTCTCGATGTACCTCCTCACACACTTAATTTGTGGGGAACTGGCTTTTGAAACCATATCAAGCATTATATAGAACACTGCAT
ACCACAGTTCTTTTGAATACATTCTGATTAACTCACTTGTAGAACAAGGTTCAATCTGAGGGATGTCCGTAAACTTAGTAACCTACG
TGACTTTGGAAAGTTTGCACCCAGATAACATGGTGCAGCTTCTAAGTAGCAAAAGGGGTGTTGTGCCAAGGTTAGCAGGACACAGTA
ACAGCAGCCTCACCAACAGACCCAAAGACCTGGTGAGACAGCTCAGTGGGTAAAGGGACTTGCCACCAATCCTAATAACCCGAGTTC
TTCAGACTCCAGTAGTGGAAGGAGAGACCTGACGCCCTCATTTGTCCTTCATGGTGGTCACTACATTGTGTGATACATACATATAC
ACAAGTATGTTGCGCGCGCTCAACTGGCCAGGAAGAACGACGCTGCTACAGGATCCTTCTGCACACGTTTATTCAGTCCTGTTTCTT
CTTTCTCTATATTTCCCTTGTTTATATCTCCCTTGTTTATATCTCTCCCTTGTTTTTATCTCCCTTGTTTTTATATCTCCCCGAAC
CCTGGGCCTCTCACTCTTTTATACTCTCAGTTCCCATCCACGCACAGCAGGCCACGCCACCTCACCAGGCACGCAGCTTCAGCTAAT
CAGGGCAGCAGGGGCAAATCTCCACCAAATTGGATTCACCTGTATCCTGGTACACCTGCGCAGCACTCAAGATGTTTGTGGCTTATA
TGAGGAAGTCAGGTGCAAGTCATATGACTTAGCTGCAGTCTCTGGGACTGCCGCCACACCCGCTCCCCACAATTCCCC
CTTTTTTTCTTTTTTGGCAGAGAGAATGTCTGTAGATAGCCCCCCGCAGCCATGTCCCTTACCCGTCCTGGGTGACAAACAGCATTG
GTTTGATCCCTGTCTTAGGTTGGTGACATGCCCAGGGAGTCTTATCACTGACTACCTCTCTATCATGCCAAGCCACTCCTGGGGAGA
TTGTGTTTTGCTTGCAGCAGGTGCATCAAAAGGCCAGGATGTTAATTAACCTATGGCCAGATCTTAATTGCTGCAAGCAAGCCTCAT
GGGTGAAGGTAGAGGTCTGATCCCCAATGTGCAAGCATAGGGGCCCTACACAAAACCATCCCTTAGGCTCATCACCCAGAGAGGTCT
TGGCCAGCTCCCGTGTCGTTTTTCCTGGGGGAAGGGAACTAGGACACTGAACCTTCATGGATCAGACATACCTTCCACAGGATGCCA
ACAGCAAGCTATCCTCTGTGCAGTGCTTAGCCTCGCACCCCACGGCATAACGCAGCATAATTTCTTTTAGAGTGGCAAACCAAATCT
GAGGAGATTGTCCCGCCTCCACTGCAGCAAAAGCCTACGCTACCATGGCGAAAGTGCGGGCCGCACACTCTGCACCTAACACATGTG
CCAAACACAAAACACACACACTATAACAAGCATACATCCCAGGGCTCTCATCCCCGCTCATCTTCCCTGAAGCAAGGGATAGATA
GCCAGAGGGGCTATCTCTTTAAGGGAAATTCAGGGATCAAAAAGCGTGGAAAATTTTGAATGTCATGTCGAGCTGGTATCGGCTTCT
GGAATACCGAAAGGATCTCCCATCTCGGGTCCATCCTTTGTGCTTGTGGGATCATCCACCACATCCACAGGGGCAACTGGATCAGGA
GCCTCATTCTTGCAGCGTCTCACCAATCTCTCCAGCACCCAAAGAGGTTCTGTCTGGTCCTGTGGAAACACACAGACCCTCTCGCCC
ATGTCAACACCTGATCCTGTCGATCCTCTCCAAATCCCGGACAGAAGGTCACCTACAGGTGGCTGCTGAGGAGGCATAGGGAGGC
GGCACAAAAGCTAATTCGCCTTCCTCCTCCACCTCGGCTCTTTTATTTCGTCCTTCCTGACCACCTGATAACACTGTGTCTCCTTTC
TTTTTCCTTTTTCTTTTTAAGCCCTTCTCCTCTTCCGACATAATTTCTTGTTGCTCTTTAAGGATTTTCTGACCTGTCTTGACTGCC
TCTACACACAGTTCAACAGTAACAGATCCTCATATATCAGAACAAGCAAGACCCAAAACTATCAGACCTACCCACAAAGGGTCAATG
GGAGAAAAAAGCATAACTACTCAGCGAGGATCTATTGATCACTACACTGAGGTTATCATAGTTCCTTAACTTGTCCCCAAACCGGGA
ACCTTAACTTGTCCCCAAAGCCGTGAACCTTTAGTTACCTTGTACCTGCTTCCTGGCAACGTTATATTCGCCTCTATTTTATCGGAGG
TCCTTCCCAAACTCCTGGGTTACTTTGTGCCTACTTCCTGGCAACTTTATGCTCACCTCTATTTTATCCGAGGTCCTTCCCAAACTC
CTGGGTTACTTTGTGCCTACTTCCTGGCAACTTTATGCTCACTTCTATTTTATCCGAGGTCCTTCCCAAACTCCTGGGGTCGCAAGT
TTCACTCACCGTGAACTTACCCTGCGGGCAACCACTGACTAGCTGAAAGTTCTGAACTCGGTGGGGGAGTCGGTTCCCCATACGGGCC
ACCAATTGTCGCGCCCCACTCTCAACCAGCAAGAACGACACGACCACCAGTTCCTTCTAACAGCAGTTTATTCCGTCCTCATCTTTCTT
CTTTCTCTTCATCAGTACCGTTCCCCAGCTGAAGAGTTCTGAATCCACGCCGGATCCTTCTCAACAGTCTGTTTTACGGGAACTTTT
ATTAACCACTCCTTCCCCGTGATGCAGTTCTGAATCCTCCCTGTAGCAGGGGGTCTTCACTCATGCCTGAAGATGTTTCTTGTCCCG
GGTTTCGGCACCAACTTGTTGCGCGCGCTCAACTGGCCAGGAAGAACGACGCTGCTACAGGATCCTTCTGTACACGTTATTCAGTC
CTGTTTCTTCTTTCTCTATATTTCCCTTGTTTATATCTCCCTTGTTTATATCTCTCCCTTGTTTATATCTCTCCCTTGTTTTTATAT
CTCCCTTGTTTATATCTCCCTTGTTTTTATATCTCCCCGAACCCTGGGCCTCTCACTCTTTTATACTCTCAGTTCCCATCCACGCACA
CAGCAGGCCACGCCACCTCACCAGGCACGCAGCTTCAGCTAATCAGGGCAGCAGGGGCAAATCTCCACCAAATTGGATTCACCTGTA
TCCTGGTACACCTGCGCAGCACTCAAGATGTTTGTGGCTTATATGAGGAAGTCAGGTGCAAGTCATATGACTTAGCTGCAGTCTCTG
GCGCCTTTGGGACTGCCGCCACACCCGCTCCCCACACAAGTAAATAAATATACATTTAAAAAAAAAGAACAGGTCTGCAGAGGTGGC
```

46

EP 2 204 376 A2

```
TTGGTAAGTTCTACAAGCACAGGACCCCACTTCAGAAGCCACATGAGCCTCCACTCCCACCCCTTTCTGGCCTTCTTGGACACGGTA
CCATTACATACATGGTGCACATGCATACACGCAGGCACCCACACATACATATGTTTTAATTTTAAATCTTAAAACAACAACAAAAGG
ACTGAGTTCCTAGCAGTAACATAAAAGCCAGTAAGCCAGTATGGTTGTTCCTGTCTGTAACCCAAGTCCTGTGTGGACAGACCGGCA
GATCCCCAGATCTTGTGAGCCAGCCAACCCATCCAGTCTGTGAGCCTAGCCAGGGTCATTTTGAGACCCGTTCACAAAAGATAAGGT
GTAGAGTGATTGAGGTAGATACCCAACATTGACCTGTGATTCCCCACACATGTGCCCACAGACATGTAGTTTGCCCTGGTTGCCTTT
AGAGACTTGGAGCAGTGGCACAGAGGCATGAAAGGCTAATTTGCTTTTCCCTCAAGTCAGAACAAGCACCCTCAAGGTGCTGCTGCT
GCTGCTGCTGCTGCTGCTGCTGCTGCTGCTGCTGCATTGTCTTAAGATGCTGGTACTTGCCATGCGTGTGCATGGTGTTGGGTTCCATCACCA
GCAGAAGAAGATGGGGAAGAGATTTAGCTAGGAGATAAAGTCATTTGAAATCAAAGAAATTGAGACTGGGGAATTGCCACAGTGATT
AAAAGCACTTGTTCTTGTAAAAGGCTCTAGATTCATTTCCCACCACCACATGGAGGCTCAGCAGTTCCTCAGGTGTCATGTTGTACC
CAGACATGCATGCCATCAACACAACTCACACACACACACACACACACACAGAGAGGAGAGAGAGAGAGAGAATAAAACTTAACTATATT
ATTAACTCCATTTTTAAATAGCTTTATTTGTTCTTTTAATGTATCTTTGTGCCTGAGTGTATGTATGTATGTGCAATGCCTGCAGAG
GCCAGGAGAACACCACATCCCTTGGACTGGATGTATAGGCATTGTCCCCTCTCTTTATGGTTTCTTTTTAAGTGTGTGTGTGTGTGT
GTAACACTCAGAAGACAGATTTGAGGAGTCATTATTTCCTTCACTGGGGTTCTGGGATCAGACTCAAGTGGCAAGAGCTCTCGTCCG
CGGAGTCCTGCCTTTTCTCAGTTTCTCTTCATATGGGGCTCCAGTAGCTTCAGCCTCAATAGCAGCTGAGGGTGACCTTGAACTACT
GACTTCCCACTTCTCAGTGCTGATATAACTACTATGTGTGGCTCAAACATAGCTATTTTCAAACAGAAATTCATCAGGAGGAGTGGC
ATGGTATTTTCACGAGCCTTTACAAAGTCCACACTAGTGGATCCTGCTTTCTCGTGTCTGCTTCTGTGGCCAATCTGTTAAAT
ACATTCATTTTGAAACATTTGAAGAAAATGTGACTTTAATCAGATACATAGTTGGGAGGGGATAAAGTTTTCACGGGATTTTCAGAT
AATTATGAACATAATGTTCTTTCTGTTTTTTATTTTTATATTAACCCCCATCTTGACAATTAGGGGTTCTTAAAGGTTGGTTGCTGT
ATGAAATTAGAAACCACACACCAGTGAGCTTTTTGTTCTCGGTGACACTAAAATGCATGTCTCGTTTCTATATTGAATGGATCTTTC
ACTCATTCATCATTTGAAAACATGCATTAATCATTTGGAAAATATGGAATCTGTGAGTTATCAAAAGTCACATTCATCAGAGATCAG
GGAAATATCAGGAAGCCAACTGTCTCCTACTGATGGATAAAGTTTTCATAAGTCTTAACTTTTCCCCATGCACATATATAATCTGTG
GTCTTCTTCCTAGCCACAAGTTCAGTTCATTTGTCTGCCCTATCTGTGCCTCAAGCCCAAATTGGAATGTTCTAGCCTCCTGGTAAA
TAGTTGTTAACAGGAAAATAGTTTTGTCAGGAAGCTCAGCTCTGAGGTGTTCCCAGTTAAGCTAAATCACTCGTGGTGAATTTCTTA
TTTGTCACCTGAGAACAGGAGTCCTCTTCCCAGTGTCCTCCAGGAGATGGGCCACAACCAGCCAGATGTCAGATGCTGCTGGTTCCT
GGGTATGATGCTATGTGTACTCTCCTGTGTATATACCTGTGAAATAGCCTAGCTGGGGATGGAGCTCATTGGTAGAGTAATAGTCTA
GTATGTATGAAGCCCTGGTTCCTCCCTAGTACCATATAAACTGAGAACACATGCCTATAATCCCAGCACTTGAGGAGTAGATAAGAG
AGGATTGAAATTCAGGGTCATCCTCAGTTACAAAGTGAGCTCAAGGGCAGCCTGAAATCCTGCCTCAAAAACAAACCCCAAACCTGT
TTAATGAATGGATTAGGCCCCTGGTTCTATTGAGGAGCAAAGAAGTGATACATATCCCACGTGGGGCAGAGCTGGGCAGCCCAAGAT
GCTGGGGTATCATGCAGACAGCATGCAGTTGACCCCTTCTGAACCACTTCTTAAATACAAAGCGTCTTTTGGGCTCTTAAAGACTTT
TTTAGAGTGTGTGTGTGTGTGTGTGTGTGTGTGTGTTTTCATTGCTGTGGTGGTGGTGGCACTGGCTGCATTAGGGCCACACTCTCAGA
AACTCTCCTTTGTGCTGGTGGTGCTTTTGTCCATATGCACAAAGGTCAGTGGGAAAGCAGCTAACGTCTCAGTGTTATTTGACGGTTT
CATTGTCCCTAGAGACCCTGAAAGGGTCAGGAACCCACAGGGATCTGTGGACTTGTACTTTCAGAGCTGGACATACTTTACGTCTCT
GTGTCCCTTTTTAGTTCTGGTATATTTTGAGTCGCTGTGCTGTCCCTTATAAAGTGAAGGGACATACTTCCCTGTTCCTCAGTGCTG
AACTAATTCCTACAAACTTACTCCATACCACTCTTTTCTGCTTGCTTCTTTCCTAGCAAGGAAATTTTAACAGTTCTATTGGCTCAG
ATGGGTACATGCTGTCCTCCCTGACCTGTGGGATGGCCCCGGTGACTTCCATCACTGAAGGACTCCAGCAGGTAAGCCCTTTCTTAG
CCAGCAACCAGGTAGACATACAGACAGGTAGACTAACAAAACAAGAATCACTGTTACCAACTTCTGTGAGAAAAAGATGAAGACTAG
AGTGCAGTAGTTTTAAATTAAAGTTTGTTTCTCTCTCTGCCAACAGTAAGTAGGCAAGCAGGTTAAGGATATGTCCTGGGCAGAAGT
GAGGAACCAGGCTGCAAGTGGAGGAGGGGCCGTCGCTGTTGTTGGGTGCTCACCCATCTTTTCAGCAAAGAATCCTTGCTCTAAAGT
GTTCAAAAGGAACACTTGGCCAAGAATTGTGGGGGGCAGCATTGTCCACAACCAGTGAAGTATTAGCTTAGAAACCATTTTCTAGC
TCATCAAACCTTCCAGTAGCTAAGCTGAAGGAGACTCAGAGAGCAGACCAGACACACCTTGCCTGTAAGAGCTTCATCCAGATTGCC
AGCACTGTCATAAAAAGCCAGTGTGGTCTCTCCTATCTGTAACCCAAGCCCTGTGTGGCAGATCCCCAGAGTTTGTGAGCCAGCAAC
CCATCCAGTCTGTGAGCCTAACAAAGATAGGTGCATGAGTTCTTTCCCCCATGTTTGTCTGTACACCACATGTGTGTAGTGCCCATA
AAGGGCAGAAGAAAACATTGGATTCCTGGAACTGGTGTTACAGAGAATAAGCTGCCAACTAGGTGCTAGGAATCGAATTCAAGCCCT
CTGGAAGAATGACGTAAGCTCTTGACCTCTGAACCATCTCTCAGGCCTGAAAGTTTACTCCTGTTGATAAGGTTCTGTGTTCCCCAG
ATGGTTAGGTGTAAGAGGGTTCACTAACCCTTAAGGAGTCTACTACCATGGAGTGCTAAGACTGGTGGGCAGTGCTAATGGCAGCCA
TGTCATTAGAGCTGTAATGGAAGGAACCTTTAGGGACAAAGCCAAGCATCATGGCTGAGAAAGCCCTGGTCTGTGTGAGGTGCATTC
AGATAACTCCTAGTTCTTTCTTTGGGCCTTTTCCTAGCCTGTCTCATTCTGAAATGTCTCCCCATCATGATTTGCTTGACCTTGTTAC
AGGTAAGATCTTTGGGGACAGGGCCTAAGTCACTCTTACTTGTCACTGGACCCCAGCAGCCAGTACCTACATAACACAGGAGTTTTA
GATTAGTGCACAGAGTCTGCACCTGTCCACCTGTCTTGCCCGTTCACACAGTGATGCCAAATCTAAAACTCAGTCTAGGCTGGGTGC
TAGGCTGGATGCTTTTACCCAAGCCAGCATCGGGCTGTGATCCCCGACCACCCTGCTTCAGCTTCTTTCAGGCTCTGGTCCCACTCA
TCACCATGCTCTTCAGATCTCTCTCTCCCGAATTCTGCATCTCTGCTGATGATCCCAGAGTTGAGACCAACATCACAGTCCTTTACC
AGATCCACATATGTTGTGAGCTAGAACATGAAAAGTGACGTTTGCTCATCTGATAGAAGCCACTCTCCTTACTACTGACTCTCCTC
TGCCACCCTCCCTAGCACTGAGATATGGGTATGAGCCAAACTCCAAGAGTATGTACATTTAAATAAAGTTGTTGCAAAATCAGATGT
TGCAAAAGAAAGAAATCCAAATTTGAGACATTTATTGGGACATTCAGAGGTCTACCTCAGCAGGCTGCTTTCTGACAGGAAAAGAT
GGCAGTCACTCCCATAGAATAGAGCATAACCCCTTCCCAATCCCTGTGACCTAATCCCCCTATGCCAGCCACTGGCACAGATGCTGA
GAGACTTTCTTTGCTGTGTTTGTGAAATACAACTAACTCTACCTTACATAAATCCATCACTGGCTCCTTGGGGCTCAGGATGAAACT
TGAAATCCAGCCACAGCCACCAGCCACCAGCCTGCCCACTTCTTTTTCCCTCTCCCTCGGCTCTCCTCATCTCCTCATGTATCTGG
AAATGTGTGTCTTTCCATGATGCCATTTGGAGTTGCTTTAATGAGAGACTGTCACTGGGAAGGGCACATGGCTATGGATAATTGCTG
CCTTGCCTCCTAGACTGGTTGGTCGCTTTCTTCTTTGATTATCTTTCCACTACAAGCCAATCGTATGCCCACCCGTAGTCGCAGCTC
CGTAGAAGGCTGAGGCCAGAGGATCACTGGTGACTAGGAGCCAAGACCAGTCTGAGCAACATAGTGAGAATTTGCCTCAATAAATAA
ACAGATCAGTTCAGGTAGGCGAGGAATGTATATTTCTCACGTCACGATCCTGAGCACCCAGCAAAAGGGCTTAATACTCAGAACAAA
AATTGTAGCCAAGGACACCTTGATTGTGAGCATAAGTATCTGGAATGCGGAATTGTACCGTGCCTAGGAGTTATAACCCCGTCTTCTA
AGAATGCCTAGTTTGTTCTTTTTATGGTTGGAATTTGTTAATTCAGACAAATATTGTTGTCTGTGTGGCCGTGCTATCCACTGTACA
AAGCAGACGTATTCTGTTCTTGAGAATTACTGGCATTTACAAAGTTTTCATTATACCTGTGCTTGGAGCAGATCAGATGTGTCCTGG
AAACTGTTTAGAAGAGACTTAACTACTATAAAATGGGCTGTTGTCCTGTCCTGTTGGATGAGCTAACAGGTCTCCCTTTCTCTTGCA
GGTGGTCACCATGGGCCTTTTCCGAACAATTGCCTTGTTTTACCTTGGAAGTTTCGATAATATAGTTCGCCGCTGCATGGTGCAGAA
AGCAAAACCTGAAGTTGTAGACAAAAACTGCCTAAACCTTGCCCCTTGGATGAAAGACTGAATCCAGTGATTTGAACAGTGTGCTGCT
AATGGAACACAAGTTTTGGCCTCCAGACTTTTGTATCTTGTTTTTGAATGTGTGAAGATTGGACCCCGTGCTCTTCAGAAATCTGGCT
GTAAGCAGAGGGACATGAGGCCATCTCTACAACTGTTAAACACTATGCAAATATGGGCAGGACACCTTTGATTTTCTGGGCTGTAG
GGGTGATAGTGTGAGAACTAATAACAGGAAAGCAGGGTAAAGAATAGCATTCCAGAACAGTAAATTCAGCTTTTCGGTCATTCTCCC
ATCCTACCCATAGAGATCAAGAAATGTCCTCTGTGGCGTTTCTGAGGTTTTGTTTTGTTGTTTTTGTTTTTGTTTGTTTTGCTT
TTTTTTTTTTTTTTGATTTTGGGTTTTAACTTTTTATGAAAGATGGTCCGGATTTTTATTAGTGCTTTTTTTCTTTTTTGTAATTTT
TTTAGTGTATGTTATTCAAGGTGTCTTTCCGAGTAGCCCGTGAGTCTGACTCTCAGCCATGCCTTGCTGCGCCTGGGACCTCGCTTCT
GCTAGTGAAGCTGGTTTCTCTCTCTTTGATCCCATAAAATTCGAGGGGGGATGAGAGAGGCAGCACAGAGGGCAAGGGGTGAGTCCTTT
GTGACGGCAAGCGGGGCTTTCTTGTCTTCTTAGACTGATGCTTACAACGTTTTCATTTTTATTCAAGGGGAAAGGCAGCCTCTTTAC
GTGTTTCGTGAAGAGAAATAAAATCTCCTAGCAGCTTAAGTTACAGTTTCTTCAGGAGCCATGATGACCTGAAGTTCACATTCCATT
```

47

```
TCAGCTCAGTTCCTAGTGCTTATCGCTCTTCCTAGTTTTGCTTATGCTACTGTAATATTTTTGTAGAAGAAAGGAAGGAAGAAAAAA
AAGATGGAGATCAGTGCAAATGTTTTTGACTTTTTTAATTAATCCATGAATTAATTAAAATAAAAAAATGAAAAGCATGACCTTCTT
ACTCTCAGAGCATACAATGGAGCTTCAGGGAAGGCAGCTGGTTGTGTACTTCTACTATTAAAAGAGGGTCAGGTTTGGAGGTTGGGT
GTTGAGCGGCTCTGTCTCGCGCACTGGCACCCAGCAGTATCTTCAGCATGGTAAGGAGCCCTCTGATGGCAGCCTGTGCGCGTGCAC
ATGCGCAAGACTGCGGTTCCATGGGCGAGCCTGCAACAAGTGCCTCAGATGAGTCCTGCCTGTATCCCACTCATCCTGTTTATTGTT
CCCCAGGCTCTAGGACAGTGTTCTATGTCCTGATATTTTACCCTGTTCTCCTTGTATACAAAATACAGCATTTCAGTTCACTCCACC
AGGAGAATACTACTTTGAAAGAAAAAACTTTATTGTTAAAAAACAGAAACAGCCGGGCATGGTGACGCACGCCTTTAATCCCAGCA
CTCAGGAGGCAGAGGCAGGCGGACTTTTGAGTTCGAGGCCAGCCTGGTCTTCAAAGTGAGTTCCAGGACAGCCAGGGCTACACAGAG
AAACCCTGTATAGGAAAAAAACCAAAACC


MOUSE mRNA SEQUENCE : mR22-022.1 (Seq ID No: 2)
TCGCTCGCAAACCCAAACACTCCCGGCTGCTGGTGCATGTGATCTCCCAGTAGTCGCTCGGCAGAGATGTTGCTGTTGGCCGCTGCC
GGCCTCGTGGCCTTCGTGCTGCTCCTCTACATGGTGTCGCCGCTCATCAGTCCCAAGCCCCTCGCGCTGCCCGGCGCGCACGTAGTG
GACAAGCTACTGCAGGCGAAGAAAGACATTGAAAAGCACTCTATTAATGACAAACAGGTGGTGCTGTGTATCTCAGTTGATGTGTCT
CAAGACTATAACCAAGTGGAGAATGTCATAAAGCAGGCACAAGAGAAGCTGGGTCCTGTGGACATGCTGGTCAACTGTGCAGGCACC
TCTATGTCAGGAAAGTTTGAAGAGCTTGAAGTTAGTAGTTTTGAGAAATTAATGAGCATAAATTACCTGGGCAGCGTGTACCCCAGC
AGGGCAGTAATCACTACCATGAAGGAGCGACGGGTGGGCAGGATCGTGTTTGTGTCCTCTCAGGCAGGACAGCTGGGACTGTTTGGT
TTCACGGCCTACTCTTCATCCAAGTTTGCCATAAGAGGATTGGCAGAAGCTCTGCAGATGGAGGTGAAGCCGTACAATGTGTACGTC
ACTGTGGCCTACCCACCAGACACCGACACGCCGGGGCTGGCTGAGGAAAACAAAACGAAGCCCCTGGAGACCCGGCTTATCTCAGAG
ACCACAGCTATTTGCAAACCAGAGCAGGTGGCCAAACAAATTGTCAAAGATGCCATACAAGGAAATTTTAACAGTTCTATTGGCTCA
GATGGGTACATGCTGTCCTCCCTGACCTGTGGGATGGCCCCGGTGACTTCCATCACTGAAGGACTCCAGCAGGTGGTCACCATGGGC
CTTTTTCCGAACAATTGCCTTGTTTTACCTTGGAAGTTTCGATAATATAGTTCGCCGCTGCATGGTGCAGAAAGCAAAACCTGAAGTT
GTAGACAAAACTGCCTAAACCTTGCCCCTTGGATGAAAGACTGAATCCAGTGATTTGAACAGTGTGCTGCTAATGGAACACAAGTTT
TGGCCTCCAGACTTTTGTATCTTGTTTTTGAATGTGTGAGATTGGACCCCGTGCTCTTCAGAAATCTGGCTGTAAGCAGAGGGACAT
GAGGCCATCTCTACAACTGTTAAACACTATGCAAATATGGGCCAGGACACCTTTGATTTTCTGGGCTGTAGGGGTGATAGTGTGAGA
ACTAATAACAGGAAAGCAGGGTAAAGAATAGCATTCCAGAACAGTAAATTCAGCTTTTCGGTCATTCTCCCATCCTACCCATAGAGA
TCAAGAAATGTCCTCTGTGGCGTTTCTGAGGTTTTGTTTTGTTTGTTTTTTGTTTTTGTTTTGTTTTGCTTTTTTTTTTTTTTTTG
ATTTTGGGTTTTAACTTTTTATGAAAGATGGTCCGGATTTTTATTAGTCTTTTTTTTCTTTTTTGTAATTTTTTTAGTGTATGTTATT
CAAGGTGTGTCTTCCGAGTAGCCCGTGAGTCTGACTCTCAGCATGCCTTGCTGCGCCTGGGACTCGCTTCTGCTAGTGAAGCTGGTT
TCTCTCTCTTTGATCCCATAAAATTCGAGGGGGATGAGAGAGCAGCACAGAGGGCAAGGGGTGAGTCCTTTGTGACGGCAAGCGGGG
CTTTCTTGTCTTCTTAGACTGATGCTTACAACGTTTTCATTTTTATTCAAGGGGAAAGGCAGCCTCTTTACGTGTTTCGTGAAGAGA
AATAAAATCTCCTAGCAGCTTAAGTTACAGTTTCTTCAGGAGCCATGATGACCTGAAGTTCACATTCCATTTCAGCTCAGTTCCTAG
TGCTTATCGCTCTTCCTAGTTTTGCTTATGCTACTGTAATATTTTTGTAGAAGAAAGGAAGGAAGAAAAAAAAGATGGAGATCAGTG
CAAATGTTTTTGACTTTTTTAATTAATCCATGAATTAATTAAAAT


MOUSE PROTEIN SEQUENCE : mP22-022.1 (Seq ID No: 3)
MLLLAAAGLVAFVLLLYMVSPLISPKPLALPGAHVVDKLLQAKKDIEKHSINDKQVVLCISVDVSQDYNQVENVIKQAQEKLGPVDM
LVNCAGTSMSGKFEELEVSSFEKLMSINYLGSVYPSRAVITTMKERRVGRIVFVSSQAGQLGLFGFTAYSSSKFAIRGLAEALQMEV
KPYNVYVTVAYPPDTDTPGLAEENKTKPLETRLISETTAICKPEQVAKQIVKDAIQGNFNSSIGSDGYMLSSLTCGMAPVTSITEGL
QQVVTMGLFRTIALFYLGSFDNIVRRCMVQKAKPEVVDKTA*


MOUSE PANTHER CLASSIFICATIONS
        FAMILY (SUBFAMILY)
                SHORT-CHAIN DEHYDROGENASE-RELATED(FOLLICULAR VARIANT TRANSLOCATION
PROTEIN 1)
        BIOLOGICAL PROCESS
                Biological process unclassified(2.99.00.00.00)
        MOLECULAR FUNCTIONS
                Oxidoreductase(1.19.00.00.00) > Reductase(1.19.06.00.00)
                Oxidoreductase(1.19.00.00.00) > Dehydrogenase(1.19.05.00.00)


MOUSE GENE ONTOLOGY
        No Gene Ontology
MOUSE PROTEIN DOMAINS (INTERPRO SIGNATURES)
        IPR002198 (SDRFAMILY)
        IPR002198 (adh short)


MOUSE mRNA SEQUENCE : mR22-022.2 (Seq ID No: 4)
CGCCCTCGCTCGCAAACCCAAACACTCCCGGCTGCTGGTGCATGTGATCTCCCAGTAGTCGCTCGGCAGAGATGTTGCTGTTGGCCG
CTGCCGGCCTCGTGGCCTTCGTGCTGCTCCTCTACATGGTGTCGCCGCTCATCAGTCCCAAGCCCCTCGCGCTGCCCGGCGCGCACG
TAGTGGTCACAGGAGGCTCCAGTGGCATTGGGAAGTGCATTGCTATTGAGTGCTACAAACAAGGAGCATTTATAACTCTGGTTGCAC
GAAATGAGGACAAGCTACTGCAGGCGAAGAAAGACATTGAAAAGCACTCTATTAATGACAAACAGGTGGTGCTGTGTATCTCAGTTG
ATGTGTCTCAAGACTATAACCAAGTGGAGAATGTCATAAAGCAGGCACAAGAGAAGCTGGGTCCTGTGGACATGCTGGTCAACTGTG
CAGGCACCTCTATGTCAGGAAAGTTTGAAGAGCTTGAAGTTAGTAGTTTTGAGAAATTAATGAGCATAAATTACCTGGGCAGCGTGT
ACCCCAGCAGGGCAGTAATCACTACCATGAAGGAGCGACGGGTGGGCAGGATCGTGTTTGTGTCCTCTCAGGCAGGACAGCTGGGAC
TGTTTGGTTTCACGGCCTACTCTTCATCCAAGTTTGCCATAAGAGGATTGGCAGAAGCTCTGCAGATGGAGGTGAAGCCGTACAATG
TGTACGTCACTGTGGCCTACCCACCAGACACCGACACGCCGGGGCTGGCTGAGGAAAACAAAACGAAGCCCCTGGAGACCCGGCTTA
TCTCAGAGACCACAGCTATTTGCAAACCAGAGCAGGTGGCCAAACAAATTGTCAAAGATGCCATACAAGGAAATTTTAACAGTTCTA
TTGGCTCAGATGGGTACATGCTGTCCTCCCTGACCTGTGGGATGGCCCCGGTGACTTCCATCACTGAAGGACTCCAGCAGGTGGTCA
CCATGGGCCTTTTTCCGAACAATTGCCTTGTTTTACCTTGGAAGTTTCGATAATATAGTTCGCCGCTGCATGGTGCAGAAAGCAAAAC
CTGAAGTTGTAGACAAAACTGCCTAAACCTTGCCCCTTGGATGAAAGACTGAATCCAGTGATTTGAACAGTGTGCTGCTAATGGAAC
ACAAGTTTTGGCCTCCAGACTTTTGTATCTTGTTTTTGAATGTGTGAGATTGGACCCCGTGCTCTTCAGAAATCTGGCTGTAAGCAG
AGGGACATGAGGCCATCTCTACAACTGTTAAACACTATGCAAATATGGGCCAGGACACCTTTGATTTTCTGGGCTGTAGGGGTGATA
GTGTGAGAACTAATAACAGGAAAGCAGGGTAAAGAATAGCATTCCAGAACAGTAAATTCAGCTTTTCGGTCATTCTCCCATCCTACC
CATAGAGATCAAGAAATGTCCTCTGTGGCGTTTCTGAGGTTTTGTTTTGTTTGTTTTTTGTTTTTGTTTTGTTTTGCTTTTTTTTTT
TTTTTTTGATTTTGGGTTTTAACTTTTTATGAAAGATGGTCCGGATTTTTATTAGTCTTTTTTTTCTTTTTTTGTAATTTTTTTTAGTGT
```

48

```
ATGTTATTCAAGGTGTGTCTTCCGAGTAGCCCGTGAGTCTGACTCTCAGCATGCCTTGCTGCGCCTGGGACTCGCTTCTGCTAGTGA
AGCTGGTTTCTCTCTCTTTGATCCCATAAAATTCGAGGGGGATGAGAGAGCAGCACAGAGGGCAAGGGGTGAGTCCTTTGTGACGGC
AAGCGGGGCTTTCTTGTCTTCTTAGACTGATGCTTACAACGTTTTCATTTTTATTCAAGGGGAAAGGCAGCCTCTTTACGTGTTTCG
TGAAGAGAAATAAAATCTCCTAGCAGCTTAAGTTACAGTTTCTTCAGGAGCCATGATGACCTGAAGTTCACATTCCATTTCAGCTCA
GTTCCTAGTGCTTATCGCTCTTCCTAGTTTTGCTTATGCTACTGTAATATTTTTGTAGAAGAAAGGAAGGAAGAAAAAAAAGATGGA
GATCAGTGCAAATGTTTTTGACTTTTTTAATTAATCCATGAATTAATTAAAATAAAAAAATGAAAAGCATGACCTTCTTACTCTCAG
AGCATACAATGGAGCTTCAGGGAAGGCAGCTGGTTGTGTACTTCTACTATTAAAAGAGGGTCAGGTTTGGAGGTTGGGTGTTGAGCG
GCTCTGTCTCGCGCACTGGCACCCAGCAGTATCTTCAGCATGGTAAGGAGCCCTCTGATGGCAGCCTGTGCGCGTGCACATGCGCAA
GACTGCGGTTCCATGGGCGAGCCTGCAACAAGTGCCTCAGATGAGTCCTGCCCTGTATCCCCACTCATCCTGTTTATTGTTCCCCAGGC
TCTAGGACAGTGTTCTATGTCCTGATATTTTACCCTGTTCTCCTTGTATACAAAATACAGCATTTCAGTTCACTCCACCAGGAGAAT
ACTACTTTGAAAGAAAAAACTTTATTGTTAAAAAACAGAAACAGCCGGGCATGGTGACGCACGCCTTTAATCCCAGCACTCAGGAG
GCAGAGGCAGGCGGACTTTTGAGTTCGAGGCCAGCCTGGTCTTCAAAGTGAGTTCCAGGACAGCCAGGGCTACACAGAGAAACCCTG
TATAGGAAAAAAACCAAAACC
```

MOUSE PROTEIN SEQUENCE : mP22-022.2 (Seq ID No: 5)

```
MLLLAAAGLVAFVLLLYMVSPLISPKPLALPGAHVVVTGGSSGIGKCIAIECYKQGAFITLVARNEDKLLQAKKDIEKHSINDKQVV
LCISVDVSQDYNQVENVIKQAQEKLGPVDMLVNCAGTSMSGKFEELEVSSFEKLMSINYLGSVYPSRAVITTMKERRVGRIVFVSSQ
AGQLGLFGFTAYSSSKFAIRGLAEALQMEVKPYNVYVTVAYPPDTDTPGLAEENKTKPLETRLISETTAICKPEQVAKQIVKDAIQG
NFNSSIGSDGYMLSSLTCGMAPVTSITEGLQQVVTMGLFRTIALFYLGSFDNIVRRCMVQKAKPEVVDKTA*
```

MOUSE PANTHER CLASSIFICATIONS
        FAMILY (SUBFAMILY)
                SHORT-CHAIN DEHYDROGENASE-RELATED(FOLLICULAR VARIANT TRANSLOCATION
PROTEIN 1)
        BIOLOGICAL PROCESS
                Biological process unclassified(2.99.00.00.00)
        MOLECULAR FUNCTIONS
                Oxidoreductase(1.19.00.00.00) > Reductase(1.19.06.00.00)
                Oxidoreductase(1.19.00.00.00) > Dehydrogenase(1.19.05.00.00)

MOUSE GENE ONTOLOGY
                No Gene Ontology
MOUSE PROTEIN DOMAINS (INTERPRO SIGNATURES)
                IPR002198 (SDRFAMILY)
                IPR002347 (GDHRDH)
                IPR002198 (adh short)

HUMAN GENOMIC SEQUENCE : hD22-022 (Seq ID No: 6)

```
GGCTCCTCCTCTTCCCCTCCGCCGCGGCCCGCCCCGGGCCCGCAAACCCAAACACTCCAGGCGCCCGCCCGCCGCGCGTGATTCTCGC
CTCGCCGCAGCCCAGCCCTGCGCGCCTTGCCCGGCGGCCCCGCCCGCCGGCCGCTCCGGGCCCCTGGCCCCGCGGAGCGATGCTGCTGC
TGGCTGCCGCCTTCCTCGTGGCCTTCGTGCTGCTGCTGTACATGGTGTCTCCGCTCATCAGCCCCAAGCCCCTCGCCCTGCCCGGGG
CGCATGTGGTGGTGAGTGGCCTCCTGTTGCTGCCCCCCGACTTACCGGCCCGCGCGGCCTTTCCGCCGCCGAGGCCGCGTAGCCTAC
GTCCCTTTTTCCAGGCCGCGGTCCCCAGGAAGGCATGGCCAAATGGGGAAGGTCACCTGCCGCCGTCCACCCCGGCGCTTTTCCCCC
GGCCCCGGCACCCGGCCTCGCGGACCCGCCCTCCGCAGCCTCGGGCTCCTCCTAGGGCCGGAGAGCGTGCGACGGGGGGACCCCGCCG
GCCGCAGGGTTCCCGGGGCCCCAGGGGTTCTGTGGCTGCTCGTGAATCCATGCAACATCCCTGGGATGTGGATGTTTTGTCCTCAGT
TTTTCCGATGAGTCAACTGAGGCACAGAGAGGCTGAGAAACTTTGCCAAGGACACAGCGGTCGGTGGTGTGCAGGGTGCGGCGCGCG
GCCCCGGGCGGGGGCAGACAGCGGGGAAGTGGGGTCCCAGCCGCCGGGACCCGCCGAGGCAGCTCGGAACTGGAAGATGGCCTGTGC
GAGTGACGGGAAGAGCCGCTGCCAGAGGATTCCAGGAAGTGACTCACCTGGGGAAACTGCTCAGAACCCAGCGGGCTGGCGGTGGTG
GCTTAGCGTATTGGGACGCCGTGTCCCGTCTACACTGGTGTCTCTGAGCTCAGCGTGGCATCTGCTGATTGCTGCTCCAATGCAAT
CCTTTGCTGTTTTCTAGTGGGAAGGATGAGCTAAACGACAAGGCTTGTGTGCGAGGAATGAAGGAGAGGTGGATAGGTGTGATTTAT
TTCTTGGCGGAAGCTCGCATATTCAGCGATCCCTGTTCTGCAAATGTGGCACCTTTCTTAAACAGAGAGAATGCCTCCTTGCCTGCG
CCCAGCACTTTCCCGTTTGCAGTGGCTTTCTCACGAGCATTCTCGTTTGGATCTCCCAAACCCGAGGCACAGTGAGGCGAGGAGTTA
GGAGTAAGGAGGGGTTGGTTACAAAATTGGCCGCACACCTCTTCCTGTCCTATCTCTGCACCGGAGATCCGAGCCTCCCCGCTGAGCT
GGTATAAAAATAAGATGGTCGTTTTTCTTAGTATGCTTTAAATCTGTACAAGGGTTTGTGAATGAGAAAATGTATCTGTTTAATTTCT
CCTTTGTAAAGCTATTAGCTTTTTCCAACACCTCCTGTTCCTAATGGGGAAAGAGTGTGGATTCATTACAAAAAATAAATAAGAGAA
AGGCAAAACTTGACACTCTAAATTCTGAAAGTTTTACATCCGCAAGAGAACTGCTGCCTCAAAAGCGTGGCTTTAAACCTTGGATCC
TTTCAGAGCTTGAGTTTATAGAGCATAACTTTGCAAAAACTAAAGCTAAACGCAGAATGAAAATTGGAATTCCTTATTGTAAGACA
GTTTCACTGAGCCTTGACTCCAGTCCTTCCAGCCCCATCCCGCGGCAGGTCATAGGTGAGATAATAAATGCTGTGTCTGTTCAGGTG
GACCAAGAAGAATTTATTTTTACCATCCCGCTTTAATACGCAGGATTATTATTTCACACTCTAAGTAATGTGAGAATGACATGAATA
GTTCACATGGTGTCACCATCTGTCTTCTTTTTTAATGTCATCAGGAGGTGGAAAGTGTAATTGTTCTTGAGATTTGTTAGGAGGTTG
ACTGTGGTATTGTTGGGAGAGGGAGCTGATGGTTAAACTGTCTGCCCATACATAATTAAGAACAAACTTATAACAATTGTTTCATTT
TTAAAACAATTTTATATACTAGTGTTTTCTATCTAGTTACGGTAGTAAAATTGCAGGAAAAAAGCAGAAAATCACCCACCTCAGCCT
CCGAAAGTGCTGGGATTACAGGCGCGAGCCATCGCGCCCGGCCAACAACAGCTATATTTTTACAGTAATCTGCTGATAACTGGCTGA
TTACCGACCAAATAAATCTCTTCAGTGGTGGCAGGTTGAAGCTAAAAAGAAGTAAGGGATGCCTATGAGTCCCATAGAGGTAGGTGC
ATTTGCGTAGGAGACACTTCCCAATGATGTTAAAATCCTAACATGGAGTCAGAGATCTTCCTCCCAGCATGATTTATGTATAACAGT
GAATATTAGGAAAAATCTAAATGTCTGGTTATAGGGAAACGTTGAAGTTTTGATGCAGCTATTTGATGAAGTATTGTGAAACCTTTT
CGTTTTTTTGACAAATAAGACATTACAATATAATTTTTTAAAAACAGGAGACAATACTATGCAATATTATGTAATTTGCATAGGGACA
GTGACTAGAACGATATGGCCGGGTACAGTGGCTCACTCCTATAATCCCAGCACTTTGGGAGGTCAAGGAGAGTGCATAACTTGAGGT
CAGGAGTTCGAGACCAGCCTGGCCAACATGGTGAAACCCCATCTCTACTAAAAATACAAAAATTAGTTGGGCGTGCTGGCGCACGCC
TGTAATCCCAGCTACTTGGGTGTCTGAGCAAGAGAATCGCTTGAACCTGGGAGGCGGAGGTTGCAGTGAGCTGAGATCGTGCCACT
GCACTCCAGCCTGGGTGACTGAGCAAGACTGTCTCAAAAAAAAAAAAAGAAAAATAATGTTGCATTGTTCACTTATTCAGTGAAATA
TATAATATATATTGAGCCCTCCCCAGGCTAAGTGCTAGGAATATAGTGGAGAACAGAAAAATAATTGGTATTTTTAATGGAGTTTAT
GGGTAGGTGATAGGAGAGGTGGTTGTGAATCAGATAATCACACAAATAAATCTAGAGCTGTTTATTATGTGCATGTCATAAAGAAGA
AATACAGTGCTGAAAAGTGTGTCAGTGTGGGGGCAGGGGAGACAGTGAAGGCTTTCCTGAGGAAGTGGTATTTGAGCTGAGATTTGA
TGGGCGAGTAGGTGTCAGATGAAGTGGGGAGAAGAGCATTCGTTGCAAGGGAAGTAGGATGTGCCAAGGTACTGTGGTTGGAGGGTT
TTTACAGTAATCTTCTGCTGATAACTGGCTGATTACCCACCAAATAAATCTCTTCAGTGGTGGCAGGTTGAAGCTAAAAAGAAGTAA
```

EP 2 204 376 A2

```
GGGATGCCTATGAGTCCCATAGAGATAGGTGCATTTGCGTAGGAGACACTTCCCAATGATGTTAAAATCCTAACATGGAGTCAGAGA
TCTTCCTCCCAGCATGATTTATGTATAACAGTGAATATTAGGAATAATTCCATAATTGAAAGCCAGTATAGCTGAACTGTAGATAGG
GGTAGAATAGTGCAAGAAGAGCTTTACTTTTCAGGATTTCCTTGTGATTGTATATTACTTTTATACTAAGCAGTCATTTCAAATAAA
TATTCAGGTGGCCATTCTTTTCATCTGTTGTAGAGCTAATCTTGCATAAATGTGCATTAAGAAAATAAGATTCAACCAACCCAAATG
TCCAACAATGATAGACTGGATTAAGAAAATGTGGCACATATACACCATGGAATACTATGCAGCCATAAAAAATGATGAGTTCATATC
CTTTGTAGGGACATGGATGAAATTGGAAACCATCATTCTCAGTAAACTATCGCAAGAACAAAAAACCAAACACCGCATATTCTCACT
CATAGGTGGGAATTGAACAATGAGATCACATGGACACAGGAAGGGGAATATCACACTCTGGGGACTGTGGTGGGGTCGGGGGAGGGG
GGAGGGATAGCATTGGGAGATATACCTAATGCTAGATGACACATTAGTGGGTGCAGTGCACCAGCATGGCACATGTATACATATGTA
ACTAACCTGCACAATGTGCACATGTACCCTAAAACTTAGAGTATAATAAAAAAAAAAAAAAAGAAAAAAAAAAAGAAAATAAGATTCA
TTTGGATTTGGAATCAGAGGCAGACTAATGTATTATTTATGACGTAGACTTATAGGGCACTTTTCCACCAGTGAACTCAGAAAAATA
TTATAGGTGTGCAATTTATGGTTCCATAAGAAAACAGTCAAAGCAGCAGGCCTTGCTGATAGTATCTTTCATGCTTACACTTACTCC
AGTCATTAAGTTTGGGATGAAGAAGTTAACAACAGCTAATCATTTAGTTCTTGTAGATTCATTTTAAGAGCCTGTCATAGTTGTTTT
TTATAAAAACTTCCTACAGAGGGGAGATTTCAAGTGCTAAGAATATTTTATGATTTTGTTTTAGGTTACAGGAGGTTCCAGTGGCAT
CGGGAAGTGCATTGCTATCGAGTGCTATAAACAAGGAGCTTTTATAACTCTGGTTGCACGAAATGAGGTAAGGCTTCTAGGTTCATT
ATTACTGACTTGCTTCTTCGACTTGAAATTAGTCAAAGAGGCTGTGCTAAACATCTTCACACCTAGAAAGCGCTTCTTCCT
TGATGTGAGATGTGTTTCCTGAACTACTGATTCTCTCTAGTATTTAGGCCAAACACTTCTCTGTTGAAATCAGGTCGGCCCCACAAG
TATTTATTTAGTACCATTGGCATATTAGGCAACATCATCATAGATACTGCAGATCCTGAGATGGGAGAGGCAAGGTCTAGTTGAAG
GAGCACACCTGGCCATTGGAAGCCAGGGAGGGCCCTTGGCCAGGTCAGAAGTGCTGGAAGCAGTGATTCTGGAGCTGATACCTGAAG
GTGTGTAGGTGTTTCTCTATAATTTTTCACTACGATTTGCTTAGGACATGATATATATCTCTAGTGAAATCTATACACCATAGAGGA
ACCTCGTCCCTCCTTAGAAGTTTAGCAAAATGTGTTTTTCTGTATTAAGTCAACACAGAATCTGTTTTTATTTTTTGTCAGGGTTTG
TTTTTGTCTTGCCAAAGTTGTTTTTAGAGCACAAAGCAGTGACACTGTGCACGTGAACTGTCATGACGTCCTCTTGCTCCCCATATG
GGGACTGGAGGAAAGTGACCACACAGCTGGCTCGTCCGCTGCAGCAATTCTAGTGCTTGACACGTACATGCTCAGACATGTTTG
TTGAATGAAGAAATGAAGGGAGGAATGATGGAGTGCCTGGGAATGTCAGCATGGCATACTTCCATGGGGATCTTGGATTCACCCATG
TGCAAGGAGGGTGATAGTATGGTAACTCCTCAAATGGGTGGAGGGCTGGACAGGACCCATTAGGAATGGTAGGCCAGGAAGGGCCTT
GGGGGAAAATTTAAATAATGTCGCAGACTTAGTCTTGGACCTCTATAATTTCTTTTATTTTTTCTTTTTTCTTGAGACAGCATCTC
ACTCTGTTGCCCAGGCTGGAGTGCAGTGGCATGATCTCGGCTCACTGCAACCTCTGCCTCCTGGGTTCAAGTGATTCTCCTGCCTTA
GCTTCCTGAGTAGCTGGGACTACAGGCGCCCACCACCACGCCTGGCTAATTTTTGTATTTTTAGTAGAGACAGGGTTTCACCATGTT
GGTCAGGCTGGTCTGGAACTCCTGACCTTGTGATCCGCCCGCCTTGGCCTCCCAAAGTGCTGGGATTACAGGTGTGAGCCACCACAC
CTGGCCTGGACGTCTATAACTTCTTTTGCAAACCAAGAAAGCAAAATTAGCTACCTTTTATTTATAGCTTGACTTTTCTAACAATG
AATTTGCTTTGGCATTGAAGCCACCTTACATAAATGAAAACACAAATTTGATGAAATGGCATTTTGTGAGTTTTAAACACCAGTTTT
GATGCAAGTAAATAAAAGTGGAGGCAGAGAAGGCCAGCATTTCAGGCCCCCAGGAGCTCATATGGGGCCCTTGCATTGCTAGAAAATG
AATGAACTGAATGAAGAACCTGTGGTGTTGAGTAATTCATTCTTTTTTTTTTTTTTTTTTTTTTTTTTGAGACGGAGTTTTGCTC
TGTAACCCAGGCTGGTGTGCAGTGGCGCTGTCTTGGCTCACTGCCAACCTCCACCTCCGGAATACCTGGGATTACAGGTGTGTGTAAC
CACACCTGGCTGATTTGTGTGTGTGTGTGTGTATATATATTTATATATATATAAAATATATATTTTTATTATATATATTATATAT
TATATATATAAAAATATATATTTTTATTATATATATTATATATTATATATATATAAATATATATTTTTATTATATATATTATATATA
TATAAAATATATATTTTTATTATATATATTATATATTATATATATATATATTTTTTTTTAGTAGAGACAGGGTTT
TACTATGTTGGCCAGGCTGGTCTCAAACTCCTGACCTTAAGTGATCCACCCACCTCGGCCTCTCCCAAAGTGCTGGGCTTACAGGCGTG
AGCCACTGCGCCCGGCCTCACTGTGTCACCCAGGCTGGAGTGCAGTGACACAATCTCGGCTCACTGCAACCTCCGCCTCCCAGGCTC
AACCAATCCTCTCACTTCAGCCTCTTGAGTATCTGGGACCACAGGTGCTCGCCACCATGCTTGGCTAAGTTTTTGTATTTTTGGTAG
AGACGGGGCTTCACCATGTTGCCCAGGCTGGTCTCAAACTCCTGAGCTTGGGTGATCCACTCACCTTGGCCTCCCAAAGTGCTGGGA
TTACAGGGGTGAGCCACCATGCCTGGCTGAGAAATTCTTTCTTTTCTAAGGTCATTTTTAATTCCAGTCTTTGCAAAGTCAAGCTGT
ATGTATCTTTAGTCATAGGACAATATTATTTCATTATAGACATATTCAGGTTTAGAAACTCTGCATGCCTTTTGGAAGAATTCGCCT
CTCCTTTGGTTCCTCTCTATTGCATGATATTATTTAAGTTACTTTTTACAGCAAATTAAGATAATCTGATATCTGTTTTCTTGAGAC
TAAGTAAAACAAACAAAATTAGGACCTTGGAAGAATTACATAATTACACAGGTAACCTTGCTTAAGAGTATTTTATAGTAGCCAGGC
CTAGTTTACCCAGTCTATCCAGAAAGTGATTCTTACTATGTTTGGGTTTATAGTAGAAGAAGAAAATATTGGACAAAAAGCAGTATT
GACAGGGAGTCACACATACCCTCAACCTTGGGGAGCAGTACATATTAATAGAGTTGTTGTTCTTGTCTTATATCACAACTAAATAATAAG
ACATTTATTAGAACTGTAGAGTCAGCCTCAGCAATTAGTACTGTTAACTGGGATTTTGAGGTTTATCTGTTTTCTACTATCTTTCTC
TAATTAATTTTCAAACTTTTATCACTTATTCATAGCTTAACATACTTCACAACTTCTTTCTTTCATAGGCTGTGGCCCAGTCTGTCT
ATGCAATGTAGATACTGTTTTGTGTGTTTTTCTGGAATTTAATACATACATATAGAAATAATTGCTTTTTTTCTCTCCTTTGCAATAA
ACGGACATGCATATAGACGGTCATCACGACTAATTATTCTGTATTGCTTTCAGGATAAGCTGCTGCAGGCAAAGAAAGAAATTGAAA
TGCACTCTATTAATGACAAACAGGTAAATCTTGTGTCTTCAGAATGCATTTTCTGTATAACTCAGCACAGCTGTATAAAGGAAAAACA
GAGTTACTTCACCTGTGAATAGTTAATTGAAAGCATATATGTACTGTTTTTTAAAAGGTGGAATTGTTTGAATGGTCTAGTAACTCA
GAATATCACACACGCAAAATGAATAGACTCCCACACACATTCATATCTACAAATGGTCAAATAACAGAATGTTTTACTGGCAAAT
AATTTCATTTTAATTTTTTTTTTAATGAACTTTATACAAATGCTTAAGAGGAAAGGAATATTTAAAATTTATTTGGTTATGGAATTT
AAATGATCAGGGTCTCTGAAGCCCCCACAGCAGCCTTGTCCCTCTTTTCTTGCTCCTCTAGAGGGGCAAATGCCAACCCGATTTTTGGT
GTTAATACCTTTCTTTTTTCTTTTTCTTTTTTCTTTCCTTTTTTTTTTTTTTTTTTTTGAGGCAGTGTCTCAATCTGTCATCCAGGC
TGGAGTGCAGGGGTGCGATCTCAGCTCACTGCAGCCTCGACCTCCTGGGCTCAAGCAGTCCTCCCACCTCAGCCTCCCAGTAGCTG
AGATTATAGACACGTGCCATCATGCATGGCTAATTTTTGTATTTTTGGTAGAGATGGGGTTTCGCCATGTTGCCCAGGCTGGTCTTC
AACTCCTGGGCTCAAGCAGTCCGCCTACCTCTGCCTCCCAAAGTGCTGGTATTATAGGAGTGAGCCACTGTGCCCTGCCTCTTTTTC
TTTTTAATTTTACCATATATGCACATAACTCTAAATAATAATATTGTTTAGTTCTTTTTTTTTTTTTTTTTTTTGAGACAGGA
TCTCTCTCTGTCACCCAGGCTGGAGTGCAGTGGCTGCCATCTTGGCTCACTGCAACCTCCACCTCTCGGGTTCAAGCGATTCTCCTGC
CTCAGCCTCCTGAGTAGCTGGGATTACAGGTGTGTGCCACCACACCCGGCTAATTTTTTTGTATTTTTAGTAGAGACAGGGTTTTA
CCATGTTGTCAGGCTAGTCTCAAACTCCTGACCTCAGGTGATCTGCCTGCCTTGGCCTCCCAAAGTCCTGTGATTATGGGCGTGAG
CCACCATGCCTGGCCAATATACTTAGTTCTACTTCTTTTATTATATCATTCTTATTTTTTAACAGCTTTATGAGATATAATTTATAT
AGAATGTTTTCCTCACCCCGTGAAGAATCTCCATGTGTATTAGCAGTCACTTGCCATTTCCTCCCAGCCTCTTCTCAGCTAGGCAGT
CACTCATCTACTTTCTTTCTCTATAGATTTGTATAACTTCCTTCTTTATGCTTCTTATGCAACTTGATTTCTTCACTCAATATTATT
ATTATTTATTTCTTATTTATTTATTTATTTTTGAGATGGAGTCTCGCTCTGTTGCCCAGTCTGGAGTGCAGTGGCACAATCTCAGCT
CACTGCAACCTCTGCCTCCTGGGTTCAAGCCTCAGCTTCCTGGGTAGTTGGGATTACAGGCGCGCGTCACTACGCCCAGCTAATTTT
TGTATTTTTAGTAGAGACAGGGTTTCACCATGTTGATCAGGCTGGTCTCGAACTCCCAACCTCGTGATCCGCCCGCCTCAGCCTCTG
AAAGTGCTGGGATTACAGGTGTGAGCCACCACCATGCCCGACCACCTCAATATTATTCTTTAGGCCAGGTGCGGTGGCTCATGCCTGTAA
TCCCAACATTTTGAGAGGCCGAGACGGGCAAATCAGTTGAAGGTAGGTGTTTGAGATCAGCCTGGACAAAGTGGTGAAACCCTGTCT
CTACTAAAAATATAAAAAGTTAGCTGCACCTGTAACCCCAGCTACTCAGGAGGGGAGGCTGAGGCAGGAGAATCACTTGGATCTGGGA
GGCAAAGCTTGAGGTGAGCAGAGATTGCACCGCTGTACTCCAGCCTGGGTAACAGAGGGAGACTCCATCTCAAAAAAAAAAAATATAT
ATATATATATATATATGTATGTATGTATGTATGTATATATATGTGTATATATATATATAATTTTAAAAATTCATTTATGTTGCTGCA
```

50

```
AAAAGTTGGAGTAAATTTTTTTTTTTTTTTACTGCTGCGTAGTATTCTATTGTATGTATAGACTACATTTTGTTCATCAGTTCAGCAAT
TGGTGGATATTTAGTTTGTTCCCAGTATTTTTGTTAGTTTGTTTTGTTTTTTACTATTAGAAAAGCTTTCAGTATTCTCTTTGTGCA
CGTGTGCAAGAATGGTACATACCCAGAAATGGAATTGCAGAGCTGTAGTGTATGCAGTTCTTCAACCTCACCAAAGTGGTTAAGCTC
CCATAAGTAGGAATGCGGTTTCTGTTGCGCCACATCCCTGCCAATACTTGGTATTGACAGACTTTTTACTTTTTGCCTTTCTGATGA
GTTTTGAATAGTATTTCACTGTGGTATTCATTGACTTTTTCCCTTCTAGAATGTTAGAGATCTTTCCATGTGCATTAAGTAAATTTG
CCTTAAATTGCTGCAGCCTAGGATTGAAGTACACCAGAGCTTATTCCGTCAGGTCCCTGCTATTGAGCTGTTTCCATTCTTTTTATT
TTATTTTGTATTTTTATTTTTGCAATTGCAGTCTGTGCTGCAAGTAAAAATCCTTGTGTATATCTTCTGCACATGTGAGTTTTTCTC
TAAGACTGTGGTCTTTACCCCCTTCTCTGAAGCCCACAAGCATTGGAAAAACACCAGCTCTGTAAATTGCCCCGTTTTTTCCCGTAT
TCCTGTAATATCTGATAGACGAAGGAGTATGGTGTGAAGTCATGGAGTCAGTGTTCAGGGCCAAGGCCAGTCTTGCCTGCTGCCAAC
ACTGTCTCCGCTCTGGGCTTCCTCATCATGACACCTTCTACCTCTTCATCTACCAAAGGACTTTAGGTCAGGTGGAAAACTTACCTT
TCACATCAAGGAAGGGAGATAGGGCTGGGGCAGGCGAGAATGACGCTTGATCCTTCTGCCCTGCCCAGGCTTCTAGGGACCTTTTGTGT
GTGTGTGTGACTTGTTAACATTTTTTTGTTCTGAGATTTCACTTGTCTGTGGCATAGATTTTATTATTAGTAACTGATCTAATACTG
CATGAAAAGGCTTGTTTTAAAATTAATCATATGATTACTACTGCCTTTTTTTTTTTTTGAGACGGAGTCTCACTCTGTTGCCCACGC
TGGTGTGCAATGGCATGATCTCAGCTGACTGCAGCCTCTATCTCCCAGGGTCAAGCAATTCTCCTGCCTCAGTCTCCTGAGTAGCTG
GGATTATAAGTGCCCACCACCACGCCAGGCTAATTTTTTGTATTTTTAGTAGAGACAGGGTTTCACCATGTAGGCCAGGCTGGTCTC
AAACTCCTGACCTCAAATGATCTACCTGCCTCGGCCTCCCAAAGTGCTGGGATTATAGGCGTGAGCCACCGCGCCTGGCCGATTACT
ACTACCTTTAAAACAATGTTTTCTATCTGAGGATGTTGGGATAAAATCCACTCTAGGTATACATTACAACCAAGGGCAACCCCTCTA
GACTAGAATGCTGCAGGTTATATTTGCAAAGAGGGCTGTTTCCTTTTCTTAGGAATTTTGATCATTTGGACAGGATTCCTTCTTGAA
AGGAATCAGCACTGAGGCCCTTATATGGAATATTTCCATCACTCCCTAGGGTTGGCCTGTGCCCTTTCACAGTTCCTTCCATCCTCC
ATGCCTGGCTGTGGGCAACCCCTGATCTGCCTTCTGTCACTATAGATTAGTGGCATTTCAAGAGGAGCATTTATATATTTTTAAAAA
GATTCCTCAGATTTTAACTCCAAAAGCTGCCAAGAAAATTAGATTCTGCTAATTGCATCATCAGCAAACACTGAAATCTGTTCATT
CAATATTGGCTTCCTTCTGTATTCAATGAACTGTCTTTTCTTTAAAAAAATATCTTTAAGAAATAGTTTGGTACCTGCAAAACTTTGA
TCACTTTTTTTCTTTTTTAACAGGTGGTACTTTGCATATCAGTTGATGTATCTCAAGACTATAACCAAGTAGAGAATGTCATAAAAC
AAGTAAGAGGTTGGGCTAATTGGAATTCACCAATGTGCTTGACTTTTGATTTGTTCTTGCCATTTTGCTATGTGGCTTGGAATGGAC
ACAACTTCTCTGTAGGTTTAATAGTTTTGTAAATTTTTATTTATCAGCATGGATTTGCTGCTGAGTGGTTTTTCTTCTCTGTTTCTTA
ATGCCTGCTCTGCTACATTTTTCTAGGCACAGGAGAAACTGGGTCCAGTGGCATGCTGGTAAATTGTGCAGGAATGGCAGTGTCAG
GAAAATTTGAAGATCTTGAAGTTAGTACCTTTGAAGTAAGTAAAAATGTTTGCTCACTGCAGTAGCACATATGCTAAAAGCAGAGCA
AGCTACGGGTAAAAGCTTTAAGAATCAATTAGATGAATGCATTCAGATGTCCATGGAAGCTTTAGAAGACTCTAGAGGAAGGCTGAA
CAAGGTGAAGCTGAACAAGGTTTTCATCCCAGTGTCAGGACCATTGAAGCTTAAGAGTTTAGACATTTTACAGCTGAGAAAACCAGC
TAAAATGGGAATGGACCTTCTAATCTGACACAGATTACTGTAGCAGTTCACTGAGGTCATATTTTCAAGAGAACACTTTGTAACCTA
AGAGATGCCGGATGTTCCTATCAAACATCAGTATTATTAACAAGGAGAAACCAAAAAAGTTGAAGGACAGACTCACTAGAGTTCTCA
AATCAAGATAAAAAGATACTGCATCTTATTGATGTTCTATTTTTAATCAGGTTTACCAAGGAACTTCTCTTGGTAATTTATTTCAG
CACCCAAAGAAATGAACACTAGATTTGAAATTTTTATTTTTTCTTTTTTTGAGATGGAATTTCACTCTGTCACTCAGGCTGGAGTGCA
GTGGCGTGATCTCAGCTCACTGCAACCTCCACCTCTCAAGTTCAAGTGATTCTCCTGCTTCAGCCTCCCTAGTAGCTGGGATTACAG
GCACATGTCACCACACCCAGCTAATTTTTTTTATTTTAGTAGAGATGGGTTTACGCCACGTTGGCCCGGCTGGTCTCAAACTCCTGA
CCTCAGGTGACTGGCCCACCTCTACCTCCCAAAGTGCTGGGATTACAGGCGTGAGCCACCAGGCCCGGCCCTGAAATGTTTTTAAAAT
GGAAAACGTTTTTTAGTTGCCATCGCATCAACAATCTTTTTCTTCCTTTTTTTTTGAGACAGAGTCTTGCTCTGTCGCTGAGGCTGGA
GTACAGTGGCACGATCTCGGCTCACTGCAACCTCTGCCTCCCGGGCTCAAGCGGAGTGTCCTCCAGCCTCCTGTGAGGATGAAGCTG
GGGAGTGTGTTGGTGTCTTCGTGTGCGCCGTCTAGACAGACTTTCCCTTAAACCTCTCTCTTTATCTAGACTTACTATCCCTAGAAA
CAGAACCTCCAGTCTCCTGCCAGGCTGGAAGAGGGGTACAGATCTACCCTGTGGAGAGGGGAAAAACATCTAAGATCTAACTGCCTT
TTTTTTTTCATTGTCCTACATTATTTTTTTAATTTGAAGTGAAATTCATATAACATAAAATTAACTGTTTTTAAAGTGAGCCATGCAG
AGGGATTCAGCATATTCACAATGAGGTGCACCCACCCCCTCCGCCTAGTTCTAAAGCATATGCATCACCCATAGGGCGGCCCTCACC
TGGTAAGCAGTTGCTCCCCATCCCTGCTCACCCCCACACCCAATCTCTGACAACCACCAATCTGTATTCTGTCTCTGGTCATTTCGT
ATGATTGGAATCATGATATGTGCACTTCTGTGTCTGACCTCTTTCTCTTAGCATCAGTTTTTGAGGCTCATCCACATGTAGCATATA
TGGGTGCCTCATTCCTTTTTATAGGGTGGTGATATTCCTTTGTATGGATCGACCACATTCTGTTTAGCTATTCATCATCGATGGCCA
TTTGGGCTGTGTCTAGCTGACTGCTTATTAAATAGACTCTTAGTCCCTACTCCTGTCTCAGCCCCATGCCAGCCCACCCTTCCCTCC
ACCCCAGAGACTCTTGCACGCTGGAGCCCTTTTGGGGTTCTGTAGTAGAATCTGGCTTTTTAGTTTCTCCACTCCCCGCTTAGCATTTA
GTTTTTTTGTGGTCTGCTAAGTTAGTTATCCCTCCTCAACCTGTTCTTCAGCTTCCAAAAGGTTATTGCTGTTATTTTTATTCTTATT
TTCTTTGTTCTTGTGTGTTCTTGCCTTAAAAAACATCTCTTTACGGAGATTTTAGTAAGGTTTCTAAAGGGAGCCAAAGTAAACACA
GGCGTTCAGTGTGACGTCTTTTTTCAGATATTCTTTTAGAACTTTCTTTTTTTTTTTGGAGACGGAGTCTTACTCTGTCACCCAGGCT
GGAGTGCAGTGGCTCAATCTCGGGTCACTGCAGCCTCCACTTCCCAGGCTCAAGCAATTCTCCTGCCTCAGCCTCCCGAGTAGCTGG
GATTACAGTTGCCACCACTATGCCTGGCTAATTTTTTTTTTTTTTTTTGTATTTTTAGTAGAGACGGGGTTTTCACCATGTTGGT
CACGCTGGTCTCGAACTCCTGACCTCAGGTAATCCACCTACCTTGGCCTCCCAAAGTGCTGGGATTACAGGCATGAGCCACCGTGCC
CGGCTTAGAACATTTTTTATTATCAAAACAATATTATGTTACGAAAACACACTTTTGGAAAAAAAAGAGAATCCCAGCATCCTAACT
CAGTCACTTATGATCTTCTGTATTTCCTTGGTCTTCCCTCTGAGCATGTGTACTTTTACTTAACCTTTGAAGTAATTACAGTTACAG
CATATATACAGTCTAGCTTCTGCTTTTTAAACTTAAATAGCATAGATATTTTTCCCTGTCACTCCCAGTGTTTCTGGTCATCATTTA
AAACAATTATCTAGGCCAAGTACAGTGGCTCACACCTGTAATCCCAACACTTTGGGAGGCCGAGGCAGGCAGATCACTTGAGGTCAG
GAGTTCGAGACCAGCCTGCCCAACATGGCGAAACCCTATCTCTACTAAAAATACAAAAAATTAGCTAGGCATGGTGGCTGGCGCCTG
TAATCCCAGCTACTTGGGAGGCTGAGGCAGGAGAATCGCTTGAACCCAGGAGGCAGAGGTTACAGTGAGCCAAGATCACACCACTGT
ACTCCAGCCTGGGTGACAAGAGCAAAACTCCATTTCAAACAAATACAAACAAACAACAATAAAAAAATCTAATATTCTCTTGAATTA
AGATATTATGATTGTATGTTCATGTTCTGTTCATAATATGAAAAAAAATTATGATTGATCTCAAGTTCCACAATTGAGGTATTTAGT
TTTTATTTACATGATGATCTAATATTTTTACAAATATTTGAAATTTGAAACATTTTAATGCAAGTATTATTAAATTTTCGAGTTAA
AATTTAAGGTTTTTAAGACGTCTCAGGCTAGGCGCAGTGGCTCATGCCTGTAATCCCATCACTTTGGGAGGTCAAGGCAGGACTGC
TGGAGCCCAGGAGTTCAAGACCAGCCTAGGCAAAATAAAAGGACCCTGTTACTACAAAAAATACAAAAATTAGCTGGACGTGGTGGC
ATGTGCCTGTAGTCCCAGGTCCTCAAGAGGCTGAGGCAGGAGGATTGCTTGAGCCCAGGAGATTGAGACTGCAGTAAGCCAAGGTTG
CACCATTGCACTTCAGCCTGGGTGACAGAGTGAGACCCCGTCTCTAAAAAAAATGATAATAGTAAAAAAGCAAAAAATAAATAAATA
AAAATTAAAGCTTCCAAGACAAGGAGAAAATACACTATTAGTACTCTTACCCATCATATGGGAGAGCCAGTGGTTCTTGAGGTCC
AGGACTGTGCTAGCGCTTGTGTTTTCTCTTTTAATCTTTGCAACAGCCCTATGCGGTCATACCCTTGTTGACCTTGTTTACAGCCAA
GAAAATAAGTAGAATGTCTGACTTTCTTATTCATCATCAAATAGGCAATAAATGGGAGACCCAAGGCTTGAAACCAAAACTTTAAAT
CACCCTCCTACTCTGTGTTTTTCTTATAGCTTAGAACTAATAGGGCTACAAATACTTGGAACCTCTTTCCTAAAACTGTCTTTCAGA
AAGTACTACCCACAAACATGCAGTATACTTTTTATAAGGAGAAAACTAACTTTGACCAGTTTTCAGGAAAAACATTAACTGCTGTCA
TGGTCAGAATGCAAGACTAGACTGAAACAGCTCTTCAAATGGTAGGTCCTGAGTCTTCAAATGCTAGGTCTCAAATCACATTTCATTT
ATATCATCATGTCATCATTCATTCATTCAGCAAGCCTGTTGGGCTTCCCAGTCACTTGAGCTTGCTGCATTGAAGAACTGAAATCCAGAA
AGCAGACTAAATTCTCCATGTGCACAGCCTTGAGGCTTTTGACCTCATGTGGCCTCTTTGTTCCCTGCAGAGGTTAATGAGCATCAA
TTACCTGGGCAGCGTGTACCCCAGCCGGGCCGTGATCACCACCATGAAGGAGCGCCGGGTGGGCAGGATCGTGTTTGTGTCCTCCCA
GGCAGGACAGTTGGGATTATTCGGTTTCACAGCCTACTCTGCATCCAAGTTTGCCATAAGGGGATTGGCAGAAGCTTTGCAGATGGA
```

```
GGTAAAAGTTGTATTCATTTTTGTATTTATTCCTCTCCGCTCAGCAATATACATTCCGCTTTAGTTTTATAGCTTCTTTACATGTGT
TGTTGTATTAAATTAGAGATTTTTAGGTTTGACATTGGTGAAAGAGAATTGGCTCAACCCTGAGTTTTGCAGTTAACATTTTTCATT
GCATGAAGACTATAGTTAACGACAGATGCAAAATGGTAATGGGATTTTTGCTTGTTTAACTTAATACACAAAATATTAGTGATATCT
TCATAGTGTTATTAGTCTAATACTGTGTGAATGTGAGTAAGGTTAACCAGATATTTTATTTTATAGAGATTTTTCTTTAACAAGAAG
TTTGTCCTTATGACTGAATAATACATGAAATTAGTATATGTTTGAGGGAAGAGCTTGAATCAGCTGCTAATTTTTAGCTTTTAGACA
AGTCATTTAACTTGCTCTCAGTTTCCTCATCTGTAAAATGGGAGGGTTGGAATAAAGCAATAATTTGACTCTCCCAATATTTATATT
TTTATATCTTAAAGAATTGTTGGGAAAATGAATGCTTTTTATTTACATAGTTAATGGAACATTTTGTGGAAGAAGGATGTAGTTAGG
ATTTGCATCAAAATAGCGTCTGTCATGATGAGTGCAACTTTACACCTCAGCTGCTTGAAGCCGACTTCCACATTATTGTCACACAGG
AATGCTTAGAGAGCATCACATACGTATCTACCCAAAAATCACATGCTGAAAAAGGCAAGGGAAAGTTCTTTCTATTGCTGTGTACAG
TCATCCCCTCTTATCCGCAAGGGATGTGAGCTAAGACCCCCAGTGGATGCCTAAAATGTTAAGACCCCAGAAGGATTCCACTGTGG
CCAACCCTCTTATAGTAAGAGTGTGCTTTTGGGAATCCTGGTTGATGATTTAGATCAGGGGTTGACAGAACTTTCACCAAAGGGCAG
ACAGTATATATGTTAGGTTTTAAAGGCTGAATGGTGCCTAATACAGCTACTTAAGTAGGGTATGATAGCAGGAAAGCAGCCTTACTA
GACAATGTGTAAATGAACAATTGTCACTGTGTTCCAATAAAACTTTACTTATAAAAAACAGGCAGAGGGCCAGATCTGGCTCATAGG
CCATAATTTGCTGACCCCTGTTTAGAAGACCCCATAAGAGACCTTGATGGTGTGTGATAGAGCACCACATTGGTGAGATATGGGACG
AGCGTTGCAGTACAGATGAGCTGCTGGGACAGCGACAGTCCTGATGTGCCTGCGCGTAGGGACTGATAGATCATGCCATTCTCATACCAT
AGGAACCAGCCACAGATTTTATTGTTATCAACTGGCCTAATACTCTTAATAATATATCGTGATAATATGCTTAGCCAATAGGACACT
GATGTCTGTGAAATTTCAAACAAGAATACTGGTGTTTCTTTTGAAGTTTTTAACTTTGAAAAGTCCTTTTGTGAATAACTGTTTTAT
TTATTTTTATTTTATTATTTTTTTTTAGAGACAGCGTCTCTCTTTGTTGCCCAGGCTGGAGCGCAGTGGCACAATCATAGCTCACTG
CAGCCCTGAGCTCCTTGGCTCAAGTGATCCTCCTGTCTCAGCCTCCCAAGTAGCTAAGACCACAAGTGCACACTACCATGCCTGGCT
AATTTTTAAAATTTTTTGTAGAGAAGGGGTCTCACTGTGTTGCCAAAGCTGGTCTCAAACGCCTAAGCTAAGCTCAAACAATCTTCTGGCTT
TAGCCTCCCAAAGTGCTGGGATTACAGGTGTGAGCCAGCACACCCAGCCACCATTTTATAAAGTTTGTAAAGTATGTGAGGGAGAT
GTTGTGATGCCACCTGATGAGAGGAAAACCCAGTTTTCAGTAAATTCAGTGTGTTGCTCAAAGTCACATAGTTACTTAGTGACAGAA
ATCACAACACGATCCAAGCCTTGACCCCAGGTCTGCTGCCCTTTTGCAGCAGATGATGCATCCAGCTAGTTGCTTCACCTGGCTAGT
TGCCCCGGCCCTTTACTTTGGTTTTAGAATCCTTGTAGGTGAAGGGGTGGTTGCGGGATGAAGAATCTGCCCATTAGGGCTTTTTGC
AGAGCTGTGTCAGCATTCACTAATTTAGAGCAAAGTATATAATCTGGGTGGCCTGGTTTCACTTCAAGTAGAACAGAGATGCTAAAA
AAGAGGAAAAAGAAAGTGGATTGCTGGTACAGACAGAGCCCCCCAACCTCTGTGACAAGTGTGATTTGCATACTGCGAGTAGCCGGC
CTGCACGCTCAAGTCTGTATGACTTCTGTCCTTATTTGGACTCTATTAAAAATACCCTCACTTAAAAAAAAGAAAATAAAATGAACT
TATCCCATGAAAAATAGGTTCTGGGGAAAACGAACGCAGTCATCAAAATGTAGTTTGTTCGTAGTAGAACTTGCATCAAACCAATTA
TTGTGTCCCTACAGTAATTATAGTAGCATTTTTTTTTGAAACCTGGATAATTTGGTCAAGGTAGGTGTGTTTTCTGTCAATGAAATCT
TATGTAATTGCTCAAACTCCAGATACTTTTTTTTTTTTTTTTTAATTTTTCTGAAGACAGAGTCTTGCTTTGTTGCCGAGGCTG
GAGTGCAGTGGTTGATCTTGGCTCACTGTAGCCTCCACTTCCTGGGCCCAAGCAGTCCTCCCACCTCAGCCTCCCAAGTAGCTGGG
ACTACAAGTGTGTACTACCATGCCTCACCGTTTTTTTGTTTATTTTTTATTTATTTATTTTTTGTAGACATGGGGTCTCGCTCGCTA
TGTTGCCTGGGCTGGTCTTGACCTCCTGGACTCAGGTGATCCACCTGCCTTGGCCTCCCAAAGTTCTGGGATTACAGGAGGTGTGAG
CCACTGTGTCTCGGCCTGTAAATATTTTTGACAATTCTTTGGTTTAATTAACTTTGAAGTCTCCCTGTCCACCTCCCTTTGCCCCCAA
ACTAAGGTAGGACCCTGGCATTGTTAAGGGTACTATGAGTAATTTAAAAGAATGCTTTGTTGGCATTTTTTAGTCTTTTTTTTTTA
CTCAGTCTTTTAGGAAGAAAGGTAAAGATTTTAAAAGTTACTCTTTTGGAAAGGTGGGTTATAGCTAAAGATGTG
GTAGCAGAGACCAAGCCCAAAAAAAAAACAAACTGATGCTCCCTTTTCTGCTTTTCTTACCCTGATCATGCCCCTCTCCTCCCATCTCT
TCCTTCTGTGGTCTGAAAGTAAGCTAAAGCTTGAATTCTGGAGTTTCATGTAAACAGCTGTGATCTTCTTTCCAGCATCCCTGTCT
TTGTTTTGATGGAATAGGGAACAGCACATCCTATGTGGATTATTTTAATAACAAATCTGTTCTTTTTTTTTTTTTTTTTTTTTTTG
AGATGGAGTCTCACTCTGTGGCCCAGGCTGGAGTGCAGTTGTGTGATCTCGGTTCACTGAAACCTCTGCCTCCCTGGTTCAAGCGAT
TCTCCTGGCTCAGCCTCCCAAGTGGCTGGGATTACAGGTGCCCACCACCATGCCCGGCTAATTTTTGTATTTTTAGTAGAGATGGGG
TTTCACCATGTTGGCCAGGCTGGTCTTGAACTCAGGTGATCTGCCTGCCTCGGCCTCCCAAAGTGCTGGGATTACATATGTGAGCCA
CTGCACCCGGCCCACAAATCGGTTCTTGAACAAGTGGTCAGTTGTCTTTCTGTTTGAGGTTGAGGCCAGGGAGAAGGGAAGAAGTAA
TAGTAGGATGGGTGTGGGGCAGAGTCGGGGGCACAGAGAATGGTCAGGCAACAGGGATGAGGAGAAGCAGTGGCCGGGCAGCAGGGG
ACTCAGTTAGTGAGGACCTGGCTCATCCATTCTTAGAAAGAGTGGTGATCTCAGGGAGCTAGCATTGTCTCATAAGAACAAGTAAAG
CCAGCCTACCCTCATTTTTTTTTATGACAGGGTTATGCAGTTGGTAGATTAAAAGAAGATTGGCATTTTAGTATTTTAGTATATTCAGATT
TTAGCAAATTATTTGACCAAGAGTTTCAGGAAACTAGAATCTTAACCTTATGCTTTGTTCTTAGAACGATACTGGCATGTAGAAGTA
ATTCAGTAAGTGCTCGTTGTTGCATGAATAAATAAAATAATATGTAAATGGTGAGGTGACTAGCCACTGGAGGTATCCAGCCAGAGA
CTGGGAGAGTCATTTGGTTGGATGGCATAGCGAGAATACTAATCTAGGAAGCGACTGGACTGGTTTCTCCCAACCCCAGCTATCTGT
GTTCCTTCCTCATGAGGAAGCTGTTCTGAAAAGCTCTGTTTTAACCTTTGGCAATGCAGCCGTAACCTTGAGTTTGAGAAGCACTGC
TCTGAAATCCTGTCTTCCATTAGCTTTGGAGCCAGCATCCTTCTTTACCATGAGGAGAAGGCAGAGGGGCAGCACAGACGTGAGTT
TCTGGGCAGTCGATGTCAGTCTTTATGTGGGCTGCCTAATGACCACTCGTTGGATTTTTTGGTGTTTTTCTCTGGTCTATGCACA
GCAGAGGGCAAGGCTGGGGGAGACAAGGTTTTTATAAGTCACTATCTCAAAAACAAGTGGAAGCGAGTTTCTGGCTCATCTGCCTTA
CTACCTGGCCCCGATCCAGAGCTATTTATGAGCATGGCTGGGCCGTGAAGCTGCATGCCCTGGCAATGCCCGGTGGATGGGCTGTCC
CACACCCTAATTGTGCTGGAATGGAACCCACAAAGACATCTCACTTTCACGACTGCACTTTCCAGGATGGCTGGCCTGGATCTTCTG
CTTGCAGGCCTGGCTGCTTCTCCTTCCCCTTCCCCTTAAAGCCCTCAAAACTGTGCTTCACTGAAGGAACCAGGGCGAA
AAAGGGCTGCATAAGATGAGACTCTGGGCTACTGTTTTTATTTTTTTGTTGTTTTTTTGTTGTTTTTTTTTTTGGGATGGGAGTCTCC
CTCCTTGTCACCCAGGCTGGAGTACAGTGGCACGATCTCAACTCACTGCAACTTCCGCCTCCTGGGCTCAAGCAATTCTCCTGCCTCA
GCCTCCCAAGTAGTTGGGATTATAGGCACCCGCCACCACACCCAGCTAATTTTTGCATGTTTACTAAAGACAGGGTTTCACCATGTT
AGCCAGACTGGTCTCGAACTCCTGACCTCAGGTGATCCACCCCCTCCCTCGGCCTCCCAAAATGTTGGGATTACAGGTGTGAGCCA
CTGCCCCCGGCCCCTGGGCTACTCTTAAAGGCCGTCACATTAGGCAGTGGTGTTGATTTTTAGCCTAGGGTACCCTTCTGAAGATGT
GCAGTGCTGATCTATCAGCTGCCTCTCCACTTCCAGATCCCTTAGGTGTTTCCTAGGGACAAAAAAGTGAGCCAATACCAAATAAA
AATCATGGCAAAGAGATTTTCATGGTAGGAACCATTGCTCTTAGAGTTGCTAATTAGCAGGCAAGATAAGCATGTGCAGCAGGGCT
GTGGCTGAGCAGTCTGTGGGAGGGGGTGTCCTGGCCAACAGCAGAAGAATCTGCTGGAATTGTTGGGGGTGTGTGTATGAGTGGGGT
CGTGAAGCGTGAAGTGTGCAGGCATGGTGGTGAGGCACACAAGTGTAACAACCTGCTCCAAGTGTCAGATTTAGAGGAGCCACATAG
AGTTTCAGATAAGGCAGAGGGCTTTCCCTATGTGATGCAGGTTGAGGTTGTCCACAGGGGAGGTACTTGGCTGGTCTGACTTGGAG
GTTCTACCCAGTGGTTCTTGTTGGTTCAGTGGAGGAGGAAGTGACTCTTCGACCTCCTAATGTGCAGAGGCCGGTTATACAGGGAGCT
CTGGAATAAGCAGACTTCTCAGGGACAGTTCTGGACTGGGTTTGGAGTAGGGCACAAGAAAGGGATAGGGCAGCAGATTTTAATGAC
CCCAATGTATTTTGTGGATGAAATCAACACAAGAGTATCAGAAGCAGTCTGAAGGAAAAACAGATGTACAGTCTGGTGAAGAAAGGA
GGGGCAGACTGGTCCCATTGCCTTGATAAAGGGCTTGGATGCTGTGCAGTGCGTGTGGGAACTGGACTCTTTTTAAAACACTGCCCT
CGTCGGACGGCTCCCAGGGCCTCTGTCTGGGCAGCAGGAGTGGAAGACACTGCAAAGGAGGTGACTTGCCATACTTTAGGGCCAGCT
GTTTTCTGGGGTCCAGGTTAGGGTGAGCTTTTTGCCTACATTCTGGAATCATCATGGAGAGGGGTTTCTCAATGCAATTGGTGTAGT
TCCCCGCCTAACAGAATCACCCCATTGTTCTCACCGCTTTTTGGGAATCAGGGCAGCCAGTGCAGGTTGGCAGTGTAGGACCTCAGT
GTGTCTTGTTACTGAAGGTACACGTGTTGTTTTGAAATGCAGGTGAAGCCATATAATGTCTACATCACAGTTGCTTACCCACCAGA
CACAGACACACCTGGCTTTGCCGAAGAAAACAGAACAAAGGTCAGTATCCTACATGTCCCAATTTGAACCTCTAATGTTTACTGCTT
TTCTGATTTTCTTTCTACTCTTCAGCTCACACTCGTTTTCACCAATCAACAATGTTTGTTGGGTAGAGTGCCTAGCTGGTTTCTGAT
```

```
GACCTTGAGATTCTCCAGGCAAATATTCCTTCTTTCCTTCCTCTTTTCCCTCACTTTATTTTCTTAAAGTTTAAATTTCTACCAGAT
TTTTTCTCTATAGAGACAGGGCCTCACTCTGCCATCCAGGCTGGAGTGCAGTGGTACAATCGTGGCTAACTGCATCCTCAAACTCTC
AGGCTCAAGCAATCCTCCTGCCTCAGCCACCTGAGTAGCTGGGCCTACAGGCATGTGCCACCACACCTAGCTAATTTTTAATTTTTT
TGTAGAGATGGAGTCTCACTATGTCGCCCAGGCTGGTGTCAAACTCCTGGCCTCAAGTGATCCTCCCTCCTCAGCCTCCCAGGTGCT
GAGATTATAGGCATGAGCCATTGTACCCAGCCATTTCCAGCACATTTTTATCAAAGAAGTATGTCACACAGTTTTAGAGTCCAATAT
CAATCCTTTGCCTCATCTCTCCCATTCCCAATTCCTACTCCCTAGAGGTAGCCATTTATTTTAACTAACATGTTTATACTGCTTTTT
TTTTTTTTAAGATTTAGAAATTATTTATTGACTTTCTACCGTGGAAGATAAAATTTAAGACCAGGCCAGGCACAGTGTGGCTCATGC
CTGTAATCCCAGCACTTTGGGAGGCCTAGGCAAGAAGATCACTTGAGGCCAGGAGTTCAAGACCAGCCTGGGCAACATAACAAAACC
CTGTCTCTACAAAAAATTTAAAAATTAGCCAGGCATGGTGGCACATGCTTGTAGTTCCAGCTACTCAGGAGGCTGAGGTGGGAGGAT
CATTGGAGCCAGGGAGGTTGAGGCTGCAGTGAGCCATGATTGTAGTACTGCATTTCGACCTGGGTGACAGAGCGAGAACCAATATCT
AAAAAAAAAAAAATTAAAATTAAAAATAAAAAAATAAAAATTTAAGGCCAGCCATGGTGTCTCACACCTGTAATCCCAGTACTTTG
GGAGGCCATGGCAGGAGGATTGATTGAGCCCAGGAGTTCGAGACCAGCCTAGGCAACATAGCAAGACCCTTTCCCTACAAAAAAATA
AGCAAAATTAGAATTGTTCCAGCTACTCAGGAGGCTAAGGTGGAAGGATAGTGTATTAGTCCGTTCTTGCATTACTATAAATAAATG
CCTGAGATTGGGTAATTTACACGTAAAAGAGGTTTAATTTGCTCACAGTTCCACAGGCTGTACAGTCTGCTGCTTCTGGGGAGGCCT
CAGGAAACTTACAGTCTTAGGTAAAGGGAAAGCAGGTACGTCTTACGTGGTCGGAGCAGGAGGAAGAGAGAGAGGGGAGGTGCCACA
CACTTTTAAACAACCAGATCTCGTGAGAACTCACTCAGTATCACGAGAATAGCAAGAGGGAAATCCACCCCCATGATCCAGTGACCT
CCCACCAGGTCCCTCCTCCAACATTGGGGATTGTAGTTGGACCTGAGATTTGCATGGGGACACAGATCCGAACCATATCAGATAGTT
TGAGCCTAGGACGTCGAGGCTGCAGTGAGCCAAGATTGCGCCACTGCACTCCAGCCTGGGCAACAGAGTAAGACCTTGTTTCAAAAC
AAAAACATTAAGACTCATATCTGTTCCCCATTCCCTGTGTACTCCTCCCATCTCTTTAGTTAAATCAGGTTTAGTGCTCATATTAAT
ATTATGCACACTTGGACCATTCTATAGTGACATTTCCTTTCTTGCCTTTTCATTTGCTTAGCTTTGTAATTATCTATAACTAATTCA
GTCCCAGATTCAAACAGAACTGAGATTCTCCTTTTGATCCATGTGAGCACATTGGACATTCTATTAGATTCATTCTTTTTTTCCTCAG
ACATTTCTCCTGGGGTCGTCCCCGTTCCTGGTCTGTTCTAGTTCTGCCACCCAGCGGTGGTGTCAGGACTTGTCTTCACCTCACACT
GCACGTTCCCTTCTCCCCTCCCTTTGACGGAGCCCATGACTCTCACTTTTTGGTTTATCTGCTCATTTTGGTGGAGCTATAATATT
TTCCACCAATTTTCTGAGAAAGGGCACATCAGAGGTAATATTGTTTTAGAAACAACATGTCTGAAAATGCCTTTATTTTTATTTTAA
CCTTTTTTTTTTTTTTTTGTGATAGAGTCTTGCTCTGTCACCCATGCTGGAGTGCAGTGGTGCAATGTTGGCTCACTGCAACCTC
TGCCTCCTGGGTTCAAGAGATTCTCGTGCCTCAGCCTCCTGAGTAGCTGGGATTACAGGTGCGTGCCACCAAGCCCGGCTGATTTTT
TGTATTTTTTTTTTAGTAGAGACGGGGTTTCACCATGTTGGCCAGGCTGGTCTCCAACTCCTGGCCCACTCTGGCCTCCCAAAGTGCT
GGGATTACAGGCATAAGCCACTGTGCCTGGCCATTTTTCTTTATTCTTATGTATTGTTTTGTTAAGTACAGATTTCTAGTTTGGAAA
TAATGTTTCCTCAGATTTCTGTGAGCATTTATTGATTTCCAGCTTCCAGAATTTCTGTGGAATCCAGTGCTATTTTGATTCCCAACC
TTTTGTATAGAACCTGTTTTTTCCATTTATTTTTGGAAACCTTGTGTCCCTTTTTATCCTTATTTAATGGATTTTGGCATGAAATCTT
CTCATTTCATATGAGCCCTTTTATTTTATCAACTTTTGTTCTGGGACATTTTATTATATTACTCCTTTTATCACTTCTTTCTTCCCA
AAGTATTTCAAGTGTTCATTCTCTCTGGAGCATTTATTATTCAGATTTTTGAACTTTCTGGACTGATCCTCTAATTTCCTTCTATTT
TCTCTCTCTTTTTTTTTTTTAATCTCTTGGGCCTTTTGTTCTCCCTTCTAGGTTTTTATTCGCTTTATCCTTAAACCTTTTTGTTGAA
ATCTTTATTTCTACTGGGTTACATAAAAATTACTTTAGATCTCTTTTCTCTGAATATTTAAAACAGTTTTTAAAAATAGCTTCTTGC
CTTATTTCATGGATTTCATATTTTCTCTTATGTCTAAGGATATTCACTCTGGATGTTTAGCCTTGTTTTTTTGTTGCTGTTGTTTTG
TTTGAAAATTTCTTCTATATCTTGTATTATTTATTTGTTCTTCCTACTTTCTTTTCTCTGTTGTTGTTTTTTCCGGCTTCCTGAAATGT
CTGTTGATCCTTGATTGTCCATATTTAAGAGTGATGCACTAAAATGCCAATTGTAAATCTCTAGCTTCTCAGCTGTTTACTATAGGA
TGATCAGGAGGGGATGCAGCCACTTCATTAGAAGATTCTCATATGCCAGCATCTGCTCACTTTTCTCATGGTTTGATCAGTTTCCCT
ACTGAGGAATCATGTCTTCTTCTGCCTTTGGGAGAGTAAGACTTGCTGTCTGTGTTGTGGGAGGCAAGCAGGGCAGGAGGGCTGGGG
AGCTCACCACCTACGTGCCAACTTTTGCTTAATTTTTCTGTTTCTACAAAGTGCCTTATAACTACTCTTAGTGGTGAGAATTAAGAA
TCTGGTTAGTAAATTGCTAATCTTTTGCCATCGTAGAGGAGATGACTTGGGGTCTGATGATTTGAGTCTCCATTGCACTTATCACAG
CTTATAACAGAAGCTTCTCATAGCACTTAAAACAGACATATCTTTTATATAAAATGACTGCCAGTCATCCTGAGCTGAATATAAGCT
TCACACTTTTTCAATGGAATATTCCCTTTGCCTTGACACATAGTAGTTGCTTTTTTGAGCAAATGAATAGGTGAAGCGGGAAGAAAG
GAATTAATCAGAACTTACCACCTTGAACTGTGGCCAATTTTAGTTTTATATATTTTTTTCTCTTTTATACGAATACTTTGTAAATGT
GAATCTCTGCAGCATAAAAATTGGCACTTAAACTCCCTAGACTGTATATTTGTAGAAAGATGTTTGTGATTTAGGTTTTTTCCCCCT
ATTCTTTTCTCTTTGGCTCTGCCTTTTTAAAAATTACTGCTATTTCAAAGAAGTAAATTTAAGCCAAATGTCCTGTCTTAGAATAAG
CAGAGATCTTCTAAGCAGCTTTGCAACATTCGGTATGCAGTCTTTTAGTTTGAAGCCAGAGCTGACAAAAGGGTGGTGATTTCCCTGT
TAAGGTTTGATTCTGATGTTGATTAAGTTGGGGTTTTTTCAGTAAAATTTTGGGCAGACTTGTTGGGGGGTAAATACAATTCTACATT
GATGTGTGGGCCCTTTAGGGGGCGCCATAGTACTTTAGAATCTTATGTGTATTTAGTGTGGTTATGCCAGCCTGGGGGTCTGGAAGA
ATGAATCGTGGCTCCTTCAAAACAGCCTTTCTCTTAAGAAAGGTTCATAAGAAATACTTGATCTTTATGAAATACTTTACTTTCCTA
TTTTAAGAAAATATAATCTGGTTTTTATTTTCTTTAATTCCTTCAAGCTTACGTGGTAAAGTTTACAGTCTCATGCAGTTTAAGGCT
TTTGTTTGACACTTCTACTTGAAATTATTTGACCATTTTGACTAAACTAACTGAAATAATTTTTGAGCACACATATGCAAGACATC
GCAAAGAAAAGCCAAAGATAAATAATTTCTTACAAAAAAGGCCCTGCCCACCAAGAAACTTAGGAGGTAAAATGCAAATGCACAAGTA
GCTATAATGTGTATTAAAACAGAAATGTTCACAGAAGAAAGAGAGAGGTTACTGCCAGTTAGGTATTTAGGGAAGGCTTTTTGAGTT
AGGTAAGAGTTGAGATGGACCTTTTGAGGAAGAAAAGGCTAAGATTTGGGGTCTGCAGCTCCAGCCTGGTAACTAGCATGCAGCCCA
TCTAGGGACCACTCAGGGTTATCTCCTAAGTGGCTCCTTCTGTTTCAGTGTCCTGTGCATTTGCCTCCTCAGCAAGTCCTTTAAGAA
GCATCTGCAGTTGTTGAATGGGGGCTGGGTTGCGACTTGTCCCTGCCTTGGGCGCCAGGTTCATCCCCAGGGATCAGCCCTCTGCTGA
CCTGGGAATATGGTCAGAATGGAGTTGCTGGTCTTGGGAGCAGGCTTGCTCTGGACACAGGTCTAGCCTCTACAGAAAAGCACCATC
ATTCAGGTGTCCGGCCAGCACTGAGGAGTCAAAGGGGGTCTTTCTGAACTTGTATCCAGTGATGTGTTCTCACTCTAGCTTTGATGG
TCCTCAGTGGAGCCAAGACTCCGTATGTTCTAAGAGAATGGCAAAGGCCTCCTTGTCTTGTGTTAGCTACTGGGTAGAATTTTACGT
AGTAACTAGGTAGACACTGATTTAGAATCCAGATGGACCTTATTCTAGAAATAGGAATCTGCCCAAAGGTAGAAAATTGGAATTATT
TGTAAATCAGGAGCTGTAAGAATAATTTATTTTTAATGTAATGGGCAATCATGATATTGTTATGTACATGTTTTTCCACTTTTAGC
CTTTGGAGACTCGACTTATTTCAGAGACCACATCTGTGTGCAAACCAGAACAGGTGGCCAAACAAATTGTTAAAGATGCCATAGTAA
GTAAAATCCTTGTTTTCTGTACTATTTGTGTTTGTAGTACTATATTTCTGAAAAGATATTTGTCCAGGACTTACTTCAAAACATCA
ATTATTAGAATTTTTTAGTCTGTCAGATTTGTCATATCTAATATTGGACTCAACTTTTCTTCTTTTAGACGCTTTATTGACTCCATA
TTAGTGAGTAGCCTCCTGAAAACTGATTGTATACCTTAATCTGAGGAACAGGTTTACTAGAAGGATGTCTGGAAACACCGGTTCTTT
AAAAAGAAAATGAGACTTTCCTATGAGTGTAAACGATATATACTATGTCCCATATACACCTGGCTGCTTTCTCTCTCCCATTCCTGCTT
CCCAGTATCATTTTCCCAAAAGTTAGCTAACGAGAAAGTGCCACCAGCCCCGTGGGCCTCCTCCTGTCTTCCCTGTTGATATGTT
TTTTAATTCAGAATCAAAAATGGTATTTTAAGAAATGATGTGCTGTTTTGTAGAAGAACAACATATGTGCCTCATGCTTTTTGACAA
CATTTCTGTTAATGCGGAAAAATGCACATTTATGAGGGATGTCTGTAAACCACAGACCCACACTGAATTTGGAAAATCTATAGGACC
ATTGGAATGAGAGGTGTCAGCACATGGTGGACAGCCTTGAAGTGGCCTAAATGCTGTCACATTAGACTGAACTGAGCAGAATGAGAA
TAGTGAATTCTGTTGAAATATATACATGTGTGAAGAACTTTGTCCTGAGTGCATTAAAAGGCTCAGGAAGATGGCACAAGTGAGACT
TTATTTTTCCTGCTTATCTCGGGTCAAAGCAAATGCATTTGGTGTTTTTTGTTGTTTGTTGTTTGTTTGTTTGTTTGTTTGTTTGAG
ACGGAGTTTTGCTCTTGTTGCCCAGGCTGGAGTGCAGTGGCACGATCTTGGCTCACTGCAACCTCTGCCTCCTGGGTTTAAGTGATT
CTCCTGCCTCGGCCTTCTGAGTAGCTGGGATTACAGGTGCTCACAACCACACCTGGCTAATTTTTTGTATTTGTAGTAGAAATGGGG
CTTCACCATGTTGGCCAGGCTGGTCTCGAACTCCTGATTTCGGGTAATCCGCACGCCTCAGCCTCTTGGCCAAAGTGCTGGGATTAC
```

```
AGGCGTGAGCTCCGTGCCCAGCCCATTCAGTGTTTCTAATGATGCTAAATAGCTCTTAGTATTTTGGCAGTATTCATTTAGTTTAGT
GGTTTTCAAACTTTTCTTCTCTTGACCACTTTACATTCTTACAAATTGTCAACAACCCCAGAGAACTTTTGTTTATTTACATGGCTA
TATTTATTGATATTTGCCATATTTGAAATGGAAGCTGTTTGTAATATTTATTGATTCATTAAAAAATAACAAAAATGGCTGGGCACAG
TGACTCGTGCCTGTAATCCCAACACTTTGGGAGGCCAAGGCGGGCGGATCACCTGAAGTCAGGAGTTCAATACCAGCCTGACCAATA
TGATGAAACCCCGTCTCTACTAAAAATACAAAAATTAGTCGGGTGTGGTGACATGCGCCTGTAATCCCAGCTACTTGGGAGGCTGAG
ACAGGAGAATCGCTTGAACCCAGGAGGTGGAGACTGCAGTGAGCTGAGATTGAGCCATTGTGCTCCAGCCTGGGCAACAAGAGCAAA
ACTCCATATATATATATATATATATATATATTCACATATATATATATATAAAGTCACATATATATATATATAAAGTCACATATATATAA
AGTGACTTTTTTTAAACAAAATTTACTACTTTTCAAAACAAAAGTTCAGTGAGGACGGTGGCATTGGTTCACATTTTCACCAGTTGC
TTTAAGGTCTGCATTAACAGCTGGATTCAAATAGCTGCTTCTGCATTCCATCTGTTGTAATAATGTTGTTTTGATGGAAGGATATAC
AGAAAATCTGGCCTGAGCACAGATACGGAGTTGGGAAAGGGAAGACTATTTTACTAGGCTTTTCAGATAATTATGGACATTCTTTTTT
GATATCAAACCCAAACTCAACAAATGGTAGTTTCTTAAAGGTTAGTTGCAGTATAGAATTGGAAACTACATCAATGAATGTTTTGTT
CTCTGTTACATTAAATTCCATTGGTCCGTCTTGTACTTTGAATGGATCTTTTAACCATTCATGATTTCGTAACATTATGCATTAGTC
ATTTGGAAAATATGGGTTGACTGAGTGATCAGAAAAATCGCTTTCATCAGTATTACTGCTGATCTTATCATAAAAGTATGCAGCTGA
CAGTCTCGTGATGGTGGATACTAGTTTTTCCAAATCTTAATTTTTTCTTGAAGGCTCAGATTTCACATTGATGACACGTGGCCAGTT
GTTTTTCCTGAAGTGACACACTTGCTTGCTTGCGCTTTCTGCACATCTGCCACATACCTAAGTCTGAATTACTATAGATTTCCTGTCA
ATGGTCTTTCATATAAAAATTATGCTCTCCAGCCAGGCACGTGGCCATGCCTGTAATCCCAGCACTTTTGGGAGGCCGAGGCGGCT
GGATCGCTTGAGCTCAGGGGTTTGAGACCAGCCTGGCCAACATGGTGAAACCCCATCTCTACTAAAAATACAAAAATTACCCGGGTG
TGGTGGTGCATGCCTGTAATCCCAGCTACTTGGGAGTCTGAGGCAGGAGAATCACTTGAACCTGGGAGGCAGAGGTTGCAGTGAGCA
GAGATCACACCACTGCATTCCAGCCTGGGCCACAGAGTGAGATTCTGTCGAAAATAAATAAATAAACAATACAAATGATGCTCCATG
AAAGAAGTGGCAGTTTAGCCCTTGACTTCAGGAGCTGCACAGGCACTTTGTTCCAGGCAGTTGTCCCGCCCTGGAATGCAGTGCTG
CGGAAGCGCGTTGGGCGCATTCTCATTTGTCACACAGAGTATTAAGAAGACATGTCCTCGAGGGTTGCCATTGAACAAAATGATGAA
TTTTCCTGCTTTATCAAGAGGATTTTAAAGCAAAACTGACACTTTGATTTTTAAAAGAATTTTAGAACTTGTATTTTCTTTGTGTAT
TATTTATTACTGCGAGTGCGTGTGGTAGTAGAGGTGACAATGACTCCTAGTACAGTTTGGCAAGAACCCCGTGGTTCTTGCCCATGT
CTCTGCAGTTTTGCAGAGGCAAAACTGTATTTTCCATACAACACACTGTGGAAAAGACAATGAATGTCTTAGTGTTATTAGGACAAT
CATTTGTCCTTGGGGGCCCGCAGGGGTCCGTGGACTCACTTGTTGAGAACTGCCATCTGCCAAGGCCGTGCGTCCATTTCTGAGTGC
TGATCAATGCTTTGTGACCATTTTATCTCACTTAAAGTGAACGAGCACACTCCACTGTTTCTGATGTGTCCGACTGATTTCTGTCCA
CATTACTAGGATTCCCTCTTTCTCTTCTTTTTCTTCTCTTTTAGCAAGGAAATTTCAACAGTTCCCTTGGCTCAGATGGGTACATGC
TCTCGGCCCTGACCTGTGGGATGGCTCCAGTAACTTCTATTACTGAGGGGCTCCAGCAGGTAAGCTCTGCCTAGGCTAGCAATCAGA
CAGGCCGATGGACAAGAGAAAAACTGCCTTTCATCAAACCTTGATGAGAAGAGGTTAAGGGAAAACTAAGTAACTTTATTTGACAAT
GTATTTTTATGGAGCTATCAACTTTTCCTCTTCCTCCACCAGGAATGAGTAAATAGGTTAAAGATATAACTTCAGGAATTTAGAATG
GCAAGAAGTCTTCAGTGCCGGGCCTTGCAGATAGAGAAATAAAACACCGTATCTGCTGTTGAGGTGTTAACCTGGATTTTCACCTAA
GAACCACTGCTCCAATGTGTTTTGAAAATGGAATACTCCTCTAGAGTAAGGGGTAGCCTTGCTTAAAAAGTACTGGGATATTAGACT
AGAAAACCTTTTCTAGGTGAATCAGGTCACACACAGCTAAATTTAGTGAGACACGAATCATGGATTAAACAGTTGCTTCGCTTATAG
AATGTATTATTTTGTCCAAGATACACAGTGTTTCATTGGTGTGCTAGGCACTGTACTTGCTCATGGGGAATCAGATGGAAGGCGACC
TCTCCAGCTTCAGGGGCATACAGTCAAGTGGAATGCACTGCACCAAAAAGGTGATAGTAGTTCCGGGCGCGGTGGCTCACGCCTGTAAT
CCCAGCACTTTGAGAGGCTGAGGTGGGTAGATCACTTGAGATCAGGAGTTCGAGACCAGCCTGGCCAAGATGGTGAAACTGTTTCTA
CTAAAAACACAAAAATTAGCTGGGCATGGTGGCGGACGCCTGTAATCCCAGCTACTCGGGAGGTTGAGGTAGGAGAATTACTTGAAC
CCGGGAGGTGGAGGTTGCAGTGAGCCGAGATCAAGCCACTGCACTCCAGCCTAGGCTACAAGGGCAAAACTCTGTCTCAAAAAAAAA
AAAAAGATAGTAGTGCAGTGTAATGAAGTAGGGGTTACAATAGAGGGAATGCTCTGGAAAGACAAAGCCATGGGATAGTTGTTAAC
AAGTACTAAATTCTGTGCAGTCAACCACATTCAGACTTATATAAATGACTTAGTTGTTGGTTTTCTTCAGGGTCTTTCCCAAGCCCT
CTTTTCTTTTCATTCTGTAATGTACCTACTGTCTTTGCCATTCTCTTAGAGGTTGACACTGTGTTAGATGTAAGCTCTTTGAGGACC
AGGCCCACACCTCTTGTTTGCCGTTGTACCCCTAACAGCCAGCGCCTGGCACTTAGTAGGAACTCCAGAATCCCTGAGCTGAACAAA
TGCATGAATTCTTTTATGTTTTAATTTTTGGGCTGGCATGGTGGCTCACACCTGGCGTGTTGTCCTGGACTCCCTGCTTCTGTCCC
TCACGTCAGAGATCATTGTCATCCTTGTCACTCAGTTCTTCACATTCTTCCATTCTCTGCAATTCTGGCAGTGCTTCTGGAGCCCAG
GCCTGGGTTGCCGCACGCAGGCCCCCTGCTTGAGTTACACACTTTTTGAGCCAGAGGTTGTAAACGATTCCCCATGGGCCTGTAAAGCA
GTTTTGTTGGGTCCATCTGGTGTGAGTGTGTGTGTGTTTAAGATTTTAAAATAGTTTTAAAATTAGGTTTTATATAAAAGGTCCAGATT
TCAGACTTTTCTTGGAAAATCAGAAGGTTTAGCTGCACAGAGTCTGCATTCTTACCTGGCAGTCATCAGCAAGCACAGGTGGCAGCT
GCTCCTGAGGGAGGAGCACAGTCCCTGCAACCACTATGGTCTGCAACCCCCTGCTCAGGCCCCCTGGATGCGTTTGAGTGTACCCTC
ACATTTTCCACACCAGTTGTTTCTAAACTGCAACTATACATTGCATGAAAACATTCAATGACTCCTTGATACCATCAGGATAAAGCT
TTAAAAATTCCATTGCACCTGAGTATGGTGGCTCATGCCTATAATCTCAGCACTTTGGGAGGCCAAGTCAGGAGGATCGCTTGAGCT
CAGGAGTTTGAGATCAGCCTGGGCAACATAGTGAGACCCTGTCTCTACAAAAAAATAGTAAAAGAAAAAGAAAGCCAGGCGTGGTGGTG
TGTACCTGTATTCCTAGCAACTGGGAGACTTCTGCTTGAGTCCAGAAGTCTGAAGCTACAGTGAGCTGTGATCATACTCCTGCACTC
CAGCCTGGGTGACAGAGCAAGACCCTGTCTCTAAAAAGGATTTAAAAATTAAAAAAATTTCCATCGCATGCCATAAACCACCTGATC
TCCTACCCCACGTACCTTTCCAGGCTCTTTTTTTTTCTTTCTTTCTTTACCCATCTTCTTCTTTTTTGGAATCTTCTCTGTGTCCTGCC
AACTTCTAATCCTTCAGGTCTCACCTTAAATATTGTTTTTTCCGGAAGATCCAAGGACTGGCTTCAGTGCTCTGAGGGTGTAGGGAA
AAAAACAAAGGGATTTTTCTATTCTCTTTTTTTTTTTTTTTTTTTTGAGACGGAGTCTTGCTCTGTCACCCCAGGCTGGAGTGCAG
TGGCACGATCTCAGCTCACTGCACGCTCTGCCTCCTGGGTTCACACCATTCTCCTGCCTCAGCCTCCCCAGCAGCTTGGACTACAGG
CGCACACCGCCACACGCGGCTAATTTTTTTTGTATTTTTAGTAGAGATGGGGTTTCACCGTGTTAGCCAGGATGGTCTCAATCTCCTG
ACCTCGTGATCCACCCGCCTCGGCCTCCCAAAGTGCTGGGATTACAGGCGTGAGCCACCGCGCCCGGCCTGATTTTTCCATTCTCTT
ATGCACGGGGACTCATGCCTATGGTCCCAGCTGCTTGGGAGGATGAGGCAGGAGAATCCTTTGAGTTTGGGAGGTCAAAGCTATAGT
GAGCCGCGTTCATGCCACTGCACTCCATCCTGAGCAACAGGATATCAGTAATCACGGGTCTCAACCCTGAGGCAGTAGTTTCAATTT
TACTTGTCTTTCCAGCCTGAGAATAAGCTCATTGAGGGCAGGGACTGTGTCTCTCACCACAGTGTTCACAGCACCTAGTATAGGGTC
TCGCTTTCATGCTCAGAACATAAATTGCAAACCAAAAAAGCCTTGATTGTAGTTTGGCTATTTGAGATGCTGCAGTGGGTCTTTTTA
AAGCTATTATAACCCTAAGACTGCAAGCAATATATATTTTATTCTGCTTATGGTTTGAATTTATTAATTGGACAAATATTTTGTTAT
GTATCTGGGCTGTCCAGCGGTGCAGAGCACACACATTCTGTTCTCACAGCTTACTGACATTTACAGAGTTTTCATTATATTCATCCT
TAGAGCAGACTAGAAGATTTGTCTTAGAAACTGTTTGTTAGAGACCTAGGTACCTTGAAATGATCTTGTACCGTTGGATGCACTAAA
AGCTCTCTCTTTATCTTGCAGGTGGTCACCATGGGCCTTTTCCGCACTATTGCTTTGTTTTACCTTGGAAGTTTTGACAGCATAGTT
CGTCGCTGCATGATGCAGAGAGAAAAATCTGAAAATGCAGACAAAACTGCCTAATCTTCTTACCCCTTGGAAGAAGACTGTTTCCAA
ATAATTTGAACAGCTTGCTGCTAAATGGGACCCAATTTTTGGCCTATAGACACTTATGTATTGTTTCGAATACGTCAGATTGGACC
AGTGCTCTTCAGGAATGTGGCTGCAAGCAAGGGGCTAGAAGTTCACCTCCTGACAGTATTATTAATACTATGCAAATATGGAATAGG
AGACCATTTGATTTTCTAGGCTTTGTGGTAGAGAGGTGAAGGTATGAGAATTAATAGCGTGTGACAAAGTAAAGAACAGGATTCCA
GAATGATCATTAAATTTGTTTCTATTTATTCTTTTTTGCCCCCCTAGAGATTAAGTCCAGAAATGTACTTTCTGGCACATAAAGAAA
TCTTGAGGACTTTGTTTAAACCTTCCATAAAAAAACAATTTTCGGTTTCTCGGGTTCTCTCTCTCTCTCTCTGTCTCTCTGTCTC
TCTGTCTCTCTGTCTCTCTGTCTCTCTGTCTCTCTCTCTCTCTCTTTCTTTCTTTGTGTATTTTATTCAAGATGAGTTGGACCCA
TTGCCAGTGAGTCTGAATGTCACTGACAGCCCTGTGTTGTGCTCAGGACTCACTCTGCTGCTGGTGGAAACTCATGGCTTCTCTCTC
TCTTTGATCCCATAAAGCTACGAGGGGGACGGGAGAGGGCAGTGCAATGGGAAGTAAAGAGATATTTTCCAGTAGGAAAAGCAATGC
```

54

```
TTTCTTGTCTTTAGACTCAAATGCTTAGGGAACGTTTCATTTCTCATTCATGGGGAAAGGCAGCCTCCTTAAATGTTTTCTGAAGAG
CGGTAAAATCTAGAAGCTTAAGAATTTACAGTTCCTTCAATAACCATGATGACCTGAAGTTCACCTATCCCATTTTAGCATCTACTT
GTTTTTCCCATCTCTTCCTTTCCAATTTTGCTTATACTGCTGTAATATTTTTGTAAAAAAAAAAAAAAAGGAAAAAAAAGACCAGCT
AAAATTTTCGACTTGACTTTTTAACTTAACTCATGAATTAATTAAAGCAAATGAAAAAATTAAAAAGTGTGACTTTTTCTCGGAGCA
TATATGTAGCTTTTAGGAAAGGCTGATGATGGTATAAAGTTTGCTCATTAAGAAAAAAAGACAAGGCTGATTTTGAAGAGAGTTGCT
TTTGAAATAAAATGATCACCTGTTCTTTATGTGACTCTCCCACTGAACCTGCAGACATTATTTTTATACCACATGCTAAGGAAGCCC
ACTCATCTAACTCTGTAGCCCTG
```

HUMAN mRNA SEQUENCE : hR22-022.1 (Seq ID No: 7)
```
GGCTCCTCCTCTTCCCCTCCGCCGCGGCCCGCCCCGGCCCGCAAACCCAAACACTCCAGGCGCCCGCCCGCCGCGCGTGATTCTCGC
CTCGCCGCAGCCCAGCCCTGCGCGCCTTGCCCGGCGGCCCCCGCCCGGCCGCTCCGGGCCCCTGGCCCCGCGGAGCGATGCTGCTGC
TGGCTGCCGCCTTCCTCGTGGCCTTCGTGCTGCTGCTGTACATGGTGTCTCCGCTCATCAGCCCCAAGCCCCTCGCCCTGCCCGGGG
CGCATGTGGTGGTTACAGGAGGTTCCAGTGGCATCGGGAAGTGCATTGCTATCGAGTGCTATAAACAAGGAGCTTTTATAACTCTGG
TTGCACGAAATGAGGATAAGCTGCTGCAGGCAAAGAAAGAAATTGAAATGCACTCTATTAATGACAAACAGGTGGTACTTTGCATAT
CAGTTGATGTATCTCAAGACTATAACCAAGTAGAGAATGTCATAAAACAAGCACAGGAGAAACTGGGTCCAGTGGACATGCTGGTAA
ATTGTGCAGGAATGGCAGTGTCAGGAAAATTTGAAGATCTTGAAGTTAGTACCTTTGAAAGGTTAATGAGCATCAATTACCTGGGCA
GCGTGTACCCCAGCCGGGCCGTGATCACCACCATGAAGGAGCGCCGGGTGGGCAGGATCGTGTTTGTGTCCTCCCAGGCAGGACAGT
TGGGATTATTCGGTTTCACAGCCTACTCTGCATCCAAGTTTGCCATAAGGGGATTGGCAGAAGCTTTGCAGATGGAGGTGAAGCCAT
ATAATGTCTACATCACAGTTGCTTACCCACCAGACACAGACACACCTGGCTTTGCCGAAGAAAACAGAACAAAGCCTTTGGAGACTC
GACTTATTTCAGAGACCACATCTGTGTGCAAACCAGAACAGGTGGCCAAACAAATTGTTAAAGATGCCATACAAGGAAATTTCAACA
GTTCCCTTGGCTCAGATGGGTACATGCTCTCTGGCCCTGACCTGTGGGATGGTCCAGTAACTTCTATTACTGAGGGGTCCAGCAGG
TGGTCACCATGGGCCTTTTCCGCACTATTGCTTTGTTTTACCTTGGAAGTTTTGACAGCATAGTTCGTCGCTGCATGATGCAGAGAG
AAAAAATCTGAAAATGCAGACAAAACTGCCTAATCTTCTTACCCCTTGGAAGAAGACTGTTTCCAAATAATTTGAACAGCTTGCTGCT
AAATGGGACCCAATTTTTGGCCTATAGACACTTATGTATTGTTTTCGAATACGTCAGATTGGACCAGTGCTCTTCAGGAATGTGGCT
GCAAGCAAGGGGCTAGAAGTTCACCTCCTGACAGTATTATTAATACTATGCAAATATGGAATAGGAGACCATTTGATTTTCTAGGCT
TTGTGGTAGAGAGGTGAAGGTATGAGAATTAATAGCGTGTGAACAAAGTAAAGAACAGGATTCCAGAATGATCATTAAATTTGTTTC
TATTTATTCTTTTTTTGCCCCCCTAGAGATTAAGTCCAGAAATGTACTTTCTGGCACATAAAGAAATCTTGAGGACTTTGTTTAAACC
TTCCATAAAAAAACAATTTTCGGTTTCTCGGTCTCTGTCTCTCTGTCTCTCTGTCTCTCTGTCTCTCTGTCTCTCTC
TCTCTCTCTTTCTTTCTTTGTGTATTTTATTCAAGATGAGTTGGACCCATTGCCAGTGAGTCTGAATGTCACTGACAGCCCTGTGTT
GTGCTCAGGACTCACTCTGCTGCTGGTGGAAACTCATGGCTTCTCTCTCTCTTTGATCCCATAAAGCTACGAGGGGGACGGGAGAGG
GCAGTGCAATGGGAAGTAAAGAGATATTTTCCAGTAGGAAAAGCAATGCTTTCTTGTCTTTAGACTCAAATGCTTAGGGAACGTTTC
ATTTCTCATTCATGGGGAAAGGCAGCCTCCTTAAATGTTTTCTGAAGAGCGGTAAAATCTAGAAGCTTAAGAATTTACAGTTCCTTC
AATAACCATGATGACCTGAAGTTCACCTATCCCATTTTAGCATCTACTTGTTTTTCCCATCTCTTCCTTTCCAATTTTGCTTATACT
GCTGTAATATTTTTGTAAAAAAAAAAAAAAAAGGAAAAAAAAGACCAGCTAAAATTTTCGACTTGACTTTTTAACTTAACTCATGAAT
TAATTAAAGCAAATGAAAAAATTAAAAAGTGTGACTTTTTCTCGGAGCATATATGTAGCTTTTAGGAAAGGCTGATGATGGTATAAA
GTTTGCTCATTAAGAAAAAAAGACAAGGCTGATTTTGAAGAGAGTTGCTTTTGAAATAAAATGATCACCTGTTCTTTATGTGACTCT
CCCACTGAACCTGCAGACATTATTTTTATACCACATGCTAAGGAAGCCCACTCATCTAACTCTGTAGCCCTG
```

HUMAN mRNA SEQUENCE : hR22-022.2 (Seq ID No: 9)
```
GGCTCCTCCTCTTCCCCTCCGCCGCGGCCCGCCCCGGCCCGCAAACCCAAACACTCCAGGCGCCCGCCCGCCGCGCGTGATTCTCGC
CTCGCCGCAGCCCAGCCCTGCGCGCCTTGCCCGGCGGCCCCCGCCCGGCCGCTCCGGGCCCCTGGCCCCGCGGAGCGATGCTGCTGC
TGGCTGCCGCCTTCCTCGTGGCCTTCGTGCTGCTGCTGTACATGGTGTCTCCGCTCATCAGCCCCAAGCCCCTCGCCCTGCCCGGGG
CGCATGTGGTGGTTACAGGAGGTTCCAGTGGCATCGGGAAGTGCATTGCTATCGAGTGCTATAAACAAGGAGCTTTTATAACTCTGG
TTGCACGAAATGAGGATAAGCTGCTGCAGGCAAAGAAAGAAATTGAAATGCACTCTATTAATGACAAACAGGTGGTACTTTGCATAT
CAGTTGATGTATCTCAAGACTATAACCAAGTAGAGAATGTCATAAAACAAGCACAGGAGAAACTGGGTCCAGTGGACATGCTGGTAA
ATTGTGCAGGAATGGCAGTGTCAGGAAAATTTGAAGATCTTGAAGTTAGTACCTTTGAAAGGTTAATGAGCATCAATTACCTGGGCA
GCGTGTACCCCAGCCGGGCCGTGATCACCACCATGAAGGAGCGCCGGGTGGGCAGGATCGTGTTTGTGTCCTCCCAGGCAGGACAGT
TGGGATTATTCGGTTTCACAGCCTACTCTGCATCCAAGTTTGCCATAAGGGGATTGGCAGAAGCTTTGCAGATGGAGGTGAAGCCAT
ATAATGTCTACATCACAGTTGCTTACCCACCAGACACAGACACACCTGGCTTTGCCGAAGAAAACAGAACAAAGCCTTTGGAGACTC
GACTTATTTCAGAGACCACATCTGTGTGCAAACCAGAACAGGTGGCCAAACAAATTGTTAAAGATGCCATACAAGGAAATTTCAACA
GTTCCCTTGGCTCAGATGGGTACATGCTCTCTGGCCCTGACCTGTGGGATGGTCCAGTAACTTCTATTACTGAGGGGTCCAGCAGG
TGGTCACCATGGGCCTTTTCCGCACTATTGCTTTGTTTTACCTTGGAAGTTTTGACAGCATAGTTCGTCGCTGCATGATGCAGAGAG
AAAAAATCTGAAAATGCAGACAAAACTGCCTAATCTTCTTACCCCTTGGAAGAAGACTGTTTCCAAATAATTTGAACAGCTTGCTGCT
AAATGGGACCCAATTTTTGGCCTATAGACACTTATGTATTGTTTTCGAATACGTCAGATTGGACCAGTGCTCTTCAGGAATGTGGCT
GCAAGCAAGGGGCTAGAAGTTCACCTCCTGACAGTATTATTAATACTATGCAAATATGGAATAGGAGACCATTTGATTTTCTAGGCT
TTGTGGTAGAGAGGTGAAGGTATGAGAATTAATAGCGTGTGAACAAAGTAAAGAACAGGATTCCAGAATGATCATTAAATTTGTTTC
TATTTATTCTTTTTTTGCCCCCCTAGAGATTAAGTCCAGAAATGTACTTTCTGGCACATAAAGAAATCTTGAGGACTTTGTTTAAACC
TTCCATAAAAAAACAATTTTCGGTTTCTCGGGTTCTCTCTCTCTCTCTCTGTCTCTCTGTCTCTCTGTCTCTCTGTC
TCTCTGTCTCTCTCTCTCTCTCTTTCTTTCTTTGTGTATTTTATTCAAGATGAGTTGGACCCATTGCCAGTGAGTCTGAATGTCA
CTGACAGCCCTGTGTTGTGCTCAGGACTCACTCTGCTGCTGGTGGAAACTCATGGCTTCTCTCTCTCTTTGATCCCATAAAGCTACG
AGGGGGACGGGAGAGGGCAGTGCAATGGGAAGTAAAGAGATATTTTCCAGTAGGAAAAGCAATGCTTTCTTGTCTTTAGACTCAAAT
GCTTAGGGAACGTTTCATTTCTCATTCATGGGGAAAGGCAGCCTCCTTAAATGTTTTCTGAAGAGCGGTAAAATCTAGAAGCTTAAG
AATTTACAGTTCCTTCAATAACCATGATGACCTGAAGTTCACCTATCCCATTTTAGCATCTACTTGTTTTTCCCATCTCTTCCTTTC
CAATTTTGCTTATACTGCTGTAATATTTTTGTAAAAAAAAAAAAAAAAGGAAAAAAAAGACCAGCTAAAATTTTCGACTTGACTTTTT
AACTTAACTCATGAATTAATTAAAGCAAATGAAAAAATTAAAAAGTGTGACTTTTTCTCGGAGCATATATGTAGCTTTTAGGAAAGG
CTGATGATGGTATAAAGTTTGCTCATTAAGAAAAAAAGACAAGGCTGATTTTGAAGAGAGTTGCTTTTGAAATAAAATGATCACCTG
TTCTTTATGTGACTCTCCCACTGAACCTGCAGACATTATTTTTATACCACATGCTAAGGAAGCCCACTCATCTAACTCTGTAGCCCT
G
```

HUMAN PROTEIN SEQUENCE : hP22-022.2 (Seq ID No: 10)
```
MLLLAAAFLVAFVLLLYMVSPLISPKPLALPGAHVVVTGGSSGIGKCIAIECYKQGAFITLVARNEDKLLQAKKEIEMHSINDKQVV
LCISVDVSQDYNQVENVIKQAQEKLGPVDMLVNCAGMAVSGKFEDLEVSTFERLMSINYLGSVYPSRAVITTMKERRVGRIVFVSSQ
AGQLGLFGFTAYSASKFAIRGLAEALQMEVKPYNVYITVAYPPDTDTPGFAEEENRTKPLETRLISETTSVCKPEQVAKQIVKDAIQG
NFNSSLGSDGYMLSALTCGMAPVTSITEGLQQVVTMGLFRTIALFYLGSFDSIVRRCMMQREKSENADKTA*
```

## Table 2

```
MOUSE GENOMIC SEQUENCE : mD27-007 (Seq ID No: 11)
CAAGTTACACTCAACTGTTTTAGAAGAGCAGTTCCCCAGATTTCTCCTTGGAGCTGTGAGTGACTACCATTGCGAGCAAGAGCAAGA
GGAAAGCACTACCTGTGAGCAGATGTCTGGTTCATTCTCACCCTGTGTGGTGTTCACACAGATGTGGCTGACTCTACTGGTTGTGAG
TGAGTAGATGGCTCAATATGGGGATAGAGTGGGTCGGACAGCATCTCTCTCCACTACAGGCCTGGTACTCTCTTAAGTTTTAGCTCC
TCAGTTTCCCCGTGTGAGGCTGTGTTCCCCGACTTTACTGTACAGCTTAGAATTTCCTCTCCTGAGCCCAAAGAAAGTAGTCACAGG
TTGAAAAAAAAAAATCCTTCAATGGACAGAGGAAGAAACGGTAGCAATGGCACTAGAGTTTCGCTTGCTTTCTTTTCTTTAACAAAT
ACTTTATGAAGCACAGAAAGTAAATCACACTTGCTTGTTCAAACTCTTTTATTCAAATGAGAACTATATTTTAATCCCGTGTAGACA
GGCTAGACCCTGGCCTTGTGCCTAAGATGATAGCTCCTTCTGCACATTAGCGAGAGCCATGAGCAGGACTGGGCGCCTTAAGTACAA
GAGGGGAAATCAGGAAGGAGAGGTACCTACAGCAGTGGCTATGTCACTGCCCATGGATATGCATGCATTTCTGTCAGTCAGTCATGCCTA
CAAAGGGTTTGCTTCTTACTAAATTTTAAATGGAATTAAATGTGGGTTCATCTATTTTTAAAAAGTTCTTAGGCCGGGCAGTGGTGG
CGCATGCCTTTAATCCTAGCACTTGGGAGGCAGAGGCAGGAGGATTTCTGAGTTCGAGGCCAGCCTGGTCTACAGAGTGAGTTCCAG
CACAGCCAGGGCTATACAGAGAAACCCTGTCTTGAAAAACCAAAAACAAAAACCAAACAAACAAACAAACAAAAGGTTCTTAGCGC
CATCTATGCACAGCATTGTATGCAATGCTGAGGACGGAGGGTTGTTTTGTGCCCACAAGGGAAAACACCAAAAAAAAAAAAAAAAAAA
GCTCTCAGCAAAGCCAGTGCACAAGAAACAGCTGGGAAACACAATTCTAGTAAAATAAAATCCCAGTGGATGGCACAGACAGATAAC
CATAACCATCTACATACCAGTCAGGTGATCAGACTAAGCAACAGATAGGGCAAGGCCCATCAGAAACAGAAGCATATCCAAACTTAA
GAAGTACTTAAGAAGGCAGGGGAAAGGAGGGAAGAAGAAGGGAGGGGAGGACAGGACAGGACAGGAAGGTGTTCATACAGTGTGCTG
TGAGCTAAGGTTAGAGTTTGGATTCAAATCTGTGTTATCACCTACTAGCCACTTAACAAGCTTTCTTCTTTCTTTTCTTCCTTCCTT
CCTTCCTTCCTTCCTTCCTTCCTTCCTTCCTTTTTTCTTTATTGAAAATAGATTTTTTTCTTACATAATACATCCTGATTATTTCCC
TCCCTCTACTCTTCCCACTTTCTCCCCACCTCCCCTACCATGTGAATCCACTCTCTTTCTGACTCTCATTAGATAACAACGGGCTTC
TAAGAGATAAGAACAAAAATATAATAAGTAAGATAATATAAAAACTATCACATCCAAGTTGAACAAAACAAACCAACAGAAAAAAA
GGAGTCTAAGAGCAGGCACAAGAATCAGCCCCACTCGTTCTTATGCTTAGGGCTCCCATTAAAAGCACAAACCTGGAAGCCATAATA
TATGCTCAGAGGACCTGGTGCAGATCCATGCAGGCCCTGCGCTTGCTGCATCAGTCTCTGTGAGTCCTGGTAACCTCTGCTTATGTT
GATTTAGAGGGCCTTATTTCCCGGTGTCCTCCGTCCCTACTGGTTCCCTTTCCTGAGCTCTGAGGGGAGAGNNNNNNNNNNNNNNNN
NNNNTCTCTCTCTCTCTCCCCCCCCCCCCCAATAATGTCAGGTCCTCTGGTTTCGTTCCCATCTACTGCAGGAGGAAACTTCTCT
GATGATGGCTGAACAATGATCTACAAGTGCAGCAGCATATCATTAGCAGACACTCACTACATTTCTATCTTGTTTTTTAACACTAGT
AATACTTGGTGCTTTCATTGAGACCTCCTCCATATATGTATATATTTTAGGAAGTTTCTACTGCATTAATTTTCCATACTAACCCTC
AAATACTCCTTAACTTTAGCTGTCTCTCCCTGAGTTCCCTCACTCAGTTCCCACCCCTCCCTCCCCATGAGATCCTCCCATCCCAGC
CTCTCTCCATCCATCCGTAATTATATATTCTGTTTCCCTTTTCTAATGAGATCTATCTGTTCCCTCTAGTCTCATCCTCTATACC
TACCTTCTGTGGTTCTAGGGATTGTAACTTGGTTATCATTAACTTAACAACTAATATTCACATATTAGTGACTATATACCATATCTG
TCTTTCTGGGTCTGAGTTACCTCACTCAGAATGATCTTTTCTAGTTCCATCCATTTGACTGCAAATCATAATGTAGGGTTTTTTTTT
GTTTTGGTTTGGTTTGGTTTGGTTTTGTTTTTTGTTTTTAATGGCTGAGTTATACTCCATTGTGTAAATGTACCATATTTTCTTAA
TTCATTCTTCTGCTCTGTTGAGAGACATCTGGGTAGTTTTCTGACTATTATGATAAGAGCAGCAGTGGACATAGTTGAGTCTTTGTGG
TACACTGAAGTGTCCTTTGAATATTCCCAAGAGGAGTATAACTGGATCTTACAGTAGATCCAGTCCCATCTTCCTGGGGAACTGCAA
CACTGATTTCCATAGTGTCTGTACAAGTTTGCAGTCCACCAGCAATGGAGGAGGAGTCTTCTGCTTATTAAATATCCTTGACAGCAT
GGACTGCCACTTGAGTTATTGATCTTAGCCATTCTGACAGGTATAAGATGAAATCTCAAAATAGTTTTGATTTTCATTTCCTCGTTG
GCTAAGGATGTTCGACATTTCTTTAAGTTCAATCAGCTTTCTTGTCTATGATATGGGAACTTAAGTTAGAATGTTTCTGACAAGATTA
TATAGAATGACATGAAAATGCATACCAAATAGAAGCCCTCAAATACAAAAGCCATAATAACCATGATATGTAGGTAACAGTGACTAA
TTATTTGGTCTCAATAAAAATAAAATAAAATAAAATAAAATAAAATAAAATAAAACAAAACAAAACAAAATAAAATAAAATAAAATA
AAATAAAATAAAACTTGGATTTAAACTGTTCTTCTATCATTTCCAGCAGCAGGGACGATATGTCCCTCACTCACAGCTGGATTTTT
ATTGCATTTCCCAACAAATGGCTGGCATTCCAGAAATTTCTTTTAAGTGAGAGGATCAAATCCATCCTTCATCTAAATAATTCATAA
ATTAATAACCCTGCTGAGCTCTGCCTACTTCCTCGCCTCAATACTCGTGTCTCCAACCTGACCGATTTCTACTATTGCAACACTAGG
GTTTTGCCTTTCTGGGTTTTGGAAAGGTGTTTGTTTGTTTGTTTGTTTAATGATTCATTACTCAAGAAAGCTTACAGCTCTCCCATTGCA
TAGCAGTCCTTACAGTCTAGACCAGGCTTTAGGAATGAGCAGCAAACACACACCAGTTCAGATAATCCTGGATAAACAACTGAAGG
AAGCTTGCCATTGCCACCACCCTGATGTGCTATTTCCTGTCTTCTCCATGGAAGACTTCTCTCTGCCGGAATACTTCATCCAAGCCT
TAAGTTCTGACTTCACCGCACTCCTTGGACTCTTTCTATAGTTCCCCGATGGCTTCTATAAGAAAAGAATGGCCCCCGCATTCTTCC
CCAACCATGTCTGTCCCACTACTCTTCACCCTTCAGAACTGCTCAGTCCACTTGGTGACACCAATGTCATCCCTTATTTCTGTGCCC
TTCTCACCTTAACCACCCTCTCTCTGTGTTCTCATGCCAGCACAGCGTCAACCTTCACTTGCATGATTCCTGTGTTCTTGGTCATAA
TTTCCTTCAAGTATCATCCATGGTATACCCACCTCCTCGGCGCGCGAGAGCGCGCGCACACACACACACACACACACACACACACAC
TGAATGTATTTTACTATTGTTACTTAATTCCCTCCAGGACTATACTTTTTTTTTTTTGTCAGAAAGTAACATTTTAAAATTTATCTT
TAAAGTTCATGGACAATGTCTTCACAAATATGAACATTTGACTAGCAATTGTCAATATTTTTATAAATGAATTTTTCATGAGAAGTA
ATTTGTAAAATGGGTACAGAAACATTATCTTTTTTTAATAGGAAAAAAATAAGGCCTTCCTCTGTGCCCTCATCTCTAACTCTTGGT
GTTTCTTTTCCAGCTCCTGTCAATGGACAGCATGGTGAGTCCTTTCTACTGCTTTGGGAGTGCTTGGAGAGTTTCTCCCTCATGCCA
ACATTTTTCTACTCAGGCTATCAGGGACTTGAAAGTGATGGCTGCCCCCTGGTATGGTTCCAAGCCAGAGCAAGTTCACACTGACCCC
CCAGCACTTGCTGGGTTAGGTGGTGAATGCACAGGCTGCCTGGGTCTTTCTGAGGCCCTGAATTGAATCCCATTGACAATGGACCCC
ACTTAGCAATGAAAAAAAAAAATCATCTTTGTCTCTGCCTTAAATACAAAGGGCTCGGGATGGAATCCTTTCCAATCAGTCAACTAGG
AAAATTGTGAAACTTTCAAGATGCCAACAGTAACACTGTTTCCTTTGCTGTTTTAAGAGTAAAATTCTCCAGGAAGTGCAGGAGGAG
TAAGAGAGTATCCAACCGTTGGAGCCTGGAGAAGTGTAGAGGAATAGAACCAGAATGAAAATGTATGTGCCACACACACACACACAC
ACACACACACACACACATTTGTATATAGCACAGAACTTAAGTGTGTTTTAAAATAGCAACAGAAGCTAAATCACATCCAAGAAGCTG
AGAACTCATAAGACACACAGTCAGAGTAGGTCCTAGAATAGCTGTCTCACACTAGAGTGGGGTGACTAGTAATTTATTCAGTCTACA
AGGCAGTAGGCCCTAATCTGGCATTCAAAGTCTGGATGGTTCCTGGAGCCTCTGGTCCCAGTCCATGATGGAAGCTTGGAATGCTGG
TTCTGCTGTCAGTGGCAGAAGCAGAGGCAGCAGCTACAGGGCAGATCAACTCAAAGGGAAGGCCAGGAGAGCAAGGGTGGGATACGC
TCCTTCTGAAAAACTGTTCTATCTGGGCTGCTACCCAAGAGTGCTGCCCACAATCAAAATGGATCTTCTTACATCAATCAAGATAGT
CCATACAGCTCTTCAGGTAAAGTTCCCTACTCCAGTGATTGTGGCAGGTTAGCATGAAAGCAAAGAGAATTATCACACATTTCTCA
GTCCGCCATCATTACTAATTTCCTGGGTCACAGGTGTCTTGGGTATTTGAGGATCCAAAGTAATATCGAGAAGTCTCTCCCTTCTCC
CCATAGCTAGCCACCCGGTCACTCGAGTGTCTAGTTCCCCTGGGGATCAGCAGGTGACTGTCCATTCCTTATTCTGACACAACTAAT
GACAGTGGGTTATGTAGCTATATCATCCTCTACGAAACAGCAGTTAAGTGCATGATCTCTCAAGCTTTTCTAGTGTAACGCCCGATG
GTAAAATGATTCACTACCTTAGGGGAAAATGCAAAGTTGTGATTGTAATGATTCTGAAATTCTTTGAGGGTTTCTGAATGACAAGAT
AGCAAAGGAAGAAGAAATGACTTTATCTAGGATCCATTCTCAGAGCAGGCTTACTAACCACACAGTGTTACAGTGCTAGAACTATCT
CCAGAAATTGTCTTGCTCCTTGCCATCCTGGGCAATCACTCTTCCCTGTGGGTGGTGAGTGCTCCTAAATGATGCTTCTTCAGCCT
TCAATGACCCTCTGGGTGGGCAGGGGCAGGGCACGAATTGGGGGGGGGGGGCACCGGTGACACAGGAGGACATGGAGGGGGGGATGGAC
ATGAAGACAGGGGAGGGGGAAGGAACACTGTGTTCTTGGGAACTGTCCAAAGCCAGGTCCTTNNNNNNNNNNNNNNNNNNNNNNGGCTC
TTCTGGATTGGAAACTGTGGGAACATCGTCTACAGATTCATTTCTCTCCTCCTCAGAAGCTGCACAGCAGTCTGTGGTTTCCCTTCA
GCCTCCATGGACCACTTTCTTTCGAGGAGAGGTCGTCACACTGACTTGTTATAGATTCGGCTTCTCCGTACCCCAGAAAACAAAATG
```

```
GTACCAGAAAAGAAAAACAGTGAAGCAAACCCCAGGTGCTTTGGTAATTAAAGCACATACCTTAAAGGTCCATGAGTCCGGAGAGTA
TTGGTGCCAAGCCGACAGCTTACTTCCGAGCATGCACGTGAACGTAGAGTTTTCTGAAGGTGAGACAGAAGTAAAAGAACTGAAGCT
TCAGACACCGTTTCTGTCCAATTTGCCCTGTAGAGTTAATTATTACTTACCACAAACAACTCATGAACAAACCATTCTATTTCATGC
TATGGTTACAGAGAGATTAAAAGTGTCTATAGAGAACCAGATATGGCACAGAATTCATGTAAATTTAAACATAAAAATTAATAAAAT
TTAAGTAGTCTGAATAAAGTCTGCTCAGCCCAGTAGGCAGTGACTTTGACAAGTGCATACAGAAGGTCATAATTGTCACTCACTGCT
CACTGAGTTCTAGATCTAAATACATTCACTCCTATCCCTGTGGTCCTTAAAGTCTAGCACAAATGAGAAGGCTTGCTCAGCTTTGTG
ACCACACAGAAATTCAGGAGCTACTCAGGATTTACAAACTCAAATCTTGAGATTATTGAGCCCCAAGAATATGTACTGTTTATAAGA
TGCTCATGTAAATGAAATTTGAGGAGCAATTTACTTGCATACTTCTTGAATCCATTTTGTTTATTAGGAATAGTAAGTACCATAAGG
ACTTTCGTGTGTGTGTGTGTGTGTGTGTGTGTGTGTGTGTGTCTGTGTCTGTGTAAATATTAAATAGATAGAAATAAATGG
ATTGATTTTATAGGTACACAAAGAGAGGAAGGGGAAAGGGAGGGAGGCATTTTTGGTTTTGGTCTTGAAGCCTTCTGTTTTAAAG
TTGAAGTAGTGTCAGCCTTGTGAAAATAGTCTTTGATTTTGTGAGCAAGAAAACATACTATTAGCAAGGAAAGAAAAAAGTAATAC
TCTTTGATCTCATGATTCTCTTGAACATTTACCTAACAAAGCACACAAATCTGCACACCCCAAAATAAAAGGCACTCCATTGAAGGG
AGACCCCATTAAATGATCTTGAGAGTATTGAGAGAAACTGTGGCTTCTGAAAATTTAGCTGCAGTGATGGTTCAACTTGAAGGGGGC
ACTAGACAAATGAAAGGCAAGCCGGCGTCATTTAAATCCCCTTAGAGTGACAGACATACAGAAACTCTGGTGTACTAACATGGAGAA
GGGACTGGAAGACTGTGTTCACAATGATGCAGGGAGCTTTCTAACTCGCCTTCTGCTGCCCAGCTTTTCTCAAGGTGCAGGTGTTTA
TTTTTAGTTCAGAAAAAGAATTCATGCTTTCTCCACTTCCGCTTCCTATAAAGCCTGTGTAGAAAGGCAACAACAAGAAGAACTGGG
AGGATGAAACAAGTGGTGTGCCTGATGTGTTCCACAGATTTTCTGGTGCTGCAAGCTCCACCTGCTGTGTTTGAAGGAGACTCTGTG
GTTCTGAGGTGCTACGCAAAGAAAGGCATAGAAGCAGAGACCCTGACATTTTACAAGGATGGTAAAGCTCTGACATTACATCATCAA
AGTGAGCTCTCTATTCATCATGCAAATCTGAAGGACAACGGTCAATACAAATGCACTTCGAAGAAGAAGTGGTCTTTTGGGTCCCTC
TATACTTCCAATACGGTCGGAGTTCAAGTCCAAGGTAGGGCTTTAATTATTCTGAACAGGTTAGTCAGAGACAGGCTTCCTGTGGAG
GTAGAATAGGAAGAAAGAGTGGGTGCCAGTCCATTGAGCAGAAGGACAGTCTTTGGGTGGGAGGGATGTGGGTAATCAAAAGCAGCA
CCTCCCTCTAGCCAGCACTCGGGGAGCGGTACCACTCCACACCTCAGTGCTGAGGATGAAATGGGGTTTTCTGCAGACTACTTAACT
GCTCAGTGGATGATTGTGATCCTTTGTAGAGTTGTTCCCACGGCCTGTGCTGAGAGCCAGACCCTCCCATCCCATAGATGGAAGTCC
AGTGACCCTGACGTGTCAGACCCAGCTCTCTGCACAGAAGTCAGATGCCCGGCTCCAGTTCTGTTTCTTCAGAAACCTCCAGCTTCT
GGGGTCAGGCTGCAGCCGCTCCTCAGAGTTTCACATTCCTGCCATATGGACTGAAGAGTCAAGGAGATACCAGTGCAAGGCAGAAAC
AGTGAATTCCCAAGTTAGAAAACAAAGTACAGCGTTCATAATCCCAGTGCAGAGTGAGTATCATTCTGAGTTTTTTTCCAGGCATGG
AAGCTATTTAGAAGCATTCACCAGTTTGGGTCACTAGCCTCTATAAAAGGTATTTTTTCTCAAACCTGTGGAAGTTGTATGGCTGTTG
TGGATAGCTAAACTGAGGAGCTTAGAACCAAGAAAGAAAAACAAACCAACAACAAAAACCATGTTCGTTTTTTCTCTCGATGCCTTT
AGGGAAAGGCTCTTCTGGTCTCAGCCTGACATGAGTTTTCCTTCCAACGCTATGATCCTAAACTGTAGACTACTAGGTGTTGCATGT
AGCTTTTGCTACTCACCATAACTCCAGGAATGGGCCACGCTACCAGCCCCCACCCAGCTTCCCTTGAATCCATAGATAAAACACACA
CACAGAGTTGTTCATTTTCAACTTGCCTTCTGGGCACAATTGCTGAGCATTATTAAACTCTCGCTGCTAACATGCCCTTATCAATTT
TCTTAACTCCGCACCTCCCACAAGCCCTAAACTTTAGTTGCTCTGTTAAATCTCGTCCCTACTAAACATCCCTGACCATTGCCTGTT
GGATAGCCACTTGCGTAGCCACTCTATTTTGAGATCTCACATGACCTGCCAACTTTTCTCCATCAGAAGCATGGTGAACTCTGTCTC
TCTTCCCTTGAATCTGTCTCTTTCCTCCGGGAACCTGGAAGTCCCCCTATTCCTTCTGCCCAGCAATTGGCCCATGGCCCCTTTATG
GACAGATCAAGAACCAACTGGGGAACAGGATGCATCAAAATCCCTACAATGTGGTCAGCCCTCAGACTGGGCTGAATGTTCTG
TTCAGCCAAAAGGACAGGTGTCCAGAACATGAGGTGCCTGGTGCAGACTGGTTAAGGACAAGAAAGCCAAAAGATGAAGTCTTGCAG
AATAAAATAGATCCATCTTGATGCCCTTAGAGATGGTCTTTCACAGACTAACACTTTTTCCTAAGGCTCAAAAAAAAAAAAAGTTTTC
TTAAGCGTGATCATTAGCCAGTGGCTTTGCCTGACTGAATTTGGCCTATCTCACAGGAAATCTAGTTGAGCATAGTAGGGGACACAT
AATCTAGAACACCTAGATCCTGGGTCTGGAGGGAAGGGAAGTGAGAGCATCACCTATGAATTTGTTCCCATTGCTAAGAAACCAATC
TGACTGACTAAATTCTATCAGTCTACTCTTTGGTTGTAAGCAATGGGTGCTCCTGTGGCTACTGATTTAAAGATGAACAGTTCAGAG
AGGAAAGAGAGGGCATTATCCATCAGTCACATCACAGTTTATTCTTAGCACAGTCTTTCCTTCAACCTAATTGCTGAACCATCCCATC
ATCCCCATCCTCTAGTCAACTATCATAGTTTGTTCATTAAGTCTGGAAAATAATCCAAAAGGCACTGTCCTAAAACAGTATGCCTAA
AACCATTCCCAGGGTGGACAATCCTTCCTTGTGAGTCGATCCTGGACACTAGAGAAGTTAAGAAGTGCCAACTCAAGGCCAGTTCAT
TACTTATGACAATGCTTCATTTCCAGTCATACACGACTGGCACTGTACTCAGGACTATCATGTTTGATGCCAACTCTCTTGTTTCCT
TCCTCCACTTTTCCTAAGTGTTGAGCCATGGGTAGCAGCTCTGGAATTATCAGGATAGGGCAGACATGAGGTCAGGTTGGGCAGAAT
AACCTACAGTCCTGCTACATATCCTTGACAAGGCCCTCAAGGAATGTGGGGCCTTATCAGCTGGCAGAAAACACAGTCTCCATTGTC
TTCTCACTTCCCAGAGACAAGCCTAATGTTACTCTAGGGCCCCTCCCCACATGGTAAAGCAGGTTTTTGTCTTCTTTCCACCCCTCAC
CCATTGCCAAAAGTCCCATGCATGTCATTGTTATTCATTTAGGAGCTTCTGCGAGATTCCAAACACACATCATCCCAGCCTCAAAGT
TGGTGTTTGAAGGGCAGTTGCTGTTACTCAACTGCTCAGTAAAAAGGAGTCCCAGGACCCCTCAAATTCTCCTGGTATAAAAAGGACA
TGCTGAATGAAGAAACAAAGATTCTTAAGTCCTCCAACGCAGAATTCAAGATCTCCCAGGTGAACATCAGTGACGCAGGGGAGTATC
ACTGTGAAGCTACCAACAGCCGCCGAAGCTTTGTCAGCAGGGCATTTCCCATCACCATAAAAGGTGTATGTTACAATATGAACTAAC
CAGTACCTCCAGAGCTCATGTCTCTAGCTGTATATGTAGCAGAAGATGGCCTAGTCAGCCATTATTGGGAAGAGAGGCCCCTTGGTC
TTGCAAATTTTATATGCCCCAGTACAGGGGAACACCAGGGCCAAGAGGTGGGAGTGGGTGGGTAGGGAAGCAGGGGTTGGGGAGGGT
ATAGGGGACTTTCCAGGATAGCAAAATGTAAATGAAGAAAAATACCTAATAAAAAATGTATAGTTAGGAGGTGTTAGTATATTCACAA
ATTGCCCAATTCTGTACTGTTTGCCTCTCAAACATGTATTTCCCTGGGACCTACAGTTGTTGACTTGTTTTTTAAAATGAGTTGTTGC
TAATGCCATTAAAAAAAAAAAAAGTGTGTGTTGCCAGGTATATCTGAGGGAATGGATCCCTCACATTGCACATGGGGATCCTTGAGGA
AGACTTGAAACAGTCAAAGTGGAAGTAGATGTTGTCAGTTACCAATAATTCAATGTTCTGAATAACTTGCCAAGTATTAGTCAAAGA
CTAATAGGAAAGACTTAGAAATAGATGGAATGGGCTGAGGGCAGTGGGAGCTCTCAGAACATCAGATGGAGGAGAAAACACTCAGTG
ACTAGGAACATAGCCTTTGTAAGTCCTCCTCAGTTAAAATGAAAGTGTCTCTTTATAAAGGAGGAACAGATGGGATGCCAGAAACAGT
ATATCTTGAGAGATGCCTCCTGGCATCTTACTGTAACCAGAAGAATTCTTCAGGGACCCATCCTGGCCATGCTGCTATGTCTTCAAAC
CCTAGTAGCTCTCTTATTTCCACTCTTGGTCTCCTACAGTGAGATGCTATGGAGAATGTAGTAGAGCTTCACCAAGAGGCTGAGGGC
TTCTCCCTCTGACCCAACATCTGCCTGTTGGTTTTAACATGAGACTGAAATCCTCTGTTCACTGAGCACTTGCAAAATTATTCCCAT
GTCCAGGCCAGTGGAGCAAGGAGAAGCTATTGTCCACCAGAAGTAAGCAGCTCCAGAAGTCATATCAGGGTCAGGTTGCATGCTCC
TAACAGGCTACAGTTACCATCCACAGAGGCTCCTTACTTACCCCCCTCCTGCTCAGCTTCTAATATTCTGATAGAATGGATGGTTCT
GGGTACCTGGAGAAGGCAGTACTTGAGGAGGAGCATCCTGCAGAGTATGTGAATTTAGCAGCTTAAAGGTCCTGGAGCAAAGCACG
GAAGTTACCCTAAGTAGAGAAATGGAGAGCAGAGAGCTGACACAGAGACTTAAAACTCGGGAGAGGAAATGGGCCACAGAGGAGCAAA
GAGTTGATTGAGAAAAGGGTATCAGTCCTATAGCAGCAAAATACAAACTTCTCCCCTGAGGGTACCATCTCTGTAAAATTGAGGAGA
GTTGGCTGACAAGGGGAGCCAGAAGGGATAAGGCAGATTGGAAAGGAACTGATGGAAGGCCATCATGGACGTCAGGAGATTGCTGAT
GTCCATCCAGCCTGGGAGGTTGAGCTGTCCCAAGATCTGGTAGAATGGAAACCAAGTATGTGCATTGGAACAAAACAAAAGTAACCT
GCAACCCACTTTCTTTCCTCCAGTCCCAGTATCTCAACCAGTTCTCACCCTAAGCACAGGCAAGACCCAGGCCCTTGAGGGAGACTT
GATGACAACTTCATTGTCAATCCCAGAGGAGGTCTCCATGTATCCTGTATGAATTCTTCTATGAGAATGCTTATACTCTCTGGAGGAGGAGCATACTTCAATTTCTCTATGAGCACAGAGCGATCTGGAAACTACTACTGCACAGCAGACAATGGCCT
GGGAGCCCAGTGCAGTGAAGCTATAAGGATCTCTATCTTTGGTAAGTCCTGGGTTCCTGCTGATACCCACCACTATACAGATTTCCC
TCTCACCAAACCCTTTTGGAAATTTGCCCCACACCTTTACTTCCTACCATAATCACCTCAATCCTGTTCTAGGCTCCTATCTCAGT
CAAGCTCATTGTCTGTGCTTATCTACCTCCTCGCTAGGCCTGACTTTTGCCTAGCTCAGAAGCCTCCACAGCTCTTAATAAATGTGT
GTGGTCATCACACTCTGTCTTCTTATGCCCTGTAATGTTTAGTTCCCAAGCTAAGAGCATCAACAGGGGCACCCAGTTCTCTACAAG
```

```
CACCTATCATCTTCCCTACAGAACAGTGGCATGAACAAATACTATGCAAAACCTTAGTTTTTCCAAAGAGTTCCAAGCAATTACAAC
TCTCAGTGTAGGTTAGGAGGAGACTGGATTTGAAAGAAAAGGAGAAAATGGCATCCTTAGGAAATGTGTTCCTTATTTTCTAGGGTT
TTCATGAGCCTCTTTCTAGAAGCCCATTTAAGCAAATTTAAATTCACTGCATGATGTAGTTTTCTAGACCTTTGTTAGAGGGTGACA
GGATATGCGTCAGATCCAAAAGAATAGCTAACTCTGGTGCCTATTAGCATACCAGAGTGCATGTTGGCCTCTAGGCTCCCTCAGCAA
GGGCCTGTTACAGAGTCCTGCTCTGGCACCCTGGCTTCTCTCAGTCCTTCTCACCCCACCCCCTGCCCTAAACTTCTCCAACCCATG
AGTTTCCCTTGCCCCAGCTTCTCATTTCTACATATAATCCTGCCATTTGACCATGCACTCTCTTGGTTCTCTTTGCCCTTTTGGCTT
TTGGCTCTCTCTCAGTCAGCTTGCCCCTCTCTCTCTTTTGCCTCCCTCCCCCCAAACCTTGGTCTTCCTTTCTTGCTCCTCCTGCCC
TCCTCTCATGGCCCAGGTCACTCTCGTGGCCATATGCAGACCACTACTTTCTCTCCCTGCTCTGAACTCTTCCAGGTGTCTTTGGCT
GTACTCTCCCTCATAACTACAATCAATCGGTCAATCGATTGATCAATCACTTCTCCTCAGCCACACCTTAGATCAGTTATATTCTCC
TTTTGTTCAATATATAGATCATGGTTGGTCTCTGTCAAGGCCACTAGTTAACATAATGGAGCCACTGAAGGAACAAATGTGCTTGTA
TTTGCTCTTAGGAGAAAGTAAAAACCTAGCTGTTTTTTTGTTTTTGTTTTTGTTTTTCAAAGCAGTAGAACAGCATTGTAGAAAAATA
TTGAGTCTAATACGTCAAACACATCTCCTTTGTATCACAAGGAAAAAGAAACCCAGGAGCCCTGGGTAGGTGCCAATTTTTAATACG
GCTGTTTTGAGAGTAAATAAATAAGAAAGACGACATAGGCAATTCAGGGAAGAACTAGAAGATTTCTCACAATGGGATATATTTCTC
AAAAATAGAATTATTGGGTTTTGGGGGAGCGGTTGTAAAGTCTGAGGCTATGAAAGCTTACATAATGAGGATCTAAGAACGTGTTTTA
GAGTTTAAGTTAGATGTGCAAATGCGAGTTATAAAGGTTAGAGGATGTGAAAGCTTAAAGAGTGATAAGAAAGTGAGTTAAGGTATA
CAAAAGGAGGAAAAATGCTTCAGATTCCTTCCCCTTGATATGCTACTGCTGTATTAAGACTTCAAAATTCCAAAGTTTTGACATTGA
CGACTGGAGTTCTGCTAATATTGTAGACATAAGCTGCGAATTTTTAAGTTTCTTTTGGTTGTCTGCTAAGTATAGATTTAAAAGTGCT
TCTCATTGTGCTAAATTTACACACACGCACACACACACACACACACACACACACACACACATCCAGTCTTCTGGATAGAGGAAAGAA
GCCACTCTTTCATGGTTTGCAATCATACTGAAAATCAAGATCAAGCAATCTTTTGCCATTTCTGCTCCATGGAAGGTTTCTGTTTTT
CTTGAGCTTGCCTTAAAGAGTGGTTATGCTGTTAACCCAAAACTATTTTCCAAGCTTCTACTATGACACAAAGATGTGAGTATGTGA
GTTTGAGTATGTGAGTATGTGAGTTTCAGTATAGACTACAAATAGTCGTAACACTAACATGTTTAAAACTTGTCTCTTTTTGTAAAC
TTAAAAAAAAAAAGATTGTAGTAAGCTTCAGGGGATCAACTTGAATCCACCTCTCATGGGTCAAATGGACTCCAGAGAAATACCCTT
ACCAATCAATCGCCTAAGGGGCCCAACCCATTACCTATTCCAGAAGGTGGGGTCCCTTACTCTTTCCCTGGTTTAGGGGCTCCTCTC
CCTCAACTACCAGGACCATGTCTCAAATGTATACAAGAAAAATTTCCCCCCAGACTCTTTAATTTTACATTCTGTCCTAATATGTGT
GTGTGTGTGTTCTAGCAGATTTCCAATCAATTAAAGACTATAAGCTGCTCTAACCACTGCCTGTACATCCCTAGCCCCAGATGTTCT
TGCAGAGCCTAGACACAACTTGTTCTTCCCAGACTTGACCAACATTCCAGCTTCATGCCCTTCCATCATTTGATAAAGATCAAATCT
TTAGCAGGAGCAGTCCTGACTGGGTCTCCAGCCTTCTACTCCATCACAGTTACTGGGCCTGTCAGACTGCTGCCCTACCCTCTGCAA
CAATTCAAATGGACCAACCTGTCTTCAGTCACTGCCCACACTGCCTGCCTCATAACCTATACGTAGTCCTCCCATATCGGATATGGC
CCCTTTCCACTGCAGGTTTTGGCATAGTCTCTGAAGCCCACTTCTTTCTCTTCTAGCAGTCCTCATCTCTCAGATGCCCAGCTTTCA
GCTGTGTCTTCATTTTCAGATTCCTCAAACATGTTTGTGTTTATGTGTCAGGTACTTCAGAATATATACAACACTATGCAAACATTC
TCACTTCCCACATTGGGATAAATAATAATTGAGCGCTGAAGAACCTGAAAAGGTCACTAAAACCTAATAATGACATTTTGCAGTCAG
AAATGCCTTTAGTCCCCTATACCCTCCCCCTGCCCAGCATTTGAAATGTAAATGAAGAAAATAGCTAATAAAAATTTTTTTTAATTAA
AAAAAAAAAGAAATGCCTTTAGTGTGGAGGGCTGTGTTCAAATGGGAGAAACCGATGGCTGGTTCATCTCCCTCAGTTCCAGGGTCC
CACCCTACCCTAATCTTAGACGTTCCCAGGGGCCCAAGCTGTAGTGTAGTGGGGGGGGGGGGGTGAAGCTAAACTGTGAGACCCTGGA
AGGCTCCCTTTCCTGATCTTGTACCATTTTCATCCTGAAGATGTCACCCTGGGGTGTAGGTTAGCCCTCTCGGAAATAGGAGAGTCC
TTCAACTTCTGTCTGACTGTGGAATGCTTAGGGAATGGGGTAGTTCTACTCCAGTGAGGCTGGCGATGTCCCAGGGCCCTAGCACAG
TGAGGCAGTGATGGTCAGTATCACAGAGACTTGTCATAAGCAAGCGCTGGCGGCACCTGCTCCTTCTGGCCGCAGGAAAGCTACTTC
CTGTTGGTATCTTCACAGCGCTCCTTTCATCTCTCATCCTCTCTAATCTTTTGGCTCTCTTCCTTTTATATTTTACCTCTTTCTTTC
TCTCAATGTCACCTCCTACTTTCTTGTCTTTGTTTATTTTATTTTATTTACTTACCTTGCCCCACTGCCTTTAATTAGGGTTGTAT
GCATAAACAGGGGTTGGAGTCATTTACTTGAACTTGCCAGATGCTATATTCCTGAGGAGTAAGGCATAACGTGTTTTAATATCAGGA
TTGTCCTGTGTTTAGCTAGCTATAAAACCTTTATTTTTAAGCTTAAAATAAATTCTATGCATCTACCCAGTCTATTCGGTTGTCTACAG
GTTTTGTTGTGTTACAGAAACATTAGAACATAGAATTCTTGCATGAACTATATACATGTACTATATAATGTTATATATAGGCATATA
ATGTATTATCATACAATATATAACATATATTTGAAAATAAAATTTCCATACTATATGATTTTCCTCTCCCACATCCCCTTCATCCAT
CTCCATGCCCTTTCTCTCTCTCTCTCTCTCTCTCTCTCTCTCTCTCTCTCTCTCTAGAATACAATCAAACAAACATTTTTTAGAAAG
AATAGCAAATAAATAAGAAAAGGCACAAGAAACATAACACAAATCACACACACACACAACATAAAAGCACAAAACATAGAAAGTATA
ATACACAAGCAAAAAAACCCAATAAGATTTAAAAAAATGCCCAAAATAAAGCAATTTGAGTCAAAAGTGTACAAAAGCACCACTGAGTT
CTTTTCGTGTTGCTCATCTGGTGCTGGGCATGAGGCCTACCCGTGACTGTGGTCGTCTTGGGCACTGCTCTATTGCTCTGAAGGGAC
ACCATGACCAGGACAACTCTTATGAAAGAAAGTGTTTAATTGGGGCGACTGCTTACACTTTCAGAGGTTTAGTCCATTGTCATCATG
ATGGGAAACATGGTGGTAGGCAGGCAGGCTGGAAGACACAATTTGCTTGGTGTCACCCATCTCTTCAATCCTTACGATCTTTTTACT
TCCTCTTCCCCAACGCTCCCAGAGCCCTCAGTGGAATAATAAAGACATCCAACTTAGGAATGAGCATTCTAAAATCTCTCATTCTCT
GCACATTGGCCAGTTGCAGGCCTCTGTTCATTCCCATCTACTTCAAGAGGGAAGCAATTTGAGTCAAAAGTGTACAAAAGCACCACTGAGTT
ACAGATACAGCAGAATGTTGCTAGGAGTCATTTTATTGCTGTATTACTTTAGCAGAACAGTACTGTTTCTTTCCTCCTTGCCCCATG
TCCTATTTGCTTTCCTCTTAGTCTCAGGTTCTTGGCCACTAAAGCAGTGTCAGATATGGTTTTCATCTCATAGAATGGGCCTTAAAG
CCACTCAAATACTGGTTGATTACTCATCCAGTTTTTGTGTCATTATTGTATCAGTAAATCATGGAGGCAGGTCAATTTTGTAGATCA
TTGTGGGCTCCAAATTTATGCATTTCTTAATTTTGTAAAATTGTCAAACTACCTTTCAAACGACTGTCCTCAGTTTCCTTTGCCA
CAATTTCATCAGTATTATCAATCTATGTAGGCTTGAAAAGGATAATAGTTTATGGCTGAATCTAATTGTACATTTAATTAATAATGA
AAATGAGTCTTTAATATATTTGTTGGCAGATGTTTCCAGTGATGCATATAACACTTTTCCCTTTTCTATTGAATATTTTTGTATATG
AAAAACTACTTGTGGATCAATGTCATTAGCTTTTGAAATCAGATGTTTTTTATTTTCTTTAATTTGCTTATAATACTACTTTTCCCT
TGTAACTGCAACAAATATGTTAGACGCAGAAGCTTCTACTGAAGTGTCATTTCCATTCAGTTCCAGTGTCCCCTCCCTGTCCTCACA
CTCAGGGTGTCTCAGGAGCTCAGGCAGTGGTGGCTGATACGGTGGAGCTTCACCATGAGGCTCTGAGAGGCTCTCCCCCAAGTCCTG
TACCAATTTTACCGCAAAAAAATGTCACCCTGGGGAATATCTCAGCTCCCTCTGGAGAAGGAGTCTCTGTCGGCCTCTCTCTGACTG
AAGAACATTCTAGAAACTTCTGTGAGGCTGACAATGACCTTAGGGTACATACTTTTCATCACTGGTAAATATGCTTTAACATACAGT
GTTCACCATGGCTGATCAGGAAAGCGCTTCTCAAAGGCAGGTCTCTTGAGCTATTGGCTCTCTAAATCATTGTGTTTTGGTTCAAGA
GAGCCAGGCTTTAAAGAGTATCTCAGAACTTAGAATTATCAGCTAGCTGCTCCTTCTCTCTGACCACAGTCTATCTTGACCCTTTGC
CTTTCCTTAGGCACAGAGACAAGCTTTCTCATAAACAAATGAGCCATCTTCTGTGCCATGTCTGTCACTACTAAGGCTTGGTACTCC
TGGTCACAAAGGTTCTTCTCCTCCCCAACACTAAAACATCAGATTCAAGACTCAGAGCCACTCAAAACTGCCCATTCCTTCCTGGTT
TTATGCTTAGGTATCCTCTGTCTCCCCCCTCCCCGCCCCCAGACATGACAAAGAACAGAAGTGTTCCTATGGCTGCCGGAATCACTG
TGGGACTGCTCATCATGGCTGTTGGAGTGTTTCTGTTTTATTGCTGGTTCTCTAGAAAAGCAGGTGGGTGACTCCCTAATCACACCC
TTCCTGTCTCTGTTCCTGCTTGCTCACACTGAATATGTGACCATCCGTAGGCTCTCGAGAGTTTCTCCTCTTTCATCGTATGATTCC
TTGCCTTTCCCTCTGGGTCCCTCAGAGGTCCTGTTAACCAAATACAGATTGTTCAAAAACTACCTGAGCGCAACACACCTTGATG
AGACGCCATCTACCTAACCTCAGAAAGGGTCCTGTGTTTATTATATTCCCCCGAGGAAGGAGAAGAAGAGAAAAACACTAAGAAATG
GAAGTCAAAACAGAATTCAAACTAGATTTGGTAGATTACTGATCCTTAAAATGTATAGTATTTTTATAGATGGTCAAGTCTATTTTC
TGGTCTAAGACTATTGAACTATCTGTAGTCATTAAAGACCACTATCTTTGAAAAATCCAGGTTAAAGGAGTATGGACTGAGCTTAAG
GAAGTAAGGACTGAGGTTAAGGGAGGATTGACTGAGGTTAAAAGAGGATGGCCAAAGTTAAGAGAGGATAGACTGAGGTTGAGGGAA
GATGAACTGAGGTTGAGGGAGGATGGACTGAGGTTAAGGGAGGATGGACTGAGGTTAAGGGAGGATGGATTGAGGCTAAGGGAGGAT
```

58

```
GGACTGAGGTTGAGGGAGGATGGACTGAGGTTGAAGGAGGATGGACTGAGGTTGAGGGAGGATGGACTGAGGTTGAGGGAGGATGGA
CTGAGGTTGAGGGAGGATGGACTGAGGTTAAGGGAGGATGGACTGAGGTTAAGGAAGGATGGACTGAGGTTGAGGGAGGATGGACTG
AGGTTAAGGGAGGATGGACTGAGAAGCCTCCCTTCACTTGGGGCCAGCATGAAAAAATCAGTTGCTTTTACCCCTCTCCCCTCTGTC
GGCATAGCAAAGATTCAGATCTCAGTAACAGAAGTTCCTGGCAGTCTCCATGTGAGCCCCACCTAAGCTGCCTCCTAGTCCCCATGA
AAGACCCCTCTTCCTGAAATTCAATGTGTTCCTTTACAGGAGGAAAGCCTACCTCTGATGACTCCAGGTAAGAACTTGGACATATTT
CAGGAAGTAATGTAAACACCCACAGGACAGAAATTTTAAGTCAAATCTCAAATCTTATTACCTTATTATCACCAAGATTACAATTTC
TATGCTCGTTGCTGAGGTGGGTTGTACCCAATTGATGCTACCCCTCCCTTGGGATGCCTCAAGTCAATTCCCAGAACCTTCACCTTA
GCTACACCTTAGAAGCCTCCTTCTCATGTCATGAGGCAATTGATTTTCATTTATTTAAATCACTTCCTTGTACTACTGTGAGCTTGGT
TGTCTAAATGATCACTTTGGCAGTTACCAGGGAAACGGGTTCCCTTTGGAGATCCTTATTCTCATTAATAAAAAAATTTAGAAACAA
GCTAAGAAGAAAGCTCAATACAAATTTACTAGAATTTAATTAAAAAAACAGGTAGATGAAAATCCTGGTGTTACTAATGAGGAAAGAT
GAAGGAAAAATTCATGCCTTTGAAGTTGGGCGGCATAGGATAGAAAATAGTTTATTTAAAAAGGAAGCATCACTCTCAAGAATGGAAC
CAGACTAGCCAGCCAAGAAGAGAGTCCCCAAAATCAGCCATCCATGTGGCTCCTGATCGTTATAGCACCACTCTCCCCATTTGTAAGT
CCTGGGCTTCAGATCACACGTGTTCTCTTTGTTAGATGATGCCAAAATGAGGAAGGGCTTCAGGTTTTCCTTCCTCTGCAAACTGGC
TTCCTTCATGTTAATCGTATCGCACAGTCATTGAATCCACCCACACCTTGAGATGACATATCTTAATTAGCCTAGAAATCTCTAGGC
ATGGCCTGCCTGATCAAAATCTGTTGCAATGACAGCCAAGAATGTGGAGCACATATCTCACACCTAGAGGAGTCATTCTGCAAAGAA
GTAGGATCTGTGAGAAGACTGCCACCATGCCACCATCCTGAAGGCTCGCTTTAAGGACTGTATAGAACTGCAGCCAGTGTACAGCAATGG
TGAGGACAAGAGCCCTCTGGTCTCGGGGTAATCACAAGATACATATTGGAGCAGAATCGGGAAGATGATAAAGCCTAGAGGGCTATA
ATCCTTCTAGAGAACAAACTGGCCAAGGGGTAGCTGAGAAGAGAGGGAAGCTCCTTATTCTCCTCTGAAATTCTGCCTTCTTCCTAA
ATAGAGCCTGAGGAAAACGTGATTTACACAGAAGTACGGAGAACTCAACCAAGACAGAAACATGCAGGTAAAAGCTGGAGACCTCTC
CGTCCCTTGCTATTGTTCAGCCTTGGCTTCTCAGCAGGTCTGGGCCAGCCAAGCCAGTCTCTTCACTAGGACGCTAGGATTCCTTAA
CTTGCACCTACATTGTTTGGAGTTAATCCAAAGCCATCATGTAGTGCTGGCTTCTTCATTGTCTATGGATGAACTCACGATAAC
CCGAGTTACAAAACCATGACAGAGTCCCCTCTGAGCATCTGTGTCAGCACCTATAAGGTGGAATAGGGCTATCTATTTCTGGGTGCT
TTTCTAGTTCTGGGTGGCTGAAACAGAACCCTGTAGACCGGGCAGCTGAAATAACAAATATTTGTTACAGATCAGGAGTCTGAAAGC
CCAAGATCAAGGTGCCAGATGGCTGAGAAAAAGTAGGATATGTCTCCTCCAAGGTATGGTTGTGGAGAAGGTTTATTGTGGATATGA
GGGAGAACAGCCAGAGGGATCTGGATGAGTCCAGACTGAACTGGGCCATGGGGTGGCAGGGCAAGTGGACCAAGAGAGACCCAGGAG
CAGGGCAAGCCAGGGGACTGGTGACCAGTCTCTGCTTTGATGTGTTAAATGGGCACCTTAGTTAGCCATTTGTCCTGAGTTTGAGA
CTTAACACTCTAGCCTTTTGGCTAATGATAAAAGATGATGGAATTTCTTCTATAACTACTTTCTGCTGAAATTGGGGTGTCATCAGG
GCCCAAGAAGACTGGAATATTGATCAAATATTCCAGGAAGGGACAGATGAGGGCAGGTTGGGGAACATCTGTTTCACTTGCTGCCCA
GGTTCTGAGGGACCGCCTGGGGCAAGGAAAGTGCAGGGCTGCTTTGGAGCAATCTGGCATGTGGGTTGCAAGGAACACTTGGGGCTG
GTTTAGGAGACACTTTTTTTTTTCCAAATCAAATATCCTTTATTGAATAAAACAATGCACTATAGAATATTTTCTTTAAACAACAGT
GAAATTTTAGAGTATTCCCATTAGATATACATATACCTTGGAAGATGATAGTTGTTCAATCACCTTTTATAAAAAGAAAAAATAAGA
TTTACATTTTACTTAAACTTGAATTTAGGGTTATTTGACTACCTTAATTATTTTATAGTGACAAAATACCAGGAAAAATTAAAGAAA
TATATTAATGTGATATATATATATATATGTGTGTGTGTGTATATATATATATATATACACATATATATATATATAATGTAT
TTTCCTCAATTACATTTCCAATGCTATCCCAAAAAGTCCCCCATATCCTCCCCCCCAACTCCCCTACCCACCCACTCCTACTTTTTG
GCCCTGACGTTCCCTTGTACTGGGGGCATATAAAGTTTGCAATGGGCCTCTCTTTCCAGTGATGGCTTACTAGGCCATCTTT
TGATACATATGCAGATAGAGTCAAGAGCTCCAGGGTACTGGTTAGTTCATAATGTTGTTGCACCTACAGGGCTGCAGATCCCTTTAG
CTCCTTGGTTACTTTCTCTAGCTCCTCCATTTGGGGCCCTGTGATCCATCCAATAGCTGACTGTGAGCATCCACTTATGTGTTTGCT
AGGCCCCGGCCTAGTCTCACAAGAGACAGCTATAACAGGGTCCTTTCAGCAAACGCTTGCTAGTGTATGCAATGGTAGGAGACACTT
TTTATTTTGGGTGTATACTTGAAAGTTCTAGGCCCATGGTCCTGGTAGTTGGGGTAGGGAGCAGCCCCAAAACCAGGGGTAGGCAGT
AGGTGGGAAAACTTAGGTTGCTGATTGACAGGAGTAATTGTCTGGAGTCCATTTGACCTGTGACAGACAGGTGGATACAAGTCATGA
CTTCCTGAAGCTTACAAGAATTTTTTTTAAGTTTACAAAAAGATAAGTGTTTTCAACATATTAGCATGACCACTGTTTGCAATCTGTA
CTGAAATCTGCATTGCATTGTAGAAAAAGCAGTGGCCTCAGGCCTTTGAGGCAGAGTAAAAGCTTGGGACCATCAAGGCATGATTCT
GAGTTCAAAGCATCAACACTGCTCCTATATATCCTGAGGCCTGGTTATATAGGTTGGACAGAGGTGGTTTTAGCGTGATTGAGAAGG
TTGTTAAGTCTATGCTTAGAAGACAATTTTGAGCTTGTGACTGTAATAGCAGCAGTGACTTACCAGGGCTAGATTAATAGCTTGTCA
GAACTCCACTGATTAATGTTAAAACTGTGAACTTCAGAAGTCTTTATATGACAACAGCATGGAGAGGAAGCGATCTGAGATGGTG
TCATCACTTTAAATCACTGTCTTACACCTTTACAAAATGTGCGCTTACACACCTTAAATTACTACCTTAGACCTTCACAACTCAAGC
TTTCACGTTACCACAACCTGCATTTACAAACTTCCCTACAAATCACTTTCCCAGACCCCCACAACGTATATAAACGTTGGGCTTTTT
CTTTTTCAGCTGATCATTTATCATGAGACACAAATGGTTGATTATTGAGAGTAGTCGCTGCAAAGCAAGTTCCTTTAAATAAAGGAGC
TGTACAGTTCATGAAAACCTGTTGTGAGTGTTTCTCTATCAGTGTGGTAAACTATGACTTTGTCTCTGAGCCCAGAGACCTAGTAGCT
CTAGAAAAGTGAGTCCTGGTCCTGCCTGCTTCTACATAGAAATGGCCAGCGTGAGCCTCAGGGAGAGGAGCTGGGTACCTGCCACTGTTAA
CATACAGCTACAGTTAGCCATCAATATCACTCAGAGGTCTGAGAAGGGTAAATTATAGCAGGAAGTACAATCCAAATGTATGTGTGTA
TCAAGTAAATGGCAGAGAATGACTGCCACCTGGACGTTTGTCCTAGGCTCCTGTGGTCTCCGTGGCTTCACTGAGGGCACCGGTCT
GAGAGATTCTTCTGTGGAGGAACTGGGAAAAACTGACCCACTTTGGCAAGGCAGAGTAGAGCAGTGAACACAATAGTGTACATAATT
TGTGCAGAGCCCTGGTGGTAGGTAAGGCACAGGCAGTTCAACCAATGGTTAGTGTGACGGCAATCCAGGTAAAGTATTCTGTACCC
ACCTTCTTCTTTTGAGAGCTTAAGGTTGCCTCTAGGAGCTTGCAGTTCTGTCTGCCATGGAAATATTTTTCAATCAAACATGTTAAA
TGCTGTATTAGTGGATTTCTGTAGAAGTTAAGTCCTGGTTCTTTCTCCCAGCCCCATGCTCCCAAAGTAAGACTCAGATTCAAAATA
TATTTGCAGATACCTTGGCCACACAGTTAGGTCTTTAGTTAGATCTCCTCTGACTAAATCATAACTAAAATAACTCAATTACTTAGC
CTGCATTCTACCATGTGGCTGGTTACCTGTGCTCAGGTACCATGAATCTATCTCCTCGCATCTCCCTGGGTAGATCTTCAGAGCCTG
ACTCTATCCCAGAATTCTCTCTCCTCCTGCATGTCTCACCTGCTATTTCCTGCCTAAGCCATAGGCCGTCAAATTTATTATTGGCAG
GTGCCACATCCGTACAGACATAAGATATCTTCTCTACAGATCTCTGAAGGATTTGAGGATCATTATCTGTTGTATATTCATGAGTTA
AACAAAATTGACTTGTTTCTTGCTTTTTAGTTACCAGTCTTAGGCTTAACTTGGAAACATATATTGAATACTAAATAAAATTTGCCCC
TGTAAATGAATTACATTTGTACTTAACATGGCTTATGTGCATAGTTTAGAGTACATTAAAGAATTTGATCATTTATAAGGTGGCTGA
CCATTAACTTGCATGTCTTAGTTATCTTTAACACTGTACAAGTTAGTAGTTTTATAATATCAGGATTTTACAACTTTATCTCTGCTA
AGTTTGAGCCTTAAAACTTTGAACTTTGTTGAAGAATTCCTTTAGCATTAAAACAAAACTTTAAGCCATAAGTGTAT
TCCATAAAAGGACTAGAAGTTTTACAAAACCATAAGTACAGTTCACATAGAGCACCAGAAACCATTTTGGCTGTCGTTTGTTCGTTTT
CTCCTTTCATGATGCACCATTACAAAATATCAGTTTCTTGTAAGACTCTGTTTATCTAAGTAACTAAACTTAGAAAAATCAGTAAAG
ACAAAGAGGCTAGACAATAGGTCTAGTCGGTTAGTTAACCTGTGTAGACCTTAGGAATGTATAAACCTTATTTATCAAGCATATGTT
TTTTATTATTTAATGTTCTAGAAGCCTGATGTTGAGCAGTTAGCAAAGGCATGAGTGGTTAAATATACATCCCTAAAACAGTCCCTG
TTCACCTGAGTAGACTTTATAACCTTAGGAACTTATTACCGCATAAAAAATTCATCCTAACCTGAATGTTTAGAAGCCCTGCTGTTGC
```

59

```
CAAGTGTGTTTGAAAGGGAGATACAATGATAAACAGAGGCCTCGGCAGGACTGAAACGTTGTATACTTGTTGCGTTATGCCACATGG
TCACCTTAACCAAGATGGTGGTAAAATTACATCGCATGTACACTTTTTGACCTTGGTAAAGATGAGCTGCCAGACATGTGGTTGCTA
TTTAAAACCATCTATTTTCTAGTAGACCTGTTTTTTAAACAAGAGTTAAATCTAAATTACATATACAAAATATTCTAAACAAGAGTT
GAGTCCCAAATATAGAATGTTGTAATAAGATTATACAGATATAGTTTTTTTTTAACTTTTAAGCCCTTCTGTTACAAGTGTTAAGCTA
AATTTTGTTCAGGTTTTTAATCATACCCAATGAACTTACAAACCCTGAGATAGTTTACAGTAAGAGATTGGGGGGAGTGAGGCAGGG
GAGGAGAGAAATTGTAAGGATAAAGGTTGTTTATGATGGAAAGTAGAAAAAACCTTAGAGGTAAGAAACTTTTCGAAGTGTAGAGTA
AAGAAAGTTTAGATATAAGAGATTTGATGTTGTTTGTTTGTTATGCGGTGGTATAAGCTGTTACCATAGGAGAGAATTTAAAATCAA
GCATGCCACACTGACTGAGCAGTACTGAGGCCAAGGCATGCAGAGTAAAACAGCGAGGCTTTAATAAGATCCTTGAGTCTGAGGAGG
AGAAGAGAAAGGGTCCTATGAAGCCCAGGAGGGGTTTAAGGAAGCTGAGGAGCTTCCAGGAGGGAGCTGAGAGACAAGGGATGGGATC
TCAGAAGCCCAGGAGGCTTATGGAAGAAGATGCAGGACCACAGCTCCTTGAGGAAGGTGCAGAAGCTCTGAGAAGGAGCTGGGAGT
AGGTCGCTCCGGAGGAGAAGAGGCAAAGAAAGGGTCACAGGCCCAGGAGGACTTAAGGAAGCTGCAGGACAGCAGCTTCTAGGGAGA
GGCAAGGAAACACCAGGAAAGAGCTGAGAACAAACAGAACAGAAGAGGTTTAAGAATTTCAATCCTGTCTGTCCCCAAGTAAACTGA
GAGACTCCTCTGAGAGAAGTGTCCCAAATCACAGAGACCAGGCATTTTTTTTCCCCATGGGAATGGAGCCCCGTAGGGCAAGAGGAG
CAGCCTGGTGCACTGCTGTGGCTTTTGTAATAATAGTGGACAAGGCAGGCAGCTCCAGGGTGACACTTACCCAGCTGAGGAGGAGAG
AAGAAAGCAGCTGAAAAAGCAGGCTGAGGCAGGCTTTTGGAGAAAGGGAGAGGGTAGATGTCTTTAGTAGAGATATGTGGGTGTAAC
TGACCAAAGAGACAAAATGATATGCACGGATAGGGAGTAGTAGGTGCCAGAGAGGACACTGAGTAGCTATATTTGGGACAC
TTAGAGATAGAAATGATAACATTTACTCTCACTAAAATGTGTATCTCATCTTCACCAGGTCTTCCAGGCTCCCTACTGGAACACCTCC
TGGACTTAAAGTCTGCCAAACAACTCACACACTAAGTTTGGCCCCAATACCTCTAAACAGCCACTTAGCAACACTTTTGAAGGAACT
TTTACAACTTTTGCCAGAGATCCGGAAATGGAAGGTTGCCTATGTGCAGGCTTCTCCATACCTTCACTCCAAACCTCTTCATTCCTC
CTGATCCCCAACTCATTTCTATCCTTGATCAGGCTAGCTTGCTTTCTCATTCTTGCTCCCCATTCCTTTGACTCTAAAAATACTCTC
CAGACTTTGTTTCAGACTTAACTCACTCTTGTTCTCGATCCCTGCCTGGTGCCCTACTCTACCACACACTCTCCGTTTGCTCCAGC
TGGCAGCCTTGAACTTCTTAAACTCTACTTCCTAAAAAAATAATTTATCTCTGATTTCTGCTTTTCCATTCTGATCTGCAACAGCTCT
AAGCCAGCTGTGGCCAAAGACAGGCCCGGTGGTTACTAGATCCCTCTGTGCACAAACACAAGAGGGAGTGCTTAAGTGGGTCCATGC
TGAGACATCTTTCCCTGAGGTACCATGATGGGTCTAGTTTAAAATAAACCATTTGGGGGCATGAGAAGGGGGTTTGAGAAGAGAGTAG
AGGCAGAGAAAGAGAAAGGACAGAGAAGGACAGAAGGAGGAGAGCGAAAGGGAGAAGAGGCCGGCCAAGAGACAGAGAGGGGGAGGG
GGGAAAGAACAAAAGGAGAAAGAGGCCAGAGAGAAAGGCCAGAGAGAATGTGTGTGTGTCGTCTGTGTGT
GTGTCTGTCTGTGTGTGTCTGTGTGTGTGTCTGTGTGTGTGTCTGTGTGTGTGCAAACAGTCCTTTCATAGCAAG
TGAGGATTGTACCTGGCTGTTGCTAGAGAACTGTTGGGCAGAGCCTTGAAGAAATGCTATCATTGACTGTTTTTACAGAGGACCAGG
GCTGGATTCCCAGCATTAATATGGCAGCTAACATCTGTAACTCCAAGATCCAACACTCCTACACAGACATACATCCAGGCAAAACAC
TAGAACAGATTAAAAACAACAACAACACAGATTAAAAATAAATAAATAAACAAATAGACATTATTTAAAAGAAGAGAGCCCATGAAA
TTGAGAGAGAAAGGTGGTGGTGGTGGGAAGTAGAAGTGGGATTGCAGACAGGACCGGACTTGATCCAAATGCATCATATGTATTTAA
CACAACATTAAAAAACAGACTTAAACCATCAAGTAAAGTGAATAAAGAAATGAAAGAAAAAAAACTGGCCATTGTTAGCCCTCAAAG
AAACCTGGGAAAATGGCTAACTGAGACCTATTTCACATATCAAACCCTGCTTCTGTCCACCAGTCTCCCTCAGTCCTTATTCACAAG
AACCTGCTGTAGAATTGTTCTAGGAAGGAACTTCTACATTGCTGTTCATCACCAAAGGAGGTCAGGTCAGGAACTCACAGAAGGCAG
ATAGAACCCAAGACTACCAACCCAGGGATGGCACCACCCACAATGGGCCCTCCCACCCTGATCACTAATTGAGAAAATACCTTACTG
CTGGATCTCATGGAGGCATTTCCTCACCTAAAGCTCCTTTCTCTGAGATAACTCCAGCTTGTGTGTCAAGTTGACACACAAAACTAGCC
AGTACAATTACTTTAAAGATTTACAAAAGTAAAAAGTTAAGGACCACTACTTTAAAGATAGGTTTAGCTTTGTTTTTGCTACTATGA
TTAAGTTACAATGTAACCTCTTTGTTTGCTAGAACAAATAATTAAAAACAGATACAAATTGTCTAACTCAGATATGCTTTATAGATA
CTAGCTCTCAATCTTGTCAGAGATCTGCTAAATATTAAATGTTTAAGCTTGCTATGACAGACAGAGACTCCCAAATCCTAACAGTGA
CCCCCGCCCCAAGGTCTCAAGAAGATATGGGCACAGGCAGCAAAGGACTCTACCTGGCCTCTATCTCAGAGGTCTGATGACCACTGA
GAAAGACTGCTCCATTGCCTTTCCTGCTGCCCAGGGCTCGGCCTGGTCTATGGACAGAGGACACCTGGAGAATTAAATGTCTCATATT
GCCTAAGACAAGATGAGTCATTTCTCCCATGTTCCTCCTCCACAGGAAAAGGCCTCTTATCTTCTGGGCCTAATGGCCAGACTGCCT
CTGCCCAAGTTTCGATGGAGACCACAGAGACCTGGGAGAACTGTTTAGGTAGTAGACTATAGACTAACCTCTGTCATTTTAATTAAT
ATATGATATCTAGACTATAATTAATTCTCCCCAGATTTCTGATTACATTGACAGCTAAACTAACTGCAGCTTGACAGCTAGAAAACA
ACTAACTCCTTACCCCAAGGTGATCCCGCCAGCATATTAGCTCATTAGTCAATTTGTTAACTAGTTAAGACTACCAGATCTCCTATCA
AAAAGGTAGACAGGTTTGCCTTGCTCACAGGTTTCATGATAAAAGATACACAAATTTCCCATGTAACCTTTTTGCAGATATAACCTT
CTGCTACTTATCTAAAGAAAATCAGTTTACAGTGACTACTCCCCAGTGATTAGATTTGGTAAATAATTAAATAAAAGGCTTAAATAT
TTATAAAGCTCTAAATGGGGGTCTGGAAAGGTGTTTTGAGGTCAGTAATCATAAGATATAAAGGTTAGAGGACATACAAACTTAGGT
GGTGATGAAAATTGATTCAAAATACAAAGTGCTTCAGATTCCTGCCCCTTGCTATGCCACTGTTAAATTGAGAGTTCAAAGGTTCAA
AATGGAGTTCTGATAAATTAACTTAACTTTAATCAAACATCCCACTATACAGTTGAACTTTTGTAATCGCAGGTTCAAATTTCAAGT
TTCCTCTGATTGTCTTTTAACTATAGGCTTAAGGTGTTTCCCATGTTAACTCTATATGATCAGTTATCATATGCACGAGGTCAAGAT
TTTGAGTTTACTCTGATTGTCTCTTAAATGTAAACTTAAAAATGTTTCTCGTTGTGCTAAATTAATATACATCTACACAGAGGAAAA
TAACTGGCTTGCCCCTTCTACTCCACAAAAGGTTTTTGTCTTCCCTAAGACTGTTTGTGCTATTAAAAAAATTATTATAAACTTATAA
CCTCCAGGAAACCCACTCAGATCTACCTCTCATGGACCAAGTAGGCTCTATCTAGATAAGTACACCTGCCAGTCAATAGCCTAAGTT
CCTACCTCTTACCTATTCCAGAGGGTGGGATTTCCCCCCCTGTTCCCTGGGTTTAGGACTCCCCTCACCTCAACCTCCAAGGCCTAAG
TGTTTCAAGTGTACACCTAAGAAAAAGTTCTTCTCCCAAACCTACTTCCTACTTAACTTTATTCTGCCTCCAATGTGTGTGTGTGTG
TGTGTGTGTGTGTGTGTGTGTGTGTTTCAGCATCTTGCCAAGTCCAGGCATTTACTCAAAATCTACATCCAGAACAGCTCCAGAA
AAGAAACCCGAAGCTTCGTCAGTCTAATCTCACCGATGCTTCTACTGGGCCTGCACTTTCCTACCCACGGATGGCTCCACAGATCAT
GGACAGCAAGGAAATGGCCAACTCTCCTAAGACTGGGCCAACATCCCCATCTTCTCTTTGGTTTCCCAGAGCCACGCCACCCCAAAG
TCAGCAGGAAGTTGCAAAAGATCACAACGACCCTATTCCTGTTTTGTAACCACCCCCAGCCTGAAGCAGGCTGAGCCAGACCTTGAC
CTTGCTGCCACTAAGGAGATTACCTAGGGTGGAGCCTGCCTCTCTAGATCACTCTATTGTTCAGCCACTGCCACTGTTCTCCTTCAA
GACACTGCTACCTGCTGGGAGGCCACTGAGCTATTCCAGAGACTACACCCTATCCTGCACATCATCACCTGTAGCCTGTTCCAGGCT
CCAAGAATGAATTGGCGGCAATGGGCCTCCCCCCTACCCCCTTTATAAGTGCATTTGCCATTAAACATTTGGGCTTTGATCAGAAAC
CTTGACTTAGCTCCATTTTTCTCTCAACCTCCTAGTTCCCCTCTCTTTTCAGCTCCAGCTTGCCTCCCAGGTGAAACCCGGTCCATG
TGAGCCTCGGGTTGGCTTCCACAGTCTGGCGTCCAAACAGGGACCTGAGGAATGGCAGAAAAATGCTGGAGGAAAGCTGCTTGGTA
TGGAGCTCCACGGGAAGAAAAAGAAAAGGATTGTATCGGCACCGAAGCAACAGGTACACATGTAGTGCTAGCTTAAAACTTCATT
TTTTTAAAGTGTTGCTAGAGGTGCGGGAGGATATGGGCTAAGTTTGGAATCGGTGCTAACCCAACGCTGGTTTCCACTTAGGTTCAGC
AGCGAATTATTTGGAATTAGGAACTGATCAGGCGATTGTCTGCAGTTTTCAAAAACTGCTTTAGGTGAGAGAAAAAAGGGTTTAAAT
TAAATAATCAGGCAGATGTCCGCAGTCAGGAGCTGCATCAGGCGGCAGGAACAGGGTTTGTAATAATAAAATAATATAGAGGAAAT
GGATTTTCAAACTCTCCCCAGAGGCAGAGAAAAATTGGGGGGATGCCCTGCCCTTTTCTCTCTGGCCCCTACAGCAGAAGTTCTCTGC
CTTCTTTGTGTTGTCTAATATCTAGTCTAATGTCTGGTGCACCGGTCCTTTCACATGTCAATCCATGTGTGTTTGTGTCTAGTTGTC
TGAATGACTGAATGTTCTGTGTTTCATGTTGGAAAATAAAGATGGCTAAAACTTTATCTGCTGGTTCAGCAAAGCCAAGAGTGGCCA
CATGCTTTAGCCAAGGATCAGGTACAGCCGAGAAGCCTCTGCTGGAAAAGCTGCTCTCAAAGAAAGGGAGTCTGCAGCCTCTTAGTT
TGGGGGCAAAAAAGCTGATAGATGTTTTTATTCCTGGAGGGTTCTCTGCAATCGTGATTAATGTGTTTGGCAAATGGCAAAGTGCCT
TTTTTATCTTATAATTATAGCTAGAAAAATTGAAATTCTTTGATTGATCTAAATTCATAAGACTCATGTAGCCACTTCATTTGGTTT
```

```
TTTACTGGTCTTAGAGGTGTAATATGCTCTGCGTGTCTTCATCGTAGAGTATTAGCTTATAAGTTATTGGGTATGATCAAAAAGGTG
TAACACTGGTTACTAGAAAGTTAGCTTTAAATTGGTAACTCAGATTTGGAGTCTTTCCAAAACATGGCATAAGGCAGGCCAAGAGGC
TGGGTCTCTAAGGATACTTCTAGTTGAGATAATATTTAATGTGATTCTTATCCTAGAAAGAATTAAAGGTAAGATTTAAAGCAATGT
CTCTTTAATGAAGCATTAAGACTTGCACTGTCATGCATTCATAGGTATAAATTGGAAGCCAAATTTTGTAACGTGATAGTGAAGTTT
TGATGTTGATTCTAAGGTGATAAATTGCTTTGAAATTGGATTTTGCTTTCCCAAAGTTGTAGTTATAGTCTAATATTGCAAAAGAAA
CTTAAAAATTCTAAAATCTGTCCTCAGGGCAAGATGAACAAAGCTCAGACCAGCCTCTGCTGGGCTTGTGTGTGACTTTCTTTTGTCTT
GCAAACAGTGCCTAAGGAAGCTCTGGAAACTTGCCTCTCCTTGCCTTCAGGGAAAATTCCTTTTAGCTAAAAGAACACTGTTGCAGA
TCTATCCTGATGTTGTTCTAAATAAAGTTCAAATTCAAAGTTTATAAAAAGGTCAATCAGGCTGTAGAATTTATCAGAACATTGCGA
TTTGACTTGCCTACTTCATGTAAAGTTATTATAGATTTAAGAGTTTGTTTTTTCCTTTGCATCTGAAAGAACCTAGTAGGGAAACCC
CCACTCAGCTCCTGATTCAGCGTGCATGCAAGAATCACGAATAGAACACAACACCTTGATGTAACAACACGAGGTATTTTAATGGCG
GAGCTCTGGGTCGAAACGTATCTCACACAGGAGACAGTGGATTCGACCACAAGTCTTGGAAGCTAGGGGTTTTTATAGGAAAGGAGT
GGGGCTGGGGGAGGAATTGGTGCGGTTTCACATGATTGGTCTATTTAAACATCAGCAGCCAGTAACATTTAACTTAGGTCAGAGGGG
TGGGAGATAGGGAGGCGATGGGCCTGCCCGGGCATGTCCTGGTCTGTTCTGCTATGTTCTCAGCCCCAGGTTTCAAAGCTCACAAAC
AACTCTTTGGGCTATTTGACATACATTACATGAATCACAGGTCTCAAGTTTTATTTCCTTTCACATCCAGAAAATTGTAAAGAGTTT
GCTTCTAGTGAGCTTGTAATCATTGGAAAATTTTGCCTCTGGGTACGGCTAATATAACTTTACCTCATGTAAGAGAAATTCTTATTG
TCTTTGCTTCAATACAAGACGCTAAGAGTTTGAATCTTTCAGTATATATTGTTTCCTAAGTGGAAAGTATTTTATTAGCTAATGCTT
CTCAAGGGAAGTTTACTTTAAATCCTTGCTCTCACACAAGGAGCTTTTAAGTTCTGGGGAGTAGCTGTTGCTCCAGAAAAGATTCAG
AGGCAATATCTTTTGAATACTTAGGGTTTTGGTTATACTATAAAATTGTGGTACAGAAATCTAAGAACAAAAAATTAATTTGCTTC
AAAGTTTTATTTTCAAAAGCTTCCAGGAGATGTTAATTGGCTAAGACCTCACCTTAAACTCACCACAGCAGAACTTAAGCCTTTGTT
AGGTGCTATTAAGTGAGACACAAAATAACTGGCAAAGAACAAAAGGCTTTGCAGAAAATAAAGAAAGCTTTTATAATTGTTATCAAT
TATAATCAATTAAATATTAGCTGCCTATTTTAGTTATTGGTCATTAAATGTGCCCACAGCAATTCTTTGGCAAGAGAAAAC
ATTGTAAAGTCAGCTTTCTTCTCCTCCCGGCCGACCATTGGCCCACGTGGGAGCCTGCCGGCTGCTGGCTGCTACAGGGCGGGGCCTG
GGGTACACAGCTTTCCCTGAGCAGTGGTGGTGTAGGAGGCCGCCAGAAAGCGGGTGCCTTTGTAGCGAACTCAGGGGAGGGCCTGAA
GCACCGGTTGCACGCGCCATTGCCCCTATGAGCATCCACTTGTTTCCATGGCCGTATTTTTCCTGCTAGGACTCGGGGTGCTCTGCC
GCCCTTGCTCGGGCTTCCTGGCCTACAACAGATTGGGCTGCTTCAGCCTCTATGGCCTGGGAGGAGGGCCGGAGGCAGGCAAGGCCA
AGGTGTCGTTCACCCCAAAGCACAGTTTTAACATCCTATTAAGGCTGCTGTGGTGCTAATAAATAATTGTAAGATAAATTTGTATAT
TTTAGAAAACCTATAACAAAAATTGTGTCTTAAGAACCTGACAAAGTGTTCCAAACAGCAATTAATTTGGTTATTACAGAATATTAA
TATTTGATCTATTGCATACCATCATTTTAAATTAAATTGTCAATCATTATCCCAAGGATAAGTTAAAAAATTGTTTTTATTCATGCT
TCTGTATCTTCTATAAATTCATGCATGCATGCATTCCAATTACTCTATTTAAAAACAGCCCTTCTATGGGAGAAAGGTATATGTGAA
TTGGATCACACGCTTATTCTTTTGAGTTTCCCCCTGCTTCAACACAGATAATTAAATTGTGTGCTCTAGATGGTGTTTTAAAATGTT
GAATAATCAAGCTTTAATTTGTATATTTATCAATATATATCTCAGAGCTTACAATTGCTTAAAATTGTTCATCCCTCAGATGCTATT
AATTCTCAAGTTTGCAATTGCTTATGCAACTCATAAGAAAAAATGCTGTTCTTTTAAGAAGACTGTTTTTGGACAGTTAAGAATTTG
TCCTGGGTTGCCTGGAAAAGACCCCCAGGATTTGCTTTTGTGTTATAGAGATTTACAAAAAGCTTTAGCTGATAAAGGATTACAGAT
AGCTCTTGAAAAGATACAAACTCCGGATCCTTATAATTATTTGGGTTTTAGACTTACTAATCAAGCTGTTTTTCCCCAGAAGATAGT
TATTTGCAGAGACAACTTAAGGACTTTGAATGATTTTCAAAAATTGTTAGGTGATATTAATTGGTTTCACCCCTATTTAAAGCTTAC
TACAGGGGAGTTGAAACCTTTATTTGATATTCTTAAAGGGAGTTCTGATCCTACTCCCCAAGATCTTTAGCCTCAGAAGGATTGCTG
GCCTTGCAACTAGTGGAAAAAGCTATTGAAGAACAATTTTTCACTTATATAGATTACTCTTTGCCTTTACTTCTGTTAATTTTTAAT
ATGATTCATATGCCTACAGGATTGTTATGCCAAAGTCTCCTCTAATGTGGATACATTCGAGGATTCTCCTAAACGTAATATCTTG
CCATATTATGAAGCAGTAGCTCAGATGATTATCCTTGGAAGGAAGTAGGCGCTAACTTATTTTGGCAAAGAGCCAGATCTTCTTGTT
CAGCCTTACAGCATAGGTCAAGACACTTGGGTAAAACAGCACAGTACAGATTTGTTGCTTGCTCAAATAGGGTTTAAAGGAACTGTA
GACAGCCACTACCCTCAAGATAAGTTGATAAATTTTTTTAAATGTATATGAGGTGATATTTCCTAATATGACCTCTTTACTACCCTT
ACACAATGCTGTTCTGGTGTTTACTGATGGCTCCTCTAAAGGGCAAGCTGGATATCTTATAAATAATCAACAGGTAATCATAAAGAC
TCCTGGCCTCTCAGCTCAATTAGCAGAATTAACAGCAGTACTGAATGTCTTTCAGTCTGTGAATGAGGCTTTTAATATCTTTACTGA
TAGTTTATATGTTGCACAATCGGTACTCTTATTGGAGACCTGTGGTACGTTTAACTTTAATACGCCAGCAGAATCCCTGTTTTCACA
ATTGCAAAACATCATTCTTGCCCGAAAATGCCCGTTCTATATTGGCCACATATGATCTCATTCTGGTCTTCCTGGACCTTTAGCTGA
AGGCAATGGTTGCATTGACAGAGCTCTAATAGGGAAGTTCTTAAATTCAAATCCTGTTGCTTGGCCAAAGGTGATCATGAAAAATT
TCATCTCTCTAGCCATACCTTAAGGCTCCAACATAAGATCACCGAGGAGCAGGCAAGAATAATTGTAAAACAATGTCCTAAATATAT
TACATTATCTCCAGTGCCACATTTAGGAGTTAATCCACGAGGTCTTATGCCTAATCATAGTTGGCAAATGGATGTAACTCATTATGC
AGAATTTGGAAATTTTAAATATATACATGTTTGTATTGACACTTGTTCAGGATTTCTTTTTGCTTCTCTACTTACTGGAGAGGCCTC
TAGCAAAAATTTTACTTCATTTTGTAAAGAAATTTGGCATCAAAATTTGGCCTATCCCCATGGGACAAAGGGAATAGT
TGAACATGCTCATCACACTTTTAAAAAATTGGCTATATAATCTCGATCTTTATATATTCAAAACAGTAATGGTTTAAGATAATATTTT
GATATTTTTGGAGAATGTGTGTGTCAAATTGTAAAAAATTTGCTCTTGGTGTCCTTAGTTCCTACCTGTTTCCACATAATGGTGTTA
ATTCTTGAGGACCTATACTGAATAAATTGCTGCAAATGAATGCTCTTATTTCTGATTAATAAATAAATTATGCACATGTGACTATTA
ATAACTTTTCAAGCTTTCTAGTTGCAACCGCTCTTCAAGAGAAACAATTGAAAGTGTAATTAGTCACTGCCCTTGTTACATTAAAAC
TGACTTTAACAGTCAAGTATTTGAGATGTTTTGTCAACAATTTAATCTCAAAGACAGGGTATTGTGGAACTTTAAAAACTCTT
CTTCTTAAAAAACAAAGAAAGGGGGAAATTTATATCCCTGTGCACATTATTTAATATATCCCCATGCACATTATTTAAATCATACTTT
TATTTTAAGAATTTTGAAATTTGGATGTCAAAGAACTTAATAAATGCCTCATAGTATCTTAAAACCAGACATAATCATGTCTAGGTG
AGATGAAAAGGATCTACTTATTGGCATATGGCTTGATCCTGAACAGCTACCTTATTGGGAAAGGAAAAAGCATAGGTTCTCTTCACA
GAACGCTGCAGAAGCATGACGGCTAATTCATGTGACAAGCTGACAGGTGAGTTCGCTGAGTGCCCTGACTGTAGTGACAAGAAAGCT
GAATTGGGTATATGTTTTTGACCCACCTTTGCTTGCCATTTTCCCAGTGTGGACAGGCTAAGCTGCCTTGCTTCAAGCTGTTTAAAC
CTTATGGGACATAGAACATTAGACGTGTGTAGTCTGACTTAGAAAGAATTAGTCACAAGAAAGAGTTATAGTGATTCTTCTCCTTCC
CTCATTGTGACCAGTTATTCTAACTCAATCTTGAACTGGTCTAGTAATAATTCCAGTATTAGAGAATCTCAGGGAAGATAGCTAAAA
TCTTAATTACCTAGCAAAGTTAATTTGGAGCACAGCCTCCACTCTCAGAGAAGCTGCAACCTTTCTGCAATTCTCAAAGCCACCTCC
CCTACACCAGGAGCTCATAGTCTAAGCTTGATCGTTGCTAATTTGCAATTGAATGGAGTACCTGGACTTCCCCAAAGAAGCTTTTGT
ACTGTTGCTTTTCACTGTCTCAACTTTGTTTTTCAAGCAGGTCAGCCAATGCCCTTCAGCCTGACTTGGCTGCAGGTGTGCAGCCTTGC
CTCCAACAGCGCAGGTGGCAGTTATTAATCTTCATGAAGAAGCATTCTAAGTGCATATTGTGGCTTTGGACTGAATTGGGAATAAGG
TGTGCTAATGAGTGCCAGACAGTCAAAGGTGGGCACCCAGATCTTGGAACCATACCAATCTAAGACAGAGGTACATGCCAAAAGGCC
GTGGTTGTTGATAACACACCACAGAGCCATGTTTGTGTGAAATATAACACAAACAGCTGCATGTAGCCTCCAAGATGGAATTGGAT
GGTTCGGGAAAATCGAAGATTTCCTAAGACAGGCGCAGCCGCCAGCCACCATAAGTCCCAGGCAGGACTAAAGAGCATAAATAAATT
TTATGCCTCAGTCCAGCTTTTTTTAATATTAATAACGGGGGGGGGGGGGATTGTAACTACCCCCAGCCTGAAGCAGGCTGAGCCAGA
CCTTGACCTTGCTGCCACTAAGGAGATTACCTAGGGTGGCCGTTCCTCCCTAGATCACTCTATTGTTCAACCTCTGCCACTGTTCT
CCTTCAAGCACTGCTACCTGCTGGGAGGCCACTGAGCTATTCTGGAGACTACACCCTATCCTGCACGTCACCTGTAGCCTGTTCCA
GGCTCCAAGAATGAATTGGTGGCAATGGGCCTCCCCCTACCCCCTTTATAAGCGCATTTGACATTAAACATTTGGGCTTTGATCAGG
AACCTTGACTTAGCTCCACTTTTTCTTTCGACCACCTAGTTCCCCTCTCTTTTCAGCCCCGGCTTGCGTCCATGTTAGCTGCGGGTCG
GCTTTCAACACTTTCACCATCACTGCCGCAGACCCTCTGGGTCCCTTTGTCTGTGTGGAACGAGTCTCTGGGCAGGTGGACAAGGCG
```

EP 2 204 376 A2

```
TCAGCGGGTGACAGACAGACCAACACGGGAGATTGTGTAGAATCTGAATGTATTCTCACAAAGTGAACACCAGTCTTATATAGTACA
GAAAATAAAAAAAGGTAGCAGGCAAAGAACATTGAAGTTACCTGATACAAAACAAAGGAATGACTTCAAAAGGATTTAGAGGAACCA
GGTAGATGTTTACAGTAAAGATAAAGCAGTCCTGCCTAGGGTCAGCTTAGTGACAGGTAAGGATTTCACACTCTAGTGCTATACCTT
TGAACCTTGTGAAAGCTAGCACCAGGGGGTTCTTCTCTTAGCAGGCCTCATGAATAATGCAACATCACAAACCCCTCCCCTCCCCAT
TTCCTAGGCCTTGCCAAACTCCCACATGAGTGTAACTTTTAAGTAACTGGAGAATCTCCATTTGTCAGGAGAATTCACCAACTTGCT
TCTAATATGCAATGCAGCCTATACTGAAGATTTCTATCTCAGTGGGATTCCCTGGCTCTATTATGGTATTCCCTTGTTTGGGTGAGA
TTTGCTACTGTCCTTAGTAAATATTTTGAAGACCAGCTCTTAGCTACTGGCTCCGTCCTTTGTAATGCTCAATTAGTAACTGAATAG
GTTAACTTCCTTACTGCATTCTTACAGGATTCTAAGCTCAGCTTCTGGGACCTGGAAACACTAGGGAGACTTAACTATATATAACAG
AATTTAATCTTAAAAGGCATTTATAATAAAATATGAAAAGAGAGCTCATAGTTCCATACACCAGACTAACGCGGGAATAGAGTATGA
GTATATGGGTAAATGAAGATGAACGCCAAAGCTCCAGGAGGTGAGTTTCCGTAAAACTCTTTTCCTCGTTGAGTGCTTACAGACTTTCAGC
CTGTCAAACAGACTTGACCGGAGGGTGTGGCATATCGCCTCTTTCTAGTTTCTTAATTTTTTTAAATTTAAACCAAAATGGAGGAATGT
TAGCATTTCTTCTAAGCTCCACCCAACAGTTACCTAGGAACAGCCAGGTACCAGTCTGGCTTGCTATAAAAGACTGTTTCTCTTTC
TCTCTCTCTCTCTCTCTCTCTCTCTCTCTCTCTCTCTCTCTCTCTCTGAATGTTTCCAGGATCAAATTCTGAAGT
TTCCAGCAACCATAACAGTATTACACACTGAACAGTTTTTATGCATTGGGTTCATTTCTAAGGCCTGTAAGTAAAAACATATTTTCT
CCCTTAGCACTACACCTGTGTATATGACTGCAGCATATATGAAGACAATGAAAGGGAACAAAGATACTGTCCACACTAGGAAGATAA
CAGGTATTATGCAGATACAGATATATACACAGCACACACATACCTGCATGTAGACACACGTGTCTTCACAGTAATTCTATATGCAGA
TAGTGAGATGGCTCCCATCATACAGATGAGAAACAGAGGCCAGGGAAGCTGAGTGCTGTGGCCCTGGCATAGTGGTGGCAAGGCATA
CCCAGGGCAAGGCATGGTACCTCTGCCTCCCTACAATGTGTGGCAATAATCTAAGATCAAACAACTAACAGAATAGAAAGCAGCATC
CTTCCCTTGAGTGCCCTCAGCAGCATGGCAAGCTGCTGTTGGGTAGGCTGGTCAGGGAAAGGATGGAAGGCAGAGGGTGACCTCACT
ATACAGGACACAGAGTCCTCTAAGCAGCACCCACATCTTAATTCTCCATTCTTTGTACAGTGGCCTCTGAACTCAGGGTCCCTACA
GATACGTGAGTGTTCTGATGCCACAGGCGTCCCTTGTGGCCCTGCCAGCTCTGCCCTTACCCTGATGCTTCATCTTCCAGGTATCT
TGTCATTTACTCCCATGAAGCTGACAAGAATCACAGACTGTAGGCTCCAGAAGTCGGTTACCTCAGCACTCCATAGATGAATCCACA
GACCTCCCACCTCCTGCCTCAGCTGGTACACCCTTAGCATTCTCCTCAGGAGAGAGAGTGCTCTTTGGTGAGGCTTCGGTGCATCCT
CCACTCTCTGTACCAAGTGGCCAGGTCTCAGGGCCCTTCTGAGCTCCCACCAACCCCCAGGGATAGTCATATCTCCTGGCTTCCTCA
GTGACCTTCAGTTTTGATGGCTGTTGCTCTCCTAGATTAGGACCCATTGACTGTATCTGGAAGTCCTGTTCTAGGGGAACATGGTGT
CAGCCGGGTGGCCCTCTGTACTGCTCCACCAAATGGGAACACACACTCTGGACCTCGGACTCATGAGGAACTCCTTATGGCTCGGCT
GCATTTACTCTCTTCTGATTGTCTAAAAGTTAAGGTCAGCACCTGGTGTGAAAGTCAGGATCAGCAGCACCTAGGACTCCATAGTCC
ATGTTGAGCTGTAGAAAGTACTGTGCAAATGTAGGTAAACCATGAGGAAGAGAGCAATATGGAATCCTATACACAAGAAACCCAGAA
GAACCCTGCTCTTTCCCAAGTGAAATGTGAGAACATCTGGGCAGGAAAGACTGTCTGCCCAGAGTTAGGGCTTCAACTTTCCATGCG
CTCGTGATCACCCAAGGACGTGGTGGACATGCAGGTGCTCATCCCAAGTCTGGGTAGAGCCTGAGATTCTGCATCTCTAATAAGCTT
CCAGATGGTACTGGGATCTAATAAGCGTCCTTGTTGATTTTCTCAAAGAACCAGCTCCAGGTTTTGTTGATTCTTTGTATAGTTCTT
TTGTTTCTACTTGATTGATTTCAGCCCAGAGTTTGAATATTTCCTGCCACCTCTCCTCTTGGGTTTATTTGCTTCTTTTTGTTCTAG
AGCTTTCAAGCGTGCTGCTCCAGTTTCTTTTTGGAGGCACTCAGCTATGAGTTTTCCACTAAGCACTGCTTTCATTGTGTCCCATAA
ATTTGGGTACATTGTTCCTTCATTTTCATTAAATTCTAAAAAGTCTTTAATTTCTTCCTTTATTTCTTCCTTGACCAAGTTATCATT
GAGTGGGGCATTGTTCAGCTTCCATGTGTGTGTGGGCTTTCTGTTGTTTTTGTTGTTATTGATGACCAGCCTAAGTCTGTGGTGATC
TGATAGAATGCATGGGATTATTTCAATCTTCCTTGTATCTTTTGAGGACTGTTTTGTGACCAATTATATGGTTAAATTTTGGAGATGGT
ACCATGAGGCGCTGAGAAGAAAGTATATTCTTTTGTTTTAGGATAAAATGTTCTATAGATATCTGTTAAATCCCTTTGGTTCATAAC
TTCTGTTACTTTCACTGTGTCTCTGTTTAGTTTGTTCCCATGATCTGTCCATTGATGAGAGTAGGGTGTTGAAGTCTCCCACTATTA
TTGTGTGAGGTGCAATATGTCCTTTGAGCTTTAGTCAAGTTTCTTTTATGAATGTGAGTGCCCTTGCATTTGGAGCATAGATGTTCA
GAATTGAGAGTTCATCTTGGTAGATTTTTCCTTTGACCAGTAGGGAAGTGTCCTTTCTTATCATTTTTTTTGATAACTTTTGGTTGAG
AGTTGATTTTATTCGATATTAGAATGGCTACTCCAGCTTGTTTCTTGGGACTATTTGCTTGGAAAAATGTTTTCCAACATTTTACTC
TGAGGTAGTGTCTGCCTTTTTCACTGAGGTGTGTTTCCTGTATGCAGCAAAGTGTTGGGTCCTGTTTATATATCCAGTCTGTTAGTC
TTTGTCTTTTGGGGGAAATTGAGTCCATTGATGTTAAGAGATATTAAGGAAAATAAGATTGTTACTTCCTGTTAATTTTATCGTTAG
AAGTGGAATTATGTTTATGTGACTATCTTCTTTTGGGTTTGTTGAAAGATTACTTTCTTGCTTTTTCTGTGGTGTAGTTTCCCTCCT
TGCGTTGGTGTTTTCCATCTATTATCCTTTGCAGGGCTGGATTTATGGAAAGATTATGTAAATTTGGTTTTGTCATGGAATATCTTG
GTTCCTCTGTCTATGGTAATTGAGAGTTTTGCTGGGTATAGTAGCCTGGACTGGCATTTGTGTTCTCTTAGGGTCGTGATGAGATCT
GCCCAGGATCTTCTAGCTTTCATAGTCTCTGGTGAGAAGTCTGATGTAATTCTGATAGTTCTGCCTTTATTTGTTACTTGACCCTTT
TCCCTTACTCCTTTTAATCTTTCTTTGTTTAGTACATTTGGTATTTTGATTATTATATGACAGGAGGAATTTCTTTTCTGGTCCAGT
CTCTCTGGGGTTCTGTAGGGTTCTTGTATGCTCATCAGCATCTCTTTCTTTAGGTTAGGGAAGTTTTCTTCTATAATTTTGTTGAAG
ATATTTACTGGTTCCTTTAAGTTGGGAATCTTCACTCTCTTCTATACTTATTATTCCTTAGGTTTGGTCTTCTCTTTGTGTCCTGGATA
TTTGGGGTTAGGAGATTTTTTGCTTTTTTGCATTTTTCTTTGACTGTTGTGACAATGTTTTCTAAGGTATCTTCTGTACCTGATATTCTC
TCTTCTATCTCTTGTATTCTGTTGATGATGCTTGCATGTATGACTCCTGATCTCTTTCATAGGTTTTCTAACTGCAGGATTGTCTCC
TTTTGTGATTTCTTTATTGTTTATATTTCTATTTTTTAGGTCTTGGATGGTTTGGTTCATTACTTTCGCTGGTTTGATTGTGTTTTC
CTATAATTCTTTAAGGGATTTTTCTGTCTCCTCTTAAGGGCTTCTAGCTGTTTACCTGTGTTCTCCAGTATTTCTTTAAGGGAGTT
ATTTATGTCCTTTAAAGTCCTCTATCACCATCATGAAGGATATTTTAGATCTTGCTTTTTCTGGTGTGGCCAGGACTTGC
TATAGTGGGAGAATTGGGATCTGATGATGCCAAGTAACCTTGGTTTCTGTTGCTTATGTTCTTACGCTTGCCTCGCACCATCTGATT
ATCTCTAGTGCTACCCTCCCTATCTGACTGGAGCCTGTCCTTCCTGTGATCCTGGGTGTGTCAGAGCTCCTGGGAGTCAAGCTGTCT
CTGGGACCCTGAGATCCTGGTGTGACCAAGCTCCTGGATCCTGGGATCCTGAGATCCTGTAGTCCTGAGCGTGTGAGTGCCTGGGAG
TGGATCTTCCTTTGGGTGTTGTGGAACTGGCTGTGTGGATTTTGCGCCCAAGGTCTGCTCAGGGCACTGGCCCAGATCTGAAGGTGAAT
TTTTGTTTCTTAACCCGTTCTGTCTGTCTGTCTGTGTTTTTTGATTGGAAAATTGAGGCCGGTTGATATTAAAATTATTTATTTAAATATGT
GTGTTGATTATGGCCATTATGTTATTTTGAGTTCTTGATGGTAGTTTTGTTGGTTTTGTTTGTTTGTTTGTTTGTTTTAAT
TATGGGGCTTCATATATCTTTTCACTGCCTCTTAGCTTTGTTCATTCTTCTCATCCAAAAACAATGCTGTCTGTATTTTCCTTAGGT
TTGTTCTGTTGGATATGTTTTTTAGGCTATTTATATCACGGAAATTTTTCTTTCTCCTTCAATCATGGAAGATAGTTTTTCTGGGTG
TAGTATTCTAGGTTAGCATCATAGTCTTTTAGGACTTGGAATGCATTGCTCCAGCTTACTCTGATCTGTTTTCCTTTAAAATGACTT
ACATTTTTTTTCTTTTACTGCTTTTCAGTACACTTTTATTTATTATATATATTTACTGTTCTAACTATGATATGCTTTTGGGAATTTTT
TTGTTCTGTCTATTTAGTGTTCCATGTGCTTCTTGTATTTGTATGGGTGTATTTCTTTCCACATTTGGGGAAGTTTTTCTTCTATGA
TCTTGTTGAAGATCTGGCCTATGCCATCAACTTGGGATTTTTCTCATCTATGTCTATAATTTGAAGGTTTGGCTTTTTCATGGTGTC
CTACATTTTCTTTGCGATATTTTCCTGTTCTGTTTTGTTTTGTTACCTTTTTATGTTTCTTGTTCATTTGGTCTAGATCCTCTACTT
TATTTTGGAGAACTAATATTCTATCTTCTGCTTGATTCATTTGACTTGTAAAGTTCTACTGTGAATTTCTACAGTAGAACTTTAGCT
ATTATCTTTTTCTTTTTTTATTTAATATTTTTTATTATTACAATTACATTTAGAATGCTATCCCAAAAGTCCCCCA
TACCCTGCCCCCCACTTCCCTACCCACCCATTCCCATTTTTTGGCCCTGGTGTTCCCCTGTACTGGGCATATAAAGTTTGCATGTC
CAATGGGCCTCTCTTTCCAGTGGTGGCTGACTAAGCCATCTTTTGATACATATGCAGCTAGAGTCAAGAGCTCCGGGGTACTGGTTA
GTTCATAATGTTGTTGCACCTACAGGGCTGCAGATCTCTTTAGCTCCTTGGATACTTTCTCTAGCTCCTCCATTGGGGGCCCTGTGC
TTCAGCCAACGCTTGCTAGTGTATGCAATGGTGTCATCGTTTGGAGGCTAATTATGGGGATGGATCCCTGGATATGGCAGTCTCTAGA
TGGTCCATCCTTTTGTCTCAGCTCCAAACTTTGTCTCTGTAACTCCTTCCATGGGTGATTGTTTCCAATTCTAAGAAGGGGCAAAGT
```

62

```
GTCCACACTTTGGTCTTCATTCTTCTTCAGTTTCATGTGTTTTGCAAATTGTATCTTTTTCAATTCTATCTTCATTTCAGCTTGAGT
CTTCCTCAATATTTCTATCTCCTGATTGAATTCTGTTCTCAAGCCTTGGATTATCTTTATCATTTCCATCAGCCTTATGCTTTCTTG
AAACCACCAGTTTGTGGTTTCTTGGACATCAGTCAAACAATTATTTTTCTTAAGCTCTTTGTTCTTGATTTCAGCAAGCTGGTTCTT
TGTTTTATCTTTAAACTCCTTGTGCTCTTTGAGGAAGTTTATAATGGTTCTTTCAAATTCTGTGTTCTGAGGTTCATCTAGGTAATT
CTCATTGTTAAACATTTCTACAAAACGGGTACATTTTAGAGAGAAGATGGTGGTTTGATTCTTCATACTGTTATTATTGCAATGAGT
AGAGCATGTGAACTTCCTTTGTTAGTTCAAGTTTCCTATGGACAGAAAATGTTGGGGTGGAAGGGAAGATGTGGGTGCAGGTTGGAT
CTAGATGTTAGAAATGGCTTAGTGGAATTGAAGTCAGGTAGACAGAGGGAACTGGGATGGAATAGAAGATGTGTTTCCTGTTCCAAT
CATGGAGTGATGGGTAGAAAGGTTAGATATGGCATGGTAGAGTCCTATAGTTTGGGGATATGTAGTGAGAGGATCCTGCCTAGGGAT
CTTTTTACAGTGAAGGCAGGGTATGTTGGTGGGAAGGTTAAGAGATCAAGGCGAAATCCTCTGGACTGTGGAAAGGGTATAAATGGGA
GGTCAGGGATACACACTAGGCTAGACCCTTGAGGGAAAACATGGGAAATCTGGATATTTGGGGAAGCCTCATCTTCACAAAGATGGGA
TATCTTTTGAGGTTGTAGAGTTGGCCCAACAAAGAACTGGGGACAGGGGCTTTGGGTAGGGCAGATTTCAGCAGAAACCTAGAGCTT
GGCTGTAGTATAGACAGTTTAGTCAGAGTAGGCCAAGGCACAGAATGTGAGACCCAGGCTAGAGCCTGGAGGAGGACATGGAAAGTC
TGGGTGCCTAATGGGGTAGTGTGGAAAAGACTTAGAGTGGATCTATTCAGTTTCAGGTAGCACAAAATTTGATAGAAGCCTAGATGT
TAGCTACAGTGCAAGCAAGAGTCTCTAGTCTTCCATTTTCTTTTCCTTCCAAGCCCAATGCCCTGAAGACAACATTGTATGTAACTA
TAGTTTAAGACCAGAGGCTGGCCCCTGTTCAATGTACCAGGTTAAGCCACTTACCAAAGAAAGCAAAGAAAAGTAAGATGAGAGGCA
AGAGAATATTTTATCAAAACAGTTGTTTAGAGAGGCACAAAACACTGGAGCCCACCTTCATGGTCCTGGCATGAGTTTTAACATTAA
ATAGAGGAAGAATTGAGGCAGTGATGAGAAGCACATATAAATAAGCCATCTTGGTCCAAGAGTGTTCCAGTTACTCATCCTCTACAG
TCTGATTCAGCCATGTGGCCCAAAGAAGTCCTTCTTGCTAAGGGAGCAGTCTTGTTCCCCATGAGATGGTTGCTCCTACTTAGGCTA
TCTCATGATGGAATGATTTGTTTGTAGGTTCTGATGTGTCTGGAATACAAGATGAACACAGATTTAGAATCTTGATGAGGGTGTGGC
AGCATTCTTTGATTACACACATCTTTGTTTCTTTAGCCTTGAAGAGAACTGAGAGTATTATCATTTTTGAGACAGTTTTAAGCCTGC
CTTCTGACTGGATTCTCCAGCCTCAGCCTCCCATGTCTAGAATTATAGCAATGCATCACACGGCTGCTCAGACAGATGTTCCTTC
GGTAATTAGCATGCCCAAGTTAACTGTCCAGTTAAGCATAAAGTTTGAAGGAACGGGGTGGGGGGTGGGGAACAATGAAAACTGAAG
TCCCAAAAGCTAGACTTTCACTCAGCTCTGAGTCACCTTACAGGCCCCAGAGAGGGAGTTGGTGCTTTTCATTAAGAGCAATGTCTAA
GAGCCTGTTATAGATTCCCTTTAATATATTTCACCTGCTAAAGAGCATCCAGAAATGGAATGTTCTCCATTCTGTCACAAAAGCTCC
CTATTTTAAATGACGCTGAAGAGCTCATTGATCATTCCACAGCCCTATTTCACAAACGTGCTCTATCCAGTCCACTAAGCTGCATGA
AACTCACTGGATGTTCATTGCTGGCTGGGGTTCTTTGTAATACGACAGTCAAAGCCTTCCTACCATTCAGTCATGAGCTCTTACTA
TTTTGTTTCTTTTTCTTTTTGAAAACTTCATACAAGAATACATTCTTGTGTGTTGAGCATTCATTCCCCCACCCCTACCCTCTGACTC
AGTGCTTCTTTCTCACCAGTCCCTGATAGTTTCATTTCTCCTTTCACAACACACACAGAGACACAGACACACACATACACACAT
TGTGGTTCAGTGACAGAATGCTAAACGAAACCAACCACATCATATTTGCATAGGATCTTTTAGTTCTAGAAACTGTTTGTTCTTTTA
TAAAAGAGGGTTTTCATTGAGTTTCCTGAATCTTGAGGCTGAGAGTCTTTTCACTGGGAGCTGGAAATCCTCACATACAAGAGTCA
CATTTGTTCCTTTGAGCCTGTGTCTTACTCCACAATACATACTCTCCTGAACATTCCAAATTGGTGGTCACCTCCTCTGGTTTTTAG
GGTTTGCTAAGAACCATTATGAAATAATTCCATTTCTTCTAGGCTCTGCCCCACAGTTACCAGGCAACAGCCAGGTATGCCTGACTC
ATTATAAAAGGGGCTACTTGCCCCCTCCTCACTCTCTTGCTCTCTTATCCTCTTCCCTCTCTGTCCCTTCTCTCCCCATTCCCCTCC
CCCATCTCTCCACATGTTCATGGCTGGCTTCTTCTCTTCTACTCCTCTATTCGCTCTCTCTCTCTCTCTCCCTCCCTCCCTCCCT
TCTCAACTCTCAACTCTCCTCTCTCTCTCAACTCCCCTCCCCATGCCCTAAATAAACTCTATACTATATCCATCGTGTGACTGGTCT
CTCAAGGTGACGGGATGCCTCAGCATGGGCCCACAGAGGCACCCCCTTCCCAGCACCATACTGTGCCTCTACCAAACATATCCCTGA
CTTCTTTTTCTTTACTTTACTTTTTTTTTTTTTTTTTTTTATGAAACACAACACCCAGGGCCAGGCAGCGTGTCAGAGAGGAGGAAGAA
TCAATACCCAGGAATGATAAGGAGGTTTTCTGCACACTGTTACTCTCATCCAACTTTTTCATATTTTATAGGAGAACAGGTGATAAA
ATGTCATACATAGATTTAAGACATGCCCAGGCCCTGAAAAGGCTTCTGCTTAGTGGCAATGAACCTAGAAAGGAGCCCCTGAGCCAG
AAGAGAAGTGTACACCAACAACTCTCCTTGCTACGGAGAAGCCCAGGCCAGTGCAGCCCTCTCCAGCTCAGACTCCAGAATCTAG
CTGACCCCTCTTTCACAGCACAGCTTCAAGACACTGTGACCTCCTGTGTTTCAGGCATGGATACCACAGTGTGAAATCCTCACTTCT
CTGCTCCAGGACAAGGTCTGACATCTGTTTCCATCCCTTTCCCCTTCAGCCTCAGTCTTTCCCTCTTGAGAAGACATAGGGCCTCAC
CCCTTTTGTAGCCTAGAGTGCCTGAGCTTGGATCCTCAGCCTCCTCTTCACTGTAAGCTAGTTGCCTCTCTCCCTTCATCCTGATTT
TGTAAATGTTAACAAATATTAAATGCTCTGAAGAAAGGCTAGAGAGATGGCTCAGTGGTTAAGGGCACTGACTGCTCTTCTAGAGGT
CCCGAGTTCAATTCCCAGCAACCACATGGTGGCTCACAACCATCTGTAATCTGATGCCCCCTTCTGGCCTGCAGGCATGCAT
GCAGATAGAGGGCTAATAGACATAAAAATAATATCGAGGGATATTAGATCTTGTCACAAGCCTTTTCTGCATCTACCAAGTTGACCA
TGTGATTTATGTCCATGAGTCTATTTAATTTATAATATTTATTGATTTATTATATTCTGAACATCCCTGAATTTCTGGGATAAACTC
AACTTCATCGTGGTAATTGCCTTTCTGATGTATTCTTAAACTTGAGTTGCAAGTATTTTACTAATCTATTATTGTCTAATATTGTCA
GGGAAACTGGTCTGTCTGTAATTTTATTTTGGTGAATCTTTATCTTTTGATAGCAGAGTAATACCAACTTCATTATAAGAGTTTAAT
AATGTTTCTTCCTTTCCTAATTCCCTCTTTAGTTGGGAAATTTTTAATTTCTGTTTATTGTAGGTTATTTTCATTTGATTATAGGAA
TTTTTTCATTTCCTTCTTGATTTTTTTTCATGGACCCATTTGTCATTCAATACTGCATTGCTTAATCCCCTGAGTTTGTACATGCTCC
AGGGTTTCTCTTGTTGTGGATTTGTAGCCTTATTCCCTAAATAGGATATTAAATATTAGAGCTCTTATATCCAGGCTATTCCCACCC
AAGAGAAGGAGTCCAGGAAGAGTTAGTTCTTCGTGAATTTGAAATTTTTATTGAGCCACATAGACCTGGCAGGGGACACATACTCTT
AACACAAGTTTGTTATCTCCCATTGACTATATAATGGCTGTTAGCAAGCATGACAGGATGTGGACCTTTAATAAAAGCAGGGCA
CAGCTGTGTCTCCATAGTATTTCTGTTTGAGATGATGAGCGAAAGCCTTATAGTTACAGAAGCAGAACAATGCAGGTTAGCAGAAGT
TCCTGTCAGTACCATACATGCCTGAGAGTCAGGCAGACCTCAGCTTTAACAATTACATCTCTCTCACCTCAGAGCAACCACGGCTGT
TTGATATTACCACTATGGTGCTGTACTACTACCCAATAATAACTATGGCCCCTTTGGCTGCAATAACTGTTGGGAGATGAACAACCA
AGGATAGTATGGAGTGAGACCATATCTTAGTTACTAAGATTAAGAGTTGGAGGAAGCTGGGCAGTGGTGGTGCACGCCTTTAATCTC
AGCTCTTAGGAGGCAGAGGCAGGCGGATTTCTGAGTTCAAGGCCAGCCTGGTCTACAGAGTGAGTTCCAGGACAGCCAGGGCTACAC
AGAGAAACCCTGTCTCGAAAAACAAAAACAAAAACAAAAACAAAAACAAAAACAAAAAAAGGAAGAAACTCATTCCTTCATTTA
AAAAAAAGAGTTGTAGAAAAATAAAAATTAAATATTAGACTGACATTGAAATGAGAAAAAAAGTGGTGGCTAATGTATCTAGGAGG
AGGAAGTGCGAGATATTGTTAGTTAGGGTCCACAGATTGTAGAGACTGTTAGCACTATGGAAGGTTATAATTAAGAAATAAATAGCTA
AGGGGATAATAATGAGCAAAAGAGAAGATGTGGGAATAAAAGGAGTAATAGCTGGGGCTGGAGAGATGGCTCAGTGGTTAAGAGCAC
TGACTGTTCTTCTAGAGGTCCTGAGTTCAATCCCCAGCAACCACGATGGTGGCTCACAACCATCTGTAATGAGATCTGATGCCCTCTT
CTGGTGTGTCCAAGTATAGCGACAGTGTACTCATATACGTAAAATAAATAAATCTTTTTAAAAGGGGGGCAGGGGTAATAGTTAAAC
TGAAGGATAAATAATGCCTTAGAGTAAATCATGCAGAAACCACAAGCAACAGCTCTGACTGTAAGCTGAGCAATGTTCAACAGAGG
TAAAACCATCATGAGACCTGTCTGAGAAAGTGGAATTTAAGTATCAGGAAATAATCAATTAAAGACTATGTATGGGGCTAACTGTGG
GCACAGCTATGCGGACGGGAAGGGTAACGTGTCCAGACGCGTCTGGTTCTAGCAGTTGCTATGCCGTGCTGGACGGTGGGGAGTCAG
GTGAGGACCTGCAGTGTCTGAAGCTTCAGCGTCCTGGGGGTCTCTTCTGGGTCTTTTGTGCATCCACATTAATTTTAGAAATTTTCA
TCTAGGTCTAGAAGATATTATTGGAATATTTAGGAAGCAGTATATTAAATGTATGGGTGCTTTAAGTTGCATGGCTATTTTTTTTTT
ATTATTAATCCTGCCAATCTGAGAATGTGGGAAGTCTTTCCGTCATCTATCATCATGGGTCTTCATGTTCTTTGGTGCCTTACAATTTTCACTG
AGGAAACCTCTCACCTCCTCGCCTAGGTTTGTTCCTAGGCCTTTGTTTTTTAAGCAACTGTAAATGGGATTCTTTTTTCCTCCTAGT
TTCTTTCTCAGCAAGTTTGTCATTGGCATGCAGAAAAGCTAATAATTTTGTATGTGTTGGCTTTATTACCTCCTATTTTGCTGGAAG
TGTTTATTGGATCTAGCTGTTTCCTGATGAAATCTTAGGGTGTTGTTGTTGTTTTTGTTGTTTCCTTTGGTTAAAGGCCATGTCCTC
TGCAAATAGAGATGAGTTGACTTCTTGTTTCCTGTGTGTGTCCCTTTTATTTATTTCTTTTATTTTCTTGCTAAGATTTCAAACACT
```

```
ATATTGAACGAATTATTTATTTATTTATTTATTTATTTATTTAATAATATCCTTCCTTGTCTCTTTCCATATCTTAAAGGAAACTCT
CTAAGCTTTCTCATTTTGAACAATGATTGTTAGAGGTTGAGAGATTAACTCGGAAGCCAGGCAAACACAGACCCCGGATTCAGTCTC
CAGCTCCAAAGAGTAGTTGTAGTGATGATGATGGTGATGGTGATGCTGATGACAATATTAATATTGTCATATGTTTGTCATGTGTAT
GCTTATTAAGATAACATGCTTTTTGTATTCCTAGTTTTTCAGGATATTTGTCAGGAAGGAATGTTAAACTTTAAAACCTGTCCTACA
TCTATTGAAATGATCATGTGACTTCTGTCCTTTAAATATATTTATATGCCTAATTACTCTTACTGATTTACATGTGTAGAACCCACC
ATGAATTCCCTGCAGTGAACTACCTTGGTCATCATGCAAGATCTTCCTAATGTATTCTTGAATTAAATTGACAAGAATTTTATTCAG
AACTTTTGCATCTATGTTCATTGGGGAGTTTGGTGGCTACTTTTCTTGTGTGTGTGTCTCGTGTGTCTGTTGTGTCTGTGTGCACATG
CGCACTCCTATCCAGTTTGGAGCATTAGGGTAATGCTGGCTTTACAGGATGCTTTGGGTACCACGCCTCCCTTTCTTATTCCATAGA
ACAGATTGAGAAGTATTCATGTAAGTCCGTCTTTAAAGGTTTAGTGTAATTCTGTAGTGACTCTGCCTGGCTTTCTTTGTTGGGAGA
TTGTTTCTATTAATGCTTGATCCTCAGTGATTGTTATGGGTCTGTTTAAGGTTTATTTCCCAATTGATTTAATTTTGATATGTTACA
TATGTCTAGGAATTATCTATTTTGAGATTTTCTAAGCTACTTTAGTATTATGTTCAAAGTTGCCTCTAAATATTCTCTGGGCTTCAC
TGGAGTCTGCAGTAACCTCCATTTTCCATCTCTAGTTTTATTAGGTTGGGTCTTCTCACTCTTTCCTTTCATTTGCCTCAAGGTTTA
TTAGTTGGGTGTTCTCAAAGAACCATTTTTTATAGATGCATTTGGTACCTTTATTAATTTTATATATTATTCTTTTATTCTCTGTTT
CATTAATTTCTGCCCTGATCTTTATTTTTTCTTGACATCTGCTAGTCTGGGGCTTGGATGTACATGTTTTTCTAGGACATTGAGGTG
CATCATTAGACACTGTTGGACAAGCTGGACGACAGCCTGAAAGCTATTCTAATACATCCCTTTCTGTAAATGATAGATAGATAGATA
GATAGATAGATAGATAGATAGATAGATAGATAGATAGATAGATAGATAGATAGATAGATAGATAGATAGATAGATAGATCCGCT
CTGCTGCTCTCAGTGCAACATCTAAAAAAAGTCAGGGTTTTTTTCCAGGAAAAAAAAAAAAAGCACTTAAAATCCATATGGAATTTCA
AAAGACCCATTTGTGCTGCCTCAAGAAAGTATCTATTACTGAACAAATTATTGTTGCCTAGCAGTTTCCTCTGAATTGAAACATTCTT
CCAGGAGAGGGGAGGAAGACTGTGAGACTCAGAAGGTAAACAGCCTTCTTCGTGTGTCTAGGATAGCTGAAACCCACAAGATTTATG
AGAGGCCCTCTGCAGAGGTGCAGGGGCAATAAACATCTGCTGGGGAAGGAGACTGAGGGGCTGAAAGGAGATTCCTGGGAGTGTAGA
GCTGCCTGTAAGCTGTGCAGGGAGCCCCAGGGTGCAGCTTTCTAAGTCACCACCCACTTTGGGGTAGGGATTGTGCATCATGCAGAT
GCAAGTGACTCCTCACCCAAAGCTGCTGTCAGTGACCCCACTAAGAATTCGCTGGATCACCAAGTTGGATTCGGTGCAATTGCTTCT
TTGGTGTGTTATGGCTGCCTTTCTCAGTGCACAGACGTGTGATGTTCCTCTTGTTGAATAAGTCTTAAGCCCCATTAGAATGAACAA
AGGACAGACAGCGACATTTAAAAAGTGACAGAGACACATGGATACAGAAATGCTGGGGTCAGGTGGGTCTTGTGTACTCTAATGGAG
CCACTCCTACCTCTGCCGCAATCTGGAACCTTAGAATATGTGTTGTATACAGCACAGAGAGAGTGGTTAGCTAGTGTATATAAAAAT
AGATAGATAGATAGATAGATAGATAGATAGATAGATAGATAGATAGATAGATAGATGATCCTGTTTTTCCCACTCAGAC
CCTGCTCCCAGAACGACGACTCTGAGACTAGTTATTAATGCCTAGGCCACAAGCTTTGGCTCATTTCCCTACTAGCTTGTAACTTAT
TCAACCTATTCTAACCATCATGTCTTCCACATGGTTAGTTACCATTCCTCAGCTTCATAGGTTCAAGTCTAGGGCAAATCTCCTCTG
TATTCTATTCTCAGTTCAGCTACCTGCTGGTTTACCACAGTCTCCTCAGCGTTCCCAGGCAATTCTCTCAAGCCTCTCACTATTTCC
CAGAATCCTCTCCCTTCTGCCCAGTGTCCCACCTCCCATTTTCTGCCTCAGCGCATTGGCCAAGGCTTTTATATTGACAGGTGTTGC
TAGTCTGAAGGAAAGGCCATCCAGATACTGCCCCCACCTGGGCTTCATCCCATACACAGTCACCAAACACACACTATTGTGGATGCC
AAGAAGTGCTTGCTAACAGGAGCCCGATATAGCTGTCTCCTGAGAGGCTCTGCCAGAGCCTGACATATACAGAGGCGGATGCTCACA
GCCAACCATTGAACTGATCACGGGGTCCCAATAGAGGAGTTTAAGAGAAAGGACTGAAGGAGCTGAAGGGGTTTGCAACCCCATAG
GAAGAACAACAGTATCAACCAACAAGACCCCCCTCCCCCAGAACTCCCAGGGACTAAACCACCAACCAAAGAGTTCACATGGAGGGA
CCCATGGCTCCAGCCACATATGTAGCAGAGGATGGCCTTGTAGGGCATCAATGGGAGAAGAGGCCCTTGGTCCTGTGAGGGCTCAAT
GCCCCAGTGTAAGGGAATGCAAGGGTGGGGAGGCAGGAAGGGACAGGAGGGGAG
ATGGAATGGGGGGGTGTCTTCTGGGGGGAATTGGGAAAGGGGATAACATTTGAAATCTAAATAAATAAAATAGCCAATTAAAAAAAG
AGAGAGTCTCTCTACAAGCTAGTCTCGTTAGGGTAGGAGATCTCTGTAGGTAAGCAGTCTCTGGGCGTTTACAAGGAGTCTCTGAAT
ATAAACATTTGAGAGGAAGAAGATAGGGTCACTGGGTTTTGCACCTATGGCTCAATCGTTCATAAACACTCCTAGTTGAGCCAGAGG
AAGACGGTTTTAACAATCCAAAGCCTGACCCATGTCATCAACAGCCATACATTCAGCACTTCACTAACTCAAGGTTTCCTCTGCTCC
CTACAGAAAGGGGAGGAGGAGGAGCTTAAAGGGAAGAAAATAGAAAGTGTAAATACTGAAAGACAGAGGGGACTATTGGGGGAG
GTGGGGCTAGCAGGTGAGGGAGCGAGGCATGGAAGGGCAGTGGGCAGCGAATGAGAACAAAGTATAATAAGGTAATGCGTGAAAGTG
TCACAGCAAAACCCATTACTCTGTACACTGACCAAAATAATAATAACTTGAACAAATATTAACTGTTGGACCCTAGTCTCACTGGTA
GAAAACCATCCATGGAATATTGATTGCCTTGTTTATGCTCCCTCTTTAACTCCAATTCCACATTCTGATGTTGCCTACATAATTGCT
ACTCTCCTCAGCAGATTGCAAGCAGCCATGTCTTCTCAACCCTATTTAAAGTGACACTGACTAATTTCCCACAAGTCCTTGGACCTC
TCAGAGGGAACTATTAAGAGATGGTCTGCCACAGGTAGCATCTCTCCTCTCCATACCACTGTCTGCGTGATTATTGTAGCCTGTGGC
TGCATCTTACTTGCTTCTGGCTCTGGGGCAGCTTTTGGCCAACCACAGCCCTCTCCTTTTATACTCAGAGGCAGAGGATCAGAGAGT
TTTGTTGTGAGATAGTGTCTGCTAGTAATATCAGAAGCTATACTCACACAGTCTTACCCACGTGACTGCCCAAACATGCGCTGAACA
AGGACAATAACAGGCATACCAAAGTGGACAGGGAAAGCCCAGGAGGCCATAGCCCTACACAAAGGACTCAGACAACGAAGGAATGCT
GACTATAAGAGAAACAGTCTGCTCCAGGGAAGAACACAGCAATGGTTAGTCAATGCCAAGGAAAACATGCATACGAGTAACATTGTA
CAGACTGAGGAATATCTACGTATAGGCATATACATAACGTGCTTGATCAATGAAAAGAGGACATGAATTTGGAAGGAGGAGTA
TATGGTACTGTTTGAAAGGAGGAACAATAGAAAGAAATGTTGTAATTATGTTATAATCTCAAAATAAGAGCAATAACTTTAAAAAAA
ATTCTAATCATAACCCAAAGGATGAGGTAGGAATTGTAAATCCATGAATGTGCAGCATTGTTCCCACCTTTCTCTCAGGTGCAAGCC
TCTTGCTGAGTTCTCTCAGGTTCGAGCCTCTTGGTGCCCCATGGCAGAAGTAACTGCTTTACAGTTGACTCTTCAGAAGATAAAAGG
CCCAGTTTCTTGTGTTTTTCCAGCTTCTCTGCTATAAATATCACCACCGTAGCTGATTGCAAACTATCAGCATTATAACTGAACACAG
AGTTTGGAAGCGACCACATAGATCTAGCTCTTAGCCTGTTCCAGCTGGCCCCGGCACATGGCAGCCAGCAAATGCTAATGTTTAAAT
GTGGAGAGTAGTTATTTTCTTTTAATTTTTGAGACTGTCTCACTGTGTAGCCCTGGCTGGTCTGGACCTCACTATGTAAACAAGGCT
GGCTTCAAACCCAAGTTCTGGGATTAAAGGTACCTGGCTTATAAGGTACACTCTTTGTATACCATGGACCCTAACTAACTGCCCCTA
TTTTTTTAAAATATGTATTTTTTTTTAGAATTTCGCACATGGATTTAATGCATCTTCATTATATTCCCCTCTTTCCTGATCCACACCC
CCTCCCCCACCACCCCAACTCCATTTCCTCTTTTATTACACTCACTGAGTCCAGTTTGTACTGCATATACACTCACGGATGTGAGAC
CGTCCACTGAGGCCTGGTCATGCTACCGGAGTCCACACAGGATGCCTTAAAGAAAAGCGGACCCTCCCTCCCCAGCAGCTCTCACCTC
TCAGCTAGGAGTGCAGGCTCAAGATCCCCTCCCTACTCTGTGCTGGATAGTTCACTGGCTTGGTCTCGTGCAGGTCTCGTGTAGCCA
CAATAGCTGCTATGTGTTCATGTATGCAGTAGTCCCTCCCGCTGTGTTCAGAAGACATTGCTTTCCTCTGCTCCTCCTTGACTGATG
GCTCTTACGATCTTTCTGCTCTCTCTTCTACAATGACTCTGGAGCCTTGGGGGTGGGAGTGGACGACGTCTCAGTTGCTGAACACCC
AACAGACACTTATCCCTGCACTTCAGTTGTGAGGTTCTGCATTAAGCACTGGTCACTGCATCGTGAAACTTCTCTGGTGAGGTTTAA
GAGATGCTCTGATCTATCGATATGGACTGATTTAGAGCCTGTCCCTCATTTTATAGGAGGAAGTGGCCATCTGCTCTAAAGCAG
AAGGGAAAACATGCAAAGAGAGTACGTTGCTCTCGGCACAGCAGCCACCATTGTGGAAGTCACAAGAATAATTTTAAGTATGATTTC
TACCAGAAACTCTTTAACTTCCTTTCAATGGCCTTAAGAAAGAATTAGGAACTACTCATGTAGTCCCGCAGGGTGGGGCTGAGGTTA
GGGTTACAATATTATAATGAAAGACCTTTCTAACACAATGTTTTCTCCTTTGCTGTCCCACACGTTACCGATGTTCTCAAATATCTG
TCCCCTGAGTGTAATCTAAAGCCACTCGGGATCCAGATAAAGTAATCCATTTCCCACTCTTACTACTAAACTGTATAATCCTAAACA
GATCTGTGACCACCAATGATTCTGGCAAATGAAGGCTTCCCTGAAGATCTGCTGGGCAGATGAAGTAAACGGGTTGTAAAGCAGCT
TCCACTTCCAGGAACAGATGTGCTGAGCCACAGGCATGCAATATTGCCACCTTGTGGCTTCTCACAGAATTGCCCAAAAGGCAAACA
TTCCTTGGAGGTCCAAAAGGAACCTTACTAGGGGTGTGGGGAGTGCAGTCTAGAGTTGTGATGAGATCCAGAGCTATGATTATGAGA
AGCCAGTCGGTATCACAGGAAGAGACTGGCCTCTACCAACCACCATAAAACTGATTTGGGGAGTTGTTGTTTTGTTTGGACAATTAT
CAGCTCTTTGGCCCCCAAAGACAAAATTATCTAAATATACATTTTACAGTTGAAAGGAGAAGATAACAGTTCCTACAGTTTGTTCAA
ACACTCGTGATTATCGGCTGCCCTTCATATTGTTCTTGCCCCTCTTTCCTATTCCAGATTCCCAAGAATAGAATGTGCCTGACTCCC
```

```
ACAGTACCCTATAGATCTGGCCAATCATCTCAGTTTTCAGTGGAGCCATGGAAATGCTCCCAATACCCTCCATCTCTTAGAAGAACA
ATCTCAGGTTCCAATCTCGTCCCCGGTGCTTCAGATCCAAACCTCCAAAATTTCACATTATTTAGTAACAGATTTGGTGGCACGCTC
CTGACAAACTCAAGTCAAGAACCCCGAGGAAAATGGCAGACTAGAAGACGTTTCTACGCCACCCTTTGAATGAGAAGAGCCACAAT
AAGAAAAATAGACACTTCTGAAGAGAAAGAAAGAAGAAACGTTTTAAAATGCACCAAAATAGTGTTAAAATACCTGGAAAGTGGGGA
GGAAAGACAGGTAACGCATGAGAAACAGCCAACGTAAACACAGACCAGCAGCATGGCTGTGATGTGGACAGGGTGAACAGACTTCTC
ATAAGCTAAAGGAAGCTGCCCTGGTGAGAGAAAACTGGCCGTCCTGCCCTCAGAGCAAGTCCTCAGCCCTCACAAGCCTGAAAACCA
GTGGCAGCTTTTGTGAGACAACCATTCCTCCGGCACTGAAAGAGGAAGCACACCCAGCAGCTGCGTGCGCCTCAGAGCGGAAGTGAC
CCGGTTTGAATGAGAAGGTCTCCCACAGGCATGTGGATTTGAACTCTTGGACCCCAGATGCTAGCACATTTGGAGAGGTTATAGAAC
CGCTAAGGTATGGAACCTTGCTGGAGAAAGCGTGCCCCTGGAGATAGGCTTCGTTAAGAGTGTAGCCTGCTTCTAGTTTACCCTCAG
CTTTCTGTTTGAGGTTGAGGATTCAATCACCCCCGCTTCCTGTTCTGGCCCCTCAGCCTGCGGTGGACTCTGCCTTTGGAACCAGAA
GCCAGAATCGGCATGTTGTTCCTAAGTGGCTGCTGACTGGCCATGCCATTTTCCTCACAGCAACAGAAAAATGTCTGATCCAGACGT
TGGTACAAAAGAGTGGGTACGG


MOUSE mRNA SEQUENCE : mR27-007.1 (Seq ID No: 12)
CAGATTTCTCCTTGGAGCTGTGAGTGACTACCATTGCAGCCAAGAGCAAGAGGAAAGCACTACCTGTGAGCAGATGTCTGGTTCATT
CTCACCCTGTGTGGTGTTCACACAGATGTGGCTGACTCTACTGGTTGTGACTCCTGTCAATGGACAGCATGATTTTCTGGTGCTGCA
AGCTCCACCTGCTGTGTTTGAAGGAGACTCTGTGGTTCTGAGGTGCTACGCAAAGAAAGGCATAGAAGCAGAGACCCTGACATTTTA
CAAGGATGGTAAAGCTCTGACATTACATCATCAAAGTGAGCTCTCTATTCATCATGCAAATCTGAAGGACAACGGTCAATACAAATG
CACTTCGAAGAAGAAGTGGTCTTTTGGGTCCCTCTATACTTCCAATACGGTCGGAGTTCAAGTCCAAGAGTTGTTCCCACGGCCTGT
GCTGAGAGCCAGACCCTCCCATCCCATAGATGGAAGTCCAGTGACCCTGACGTGTCAGACCCAGCTCTCTGCACAGAAGTCAGATGC
CCGGCTCCAGTTCTGTTTCTTCAGAAACCTCCAGCTTCTGGGGTCAGGCTGCAGCCGCTCCTCAGAGTTTCACATTCCTGCCATATG
GACTGAAGAGTCAAGGAGATACCAGTGCAAGGCAGAAACAGTGAATTCCCAAGTTAGAAAACAAAGTACAGCGTTCATAATCCCAGT
GCAGAGAGCTTCTGCGAGATTCCAAACACACATCATCCCAGCCTCAAAGTTGGTGTTTGAAGGGCAGTTGCTGTTACTCAACTGCTC
AGTAAAAGGAGTCCCAGGACCCCTCAAATTCTCCTGGTATAAAAAGGACATGCTGAATGAAGAAACAAAGATTCTTAAGTCCTCCAA
CGCAGAATTCAAGATCTCCCAGGTGAACATCAGTGACGCAGGGGAGTATCACTGTGAAGCTACCAACAGCCGCCGAAGCTTTGTCAG
CAGGGCATTTCCCATCACCATAAAAGTCCCAGTATCTCAACCAGTTCTCACCCTAAGCACAGGCAAGACCCAGGCCCTTGAGGGGAGA
CTTGATGACACTTCATTGTCAATCCCAGAGGGGCTCTCCATGTATCCTGTATGAATTCTTCTATGAGAATGTCTCCCTGGGGAATAG
CTCTATACTCTCTGGAGGAGGAGCATACTTCAATTTCTCTATGAGCACAGAGCGATCTGGAAACTACTACTGCACAGCAGACAATGG
CCTGGGAGCCCAGTGCAGTGAAGCTATAAGGATCTCTATCTTTGACATGACAAAGAACAGAAGTGTTCCTATGGCTGCCGGAATCAC
TGTGGGACTGCTCATCATGGCTGTTGGAGTGTTTCTGTTTTATTGCTGGTTTCTCTAGAAAAGCAGGAGGAAAGCCTACCTCTGATGA
CTCCAGAAACCCTTCAGATTCAGAACCCCAGGAGCCCACCTATTACAACGTACCAGCCTGTATAGAACTGCAGCCAGTGTACAGCAA
TGAGCCTGAGGAAAACGTGATTTACACAGAAGTACGGAGGACTCAACCAAGACAGAAACATGCAGATCAGGAGTCTGAAAGCCCAAG
ATCAAGGTGCCAGATGGCTGAGAAAAAGTAGGGATATGTCTCCTCCAAGATTCCCAAGAATAGAATGTGCCTGACTCCCACAGTACCC
TATAGATCTGGCCAATCATCTCAGTTTTCAGTGGAGCCATGGAAATGCTCCCAATACCCTCCATCTCTTAGAAGAACAATCTCAGGT
TCCAATCTCGTCCCCGGTGCTTCAGATCCAAACCTCCAAAATTTCACATTATTTAGTAACAGATTTGGTGGCACGCTCCTGACAAAC
TCAAGTCAAGAACCCCGAGGAAAATGGCAGACTAGAAGACGTTTCTACGCCACCCTTTGAATGAGAAGAGCCACAATAAGAAAAAT
AGACACTTCTGAAGAGAAAGAAAGAAGAAACGTTTTAAAATGCACCAAAATAGTGTTAAAATACCTGGAAAGTGGGGAGGAAAGACA
GGTAACGCATGAGAAACAGCCAACGTAAACACAGACCAGCAGCATGGCTGTGATGTGGACAGGGTGAACAGACTTCTCATAAGCTAA
AGGAAGCTGCCCTGGTGAGAGAAAACTGGCCGTCCTGCCCTCAGAGCAAGTCCTCAGCCCTCACAAGCCTGAAAACCAGTGGCAGCT
TTTGTGAGACAACCATTCCTCCGGCACTGAAAGAGGAAGCACACCCAGCAGCTGCGTGCGCCTCAGAGCGGAAGTGACCCGGTTTGA
ATGAGAAGGTCTCCCACAGGCATGTGGATTTGAACTCTTGGACCCCAGATGCTAGCACATTTGGAGAGGTTATAGAACCGCTAAGGT
ATGGAACCTTGCTGGAGAAAGCGTGCCCCTGGAGATAGGCTTCGTTAAGAGTGTAGCCTGCTTCTAGTTTACCCTCAGCTTTCTGTT
TGAGGTTGAGGATTCAATCACCCCCGCTTCCTGTTCTGGCCCCTCAGCCTGCGGTGGACTCTGCCTTTGGAACCAGAAGCCAGAATC
GGCATGTTGTTCCTAAGTGGCTGCTGACTGGCCATGCCATTTTCCTCACAGCAACAGAAAAATGTCTGATCCAGACGTTGGTACAAA
AGAGTGGGTACGG


MOUSE PROTEIN SEQUENCE : mP27-007.1 (Seq ID No: 13)
MSGSFSPCVVFTQMWLTPLLVVTPVNGQHDFLVLQAPPAVFEGDSVVLRCYAKKGIEAETLTFYKDGKALTLHHQSELSIHHANLKDN
GQYKCTSKKKWSFGSLYTSNTVGVQVQELFPRPVLRARPSHPIDGSPVTLTCQTQLSAQKSDARLQFCFFRNLQLLGSGCSRSSEFH
IPAIWTEESRRYQCKAETVNSQVRKQSTAFIIPVQRASARFQTHIIPASKLVFEGQLLLLNCSVKGVPGPLKFSWYKKDMLNEETKI
LKSSNAEFKISQVNISDAGEYHCEATNSRRSFVSRAFPITIKVPSQPVLTLSTGKTQALEGDLMTLHCQSQRGSPCILYEFFYENV
SLGNSSILSGGGAYFNFSMSTERSGNYYCTADNGLGAQCSEAIRISIFDMTKNRSVPMAAGITVGLLIMAVGVFLFYCWFSRKAGGK
PTSDDSRNPSDSEPQEPTYYNVPACIELQPVYSNEPEENVIYTEVRRTQPRQKHADQESESPRSRCQMAEKK*


MOUSE PANTHER CLASSIFICATIONS
        FAMILY (SUBFAMILY)
                CARCINOEMBRYONIC ANTIGEN(FC RECEPTOR-LIKE PROTEIN)
        BIOLOGICAL PROCESS
                Biological process unclassified(2.99.00.00.00)
        MOLECULAR FUNCTIONS
                Cell adhesion molecule(1.05.00.00.00) > CAM family adhesion
molecule(1.05.01.00.00)

MOUSE GENE ONTOLOGY
        BIOLOGICAL PROCESS
                phagocytosis > phagocytosis, engulfment
        MOLECULAR FUNCTION
                GO molecular function > cell adhesion
        CELL COMPONENT
                cell > membrane fraction
MOUSE PROTEIN DOMAINS (INTERPRO SIGNATURES)
        IPR003598 (IGc2)
        IPR003599 (IG)
        IPR003006 (ig)


MOUSE mRNA SEQUENCE : mR27-007.2 (Seq ID No: 14)
```

```
CAAGTTACACTCAACTGTTTTAGAAGAGCAGTTCCCCAGATTTCTCCTTGGAGCTGTGAGTGACTACCATTGCGAGCAAGAGCAAGA
GGAAAGCACTACCTGTGAGCAGATGTCTGGTTCATTCTCACCCTGTGTGGTGTTCACACAGATGTGGCTGACTCTACTGGTTGTGAC
TCCTGTCAATGGACAGCATGAAGCTGCACAGCAGTCTGTGGTTTCCCTTCAGCCTCCATGGACCACTTTCTTTCGAGGAGAGGTCGT
CACACTGACTTGTTATAGATTCGGCTTCTCCGTACCCCAGAAAACAAAATGGTACCAGAAAAGAAAAACAGTGAAGCAAACCCCAGG
TGCTTTGGTAATTAAAGCACATACCTTAAAGGTCCATGAGTCCGGAGAGTATTGGTGCCAAGCCGACAGCTTACTTCCGAGCATGCA
CGTGAACGTAGAGTTTTCTGAAGATTTTCTGGTGCTGCAAGCTCCACCTGCTGTGTTTGAAGGAGACTCTGTGGTTCTGAGGTGCTA
CGCAAAGAAAGGCATAGAAGCAGAGACCCTGACATTTTACAAGGATGGTAAAGCTCTGACATTACATCATCAAAGTGAGCTCTCTAT
TCATCATGCAAATCTGAAGGACAACGGTCAATACAAATGCACTTCGAAGAAGAAGTGGTCTTTTGGGTCCCTCTATACTTCCAATAC
GGTCGGAGTTCAAGTCCAAGTCCCAGTATCTCAACCAGTTCTCACCCTAAGCACAGGCAAGACCCAGGCCCTTGAGGGAGACTTGAT
GACACTTCATTGTCAATCCCAGAGGGGCTCTCCATGTATCCTGTATGAATTCTTCTATGAGAATGTCTCCCTGGGGAATAGCTCTAT
ACTCTCTGGAGGAGGGAGCATACTTCAATTTCTCTATGAGCACAGAGCGATCTGGAAACTACTACTGCACAGCAGACAATGGCCTGGG
AGCCCAGTGCAGTGAAGCTATAAGGATCTCTATCTTTG


MOUSE PROTEIN SEQUENCE : mP27-007.2 (Seq ID No: 15)
MSGSFSPCVVFTQMWLTLLVVTPVNGQHEAAQQSVVSLQPPWTTFFRGEVVTLTCYRFGFSVPQKTKWYQKRKTVKQTPGALVIKAH
TLKVHESGEYWCQADSLLPSMHVNVEFSEDFLVLQAPPAVFEGDSVVLRCYAKKGIEAETLTFYKDGKALTLHHQSELSIHHANLKD
NGQYKCTSKKKWSFGSLYTSNTVGVQVQVPVSQPVLTLSTGKTQALEGDLMTLHCQSQRGSPCILYEFFYENVSLGNSSILSGGGAY
FNFSMSTERSGNYYCTADNGLGAQCSEAIRISIFX


MOUSE PANTHER CLASSIFICATIONS
        FAMILY (SUBFAMILY)
                CARCINOEMBRYONIC ANTIGEN(gb def: immunoglobulin superfamily receptor
translocation associated 2 [homo sapiens])
        BIOLOGICAL PROCESS
                Biological process unclassified(2.99.00.00.00)
        MOLECULAR FUNCTIONS
                Cell adhesion molecule(1.05.00.00.00) > CAM family adhesion
molecule(1.05.01.00.00)


MOUSE GENE ONTOLOGY
        BIOLOGICAL PROCESS
                phagocytosis > phagocytosis, engulfment
        MOLECULAR FUNCTION
                GO molecular function > cell adhesion
        CELL COMPONENT
                cell > membrane fraction
MOUSE PROTEIN DOMAINS (INTERPRO SIGNATURES)
                IPR003598 (IGc2)
                IPR003599 (IG)
                IPR003006 (ig)


HUMAN GENOMIC SEQUENCE : hD27-007 (Seq ID No: 16)
AATTCACTAATGCATTCTGCTCTTTTTTGAGAGCACAGCTTCTCAGATGTGCTCCTTGGAGCTGGTGTGCAGTGTCCTGACTGTAAGA
TCAAGTCCAAACCTGTTTTGGAATTGAGGAAACTTCTCTTTTGATCTCAGCCCTTGGTGGTCCAGGTCTTCATGCTGCTGTGGGTGA
TATTACTGGTCCTGGGTGAGTAAAAGGGCTCACCATGGGCCCTGGGGATCGGGACAGCTTCTCTCTCCACCGCCAGGCCTGGTCC
TTTCTTGGGGCTCAGCCCCTGCTGAGCAGACTCTCTCCTACATTAGGGAGACGTGTATTTTTTGAACTTTTACTCTGTAGCTCAGGGCTTTC
TCTTGAGGCTGAAGAAAAATGGCCACAGCTGGAAGGAATTTTCAATCTTACAGAGTAGGAAACCACAATAACCACAACAATTAAAATT
TCATTTATTTTTTTTCTTTAAAAAGTACTTCTTAAAGTCCAGAGAATGTTTCATGTTTGTTTGTTCATATTGTCTTACTCATAGAAGA
GCCCACTCTCCTCTCTGCAGAGGAGATGTATTTATTCCTTGTATGACAAGGCCAGATCCTGGTCCCCAGCATAAGAGGCAAGGTTTC
TAACGGGAAAGACAGGTCTTTCTCTTGTACATAATTGCAAACCACTGCAGCAATTGGCTGATTAAGAACATGGGGTGGAGGAGGTCG
CAGAGGCGTAGGTGGACAAAGCAAGGATAAATCTAAAGCATATGACTTTCTCTTTGTCCCTGTGGGTGTACGTAAGTCTCTATCAAC
TCATATGTGCGAACTGGTTGATTTTTACTAAAGGTTAAATATTATAATTAAATATGTTTATTTATTTGTTCAGCAAATGTGTTTAGT
AATATCTATGCACAGGTGCTGAGCAATTAGAGTCCATAGGAGACACTACGTCTATGTTCTGGAAAGTCATGACATAGAAGGGCTCCC
AGCAAGGCCAATGTACTTTCAAGCAGCTATGCAACAAGCTCCCAGTTAAATAATTGTCCAGGCAAAGTTACCAATGAATGCTGTAGG
CAGATAATAAACTGTATTCAAAATAGCTAGTAAAGACTTCATTAGAGGTAGGTAGGAGCTAGAGGAGCAGAAATGGAGCAG
GCGTGATATAAGAAAAGAGGACCTCACAGTTGGAAGGTGCATGGACTATAATTCCTGAAGTAGGAATACATCTCAGTGTGGCCCCAC
ACAATGTGGGTAATGGCCAGGTCCTGAGCATGAGAGGGCAAAGTTTATAACATGGGCTCTGGGGTCAAACAGGATCTAGGTTCAAAC
TTCATCTTGCCACCTACTGGCTACCAGATTCCTGGTCTACAAAATGCAGTTAATCCTAGTGCTTACCTCACAGGATTATTGACATGG
TTATATGAAATAACCTACGTAAATGCAGACCATATAGAAACCACTCACAAAAAATATTAGCTTCATTTAAAACAATGCAAAGATGAAG
GAAGCCAAAATATTCAGGTGAATGGTGAATAATTTGTTGTTCTCAGTAAAACACTTAAGTTTGGATAAGTCTCCAATAGCCTCATGT
GGACAGGGATGATTTGTCTCTCACACACTCCTGGATTTCTATTGCCTTGCTCTGGAGTATGATTGGCATTCCAAAAATCTGTTGAAT
AAATAAATCAACCCATATGCAGAATACCTACTGCAATTGATAAACCTGCCAGTTTTGTGTCCTCTCTCTCTTCATTACCCATTTCTCA
AATCTCTATCCTTCTATTCCTATATTATTAACCTAGTTCTTTAATTAATTCATTATACAACACAAATATATTTAGTGAATATTAAAG
GAATATCTTTTATATGCCATTCACCGTTATAGGTCCTGGAGGATATATGGTGAACATAAGGAAAGTCCTAGGTCTCATAGAGCTTGC
ATTATAGAGAAGGGAAAATAAACAAGCAGATAATGAAGGATAATTTGGATAAATACTCTGACAAAAATTACACATTACCATCTCTTGG
GCTGAGCATCAGGTCTAAATAATCTGAGCATCAGTTGATTGTTTAAAAAATTAGGCATGATGATAATGAGCCCTTGGATTCAATGATC
TCATGAAAAGAGTATAGCCTGGAGTCTGGTGCCCAAGGGAGGCTTAATTCATGTCCATTTCCTTGCCCTGCCTTCTAATCAGTCTCC
CTGCTTCATATTGTCCCATTAAATTCTGTCCACTGACAGAGTGCTCCATCAAAGTCTGAAATTCTAACCTTGTCAATCTCCTCCTTG
AAACCATTCCATGACTCCTTATCACTTTTATAATAAAATTAGAACCACTTACTCAGGCCCTGCATGCTTCTCTGGTCTTATTTCCCC
CAACACTCCCAGTTCCTGAGCACCAATGTCATTTCTCCACTTCTGTGCCCTTTCTACCTTGAAAGGTAGAAATCGCCTCTTACCACA
CACAAATGCTAACATCCACCTGCAGACTCATGTTCTCACTCACAGTTTTGTGAAGTATCATCTCCTTCAGGTACCTTCAAGGATCCA
CCCATCCTGTTCAACACATGTACACACAAACACACACTCTACACACACACATATATCCTACACACTGGGCTAGTGGCCCTTCTGCCC
CCAACATACCCCATGTGTATTTCTGTTATTGCACTTACACCCTGTGTTATATTATTTTAAATCGTGTTTCTGTTTTTCCACTAGTCA
ACTTCTTGATGGCAGATACCATTTTTAAAATTCATTGTAGAAGTCCAAATACCATGCCATATATATATGTATATATATGTATATTTA
TGTGTATATATATACATTTGATGAATAATTATTAGAGTTTTTAAATGAATTTTTTAAAGGGACAAGTAATACATGCATGGGTAAAAA
AGGGAATTATCTTCTTAAAACAAAATAATCATGGTTCTGTGCTCATCTCTGGCTCTTATTTTTTTGCAGCTCCTGTCAGTGGACAGT
```

EP 2 204 376 A2

```
TTGGTGAGTTCTCCTGTCTTTGGCTCTTCAGCGTCTCTGGTTAAGAGTGTCTCTTCCTAATAAATAGAAAACATTTGTGATGGCTGC
CTGAGATCTCCAATATCTCCTGTTTTCTTTCCTTTTTTTTATTATACTTTAAGTTTTAGGGTACATGTGCACAACGTGCAGGTTTGT
TATATATGTATACATGTGCCATGTTGGTGCGCTGCACCCACTAACTCGTCATTTAACATTAGGTATATCTCCTAATGCTATCCCTCC
CCCCTCCCCCACCCCACAACAGGCCCTGGAGTGTGATGTTCCCCTTCCTGTGTCCAAGTGTTCTCATTGTTCAATTCCCACCTATG
AGTGAGAACATGCGGTGTTTGGTTTTTTGTCCTTGCAATAGTTTGCTGAGAATGACAGTTTCCAGCTTCATCCATGTCCCTACAAAG
GACATGAACTCATCATTTTTTATGGCTGCATAGTATTCCATGGTGTATATGTGCCACATTTCCTTGATCCAGTCTATCATTGTTGGA
CATTTGGGTTGGTTCCAAGTCTTTGCTACTGTGAATAGTGCCGCAGTAAACATACGTGTGCATGTGCCTTTATAGCAGCATGATTTG
TAGTCCATTGGTTATATACCTGGTAATGGGATGGCTGGGTCAAATGGTATTTCTGCTTCTAGATCCCTGAGGAATCGCCACACTGAC
TTCCACAATGGTTGAACTAGTTTACACTCCCACCAACAGTGTAAAAGTGTTCCTATTTCTCCACATCCTCTCCAGCACCTGTTGTTT
CCTGACTTTTTAATAATTGCCATTCTAACTGGTGTGATATGGTATCTCATTGTGGTTTTGATTTGCATTTCTCTGATGGCCAGTGAT
GATGAGCATTTTCTCATGTGTCTTTTGGCTGCATAAATGTCTTCTTTTGAGAAGTGTCTGTTCATATCCTTCACCCACTTTTTGATG
GGGTTGTTTGAATTTTTCTTGTAAATTTGTTTGAGTTCATTGTAGATTCTGGATATTAGCCCTTTGTCAAATGAGTAGATTGCAAAA
CTTTTTCTCCCATTCTGTAGGTTGCCTGTTCACTCTGATGGTAGTTTCTTTTGCTGTGCAGAAGTTCTTTAGTTTAATTTAGATCCCAT
TTGTCAATTTTGGCTTTTGTTGCCATTGCTTTTGGTGTTTTAGACATGAAGTCCTTGCCCATGCCTATGTCCTGAATGGTATTGCCT
AGGTTTTCTTAAAGACTTAAATGTTAGACCTAAAACAATATCTCCTGTTTTCTTGCTGAGGTCAGCAGGGATTTCAAAGTGATGCCT
GGTTTTTTGGCATCGTCCAAGGGCCAGGATAGCTTCATGGTCACCCTCTGACCCTCCCTGGGGGAAGGGGTGAATGCCCAAGCAGCT
CCCAGCCTTTGCTAAGCCCTGCATTTACTATTATTGCCAACAGACCCCATTTAGCCAGGCAGGACATCTTCAACTCCATGGCAAGTA
TATTGACTCAGGATAGGATCCTCTCAATCAGCGAGCCAGAAAAATACCCTGAAAATATCCCACTGTGCTGATGCCAGCAGGAATGCT
GCATTCTCTACACTTTGGAGTAAAATCCTCCAGGACATCCAATAACTGGGTCCTTACAAAAAAGAAAGGCACAGAAAGGGGAGATGG
GGAGAAGACAAGTTATCTGCATCCCACATTTAATTAGTTCTGGCTCTTACTGCCATTCCTTTGGCTCTGAGTGGATAATTGAGAACC
CATGAACAGCAACAACCCTCTACAACCCCTCTCCTTGTCCAAATCAGTGGCCCCAGCTGTAACTTTGTCCTGGTCACCTCTGGATGT
CAGTGAGTGACTTGTCCCTTCCTTGCTCTGCCACAATTAGCTGTGATGGGATTCTTCTGGGCACTTGAATCATCATCTACAAAATGA
AGATATTAGGTAAGTTGAGCTCTCAAGGTTTTCTCAGGGACAAATCTTAAGATAATATGATTTATTCTCTTAGGGAAAGTGCAAAAT
TGTGATTTTAACATAACCTTTGGAGTTTCTTATGAGTGTATGAACATGATGAAATGGCAGCGCAGCATTGATTTTCTCTGGGACCCA
CTTCCATGTAAGGCTACCTAACTGTGATATTTGATGGCGAATCCTGCTGGTGTCACCCTCCTGAAATCATTTTGCTCATTGCCATCC
CCTGGGCAATTTCTCCCTTGGGTGAGGTGAGGGCCACTGGCTGCACAGGGAGCTTTGAGCTATCAGTGACCCTCCCAGAGGACCTCTT
TCAGATCCTCCAAACCCAGGTCCTTCAGGCTATGCAGCAGGAGGCCCTGAGGCTGGGTCTGCAGGCGCCTCCACCTCAGACTCCTTT
TCTCTTTCCTCTTCAGCAAGGACACCCAGGCCCATTATTTTCCTCCAGCCTCCATGGACCACAGTCTTCCAAGGAGAGAGAGTGACC
CTCACTTGCAAGGGATTTCGCTTCTACTCACCACAGAAACAAAATGGTACCATCGGTACCTTGGGAAAGAAATACTAAGAGAAACC
CCAGACAATATCCTTGAGGTTCAGGAATCTGGAGAGTACAGATGCCAGGCCCAGGGCTCCCCTCTCAGTAGCCCTGTGCACTTGGAT
TTTTCTTCAGGTGAGAGAGCGTGAAAGACGCACCAACTTTAGAAATCAATTCAGCCCATGTTTCTCTCTAGCTGTTATTACTGTTTA
CCTCAGACCCTTCATGAGTGGACAATTTTATTTCACACTGTGGTAAGCAAGGAGAGAGTAAAAAGCTCAGAATACAACCAGACAGG
GAATGGGGTTAGCTGCTTAATTTTAAACATTAAAATAAATAGAATTTTTACTTGAGTGGATTAATGTCTCTAAACCTGTGGTTCTTA
AAGTTTGATTTGAGTCAGAAAGCCTGGGCAGCTTATCAACCATACAGATACTCAGGACCTACCTATTGCTTATTCAATAGAAACCTGT
AGAAGTGGGGTCCAGCAATTTATATTTTTTACAAGCCTCTCCGGTACCAGAAAGTCTGAGGATTAATTTATTTGAGTGGTTCCTGAA
TCTATGTATAGCTTTTTGGTTTGGTTTGGTTTGGTTTGGTTTGGTTTGGTTTGGTTTGGTTTTTTGAGATGGAGTCTCGCTC
TGTCACCCAGGCTGGAGTGCAGTGGCGTTATCTCGGCTCACTGCAACTTCTGTCTCCCGGGTTCAAGCAATTCTCCCACCTCGGTAT
CCCTAGTAAGTAGGACTACAAGAGCCCGCCATCACACCTGGCTAATTTTTGTATTCTTAGCAGAGATGGGATTTCCTCATGCTGCCC
AGGCTAATGTTGAACTCCTGGGCTCAAGTGATCTGCTCACCTAGGCCTCTCAAAGCGCTGGGATTACAGGTATGAGCCACTGCACCC
GGCCTCTATGTATAGTTTTAAGTCACATTATTTACTTGGAATAATAAGTGGCAGGAGAATTGTTCATACTTATGGGTAGATAATGCA
TAGGTAGATAAATATACAGAGAAAGAAAGATAAAGGAAGAGACATTTCCTTCCTTGTCCTAAAGCCACCTGTTTTGAAGTTGAAGTT
ACATCATTTTTTAATGTAGTGGAAGCAGTGTACTAGCAAGGAATAAACAAAAATGTTTATACTCTTTGACTTAACAATCCGTCTCTT
AAGAATTTGCCTACTGAAACAATCACAGATGTCCATCCCTGAAAATGGCAGACCTTAACATGGTTTAGAAAAAGAATACCTGGCCAG
GCGCGGTGGCTTACGCCTGTAATCCCAGCACTTTGGAAGGCCAAGGCGGGTGGATCACGAGGTCAGGAGATTGAGACCATCCTGGCT
AACACTATGAAACCCCGTCTCTACTAAAAATACAAAAAATTAGCCGGGCTTGGTGGCGGGTGCCTGTAGTCCCAGCTACTCGGGAGG
CTGAGGCAGGAGAATGGCATGAACCCGGGAGATGGAGCTTGCAGTGAGCCGAGATAGCACCACTGCAGTCCTGCCTGGGTGAAAGAG
TGAGACTCCGTCTCAAAAAAAAAAAAAAAAAAAAAAAAAACTCATTAAAGAAATTACATTACTGCCAGTGAAGGAAAACCGCAAACATT
AAAAATCAGGTTCAATAATGATACTAATGATGTAGGAAAGTGTTCATAGCATAGTAAGTGAAAGGAAAGTTATCAAATCTACAAATA
TTAATATTATTCCATAAAAGAAATACGCATGAGCTCCAAGTACTTGAAAGTGGAAGAGAAAACTGGAAAAACATATGCCAAAACATG
GGTGGTGAGATGGTAGGTGATTTTACCTACATTTCTGCTTTTCAACTTTTCTTAAGGTGTATGTGTTCATTTTTAAATCAGAAAAAA
AGAAATGATAATTATTTTAAGAGTTCTTTTTCCCAACCCATTTTTTTTAAAATAAAATCTACTTGGAAAAACAATGAAAAGAAAGGGG
AAACAGTAGGATGAACCAAATAACTCTATTGTGTTTCTCATAGCTTCGCTGATCCTGCAAGCTCCACTTTCTGTGTTTGAAGGAGAC
TCTGTGGTTCTGAGGTGCCGGGCAAAGGCGGAAGTAACACTGAATAATACTATTTACAAGAATGATAATGTCCTGGCATTCCTTAAT
GTTTCTTCCAATACAGTCAAAATCCAAGTCCAAGGTAAAGCCTTCATTATTTTGTGAAGTGAGTTGGTCAAAAGGAGATTCCTTGGG
GTCATAGAGATAGAACAGGAGGAAAAGGGTGGACTGGAATACCACTAAACCACAGGACACATTCCTGCAAGGAGCAGAGGAAGTATA
GTAGTGTTTTGGGCTGAAGGAAGCTAGGGATCAATGCCATTCCTTTCTTTGCCTGCTTTTGGATGTGCTGCTGCTTGAAGTGTGCTT
TTCTCCAAAGCAGTTCCAGCCTCATTGCTCTGGGACTGTTGTGATTCTTCTCTCTAGAGCCATTTACACGTCCAGTGCTGAGAGCCA
GCTCCTTCCAGCCCATCAGCGGGAACCCAGTGACCCTGACCTGTGAGACCCAGCTCTCTCTAGAGAGGTCAGATGTCCCGCTCCGGT
TCCGCTTCTTCAGAGATGACCAGACCCTGGGATTAGGCTGGAGTCTTCCCCCGAATTTCCAGATTACTGCCATGTGGAGTAAAGATT
CAGGGTTCTACTGGTGTAAGGCAGCAACAATGCCTCACAGCGTCATATCTGACAGCCCGAGATCCTGGATACAGGTGCAGAGTAAGT
GTTGGTGGAACCTGAGATTTGCTGCAGAGAGGGCTGGAAAAGTGGGACAGGGCTGCATATCAGCGTGGGTCACCAGTCTCTGTAGAA
AAAACTCGTACCTGTGAGAATGGGAGTTGTGCAAGCAAAGAGACCTCTACTCTTGTAACATCTTAGAAGAGCTTAGACCCAGGAAA
ATCCAAGAAAGAAAGGTCTAATTTTTTTTCTTTCAATGCCATTAAATACAATTCTTTCTTTTCTCTCGCAGGGTTCTCTTCCCTGA
CATCATTTTTTTTTCTTCTAGGCCCATAGGGATAAGCCATGGGCTGTGGCATAGCTCTTCGACTCTGGGGTACAGAGGTATCTGTTG
TCCCAATTAACATTACTTATTGACAATAACCAAAGTGAAAATTTCTGTAGGATACAAAGACAGGTTGCAACATTATAGTTACTCCAA
ATATGTGACTCTGGAGGTTTACCATTCCCTAGTTATGAGTACAAATATGCACCCAGATTACATCACCTCCCTTACTAGTATACAGC
ACCATCCTTCCTTCAAAGCTATTATGGTAGTTACAGTGATGAGGGAAAAACAGCTTCTGTAGAGCCCTGTGTCATCCTCCACTCAT
GGCAAACTCCGGGCTCACTTATAAGGAGGCAAGGTGATCATCACCAGACTAGGAGTCAGGACACCAACTCTGGCCCTGGCTGGCCCT
GAAAACAGCCTACACAGGCAGGTCTCCTAGTCTCTCCTTGACTCAGTTTTCTCACCTGTAAATAAAGAAGGTCAGTCTGGGCCAAGG
GAGAGAGTAGGACGCAGAAGAAAAAGCTCTAGAAGACTATGCTAATCTTTAAGGAAAAACTAGCAATGGCAAATAAATCCCTGAGGCC
AATGCAAGTGAAGGAGAAATAAACAGGGATAGGAAAGAAGACTCAGAATAATTGGGCACTTCAGGTGCAACATTCCATAGTCATAGG
GCAGAAGGAAGGAGCAAGAAGCAGGTGCAGGTAAGGGTGTGTGGTTCTGATGGCAGCAAGTTAAGGGTGGAGACTGGAGACTGGGCA
TTTGCACTGGCCATGCCTTGTAATCTACAGTAAGAGGCAAAAGCAATTCTTGACTAATTTTTTCTCTTGCTCAGTCCCTGCATCTCA
TCCTGTCCTCACTCTCAGCCCTGAAAAGGCTCTGAATTTTGAGGGAACCAAGGTGACACTTCACTGTGAAACCCAGGAAGATTCTCT
GCGCACTTTGTACAGGTTTTATCATGAGGGTGTCCCCCTGAGGCACAAGTCAGTCCGCTGTGAAAGGGGAGCATCCATCAGCTTCTC
```

67

```
ACTGACTACAGAGAATTCAGGGAACTACTACTGCACAGCTGACAATGGCCTTGGCGCCAAGCCCAGTAAGGCTGTGAGCCTCTCAGT
CACTGGTAAGCCCTGCATTCCTGCCAATCACCCACCCCTGGCCAAGAGTCCTGCCTCACCCTGCCCTTCGGAAGTCAGCATATACCT
TCCAGTATCCTCAGTTTCTTTCTTGGGAAAACCTAATGTCCTCCTCCACTCTCCGGCTCACTGAACCAGCTGTCTGCCTCTTGAGTC
TCGTTGCATTGCAGTCTAGGTGCCCTCATACCCCTGCCTGATGGTGCAGTGGCTTTCTATCCCCTGGTTCCCAATTTCTGAGAAGCC
AAGTGCTTTTCTTGTGCTTTCCACTTGTCCTGTGGCTCCCCAGATGACTGATTTTCATTCACTATATTATTCTCAGTAGACACAGAC
TCAATTTCCAGGAGCACAGAGGTATCTCCTGTTCTGTTAATATCAGACATTAATCAATAAACCAAAGGCAAAGACATGTAAGACACA
AAGACAAGTCGCTGGATCATAGTTAACCCAGCATGTCCTTCTTGCTCAAGCCTTTTTTAGAGTTGATGGGCAGTAGGTAAGAAAGGC
ACGGGCAATGGTGGGCTGGGGGCAGGCCAGGAGATAGCTTCACCTCTACTTGCTTGGCTTAATGCCCTCAGAGCTATTCAGGTAGCT
GCCAAATAGTTATGACATCAAAAAGGTTTCCTTTGAGCTTTGGATCTCAGTTTCGAACCATCCATGCATGGCTGAATGCCAGGTCCA
ATGTTGAAGGAGACAGTGTGATTTGGGTCTATACTGGTGACAGAGAGTCCAGTCTTGGTTGGTCCTGGAAGCGGCATCAGTTTCCTT
TCCTGTAAAACAATAATACCAGCTTGGAAGACAAGCAAGAATCCAGTCCCTCCCACATGGATGTGTGAGATTATAAAGGAGGAGACC
AGGGAACCCAGCGGGAGACATCAAATGACGTAGAAAGAACAGTGTCCCTGACATTCAGTGGCCCAAAGAAAGTGGAGACAGTGAC
ATCATGATCAAACATGCTCAGAGATGTTTAAAGTCCTATTCAATGACTCACTTCTTCACCTAATAAATGCTGATTGCATATATTCCA
TGGACCAGACTGTACTGACATCTAAAGATGCATCTACGACTACACTAACCTGGCCTTCCCCACACAGTTCGTAGGGGCTAATAGCAA
ATGCAGTCAAGTAAGAGATTAAACATCAACCTGAGGATAGAAGCACCTGGTGGCTGCAGGAGCAGATGGAAAGGCATGCCAATCTGA
GTTGGGAAGTGAGAAAAAAATTTCTTGGAGAACTCATGTGTGAGTTGAGACCTGAGGATGAGTGGGAACATTTCAGGAGAGAGGAT
TGTGGTGAGGGAGGGAAGGCATGTTCCAGGAACTGGAGGGGCAGTCGGTTCTTGACGACTGCAGCACAGACTGAACAGTGGAGAAAG
GTAGGATAAGGTTGGAGAGGAAGGTATGTTTAGACCATATTTAGAAGCAGAGTAAGGGCTTGGGCCATATTTTGCAGGGCAATCTGG
GCTCCATGAATGCTTTAAATCTTGGGGAAAACCACTCCAGCAGCACAGTGAGAAATGAGTCATAGAGCAGCCAGTTTGGGGGGCAGC
CAGGAGTAAAAGAGAGGGTTCATCAAGAGAGCGTCCAGTTGGAGCAAACCCCAGGCCAGTCCCAGTAGAGGAGTTGGGACTTGAGAA
GGAAGGACTCCAGGTTGAAGCACGTGTGAGAGGTGCCCCAATGTCACCAAGGGCATAATGTGTTCTAAGCAGAAGAGAGGG
AGCTGGTTTGGATAAGGCAAAGCTAAGACGATGATGACAGATAAAAACTGAGTCACATCCAGTGAATTTAGCCACAAAGTACTCTTTG
GCAGAAGACAGACTAGAATCAGAATACCTGGGTTTGACTGCTGCCTCTTCACTTACTAGCTGTGTGACTCACCCACTCGGGGCCTCA
GCATGTTCATCTGTGAAAGAGGGGCAACAATAATACCCACCTCAATGAATTGCTTAGATGATTAAATTAGAGTCTCTCTATATATCG
ATATTTACAAATTACATCCAACAAATATACAAACACCAGAACTTACTTAAAGACACTATAATGTCATTATAACAAACATCTATTTTA
TTATTTTTATCTTTATCATCATAATTATATTCAGAAACATGAAACTCCACTTTAGTAGATTGTTGGGGATTAAAACCAAGTTGTAAT
GGACAAAACATGCATATGAGATATGAGATAAAGAAGTAGAGATGTTAAAGTAGGTCACTTCTTCCGAAGACTGACTACAGAAAGG
TGGAGAAACCGACTGTGAGGAACTGCAAATGCAAATAGGGTTGTTTTTCTATTGTCTGATGGTTTTTTTTTTTTTTAAACATGGAGA
GGGTTAAACACATGCAAATGGTGTTTGCAAGAAGCCAGTAGAGGTAAAAACCTTTTATCTAGGACAGAGGCAGGTGAGCACAGGATG
GTAAGGGTGTGGTTCTGCTGGTGTGAAAGACAAGGGAATTCTTTGAGAGCAACATCTCTGCACAACAGAGGCAGGTCCTACAACAAGG
GGAGCTGGGAGAGGAGAAAACAAGAGGTCTGGTGGAAAAGGAAAAGACAGGGAATGACTAGGATGGAGGACAGGAGAGCTGGTGA
GAGTAGCCTGGGAAGTGGCTGGGTCACCCTGCAGGTGGGTAGAGCCTGTGGACTGGAGACTGAACAACTGCACTGGTGAAACATTGA
ATCTGTGAACCAAAAACTATCTGAGACAGGTCTCAATCAATTTGGATAGTATATTTTGCCATGGCTAAAGACATGTGCCCCAGATTC
AGCTCCCTGCCTTCTCCAAAGATGATTTTGAGGGTTTCAATATTTAAAGGTGAAAGGGAAGATATGGAGAAAAAGGAAGAAATTTTT
TTAAAAGTATGTGTAAATAACAGACAAACAGTTGCATTCTTTGATCACCTTTGATCAACCTTTAGCAGAATACACAATTTACATGTGA
GAGAGGGGCAGAGGAATAGTCACTTCTGTCTTCCTTTAGCCCAGTGAGTCTGCATTTTTAAGTCAGGGAAATAATCAGATATGCATT
TGTCTCCTGTAAGCAGCAATGACTTAAAGTTCTGTCTTTTGTCCTGCACTTGTGAAAATCAGCTATCAATTTACACTGTTAGGGTAA
AACAGAACCATTTAAAACAGAACCATTTTAGGGTAAAGATCTTGGGGCCCACAAATAATATTTCAGTGGACAAATTTTGAGGGAGGT
ATGTAGCTTTTAGAAAAGTCTTTGAAGTTATCTTATTAAGGAATAAAATAAAAAGCAGGTTTGCCTGAAATAGTTCCCAGCTTGACT
TTTCCCTTTGGCTTAGTGATTTGGGGTTTCCCAAGATTTATTTTCCTTTTATGAAAACTACAGCAGGAGCAGCAGAAACAAATCTTGACT
AATTTTTTCTCTCTCTCAGTTCCCGTGTCTCTCATCCTGTCCTCAACCTCAGCTCTCCTGAGGACCTGATTTTTGAGGGAGCCAAGGTG
ACACTTCACTGTGAAGCCCAGAGAGGTTCACTCCCCATCCTGTACCAGTTTCATCATGAGGATGCTGCCCTGGAGCGTAGGTCGGCC
AACTCTGCAGGAGGAGTGGCCATCAGCTTCTCTCTGACTGCAGAGCATTCAGGGAACTACTACTGCACAGCTGACAATGGCTTTGGC
TTACCCAACCCTTCAGAAGTCAGCATGTACCTTCCTTCTAGTATCCTCTGTTTCTTTCTTGAAAACAACCTAATATCCTCCTCCACTTCTA
GCTCACTGAACCAGCTGTCTGCCCCTTGAGCCTCATCCCATTGCAGTCTGGGTGCCTTCCCACCCCTGCCTAATGATGCAGCAGCTT
TCTTTTCCCTGCTCCTCAAATTCTGAGCAGCCAATTGCTTTTCTTGTGCTCTCCACTTGTCCTGTAGATCTGCCAGATGCCTGATTT
TTGTTTACTAAATTATTCTTAGTAGATATAGACTCAGACTCAGTTCTCAGGAGCACAGAAATGTCCCCTGTCCAGTTAACATCAGAC
ATCAATGAATAACCAGATGCAAAGTCATGTAAGACACAAAGACAAGTCGCTGGATCACAGTTAACCCAACATGTCTCCCTTGCTCAA
GCCTTTTTAGGGTTGATTGGGCAGTAGGTAAGAAAGGCACAGACAGCAGTGGGGAGAGCTTAACCTCTA
CTTGCTTGGCTTAATGCCCTCACGGCTACTCAGGTAACTGTCAAATAGTTATGACATCAAAAAGGTTTTCTTTGAGCTTTGGATCTC
AGTTTTGAACCATCCATGTGTGGCTAAATGGCCAGGTTCAATGTTGAAGGAGACAGTGTGATTTGGGCCTATACTGGAGCAGAGAG
TCCAGTCTTGGTTGGTCCCGGAAGCGGCCTCAGTTTCCTTTCCTGTAAAACAATAATACCACCTTGGAAGACAAGCAAGAATCCAGT
CCCTCCCACATGGATGTGTGTGATTATAAAGGAGAGGGCCAGGGAACCCAGGGGGAGATATCAAATGATGTAGAAAAGAACAGTAC
TCCTAACATTCAGAGGCCCAAGGAAAGTGGAGACAGGGACATGATCAAACACACTCAGAGATGTTTAAAGTCCTATTAAATGAC
GCCCTGCCCACACAGTTCACAGGGGCTAATAGCAAATGCAGTCAAGTAATAGAGAAAACATCAACCTAAGTTTAGAAGTACCTGGTG
GCTGCAGGAGCAGATGAAAGGCATGCCAATCTGAGTTGGGAAGTGAGAAAACAATTCTTGGAATTTGAGATGATATCGGGGGAAATT
CACCCCCAATATTTTACATAGGTTCTTTTCTATTTTCCCTAAGTGTCAGCTGGTCTCAGAAATAAAGGGACAGAGTACAAAAGAGAA
CAATTTTAAAGCTGGGTGTCCGGGGGAGGACATCACATGTCGGCAGGTTCCATGATGCCCCTTAAGCCATAAAACCAGCAAGTTTTTA
TTAGTGATTTTCAAAAGTGGAGGGAGTGTACTAATAGGGTGTGGCTCACAGAACTACACATGCTTCACAAAGTAATAAAAATATCACAA
GGCAAATGGAGGCAGGGTGAGCTCACAGGACCACAGGACCGGGGCGAAATTAAAATTGCTAATGAAGCTTCGGGCACGCATTGTCAT
TGATAACATCTTATCAGGAGACAGGGTTTTGAGAGCAGACAACCAGTCTGACCAAAATTTATTAGGCAGGAATTTCCTCGTCCTAAT
AAGCCTGGGAGCACTACAGGAGACCAGAGCTTATTTCATCCCTCAGCAACGATCATAAAAGACAGCCATCCCCAAAGCGGCCATTTC
AGAGGCTCCCCTTAGGGACATATTCTCTTTCTCTAGTGATGTTCCTTGCTGAGAAAAAGAATTCAGTGATATCTCTCCTATTTGATT
TAGAAAGAAGAAAAAATATGACTCTGTTCCACCCAGCTCACAGGCAGCCAGAGTTTAAGGTTATCTCCCTTGTTCCCTGAACATTGCT
GTTATCCTGTTCTTTTTTCAAGGTGCCCAGATTTCATATTGTTTAAACAATTTGTGCAGTTAACACAATTATCACAGGGTCCTGAGG
TGACATTCATCCTCAGCTTACAAAAATGATGGGATTAAGAGATTAAAGTAAAGACAGGCATAGGAAATCACAAGGGTATTGACTGTG
GAAGTGATAAGTGTCCATGAAATCTTCACAATTTATGTTCAGAGATTGCAGTAAAGACAGGCATAAGAAATTATAAAAGTATTAATT
TGGGGAACTAATAAATGTCCATGAAATCTTCAAAATTTATGTTCTTCTGCCATGGCTTCAGTTGGTCCCTCCATTCGGGGTCCCTGA
CTTCCCGCAACAAGAGACCTGATGGAAGAGAGGATTGTGGTGAGGGAGGGAGGACGCACTTTCAAGGAACTGGAGAGTGGTCAGTT
CTTGATGACTGAACCATAGACAGAACAGTGGAGAACCACAAGATAAGGTTGGGGAAGCAACATCATTAGAACCTACTTAGAAGCAG
AGTAAGGGCTTGGGCCATATTCTGCAGGGCAATCTGGGCTCCATGAAGGTTTTAAATGTTGGGGAAAACCACTCCAGCAGTACAGTG
GGGATCAGGTCATAGAGCAACCTGTTTGAGGAGCAGCCAGGCGTGAAGGAGGGAGCCCAACAAGGGAGTGTCCAGTTGGACAGAACC
CCCAGTCTAGTCCCAGTAGAGGAAATGGGACATGAGAAGGAAGGACTCTGGCCAAGGGAAAAGGCACTCACTGTGAGAGTGTGCCCC
```

```
ACTGTCACCAAGGGTAGAACATGTTCTAAGCAGATGATAGTGAGCCAGTTTGGATAAGGCAAAGCTAAGAGATGATGAAAGATAAAA
ACTGAGTCATGGCCAGGAGAGGTGGCTCATGCCTGTAATTCCAGCACTTTGGGAGGCTGAGGCAGGTGGATCACCTGAGGTCAGGAG
TTTGAGACCAGCCTGGCCAACATAGTGAAATCCTGTGTCTACTAAAAATACAAAAATTAGCCAGGTGTGGTGGCGGTCGCCTGTAAC
GCCAGCTACTCAGGAGGCTGATGCAGGAGAATTGCTTGAATTCGGGAGGCAAAGTTTGCAGTGAGCTGAGATCATGCCACTGAATTC
CAGGCTGGGTGACACAGTGAGACTCCATCTCAAAAAAAAAAAAAAAAAAAAAAAAACTGAGTCACATCCAGTGAATTTAGCCACAAA
GTACCCTTTGGCAGAAGAACACAGACTAGAACCAGAATACCTGGGTTTGACTGCTGCCTCTTCACTTACTAGCTGTGTGACTCAACC
ACTCGGGGCCTCAGTATTTTCATCTGTGACATAGGGACAACAATAATACCTACCTCATTGAATTGTTTTGATGATTAACTTAGTGTC
TTTATACATATAGATATTCACAAATTACATCTGACAAATATACACACCAGAACTTAAAGGCACTATAATCTCATTATAACAAATA
TCTTTTTTATATTTATCATTATCATCATTACTACATTCATAAACATGAAAAATCAGCTTTAGTAGATTATTGGGGGTAAAAACTGAG
TTGTAATGGACAGAATATGCATATGAGATATGAGATAAAGAAGTAGAGATGATGATAAATGTAGGTCACTTCTTCCAGAAGTCTGTCCAC
AGAAAAGTGGAGGAACCAACTGTGAGGAACTTCAAATACAAATAAAGTTGTTTTTGAATTGTCTGATAGTTTTTTCTTATATGGAGAT
AGTTAAACACACCAAAATGTAGTTTGGAAGAAGCCAAAAGAGGTAAGGACTTTATGTCTAGGACAGAGGCAGATGTGCACAAGGTCA
AGAGACATAGGAAAATCCCCCTTGGGCAGGCCAGGCATGGCTCTTCCATGGTTGCAGGGAAGCCTGCATGGTTGCAGGAAGAAGTGA
GGAGCGGTGCAAGTGAGTCTGGTGGTGGCAAGACAAGGAATTTTTTCCTTGAAAGCATCATCTCCTCTGCAAAAAGGAGGCAGGTCA
CACTACAAGAGGAGCCATGAGAGAAGAAGAGAGTATTGGTCTCCTGGAAGATGAACAGATAGGGAAAGACCAGCATGGAGGGCTGG
TCACAGAAACCTGAGAAGTTGCTTGGCTGCCCTCCAGGTAGGTGAAGTCTAGAGATCCTGTACTGGAGACTGGGCATCTGCACTGGC
CAAGTGTTGAAATCTACAGTAAGAGGCAAAAGCAACTCTTGATTCAATCTTTCTCTTCCTCAGTCCCTGTGTCTCATCCTGTCCTCA
CCCTCAGCTCTGCTGAGGCCCTGACTTTTGAAGGAGCCACTGTGACACTTCACTGTGAAGTCCGAGAGGTTCCCCACAAATCCTAT
ACCAGTTTTTATCATGAGGACATGCCCCTGTGGAGCAGCTCAACACCCTCTGTGGGAAGAGTGTCCTTCAGCTTCTCTCTGACTGAAG
GACATTCAGGGAATTACTACTGCACAGCTGACAATGGCTTTGCTCCCCAGCGCAGTGAAGTGGTGGACCTTTTGTCACTGGTAAGT
GCTGGGTTCTTGCCAGTCACCCACCCCTGGCTGAGTTCTCTCTCACCCATTCCTTTAAAAAATCTGTTTGCACTGTCCAGTTTCCTCC
CCTAATCAACTTAATTCCCCTTCTTGGCTTCCTCCTCAACTAACTAGCTGGGGTTTTCCGTACTCATAAGTCCTGGCTCAGCCAGACC
CCTAAAACAGCTCAGTAGATTCCCCAGCTTTTTACCAAATGAATTTATTTATTGTATTTTCTCCTCATTCCTTGTATGTTCCAACAGT
ACGCCAATTTTTCTTGATGCACGGAGCGTGTCCTACTTCTCTACTGACATTTACATATTAACTTAGCTACAAGCACAGTCTTATAGA
TAAATATTGGTCAAGACCTTAAATTCTCCAAAGGATTTCCAATCTTATGGTAGATTTGGAGAAAGCTGCTGGTGAACAAAGGGGGAA
ATGGCTCCCTAGGAACCAACTCCTCAAACTTCTGGAGTTTTTATGATCCCTTGTTTTCTAACCTGCTAAAATCAGTATCATTTTATT
GTATTATTTTTAAAAAAAACTATTGTTGAAGTATGACATACATTCAAGAAACGTGTGCAAATTGTATGTGTACGATTTGGTGTCTTTTT
AGGAGCTAAGTTGCTTCTGTTTTTACTTGAATCTTTGTTTATAGAAACTGGGGGAAAGTTTACTTTCTTTTCAGAGAAGCCAAATGG
TATGATAGAAAAATCTTGAGCCTGATGTGTCAGACATGCCCCTAGCATAACTTGTTGAGTAAAGAGGTTATTTTTAAAATGTGAATG
TTCTGAGACTACTCCAAAGTCAGAGCCAAATCTACTAGGAAGCTTCTAGACTTCACTCATTCTGCATCCCATTACTATCTTTTTATC
CATGTTTTACTTTCTTCTCATATTCAGCAGCATCTTAAGCCTCTTTATTTTCTGTTTCTTGACTGTCACCCTTAATGCCAGTAGAAT
GTAAGCTTCATGAGAACAGAACTGCATCCATCTTGGTCTTCACAACATCCCTGTGCCTACTCAGTGTTTGGCACACAGTAGGTCCTC
AGTCAACATTTGTAATTTAGTGGACAGATGATATGACAAGATGATAAGAGGGGGATTTAAAAAAAATCATCTAGCAAAGCCCAAGAGGA
AAAAAAACAAAGCTATTTTAGAAATGAAATACCAATTTGAAGCAGTAAGAATAGATTGGATATCTTTGAAAACCATTAATTGAATGA
AGAACCAATTTGAGAAAACAATACAGAATGCAAAGTAGAAAGATAAAGGCAAAAATTATAATATGGAAATCAGACAAATG
GATTTGTCTGTATCCAGTTATGTGGATAATTAAAATGGAGACCCTCAGAAAATTGAACCGAAGAGTAAAATGAAACTCAAAAATGTA
GTAGAAATTGTTGGGGAAGTAAAGAAAACTTGAATATGTAGATCAGAACATATATGTTGATGACGTTATTGACTTTGAGGTTAAAAAT
ATATATATGTGCCTATGATTATGGGGAAAAAAGCAGTCGTCTCAGAAAGAAAAACATCAAGTTAGTCTTAGACTTTGCAGTGCACTC
AGTACCAAAGAGAGAGGAGGTCCAGACTTGGACCTGCGAGGGAAGAATAATAACCGAAAATTTTATATCAATTCAAAAAGACATTGT
CAAAAATACAGGGATTCAGGAAACTGAGAATGCACTAAGCCTTCTGGAAAAAACACCTAATGACAAAATCTAGCCCAACAAGATGTA
AATGAATATAAAGGACTCATAATGAGGAAACCGCATTATGACTGGCTCTCAACCCTGGCTGCATATTAGACTCGTCAAAGACCTTTG
TAAAAGGTCACACATTGACTCGTCAAAGCCCCTCTCCAGACTAATTCAATTCAGAATCTCACAGATGGGGCCACAGAATCAGTATTT
TTTGACACAACCTCAAGTGAGAATATTGTGTAGACAAGATTGGAAACCACTGATTTAGATATAGAAACAAAGGCTAATCAACTGTGA
GAATTATGGTCACAGAATAGAAAGTAACTATTATGAACACTGAAAATGTAAAAAAAAATGTAACAAAGAAAAATAGTTAGAGGAAGGA
GAGGAAGTAAAGGAACAATCATTTTCTCATGATTATTATTATTATTTCAGAGTAAATTGTGAGTTATTTCACAATTCAAAAAGAATGGAC
TGTTTTAAAAAATTAGTAATAGATTTCAAAATGTCCATTTTGTAAATCGTTTCTGAATACTTTGTCAACAGTTACTCATCATTAATG
GCTTATACTTCACTAAAATTCCATGGAAAACCAACTAGTAGCCTGTAGAGTCACATAGGAGAGAACAAGTGAATTCTTTGGGTGGCG
CAAGCATAGATGTTAGGACTGACAAAAAAAAAATAATAAAAATAAACCTGTGCATTGATATGATCACAAATGATCAGGGAAAGAGGA
AACAGAAACTCTCATACGCCATTATTACAAGTGTAAATTGGTTCAACCTTTTCGTCTTAATTGACACATTGTAATTGTATATATTTA
TGGAAGCACAGTTTGATATTTTGATATACATACATGGTATATAACGATCAAATTAGGATATTTATATGTACCCATCATCTCATGCATT
TATCATTTCTTTGGAATAAAAACATTCAAAAGCCTCTCTTCTAGCTTTTTTGTAATATATTATAACTGACCATTAACCATCATCACC
AATAGAACAGAATTTATTCCTCCTGTCAAATTATAACTTTATACCCATTGACCAACTTCTCCCTATCCTCCTCTTCCCTCTCCCCTC
CCCAGTCTCTGATAACCACAGTTCTATTCTCTGCTTCTATAGTATCAACTTTGTATTTTTTAGATTCAAAATATGAGTGAAATCAT
GCAGTATCAGTCTCTCTGTGCCTGGATAATTTCATTAAATATGATATCCTCCAGGTCTTTTTATATTGCTGCAAATCACAGGATTTC
ATTATTTTTTATGGCTGAATACTATTCCATTGTGCATATATTCCACATTTTATTTATCTATTCATCTGCTGGGGGACAACTTAGATTG
CTTCTATATCTTGGCTATTGTGAACAGGGCTGCAATAAACTTGAGAGTGCAGATATCTCTTTAACATAGCGACTTTATTTCCTTTGA
ATATCTCACCCAGCAGTGAAATTGATGGATAATATGGTAGCTCTATTTTTATTTTATTGAGAAACTTCCATGCTGTTTCTTGTGGTGG
CTGTACTAATTTACAATGCCACCAGCAATGTAAGGGTTCCTTCCTCTCCACAACTTAACCAGCACTTGTTTTCTTCTGACTTTTTGA
TAACAGTCATTCTAACTGGAGTGAGGTTATACTTCATTGGCGTTTTAATTGTATTTCCCCAATAATTAGTGATATTGACCATTTTTT
TCATGTACCTGTTGTCCATTTTTATGTCTTCTTTAGAGAAAGTCTTTTGCCCATTTTTTAATCAGCTTGTTTGGTT
TTTTCCTGTTAAGCTCTTTATATATTCTGGATGTTAACACCTTGTCAGACATAAACTTTGCAAATATTTTCTCCCATTCTATAAGTT
GTCTTTTTACTCTGTTAATTGTTTCCTTTGCTGTGCAAATTCTTTTCAGCTTGATGTAATCCCATTTGTCTAATATTTGCTTTTGTT
GCCTGTACTTTTGTATTTTAAAAGGTCCAGTCTTTTAGAAGAACAATTTGACATTATATGTTATTACTTAAAATGTTTGTATACTTC
ATCCCAGAAATTCCATGTCTAAGAATTTATCCTAAGAAATGCATGTGTAAATATACAAAAATATATCTACAGAATTACTTAATGCAG
CATCACTTGACATTTAAGAAACAACAACATCCCAAACATCCATTAATGGGAAAATGCTTAAATGTATTATAGCATATCTATACAATG
GAATACTTTGTCATTGTTAAGAATAATCAGGTAAGTGGGGCATGGTGGCTCTCGCCTGTAATCCCAGCACTTTGGGAGGCCGAGGCG
GGCAGATCACCTGAGGTTGGGAGTTCAAGACCAGCCTGATGGAGAAACCCTGTCTCTACTGAAAATACAAAATTAGCCGGGTATGGT
CGTGCATGCCTGTAATCCCAGCTATTCAGGAGGCTGAGGCAGGAGAATCGCTTCAACCCGGATGGCGGAGGTTGTGCTGAGCCAAGA
TCGCGCCATTGCACTCCAGCCTGGGCAACAAGAGTGAAACTCTGACTCAAAATAAAATAAAATAAAATCAGGTAATATATTTCCAAA
ATTATCAAGTTGTAAATGTAAAAGTTAATCTTTATTGTGTGTAAATTATGCTTTAATAAAGATGAGTTTTAAAAGTTAGTGATGTAA
ATTTTTAAGTAATACCACAGAAAGATGCCCATAATATATAGCTAAGGGGGAAAAAGCAGGTTGTAGACCCAGTATGTTATTGTGATCCC
ATGCACGTGTGTGATTTTAATGTATTTATTTATGTGTCTATGAGTGTACTTATACATAGAAAAGATCTAGAAAGTGTCACCAAATAC
TCAACAGAAGTAATTTCTGAAAAAATAAAGAAAATGGTAAAACTTTTTATTTTTACTTAGATAATTCTGTAAAGTTTGATTTTATTA
AATGAATAGCTGGGTTTTTCATTTTTAAAAAAATTTCAGTAGTTTTGGGGACCAGGTGGTGTTTGGTTACATGAGTAACTTCTTTAG
CAGTAATTTCCGAGATTTTTGGTGCACTCGTTGCCAGAGCAGTGTACACTGAACCCAGTGTGTAGTACTTTATCCATCACCCCACTCT
CACTCTTCCACTGAATCCCCAGAATCTATTATATTATGCTTAGGCCTTTGCGTCCTCATAGCTTAGCTCCCACTGATAAGTGAGAAC
```

```
ATACAATGTTTGGTTTTCCATTCCTGAGTTACTTAGAATAATGGTCTCCAACTCCATCTAGGTTGCTGTGAATGCCATTATTTTGTT
CCTTTTTATGGCTGAGTAGTATTCCATAGTGTACACACACACACACACACGTGCGTATGTATCACATTTTCTTTATCCACCCCTTAA
TTGATGGGCATTTGGGCTGGTTCCATAGTTTTGCAATCATGAGTTGTGCTGCTATAAACACGTTTGTGCAAGTGTCTTTTTCATATA
ATGACTTCTTTTCCTCTGGGTAGATACCCAGTAGTGGGATTGCTGGATGAAATGGTAGTTCTACTTTTAGTTCTTTAAGGAATCTCC
ATACTGTTTTCATAGTGGTTGTAATAGTTTACATTCCCACCAGCAGTGTAAAACTGTTCCCTTTACACCACATTCATGCCAACATCT
ATTATTTTTTGTTTTTTTAATTATGACCATTCTTGCAGGAGTAAGGTGGTATCGCATTGTGGTTTTGATTTGAATTTCCTTGATAGT
GATGAGCACGTTTACATGTTTGCTGGCAATTTGTGTATCTTCTTTTGAAAATATTCTATTCATGTCCTTTGCCCACTTTTTGATGTG
ATTGTTTTTTCCTGATGATTTGTTTGAGTACTTTGTTGATTCTGGATACTGGTCCCATGTCAGATGCATAGTCTGTGAATATTTTCT
CCCACTCTGTGGGTTGTCTGTTTACTCTGCTGATTATTTCTTTTGCTGTGCAGAAGCTTTTAAATTTAATTCAGACCCATCTATTTA
TTATTGTTTTTGTTGCATTTGCTTTGGGTTCTTGGTCATGAACTCTGCCTAAGCCAACCAATGTCTAGAAGAGTTTTTTAAAGTATA
TTCCAGGGTTTATATGGTTTTAGGTCTCAGATTTATGTCTTTGATCCATCTTGAGTTGATTTTTGTGTAAGGTGAGAGATGAGGATC
CAGTTTCATTCTTCTACATGTGGCTTGTCAATGATGCCAGCACCATTTGTTGCATAGGGTGTTCTTTCCTCACTTTACATTTTTGTT
TCCTTTATCAAAGATCAGTTGGCTGTAAGTATTTGGCTTTATTTCTGTGTTCTCTCCTTCCATTGGTCTACCTGTCTGTTTTTATAC
CAGTACCATGCTGTTCTGGTAACTATAGCCTTGTAGTATCGTTCAAAGTCAGGTAATGTGATGCCTCCAGATTTGTTCTTTTTGCTT
AGTCTTGCTTTGGCTATGTAGGCTCTTTTTTTTTGTTCCATATGAATATTAGGATTGTTTTTTTCTAGTTCTGTGAAGAATTATGGTGG
TATTTTGATGGGAATTGCATTGAAGTTATAGATTGCTTTTGGTGATGGCCATTTTCACAATATTGATTCTACCCATCCATGAGAAT
GGGATGTGTTTCCATTTGATTGTGTCATCTGCGATTTCTTACACCAGTGTTTTGTAGTTTTTTCTTATAGATATCTTTCACTTACTTG
TGAAGGTGTATTTCTAAGTATTTTATTTTTGTAGGTGTCATAAACAAGTTTGAATTTTTTATTTGATTCTCAGCTTGGTCACTATTG
GTGTATAGCAGTGTTACTGATTTGTGTACATTGATTTGTATCCTGAAACTTTACTGAACTCATTTATCAGATCTAGGAGCTTTTTG
GATGAGTCTTTAGGGTTTCCTAGGTATACAATCATGTCATCAGTGAACAGCAACAATTTGACTTCTTCCTCTTTACCAATTTGGATG
TCCTTTATTTCTTTCTCTTGTCTGATTGTTCTGGCTAGGACAAAGAGCTGTTTCTAATTTTAAAAATACATGTGTCCCTAACTCATT
TTCTCTCTCAGTTCCAGTGTCTCGCCCCATCCTCACCCTCAGGGTTCCCAGGGCCCAGGCTGTGGTGGGGGACCTGCTGGAGCTTCA
CTGTGAGGCCCCGAGAGGCTCTCCCCCAATCCTGTACTGGTTTTATCATGAGGATGTCACCCTGGGGAGCAGCTCAGCCCCCTCTGG
AGGAGAAGCTTCTTTCAACCTCTCTCTGACTGCAGAACATTCTGGAAACTACTCATGTGAGGCCAACAATGGCCTAGTGGCCCAGCA
CAGTGACACAATATCACTCAGTGTTATAGGTAAGTTGACCTAACCAGCAGCACAAAAATAAAAAGACCATCTCCCCTAACTTTACCA
GTAGCCAAGATCAGAGGTACAGTTTCTAGAGTCATTTGCTGTGTGACATCCTGTCAGGCTTGGTGCCTACACAGCCTCCATCTCTGA
GTTTTGTATCCTACACGTTCAGGAACTTGCACCTTCCCTTCCCTTGCTGTACTTTTCCTGCTAGCAGCAGTCCAGCCATCACGACCA
AAATTCCCCTAACATAGGAGTAAAGGCTTTCAAAATACAAACTATATTCTGGCCAACAGTGATAGTAAGAAAGAAAGTTATAAAGTT
CTGTGTGCTTCACCTTGGAAGAGATTTAGTTGTGGTTTTCTCTGTCTGCCAATCAATAAAAAGGCCAACACAATTCTCTTATTGGCT
CACACATTTCTCTCTAGCCTCTCCTGCCCCTGTCCCAGCAGTGGGTTGGGTTCCCACCCAAGACCCTTGTTAGAAGACATAATTCTTC
TGCCTCCTTACCTGCTCCTGTTCCATTCAGCAACATCAAGATCTTCCATGCATCCTTGTCACAGGCAGACTCCTAGTTATGCCCACT
CTCAGTGAAGGCTTTGGCACTGTACTTTCAGTCTTTCTCCATACTCCATAATCTGCCATCATTTCATGACTCTCATGGTCACATGTA
TAATCCCCTTAACACCATGGCCTGTCCTCTCCACACCAGTCAGCCACTCCCACATACATACACTACTTCATCTCCAAAAGGACAATT
TTAAGCCCTTATTATCTGACAACCACCCCCCTGGCCTTCTGCTTGCGTCAGCTCCTCCTGTCACTATTGTTCTCAGACCTCAACAGAA
CTCCCAGGTCATTAATACCTCCACTAATTCCCTACTCCACTAGACAATAACCCCTCCACTCCCACCACCAAATTGAGTGTCTAGCT
CTGTATCCACCCAATAACTCATCTGTCCTTTTCCTAAAATATAGAGGAGAGGACTTCCCTCCCCATAGGTGAACATCTCCATCTGGT
CTCCAGATTTCATCATTTTGTGCATTCTCAGAAGCTATGTAACTGATTATCCCTTTCTCTTCTGTGGCCCCCAACATCTCTGTCTCT
GTAGGATCCTTTGCACTGGCACGTAACATGGTATATCTCTCCCATATCCAAGGAAAACCCTCTCTTGGTCCCACTTATACCTCCATA
CACTACCCCTTCATGGCTAAACTTCCAAAAACAGTCAGTTACCCAGACTGTCTCCATGTCCTCACTATCCACTAGGCTCTAATCTAC
TAGGTTTTTCCAACCTCTCCACCAAAATGTTTCTCACAAAGGTCACCGATGACCTTCAAGGCTTTAAATCCAATGGCCATTTTTCAA
TCCTCATCTTCCTTGACATCTCAGATATATTACATGTTGTTTACCATTTCTTCCTTCTTGAAACCCTCCCTTCAGTGAATCTATCTT
CTTCTGGCTCTCTTCTCTCCCTGGCCACTTCTTCTCAGTCTTCTTTACTGGCTCCTCCTTTTATAGAAAACCTTTAAGCACTGGCAT
GTTCCAGAACTTTGACTTATGCCCTCTTCTCTTATCTAAATCCTTTGTCTGGATGATCTGGGTATGTCATGTATAAATGGCCTTGT
CATCTATAAATAATGATTCCTAAACTTCTATCTCTAAAAGGGACCTCCCACTTGAGCATCTGCACATGACATACCTTCACTCAAATA
TCTCAAAAGCACATCAATCTGAGCATTTCTAAAACTGAACTGCTGACATTCCCTTCCTCTTTCAAGGGTTCCTACCTCTGTGAAACA
CATCTCTTCTCAACAGATACACAAAGCAGGAACAGAGGAGTCTTCCTCAACATTTCTCTTCCTCACCCCTCACAGCCAGTTAATCAT
TAAGTCCTGTTGATTTGACTTCCTAAATATTTCTTGGGTCTCTATCTCTACCACCTCTATCTAGACAAGTCCACCATCATTTTGACT
ACTGCAGTGCCACTCATCTATCCCTCAATTCCACTTCCATTCCACCACAGTCCATTTCATGCACAGAGGTCAAAATGGTCTTTTAAA
AGGGACAAATTTGATCATCTCACAGCCCTGCATAGATAGACTTATAGACTTCCCATTGCTCTGTGAAGAAGGATCAAAATCCTTTCA
TGGCTTATGAAGTCCAGCCTAGATTCCAGTCTCCTATTTCTTGGTAGTTTGCAGAATCTTCTCAGTGTGATGCTACTTTATTTCAA
TAATTCAAATGTGCCAGTCTTATTCCCAACTCAGGGCGTTTGCACATACTGTTTGCCTGCCTGGTGCACTCTCCACCTCCATCATCA
ACTGGTTAATTTCAATGTTAAATTTCAAAGATCATTTTGTGAGGAAGCTTTCCTTGACTAACACATACATTTCATAGTACCCTGTCCT
TTATCAATTATCAAAGTTTTAATTGTAATTTACTGTATTTTTATTTAATTGCTGCCTCTCATCCTTCTAGATTGTAGGCTCTATGAG
AGCAGTAACTATGTCTTTCTCATCACTGATTCCCTAGCATGTCATGCCATTCTTGGGACTTATTAAATGCTCAATTAATTTTTATTT
TTGCTCAATTTTTAAAAAAAATTTTTGTTCAATTAATTTTTGATGGATAAAATGATGTAAAAAATGAGCAAAATAACTTGACAATACTCA
TTCTCTTGTGGGCCCTATTTCTGATGCAGCCCAGATGCAATTACATCCCTGGTGATGTAAGCAGCAAGTCTCTCCTGTTCTTCATTT
TCTGTTGAACTGATTTCTCCTCTCTCCAACTGTCTCTTTTACCTCTAAGACACCTGAGTTTCAGTTTAATCTTTGTTTTCACATTCTT
CAAACACATTTGAGTTTGTATATTCCACTATTCCAAGCTGTTTATAGCACTACACAAATGTTGTTCTTTCTGGAGATCTGAAAATAT
TGCTGAGTTTTGAGTAATGTCACAAGGGCATTAAAAACTGAGAAAAACATTCTTCCATTTGAAATGTCTTTAGCATGAAGGAATATA
TTTTAGCAGAAGGAGCTGATACATAACGTTTTTCCTCCCTCAGTTCCAGTATCTCGTCTCCATCCTCACCTTCAGGGCTCCCAGGGCC
CAGGCTGTGGTGGGGGACCTGCTGGAGCTTCACTGTGAGGCCCTGAGAGGCTCCTCCCCAATCCTGTACTGGTTTTATCATGAAGAT
GTCACCCTGGGTAAGATCTCAGCCCCCTCTGGAGGAGGGGCCTCCTTCAACCTCTCTCTGACTACAGAACATTCTGGAATCTACTCC
TGTGAGGCAGACAATGGTCTGGAGGCCCAGCGCAGTGAGATGGTGACACTGAAAGTTGCAGGTGAGTGGGCCCTGCCCACCAGCAGC
ACATCTGAGAACTGACTGTGCCTGTTCTCCCTGCAGCTGAAAATGGAGCCACAGAGCTCCTCAGGGCTGTTTGCTTGTGTGGCATCC
CAGCACACTTCCTGCCTGCAGAACCTCCCTGTGAAAGTCTCAGGTCCTTTGTGGTGACGTCTTCAGGATCTGATGTTTCCCAGCAGT
CTTCTTGAAGATGATCAAAGCACCTCACTCAAAAAATGCAAATAAGACTTTTTTAGAACATAAACTATATTCTGAACTGAAATTATTAC
ATGAAAATGAAACCAAAGAATTCTGAGCATATGTTTCTCTGCCGTAGAAAGGATTAAGCTGTTTCTTGTCCGGATTCTTCTCTCATT
GACTTCTAAGAAGCCTCTACTCTTGAGTCTCTTTCATTACTGGGGATGTAAATGTTCCTTACATTTCCACATTAAAAATCCTATGTT
AACATAAGCTATGTGTACAGCCCTTCAGGAGACACACTCAGCGATCTTTAAAGCAAGGGACACAGGTGACCAGGGCAGAGAGTGGGG
AGCAGGATGACATGGAATGGAAATGGACATAACGTTTTTCCTCCATCACCCAAATTCCTCTATTCTCGAAGCTGTTCTTTGATCTCTTTCAATTAAGTG
TAATAACCCAAGGAAAGCTGCTTCTAGTGAGCTCTGAATCTTTTTGGTGATGACCTCCTACAAGCCAAACTTTGAAACCTCTGTTTA
GAGAGAACTGGTGTTTAGCAAAAGTATGCTAAGGGGTGTTGAGATGAATCTTCTAGACTTGGATCTGAAGTAAACTAGCTGTATGAC
ATTGGCCAAGCACCTCTTCCCATACTGACCTCAATTTCCACACCCACAAAGTGGGAAAATCAGACTAGATGAAAATCATGATCATCC
TTTGTCCTAGAATTCTATGATTTTTGGTAGACAGGGAATATGGATTATGCAGACGTACAGGTTCCTAGAAGGAGATATGCAAAAGTAA
AAGCAGGTTGTACCTGCCCCTGGAATATATCCTGGAGAAACACGAGACCATACAATGGTATGCAATCACCATTCCAGTCATGTAAAG
CAAAAATATACACTGATGCTCTTATTCCCTTTGGCAAGGTGCCTGGGTATCATAAGGAATAGACAGAGTCACATAAGCAGGTTGATCT
```

```
GGGCATGCTAACCCATGATCCAGCATCTCACAAAGTAGCCAGTGCAACCATCTCATTGTTATCCAGGAGGTGATGACTTCAGATTTG
GGTTTTCCCATTCAGTCAGCTCGTTCTTCTTCCCCCACCAGCAGCACCAACCTCATCCAATTTTCCTGAGATTTGCAGGGGCTCTCC
CCACACAGTTTACACACCTATCCCTTAGAGCCAGCACACAGGCATGTACCACGGACCGAGGGCTGCTCCATCTTTCCTCACAATACA
GCAAGCCAACACATCATACCTCCTACATTCTATACTTATGATTTCATGATTTTCTGCTCATCCTCACTCCCTCATTTCTATAATTCT
CTGTCGCAAATGCATGTTATTTCTGCTGCTGTTATCACAGCTAAAGGGTTTCTTGCCCAGCCTGAATGAATTGTGTCTGAATCCTGA
AACTAGGACCCAGGTCATAGAGACCAGGAGCTGCCAGAAAAGGAAATATTATTTCTTATTATCATCATAACCAGTGTCATCATCATC
AACACTCACTGAGCCTTCACCAGGTTCCAGGCTCTGTGCTAAATGTTTAATAGGCAACATCTCATTTAATTATTAAGATAAACCTGT
GAGGATAAATTTTAAGCCATCGTATCCCCTATTTGCGGAAAAGAGACTGAGGTTTAGAGAAGTGAAGTAACTAGCTTGCAGACACAC
AGCTAGCCAGGGTAAAGCCAGGTTGAAGCACAGTCCTCTCTAACTCTTCACATTAGTCTGATACATGATGACATTTTCTCATCCGTT
GTGGCCACTTTTAACACAACATTTTAGTTTTTAGTAATTTGTTATTCTGAAAGAACACATGAATGTTTGTGATGAAAAAAATCAAAT
AATACAAAAGGGCCAATATTGAAAAGTAAAGGTCTCTTTCTACCTGCTCATATCACTTCTTACGCACATATTCTCCAGACATAACCA
CTGTTAATTGTTTCATGTGTATGCTTCTAGAAATAATCTACAAACCTAAAGGTAGATAATTTTTAGAAAGCTAGAATAATATTACAC
ATATAGTTATGCCAGTTGCATTTTTTAACTTAAAATACATCCTGACTTTTGTTTAAGAAATGCATCTTGGATATCATTCATGTTTCA
TTACCCAATCCAGTAGGTTGTCTCTAATTTTTAGCTCTTGTAAAAATGCTACAATAGAAATTCTTGTAGAAATATATTGGCATATTT
TATGTCAGTATCCCTAAAGGAGATGGCCCTAGAAGTAGGACTTGAACTCCTGGGCTCAAGTAATCCTCCTGCCTCATACTCCCAAAT
CGCTGGGATTATAGGTGTGAGCCACTACACCTGGCCCACTAGAAGCAGATTTGTTGGGTCAAAGTTTATGTGCACTTTAAATTTTGT
AGATATTGCCAAGTTGCCATTCAAGGAAGTGTGTTAAATTTGTATTACCACCAAGGGCATATGTAGGTGCCTGTTTATCTACAAGCT
AATCAGTATGATCAGTCTATTTAAGCTTTGCCAACCTGAAAAATAGAAAAAGAATAGTATTGTATGGTTGTCTTTGTGTTTAGTTA
ATTAGTAATGAAGATGAGAATCTTTGTATATATTTATTGTCTATTTGAAGTTTTCAGTGATTTATACACTTTTTACAGTTTTCTATT
GGATATTTTTGTACACTGAAAACTATTAGCAGGTCAAAGTAATTAGCTTTATCATCTATGTTTTAATCTACTTTCCAAGATTGAGTT
ATGACTTTTTACATTTGTTTATGATACCTTTTGTTTCTTACAAATATGTTAGAAGCAGAAACTGCTGTTGACTTTTTGTTCTCTTTC
AGTTCCGGTGTCTCGCCCGGTCCTCACCCTCCAGGGCTCCCGGGACCCATGCTGCGGTGGGGGACCTGCTGGAGCTTCACTGTGAGGC
CCTGAGAGGCTCTCCCCTGATCCTGTACCGGTTTTTTCATGAGGATGTCACCCTAGGAAATAGGTCGTCCCCCCTCTGGAGGAGCGTC
CTTAAACCTCTCTCTGACTGCAGAGCACTCTGGAAACTACTCCTGTGAGGCCGACAATGGCCTCGGGGCCCAGCGCAGTGAGACAGT
GACACTTTATATCACAGGTAAGTTTCCCGTGCCCGGGCCACAGATGCGGACAAAATGTTTCCTCCGCGGACCCTCTGATAACCCTCA
CAGTGCTTGATCAGAAAGGCACTCCTCAGGGCGAAGTAACACAGCTGGCAGCTCTCTAAACTGCTATATTCCTGAAGTGCTTCTGA
AGTGATGGCGTTGGGGCTGAGAGCGCGGGTTGGCGGGCTGGGCGCAAGAGCGCCACTGACTTTAGAGGTCCTCACCTGGCTGCCTCC
TCTCTCACTGGCCACCGGCCACCCTGACCCTGGACACTTGTCTCAGGCCAGAGACGGCTTCCTCACAAAGACAGGAGCCATCTTCT
GTGCCACACCAGCCCCTGCTTAGCTGCCGGGGACCTGCTCACCTAGGTTCACCGTCAGGCCCAGGCAGCAGCTTCCGGGAGCGGGCA
GTTCCAGACCACAGCCTCTCCTCTGGCTCACACACAGGGTCTCCCCCTCAGGGCTGACCGCGAACAGAAGTGGCCCTTTTGCCACAG
GAGTCGCCGGGGCCTGCTCAGCATAGCAGGCCTTGCTGCGGGGGCACTGCTGCTTCCTACTGCTGGCTCTCGAGAAAAGCAGGTGGGT
GACTCTGGGCACACCTTGGCTTGAGTTCATGCCCTTTCCCAGCAACGCTCCCTTGGGCTTCATGCTGCCTTTGTGACCATCCCAGGCT
CGCAAGAGCTCATCCGCTCTGTCCCTCAATCCTCTTCCTTTCCCGCTGGGTCCTCCCGAATTGCTTTTACCCAAATGCACAGTTTCCA
AAACAGACCTATAAGGTGGGAGACGTCTTGATGAGATGTAGTTTATCCAACATCATAAACTACAGAGTGTCTTCCTCTCGACCCTTA
TAAGACCCCCCCAACGAATGGGAAGAGGAGGAGAAAAACTCAGAGAAATAGAAATGAAAATAGAAACCAGATAGGATTCAGTAATTT
TCCGATTTTTTAAATGTACACTATTTTTATAGTTGATCAAGTCTGTTTTCCTGCCTTGGAGATTATCTTTAAAATCTCCAGGTTAAA
GGATTACGGATTGTGGACTCCCGTTTCACATCATAGCAAACCCCTCTGCCAGGGTCAGTAGTTTTTGCCCAATCTGCTACCTCCCTG
GGGCATAACAAAGAAGGATTCAACCTTCAGTGACAGCAACTCCTGGCAGTTTCTAAGTGAGCCTTGCCTGAGCTGTGCCCCACTGAG
GGTCTCATCTTCCTGAATCCAATGCTCCTCTGCTTTCCTTTACAGGGAGAAAGCCTGCCTCTGACCCCGCCAGGTAAGAGCTGAGCT
GCATTCCTGTGTGATTAGTGAAATGTCCACAGGGTGGAAAAATGGAATTGAATCTGATGATTACCAAGAGCACAGTTTCTGCCAGGT
GCTGCTGAGCTGGGGTGGCCACTACCTGTTGTACGTCCTGGGGATATCTTAAGGCAATTCCCAGGACCTTTGCACCCACCCCCT
TAGCTGTATAGGCTGTTATCTCATCAAGTATTAATTGTCTTTTCATTTATTAACCCACATCCTTGGACCATTGTTGGCTTGGTTGTC
TGAATGACCACTATGACCACCAAGTCTTTCCATATCTTGAGATGACCTTAGCTCAGTCAACCTAGAAATCTCTGGAGATGCTGATCT
CATCAACACCTCTTGTGATAACAACTGAGAAGATGGAGCCGCACAACTTGGCCACCCAGAGAGGTCACTCTGCCAGCAGGAGGTAGT
CCCTGTGAAGAAGGGGCCACCATCTTGTATCCCTAAGGCTGACTTTAGGAGCCACACAAAGGCTCACAGCTATCCCTGGGCAAGCCC
ACGATAGCAGCATCCAAACATGTAGAGCACCTCGGAGCCTTCTGGTCACTGATCTCTCTGAGCATGCAGGGTTCGGGAGCCAGCTTTG
AGCCTTGGCTCATCCCCTTTCTGGTTACATGATCTTCATAAGTCACTTATTGGAGCCTCCGTTTGTTTCAGCTAAAACGAGGATGAA
ATAGAACTAACAACGTATTGCTGTAAGCATCAGCACCATGCCTGTTACACACTAGATGACCAGAAAGCCTGGAAACATATATAATAT
GATTATGATTCATAATAGAATGCCCATGGTCAGTTTGTTATGTACTTGTTCATGTTTCCAGGCCTGGCAGCACCCTGCAGTAAGAA
GTCACTCACTAGGAGTGATCATTATAGCTACTTTGTCTGGATTCCTAAGAAACATTCACATTTCTCTGTCTCACAGGAGCCCTTCAG
ACTCGGACTCCCAAGAGCCCACCTATCACAATGTACCAGGCTGGGAAGACTGCAACCAGTGTACACTAATGGTGAGGCCCGAGGAT
CCTGCATTTGGCAGAAGCTGGAGGGCTGGTGTGGGAAAGCCGGTGGGGCAGGAGATGGGAGGGACTATGCCGAGGCCTAGAGGGCTC
TAGTCCTTCCAGAAAAGAGACTCTGGCTGGGGGAGGAGGAAGTATCTCTAACAAGAGGCAAGAACCTCAGCATCCTCTGAAACTTCA
ACTTTTTTCTAAGCAAATCCTAGAGGAGAAAATGTGGTTTACTCAGAAGTACGGATCATCCAAGAGAAAAAGAAACATGCAGGTAAG
ACCCAAGGATGTCTCTGTTCTGGCCCACCCTCAACTTAGACAGTATAGCTCAGTTTCTACCCACTCAGCAGATCTGAGCCAGACAGG
CATAGAGCCGCCCCTCCCACTGGGTCATGGGGCTTCTTTATGTGTGCCTATGCTGCTTCAGGTGAATCCAAGCCTCCCCTGCACTCT
CCTTTCCCTATTCTCTGCAGAGGGCCCCAGTATAACCTGAATAACAAAACTATCTTTTTGGGCATTCGCTTAACCTCTCAGACCTTC
CATATCCCTATCCATAAACTGTGACTACTTCCCTACTGTCTTAGTCAGTTCTGGCTCCTGTAACAGAGTACCATAAACTAGGTGACT
TAAAGAACTAATATCTATTTCTCACAATTCTGGAGGCTGGAAATCCAAGATCAAGGTGCCAGCATGGTCAGGTTCTTAGTGAGGGCC
CTATTCCCATTTTCCAGACAGATGACTCCTTGCTGTATCCCCCACACAGCAGAGAGAGAGGGAGAGATCATCTTTCATGTCTCATCA
TGATAATCATCATGACATAATTGTCACCCAAAGGCTCCACCTCCAAATGCCATCACATTGGGGATTAGGTTTTAACCCATACATTTG
GAGAGCAACACAAATATTTAGTTGATAGCACCTACCTACCTGTACTAGTTTCTGATTGCTGCTGTGACAAATGACCATAAATTTGGG
GGGATGAAGCAGTACAAATTTATCATCTTACAGCTCTGTGGGTCAGAAGTCTGACGCCGGTCTCACTGGGCTAAAATCATGGTGTTG
GCAGGGCTGTGTTCCTCCCTGGAGGCCCATCCACTTTCCTGCACCCATGTCCCCTTCCTTTCCCAAAGCCAGCAAAGTCACATTTCT
CTAATCATTCTTCCATAGACAGGCCTCCTTCTGACCTCACCTGAGAAAAGTCCTTGGGTTTTAGAGAATCATATGAGTATATTGGGC
CCATCTGGATAACCCAGAATAATCTCCCTATCTCAGGGTCTGTAATCCTAATCACATCTGAAAGGTCCTCTTTTTCCACTTAGGGTAA
TATAGTCACAGGTTGACGCAATTAGGGCGTGACATCTTTGGGGGCACATTATTACAGCTACCATGCTGTTTCATGGGGTAACTGTGG
GGACTAAATGAGAAAATAGGTGTGGAAATGCTGTCGAAACTGCAGAGGGTTGAGCAGCTGTGACGATGACTACATGCTGGAGGTCTG
GCTATGTCATCAACCCTCACTCACAGTCGGTGGTTTGAAATGAGGGAATCCCAGCCCGGCTGCCTCCTTACTCGGAGCAGGAAAGAG
GAACAAAGTCCTTCACGGCAGGCTTTTCTTTCTACCTCTGCTTCATAAAAAGTGCTGCGTGATTTTAGCCAGTGTGCTTCCCTCTCT
GGTTTTCTAATTTTTCTTATATAAAGTCAAGTGAATTGTGTTTGTGCCTCATTCTTGGTTCTATAGCACCCCTCTGAGACATGTGAA
CTCATTCACTCTTTACAAGCCCCTTTGAGGTATGCGCTGTTTACTACTCCCATTTTACAGATGAGGAAACCAAGGCATAGACACCTTAA
ATGACTTGCCTGAAATCTTATAGTCAATCACCAAACAGTCTTCTCCAGAGTCCAAACTCTAGACCAAACTCTGCTTCTTCATGCAAT
TCCAGGGAGTCCTGATGCTCATTCCAAGCCAAAAGGGCAGAGCCTTGTCTGGAGAATGTCATATTTCTCTCCAGTTTGATGATAATA
TAACAATAATAATTTATAATATCTATCATGTGCTGAATAGTTCCTATTATGTGCTGGGTATAGTTCTATAAGTGCGATAAATAAATA
CACTATATTTGTATGGATAGCTCTTAGAACTATACCTGTAGCACTTATATAGAACTATGGCATGCATACATATGATAATAGAGAGAG
```

```
AGAAAATGTATTGAATATAGCAATCAGGACTGTACCTGACACATAGAAACTGCTCAATACATGACAGATGATAGAAATAATATATGC
ACAGACACACAGATACATGTGCTCACACATATGTGTAAACTCATATAGCTTCACCACAACCTTACAGGCAGAGGATATTTTTATTCC
CATCATATAGATGAGGCAACTGAGGCAAGAGATGTTGAGTGACTTCCCCAACCACGGAGGTGACAAGACAGGCGGCAGGCTCAGTT
TCAGGTGGTCTAGACCCTGATGGAGTGTGCTCTCTAAAACTTGCACCCACCACACCTTCCCAGAACAATAATACCAATTATGCTCAA
ATAACTAACAAGATAAAAGACAAATGACTTTCTCTTGAGGGCTCAGAGTGGGCCACAAAGTGTGAAGGGGAGTTGA
AGAGAGGGTCTGTCAGTGCTGAGAGGCTGAGGGAGTTGGGAATGGAGAGGGACTGGGTGAGCACCAACTAAGAGGAATGGCTACTCA
CCGCACAGAGGGAGAGCGGGCCTCCAGGAGGCTCAGGGCTGGCCCCACAGCGCAGGCGCAAGGGAAGAGCCCCATGCCAGTCTTCCA
CAACTGAACACTTTCCTCTTTCCACAGTGGCCTCTGACCCCAGGCATCTCAGGAACAAGGTGAGTCTCCCCTTCTGCATGCCCTCCC
CCACCCCCAGGCCCACCCTGGTCCTCACGCATGTGCTCTTGCCTCCTAGGGTTCCCCTATCATCTACTCTGAAGTTAAGGTGGCGTC
AACCCCGGTTTCCGGATCCCTGTTCTTGGCTTCCTCAGCTCCTCACAGATGAGTCCACACGTCTCTCCAACTGCTGTTTCAGCCTCT
GCACCCCAAAGTTCCCCTTGGGGGAGAAGCAGCATTGAAGTGGGAAGATTTAGGCTGCCCCAGACCATATCTACTGGCCTTTGTTTC
ACATGTCCTCATTCTCAGTCTGACCAGAATGCAGGGCCCTGCTGGACTGTCACCTGTTTCCCAGTTAAAGCCCTGACTGGCAGGTTT
TTTAATCCAGTGGCAAGGTGCTCCCACTCCAGGGCCCAGCACATCTCCTGGATTCCTTAGTGGGCTTCAGCTGTGGTTGCTGTTCTG
AGTACTGCTCTCATCACACCCCCACAGAGGGGGTCTTACCACACAAAGGGAGAGTGGGCCTTCAGGAGATGCCGGGCTGGCCTAACA
GCTCAGGTGCTCCTAAACTCCGACACAGAGTTCCTGCTTTGGGTGGATGCATTTCTCAATTGTCATCAGCCTGGTGGGGGCTACTGCA
GTGTGCTGCCAAATGGGACGACACACAGCCTGTGCACATGGGACATGTGATGGGTCTCCCCACGGGGGCTGCATTTCACACTCCTCC
ACCTGTCTCAAACTCTAAGGTCGGCACTTGACACCAAGGTAACTTCTCTCCTGCTCATGTGTCAGTGTCTACCTGCCCAAGTAAGTG
GCTTTCATACACCAAGTCCCAAGTTCTTCCCATCCTAACAGAAGTAACCCAGCAAGTCAAGGCCAGGAGGACCAGGGGTGCAGACAG
AACACATACTGGAACACAGGAGGTGCTCAATTACTATTTGACTGACTGACTGAATGAATGAATGAATGAGGAAGAAACTGTGGGTA
ATCAAACTGGCATAAAATCCAGTGCACTCCCTAGGAAATCCGGGAGGTATTCTGGCTTCCCTAAGAAACAATGGAAGAGAAGGAGCT
TGGATGAGGAAACTGTTCAGCAAGAGGAAGGGCTTCTCACACTTTCATGTGCTTGTGGATCACCTGAGGATCCTGTGAAAATACAGA
TACTGATTCAGTGGGTCTGCGTAGAGCCTGAGACTGCCATTCTAACATGTTCCCAGGGGATGCTGATGCTGCTGGCCCTGGGACTGC
ACTGCATGCATGTGAAGCCCTATAGGTCTCAGCAGAGGCCCATGGAGAGGGAATGTGTGGCTCTGGCTGCCCAGGGCCCAACTCGGT
TCACACGGATCGTGCTGCTCCCTGGCCAGCCTTTGGCCACAGCACCACCAGCTGCTGTTGCTGAGAGAGCTTCTTCTCTGTGACATG
TTGGCTTTCATCAGCCACCCTGGGAAGCCGGAAAGTAGCTGCCACTATTCTTGTTTCCCCACCTCAGGCCTCACACTTTCCCATGAAA
AGGGTGAATGTATATAACCTGGCCCTCTCCATTCAGAGTTGTTCTCCCATCTCTGAGCAATGGGATGTTCTGTTCCGCTTTTATGA
TATCCATCACATCTTATCTTGATCTTTGCTCCCAGTGGATTGTACAGTGATGACTTTTAAGCCCCACGGCCCTGAAATAAAATCCTT
CCAAGGGCATTGGAAGCTCACTCCACCTGAACCATGGCTTTTCATGCTTCCAAGTGTCAGGGCCTTGCCCAGATAGACAGGGCTGGC
TCTGCTGCCCCAACCTTTCAAGGAGGAAACCAGACACCTGAGACAGGAGCCTGTATGCAGCCCAGTGCAGCCTTGCAGAGGACAAGG
CTGGAGGCATTTGTCATCACTACAGATATGCAACTAAAATAGACGTGGAGCAAGAGAAATGCATTCCACCGAGGCCGCTTTTTTAG
GCCTAGTTGAAAGTCAAGAAGGACAGCAGCAAGCATAGGCTCAGGATTAAAGAAAAAAATCTGCTCACAGTCTGTTCTGGAGGTCAC
ATCACCAACAAAGCTCACGCCCTATGCAGTTCTGAGAAGGTGGAGGCACCAGGCTCAAAAGAGGAAATTTAGAATTTCTCATTGGGA
GAGTAAGGTACCCCATCCCAGAATGATAACTGCACAGTGGCAGAACAAACTCCACCCTAATGTGGGTGGACCCCGTCCAGTCTGTT
GAAGGCCTGAATGTAACAAAAGGGCTTATTCTTCCTCAAGTAAGGGGGAACTCCTGCTTTGGGCTGGGACATAAGTTTTTCTGCTTT
CAGACGCAAACTGAAAAATGGCTCTTCTTGGGTCTTGAGCTTGCTGGCATATGGACTGAAAGAAACTATGCTATTGGATCTCCTGGA
TCTCCAGCTTGCTGACTGCAGATCTTGAGATATGTCAGCCTCTACAGTCACAAGAGCTAATTCATTCTAATAAACCAATCTTTCTGT
A


HUMAN mRNA SEQUENCE : hR27-007.1 (Seq ID No: 17)
AATTCACTAATGCATTCTGCTCTTTTTGAGAGCACAGCTTCTCAGATGTGCTCCTTGGAGCTGGTGTGCAGTGTCCTGACTGTAAGA
TCAAGTCCAAACCTGTTTTGGAATTGAGGAAACTTCTCTTTTGATCTCAGCCCTTGGTGGTCCAGGTCTTCATGCTGCTGTGGGTGA
TATTACTGGTCCTGGCTCCTGTCAGTGGACAGTTTGCAAGGACACCCAGGCCCATTATTTTCCTCCAGCCTCCATGGACCACAGTCT
TCCAAGGAGAGAGAGTGACCCTCACTTGCAAGGGATTTCGCTTCTACTCACCACAGAAAACAAAATGGTACCATCGGTACCTTGGGA
AAGAAATACTAAGAGAAACCCCAGACAATATCCTTGAGGTTCAGGAATCTGGAGAGTACAGATGCCAGGCCCAGGGCTCCCCTCTCA
GTAGCCCTGTGCACTTGGATTTTTCTTCAGCTTCGCTGACTCTGCAAGCTCCACTTTCTGTGTTTGAAGGAGACTCTGTGGTTCTGA
GGTGCCGGGCAAAGGCGGAAGTAACACTGAATAATACTATTTACAAGAATGATAATGTCCTGGCATTCCTTAATAAAAGAACTGACT
TCCATATTCCTCATGCATGTCTCAAGGACAATGGTGCATATCGCTGTACTGGATATAAGGAAAGTTGTTGCCCTGTTTCTTCCAATA
CAGTCAAAATCCAAGTCCAAGAGCCATTTACACGTCCAGTGCTGAGAGCCAGCTCCTTCCAGCCCATCAGCGGGAACCCAGTGACCC
TGACCTGTGAGACCCAGCTCTCTCTAGAGAGGTCAGATGTCCCGCTCCGGTTCCGCTTCTTCAGAGATGACCAGACCCTGGGATTAG
GCTGGAGTCTCTCCCCGAATTTCCAGATTACTGCCATGTGGAGTAAAGATTCAGGGTTCTACTGGTGTAAGGCAGCAACAATGGCCTC
ACAGCGTCATATCTGACAGCCCGAGATCCTGGATACAGGTGCAGATCCCTGCATCTCATCCTGTCCTCACTCTCAGCCCTGAAAAGG
CTCTGAATTTTGAGGGAACCAAGGTGACACTTCACTGTGAAACCCAGGAAGATTCTCTGCGCACTTTGTACAGGTTTTATCATGAGG
GTGTCCCCCTGAGGCACAAGTCAGTCCGCTGTGAAAGGGGAGCATCCATCAGCTTCTCACTGACTACAGAGAATTCAGGGAACTACT
ACTGCACAGCTGACAATGGCCTTGGCGCCAAGCCCAGTAAGGCTGTGAGCCTCTCAGTCACTGTTCCCGTGTCTCATCCTGTCCTCA
ACCTCAGCTCTCCTGAGGACCTGATTTTTGAGGGGAGCCAAGGTGACACTTCACTGTGAAGCCCAGAAGAGGTTCACTCCCCATCCTGT
ACCAGTTTCATCATGAGGATGCTGCCCTGGACGTAGGTCGGCCAACTCTGCAGGAGGAGTGGCCATCAGCTTCTCTCTGACTGCAG
AGCATTCAGGGAACTACTACTGCACAGCTGACAATGGCTTTGGCCCCCAGCGCAGTAAGGCGGTGAGCCTCTCCGTCACTGTCCCTG
TGTCTCATCCTGTCCTCACCCTCAGCTCTGCTGAGGCCCTGACTTTTGAAGGAGCCACTGTGACACTTCACTGTGAAGTCCAGAGAG
GTTCCCCACAAATCCTATACCAGTTTTATCATGAGGACATGCCCCTGTGGAGCAGCTCAACACCCTCTGTGGGAAGAGTGTCCTTCA
GCTTCTCTCTGACTGCAGAAGGACATTCAGGGAATTACTACTGCACAGCTGACAATGGCTTTGGTCCCCAGCGCAGTGAAGTGGTGAGCC
TTTTTGTCACTGTTCCAGTGTCTCGCCCATCCTCACCCTCAGGGTTCCCAGGGCCCAGGCTGTGGTGGGGGACCTGCTGGAGCTTC
ACTGTGAGGCCCCGAGAGGCTCTCCCCCAATCCTGTACTGGTTTTATCATGAGGATGTCACCCTGGGGAGCAGCTCAGCCCCCTCTG
GAGGAGAAGCTTCTTTCAACCTCTCTCTGACTGCAGAACATTCTGGAAACTACTCATGTGAGGCCAACAATGGCCTAGTGGCCCAGC
ACAGTGACACAATATCACTCAGTGTTATAGTTCCAGTATCTCGTCCCATCCTCACCTTCAGGGCTCCCAGGGCCCAGGCTGTGGTGG
GGGACCTGCTGGAGCTTCACTGTGAGCTTCACTGTGAAGACTCCTCCCCAATCCTGTACTGGTTTTATCATGAAGATGTCACCCTGGGTA
AGATCTCAGCCCCCTCTGGAGGAGGGGCCTCCTTCAACCTCTCTCTGACTACAGAACATTCTGGAATCTACTCCTGTGAGGCAGACA
ATGGTCTGGAGGCCCAGCGCAGTGAGATGGTGACACTGAAAGTTGCAGGTGAGTGGGCCCTGCCCACCAGCAGCACATCTGAGAACT
GACTGTGCCTGTTCTCCCTGCAGCTGAAAATGGAGCCACAGAGCTCCTCAGGGCTGTTTGCTTGTGTGGCATCCCAGCACACTTCCT
GCCTGCAGAACCTCCCTGTGAAAGTCTCGGATCCTTTGTGGTATGGTTCCAGGAATCTGATGTTTCCCAGCAGTCTTCTTGAAGATG
ATCAAAGCACCTCACTAAAAATGCAAATAAGACTTTTTTAGAACATAAAACTATATTCTGAACTGAAATTATTACATGAAAATGAAAC
CAAAGAATTCTGAGCATATGTTTCTGCCGTAGAAAGGATTAAGCTGTTTCTTGTCCGGATTCTTCTCTCATTGACTTCTAAGAAG
CCTCTACTCTTGAGTCTCTTTCATTACTGGGGATGTAAATGTTCCTTACATTTCCACATTAAAAATCCTATGTTAACA


HUMAN PROTEIN SEQUENCE : hP27-007.1 (Seq ID No: 18)
MLLWVILLVLAPVSGQFARTPRPIIFLQPPWTTVFQGERVTLTCKGFRFYSPQKTKWYHRYLGKEILRETPDNILEVQESGEYRCQA
QGSPLSSPVHLDFSSASLILQAPLSVFEGDSVVLRCRAKAEVTLNNTIYKNDNVLAFLNKRTDFHIPHACLKDNGAYRCTGYKESCC
```

EP 2 204 376 A2

```
PVSSNTVKIQVQEPFTRPVLRASSFQPISGNPVTLTCETQLSLERSDVPLRFRFFRDDQTLGLGWSLSPNFQITAMWSKDSGFYWCK
AATMPHSVISDSPRSWIQVQIPASHPVLTLSPEKALNFEGTKVTLHCETQEDSLRTLYRFYHEGVPLRHKSVRCERGASISFSLTTE
NSGNYYCTADNGLGAKPSKAVSLSVTVPVSHPVLNLSSPEDLIFEGAKVTLHCEAQRGSLPILYQFHHEDAALERRSANSAGGVAIS
FSLTAEHSGNYYCTADNGFGPQRSKAVSLSVTVPVSHPVLTLSSAEALTFEGATVTLHCEVQRGSPQILYQFYHEDMPLWSSSTPSV
GRVSFSFSLTEGHSGNYYCTADNGFGPQRSEVVSLFVTVPVSRPILTLRVPRAQAVVGDLLELHCEAPRGSPPILYWFYHEDVTLGS
SSAPSGGEASFNLSLTAEHSGNYSCEANNGLVAQHSDTISLSVIVPVSRPILTFRAPRAQAVVGDLLELHCEALRGSSPILYWFYHE
DVTLGKISAPSGGGASFNLSLTTEHSGIYSCEADNGLEAQRSEMVTLKVAGEWALPTSSTSEN*


HUMAN mRNA SEQUENCE : hR27-007.2 (Seq ID No: 19)
AATTCACTAATGCATTCTGCTCTTTTTGAGAGCACAGCTTCTCAGATGTGCTCCTTGGAGCTGGTGTGCAGTGTCCTGACTGTAAGA
TCAAGTCCAAACCTGTTTTGGAATTGAGGAAACTTCTCTTTTGATCTCAGCCCTTGGTGGTCCAGGTCTTCATGCTGCTGTGGGTGA
TATTACTGGTCCTGGCTCCTGTCAGTGGACAGTTTGCAAGGACACCCAGGCCCATTATTTTCCTCCAGCCTCCATGGACCACAGTCT
TCCAAGGAGAGAGAGTGACCCTCACTTGCAAGGGATTTCGCTTCTACTCACCACAGAAAACAAAATGGTACCATCGGTACCTTGGGA
AAGAAATACTAAGAGAAACCCCAGACAATATCCTTGAGGTTCAGGAATCTGGAGAGTACAGATGCCAGGCCCAGGGCTCCCCTCTCA
GTAGCCCTGTGCACTTGGATTTTTCTTCAGCTTCGCTGATCCTGCAAGCTCCACTTTCTGTGTTTGAAGGAGACTCTGTGGTTCTGA
GGTGCCGGGCAAAGGCGGAAGTAACACTGAATAATACTATTTACAAGAATGATAATGTCCTGGCATTCCTTAATAAAAGAACTGACT
TCCATATTCCTCATGCATGTCTCAAGGACAATGGTGCATATCGCTGTACTGGATATAAGGAAAGTTGTTGCCCTGTTTCTTCCAATA
CAGTCAAAATCCAAGTCCAAGAGCCATTTACACGTCCAGTGCTGAGAGCCAGCTCCTTCCAGCCCATCAGCGGGAACCCAGTGACCC
TGACCTGTGAGACCCAGCTCTCTCTAGAGAGGTCAGATGTCCCGCTCCGGTTCCGCTTCTTCAGAGATGACCAGACCCTGGGATTAG
GCTGGAGTCTCTCCCCGAATTTCCAGATTACTGCCATGTGGAGTAAAGATTCAGGGTTCTACTGGTGTAAGGCAGCAACAATGCCTC
ACAGCGTCATATCTGACAGCCCGAGATCCTGGATACAGGTGCAGATCCCTGCATCTCATCCTGTCCTCACTCTCAGCCCTGAAAAGG
CTCTGAATTTTGAGGGAACCAAGGTGACACTTCACTGTGAAACCCAGGAAGATTCTCTGCGCACTTTGTACAGGTTTTATCATGAGG
GTGTCCCCCTGAGGCACAAGTCAGTCCGCTGTGAAAGGGGAGCATCCATCAGCTTCTCACTGACTACAGAGAATTCAGGGAACTACT
ACTGCACAGCTGACAATGGCCTTGGCGCCAAGCCCAGTAAGGCTGTGAGCCTCTCAGTCACTGTTCCCGTGTCTCATCCTGTCCTCA
ACCTCAGCTCTCCTGAGGACCTGATTTTTGAGGGAGCCAAGGTGACACTTCACTGTGAAGCCCAGAGAGGTTCACTCCCCATCCTGT
ACCAGTTTCATCATGAGGATGCTGCCCTGGAGCGTAGGTCGGCCAACTCTGCAGGAGGAGGTGGCCATCAGCTTCTCTCTGACTGCAG
AGCATTCAGGGAACTACTACTGCACAGCTGACAATGGCTTTGGCCCCCAGCGCAGTAAGGCGGTGAGCCTCTCCGTCACTGTCCCTG
TGTCTCATCCTGTCCTCACCCTCAGCTCTGCTGAGGCCCTGACTTTTGAAGGAGCCACTGTGACACTTCACTGTGAAGTCCAGAGAG
GTTCCCCACAAATCCTATACCAGTTTTATCATGAGGACATGCCCCTGTGGAGCAGCTCAACACCCTCTGTGGGAAGAGTGTCCTTCA
GCTTCTCTCTGACTGAAGGACATTCAGGGAATTACTACTGCACAGCTGACAATGGCTTTGGTCCCCAGCGCAGTGAAGTGGTGAGCC
TTTTTGTCACTGGTAAGTGCTGGGTTCTTGCCAGTCACCCACCCCTGGCTGAGTTCTCTCTCACCCATTCCTTTAAAAAATCTGTTTG
CACTGTCCAGTTTCCTCCCC


HUMAN PROTEIN SEQUENCE : hP27-007.2 (Seq ID No: 20)
MLLWVILLVLAPVSGQFARTPRPIIFLQPPWTTVFQGERVTLTCKGFRFYSPQKTKWYHRYLGKEILRETPDNILEVQESGEYRCQA
QGSPLSSSPVHLDFSSASLILQAPLSVFEGDSVVLRCRAKAEVTLNNTIYKNDNVLAFLNKRTDFHIPHACLKDNGAYRCTGYKESCC
PVSSNTVKIQVQEPFTRPVLRASSFQPISGNPVTLTCETQLSLERSDVPLRFRFFRDDQTLGLGWSLSPNFQITAMWSKDSGFYWCK
AATMPHSVISDSPRSWIQVQIPASHPVLTLSPEKALNFEGTKVTLHCETQEDSLRTLYRFYHEGVPLRHKSVRCERGASISFSLTTE
NSGNYYCTADNGLGAKPSKAVSLSVTVPVSHPVLNLSSPEDLIFEGAKVTLHCEAQRGSLPILYQFHHEDAALERRSANSAGGVAIS
FSLTAEHSGNYYCTADNGFGPQRSKAVSLSVTVPVSHPVLTLSSAEALTFEGATVTLHCEVQRGSPQILYQFYHEDMPLWSSSTPSV
GRVSFSFSLTEGHSGNYYCTADNGFGPQRSEVVSLFVTGKCWVLASHPPLAEFSLTHSFKNLFALSSFLP


HUMAN mRNA SEQUENCE : hR27-007.3 (Seq ID No: 21)
AATTCACTAATGCATTCTGCTCTTTTTGAGAGCACAGCTTCTCAGATGTGCTCCTTGGAGCTGGTGTGCAGTGTCCTGACTGTAAGA
TCAAGTCCAAACCTGTTTTGGAATTGAGGAAACTTCTCTTTTGATCTCAGCCCTTGGTGGTCCAGGTCTTCATGCTGCTGTGGGTGA
TATTACTGGTCCTGGCTCCTGTCAGTGGACAGTTTGCAAGGACACCCAGGCCCATTATTTTCCTCCAGCCTCCATGGACCACAGTCT
TCCAAGGAGAGAGAGTGACCCTCACTTGCAAGGGATTTCGCTTCTACTCACCACAGAAAACAAAATGGTACCATCGGTACCTTGGGA
AAGAAATACTAAGAGAAACCCCAGACAATATCCTTGAGGTTCAGGAATCTGGAGAGTACAGATGCCAGGCCCAGGGCTCCCCTCTCA
GTAGCCCTGTGCACTTGGATTTTTCTTCAGCTTCGCTGATCCTGCAAGCTCCACTTTCTGTGTTTGAAGGAGACTCTGTGGTTCTGA
GGTGCCGGGCAAAGGCGGAAGTAACACTGAATAATACTATTTACAAGAATGATAATGTCCTGGCATTCCTTAATAAAAGAACTGACT
TCCATATTCCTCATGCATGTCTCAAGGACAATGGTGCATATCGCTGTACTGGATATAAGGAAAGTTGTTGCCCTGTTTCTTCCAATA
CAGTCAAAATCCAAGTCCAAGAGCCATTTACACGTCCAGTGCTGAGAGCCAGCTCCTTCCAGCCCATCAGCGGGAACCCAGTGACCC
TGACCTGTGAGACCCAGCTCTCTCTAGAGAGGTCAGATGTCCCGCTCCGGTTCCGCTTCTTCAGAGATGACCAGACCCTGGGATTAG
GCTGGAGTCTCTCCCCGAATTTCCAGATTACTGCCATGTGGAGTAAAGATTCAGGGTTCTACTGGTGTAAGGCAGCAACAATGCCTC
ACAGCGTCATATCTGACAGCCCGAGATCCTGGATACAGGTGCAGATCCCTGCATCTCATCCTGTCCTCACTCTCAGCCCTGAAAAGG
CTCTGAATTTTGAGGGAACCAAGGTGACACTTCACTGTGAAACCCAGGAAGATTCTCTGCGCACTTTGTACAGGTTTTATCATGAGG
GTGTCCCCCTGAGGCACAAGTCAGTCCGCTGTGAAAGGGGAGCATCCATCAGCTTCTCACTGACTACAGAGAATTCAGGGAACTACT
ACTGCACAGCTGACAATGGCCTTGGCGCCAAGCCCAGTAAGGCTGTGAGCCTCTCAGTCACTGTTCCCGTGTCTCATCCTGTCCTCA
ACCTCAGCTCTCCTGAGGACCTGATTTTTGAGGGAGCCAAGGTGACACTTCACTGTGAAGCCCAGAGAGGTTCACTCCCCATCCTGT
ACCAGTTTCATCATGAGGATGCTGCCCTGGAGCGTAGGTCGGCCAACTCTGCAGGAGGAGGTGGCCATCAGCTTCTCTCTGACTGCAG
AGCATTCAGGGAACTACTACTGCACAGCTGACAATGGCTTTGGCCCCCAGCGCAGTAAGGCGGTGAGCCTCTCCGTCACTGTCCCTG
TGTCTCATCCTGTCCTCACCCTCAGCTCTGCTGAGGCCCTGACTTTTGAAGGAGCCACTGTGACACTTCACTGTGAAGTCCAGAGAG
GTTCCCCACAAATCCTATACCAGTTTTATCATGAGGACATGCCCCTGTGGAGCAGCTCAACACCCTCTGTGGGAAGAGTGTCCTTCA
GCTTCTCTCTGACTGAAGGACATTCAGGGAATTACTACTGCACAGCTGACAATGGCTTTGGTCCCCAGCGCAGTGAAGTGGTGAGCC
TTTTTGTCACTGTTCCAGTGTCTCGCCCCATCCTCACCCTCAGGGTTCCCAGGGCCCAGGCTGTGTGGTGGGGGGACCTGCTGGACCTTC
ACTGTGAGGCCCCGAGAGGCTCTCCCCAATCCTGTACTGGTTTTATCATGAGGATGTCACCCTGGGGAGCAGCTCAGCCCCTCTG
GAGGAGAAGCTTCTTTCAACCTCTCTCTGACTGCAGAACATTCTGGAAACTACTCATGTGAGGCCAACAATGGCCTAGTGGCCCAGC
ACAGTGACACAATATCACTCAGTGTTATAGTTCCAGTATCTCGTCCCATCCTCACCTTCAGGGCTCCCAGGGCCCAGGCTGTGGTGG
GGGACCTGCTGGAGCTTCACTGTGAGGCCCTGAGAGGCTCCTCCCCAATCCTGTACTGGTTTTATCATGAAGATGTCACCCTGGGTA
AGATCTCAGCCCCCTCTGGAGGAGGGGCCTCCTTCAACCTCTCTCTGACTACAGAACATTCTGGAATCTACTCCTGTGAGGCAGACA
ATGGTCTGGAGGCCCAGCGCAGTGAGAGTGGTGACACTGAAAGTTGCAGTTCCGGTGTCTCGCCCGGTCCTCACCCTCAGGGCTCCCG
GGACCCATGCTGCGGTGGGGGACCTGCTGGAGCTTCACTGTGAGGCCCTGAGAGGCTCTCCCCTGATCCTGTACCGGTTTTTTCATG
AGGATGTCACCCTAGGAAATAGGTCGTCCCCCTCTGGAGGAGCGTCCTTAAACCTCTCTCTGACTGCAGAGCACTCTGGAAACTACT
CCTGTGAGGCCGACAATGGCCTCGGGGCCCAGCGCAGTGAGACAGTGACACTTTATATCACAGGGCTGACCGCGAACAGAAGTGGCC
CTTTTGCCACAGGAGTCGCCGGGGGCCTGCTCAGCATAGCAGGCCTTGCTGCGGGGGCACTGCTGCTCTACTGCTGGCTCTCGAGAA
AAGCAGGGAGAAAGCCTGCCTCTGACCCCGCCAGGAGCCCTTCAGACTCGGACTCCCAAGAGCCCACCTATCACAATGTACCAGCCT
```

73

```
GGGAAGAGCTGCAACCAGTGTACACTAATGCAAATCCTAGAGGAGAAAATGTGGTTTACTCAGAAGTACGGATCATCCAAGAGAAAA
AGAAACATGCAGTGGCCTCTGACCCCAGGCATCTCAGGAACAAGGGTTCCCCTATCATCTACTCTGAAGTTAAGGTGGCGTCAACCC
CGGTTTCCGGATCCCTGTTCTTGGCTTCCTCAGCTCCTCACAGATGAGTCCACACGTCTCTCCAACTGCTGTTTCAGCCTCTGCACC
CCAAAGTTCCCCTTGGGGGAGAAGCAGCATTGAAGTGGGAAGATTTAGGCTGCCCCAGACCATATCTACTGGCCTTTGTTTCACATG
TCCTCATTCTCAGTCTGACCAGAATGCAGGGCCCTGCTGGACTGTCACCTGTTTCCCAGTTAAAGCCCTGACTGGCAGGTTTTTTAA
TCCAGTGGCAAGGTGCTCCCACTCCAGGGCCCAGCACATCTCCTGGATTCCTTAGTGGGCTTCAGCTGTGGTTGCTGTTCTGAGTAC
TGCTCTCATCACACCCCCACAGAGGGGGTCTTACCCACACAAAGGGAGAGTGGGCCTTCAGGAGATGCCGGGCTGGCCTAACAGCTCA
GGTGCTCCTAAACTCCGACACAGAGTTCCTGCTTTGGGTGGATGCATTTCTCAATTGTCATCAGCCTGGTGGGGCTACTGCAGTGTG
CTGCCAAATGGGACAGCACACAGCCTGTGCACATGGGACATGTGATGGGTCTCCCCACGGGGGCTGCATTTCACACTCCTCCACCTG
TCTCAAACTCTAAGGTCGGCACTTGACACCAAGGTAACTTCTCTCCTGCTCATGTGTCAGTGTCTACCTGCCCAAGTAAGTGGCTTT
CATACACCAAGTCCCAAGTTCTTCCCATCCTAACAGAAGTAACCCAGCAAGTCAAGGCCAGGAGGACCAGGGGTGCAGACAGAACAC
ATACTGGAACACAGGAGGTGCTCAATTACTATTTGACTGACTGACTGAATGAATGAATGAATGAGGAAGAAAACTGTGGGTAATCAA
ACTGGCATAAAATCCAGTGCACTCCCTAGGAAATCCGGGAGGTATTCTGGCTTCCCTAAGAAACAATGGAAGAGAAGGAGCTTGGAT
GAGGAAACTGTTCAGCAAGAGGAAGGGCTTCTCACACTTTCATGTGCTTGTGGATCACCTGAGGATCCTGTGAAAATACAGATACTG
ATTCAGTGGGTCTGCGTAGAGCCTGAGACTGCCATTCTAACATGTTCCCAGGGGATGCTGATGCTGCTGCCCTGGGACTGCACTGC
ATGCATGTGAAGCCCTATAGGTCTCAGCAGAGGCCCATGGAGAGGGAATGTGTGGCTCTGGCTGCCCAGGGCCCAACTCGGTTCACA
CGGATCGTGCTGCTCCCTGGCCAGCCTTTGGCCACAGCACCACCAGCTGCTGTTGCTGAGAGAGCTTCTTCTCTGTGACATGTTGGC
TTTCATCAGCCACCCTGGGAAGCGGAAAGTAGCTGCCACTATCTTTGTTTCCCCACCTCAGGCCTCACACTTTCCCATGAAAAGGGT
GAATGTATATAACCTGAGCCCTCTCCATTCAGAGTTGTTCTCCCATCTCTGAGCAATGGGATGTTCTGTTCCGCTTTTATGATATCC
ATCACATCTTATCTTGATCTTTGCTCCCAGTGGATTGTACAGTGATGACTTTTAAGCCCCACGGCCCTGAAATAAAATCCTTCCAAG
GGCATTGGAAGCTCACTCCACCTGAACCATGGCTTTTCATGCTTCCAAGTGTCAGGGCCTTGCCCAGATAGACAGGGCTGGCTCTGC
TGCCCCAACCTTTCAAGGAGGAAACCAGACACCTGAGACAGGAGCCTGTATGCAGCCCAGTGCAGCCTTGCAGAGGACAAGGCTGGA
GGCATTTGTCATCACTACAGATATGCAACTAAAATAGACGTGGAGCAAATGCATTCCCACCGAGGCCGCTTTTTTAGGCCTA
GTTGAAAGTCAAGAAGGACAGCAGCAAGCATAGGCTCAGGATTAAAGAAAAAAATCTGCTCACAGTCTGTTCTGGAGGTCACATCAC
CAACAAAGCTCACGCCCTATGCAGTTCTGAGAAGGTGGAGGCACCAGGCTCAAAAGAGGAAATTTAGAATTTCTCATTGGGAGAGTA
AGGTACCCCCATCCCAGAATGATAACTGCACAGTGGCAGAACAAACTCCACCCTAATGTGGGTGGACCCCGTCCAGTCTGTTGAAGG
CCTGAATGTAACAAAAGGGCTTATTCTTCCTCAAGTAAGGGGGAACTCCTGCTTTGGGCTGGGACATAAGTTTTTCTGCTTTCAGAC
GCAAACTGAAAAATGGCTCTTCTTGGGTCTTGAGCTTGCTGGCATATGGACTGAAAGAAACTATGCTATTGGATCTCCTGGATCTCC
AGCTTGCTGACTGCAGATCTTGAGATATGTCAGCCTCTACAGTCACAAGAGCTAATTCATTCTAATAAACCAATCTTTCTGTA
```

HUMAN PROTEIN SEQUENCE : hP27-007.3 (Seq ID No: 22)

```
                MLLWVILLVLAPVSGQFARTPRPIIFLQPPWTTVFQGERVTLTCKGFRFYSPQKTKWYHRYLGKEILRETPD

NILEVQESGEYRCQAQGSPLSSPVHLDFSSASLILQAPLSVFEGDSVVLRCRAKAEVTLNNTIYKNDNVLAFLNKRTDFHIPHACLK

DNGAYRCTGYKESCCPVSSNTVKIQVQEPFTRPVLRASSFQPISGNPVTLTCETQLSLERSDVPLRFRFFRDDQTLGLGWSLSPNFQ

ITAMWSKDSGFYWCKAATMPHSVISDSPRSWIQVQIPASHPVLTLSPEKALNFEGTKVTLHCETQEDSLRTLYRFYHEGVPLRHKSV

RCERGASISFSLTTENSGNYYCTADNGLGAKPSKAVSLSVTVPVSHPVLNLSSPEDLIFEGAKVTLHCEAQRGSLPILYQFHHEDAA

LERRSANSAGGVAISFSLTAEHSGNYYCTADNGFGPQRSKAVSLSVTVPVSHPVLTLSSAEALTFEGATVTLHCEVQRGSPQILYQF

YHEDMPLWSSSTPSVGRVSFSFSLTEGHSGNYYCTADNGFGPQRSEVVSLFVTVPVSRPILTLRVPRAQAVVGDLLELHCEAPRGSP

PILYWFYHEDVTLGSSSAPSGGEASFNLSLTAEHSGNYSCEANNGLVAQHSDTISLSVIVPVSRPILTFRAPRAQAVVGDLLELHCE

ALRGSSPILYWFYHEDVTLGKISAPSGGGASFNLSLTTEHSGIYSCEADNGLEAQRSEMVTLKVAVPVSRPVLTLRAPGTHAAVGDL

LELHCEALRGSPLILYRFFHEDVTLGNRSSPSGGASLNLSLTAEHSGNYSCEADNGLGAQRSETVTLYITGLTANRSGPFATGVAGG

LLSIAGLAAGALLLYCWLSRKAGRKPASDPARSPSDSDSQEPTYHNVPAWEELQPVYTNANPRGENVVYSEVRIIQEKKKHAVASDP

RHLRNKGSPIIYSEVKVASTPVSGSLFLASSAPHR*
```

## Table 3

```
MOUSE GENOMIC SEQUENCE : mD27-009 (Seq ID No: 23)
GCTTTGGGGTGGAGGGTCGTGCTGCAGGGAACTCAGCCAGGCCCCAAGATGGACACTTCTGGGCACTTCCATGACTCGGGGGTGGGG
GACCTGGATGAAGACCCCAAGTGTCCCTGTCCATCTTCTGGGGACGAGCAACAGCAGCAGCAGCAACCGCCACCACCGCCAGCGCCA
CCAGCAGTCCCCCAGCAGCCTCCGGACCCTTGCTGCAGCCTCAGCCTCCGCAGCCTCAGCAGCAACAGTCGCAGCAGCAGCAGCAG
CAGCAGTCGCAGCAGCAGCAGCAGCAGGCTCCACTGCACCCCCTGCCTCAGCTTGCCCAACTCCAGAGCCAGCTTGTCCATCCTGGT
CTGTTGCACTCTTCTCCCACGGCGTTCAGGGCCCCCACTTCAGCCAACTCTACCGCCATCCTCCACCCTTCCTCCAGGCAAGGCAGC
CAGCTAAATCTCAATGACCACTTGCTTGGCCACTCTCCAAGTTCCACAGCCACAAGTGGGCCCGGTGGAGGCAGCCGGCACCGGCAG
GCCAGCCCCCTGGTGCACCGGCGGGACAGCAATCCCTTCACGGAGATAGCTATGAGCTCCTGCAAATACAGCGGTGGGGTCATGAAG
CCCCTCAGCCGCCTCAGCGCCTCTCGGAGAAACCTTATCGAGGCTGAGCCTGAGGGCCAACCCCTCCAGCTCTTCAGTCCCAGCAAC
CCCCCAGAGATTATCATCTCCTCCAGGGAGGATAACCATGCCCACCAGACTCTGCTCCATCACCCCAACGCTACCCACAACCACCAG
CATGCCGGCACCACGGCCGGCAGCACCACCTTCCCCAAAGCCAACAAGCGGAAAAACCAAAACATTGGCTATAAGCTGGGACACAGG
AGGGCCCTGTTTGAAAAGAGAAAGCGACTGAGTGACTATGCTCTGATTTTTGGGATGTTTGGAATTGTTGTTATGGTGATAGAGACC
GAACTGTCTTGGGGTTTGTACTCAAAGGTAGGGACTGTGGTTTCTCTTTATACATTCAACAAAAGGAGTATGTAGACGAGAGAGAGA
GAGAGAGAGAGAGAGAGAGAGAGAGAGAGATTGATTTTGAGAGATTTCCTAACTTCCCATGTTTTTCTTCCATGGTCCTGTGA
GAATTCCTTTCTTGCTTCCCTTTGTGGGGACATCCCCACACACTGTGTCTAATGTGCAGACTCTGTGTTTGGGAGGATTAAAGAATC
CCTTCTGAGAAATCGCTCTTTTCCTCCGGGCTCACGCGTACTAAAGCATAACCCAGCAGCTTAACGCACCAATTAATTACACTCTGC
CTTGATGTGTTTGCCAGTTCCTGCTACTTCCCTCCTCGCCTCCCCTCCCTGCTCCACTCCATTCCCCCCCCCCCATAAAAAAGAAAT
AAATGTCTGGGTGGGGGTGGGGATCCTGTACCGTTCCCCAGCTCCCCCCCCCCCCCTTCCCAAGTTTCTATTTCCCTTCTCTTCCCA
GAGAGACTCCAGGGTAAAATGGAGATGTTCCCGAGTCTCTCCCAAGGGCACCGGGTTAAAAATATAGGTGGAGGAAATCAGCAGTCT
CTTCCCTCAGGAAAGAGGACTCAGAATAAACCTGCCGCTGCCTTGTAAACATGCCCTGATAAAAATGGCTACAGGTGTTACCCAGGC
```

```
AGAGCATGGGCACCGCCTTGCCATCACTCGGGGAACGGGGAGCGCCTCTCCTTAGGATGGGATGACAGGGCTTCCCCAGCGGGTCAC
CGCGGAGTCGGCACCCCAGCTTAGCCCTGGTTCTTCCCTCCCACCCTAACTCCTTCTCCTTGGGATAAATAAAGATGAGTGTACGTG
TGTGTGCACGCTGGATGGAGAACGGCAGGCACAAGGCACTGTCTTGGGGGTGGGGTGGGGTGGGGGACGCTCCATTGCTCTATCCTT
TCAGTTGGAGGAGAGAGCTTCCTTCCCAGAGGGGAAGGAGGTCGAGGAAGGTGGGTTTTTGTCAATGCTGCATTTGCCAGGAAGCTG
CATGCCAGAAGGCTTGGAGGTGCAGGGAATTGTTGGGCAAACGGTTTCCCCTACTGTACCCACTTGGTCCCAGTTTGCTGGTGTTTGC
ATGTGTATATGTGTACTCGTGTGAGTGCCTGCTTGTTCTGTGTGTATTTGTGTGATTTGTTTGTCATTTTGGACAAGGGGTGGCTGGGCT
GGCTGGCGGGTAGCTGGGAGGGGGCTCAGCTGTTGGAGGTTTCAAATAGAGCCTGTTTCAGGCAGCGCACAGCTGCTGGAGACTGTCA
CATTCCTTGTAGAGATCTAGTCACAAAAACCTGGGAGGTGCTGATCAGATTTCCCCCATTCCCAGAGTCTCCCTTTAACCTGATGTG
GGCAGTGCTCCTTGTCAGGCAACCTGCATGGACAGAGCCCAGACTTCCCAGCAGAGAGTCCTCCAGGGACCTGCTGCTTTGCAGGGA
CTGCCCAAGCCAGAGCACTTGGAAGCTGCCTCAGTCTAAATAGCTCCTGGATGAACAGACCTCTCCATGAGCAGAGTGGGGTTTCCC
CTGGAAGCCCAGCTCACTTAGTCATTCCCTGTAATGAGAGCCTGGGAGGCTGCAGGTCCCTCTGTTCCCTGCCTTCTTAGAGCTGCC
TCTCTTGCTGTGTGCTCTGCTTAGGAGATGGGCTGGCAACCCCCCCCTGGGAATTAGGAACACTTGTTAGGGGGAATCCGTGGAAGC
CCACATCCCGGAGGTGTGGCATGGTGCTAGCATGTTCTTTTCAGTGGCCCCCATGGCGGCTGTTAAGGAAATGGCTTTTCTAGGAGT
CAGTGGCTGTGTCCGGCTCTGAGTGTGGGATCCATGATCATTTGTCTGAATGTGTTTTGGGGGTAGGGTGTCACCGGGCAGGGACGA
CTATCTTCTCTGATGGAGAGAGGTTTTCCCGGGCCGGGAGGCTGTAAGTGTTGACTGGGTGATATCACCCAAGGTGTGTGCTTCCTC
CACTCCTAAGTCGTTGATTCTCTGGTGGCGATGGTACTGAGGGACAGTGACCAGGAAGCTCCTGCGTGCCTCAGTCAGTGAGGTGAC
CACTTCCTGTGTACCTCGGGGATGGGATTGAAACAACAAAATGTTTATTTAGTGAGGCAAATGTCCCTATCTACCTCGGAGGCAAAG
AGGGTCATATCAAAGAATAGGACCAAATCAGGAGTTACAGCTCTGTCACTGCTGGCTGTGTGATTCTTGGAAGTCAGGATACCCTGC
AAGGCTGCTTCCAGCTTTCTGGTGGGGAGACTGAGTCAGATGAGGTCAAGGCCCACCAGAGCACCTGCTCATGTAAGAGGGGTCGGC
TGTGGCTGTCTGTGATGACTGAGGAGAGAGACACCTTCTTTCTCTGTGATTAGATGGAAAGTAAGACGCAAGGACAGAGGAGAAAGT
TCTACAAGGAATCTGGGTCTGGTTGGAGACTGTCTTTACACATGAGGCAGCTGTTGGGGAGAGGGTGTCAGCTCCCCAGCTTTGGG
ATACAGGGGGATGGGCTCTGTGACAGAGAGCCCAGAGACTCAGGGTGGATGAGGGACTTTCCTTTGCTAGGATTCTTGTGTGCC
TGCCTGTCTTTGCTGATGATTCTGGAAGCCCGAGGATAGACCAGGTGACCAGTCATTTTGGGTACCTACCATGGCCATTTCTATTTA
AAGGCCCTGGTGAGGGACAGGTTCTTTTTCCTCTGAGTGACTGATGGCCTGCAGCCCTGGGCCTTACCTAACTCTGAACTTCTCTGC
TTTATCTCGTGAGGCCGTACCTGCCCCGTCCACCTACTGTACCTGCTGCTGGGAGAGTCATTCATCGCATGATGAATGCAGGTCCAG
GGGTCTGGGAGAAAAGCAATGGGGCAGATACCCTGAGTGTGCTCAAAGGTGATGCAAAAGGGAGCCAGCGTGAGGAGGTTGAGAAAT
GGGGACAAAGTGCTTCCTGGCTCCCACCCTCACAGGCTGTAGGCGATGACACTGGCGGGAAGGAAGTTCCACTGAGAAAGGAGATGG
AAGCCTGACTTGGGGAGGGGGGCGTTGTGGACCCCTAAAGGTGCAAGGCTGTCTCTGAGCTGAATGGAGGCTCTGGTTTATCCATCC
CTCCCTCCCTTCCTTCTCTTGCTCCTCCCGTTCTTCCCTCCCCTTCTCATCCTCCCTCACCTTCCTTCCTTTAGTAGGTGGGCTTCC
TCTTTGGAAACTTAGACAACAGGAGAGGGCTGATTCTCAAGATCCTGTTTGGAAGTCTTGATGATCCCGCCTCTGCCTATGGCTCCC
TCTAACTGTTAGAGTCTAACAGGGTTAGGGGATGGGGATTTAGGTTGTTTTTTTTTTTTTTTTTCTGACTGTGTTCTTTAGTTTTTTT
CTGGAGCTGGTTTGGCCCATGTCACCCTTTTACCTTAAGCTGTCAGTAATTCTCTACTAGATCCAAAGAGTTAGGGGTAACCTGGCC
TGCTTTCTCTTCCTTAACACCCTGACAGGACGTCAGGCCTCAGCCTGTGGCAGCTTCCTCAGTCTGGGCAGCAGGGTAGCTGTCTGT
TTCCACAGATTTTGTACAGCCTCCGTAGCTCTCCACTGCTGGCCTAGAGATCCATTTCAGCCTTACCTCTGCTCCCTTGCCCCTCCT
GCCTGCCCAATCAGACTTCCTGTCCTGTTCCTTTCTGATACCCCAGCTTGACCTGTTGCTAGGATGAAGCAGTCACCGATACAGAAG
TCCCTATCTGCTTCCTTGGGTACATTCTCCTCACTTCCTGTTACTGTTCCTTCTTCAGACCACCAACTGGGCCGCCACCCTGAGCTA
GGTCTACTGAGCAGCTAAAGGAGGTCCCTACTGCATACTCTCCAGAGAATGGTGGCTTCTAGGCCTGGGCCAATTTGTTGCCTATCT
CTTTGCTGGCCTCACATATATCATAAAGTCCCCCCAGCCTTCAGCTGGAATGATGGATTGTATTCTAGGCTTTTATTTTTGGTCTTTT
GTAGGGTGTGTGTGGGGAGGTGGTAGCCACAGCTTCACACCGTAGGTAAACATTCTACCGAGCTTGTCTCCAACATCCAAGATGCAT
CTCTGTCCCGATCCTCTGCCTTCCTGGGATGGAGGCCCTAGTTGCACAAGGACAGAGTGGTAGCCAGGGTACATAGGATGAAGGACC
CTGCTTGTCTCTTTGGGGCCAAGTGCCTTCTGAGGTTAGGTTACTGGTTGCTGGAACATGTATTTCTGGAGGGTTTTGGGGGGGCACC
AGTAACTATGATTTCAGGCATGCTGGGGAGGTAGACATGGACCCTGGTGTGCACCTCCTGAAGGATGTCCTCTGAGAATAAG
AAGGCAGAAGACTCAGTAAAGCACTTTGGCTGCAGGACCCTGAGAGCCAGCAGCCAAGGGCCCCAGGCCTGCATTTTAATTGTCACA
GGGTATACTGGCCATGCCTGCCCTGGGGAAGACAAGCAGATCTGATAGTATGAGACAGCCAACCCTGCACCCATGGTGACCCTTTAT
TTATATTCCTTGTGGACAGTGAACTAATGAGTCAGATCTTGGTAACCTATAGGATTACCAGTAACTTGTCACCTACTTTCCTTCCCA
TGTCACCCACTGTTTATAACTGTCCTGGGGACAGGCTCCACAGGGCTTCCAGACTTCCCATCCCCACAGGCAGCACTGGGCATTGGG
ACAATGGCTGGACATTTGTCTTCTGAATAAGCTAATTAGAAAGCTACTCCATGGCCCTTTGATTCCCTTATTTAGCTTCCAGGAAGGA
GGATGTATGAGGCCATCCTCAAAAGATTCCCTTAAAAAAATTAGGTAGAGAAAAGCAGAAGAAAATTGGAGTAACCTGTGTTGTGAC
TCAGGCTCCAAGGCAAATGTAAAATCAGAACAGAGAGCTCTGGTTGCATGCCAGTTACTTATGAAGCACACTGGGGGGAAGTGTGTG
TGTGTGTTGTGAGGGGCGGCTGTACTGTACTGTACTGTACACAATATCCCCTTTCTTTGCGCCATGACAGGACCACCAGAGCATTCG
CTGGGTAGTTGTTTTTCTCTCAAAACCCCAAAGGGAGAGAGAGAGAGAGAGAGAGAGAGGGAGAGAGGGAGAGAGAGAGAGAGAG
AGGGAGGGAGGAGAGAGAGAGAAGAAAGCCATCAGTTCCTCTTTAAATAATGAGGGTTGGTAATTCTAAGACTGCTCACTCTCAGG
AGCCGTGGGGAGGAAAACTCGAGTGCCTTTTCTTGTGTGGAAAGTCCGGAGGAGGCTAATGGGTTTTCAAGGCCCCCTCCCCCTTGTC
ATTCCACCCCCAGGTGCTCGGGAGGCAGGGGAGAGGCTGGGGGCAGGGACCACACTGTTTCTTTCTGCCCCACACTGCTCCTGCGTG
AATTGCTGCTGAGTGACAGCTGAGGGAAGGCGAGGACCTTTTTCTTAAGAACTGGATGACTTTACACCCAAGAGTCCCAGAAGTAGG
TGGTGCTGGAGGCATCCTATGCAGATGAAATCACTCACTAAGCTAACACTGCCAGTGGGCGTGGTACTGAGATAAGAATCCTGCCCT
TTTGACTCTAAGTTTCTGTGCCTGCTGTGGGAGGCCTTGCTTACATTCCTTGTGTGTGTGTGTGTGTGTGTGTGTGTGTGTGTGTGT
GTGTGTGTGTATTTGTGTGTGCCCGCATGCGCGTGCGCGCGCGGCGCATATATGTTTCCAGTTTGGAATTTCAGTGCAGAACCTTGCA
CCTATTAGGTAAACAGTCTGCAGAGGAATCTCCCCAGCCCAGATTGCTTATACTCTGTTTTGGTTTTGTCCTTTTAGATTGCTTGCA
TTTTTCTCTGCATGTGCTGTGTTATATGTACATGTGCATATAACATGTGTGCATGTGGCTGGCCTGCGAGCTCAAGGGCTCTCGTCTG
CTCTCACCGTATCCCATCTACCCCTGTATCCTAGTTTTCATATGGATACTAGAGATTGAACTCAGACCCTTGTGCTTGCATGGAAGG
TGTTTTGCTGACGGAACCATCCTCCTGGTTCAGCACCCCTCCAGCCTCTGATATATTCTTGGAAGAAACAGATCAAAATCTATGGGC
CAGAGACCAGAGATACAAAATGTCGGGCTAGAAGAAGCTTGGCCAAGACATAGGCAGAACACGATTGGGATGGGAGCTGTTGGTGCA
GGGACCCAGACCAAAGGTTCTGTGTGCAAGCAGCTTCCTGGGGGCAGGCAGTCTGTTCATTGAGGTGGAGGGAAGGCCCATGGTGG
GTCCCTGAGATGTTTCCTCTATGGGGGGGGGGATGTGGCCATCCACTGTCAGGGTGACTCTGAATAAATTGTGAAGAAGTAGTTGTGG
GGATTACAGGGAAAGTGAGCTTTGTATAGGTTTGCTGTAGGTACCTACCACAGCACCATTGCCCCGCCCCCAAGCCACTGTGCCTCC
CCCTCCCCTCCTGAACTAGCTGGCTTACCTTGTTCCCTCCTGCCTGGATCTTCTTTTAATTTTCTCCTTCCTTTCTAGTGCTAGCTG
CCGAGGTGGTAATGGGGAGTTGGGGATCAGCATGGTGTATCTCGCAGGCATGGCTGCAGCTGCCGTTTCGCATGGTCCCCACAGTGGT
TCAGGATCGTTACAACCAACAGTACTTTCCAGCTGAGAAAATCGAGCCATCAGACCCCCTTCTTAGATGGTTCTAGAAGGAAGCTCA
GAGCTGAGGTGCACGACTAGCTTCTATTTTAATTTTCATTTCATTTATTTATTTCCAAACCAGAAAGCTGGGAACTGAGTAAGGAAG
TCTCTGAGGGCTTTTCCATTGCTATTCTAGAATTTTCTTCCTTCTGGAGTTCCTCCTCCCCCCCTCCCCCCTCCCCCTCTCTCCCGG
CTCTCTGTGCCATCAGCATAGCTGGTGCTGGAAAAGGGTTCGTCTTTGCCTTTATGGCACCCTAAGGAGGTGCTGGTACTCGGTGCTT
CTTGCGATTGTTCTTAGCTCACTTAAACCTGAGACCTGAGCTCTTTTTCTGCCTCCAGGTCTTGTCCTTGTTCCAAGGACTGTCTGA
GCCACTGTTCTCAAAGCCATGCTCATCAGAGGTGTCCCTCAGTCAGTCAGAGAGGCACAACGTCATCCAGTTTGAGAGGAACTTG
GGCTAGCCCTGCTGGAGGCTCCCTGTAGAAGGTGGGGTAGTTACTGAAGGGTCAGATGAGCCATTGTGGAAAGAGAGACTCATTTCT
GTAGGACAGGGGGACAGAGGATAAAGTGTGTGGGAAATGCAATTTATGCAGATATCTTGAAGATGCCCCAGGATGTGCATGCAAGGG
```

```
CAGATGGGAGGAAAGAGAAGATTGTGAGAGTTATGGGGGCTGTGCTTCTCCTGAGAGGTTCTGAACAGGGATCAAAACACTCCTGCC
AGGGGTGTGGGTTCAGTGATTGACTTAATAGTGAGTTGCCAGGAACTCTAAAGATCCAATGCTCTTTTCCCCCTCAAATATGATCTT
ACCTGTTACGGATGGCCTAGTATGGTAGCTAAGGGGTTTGCATCTAAGTTTATTTCTTAACTCTATGGCATACCTGTGGTGGGGAAT
TTGCTGAACCCTGTTTCTTCATATGGGAAGAGGGAACTCCAAGCCCTCCTTAGATCATAGGACTCAACACTTTTCCCTGCAATATAA
CATCTAGAATCCTTCCCTGTACCATAGCATGCAGCATCCCTCTTTGAAGCACAGTCCGTAGCACTCAATACTTCTGTCCGTATTGTA
GTACCTAGCCTAGTGAGTACCCTGCAGTGCAGCACTGAGCTGGGCGTGCATTGAACTTCAGCAGCTGCCAGATGTTGCTTTTATTGG
ATGCCGCTTGGAGGACTGAGCCTGGAGGGACACAAGTACACATGTCCCCTGTGTATGCTTTCCAGGTGGTTGTGGAGAAGAGAAGAT
GGGCTTCAGGTTAGGAGAAAGGACCCTGGAAGAGTTGAGCACACGGGACCTTGCCCTCATTCTCAAGAAAAATGGACAAGCCACCCA
GTGTTACTGCTGATAGGTCTCAGTGTGTAATGAGAGGAAGGGCTACTCTTGGGAAGGCGGGACTCTCATGATGGCTTGGCCTGGCCT
TCTAGAGTCTCCTATCATGAGTATATTCAGGGTTGGCAGTCTGTGGCTTGGGCATAGCTAATTGGGTGTAGGCATTCAAATCGGACA
GGTGGAGAGAGCACAAAAATAGACCCATGGGGCCAGGCCAGAGGCTGAGAGGAAGGTCAGGGGGACACATGATGATCAAGCAGAATG
AGATTGAGAGCTTGGGACTCTAGGGCAGGAACTAGCCCAGAGACAGATCCAGGCAAGTGAAGACTGCTACATCTTGCAGAAAGTTCC
ATCAGGAAAACAGTTTCCCTTCAAGACCAGAGACAGGGGATACTTTGTATAACTTAAAAGGTTCCAGCCTGGCATGGTGGCAAGCC
TTTAATCCCAGCACTCGGGAGGAAGAGGCAGGCAGATTTCTGAGTTCAAGGCCAGCCTGGTCTACAGAGTGAGTTCCAGGACACCCA
GCTCTACACAGAGAAACCCTATCTCAGAAATTTAAAAAAAAATGGTTCCTCTACCTCTTTTACCCTGCTTAACACTAAGGCCATGTT
CCTAGCCAGGCACCAGGGGGCACCAGCAGTAATTGGGTGGCGGAAGCTATGCCAGGGAACCTGGACTTGCTGGGGAGGAAGCAATTC
CTCCCATCCCAAGTCCCTATCCCAAACTACTTGAATTATATTCTGAACAGGACCTTGGTGATTCAGGCAGAAGATGTGTGAAACAAT
ATGCAAATTGGCATCTGTTGTTGTGGCGTCAGGTCGATGGGCATTTTTCTTAAAGGACAAGAAGGTCGTTCAGATGTCAACTGAGCA
TGAACATTGGCTTAGACGAAGCTTGGGGCAAGCTTGTTGCTGAATGGGCTGGCGCTTGGACGTTGGAGCTATAGGTTCTTCCTCTGC
CACCTAGCATGTTCAAGGCTAGTTATTCGGGGTTTAGCCTGGAGAAGTCTGTAACAATGAACACCTTGCACAGAGGGTCACCCAGCA
AAGAGGGAGGAGGCTGGAAGATCATCTGATCTCCAGAGCCCTTTCCTAGTGGGGAGCTTGGGACCGGAGAAGGCTGCAGGGAGTCAG
GACTGCTCTGGGTTCTAACTATGGCCTACCCTTACTGCTGAGTCACCCTAAGGACCATGACTCAACCTCTTTGAGCTTCAGTTTCCC
CATTTGTAAATGGGAATACTGACATTCACTGGCCCAGTGAGACTCTCATTTGGTGATAGTTGAGTAAGTGGCCAGCGCAGTGGCTGG
CTGACACCTTTCCCAGGCTGGCGGTTCCTCCTCACACCTTCGCCTGAGCTGCCACCTGCCCACATTCCAGCGAGGTTTTACTAGCAG
GGCTTTTTTTTTCCCCCCCTGGGCCTTCTCTCCTTTGTGAGGTTTCTGATGTTAACAGATTTTTTTGTTTTTTGTTTTTTAATCTAT
TCTGACTTCCAGCGGGTGAGATGCTAACTTTTCACACAGATCTTGCACATGGAGACATCTGGAGCTGCTGACAGAGGTGCTGTGCCT
TCTCATGTTGAGAGTGTGAGAGTGATGCCATGGTTTAGGCCACCGTGTCGGGAGGGGCTCGGACCTGGAGGCCATTGGGAAGAGGAC
CCTAACAAGCCTTCTCTGGGGATAGCTGGGGACATTCCTGCCTTAGGTAGCATCCACCAACCTGGGTGGGCATGACTCCTGCACAGG
GCCCACTTCCATGAGGAAGGCAGGGCATGGAACACCGGTCAGGACCGCAAGGTTCAGAGAGGTTATGTGAGTTATAGGACCGCAAGG
TCCAGAGAGGTTATGTGAGTTATCCAAGAGCACTAGAGCATTGTAAGAAGCAGGTACAGCAGGAACCCAGGGATTCTGGGTTTTGT
CCTTTCTTTCTTTCCCGTTCTCCTACCCTTCTCCAGGCAATCTAGGTGCTAGGAGGGGAAAATCTGTGACCTAGAGGATTAGAGCCA
GCTCTGTCTGGGGTCTCTCCTTACCACTACTCTCTGCCATCTCATCTATCTATGTATCTTTCTCTGTGTCTATCTCTGTATCTTCCT
ATGTAGGTATGTATCTCTGTATCTATCTACGTATCTACCTACATATCTACCTATGTATGTATGTATGTATGTATGTATGTATGTATG
TATGTATCTATCTATTATCTATCTATCTATCTATCTATCTATCTATCTATCTTCTATGCCTCTTTATCTACCTAACATGTATG
TATGTATCTATGCATGCATGTATGCATGTATGTATGCGTCTATGCTGGGAAATCTATCTATCTATCTATCTATCTATCTATCT
ATCTATCTATCTATCTATCTATCTATCTATCTATCTATTGTGAGAATTGAACTCAGGACCTCTGGAAGAGCAGTCAGTGCCCT
TAACTGTTAAGCTATCTCTCCAGCCTGCCATCTCTTCTTAAAAAGTGCTTATGTGGGAGACAGGGAGGCAGCAGATTTCTGAGTTTGAGG
CCAGCCTGGTCTACAGAGTGAGTTCCAGGACAGCTAGGACTACACAGAGAGATCTTGTCTTAAAAAACAACAAAACAAAACAAAACA
AAACAAAAAACAAAGCAAAGAGTGCTTATGCTTTCCCAGCTTGGCAGCAGTAACTGATTGCTGAGCTGGCTGGCTTTGCAGAGCAGA
AGATAGGATTTTTAAATGCTTATTTTTTAATGATTTATTTTTATTTTATATGCACTGGTGTTTTGCCTTCATGTGTCTCTGTGTGAG
GTCTTCAGATCCACTGGAACTGGAGTTACAGACAGTTGGGAGCTGCCATGTGAGTGCTGGGAATTGAACCCCGGTTCTCTGGAAGAG
CAGCCAGTGCTTCCAACCATTGATTGAGCCATCTCTCCAGTCACAGAAGGTGGGGATTTTTAATTCCTGTTGTTGAGGGTAGCGGTGG
ACTAGAAGTGGATGAGGCTGGGGATGAAAATCACTGGTCTGTTGTTTTACCTGACTTATTCAAGGCACTGGGTCTCATGTCCAGGATG
GCAAGGAGAATGTGAGCAGGCAAGCTGGACAGGCATGGCCTCTTAGGGCTCTGGTTCTTGGCAGTGATGACTTTTGCCTTTGATGTC
TGTCCTGAGAGTCTGTGGCCTTTGTTGGCCCGTGGTTCCCACTCCTCCTTGGTCCAGACTGCTATCAAGAATCCTGAGATCCCTCGG
GAGTGAATGGGTGGGAGCAGGGAGTCAGACTCGGATGTAGGCTGAGTCGGGAGGGTTTCATCATGGGCAGGTGACATCATAGACAAT
ATTTTTGTCCCCGCAGCTTGGGAGGGTACCCCACAGTCTTGAAGCAGATGCTTCCTCTCTCTTCCTTGGAAACAATAGGAGGCCAGT
AGGATAACGCAGGCTCAGAGGACAAAGCTGCTCTATGCCAAGGCTGACTATCCGAGTTCAAACCCCAGGTCGGGGAAGGAGAGAACT
AGTTTCTGTAAGTTGTCCTCTGACCTCTACATACTCTTCCCTAAATGAGAGAGAGAGAGAGAGAGAGAGAGAGAGAGAGCGAGAGACTGCA
AGTGGGTATGGGTATCTAACTCTGATGAAGAGAGGAGACAGAAAGGAGAGAAGACAATGAATATTTCCAAAGGGTCTATTCCGTGTC
TTCACATTCCTNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNTCCTTTCCTTTCCTTTCCCTTTCCTTTCCTTTCCTTTCCTTTCCTTTCCTTTCCTTTC
CTTTCCTTTCCTTTCCTTTCCTTTCCTTTCCTTTCCTTTCCTTTCCTTTCCTTTCCTTTCCTTTCCTTTCCTTTCCTTCCTTCC
TTCCTTCCTTCCTTTCTTTCTTTTAGTTTGTTTGCTTTTTTTTTTTTTTTTTGAGACAGAGTTTCTCTGTGTAGCCTGTAGTTGTCCTG
GAACTTGCTCTGCAGACCAGGCTGGCTTTGAATTCTGAGATCCATCTGCCTCTGCCTCCCAACTGCTAGGATGAAAGGTGTGCAACA
CTACCCAAGAGTGTGTTCTTTCAAGCCACCCCCCCAAAAAAAACCCTATTCAGTCTGTCAGTCTCACCTGGCTCATTGTCCCCTTAT
TTTCCTTCTTATTGGCCTGCTCACCTGACCCGACACCCCTGGGGTGTGCGGAGGGGCAGGCAGGGTGCTGTCAGTGTGATGCATAGG
TTTGTGGGAGCTCTGGGAGCCAGGTACAGGGAAGGTTGGGCTTGGACATCCTTTGAGTTTCTCTGTCCCGGCTCTGTGACTCTGTGA
GTCTCAGTCTCTAGGTTTACAAATGAGGACAATGGTGTTTTCTCTCTAAGAGAGTGTGTTGTTGTGATGGAGACAAGATGAAGAGAAG
GCTTTGGGCAGGGCATGGTGAGTGTCAAATAATATCACATCAATAATGTTCTCAAAATCGACTTCCAAACCTGGCTTTGTTGTCCCC
TCACCTGAGGGCTAGGATGGGTTCCGCATCTTTCCTCCTCCAGGCAGTCTTCTGGGCCCTGTGTGCCTACCTGCTATCCTGCCACAG
GTTAGGTGAACTCTCCAGATCTCAGCCCTGTTTATCACTTGCTAGCTTTAGGAAGTTGCTCTAACAGATCGACTCGCAATGTTCCCA
GTGGTTCCTGGTAATCTGTCTGTCAGTCTTCTGCAGGAAAACGCCAGGCTGCTGTCAGTAAGCTTGTGACATGGCTTTTGCCATACC
CTGGGCTCTCAGTAAGGGTCAGCTGAGATTGTGGCGGCTGGTGGGAACCTCTCTGGCTAGGCATGGCCAAGCTTGGCGGCTGGCCAT
TTGTTTGGTCATCTGGAGAATGGAGCAGGGAGCTGGAGGAGTCTCCTTCCATCTTAGGCTGGTGACCACATGGAACTGGGGGAATGA
ATACTTGAAAATGGCAGTGCTCTGGTGACAGGGCACTGCCTGTCAGTGAGTGCAGGGTCTACTGGAAGAGTGGGTTGGAGGGTTATC
TTCATGTCCCATGTGTGAAGGTAATGCAGCTAAGTGGATTTCCTCACTATCAGGAGATGGCTGAGAGACAGGCCCTTTGGCCAGCTG
CCCTCCCTGCTGCCTCCTCCCCGCAGGCCAGCCCTTGGCATGAGGCCTGCTTGTCTCCTGAGCCCTCTGCGCAGCAGGGATTTAATT
ACTATGATGTGAAGAGCCCAGGCTCGAGGCTCTGGCAGGAGGGCAAACAGATTTGTCTTCTTACTGGGAGAATAAAAACGGACAGAGG
AGAGAGAAATGACTGTCCCCAACCTCCCCCTCCCCCAGAGGTTCCAAGGCTGTTGAGCTGATGGGAGATTTTTTAGGGGGATAAGGG
GGGAGGAACAGGTAGCAGTGTGTGTGGGGGGGCGGGGGCAGGTGCAGGATACCCTGTTCAATTCAGAGCCTTTGCTATTCAGTTTTGC
CTTCTATGACTAATTCAGCTAAGCTGCCTGTTTTCCCAAAGACTGGGCAACAGCCTCCATCAAGTACCTACTATGCGCTAGATGTTG
```

```
GCCTTGAGAAACTTCCTGTCCAGGCAGGAAAGCATGTGTTTTTATGCAGCCAACAGATGGCTCCTGACCACCTGTGAGGGGCCAGCC
AATTATTCTAGGCACAGCACAGTAGGAAAAACCACAGTAGGCCAGAGTCTTGCCCTTGGGGATGCACATTTCCATAGCTGAACCCAA
CAGTAAAAGATATCAGCTAATTATATATTGTGTTTGAAGTCCTATGAAGGAACAGAAAGCTGGGCTAGCAGGACACGGCGTTCCACA
GGGAAGCTAGTCATCTCGAATAAGTGCTCAGAGGAGGCCTTGATGAGAAGGTGACATTTGGTGAAGGTCTGGGGTTGGTGAGCAAGC
CAGGCTTCCTTGGCCATGGAGGTGGGGACTAGCTTCTCCAAGGCCTTCGTCAGGTTTGTGCTTGCTGTGGTCTGGATAGTTCTGTCC
AGTAGACCTAGAACACGGAACTATCTTGGTCCTTTTATGCTTCCTGGTAGCTGTATATTTTTAAAAGTAGGACATATGAAATTTGTT
AATATGTTCATTTCATGTGATGAAGAAAAATATTACAATTTCAATGCGTAAGCAACATAAATATTTTCAATGAGACATTTTCCATCC
TATCTGCCATGCTATGTCTGGTGGATATCTGATGGCTCATCTCAGCCAGCCACTAAATCTTCATTGGAAACACTTACCCGGTTTTAG
AGTCCATAAAATTTATAGTTAAATAAACACTCACGTTGTTGCAAGCATACTTTAGGAGTTTCTAATAAATTAATGGTGAACCAGTTT
AGAATTTATGGTTATTTATTTTGGTACTAGGGGTTGAACCATGGGCTTCACATGCGCTAGGCACATGCTAATACTGCTGAGCTATGT
CCCCCACATCCTGGTACATCAGTTTTCAGATGAATGATTAATAACAGAATGTACACATAAATTCCTCACCCTCGCCATGTCAGTGGG
GTGTGGTGGAACGTGTGGCATCCATCAGAGACTCCCCAGGAGTGAGGAGCCCCTCCCTCTGCCCGGCTGAGCATAGGGAGCACGGGG
ATGAAGCCAGCTTGGAGGTAGGACTTGGAGTGTTCTGTGCAGCACTTGCCCTTTACCCTGGCAAGATGGCATCAGGTGAGGGCTCTG
AGCTTTGGTATTAGAGTTAGGCACAGACAGGGAAGCCTCACCAGAGGTTTAGGGACACTTCAGGTGAGGCTGGTGGTGATGGAGCAC
CCAGGTGGGAAGTAGCAGCCAGGTGGTGAAAGTGGGTGGTCCTCGCAGTGTTTGGAAGGGTAGGTAGGCTTCTGGTGTGTCTGCTGA
AGTGGGGGGTGGGATGGGCAAGGGGTAACCCTAGAAGCTGGGAGATTTGTGAGACCCACACTTGGGTTATACAAGGTTCTTGCCCAG
TCCCTTTCTTTGGTGGACGGCTCCCCTTGCATTCCAGAAGACAGTTTGGGACAAGGCTGGCATCCTTTCAAACAAAACCAAGGGGTG
GGCAAGATAGCAGATCAGGGTCAGGGTTCTTATGGCTAAGCCTGTTAACCGGAGGCGCCCAATCCCTGGAACCCACATGGTGGAAGG
GGAGAAGGGACTCCCTCGAGTTTTCCTCTGACCTCTACATGTGTGCCTTGGCGTGGCTAAACCCCCAGCACCACAGAATAAATAAAT
ATTTAGAAAAAATTAAGAAGGGGGCTGAGGAGAGAGTTGCACGGGGAAAGCTTCTGTAGTATGTGTGTGAGGACTCACCTTTGAATCC
CTCCCCCCAAAAACATTGAACCCATATAAAAAATGGATGCGGGGTGTGTGTCTGCAATCCCAGGGCTTCTGAGCGAGGTGAGAA
TCAGAGACAGGAGACTCCCTGAATGCTCACAGGCCAACCAGCCTAGCGTGCAGTTGTGAACAGCAAAGAGATTCTGTTCAAAGGA
AAAAATGTGGCATGGAAGAACTACGCCCAAGGGCCATGTGCACACCATGCCCCCCGCCCCCAACCAAGAAACAAAGCGGCACACACA
GAACCCAAAGAAATTATCGGTGCTTACAGTGTGATTAGTGTTTAGTTCCCAAAGGTCACAGGAATCTAATAGTCAGTCCACATTTGA
AGATCAAGTCTCCCTGTTCTGGGCTGACCCCAGAATGCTTTGATGGATAGGGTCAGAAGTGGCCAGAGATTCCCATGGTATGACCAG
GAGATTACTGAGATAAGATGGATCCCATGGATTCCTAGTGTTCAAACACTTTCAACTCTGTGGGCAGTGTGTGTGTGTGTGTGTGTG
TGTGTGTGTGTGTGTGTGTGTGTGTGTGTGTGTGTAGATCAATGAGCTTGAAAAGATAGCTCAGAGGTGAGGTCTAGGTGTTGGGAG
GTGCCAGTCTTCTGCTCACTAGCCAAACTGCTATGTCCAAAGCCCGTTGACTCTCCAGGGCTCTCAAGGACCACAAAGCCATGCCAC
TATAACTGTTTCCTTTCTAAGTGTGGAAAGAGCAGGGATGGCTTTAGATGTCTTCTCCTGATGTCTTGATATCTGATGGTTGGCCTT
GGGCCCAGAAGGTGAGGGTCTGAGGCTATGGGACCTGTGGGCCAGCTCCTCTCACTGGTCAGTCAGATATGCTGGGTGTGGGTCTGC
TGCTGCATTCAGAGTTGGTCTGTGCCACTAGTTGACCATATGACTTTAGTACCTAAGTCTTCATCTGCGTTTGCTCATTTTGTGGTG
CAGGCAAGGGTGATGTAAATAGATTGTGGTGATCCGGCTCAGTCAATGTTGACTCCTTTCTTCTTCTGTGAGGTACCTCAGGAATAC
CTCACACAAGCTTCTCTGGCCTTTGGTGAAGAGAGACAGCCTGTCTGGGGTGGGATGTTGATGAGGAACAAGAGGCAGGGGAAGGTG
GTGATGGGGAGACCTGGGATCTACCTGGCCTGTGAGCCAAGGCTTTCCACCTGAGGTGCTTCTCAGAATACATGTAGCAGAGTCTGG
AGACTTGGTTGGTCATCACAGCCAAGGGAAGGATTTGATAGATACCTAATGGACAGGGGCCAGGAATACTCACCAAATCAAGGTATT
ACCTGGTTCCAAACAGCAGAGACTGGAGAAGCCTGGATAGATGGCTTGTTACAAGGACAAATGGAAGGATGAAACCGTCATCCTAAC
TATAGCATGACACTAGTCATCTCTGGGATGCTGGACTAGGTGGGACTCAAAACTTCCATTTTATTTTGAGGGTCTCACTATGTAGGC
CCCTGCTTGTCCAGAACTTGCTAGATAAACCAGGCTGGCCTCAAACCCACAGAGATCCACTTGCCTATGCCTCTCAATAGCTGGGAT
TCAATGCATGTACAACTATACTAGGCTCTCAGATTTGAATTCATGATCATGTTCAAATTCATGTTCAAGTTCATGCCCCTCCAGAGT
TTTTTCTTTGTTTCTAATCTCTGTGTCTTTACATCCTTCACCTTGGAGGTTTTATCTGTAGACGAGGCTGAGGTATGAGCCACATTA
GAGCCTGTGAGAAGGTGAGACTTAGGGGCTTTTCCAGGACTTCCTCTTACCTCCAAGAGGCCTCATATACTGGCCCCACTTTATTTT
GGTCTTTTTTAAAAAAATGTGCATTGGTGTTTTGCTTGCGTATGTGTCTGTGTAAGGGTGCTAAACCCCCGGGAACTGGAGTTACAG
AGAGTTGTGAGTTGCCATGTGGGTGCTGGGAATTGAACCCAGGCGCATCTGGAAGAGCAGCCAGTGCTCTTAACCCCTGAGCCATCTG
TGCAGCCCCACATCTCCGTCTTTGTTGTTTCTGAGAATGAGTAGAGCTGAAGTCTCCATTTAAGAAAGAGGGGATTTTCAGGGCTTA
CGACTCTGTGAGTGGGAGACTCAGCTGGTTTAGTGGCAGACTTCCCGGCATGGGGAGAAACAGTTGGTTTGCCAGCTTGGGTCAAGC
TAGAAGCCATTTGAGGTTGTGTGGGTCACTGCTCTCTAGGTCTACCTGGGGAAGCAAGTACTTGTGCTGAGTTGCCGACCGGTTCCT
GGGGAAGACAGATCTAGGAAAGGTCTGTGGACTTCCCACTGGGAGGACTCTCACTGAAGCCTGCTTGCACTGGGAGGACTCTCACTG
AAGCTCGCTTGCTGTTTTGCTTGGTGCTGACTCTGGTGATGTTAGGGAGCACTGAGATGACAGCGTATGGTTCAGGGGTGTGCAGGC
TTAGTAGCTTGGAGGCAAGCAGACTACTGTGAGTACCACACTGGCTCCTCCTCCTTAAGGCTGTGCTGTGAATCTGAGTGTCTGACT
TCTCAGACATCGTGGGGCTTGAGTCTGGCTTTCAGGGCCTCACGAGGCAGAAGCGGGAGAGTGTTCTGAGCTTGAAGGGACAGTGGG
TGGGGTGGGTTGGGTGGGGTTGGTGGGCTCCAAATCAGGAGGTGCCAGGTGTTCTTCGGGAGGAAAAAAAAGGAAGCAGTTTTCTC
AGGGAGAGATAAAGAACAGACTTGAACGGGACAGGAGGTCCCCATTCACTGGGTCTTTGCATACCAGGCAGAGGGCTTTGCACGCTT
GTTAGAAGGCAGGGGAAACATGGCAGTGGTGTGATTAAGCTTTGGCACAGAGAAGAGTAGTAGGAAGAAGCTGGCAGCATGAAGTGA
GGGCTTGAACCAAGCTAGGGACAGTGGAAAGGAAAAGGTGGGAACCCACAAGAGAGCAATGTGGGCATCCTCCTTTCGGCCTGGGA
TGACGTATGCTGTCTGTGAGGGTAGATACAAGAACTGTGGCTTAAGTGCCTCTCTACATTGATCAAAATCCAGCCCTTCCTTGGAGG
GTAGACAGCAGTGGACAGGTGAGCAGCCAGGCAGCCTGCTGTCCCAGAGAGTAGGGTGGCGGAGTGCCCATACCAGCCGGCCACCAGGCACCG
ACACCCTGGCTGGGGGCTTCTCCTTCTGGAACCCATCAGCCCTGGCCTCCAGCCAGACCTGATGGAGACGGATCACCCTCTTCCTG
CATGTTTTCAAAGATATGAGTGGGGTAGTGACAGCATGCAGAGCTAGCCCCTCTAGGCTTCATGTAGTAGCCTGACTATTTTGTGAC
TATGCGTAAGGAGCAGGTGGGCAACTATCGGGGCACTTTGGGCCTCAGTTTCTGGGAGTTTTCTGAGTGCACTGGTGCTCAATCCAT
GCAGTGATTGTAAGAGAGGATTCAAGAACACAGCCGGGAGGTTCTTGGCTCAGCCACAGTCACTATCTTTAATTGACAGGAAGTGGT
GATGGACTGGACAGGATGTATAGGAGAGATGAGCTATGGTTACCAGGAGATAAGTTGGACAAAGTGGGGTTGGCAGTTCTCC
TAGGCCATAGCTCTGATGGGCTCGGGTGGGGGGCGGGGGGGGATTGCAGGAGGCTACCCAGTGGCAGCCTGGGGTCGTCCCACCTCG
TGATTCACAGATGTTTTTTTTTTTTTCAAAGATTTATTTATTATTGTATCTAAGTACATTGTAGCTGTCTTCAGATGCACCAAAAGA
GGGTGTCAGATCTCATTACGGATGGTTGTGAGCCACCATGTGGTTGCTGGGATTTGAACTCAAGACCTTCGNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNTCTCCACCCCAGATGGTTTTTATTATCTACCTTAGGACCACCTTTTCAACACACCAGCATCCCTACGAGCCAC
CTCAGTGCAGTGACCAGAGAGGTCAGTGTGTTCAGCCGATCAGCTTAGCCTCTGGTCTTGTCTGTGTTCCTGGAGACTTTCCTGGGC
TCACAGTGCCTCCCTCCATCGCGATGCCCCTCAGAGGCTAGCTGGATCTCTTTCCAGGGTTTCAGTTAGGGCCTGGCTTAGTTCAGG
CTCCAGAGAAGGAGTACTGCCTGGACTGGCATGTTTGTACTGATGTTTGCTGTCCTCTGTCCACTCTGTGTGTGAGCTGGACAGACA
TGAAGGCTCCCCAGGGGCAGCGTCCTGCATAGCACAGGATCGTGATGCGGCTATCTGGAGTCCTCAGGAAGACTCCAGGAGATCAAT
GATGTGCTGACCAGCACACAGAGAGAGGTTGCTTGCAGGTTGAAGGGAACAAGGCTGAGGTGGGCACAGAATGGCAGAGGTGGGT
GAGTGTGAGACAGGCCTAAGGGGAGGCCCACAGCAGAGAATCTGTGATAAGAGGCTGGGCAGACTGGGTCAGCCATGTGAGAGCAG
AAAGAGTGTTCTCCAGGGTCTTCACTAGCTGGCTGAGCGTGCAGGTGGAGGTGACGGCTGTGGGTGTGTGGGGGGGGGGAGAGGCT
GGGCTTGGGGATGCTCCAGTGTTTTCATCACAGCAAGTGACAAGGTGTGGTTGTGTCAACAGCCTCAGTGACTGGCAGACTAATCAG
CATCCATTCTTCCTTCCGTGGGTACAGCAGTCCCCAAGGGCAGTCCCCAATGCCTGGATTACCTCCAACAGTGGCAGAAGAAGCAGA
GCTCTATCTGCTGCTGTACCCCTGTCCCATGTGACTTATGGCTGTCTGCTTCTCCTAGAATTTGATCTAACCTTGAGGAAACTAGCC
```

```
CAGGATCTATACGAATGCAAACACCAAGACTACTCTTGAGTCGTGGTCCAGGAGGCCCAGATGAACCCTCCCAGGACTGCTTAGATT
TGGGAGCCAGGCGACCAAGAAAGGGCAGAAGGCCTCTGTGAAGTAAAGGCAGAGGAATAGGTGGGCAAAGGAGGAAGAGCAGACTGC
AGCCAGGGGCTCAAGCAGGGGAGGCACTGGCTGGGCCCTGGGTTTAGGCAGAGGGGAGCATGTCTTATCTATCTCATCGGGCCTGGC
TAAAAGCCAGTGCTGATGAATTCCAGAAGACTTTCTAGACAGTGAGTTCTGGAGCTAGATCTGGACAGCTTTGTCTTCCAACTGGGA
AGCAAAGGAGCCAAGACTTTCCTCTGGGCTTGAGGATTGTAGAGCACCAGGGAAAGGCAAGGTTTACGTTTTCCTGCCCTAAATAAC
AAGGGTGTCCCTGAGGGTCACTGTGAGCACTTTAGGGGATTGTCCTGCAGTTATTCAAACAAGGTTGCTTGGAGGGGTGTGTGTGTG
TGTGTGTGTGTGTGTGTGCGTGCGCGCTGGGGGCAGAGTTTGGCTCCCTTTCTGGATGAACCCTTTGGAGGGCCTTGTTCCAGAA
TACCTAGGCCAGAATCATTTTGGAAATGTCTCTGTTGCAACTGAATGTGGACACTGGTTAAAGAGGCTCGTGGTCCTGTGTAGTAGG
GCTCCTGCAGTAACAGCAGAGACTTCCTTGGTCCATGTGATCCCTTCCAAAGGCAGGTGAGTCCCATTCCTTCAGAACCCATTTGGA
ACTGGTCAGCAAACTAAGAGGTGTTGTATGGGACTCCTGAGGCTGAGCAACCAGGTTGGGCATAGCCTACAAGCTCTCCTCAGAAAT
GTATGGCCTTTGGCACCGCATTGGCTGGGTACTGAAGAGTTGACAGGATGTAGAGTAGGCACAGCAAAGAGGTTTTATCTGGGTGTT
AATTCTGAATGGTTGGTACCAGTTGCCAGAAGAGCTGTGATGAGAAGGATGGTGAGCTGCTATCTGAGCTCAGTGAGAAAAGTGCTG
TGATTGATTGATGATGTCTATCATGGCAAGGAGAGATGGAGGGAAGAGGGGATTTGCTTTCACTGGTATGGCAGAAGGAATGCAGAG
ATCCTAGATTGACCATTAGCGAATAACTCATATGTCTTTGCATCCCAGTTTCCTTAGCCTTAAAGTAGGAGTGATAAGGTTATCGGG
TATTGAGTAAGATAACGGAATTAGACATTTTATTCAAGCGAAGACACAGAATGCGGGTGTCATTGTTTTGGACTCACTCAGGAGCCC
AAAGTGACTTCATAGCTGTAGTGAAGGCTTTCTTTGCTGCTGAAGGACCACAGAACAATGTGTAAGACAGTTGATTGGCATGA
CCTTTGACCAGGCCTGTGCCAGGCATCTCGGGACCATGATACGTTCTTGACTAAAGTGTCAAAGAAGTGTTTGCTTATTTTTCTGGTT
GGTAGTCACGTAATTTCTGTCAACGATTCCCCAAATGAAGATTTAAACACTGTTTCTGTGTGTAATGAGAGAAGGCCAGATAGCTCT
CTCTAGAACTTTGTCTCTGATTCTCCATTAAAGGTAGCAAAATTGCTTTATAAAGTCTGAAAGCCTGACAGGATCATGTATTAACAA
TGCATACATTATGCAAATGTGGGTGTAGTTACTTAATTTGCATAATAGATGGGCTGAAGGAGGGCTGTAAAGGGGAAAGGGAAGGAG
TGAGGGTACTTGTCCAGATCTGTGTATATTAGGGTTCCCCAGTCACCCCACAGGGTGCGTTTCCCCCTTCCCATGCTCCTACTCAGC
CTTGAGGTTACTGTGTGTCTTTCCCTGCTGGCTAGCTGCCTGTGCCTCTGTGTCCATCCTTCACCCAGGCAGGCGCAGGGGCCCTCA
CCCTTGCTCCTTGAGCAGTGAGGCTTCATTAACTCTCACAGTCCCTCTGGGGACAGACAGCTTTCCCCAAGAGCTTCCTCGGGTCAT
TGCGTGTCCTATATCTGTCCTGCTGAGGCGCTGGACTTTGTTAAGTGACATCTTGAATTGTATGTGAGTCTTGTCATTGGGTAACTT
TTTCTTGGGCTCTGTGAAGCAGAGGAGGGGAGGTATTTTAGGGGAGCGTACCTCCTGATCCATCTACATTCTCCCTGGACATATCTG
ACAGCTGCTTTGAAAGGTTTTTGTGCTCTGGCTATAAGGGTTTCAAAGTTCTGTTTCTCCACCCTACTTCCCGAAACCCCTTTTAG
CCCTCCTCTGTCCTATACTCCCAGGGGCTGGGACTCAAACCCAGGGTCTTCTGCATGCTAGGCAAACTGAGCCCCAACACTAACACC
CACCCTTCCCCTGAGTTTGACAAGAACTGAAACTGAGACCAAGGGTATTTGAGGTCAGAAACCCAATGGATACTCCCTGGAAGCCTA
GATTTGGAGGGTTCAGGTTTAAGGGGTGCTGGGAACCATCTGTGTAGGTCAGACAGATGTGGCAAGCAGCTGGCTAGCTGGCTTCTC
TGGATGGTACAGTGGGTGTTCTGTGCAGTTGATAGACACAAGAGCCCTGCCCCCCTCTGGCAGAGGGTGGACAGGGGGCTAAGTCTG
CAAACAGTCTAGAGCTTGGTGTGGTCATACCTGCTCAAAGTTCCCAGTATTAGGGCCCATGCAAGGCTCTGAGACCCTGAAGATAAA
CAAGAACTTCCAGCTTTCCTTGGCTGAATTCCTACTGAGAGTCCTGGTATTTCATAAAGTCACTGGGTTTATTGTCCCCATTTCATA
GCAGAGTATGATAAGGCTTGGTCTCATAGCCAGGGAGCACCCAGGAGATCTTGTGGAGATCTAAGGGAGATCTTGTGACCCTCCCCC
TCCACGGCCTCTTCTCTTGAGACTGGAGTTTGACCTGGACCTCTGGTGCTGGGGGTGTGAACAGAGGGTCCAGGCAGTGTGATAGAA
GTAAGGAAAGGGACTAGAACTGGAGTCACAAGAGTCAGGAATATGAAGGACAGTAGAGAAGGCCCTGAATGAGGCATGTCAGGGAGT
CTGAGTTGACCTGGTCTTTGGCGAGCCAATGGAGTAATCATAGGGGTTCTGGCTCACAGGTCACAGGAGCCTCTCCTCACTTG
CTCCACTTTGGGTTTGCAACATTGCCTCCTGGGAACATGAATGCTCTTGTATATATATAATATATAATATATAATATATAATATATA
ATATATAATATATAATATATAATATATAATATATAATATATAATATATAATATATAATATATAATATATAATATATAATATATAATA
TATAATATATAATATATAATATATAATATATAATATATAATATATAATATATAATATATAATATATAATATATAATATATAATATAT
AATATATAATATATAATATATAATATATAATATATAATATATAATAATAATATATAATATATAATATATAATATATAATATATAATA
TATAATATATAATATATAATATATAATATATAATATATAATATATAATATATAATATATAATATATAATATATAATATATAATATAT
AATATATAATATATAATATATAATATATAAATGTGTATATATATAATATAATGTGTATATATATAATATATATATTATAT
ATATATTATATATATAAACACCTCTTCATCCTCTTGGGTTAGGCGGGACTTTGTTCTGGAGTGCTCTCACACGGTATCGCCAGCGGA
TGGAGCAGAGTCTACTGATCCTACATCTGTCAGGTGGGGCTGTGCACACTCACACAGTTTGTTCTCCCAGCCCTGCACGGACCCTTG
TGCTCCCTCTCGGTGCCCAGGCTTCTGACCCTGGAGGGGCTCTGGTTAATGTCGGCGGTGGGCAGTAGGGAGGAGCACCTGGCATGC
AGATCTGAGAGGCTGGCTGAAAGTGATGGCTGTTTATAGACTGTGGGACTGTGGGACTTTAGGTGGGCAAATTCGCCTGTTAATTTTCTCATGAA
TGAAATAGGGGATAGTAACACATTCTTTGGGGGGTACACCAGCCCTGCTACATTTTTTCTGAGACAGGGTCTTTACAGCCTTGGGTG
GCTTCAAACTCTATGTAGACCAGGCTGGCCTTGAACTCACAGAGATCCTCCTGCCTCTGCCTGCCAAGTACTGGGGTTAAAGTTGTT
TGCTATCTTGTCCAACCCCTGTTATATTTTGGAATTACCTTGGGATCTTTTTTTTTTAAACTGATGTAAAGAAAAGAAACGACAACAA
CAGCACCCCACGCCTGTCCTGTCCCAGCTACCTACCTTCTGACCTCATTGGTCAGGACTACAAGCCTGGGTACCAGGGAATTTCCCA
AGCTCCATGGGTTATTCTAATGTGCTGCAAAGTTTGGGAACCACTCTGTGGGTTTGGGGGATTAAAGAAGATGATGCAGGTTCCTG
GGGAGAACTCAATAAATGGTGGTAATTAGGATTTTCCTCATTGCATTTAATGTGGGCTCTGATAGGAGAGGGGGGAAGGGAGGGAGG
GTTTCGACTTTATTCCACTAAGAGTTTGTCCTCAGTGGCTAGGCTTTCCCTAGAAGAGTTGTTCCCTCATTTCTAACACTATTTCTG
TCACTTTAGAGCCAGTTTTAGCCTTCCTCATAGCCATCATGGTTATGATTAGGTTGGGTGCCCAGACGGGAGCTGGGGTGTGCTGGA
CCCAAAGATAAGATGTCAGGTCTCTCCTGAGTCTCCTCCTGGTGCTTTTGAGTCCCCAGGATGGCTAATGGAATCAGAAGCTCAGGA
TGCTTGCTGTATCTTTGAAATGCTCTCCGCTGTTGCCTGGCGTGCCCAGCTGAGCTGTGAAGGAAGACACATTTCCCCAGACTCCTCTG
CTGTCCTACAGGAAGTTGTGCATCAATGGGCTGACAGGAAGAAAATGGCTCTGCTGCTGGGGAGGATCTGATGGGGGCTGAGGGAAA
ACGTGGAATCTTCTGGTCCACACAACAAGGCCCCTCTTTCCTCAGGCAATTTCCCCGTTTATTTTCCTCGCAGTGACAAAGATTGGG
TTTAGCTAATGAGGAGCCGTGAGGGGAGAGTGAGGGGGGGGGGCGAACCTGTGCAGGTGCGCCAGGTGGGAAGCGCTGTGGCAAGC
TGCCCTCGCTTCAGTCAGTGCCATTTCTCCCAGCCCCCTGCCTGGGGGGACTGCACGATGACTAAGCATTCTATGATCTCAAATGCT
TTTTGCTCTGTCTACCAACCACACCTTTGCAGACGCCTTTGATTTCTCTTTTTACCAGCCCCCAAACCCCACAGTGAATCTTT
TAATAGCATAGGCCTTTTGGATCTCTAACCAATCAGTTGCCAAATCCTATCCAAAGTACACCCAGAATAGATTCTTTTAATAAAATA
GATCACATTTAAGCCAGCATAAACCTGTGAAATTTGTACATTTCTTCTGGAGCACCTCTTCAAATTTTACAATATTGAAATCACCAC
CCAGTGACCTTTCAGGAAATACTAGATAAATTGCAAGTGGCAAATAGACAAACTAAATGGACTAAGCACCGTTTCTAGCAAGTGCCT
TTCCTGGTGGGCTCTTAGTCCCAGTCTTTGAAATCCCTGACCCCCAATCCCCTACCCCATCCAAGCCCAGAGATCTACCCTGTTCTA
CCTTGCAGGCCTGCTTGCTGCTTTGCTCCTGGCAAAGAGCTGATGGAGTGGTGCAGCTCTGAGAAACAGTACTGGTATGTGGCTCAC
GTGGCTCGCAGTATGTGGCCGCAGCACCTTGTCCAGTCTGTGTTTGTCTGCTCTATCATTCAAACCCACTTCCACCCATGG
TCAAAGCCCCCAGGGTGCTCTGGTGGGTGGCTGAGATCTGCTTTCCTAGGCAATGGAGGGGGTCCTTGGCTTTGCCAAACATGAGC
TGGGCTGTGGTTTGAGCTGGGTGATCTTCTTGATGTGGAGACACTCCGTGCCTAGGCTTGGTTACCTGAGTGTGATGGCACACTCTG
TGTAAGCTGTCATTTGTACATGATGCTGTCATTTGTATCCCCGGGTCTAGCCTCGGCTATGATTCTGCTGCATGGGTATCATCTTGC
TTGCCAGCTGTTGGTGGACAGGGTGTGTGGTGCAGGAATAGCATCCTGTTTGTGTTTAATGTAGGGAAAGCCATTTTGGAAGGCAAT
```

```
GATCTATAAAACCATCACCTCAGTGGAAACCCTTAGAGGGTCCTGGTCATTCAGTGTGCCTGTGAGTCAATGTGTCTCTTGGGCAGG
GGCAGGGGCCATTGTGCTCTGCTTCCTAAAGGCTGTTAATTGCAGTTGACTCTATGGTCATGGTCATGCGTTTATGGGCTCATTTCA
TGGATAAGCAAGCTCAGTCACTAGCCTGACTTCTTGGCCATCACCACTTGATGACCCGTGTCAGCACCAGTAGGAGCTGATCTTCCG
GCCACTTGGATTTCCACCACTATTTCCCTGTGACTCCTCACACAGTTCAGTTGTACCTGAAACCCCACTTGTCTCCCATGTAGGGAG
GGCAGAGAGAAAATTACAGCATGTCCACAGCAGTCTGGCCTTTCTGTAGATGAAGCAGCATCCTGAAGGCAAAGAAGTCTGGGTTCA
CTTCCTTCTTATTTTCCCCCCTCATATCTCAGGCAAAATGCTGGGTTTCTGGAGGAAGTAGAGAGAGCCCGAAGATAACACACTGTGT
GTGTGTGTGTGTGTGTGTGTAAGTGTGTGATGTATGTTGGGGGTTTCTGATTGTCAGATCCCATGATGTGAGTGTGAGCTCTGGG
GCTGTAGACGTAGGCAGGGCTTGCACCCTGCAGTACCTATCCCTGTCCCAGACTCTGGGGAAGTGGCCAAAGTCTCAAGAATGTGTG
TGTGTGTGTGTGTGTGTGTGTATGTGTGGTGTGCAGCTTGTATCATGCAATGTGCAAAGAACTTGCATGGTGCAACAGAAGGAGC
CCCGGGGGTGCAGATCTAAGATCTAGCCACTAGGTCCTGTTTCTAGATCTGGATTCTGCAGCGAGATGACTGTGACCCAGGCCTCAGT
GTCCCGGCATGTGAGGAGTCCGAGGCCTGGCCCTGCCCCTAGGATTCCCGGCCTCTCCCTCTTTGGTGGCATCTCTTTGTTTTGTATT
TGTCTCCACCCCTTGCCATCTGACTCTGTCTCCTGGCTTTACCATAACCAGCAGCTGGCAGTGGCTGGAGCCAGAGGGATGGCTCCT
CTAGGAGGAATGTACTCTCCTGACTCTGCGGACCATTGGCTTCCTCCCTCTGACCCCAGCACACTGGGCTCCCACCTTCCCAGCCTG
GGCCCAGCTGGGTTGCTTCAGTGAAGTAACCACTTCTCCTGCTTACAACACTCCACCTGGTCTGAAAGTGACAAGAGACAGCTGCTT
GGTAGTCAGTGACGTCTTTGTGGAGAAATGACAGTGCATTTGGCCTGCACTGTCTTTTTCCTTTGCTGCCTTGGGTTTTAAAACTCA
GCTGTTGTTATCTACACTGGTTGTCTGGGAACTCCAGGGTCGGCTCTTCCTGCCCAGGGAATAACTTTTGAAAAGGCTCATTGGCCCC
GTGCTCCCTATCCCATCTAAGTGTCCCTCCGGGGCAAGGAGCCTCGGGAGGGCTTGGCAAGGCTCTCTGTTTTCCTTGGTCTTTGAT
CACAGCTGTTAGAGTTAAAAGCTTAAATAATGCATGTTAAACAAATAAGTATCTGTTTAATCAGTGCGATGGGGAAATTAAGGTGAC
TCAGGCTTTAAATTCCTAAGTGTCACTACCTGAAAGATGGCCTAAGTGGTAGAGTAATGGTCTACTCCTCTCTCCTTGGGAATTCAT
ACCTCTGGTGGCCAGAGGAGGAAGACCAGGGCCTGTGGGACCTTCCGGGCTTCTAGCAGGCTCCTCTGGACTTCACTCTTTAGTTGGCAA
GCTTAAAGCTTGTTCCTGGAAAACAGAAATAGTGCAAAAGGGAAGAAGTGTGGGTGAGGAGCATGATCGAAACTGGAGGAGAGAGA
TGGGAGAATCTAGCAAACTTTCCCGTGAGGGAATGCTCCTCGCAGCGCCACAGTTCCCTGTTGAGACCCTGGCCTTGGGCAGCAGGA
GGCTGAGGAAGGACAGGTCCCTGTTCATTCTCTGCGCTGCATGGCCTGAGTTTTATCATGCTTATTTTTCCAAGACAAGGTTTGTCT
AAGTAGCTCTGGCTGTCCTGGGGCTCACTATGTAGATGAGCCTTGAGCTCAAGGTGAGGCACCTACCTGCCTGTCTTCCTAGTGCTG
GGATTAAAGATGGAGCCACCATACTTGGCTAGTTTCATCATCTGTAAAGGCAGGTTTGATGAGATGCTACAAAGGCCTGCTTAGA
TCTACACTTAGAGAGGAGGCGTGGGGCTGGTGGACCAAATAGAAGCCACTCAACTATAAAATGATTTTCCTTGGCTTTTCCCATTTTG
ACACTAGGAGACAGCTTGTGGGGCCAACGAGCACCCATTACCTCCTCAGTCGCACTGCTGGAAGTTGGAAAGTTCTGGGTCTCTG
TGTTTGCATATTTTTCCGATTTGGACATGCTCTTCTTAGAGTTTTAGGAATTGTTTTCTTTCTGTCAGTGAGGCCCCTTGAAGCTTT
CAGGGCAAAAGGCTCTTGAGTTGGGCACACATTCCCTGATGCATTTGCCTAGCTTTTTGTCAGGCCTGACACCAGGTGCCCGTGGTC
ATCCCAAGTACCCAACCATGGGGCTACTTCAAAGCATAGAGAGGAGCCCCCAGTACAGCTCATGCTTATGGGTCTATGGGGGAAGACT
TGACAATGTTTTGGCAACATTGTGTTGGAAGAACTGGACTTCTCACCTTGTGGCTCTAATTGCTTAAATATGCTGGGCCTCAGGTTT
TCTGTCCATGAGATGGGGAGCAACTTGCTCTATCTCCTAAGGATAAAATAGGAAATTGAGAACAATAAAACCAAACAAAACCCATAAA
TAGGAAAATTCTAAGTCGATGTCTAGGACTCACTGATTTGATATCAAATATATTCTGATAGTTAGCCTTTGACAGGTTACTATCTCT
CTCTGTTCTGAGAAACTCAAGCCAGCAAGGGTTAAAGCAAGCTAGGCTTATGACTATGACTGTCCATAATTGATGTGATAAACCTTA
AAGGAGTGACATATAAGTGCAATTAATCCAGGCACAGTGACACTGCCTTGCTAAAAATAGTGTCTACTAATAATTACTTTGATCTAG
CAAATGATGCCTGTCTGCTAGGCTGGCTGGGGCAGGACGGGTGGAGGGTCTGCCAGTGCAGTAGAAAACTCTACTCCTCTCTTCTGC
AAGGCGAGAGGCTTCAGGGTGGAGACGCAGGGAAGGGGAGCCCATCTCTCAGGCCCTCTGGCAGCAAACAGTGTGGACGAGAAGGTA
TCTGACACACCATTTAGTATCATACACTGTTACTATTCATATTAATAATTATGCCAGACACAAACTTGACTTTCTGGGTTAGCAGGG
AGTATGGACGACCTGTGTATGGGCTGTACTGTGAGCTCATGGGATGTCTGGTCTTTTTCAATCCTGGAAGGAAGAAGAGATTTGCCG
TCACTTAATTGATATTAAAGATGGTAACAGTGCCAAATGGTCCACCTGCAATTTTTGGACTGGCTCCCTAGGTGGCTGGCCTCATGA
AGGTAGAGGCAGCTGGCTGGGATCAGGGCGCTCTGTGGCCATGGAAACAGATGGAGCACAGAGGGAGGGATCAGTCAACCTGGCTTC
TGTGGAAGGGGAGCAGGTGTTAGCAAGAGCAGAGTGGCCTTAGCAGGGCTCTGTCTGGGCTCCTACAATCTTGGACATGGAATTAGA
AAGCTTGAGGTTAGTTGGGCCTCTGATCTGACACTCAGGATAGGCAGACCCTGTGTGTCTGGGGCCAGGCTACACAGTGAGTTACAG
GAGAGAGCTTGAGGTTAGAGCCTAGCTCCTTCACCACCCGAGTAGCCTTGGGAAAGCTTATGAACAAGCTCTCTACACTTGTTTTCT
CCATTTTCAAGTGGCAACACAAATTCCATTAAAAAAGAAAAAGAAAGAAAGAAACAGCCGGGCGGTGGTGGCACACGCCTTTAATCC
CAGCACTTGGGAGGCAGAGGCAGGTGGATTTCTGAGTTCAAGGCCAGCCTGGTCTCTATAGAGTGAGTTCCAGGACAGCCAGGACTA
CACAGAGAAACCCTGTCTCAAAAAACAAAACAAAACAAAAAATAAAATAAATAAAAAGACAACCAGCTCCTCTGGGTGGTGGTGGCG
CACACCTTTAATCTCAACACCCAGGAGGCAGAGGCAGATGTATCTCTGTGAGTTTGAGGCCAGTCTGGTCTATAGAGTAAGTTCTAG
GACAGCCAGGGCTACATAGAGAAACCCTGTCTCCAAAAACCAAAGCCCAAACCAACCAACCAACTGACCGACCAACCAAACAAACGA
AAAAACAAAACCCCAAGAAACAAACCAGCTAGCCCCAGGATCTGCTGGGCAGCAGTTCCTCAGGAGATTCTTGAGTGTTTAAGCTTG
GAAGGTTCCCAGGAGCAACCTCCACGGGGCCCCCAAAGCTGTGGACAATCTACTGTTGCTAGACAGGGACACGCTCACATTGTGGTT
TCTTTTAGGGTCGTTTCATACCCATTTGCCGCGCCACATTTAGTGCGCATGCGCACTGATGTGTAGGCCACAGCTTCCAAAGCACAG
ACTCTTCACCCTCGTTGTATTTCTGGACCGTGGAATTGAGACAGTCTCTGTTCTTCCAGCCTAGACAGCTTTGTTGACCCTTGTTTC
ACTAGTGTACATGTCGGGACCCAGTTCCCTTCATAGCAGATTCTTGAATGTCCCAGAGCGTGTTCCTTGGATGTCTGGGGAATTCGTT
CTTAAAGCCGATCATGGATGCACTCACGAATGTGGGCGATGTCTGCTTGGTTCATCACCGTGATTAAAGATCAGGAACGGCTTGTCA
CCGTTCTTTGGGAAAGCCATATTTTCAGGTCCCAGCTGGAAAAGAAGCGTGCAGGGGATTGTAGGGCGGAAATCCGCTGCCTGCAAC
TCCCTGATCTCCAAAGAGGCGGGTGACCGACCATGTGACCTGTAGCTAGCCTTCTGGGCCCTCAGATAAGCTTGCTTGATCTGGTTG
GGTGACATGGCTCCCGCAGTGCCCCATTGCCGGCAGTCATGGCAGTCAAAGCAAAAGGTAGGCAGGCTGGCAACAGTTTGGTGCCAG
AGGACTAGAGAAGGTAGCATGAGTTTATAGAGTTTATAAAGGTACCATGTTCAAGCCTATTAATAGCCCATGTTTGGCCTCCGCTGC
TCTGTCAACATCATCTATCACATTCAAACAATTGAAGAGCCCATGCCAAATGGTTAGGAGTTAACAACTCACGGCTGGGCTGAGTTT
CTGAAAACAGAATTTCCACTTCTTATGCAGACTGAAATGCCAATTGTTCCACGCCGACTGGTTATTTTTCCAGACCATTTCCCTTTA
TTTATGTTTAAGCGGTTGTGTTTTTAAGTGAAAGGAAGATGGAGAACCTGGAGTAGGTAATTGTTATTTCTCTTGATTTGCCCTTGA
ATGAGCCATCAGAGAAATAGTCCTTTTCCAACCTTGTGATGAGAACACTCAGAAGCTGGGAGGAGTCAGACAGGACCCTGCCCTTTA
GGGGGCGTGTTCAAGATAGATGCAGGAAGGACGGGGCAGGGAGAAATGGCAGCCCAAGCTTTGGGAGAAGGGGACTCTTGTCCCTT
TCTGTTTTGAATGTGACCTTGGGTGGGAGGGAGCTCTTTTGGGGCCTCAGTCTCCTTATCTGTAAAATAGGGAAGCTGGCTTAGCGG
ATCTGAGGCCAGTGGCTGATGGCAGGTGTGTGCTCTGCAGTACTTTTAGCACTACAAGACTCACGAAATTTCTGACTGGCTGAGGA
AACTCACTGGCCACAGCAGAGGAAGGAGAAGGACCAGGAGGAATAGATTGACTGCTGGAGAAAGCAAACGACTAGGTGACAGGTCTG
GAGGTGGTTTCTCTGACAGGGATTCAGGCTTTTGCAGAGCGGTTGAATTTAAGAATGGCTGGATGGACCACCTGATGAGCCTGGCAG
CTCAAACCTGGATTTGAAGGTTGCCCTAACACAGGCTGATTTCTGCTGGGGACAAACCAGCCATCCTGTCCTCTGTGTCCTTACTTC
CAGACCTGGCATGACTGTGTCCTGAGAGGGGAACTGTCATAGACCCCTATGAGGCAATGGTCGGAGCATCAGCTAAGCACTACACC
TAAGAAAACTGAGATCAGATTTCTGAGAAAGTTGGGGCCTTCCCAGGTGTCTCCCAAAGGCTGTCTGGAGAGGGAAGTGAGCCCTCAG
TCTCTGAAACCATCAGTCAGACAAAGCCTAGATGACAACTGCAGCAACTCTCACACAGGGAGCAGGGGGGGATTTGGCTGTGCGGCTT
CAATTTATTTATTTATTTATTTATTTATTTATTTATTTATTTATTTACTTACTTACTTACTTTATTTACATTTCAAATGTTATCCATTTTC
CTGGTTTCCCCTCCGAAAGTCCCCTATCCCATCCTCTCTACCCCTGCTCACCAACCCACCCACTCCCGCTTCCCTGTCCTGGCATTC
```

```
CCCTACACTGGGGCATTGAGCCTTCATAGGACCAAGGGCCTCTCCTCCCATTGATGTCTGACAATGCCCATCCTCTGCTACATATGT
GGCTGGAGCCATGAGTCCCACCATGTTGTCTCTTTGGTTGGTGGTTTAGTCCCTGGGAGCTCTGGGGGGTTCTGGTTGGTTGAAATTG
TTGTTCTTCAACCCCTTCAGCCCCCTTCCTTCCTTTCTCTAGCTCCTCCATTGGGACCCTGTGCTCAGTCCAATGGTTGGCTTCGAG
CATCTGCCTCTGTATTTGTCAGGCTCTGGCAGAGCTTCTCAGGAGACAGCTATATCAGGCTCCTGTCTGCAAGCACTTGTCGGCATC
CACTATAGTGTCTAGGTTTGGTAACTGTATATGGGATGGATCCCCAAGTGGGGTAGTCTCTGGATGGCCTTTCCTTCAATCTCTGCT
CCACACTTTGTCTCTGTATCTCCTCCCATGGGAGATGCCACCAATGTCAAGATTCAGGAACTTCATTATTTATGGGTACGACCATGT
GTCTGGTGATATCTCCGTGATGATGTTCATTCCTTCCTCCTGGGGGTTCTTTCTTAAACCCCAAAATAGAAGCTCTCACCTCTAACC
TGTATATCATAATTGGAGAACTTTTTAAAAAGTCGGCAGAGATTCTGAGTTCATTGTTTTGGGTGGGGCCAAGGGCACTTAATCCTA
GGACCTGGGTGATTGTACCTGGTGGAGTTGCCACCCACATCCAGACTCACTGTCGGGATGAAGAGACACAGCTTAGCACGATGGAGC
TCATTTGCTTGTGTTTATCTCGTGTCCGGAAGGAGCCTTGGTGGCTCCCTTTGCAGCAGCAATCGAGCTTTAGGAAAGCCCTAACCT
GTTGTGTCTGCCTCCCACACGACACAGTGCACAACACATGGCACGCACACAATACTAGTTACAACCCCGACTGATGGTCGGCGATCT
AAGTGTGCCAGGAGTTACATGACAAGCAGCACGCTGCAGAGATTTCCTGCTTTGACAGCTCCTTTCTGCCGAGACCTTGTAAAGGAA
ATCATCTCCTTAATCAGAATTGTGTTTGCAGCCGAAGGGGCAACTCATGAGACTTGATATTTGGAGAGCTTGGCTCCAAAGTGACC
TTCTCTTGCTTTCCTTGGGGTACCACAGTGGGAGAGTCAGGCCTGGGGAGGATCGCTGCCTCTTAATTGATGTTCTGGCCATCATGC
TGGCTCCACCAGACAATGCTTGGCCACCCTCTGCTCAGAGCTGAATGTCCTTTCTATTTTCACTACATGATGTCCCTCAGACATCAG
GTCCTATGTCCTTCTGGAAGTGACAAAGTAGAAGGCCACAGAGAAATGCCCTAGGCCAAACTGATTTTATGTACTTTAGTCCAAGAG
GCTGTCTTCCTTAACTCGCTAGCATTTCCTGTCTTTCCTCCCCCATGCCAGCCTAGGCCCGGGCTAGACTTGCAGGAACATCCCAGA
CCGCGAGCCCTGACCTCAAGCTGTCATTGTTAACATGCCTCAAAAGCACACAACTGGGACCTGGCTGTGAGATTTCCCTCCAGACAC
TATGCTATGAGAGCACAAGTCTTAAGAGTCAGGCCTGACTGCCCAGGTTCAAATCTTACCCCATTCCTTCCCTGTCTAAGGACTGTG
GGTGAGAGACAAGCTTCTTCTCAGCTTCAGAACCCTCGCTGTAGTGTGGGACCACATAGCAAGCCAGTATGGGACCCATGGCAAGTG
ACCTTTTGATCACCCTTGCCCCCTGCTTCTTGAATTTGCAAATCCAGGACAGAAGTGTCTACCTGTTGGCACATCTCTAAATTTTGT
TCCCTTCTCTGAAATAGTATTCAGCATTCAGCTGATATTTATGGACTACTAAGCACTGGACAGCATTTCACATGTGATGACATAGG
AATGAATTTATTAAGAGATGCTACCCTCTATGGCTGATACTCTGGCTGGGACAAGCAGTGGTGAGGGGTAGTACTGACTGGGAAGAGA
TTCCTGTGCAGCCCCAGGGAGTGTGGGACCAGATCAGGTCCCTGGGATCATCTTAGGCAGGTAATGCTCCCAGCTGAGAAGATGAGT
GAGTCAGGAGGTAGAGAAGGTGGTTGAAGAGGGCACAGCAAGGAGGGTTCCAGGAAGCTCTCTGCCCACGATTAAGGTCAGACCCCC
CCCCCTCCTAAGCTGGAAAGCCTGCACATAATGGCCCCGCTGAGCCTAGGACTGAGAGGGAGAGAGTGTTCCATTGAGGCCTTCTGC
CAGGGCACTATGCCTCAGATGCACCCCAGGAGTGGGAAAGCTCAGAACAGATGTGCTACATATAAAGGAAGAGAGCCACATGCACGG
GCCACTGTGAGGTTGAAGCCCCTCTGTGGCCACTGGAATCTTAGACAAATGGTCTCAGGAAAGGTGTCTGAGGCTTAGAGCCTAATC
AGGCTGACCCACACCCTCAGAGGTTACACCCTGGAGGAAGGGAATGCCCTCTAAAATCTCATTGCCTATGGATAAGGCTGCGGGCTG
CAGCTCATTCACGCCCAAGATGGAGGCGAGGGCTGTTCAGACACCTGCTAGTGCCACCTCTCCTGATTCTAGTCACTCCCTGATGAC
TAAGGCCCAAAGGAACCAGGTCTTCCTCCAATTGGAGCTTCCTGCCCTCCAGACCCCCCCTGCTAGTTGCCTCTGTCTCTTCTGACA
TTTGGCTGTGGGCGGGACGAGTGTGGAACACGATGGAACAGGGCTCAGCTGGTGGGGCAGGATTCAGTCTCAGTATACTGTGACAACA
GTGTTCTGAGGCTCAAGGCAGGGCTCAGCCCAGAGTTTGGGCGAAGCAAATGCTCAGTAGTCTGTCTGGTTGAGTGAAGGAGCGTGT
ATGTGGCCTAGTGACAACCCAAGGCATCTCTGAAGTACCAAGGACAGGGGTGCTGATAGTGGAGCTTGTCACTGGGACCGATTCGTT
TTTCTCTGCTCTGGGTGGCAAGAGGAAGTGCATTTGACTTAAAAAATATTGTGGAAAGATAACCTTTTCTTTTTTCACTTGTATCTC
GTGTATGTGAAGGAGGAGCAGAGGAACTGGGAGACCCTGGAGGCTTCTCTGTCCCGAAAGAGAGTCTGGGAAGCCCAGTGTCTCAAAGGCTT
GACTTGAACCCTAGTCTTTTGTTGTCAAGTTCTATGTTATAGAAAGACACAATAAAAACCTTCTCATTAGCTCCCACACTAACAACA
GAAACCACTCTTTGCTCTGTTGAGAAGACAGAATGAATGGGATCTGGAGCTGGCCGTTTCAGACCCACAACTTAATTCCTTACAAAA
GAAATGTTTACAAAAGGAGTGTTCCTATGACCCGTATGTGTCCTGGCCTATAGACTGATGATATAGGCCATCGAGCACACATAGCTA
AATGTCAGGGTTTTGAATTCCAGAACCTTCTTTCCTTGGAGGTCTGAGGTATGCACAAGGCCAGGCATTTAACTTGCTGTAGCAGTG
ACTACTGGTCACTCTACAGCGAGTCTAGAGCTGACTTCCAAGCCCTCTTCCTGATGAAGTCCCCAGGGTCTTAGGTTCTGTTTCTCA
GATCTGTGCAATGCCCAGCGACCATCCCTACCCACTGTGATGGTGGCATCAGATTCCCCTGCTCTCAGGAGGGCAGGATCGAGTGGG
CTGAGCCTTCCCATGATTCCATTCTGGACCATGAATACCTACAATGGGAGGGGACATCATGCCAAGGATGCTGGTAGGCCCAGCATG
ACCTCAGGTGATTTCATGACCCTGACGACCTCAGGCAGCTACACCCAGGAGGAAGACCAGCTAAGGTCTGATCGGGCAAGGTAGTGA
AAGTTCAGAAGGCAAAGTCAGGAGAAAGGTGCAGATGGGAGCCAGGGAAAAGGAAGGCTAGGCTAGGTGCAGAACCAAAGCCCAGGT
AGGGAAGAGAAGCCGGGTGTCCCTGGAGATCAGCAGCCAGGAAGCGCTGACTTCCCTGCCAGGGGACTGGGGGTGGGGAATCAGGGAA
GGAAGGCCTGGCTTAATCTGCATAGCATTAATTAGCAAGAGTGTCTAATTAGGACAACCATTACAGACGCTACAGTACGAAGCCAGTG
CTGAATATCAATCAGTCTTGGAGCGGTTCCATGAAATTGAAGCAAAGGTTGACCTTATTAAACAGAAAGATTTCTAACTCAATTAT
TTCCCCAAGTCGAATGCACCAACCGAGGTGAAGTCAGGAAGGCGCATCAGACCTTAACTCGGGAAATACAGGTTCTGGATGCTATCA
CCTTTCTCCCTTGAGGGCTTCTAACGAGTGACAGGAGTCATGAATGGGCCATGTGCGTGCACTGCGTGCTCACATGCACAGCCCTGG
AGCTCATTACCTTGGCTGGGGGAGGAGCCTGCGCATCTTGTGCTGGGGAAGCCTTATGTTGACTTTGGCTGGTGCCCTTACCTGACT
CCGTGCATCATCTTCTGCCTGCCCCCCCCTTCTCAATGTTCCAAACAATAAAACACATTAAAAACTTCAAAGTCTCTGGGAAGATCTA
CTGTAATCATTACAATTCTGGCCTGTCTCTGGAAGCTACCAGGCAGGCCAGGAGAGCAATTTATACCGCTGGCTCAGAGGCCTGGGA
ACCACTCCATCACCCTGTGCACAGCCCCAGAAAGGGACCCAGCAGCCGTGAGCAGCTTCTCTTTGAATCTGGAGGCCAGTGGGCTCA
TTTTTTGGCTATGCCCTTGACAAGTTAGGCTTTCTTGGAGCCTCCTCAGTTTCTCTGTCCCCGAAAGAGAGTCCATGATGCCTCCCTTGC
ATGGTAGGTGCCTGTAGCAGCAGCAGCAGCAGCAGCAGCAATGCTTTGTTCTAGATTACTATCAGTGGGTAATCACAGACCCGA
GAAGGGCTGCCTCAGGAGCAGGACAGGGACCACACTGTAGGAACATCTCCAGGAATGTGTCTTGGTCTCCTACCCAGCCATTCGTCT
CTTGTGCAGCCCAAGGCTCCACTGAGGTAACAAGATACACAGTACCTGGGACACTGAAGGGCAGGGTAAGTAAAATCTGGCAAACTA
AAATCCGAGTTCGTCGCATGACCTTGGAGAAGTCACTTTAACAACTCTCCAACTAGTATCGTTTTATCAGTAAAATGGAAACATTAG
GTTCTACCAGAGGTGAGATAAAATGCGTCTAGGTGTGGTGGCTGGAGCACATGATCAGAGTTCAAAGAAGATTAGCTATAAAG
TGCTTACTAAGCGCCTCTTAGATTCAGGGCATCAAATAGGTGAGGGGACCATATAGTATAAGTGTGTTATGTTCTGGTCCTCTGCAA
AAACAGGTTGCACTCTTAACGGCTGGATCACCTCTCTGGTACTCTATCGGGAGTCGTTTTTATATTTGTCTAGTCCTGTGAATCCAT
GGAGCAATAAGACTGGTCTGAAGCGGGTGCGAGTCCCTAAGCCCAGCCCAGGGACTCAGAGGCCGAAGCTCCCAGCTGGTGTCAGTT
CGAATTTCCCTGCTCAGGCTGAGAATAGTTACAATTCCAGCCTGACACTAGGAAACGGGCGTGAGTAAGTGAAACGGAGTCTCAAAG
CCCAGAGAAGCTTCGGTGGTTCATCCCCATTGCAGGGATGGACCTGAGGCTTGCATGGGCTCCTGCTGTCACCCGTGGGTGGTAGAG
TGCATGTGACACGAAAAGCTCGATCTCTCCACTGCACAAAAGCCTCTTACTGGCAGCGCAGGGCTCAGATTTTGAAAAATAAACTTG
TCAATAACAAAATGCTATGTCCTTATTAATAATTTGAGGAGAGAAGGAACATGGTAAGAAAATGCCCTACAATGCTGAATATTGATA
TTTTCCACAATCTTTTTCTACATGGGAAATTTCAAAAACAATCAGAACCAGAACGGTTTGGAGTTCAAAGCTTTCCAGTTAATATTC
CGTCACAATTTCTCTCCATGTCTTCAGATACTCTCTGTCTTTAGGGATGTACTAGTGTCCATCATTGGGTGTCCTTAGATGGTCTGA
GAGTGCAACCTGTTTTTGCAGGAGACCAGAATGTGGCTCCCAGCAGCCACATTGGGAGATTTACAACTGCCTGTAACTCTAGCCGGG
GATCTGACTCCCTCTTCCAGACTCCGTGGGTGCTGCACTCACATGCACCCCTTCTTACATTCAGATTCTAAGACTAAAAGTAAAATCT
TCACATATAAAAATGCCCCTGTGTATTCTTCGTGCTGGGGATTATTTCTTTGTTTTTAAAAATGCAAGGGAAAAGGGTTGATCAGTT
GGTCATGGGACTCCTCCCAAGCCATCGAGTCAATTTTAAGGCCCCAGATGGAGCAGGTTGGGGCGCAGCAGGTATGGCAGGTGCCCC
ACCATGTGGTGTTTGGTGACTAATTAGTCAGCCTGTGTCATTGGTGTCACCCGGAGAGTCATGTCAGTGGAGTTCAGGTGGAAAAGA
```

```
AGATAATTCAACATTTTAGCGAATAGAAAAATGTTCTAGATCCAGTTAGACTTCCTCGACTTATTTCCCAGGGGATGAGATCTCAAG
GCTGTATGTAACTCTCCATTAGTCCAGTGTAGCTGTGTCTCTCTGCAGGCTGCTGCTGGCCCAGCAGGCTGCACCAGGCAAAACCAC
TGAGCAGCTGGGAGCTGCCTTTTAATAGAAAAGCAACAACTTTGAGAAGAAATGGAAGAGGTTTAAAGGAGAGGAGACAGCGTCTGG
CCCCCATCCTTTATCTCTTTCCCTGAAAATGATGGAAACACACTTAGAGGCTGACTTTAGCTAATTTCTGCCAAGAACATGAAATGG
AGGTGGAAGTGTGCCACTGGTCACAAGCCATCCTGGAGTGGGCGTGATGCTCCCTACATGCCTCTGGGCCCCTCTGACAAGGACCTT
ATGCTCACTTCTGGCCGGAAAGCACTGTAAGCTGTCCCAGTTCTTTCCTGTAGAGAAATTCATCTGGTCCCACATCAACCTTGTGTC
CCTGAGCTTCCAGCCTCCCCCCACCCTCTTTTTTTTTTTCAGCCCCAGCTAGGTCTGGGATCAGCATCAAGTAAGCTTTCAAAGGTTGG
AACCTTCTGGAGGGTCAGAACTGTGTTCTATTTTCCTTGTTGCCCACATTGCATCAGAAAAGCACCAGGACTCCCCGAGCACCCTT
ATATACGTGAGCTCAAGGAGGTACTCTGGGTACTTAGCAATTCTGAAGGTCATGGAAGATGCTAAAATAGGCTCAGTGCTGGGAACG
GAACCTGGGACCTGTGTGGCTAGTACTCCTCTACCTCTGAGCTACACTACTACTGAGTTATACCCCTAGCCTTTGTCCGATTTTGGG
TAGTACAGAATCTACACATTCCTCCACAGCTAGATCATATAATGCAAGTAAGTGAGAGTTTGTGCAAGAAGACAAACTGAAAATCTC
TGGTGGGCGGGGCCCTCCTGGTGGGCGGGGCCGCTCTGTCTGTCCCCCAGCATGGACCACATGGTAGTGTATCCAATAGGTACTCAC
TCAGCAAGCAAGAACTGGTTGAAATCGTTGTAAAAATTAAATGAATTATAACTTGGGATGCTGAGGTAGGAAGATGGTGAGTTTATA
TAACCAGACCTTGTCTCAGAAAACAACAAAATGGGGTCTTGGGGAGATGGGGTCGCAGGTGAAAGCAGGTGCCACTGAAGCCCGATG
ATCTGAGTTCAGTTCCTGGACCTCACATGAAGGTGGAAGGGGAGAACTGACTTGCAAAGGTTGTCCGTTGGCTTCCACGTGAGTGCT
GTAGTGCATGCACATGCCTGCTGTTACCTGTGCACATCAACAGATGATACAAACCCAAACACACAAGGAACCAAACTGCGAGTGAAT
GAACGCATGAGTTATTTCACTGGTTCTCCCTTATGGCTCCAGGGCTAATTTGTGCCTGGCATAAAAATTGGTCTCAATAGCCATATCA
AAGAAATGAATGCTGGAAGGGCCAGACACACCAGAGAGAGAGAGAGAGAGAGAGCAGCTCCCGTTCACAGTGGGTGGGGGAGAGT
GTCAGCATGGGCACCCGCAAGTCCTGGATTCAAGGATCAAGCTGTCCCCTTAACAGTAGCTGCCATATTGAACCATATGGCTCTGCA
GGTCCCAAATTCCTTTCACCTCTGCCTCAATTCTCCCATAGTATCATTTGGCAATTAATCATGTCTTGCTGTTGAAATCTCTTCCAT
TGTTATCTTAGTTGTTTTGGCTTGAGACATAGTATTTGTACCCCTTCATGGAAGTTTCATTAGTGGCTTGGAAACGTGCATTGGTGT
GTAATTATCATATCACCTTGATCAGCAACCCCCATCACCTCAAACTTTGTGCAGTTCTATGTGGTAAGAACCTTGGTCCTTTTCTAG
TTGTTTTTGCAATGCACCATAGTTGCTTATAACCATCGCTGCTCTAGAGAGAAAACTAGAGTTATAGTCTTTATCGCCTGATTGGGT
TTTGGTCTCTTGTCTTGATCTGCCATTTGGGTCTTGTCAGGAGCATTCCTGTCTCCTGGTGGAGAGTTGGCCCAGGGCAATGAGCAG
TGATCTGGCCTGGGGGTCTGAGGCCCTGGCTCTGAACTCTGGTGTAATATATTCACCCCCTCCCCCATGGCATTCTATACAGGTGGT
GGGGGGGAGCAGAAAATTGGGCATTATTGATTAGCATTGTTTCTTTGGTCTTCCTTGGCTCTCAGACCATGTAGGAGCCCACTTGGT
GGTCCTTGGAAATGCTGCCTGACCTCCCAACGGCCTTTGCTCAGGGCTCTTCCTGTGTTCCTTTATGCTGGAGATAGCTCTCCTTA
CAGACTCACCCCACTCAGGAGTGTGGCAAGCCCTAACTAGATGCTCTGATGGAAAGGGGCGAACAGTACTGACAGCCAGCACTCACT
GAGCCAAGCTGCTTCAGGTACCATTGATGGTCATGCAGCTCTGAGTGGCTGGGGCAGGTTCTGGACTCAGCAGACAGCCAAAGAGCT
TACGCACAGACTCTAGGCTCTCTAAGCCATCTGGGCTGAAGTTGCAGGGAGCATCCTCTGCCCTGAGCAGCAGGCCACCACAACCTT
CCCCTGCCCTGGACCTCTTTCTGCTCCTTTGCTGGCCCCTCATCCTCCAGTGATTCTTCTGCTGTTTCTGTCTCCCCTCTCCTGCCCTTGG
CTTGAGCCAGGGGACAGAGCAGCGACCAGGACATTCTGATTTATTTCTGATGGAGCTCTTCATGGCCAGAGACAAAAGCGACCTCAA
AATCTTCTGGATTGTGTTACGATAGAAACACAAGCTGTCATGGTACGGACACCCAAACCATATAATAGTGGCACAAAAAATAATTAC
GCCCATGCGACTTATGAGCGTGGAGGCAAGTATTCAACACAGCAGTAACTAGACTCTAGCCAGGTATCACATACATAAAACCTTGTG
CATAGTGGGGTCATTCTGGAAAAGCAAGGATGTTTTCTCCTTGAAAGCAAAGGCTCACAAGGGGCAGCCTGGGCTCAGGCTAACTGC
TCTACCCTGCTCCTAGGAGGAACTGGGGATGGTTTGTCCTCTGCCTGCTGTTTCCTGTTATACCTCTTCCCATGTGCTGTTTCCCGC
CTGGCTCCCTAACTCTCTTTCCCTCTCTCCTGCAGGATTCCATGTTTTCGTTGGCCCTGAAATGCCTTATCAGTTTATCCACCGTCA
TCCTGCTTGGTTTGATCATCGCCTATCACACAAGGGAAGTACAGGTATGTCACTCCCTGACTTCTAGTAGCAGCCTATCCTGCTGCC
TTGGTCTGGACTGAACCCACCCATCCCCAACCCAACTTGAGTGGCCCCACAAGATGGTGTTTGAAATCTTCTCTTTGGGTCCTCACG
GAGGGTGTCTTGAGGCTACCCTGTGACCCTCCTCTCGGGTGATCTCATAAATAGGCATAAGGCTTCATGAATGGTGGCTGTGTGGGG
GTGCACACATATAGACACACACACACACACACACACACACACACACAGTGCCTCAAACACCAGCAGGGAGCCCTGCAAGT
TTTGAATGTGGAGACTCCTCCTCTGAAGCCACCAGGCTGGGATGTGCCTGTCATTTTCTAGCAAGAGAAGATGTAATGTGTGATGTG
GTTGAGGTAAGGCCTCTATTTCTGGCTGCCTAGGGTTGGTTCAGTACTCTGGGAAATGTTTCCTGGACATCTGGGTATTCACCCAGC
TTGGCTGGTTATATAGGGAGGTTGGTTCAACTTAAACTGTGAGACTGAGGCAGGATTGAGTGGACCCCGGCATCCTCTTGTGGCAGT
GAGTTGTCACCCTTCCCCCACCCCCGGGGCCTGCAGACTGATCTGCTCACTGCTGACCCAGGTCATCCAAGATATCCAGATGTTTCT
GATCATGTGTGTGTGAATCTGGATACTCCCCCCGCCCCCGCCCCCCATCTTCTGGTCAGGAGGCATGGACAGAATTACATCTGGGC
AGCAGGAAGAGACTGACGAAGACCAGAACTCAGGAGTCAGAAGACTCGGGGTCAAGTCTTGGCCAGGACTTCTTCTTACTGTGTGTT
TTTGGCTGGTCCTGTGACCTTTCTGGGTCTCAGCCTCCTCACCTGGAACAAGGAAGCTTTATTCAACTTTGCTGGGCTCCTGTGAGG
GTAGAGTAAAAAGGTTGTGTGTATGTGTAAATGCTAGCTGGCATGCTGGTAAGCTTAGGTCCATTTGTCCTCATTGTCCTGACACAG
AGATATCATGGGGAGCCTCCCTGTCCCTGGGTCCAGCTTCTAGCCAAGTTCTGGAGTTCCTGTGGCTGGATGGGATGTGGTGGTTTT
TACTGTAGGTCTTCTTCTGGCCCATAGGACTCTCAGTATGTCAGTAGGACACCCATTTAGAGCGTGGGACTCTAGTGTTTTGCA
GTCTTGTGTACTTAGATTGGGCTATACTTTGAGGGCCGAGTACAGAGGTTACTAGGCAACTTTTTTCATAGCGTTCTAACTGGGCAA
AGCTTCTGGACTCAAACCCAGACCACCGTCCAGCAAATCTCTGAAAGGAGCCAGTAGTATAAGTGACGATGACCAAGGGACCATGCC
AGGATGGAACCTTCAGCATTGGCTTCATCCCTACCTGGACCCAGCTGGACGGGGTTCTGAGGCTCTTCAACCTCTGGGTGCCCTCAT
GTTTTCATCTGAGATGGGCTGTGTGGGAAGTTGTCATAGAGGCCAGGTTGAAAAGAAGAGTCAAAACTCAGCTGGACCTGGATTGG
GAGGAAGGGCAGCGGGCAGTTTCTAGCACACGGAGGGCCTCCCAGACACCTCTCCTTTCTGCTCCAGACATTGTGAGGAAGGAGCAA
GGTCTCGGATTCTCTTAGGGTCAGGCCTCTTTCCTACTTCAGGTCCACAGACCTGACCTAGTTCAACTGCAAAGCCTCTGGTCTTGG
GGGACAGAGCCATGCTGCTGGCAGACCCTAGTTCACCCTCAGCTTAGGACCAGTCTGTTTCAGCCCTAACCACGCTCTCTGGGTAAC
TTACATTGTTGGCCCAGCACTCACAGTTTTAGAGAAACAAGGTGAAATGGGTGGCTTTGGTCTTTTTAATTAATATTATTTATTACT
GATATTGATTTAATTATATAATTGTTGTTATGTGTTGTTCTGGGATGGAGCACAGGCTCTGGGCTTATCTGGCCAGTGTTCTGCTTC
TGAGCTACACATCAGACACAAAGTAGTGGCTTTGACTCCTACCTCAGTCACTGCAGGCTGGATGACCTTGGATGATAATAAATCCTCA
GTTTCCCACCCAAAGAATGAGAGTCCCACCATCCATTCTGGTGAATTCTTAAGGGTGTGGGAATGGTACCGGGAACAGTATGGTTTC
TAGAAATGGAAACCTTACTCTCTTTCCTGTTCCACACACAGCTGTCCTCTAACTTCTAGCTGCATTTTGGAATATGCCAGGTGAGCC
AGCCTCATGGCCACTAGCACTGCTGCCCAGAACTGCAAGTGTCTGGCTCTCCTCCTGCTGTAGCTAGGCTCGGTTTCCATGACGTAG
ACCCTCATTGTGCTGTACCCACTGACTCCCAGGGGAAGTGACAGTGTCTTCACCTTAACACTCGCATTCTCCCTACAGCTGTCTGGG
CTGTCTCTTGTCTCCTTGAAGGGAATCGGAATGGTTTAGCCCCAGAGAGCCAGCAAGAGAGGTGTTCCCATCTGCAATCACCTGGCC
AGACTACAGCTTTCTGCATGATGGCTTTCTCCATCACTTCAGAAACAAAGAAACACAGATGTCGTTAATATTTGCATTTCCAAAAAT
ATACAATGCCTGACAGAGAGATGGGATAGAAGGTACTGGACTAAAGAACTCTTGTTTCTAAAACTAGTGACATCATGATTTTGCGTG
TGGTCCTGAGCCTCAGTTTCCTTGTCTAATTATTAGGGTCCTGGTTTTAAGTTATCCAGTCTAGGTGTGGCTGCTCATCTTTCAGGG
TCCTGGTTACTGAGTGTTTAGTGTGACTAACAGAGAGTTTCCAGGGGGAAGCAATGGAAATGCCATCCTCAGGGCAGGTCAGCCACT
GCTGGGGTGACAGGGTGGGAAGGCTGTGGCTGTGCCATCTCCTAAGGCCACCTGCCTAGCACAGGGCCTGCTGGGCTCTCTGAGTG
CTACAGTAACGGCCTGGGGAGGGTCACCTACCAGTGACAGCATGCATTTAGAACACACCCCATTCTGCAGAGAGGGAACTTGCTCTCA
GCCCCACTCCTGTCTCTGGAGCATCAAGGACGAGCCAGGTTGCCATGTGGTCACTTCTGTTGTTGAAGATGCTCACCCCGCCTCTC
CTCAGCCTTCATCTTTTGTGGCATTTTTAGAGTGGAGCTTTGAAAGATTCAGGTACCTTTTATCCCCAGGCCAGTGTATTCTGAGAA
GAAAGGAAAATGCAAAAGCTATGCAATTTGGACCTTCGCTAAAATGTAGCGGGAACCCATACTTGTTCATAGACAATGCACTCCTGT
GTCATTATGTTTCAGATGCCCTTGGTGCACCCCTGCTTAATGCAGAAGGCACTGGGCTTGGCTGCCCTCCTGATAGTTTGCTGTCTC
```

EP 2 204 376 A2

```
TTCTCCACTGCCCATCCAGCTCCATTATTTATTCAGCAGGGGTTTAGTGAGGTCACTCTGTGCACAGCCAGTGAGGAGCTCTTATCC
CTAGAAACTCTGACATCACCATCGCTTCTCTGAGGGGTTGCCAGCCTCAACTGTGTCTGATGCTGTCCTTGAAAGCAGGTTTTCTTT
TTCTTTCTTTCCTTCTTTCTTTTTTTAAAAAAAAGATTATTGTTTCTATGTATATTGTTTATTTATTGTTATATGTAAGTACACTGT
AGCGGTCTTCAGACACACCAGAAGAGGGCGTCAGATCTCATTACAGATGGTTGTAAGCCACCATGTGGTTGCTGGGATTTGAACTCA
GGAGAAAGCAGGTTTCTGATAACATGGATGTCAGAGCAGCCCAGCTTGGGTAGAGGCCAGGCTCTTTCACCTTTTAGGGTAAAGATA
TATGGCAGGTGACCTTTAGGGTTTTTTGTTGTTGTTGTTTTTAAACTCTCCTTTACTCTATTTCCTTCTTGAAAAATCAGATTAGTGG
TAGTCCCTACCACAAGGACTGTTGTGAAGAACAAATTTGGGCATTACGAAAATGCTTCAGCTCAGATAAGGGCCTATATGTCTTGCC
CACAGCCATGGGAAGGCTGTTTGTCTCAGTCACTGTTCTATGGCTGTGAAGAGACACCAAGACCAAGGCAACTCTTATAAAAGCATTC
AAATGGGGTCTTGCTTACAGTTTCAGAGGCTTATATACTCCATTATCATCAGGGTGGGGAGCTCGGCAGCTCACAAGGCACTGGAGC
AGTAGCTGATCTGAGGGTGTGTGTGAAAGAGAGAGAGAGAGGGAGAGAGAGAGAGAGAGAGAGAGAGATGCCTTGCATGGGCTTT
TGAAAACTCAAAGCCCATGCTAGATGACAAACTTCCTCCAACAACTCACTCCATCAAGGCCATACCTTAGACAAGGCCACACCCACT
CCAACAAGGCCACACCTACTCTAACAAGGCCACACCTCTTAGTTCTTCTTAAATAGTGCCTCTTCCTGATGACTAAGCATTCAAATA
TATGGGCTTATGGGGGGCATTCTTATTCAAACTCCACAATATCCCAAATAGAAGTCATAGAATAATCAGAAGCCCAGGAGTGGAAAC
TTCAGTGTCTTGTTCAGAATGTCTCCAGGCTAAGGTCTCAGACAGTGCTGGGGGGCGGACAGTGATCTGGGGGTGGGGTGGGGTGGT
GGTGCTCCATTGTGGAAGGTGGGCAGGCCCAGTGGCACCTGTTTGTCCTGCTAAGGAGCTTTGAAAGGCAGTAGCAGGAATCACAGA
AGACTGCCAAGCCGGAGGACATTTACATTTTAGACATGCTCTCTGCGAGTCTTAGAAGAGTCAATTTTAGAGGCAGGAAGATA
GTTAGAAGCCGATTACAACATCCAGGTGAAGGACAGTGATGCCTGACCAGGATGAGTACAGGAATGGGGACAGAGGAGGGAACATGT
GTTAGAGAGGCTGAGAGAATGGAAACGACAAGACTTGGTTACCAATTAGGGTGGTTACTTTCTTGGCAGGGAGAGAGAAGTCTCCCA
ATTGCTTTGGATAACTCAGTGCTGGAGAATGCCATTCATTAAAACTGAAAACACAGAAGGAGGTCAAGGTGGGACGGGTGTTGGGTT
CTACTCTGGCTTGGGGGGGGGGAGCTCTGGCTGCCTCCAGCGTGGGAATGGGAGCTAATGATGTCGAGTAGTTAGGGATGGGTGTGC
AGGTGCAGTGAGATAAAAGAAGGGCGCAGGGCAGGACCCCAGGACAGCATTGACAGGTGATAAGTAAGTGGGGGAGGAGCTGCTAG
GAGACTGATAAAGATTACAAAGAGGGAGGGACAGGAAGCAGCCTTTGCATCTGCAGTGTGGAGGGCAGGGCTGAAGAACACACTGTCC
TCGCCCTAACTTATTCCTTGTCATGACAGTCACTGGGAATGGCAGTGTAGGTGAGGGGGCAGACTGAATAGGTGGCGGCAGGGAACA
TAGACCTGTTGGTGGGCTACCAGCAAGGAGCCAGCTGAAAGGGAGAGGGAAAACCAGGGGTTTACCTTCTGATCCAGGAGCCTGCAAG
AGAGGAAGGGTAGAGAGCCCCAGGGAGGAGGTGCTGGCTTAGGCTTGGGGTGTGGACAGCAGAGAGAATTCACATCACGAAGCCCCT
GGAGAGAAGCTGCAAGCACCATCAGTTACAACTTGTGTTATTTCATTAGCTGACTCAGGGGGACAGGGCTTATCTACTCCCTGCTG
GATCTCACGGAAGCCTCTGCACACTTCTTTAGTTTCCTCTAATGTGACAGAAACATAACGATAGTGATAAGATATGAGTCTTCAACT
TTATGAGTTTGGAACCCAAGGGACCCAAACTGGAGATTTTCATTTATTAGGTATATAAAAGTGGATATTTTTTATTAAATACCAATA
GGGTTTTCAGAAGTCAAGGAACTTGTTTAAAATTATAGGCCATAAATTATAGTCAAGAGACAGCTCACATTTGTAAACAGCTCACCC
ACTGTGGTGCTGTTGTCCAGGAGATCCAAGCAGCCTCCTGGGTGATCACAATGTACTGTCACCAAGCACGATGCCTGTGATACAGG
AGGTGTCGCTGGTGCAGACTCTTTGGGGTCCTTTTGCTAAATAGGTTAGTGGCATCTTCTCATCAGGGGACAGGCAGACAGTCCAGT
CTTTTCTCTTTGCCTCCCTCTTCCTAGAGAGAAGTGAACTCAAATCACATTCTGAACTTAGAAGGTGACCTGGCCTTAAGAGGAAGGG
TGTGACAGCTGCGTTCTGAACTGATGTCTCTAAATTAGCGAGTTTACCCACATGTGATTTATAACACTGAGCCAGAATGCCCATCTA
TTTACTTAACGCTCAGAGCTCATTCTACACTCCTGCCTCTGGGATGTGATTGACCACCACCTTGAGCCAGCTGACCCATCGTCTTAA
GCACCATAAGAGGAGGCATGAGTCCACGTGTCAGGAAGGAAGAAGCAGTGGGGCATGTCTCCTAGAGGGGGTAGGTCAGTTTCCTAG
GGAGGTGAACCTGGGCGCTGGAATCACTTGGAGCACGAAGGCCTTATGTGTAAGAACAGAGCTGGTCTGGAATTACACAAGAAGAGGA
GGAAGAAGTTATGCCTTTCTTCCATGGGAGCTAGAGGCTGTCCGTCCTTTCTGCAGATACACCATAGGCAGTCCTGAGGGTGGAATT
CCTGGATGCTTGATTGATGCATTTGAAGTACTAGTTCTGGGGCTGAGGAGATGTCCCAGTAAAGTGCTTGCCAGCCAACCATGTGGA
CCTCATTTTAGGTCACCAGCACCTACGCAAAAACTGGGCTTGGTGTGTTTGCAACCTCTGAGGGGTGCATATGTGTATGTATATGTG
CGTGTTTCTCTGTGTCTGTGTGTAGAAAGGTAGATCTCTAGAGTTCACTGCCAGTCAAGTTAGGAAAATTTAAAAGCTCCAGTTTTA
GTGAGACACTGTTTCAAAGAATAAGGCGGAGAAGGACTTAAGAAAGATAGCTGTCTTAGTCTACTGTGGTGATAAGATGCCATGATCA
ACAGCACCTTGGAGAGGAAAGGATTCATTTTGTTCTACAGCTTATAGTTCATCATCCGGGGAGGTCAGGACAGAGAGGCTTAAGGTAGG
AATCTGGGTGCAAGAGCTAATGCAGATGTCATGGAAGAATACTGTTTATGAGTCCGCTCAGCCTGATTTCTTATAACATCCAGGACT
ATCCATCCAGGGGTGGCACCACCCTCTGTAGGATGGGCACATTAATCAAGAAAAATGCACCACTGGCTTGCCCTTGGGCTAGACTTG
TCGGGGAATAGTTTTCATTGAGGTTTCTTCTTCCAAAATGACTCTAGCTTGTGTCAAGTTGACACAAAAAGTACAAAACCTAATGTT
GATCTCTGACCTTCACATGCATGTGAACACACATGCACACACAGCACACGCACAGGCACACGCACACACAGCACAGCACACACAACAC
ATGAAATGAATTCCGGCTCTGAGTTCCTGAGATACGAAGAGCCTAGTTTCTCAGCATGGCTTGATCTCAGGGTGTCAGTTCAATCCC
AGTGTGGTCAGCACAGAGGAGAGCAGGGCTCAGGTGGTTCTCAGGGGAATCCAGCCTGACAAGGACATTCTGTGTGGCAGCCTCTTC
TGCCAAGTGTGTGTCCATCTCCTGTTCCACTTTCATTAACACTGCATTGATAGATCACAGCACACGTGAGAAACAGAAGATGGGGAGA
GGGTGTTCCAGGGACTCAGCGCGGTGCTGTTTCTCTTGGGATCAGCTTGTGCTTGATAGGGCTGGAGAGAAGGAAGCAAGAGAGGAG
AGCCTGCCATGAAGACACCCACAAATGCCTGTACCCCAGCATTCAGGAGGAGGCAGGAAGATCAAGAGTTGGAGGCCTGAGCTACAG
TGTGAGATCATGTCTCAATAAAAGAAGAAAAGGAGAGAGTGAGAGTGAGAAGGCTAGAGAGTGAGGAAGCGAGAGAGCGAGCAGCAAG
AATACAGATGCTTGGCTTTTGGCAGGGGACCAGTCCACCTTCTTTGAAGATAGGAGACTTAGTGGCTGGCACGTGGGCAGTGCCTGG
GTGGCCGTTGGTTGCACTGTGCCTAGTTGGTGCTTTATAATCTGGGAGTGTCCTCACAGACCTGCCCCTTCACCATCTTCACTTTTAG
CCTGCAAATCCCTCCTCTAGGAACTGTCTCTGGATACCTCCAGTCTCCAAGCCAGGAGCCTGTCTAGGCTGTGACTTAGCATTAGCT
CTTTGCCTGCTGCGGCATGCCTGATTGGTAGATGGTAAATGCTAGCTTTAGTCAGCCCCACTGCTTCTAGATGTGGGCTTCATGTGA
TGGAGACTGTATTGGAGCTAGAGATGTCTGTCTAGCACATAGTGAGACTCAGAAAGAGCGGCTGAACAGCGAGTGAGTGAACGGAA
CACAGATGCGGTCTTTCTTCCTTCTGTGGAGAAAACTCATCTCCAAAGACTGGACTAGTCTCAGTCCACAGTGCTGGGTTCTGGCT
CGTGAGTTTTGTATCCCAAAAAACAAAGGTCTCAACAGGCTACTCTTGTGAATTGGTCTTGCCCACTGCTGCTCTCTGGTCCTCTCT
GCTGCACTCTGGTTAGCCTTTCTCACGCCCCGGCCATGAATCGCCATGGTTACTATATCAGCCCTGATGGTCAATTTTTATGTGACA
CTGTGTCTTTATTTTCTAAATATTTAAACACTCGAGCTTGACCCTTGCCTCATAGCTGGCTTCCTGCCTCTGTTCCACTCAGCAG
GCATTAAGATGCCTGCTCTGCCATGATATCAGGGGCGAGGGGAGGGCCAGGCTCTATCTTGGCTGTTGGTACTGATCTCCAGTTTTG
TCTCTGGGAAATGGGTATCCTGAATCATACTGTTTCCTAACAGAATGTGTGCCGTGCTTATTAAAGGTATGGAAACCCATATCCGGG
ACATGTTGAGACTTGTCCCAGCCAGGATAAACAGCTCTAGACTTCCCAAGTCCACTTGCTGTGTGCCATTGACGATGCCTCTTTAAT
TGTGGGAGAGGATGAATGAGGATGTGTGTGTGTGTGTGTGCTCTTTCACTTATATGTGGCCAGAGGTTGACAGTGGGTACCTTTCTC
TGTCCTTCTTTTGAGCCAGGGTCTCTAATCATTTCCCCTAAGCTGACTGGCCACCAAATCTCCAGAACGCACGTGTTTTGTGGACTT
ACAAGTGCATATGGCCGCTACACCCAGCTTTTAGGTGGGAGCTGGGGATCTGAACCCAGGGATTCACGCTCGACGCTTGGACAGCAT
GCAGGATCCTCACAGAATCATCTCTCCAGCCCTGCAAATTCTTCCTGCCAACTCTTCCAAAATCTTCCCGGGCCCTGGGAGTCTCTG
TCCTAGATCCTGACGGGGCTCCGGGGCAAATTAGTTTGGGAGGAATTATGTTTGTAAGTGGAAGGTGACTTCTCCCTTTTCTTCTCC
CCTCACTGGCTTGAGACAGGCCCCAGCACCTGAATATAGGTTGCCTTGGAAACCACTCAGTTGTCTTCACCCTGGGCTATGTGAAAG
GAGTGAACATTGGGTGTGCTGTTCAGGAGGCTTCAGCAGTGTAAAGTGTCATCATACCACCAGGGGGACTGAGCTAGTGTGAAGGGC
CTCCTATCCGCCCCACCCCAGGGTCCGGCTCCAGTGAGCTCTGTGCTCACAGGATGTAATAGCCTGTGTCTGGAGTATTGGTCACT
GTAGCCAGATAGGCCACCACTAGTTCCACTTTATTTTCTTTTATTTATTTTTTGTGGTTGTAAAGTAGATAGATCAGGCCAAATGA
TCTCTGGGGCTTCTGATTCTGTGAAGGTACCACAGCTGAGGGACACTTGATGACTGGGAGATAAAAACCAGAAGGAAAGTGGCTGAG
AGCTTCAGAGGACAATGACAGACAGTGCGTGGTCAAGAGGCTCCTGGAGGGGCCTATTTGAACAACCTCATCTTTCTTTTAACAGCC
TCGCCTCCTTTCTCGGAAGCAGAATTGGACACCTAGCTAATGGCCTCAAAGTTGAAATAGAAAGCCATCAAAATGACAGTTTTTTGGG
```

82

EP 2 204 376 A2

```
GGGCTCTGGTATCTTGTAGTTTAAAAGAGACTTCTCTCAAGTCTTTCCCAAAAACAAACTGACCGGCCTTCTTTAAAGCCTTGAGAA
TGATCACGGTATGTGCCCAAGCTCCCCCACCACGCAGTTCCAGCCTCCTTCTCCAAAGTCCCTGGGCAGCGAGCTCACATCATGAAA
AATCTGTCTCTGACTGTGGGGACTTGCACGTCTGAGGCTTTTTCAGAGTTAATTCATCCAAATTTGTATTGCTATGTTGTGTGTATT
TGTTTATTTCTTCATTTGAAGTAGCTGGGAACTTGTGGCTTTTCCTGCCTCAGCCTCCTAAGAGCTGACCTGATTTTCAGACGTGTG
CCACCATACCCAGTTATCTTCCTCCTCTGTTTATTTGGGGCTATTAATATAGGGGTCCCACTTAGGGCTGAGCACTCAGTATACATT
GATTCTCAGTATTATGACTAACTATTGATCTCTGCATTAACTGTGACCACTGCTCAGAGAGGATTCTTTGGCTAATGTTAAGCACAG
CACTACGCTATGGATCTAAGCATAGATATTTGGAAGATAGTTTGACAGCATGTCATTTAGCAAAACAACAGTAACAGGTTGTCCCCT
AGGGCCTGTGACCTCAGGACTCATGGACTTTGGACCAGCACCAGGCCCAGATTCTTTACTGTGAACCCAGTCTCAAATCTAACCCTA
AAGCAGCTGGTTACATCTACAACCTTTGTGCCACTATTGCACTTGTGGGCACATCTCATCCGGCAGATTGGTACTATAGCATGCAGG
GATGGCCTGGCTACCACCTTCTGGCATGATGAAAGTTAGTCAGCAGGGTGGGGGGATGTTTTCAAGTTTGCCTGATTTCACTATGTC
CTTCAATGAAAGTATGTTGTACCTTCTGCAATTGGGTTTTACAGTTTAGTTTTTCAGGGAGGAACAAAGAGCTATGGCAATAGCTCG
GATTGTATTAAGGGCCTATGGGACCTTCCTGACCCCATAGGGAAGTATCCTATACTTGGTACTGAGGTTTTTATTTTTATTTATTTA
TTTATTTATTTATTTGCCTTTTTTTTTTTTTTTTCCTTCAGGACAGGGTTTCTCTGTGTAGCCCTGGTGGCTGTCCTGGAACTCACTC
TATAGACCAGGCTGGCCTTGAACTCAAAAATCTGCCTGCCTCTGCCTCCCAAGTGCTGGGATTAAAGGTGTGTGCCACCATCGCC
CAGCTTGAGGTTTTTATTTAGTAATCCATGACTGACAAGTCTGACTCTGCAGGGTATAGCTACCATATTTAAATGTTGATCTCTCTC
CACAATGGTGACAATCCTCCATCAGCAATGTGTATTAGCCCCACTGTACTGTGTCTGGCCTCCCACAGGCTATGAAACCCCACTTCC
CTAGGGGTATGCGCTTTGACAGCACCACCAAGGTTCTCTGCACACTCAGAGGAACAGTAGCCATCTCTGTAGTTGCTCCTTCAATGC
CTAATACACAGCAGGAGCTCGATGATTCTAAAGTGGGCGAGCAGTATCTGACTCTGAGGATTTGTAAGATGGGAAGACACTGCACAG
TTTTTACCGTCTAGAAATTTCTATACACTTGTTATTGCATTATGCTGCCAAGAAAATTCTGGTAATTCTAGTTTCCTGTTGGGCAAG
GAGCTAGTCAAGTGACATTTTCCCGTTTCTAAGAAGAAAGATGGCGAAATCGGCACTGCTCAGATCCTTCTAACACAGTAGTTGGAT
TAGGAGAAGACAGAACCCATCCTTCATTTCTATTTTGTTTAAAAGACTTGGTGCAAGTTAGAAGACATTCCATCACTCTGAAACTTC
CCCTGCCAGAGGGGAGGGACACCCATGGGCACTTGACCTGGGTCTAGGTTGGATTTTGTCATTTATTTCTTTTTTCTTTTATTCATT
TAATGTATATGAGTACACTGTAGCTGTCTTCAGCACACCAGAAGAGGGCATCAGATCCCATTACAGATGGTTGTGAGCCACCATGT
GGCTGCTGGGAATTGAACTCAGGACCTCTGGAAGAGGAGTCAGTGCTCTTAACCACTGAGCTATCTCTCCAGCCCTTAAATTTTTTT
TTCTTAAAAATGGTAGTAAAATATATGTAACATAAAAATTTGCCACTTTACAATACAGTTCAATGGCATTAAGGGATATTCATGTGT
GGTTCAAGGATTATCATCAGAATTGACTTCATTACCTTCTATGTAACCTATGTGCTTTGATACAAAATAGATTTGCTTCATGCCCTC
ATTTGCACAAGCATCCTTTATCATCCAGCTCCTGACCCCACCTCCATCTGTGTAACACTGTATCTCAGGCTGTGGTCCTGTTTC
AAGCCTTGGCCTGGCATGCAGCTGCACACACGGCGACAGTGGCAGCAAGCCACATGTCCATTCATCTCATGTGGGCGAGGGATTCCA
GTTATCCTTGACAGCAGTCTCATTTTACAGATAAGTGAAGTGAGGCTGAGAGGGGAGAGATTACCTATTCAAAGTCACGTGGCTGAT
GAGAGGCAGAGCTAGCCTCAAACAGACAGCCATCTCCAAATCCTGCAGACTTCTTTGCTAGTTGGGGTGCACTCCACTCTCCAACCC
ATCCGTGCCCCTGACTCCTGTGGTATCCAGAAGCACTCAGGAACACGGGTGGGTTGTGGGACTTAACATAGTGGAGAAGAGTGAACG
TGATGGGGTTCTGGGCTGAAAAGCAACGAACACAATTGGTCCTACCTTTCAAGCTCCCGAACACCTTGGCATCCCCATCTGCTAGAA
CCTCGTTCTCTTTCTGGTTTTCTTGCTGTCTTCCCCTTGGGCCTCCTACAGTTGTCTAAGAGAGGGGTCCAGTTTGCTCTGAGGGAAGAGA
GGTTACAGTGTTGGTGATATGCCTGAGTTTCCTTGCTGGCCCTCCCCTTTCTTGCACGTGTAGGTAGGTGCTGGCTTTTCTTGCACA
CTGGCTGCCTGGCTGAGCCCTATAGAGTCTTGATCTTGCTTGCCTTCCTGCCCCGCTTGGGCTGACACCCATTACTTGTGCCCTCCT
CATCTTGTCTGGAGTCACTAGCCCTGTTATTTCTTGCATTCTTATTTGCTTCAGCCCAAGGCCCTGAAGACTGGACAGTCTGGGGCC
TGCTTTAGAAGTTTTGTCTTTGCTTGCTATAATCCCACAGGCCAGGGTTGGTAGCCTTCTCAGGCTCTTCTACTCAGTGACCATTCTT
ATGCTGTGCATCTGCAGTCTTATACCCAAACCTCCTGAGGGGGGACCTAGACCTTCCCAGCACAGTGTTTTTCCAGATAGGTCTTGGG
GTCCTGTCCATCGCGGCTGCTCTGAACAGATCCCATTAGTGCTGCACTGGGTCCATGCACCCACCCTAACCTGGACAGAAGGGGGCCA
ACTCCTGGTTTTCTCATTCTCCTGACTGTAGCAAAGATGGCTTCCACGGCAGCTCATTGGTTCATTCAAAGCATGCTGTCTTCATTG
AGCTGATGATCCTTCAGCTGTTCACCGGGTCCCTCATCCTGTCATCTCTGTTGTAGAGGCAGCTTTTGGGGAGGGGAAGGTGAGGGA
AAGCTGCCCCTTTGGTTGCCCCAGTGAGATTTCCCTTGAGTTTCTTTTATCTCATTCAGCAGCAAGGTATTTCGAGCAGTCCCAGCT
TCCTGTTCTGCGTTCGTTGGTGTAAAAGCCTTGTTCAAATCACTGAGACAGATGTGGAACAAGAAATTGAATCTGGGACAGTCGTTTTCT
TTTGTTGGACTAAACAGAGGGAATTGGGATTAAAAAAGAAGCCCAAATAGTTGCAAGCCTTTTATTCTAAAAATCAGTGGTCTCCCT
TAGGCTGCAGTATCCTGCCCCTGTCCGCCCCACCATTCCCTACACACCAGGCACACCTGTGAAACACTCATCTTCTGTAACCAATGC
TTACTGAGCTACAGTGCTTGCTTGGTGCTGGGAAAGCAAGCTGCGCCTGGGCTCTGAAATTGGGTGACACATGACACTTGACCATTT
GGTAAGACTGTGTTGTAACAATGTTGCTCCAACAGCCTGTGCTACAGGAGAGTGTTGACACAGAGCCGCATGCCCCGGGGCTCTAAT
ATTCCCAATTGCTCCTCCCCAGCAGCTGGGCTTGGCCCTTGCTGCTGACCTGTGACTAAGCTCCCTACAGCCACAAAGCAGGGATCA
GTAGCCAGCCACGGTGGAGCCGTCCGCCAGGTGCAGAGCAGCCTCAGCCTCCGAATTCAGACCATGAGGCAGCATCGCAGGAGTGAA
GGCATAACATCCAACGTCCCGGACACCTTTCTGCTCACGGACCCCACCCCTCGCTTCTCCCAGTTCCTTCTTGGGTGTCACTGGAGC
AGGAAGCTAAATGTTCCTGTCAGTTCTCTGCGGTACCACTTGTTAGAATGTACTAGAAGTGGTAGCCCTTTGAGAGCCAAGAGGAAT
CGCCTAGGCTGGCGTGTGTAGGATGGAGGTGGGGAGGGGCAGGCCAGGCGATGTTTGCCTTCCCTCTGTTGTTAGAAATAGCAACC
GTCCTCCACCTTGCTGAAGGACTGGACAAAAGGTGATTAGTCCCCTAAGTAAGGGAGCTATTGAAGGTCTGTTCAGAGCTAAGCACC
ACAGGGGACAGCAAGGCTCTGGTCATGAACAGGCTTGTGATCTAATCTGAGATAAACATCTCCACTTGTAGGCACAGTGAACAGCAA
GGGGGGTGTGGGGGCTTCCCACTTAGGTGGGAATAAGGACCTCACACTGGTGCTGAGGAGCAGGAGGACAGAGAAAGCTGGAGCAAGA
GAGTGTGGGATGGGTGGGTCCTGTAGGGGAGCGAGCCTGCTGAGCAGCCAGGAGAGGTGGCTGGGAAGAGGGTTGTTTGTGTGTTT
GTTTTGTTTGGTTTTTTTTTTTTTTTTGGTTGGTTACTATGTTGGTCACTTTTTAACCAAGATTGGAGTTAAGTCACACGGGTAA
AACCCACTCTTTCTAACCCAGCAAAGATGGCTGTCAGCCATGTGGCAGCAGCTAAGACAGTGGCAAGCCTCCTAGTGCTTTGAGGAA
TGGAAAAGATACAGATAAGTGTAAAGGGACTAGAGAGAAGTGAGAATGGCTGGGGTCGGGTCATCCCGGTGAGTCTAAAATGGTGGC
AGCCAAAAACGAATGTCTCCAAGCTGCTGTGGCAGAGATTTTAGGAAAGAATGAGAGCTCACTCATAGCTTATCAAAGACCAACGGG
TGGCAGTGGCAGTGAGGTGAGGAGACAGACACACGTGGGGGACATAGTTCCCGGGTCACAGGCGCCGGCCTGGATTTCCAAGTTC
AGGTCTGTGCTGGTTGAAAAGTCTCCCATGTTGGAGATCTCCAAGACCAGCTACCATATGCACTTGGTTGAGAATCAGGAATGACTA
GTCCTAGCCCTAGAATCCAGAGATTCCACATCCCATCAACAGAACACGCTCCCAGGGCAGGGCCCGCAGGAACCCCAGTTTCTCTCG
CACTGTCAGATCTTTTTGGATGGTTGGAAGGAGTAGCGTCCATCAGCTTTACAAGATTTTACAGGCTTGCTGTTCTCTTACTGTTTT
CAATTACCTCTACCTAAAAACACATCCGTCCCCAGCAGCTTTGGGGGTCTACCTTCTCACAAAGGCAGGGTTTTGAATGGGTTTCCT
GCCTTCCAGATTCAAACTTGATAGTCTGGCCAGGTGGCTGAGAACTGCCATTATCCCAGAGCGGCAGCGGCAGGGGCTCCTGTGCAG
CTGTGAGTCCCCCAAAGCCAGCATGGACCATCTTGTGTGTAGTGGGTTGAGAGAAGGTGTCCCACGTTGGGGTCACGTAGGAGGAG
ATGTGCTTGCTGGGCCTGGGCAGGAACTCAGGGGCATGGCTGTGGCTGGCCCACAGAGCAGAGGCCTAGCTCACAGCCTGTTTCCAT
TTCTCAGCTTCTCCAGCCCTTTCACTAGTATTGCCCAGCCCCTTCCCTCCTTCTTTATGTTTTCTCTGTCTTTGACTCTCTTTTTGT
GACTGCTTCCATCTTTTCCTCTTTGTGTGTGTGTGTTGAAGCATGCGGTGTTTGTCACTGTGGGCTGACTCTGCGTCGTGTGTGCT
GACAGGATCTCTGAGGCAGAGAACATTCCGATCTAGGTGTCTTTGGGGGATGAATTTAGTTCTCAAGATGGCTGCGATGTTCTAAT
TGTGCAAGAGTCCTTGAGCACCCACAGTGGGGGGTGGCCATGTTGTCCCTCATCTGCTGGCTCAACTTCAGGGACCACAGGAAACCC
CAGGCTTCTAAATGTTATGCAGAGCTACAGTTCCAGCGGCCCTGAGCTGACAGAGATGGATGCCCAGCATTAGAGTCTCTCCAGGCC
CCTTTCTAGGTGCTCGCGGTTTTGGGAGCACTTCAGTTTTTATGCTGGCATATCTCAAAGAGGAAATGCAGATTAATGGTGTGTTC
TGCCATGTTCTCATGTTCCTGGGACTAAATCAGTAAGATCTGAAATATTAATTGGGCTTTTATGAAGAGAGGATGAAGTACGGCGTG
```

83

EP 2 204 376 A2

```
GCATGGAATATTCTAGATTTAGGATCATGAGTTTGACTGTCAACCCTGCTGCTGCTGAGATTTGCTCTCTACTGAACTGCTAGCTTT
CTCCGAGTCTGTTTCTTTCCATGTATACCAGACTGGGCACACACCCGTGTTGTATCAGCTTAGAATATCACCAAGCCTGGCACAGCC
AGGGGCTTTTGGTGAGTATATTCTAATTGCCTCTTCCTTGATAAGACCGCCCTTAGCTCACGACACAGGATCCAGTTCTTGCCTGAT
GTGGGTACCTGCTGAAGCTTTTAATGGATTCAGCCCTTTGATGAGGTCTCTGAAGCCCTTGATGGCCATCCTCAGGGGCTTCTCATA
GCTGTGGTCAGCACACTCCTAGGGGCCTCCCTTCATCATCCTTCTTCTCAGTGTTGATGGCCAGCCTAGGGCTGAGGGGAGCTTCTT
ATCCCTCCTGTTAAAGATTGGCCAGTCCCATTGCCCGACTCAGGTCCCAGATACAAGCTTGTTGTCAGTACATGGGTCATGGAGCCC
TCAGTGCCCTAAGTACAGGGAACTGGTTCTCATTAAAGGCGCCTGGCTGGGTAAGATGGTGACAGTGAAACAAGGGAAACATTGAGCA
CAGGGCCCCGTGATGCACAAACAGGATCTTATTTAACCTTCATATTATCCTCAATCTATTCCCATCTATTTTGTATTTGCGGCTGTA
GCTCGAGCCGCCACACATTTATTATCTTACACTTGGGGATTGGGGTCAGAAGTTCAAAATGGCGCCAGCTAAAGCCTAAGTGTTGGC
AGGGCTGCTGGGGTAATCCATTTGCTTGGCTTTTCCAGCTTCTGGAAGTGGCTTGCCTTCCATCTTCCAAGCCATTAGTGGCCTGGT
TGAGTTTTCTCATGCTGTGTCACACTGCTGCTGACTCTCCTACCTCCTGCACTGTATTTCAAGGGACCTTTGTGACAATATGGTGGC
CATCTGATAATGCAGGGAGAACTCCACACCCCACAGACCAACCATCTTATTGAGTCTGCATCCCCCATTCTTCTTTGCTGTGGTTTG
AGAACTGGATGTGAGTGTTTGAGGGATGCTGTTGTGATTCCCACGGCCCCCTTCTTGCAGTTGAGGGAAACCAAGGCATAGTTTTCT
CATGGGATATTAGCTCCCACGGCCAAGGTTATGTGGCTGCTAAGTGGCAGACTGGAAGTAGAGCGAGAATTGGTAGATCCAGGAAAG
TCTGAGAAACACACTAGTGCAGGCTAGCCCTCTGCCCATGGCAGTCAGAGGAGAGGGGACAGAAGGCGCCCTAGTTTGAACCCTAAC
TCGTCTTAGTCTAGAATGGGGAGGACAAACTGGCCTGGTGACACTGCAGGAGAGGCAGTGGTTCTTCAACTCGGCTGTACACTGG
AATCACCCTGGGAGCTTCCAAATGATACCAGAGCTTCGCCCCTAGAGAGCCGGGCTTAATTGCTCTGGGGAGACCCTGAGTTTAGGG
ATTTCCCAGGCTCTTCAGCTGATTCTGGAGTGTAATAGAATCATCACTTTCAACTAATCCACAGCCCTGGCTGTGCCTGTCACCCGA
GTAACTGCATCAGAGTCTCCTTAGAGTTGTCTGCAGCCCCCTCCCATTCTACTCGGGACCCTGAGCAGCAGACCCCTCTGCCCTGCT
CTGCCTCCGTGTAGCCAGGGCTGTAGGCTAGAGGACATCACGTGTACCCAGAGGAGCCAAACCTTTGTGATGAGCAGGTTCTTTTCT
GGGTCTCCCTGGTCTCTCTTTCAGGGCTGTCTGCACAACTGTCCACTTCCCCTTTGTTCATGTTAGCACACTACCCAGGTTCAAACC
TCAGCCCGTCAGGTCCTGATTTAGCTTGGACCTCTCTGTGCCTCAGTTCCCTCACCTATAAACTCAGGATGCTAATAATGCTGTCCT
GGTCAGTGTGAAGATGAATAGCTTTGCAGATGAGGGAGATTACAGCGAAATGTTTGCTAGTGTTACCCGTATTATCACCTAGCCCTG
TGGGCCCAACTGATTTCAACAAACATTGATTCTTTCTTTACTTAGTGCAAGGTTAGGAAAGGGTAAAGTGTCTTCCCTGCTCTGAGA
TAGCTCGCAGTGGAGGAAAAGGTAAGTCAGTCAGATCAGACAGCTAATTACAGTACAGCATTCGATGCCGGAACCAGATGGCGGTGA
GAAATAAAGGCAGGGAAACCGCCGTGCAGGGAAGGACAGGGGGCCATGGATGTGAAGGCACACCATTAATTTTCAGCCATAATAAAATC
TTAGCGAGTTTTGTCTGTGTCTGCACATTTGAACTTGCGGGTTATGATAATCTGTCAAGCCCCTTAGACCAATACCGTGAATCCAGG
CGACTTACAACAGCATTTACTCTCAAGTGCAAAAGTGCCAATGGGGCCCCAAGGTCCCCTGTGAATTTATGTGCTCTCTGCAAGAGG
ACAGTTACATGCTCCTCAACAGGGTGAATCCTGTCTCCTCCTCAGGAATCCCCCGGAGAGCATCAGGAATCATTGCAGAGGTGACTA
CGTCATGCAGACAAAGGGGCCCTGGCAGCAATGAAAGGGGCCATAGCTGCAATGATGAGGGTGTGAAAGAAAACTACAGACACACAG
GGTACGAGGGCTTTAAAAACAGACAGAGTCTAGGTTGCAAATGGTCAGAATAAGTCAAAAGGTTACCTAGCTGGGGAATGAGGCTAG
GGAAGAGGAAGGTCTTAGCATGACAGCCAGAGGGAAGTCAGATGATGGGGGCAGCTTTAGGGGGAAAGGTTGCTTGCAAGGCAAAAC
ATGTCCTTCCTGTTCCCTCTGGCCAAATGGCCAGTGGCTCCACTCACACATCTCATCAGGAGCACTGGAGCACCGGCTTGATCAGTG
TTGGCTGATGCTGCTGGCAGCTGTGGGCGTCTCTGCCAGCTGTGTGAACAGCTGTGCACCAACCCTGGGCTCTTCTATGCCCTGGCT
CATTTCCCATCCCTGCTTGGTTTAATACACTTAAAAACAGGATCCCACACTCCCTGGTTCTAGCTGTAAAATATGCTGTTCAGAGGA
AAAGACGGTGTCTGCCTGGGCAGGACAGTGAGACAAATGCTAGATAGACGTGTAGGCTTTTCTTGTTTTGAAGAAAATCTCTGTCTG
AAGCAGACGGGTGTTGATTAGTGTGTTTAAAGATGGTGCAGTAAGGGCTAATAACAACACCAGATCGTTACTGAATGGCGGTTGTCA
ATTCTGACTGCTTCTCACAGGGCCCACCTCCGTTGGTGCAGCTTGTGTGCTGACCTTCAGCCGAAGCCCCAAATGGGACTTCTAGAA
ACCGTTTACAGTACCCTGCCTGGAGTTTGCCCAAGCATTCACAGATCCACTAGTTTGGCTCACCCGGAGTAAGAAATGGCCTTTCTT
CCTGGGCTGCTGGAGTCTATGAGATTCTGCCGAGGGAAGAGAAGCCTGGGACTCCTGGGTGGGCAGAGTCAGGATTTCTGAACTGTT
CACAAGGCCCAGTCAAGACCAAGGCCCAGGGCATGCAGCTGGCATGGAAATAGTGGCCTCCATTCCCAGTGAAAGGTGTCAGAGCCTT
GACTTGTCAGGGCACAACATGAGAAGACCCTTTCAAAGACAGCCTGTTTCTTCCCAAATGATCAGACATCCCAAACATCAAGCATCTAG
TAGTAAACTCCAGGCTACTGTAACCGTTTCCTGCGCTGTAACATAACACAACCAGAAGGAGTGATAGGAGCCTCGGGGTTCGAGGGG
TTCTGCCTGCTGTTGGGAGCTCCCTGCATGCTGGTGATAGCAGGTGGGGCTGTACAGAGACATTCTGGTTTTCTGGGGTAGAGTCAC
ACAACATATTCCTGGCTGGAGAATTATGATGACTGGATCTAACTTTTTTTCTGTAATTTTCTAGGTAATATTTGTATGTGTGTTTGT
GTGTGTGTGCGTGGGTTGTATTTGTGGGTTATGAGGTTGCCTTTCTAAAACATGGTGGCCCATGCCCTACCCAACCACCCTTGCATT
TAGACTTCAGGGCTCTGTGCCTGGCTGAGCTTTTCCCTGCCCTGAGTGCTGCCTCACCTCCACCTGCCTTGCAGTCACCAGACAGGG
GACTCTACATCTTACCTGGACCTTTTCCTGCATGTTCTGAGGTTTCCCACCTCTCTGCTCTGGAGGAGCTGCTGGGTGTTAAGGAG
GTGTCTCCTGCCACTCTGCGCAGGTTTCCTGAGTCACAGTCTTTGTCCCTGCTGACAAGCTCGATAAGTACACATGACATTTGTTTT
CCTCTGGCTCAGAGTCCAATGGATCTGGATCTAAATTTCAGCTCTGCCTTTTACTGGCAATATAACCCCAGGCTAGTGGTTTAGGCC
TTTCTACTTCAGTTCCCTCATCCATAAAGACGAGTGCTGCTCCCTAACAGTGAAGTGATGTAGTGAGAGCTGTGGGTAT
GGGGGCCATATGGAGGCTCCCGTCTCATCATCCCCCTCATCGGGACCTGCTACTGGGACCCCATATCCTTTTGTGGTGTCAGAGCTT
CAGGGTCTTCTCAGAGGACTTTCCCAGGTCCTGCTGTGGTTGGCGAGTGCTGCCAGCTGCAGGAACAGCTGTACCTCTGTCTTCTTT
CTTTTTGCCTCCCCTGGGTTTTTTGCTGTCACGTTAGCAGGCAAGTGCGCTTAAAAATAAGTGGCAAAAGCTGTGCTTGTTTGAAGTG
TAAGGTATGTCACTCTCAGAAAGCGCATTTGTGCTGTATAATAGATAAGAAAGAGCTATCTAGTTAAAGAACTTTTGGGGCTAGCTT
TTCTTCCAGGGCGTGGGAGGAGCATCCTCCATCCTGGTGTAGGCTGTTGGGAAGAATGAAAGTGCTCTTCAGAGGCCCTGCACAGGC
TCTCGGCCTGTGGTGTTTCTCATTGATCTATTTCTCTCGAGTGAATGGGCTTAACAGAAATAGAGAGGATTTTTTTCCCTAATGATC
AGGATAGAAAAGAATAAAAAGTGACTTTAGGGGTATCTTGGGACCTTAGCTGTCATTGTGTGCTGACACTTCATTTCGGAATGTAAA
GGGCCTGCTGCTGGTTTGTAGGGATAAATGGTCTTCTAGTCGGGGGCAGCCAGGAAAGAGGCAGAGCCCTTCACTTAATAACACACA
GCATGGAGGGGCCTCTCTTGGGTTGGGTGATGGTCTCTCTCTCTCCCTCTCTCTCTCTCTCTCTCTCTCTCCTTTTCTCCCTC
AGAGAAACAAGGCTGGAGCTTGGGCCAGCTGGAGCTCGGGAGCTCAGTATAGAGTCCTAATTAGTTGTTTGGACCAAATGTTTCCTA
GTATTCCTAGGAATCCAGGGAGGGGGTTTGCTCAGTGGGCCTTGGAGACCTGCTCTTGGGTTGCTGCTCTGGGGAACAGGTGCCCAGGC
AGGAGGCAGCTGTATGAGACTGCTAGAGTAGAGTGATGTTCAATCACGTGAGGGTCCTTATCCTACCAGGTGACAACCAGACTAGCT
CTTGGAGAGTCTTACATTTGTCCAGGACTCTGCTGTCCTGCTCTCTGTCAAGCATATATGTAGACTGCTAGGAAATTCCTGTGCTCA
GGGATGTGCCCAGGGTATAACAAAAGGCAGGGTGGGTGCATGCCAATCTACCTCCTCTCAGGACATGCACCCAGCTCCTGTAGTGAG
AGGGTAGCCCAGCAAGATGACTTCTGCTAGTGGGGTGGGTGCAAGTTGGGAGGCCACAGCTCTGTCAATAAGGTTCCCTGACAGAAG
AGACAGTTGTAATCTTAGTTCCACAGAAGCTCTGAGATCTGCAGGGCTGCTCTCTCGCTACAAGCTCCTACAGTTGATTTGTGTGGC
ACTTATGAAGCCGAAAGATACCTGCATAAGCTTCGCCAATGGGCATGAGCTTTGGACTTGGGTATGTTCAGTCACCTCCATGAGTGA
GTCAGAGACAGAGCCCAAGGATACCACAGGTCTCAGCAGCCATCTCCAGACTGTCCTCTGATTGAGCTGATGAGGAAGCTGAGGCCT
GGAGCAGTGGGAGACCCAGTACAGTCCAGACAGCCTGGTATGAAGACTTGCCCTGTTTAGTGTCCAGCACTTTAGCTAAGCCTTAAG
CACTTGGCATGAGGGAGGCTCTGCTCCACACTCACCAGTTAAAACCAGTCATTTTCCCAATCCCAGGCACTGGAGATGCAGTAACAG
GCAGAAGAGATCTGACCCTGCCCTCACAGAGTTTCCACCTCCAGAGGGAGGGGAAGGGGTGGGCCATAATGAGGGGAAGTCTTAAAGAG
GCCAGGGTAGCCAGGCATGGTGGTGCACCCTGCAGTGTCTTTAGTACTAAGGAGGTGGAGGCAGGCATCAGAAGTTCAAGGCCACCCTTC
GCTACACAGCAAGCTCAAGGTCATCCTGAGCTACCCGAGACCCTGGCTCCAAAGAAGCGTTTTCGGTAAATTCGGTGAAGGACAGTG
AAGAAACCAGGACAATGGTGGAAGATGATGGAAGGAGAGATGGATCAGGCTGGGGTTTCAAGGGAGACAGAGCTCTTTGATGGTAAC
TGGAATATCGCAAGATCTACTTCTGTTAGTATTGTTTCCTTTTCCTGTATACTGAAGTATTATCCGCAGGAAAGTGAGGCTTAGAGA
```

84

```
CTAATTAAGCTCTCCAGTCACACAGCTGGCGGACAGAAGGACCAGGATTTGAATTCTAGTTTGCCGGGCTCTTAGCCATCTTCCTTG
GCTGTTTCCAGTTCAGTTGACTGAGTTCTGAATCTTCATAGAAATGAGGTGACCGTTTATGTCACCGCAGGGCCAGGTCTGTGAGTC
TACTGCCACTGCTCTGACACCCCTGTTGTAGGAAAGGGTGAGCTAAATATCCCAGTGGAGCACCTCCCTTCCTTCTGGAACTGGCC
TAATTAGGAATGAGGTGGCAGTGTCCCAGGGCCACCTTATTAGGCCACAATCTCTCTACCCTAGGCTACTCATGAATCCGAGTGAG
CTCCCTGCAGGTTAGTACCCTGGCGCACCTCCTCCCAAATGTTTTTCTCATTTCCGGTTTCACGGCTGTTTTGAATCTACCAGCTTC
ATTTCATTCATCACCAAAGCAAGGCACGCGATAGAAAAGGCGCATCCCGTCCTCTGGGCGCCGCCTCATTTCCTGCCTGAGATTGAC
AAGTGCCACTGAAGCATTTTGCACACCTCAGAGAGGGTTTGCCATTCGTGTGGCCAGAGAAGTTGTCATGCAGACAGTTGTAGGGGC
TGGGTCTGCCCTGAGGCCCCAGGCTGCAGACACAACTCAGACTGTTTTATGGACAGAGTCGGTTCTTCAGTCATGACTTGTCTCCCT
AGCCACACATTTTTAGCCTGATCGTTGTGAGCTGTCACAGCCTGCACACCTCTCAGGTTTTAACCAGTTGAACGCTCTTATAAAGTT
CTGTGGACTGGTGCAAGAGCATCTCTGTGTTCATGTGCACGTGCCCACGGTGGGAATGTGTGGAGTGTCCTTTCAAACCTTCAGCGT
GTTTCTGCTGGGGACAGGAGATGTGTACTCCATGAACTATAACTCCAGGCTGAAGAGCCCTGGAGACCAGGGAGGGAAGCGATTCA
GACCTGGAGCTGAGGCCAGGAAGAGGGGCCCCGGCTGTGCTCTGCGAGAACTGCGGACCTGGTGGGAAAGAAGGCTCTTTGAGCTCC
TTAGTTGCTGCTCATTGTAAACCTAGAGCATTTCCCAAGGGACACAGGGCATGTTTATTAAGTTTTCTTTCTTTATCCTTTTGACAG
TGGATTGAGCCTGGGACCTGGGCAGCTAGGCAAGCACTCTGCCAGTTACTTCTCCAGCGACTAAGCATTTTTTTTTTAACCATCCCAC
TCATCGCCTCCCACTGGCTCTCACGGTGCTATGACAGTGGGGTCCTCTTCCTGGTGCTCCTGCTTCCTTCCCTTTCTGTGTCCTTTC
CTCTTTCTTCACAGCTGGCCAGGCATCAGACAGACTCACACATGCAACCTTGACCTTGACCTTGCCTGCAAGCTTCCTGAGGTTACT
TACTCATGGTCCTTTTGATACAGTTTAGACTCCATGAGACAGCTTGTGTCTTGGAGCTGGCCCCACCCTGCTCTGAAGCCATCTTTATG
CCACCTCCTTAAACACTCTCCAGTCACTGTGAGGTTCTGTCTAGCCTTGGTCCCATATCTCTGGACCTTTGCACATGTCATGTATCC
TTTGTTCCAAATGCCATCCCCTTGTGTGAAGTATCTTTTATCTCCCCAACTTCTCTCTTCCTTGTGTACATAGACATCTCCTATGTC
CTTGTCTGCCATGCAAAAACCTCTTCTTCCCTTCCCTGAGCTCTTCAAGGGCAGCCATGGCCATACCTCTGGAGCTCCAATCTCTAT
GGCATCAGACAGAGTAGGGGCTCAGGGAGCGCTTGGTAAATGAATGGATCCCCACAATGTGTACCCATGTGTTAGGCAGCAGTTCAGT
CGACCTCCGACCTGAGCCACCACTCCTAAAAAAGCCTCTTTTTATCTGCAGGGACTCAAACCACCCATGTCAGAGGTGGCTTTGGGAT
ATGACTTCATGTTCCCTCATATGTCCACAAAGCATATGTCTGAATGGGCGACTAGATTCTTTGCACAATATCAAGCCAGAGAAGGGA
CCCAGCTCCCCGTGATACAAAAGGGAACAAATTCCCAGAGGTTTGTTTGTTTTTGTTTTCTTGGATGATAAAAATGTGAGCGTCACA
AGGTCACAAGAACACAGAGACTGAGACTTTGGGACTTGGCCATTGTGGTCTATCTGTTCCCCTGGGCAAGATAGCAGGGTTGTTGTT
GTTGTTGTTGTTGTTGAGACATGGTCTCACTATATGGCCCTGGTCTGGAGTTTGCTATGTAAAACAGAATGGCCTCAAATTCACAAA
GATCCACTTGGCTTTGCCTCCTGAGAGCTGGGGGGGCGTCACCTCTTCCAGGGCTCCATCAGTGGCATTTCAGAGAATGTTTTGTGA
AAGTATGAAAGAGCTGACAGGATGCCTGCATGCTTCAGTAACTCCAGTGCAGACTCATAATGGACCCACCCAGTTGTTTGAAGTCCT
GGCATGAGTACTAACTATGAAGAAATGGCCTCCTTTCTTACATAGAGCCCTGTTCCCAGACTGGGTGAGAAATCGCTCTTCGCAGGT
CAGTGAACAATGAGCTCTTCTACATCTTCCAGGCAACTTGTCATAGAGTTCCTATGCCGCACACACAGCAGACATGACCAATCTATC
CCACCATCCTTCCTCAGTGAGCTTATGGGCCACTGCAGTGTGAGATGCCCAGCCTGGAGTGCCTGCAAACCATTTTTGGTCTCCACT
CCTTGAGGCTCTGTCCCAGTCATGACTTTGGTCTTTGGCGTTTCTTCACTTCCATCCCCTAACCTGGCTGCTCTGGCCATTCTTGGA
GCTATTTTACTACAGTCATTTTGTGTTGTGTTTCTGCCACATCCACAGCAGTATGGCAGTAGCCCCCAGCATCTTAAGTAAGTTACA
GGATATACTATTTTTCCATTATTAAAATTATTAATGATTCATTGATATTTTGAAGAGTTCATTAATCACGCTCATTCATGGTAACTA
TTAGCATGGTTTATTACTTTCAGCTGGGGTCTGTCTATCTTCCAGCCTGGTGATCTTGCCCTTGGGCTGCACTGGAGTCTTCTGACC
TTCCTGGTTGCTCCCCTGTGCCTCACCACTTCATCTCTTCTGTTTGCTATAAGGTGCAGTGAGGGCAGCTGGGGTAAACTTCTGTCT
GCCTCTTACAGGCATTTGGGCACCTGCCTGGCCAAAAAGCAAGGTGGGTGCTTCCTGTCTGGCTTTCTCCCGGGACGGAGGGGAGGA
GGCCACCAGCAGTTGTTCACCGCCCTGCTGCTTGAAGACTCTGACTTAGCATTCCTGGTTTGCATGTCTGACACCTCACTTGGCACA
TGATTTATTACAGTTTTAAATAGCCCTTTCTTTCTGGATTGAACTGTCCATATCCATTTGGAGTGGAAAGACCTGGCTTGTGGTGTT
GGTAGTGCAAATGCTTTTCTGAGTGCGGATTGTTTGGGGTTTTTTAGTGTAGCTGATGCTCTCAGACCAGAGTGCTGCTCCCTGTGT
CTGGCTGGATCCAGGACAGAAGCAGACATTTGCCTGCCACCTGGATGAAGGAGGACATGGATGCAGAGAGCCTTTTGTTTCTTT
TCGTCTACATTTCAGAGACAGAGCATCCCAGGTGAGGGAAGGAAGCTTTATGAACAGCCTGGAGGGGGGTTCAGAATCCATCCGAGC
CATTCCCCTGCCTTTGTGGTGGGCTTTACCTCTAGGTCTAGGCTTTGTTAGGAAAACACTTCCCAGGGAGAAACTTTCCTTGGTTTC
TCTCTCTTTCTCTCCCTTCCTTCCTTGGGGGTTTGGCAAGTCCTTCTAGGCAAATCCTTTTTGCCGTAGTTAGAGCACCTCAGGGATA
GTTAAACTGGAGTACAGGATGCTTACAACAAAACATGCAAATTCCCCTTCCCATTTCAGAGACATATGTCCCCTAGATGTCACCAGA
ATTAGCTCATCAGATTGTAATCACCATCCTGGGATTATGATGCTGTGTGGAAAGCAGCAGAAATCCTTTTGAAACCCTTTCTCCTCCAG
GGGGAGGGGGAGCCCTACCAGCTTCCTGCAGAACATTCTGGTTCCTTTTGTTATCCTGCATTTCTCGACTGAGATCCACTAACAGCC
CACATCCTCTGTGCTGCTGGTTGAAAGGCCATTAGTGTGGTCCCAGTGAAAAGAGAGAATTTGCATGGCTGTAAGAGCCTAGCAG
AGAGAGGAGCTGAGGGGACAAGCCACCTGGGAGCTGCATGAGTCTCAAAGGGAGGCCCACCAGCCACATCCAGACCCTTCATCCCTC
ATCAAGATGCCACAGTGAGGGCCACCAGCCCATTTCCACCTTAATTCCATTGGAAGCTATACTTCTCCCTGGAGATTCTGACCCCTC
CATGGCCATGAAGCAGAAACCACCCATCATGGTGGCTTGGGTTGTTTGTTTCTTCCAAGGGTTTCCTGGCTAGTTAGAAGGGTGATT
TTTTTCTTCAATATGGGATGAAAGAAAAAGACAAGATTTCTGAGGGTTGGAATCATCCAAGGAAGGAGAGTTAGTTTCTCGAGGTAGG
TGTGTCTTCCCCTTCCAAATTCACACAAACAATTAAGAGGTTGCAAGAGTTCTCTGGAAAACTACAACCTACTCATTTGGCCACAAG
AGAACACAATAGTTAGTGTCCACATGCTTTAAGTACGATGTCTTTCTTGTTGGAGAGAGCTCTAGGTTATCAGTAAAATACACAGCT
CCACTTGAAAAGCTTTTTAAAAAAAGAAACCCAGCTGAGAGCCCCTTCCTCTTCCCGCTCACGAGCCCCCACTCCTGCCTCCTCCTC
CGTTCACAGACGTGCTCTTGATCCATTCAGTTGTCCAGGGCAGACCCTCGTGGAGCCCTACTATGTACCAGAGTTATAGAACAGACAGA
ACGACGGACTGTGGCTCCTGCCCTGCAAAAACTGTTAGCACCGAGCAGGAAGTCTTTCTTTTTCAAATTATGGAAAAATTCACATAT
TATAAAATTTACCATTTTGGTTTTTTTCCTGAGACAAGGTTTCTTGTATTCCAGGCTGGTATCAAACTCTGTATGTTGCCTGGGAGGG
AAAGGGGGAAGTGGTGTAATTATAAAAGAGCCATCTTGGTGGGTTTAGTCAATCCCTTCCCCCCACCCCCCAACCCTTAATCCCTAG
ATTAAGTGTAGTTAAATCAAGGACCCAGAGATGGGAGCCAGTCCTGAGTTATCTGAGGGCTCTGCTGACCACAAAGTTTCTTGTAAA
CAGGGAGGCGGAGGAGGCCTGACCCCAGAGGAAGATGTGAAGCAGAGTCACAAGAGGTCCAAAAGACTGGGAGGGAAGGAGGAGGGGA
CTGGAGAGGGCCAGGAAGTACAATTCCTCAGCCACCCTACACGACACCTGGACTTAGTGCAGAGTCATCATCTGATCTCCCGAACTA
TGAGACCACACATCTGTGATGCTGTGGTGTGTGGAGGCCGCAATAGGAATCTGACACATGGACATGCTGGAAAGGCCGGAGAGGCTG
GAGAGGCCGGAGAGGGGTCGATCCAGGCTAAGCTGCCGAGCAGCTGTGTGATTTAGTCTCCTCGTGCCTCAGCTTCCTCCTCTTTAA
AAATAACTGGGCTCCCACAGCGGTTTGTTAAGGGTATGGAGTTAATATTTGTAAGTCACCGAATGCCTGGAACCAACAAGTGTGCAT
TTGTCAGATAAATAAAGCCCAAAGCGCCCAGCCAGGTCTGCCTCGTAGGTCTTCAGTGGGATGATCTCATAGCTGAGTCAGTGACTTG
AGTCTGGTCAACTGGGAGCTGGAGCCGGCCAGGAGGATGGCAGCTTTTGGAGCCTGCTGCTACATCATTAAATCGGAGCACAGATG
CTTGAAGGGATGGCCCCTCTATTCCCCGTCACACCACACCAGGCCCAAGTACATCTGCGAGGCTGCATCTCAATGCTGACCAGTCTG
CTTCCCTTTGGGAACCACAGAACGAGACCAGTGGATTCTGGGAAAGTGCTTCAAACAGCCAGCACTGGCCCCTGGTTCCTGTGCTTC
TCCAAAGACCAGGGATCTCTGAGCCGGTCTGCAAGCCCTGGGTTGGTACAGAGAGCAAAAGAAGGCAGGACAGGCCAAGAGAAGGCA
TCATCACCATATGCCTGACCTTGGAAGGATCTAGAAGATATGGTTGGCTTCTACTGCTGCTATAATCCACCTCTTAGATGGTCAG
AGGCAGTTGTGACAGATCCATTTCTGACATTTGCAGGGCTTACAGGTCAGCCTCACATGGCAGCACCTAACATCACTTCAGTCGTCA
CACCCAAGCTCCGTCAGGCTCCCCACCCCCAGGTATGGGGAGCATCTCAGTACCATGTGGCCCCGGGGGGAAGGCTTGGCTCTTTGG
ATAGGAAATTCTGGGGTAACTGGATATCTGGACATGAGCTTTTGAGGCGGCGTGGCCCATGGGTTTCCAGCTCTGTGAGATGGGGCG
GGGCAGAACCCTCATGTGGCTTATAGATCAGGAGCCCTGGCATGTGGGTCCAGGGCAGTGGTGGTTATCAGTGCAGTGGTCAGGGCG
```

```
CTGGATGGTAACTAGCAGCACTCTCTCCTCTGCCCTGTCACCATCGCAGCCACCCCAGTCTGAGCCTCACTTCCAGCACAGGCTTCT
GTGAGACAGCTCTAAACCTGTCTCAGCGTGTGTGTGTGTGTGGGGGGGGGGGTGTCACTTGAACCCTACAAGAGGCCCAGGGACTCT
GTCTCCTTGGTGGTTTTGCCTCTGTGGCTTTCTTCAGACCCTCCCCTGGAGGGAAGGGGCAGTGGTAGGTGGCTGATGAGTCCAGCG
GTGAGGGAAAGCACCTCCTTTGGAATAGGGAGCCAGCTTCCATGGTACTGGGCAGGCTGAACACGCTGTGGTAGCTTTCAGGACCTGT
AATGGGGGTCGGCCTGAGAACAAGAACCTTGTTTAATTCTTATGCATAAGAAATCTTTTTCACAGGGTGGCTTAGTCCGGAGGAAGTC
ATGGAGCTTGCCTTGGAGCAGAACACTTTGGGGACCCCCAGGAGAGGCATTGTCCAGCAGTGTGGGGGGAGGCTGGAAGCTCTGCAG
AGAAAATAGCAGGGCGGGGGCACACTCAGAGGATCAATTTCCCTGCAAGAATGGCCAAGCTTAAAAATATCTTCAGTAGGCTCCAGG
AGTGGCTTTCCCGGGAGAGTTCTACTAAAAAAACACCCCATTTTATTAACTTTTTAAAATTACAGAAACAATATTGGCTTGTTTTA
GTGCAGAGGTGTGTTCTAACTATTAAAGGTTAAAAAAAAAAACAATAGTTCCCTCGATTCCCGTTCTTACCTCCCCAGAGATGTAAG
ATGTCAACTATGTGATATTAGCCAGAGGTGGTCTGCGCACCTGTGTGCATATCTGTTCACACTTACCAGAAAACCAGGTCACCGTGT
ACACATACGGAGAGATGCTTGTCGGCATGCTGCCGATATTAAAAGGGGGACTCACACTATGCCTCACTGCATGGTCATTTTCACTGT
TTGCTTAGCAATACACTGTGAACCTTTGCTAAGTTAATACATATAGACCGGCTTCATTTTAACCAGCTGCATAATGTTCCATAGTGT
GAGCACCATGGTTAGTTAACTGGCTCCCTACGGATGTGTATTCGGGTCATTTTCCAGGTTAAAAAAAAGATGTTAAACAAGCAGCTC
TTTAGAGAATGCTATTTGATTTCTTTATTTTGGGCACGTCTTTATTCTCTTGCTTTGGGAGAGTTAGAGAGCTGGTGGTCTTGGGAG
TTCTTTTTCTTTTCTTTTCAGTCACATTGACATAGGTGAGAACTTACTGCATCTAGATCCATCTGGAAAGCCCATACTTTCCTGAGCT
TGTGCTTGGCGCCCTGGCCACGTGGAAACCAGTCCTCAAGAGCACCGTGTCAGCCAGGGGCCGACAGGCAGCAGCAGGTCAGGTATC
CTTAGTTCAAGCAGGAGCTGCTCGCTTGGGAGCAAGCTGGAGACGGGGGGTTGTGGCTGGAAACAGGTGTTTATTAATGAGGGTGTGG
ACTCCATCTCTCTAACCTGAGGTCTTGGGAGACCCAGCTTAGCTGTGGGCTAGAGACCCAACAATGCTCTTCTCGTGGGGCCATTGC
GAGGAAAGAATGAGCGACTGCACCCCAAACTCTGTAGGACAAGAATAGACTCAAGCGATCTAGAGTACAACGGAGTGACCAGAGTAC
ACAATAATCTGTTGTGTATCCTATGAAGCTGTAGACATAGGGGTGTGTAAGAAATGGCAGAGGGACAGGGGAGATGGCTCAGCAGTA
AGAGTACTGTTACTTTTCCAAGGGTCCTGAGCTTTGAATCCCAGCATCCACGTGGCAGCTCACAACTGTTTATAACTGCAGTCCCAT
GGAATCCAATGCTCTCTTCTGGACTGCAATGCAAGCAGTCAAAACGCCCATATATAAATAAATAAATCTTAAAGAATTGGTAGAGAT
AAAAAGCTAAGCCCCTCTATTGGCTCAACACACATGTATTAAAGTATCACATACTGTGCCCCATAAACATGCAGTTACTACATGTTA
ACCAGAAAGAGACAAAGAACCATCACTCCATCAGAACTGTGCAAATGGAGTAAACAAGTGGCCAGGTGCTCCCTGGGAGAGCAGTGA
GTGCTCCCTGAGGGCTAGTACTTCAGAGAGTCCTTTTTCATTTGTTTACTTTTAATTTTTAAGAATTACATATAAATGTGTTTATTT
AGCTACTCATGTGTGTGTACATATGGGCATGTACATACCACAGTGAATGTATAGAAATCATAGAGCAACTTCCAGCTGCTGGCTCTC
CCCTTCCATCCCTGTGGGTTCTGGGGGTTGTATTTAGGTCATCCATCAGACAGGCATCTTCACCCACTGAGCTGTCTCACTGCTTCT
TGCTGTCTGTCTATCTGTCTATCTGTCTATCTATCTATCTATCTATCTATCTATCTATCTATCCATCTATCTATCAATCGCAGGGTC
TCACGTGGCCTATGCTGACCCTGTACTGCCTCGTGCCTTTATTTAATTTGGGCAGGCTCTCGGGTAGTTCAAGCTGGCCTTGGACTC
CCAGGATAGCTGAGGATGACCTTGAACTCTCGATCTTCTTGTGTCCACCTCCCAAGGGCTGAAATCATAGGTGTTGCTCCTACATCT
GGTTTCTTTTTGTTTTTTTCTGAGACAGGATCCTCTCTGTCATTCAGGATGGCCTTGAAGTAGCTGCCCGGTGCTGGCATTGCTAGC
CAAGGCCTCCGCAGCTGGTCCTGGACAGTGAGTTGAAGAGCCCTTTAGTCGGCCCATGACACCAGCTCTCAGATCCCAAGGGTGC
TCAGTGATTCCAAACCTCTTGACCCGAGTTGTTGAAAACACCCTTTAAGCCATGGCTGAAGAATCTCCAAACAGTAAATAGGATGGG
TTACTGGCCTGGTGGCCGGTGCTCAAATGGTTAACCCACTCCCCATCCTAATCTTCCCAAAGATACTTTCCTGTGTGTTAAGCTAAAAC
GAAAACTTAAGAAACTTCCTGTTTGGAAAACCATTCTACTTACCCCAGGCATTACAGGTGCCAGAAGCTGCCACTATCAAGGTAGAA
AGAATTGGAAACACAACACCAGCTTGGTCCATGATAGGCAAACACCAGATCACCAACCCCCCAGCATAGCCTGGGCTGGGCTGCAGT
GGTCAAGAACATAGACGTACAACAGAGTAGATTTTAAAAGAGTCAAAACCTGCCCTTCCTTAACTATATGACCATTATTAAGTTCCC
ACAATTTTCTGAGCCTCAATTTTCCATCTGTACAATGGGACTAACCACACTTTTGGAACCCTGTTCAGGACTGAATGAGATGGTGCT
GTAATGCGCAGTTTATAGGGAATGGCATTGAGAGAGCACTGTTGCTATTGAAGGGGGTAGCTTGGAATTGGGGGTGGCTGGCACTG
CTAACTTCTGGCCATTCTTTCCCAGCCTCTGTGAGCTGGGGACCAGTACTGGGAAGACCCTGGGAAAGGTGTGAAGATGAGTGGAAC
TGGAGAGATGGATCAGTCCTTCAGGGTGCTCTCCCGGGAGAAGCAGGAGAGTGGCACAGTAGTTGCTAACACCCCACGGGACCCAGC
TTGCCATCTGACTGTGGTGCTGGGTGCACTGTGGCTGATGCAGGAGGGAGGTTCAGCTGAGAGCAGGTGGGAAGAGCTCTTTTTTT
TCCAAGTGAGCTAAGTGGTTGAACTTGACCCAGAACCCCTGGCGGACAACTGGCAGGGTCCAGTGAGAGAAGTTATGTGGCTTGGT
TCTGTTTGGGAACACTTGGCACTGGAAGGTGGCTGGAATGGGGCACTTGCTAAATCTAGGGCGTGGCCACTGTAACCAAGTCTGGTT
GAGTCACTGTGGCCTTACTGCATTGAAAAGAACTTGACAAACTCCAAACCTACCTGAGGAAGAAGGCCTAGTGTCTGCAGAAGTCTC
GGGGAGGGAGGCTTATTGGAAACTTCCAGAGCCAGTCTCGTGTTTCTGCGGAGTTCACGCCTATCAGGCACCAGGTCGTCTCCATTC
CCTCCCTTCTCTCCATAGGCTGAGCTGTCCTGGATGCCGGGTAGGACGCCATTGTCTGCTCCCAGACTTCTCTATTTCCTTCTCCGG
TCTATTTTCACCGCAGCAGGCATCACGTAGGGAATCCCATGGTGCATGTTTACTTGTTTCCCCACAGCGCATGCTCCGTGGCCCAGG
CTCCACTTGTTTTCTGGTTCTTGGAGTTTAGTCCACAGTCAGAACCCAACATGGTGGATAGATGGGTGGACCCATGAGCCCTTAGAG
TAAAGCTCCATGCCTGTGCATCCAGCAACATAACATTTTCTTTTTGTAATTTTTAATGGATTTGGCTGTTTTGCCAGCATGTACATC
CGTGCTGGAAGTGGGTGTAATGCCCATGAAGGCCAGAAGAGGGCACTGAATCACTTGGAACTGAAGCTTCAGAAGGCTGTGGGCCAC
TACAGGGGCGCTGGGAATCAATTCTGAGTCTCGTATAAGCACAGCATCTCTGAGCATCTCTCAGGCCGTGCATTCAGGAGCGTTTGA
ACGTGACCTCTCGTTCTAACATCAAACCTTGTCTTTAACCACAGCCAGGGGAAATCAACAGTTTCCCTGCTAAACCGGTGGGGCACG
GAGGTCGTACAGAGATCTGTGTAGGTGGAGGGGAGTGTGTTGGCACTGAACAATGTTCTCCACCCTTTAGCCTAGAAAGCTGAGTTT
CGTTCTCATATTGACCTCACATTAAAAAAAAAACAATTCATTAGTAGGAAATGGAACGCTGCAAACTGGATCCAACCCTTGTGTTCCAG
ATGAGGAGTCTGAGGGCCAGGGAACTGAGAAGCACTTGCCAGCAACAGAGCGCTCTGTCCCCATTGGCCCTGCTCAGGGATAGACTC
TTGCTGTGTGACTAAAACCTTCTCACATGCTGTAGCTTGACTTTCATGCCTCCAAGAACTGGGGATGGTCACAATGATTTATGGTCAT
CACTGGCTTCCATTAGCCCCATGTGAACTTCAGACGCCAAAGTCCCAGAGGGTTCTCATGTCTACAGCAATAGACTAGAGAGTTCTC
AGGGGCACTTAGGAGGACCTTGGTCTGGCTGGCCCAAAGCCGCCATGGGACATCACACATGGCCCAGTGCCACCAGCCACAGGAAGT
TAGTTTCAGGTGGGGAGGATTTCTTCTTAGAAAGATGCAGGGCAGGAGAGCAGCACTCACCGTGCTCCGGCCTTGCTGTTGCCGCAT
GATGGCTTTTCTGACAAGGCCAAACAACGTGGGTTTTTGGTTTTGTTTTGTTTTGTGTTTGAAATTTTGTTTGTTTGTTTTTTTGG
GGGGGGGGGTCTTGGGTTTTGTTTTTTGTTTTTTTTTTTTTGTTTTTTGCTTTTTTAAAAAAAATTTAGTTATTTTAATTTCAC
GTGTTATTGGTGTTTTGTCTGCATGTTTTTTCTGTGTGAGGATGTTGGATTCCTTGGAGCTTTGCCATCTGAGTGCTGGTAATTGGACC
TGGGGCCTCTGGAAGAACAGCCAGCACTCTTAACCTCTGAGCCATTTCCCTAGCCCAACTTTTGCTTTTCAATTTTAATTTTTCTTC
GTCCCTTTCCTCATTTAGCTCCATGTGGCTTCTGCTTACTGGAAATTTTATGTTCATGGCTGCTGTAGACCTGAAGTTCATCTCATC
ACCGACAATCAGATTTTGTCCTCAGGGAAACCCAATTAGGGATCACAGGACTTTTTGATGTTGTTTCCATGGTAACCCCATCGTGAT
GGTGCACATACATAATATATAGTCTAAGGAGTTTATGTTTATTTATCTAAAAGCTATATATTAATAACACATTTTAAGAAAATTTTG
ATTTTTAAAATCTGAAATGTTATCTTCTGCATTAAAACATCATTCATTTATTTTTAACTTTGTGTGCATTGGTGTTTTGCCTGAATG
TATGTCAGTGTGAGGATTTTGGATCCCTGCAACTAGAGTTACAGACAGTGGTGAACTGTCAGGTGGGTGTCGGAAATTGAACCCAG
GTCCTCTGGAAGAACAGTCAATACTTTTTTTTTTTTAACTGCTGAGCCATATCTATAGCCCCTTCTTCTGCTTTTTTTTTTTTAAA
TGATGGAAAAATCCCCTGAGGGTTTTTCTCCCACAGAGGGAACATAAAAGTTCCCCTCCCCCGTCTCTTCATTTTTCTCTCTTTAAT
TTAGAAAATTTTATAGTTGAGTAAGAATGAACGCTTTTCATTTGGCTGGAATCTGTCTACACATTGTCTCCCTTGATGGCTGAGACAAT
GTTTTATGAGACAATTTTGCTCAGATAAAGAGCGAAGGGCCCAAGTCTTTCAAATGGAAGGGAACTACTGATCTAATGACAGCAATT
CAGACACACTGCGACAGACAGGACAGTTAAGGCCTCGCCTGCTTCCTTGCCAGGGCAGCCACTCCCCAGCCTGCAGCTGTCCCACAG
GAGGCTCCAGGGTGTCTGGGTGAACTGACCTGTTCTGAGGTGACCAGATGGCCCTTCAGAGCTGGCTGTCACGAGCCAATGATGATA
GAGCTTCTCTGAGAGACTCTGTGTGGAGGAAGTAAGTCGCACCCTAGTGAGCTGGAGGGGCCAGGGGCTCCTCTGAGCTTAGCCTGT
```

86

EP 2 204 376 A2

```
GGAGAGCAGGCATCTGGGGCAAGCTCAACGCTCGCTGGCACACCGTGTGCTTTCTGAGATCATTTTCTTTGGTACCATCAGTGGCTA
CTCAGAGTTCGGATACTCGCAATGGATACCTTCGCCCCTGCCAGCTAATCCCGGAACTCCCTAGATCTGCTAAGGAGACGAGCTGAT
CTGGCACTCCATAAATTCTGCTCACCAGTGAACTTCCTTAAACACTTCCAGAAAATGCTTTAAAATAACGGAACAGGTCAGAAAAAT
AACTCTTTAACACTTTATTTCTTGGTGTCTGAAATGAAGTTTCCGTAAGGTGGCAGAAGGAGGGATGGCTATGATCTTGGCCCGCAC
TCCCCTGGACCGGCCAGCCCTATAACTACAGGGGTTAGAGAGTGTCAATAGGTATTGTGGGCTCTTTCTAGAGCTCACTGCAGGAGT
ACTGAAAAGGGAAGTGTCAGGTGTGGAAGGCCAATCACCTAGCTGTCCTAATATCTATAGGCACACATCAGTATAAGTATATGTTGA
TGTGGTATTGTATAGCAATGCCCCTGAAAGACAATAGCTTAAGAGTACATCTGTGTAACTGGGCTCAGTGTGGTGGTCCTGTGCAGA
GCTTGGTCATGTGCTGGTCTAGGAAGGCCTTGCTGTCCCCCCTGACAAAGTTAGGTGAAAGAGAAAACGAGGCCATATATCTGCAGC
AAGCCCCATAGAACTGATCTTCTAAATGTCAGATGCATCGAATTTTATAACATCCCATTTCCCAAAGAAAGGCACTCGGTCAGGTTA
AACTCAAGATGTTGAACTATAGGCTATGCCTCTTGCAGGACGAAGAGGCAATGCCATACTCACAGTAGCAAGTGGCTCTGTGGACAT
TTTCTGTAATCGACATCTGGTACTTGTCTCCAAGCAGTGCTGAAATCCCAAGTTCTCCTTTGAGGGTCCTCATGTGCCTCTCTGATG
CTAGAGCCACTTGAGACTGCAAACTGGCACCCGGGACATGTGTTATGTCCCTACAATGGCCTAAGCTGGTTGTTTCTCGCTGGTTGT
TTCACTGAATTGAGCTGAGTTGAACTGAATTACTTCTGGCTTGACTCTAATTACTGCACATTAGGAATCACTCTGTTGTGTCAACCA
GCCTCCAGAAGCTCTTGGGATTGCAGTAACTCCCGCTCACAGGAATAAGAGAGGTAGTAGAAGGCTCCAGCCCCGAGCAACCTCCCA
CCATCCGAGGAAGGAGTAGAGTTTTGGGAAGTCCACTGGCTGTGGTGCCACATGTCCCTTCTGCAGACTGACCTCCCCCACCCCTGG
AATGGGGAAAATAGCTTTTCCAGTGTGAGGCAGCACAATTCAGCAATTTAGAGACAGAGTTTCTTTTCCTACCCAAGGAGTCAGTGG
ACGGAGCAGGAATGGAGGCCAAGCCCAGGCAGGCAGGGCAGGAGAGAACTTCCTTGTCTCTGACATCTCTCCCTTCCAGGGCTGTCTGCA
GCTCTGCTCTTGCCGCCTGTCAGCATCACCCAGGGCCTTTCAAAACCCACCAGAGGCAGTCAGGCTGAGCCATTGAGATCAAGCCC
CGGGGGTGACTGGTTCCTGGTGGAGACAAGGTGATTGGTTCCTTGCCCATTCCCCCCAGATCACTCTGATCCAGCACCACTCGAGAT
TCTGACTCCAGAACCAGCAAGATGTTCAATAGGCAAAGGTGCTCAGCCGCAAGCCTGATGACCGTGCATTCAATCCCCAGAACTCAT
GGTGGGATGGAGAACCAGGTCTAGCAAATCGTCCTCTGGCTCTCCACACAGGCACGTCTGCATCTCCACAAACGAACGAACAAACA
TAATAAATATAATAATAGACGTCACTTTGAGAAATAATACTAGCTGCCTGTTCCAGGTTAGCTCTGGAACTTTCTGATGTTGGAGAT
CACCTGGGGCAGTTTGTTGCAGAGTGGGCCATCCTCTGGGAAGGGGGCAGCATCCTCCAAGGTGCAAGATGTATTGCCAGGCCCTTT
GGCAGCCATAGGCCTAGGGATGAATTTCCTGCAGGAGTTGTCACTCATTCAACAGAAGCTGACTTAGTGCCACAGTGTGCCAGCAAT
GCTGTAAGCCAAGCGAGCCAGTATGGGTTCTGCTCTAAGGAGCTCACAGAACACACGCTAGGTAGGAGGCGCCGGCTGAAAAGTGCC
ACCGAATTCTGGCTCCTTAGAGCAGGATGCATGTGCCAGTTAGGTTCCCCCATCCCCAGCTTGACATAAGCTAGAGTCAACTGGAAA
AAGGGTGCCTCAGTTTTAGAAATGCCTCCATTCCTTGATTAATGACTGACGTGACAGAGTGGGTGAGGCCACCCCTGGGCCAGTGGT
CCTAACAAAAATCAGGCTGAGGGAGCCTGGGAGGAAGCCAGTAAGCAGCATTCCTTCCTCCATGGCCTCTGCGTCAGCTTCTGCTTC
GGCTCCTCCTAATGTAATGATAGTCGGTAATCTGTAAGCCAAATAAGCCCTTTTCTCTCCAGGCTGCTCCTGGTCCTGGCATTTTAT
CACAGAGACCGAAACCTAAGCACAGTGCACACTGGGGACCATCATGAAATCTTTATCTTCAGAGGAGGAAACTGCCCAAGAGAGGGAA
GATTAGCTGCCAAAGTCACATAAGTGGTAGCATTTAATAAACATCTACTACTTGCTAAGATTGAGCTTAAGCATTGGCAATAGTAG
AGATCAAAGAAGCTCCCACTGAAATCACTGGGTCCCAGCCCTGGTTATCCTTTGGTATCCAGGAGCTGTAAGAACAATCCAAGGC
TGGGCAGTGGTGGCGCACACCTTTAATCCCAGCACTTGGGAGGCAGAGGCAGGCGGATTTCTGAGTTCGAGGCCAGCCTGGTCTACA
GAGTGAGTTCCAGGACAGCCAGGGCTACAGAGAGAAAACCTGTCTCAGAGGAAAAAGACAGAGAGAGAGAGAGAGAGAGAGAGAGAG
AGAGAGAGAGAGAAGGAGGAGGAGGAGGAGGAGGAGGAGAAGAAGAAGAAGAAGAAGAAGAAGAAGAGGAAGAGGAAGAGGAAG
AGGAAGAGGAAGAGGAAGAAGAAGAAGAAAAGAAAAAGAAAAAGAAGAATAACTCCAGGACCCAGGATGCTGAGGCAGGGAGATTGTAAGTTCAG
AGGCAACCTGGGCTAGATAACAGATGCTATATCAGAAAATAAGTACGAACACTCCAATGTCAGCCCCTCCCTGACCCACCTCCCACA
GGTTCTTGGTCTGAGAATGGACCTTGGCAGAGGGATGCTTTTCTTTCCCTTCCCCCTTCCTTCTTTCCCCTCCCTCCCCTTTCTTCT
GCTAAGCCCCTGTTGTGCAAGGCAAAGGGATTTTAACAGGCCTCCAAAGTGGCTCTGATGTGCAGCCACTTTGACAGCCATTGGTCT
AAGTGACAAGACGGACAGAAATGCAGAAAAGGCCAATAAATAACTGAAACTTGTGATTGGCAAGGAATGAGCAGGAGGCAGAGGAGC
TGCAGGGTGATCTGCAGGAAGCGAGGTCACACCACAAAGCTGGTCACGGGGTAGAAGGCACAAGGCTGTCCTGTATAGGAGAGC
CGTGGATGTTCTGGGAGATGCCCGGCTCTACAGAGCCATCAAACCCTGTCATGATGCCGTGTTTAGTAGGTACTCTCTGTCTGCAGCC
TGAAATAGTTCTCCTTGTTCCTCTACTCCAAACAACCTCCTCCCCCAAGTACACACTGCTTATAAATGCTCTAGCAGCAATCTCTGT
GATCCACCTAGAGACTAGTCAGTGGAGTGCCCAGGAGCAGGTGTCCCATAATGGAGACAGTCGAGCAGACTCTGAGGTCTAACACCT
GATGTGGGAGGTTCCAGCCAGCTCACCAGCTTCTGACTTACTACGCCCATCTTCCTTGCATCAAGAAACTGAGAGGCCAGTGGGGAC
AGCACCCCAGGCCAGTTCTTTCCTGTTGAGCTGTGTAGCTCAGTGGGGGAGACCCCATCCTTCCGTTTAGATGATGTATCAGCTCAGT
CTCCACCAAGCAATCAAAGGAATCCGTTACTCATTGCTTCAGTTCCTACTGCATGAACCCAGTTACTATAGCAACTGAATGTGCTTC
TTTATTACTGGATGGAAGGCTTTAATTTTGTATTTAAAGAGTTTGGCCAGAGAGTCATGTTGCCATGACTCGGGCTGTTGGATAGCA
GTTAGGAGCAGACAGACACCTTGGAAATGGCCATAGATCTGTCTTTCTAATTGACAGATCGGGGCCTGGGAAAAACCTAGGCTGCACT
GCCCTGAGAAGTCTCCCCTGGTGCTGCGCTGGTACCTTCCCCTGTCCACGGATGGTTCCTGATGAGCAGAGGCCCCTGAGCCAGGCA
TTCCCATGGTGGCCTCTGCTTTCCCTACTGAGCTTCACCAGGGGCCTTGCTTGGATGTTTTTGCTTTTACCCTCTCCCTACAGGCGG
CAGGCCTCTAATGTGTTTGCTGAATACTGGTTTATGACTTCATTGAGTTTCACATTGGTTTATGGCCCTGTATGGTCTCAGGACAGGA
AGAGGGGAAGCTGAGGCTCAGTGAGGTTAGAGTCTGGCCCACCCCACAGCCTCCCCTTTTGCCTCTCAGTGGGCTGCTAGGATGTCT
GGAACTTGATCCCACAGCACACAGATGTCACAGGCAGACACTAAAAGCAAGAAAGGCCATCTCCACACAGCCCTCCACGGCACACAT
GTGCTGTCTACCTCATGTGACTCACCTGTTTGCCTTCTATTTGTATCTCGTGTGCCAGGGACTCTTTGACTCTCAGGTTTTAGAGTC
TTCTCCTTGGATGAAGGGGCTCATGGGAAGGCAGTGGAAAGGGGTTAGGGGGATGGCTCTCAGTGACTGCCCTTCGAAGCAGAAGGGTAGG
GTCCAGACCTTGGAGGACACAGAGGTAGTCCACTGCAGGCAGAAGTGGGCGGAGCAGGAGCCTTAGTTCATGTAATCTGTGACCAGC
CCTCAACTCCCCACACAGCTGTCTTTTCTCCTCTGTGAATGCTGGATTTGTGGTTAAGCTCATCTTTCCCCAGCTTCTACAACATCA
TCGGCTTCTTTCTCTCCCATCCTGACTTTTCACTGGCCTTTCAGAACTGGTCCCAGGCAAGATTCAGAGGTCACTCTCCCTTCATGA
TGTGCAAGTGGGAATGCCTGTCCTCCAAGGCTCACTGCCCCCAAACACCAACCAAGTTAGGCCATAAGACCTCCACCCTACGTCTTC
TCTAAAGGCTAGAAGAAGCCATGTTGGAGGGCAGTAGTTCCCTGGGGACCTGGGCAGAACACATGGGGTGCATGGCAGCAGATGGGCA
TCCAGGGAGATGGATGTATACAAGCAGCCTCTGTAGAGGGTATAGCGAACACCACCATTGTCCTGGCTATAGCTCCAGCCTTATGCA
GGCCCAGTTGCTTGGGACAGAGTTGCTGTCAACAGAGAAGACCAAGCTTGTTGAAAGGCCAAAGTGGAGTTCCTGAACAGAACACA
GAGGAATCCCATAGTAGGGCTTCCACAGAGACAAAGCCTGGGCCTCCCTCAAGATGTGAATCTCAGCTGAGTATGCACAAGGCAGTA
GTAGACACCCAGGGAATGTCTGCTGGAGTGCATCCTCATGACATGGCCCCAGGATTCGGGGCTCTGAGGAAGACTGGGGTTAGAAAG
AAATGGAGACAGCCGAACCCGGCCCTCATTGTATGGAAAGAACAAGATACGGCTAAAAAGAAGGCATAGCTAATATTAACAATAGCT
AATAGTGACTGCTTTGCTCATGGGGAGTGCATGGCGGGTTTGTTGAAGATCAGATATACTATTGTATATGATGATGCAGTGCACCC
GTTGTAGCCTCTGCAGCAGATGGCTCATCCCCAGGCAAGAGGATGGAGAGGCAGAGAGGTTCAGTTACTCACCCAGGGTTCCAGCCA
GACCTGGCTAACAGTAGGCTCAGGAGCCATGCAATGAATGCTCCATGCATTACCCAAGAGTGCAGAGTGGCCTGCAGACACTTGCTG
CCCCTTCTGAGCTGGGGTCAGGAACCTGGCTGCCCTGTAAGCTCACTGCTACAGGAAACATCCCTTTCCCCCATGCCAGGCCTGCTG
AGACTCAGGGGTGTGGGGTTTGCTTGCTTTTGTTTTCTCCTTTAAATACAAAAGCACTCTTAATCCTCCTTAGCACAGAGTAACACAT
GGAGGCTGGATCCTTCATGCTGGAAGGGACTTGGCAGTGGAGACAGCAGAAAGAGAGCGGACCCCAGCAGCTCTCCTCATTCCCTCTGCTC
ATTGGATTTTGGGGAGTGCTAATAATGTATTTATTGTCATTTTATGTGCACTGGTGGTTGCCTGCATGCATGTCTGTGTGAGGTGT
CAGATCTTGCAATTGTAGATGGTTGTGAGATGGCACGTGGGTGCTGGGGTTTGACCCTGGGTCCTCTGGAAGAACAGTCATTGTCCT
CAATTGCTGAGCCATCCAACTCCGATTTGTGGGAGTTCGGTCCACATGCCAAACAAGAACAACAAGGGGGTGTTCTGCAGTTTAACC
CAGCTTTAACACTCTACTTGAAGCTACATTAAATGCCACTGGCTACTGGCCTGGTCCTTCAGCTCTGTCCCACTTCAAAAGTCCACA
```

87

```
CGGGGCTGACTGAGCTGTGAGTCCAGGCTCCCATGACCCTGGGCCTTGGATTTGAGTCACCTGATGGAATCGAGTGCACAGTTCAGG
GAAACATTTGCACAGTTATTCTTCGGGGTGTTTCAGAGAGTCCTGGTGAGCAGCAGCCTAGAGTGGACTCCAAGGGCAGGAGTTCGA
GAAGGGTATGAAGCTGCCGGGGAGCCTGTGGGGGTGTTACCTCCCAGGTGCCTCCATGTGGTCAGCTATGTAGAAGTCTCCAAACTT
GGTCCTTGGGCTTGGCAGAGGCTTCTTTGTGTAGCACACTTGATGAAAGCGTTTGCCACTGATGGTCAGCTGCTAGGGAGGGTGGGG
TTGGTAGTCTTTCTCCTCCTTGCTCCCATCCCAAGGCTGGCCAGTCAGGATGTGGGCGTTCGAAAGACTCTGACCCTGACTGTGCTG
GAGATGTGACTCAGTGGCAAAAACACCAGCCCGGCATTTAGGAGACCCTGGGCTTAAACGCTAAGTAACAGTAGGCAGCTGTGCACA
CCTTTAACCCCAGCACTTAGGAGGAGAAGCAAGTAGACCTCTGCGAGTTCAAGGTGAGTCTAGTCTACAAAACACAGGCCTGCCAGG
ACTACATAGTGAAACCCTGTCTCAAAACAAAACAAGCTCCAAAGACTCATCATTTTTGAAGCTCACTGTGTGTGTGTTATGTATATG
TGTGTATGTACATATGCGTGTGTATTTGTGTATGGGGCCTGGGTATATATGTATGGTGTGTGTGTGTGTGTGTGTGTGTGTGTGTGT
GTGTGTGTGTGTGCCTGTGCACATGTGTGTGTATTGCTGGGGGTTAAACCCCAGTGTCTTGTACATACTCTGCTATACTCCAGTCCT
CTCTGGGTTTTAGTAGTTGCATTCTTCAGCCAAGGTGAGGACCAAATGTATTATTTCTTATTATGAATGTATTATTATTATCCAACTTG
TCATGATAGCCTGTTCTTGCAATCCCAGAACTGGAAAGGTTGAGGCAGGTGGATTTCCATGAGTTTGAGGCTCGCTGGTTACAGAGT
GAGACCTTGTCACAAACAAACATATCTTAGGAAGCAACTTCCAGAGGAGGGAAGTGAGGTTCAGAGAGTGGGTGTCTTAGCTAAGAC
CTCCATCTGGATCCAAGCAGGTCTTTTGACTCCGCTGGGACTCTCACACAGGTAGGCTCCTAGCCTGCCCCTGAGGTGGTCAGGCTC
TTTGGACCCCCAGCACCCCCACCCTGCCTCTTGTGTGACACTCCTGCTCACCTGTCCTGGCCACACTAATGTGCTCTTCACTCCTCCA
GCTCTTTGTGATCGACAATGGTGCAGATGACTGGCGGATAGCTCTGCAGACCTACGAGCGTATCCTCTACATCAGCCTGGAGATGCTGGT
GTGCGCCATCCACCCCATCCCTGGAGAGTACAAGTTCTTCTGGACTGCACGCGTCTGGCCTTCTCCTATACCCCATCTCGGGCAGAGGC
TGACGTGGACATTATTCTGTCCATCCCCATGTTCCTGCGCCTGTACCTGATCGCCCGGGTCATGCTGCTCCATAGCAAACTCTTCAC
TGATGCCTCCTCCCGAAGCATCGGGGCCCTCAACAAGATCAACTTCAACACCCGATTCGTCATGAAGACGCTCATGACCATCTGCCC
TGGCACGGTGCTGCTGGTGTTCAGCATCTCTCTGTGGATCATCGCTGCCTGGACTGTGCGAGTCTGTGAAAGGTATTTCAGGGAGAC
CCTGAGCGTGGGATGGGGAGGGACAGGGACCAGGGATGTCCAGGTCTAAGGTCTGATGGAGGAGGCTGGAATGGTCTCAGATTCCT
CAAAGGAGAAAATGAGAACAAGAAGAGGAGGGCAGAGCTGGTGTAGAATATTGGAACATTGGAGCCCAGGTAAGCTGGGAAAAACAT
ATATAACTAACGTTTGTTTCTTCTTTTAGACCAAAGTGTCACACCTCCCCCTTTTATAATATTTATTTATTTATTCATTTATTTAAT
GTATATGAGTACACCGTATTCTTCAGACACACCAGAAGAGGCCATCAGATTCCATTATAGATGGTTGTGAGCCACCATGTGGTTGCT
GGGAATTGAACTCCGGACCTCTGGAAGAACAATCAGCCCTCTTAACCATTGGGTCATCTCTCTAGCACCCTGGTTACATCTTTAACC
ACAGTATTTATTGATTGACAAGGTTTTGAATGTTCTCCTCTTAGAGATTAATGATGCTTGCAAATGTGTATAGGGCTTGGAAAAGCC
CTGCAGTGATGAAGCCTATTTGTTTTTGCTTAGTTCAAATTTCCTCATATAATTAATTTTTGCCCACAGATCTTTTATTTTTAATCA
CACACCTCTAGTGATATGTCTCTGCTCAAGGTTTGGTCCACTGATTGACAGCATCAGCATCACCGGGAGACTTGTTAGAGCACAGAG
CTTAGCCCCAGAGTTACTGAAGCAGCAGTGGCCTTTTAGCAGTGTCCTGCTTTGATTCATGTGCACTTTAAAGTTGGAGAATCACTA
TGGTGGTGAGACAGACTGAGGCTTTATGAGGCTGGGCGGTGATTCGGAGGTCTGCAGATGGGAGTGGACGGAGGGACTGTGGTCTCC
CAGAGACCCAGGGCTCTGCCATGACCTGACAGTCTCAGAACATCCACCCTTGTGGCTCCCCATTAGGATGATACCACCACTCCGCT
GTACCCCTCAGGCCCTGTTGGTGACACCTGTCCCTTTCAAGCTGACCGTGTCTCGTTCTAATCAGATTTCCTGTGTAGCGTCTGCCC
GAGAAGCCGTGGTTTCAAATTGAGTGTCACCTGAATTAGAATAAGGCCATGGTCCCGAGATAGAGCGCTTTGGTGTGGGAAGGCACT
GAGGAAAATCCATGCTGCTTACACAGGTCCTGGTAGCTGTCCTTAGTGCATCAGCACTTGCGATTTGACTGTCAGATCTCCCACATG
CCCATCTCTGAGCTGAGATAAAACAAGGCAGAGAATTCAAGCAGACAGCTGGCTGAATAGTTAGCACATGGGAGCCACGAATGAGAT
TTCCCACTGGCTTTGGGATATTCAGCCAGTAGGTATGGCTTTCTGATTTCAGACAGAGGCATTGTTCTGCCCAGAACATGGTCCTTA
TATGAGAGGAGCAGCCTCTGAGAACCAAGCTGTTCTCTCTGGGGAACAGAGAGCCCTAAGCGACACCACCCAGAGCCCTACCATATC
TCAGCAGCTAAGTTTCTCTTCCAAGCAGGAATCCTTACCAAACAGCTCTTCACAAACCTTTCCTATGACAGAGAAATGGTGCTCTGC
TTCCTACCCACAACTCCTCACGGAACCATCTTGCTCGTTATTTGAGACAGGGTAGAACTTGTGACCCCTCGGTCCTATTTCCTGCCT
CACTATGCTGGGATGGCAGGTGTGCACTACCACGCTGGCTGCCTCATGGGACTCTCTGCTGAGGAATACCAGTCACTCTGCCTTCCT
GGGATTCAATGATGAATGAAGAAATAATAATTCCTGCTTTATAGGATTCATTGTTAGAGCCAAGCAAAGCAATCCTTGCAAACTGTAC
CTTGCCACACATTGTGTAATCCCCGGGTAACTCGGGATCTTTGTGGCTGCCTTTTTTAAATAACCAGGATAGACAGACTGCTACCAA
ATGCCCACTGATACTCATCCTATTTTCCTTTCTAATCATCAGAAGCCAGGCTTTCCAACAGTGGTGATTCCATTTGTATAGCCCTTT
GGGGCACTTGAGGTTTTTCTTTTGAAATACATGTATTTCATGAATACTTGTCTGGCATGTATTTCAGATGAATAGGCTGGTTAAACA
TACAACCAGGTACCTACAAGTGAGTTTTATTGTTTATTAAAACTGTCACAAGTGTGGTGGCTTATTTATTCATTTCTGAAGAATAGC
CACATTTTGAAGGGCCTTGTTTACAGAAGTCTTTGGATCTTAGTTGACATTCCTTTCAAAGCTCCCAAGTTCAGGCTGGACT
TGTCTGGTTAGATGAAGTTAGTTTGCATTAGGCTGCAGTAGATTGTGTTAGTCTCCATTAGCCCAGGTTAGACAAGACTAGCCCTTG
TTAAACTAGGTTTGCCTGTGCTGTCACAGAAGCCCCCACAGTTACTCTTGTTGCAAACATGGCTTCTGAGCGTCAGTTGAACATCGG
GAAACCAAGCAAGCCAGAGCCTCCCATAGTCTGCCCTCTAGGGAACAGACTTTGTGATCACAGGCTCAGAGGTGGCTTAGGCATATG
TGGTGCTGAGCACAGGAACACATCCTGCTACTGCTTCTCACTCTGTGCTGGTTTTGTATCACGTGCTTCATGCTATCCCAGCCTAAC
CCAGCGGCAGACTCAAGGAAGCCAGTCTGTCCTCACCTTAGGGCTGCTGCTGGCGTGGTTTGAGGTCTCAGGGCTCCTAA
GTGCACAGTCAGGGCGTAAGCATGAGTTCAATTCCAAACTTCAAATTTACCTTGTCAACCCAGGCTGGATCCTGCAGGGACAGGAAA
TTGTGCTCTAGACTGCCCTAGACACATCCCCACTCTGAGGTCTGAGGGTTACTGTGGTGACACAAAAATGCCAGGCTTGTCCTTTGG
TCTTCTCAGATGTCTGTGTCCTTGATCGCTTTGTCATGCCGTTTCCAGGGCTGTCTTTATGCTCAGTGAAGTCTTCATGTGTGTGCA
CATGCGTTTTTGTTTGTTCATGTGTGTATGTGCCTGTGTACATGTGTGGGTGTGCATGCACGTTTGTGTGTGTATGTGTATTTAAGT
GGATAGAGTGCACCCACTCTGCCCTGTGTTGATTTGAACACCAATCACTTCCACCTGGTATTGCGCTGGCTGTGAAACGCCACCTCT
CAAGGGAGCTGCCTGACTCAGGGGTGGCAGGGAGCCACTGATAGGGTATAAAGTAGAATTTAAAGGCCCCTTTACCTCCTAGAGCAG
TGGTTCTCACCTTATGGGGTTTGAATGACCCTTTCAGAGGTCTGCCTAAGACCATCTGCATGTCAGATATTTACATTATAATTCATA
ACAGTAGTAAAATTACAATTATGAAGCAGCAATAGAATAATTTTGTGGTTGGAGTCACCACATGAGGAGCTCTATTAAAAGGTCTCA
GCATTAGGAAGGCTGAGAACCACTGCCCTAAAGGATAACTGTCTAGGGTCATCAGACTCTAGGGGTCCCTAAGGAGTCACCTGAGGT
TTCTATTCCCACACCTAGCCTGTGTCCCACTTCCTGTGCTGATTCTGAGAGCATCCCCTTCCCCTAAGTTTCTGTCTCCAGATCTCA
CAGTCTTTGACTCTTGCTCAGAGTCCACCTATAGTACTGGGAAACATGTACAGAACTCGGGTAATCAGACAAAGCTCTTCTCTTGG
AATGGCCAGCGAGTTTCTTTTAGATCTGTAGGTGACCCTGCGCTTTCCAAGGCCACATCCTAAGCATGACTGGCTGGGACACTGAGA
CATGGGTTTCGCTGGGGGACTGGAGGATTGGGCCAGGGTGGGAGGAGTATACGGAAAAGATGACCTTGAGCTCTCTCAGTTTGATCC
GATGATGGCTCTGCTACCTCTGATGTTAGTGAAGGTGGCTTGTCATTGGCATTATGCACCATGACAGGTTCTAGAACCTGTCCTGTC
TGCTGTTGCCCCGACTAGGATTTGCATACAGCCCGGGTGGCCTCCCTCAGCTGGACTTATCTCTAGCTGTATACACACACTTCTGAA
GTTGCTATGGAAATCCTCTTTAGCAAGATGTGTTCCAGAAGTCATTTCTCCTATGTGGGGTTGGTTTTTAATGGGACAATAAATGTT
TTTACATCTCCTCCTGCCAAGGACAGACGGGAGTTAATAATGGCGCAGAGGAGCCCTGTGTTGGGAGAGCAGCACCTGGTTGCCAGG
AGGGCAGTAGCAACATTGGAGGCTTGTTTTGCTGGGACCCCAGAGATGCATTACATCACTGCCTCCGGAGCCTGACAGTTGCAGCGC
TCAGCATTCTCCAGGATTCTCTTGGCTGCGTGCCCACCTCCCACAGGCCTCCCAGGGCCACCCCAGGAACCTCCTCATTAAACACTG
TCAAATGCCAAGCAGCAGTCGAAGGGGAACAGTGGAGGCCCCAGGTCCCAGTGCGCCAGGCAGGGAAGTCGGCTTTCCCTGCACAC
GCTGAACAATATTCTAGCCGCCTACTTGCGCGTGTTTATCTCCTTGGAAGAAGTGGAAAGGCCTCCACAGTTCTGACAAACACAGCC
TGAGTCTTGCTTTGCATGTAAACCCTCTCCATCTTCTCGACTGGCTGGGCCCTTTTGGATTCTTCCCTCCCAGGACTCCACAGTCTG
TACACAGGCTGTCTGCCTCAGGGGCTCAGGCCTTGTCAGCAGGTGTTTGGTTTTGACTGTGTGAATTTTTAATTTTTTTACACAGGCAT
TTTAATGAAGAATTTAAATGTAAAGATCTTGCCTGTAGAGTCAGCGAGAACCAGGCCATTGTGGGGTTAGGGTTACAGCTCAGGGGA
GAATACTTGACTCTCGTGGAGGAGACCTTGGGTTGATTCTCAGAACTGTGGATGAAGAGCAGGAGGAGGAAGAGGAGGAGGAGGAGG
```

EP 2 204 376 A2

```
AGGAGGAGGGAGAGAAGGACGAAGAGCAAGAGGAGAAAGAAAGACCAAACTATATTCTTTTTCTGTTATTGTTTTTTGTTTGTTTGTT
TGTTTATCTGGTTGGTTGGTAGGCTTTTGTTTTTCAAGGCAGGGTTTCTCTGTGTAGTCCTGATTATCCTGGAATTCACTCTGTAGA
CCAGGCTAGCCTCGAACTCAGAGAGATCCACCTGCCTCAGCCTCCCAAGTGCTGGGATTAAAGGCATAGGACACCAATGCCCAGTTC
CAACCTACATTCTTGTTCTCTGTTGGTGATTGGTGAAGCTCTGAGGCAGGAGAAGAGAGGAACAATGGGCATTGTTCAATGCCATTG
GGCAATGGGCAATGGGTGTGACTTGAGATGGCAATGGACGTGACTGGAGGAAAGCAGTTTGGAGCTTGTCTCCAGGATGAGGCTCAT
CAAGGAAGCCCCCATGCTGAGCTGAGCAGAGCTTAGCCAAAAGAGCTTAAGAATGGGGCTTGAGACTGTGGTGGTAGTGGTGGGGC
CTTCGCTGAAGCACACACTCCACTGTGGACTGTGGTGTCGGGGCCTTCGGTGAGGCACACACCCCACTGTGGACCTTGCACAAAGAT
AAGTGGGAACTCTGAGAAAATAGTAAGCACTTCTAGACATAGCTATTTTATCTAAGATAATTTGAGTTAAGCCTGTATTTAATATAC
AGGTGTTCAACACACACACACACACACACACACACACACACACATGCAGGGTGGTATATACTGAAAATTATTTTACTGTTAGA
AGTCTGTGCTTTTAAGTGTTTGTAAATGACTGGTTGAGGCAACTGGATGATGAATCAGGAGGGATTCCATGCGTTGGGGGCGGGGCG
GGGGCGGGGCTCACCTCTAGTCCCACCGTTAGCTGGGGGAAATGAGAAGTTGGTCTTCTTTACCAGAAACCAGTCAGGGTGGTCCTG
CTCCTCAGTCAAGGAATAGAAAAATTACCCCGAAGATGCAAAAGAACATGAATTATGGGGTACCCAAAGCAGCCTTGGAAGACGAGAT
ATGCCTCAAAGTGCGAGAAAGAAGCAGTCTCTGTTACGAAGAAGACAGCAGTCGAGGAATGAAAACACACCTGCACGTGCTCAGAGC
ACCGACTTTGTGAAAAAGAAAATCCCCATTCGTGATGATGACTTTCTTCAAATGTAGAAAGTGGAGGGCCAGCTAAAAAGCTTCCCC
AGAGGGCCAGAGAGAGTGCTCATGGGAGTTAGAGCTCTGGAACCCATGTGGTGGCATGAGAACCAGCTCGTGCAGGTGTCCTCTGAC
CTCCATGGCTGTGCGCTTGCCTTCCTCCAGAAATATAAATGAATGAGTGAATGTGGTGAATGAATGAATGAACAAATATCTTAAAAGT
TGTTTCAGAATCTGAGAGATGAGTGGAAGGTGGTTAGCACAGACAGGGCCCATGAAGTTGAAGTTTGGACAAAAATATAGTCAAAGA
TATGCATGAAAAAAAAAAAAACAAAACTTTTCCAGAATGGCTGACAGATAGGCAAGGAGTTGGTAGACCCGGAAAGGCCAAGCAAGAC
ACCACCTTCTTTCTGATCGGAAGAGCAGTAGGGTCCACCTCTAACATAACCACTACTGTTTCTGGAATCATAGCAAATCATAAGATT
AGCCAACCTAGAATCATAGAAACAACAAAACTGGTTTCCTCACACCCGCTAAACTTTAAGAGCCAGTAGGCCATCTTGACAGAGTCT
GCAGTCTGTGCAGTGACCCCCTCTGTCATTCGAACGAAACAACAAGACTCATTATTAAGATATTAATAATGGGGGCTGGAGAGACG
GTTCAGTGGTTCAGAGAACTGGTTGCTCTTCCAGAGGACCTGGGTTCAAATCCCACCAACCACATAGTAGCTCACACATGCCTATAA
TTCCAGTTTCAGGGATCCAACACACTCACTCAGAAATATATGCAGGCAAAACGTCAATGCACACAAGATAAAAATAAAATTTTAAAA
AGATCTTAGTAATAGACTGATGTGAAAGCCAGAGAGATAGACAGAGCTATGCATGATCGCCAAACCAAATTTTAATGTCAAAGGAAA
AGTAGATTCTACAGAGTAGGTGCGTGAAACAAAAACAATAATTCAACAATAGTTAGGGGATTACAAATATTTAGATTAAACATTTAA
AAGGAGAGGGCTTGAAGGGTTTAATTCATCATCTGATGTAGAACATTTTAATCCTTAACATTCAAATTCAGTAATTAGAAAAATATA
GTTGAGATCATAGTTTAAACCTTAAGTTTACCTCAAGTTAGGATTTAAAACAGGATTTATTTGTTTATTTATTTATTTATTTATTTA
TTTATTTATTTATTTAATTTTACATGTGGGTATTTGCCGGCATGCGTCTGTGTACCACCGATGTGCAGTATCCACGGAGGCCAGAAG
AGGGCGTCAAATGCCCCTGGAACTAGAGTCATAGACAATTAGGTGCTGGAATCAAACCTGGCTTCCCAGAAGAACTGCTGGTGCTC
TTAACCACCGAGCCACCTCTCCAACTCCCACAGTAGGACTTAAAAAAACATACAATAAGGGTAACAGTAGCATCCCCTAAGAGTTGT
TCCACCCCAAACTATCTCATCTGTCTGAGGCCTGGGGGCTGTTACACCCCATTCCATGTAGGATGAAGCTGAGACCTGAGGAGCTGA
AGCAACGTGCAATTCCCAAACATCAGATCTACCAAGCTAAGGCAGAAGAAACAGGGACTCAGATGGGGAAACGTAAAGACAAAACAC
AAACACAAAAACCAAATGACAGAAAACCACATCTAAAAGGTATAATGATCAACATCAGTTGAGTTACCTCCTCAATTAAAGGACAGT
GTCCTTTGCTTTGATAAGATTTTAAAGCAGAAGTTGTTTTTATGCTAATTTTAAAAGAGGAACCTAAGGCAGAAGGGACCCAGAAGA
AAGTAAGAGCGGAGAGTTACATAAATTCAAAGCAGAGGGCAATGGAGACAGGCTGGTTTCATAGCACAAATTATTTTGTGGTTCAAA
GGAATCATTTAACATTTCAGATGGGAAGGGGTGTAGAGATGGCTGATCAAGTAAGTAAAAACACTGGCTGCTCTTTCAGAGGACCAG
GGTTCAATTCCCAGCACTCCTGTTACAGGATATCTGAGCCCCTCTTCTGGCTTCTGTAGGCACTAGACCCACCAGTGATACACAGAC
ATACATGCAGATAAAACACCCATGCATATGAAATGAAATGAAATGAAATGAAATGAAATGAAATAAAATAAAACCAACAAAA
ATACCATAACATAGGGTAAACTTTTGTCCAGAAAGGGTGAAATTGAATATCTGGGGGCTGGAGAGAGGGCTTGGTGGTAAAGTCCTT
GTCATGGACGTGGAGGACCAGGCTTTGGTCCCCAGAACCCACATAAAAGCCAGGTTGGTGTGTAATCTCATTGCTCAGAGACAGAGG
ATCTCCCAGCAAGCTGGATAGCCAAACTAGCCCTAATGGAAAACTCCAGGTATACACATGTCTATGTATACCAGCGCGCACACACAC
ACACACACACACACACACACACACTCTTTTAAAAATAAGAAAGAACACCTGAAATACTAAAAGTTTTGATGGAGAACTACCTTAAGAC
TAATAACCTGACCAAAGATGTACTCAGATGAAAAGTACTCTCAAAGTTTTCATTATTAAAAAGTAAAAAACAGTGGAAAGATTGTGC
AGTAGTTAAGAGCACTGACTGCTCTTCTAGAGGACCCAGGTTCAAATCCCAGCACCCACATGGCAGCTCATAACCACCCTTAATTCC
AGTTCTGGGGGATCTGACTCCCTCTGTGGCCTCTCTGTGTCCTGCACAAATGTGGTGTACTGATGTACATGCAGGCAAAACACCCCT
ACAGATAAAATAAAATAAAATACATATTTTATTAAAAGTGAAGAAGGAAGCCAGATACTGTGGAACATGTCTATGTATATGTGTCAG
GGATCTGAGGCAGGAGGATTATCTTTAGTTTTAGGCCAACATGGGTTACATATTGAGTACCAGGACTTTATACCAAGATCCTGTCTC
AAAAAGCCAAGAAAAAGAGAAGAAGGAAAAGAAATTATGTTTATTAGAAGGATGAAACAAACCTTAGATGGAGAGCAGACTAAATTT
ACAATGAAGAAACGGGAACTAATAAAATTTTTAAAATGGGATTTATAAATCTGAAAGCTGACAAGTTTAATAAAATTTAAACAATTTCT
GCTGTATTGGAAAACAAAATTAGAAATTTTAAGGGAAAATAATGAGTGATTCATAGGGCATTTTAATCAATAATCGAGAGCAGCTTG
GCTCATACAAGAGAAGCCCAGGAGGGAACTTTCCCTCTGGATATTAAAATGTATTATAAAACAAAGAATAATTAAGTCCCTCAGGCA
TTACCCATTACTAAAGAACCCAGTAATGACAGATCGATGAAATGCAATGGAAAACCCTGAGATAGAGCCTAGTGTGGAAACCCAGTC
ATGGAGAAAGAAAACCAATTAGGAAGCAATAGATTACTCAATAAACGTTCATGGGGAAATTGGAAGCTTGGATGAAATATCAAATAG
GCACGCCACCTCTCAACAGCCAGCAAAATAAATTCCAGGTAGATTAAAGAGTCGGATAGATATGAGGGAAGAAATACGAGCAGGAGC
TAGAAATCAAAGTGAATATTTAATCAATCCTGGGGTGAAGAGAAATGTTAGGCAGAACATCTCAGGAGAGAAGAGCTGTGAAAGGAA
GCAGGCATATTTGATTACATCATAAATACAAGTGTAGATTTCTGTCCTTTAAAATATTAAAACAGGGCTGGAGAGATGGCTTAGTG
ATTAAGCAGCACTGGCTACTCTTGCAGAGGACCTGAGTTCAATTTCTAGCACTTGCATGGTGGCTCAGAGCCATTTGTTACTCTGGTT
CCAAGGTATCTGACACCTTCTTCTGACTTCTACAAGCATTAGGCATGCCAATGGTGCACAGACATACATGCAGGCAAAATACCCATA
CACACGATATATATTATTATTTTATTTACTTATATTATAAGCATACATGTTAATTAATTAATAATTATTATATGTAATAAGCTAGAA
ATGGTGGACCTTCTCTGTAATCCCAGAGCTAGGGAAATGGAGACAGGAGGCTCAAGAGTGAATTTAAGATCAGCCTGGGATACATGT
CTCAAAAAAAAAATCTATTTAAAAGCAAATGTTATATTTAAGAAAGATATTTGCAACAAATGTGACAAGAGAGAAAATATCTCATTGT
CCAAAGCAGTCCTGGAGATCAGCCACAGTCCACCTCACGACTGAAGGCAGGCAACAACATAGAAGGCACACAGCGAAGACACCAACAT
GAAATGCAGCGAGTCAGCTGTTAACCCTGAGTTTGCCGCAACTTGAACAGTGGGAAGCTTTCACTTGTTGCTGTTAAGGTCTTCCTG
TTGATGGTGGGGGTGGGATTGTGTCTCACACTAGAGCAAACAGGGAATTTAGGACCATCTTTCTGGAAACAACATTGCTGGGAAGCC
CTTGACGGTTTATGGCTTTAACCTACTAAATATCTTTCTAGAAAGTTCTTTGAATCAAAGACTCAACTGATACTCGAGAGTCTTCAA
TGTAATGTTTATAATATCAAAGACTCAGAAATGAAGTGAACATGTAGATTTCTGTCTTTAAAATAAAGGGGCTGGAGAGATGGCTTAGTG
TCAGCAGTCAAGAGCACTGGCTGCTTTTTCAAAGGATCTGGGTTTGGTTCCCAGCACCCGTGTCAGCACCCGTGGCTGCTGACAAAC
CTGTAACTCCAGTTCAGGGGATCTGATGCTCTCTTCTGGCTTCCCTGAGCACTGCATGTGCCTAGGTGCAGGCAGGCAAAACACCCA
TGCACAAAAACTAAGACCTAGAGTCTTTTAAAGCATGTTTGAATAAGGCTTGTGCATGCCGACAGCGAGTTCTTAGGCAACGATGTT
ACAAGTCCGTACTGATGTGGGAAAACNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNCTAATGGTATTTTTGTTTGTTTGTTTGTTTTGTTTTGT
TTTTCGAGACAGGGTTTCTCTGTGTAGCCCTGGCTGTTCTGGAACTCACTCTGTAGAGCATTTTTTACGTATATATGTGTAAGAAAT
GGGTTTGGGGGCCACAAGAAAATGACCCCACAATCCAAAGCCTTTCAGTAAGGTGGTGAGTTTTACTTGTTCGGGAGGGGTGTAGCC
AAGGCAAGCATGCAAGCACACTCGGCAGCAGGGCTGCCTCTGCTCTCTGTTCCCTAACACACAGATCCCATGTTTCCATTTTTCTGT
```

89

```
GATTGGCCAGCTTAAAAAGCCCAGTTTATAAGCAGAGAGCTCAATCTCTTGCCAGGTGCAACCCTGAGCAGGCACATATTTTGATTTCA
GGAAGTGCTGACTCTGGAGGAAATTTTAATTTCTGCCAAGTGACTGGTTAAAAAAAATTAATAGGGTTTTGTCTTATTTTAACCCTT
TGACAGAAAGACAGTGCAATTTGTTTATAATTTCCACACTATATACTTTTGTTGTTGTTGTTGTTCTGTTTGGGTTTTTTAATT
GTTGTTATTGTTCTGTTTTGTTTTTTGATTTTCCACACAGGGTTTCTTTGCATAGCCCTGGCTGCCCTGGAACTTGCTTTGTAGACC
AGGGTGGCCTCGAACTCACAGAGATCTACTTTCCTTTTCCTCCAAGTACTGTGATTAAAAGTGTGTGCCACCACAACCACCATGACT
CAGATCTCTGCATTTTTAATGGAGAGAGGCAGCTGGCTTTGAGATTTGGACAAATGTCATTTTAGCCTCGGCACGAAGCCTTGTCATCA
ACAGGGCTTGGGGAGTAAGGTCGGGTGGTACAGTTTCCACATTTAATTCCCTGGAGGATCACCAAAGAGTTCCTTCAGAAGGGCTCC
CCTTCCTATCTAGCAGCTAATGGCCTTAGAGATAGGGACAGTGACTGGGCAGGAGGCTTATGGGTCATCTCTCTGGGGGGCCTCCCT
GGGTGGGCTTGTGACACTACCACGGAGTAGGTGGGTGAGGCTCACAGATGCTAGCTTGCTTGGTGGCAAGATAAGGGAACACTGCAA
GTTAGAATGCATTCAGAAAGCACAGAAAAAGACGCTGTGGTCTTCCTTGCAAGAAAGGTCCTGCTACTGAGCTGTGGGACCTGGGTG
AGAAACCTTTCGTTCTGCACCTGCCTGGCCACAGGGGCAGCGGCACCCAAATCTCCTTTGAGCCCAGAGCAGTGAGACCCTGGCAGT
GTGCTGCCATGCCCAGGTTCAGCCGGCTGAAGCCACGGTGACTTCAGCCACCAGTCCCAGGGCCATTTCTTCTCCCTGTCACTAGTG
ACACTAGTCTCAGGTCTCTGTTTTGAATCTCTTCCATCTTCACTTTTTTTCACAGCTGAGTTTCTCTTTCTGTTATTTCTGATTCAC
ACTTGTTTTGTTTTATTTCCTTTGCATTATGTTTCTGCTCTTTGGCACTTAAAGAGCAAAGTTGCATATTTGTTACTGTTTTCTTCA
TACTTTTATTTATGTTAAATATACATTAATTGTAAGTTGCGTTGCAATTAAGGGATGGCCTCTCTTAGAAGAGTCTATAAACGGGAA
TCAGGAAGGTTTCCTAACATGGGCAATGCTTAGTTCTGGGTACAGGACAGCACCTGAGGGTATAGTGGGAAGCTCTGCGGTCTCTGC
ACACCACCCAGCTACTCCAGCCTGTGGTACAACTGTGAGGTTTCACTCCTCTGTCTTTGGCAAGCATCTTTTCCCTCCAGGATGTCT
GCTTTGGGGTTACCCACAGGAGTGGGGGGTTGTACAGCTTTTCTCCAGCCACAGTGCAAGTTGCTTTTTTTCTTACAGTGGATACTA
AGGACTCCTGTACATGTCCAGAAGGCCTGGGCTCTGAGCAGAGGGGAGGACTCAAAGTTTGCCAGGCTGGGGCTCCTTAGACAGTGA
TGGAGGAAAAGGGTGAGGTTCTGTGAGCAAAAATTACCCATGTCTGTCTCCCAGCTCAGGTTAGCCCACACATGAAATCTGTCTTCT
GTCGAATTTCCCACTTTCAACCCTTGGCTGCTTTGTTTGGTCCTTGAGACCCCACCGTGTCCTTCATCTCCATGGGAGGGACCTTGG
CAGCAGTGGATGGCCGTCTCTGCTCCACAGGGACTGAGCTCCGTGTTGAGGGGTTGACATTCATTGCTATGGAAACCTTTCAATCCTT
TCAATAGGGGAGAGCCATCCGAGCCACGGAGGGACTGGCACCTTCACCAGTGCCTGACAAGCAAGGAAACCTCTCAGAGAACAAGAG
GCTGGGGGTGGGGGAGGCATCCCAGCAGGCCAAGCAAGGGACACATGTGACTTAGTCTTACATTCCAAATACATCCTTGTGCCTGTG
TGTCCTCATGCACTAGTGTCCCAGGCACTGGGTCTATGCTTGGACTCTCACATTGCCCTAAATTCTCTCTGCTTTACTGTTTTGCTT
TTTAAGATTTATTTATTTAATGTATGTGAGTCACATGTTGCTGTCTTCAGCACACACAGAAGAGGACATCAGATCCCATTACAGATG
GTTGTGAGCCACCATGTGGTTGCTGGGATTTGAACTCAGGACCTCTGGAAGAGCAGTCAGTGCTCTTAACCTCTGAGCCATTTCTCC
AGCCCTACTTCACTGTTTTTAACTCTTGGTTCCCCCTCCTCCCCCCACCCCCTACCCCCGGGAACTTTATCTGTTTTGTTCTATCA
TATTTAGATTTTTGACTCTGGCAAAGAAATTAGAAACCTTTAAACAAAATAGTAAAATGTTGGAGAGGTAAAAGAAGATGGAAAAAGG
GTCAGTCTAAGCAAGAAAGGAGTCTGAAAACGTCAGGGGCAGAGGCTCCTCTCTCTTGTTACCCTACTGAGCTGGAGGCGTGGCTTCAC
TTCTCATTGAAGGCGCTGGAGCTCCATCAGCACATCTACCTTGTAGCCAGCAGGAGGAAGAATGTATGCTGCTCTCTATGCAGGCAT
GCTCTGCGCTCTATGGAGCCATCCCACTCACACTCACACTGAACATTTCTGGGGCTGGGGAAATGGTATCTTTATCACTTATGTTAA
TAGTCCAGGTTTGCAAAAATGGACTACTGGGCCACAACTGCTTCCAAAGTCAACATTGTTAGTTATCCTGTGTCCCTGCCAAACCAA
TCCAACTCTCCACGAGGGGTTTCAAGCTCTGGTTCTTGTCTGTAATTCCCCTCAGATTCCCCTTTCTCTACACCTGCTGGCCCGACC
CCTGCTGCTTCCCTGTTTATAGAAGCTTCAGGTTCTGCTACACTCTCGTACCCTTCAAACGCTCCCCGTGTCCCCAACAGAAGGTTA
ATTCATACTCTGCTTAGAGTCTGCAGCTTCCACTGCCCAAGGCCAGTGACCTATGATTAACTGGCTGAGGCCTAGCAGTGTTGGTCA
TAATGCCACAGATCTCTTCATGGAAACTTTATTGTAGACAGTGAAAAACATTGCACAGGAGTGAGCGGGCCTGGGCCCCAGCTGCCA
AAATGGTGCCGGCCTGTGTTGCAGCATCCATGAGGTCTGGGAGGCAGAGGCAGGGAGACTGTGAGTTTCCAGCCAGCCAGGGCAGCA
TAATAAGATCCTGTCTTTAAAAAACAAACCGAACAGGACAGGGGCTGCTGATTATGGCTCAGTGGGAAAAAGGGTTTACATTCTAGC
CTGCCAACCTGCATGGCAGAAGGTAAAGAAGCGAGAACCAAGTCCTGTAAGATGTCCTCTAATCATCCTTCAAATGTGCCATGATGT
GTATACACACGCACACACACAGAGGCACACACCTGTGTGACCATACGCATCCATGTGTGTGAGCACATGCATGCATGCACACATATG
CTCATTCACACACCATCTTGCAAACAACATACACACATCCATGTGTGTGAACACATATGTGCATGTACTCACATCTCTGTACAC
ACAGAGAGATGCACAATACACATACGCATGCACGTGTGTGAATGTATATGTACACATAGCACACATGCATTGTGCACACACACAC
ACACACACACACACACACACGAGGGCAGGGGAGGGGGAGGAAATAGAAAGGAGAGGGAGAAGAAAGAGGCAGGAGCAGAGAAGGTGG
GAAGAGAAAGGGCGAAGAAAGCGAGCTCAGCCCATTGCCTCACAGAGCCTCTGACTGTGCCTGGCTCTTAGCCAGCCCCGGACCTTCG
CCCTGCTGGGCCCCATGTTCCTGTCTACAGATGGGAGTCAAACCTGCCCTGCTTACACCCTAAGTGGGTTCAAGAAATGGAAGAGCCT
AGAAGCTGGGAACCCCAAAACACAGCTCACATGCTCTCCTGCTACTCCTGACCCACAGAGCAAGTAGGCAAGATGAGAGCAGGGGAA
CCCCATCTGTGCATAAGGATGCAGGGACTCTGGCAGCAGAGGGCTGAGGCCTGGGGTGCTGAGCTGGGGAAGAGGGGGAGGGGAGGG
GAATGAGTTAGCTTTGTCTTGCATTCAATCCAAGTGCTTATTTTACAACTTCTTGAGGGCATGCTGTCTGGTGTCTCAAAAAAATAT
GATTGGTGTGATTTAAAAAAAAAAAACAACAACAATAGTGATTTTCATAAGGGAGCAGATGGAAATGGCCTGCTGTTCTTTGGGGGGG
ATGGCAGGTGATTGGATAGGCTTTTCCTGGGAAATACATACCCAAACGTATAGTTGCCACTTATATTGTCAAACTTACAGAGTCA
AATGAATGCTGCAGTTGCCTGGCAGTAGTTTTTAATTAGCTTTTTTGTCAAATTTAACACACCATTGTTCTCCTTAGCAGTATTCTT
AATGAGCTTTGCCCCTGTTTACAGTTTCAGTTATAGATTTTATGTAGGGAATAGACATATTTTGAGGTTGTCCATTACTTCAGAAGT
TAGACGGTTGCCTCCTGGGATTATCAGCCTGTCACTATGGGACAAGCTTGTCAAAGTGCAGCACAGGGAGAATAATGGGACCCAGCC
AGACTCAGAGGAAGTCTCAGAGGAAGAGAGAGCCCTTGCTTGCCAGTGCTTGCTGCAGTCTCTGCCATGATGCTGCCTCCTCACAGC
GGCCAGTGATGTCATCGGCTGCAGGCCACCTCCATTTCAATGATGGAAACCCGGAGGCCTGAGTCAGGGCCCCTCCCGTCATGGC
TGAAGTTGTCGTACGTACATATGGTCGCCTCGGATAGCTCTTGGCAAGTCCATCTCACGGGGTCTCCCAGAGCACTGCCCTTGCAGG
AGCCAGGGCTTGCTCTGCAATAGACAATGCAGTTCTGTGCTCCCTGGTCCCGCTGTCATCTTTGGCCTTTCCCAGCACTGTGTGGTA
AAGGGAGTAAGACAGGAGTCATCCTCCCACTCTAGAAATGTAGAGGCTCAGGAAGCAAATGCATAAGGAGGGCCTGACCTGGCTGGG
GTCCCCTGGGCAGCCACGTGTGGTATCTTGTGTCACAGAGCCCAGGGTGCCTAGGCCAACGAGAAAATAGATGCCCCGTGTGGATGC
TGGCTGGGGCCATCTTTATGTCGGAATGTAATTTTGAGTCTTTGAAAGTCAGTGGAGAATCAGGCCATTGTTCAATAAAAGCACTTG
TCAGTCACCCCCATCATCTTCCTTAATTAAACCTCATCCCCAGTAGACAGGATCAGTACAGAAGAGAGGAGGCCTTGCAGGGAGCTG
CCCCCAGCCAGCTTCCAGGCTGGGTTCCTGGTCTCCCTCCCATGTGTTGAGTCATTCCTTCCTCTTCTGCATCCGCTTGGCTCTGCAC
ACCTGTGGGCTCACACCTTCCTCCCTGGGGTTCCCCCAGCTCCAGCACCACCACCACCACCACCCCCCCACCCCCCCACCCCCGTCCCT
CCACATGTCCTTTGTTCCATGCCTTGCTGCTCTCATCTGAGGGTTTCCAGTCCTGTGAAGTGTCAGAGGTGGGCTGTAGTTTTCAGA
CATCTTTGTTGCCTAGCAACATGCTACAAGCACCTTCTTCTGTGTGGCGTGCTTCCTCTAAAACAGCTGGGGCCAGGGGAATGGCGGGA
GGGGTGACCAAGGAATTCTGAGGCCTCTCTGGAGTAATGAGGGGAGGAGGAAGGAAAGGGACAGCAGGCAGACACACCCGACTGAC
TGACTTTCTTGCATAACTTTGAGTGTAGTCATTCCCACTTTTCAGTGGTGTAGCTGCCAGAGTCGCTTTACTGTCCTGTCATAGAT
CCTTGCAAGTGGTCAGTGGTCAAAGTCCTGACTTGGAAGCCATCAGAGACTCTTGGAAGTCACTACTGGCTTGGGGAGGCACCTGCA
GTGACTGCCTAAGGCAAAGCCAGTCTGAAGGCTCCCGACTGAGCTGGCCTCTGGCTTCGTCTTGTTTCCTGAGATGCCATAGCCCCC
CTGTCTTCTGCTTGTGTAGCCAGCCCAGCGATAGTTGCCCTGAGAGTGGGAGGGGAAGATAGGGCAGGAGAGAGAGTGGGATTTTAA
AATGAAACAGGGGCTGCGCAGTGGTAGCACGTGCCTATAATCCCAGTACTTGGGGGGCAGAGGCAGGTGGATTTCTGAGTTCGAGGC
TAGCCTGGTCTACAGAGTGAGTTCCAGGACAGCAGCCAGGGCTATACAGAGAGAAACCCTGTCTCAAAATAAACAAAATAAAATAAAA
ATAAAATAAAATAAAATAAATAAATAAATAAATGAAACGGGCCAGAGAAAGGGCTCAGTGGGTAAAGGCATCTGCCACCAAGCCTGAGT
GTGATCATGTGCAGATTCAAGACCAGCCGCTTATGAATCTGTCTTCTCAAAGATGGCCTGTAAGGGCCGAGGGTGCTGTAGATGCTT
GGTATTGCTCAGTCTGCCCTGCTGGTGTGGGCTGCTGGGTGGAAAGCAAGACAGTGCTCCAAAGATCACAGCCACTCTGAGATGGGAG
```

```
GGGCCCGTACCCACAGCCCCCACCCTTCATCTACATGAGGACAGTAGGCCCCAAGTGATAGGTCCCAAGCAGCACAGATCACACCTT
CCTCCAGGCACTGCAGTTGACAGAAATCTCTGTGGGTCATCTTATCTTTGCCCTGCCCCTCATTTTCTTCCAGCAAGCACCTCTTAG
TTCAGGAGGCAGGGAAAAAGAGTTCTAACAGTGGCCCCCCTATGTGGTTATAGAAGTGGCCTGAGGTTGTGGCCTAAGTCCCAGCCA
GGAGCCCTGCTTCCAGGGACTGCTCTCATGGGTGGTAGGGTGTGCCATCTTTGAAGACTTAAAAGCCTATCCAGCAAGAAAATTCCT
CTTGAGGGGCGAAGTTGGGTGTGATTGGCTCTCTCCATCCTTTCCTTCACAAGACTGAGCTTCAGCCTCTGAGAAGTATTCCAGAAC
CTTCCAGCTAATCATACAAAGGGAAGTTTCAGGGACTTGGCAGTGGGAAGGGCAGACCTGAGCAAGCTCCTGAGGACTTATGGTCCA
CCTCCCCAACACTTGGACACCCTTTGCTTGAAAATATGTGGTGGTCAGGCCAGTGGGAACACCGAAGAAGGAGATAGCATAGTCCCA
TACCTCTGCCACGAGGGCAAAGGCTAGCTCTGTCCTCTGCCTGGCCCAGATGCCTCTACAGTGACCTCTTTCTCTCTCCATGGCTTT
CTAACAGCTGCAAACTTAGCCTCCTAGAATTCTCCCATCCACACCCCCAGCCTCCAGGGTTCCTCTTTAGCACGCTACTTCTCAATG
GCAGGGGAGGTCTCCCATCCAAAGCTCTCACCCCTTGCCCTTGGGTAAATGAACAAGAAAACCCTTTCTAGGGGCATCCCTCCTGTT
TCTGCCTGTTCTTGGTCACGAGTGATCTCCACCTCCCTCCCACAGGAACCCTGTCTAGGTGAGACATTTGCTTGCCTGGCTGCCCA
CTTTGCATTCTCTGAGAAAAAAAATCCCCTTCTTTCCTTTGGGGCTCTATCCAGGACCAGTGTCTCTGCAGGAGTTCCCTCCAGGGA
GTGCCATTGAAGCCTTTGTGAATGTCAGTTGCTGGGGCTGTATATAACCCACGCAGCTGAACAGGACAACCCTAGCTCTAAGTTACA
AGGGGTTCTTTCCATATAGAGATTGAGGCTCAGAAGCCAGAAAAGGGGAAGTCCCCAGGAACCTCCCCACCCTACTTCCCTGGGCCTT
TGTTTTTTTGGAATCATAATATGGTTAAGCACAGGGCTGGGGACTTGGGCAAAGCAAGCCTATACTCAGTTTAACTGCTCAGTTGGT
GTTTATTTATTTATTATTTTCTTATTTTTTGAGGTGCAAAGGTTGAGCTCAGGCCTTGCAGATGCTAGGCAAGTGCTCTGCCTCTG
CTTTGTCTCCATCCCAGATTTTGTGTTCTTGTTTGTTATTTTAGTCCAAGTCCTGCTGTGTAGCTCAAGCTGGCCTTGAATTTACTT
ACTATGTAACCCAGACTATGTTCGAATTCTAAGTCCTCCTGTCTCAGCTTCCTGAGTGTTGGGATTATAGCGTGTACCTCCATCCTC
AAGCAAATGTTTTATTTTGTAAATATCTCCTAATTAGGTTAAACTGAGTTTCCAATCAGGAAATAAGAAATGATGAGAAATCATCTC
TCATATCAAAGAGTGCATACAGTTGGCACACATGACTCAGTGCTGGTACTCGATAAATGGTAACAATGGTTATTCTTTCTTAAAATA
AAGCTACCACCCGGCCCTACCTACCTCATAGAGATAGCTCCAGAAATAAAGTGAGAAATAGAACATTACCATGATTACAGAGAAAAT
ATTTTGCAAGAAATCCAACTACTTTTATTTTTGTCTCTGTCCAAGAGGACTCAGCCCTCTGAAGCCCCTTTGCCTACAGCAACACTC
TGTGACAGATGGCAGATGTCAAGAAAGGCTCAACCAGGAGAGAGAAAACAACCAGACGCGGAGAGAGGCCCTGGGCAAGTGGAGTTG
AATAAAAATCAAAGACAGAAGAGGCCCATAGCGGAAAGAAATGAGACAAACTTGAAATGCCATTTCTCCCAAAGCCACAGTTGATTC
TTGCCTCTTTCAATAGTTTGCCTGGTGCCACTGACCTGGCTGAGGGACTGGGCCACGTACAGATGGGCAAGGCCTGTGCCAAGAAGT
TACAGGGAGCTGACAAGCTCAGGGGACACTTAGTCAGGAGAAAGTCTAAAGGAGGTGATGTCTTCTGTCCAAAGGGGTCTCCAGAC
AGGAAGAGCAGTGTGAGCAGCCTCCAGGAATGTGAGAACAGGCCATGGAGAAGAGGCAGCTGTTTGCTGAGAATCAGGCCTTCCTTA
GAGCCAGAGACGGAGGCGGGAGTCCCAATCTAACAGCACAGTCCTCACTTGGTTCTTTAATCAAAGCCAACAGTTCCTAGACTGATT
TCCTTCCTTCCTTCCTTCCTTCCTTCCTTCCTTCCTTCCTTCCTTCCTTCCTTCCTTCCTTCCTTCCTTCCTTCCTCCTCCTCCTCCT
TCTTCTCCCCCTCTCTCCCTTTCTTCCTTTTGTTCTTTCTGTTTTTTCTGGTGACAAAGGAGAGCTTCTATTCAGATCCCTGGCTTC
TGCTCCCCATATGCCTCAGGTGTAAGTCATTTCCATTATCATAGCTCTGAGCTTCCTTCCTATGGAGGAAAGCAAGTCAGAATCCTG
AACAAGGAAGGTCTTTACAGGGCTGGGAACCAGCACAGCCAGAGGGCAGGTGCCTCAGCCTTTAGGAGTCTCCGGAGGACATCTCTT
CTTCCTTCTCACTTTCCAGTGGAAGCTGTTGTTGGACGTGCCTGTGTTTTGTTTCTCGGCTGCAGTTTAGAGGATGCTAAGAAGGCA
CAGGCTGACCGTGAGGCAAGGTGCCCAGTGGGGGGCACTGCTGGAGTGATGTGCTGCAGGACTGTGGAGCCTAGATCCGCAGCATGGC
TCACTGGGCACACAGTGATGGTAGAACAGACTCAAGAACAATGCCTGGGTCTTAGAGACTGGGAAGTGGTACTGCTTACGAGAAAGG
GAGACAAGTATGGAGGGCATCTGGGCATCAAAGTAAGGCTTAAGAGTAGACTCAGAGATCAGTTGATGTATCTGCTGCCCCCATCCCC
ACTCCTAGGTTGCATGCCAGCAGACAATGGCTCTCCTCCTGGCTCCCCTTCCTACTCACTTTTTCCTGCCCTGACTCCTCAGGGGCG
CCTTGGAGTTGGGACCTTGTCCTGACCGAATTTTCTGCCCCCAGTATCTGATCACCTGATATCTGGTTGGTGTCCCCATGGAATGGT
CCCTTGTGGAGGCACGAATGACCTTTTTTTGAGAGTGGGGCTCCCACTCTGAGTCCACCCAGGAGGCTGTGTAAGTGAATGGAAAAC
ATGAGAGGGGGAAAAGAAAGAGGAGGGGAAGAATGACAGAAAAGACAGACAAGAAAAAGGAGAAGGCAAGGGAAGGGCGGCGAAGGAAG
ATGCATGGTTAGGGAGAGGCAGCCAGCACAGGCCCCCGTGCCTGTCCTCACATACTAACACCCTGAACTGGTGATTTGGGGGAGAATGTGT
CTTGACCTGCATAGCTTCCATGGCCTACCTCATTTGTCTCCTCAGTTACCCTCCTCAGCTGGGCTCTACCATGTCAGTCATTCTGGT
GGCTCCTTATAAGCACACTGGTTTCAGAGAAGGTCCCTTGTTCCATATGTGTAGGCCCATGTCTATACACTTGTCTCAGTTGAACAT
GGCTTTTGTCACAGTGGCATTGTTTGGAGTCTAGCTTCATGATGTAGATACCAAGTCCAGCTCACTCTTCCTCATTGACTGAATGAA
GAGGCTAACTGAGGCCAGGATGTGTGTGAAGAGCTAAGTTCGATGGGAGGCATGTTCGATCTCTCCTGTAATGGTCCCACGAACCCA
GGAGAGCTGCAGTGCGCTCCCTGTCAGAGGGAAGCAAGCCTAAACCTGGAGAGACAACCACCCAGCACAGCTGGTTGCGTGGCTGGTGC
GGGGAACCTCCTGATGGTCTCTGCCCACGCGCCCTCTTCCCCACATACAGCCTACTGCCCTGGAAGAAACCTCCCCAGGAATAGCGCTT
CTACCAGGCATTGGTTTTCTCCATTTCCACACATCCTGTGTAACATTTCTCAATCATAAAACATGGAAATATTTTTTAATAAGAAGA
AAGAAACACTGCAAATTGAGAAAGCGTTGCCAGATGAGCCATCTGGTTTTTGATTTAATAACCATCCAAAGAGGTGGACAGCGCTCC
CTCTGAGAAGATGGACTGTGGAGGGAACAGCAGCTGGCTTCCCTCACACGCTGCTTCCTCTGGCAAGGTTGGAGTGGGGTTAGGGGA
GTGGAAACAACCAGAGATGCAGGGAAGCTCAGCTGGTGTCATCAGATGTCCCTGGACTCTGGTGGCTTCCCTTCCTCGCCTCTGGG
CACATGTTGTTAGTTCTTAGGCCTAGAAACAAAGAGCTAGACTTAGATGTGCCTTGGAAAGATCCATCTGTCAAAGGGGAGCATCGC
TATATCTCTAAACTCCAAGTTAGCTCTTGAAAAGCAGTACTGGAAGTGACTTTTGCCTGCGCCATGACAAGTGTTCTCCAGGCCTCG
TGCTCGGCCTTTTAGCTCACGTCTGAAGACGTTTAAAGAAGTAGCTGACTTTTGGGTGGGGTAAGCAGGAATAGTTCTAACGTCCTT
GAAGCCATTTACTTTTCAGTTACCTCTGAGTTTCAGTGACTTCTTCACACACCTCCCACATTTCCCGAGAGCCCGCAAGTCCTTGAC
AAATCATAGCAATGGCAGGCCCTGGGGTGCTAAGGATACTTGATTCTTAGAGTCAAGTAGGGACCTGGACAAAGGCAGAAGTAAGGA
CTCCATGACGTGTCTCCCCTTCAGCACTAGTGTATCAGGATCCCTCTGAGGAGCTGGCAATAGCCAGTCACACACACACAGACATTA
GCCGGAGACAGAGGTACTGCCAGACCAGGGTCAGAGGCTGCCCCCATGTCTTGTCCTGCCTACTGTGGCAGCTGCACCTGGGCTGGA
AACAGAGATCAGGTTCCAGCCTTCATCTCCTTTCTCTTGCTGGCGGAAGCCAGTTTGGTCAGTTATCATTTGGGGTTTTTTGTTGTA
TACATGTGAAATTAAGGCAACAGCTTTCTAGCTCTGTTAACTTTGGCCTGCCTGCTTGTGCCAGGGCTCTATGACTAAATGGGCTGT
CCCCTGTCTTCTTCCATTTGCTTAGAAAGCATTCACTGAGTAGCAGGGACGCATTGGTAAGGGGGTGTGGCAAATAGGAACA
GCAATGACAAAGCCAAAGTGCATCATGTTGGATGTTGTCAATGCAGGGGTGGTGGGGAGGAGGTGTCCTAGGACAGGAAGCCACGGG
CTTGGTAAACGCATCGAGGATGCAGGTGGGAACAGGGTAACCTGGAGTTTTACCAAGACCCTGCCGTGTGAGCCAAGGACTACTGTT
GTCTGGGAGCATCAGTAACAAGCAGGAAAGCTAGGAGATGGTACTAAACCAGGCAGTTTGGTCGCTGAGTAGATGTCAGAGGAGGAA
GGGGGAGGTTTATGGCACAGGAGCTCACAGAGGCTCCATGGGTCTGCAGGGATAGTCCTTAGGCACTTGAAAGTGCTGGTGTTTGCTC
TGAAGGACCCAGGGCTGTCTGTCGTGTTCTTTGATAGGGCCATTCCCTTGCTGCAGTGACACTATGTGGCCCAAGGTGTGGCTGTACA
AGCTGAAACAGGAGTACAAATTAGCCAGCACCTCCATGGGCCTTGTGACAGGACAAGGAAGGCCTGAGTGCTCACATTCTGGAACT
TCCTTTAAGGCAAAGATTTGCTGGAGCCAAGGAAGGGGCAGTGGTCCACACAGAAGGCAGAGCGACCTGGCAAACTGCAGAGAAGCT
AGGCTTTTCCCGAAAAGCACTGATGCCTGCTTTCCCTCCACGTCTGTGCCACCGTCCTTGCCACTAGGGCAGAGCCCCAGGTGGGAG
GCTCTTCCGGAGGCAATCACATGCCTCTATCTGTGTCACGCACAGAAGGCTGACAGCTCTTCCACACTCTGGTTAGATAGGGCTGGA
GTGATTAGACCTGATTCCCTGAAGACAGGACACAGGGACAGGATTTGTTCAAGGTCACAAAGCCATTAAGGTATGAAGGACAGTCTTTG
GGTGCTTCTCCAGTTCTGCTGAAACAAGTCTCACCCAGGGAGACTGTCATGACAGAACAGCTAACAGTACTTAGACCCGTGCCAGGCT
CTGTCCTCATTCACTGAATAGGAACTCTGTGAGCCGGCATCTCCTTTTACTGGAAAACCGGGGGACAATGTACTGCGTGTACCGTGG
GGATGGACCAAGAATTTGTGCTCTGGTCCCAGAGCCTGCGTTTCACATTGACACCAAACAGCAGCACAGAGCCAGAGCAACAGTGG
GTAATGAGGATGCTCTATGGTGTGGGCCAGAGCAACAGCTGGATAATGAGGATGCTCTATGGTGTGGGCCAGAGCAACAGCTGGAGA
GTGGAGGTGCTGTATGGTGTGGGAGTCAGGGGAAGAGCTCCAGCCACCAGAGGGTTTGAGCTGGGTCTGAAAGGATCAGTTAGAGGG
```

```
ACCAGTGAAGACCAAGGCAGACATGGTGGGATGGAGGGGCATGTGCACAAGGCAAGCTTGGGACACCAAGGAAGTCAGGGCAAGAGG
CTTAGAAAGCAAGGCAAAAGGGGGAAAGGAGGACAGCAAGCTACCGAAAATGCCAAGGGGCAGGGTGATGTGAGGAGAGCAGTGCAG
TGGGGTCCGAGGCTGCCATGGAGCCAGATGCATGCCGCGAGTTCTTCACTGGAGACCTGAGCAGGGACTCCAGGCTCAGCGTGGTAT
TTGACTATGGGAATCTGAGCGTGAACTCCAAGCTGAGGCCATCATCCATTTGGAGGGAGTTATGCGGAAGGCCTGGCTTGAGCTGTT
CGACATTCCAACAAACAAACCAGCCCCAGGACAAATGGCTGGAGCCATCATCCTTGGGATCCATATGGAAAGACTCCTGAAGGAAGT
CTACTTTACACAAGTGTCCAAACTCATTTGGAGACAAAGGGGAAGAGGCGTATGCTCTTCACGTGGGCCTTGCTCCCGTCACCCTGTG
CTGGAACGGGTCACAGCCAGTGTCAAGAAGCTGAGTTCTTATTAATAGTGAGAACCAACTCCTAGCTCAAGGAATCTGGCAGCCAGT
TTGAGAAAGTCAATGACTGAAGACAATGGCTGGAATCCCAGCACACTCTACTGGGCACTCCAGTTGGTGTTTCTTTCAGGATGGC
GCAGGAGCGGCAGGAAGAGCCATCTTCGTGCCACAGACGTTTAGTGAGCACCTACTACATGATAGGCATGGAGGTGGTACCCAGAAA
AGGCTACAAAGAGAACTCTTATGTAACTGTAGGGTTAATAGGCCAATGGAACAGTGCGAAGAACTCAGAAATAGAACCACTTCCACA
TGGAGGAAGCACGGAGAGTCAGGGGGAGGGAAACCAGTTTCAGAGGAACTGCAGAAAGTCTCATGATCTCAGGAAGAGAGAGGGGTTTCC
TAAACATCACACAAAGCGCTAAGCTTAAATGACAATGGAGGAACACAACTTCAGTGAAGTCTAAAATGTCCATCAAAGACACTTTAA
GGCTGGCAAGGTGGCTCATCAGGCACAGGCGCTTGTCATCAAACCCAACGATCTGAGTTCCATCTCTGATCTACTTGATAAAAGGAG
GAAAATCTTCTACAAGTTTTTCTCTGACTATCATATCATATCATATCACATCATATAATATGAGCCCCCCATAAAAAAAAAACAAATG
TGATTATGATCTCCAAGGGTCAGGCATTCCAAGGCATCTTCAGTGGGTACCTCTGGCTCAATATATCTCATGAAGTTACGACCTTTC
TCTCTTCCAGTGTTTAGGGGTCCAAACTCATCCCTGTTGGTGGCAGGGGTGGACCCTTACCATGGGGGCCTCACCATTGGTTT
GCTTCAAGACACGGCAATGAGTTCTCTCAGGAAGAGCAATGAGAGAGGGTAGGGGAGAGATTACCAAGACAGAAACTACAGCCCC
GCCGCAGTCTAACTTCTGAGCTCTGTGTCAGTCTAACTTCTGAGGAGAGCTTCAGTTGTCTTTGCTACATTGTATGCATTAGAAGCG
AATTAGTAGTTCATTCCATATTTAGAGAACAGGGCTTAGAAGCGAATTACTAGTTCATTCCATATTCAGAGAACAGGGCTGGTTCAA
AGGTGTGTGACTACCTCCGGATGGAGGGACGACTAGCAACCATTCCAAAAGCTACAGAGTACACTGTGAAGACGAAGCTCACGGTGA
AGAAAAGGAAGAGAGAAAACATAAAATCCAGGGTAATGATCACCCTGGGCAGTGGTGATGGAAGACTGTGGGAATAGCTGGTGGGG
GTGAGGGCCTGGGGGTTGGACCCCTTTCTAGATGCTTATTATTTTTTCTTTTTATGTTTTTATGTATTGGTTACTTTTAAAAAAAATTATCC
CTGCAACAAAATGTCCAGCAAAAGCAACTTAAAGAGGGAAGGCTTTATTCTGGCTTGCAGTTTGGGAATGTAGTCCACTATGACCAG
GAAAACATAGAGATCAGGTATGAGACCACCATCACATTGTATCCTCAGGCAGGATGCAGAGACACTGTGCTGGCACTCAGCTCACTT
CCACTGGAGCCCAGCTGGTGAGGCAGCACCCTCATGTTCAGACCATGTTCTCATGGTCTTCCTTGTCAATTAAACATCTCAGAACTC
ATAGATACACACAGGGGTGGCTCCTTGCGATTTTAAACCCTGTTAAGTTTAAGCATTTTACATCATAGCAATTGTTATATGTATTTT
TTTCTTCTCCCTGAATTTGTTGTTGTTGCTGTTGTTTGCTTGTTTTTGAGATGAGGTCTCTATCCCAGACCTCATCTTGAACTTGCT
AAGTAGCTCATCTCTTGATCCTCCTGCTCTGACCCTTGAGGGCCGGGAACCCTAGGTGTGTGCCACACTTCAGTTATATCAGCGCTG
GTTTTAAGGCCATGGCTTTGTACATGTTAGGTGTCTTGGTTACTGTTCTATTGCTGTGAAGAGACACCATGACCAAGGCAACTTATA
AAAGATACTGTTTAATGGGGGCTTGCTTACCGTTTCAGAAGACTAGTCCATGCCTCTCACTGCAGGGAGTGTGGCAGCAGGCAGGTA
GCTATGGTACTGGAGGAATAACAGACAGCTTAAATCTTGTCTGAAAGATGGGGCCGAGAGAGGATGACTGTGCCGGATGTGGGCTTT
TGAAATCTTAAAGCCCACCCCCCAGTACCACTCCTTCTCCAGTGGGGCCCATACTTCCTAATCCTTCCCCCCCAAACAGTTCTAGCAGC
TGAGAACCAAGCACTCTGATCTATGAGCCTATGGAGTCCATTCTCATTGAAACCATAACACTAAGCAAGCACTCTACCAACTGAGCT
ATGTCCCAGCCCTTTGGTTTGCTTTTATGTTTGATATGCATGGCTTCAAAAACCTTTAACATTTACTGATTTATCTGATTATTTAT
TATTTGATTTCTATTTTAGGGGTTTTTTTTGAGATGATATCTTGCTGTGTAGCCTCTGAACTCACTACAATCCTTCTGCCTCAGACC
CTTGTGTGCAGAGATTGCAGATATGTGCCTCAGATCTTCCTGCAGGCTTCCTGCAGAGACTTCTAATGTCTGTGCAGCCCTAGCTCTC
CCTGCTTATCCGTTTCCGGAGGCATCTGACTCCCAAGCCTTCTGCTGACGAACTCAGCACTTTCCTTCCAGGTCCCTAAAAGTCATGCT
TGGAGTGTGCGCTTCTGGTGGCTTTGGTTTCAAGAACTGTCTGTTCACTAACCGTTGAAAGGTGAAATCTCGTTCCTTGCTTTCCCC
TCATCCCCTCAGCTCTTCCTGTTCCCAGTTGTTTTTTTAAAATAATAACTCTATATTATTTTGGCTAAATCAAAAATCAGTGTTTAC
GTTACTTAATCAAGACTAATTTGACTCTATTCATGGTCGGATCCCATGGTAAAATCCAATCATTTTCCCTCTCTTGCAGCCCTTTCA
TCTTCCCTAGAGTTGCTAATTATCACTTTTCTTCATTTGCTTAGGTTTTCTGTGTACTTATCACTTGTTAACTCCAACCTTGCCGTTT
GTCAGAAGTTCAGGCCTCTTATACCTTTTTAAAAAAATTGTCTTCCTAAAACTGGCCCTAGAGCCTCCAGCCTTCAGACTGGTGCCCT
GGTCTTTTCTCCTCTGCAGGACAGATTTCTCTAGCCCTGGTCTTCCTTCTCCTGGCCTTTGGAGAGCCCAACTCCACTGGGCTGTCT
AGTTTAATGTCAACTTGACACAAGCTAAAGTTATCTGAGAGGAGGGAATCTTAATTAAGAAAACACCTCCATAAGATCTGGCTGTAG
GGCATTTTCTTAATTTGTGATTGATGGGAAGGGCCCAGCCTGTGGGTGTTTCTGCCTCAGGGCTGGTGGTCCTGGGTTCTGTAAGAG
AGCAGGCTGAGCAAGCCATGATGAGCAAAGCAGTCAACGGCACTCCTCCATGGCCTCTGCATCAGCTCCTGCCCCCCAGGTTCCTGCC
TTTGTGCATTTCTGCCCTCACTGCTTTTGACGCTGACTTGTTATATGAAACTGTGAGTGAAATGAGCCCTTTCGCTCCCAAGTGCTT
TGGGTCATGGTGTTTCATCACAGCAATAGTGACCCTAACTAAGACACTCACATGGCACCCTGGGGAAAACAAAGCTTCCGGACTGGT
GGTTCCCAACTTTCCTAATGCTTCCACCCTTCACTCCAGGTCCTCATATTGTGGGGACCCCCAACCATAAAACTATTTTGCTGTTAC
TTCATAATTGTAATTTTGCTACTGTTATGAGCTGTGATATAAATATCCCATATGCAAGATATTTAATATGAGATCCCCCATAAGGGT
CTCATACCCACAGGTTGAGAGCTGCTGTTCTAGAGCCTTGTGATAGGGAAATACCCTTTACTCTGTCTCGTTCAGACTCAACTAAT
GCTTCGGCGCTGTCTTTGTTACATTTCCATTGCAGTAAGCACCATGAGGCAGGGAACTTCAGAAATGAGAGTTTATTGGGACTTA
CAGTTTCAGAAGATCCATAATCATATGGCAGGAAGAAGAGCAGCAGGCAGGCAGGCAGGCAGGCAGGCAGGCAGAGCACTGGA
GCAATGGTTGAGAGTGCATGCCTTGAGACACAATCACAGAACAGGGAGTGCTAACTGGAAAAGAACCTTTGAAACCTTAAACCTCAT
CCCCAATGACACACCTCCAACAAGGCCATACTTCTTCTCTTAGTTGGGGTTACTATCACTGTGATGAAACGCCATGACCAAAGCAAC
TTGGGAAAGAAAGGGTTTATTTAGTTTACACTTCCACATCACCTGTTCATCACTGAAGGAAGGAACGGAACTCAAACAGGACAG
GATCTGGGAGGCAGGAGCTGATGCAGAGGCTATGGAGGAGTGCTGCTTACTGGCTTGCTCTACATGGCTTGCTCAGTCTGCTTTCTT
ACTGAGCTAGGACCACCAGCCTGGGATGGCCACACCCACAATGGGCTGGCCCCTTCCACATAAATCACTAATTATGAAGATGTCCCA
CAGGCTTGATTACAGTCCAATCCCATGGAAGCATTTTCTCAATTGAAGCTTCCTCCCTCCTATCTGATGACTCCACCTTGCATGCAA
GTTGACAGAAAAACCATCCAGGACACTTCCTAATCCTTCCCAAACAGTTCCACCAGCTTCGTACAAGTCTTCAAATATCTGAGCCCA
TGAAGGAACACTCTCACTCAAACCATCACAGATGGTCTCTCCTGACCAGAATTCTGATTTGGAAATTATTTTCCTTTAGACTTCCTC
CTAGACTTTATTTTATGTGTATGAATCTTTTGCCTGCCATGGATAGACATGCCACCATGTGATGCCTGTGGAGACCGAAGAGGGCCAT
CGGATCCCCAGGAGCTGGAGTTACAAACGGCTGTGAGCCACCATGTGGATTTTAGGAACTGAACCTGGGTTCTGTGCAAGAGCAACA
AGTGTGTCTAACCACTGAACCATCCCTCCAGCCTTGTTTTTCCTTTAGACTTTTGACAGATGTATCCCATGCCTGTCTAAGTTCCAT
ACCCTGCAGCTCTCCTCCTCCTCAGTGGCACTGAAAAACCTACATAGTCCTCGCTGTGAGCCAGTCGCTGCTGTCCATTGTGCGG
GGGACTTTGGTAGAAGCTCTCCGGATGGCCTCTCCAGCCCCGCCCCACCCAGTTCTTTCTCTCTGGAACTGATACTTGTATCTGTAGAC
AAACCTACCAGTTTCCTTGTATGTCACGTTCCTTTTTGTTCTTTCTGTTTTCTGCATTTGGGAGTCTGCTTGATTTCTCTTCCCGCT
CCGTCCAACTGACTTTCCCCGGCAGAGCGCCCCTTCTTTTTATTTTGTTGAAAATAGACTCTTCTGTCACACAGTACATCCTGGCTA
CAGGTTCCCCTCCCTCTGCTCCTGCTTCACAGGTGCGACATCTTATTTTATCTCCCTTGTGATGATAATTATAGATTCCCTCCTCAC
TCCCACGTCTGACTAGCCCTGCCCAATGGTTCTTTTCTGTGAATGATCTCGTTCGATCGATCCCGATTCCGTTTCCTTTGNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNAGCCATGGTCCACCCTGAGGGGAAAAATTAACAA
TTATAATAGTATTCTTGAATTGAAAGAAAAACTTCCATCTTTTCTTCCTCAGCCCTTAGAGAAAAACATGAAACTACTTCCTCTCCA
```

```
AAATTCTTTATTGCAAAAAATAGCAGGAGATTAGTACGTGGATGTGTGACATGTGTAAACCACTTAAAGCTCTGACTAGGCTTCGAG
CTGCTCTCCCTCCCACAGCCCTGAATCACCAGCTGACTGCTCCTGCATCACTTCCTGTTGGTGAGTTGCAAGTGGGTCTCATCTCCA
TCACGGCTCTCCTGCCACAGCAGAGCAGTGGCTCCCAGCAGCAGAGCCAGGTCAATCAATGTCAGCTTCTCTGTCACCCCACAAGAG
GAGGGGGCAGTGGTGGGCACCTAGTGAATGGGTGGCTTTCTCAGGATATATCCTCTCCTTGTCACACAGCAAAGGCTGTTTTTGTCT
ATATATGTATATGTTCAAGGAAAAATTTTATTTTGTATTTTAGGCATATATATAGATAAATAGATAGATAGATAGATAGATAGATAG
ATAGATAGATAGAGAATGAAAAAAAGGAAACCCACAAGAAAAATTTTTAAGAATCATTAAAAAAAGCAGTTTTATATCATCTTTAAATG
CTTGCTTGCTTTTTTTTTTTTTTTTTTTTTTTTTTTTTTTTTTTTTTTTTTGTTTGTTTTTTGAAACAGGGTTTCTCTGTATAGCTCTGGCT
GTCCTGGAACTCACTTTGTAGAGCAGACTGACCTCAAACTTCAGAAATCTGCCTGCCTCTGCCTCCTGAGTGCTAGGACTAAAGGTGT
GCACCACCACACCCGGCTTGCTTGCTTGCTTTTTGTTTTGTTTTGTTTTGTTTTGTTTTGTTTTGTTTTGTTTTGTTTTGTTTTGTTTTGA
GATAAAGTCTCTGTATCTGGCCCTGGCTGTCCTGCAAGTCACTATGTAGACCAGTCTAGCCTCCAACTCACAGAGACCTGTCTGCCT
CTGCATTCTAAGTGCTAAGATTAAAGCTGTGAACCATACCTAGCTTGCCCTGTATGTAATTCCCCCTTTGATCTCTAATCTCTGCAC
CTCAGAGTTCACAAGCGTGAAAATGGTTGGTGTGATGCTGAGGCCAGGCAGCCGCTCAAGAACACTAATGTAAGCTGCAAGGTTTGC
CAGGAAAGACCTGTGAGGTGCTGCAGGCACACGAAGCAACAACATGCACAGTACTGCACTGCCGCATACTAATCCCAGTCAGCGGGC
TGACACTCTACGGTGCCGGTGCCGGTGCCGTGCTGCTGCTCTGCGTGCACAGCAAAGGTGCTTCCTGCTGGGGCCCGACCTCTGTAT
GCTGTTGTCACCTCGGCACGCTCAGATGGCAAGCTTCTTGTCAGTGGCCTTGGAGCAGGCTCTTCTGTTCCCAGGTCAAGCACTTCA
GCCTAAACTTCAAGGCAGTCCTGTGAATGCCCTGCTATGCCTTTCCTCTGGTGTCATAAATCCTACCTTTTCTGTTTCTGCCCTTCC
CCGTACAGCCACTGCTGATATGTGCTAAATACTTACTGTGGGCTACAGATTACACTGCTTGCTTGCTTGCTTGTTTGTTTTAATCT
TCCCATTAGCAGATAGGAAACTGAGTCACAGAGGGATTTACATTACCTGTCCACAGCCACGGAGCACAGTGCAGCTGGCACGGACC
ACACCCAGATGTTGCTCATGCCTCCTGACTGAATCCCAGTATTCTTAGACCGGACCCAAGCTTCCCCTCCAGAAAATCCTCTTCCTG
ACTAGGCGCACTGGCTGTGTGCTACCGGCTGTGCCCCTGCAACGTTTGCTTCCGTGCTGCCGACTCATTGTGCAGGCTCTGTCCGTC
TGTCTGTCCTTCCCACTGCCACTGAGCCTTGCTAGGCCGCCAATCGCAGGCTCGGTTTTGTAGTCTAGAGCCTGGGAGTCAGAGCT
TGTTGTCTCCTCCCAACATCCAGGAAAGAGCTACAGACCAAGGACAGCAGGCAAGTGGGACAGTGGACAGATACCTGTCAGAGCAGGGCT
AAGGGTGGGAGAGTGGAACAGGATCTGGCTTGGTCCTGGAGGAAACAGTGGGGTGCTAAGCCTGAACCTCTCCAGGATGGCCATGGG
AACAGAACACGCCAGCCTGGGCACATGAACCTAGAAGAAGAAGCCAGCCAGGCTCCTAGCATGGCTGAGAATTGGATGACTGGGCGA
TTAAAACTATCTTCAACTGTCCCCAGAAGCTCCAGGCACCCAGTCCCTTGTACAGGCAGCAGTAAAAGCCACTGCCATGGTGATGAT
TTGGAACTGGATTTGAGGGCAGTAGGCAGTGGTGGCCATCTTGCCCCAGTTCCGCCACTCTGCAGACCTAGCTTGCTTCCTTGCTGCTGTGC
CTCATCTTTTCAAGCATCCTGCTTGTTCTTCCTCTACCAGGGAACCCAGAGTTGCCTCCAAGCCCTCTAATGCCAAATGCAAACACC
TGCCCATAGAACCCCAGGCCCTCCAGCCTCTTCTGAGCACTGGTCTCCCAAGCTGCGTGTCCACTGCAGAAGCCCAGGTGACAGGTG
ACCGCCTCCCGTGGACACTTTCTCTCTGGGACACGTGCCAGTCTCTTCTGTTTCTCTGCTGTCCAGTGGAGCAGCAGAAGTCATGGG
CTTTGCATCAGTTGCTTCCTATGAATCACTCAGCATCTCTGTGCGTCTAGCCGCTTCCCCGTCAGGACTTGAAAGGCTGCCGTAAGA
AATAGAGTGTTTATGTTCAGAGCAGCGCCAGTAGATATAACCAGACAGCAGGTGCTCTGTAGATAAAATCCTTTGCACTGCCTTGGT
GTTCCTCAACTAGGTGCTTGTCAGTGACTAGCAGCTTTTCTGCTTCCTTCAGGGGCATCTCCAGATACACTTGTGCTTGCAGATAGC
CCTGCATGCCTCACTAGGAAGGTGGGTCTGGGGAAGCAGTGCTGGGTGATCACTCATGGGGTATCAGTCTCGGTGCATGGAGATTGC
AGCTCAGTATAGCTCGGGCTCAGTCCTTACGTATGCCATGTGTGTTAACAAGTGTCAGTCTCCTTGGTTACGACATTCCCAGAGAGC
TGCCCCCTTTACTCAAACAGGCTGGCTTGATCTCCCTGCCCCTGGCTCTCTTCCCCACTGTTTATCAGAAGTAGGATTGCACTGCAT
GCCACTCACGCTGGCCCAGTAAAATGACCCTTTGAGAAAGGACCTTCCTTCCTTGCCATCCCCTTTGCCATGCTGAGTTTGGG
TTCCCAAGACCTCCTGGAGAACTGCAGCCCTGCCCCCACCTCAGTCCACGGTGCTACTGTCACAGAGCACCACAGACTGGGTAACGT
ACGAAGATGAAAAGGCTAGTTCCCATGGTTTTGGATACTAGGATGCCTTTTATCAAGGCATTGTCGAAACACCAATAAGTTTAGCGT
CTGGGAAGGCACCTGGTTGACACATCCTCCAGAGGGAAGGAAACTATGTCCTCATGTCTAGAAGGCTCAAGAGCAAAGCAGTAGACT
GGTACACAAAAACCCTTTTAAGTCAGGTGAAGGGACACGGGCCTGTAATCCTGTCACTTGTGAGATGGAGGAAGAAGGTCAGAAGTT
CAAGGCTAACAGCACATGAGAATGTCTTTTTTTTTTTTTTTTTGAAGGTAAGTAATAAAAATAAAATAAAAATAAAACAAAA
CGTTCCCAATTCATTCATGAGAGGATACTTCACAGCCTAATCATCTCTAAATGGCCATCTCCTATCATCTTGGGGACATCGGCTTTT
GAGGAGGGGAAGACATTTGAGCAATGCCAGGCACCTTCAAGCTGAGTGTGGAGGTTCTTGCCTTTGATTCCAGCACTCAAGGAGGCAG
AAGCAAGGGGATCTCTGTGAGTTCAAGGTCAGCCTGATCTCCATAGCAAATTCCAGGCCAGCCAGAGCTACAGAGTGAGATCCATTC
CAGGAAAAAAAAAAAAAAAAAAAAAAAAAAGGAGGCACCTGCCAGTGTCTCCTACAGAGCCCTCTGCAAATGTCTGTATATCCTCAGAGCAG
AGCCCCAGGGCCAGGTTCTCCCGAGGAAAGGAAGGAAATACAGAGGAAATGGCCTCCAGGCAGGAGGAAATGAGATGATAGTTCTAGC
TGTGTTCTGCCACTAATGTTTGTCTGGGTCAGAAAGAGGCTGAGCCTTTGTGAACACTTCCGCGTTCCCCCACCTGTGAGGTCGGTT
TCAAGAGAGAGACCCAAGCTACAATGGCTTCCTTCTACATACACAGGAGATACCAGTCCTGAGAGAGAGAGAGAGAGAGAGAGAGAG
AGAGAGAGAGAGAGAGAGAGAGAGATTGATCTTGTCCAGGAGTCTGTGATTTCAGATTAATCATATAGACGGTCGATTTAGAAAG
AAAATTAGAATATTATGCATGCATGACCTCAGCTGAAGCTGTCCTACCAACAGCAAAAGGAAGTCTACAACGCATCACAGATCGTGA
CAAAACATTTTTGCCTTTGGGACTCAGGACTGCAGGCAGGCAGGCATGGGATCTGGATGGGTCTGATGCAGCCAGCACCTTCGGGAAGGCC
TAAAGAAAGGTGTCTCACTGACACCACTGTGTGTTCCTCAGTACCCCACCCCACCCCCCTAGACTGTTCCACAGTCTCTGTTCCCAG
GAATAGGAGTTTCACAGACTAACTCCCGTCTTCCCTCGGCAGGTACCATGATCAGCAGGATGTAACTAGTAACTTTCTGGGTGCCAT
GTGGCTCATCTCCATCACGTTCCTTTCCATTGGCTATGGGACATGGTGCCCCACACATACTGTGGGAAAGGTGTCTGTCTTCTCAC
CGGCATCATGGTGAGTACCGCCTCTGCCCATGGTCCTGCCCTCCCAGAGGGGCCTTCCTGGGGAGGGGGAGGGGGAGGGGTGCTGGT
CACTAGGCACTCATGCCTCCCCTGTGCAAGTCGAGAGGCAAGGCTGAGTTGTGCAAATCTAGTTTCCAGAGCCCACCAGCTGGAGTT
GGCTGTCGCTCTGACCCTCTGCTCACCACTGGGGAACCTCAGGCTTATCATCTGCCCATGCTCCTTTCTGTGTCCTGGATTCCATAGA
CCTCAGCACCAGTTCTGATCAGGTTTAGCTGGAGCTCAGAAATAGACCATTGAGCTGGCCTGAAGGGCCCTGGGTCCTGAATGGGCT
TGGCTTGCTGGCTTGAGTTCTGGTTTGTCCACATGGCTCTGGGACAACTCTTGGCTTTCCCAGGCTTTGTCTTCCACGCTTGCACAT
TGGATCGCTGGCTCTGAGTGACAAATGCTTTCCAGCATGGGTATCTGGTAAGACCAACAGGTCTGCAGGCACCCAGCACCAAGGTCT
GCTTCTTCCCACCCTCAGCCAAATGCCTCAGGCTCAGGATGGGCACACGGCACACGTGTTCCTACCTTTCGCTGGGCCGTGTGCGGCAT
ATGGGAGGGATGTGGTGTGCAAGTAAGACACAGGGCCCAACTTTTCACTATCCCTTCACTTTGGCCCCTGGGCCAGTCACTCAGCGG
TCACCTGTGGAGCCTACAATCTTTGGGTATAGCTAGATACTCAACTGGCCTCCTCACTGACTTGTGGGCCCCAAGATATAACTGATG
TGAACATTTTGGGTAAAGAGTGAGTACTGTATTCAAGCTAGCCCCACATTTGAAGCAATCAGGATTGGGGGATATTTTAGGGGTAGG
ATAGGGAGTGTCTACTATGATGTCCTATGTGGAGTCTTAGAATCAATGACAGACTTCCTTTTTAGCCCAGGGAGACCTGTTTCTTAG
AAGAGAAAAAAAAATCCTGGAGAGAGGGGACCAACAGTCCTGCTGGGAGCCATTGAAGCTGATAATGGGGTGACAGTACAGATAGGAT
GTTTGAGGCCTGGTTCTAGGTCTCTCTCCACAGTCTGACCCCCAGATCTCTCAGTGCTGTCTTGCTCTGCACCCCGCCCAGCTGCTG
TGAAACCCCTTTCAGGGCTCCGTTCTCACTGCCCCCAAGAGATAGGACAGCCTTCCTGTGAGGCAGTGACTGGTGTCTACCGAAGGA
GCCAGTCGGCTGCTAGGTATAGAGGAGACCCCAGGGCGGTGGCTGGCTGACCACAGCCTCCTGTTCTTTCCAGCTCCCAAGCAGAGC
GGGCCCATGTGAGCAAGCAGCCTTGAAACAGCCGGTAGTGACAAGGGCATAGTGCCAGGCTGGGATAAATCACAGTAAGTGCAAGGC
TTTAGAACAAACACCTCCCATCGTTCAGTAGGAGTCTGTCTAGGACAGCGCCAACAGTCACCAGGAAACCAGGCAGAGTCTTCCTAC
CAGAAAGGAGGTTAAATGCCACCTGAAGACGTGTAGCTTTCCTTCTGAGACTCTTGGAGTGAGCTTCAGGCCCCACCCTAGTTCTCAGG
CAGGGTACTAGGAGCCTCCTTGCCAAGAGGTGCAAGAGCCTCTGTTATTTTTAAATGCCAGCCATTCTTCCTCTCAGAATGGAGCTG
TGCCCTATCCAAGAAGCTGCCTGGGCCCACTGTTTCCCTGGCTCCCCGGTTTCACATCTCCTGGCTTTGGCTCTAAAGCATTGGTAA
AAAGACAGGAGGCCCTGGTCTGAGACACTGCTTATCCGTAGGCAGTGGTCTGCGTTGGCCTCCCCAGCACCCTCTATGGCTGCCTGG
GATCCACGGTGTTGCTTGTGGTTTGTGGTGAGATTCAGGTGGAAAGAGCCAGCAGGCAAGAGGCCACACTGCCCTCCTTAAATTCCA
```

```
ACACAGGCTTTTCCTGGGAGCGCTTGCCTAAGGCCTAGCTCCCCACAAAGGCTCCAGCATGGGACGGTCCCTTTGTTCTTTGTCAAT
GTCTTCACCCATATTAAATGCCACTCACTCTGGAAGCCTGGTGGCTTTTTGTTTTTCTTTTTCTCTTTCCTATTCAACAGGACTCCA
GCTGAGTCATTCCTTTCTAGGGGCTCACATTGCTAAGCCCACCCCCAACATGATGTATATCTCCTGATTATTATTCCAAGGGCCTTG
ACTAAAGGTAGGGCTTCCTGTTTGGTTGCCATGGACACCACCTAGGCCAAGGCAAGGGCTGCACAGGAAGGGAAAGGAAGGATTTTG
TGTGCCTGCTACAAATGGCCTTAATCATTGTTTTCCTTTGGCATCCAGCAACTGCATAATTATCCACCATTAGGCAGGGCATATAGC
TGTCTGGACAACTTGACACCGTTGGCTTAGGCCTGGAGCACTTGAGAGTTCACTCATTAATGCAAATCTACCTTGAGCCCCAGCACC
TTCCCATGAGGAATTTTCCAGGATACTTTACAAGGTAGTTACTATTCACCACCCAGAAGGAAAATGAAGCTTTTGGCTTAGCTTCTG
TGGGTATCTGGGAGAGCCTCTGGGCCCCCAGAGACTTTCTGGTGGTAACTTCATGTCAGTGGCTCTCTCTGTGTCCCAGATTTGGA
TGCTCTCTGTCACCCTGATGCGGGTTGTAGACAAACAGGCCTTGTAGCCATCCATCCAGTGGAACAGTGAGCTTTCCCCAACCGGGT
CTCGAGAGATGCCTCTTGAGCTGATGAAGAAGAGGTGACACAAACCGAGTGACCTTGGGGGCAGCACAGATGGTCTCAGGAAGACAT
CTGCTTAGTGTCCTGTCCCATGCAGCTCTCCTCAACCGCTGGGGTTTCAGAGCTGCTGATGATATTTTGGCTGTCCCACAGAATCTA
TTGCAGCCCCGAGGCGACCGTAAATGTTCCTGGCTGCCTTCTTCCATGTGTCACGTTCATTTATGTTTTATCTCCTTTTATGCCTCT
TTAGTTAAGAACACCTCATACTTTTCCCCCTCGGGATACTTGTTTTTCAACATCTAAGATTTTCCAGCTGGACATTTTTCTTGGAA
ACGGGAAGTGGGAGGCATGGTGTGGAGCGGTTGGTGTCCAGATTTGGGTGACGACTTAGACATGTTCACCCCTTTAGAAACCCCTTC
TCTGGGCAGTCTGTAGCCTCTGCTTACCTGTGTACCTTGGGCAGTCTGACTTTGTTGGAGGCAGGTCCTGCCCCGCTCTTTGCAAAG
AAGACTTGGGTGGAATGGACTGAAGATTCCCATCAATGTCAAGGGAGTCAGGTCTTAGCATCTAAAGTCAAGAGGCTGAGTCACAGA
TTTGCTCCCCATTTTAACCCCACAGAAGTCACTCCAGCAGCTGTCATCTTTCCTATCCCATCACCCCCCCTCCTCATGCCCCTCTGT
CCCCCTCTGTCTCCCTCTGTCCACCGGCCACCATCACTGCGTTTCCACACAAGGCACAGCGTGAGCTCACATGCTGCACTTAAGCAGG
TCCCTGAGAGTCCATCCATTAGAGCAAGCTGCACAACGGCTGAATCTCACTAATGCGCCCGGGTGGCTAATACAACCCTGGGGTCAC
TCTCAGATACAAGACATTTCCAAGGGAACACAGAGTGGTCACCATCAAACCCATAGGGATAGACAGGCTGGCCATTGGGAAGGGGGC
CTCATGGCACTTCCTGGGTGTCCTCTAAAGACCTCAGATGGATCATTCGCTCTAACCCTGGCTCTTCACTCTTCCTTCTCACACCCA
ACTGTTGTTTGGTTGTTTCGTTGTTTCTTATCCCTGGCTTTCTCCTTCTGTTTTCTGATTAGTGCTGATTAGAATATGGCCAAGTTG
TTTTCTATCTCATTCTCCATTTCCCCTGCTCCCCCATCTTCTCAGTCATCCTCGAGAGGACCAGCAGCCTGGGCTGTAAACACCTCA
CACCCATTTTCTGGGGGTTGTTGCTGCTTGATACCACATTCACAGAGTTGCCTCTTATACACATTAATAGCTCTGGCATCATCATCCG
TGATCTCCCTAATTACATCCAGTCATTGAATTATCTAAAAGAAAGCAAAGGGTGAAGGCTGGCAAGACGGCACATTGGCTAACGGCA
TACTGCCAAGCCGGATACCCTGAGCTCAATCCCCAGGACCCATAGGTACAAGGAAAGAACTGGCTTCTCTTCTGTTGTTCTGACC
CCCATGTTTGCACCCGTATACATACACATGCACACAGATAATTAAATCTAACAAATTTCTCATTAAGATTGTGCATACCCACTGAAG
TGGGAATGGCGTGAAGAAGGTAGCATCCTCAGAAGGGTATCTCTGAGGGAATGTTTACATTTTTAGGCTGAATAGAAACCGGGCTCT
GGGATTTAGTCTACTTTCTTGCCTCCATGCTGCCCCCTCAACCCCCAAAGTATCCTTCTCATGTAAGATGGGTATTTCCTTAGTTAA
GCACTGCCCTTCGCATTGGCAGTCCACTGTAGTCACAGCGTCAGCCAGCTAAGCTGTGGGCCAGGGCTGGAGCTGCAGGGCTCCCGT
CTAGACCCCAGCAATTGGGGCATGACCAATTGTTTTGATGATGTTCCCAAGATCCATATAGCAGAAGGGTGACTCGCTCTTCAGATG
TCACACCCGGCTTTCCGGGGTGATTTAAGTGGAATTATTTCTGGACCCAGGAGAGCCTGGGGAGAACCCTCTCCCTCTGATGTCCAT
TTTTAATGCTAGGACACATCCTTCTTTCTGGATGTTCTATACAAAGGAGATGGATGGCGCTGGCTTTGCTTGGGGGTGGGGTATCCT
CCCTCCAGGACAGTTTCTTTTAAGCTATTTTTGCTTTTGACAGGCTGGTTAAATTGATTACAGCTATAGACTGAGCTGTCCAATTGC
CCATATCCTCTAAAAATTGGGCACCAAGTCTCCAGCAAGTCCCAGATGCCAAGAAAATGCAGCCATCCCCAAATGGACAGGGGAAAT
GAGGCCAGAAAAGAAAAGTTCACAGACTGGGCTCTGCCCTCGGATTAGGTGGCCGAATGGGCAGAGCGTCCTTCTGCTGTGTGTTAATG
GGATCCGTGTGGAAAGCTGGGGAGAGGCAGTGCCACTTTCCACATCAGCATCCCACACAGGATGGCCTTCATCACAGAGAAGGGGGT
GTGTGAAATACATTTGCCCCAAGTTGGCTATAGATTGTAGCATTTCTGAGAAATATTCTCCTCATTTCTCACATGTAGTATAAAACC
CAGGAAGCCAAAACTGTTGCTGGTAGATTCCTTCATCCCAGCATCTCAAACTGCTAATCTGGGCTCCATGCTGGGCTTTGTGGCATG
GGGACTGGTTGCTAGAATTCCTAAGTCCTCTACCTTTCTCCAAAGTTGGTGATCAGGATGGCACCATACTCAGAGAGCAGTGGTCTG
TGAGGAGAGGCCAGGGATGGCTTCCATGGGCCACTAGAGAACCAGGTGAAGGGATTCTGTCTCCCTCCCTCAGGACTCGCAGGCCAC
CAAGCAGCACCAAGGAGTGTTTCTAAACATAGGCGTACACTGCTTTCTTGACCTTGCTCCCTGCTCCGACAATGACAGTTGGGTGGA
TAGCTGAAGGACTAGGCAGGAAAGGCAGCCTGCTTTTCTGTGGCTCAGCTCAGGCCTGCTGCTTTGCTCCTCCTCTGTGTTCCCCTG
AGGGAGTTGGGAGATGGGAGCTACCTGAGCAGACTGAGAACATGATTCTGGAAGGGCTGAGAGTGTTCTACTGGGTCACTTAAATCC
TGGCTGTCGGCAGAAGTGTTGTCCCCTTTAAAAATGTACTTGAGGCTCCTTCTGAAAGCCTGTGCTGAGTCACAGCCTAGAGCCTCAA
GAAGAAAGACCCACACACAGGGCAGGCACAGCAGTCAGTGCTATCCAAGTATCCCCCTGGGAGATACTCATCCCAAAGATGGCCAT
ATACCCAAAGATGGCCATATTCATCTTTGGGGAAAAGTTATCTGCTCTTCATTTGGCTGTTCTCGGGACTCTCTCAACTGCCCGGTG
TGGGCTACACACTGCAGTCTGCACATGGGTTTTCCCCCTGGGCCTGTGGATGTGAGCTTGGGACGAATGAGACAAAGCAGACGTGGC
CTCCAGGTGCTGGACTGTCTGTCACTTGCCACACTGCAAGGGGCCTGATTACCCTAGGGTTTCCCTCCGGGAAGGCTCATTCTAGCC
ACACAGCACTGAGAACTTGAAGAATCAACTCCCACAGGTCAGCCAGGGTCACAAGGCAAGAAGCATTGCAGACCCCCTCTTTCATGG
GGCTATTGGGGCGCAAGCAGGTTTTGACAAGCTTTATGGCATTGCTTGTCTTGACCAATTAGAGACTCAAGGTCTTATACCATTCCCTTGAA
ATGCCAACACTAAGCCTGGGGCACACCAAGTCTTGTGTTTTACCTCCTAACTGTGTGTTTTCTCTCCTGGGACCCTCTGACGTCCTC
TGGCAGGGTGCAGGCTGCACTGCCCTTGTGGTAGCTGTGGTTGCCCGAAAGCTCGAACTCACCAAAGCAGAGAAGCATGTGCACAAC
TTCATGATGGACACTCAGCTCACCAAACGGGTAAGCCACCTCACACCCATGCCTTCATGCAGGTCCACAGCCGAAGTGGGAGCTGAT
CAGAAAAAGATCAGACACCGGGTGTGGCTTAGCAATGCTCCTTTGAGTGCAGGTTAGGAGCATGCAGCTCAGGCGCACAGCCCCTAC
TGATGCATACCCAATGCCCCTACTGAGTGCATGAATCTCAACAAACCAGGTGAGTCACCACATGCTTCCAGCAACCAGATAATTTT
CCTGATAATTAGATCAATAGCACACAGTGATGAAGCCGTCTCGCCTCCCTGCCTGATGGGGTCAAATCAACCACACAGGATGATGC
TATATTTTTGACTATGCTCAAATATTTTCATGATTCTCATTAAGGCCAGTAGAGAGCCTTAAAATTATACTGGAGTTGTACCTAAAT
CCAATCCCTTTAAAAAGTCATTTAAAGAGATTATTCCAGTAGAGCCCACATTCCCAAATATTGTTGCTCACCAAAACGCCACCATCC
GTTTTCCCCTTTTGGGCTGTCTGGATAATGGCTCTCACTTGCATCCTTATGAACTCAGCTTAGGAAGAGGATAAACTCAAAGTGCAG
GGATGGGGTGATTAGCTGCTGCTGCTCAGATTCCCCCACTGAATTGTCAGAGAGAAGGAAGCTCATTTCAGCCAGGCACAGTGATCCCAG
AACGCAAAAGGCTAAAGCAGGAGGATTGCCATAAAATGTGAAGCCAGCCTGGGCCATCTAGCCAACTCCTGTCTTTAAAAAAAAATTA
ATAAGCTCCTCTAAGAACAGAAAAGAATAAGGCTTCCAGGACAACAACAGGCCCAACAAACTAGGTAGGAGCTATTTCTTTAAGGAC
GGTTTTCAAGCGAGACTCTGGAAACAAATGGGAGCCTGAAGGAAGGTCAGATGTATGCCCTCCTGGGTGGCTGATCTTCTCTAGAGG
AAGAACCAGGACTCTGGGCGCTAACTGGGCATTGATCCCCAGAGAAACGCTTCAAGGTGACATGAATCTACCAGCAAACCCCACAGT
CTTAGTTGGGGATTTGAGTACTTAGAGAAACTCTTCTGTGATTAACGTTTCAAAGCCATAAAAAATGTGTTAGAAATAGAGACAGGCG
GGAAGACAGAACAAAAAGAGACTGGGAAGGAGCAGGCGGGCCCTCGGGGAAGCCAGCACAGAGTTTGCGGTTGCCAAGGCCTCTCAG
CGCCTCAGCAGAATTATTTATGGCGTTTGATTGTTGGGTCTTTGATTGGGGGTTTCTTTAAAGAGCTGCCACAGATATAAAAAAGGA
ACCTGTACAAGCAAATCTTGACATATCATTGGCATACAGACCACAAGGAGCATCATGGCCCCGAGCCACCAGCCAAACAAAAGAAAA
CAGAGCTCAGGCATGACAGTATGTGACACTGTCACCCTGCAGCAACCATACCAAATGCTAGGAGGACAGCCTCAGCCTCAGCCTCAG
CCTCAGCCTCAGCCTCAACCTCCTCTCTAAGCCAGCCTCAGCTCAGCTCAGCATGGAGGGGAAGGCTAGGGTATGGGCAGTCGAAAA
TGCAAGTGTCATCCGGAGTCTACTCAGCTCACTGCTTTGAAACCCCACGGACGAAGGGAGGAGGGGTTTGTCTCCCAGATCTTTTCTG
AGGCTGTTTGCCAAGAACAGAAACAGTAACATTCCCTGTTTGCTCCCGTGGCGTTTTAGAGCCAAGAGTATACTTTGTATTTTCTTT
CTATTCCTTTGTCCTACCCCTTTTCCACCCAGTAAATCAGATACAAGAGGAAAATAAAATGAGGAAAGCCCGCTCTCTCAGGGGCTA
GGGAAATTAGCACACACACACATACCCCGCACTATAATTGTAGAAGGAGATGAGGTTGTGGTTGTTAATTTCAATCTTTCAAAAA
TAATTCACTAAAATCCTCTTCAAGGCCACTTACTTTTGGAGGAATAGGGCTGAAAATCAAATCCATGGATATTTATTGCTTGCCTGT
```

```
GATGTGAGGTGCCTACTTAATTCAATAACATCTCACTTTTCTCCGAACACTCAAATCTGCTGAAATGTCCCGGTAATATCTTATATC
TTTTCCCAGGGAAATAAACACAAAAGGAATGATTCTCACAGAGGGAGATTGACAGCTGGCATGACAGAAATGTTACAAAGGACCTTC
TGGGCATCCTTGTCTGAACTGAAGCTGGCTCCGACCTCTGTGACCACTAGGGAAGCCTGTTCTTTGCCCTTTCCATATAGCCCATGC
ATCCAGTTAAGGTGACTCACAGGTCAAAGGCTGTTCCCAAGACTCCAGTGAATTCTCAGAGCAGGGTCCAGCGTGGAAGGAGTTGGT
AGTAGACGATTAGTGGCTTCCCCTGATGAAAGCAGATCCTTGCAAGAACAGGCAATATTAATCCCTTGAATATCTAAGCAGAAAGAA
AACATGAATTGTAGTCTGTGAATCACACAAATGGGAGTCATTCTGACATTAATTCATCCGGCCTGTCTAGCTTCCACCCAGTCCTTC
CAGGTAGCACCGGCTGTTGGCTTGTGTTTACTTGTGGAGAACGGAGGCAGCCTGGTAGCAATGGAAATGGACTCCTGACTTTCCCAA
ACTCCTGGCATGTCTCCTTTCACCTGAATAACTTGTCAACCTTGGTGGCAACGTCACTTCTGTACTTGGTGGGATCACAGCTTCCCA
CACTGACTTCGCAGTCACTGCCACTGTTAATCTATAATTATAACTTATTGCCAGCCCTGGACTACTCAATAATGTCTAAGGAAGTGA
CACATAGCAACGCTGTGCATGATCGAGAGATGACCCGTGTATCCCACCGACTCCCCACTCAGACCTGTGGTAGGCTCTCCAATCAAT
TTGATAAGTGTGTGCTGTTGGCTGGGGTGGCCTCTCTCTCCTTGCGAAGGGTTCCTCGACCCCATGATCTCATCGAACCACATTTC
CCATGAAGTCAGTGCATAAAGCCAAACCCAGCAGCCCTGAACTACTTTAGGGTCCTGACTCTCCAGGGCTGCTTACATACGTCACTG
TCCCTTCACAGTCCAGGCCCAAAGACAGTGCCAGTTCAAGCTAGTCTGTACCTAGCAGTGCTCAGGGCCCCGCCCCTCACTCAGTTG
TTTACATGTGATGATCTCTCCAAGGCTCCTCCTGCCCATCCTTAAAGCACCCTCCACCTGGGTTTTCTTGCCATAGACCTAATTAT
CCTGCATCTGCTCGTGGTCTTCCCAGTCATCCCTTTTCATCCCCCTAGACCTGATTCCACCCTGAACATACACATTCAAAAAACCTC
AGGCAGCCCGAGAACCTACTGAACAGTCCTAAATAAAACCTGAACTCTTAGAAAATGTCAAAGGCTTGAGGGAAAATGTTTCCAGCA
GGCTTATTCAAAGACTTAATCTTAGCAGGGGTGTTTTATAGGGGACATAATGGCAGTGGCTGTTATGCAGACATGGAAGGCTGGCTA
AGGACCTCTAGGTGGATGGCCCATGGCTGGCATGGCTCATGAGTAGGTCCCCAGAAGAAGCGTTTCATTAGGTAATCAAGCATGCCA
GGACTAGATATGCTAATTCTCCCAGAAAAGGATTGCAAATGACATTCCCCACAAAGGCTGTGACCCAGAGAGACTGGGCACAGTGTT
TCTTTCCCCCACAATAATAGAAAAGCATAGAAACAAACAGGAAAGATCCTGGGGATGAGCTGGTCCAAACCAGATATGAATACTCAA
GTCCAGAGAGGGGGAGGGGGTGGATGCCCAACACGTGCACCTAGTTGGTGCCTGTGGAGGATCTGGTTTGCACACAGGAAGCATTAG
CACCCCAGTCACTACCCAGCATGGTTAGTTCCTTGAGCTTTGCATGATGGTTAAGGTCCCAGGCCTGCAAGACGAAAGAGCCAGCTT
GACTCCAATAGCCCAGTGTTACATCAATAGCACGGTGAGTAACCTGCCATTCATCACCGAGTTGCTATGGATAGAGCTCAAACTCCA
ACCCTGAACCACAGACTACATCTCCTTTCTGCTCCTCTCCCCATTCCAAAGTCCTGACAAGAATGTTGATGAAGCAAGCCCCTTGCA
GGTGAGGCAGTGAGCGTGGGTTATGATGGAGGAAGGAAGAGGGGAGGCAAAGCAGGTCGATGAGAGAGGGTCTTAGGAAATTGTCCC
TGGAGGAAAATGAGGAGGAGAATGGCTGGCAGGAGGGGGAGGGCCACTGCTGTTTACAACAGACACCTCCAACCAAGCTCTGGAGACA
GGCTGCCTCCATTCACGGTCTCCCTCCATGATGTTCCTCATCCCTGCTCATTGGACTCCACCTATTGACTGTTGAGATGGTGACTCA
TGGCTGCCAGGCTGCTCTGGATTCCCTCTTTGCCCTCTCCAACCAAGAAGGACACTGCTCCCTCGGCGGAGAGGTGGCCAGAGCAGC
CTGTCGCATGGGATGCTGTGGTCAAGAATGCAGGGAACAGACACGTGTCCTTCTGCATACTTTCTTCTCTCGGGGAGGCTAGGGCCA
TGAGAAGAAGCTGACATGAATTGGAAAAGTGCTATCAATAACTTTTCGTAGGTCTACGAGATGGCTCGGTGGGAAAAGGCTCTTCCC
GCCAAGCCTGTTGACCTAAGTGTAATGAGCCCTGGGACCCACATGGAAGGAAGAAAGAATTGACTCTTGAACGGTATCCTCTAACTT
CTACCCATACACTGGGATGCATATACCATCCCATAATAAACAGATAAATGTAATGAGAAATATATGTTTACAAAAACAACACCAAAG
TAACTGTGCCTTGAAACACACCATGGGTGGTAAATAGAATGCTGAACAGTCTACCTGTGCTCTCATCTAATCAACACAGAAGCCCGT
CTCCAAGATGAATGACACTTGCAAGACCCTAACCCTCTGCCTGCTCTAATTTTCTTTGAAGCTCTTAGAATCTGATACTGTGGTTTT
TAACTTCGTACTTTCTCACCCAAAGATTACAAATTCTGTGACAGCAGCAATGGCATCGGTTCACCAAGAGTGGTAGCATGCACTGTA
ATCCCCAAAAGCTGCCACAGAAGGATTTTGTGTTTGAAGTCAATCTGGGCTAATAGTGAGTTCAAGGTTGGTCTAGCAACAGAGTG
AGACCAGGTTTTTTTGTTTTTTAAAGATAACATTATTGATTGTTGAACTCTGTCCTCTCCCTTTCTAGAACAGTGTCTGGCTCATCA
TAGGTCCTCGTTAGTATTTATTAACTATCCTTCAAATGGAGAGAGGACGAGAGTCTCAGGTAGCTCAGCAATAGATCCATGGAGGAT
CCACCCACTTACCACCTTTACCTGGAGGTTAATCCATGAAACGATGCAGACAGTCGTGCTCTGCAAAGCTTGGCACAGCAGTTAACT
CACAGTTATTTGGGAGATTTTCCTCAGACTTGGTTCTTCCCTAGTCCCACTAATGTGCAGAGTCTGTCCTTCCTAGCCAGGAAAGGT
AGTGTGCTTGTTTAATCCCAGTGCTCAGAAGGCAGAGGCAGGTGGATCTCTGTGAGTTTGAGGTCAACCTGGTCTACATATCAAGAT
CCAGAACTTCCATGATTCGTGTGGTAAGACTCTGTCTCAAAACAAAACGCTCATTGTATATAGTATAAGGTATATGGTATATGGCA
TATGGTATAAGGTATAAAGTATTTAATATATGGTATATAGTATGTAGTATATGGCATGTGATATATGGCATGTGGTATATAGAATAT
ACAACTTAAAGTGAAAAGAGCAATGTGGGGATTAGAGAGTTTATAGACTCAGTGAACACCATCAGCCTCAGTTCTTATGCATCTGGA
AGCCAGAGAACTAACCATTCACACATAAAAGATCCCACCCAGCTATCTTCAGTGGCACCTCAACCCAACCAGTCAGAGCCAACCTTG
AATGGTCCTTGCCAGTTCGCTGCTTCCCTCTCAAGCCCTCGACCCTCCCAACCCTGCATCCTGGGAACATCTTTTAGGTGTGGTATCT
TTGTTCATCAGGAGAGCTCAGAAGGTAGCTAAGCCTTCATCAGGGAAGGGTGTCTCACACTGTGGAGGCTGAGCAGGCCCCAAGAGG
GGCTGAAATGGTGCTGTCTTTTCAACCATGCTCAGAGCCTGGCACCAGGTGAGATGGAGGGAAACTCAAGAGAGCCAAGTGCAGTTA
AAAGTCTATTGCTTTTTCTGTTGCAGATATCGATCAACTTAGCTTTGTGCCAGGCCGTTTGCTAGCAATTAATTTCAGTCAGTGCAA
GGGCTTGAGTTTACCATTATTTTCCCGAATGCTCAGGGGCCTCCCTCTCAAACCCATAAAGATGATGTGATTCCGCTCTGCTCCCTG
GCCAAACCATGACTTCAGGATCTTTCATTTAGGCTGGCTGAAGGCAGGTCTGTATTCTGTCATGTTGCTGAATGAGGGC
CAGATGCCTTAAAAAAGAAAGACAAAAACCAAACCAATGTCCTTCTCAATTCCTTGATCCTGGGAAGAGGAAATGATTCTGTTTGT
TCAGATGTGATCCAGACCTGTCTTCCTTCAGACCATGTGGGAATTGTTTGCACCTCTTGGACCTGACTCTTGCTTAGACTTAGCCAT
TAGCTCTCTGTATGAGTCTGGCCTTTTATCCTCTTTCTGAAACTGTTTTCTTCTATTTTAAGTGGGGATAATAATAACCAATATCTG
TCTGATACTTTAAAATTTATATAGTGCTCAAATCCACACTGACTGTTTGCTATTGGCAAGCTGATGGGCTGAGGCTATGAAAAACCA
AAATGATGTTAGCCCGACATGATAGTGGGTTTGTGAGGCTGTCAGCCAGACCTGGATGGGGGAGCCTTGCTAAAACCAGCTATTCCC
TCCACACATCAGCTGTGCCTCACAAATGTGTGTGTCTATGTATGTGCCCAAGCATGTACATGTGTGTATGTATGTGTACCCAAGCTT
GTGCCCATGTATGCATTTGTGTGCATGTGTGCATGCCTGAGCATGTATGTATGTATGTATGTATGTATGTATGTATGTATGTATGTA
TTCGTATGTGTACCTGAGTGTGTGTGCCTGAGTGTATGTGCCTTAGTGTGTGCATGTGTGTGTGTCTGAGCATGTATTTGTGTTTAC
ATGTATGTGTGTACATGAGCTTGTATGCCTGTGTGCATGTGTTTGTGTACCTGAGCATGTGTGTATAGTGTGTGTGCATATATGCAC
GTGAATATGCCTGTGCCTGCATATGTGTGTGCATGTTTGTGTGCATGCCTAAAATGTCTTCCATTCACCATTAAAGATAATTAA
GCATTTGCTTCTAAACTGCTTAATGATGCATACAGCCTATAGGCTTCCTCATTTCAAATGTTTGTTAGCAAGACACAGAGAGCATTG
TCAAAGGTTTGTCAGTTTGGCTATTCTGTGGCACTATCCACACTCCTAAGAAGGGACTGTATAGCTAGGGCTCTACCCGTCCTTGAA
ACCACTTGGGCAGACTTTGCCCTCTCCTGCTTGTCTTTAAGGCTTCCCTTGAAGGCCGAGCAGCCACAGAAACTGAAGAGGTCCTAC
ATCAAATTGCCCACCCCCTTAAGGGACAACAGCAGCATTCTGCTGAGAGAAAGCCCTCCCTTCCCCACCACCTACTTAGCTAACCAG
ATCCAAGGCCCAGAGCCCATCATCTGCTTGCCCTCCCTTGGTACGCCAGACACTGGCTACCTCCAGCCTCGTCTTACTGCCACCTGCT
CCCTGTCAAACAAGAAAGTGGCAGCACAGGTGGCCAATGTAACCACTTAAAACCATTTCACAGTAGATTTTTTCCTGAACAACAACAAAAA
AGTAATATTTATGTCACTTAGACATTTCTGAAAAGACTTAAGATGTTCTTTTAAAAACATCACATCGACGCCAATGAAGAATACAGC
CCAGCTTTAGTAATAGATTTTGAAATACATACAAGTTATCAGTCTGCAAAGCAGTGTTTCTTAGGCCTTCCCACGTGTCTCAGCATG
TTAGCAACGCCCCCATCTGTTGGTGTCAGTCTCAGAAGCTGATTTGTAGTCTCTGATTTTATTAAAATCCTATCTGGCAAGTCACTT
GATCTCTTCTAGTCTGGTCTCCTCACTTCCATGCCCTGGGCCTGGGGTCCTGTGCCAGTGTGTATAGCTGACAGAAATGGAGACCAT
TAATCAGAAATACATTTAGCTTGTGCTTAATAAGGAGTATTTTCTCTTGTTCTCGCTAGTAATTCTGAAGATGTCTGTGTTTGGAT
TAAAACTTTGTCCCCTGCCATCTGGATGCCATTGATACGTCTCAGCTCAGTCATGAGCTGGATGATATCAGTGGTTTCCATGTCAAC
AGATGACAAGAGTTGTGTATGGAGCCGTGTGACTACCGTGCCTGGGGAAGCCAGCCCCGGGAAGGAGGCAGCTGTGCTGTCACACCT
AAGCATCTTCCCTGATCTCAGACACCACTGAGATGATTTCAGTTTTGTGCCAATTACATTTACATGTGAAATCGCAACGCATAATTT
TTGGCCAGTTAGGAAAGGAACAGTGGTCTTGTTGGTACCTGTCATTGCAGTTTGTAAGAAAGACACTTTCTCAAAGACACCTTTTAA
```

```
AATCTCCACTTGGTAGGAAACCTATTACTTTCAAGTAAAGTGTGGTACCCTGATTATGAGGGCATTGACAGCATGTATTTATTGATC
CAAACTGTACCCCAGACTTCAAGAGCTTGCTAAGCAGCCTGTCTAAATATAGCATCATCAGCGGCAGCCGAGAGGCAGCAATGAAAC
CATTAAGAAGGAGGAGAAGTGCCTCTCGGCTCGAAGCTCCAGTCTAGGCCTCAGATACAAGATTAATAAAAAAATGTGTTTTAATCAT
CTTTTCTAAGAGTTTCCTGATCTTGATTAAGAAAGTACATCTCTGAAGAACCATTTTCTCAGGAAGTAGATGGTAGCCACACATGCA
AGGAAAAAACATATTGTGTGAACCCAAAAATTAACCCAACAGCTGCTTGTTACCTTTCCCTCAGATCAAGAATGCCGCCGCCAATGT
CCTCCGGGAAACGTGGCTAATCTATAAACATACAAAGCTGCTAAAGAAGATTGACCATGCCAAAGTCAGGAAACACCAGAGGAAGTT
CCTCCAAGCTATCCACCAGTGAGTATCGGAGAACCAGAGCAGTGTACTGTTTGGTCAAANNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNTCTCTTCTTGAAAATCAGCACCGTGGGCAGAACTAAGCGTGGG
TAGTCTGACCAGGAGGGCTCCCTGCCCCCCACTACCTTCTGATAGAAATTATACAGCAAATGCATAGCCCAGGGCTGGGCTGGCAGA
ACATTCCAGCAGATGTCAATTGTAACGGAACCTTATTCCTGGGAAAGCAAAGGAAACTCCCTCGGGCCAATTAGGCCCCCTTTGAA
AAGAGCCTGCAAGCAGAAATTATTGGTTGCTGGGCCACGTGCAGGCTCCTCATTCAATAGAGGGATCAAGAAGGCAAAGAAAGAACT
TCCTATGGCAGTGAGGACCTCTGTCACAGCTAGGGAGACGCCCACAGACACATGTGCCATTGGAAGGGGCATTCAGGTGGAGTGTGG
GGTTTCCCAGAACCCAGTGTGGGAGTGGATGAATGGACCTCTGGAGTCTTAGGCATGTTGGTTAATACACAGAGTTCCAGCAAACCT
ACTGGAAGTACTCCCCATATGTTAGAACCACTTATTCCCAGGAGATAAGGGGGTGTCTGGAGACACCTATGCGCCACCTCCCCTCTC
CTGCTCAGGTGCTGTTCCCCCACGCCAACCTTCACCTCAACCCCCACCGCACCCCAGGTCACCTTGGGGCATATTTGACTTTCATTT
GTTCTTAATTCTAAATTCTTGAGAGAGGTGATTTGATTGGCTGGTTTTACTGACGTGTCTGTCTTTAGACAACTCAGCTATGACTAG
GAAGAAGGAAACAGCTAGGACAGGACTCTGTGAGCCTCAGCAGTGGCTCAGTGAGATGCCCCAGGCAATGTTTCCCATCTCACCCAC
AGGGCACACTGTGGCTGATGCTCCTCCAGCAATATTAGGTTAATCTAGGTTTTGAGTTCATCTTCCTGCAGGGCTGCCCACAGTTTC
TCATTGACGGAGTGAAAGCAATGGCTTGGTAGACTTGGAACACTTGCTGGCAGAGAAAGAGGATCTAAATTTGAATGCCCAACACTC
AGAGCTGCATGTGCCTTCAGCACTGGTGGGCAGAAAAAAAAATGAAGTTTGATGGCCAGTGAGCTTGGCCAAGACAGCAGTTCCTA
GTTCAGTGAGAGGCCCTGTCTCAAAGGAATAAGACAGAGAATAATAGAGAAAGAGACTCCATATCCTCTGGGCTTTCACATGCTTGT
ACCTATGAACTGGCACCCACAGATTCACATGAAATACACACACAGACACACACACACACACACACACACACACAGAGAGAGAGAGAG
AGAGAGAGAGAGAGAGAGAGAGACTCCTGCACTGGACTGGGAAATAAAGCCATATATCACAGAGGAATGAAGTCTGTGGGAAGAGAG
TGTGCACAGAGCCATTGTAAAGTCCATGCGTGAAACCGGAGGAGGAGGCAGACCCTGCTAACCATCCATTTAGTCCTCCCATGAGAC
CCAGATTCCAGTGGCAGTCACGTGTGCTTCGGTGTAGGTCCCCGCTCCCTTAGCATCCTGTTAGCATTGCGAAGGGAAAGCATCCAT
CGTAATGCTATGCTGGCTCCTTTTCCCGCCGTGCTTTGCCTCCCATCTCCCCGCCCCCTCGCCCTCTGTTCTTTGCTCCTCCCTAGATT
AAGAATGTACCCGTCCACTGCATGTTCTGTATTAGACAGGCCTCATGTCATGTATGCTGTCAGAGCTGGGAGGGCTGGGGCCCCTCA
CCCAATTCCTTCAGTTTCTTCAACTGGGAAATAAAAACAGTTCCCACCATCTGAAACTTGGGTGGTCAGATAAGAACTGTAAACCTG
CTTTGTGAGCTGTAAACGTTTCAAAAATGGCAGTTCTGATTAGGAACACAGATAAGGAAGGAACCAGAACCTAAAGGAACAAGATGG
CCACCACCTGGTTCCACACTGGAGTTCACCAGGTGCTCTTGTGGCCACACATGGGATCCGTGGCTTGTCCATTCCGGAGGGACCATG
CCCTGTGAGGAGGCACTTGACCACCCCCACCCCCATCCAGGTTACCCTTTCCCTGGGAGGGCAGAGCTGACAAGAGAAGAGCTGTCT
TCAGACCTGCTCCTCCAAAAATTCCAACTGCAGGGGGCTCTCCTGCAGTTTCACCATGGCTCCTCTGCTGCTGCCTCACGTTCTGTTC
TCCTCCCTTGTCCTCTGTTCCTCTCTCATCCTGTTGCCTGCTCCTTCCACACATCCTAGAGCAGTGGTTCTCAGCCTTCCTAATGCG
GTGAGCCCTGAATACAGTTGCTCATTTGAGGTAACCCTAACCACAGAATGATTTCATCTCAGCCTCATAACTGTAATTTTGTTCTTG
TTATGTATGAATCGTCATGCAGGATATCTGATATGCCAGCCCCAAAGGGGTCTTGACCCATAAGTTGAGAACTGCTGTCCTAGAGAA
ATGCCAGGTCAGATGAGATTAGGGTAATTTCATACACACACACACCACCACACCACCACACACACCACCACCACACACCACCACACA
CCACACCACATGGGCACGGGCATCAGAACCCCCTTTCCTCTCTCTGGGTGTGAGACTTGAGAGCCAGCATTCCCGGTGCTTGTTGAT
TGTCTGCCGTGTATAGTCTAGGCTTCTGCAATCAGCATAGGAATCACGGGGTGTCTGTCAGGGAGCTCTGATAACAAGTGGGACCC
AAGAGCAGTGTGTGACAGTGTTGGAGACAGGAGGCCATCAGGAGCACCCTCAATGCAGCCTTTGCCAGGCAGCATGGGAGGCGGGCC
TGCTCTGAGGAGTCAAGTGTCGTCTGCCCAACCAAAACCTCAGCCAGGTCAGACAGCCCTTAAAAGAAGCAACCTGCTCCAGGGCTT
CTCTACCTCCTCTGGAGCCAAGGAGCAGACGGGTGGCTCGTCAGGGATGCAATCTCATAACTATGTGTAGTTATTAACTCTGATTTTATAA
GGAGGTCAGGGTAGGAGAGGTTAAGTGGCCTACTAGTGAGCTGAACTTGCACTTGAACCTTGACTTGTTAATTAGGAGTCTACAGCC
TTGGCCATTCTCACCTCAGTGTCTCCTCTTGAATCATTAGACGGCAGCACTCTGTGTGAATTGACAACCATGTAGAGCCATATTCAA
TTTAAGAAAGGGACGTGTTTCTTTTCTTCCATGCATATTTTTTATTTAAAATAGATAACTAAATTGGGCGTGGTGATGTATATCCTT
AGTCCCCACCCAGCCTGTCCATCACCTGGGAAGTCCTGGAATTCTCCTTTGGTGACACGGGAGGAAGCTGGGCGGAGGTGGCTC
CAGCTGCAGGGTGGAGGGCATTTCATCTTCCCACAACAGAGTCCTCTGTGGTGATGGTCTTCTTCCTGCCTCCCCCTCTGTCTCTCC
```

```
CAGATGCAGAACGTCATGTATGACTTAATCACGGAGCTCAATGACCGGAGTGAAGATCTAGAAAAGCAGATTGGCAGCCTGGAGTCC
AAGCTGGAGCACCTCACAGCCAGTTTCAATTCCCTGCCCCTGCTCATCGCAGATACCCTGCGCCAGCAGCAGCAGCAGCTGCTCACT
GCCTTCGTGGAGGCCCGGGGCATCAGTGTGGCCGTGGGAACTAGCCACGCCCCTCCCTCTGACAGCCCTATCGGGATCAGCTCCACC
TCTTTCCCAACCCCATACACAAGTTCAAGCAGTTGCTAAATAAAACTCCCCACTCCAGAAGCATTACCCATAGGTCTTAAGATGCAA
ATCAACTCTCTCCTGGTCGCTTTGCTGTCAAGGAAGTTGCAGACCAGGGAACAGAACGAAGAGAGA
```

MOUSE mRNA SEQUENCE : mR27-009.1 (Seq ID No: 24)
```
GCTTTGGGGTGGAGGGTCGTGCTGCAGGGAACTCAGCCAGGCCCCAAGATGGACACTTCTGGGCACTTCCATGACTCGGGGGTGGGG
GACCTGGATGAAGACCCCAAGTGTCCCTGTCCATCTTCTGGGGACGAGCAACAGCAGCAGCAGCAACCGCCACCACCGCCAGCGCCA
CCAGCAGTCCCCCAGCAGCCTCCGGGACCCTTGCTGCAGCCTCAGCCTCCGCAGCCTCAGCAGCAACAGTCGCAGCAGCAGCAGCAG
CAGCAGTCGCAGCAGCAGCAGCAGCAGGCTCCACTGCACCCCCTGCCTCAGCTTGCCCAACTCCAGAGCCAGCTTGTCCATCCTGGT
CTGTTGCACTCTTCTCCCACGGCGTTCAGGGCCCCCACTTCAGCCAACTCTACCGCCATCCTCACCCTTCCTCCAGGCAAGGCAGC
CAGCTAAATCTCAATGACCACTTGCTTGGCCACTCTCCAAGTTCCACAGCCACAAGTGGGCCCGGTGGAGGCAGCCGGCACCGGCAG
GCCAGCCCCCTGGTGCACCGGCGGGACAGCAATCCCTTCACGGAGATAGCTATGAGCTCCTGCAAATACAGCGGTGGGGTCATGAAG
CCCCTCAGCCGCCTCAGCGCCTCTCGGAGAAACCTTATCGAGGCTGAGCCTGAGGGCCAACCCCTCCAGCTCTTCAGTCCCAGCAAC
CCCCCAGAGATTATCATCTCCTCCAGGGAGGATAACCATGCCCACCAGACTCTGCTCCATCACCCCAACGCTACCCACAACCACCAG
CATGCCGGCACCACGGCCGGCCAGCCACCCTTCCCCAAAGCCAACAAGCGGGAAAAACCAAAACATTGGCTATAAGCTGGGACACAGG
AGGGCCCTGTTTGAAAAGAGAAAGCGACTGAGTGACTATGCTCTGATTTTTGGGATGTTTGGAATTGTTGTTATGGTGATAGAGACC
GAACTGTCTTGGGGGTTTGTACTCAAAGGATTCCATGTTTTCGTTGGCCCTGAAATGCCTTATCAGTTTATCCACCGTCATCCTGCTT
GGTTTGATCATCGCCTATCACACAAGGGAAGTACAGCTCTTTGTGATCGACAATGGTGCAGATGACTGGCGGATAGCCATGACCTAC
GAGCGTATCCTCTACATCAGCCTGGAGATGCTGGTGTGCGCCATCCACCCCATCCCTGGAGAGTACAAGTTCTTCTGGACTGCACGC
CTGGCCTTCTCCTATACCCCATCTCGGGCAGAGGCTGACGTGGACATTATTCTGTCCATCCCCATGTTCCTGCGCCTGTACCTGATC
GCCCGGGTCATGCTGCTCCATAGCAAACTCTTCACTGATGCCTCCTCCCGAAGCATCGGGGCCCTCAACAAGATCAACTTCAACACC
CGATTCGTCATGAAGACGCTCATGACCATCTGCCCTGGCACGGTGCTGCTGGTGTTCAGCATCTCTCTGTGGATCATCGCTGCCTGG
ACTGTGCGAGTCTGTGAAAGGTACCATGATCAGCAGGATGTAACTAGTAACTTTCTGGGTGCCATGTGGCTCATCTCCATCACGTTC
CTTTCCATTGGCTATGGGGACATGGTGCCCCACACATACTGTGGGAAAGGTGTCTGTCTTCTCACCGGCATCATGGGTGCAGGCTGC
ACTGCCCTTGTGGTAGCTGTGGTTGCCCGAAAGCTCGAACTCACCAAAGCAGAGAAGCATGTGCACAACTTCATGATGGACACTCAG
CTCACCAAACGGATCAAGAATGCCGCCGCCAATGTCCTCCGGGAAACGTGGCTAATCTATAAACATACAAAGCTGCTAAAGAAGATT
GACCATGCCAAAATGCAGAACGTCATGTATGACTTAATCACGGAGCTCAATGACCGGAGTGAAGATCTAGAAAAGCAGATTGGCAGC
CTGGAGTCCAAGCTGGAGCACCTCACAGCCAGTTTCAATTCCCTGCCCCTGCTCATCGCAGATACCCTGCGCCAGCAGCAGCAGCAG
CTGCTCACTGCCTTCGTGGAGGCCCGGGGCATCAGTGTGGCCGTGGGAACTAGCCACGCCCCTCCCTCTGACAGCCCTATCGGGATC
AGCTCCACCTCTTTCCCAACCCCATACACAAGTTCAAGCAGTTGCTAAATAAAACTCCCCACTCCAGAAGCATTACCCATAGGTCTT
AAGATGCAAATCAACTCTCTCCTGGTCGCTTTGCTGTCAAGGAAGTTGCAGACCAGGGAACAGAACGAAGAGAGA
```

MOUSE PROTEIN SEQUENCE : mP27-009.1 (Seq ID No: 25)
```
MDTSGHFHDSGVGDLDEDPKCPCPSSGDEQQQQQQPPPPPAPPAVPQQPPGPLLQPQPPQPQQQQSQQQQQQQSQQQQQQAPLHPLP
QLAQLQSQLVHPGLLHSSPTAFRAPTSANSTAILHPSSRQGSQLNLNDHLLGHSPSSTATSGPGGGSRHRQASPLVHRRDSNPFTEI
AMSSCKYSGGVMKPLSRLSASRRNLIEAEPEGQPLQLFSPSNPPEIIISSREDNHAHQTLLHHPNATHNHQHAGTTAGSTTFPKANK
RKNQNIGYKLGHRRALFEKRKRLSDYALIFGMFGIVVMVIETELSWGLYSKDSMFSLALKCLISLSTVILLGLIIAYHTREVQLFVI
DNGADDWRIAMTYERILYISLEMLVCAIHPIPGEYKFFWTARLAFSYTPSRAEADVDIILSIPMFLRLYLIARVMLLHSKLFTDASS
RSIGALNKINFNTRFVMKTLMTICPGTVLLVFSISLWIIAAWTVRVCERYHDQQDVTSNFLGAMWLISITFLSIGYGDMVPHTYCGK
GVCLLTGIMGAGCTALVVAVVARKLELTKAEKHVHNFMMDTQLTKRIKNAAANVLRETWLIYKHTKLLKKIDHAKMQNVMYDLITEL
NDRSEDLEKQIGSLESKLEHLTASFNSLPLLIADTLRQQQQQLLTAFVEARGISVAVGTSHAPPSDSPIGISSTSFPTPYTSSSSC*
```

MOUSE PANTHER CLASSIFICATIONS
       FAMILY (SUBFAMILY)
              CF10153 (POTASSIUM INTERMEDIATE/SMALL CONDUCTANCE CALCIUM-ACTIVATED CHANNEL)
       BIOLOGICAL PROCESS
              Transport (2.15.00.00.00) > Ion transport (2.15.01.00.00) > Cation transport (2.15.01.01.00)
              Neuronal activities (2.18.00.00.00) > Synaptic transmission (2.18.01.00.00)
       MOLECULAR FUNCTIONS
              Ion channel (1.03.00.00.00) > Voltage-gated ion channel (1.03.03.00.00) > Voltage-gated potassium channel (1.03.03.03.00)

MOUSE GENE ONTOLOGY
       No Gene Ontology
MOUSE PROTEIN DOMAINS (INTERPRO SIGNATURES)
              IPR003931 (SKCHANNEL)
              IPR003931 (SKCHANNEL)
              IPR004178 (CaMBD)
              IPR003931 (SK channel)
              IPR000694 (PRO RICH)
              NULL (GLN RICH)
              IPR001622 (CHANNEL PORE K)
              IPR000694 (PRO RICH)
              NULL (GLN RICH)
              IPR001622 (CHANNEL PORE K)

HUMAN GENOMIC SEQUENCE : hD27-009 (Seq ID No: 26)
```
CTTACAATGTAACAGTGGCAGGAGGAGGCGAGCGAAGCTATTGAGCCAGCGAGGAGTGAAGCTGAGCCTGGCCTCACACGCTCCTAG
AGGACCACCTCCTGAGAGAGTTCTTTCACCCCCTCTTCTTTCTCCAAGCTCCCCTCCTGCTCTCCCTCCCTGCCCAATACAATGCAT
TCTTGAGTGGCAGCGTCTGGACTCCAGGCAGCCCCAGAGAACCGAAGCAAGCCAAAGAGAGGACTGGAGCCAAGATACTGGTGGGGG
AGATTGGATGCCTGGCTTTCTTTGAGGACATCTTTGGAGCGAGGGTGGCTTTGGGGTGGGGGCTTGTGCTGCAGGGAATACAGCCAG
```

```
GCCCCAAGATGGACACTTCTGGGGCACTTCCATGACTCGGGGGTGGGGGACTTGGATGAAGACCCCAAGTGCCCCTGTCCATCCTCTG
GGGGATGAGCAGCAGCAGCAGCAGCAGCAGCAACAGCAGCAGCAGCCACCACCGCCAGCGCCACCAGCAGCCCCCCAGCAGCCCCTGG
GACCCTCGCTGCAGCCTCAGCCTCCGCAGCTTCAGCAGCAGCAGCAGCAGCAGCAGCAGCAGCAGCAGCAGCAGCAGCAGCAGCAGC
CACCGCATCCCCTGTCTCAGCTCGCCCAACTCCAGAGCCAGCCCGTCCACCCTGGCCTGCTGCACTCCTCTCCCACCGCTTTCAGGG
CCCCCCCTTCGTCCAACTCCACCGCCATCCTCCACCCTTCCTCCAGGCAAGGCAGCCAGCTCAATCTCAATGACCACTTGCTTGGCC
ACTCTCCAAGTTCCACAGCTACAAGTGGGCCTGGCGGAGGCAGCCGGCACCGACAGGCCAGCCCCCTGGTGCACCGGCGGGACAGCA
ACCCCTTCACGGAGATCGCCATGAGCTCCTGCAAGTATAGCGGTGGGGTCATGAAGCCCCTCAGCCGCCTCAGCGCCTCCCGGAGGA
ACCTCATCGAGGCCGAGACTGAGGGCCAACCCCTCCAGCTTTTCAGCCCTAGCAACCCCCCCGGAGATCGTCATCTCCTCCCGGGAGG
ACAACCATGCCCACCAGACCCTGCTCCATCACCCTAATGCCACCCACAACCACCAGCATGCCGGCACCACCGCCAGCAGCACCACCT
TCCCCAAAGCCAACAAGCGGAAAAACCAAAACATTGGCTATAAGCTGGGACACAGGAGGGCCCTGTTTGAAAAGAGAAAGCGACTGA
GTGACTATGCTCTGATTTTTGGGGATGTTTGGAATTGTTGTTATGGTGATAGAGACCGAGCTCTCTTGGGGTTTGTACTCAAAGGTAG
GGGCTGTGGTTTCTCTTTATACCTTGAACAAAAGGAATATGTAGGTAGCAAGAGAGGGATTGAGAGAGGGGGATTGTGAGAGAGAGA
GATTGAGAGAGAGAGAGAGAGAGAGAGAGAGAGAGAGAGATTGAGAGATTGGGAGGGGAGACTGGGAGAGAGAGGTGGTGGTGGTGGT
GAGAGGCGCTTGCTCAGTTATATTCAACACTCTAACTTCCCGTGGTTTTCCTTCTTGATCCCGGGAGAATTCATTCCTAGCTTCCTC
TGGGGGGTAGGGGACATCCCCACACACTGTGTCTAATTTGCAGACTCTCTGTTAGGGAGGATTCAATAATCCCTTCTGAGAAATCGC
TTTTCCTCCTGGCTCACTCGTACTAAAGCATAACCCAGCAGCTTAACGCACCAATTAATTACACTCCGCCTTCATGTGTTTGCCAGT
TCCTGCGACTTCCCCTTCTTTCCTCCCCTCCCTGCTCCACTCCATTTTCCTGAGGATAAAAAAGAAATAAATGTCTGGGTGGGGGGT
GGGGATTCCGTACCATTGCCCAAGTCCCTTCTCTCCCTGTCTTCTCCATCTCTTCCCAAGTTTCTGTTTCCCTCCTCTTCTCAGAGA
GTCTCGGGCAAAATAGAAAACCATCCATGACCTGGGTCTCCTGAGGGCATGGGGTAAAAATATAGGTGGAGGAAATCAGTGGTCCTT
TCCCTCAGGAGAGAGGACTCAGAATAAACCTGCTGCTGCTTCGTAAACATCTCCTGATAAAAATGGCTACAGGTGTTACCTGGGCAG
AGCAGCTGGGCGCCGCCTTGGAATCAGCTGGGGGAGCACCTCTCCTTGGGAGGCGGTGACAGGGCTTCCCAGGCGGTCCCCACAAGC
CCGCGCCCCAGCTCAGCCCCCAGTTCTCCCCTCCCACCTCAACTCCTCCTTGGGATAAATAAAGATGAGTGTGTGTGTGAGTGCGCGC
CCGGATGGAGAACAGCAGGCACTGGCTTTAGCGGGGAGCTGGCCCCACTGCTCCAGCCTCTCAGTCCAGCCCCAAGACGGAGGAGGG
GGTTTCCCTCCCAGAGGGAGTGGAGATGGAGGAAGGTGGGTTTCTGCCGATGCTCTGCTTGCCGGGGAGCTCTCCTGCTGTGGGCTG
GAAGGCTTGGAGACCTGGGAATCGGTGGACAAGTGGCTCCACGATACCCCCACCCCCAATTTGTTGGTGTGTGTGTGTATGTGCG
TAATGTTTTGTACGTATGCATGCGCCTGCTTGTTGCTTGGGTTTTTCTATTTATTTGTCATTTGGGGCAGGGGGTGGCTTGGTGGGGG
GGTGGCTGGGCGGGCTGGCCATCGGGTAGCTGGGAGGGGTTCAGCTGTTGGAGGTTTCAAATAGAGCTTGTTTCAGGCAGGGCACAG
CTGCTGCGAGACTGTCACATTCCTTATTGAAATCCAGCCGCAAAAACCTAGGAGGTGCTGATCGGATTTCCCCCATTCCCAGCATCT
CACTTTAATCGGTGATGGGCAGACTTTCCCGCCCTGGAAACGGGTGGGGCAGACCTGTCAGACGACCTGAATAGCCAGGACCAAGAC
TTCCAGGCAGAGGGTCCTCCAGCAACCCGGCTGCTCTCTCAGTGGGCTGCCCCAGCGGAGGGCAGCACCTGGAAGCCGCCTTAGTCA
AAATAGCTCAAGGATGAGCGTCTCAGCGAGCAGAAGCGAGTGGGGTTTCCCTTGGAAGCGCAGCTCACTTAGTCATTCCCTGTAATG
AGCCTCTGCGTCTGCGGGTCCTTCTGCTCGCTGCCTTCTCAGAACCGCCTCTCCTGCTGGGCACTCCAGGATATGGGGCTGGCAGCC
TCGCTAGGACTGGCAGCTGCCCTGGTCACAGCCCTGGCTCTCTCCAGTCCTCCCTTGGAGACCAGGGGCCCTCACTGGGGAAAACTG
ATGAAGGCCCAAATGCAAGGGGGGTGTGGGGAAGGGGCAATCCCAGCACCTTCTCTTTCAGTGACACCCGTAGTCAGAGTTCTTAAG
CACTCTGCAGGATCAGGCCCTCTTCCCTGCCTGGGGACGGTGCCACCCCTGAGGGGGAGCTCAGGGAAAGGGGCTTTCCCAGAGATG
CGGAAGGCAGATTTTAGGTGAATGAAAGGGGAGGCCACCTGAGGTGCTTGGCTCCCCGCATTTCTGAATCACAGACCCTCATAGCAC
CAGTGGCGTGCACCGAGGGGGGCAGTAGCTGAGAGGTCCTTCTTGCCTCAGCGGCCACTTCCTGTGTGCCTTGGGGCTGGTCTTGAGA
TAAGCAGAGCTCATTTAACTTGCTCACTCATTCACCCATCCCTGCACTGTCCATCCATGTTTTGAGTCATTTATGAAACAAGTATTT
TCTGGGTGTCTCCTATGTACCAGGCCTTGTACACACTGCAGGGCCAAGATAGATATCCCTCTCTCCTGGAGGCAAATGGGAACATTT
CAAGGAACTGGGCCAGAGTTGGAATCCCAGCTCTGCCACTACTATTTGTGTGATCTTGGATAAGTCATGGAGCTTTAGCGAGCCTGT
TTCCCCAGCTGTCGAGTAGGGAGAATTAAATTAGAGACGTGTAAGGCACGTATCTGCCTGCTCAACAAATGGGACCTATTGTGATTG
TTACTGATGATGACAATGATGAGAGGAAAGGAGATATCTTTTTCCTGTTATCTAACTCTGGCTGTAAAATTAGTTGGAGCTTTAACA
GATTGGGATCTAAGACTTGTCAACGGAGTGAGAAGTTGTATCTGGGAGGGAAGAAGTCCTGAGAAGGGACTGGGTCTGACTAGAGAC
CACCTTTAAGCATGGTTTATTCTTGGGGGGGAGGTGAATATCTGTGTCCCAGTTTCATGGAGGAAGTGGTCTCTACTCCTCCATGGTC
TGGGTTAGTCACAGCCCAGGAGAGCTCAGGGACATTCAGTGACCCTGGGGAGGGATGGGTGACACTCTTTTGCTGGAGAGCTTTGT
GTGCCTGGTTGGCCCTGCTGACCAGTCTGGAGGCAGGCGGATGGCACAAGGCAACCTCAGGGGTACCAGGCAGATTAGGTAATTCTGC
CCATCCAGTGGCAGCTCCCACTCAAAGGCCCTTGGGAGGTGACAAACTTTTTTTTTTCCCCAGGTGATTGAAGATTAACTAACCTTGT
GCCTGAGCTGCTGCTTTCTGAAGTTCTCTGCTTTTTTTTCCGTGGGGTTGTGGCCTTGCTGTTCACCTATGTTTGGCGGGTGGCTGC
TGCTGGGGAAGTAACTTGTCATGCAGGCCTGGGTCTTTTGAAAAGGAGGGTGGCATGTGCCCTGAGCATGTTCAGTTAATGCAAATG
GGAGCCAATGTGAGGGGGTTGAGCAACCGGGACAGAACATCCCCAGCTCCTGCCTCACAGGCCTGTAGGTGCTGATGATGGAAGTAA
GCTCTGCTAGGAGGCATCAGCCTGGAGCAGGGAGGTGGGGCTTGATTCCTGGGTGAGCTCAGATAAGAGGAGAGCTTCTCATTATTA
TGCCTGGGACATGAGATCAAACGGCAGAGATGCCTGAGATGGCCCCAGTGGGCTCCTAAAGGTACCAGGTGACCCTTGAACCAAATG
AGGAGTCCTTTTTTTTTTTTTAATAGGCAGGTTTACTCTCTGAAGTCTTAGACATTGTGACGAGGCCTGGTCTAGAGATTCTGTTGA
GAATCTTAATCACATCATCTGTACTGATCGGTGCCACCTGGGTAGACCAGCAAGGACCTGGGGAGTCCTTCTTAGTGTTCTTTATTT
TCTTTTAGAGCTGGTTTGGGGCACACCCACTCCATTGCATTAAATTACCTGTATGCTCTCTTCCCTGGGTTCAGAAAGAATTAGGGA
AGGGAAAGGCCTGGCTGCCCTCCCCTTTCCCCAGGCTGTGACTTCACCACCCTGACCCTGCCCATCATGTTGGCTGAGCTGGTAC
CTCTGTGTGGCTGGCAAGGCACCCAGGGGGCCTTGTTTCTCGGGATCTCAGGCGTTGGGCTGTGGGATCTTCTCAGGGGAGGCTGGAG
GCATCTCTCCTCCCAGGCAGAACCAGTCCTCTGCCCACTCCCCCACCATGCTCCTGTCTCGCCTGCCCCACTGATTTCCTGCCCCTC
TGCTCCTCCTCTGTGGCCCCTGCCTGGCCTCGTTGCTGGGGTAACCCAGGTGGCAGAGAGGAGTTGGTGTGAAGATGCTTATCCAGGC
TGCCCTGTCTGCTCCCACAGTGTCTTCTTTTCCCACTTCTAGTTACTCTTTCTTCAGCAGAACCTCATGGGAGGCCGCTTCTCCGTAT
GCCCAGGGGAGCCTGGTCGTCTGCCCATTCACTCTTTAAACAGAATGGCCAAGGCCAACTTGTCTGTCTCTTTGCAGACCTCACAGA
GGTCCCTGAGTCCATCCCCAGCCTCTAGACCAAATGATTGAGTAGTATCACTGGAACCAGTTCTGATCCTCTGCTCTCCCTGGTCAC
GGAAGCCTTGTCTGCAGAAGGGCAGAGCAGCAGTGCAGGCTTGGCTAGCCAAGTGGCTCTGCACCTTCTGTTTGGACCCAGGTGCTG
CTTTGGGATAGGTTGTTGGCTGTCACAGTGTCCTGGTATTTTTGAGAAGATGCCTCTGGGTACTGGTGCCTGTGTTTTCAGGCCATGG
TGGAAAAGTAGGAGTAGGATAGACCCTTTCCAGCCCTTTCCATGGACCCTCCCTGTGGGGTGACCTGCAGGACCAGGATGCAGAAGG
CAGGAGACTGGCTGAGAGCCTGCGAGCCCACTGCCGAGGGCCCTAGGCACACATTTGTCTTCACAGGTCACACTGAGTGTGTCGGC
CCACAGGAGGGCAAGCGTGAAAATGGCCTTTTGACAGCGTGAAAATGGCCTTCTCCGCCACCCGCCCCAGGGTGGTGGTGACTTTAT
TTATAATCTTTGTGGACAGTCTGTAAATGAGTCAAACATCTGTAACCCACAGGGGGACAGCTAACTTTTTATATATGCTTCTCCTTT
GCGTGTCATCACACTATTTATAACTGTCCTGGGGAAAGACTTCATTCACTCGGACTTTCCCTTCCCCACAGGCGGCATTACCCAG
GTGCTCCGCCAGGGGCATCCTGGCTGGGGCACTTATCTCCTGCACAGCCTAAGTAGAAAGGCGGCTGATGCCTCGTATTTCTCTTAT
TGTGCTTTTAGGAAGAGGGATTTGTAAGGCCATGGTCAAAAGATCTGCTTTAAAAAAAATATAAGGTAAAGAAAGTCAAAATGAAAAG
AAATTTGAGTAACCTCTGTAGAGAATCTGGGGCCTGGATGGAAATGTAAGATCAGAATAGAGCGCTTCAGCTCTGTTGCAGTTATTT
GTGAAGCACATTTGGGGGAGAAAAATGAGTGTGTGCGGGGGGTTGTGAGGGGCGGCTGTATATTGATGAAATACCACTTTACTCAGCTC
AGTGACAGGCAGGCCAGCACATTCATTTGTAGTTCTTTTTCTTCTCTTTCTTTCAAAACCCCAAAAGGAGAGCAAGAAAGCAAGGCA
```

```
CCTATTCCTCTCTTTAAATAATGAGGGTGGGGAATTCTGAGACTGTGCTTTTCCCAGGAGCTGTAGGGAGGAAAATTCTAGTGCCTTTT
CTTCGGGTGAGGAAAGTCCGGAGGAGGCGAATGGGTTTTTAAGTGCCCCCTCCCCCGACCCTGTCATTCCACCCCCAGGAGCTCAGC
TGGGATCTGAGGGAAAGGAGGGGAGGAGCAGCTGGGGGCAGGGACCACGCTACTTTCTGGTCACCCAGCACTTCGCTGGGCTGGCCG
GGATGTTGCTGAGTGACAGGAGAGGGGAAGGCAAGGACCATTTGCTGAGTGCTGGGCTGGCTTTACATTACACCCGGGAGTACAAGG
AGCAGGTCGTGCTCACCGCCCCCTGTGTAGATGAAGTCACCGGGCCACACTGACACTGCCAGCGGTGGTGGCACTGAGATAAGAACC
CAAGCCCGTTTAGCTCCCAAGTTCATGCCCTTTCCCTTGCCTGCTGCTGCCCATATCCTTAAAAGAGAAAAATTGAGTTTATGGCAC
TGAGACCCGTGATGCAAAATGTTGGAACAGGAGGAACTTTACCACGAGCTGGGCAGGAAGGGCTTGATGGAGGGCGGGAGATGCTGG
CGCAGGTCCTGGAGAAGACAGTGCTGGAGGGGTGGCCAGGGCAAGTGTGGGCACCCCAGGTTGGCCTGGGATCAGGGTGAGATTGTG
GACTGGCCCAAGGTCAGTTCTTGATGCCTCCTCTCTGGGGTCTGGTGCTGCTGCCCTCAGGGTGATACCGGGCAGGTTGTGAGGAGG
GTAGGTGTGGAGAATACAGGGAAAATGAAAGCCCTTCCTGCAAAAGCTCCTTCCACAGCCCCTAGCACCCCCACCTTCCACCCCTGG
AGGGCCAGCAGGACCCCTAGACCTGACTGGACATGTCCATACATAACTCTCTCACCCCTCACCTCCACCTTGCTCTTCTCTTAGGCT
GCCTTTCTCCTCCAGCACTGGCTGTGTGGCTGTTTATCGGGGGACAGTCACATGAGCTCAGCATCTTTACATCTTACAAGGCATTTTC
ACCCCTGGGTCCCTCATGACCCTCACGGTGGCTGGCCCAGGGCTCATGATGACCAACTATCTCAGGGATGAGAAAACTCGGCTGCAC
AGGGGTTCCCAGGGTTGAGTGACTCACTCCAGGTGCCCATCTCAGGAAAGAGAGGGTTCTAGAATGAGAGCTCAGGTCTCAGTACAC
TTCTGATTTCCCTATTCCAAACCCCACCACAAACTTCTGCTGGGGATTTGAGTGAGGGACCTCGAAGGGCTTCTCCATTCCTGGTTC
TAGAAGTTTCTTTTTCATTCTTGAGTCCCATTTTCCTGCGTCCCTGTCTGTGCCTCATGCCTCTGTTGAGCTCTGTCATGGGCAGTG
AGTGCCGAGAAAAGGCCCGTCTCTGCTTTGAGCCCCTCAGGAGGTGCTTCTGCCTCTTGCTTCCAGCTTGTTGTGTGATCTTTGTCG
AGTCCCTTCCCCTCTCTGGGGCCTCGTTTCTCTGGCCTAAAAACAGGAGCTGAACTCCCTTCCTGTGCCGAGGCTTCGCAATCTGATT
CCAGATCATGTTTCATCACGAGGATGAGCAGCCACAGTCCGGAGAGTCCTGTTCTTAGGGGTGTCAGACAGCACCCAGATGGCATCC
ATCCCAGGCGGATGGTGGTTTATGGTGTTGGTGCTTGGGAGGACTGAGGAAGGAGACAGTGACTCAGACTGGGGATGCTGGGTGGAT
TCCCTGTAGGAGGTTGGGGGGTCCCTGAAGGACTAGACAAGCCATGATGGGAGGGGCTCGGGCAGGGGCTCAATTCTGAGTGTGAGA
AGCCCGGGGAGAGGGGCAGAGTGTGTAGGGAATAGGACAGGCCTGAGGGCAGCTTCAGGTTGCTGCCGTGTGGTGCACACAGGGGC
ATGTAGGGAGGAAAGAGGAGACTGTCTCGTCTGGAGAGGCTTTGGGCAGGCAGTGAACACAGGCCCAGGGCGAGGGCACAGTGATGG
ATGGAATATTTGGTAGCATCAGTAACCACCAGGGCTGCAGAGTTCTGCACACTCAGGCCCCGAAGACCTGATATCCTTCCTTTTAGA
GGTGGCCAGGCTTGGTAGTTAAGGACTTGGGGCCAGGCGCGGTGGCTCATACCTGTAATCGCAGCACTTTGAGAGGCCAAGGAGGGT
GGATCACCTGAGGTCAGGAGCTCGAGACCAGCCTGGCCAACATGGCGAAACCTCATCTCTACTAAAAATAAGAAAATTAGCCGGACC
TGGTGGCTCACGCCTGTTATCCCAGCTCCTTGGGAGGCTGAGGCTGGAGGAATCGCTTGAACCCGGGAGGCGGAGGTTGCAGTGAGCC
GAGATCGCGCCACTGCACTCCAGCCTGGGCAACAGAGTGAGACTCCATCTCAAAAAAAAAAAAAAAAAAAAACTTGGGTGGCACACC
AGTGATGGGCGCTCTGGGAACTTACTGAACCAAAGCCTATTTCCTCAGTGTTAAGAGGGAATCACAACTACTGGCTGTAGCACAGC
AGCCACCACAGAGCCAGCCACACACTGGGTCTCAGCAGCTGGCAACTGTTGCCTTTATTAGAGTGGTGGTTAGAAGATGCCACCTGG
AGCATCAAGACAGGAGGGGCATATGCGCCCATGTCACCCCTCCACTGTCTGCAGGTGTCCCTGCCTGGAGAGCACGTGTGGAACATG
CCCCTTTTCTCATGAGCCAAATGGCTTCCCGTCCGTGACAAGCTCCCCAGTCATGTTTCTGGATAGACCTTGTTGGGTGAATCTGTG
TGAGTAAAAAGGAAGTGGGCTGTTCTCCGGAGGGACAGGGCTGCAGTGGTGGCCTGGGCTGGACTTCTGGAGCCTTCTTACCCCATGG
GGCCCTGTTATTCTACACTCAGTTAGCAATTTGTGGTCTGGGCAGAGTAACCGGGTGTGGACATGCAAATCGGACAGGTGGAGAA
GAACAAAAATAAACCCGTGGGGCCAGGCCAGAGGCTGAGAGGCCTGGAGGAAGGTCAGGGGGACACACGATGACCAGGCAGAAAAG
GCTGAGAGAGTAGGGCCACAGGGAGAGCCGAACTAGGAAGCTGACCTGGGACAGAAGCAGGGAAGAAAAGCATTGCCACAGCTTGCC
TGATGCGTCGTCAAGAGAACACAATTTCCCCTTCAAGACTTGAGTCAGGGTGCTTTCTAGAATTCTAAAGATTCTTTCTGCCCCTTC
ACCCTGCTTGGAGCACCAGGGCTGTGGCAGTGGGTGACGTTCCCAGCCCAGGTGCCAGGGGGCCAGGCAGCAAGAGCAGCTGGGCAGGG
GAGGGACGTGCCCAGGGCTTGCCCTTTGTGGAAGCCATATCTGTTTCACAAGCCCTGGGCACAGGGTAACCAGAGTTGTATTCTGAG
CAGAGCCTTGATGATACTAAATGAACAGCATGCAAATGATGTGTGAAATAATATGCAAATTGGCATCTGTTATTTTTGAGTCAGCTT
GTTGGGCATTTTTCTTAAAGGACAGGAGTGCTGCTCAGTGTCAAATGAACAGGAAAGTCAGCTTAAAGGACACTCCTTACAGGGACT
GAGCTGGCACCTACTCCTTAGAGACGTTGCTGATACCAGGCCTGCCACGCGACATCTGCAAGGACAGTTGTTGGTGTTTGCTTCAGG
TTATAGATGGAGAGACCTATAAAGGTGGGTGCCCTGCCCAGGTTACCCAGTAGGTAGGGAGGAGGAAGCAGATGACCTGTTGCCTGG
CTTGAGCCATTGAACCTTGCTGACCTGTCAGTGGCTTTGAGCTGAGGGGGCCCTGCAGGGAGGCAGGGAGAGGCTGCCCTTGCTCAT
TCTGGTCCATCTGCCACCGTTGGTTTTCCTTAAGCTCTGTGCCTCAAACTCTCCGAGCCTCAGTGTCCCCGTTTGTAATGGATGTGG
GGAGACTGACATCCACTAACCTGGGGAGGATGACACTTGCTAGTGGTTGGTCAAGTGGCCAGCCTGGCTGTGGGTGCTCCTGGGCTG
CCAGCAAGTGGTGGCTCCTCCTAGCCCCTTTGCCTGAGTTGGCATCAGCCTGCCTCCTCGCAAGCTTCCACTAGCAGCGATTTTTGC
AGAGCAAAACCCCTTTTGAGATTTCTGATTTTAACGAATTGGAGTTTAGCTATTTTCTTTCTTTTCTGGCTTCCAGTGGGTAAGATG
TTGGCTCCCCAAAAATCTCCCATACATGGGAATGGCCGGAGCTGCCTGCATGGGTGATGTTACCTTCTCCCAAGGTGAGGGCTTGGG
GTAGGCATGTTGCTCAGGGTTGAGCCACCGCCTTTGGAGGGCTCAGAGCTGGATGGCATTGGTGGAGGATGCAGGGGAGGCCTCCTC
AGGGGGGGCACCATACCTGCCCTAGATGGCCAAGCTCTGTAGGCCAGCCAGGTGTGCTAGCCTGGGTGGGCAAGATCCCTGCATATGC
CTCCCAGCCCTGTGAGGAAGGCAGGGAAAGGACCATCCTCCTCCTTATAGGACCCCAAGGCCCATAGAAGTTATGCGGTGGCTCACA
CCTGTAATCCAGCACTTTGGGGAGGCTGAGGCGGGAGGATCGGTTGAGTCCAGGACCTCAAGACCAGCCTGGGCAACATAGTGACACC
TTGTCTCAAAAAAAAAAAAAAAAAAAAAGTTACATGACTTGTCCGAGGCTACTCAGCATTGTTAGTGGCAGGGGCATAGCTGGAATCCTG
GAATTCCTGGTTTTAGTTCTTCCTCTCTTTTTGTTTCTCCCATCCCTCTTCTGGAAACATATCCTGAACACCCACTGTGTGGCAAGC
CCTGCTCCAGGCGGCAGGAGTGCAGAGAATCTGTGCCTTACAGACTCCAGGGGGGTCTCTCCCCAGCCAGATGTGTTGTTCTCCACC
AACTCTTCCTAAGAGAGCACAGGCTTTCCCCATAGACCTGGATTCAAATCCTGGCTATTTGGCTGTGTGGCCTTGCGCAGCGGCCCC
TTTATTTGGGAGGTGGTATTTTAATTCCTCTTGTGTGGGTGGAGAGGTGAAGAATTCAACGTGGGCCAGAGCTGTCACCCAGGGGGCTC
TTGTCCATGCTGGGCAATGGTGGGTTTTACCTTGGTTGTTTCTTCTGAGAGCTCAGTGGCCTTGGGATCATGCAAGGTCCCTCACAG
CTGGGGCCCATGGTTCCCAGCCCCTCCTGCTCTGGGCTGGACCTGGTGTTAAGCTCTGCCAAGATGCTTTCCCCTGCTCTGGGAGAGT
AATGGTGCTGAGCCTGGTGGGAGGGCATTTTGGCAGAAGACACCTGACGGGGTGCACACTATGTGCCAGCGATGGAAAGATCCCTTA
AGAGTGAAATGAAATTGATGCAAGCTGAGAGCAAGTCTAGGATGGAGTTGAGTCATGAGGGTTTCCTGGTGGGTGGACGGGATCATA
TAGAAAGAAAGCCTGTGTCCACAGCAGCTTGGGGGAAGGCACCGCTCTCATTACATGGGAACTTATTTTTCCTTCCGTAAGAATGAC
TCCAAAGCAACACCGGGTCTTATTCAGGAGTGAAGAGGTGACAGGCGGTGGACAGAGAATATTTATTAAGGGCCTACTATGTGTGT
TTACACACCTCATCTCATTAAGTCCTCACAACAACCCTGTGAGGTAGATATAATTCTCCCCATTTTACAGATGACAGACCCAAGGCT
CCGAGAGTTTAAGTGACTTGCTCAGGGTCTCACAGCTGGGAGGTGGTGGAGTTTGGATTTGCACACAGGGGAGTGTCTCCGACGCCC
TCACATTGTGCAGCAGACATGCTTTTGCATATCGATATCCTAGACTGGGCTCTTTCAAGCCAAAAAGCTTCTCCTGCCCTGCCTGAGC
TCTCCACTCCCGCCCACCCCACCCCTTACTGTGCCACCCTCAGTCCCCTGCCTCCCCCAGAGCCTCAGGGGATGTTTTGTGCT
CATGGAGTGGGGTAAGGGTAGAGGGAGGAGGGGAGGAGGATGCTGGCAGTTTAGATACAGGGGTTTGCTGAAGCTCCAGGTGCCGTG
CTGGGGCTCTGGGGCTGAGGAAAGTCTCCTGTGGGCCCTGGAGGGGGCCACCCGAGCCCTCAACAGCATGCGTCCTAGTGTTCTTGCCG
CAGCTCTGTGGCTTCTCGGCTGTGCAGCCTTGTTGAGAAAACTGACCTCTTTGAGCCTCAGTCTCCAGGTCTGTGAATGGGGTTAAC
AGTGTTTTCTCTCTCATGAGAATGGGTGTCATGAGGAATAAATGAGACAAGGGCACTGCCTGGCGTGTTCTGGTTGGTTCTGGGG
GTTCAGCACCAAATAGACATGGACTTCACAGGCTGGTGGGGGAGGCAGGCACTGAACAAGTAACAACAGGTCTGATGTGAGCTTTGC
GAGAGACAAGGGCACACACATGTGGCTTGGGTTTGACCCCACAAGCCTCGAGACTTGGTGTCCCACGCCTCCGTCTGCTCCCCTTCCCC
CCATTTCCTGAAGGTCCATCTCACCCTCACTTCTTCCATCCTCCCTCTATATCGTCTCCCAGGCCATCCTCTGATTCTTCTATATGG
GCTATCTAGCCTGGTGTTAGGAGAACAGGCTTTAGTGCCAAGCAGATATTGGGTACAGCGCTGGCTGTGTCACTTACTGTAGGCTGT
```

```
GTGGCTTTGGGAAGCTGTCTCAGTGCTTTGATTCACAGTTTCCATGGGGGTGAATCCTAACCTGGCCGCTGGGGCTGTCATGAGAGT
TAATGAGATATTGCTGTTTGTAAGGCAGAGGACATGGTTTCTGGCACACATGGAGCGCTCTGTGAAGGCCAGCTGAGTTGCAGGTGA
GGAGAAGCATCCTAGGCTAGGTGTGGGGCAGCTTAGTTTCTGGTCCTCTGTTTTGTCATCTAGAGAGTGAGAGAAGGAGCCTGAGGA
GGGGATGTTTCCTGCACTGGCCATCTGCGGGAAGGTCGTGAGCACCAGATGGAATTTCGGAGAGAACTGGAAGAAAGCTACGCTATT
CCAGCTGGCGGGGGTCCTCATCGATGGTGCATGTTGGCAGGGTGGGCTGCGGGGGGCGTTATCTTCCTATCCCACAAGCGAAAGCCT
GTGAAGACATCGTGGCTAAGGGGATTTTCTCACTCTCAGGAGACAGCTGAGAGACAGGCCCTTCTGGCCAGCCTCCCTCCCTGCTGC
CTCCTCCCTGCCAGGCCAGCCCTTGGCACGAGTCCTGCTTGTCTCCTGAACCCTCCTAGCAGCAGGGATTTAATTACTATAATGTAAG
TAGCTGTGGCTTGAGGCCTTCTGGTGGAGGGGAAACAGATTTGTCTTCTCACTGGGAGAATAAAAGTCTATGGAAAGGAAAAGAAAT
GTACTTTTCCCCGAGGTACCTGAGGCTGTTGGGCTCAGGGGAGCTTCCCTCAGGGCGGGGAGAGGGAGCATGGGTGGCAGCGGGAGG
AGGCACCACGTCTGACTCAAGGACCCTTGGCGCTTGCTTTTGCTCTCTGTGGGATCACTCAGCTAAGCTGCCTCAGTTTCCCCAATG
ACCCTGCTAACAGATCAGCCTGACATTCAGTAGGCATCTGTCAAGCACCTACCATATGCTAGACACTTAGAAACTTCCAGCTGAGTA
GCCAGCCCAGGCGGAAAAATAATTTCATTCATTTGACAAAGTTATTGAATGCCTGTTATGTGCCAGTGTATTGTTCTAGGCATCACA
GATATACAGTCATGCGTTGCAACGACGGGGACGCAGTCTAAGAAATGTGTCATTAGGCAATTTCATCATTGTGTGAGCATCATGCAG
TGCACTTACACAGACTAGGTGGCATGGCCTTCTGCACACGAGGCTGTATGGTGTAGCCTACTGCTCCCAGGCCTGTATTGAATACTG
TAGGCAATCATAACACAATGGCAAGTATGTATCTAAACATAGAAAGGTACAGTAGAAATACTGTACAATGGATACAAAATACTACA
CCTGTATAGGGCACTTACCATGAATGGAGCTTGCAGGACTGGAGGTTGCTGTGGGTGAGTCAGTGAGTGAGTGGAGAGTGAATGTGA
GGGCCTAGGACATGACTGTAGACTAGTGTAGGCTTTGTAAACACTGTTCACTTAGGCTAACTACACATTTATTTAAAATATTTTTTCT
TCAATAACAAGTTAACCTTACCTTACTATAATTTTATTTATTTATTTATTTATTTATTTATTTTGAGACAGAGTCTCACTCTGTAGC
CCAGGCTGGAGTGCAGTGGCACGATCTCGGCTCACTGCAACTTCAACCTCCCAGGTTCAAAGAATTCTCCTGCCTCAGCCTCCTGAG
TACCTGGGATTATAGGCACGTGCCATCACACCCGGCTAATTTTTGTATTTTTAGTAGAGACAAAGTTTCTCCATGTAGGCTAGGCTA
GTCTCAAACTCCTGACCTCAAGTGATCCACCCGCCTTGGCCTCTCAAAGTGCTGGGATGACAGGCATGAGCCACTGCGCTTGGCCAA
ATTTTTCACTTTATAAGCTTTTTATACTCTTTTGTAATAACACAAACACATTATACAATTGCACGAAAGTATTTTCTTTATATTCTT
ACTCTATAAGTTTTTTTTCCATTTTTAGAAATATTTATTTAAAAAGATGCATTTTTACTTACAAAATCTTTTTTGGTTAAAAACTGAGA
CACAAACACACGTTAGCCTGGGCTGACACAGGTTCAGGATCATCAAGATGTCACGAAGTGATAGGAATTTTTCAGCTCCATTATAAT
TGTACAGGACCTTGACTGAAATGTCATTATGGGACACATGACTGTAGTAGAAAAAACCCACAAAAGAGAAAAATCTTTGCTGTCACG
AATTTACATTTTAATAGATAAGCCTGATAGTAAACTATCTAAGTTAATTATATATTATGTTCGATGGTGATAAGTGCTATGATGAGT
GTTATGAAGAGCCTCTTCCCTCCAAGATACAACTTCTTACTCCATTGACAAGTCTTAGATCCCAATCTGTTAAGGCCCCAACTAATT
TTACAGCCAGAGTTAGATAACAGGAAAAAGACATCTCTTTTCCTTTCATCATTGTCATCATCAATAACAATCACAATTGTTAGGACC
ACTCTGGATGGGAGTGCAGGGGCTTCTGAGAGGAGAGTGGTCATATTATTTTATTTATTTTCAAATTAATTTATTATTATTATTATT
TTTGAGATGGAGTCTTGCTCTGTCGCCCAGGCTGGAGTTCAGTGGCCCAATCTCGGCTCACTGCAAGCTCCGCCTCCCGGGTTCACG
CTATCCTCCTGCCTCAGCCTCCCGAGTAGCTGGGACCACAGGTGCCTGCCACAACACCTGGCTAATTTTTTGTATTTTTAGTAGAGA
CGGGGTTTCACCATATTAGCCAGGATGGTCTTGATCTCCTGACCTCATGATCTGCCTGCCTTGGCCTCCCGAGATGCTGGGATTATA
GGCATAAGCCACCGCGCCCAGCTTTTTTTTTTGTGAGACGGAGTCTCACTCTGTCACCCAGACTGGAGTGGCGTGGATCTCGGC
TCACTGCAACTTCCGCCTCCCAGATTCAAGTGATTCTCCTGCCTCAGCCTCCTGAGTAGCTGGGATTGCAGGCACATACCACCATGC
CCAGTTAATTTTTGTATTTTTAGTAGATACTGGGTTTCATCATGTTGGTCAGGCTGGTCTTGAACCCCTGACCTCAAGTGATCTGCC
TGCCTTGCCCTCCCAAAGTGCTGGGATTACAGGCATGAGCCACCATGCCCGGCTGAGAGTGGTCATTTTAAATAAATGCTCAGAGCT
AGGTGCAGTGGCTAATGCCTGTAATTCTAGCACTTTGGAGGGCCGAGGCAGGAGGATCGCTTGAGCTCAGGAGTTTGAGACCAGCCT
AGGCCATATAGTGAGACGCCTTCTAAACAAAACAAAAAATTAGCTGGGCATGGTGGTGCGTGCCTGTGGTCCCAGCTACTTGGGAGT
CTGAGGTGGGAGGATCGCTTGAGCCCAGGAGGTCAAAGCTGTAGTGAGCTGTGATCGTGCCACTGCACTCCAGCCTGGGGAATAGAG
TGAGACCTGGTCTCAAACAAAAACAAAAACAAAAACAAAAAACAAAACCACAAATAAAACAAATAAAGAAGTGCTTAGGGCCAGGC
ATGGTAGCTCACGCCTATTATCCTGGCACTTTGGGAGGCCAAGGCAAGAGGCTTGCTTGAAGCCAGGAGTTTGAGACCAGCCAGGAC
AGCATGGCCAAGACTCTCATCTTTACAGAAAAAAAAAAAAAAAAGCTGAGTACATTGGTGTGCACCTGTAGTCCTAGCTACTTGGGTGGCT
GGGGTGGGAAGATCCCGTAAGCCCAGGAATTCGAGGATGCAGTGAGCTGTGATTACGCCACTGTGCTCCAGCCTGGGTGACATGTGA
AACCCTGTCTCTAAAAATAAACACATAAATAAATAAATTAAATAAGTGCTCAGGGAAGGCCTTACCAAAAAGGTGATGTCTATTGGA
GGATCTGAAGTGGTGAGGGATCCAGCCACCTGGGGGAAGAGGAATAGCAAGGTGAAGGCCCTGAGGGGGGATCATGCCTGGTGTGTT
GTAGACAGCACTGTCCGGTGGACACGAAATGGGAGCTACCGCTGTCATTTTAAATTCCTGAGTAGCCATATTAAAAAATAGTGAAAAG
AGAAAAGTAGAATTATACATGTTAATAATATTTTAATTTAACCCAACATGACCAGAATATTATTATTTGAATGTGCAAGCAATACA
AAAAATTATTGAGATCTTTTACACTTTATTTTTTGTACTAAGTCTTTGAAATCTGGTGTGTCTCTGACACAGCATATCTCAATCAGA
CATTAAGTTTTCATTGGAGATACTTGATCTGTATTTAGAGTTTATAAAATTTACAATTAAAAGAACAGACTTGCTGGGCATGGTGGC
TGACACCTGTAATCCCAGCACTTTGGGAGGCAGAGGCAGGTGGATTGCTTAAGGCCAGGAGTTCGAGTCCAGCCTGGGTAATGTAAC
ACAGTGAGACTCTATCTCTCTAAAAAAAAAAAAAAAAAAAATTAGCTGGGTGTGTGGCATATGCCTGTGGTTCTAGCTACTTGGGAG
GTTGAGGTGGGGGATAGCTTGCTGTTGAGGCTGTGGTGAGCGTGCCTCTGCACTCCAGCCTGGGCAGCAGAGCAAGACCC
TGACTCCCCGTCCGAGAAAAAAAAAGAATAGATTCACACACTCAAGTTGTTGAAAACGTACTTAAAATTTTTCCAATAAAAGAGTG
GAGTGTCTTTTTATATTTAATTTTAAATGAAATGGACTTAAAATCCAGTTGCACTGGTCATGTTGCAGGGGCTCAGTGGACACACGT
GGCTCGTGTCCTCACACTGGACGGTGCAGGCCTGGGAGCAGAGAGGATGCCTGTGAGGCTGATTCAGTGAGTGGAGGGATCATGTGG
AGATGGAGCAAGAGGAGGGCTTGGAGTAGAGAAGTGACAAGATCTGACTTACATTTTAATGGGACCACTCTGACTGCAGTGTTTGG
AACAGACAGGGAGGGTCAAGCCAGGGTCAGGGAGCCCCGCTGAGAGGCTGTGGGGACAGCTCAGGTGAGGGTGGTGATGGTTTGGAC
CTGGGTGGTAGCATTGAGTTGGTAAAAAGTGGCTGGACCCTTGGGCTATTTAAAAGGAGGGCATTCCTGATGAATTGGACAAAAGGT
CATGGTGTGCAGGTCAGGCAGTGGTGAGAGAAAGGGGAAGTCAAGGATGAGGCTGAGACTTTTGGCCCAAGCAACTGGAAGGATGGA
ATTTCTGTTAATTGAATGGAGAAGACTGTGGGAGAAGCAGTTTTTTTGGGGAAGACAGGGAGTTTGTGGTTGGACTCGTTAAGTTTG
AGGTGTATCAATTTCCATATAATGCGTGTAGCACCTGCCCCAGGGGTTGTAAGAGGTCACAGGATGGTGCTTGAGGCCGTGGCAAGC
TTCTGCCTGGGCTCCTGACCCAGAACAGATGGCTCCTCTCACCTTCCAGAGGTCTGTTCGAGGCATGGCTGGCAACGTTAAAAAAAA
TTTGGCCGGGCACGGTGGCTCACGCCTGTAATCCCAGCACTTTGGGAGGCCGAGGCGGGTGGATCACGAAGTCAGGAGTTTGAGACC
AACCTGGCCAACATGGTGAAACCCCGTCTCTACAAAAAATACAAAAATTAGCCGGGCATGGTGGTGCACGCCTGTAGTCCCAGCTAC
TCGGGAGGCTGAGGCAGGAGAATCGCTTGAACCCGGGAAGGCGGAGGTTGTGGTGAGCCGAGATAGTGCCATTGCACTCCAGCCTGG
GTGACAGAGCGAGACTCTGCCTCAAACAAAGCAAACAAACAAACAAAAATCAGCTAAGTTATCCATGCCTCCAGCACCGTGGA
TGTTGACTCCCTGAAGGTCACTGGAAGTTGCTAGGGTCTTTCTGAGTCAATCCACATTTCAAGGTCAAATCTCCCTGCTCTGGGCTG
ATGAATAAAATGTGTCATAAATGCTCAGAAGCTCCCCCAGTGTGGCCTGGAAGCCTGATTTTATGGAAACTTACTGAAATGATACAA
AACGCTAAAGATATGCATTCCTAATTTTCTAGCACTTTCAACTCCACAGGGATGTTGGGGGAAGGGATTAATGAGCTTGGAGAGGTG
GAATAACCTCCTCAAGGTCTCCCAGCTGGGGGAAGGTAAGCACTGGATGTTGGGAATCACCAGCCTCATGTGCACCAACCATGTCGC
CAAATGTCCAAAGCCTGCAGAGGGCCCAGGGCTCTCACGGACCACACAGGGAAGCCACTGCACCTGTTCTTTTTAAGACTGGGAGGA
GAGTGGGCAAAGGCAGCAGGTATGGCTTTTGAGGTCCTCTCCTAGCCCAGCTGGTGTCTGATGCTGGCACCCGGGCTTGGAGGGTGA
GAGTCTTGGGACCTGTAGACCAAGGCCACCTCACTTGGGCAGCCCAGGCCTGCTGGACATGGGCCTTCTGACTTAGTTTCAAAGCTG
GACCCTGACCCTTGTTGGTTGCAGGACTTCAGTGATTTAATCTTTGAGTTTATCTGTTTCATTGGGGTGATGGTGCCTGCCTTGGAG
GAGTTTTGTGAAGTGATGTTAATGAAGGAGCTTAGGCCGGTGACTTGACACATGCCCAACATAAGGAATGCCTGTGGGGCCGCCTGG
```

100

```
CTCTGCCCCTAGAGTGGGAGGGAGATGGTGCAGAGGGAACAGTAGAACTGGTATGGGCTGCGGATGCCTGGCGAGTGGCATGAGTAGG
GGAGAAAGAAGCGCCTGCCAGAATGGGAGTGAGGGAGAACAGTGGGGGGTCCCCTCCCTTACCACAAGACCTGGGATCTAACTGTGC
CTTTAGATCAGAGCTTCTCAACTGGGGCACTTTTGCTCCTCAGGGGACTTTTAGCAATGCCTGGAGACATTTTTGGTTGTCACAGTC
AGGAGAGGGATATTATAGTCCTTTAATGTGGAGAGGCAGGGATGCCCCCTACAACAAAGAATTACCCAGCCCACAATGTCAGTGGT
ACTGAGGCTGAGAAACCCTGCTTTCGATGACTCATTCTCACCAGCTTGTTATGAGGATGAATGCGAAAATGGAGCCTGCATTTCTAA
CTGCAGCCTGCCCTTCCAGGTCCTCTCGTTCCCTCACCCTTTCCCAAGGGGGCTGCCTCTGTCATCAAGCAGCCTGGTCCAGGAGTC
AGCAGACCTGAATTCAAGTCCTGACTCCTCATGCCTCAGTGAAGTTTTTCCTTAGTTTCTAGTCTCCATGTCTTTTCATCCTTGGAA
ATCTTGTCTTGAGTGGGGCAGTGGTGTCTACACAGTAATGGGGCTGTGGTGTGGACTGAGTGGGAACCCGGATGGGAAGCATGCTTA
GTCAGCTGTGATGTGCCGGCAAAGGGAGGCAGCAGGCCAATCCGCTTTCTAGTCCAGCACTGAATAATAGCCTTGGGGGGCATTGCC
CAGGAACCCCCATTGGCCCCTGCGAGGCCACATGGCTGTTCTCCCAGGCCTGCTCCATCTTGCTTCTTGCTTGTGTTTCCTTGAACA
GGTGGAGCTAGTCTCCATTTGGGGAAGATGGAATTTGCAGGAAAATGCAGGGCTATTATTTCTGTGCTGTGTGAGTGGGAGACAGAG
CTGGTTGAGTGGCAGATTTCCTGGCAGGGGGAGAAACAGCTAAGTGTGCCAGCCCAGGCATCGCTAGAATCCTCTTGAGGTGAGTGG
GCCAGGATTCCCCAGTGGCCACCTCGGGAAGCAAACATTTGTTCTGAGCTCAGGACCAGCCCCAGGAAAAGATGGGGTTTGCTGTCG
GGGAAAGGTCTGTACCAATATATTGAAGCTCTGAGAACATTCTACTCGGCAAACTTTCACTGATGCCCATTCTGTGGCAGGCACTGG
CCCCGGGGACGTTGGGGGACACAGAGATGGATGAGGCTGAGGGGGTGTTCGGGCTGCCGTAACTCTGAGGCAAGGCAGACTGTGCTA
AGTGCGGTAGAGAGGCTCTGCATCCCGTTAAGGGCCTGCCGCGGGATATTAATGTTTGGAGGCTTCTTAGAAGAAGTAGGGGTTGAGC
TGGGTTTTGAAGGAAGGTAGGGCTTCATGAGGCAAAAGGGAATGCGTGTTCTGAGCCGAGGCAACAGTAGGGGCAGAGGCTCAAAGT
TGGAGAGTGGCCATGTGTTCTTAGGGGAGGGCTGGAGAGGTAGGTTTTCCTAGAAGTTCTGGGAAGAGAAGACTGGAGAGGGACATGGA
GCCCCCTGCCCGTGAAGCTGGTGGAGTAGTGGGGGCTTCACGAACTAGGCTGATGAATTTGCACTTTTGTCTAAAGGCACAGGGAAC
AGTGGAAGGTATGTGAGCAGGGCAGTGGTGTGCTCCAGCCACAGCATGGAGGATGGGTTATAGAGAGAGGAAGCTGGAAGCAGTGTT
ACTGCGATAGTTGTGAAAAGGAGGAATGAAGGCTTGAACCCAGCTCGGGGCAGTGGGAAGGGAAGATATGGGGGGAGGTCTGGGGAG
GCAGGCAGCTTGTCAGGCTTCACCTTTCTCCATCCTGGGTATCTGAGTTGTGTGTGAGGCTCGAAGTGACTGTCGTGGCCCACTCGC
CACATTGCTCTGCTCAAAGCCCAGCCTTTTCTTGGAGAATGACAAGCAGCGAGCATCAGGCAGCCTTCTGGTCCCAGAGAACAGGAT
GGCACGGTGCCCACACCAGCCGGCACCAGCCACTCTGGCTGGGGAGGGGGGGAGGCCTCCTTCTGGAACTCATCAGCGCTGGCTTCT
GGCCAGGCCCAAAGGAGGTGGAATACACCCTCTTCCTGCTCGTTTTCAGAGATTTGGGAGGAGTAGGTATGGAGGACAGCAGCAGCG
TGCTGCAGCCGGCTACTCTCGGGTTCGTGTCGGAGCTGGGGGAGTGACTGGCTGTGCAGTTGGGCAAGTTGCCTGGTCTCCCTGAAC
TTCAGTGTCTGTGTTTGTAAAGTGGGCATTATGATGCTTGGCCCACGGGTGGCTGTAAGGGCATCACACAGGTGTAATTACGCAGGT
GTCATGTTCTTAGCTCAATAAATGGCCGCTGTTTTTAATTGTCAAGATATGATGATGGACTTGACTTGATGGGTGGGGAGAGGCAAG
ACCTCCTTGCCTTTAAAGGAGTTGAAGACTTGGGAAGCAGTTGGAATGGTGAATTCTATCTTGGGCGTGTTGGCTTTGAGGGGCTGG
GGAAAATCCTAGGATATTCAGAAGAAATCTAGGGTCTTCTAATCATGTGGTTCACATAAATGACCCTTACTATAATATCTATCTCAG
AGTCAACTTTTCAAGACACCAGCCTCCCTGCAGGCAACCCCAGTGTGGCATCAGGAGAGCGGGGTCTTGGGTCAGCATGGCCAGCCC
AGCCTCTGGCTTTGCTTGTGTCATTGGTGACTCCCGTGGTCCTCGGTGCCTTCCTCCGTGATGGCATTTCCCATTGGCTGGCTGGG
CCTCTGCCCAGGGATCCAGCTGGGTGCCGGCCCAGCTCAGGCTCCAGAGAGGAAAGAGCCCTGGATCTGGGGCCCAGTGACCTGATAT
TCACGCTGGCTCTGCCCCTTCCCTCTCGGTGTGGCCTGGAGAGATGTGCAGGCTCCCCGAGGGCAGGGGGGGGTAGTGAGGAGGGGTT
CCAGGAGATGAGGGATGTGAAAGCAGCAGCACGTGCCTGGCACACAGGGGCCCTGGTAGATGCTTGGCGGACAAGTGAGGGCAGGCA
GGGGGAGGGGGCAGACAGGATTCAGGGAGGCAGGCCTGGGAGGAGCCCAGGGCTCAGGGAGGGGGGTGAGGGGTCAGGCGGAGCCGGC
TCAACTCCTGGCTCTGCCGCTTACTAGCAGGAAGACTCCGAGTCCGGTGCCTGCTTTGGTCACATCCATGTGTGGGTGGTGGCGATG
GGAGGTTGGCCTTGGGGGCCGCTCTAATGCCTACCTCACAACATAGTGATAGGTCTAGATGGATACACAGCACCAGGGCCTGAGAGT
GCTGAATAAACCTTCATTCCTCCTCTAGTAAGCACAGTAAATCCCTAGAGGGCAGTCCCCAAAGGCCTTGATTACCCCAACTGTGGA
GTACAGCGAGAAGTGGAGCTTCTGGAGCCAGTGCTTTCTCTGCTCAATTTTGTGTATTCTTTCACTCGTTCATCTGAAATCTGACCC
AGCCCCATGGAAACTGGCTGCAGACACCCAATGGCAGTAGGGTACCAAGGGGCAGGGGAGCAATCGGCTCCCGGTGCAGACAATGAG
GGGTACACTGTCTGTAGAGGATATAGAAAAATAAATAAAGCCAACAAAAAGTCTGGTTTTTGTTATTGCCGTGTACCGGCACTTCTA
AACAAGGTCAGTGATAAAACACTCCTCCTCCCGGGGGCAGACGCTCCTGGCTCCCCCTCAGTATGCTGCTGCCTAATCGTCCTCCA
GAAAGGAGCCTCGGGGGCTGTAAATATCAGGAAGGAACTCAGCAAGCCTGGCAGAAGAAGCCAGCGATGATTTGGAGAATAAGCCC
ATTGATGATTGCAATAGCAGCATGGATTTGGGGCTCAGGAGACCAGGAAGGGGAGGGTGCCACACTGTGAAGTAAGGGTAAGGCAAG
AGAGTGGAGGTAACTGCTGTGGGTTCTGGCTGGGAGAGGCATCGTCTAGGCCCTTTAGACGTGGTCAGGAAGAGGGTGTACTTCATC
TCCCTTGGGCCTGGCAGGAAGCCAGCGCTGATGGGTTCCAGAAGGAAAGCCCCCAGCCAGGGCAGCGCTGCCAGCTGGTGTTGGC
TGGTGTGGGTGCTGTGCCACCCCACTCTTTTGGACCGGCAGGCTGCTTGCCATCTTCCAAAGGGGACTGGCCTCTTGACAAGGGTAG
GAAAGGAGCCAGGGCTGCCCTATGGGCTTGGGGGCTGGTAGGGCGTTGGGGAAGGCAGGATTCACATTTCTCTAGCCTGGACAACAA
GCATGTGCCTGAGGATCAAAATGAGCACTTTTAGGGTTTTATTACTATTATTTACTAAACAAGTTTCCTTGCAGGGTTGTGGAAGGG
GCCCATGCAGAAGGATCTGTCTAGAGGAGGAGGCTCCGCATGTGTACAAGGGAAGGACGTGTGCCCTTGGGACTGGCTGGGGCACGG
CCCTAGGGAAGTGTGCTGTGTGTTTGCGGGGGTGCTGTGGTCATGTGGCCCCTTCCCTGACTGAACCCCTTGCAGGGCCCCATGCC
ACACAGCAATGCTGCGTCCGTTGCAGAAGCAGACACCTTTGGCTCCTGCTGTGTGGGAAGCAGGTGTGCATTAGTCAAGGTGGCCTC
AGTGGTCCTGGGTAGTGGGGCCCCCGCAGTGAGAAAAGCAGAGACCTCTTGGGCCCGGGGGTGTAGGAGTCACTCTCCCTGATCTGC
TCCCAAATGCAGGTGCATCTGGTTCCATTTGGAGCCGGTGAGCTGGCTGATGGTGGCTGTCTGGGGCTCCTGGGGCTGAGCAGCCAG
GCTAAGCTTAGCCCACAGGCACTTCCCAGAAACTGGTGGCACTGTCACAGTGTTGGCTGGATGCTGAGGAGTTGGCAGGATACAGTG
CAGGAACAGCAAATAGGTTTTATCTGGGTGTCAAGTTGAAACAATTGGTACCAGCTTCCAGGAGGACTGTGTTGGGAGGATTCTGA
GGTGTTGTCTGAGCTCAATGGGGAAGAGTGCCCTGATTGATTCATGAGGTCTATCTTGGGCAGGGGAAATGGAAGAGTGGCAAGGGGT
TTGCCGTCCCTGGTGTAGTGGAAAAAAATACAGAGAGCCTAGATTTGATCTTAGGTGAGTGGCCCCAGTTTTTTTTTCTCTCTCTTTTT
TTTTGTTTTTTGAGACGGGGTCTGGCTGTGTGTTGCCTAGGCTGGAGGGCAGTGGCACAATTTCAGTTCACTGCGACCTCCACCTCCTG
GGCTCAAGCCATCTTCCCACCTCAGCTTCCCAAGTAGCTGAGACTACAGGTGCCCGCCACCATGCCCAGCTAATTTTTGTGTTTTTA
GTAGAGACGGGGTTTCACTATGTTGGTCAGGCTGGTCTTGAACTCCTGACCTCAGGTGATCCACCCGCCTCAGCCTCCCAAAGTGCT
GGGATTACAGGCATGAGCCACTGCAACTGGCCCCCTAGTTTCCTCATGTCTAAAATGAGGGTAAAAATGTGGCCCTTCCAAGCCTTA
TTGTGGGCGCTGAGTAAGATAGCAGAGCGAAAGCCCTTTGTAAAATATAAAAAAAGCGATACAAACCCAGAGTGGGGGTTTTGTTGTT
ACTGAGTCTCCCAGGAACCTGGAGTGACTTCCCAATAATAACATCTGCCCCACTGGGGACTACTCCATGGTCTGAGTGCCTGAGAGG
CCTGACAGGATGTGTGACCGGCGCGGTGCCCGGCACCCAGGCCTTTGACACCAAGTGTCAACGAGGTGTTTGGTCATGTTTCCAACT
CTAAGTCACTTCATTTCTGTCTGACTCTCTCCCTGGCTTAAGATTTAGAGACTGTTTTCGTGTCTAATGAAAGAAGGACAGAAAATC
TCTCTCTAGAACCTTGTCTCTTACTCTCCATTAAAGGTAGCAAAATTGCTTTATAAAGTCTAAAAGCCTGACAAGAACCGTACTAA
TAATGCATGAATTATGCAAATCTGTGTAGTCACTTGATTTGCATAATGGAGGGGCTGGAGGAGGGTTGGGGAGGGGAAGGAGTGAGG
GTGAGGGCGTCTGCCCAGATCTGTGTGTAACGGGGGACATTCATTTGCTCCCTTGGGTGAGTCCCCTTCTGCAAGCCCCTGCTGGT
CCCGAGGGTGTGTTCTTTGGCCTTCCCCTGCTGGCCTGGTGTCTGCCCTTTGTGGCCTGGCTGTGCAGCCCCCCCTCCTACTTTCGG
GGCAGTCACCGAGGCAGGGGCACCGCACCCTGTGCTGGGACTCTTGTGTAGAACTGGCTTCATTAAATCTCACAGCCGCCCCTCCATG
TCCAGACAGCCTTCCCTGAAAGTCCCCTGGGTCATTATTTCTCCAAACGTCTGTCTTGCTGAGGCATTGGACTTCGTTAAGTGCCGT
CTGGAATTGTAACTATTGCCAAGTGCCTTTTTTTCTTGGGTTCTGTGGGAAGCAAGGGTGAGAAGGAGTCTCCGGGAGAGAACTCCCA
CCTACCCCGCCTGGTTTCTCTCTCAGCGTTTCTCACTGTTGCTTTAAAAGGTTTCAGCCTCTGCTGTAAGGGGCCTCTTGCTTTTCT
CTCCCAGCCTCCCTGCATACTCGCTAACCTTGGGACTTCAGATTTGATGAGGGGCCGGGCGCAGTGGCTCACACTTGTAATCCCTGC
```

```
ACTTTGGGAGGCCGAGGTGGGAGACTTGCTTGAGGCCAGGACAACATAGCCAGACCCCATCTCTATTAAAAAAAAATATTTGATGAGG
GCTGAGGCTGGGACTGAGGATACTTGAGGCTTGAGCTCAGTGGGTGCTGCCTGGAAGCCCGGGGATGTGGGCTCTCAAGCTGAAGGG
ACGCTGGGAACCAGCTGTGGAGAGCCAGGCAGCTGGCTTTTTTGTCTAGAACCTGGATGGGGCAGCTTCTTGTTCTGGGGAGGGGAA
CTGTTCTGCAGGGTGGATGGATGCAGGGAGCCAGCCCTGGTTGGGAGGTGGGAGGGGCAGGGACCATATCTAGCTCCTGGAGAGGCC
AAGCAGACCGTCAAGGCTACAAACAGGCTGGGGTTCAGCTTATTTCAACATACTTCTTCAGCATCTATGTCTATGGGCTGGGCTTGT
GCTAGGCCCTAGGTGTCAGAGATATGAAGGCACTCATAGCTGTCATTTATTGAGCTCCTATTATAAGTGTCAGGTCCTTCGCTAAGC
CACTTATTTACACGTGTCATCTCATGTAATGTGTATGACAACTTTATGAGGTCACTACTCTTACTGCCCCATTTTATAGATGAGTAA
GTTGAGGCTTGGTCACATGGCGAGGAAGTGGCAAAGTGGGGCTCTAAGTGAGGTCTTCGGACTCCAAAGCCCCTTCTGCAGACCGTG
ATGCTTTGCTGCCTCTGTTGAGTCAAACCGGACTCTTCCCTTGAGGCCCTGATGGTAACAGCAGTGTAAACAGATGGTGAGCACAGT
GTAGTGTAATATATGCAAGGGGAGAGGTTGGAACCAGGCACAGAAGTAGCACTGAGCATTTAATTATGTCAGGGAGGGCCGGGGAAA
GCTCACAGAGGAGGTGGTGGGGGGGGGGGTCCCTGAATGAGCCACAGGAGGGAAACCAGATTCTGTCTGGCACTTAGGGAAGCAGCC
AGTGGAGGCTGCAGGTCAGTGGGCTGGGGTGAGGGGTAGGGGCTTTAAATCCCAGTTGCTTATAGCCCCCTTCTGCCCCACTTTTGG
GGTGCGACCAGGTGATGTGAAGCTCAGGAGGTCATGGCTGGGACACTCAGGAAGGTCAAGTGTGGGGTGCTGCCCCAGGGCCCCTCT
CCTCGGCCTGTATGTTCAATAGGTGCACTGGCCTCCCTGTCTTGGCCAGACAGCTCTGAGAGCCAGGGCTCTGTGGGTGCCTGAACT
GTGTGTGGACCCTGCCTGGGATGAGGCCACAGCTGCTCCCACACCCGCACCTGCTCACACCCAAGAAGGGGGCCGCCATGATCACTG
TGGAGGCCAGGGTGGGAGAACCCTCTGGACCATAGGGTCCCACCCCATTGCAGGGCAAAGAGGGGATCTGAGGCCCAGTGACTGGAA
GTGATTTGCTGCAAGTCACACAGGCAGCAAGAAGTGGGTCAATCCCCCAAGGTTCTGGTTTGGAGCACTCCCGGGGGTCTCCCCTCA
CCCCTTACCCTGGGCTCGCCCTCTCCAGGCTGTGTGCCATCCTCACCACCTCTGTCGTGCCCTCGCTCACCCACGTGGCTACACTCT
CGCGTGCGTTTCCCTCACACCTGGCCATCCTCTTAGGCCAGGCCAAGGCTTTGCTCTGGAGAACTTAAATGGCATTGTCAGATGA
GGAAGGAGCAGAACCTGCCGATCCCATGACCTCCCAATAGGCCATCTGCACACACTCAGCCCGCTCTCCCCAGCCCTGCACAGCCCC
TTCGGCTCGCACATCTCATCCTTACTCCTGCTAGCAAGCAAAGATGTCTGCAGGTGCTCTCATTCGGGGATGGGGATGTAGTAGGGA
GACAATACCTGGTAGATAGAGCTGAACAACTTTCTGCAGAAAGTTTGGAGTGAGGGGGGCTCTGTGATGGAATAACAGGCATGAACC
AGAAGCCTAATTCTATTGCTTAGAAGCTGTGTGGCTTTAGGCAAGCGACTTAACCTCTCTGAGTTTCAATCTCCTTATTAATAAAAC
AGGTACGAACAGTGCCTTGCTCAGTGGTCCCCTAACTTGGCTTCATTTTGGAATCCCCTGGGGATCTTTAAAAAAATGCTGATGCCT
GGCTCCCAGCTTCTGACCTGATGTCACCGCCCTGAGGTGCAACCTGGGTGTGGGGATTTTAAAAAGCTCCCTGGGTGATACTAATGT
GCCGCAAAGTTTGGGAACCACTGTCCCAGGGTTTGTTGGGGATTAAAGGAAGAATGCAGACACGTGGAGAGCGCTTACTAAATGGAG
GTGATTATTTCAGTGTTCATCTCCCTGCCTCTCACTCCATTTAATGTGGAGTCTGTGGGACTGAGGTTTTTATTTTTATACCACAGT
GGGTTTGCCAGAGTGCTGACCTAAGGGGGCTGTCAGCGGCCCATCTTTACCCGGAAAAGCCCCTCCCTTGCGCCCTCACCATCTCTG
CAGCCCTAGGGCAGGCCTCAGTCTTTCTGACAGGCCCCAGGGTCCTGATTAGGCCGGGGCCTGACCCCGCAGGGGCAGGCCTGCCCC
TGGTCTCCACCTGGTGGCTTTGGGAATGGAAATGCTGGTTCCCACAAAGGCTTCTGGGAGACAGAAGCCTAAGATGGCAGGGTGCGG
TGCACACACTCAGTCTGGATTCGGTGGCCTCTGGGGGATCCGGGGAGGGCGTTGGTGGGGGCAGAGGCGTGGGAGGGGCTGCTT
CTTTGGAGCTCTCCACTCTGCTCCCTGGTACTGTCTGAGGCCCTGCAAACCTGAGCGAGAGCGTGTGAGGGAAGACACATTTCTCCAG
ATTCCTCAGCTGTCCTGACAGGAAGTTGTGCATCACTGGGCTGACAGGAAGGAAATGGCTCTGCTGTTTGGAAAGAAATAGAGAATT
CTGACTGGGGCTGAGGGAAAAGGTGTCTGCTTGGAGAAGCCGTTTTGTCCCGATATGAGGACTCTTTGTCTCTGCAGGCAGTTGCTC
CCTGTTTATTTTCCTTTGAATGACAAGGATTGGCTTCAGTTAATGAGGAGCGGTGAGAGGAAAGTGAGAAGCGGCTTGTGAGATTTT
GGGACACGGAAGCCTTCGCAGGAAACCTGGCCCTCTGCTTGCTGTAATAAGCATCTTTCTTTCCCAGCTCCCTGCTTCGGGGACTGTGAG
GTGACAAAGGATTTTGTGATCTCACACGCCGCTTTTTCTCCATCCACCAACCATACCTTTGCAAATGCTTTGATTTCTATTTCTTTT
TACCACCCCAAAAACACAGTGGATCTTTTAATAGCACAAGGCCTTTTGAATCTCTAACCAATCAGTCTCCAAATCCTATCCAAAGTG
CACTTAGAATAGACACTTTTAATAAAATATATTGCTTTTAAGCCAGTGTAAACCTGTGAAATTTGTACATTTCTTTTTAGAGCATCTC
TACAAATTTTACATTATTGAAATCACCACCCAGTGACCTTTTAGGAAATACTAGATAAATTGCAAGAGACAAATAGACAAACCAAAT
GGAGTAAGTACAATTTCCAGTGCCTTTCCTGCTAGGTTCCCAGTCTGAGTCTTTAGAAATCCCTGACTCCCTGTGCCCCTCCCCCCAG
CTCTGTGACCTAGCCCCTGCTCTGCAGGCCTCTCTGCCCACCCTGCCCCTGGCAAGGAGCTAGCAGCTCGGGGTGTCATCTGAGAGCC
ATTTGCAGCTCTGAGAAGCAGCTCTGGTATGTGGCTCCTGGCACCTTGTCCCGTCTGTGTTTGTCTCTGCGCTCCATCATTCAAAAC
ACTTCCACCAGTGGCCAGGGCCCCAGAAGGGCTCTTGGGGGTGGCTGAGGACTGCTTTAGAAAGGCTCAGATGCTCTGTGGCCGAG
TCTGCCCCAGGCGAGGTCCTCAGCCTTGTCGAGTGAGAGCTGAGCGGGGGTCTGAGTCCAGCAACCCTCACTCACTGTGGAGCACTC
TCTGTGCCTGGGTCAGCCAGGTGTGGATTATCCACTTTGTGGATTGCCTGTAGTGTCTTTTCCAAACCCAGGTCACGTTCGTCCCTG
CTGCTGTCTCACTGCATGGGGACCTTTCCTCCCTATCAGCTGGTGGTGCTTAGGGGATGTAAATCAGGTGTCGTATCTTGTTTGTAT
TTTGTACAGGGAAAACAGAATTTGGAAGACAGATGGTCTCTAAAATCACATTACCAGAGCCTCCAAAGGGTCTCCTGGAGGCTCAGT
GCTCCTTTGGGCTATGGGATCTGTTCCCCTTCTGGTGTAGGTACGCAGAGCCTTGGAGCAGTGATGGGGGTGGAGGGGAGCACAACTG
CGCCCTGCTTCCTGAGGTCTGCAGATCGCAGGTCACTCGATGGTGGCGGCACGTGGTTATGCACTCATTTCATGGATAAGTGAACT
CAGTCACCATCTCAGCTGCTTTGCCATCGCTACACAGCGACCTGTTGCTGCAGCACAACTGGGAGTGTTACTTGGGTCCCCTGTATC
TCAGTTGCTGTTTTTCCCATGGAGTGACCCGACTGCCCTCCAACATGGGCAGGGAGGGAGAGCAGAAGGCAGATCAGCGGTTAGAGA
TGTCCTGAGTGGCACTGGCCCTTCTGTAGGTAAAGTAGCTTCGGGAGAAACAAGGTACTGTCAAAGCTGCCCAGGCTTCATTCACTC
CTTAATTTGATAATTAATTCCACAAACATTAATGGAGTTTTTTCTCACATTCCAGGCACTATGCAAGGTGCTGGGGGTGGGGAGAAA
CAGAAAGATGACAAAGACTTGGTTCCCACACTCAAAGAGCTCACAGCCTGGTAAGCAGATGCTCAACGCACTTAGAATTGTCTCCCG
AAGAGGCCTGAAGGGACAGGGTGGAGGAAGCGCAGAGGCGGAAGGCAGCTCTGGAGGGTAGGGTGGCTGGGAGTGGCGAGAACGGAGCC
GGTCCTGAAGGTTGACTGGGAACGGGAAAGCCTAAGGCAGGGAGGTTGTTCCCGGCCCAGCAAGCACATGGAAAAGGTTGAGGGAAAC
GAAAGCTTAGTGCATTTTGAAGGAGTGTGAGCAACTCGGTATGGCTAGAATTTATGGTGTGAGTATGAGATATGGGGCTGTAGAGGC
AGGCAGGGGTCAGATCATGCCTTGCTTAGGGGGTCTGCCTTCATCCCAGAGGCTGTGGGGAGCCGCAGAAGGGATTTAAGGAGTGGG
ATGGCACGATTTGTGTCTTAAATTACTTGCTATGGAATCTACATGGTATAGCAGAAGGAGCCCGGGAGTAGGGGAAGGAGCTAGGCT
CTAGCTCTAAATTCTAGATCTGGATCTAGCAGCTGGGTGGGTTGGCCAAGTCGAGGCCTCAGGGTCCTCAGGGTCTGTAAGATGCAAAG
ACTCTAAGGTCTGTCTTAGCCCTGACGTTCTCCACACCCGTGGCCGCCTTCCTGTTTGGCCCCCACACTCTCTGTTCTGTACTTGTC
CATCCACCTCTGGCCGCCTGACCCCTGTCAGCTGGCTGTCCCACCGTGGTCAGCAGCTGGTGGCAGCTGGAGGGAGAGGGGTGAGTC
CTCCAGGAAGCATGTGCACTCCTGCCCTCGTAATTCAGGCCTGCCTGGCTTCTCCTCTTCCAGCCCTGCGAGAGCGCCTTCCTCCCC
GCTGACCTCAGCACACCTGGCTCCCCACGGCACACAGCCCTGGCTGGGCCCAGCTGTGTCTCTTCAGTGAGACAGTTCACCCCCTTG
GGCCAAAATCCCCACCCACCTGGCCCACCGTGACAAGAGGCAGCTGCTTCGGGGTTGCCACCTGGTAGCCCGTGACATCTGTGAGA
TGTCTGTGCATTTGGCTGGTGTGAGCTTTTTCCTTTGCTCTGACCCAAGCTTTGATAGCTCAGTGTGTCATCACCGGCAGTGGATGC
CTGGGAGCCCCAGACTGGCTCTTCCTGCCCAGGGACCCCCAGACCCACTTCTGCAGGACGCCTTGAAACAGCTGTGGGTGGAAAGCC
AGGTTTTCCCCCATAAGCATCACTCGTTCTGCTGCCTTCCTAGCTTCTGTGCTCTCGGGCCCAGAGCTCCTCCAGGGTGAGGAACCA
GGAGGAGGAGTTGGCTGGGTTGGCTGTTTTCCTAGGTCTTTGATCACAGCTGTTACATGTAGATAGGCTGGGCGCGGTGGCTCATGC
CTGTAATCCCAGCACATTGGGAGGCCGAGGCGGGTGGATTACGAGGTCAGGAGATCGAGACCATCCTGGCCAACATGGTCAAACTCT
GTTTCTACTAAAAATGCAAAAAAAAGAAAAACACAAAAAATGAAAAACAAACAAACAAACAAAAAACTAGCCGGGCATGGTGGTGGCG
TGCGCCTGTAATCCCAGCTATTCAGGAGGCTGAGGCAGGGGAATTGCTTGAATCTGGGAGGCAGAGATTGCAGTGAGCTGAGATTGT
GCCACTGCACTCCAGCCCAGCAACAGAGCAAGACTCTGTCTCAAAAAAAAAAAAAAAGGCTTAAACAATGGGTTCTAAGCAAATAAGTA
TCTGTTTAGTCAGTGGAACGAGGAAACAAAAGTTGATGGAGTTCTCCCCACAATTGGAAGTGTTGTTCCCCACCTAGATGATGGGGC
CTGCAGGGAAGTGATTTTGCACGATTCTCCTCTTCTTGGGAACAGGCCATAGGGTAGCATCCCTTCATTCATTCATCAAGTATTTAT
```

EP 2 204 376 A2

```
TGAGTGCCTACTGTGTGCCAAGTCCTCTGTGAGGTGATGGAGCAGAGAGACAGAAAGGACCCCGACTGTGCCATGAAGCAGCCCCGA
GCCTGGAGGGGCCACTCAGAGGGAGCCCAGGGGCGGCGGCTCTCGGGCTCCAGCCGAGTTTCCTTCAGACCTAGCCCCTTTAGCTGA
CAATCTTAAAGCCTGGTCCCGGAAAACAGGAATAGTGCGAGAGGGATGAAATGTGGGTGAGGAGAGTGAGACAAGGGTGGAGGTAAG
AAAGAGGGATGGAAGAAAAGACGCAGCAAACTTTCCCGTGAGAGAAACGCTCCAGACAGCACCCCAGTTCCCAGCTGAGACTCCCAG
CCACAGTCAGCAAGAACCCACAGGAGGGGAGGCCCACGTCCCTGCTCAGCTCTGCCCTGGCAAGTGGGGTCGGAGATGGGGCATTCC
CTGCTTGGGGTCTCAGTCTCTCCATCTGTAAAACGAGGCTTTGGACTAGGGGAGCTCAGAGATCTGTTTAGAACTAATATTCTGTAA
CCGGTGCAGGGCAAAATAGACGGGGGGCAGGAGCCCAAATTAAGGCCAGTCGAGTATCAGGTGGGGTGTTCCTCTGGCTCCTCCTAT
TACAACAGCAAGAGGGACCCAGGGGTACTGTGGGAGTCCTACCATCTCCTCACTTGTACGAGGTACCTTCTGGCTGCTCTTCCGGCT
GCTCAACACTCAAAGAAGGAAGGTGCTGGGTGTCCTTGTTTGCATATTTTTCTGGCACGGACACATTCTTCTTGGAGTTTCTTGACA
TTAGGGCCACTTGAAATGCTCAAGGTAAGGGCTGCTCTGCCTGGCAGACATGCGTCAGGTGCCTTTGGCTCCTCCCTGGCAAGTCTG
CCTCCAGACGCCCTCAGATCCTGTCCCCATGCCTGGCCATGGGGCTATCTGACCACTGATGCCAGAGGGTCACACTCATCAGTCTAT
ACAGAAGACTTGGAAATGTTTTGGCAACCCTGTGTTAGAAGAGTGGGGTTTCCTAACTGTATGGCTTTGGGTAATTGCTTAAATCCT
CCCAGCCTTAGTTTTTCCATCTGTGGGTTAGGAGTGGTCCTCTCTACCTCCCAGCATCAAATAGGAAACTGTGCAAGAAAATGCTTT
GTAAACAGGAAAGCCCTAAACTGACATCCAGGACACAGTGACTCAGTAGCAAATATATTCTGATATTCAGCCTTTTCCATGGTTACA
GCCTCCTCTGTGCCAAGAAATTCAAGTCACCAAGGGGTTAAAGCAAGCAAGGCACATGGCCGTGATTGTCCATAATTGATGAAATAAA
CCTTAAAGGAGTGACATATAATTAATTGCAATTAATCCAGGCACTGTGACACCACCTTGCTAAAAATACTGTCTACTAACAATTGCT
TTGATCCAGCAGAATGATGTCTGCCCATGAGCCTGGCAAAGTGTCTGCTAATGGAGTGCACATAGTGGGTGTGAAGCTGGCAGGTG
TGGGGGCAGAGGGCGGGAAGGGGGCCCCAATTCTCTCAGACCCTTTGGCAGCAAATACTACATATATAGGGTGATCATATGATTCAT
TGTCTAAACTGGGACACTTTTGAGATTATTATATTAATGATTACCATTCATAGTAATAATTATGCCAGATAACAGGCATACACTGGA
CTCTCCTGGGTAAACCCGAATGTATCGCCACCCTCTGCATAGGCTGTTTTAAGCCCGTGGGAACCCTCGACTGGGAGAGTATTTTCA
CGTTCATTATCTGCTCTTCCTCACAACAACCCTGGAAGGAAGAAAGGATTTTTTAGTCTCATTGAATAGATGTTAAAGATGGTGATG
GTGTCAAACGGTCCAGCTCTAGTCTTTGGCCTGGCTTCCTCGGTGGCTGACCCCATGCAGGTAGAGGACAGCTGGTCAGGACCGGGA
GTTCTGGGGATGTGGAAACAAGTGCAGCTCAGGGGGAAAGAAGTAGCAAATCTGGCTTCAGTGGAGCAGGAACAAGCGACCTCCTCC
GTGAAGAGCTCAGGGTGTAGGTCTCAGCCGGGACTGTGCTGCGGTCCTTACCAATCCTGGGCTTGGAATCAGAAGCTGTGTGTTTCA
AGTCTGGCTCCCACAGTTCAGCTGTGTGATCTTGGCAAATTGTATAATCATTCCGTGCCTCAGTTTCTCATTTGTCAAGTGGGGACA
GTTCCTTCCTGTGAGGATTAAGTGAGAGTTTATATGAAGATGGATGGGTAGACTCAGAGCCTGTCATATAACAGGTACTCAGTAAGT
CCTTGTAGAGTTTGAATCTGTGGGAGTTCCTAGAGGCAACACCTGTAGGGGAACCATGGAGATCCTTCTGCCTTGAAATGGCTCTA
ACTTGAACCAATTAGTGTGGAATGAGCTCCCGCCTTGAGATGGGGCTGCCTCATGTTCTTGGAGAAAGAAATGGAGGAAATAACCAC
ATGGAGGAATTGGGAGGTTCCCTGACCCCCCCTAGACCATGGATAGTCTACTTGGGAGACTCTACTGCATGCCCCGTGATAGGCAGG
ACCAGCACCCAAGGAGTCTTTGCATCTTGCAGTTTCCTTGAAGCCCTGTACAGGCTGGTGGCCCACGTTGGTGTGTGGCTTCCAGCC
TTCCTCCAGGCTCTCAGATGAACATGCTTGGACATGGGCAGGCAGTGGGGCTCGTGTCGTGCCCCATTGCTGGAATTCATGGCACCT
CAAAGTAAAAGGTAGGCAGGTTGGCAGCAGTCTGGTGCCAGAGAGCTAGTGAGAGAGAGAAAGAAATTATACAAGTGCCATGTTTGGT
CTATTAATAGCCTGTGTTTGGTCTCCACCACTTCTATCAACATCGTCCATCACATCCAAACAATTGAAAAACCTGTGCCAAATGTTA
GGAAACTCACGGCTGTGCTGTTTCTGAAAAACAGCATTTCTACTTCTTATGCCGCACTGAGATGCCAATAGTTCCATGCCAACTGGT
TTTTATTTTTCCAGACCATTTCCCTTTATTTATGTTTAAGCTTTTGTATTGTTAAGCCAAAAGAAGATAGAGAACCTGGAGTAGGTA
ATTATTGTTTCTCCTGATTTGTCCTTGAATGAGGCATGGAGGGAAATGTTCACTCCCCAAACCTGAATTGAGTAGCTGCAGAGGGGTG
GAGAGAGCAAGGATCTAAGACAGGGGCCTGTCCTTTCAGACAGTGTTCAAGCTGGTGGATGGGCAGGTGAGGATGTGGCAGAACACCAGC
CCCACCCTTGGAAGGCTTAGGGCATTATCAGCCCTTTCTGTTACTGGTGTGTGGCCTTGGGTAGGCCAGAGTCCCTCTCTGGGCCTT
AATTTGCTTGCCTATAGAATCGGGGAGTCAGACTTGATGGCTTAGAAGGCCTCTTCCAGCTAAGTCGAGTGTTGGGTGGAGGACTCA
CTGTCTCACGTTTGTTGGCAAGGCCGTGGGTGTGAAATGGAAGGAAGGTGATGCTGCCCTCAGAATGCTGGCAACTCCGGGAAGCC
TGGAAGCATGGCTGGGAAACACGGACGAGGCTGTTCTTTATCTGGGAAACTCCAGAACAGCCTCCAGAGATGTCTGTGGAGCCTGCA
GGCGGTTGTTTTTCTCACCATATGTGATTTCTGGGTGTCTTAAAAATTTTCATTTGGCCCAAACATTGCCGGGGTTGGGGGATGATT
TTGCCCAGTGTGGGGATGGAAATGGGTTTGCCTCTGAGCATCAGGTGGGTAGTAGTGGTTTTCTCCATTTTGGAATTCTCTGACGAA
GTTGGAAGGTTCAGCTGGGGCCCTAGTGTTGCAGCAGCTTCTGGTGCAGACAGAGGCAAGGGCTGGAGGGAGGAAAGGGGGAAGGCC
AATTGGAGGGGTGATAAGGCTGAGAGACCCAGGGTGGGGAGTTGTCTGTAGGCCTTGCTTTGTGTCTTTAAAACCCACATACCTCTC
TGTGGCTATCAGGTGATGAAAATCAACATTATTTCAAGTTATAGCCATCTGAAATGAATGTAGCCAGTTCCCTGTAATTAGCTGACC
ACAGCTCCTGCAGAGAATCTGGTGGTGCGTCTTGTCAGCGATGGCTGGGGCAGGATGGGGAAAGGGAAGCCCAGGCCTGG
GACAAGCCCAGCTAGGCTGGTTTGGTTCTAGTCAGCAACCCAGTCTAACAGGGAGCCCACAGCTTTCTGTAGAGCAAAGTGATTTCC
TGCTGTTTTATTCATTTGGGAGCCAGATAGGTTTTACTAAGAGAAAGCAAAACTACTTCAAAGCAAGCCATCCAGAAAGCACAATTA
TTTGTTTCAAATATTCTAGAACATTTAAGAAAATGGGGGTTCCTGCAATCTGGATGAAATATGGGGGCCAGATCCTTGGCAGGTGGA
GCAGTAAATATTTGCTAGGTGAACCCCTCATGGTGATGGCAAATTGACCAGCGTTCTGGGAGGCGCTAGGAGGGTGAATAAAGGTTA
AGTGTTTCACTGTAACTCTGTAACCTGGATGCCAGGACAGATCTGGGGGCTGGGACTAGGAACCTTCAGTATGCTATACAGTTCT
CCTGAGATAAAGGTATGGTGTCAGGACCTTCAGAACCTGATGCATAGTGGCAGGTACGCATTCATCAAAGCCAGAGGAGGCTGTAGG
GACTTCTTGACACCACAAGAGTAATGTAATTGCTAACTGGCCAAGAGCACTCACATTCCACAAGCGAGGAGGCTAAAAGCTCATCA
GGAGCCCATTTTTTTATTGGAGAAGGAAACAGTGACTAAGTGACAGTCTTGGAGGTGACTTCCAGGATAGGGATACAGGCTTTTGGA
CCGGGAGCCCCAACACCTGGGTTCTAAAGTTTTCTCCAAGGCATGCTGTATGATGGGGGCAAGGCAGCCTCAGTGTCATCGTGG
AGAATGAGGGTTGGGGAACTAGCTGATCTCCTGGTGTTCTGTACGTTAATGAGTGTGTGTCCCAGGGAGAGGCTCACTTGTCAAAGT
CATTGACATAGACACCGGGATGCTAAAGTCAGAGCAGGTCTTCACAGAGGCATTTGTTTCACTGGGACCAAGCACTGCAGGCAGATC
TTTGAAAAGAGCCATCCAAAATAGAGTCTCTCCAGGGCTACATTGAGAGGAAGTGAGTTTCCAACCCCTGGAGAAAATCAAGCAGA
GACTGGATGGCCATGTGCCAGAAATCTTAGGAGACTCATGGGGCACTGAACTAGATGGCTTCTAAGCTCCATTGCTTGTGTGTATGA
CTGTCTGTTTCACAGTGTCTCAGAGCTGTGAGGGCAACCCCAGCCTCCTGGCTTCTTTCTTTGTGCCCCAGAACAGCAATTCTCATT
TCTGGCCCGCACATTAGATTCACTGAGATCTTTTTTTAAAAGTTAGTGCAGAGACCTCACTTGCAGGGATTCCAATTTAATTGCTCT
GGGGTGGTGCCAAGCCCCTCAGTTCTACAAGGACCCGGGCTATTGGTGCTTGATGGGCTTCCATCCCACCTCCATCCTCCCTGTAGG
GGTGGGGAGTTACAAACCCTTCTGCTCCCATTCTGCCTTTGTATGGAGCCCAAAGGTTTAAGTCTGTATTTACCGTTCCATATTCAT
TTACCCAAGAAACACTTGTTAAGTGCTTGCTTTGTGTCTGCAAGAATCCTAGGTGGGCCAGCTTTTGGCTACCTGAGGAGCAGCAAG
GACCAAACTGCCTTTAAAAAGGCCTCATCCTGTATGTTTCCACCTCCCGCACAGTCCTGGCACATGGCAAACATGTAATAAAAAGT
TTGATTGAATCGCATGGAATCTTTGCCAATATTGTAAGTGGGGCAGGAAAATAAATTATAAGCAAGCATGCAGTAGAGAATTCATGA
TATGCCAGTTCCCCTCTGCCCAGACCTTATAAAGGAAACCATCTCCTTAATTGGAATTATGTTTGTGGCCTGAAGGGGTGCCCCGTC
TGACTTGATATTTTGGGAGCGTGGTCTCCTAAATGACCTTTTTCTGCCTTCCTTGGGACATCACACAATGGGAGAGCCAGGGCCTGG
GGAGGATCATTTTCTCTTAATTGGTGTCCTGGTCATCATGCTGGCTTCACTAAAAATTAGTAGGCCACCCTCTGCTCAGACCTGAAT
GCCCTTGTTATTAACTGCATGATTGTCCCTCAGACATCAGGTTCCGTCTCCTTCTGGGGAGTGGCAACGTAGAGATCCAGGGAAATGCT
CCAGGGCAACTTGACTTTATGAGTTTTGTGTCGAGACCACTGGCTTCCTTCACGCCACAAGTATTATCTGCCTAGTTTCCCCGTGCC
GGGCCCAGTCTGCAGAGACGGTGAAGACCCCAAGCCCTGAGCTCAGTCTGTTCACCATCTATCACACCACTAAAGCCACACAACTGG
GGGCTTCTTCAGAGAGCAGGTGGGACCGGTAAGGGCAGGGCCTGCAAGGGTGATGTTCTGGCAATTGCCATCCTGTCTTCCTGAGGT
TCCCTCTGGATCAGTGGTGAGAGCACAGGTTTGAATCTAGCCCCCACCTCTTCCTCATCCAGGGACGGTGGGAATGTGACCCTCTCC
```

103

```
AGGCTTCAGATCCCTCATCTCTAGAATGGGGCCATGGGTTGTTCTGAGGACCTAACGAAATTAGCACAGTACCTGGCCTCTGGTAAG
TGAATAATAAATGTTAGCTGTTACTTTCATCACCTCTCTTTAGTTTTGATGAATTTGGAAATTCAGGATGGAAGTGTCTACCTGCTT
GACATATCTCAAAAATTTTGCTCCCTTCCTTCAAATATTCAACGTTCAACTAATATTTATTGACTTACTATGTGCCAGACCAGTGTT
CTAGGCATGGGGCTCTAGGAGTGAACAAATGCAATGTAAAAATTCCCTGCCCTCTGGGGTTGACACTCTGGTGGGGGTGTGAGAACA
ACATCAGTGGGAAAATGGGGTCAGATGGTGAAAGGTGCTCTGGTCACCCCAGCTGGGCAGTGCCAATGGGAGACACCAGGCATGGCC
AGCCTCGGGAAGGGTGTGGGGCCCAGGGCAGACCAGCGGGGTTCCTGGTGCTGAGGTTACCCTGTGTACTATTAAAGCAGGTAATGG
CTCCCAGCTTCGATGGTGGGTATATGGACCCCGTTCAGGGATGGGGGCTGAGGAGGGCACAGGAGGACTGAAGACAGGGCTCTGAGG
AGCCACCTGCTCTCGGGTCTAGGTCAGACCCCGCATCCTGATGCGGGTCAGCTGGCTCATGATGCCTGGCTGGCTCCTGGGACTGAG
AGGAGAGTGTGGATGGTCTTGTCTCAGCTTTCCGCCCACTGGGGATCCACCTCAAGCATCCCCTGGCTGTGGGGTTGAGGGGTGGGC
CCAGAACAGAAGCTTCATGTAAGAAGGTGACTGGGCTGGAGAAAAGGTGGCAGTGAGGTTTAGCCCCATCTCCAGCCCCTGGGACCT
GTGGTAAAGATGTCCTCACCCACACAGTGGGCTCAGGCAGGCAGGAAAGGTTCCTGGAGCTTGGCCTCTAATATCCATTCTTGAGCT
CTGCCGTGGAGAGAGCTGGTCACTCGAGCGCAAAGGAACCACTTGTGGGTTTTCTCCCTCAGCCTGGGCTTCCTGCCCATTCCTGGC
TGTCCCAGCCTGATGTGTCCTCCAGGGCCCCCTGACCTGACCCTTGGCCCATCTCTTCTTGACCTTGCACTGTGGGCAGGACAGAGA
GGCCAGGGAGAGTGTCCAAGAGGGACAGGATGGGATGTCAAGGTGACGGGGCGATGATGGTGCAGAGGGCAGCCCTTGGGATGAGCT
CCTGGGAGCTCCTGGATGGGATTCAGGCTCAGCAGGTTGGGGTTGGAGCGGGTGGGGAATCCCTGGCATTTGGCAATTGCCCAGCAC
AGTGCCTGGGCATGGTAAGTGCTCAGTGAATATATGTTGTATGAATGCACAAGTGAATGAATGTTTGGCTATGCGGATTGCAAAGGT
GTCTTTCTGAAATGCCTCAGGGATATCTTTATAAATTATTGCAGCCATAAAATGCTGATATTGGGGGTGCAGTGAGCAGCTCCGGAC
TGAGGATCAGCTGCATTTACCTGCACTGACATCTGTATGGCAAGAGAAGGGGTGTTTGACTACAAGGGAATGCATGCTTCTCTTTGT
GTGAAAACAGAATGAGATTTTTTCGTATGCATGATCTCATCTCATCCCCTAACAGCTGTGAGAAAAGTAAGGGTAGTTCTTGTTTGCA
GAGAAGAAACCAGAGGCCCAGAGAGGTTAAAGGACCGATCCAGTGTCACACAGCTAGTTAGTAGCAAAGACATGACTTGAACTCTGG
CCTTTTGGTTTCAAATCCTATGTTATTTAGAAAGAGACAAGGAACATCTTCGTTAGCCCCCCATGACTAAAAATAGGAAGCTTGCC
TTGTTGTGTTTGAGGAGAAGATGGTGTGAGTGGGTTTTGTGGCTGACCGTTTCACACCCAACATTTCATCACAGAAATGCCTGGGAA
AAGAAAACTCCTAAGAAAAAAAAAAAAGGAATGAGTGTTGACATGTCCCTATGACCTGTTTGTCATGGCATGTGTGCTCGGCCGCC
TTGGTCATCAGGCTTAACTGAACTTCGGAGAAGGTTCTGTCCAGGAGTCTCATTTTACTGGGGGGTCGAGGCTTAGGAGAAGGAAGCA
ACTTGTCCAGGCCAGGCATTCACTATGGAGGAGGCTGAGACTCCTGCGCCCTCGGATGCACACCCTGGGCCTCTGTTCCAGAGCTGT
CTTGGCTAGCAAGTATCAGGCCCTTCTCCCTTCTCAGGCTGGTCAGAGGTCCAGCTGCCCTCTGGAGTCCCCAGAGTCTCCCTTCCC
ACTGTGACAGCATCGGCTGTCTCTCCCGGTCTCAGGTTGGACAGTCCTGGGAGAGAGAGGGTCTGAGTGACTTCATTCTGTCACTTC
ATTCATTAGTGACTGAAACAGGATTCTGTTAGCTGACTTCTGGTAGGACCCAGGTTGGGAAGCGTGAATCGTGAGGATGCTGGGAGGGCC
AACAGCTGCAATTATTATTATTATTGTTGTTGTTATTATTATTATTATTATTATTGAGACAGAGTCTTGCTCTGTCTCCCAGGCTGGAGT
GCAGTGGTGTGATCTCGGTTCACTGCAACTTCTGCCTCCTGGGTTCAAGCCATTCTCCTGCCTCAGCCTCCTGAGAAGCTGAGACTA
CAGGCTTGCACCACCATGCCCAGCTAATTTTTGTATTTTTAGTAGAGATGGGGTTTCACCATGATGGCCAGGCCGGTCTTGAGCTCC
CGACCTCAAGTGATCCACCTGCCTTGGCCTCCCAAAGTGTTGGACTTACAGGCGTGAGCCACCACGCCTGGGCTTGCAATTAAATTA
GGGTGCTGCACCTGGGAGGAGGAGGCTGTGCTAAGATAGGAGCTGAGGATGATGGGTAAGAATGAAAGATCCCAAGAACTGGACAGT
GTGAGATTGAGGAGTAGGAGGAAGGCCAGGGCTTGGTGCTGATGTGGAGGGGAAAGAAAGCCTCAGGTCTCCATGGAGAACAGCGACCAGT
GATGCGGTCTTCCCTTTAGCGGAAGAGGGGCTGGTTTAATTTGCATAGCATTAATTAGCAAGAGTATCTAATTAGGACAACCATTAC
AGAACCACAGTCATAAGCCGATGTTGAATATCAATCAATTTTGGAGCCTCTCCATGAAATTGAAGGAAAAGTTGACCTTATTAGATA
GAAAAGATTTCTAAATCAATTATTTCCCAGATTGAATGCACAAAACATAGTGATGTCAGGCAGGCACATCAGACCTTAATTCAGAAA
TTATGAACTTCAGATACTATCACCTTTTCCCTTTGGGGGTTTCAGCCAGTGACCAGAAATCGTGAATGGACCACATATGTGTATAAAC
ACACACACACACAACCATACACATATACTCTTTGTGGGTAACAGCTTGGTTGGGGAAGGAGCCTGCGCATCATTATCTCAGTTAAGC
TAATATTTCAGTTTGGTTCAGTGCCCCTCCAGGTCTCTGTCTGTAAGCATAGCTTTCTCTTTTTTGCGATGACCCAAACAATAAAAT
ACATTTAGAACTTCAAAGTCTCTGGGAAGATATACCATAATCATTACAGTTCTGGCCTGTTTTTGGAAGCTACAAGGCAGGCCAGGA
GAGCAATTTATGTGGCTGGCTCAGAAGCTGGGGAACACACTCCATCACCCTCTGCACAGCCTCAGGGGAGGAAAGGCTGGAACAGCT
CTAAGAAGCCTCGCTGCAGATATTGGAGGGCCAGCAAGGCAGGGCGCCAGAGCAGGTGGGGTGCTGGGACACAGGCGGGGCAATGG
AACCTGGAGGCAGACAGATCTGGGTGTAAAACCTGGCTTTTGCAAATCCTGGCTTTGCCATGGTGAGAAGTTAGGTAACATGCCCTT
GGACAAGTTACCTAACTTCTGAGAGCTTAAGCTTTCTCCTCTGTAAGAGTGGGTTGACAATGCCATCCTTGCATGGTAGGCCCCTAT
AGATGTGCTTTATTACAGATTATTGTCATCAATGAGAAATCACAGATCTGTGAAGGGCTGGCTCGTGGCCTGGAGCAGAACAGGGAC
CACATGGATGGAGGCAGCTCTTAGGAATTCTATTGACATATTTTCAGTCTTTTTCTACACTAAAAAAGCTTAAAAATGAAATAATTG
GAAATATACTACATATCGTTTTGGGGTCTGAGTTTATCACTTAATATTCTATCATCAACCATTCTCCATGTCTTCAGATATTGTCTG
CAGAAGTGTTTTTTGGTTTTTTTTTGTTTTTGTTTTTGTTTTTGTTTTTGTTTTTTTTTAAGATGGAGTCTCGCTCTTCACC
CAGGCTGGAGTGCAGTGGCACTATCTCGGCTCACTGCAAGCTCCGCCTCCCGGGTTCATGCCGTTCTCCTGCCTCAGTCTCCCGAGT
AGCTGGGACTACAGGCGCCTGCCACCACGTCCCGCTAATTTTTTGTGTTTTTAGTAGAGATGAGGTTTCACTGTCTCACCCAGGATG
GTCTCGATCTCCTGACCTCGTGATCTGCCCACCTCGGCCCTCCCAAAGTGCTGGGATTACGGCCGTGTGCAGAAGTGTTTTTAGTGGC
TGTGTAGTGTTCCATCACTGCCTGATCTCAAATCTACTTAACCACTTGAAAATTGTGGGCATTTAGGAATAAATGACTTTAAAACA
TGACTCTCAGCTGGGCGCGGTGGCTCACGCCTGTAATCCCAGCACTTTGGGAGGCCGAGGCGGGTGGATCACTTGAGGCCAGGAGAT
CGAGACCATCCTGGCTAACACGGTGAAACCCCATCTCTACTAAAAATACAAAAAAATTAGCCGGGTGTAGTGGCGGGCGCCTGTAGT
CCCAGCTGCTGGGGAGACTGAGGCAGGAGAATGGCATGAACCCGGCAGGCAGAGCTTGCAGTGAGCTGAGATCGTGCCACTGCACTC
CAGTCTGGGAGACAGAGCAAGACTCTGTCTCAAAAAAAAAAAAAAAAAAAAAAATGATTCTCATCAAATGGGATATAATTCCTAAGCATTC
CTGTGTGTTAAGGATTGTTACTCTGTGTTGAAAAGTTCTGAGTCACGGGGCAGAGGGTTGACCAGTTGACCGCTGGATTCCCTCTG
TTCTACAAGCCCCTTATTGGTCCTCAGATAGGAGTGAGTGGGGCAATGCGTGTGGCAGGTACCCTATCCTGGGATGTATGGCAGCC
CGGTGTCACTGCCATCCCCCAAGGAACCATATCAATTTCAAGCTTCACTGATGTTCAGGCAGCAAAGAGAAGGATGCTAATTCAACAT
TTTACCAAATAGAAAACATTCCTAGTTCAGCCAGACTTCTTTGACATGTTTTCCAGCTATTAAAAGCGGTTGGTTAATTTTTTTTAT
TGACATGCGATGGAGAATGCAAAGAAATCTGTAAGATATCGAGAGTCCCTGTTGATCCAGCACACAGCTTGGTCATCCCACGGGGCT
GGAGCTGGCCAGGGAGGTTGTACTGGCCAAAACCAAGAACGCAGGTGGGAGTTGCCTGTTCACTGTAAAGGTTTTGAGCTTTAGGACAA
AAGGAAGAGGCCCAAAGGAGAGAGCATCTGGCCCCTTTCCCTTCACTGCTTCCTTTAAAATGATGGAAATAGACTTGGATTCTGATG
TCAATTCATTGCTGCCAAAAGAAATAAATAAGGGGCTGAAATGTGCAGCTGATCCCAAATCCTTCCCGGGGAGGTGTGATGCTCCCA
GATGCTTTCAGACCCCTTTGAAAAGAAAGTTATGGTCATTTCTGGATGGAAAACATTGTGAGCTGTCCCCAGTTGATCCCCAGTTGA
TTGATGTAGGGAGGTTTGTTTTACCCTAATACAGTCTGAGTGTTCCTGAATTCACCATTTGCTCTTTTCAGCCCCATCTGGGTCTTG
TGGTTGGGTAGCATTTCAGGCACCAGGAAGTGCTTGTGAAGATCAAAATATTTTTGAGGGCCAGCACTGTGTTCTGTTCTCTTCTCTGT
GCTTATGTTGGACCAGAACAGCACCAGCCCTCCTTAGGCCCCCATACATGTGTGCTGAGTAAATGATTGCAGGCTGCTCTTATGGAA
CTGTGGAACATGAAGAGGCTAAAAGAAGACTGGCTTATGTGTAGATCATCTTATGCATAGCAAATAATGGAGACCTTTTCCCATAGT
TGGACGACCCCATGCAATGTAGAAAGTCAGAGTTAGTGAATAAGCCAGATCAGAAGCTTCTGGTGTATATCACTGTATATATAGAAT
CTAGCACACTGTTGGATATCTAGCAAGTGCTCAGCAAATGAGAAGTGGTTGAAATTTTTGTGAAAGAGTGAATGAATTAGTTACTTT
ATTAGTTTTCTTTGTATCTCCCAGTGGGCCTCAAGAACTGTACCAATTAAATGAATGGGGAGATTAATAGACATCACAGGGAGAGCACTT
TCATTCCATCTCAGCACACAGAGTGGGGCCGAGAGCATGGACCTGGGCACTTGGAAGTCCTGGGTTCGAGTCCCCAAGCCCTCCTCTC
ATTCCTTGTGGTTGCCATATTGGACATACCGCCTCACCCCACAGATCTCAGCTGCTCCTCACATCTGCCTCTGAAAGCTGTCTCTGAC
CCATCCACAGTGGCCTCCCTCCTTTGAGCTCCCCTAGCTCTACTGTAGGTTTCACTCTGGCAATTGATCATACTCTACCTTACCTTG
```

```
TTGTCTTAAGATGTCATTTAGGTCTATTTAAATGGATGCTTCCCATGTTTCTAAGTTGTTTCCCATTGGAGAGTGTGCCTGAGGGCT
TGTGGGCAGTGCTCTGTACTGCGAGTCAGAGGCCTTGACTTTGAATTCTGGCACAAGCCACCCACCCTCCTCTGGGCCTCAGCCTGT
AATATCTATAAGGTAAAAGGCCGAGGGCTGTTGTGAGGATGGATGAGAACAAATACGTGAAACCACCTTGTAAACTGTAAGGTGCCT
TCCAGAAATCAGGCATCATTCTTCTAATGACCAGCTCCCTGAGGAAGGGAATGTTTCTTTTGTGGCGTGCACCACTCAGGACCAAGT
GCAGGGCCGCCATACACACAGTCACTGCTTGGAAATGCTTCCTGGATCTCATCTGCTGCCTTTGCTCAGGGCTGTGTTCCCGCCTTC
CTTTGGAACTAGGAGCTATCTCTACTTCCAGCTTCGAAATGCCCAGAGGTGCACCAAGTTCCAACCCAGACACCCTGATTTGTAGAG
GATGCAAAGTAAATGATAAGGGTAGTTAACACTTACTTGGCCAGGCACTGTTCTGGGCACTTTGCATACATTAACTCCTTTATTTTC
ACAACAGCCCTGTGAGGTAGTCCAGAATATTATCTCCATCTGACACAGGAGGCATGCTGGTGCAGAGAGGTGGAGTGGGCGTTCCAA
GGTCAGGTAGCCCTAAATGGCTGTCGCAGGGCTGGGACCCAGCAGTCTGGCCCCGGAGGTCATGCTCTTAACCACACTTCTCGGTTT
CTCTAAGCTGCCCTGGGACTGAAAGGCTGCAGTGGAGGCCACGGGGCTCACCTCCCCCACCTAATCCCTAGCAGGAGGGACCATCAG
GGTACTTCTCCTGCCAGGACCCCTCTCTGCTCCTGTGCTTCCTGGCTTCTCTCTCCAGTAGCTCCTCTGCCGTCCCTGCCTCCCTCC
CCTGCCCTTGGCCTAAGCCAGGAGATGAGAGCAGCTGCCGGGACATTCAAATTCATTCCTGAAAGAATTCTGCCTCCGAAATCCTCT
GGTTTGGGTTTAAGAGAAAACAGAGGCCCAAGCTGGCGTAATATTGATGCCCAAACCATGTAATAGCGGCACAACAAAGAATGACGT
TCATATGAGTTATGAGCATGGAAGCACAAATTCTAAATAACACATCAGCAAACTGAATCTAGCCGTGTATAACAAACATAATACATC
ATGCCCAAGTAGCGTTCATCCCAGGAATGCAAATACGACTTAGCCCGAGAAAACAGAGGCTCAAAGGGGGCAGCTGCTGAGACAGAG
CCGGCCCGGGCACAGCCCAAGGCTTTTGTGGGGCTCAGCCTGACTTTGTGCTACACCTGCTCCTAGGAGGGGCTGGTAACAGTCCCT
CCTGTGTCCCCACCCCTTCTCTTTTACACAGCCGCAGAATAAAGTGAGACGCTCCTTTCCCCGCACTCCCTAATTCTCTCTCTCCAT
TGTTCCTGCAGGACTCCATGTTTCGTTGGCCCTGAAATGCCTTATCAGTCTGTCCACCATCATCCTTTTGGGCTTGATCATCGCCT
ACCACACACGTGAAGTCCAGGTAGGTGCCCCCACCTCACCCCTTCATGCAGCGGCTGATCCTTTGGCCTTGCTCTGAGCCGAGCCCA
TCCCCACCCCCAAAACCCACTCCCTTAGCAGCTACTTGGGTGATTTTACAAGACGGTGCCCAAGATCGTGGCTCTTCTCTTCTGGTT
TGGACCCAAATGGGAGAGGAAACTGCGAGGCCGCCCTGTGGCTTTGAATAACTGTATTAAGTTGTCTTCAAAGTGGGGATCCCAGAGG
CAAACATAGGGCTTCATGTTGAGCGAGGCTGCCGTATTCGGGACCCTCACTGAGCACCATCGGACCGGGAGAGCCCCTGATGCATTG
CTCTGCCTTTTGTGTCTCTTGTCCTTTCTGAACTTGGGCCACTCAGTTCATCCCTTCAGTGAGGCAGACCTGCCTTGTGTACCCCCA
CATATGCTGCACCAAACTCCACCAAGGAGTCTGCTTTTTGTGAATGGTGTGGTCTTCACCCTTGTGGGTCTAGGCTGGGGGTGTCAT
TTTCTAGACAGAGTAAGAGTCTGCTGTGGCCGAGAAGGCCTCTGTTTCTTGCTGCCCAGGAGTGACTTGGTACCCAGAGAAACATTG
GCTGGGCCTCCAGGGTGCACTTCCAGTTTGGCTGACATAGGGAGGCATGGTTCAACCCGGGCCAGGAGGGTCAGACAGGAGGAAACAG
ATCTGAAGCATCTTCTGGTGACAATGAGCTTCCCCCAGCCCCGGCCCTTCAGGCTGACCTGCACTGCTGTGGACCTGAGAAGTTCT
AAACTATCCAGATGTTTTTCCTCATGTGCATGTGAATCTGGAAACCATCCCGGTCCTTTGGCTGGAGGAGCACAGATAGATTCACAT
CTGGGCAGCAGGCGAGAGATTGACGAAGACCAGAGCTCAGAGTCAGAAAAACTGGGGTCAAGTCTCGGCCATGACCTTTTTTCTTGG
TGTGTCTTTGGCCAGCCCTGTGACCTTCCTGGGTCTCGATCTTCTCATCTAGAACATGGGGCCTTTATTCCAGCTCTGCTGAGCTGT
TGTGAGGATGGGATGAGGAGCGCATGGGAGAGTGCTTTGTGAAGCGCTATTTAACAACACCGTTCAAATCCAGTCCCATTTGTTACT
CGGCATTTTCAGGGTGGAGCAGCAGTGCGGACGCAGGTGGGAGTGCCTCCTCCTCTTAGACGAGCCTTGGCTTTTTGTCTGAAGGTCA
GGGGCCACTGTGGCTGGCTGGGACATGGAAGCCTTGGCTCCAGGCCACCTTGGCAGGGCTGCATTCTGTCAATGACTAGAGGATACT
GACCTGGATGCTGGGACTCTTCAGCCTCTAAAGGGTTGCAGATGCTTGGACAGGGGCTGTGCCTGTGCCAGATGCCACCAGGCAGCT
TCCTTCATGGAGTCTGAATTGGACAGAGTGGTGGACTTTAACTCAGACCATTGTCCAGTCAGCCCCAGAAAGGAGCCAGGAGTACAG
GCAGGGAGGACTAGGGCCACGGAATCGAGACAGGACAGAAGTTGACAGTCTGGGCTCCGGGCCTGGTTCCACCCCACCCAAAGCCAG
CCGCGGGATCCAGGCAGTCCCTCAGTGTTCATGTTCTCATCTGTAGGGAGACTGTATTACTATGTGTCACCCTGACCTGTGTGGGGA
GGTGTCACACACAACAAAGAAAAAAGGCAGAACCATAAAACTCAGTTATGACCCAGGCTTGAGGAAGGGGAAGAAGAGGAGCTGTTC
TCAGCCTCAACCCCACACTATGGTGGTGCTAGGCCTGATTACTTCCTCTTTCTGCTCCAGGCATTGGGGTGGGTAGAGCCAGGTGTC
CCTCCCTCTGATGCTCCTGAGGTCCTTGTTCCCAGTCCAGGGCCTGCAGCCCTAGCTCTCAGCCTGTTCTGGTCCAGCTGCAGCTTG
ACTCAGTTCCGGCCTGGTGGCAGGGTCATGTTGCTGACAGCCCAGAACCTGGCTGGGTACCCATTGTCCCATGTCCAGCTCATGGCC
AATCTCTGTTTCAGCCCTAATCATGGGCAGTAGATGATGCTATTGGGGGTCATTCATTTTGGCTGACACAATTTGGAGAAACAAAG
TGAAATCAGTTGTGTTGAATCCTGACTCTGGGGCTCAGTTTCCCATCTGAAGTATGGGAACCACATCACCCACTTTGCTGAACTCT
TGCAGGGGTTGGAAACAGTGTAGAGTACAGAGTGGGCTTTCACAGAATGGAAACTTTGGTCCCTTTCCTGTGACCTTTGTCCCACTG
CTTCTAGCTGGGCCTTGCACTTACCAGCTGGGTCCACCAGGTGGTCACTATCACGACTGCCTGGTACTGGCAGGTGGCTGGGCTTCC
TCCTGCTCTGGCCCAGCCCTGCCTGTACCCTGAGCTCCCACCCAGCCCCTTATCCTCCCTGCAGCCTCCGTCACTTGAGGAAAG
TCACCAAAACCGGCTTCCTCTGTGCCTCTTACCTTTCCAGTAAAGTTCTTTCTGTGCCCAGCTAGACCTTCTCTTGCCAGAGGGAAA
TGGGAACAGTTTGGGCCCAGAGAGTCAGTGGGAGGGAGGTTCCTACGGAGCGCCCTTGTATATACCGCACTGGGCCATATTGTAGTT
TTCTGTTGGGTGTCTTTCTTCCCTACCTCAGGGACAGTTATCTTTGCATTAATCTTTGCATGTCCAGAGACTTATAGTGTCTGACAG
TATACTCTTCTCGGCAGAGTATAGCACGTTTGTAGCACGTTCTTTGAATGGAAAGCATGCTGAGTTCAAGGTAACTCTTTTCCAAAG
CTGGTCGTACCTTGACTTAGCTTTTTGGTCTCGGGCCTCAGTTTCCTCATCTATTACTTAGCCCCCCAATCTTGTAATTCTCTGATT
TAGATCTGGCTGCTCAGCCAGCTGCGGAACCTGCTTCCTGCGAGTGGAGGAAGGAGTTATCCCAAGTGTGACTGATGAAGTTATGCA
GAGGGGAGTGCTTAGCAAGCACCCTTCCACAGGCAAAGGCCAGCTGCTGCTCACCTGTTGCGAAGCAGGCAGGGCAGGGGGTGGCTG
CCGCATCTCCCCTTTGGGTGATGTCAGGACCTTTGCTAGGAATCTGCTCACAGCTTTCCTCATCCACAGTGGTTCCTGATCACCTAG
CTAAATTTTAGCAGGGACCAGATGATCATGTCCCCGTCTTCCCCAGCCTTGCCCCAGAAGCTCACACCCAGGACCCAGGCCCTGCCC
AGCACACATTGTGAGGCTGGGGGAGTACCTTTTAGAACAGTATTCTTACTTGTTCATTTAGATGGCTTGAGTTTTTCAGGAAGAAGAA
TTCTAAAACACTGTTGGGTAACTCTCACCCAAGCTGGGTAATTTATAAGGAACATAAATGTATTTCTCACAGTTCTGGAGATCGGGA
AGTCCAAGATTAGGGTGCCAGCAGCTTCAGTTGTCTGGTGAGGGCTGCTCTCTGCTTCCAAGACGGCGCTACGTTGCTGCATCCTCA
GGCAGGGAAGGCAGGAGGCTGAGGGACTGAACACTGCATGGGGCCTCTTTGATACAGGCCTCAATCCCATTCACAAGGGAGGAGCCC
TCATGGCCTCAACATCTCTGAAAGGCCCCATCTCTTGGCACTACCACATTGGCAACATCTGAATTTTGGAGGGGACACGTTTAAACT
GTAGTGTCCACCTAGTGCAGGAACCCCAGGGCCTCTGGTGTGTGCTACTGTGACAGGGACTGCTGGCCCCCCCTGGGAGGAGGCTGCCCTA
CCAATGTCTTTAGAAAGCCCGCCCTTCTGCCTGGAGAAGAGGCAGCCTCCCTGGAACTTGCCCCCTGGCCTGGTTCTGCTTCTGGAGCA
GGGAAAAGAGGGGGTTGGTTTCCCGCATGGTAGCCTCCATTGTTGGAAGATGGCACCCCACCCCATCTCTCCTCAGTCTCCCTCTCT
GTTGGCGTTTTGGAGCACGGACCTTTCAAAGACTCAGGCACCTCTTTTCCTCAGGCCAGTGTGTCCGGGAGAGAAATGAAAACACAA
GAAGCAATGCATTTTGTTTATTTACCAAAACTCTGACTGAAAACCATGCTCTCCCTTGGAGAGTCCATTTACTTCTGTGGTTTAGCT
CTCACCAACAGCTCTCAGACCGTGCTAATCCCCTCCCAGGCCTGCACGTTTTACATGCCACGGCAGCCCCTGCCTCCACA
CACAAAACACTGAACTCCTCTTGCTCCCCAGTATCCTCTCCTCCTTTGCCCTTGATGACCCACCAAGTGCAGTCATTGGTTCAGCAT
CTATTTAATAATGTTCTTCTAGGTGCAGAGCCCAGGACAGGAGCCCAGTTCTCTAGATGAGAAACTCAGAATTCACCATTGCTTCTC
CCAGGGGTCATCAGCCTTGAGTGAATCTATGGCTCTAATGAATGGCCCTGGTGCCATCCTGGAAGGCAGGCTAGCAGGCAGAATCAT
AAAACTCAGTTATGACCCAGGCTTGAGGAACGTTCTCTACTGAGAATGTAGACTTCAGAGCAGCCCAGCTTGGCGCAAATCATGCTG
TTTGTCACATTTAGTGTGTAGGTGGCCTTTAGCAAGTTACTTAATCCTTCTTAGCTGTCTATTTCCTTTCTGTAAAATAGGACTAATAAC
CATACTGTGGAGGCCACAGCAACTCCATCTTGGATGCTGATCCTCCATATTGACTTCCGATTAACCCAGTTTCGGGAAAGCCTCTAA
GATTTCTCCTTTATCTACTGTTTACTGTAAGTCCTGCCCTTAGGCAAATTGCTTCTGAAGCACATATACCCCTTTCCTCTGGTATTT
AATCCCTGGGACTAGGGGGTCGTGGTGCAGGATCCACCATCTTGTCTTACTGCTGCCAGAGACTGTGGCTTTTGTTCATAAGTCCCT
ATTAAATGCTTCTTTCTGAGAAACTGGATATGTCAGCCCCTTTCTTCAGCCTTTCAGCTTCCTCAGCCTTTGGGGGTAGGTTTGAAT
ATGCCTACCCACCCGGAACCAGTACCTACCAGAAAGGGTTATTGTACAGATTAAATTAGATAATATATGCAGAACTCCTTAGCTTAA
```

```
GTAAGAATGTGTTTGTCTGGCTGACAGAGTGGAAAGGATACCCCAGACAGAGGTCTCAGAGTAAACAAAGGCTCTGAGATGGGGAAC
TGCAGAGCACACTGGGAATGTCTCAAGGACAGGGTCCTGGGTAGAGGGGCAAGTTCACTATGGAGGGTGGGAAGGACCCGTAGGGAC
TTGTTTGCCTTGCTGAAGGGTTTTGGAATGTCCTGTAGGCAGCAGCAGAAACTATGAGAAGGTTTCAAGCTGGGGACAGAGATGATC
AGATTTACATTTTAGACAGCACTCTTCATCTGCAGCATGCAAATATAGGAAAGGCCAATTTCAGAGGCAGGAGGACAGTGGGAAGCA
CGTCATCATGGGCCAGGTGAGGGGTGTTGATGCCCAGAGCTGGGGCAGGAGTGAGGAAGGGAGGGATGGGAGGGATCCAGTACCCCTG
GGAGGAAGTGCCTGGCTCAGAGCCCCAGGCATGGAATAAGGGGTGGGGTGCAGACACATTCACATCCGGAGGCGTGGAGCAGAGCCT
GGCATCCTTAATAAAGTTGCAGGGATGGGAGGTGCCGTCCCTACATCCAGGCCAATGCCGGGCACGTAGCAGGTAGTCAGTGAATGT
TTATTGAATTAAACTGAAGAGACTACTGTGACCCAGGATGGGATACAGAGCAGTGGCAGAGTTCATATAATCCAGTCCCAGAATGGG
AGCTTCAGAGATCATTAATTTCAAATTGTGGTTCTTGGCCATATTCAAAGTAAAAGGCATCAATCAGATCAATGGGGTGGAGATCCA
CTCTTGCCTGGGGATATCAATCCCCTCAAGATCTAGCGTTGGTCTCAGATGTTTCCAGTCTTTTTCAAACTCAACAGAAACGTAACG
GTAGTAACAAACTATGGGCCTTCAACCTCATGAATTTGAAATACAAGGGACCAAAAAAAGGGGAGGCTTTTAATTTATTAGGTATAT
AAAAGTGGATATTTTCATTAAATACCAATAGAGTTCTGAGCAGACAGGGAAATTACTTGAAATTACAGGACATAAATTATAGGAAGC
CTCACTTCCTGTGGCATAATTGCTCTGTGGATGGGAGTGTCCTCCTGGGTGCGTGTAATGTGCTGTCACAGGGACATGGGTGTCTGT
AATGCAGGAGGTGTTGCTGGCCCAGGTGCTTTGGGGTCCCACCGCTAAATGAGTTAGTGGTGTCTTCCCCTGAAAGAACAGACAGGA
CAACTCACGTTGCGTCTTGCTCTGTGCCCTCACCCTCCCTTCTCCCTGTAAAAGAAAGCAGGCTCAAGTCACATCCTGAATGTAACC
TGTAAGGTGTCCTGGCACTGAGAGACAAGGTATAATAGCAGAGCACGTACTGACCTCTCTAAATTTTCCAACCCTAGTACAGTCTAT
AAAAATGGTGAGTCCATTCATTCGTTTACTTATTCAACAAACATTTATTGTAGGACTACTTTGTGCTAGGCATTGAGAATCCAGAAA
TAAAGCAGACCTGATTCCTGCCTTTGGAAATTGGTTTGTCCTAGACCTTTATCCACTGTGACCCAGGGTCATAAGTGCCATAATATG
AGGGTCTGTGAAACACCAAGTGGCCACGGAGGAGGGAGTGATGCATTTCTCCCAGAGAGGCTGGGCAGGTTTCACGCGGAGATGATG
CTGTGCCCTGCATTCGCCAGGAGCAGAGACCTTGTATGCTCAGGACAAAGCTGGCCCAGAGCGTTTCAGGAGAGGAAAAAAAAGAG
AAGGAAGCTGTGCCTGTCCTCCGAAGGGAGGTGGAGGCTGCCCAAACCCCCCAGGTAGGGGCTAGAGTCCCCATCCTTCTTCAGACA
CCTGGTAGAGGTTTCCTAAGGGTGTCTCTGGCTGGTTGGTTGATGAATTAGAAATACCTTCATTGAACCTCCGAGATCTTGAGACCC
CCAGACAGACTTCTCAGCCTGGCTAGGTTTGAGCATGTCAGCTCCTTAACTCAGGTGTGATGAGCAGATGGAAGGAGGGAGCTCGGG
CCACTCGTACTGAAGGGGAGGAGACCTTGACAAGGAAATTCCAGATGGGGGCCTCCTCTGCCAGGTTTACATCCCTCTCCTGTCCCA
CTTTCATGAGTGTGCCCCTGGATGATCACAGCACACAAGGGAAATTGAAAATGGGGAATGTGTTCCAGAGAACAGCGACTGGTGATG
GGGATTCACTGGAGCGCTGCTCTTCTTTGGGCCTCGGCCTCCTGCCTGGCCCAGCTGTGCACAGTGGGGCTAGAAAGAAAGGGAGAA
ATAGAGAGGAGCTACACCACCAACTGGGCCCGAGAAGTCAGAACACCATGGAATAAATCCTCGTCAGACTACACTTTGAAGACAG
AAAGCTCAGAGCTGATACATTAGCAAGCGCCTCAGAGACGATGGTGTGCCACGTCTTGTCTAGCGCTTTGTATTTTTTAGAGGGTT
TTCACATCTATGAGGCCTTGGGCCTGGCCGCCTTCACCTTTTTGCCTTTTAGGTTTCAGCTAGATATTTCCTCCTCCAGGAACTCT
CTCTGGCCATCGTCCCCCTTCCCTGACCCCAGGGTGCCCCTTCTAGGAGTGGACCTAGCACCAGCTCTTATAGCAGCGGCTCTTACT
TGTCCCTGCGTGTCTTATGTTGGTTGTGAATACTATTTCTTGCCTGCAACTCCCTTCTCTGACTGTGGGCTCCATGGCAGTGGGGAC
TGTATTGGTTTCACTGTTGGACCCCCAAAGGCATGAGAGTGCCTAGCACATAGTAGGTGCTATTCAATGAGTGTAGGCTGAATAAAT
GAATGAATGAATGACAAAACTGGGGTAGGCATGGCAGATTTACTCCCTCCTCATTCTAAGGAGAAAGTAGAGCCTCTAACAGGCTGA
GAGCCTGACCTGAAGCCCACAGACCTGGCATCTGGCCCGTGCTTTCTGCATCCTAAGGGGCTGCGGGTCCCAGCAGGTTGCCCAGTG
AGCTGGCCATGCCCACCATGGCCCTCCCTGCCACTCTCTGGGAGGCCCACCTCGATCCCAGCACCTTTTCACTTGGATCGGCCGCTG
GCCATGGATTGCCATGGTTACTACACCAGCCCTGGCAGTCTCTGTGTGACATTTTGGTTTTATTTGCTAAACATTTAAACACTTGGG
CTTAACCCTTTCTGCCTCAGAGGGGGCTTTCCTGCCCCTGCCCCTCAGGTGTCCCTCAGGTGTGAGGGCCCCCGGCTGCTCTCCCTGGCCCC
TGGCTGGCGTCTCCGATGGGGTGGGTGTGCTCGTGGTGGCAGCAAGGCTTGTTTCTCGGCTGCCTGTATTGACTTTCTGTTTTGTCT
CTGGGGAATGTCATCCTGGATCATGCTCTCACTGGGTGGTGCTGTGTCCATTAACAGGTGTGAAAAGCCCTGCTCCGGGCATGTTAA
GGCTTGACCTCCCCGTCAGCTGGGGTTCTGTTGACTTCCAGGCAAACGCCTCTTGCCAAGTCCTCCAGGAGCTTCCTTAGCTCCAGG
AGTTCAGGACGGGGTGGGGAGGGGTCATCTGCCCTTGAACCCTGGCAGGAGGACAGAGGGGAGACTGGTTTCGGGAAGAGTCCGTGT
TTGTGAATGGGAAGGCGGTGCGTCGCCTGGCCTCCCACGCCTCCTCCCCTGCGGGCTTAAGATGAAGCCTCCCAGGAAGAGGAGGCG
GCCCGGGAGGCTGAATCCACCCAGCTATGTGTTACCTTGGAAACCACACACTTGTCCTCGCCCTAGGCCATGTGAAGTGAGGGGGCC
GTGGACACGCTTCTCAGGAAGCTTCCAGTGTAACTCAGGATACAAAAAATCACAGTCCCAGCATGGGCAGCTGAGAGAATGAAAATG
TTCAAAAAAAAAAAAAAAAAAAAAAAGCAGCCTAACAACTGCCCTGCCCAAGGGGCTCCCACCCCAGCAACCCTCTGCCCATTCTACA
TGGGACGAGAGGCCAGGGCCAGTTTGTCTGGGATGCTGATCACTTGTGAGCTTGGACAAGCTACTTGTTCTAGCTCAATCTCAGTTG
TGTTGTCTGTAAAGTGGGCAGATTAGGCCAGAGGATCTCTGAGAACTCTGATTGTAGCCAGAATTCCATGTCTGAGGGGCACTGCCT
GACAAGGAGAGGAAAATGTCGGGAGAGTGGCTGTGAGCGGTTTTTGGTAACAATGACTGACCTGGTTTGTGTTCAGGAGGCCCTTTG
TGGGACTGTCCGAACAACTTCTGTCCCTTCTCTTAATAGCCTTGCCTCTGCCCAGTCACAGAGCTGGCTGTCCCCTAAACACAGCCT
CAAACTTGACATAGAAACAATAATAAAGACAGCTCTTTGGCTCCAACATCTTAAAGTTTAAAGGTGACTCCTCTTGAGTCTCTTAC
ACACAGGAGCTGGTCAGACAGCTTAAAGTTTTCAGGATAATAGAGTAAGTGGCCAAGTCCCCTGCCTTAACAATCCATTCCAAACTC
CTTCCATGAACGCGCACTCCCACTTCTCCAAAGTCATCAGGCATCGTCAGCTTAATAGAATATTCTGTTTCTCCATCATTCTC
AGCAAACTATCGCAAGGACAAAAAAACCAAACACCGCATGTTCTCACTCACAGGTGGGATTGAACAATGAGAACACATGGACACAGGA
AGGGGAACATCACACACCGGGGCCTGTTGTGGGTGGGGGGATGGGGGAGGGATAGCGTTAGGAGATATACCTAATGTAAATGACGA
GTTAATGGGTGCAGCACACCAACATGGCACATGTATACATATGTAACAAACCTGCACGTTGTGCACATGTACCCTAAAACTTAAAGT
ATAAAAAAAAGAAAAAAAAAGAATATTCTGTTTCTCTGAGAGTGCCAAGGTGTGTCATCCCTGACTTTTTCAAAGTTAATCCGTTC
TATTTGGTTTTGTTGTTACCATTTTTCAATGATGATCATTTCCCCCGTGGCAGCGGTTCTCAATCAGGGGTGTGCCTCAGATCCCCC
GTGTCATATCTGGGTCCTGCATAGGCAGGTGAACCCCACTGCCTGGGAATGTGTGTTTTGACAGCACCGCAGGGGGCCAGTGCACA
CTCTTATTAAGAAACATGGCACTCAATTAAGCTCAGGGAGGCCAGCGTCTTCCCAATCATCCCTGCAGCCACCCCTGCTGTGCCTAA
CGAAGAGCACTGTGCATAGTAGGACTCAGTGACTCCTGGGGTAAGGATCATTGACTCTCTGAGAATTTGTAAAGTTGGAAGGCACTG
TTGAATCTTTAGTGTTTGGAGGCTTCTATACAAGTGTTGTTGCATTGTGCTATCAAGAAAATTCTGATAATTCTAGTTTCCTGTTGA
GTACAAATCTAGTAAAATGACATTTTCCTGTTTTTAAGAGAAAAAATGGCAAAATCACCACTGCTCAAATTCTCCAAAACCCAACAA
TTGGACGAGGAAAAGAGATGAACTGCCCCGATTTACCTTTGAATTAAAGAATTCGATGCAATTCAATTGAGCAGTTTTGTTTCCTCC
AAAGGCATCTCCCATACAAACCTGTCCTGTTAGCATGATGGTGGGAGAGACACTGAGCCTGCGGTCCGTAGACCTGGGTCCAAGTTG
AGACTTGTTCATTTAAAACACTTTTTAAACTTGTGGTAAAATATACATAACCTATAATTTAGCATTTTAACCATTTTTAAGTATATG
GTTCAGTGACGTTAATTGTCTAATTATATTCTTATATATATATTTGTATCCTTATTATTGTATATTTGTCACCACCATCCATCTCCA
GAACTGACTTTTGTCATTTTCTGTGTGGCCTTGTGTGCCTCCACCAAGGCTTGGTGGAGCTCTCTTCGTGCACATCATCTCCACAAGC
ATCTCCCACTTTGTCAGCCCCTAAACTTACCTTTACCCTGTCCACTCGCCCTAACGTCACGGGCCGGGGTTCTGATTCCAAGTTTCG
ATTGGGGAAGTGGTTGGTAATCCCACAGGTGGTAATAGTGTCGCCTCCATCTGTTGATCTCTTGTGTGCTGGAGACTGTGCCTTTAC
TTTATCATCTCAGTGATTCTTGGCGACAGTCCCGTAGTTAGGCTCTGTATTCATACCCCCTTTATAGATAAAGCAACTGAAGCGAGA
AAGGTAAGAGATGTGTCCACAGTCATGTGGCTGGTGAGGGCAGAGGGTTTTGAGGTCAACTGTTTGAGTTGACCATCTCCAAAGCTA
GCCAGGTTGCCCTGCCTGGGGGAGAGAGGCCCGTCCTCAAATTAACTCTTCTACCCCTGGTTCCTACAATGGCTAGAAGCAGCCTAG
AAGGTGGGCTGAGTCATGGGGACTTCCCTCCAACAAGGGTGAATATTAGGAAGCCTAGGGCTGAACAGTAACCCAGTGTTGGTCCAA
CCTTGCAGCAGCTCTGGAGGCCCTCATCCCAGCCACACCCCACCTTGGCTGTGCCTTGTGGTGGCTCCTCTGCCTATTAAAATCCC
TTCCTTCTCCTGGTTTCCCTATTGTCCTCCCCTTGGGTCCAAGGCTTGCCTCAAGGATGAGGCCCAGCACCCTTGGGGAGGTAGAAG
GGCAGCCATCTCAGGGTAACCTGCCCAGCCATCAGAACTGGGACCCGCTTCAGTTTCTCTGCCTTGCCTTCTGATCCCAAGGAAGGC
```

```
TAGGGTCTTCCTTGAAGCCTCACTGAGCCCCATACTGGGTCTTGTTCTTTCTTGCCTCCCTGCCTTCTGAGGGGCTGACTTCTACTT
CTGCTCGCCTGGAGTCACAGTGTCCCTGATAGTCCCCAGGCCCTCAGGATCAGACCTGCCTCTAACCCCAAGCCATCATCTGCCTGG
GCTGAGTCTGGTCTTTCCCTTAGACACTGTGCCTTGGGCTGCCAGTGGCCTGGGTGAGTCAGGCTTCAGCAGCTCTTCCTGTTTCCC
CGCCCATATTCTCCCCTCCTTCTGCCCGTTCCTATGGTAAGGTGCCAGCAGCCTTCCACCCAAACTCCCCTAAGCTGGACCCAGGAC
TTCCCTAGATTAAATGCCCCCACTTCCCTGCCATAATCATGTCAATAATCCTGTTTCCACCCTGATCTTGCTACTCTGAACAGGATC
TGCTTGGTCCACAGCAGCCCAGGGATCTGGTGACACACTGACCCTTACACACCACTGGGATCAGGTCAGTACAGAAGGCACCGACCT
GCTTTCTCACTTTGTTACTGCAGCCAAAGACAGCCAGTGACTTTTAGCTTTCATGGTAGCCTGTTAGTTTTATATTGAATTGAATATT
TACTAAAACCCCTCTGTCTTCTCATGAGCTGCTAAGCAGCTCCCCACCCTCTGCCCCCTCAATCTCCCATCCTTCCTAATACCGTTG
GCTTTTTAGGTGAGAACTGTACCTTTCATTTTCTTCTGTTACGTTTCCCTGCAGCTGTTTCAGCCCTTCATTCTAGTCTGTCCAGGC
CTTTTTAGCATCTCACTCAGCAACATGTATTTGGGGGCCTCCCAGCTTCCTGCCCTCCCACATGTGATGAGCCTGAGTATAATAGCTT
CATCTAAACCATTGATACAAATGTCGAACCAGAAGTCAAATCTGGGAGGTAAACTTTCTTTGGTTGGACTAAAGACAGGAGAAGTAA
AGAAGAAACTTCAAGGATTTGCAAATCTTTTTCATCTTAAAAGTTTTGTGGCCTCCTGTGAGGTTGTAGTTTTATGTGCCCTCCCAC
CCCAGCCCCCAAATAAGCCTGCTCTGCATTCATAGAACACGTGTTTATTGAGCCATGCTCTGTGAGGGGCACGGTGCCTGGTATGGG
ACAGAGCTGTGAACTACAGACTGGACCCGGTCTCGTGTTACTCACAGTCTGTGAAATGAAAGCGTGGCTGCTGGTGCTGGGGCTTGG
CTGCTGGGGCTGAGTGTTGAGTCTGTGAAATGGACGGGTGAAGCCGTACACCTGGTCACTCGGTGAGATCACGTTGTGTCACAGGAG
AGTGCTGAGGCATGGCCACCGTGGCTGGACGTTCTCAGAGACACTGCTCAGCCACAGCAGCTGGGCCTGGCTATGACCACTGACCTG
TGACTACATCAGGCACGGCCACAAACCAGAGATCAGTAACCAGCACGGCGGACCCATCCAGTGGGCGCAGAGCAGCCACCGCCTCT
GAACTCAGAGCCACGAGGCGGCTCTGCGGGAGCCGCCCGACACCTGCCTGCTGTGTGATCGCCAGCAGACCCTGCCCCTCACTGCCC
TCAGATCCCTCACTTGTGCATCTGGGGAGGAAGCTAAGGGCTCTCCAAGGTCCCTATCAGCTCTTTATGGTGCTTCTTGCTAGAATG
TGCTAGAAACCATATCGATTTGAGAGCAAAGAGAAATTGCCACCAGTGGGTGTGTATGTGTGGAATGGGAGGCGGGGGAGTGGGCAG
GGCGATATTTGTCTTCCTGCTGCTGTTAGAAATAGCAACTATTCTCTCCCCTGAGGACTGGACAAAAGGTGATTAATTCATTCAGCA
CAGAGCTATTGAAGGGCTGTTCTGAGCCAGGCACCGTGGGGACAGCAAGGTCCCTGCCCTAAACAGGCTTGTGATCTAATGGAGAGA
TAAGCACGCAGACACACACGCCTTGGCTGCTGGTGCTGAGGACAAGGGTGTGGAGGGCACGGCGCACAGTTCCACCTAGATGGGAAT
GAGGACGCTGCTGTGCAGGGCATCTGAGATGGTCATGGGGTCAGTTCTAGCAGGTGCAGGTGGGTCGAGGGTCCTCTAGGGGAAGGG
ACCAGCTCCAGCAGCTGTGAAGAATGCAGAAGCTCAGGGCTCTGCGGAATGGGAGGCATGCAGCTGATCGTAAAGGGAATGTTGAAA
GGTGAGAATAGAAAGAAGGGCTGGAGCCAGATCCTGGTGAGCAGCAGCTGGAGCGATGAGGCACCCAGGTCTAAAATGGTAGTAGCC
CCATTCTATCTATGAGAAGGGGTTGGGGGATGGCGATCTGATGGCGGGAGAGGGAAGCCAGGGAATTTGTCTTCAGGTTGCCACCTGC
AAAGCATTTTATGTAAGATGGGAGAGAATGTCAATAGTCTGTATCAAAGACTGAGAGGTAAGAATGGGGATGGGAGACGGGTGTAGAT
TATGGTACAGGTTGAACCCCCCTCAATCACTGGGACCTGCTCTGACCTTAGAGTACAGGGTTGGGCTAGAGGAATGGTGGATGAATC
TCCCTCCTTGGGAGATTGTTAGGATTAAAGATGAGAGCCACCTGGTACACTCTTGTTTAAGAGTCAGAGGAATGGAAAAACACCCCT
CCCACCCCAAAACGCTGAGGTTCTGATCTCACTGACTGAACACCACCCAACCAGGAGCCAGGGCTTGAGGGCAGCCCGAGTTTGTCT
TGCTCTGTCGGATCTTTTTGGATGTTTAGCAGGAGCCGCAGGAAACCCAAGCCTTATGAACATTATACAGAACTGCAATTACAACAATCC
CCTTATTGTTCACAAATCAAGCCTATGCCTAAAAATACACTCTTCTCCAGCAGCCTCGGGGACTTAGCTTCTCCCCCAGGCAGGGAG
TTTACATCCTTCCTTTGCCTGCAGAATTCAACCTTGCAGGTTTGGTGGGGTGGCTGGAAACCGCCACCATCCCAGAGCAGGCTTCTA
TCCCCAGGGCCTGGCTGTCACCCGCAGCAGCAGTGACATCACCCAAAGCCGACTTTGTGCGGGGGGGCTTCTGAGTGTTAAGTGGGCT
CAAAAGGTTGAAGTGCGGGGGCAGGTAGGAGGAGCTGCGTGGCTTGGGAAGGAGCAGCTGGCGCTGTAGAGGGAATGACTTGCCCCA
GCTGCTGTTGGGTTACGGGCTCGAGTTGTCACAGCCACCAGGGGACATTTTTTCCTCTTCTCAGTTCCTCTTTGACCCTGCGCTCA
GCTTCACCAAAACCACCTTGTCTCACCTTCTCTGTGTGTCCTCGGCCTCTCCTTTCCTGCTTGCCTCAGTCTTTCCCATCTTTCAAA
TCTAAACATGTGGTGTCTGTTGCTGCGGGTTGACTGCATGTGTATGTGTGGTTTTCAAGCTGAATTGACAGGGCTGAGTTTTACAGC
CAATACTGCCTGGCTCTGGAAGAAGAGAAAGAGGAGGACAATGAGGGACAGGATCTCTTGTAGCAGAGAACATTCTGATCCAAATGT
CCTTTCAGGATAAATTTAGTTCCCGGGGCTGGGACCACATTCTAATTTGGTGGGAGTCCTTGGGCACCCTGGTGGGGAATGGCTGCT
TCCTTCCTCACCTCCCTGGTCGATTTCAGGGACCGCAGGAAACCCAAGCCTTATGAACATTATACAGAACTGCAATTACAACAATCC
TGAGCTGACATAAGAGATTTATTCATTGGGCACCAAATTTCCTTTAGGCCCTTTCTAGTTTGTAGTTTTTGAAGCTGCTACATTTTTT
ATGGCTGGCACATCTCAAGGAGGAAATGCAGATTAATAGTCTGTACTGGCTGTGTTCTCATGCTCTCAGGATTGAATCAATAATGAC
TGCAGTATTAATTAGGCCTTTATGAAAAGAAAATGAAATATGGTATGACATGGAAGATTCTAGATTTAGAATCAAGAGTTCTAATCT
CAGCTCTGCTGCCGTCTAATGGGTGACCTCGGGCAAATCACTGAATTTCCCTGAGTCTGTTTCCTCCTCTGTATAAACAGGTCATAA
TGCTGGCCACATGCTGATGTTGTGAGCATCAGCTGAGAGAGCTGGGTTAAAGGGCCCACCAGGGCCTGGCACAAACAAGAGACTTCG
GTGACTTTAATTTTGGACTCTCCTTGCTAAGGAGATCACCACTGCAAATCCAGCCCTTTGCTGGTGCACAGTGGTGCAGCTCTTGCC
TGTTCTTGGCAGCAGGTGGGGTACCCAACACCTTGAGATGAACTCTCCAAAGTCCTTGCTGACAATGTCCACGGTCCCTTCTTAGCT
GGTGACCAGGAACACTCCCTAGGGGCCTCCCTTCTCAACCTTCCACTTCTCCTACCCATACCCCACAACGGCCCCATTTCCTGTTGT
TTCTCATGAATAGAGTCTGGAAGTAGAAACACTAGTTCGTGCAGTGTTCACGGACATCACGGGGCTCTAGGAAGCCTCACATCACAT
CACTCTGACACTGACTCTCCTGCTTCTCTTTTCCATATTTAAGGATCCCTGTCATTATGTTGTGCCCATCTAGATAATCCAGGATAA
TCTCCCTATTTTAAAGTCAACTGATTAGCGGCCTTAATTCTATCTGCATCCTTAATTCCTGCTTATAACATAGTCACAGGTTCTGGT
GATTTGGACACAGACATTTTGGGGGGGCCATTATTCTGCCTACCACAATCCTCTATTTGTAGATGAGGAAACTGAGGCTAGGGTATTA
GATCACATACCCAAAGTCACACAGCCGGTAAGTGGCAAGGTTGGAATTTGAACTGGAGCTGCACAATCTGAAGTCTACTACTACCCT
AAAGGGCCCCCTAGAAACCCAGAGAAAGCTCATTCCCACAGAATGGCCCCAAACAGCCGACCACAGCGGGAGAAGTAGAGATCAAAA
GAGCCCACCCATTGGAATCCCCACACACCGTTTTAGTTCATAATGCAGAGAAGGACTGGTGAATAATACTGCATAAACTCTGAACC
AGTGCTTCTCACATTTGGATTGAGAAAAAGTAGTTTGTAATTGCCCTGGGAGCTTTAAAATAATACTGGTGCCTGAGTCCCACCCCT
AGGGATTCTGATTTAATTGCTCAATTGCTCAATTGCTCAGGGGAGGCATTGAATAGAGGCGATTTTTCCAAGCTCCCCAGGTGATTT
GAAAGTGCATCAGAATGTGAGAATCATTGCCCTGAACTAACCCATGGCCACAGCCATTCCCCCTACCTGACAATTGTGTCCATAGTC
TGATTAGACCATGCCCAGAACACACCCCTCCCACCTTTTGGGCCTTTTCCCTGAACGCCCAGGTCCCCCTGTTCCTCTTCACCGCC
TCACTCGAACCCTGACTGTGTCCGAGCAGCATTAGATGTGTAGAGAGGACCCTCCCATGGGAAATGGGGCATATTCTCTCCTCTGG
CATCTCTCACGCCCCTCTTGCATGGCTGTCTGCAGAATAGAATTGTTTTAATGTCCTCTTTGTATATGCTATATTTCTATAGAGTCT
TTCCTTGAAAATGTAATTCACTATGTTTTCGGTGTCTGCATCAGTTCAGCTTGAAAGGCAGTGTGTGCAGAGGGTTGGAGGTCTGAC
TCTGGAGCCAGACTGCCTGGGTGCAAAGCCGAACCCTGCCAGTTCCTGACTGAGCTTGGGGTGAACGATTCTAGCTTTCTGTGCCTC
AGTTTCCTCATCTATAAAATAGGGATCAGAATAGCCGTTACCTAGGGTTGGTGTGAGAACTAATGCTTTCATATATGTAAAAGGGAT
TAGGACTATGTATTTGCTCATGTTACCAGTATTGCTACTACTCTTGTCTTCACCGAGATCTGTGGGATTCAGTTAATTTCAGCAGAT
ACCGATTCCCTACTCAGTGCCCAGGATGGGAAAGAATAAGATGTGTTCTCTGCCTTCAAATAGTTCATGGTCTTGGGAGGGAGAGGGC
TGGTAAGGAGAAGGGAAGGAGTCAAATAGGTGAACAGCTAATTAATGATATAATACAGTGTTGACTTGGAACCACGTTGCTGTGTGGAA
TAATAAAGATGGGGACATCATGATGTGGGGATTCACCATGGTCGTGAAGGCATATCATGAATTCTAAGCACTAATAAAAAACTCTTA
GTGCGTTTTGTCCTGCCATCCATCCGCATTTGCGAAGCTGTGGGCTGTAACACCCTGTTAAGCCCCTTAGACTCATTTCATGAGTTG
TGAATTCGAGCCAATTGTGTTAGTGTTTGTGAGTGAACGCATTAATCTGAAAGGTGTCAGTGGCATCCCAGTTCCTGCTGCAAATGC
CCCTCACAGGGACGGAGGAAGCTGGCTTTCTCCAAGAAAACAGCCAGTCTGCTCTTCAAATCCTAGCCCTACCCTCCTTTCAAGGTGCA
TCCCAGAGCGGCTGGAGTCCACTGCAGAGGGTGGTAAAGGAGGACGTGTCATGCCAACAGGGGAGCAATGCACAGCAATCAAAAGG
GACATCAAGGAAAATGCAGAGTTGCACGGGGAGACGGGGTCTTAAAAGCCCACACAGGCAGGGATTGGAAAATGCTCCTCTGGGAA
TGCTCATGTGGTAAGGTGGCCATGGGTGAGTGGGAAAGGAGGGAGGGGTGAATCCTTGGTGTGTGAGGCTCTGTAGAGAGAATGGTT
```

107

```
GCTGCCTGGGCAGATGCCTCCTTCCTGTCCCCTCCAGCCAAATGGTCACACATCTCTCGCTCAGATTAACAGCTTGCTGGCCGGTGT
GGCGGGCAACTGTAGGAATCTCCGTCAGCTGTGGGAACAGCTGTACACTGCCCAGGGCCACATCACACCCTGGCATGTTTCCCATTC
CCTCTTGGTCAAACACACTTAAAAACAGGACTCAACGCTCCTTGGTTTCTAACTGTAAAATATGCCACGTGAAGGATGCAAACTCAT
GTCTGCCTGGGGACTACAGTGGGGCAGGCAAATGTTTGATGGGCTTGTGAGTTTTTGTGTTTTGAAGAAAAACAACTGTCTCAAGGC
AGACAGATGTTGATGAACGTGTTTATTTATAAAGGGCTTACTGAGCTAATAATGACACCAGATCATCCCCAAATGACAGGTGTCACT
GCTGACCTATAGCTCAGAGTGTCACTGCCTCTGTTGGAGCAACCTGCTCCATACTGCTGGCCTTTTTCATCTGCAGCCCAGTGTTGC
TTGTGGCAAAATGTTTACAGTAGCCTGGGGCTTGCGTGAAAGCCTTGCTAACCCATCCCTGTGCCCTGCTAGTGGTCAGAAATGTCT
GTTTTTGTTTCTAGGCTTCCAGAGTCACTAGGACTCTGGGCAGGGAAGGAGGGCCCTCAGGCTCATTGGTGTCTAGGGCCTGGGGGC
ATTTTTGAGCCACTTCACAAAGCCTGGCCACAGTCTGAAGTCCAGTACATACAGCTGCAGCCCAGGTGACTCCATGGTGAATTGCTC
CATCCCTAACATAAGGCACTTGGCACAGCCTCAGTGAGCTATGAGAGAGGCCAGAGTGGGAAGGTGTCATGCACGGCGTGAAAAGGG
CCCTCACAAAGACAGAGAGCCTCTCCCCTCCAACCAGGCAGAACAACTAAGTAGAAAGGCTGGTGCAGCTGGAGAATATGGCCAGGA
AAGGAGCCTAACAGTGACAGCCAGAGGAAACCCAGGCCGAAGCCACTGGGCCTGTGTGGGTCTGCCTCTTGGATGGGGCTCCATGCA
CACCCATGTTAGTAGGTGGAGCTGTGTGGGAACATTCAGGTCCCTGGGGTTAAGTCTCATGCCTAGCCCTGGCTGGAGGGTTTTGAT
GAATGGATCTTTTTTTTTTTCTTTAATAGTGTAGAAATTGTATTTTGCCTCATATTCTTGGGTTATCCATATTAGTCTGCAGCTGTTG
GTCTTCCTAAACCATGACTGAGTAGGTCATCCTCTGTTCACCACTGGTGATTTCTTACCATCTGGTGATTTCTGGATAATGCTGAGC
CCCTGAGCTTGGACATCAGGCTCTACAGCAGGGCTGGGCTCCATACTTCCCGGGCCCTGCCCCTGGCCAGCTGCCCTGAGGTCACTG
AACACCTGGGGTGAGAATAGATCTTACCCACCCCTGCTCCCTGTGTTTTTCAAACTCCCCTCCTTCCTGGCTCTGGAGTGAGCTCC
TGGGGTGCGGGGTAGTGGCTTGAGCTCCATGCAGGTTTGGTGCCTTGCTCATACTGAGAAGCTCAGTAAGCCCACATCAACCATTGT
CTTTCTCTAGCCAAGAGTCAAATAGATCCACGTTTAAATCCCAGCTCTGCCTTTTATTAGCTGTTGGACTAGTTGTTTAAACCTCTC
TGCCTCAGTTTCTTCATCTAATATCAGTACCTTCCTAAAGGGGTTGCTGTGCCCATTACATGAAATAATGCTCAGAAAGCAGTGGTA
CAGGGTAGGAATTCAGTATGGTAGCTGCTATTGTTTTTGTTACTATTATTATTTCATCTCACCCCTACTGCCAGTGCCCCGCATGCA
GTAAGTGCTAATAATTGTCCAATCAAGAAACAAGTGACAAAAGGCCATGTTCTTATTCCCTTGCTCTTGTGGGTTGGCCCCTTCCTC
TTCTGCTGCCTGCATGCCCACTCCCATCAGACTGCTGAGAGCGTTCCTGGCGCGCCCCGACTGGTGCTGACATCAGCCAAACAGAGC
CCCGGAGGCCGAGTCTTTCACTCCGGGGTTGTGCATCCTCCGGGGGTGTCAGCTCTTCAAGCTCTTCCAGGTCCTGCAGCTGTTGGC
TAGAGATGCCAGCTATGGAAACAGCTGTGCCTCCACCATCTTGCCTCTTCCTCCCCCTGGATTCCTGCTATCCCTTTGGCAGGCAAG
CGCACTTAAAAATAAGTGGCAAAAGTTGCACTTGTTTGAAGTGTAAGATATGTCACTCTCAGAAAGAGATTTTGTTCTTACTGAATA
AAGGATAAGAAATAGATTCCTAGTTAAAGAACTTTTTTGGCTAACTCTTCTTTCAGGGGGTGGGAGCGAGCATCCTTTGTCCTTTTG
GCGTAGGCTCTTGGGGAAGAATGGATGTTCTTGCTTCAGAGCCGCCAGCACAGGCTCTCTGGGCTCTGGCTTTCTCATTGATCTATT
TCCCTCTAGTGAATGGGTTTTACAGAAGCTGCAGAGAGATTTTCCCCTAATGATTGTGGATAGAAAAGAATAAAAAGTGACTCCGGG
GGTATCTTGGGACCTTAACTGTCATTGCACGCTGATGCTTCATTTCAGAATGCAAACGGCCTTCTTCTACTTTGCCGGGATAAATGG
TTCCCTTTAGCTGGGGACAGTGAGAAAAGAAGCAGGGGCTTTCCAGGTAAAATTGAACGGCCTCCTGTGTTGGGGGTGAGGCTCTCCC
CCCCACCTCCTTTCCTCATCTCCCCCAGAATCCTAAAGAAAGGAGGACGGAGGCTGGAGTCCACTGGCCAGCTTGGTGTATAGAATC
TTTTTCTTTCTCTTTTTGAGCGGGGATAAGGTCTGGTTGGAGGGAAGTGGCACCATCTTGGCTCACTGCAACCTCCACCTCCCAGG
CTCAAGCCATCTTCCGACTTCAGCTTCCCAAGCAGCTGGGACTACAGGCGCGCACAATCATGCCCGGCTAATCTTTGTTCTTTGTAT
TTTTTTGTAGAGATGGGGTTTTGCTATGTTGCCCAGCCTGGTCTCAAACTCGTGAGCTCAAGCCATCTGCCTGACTTGGCCTCCCAAA
GTGCTGGGATTACAGGCGTGAGGTACCATACCTGGTGATGGATACAGTCCTAATCTGTTTCCTCCTAACAGGCATCTCCCGGTGAAG
CCTGGAGTCTAGGGAAGGAACTCCTGCTTAATGGGCCCCCAGGAAGTCACTTTCTCAGGCTGGCTGGCATCAGAACTGGTGCCCAGG
TAGGAGGTGCCAGTGTCAGTCTTCTCAGGGTGGGGTGGTGTCTGCCTTTTCCTGTGGGTCTAGCATCCTCACCAGGTTATAACAAGGC
CAGCTCGGAGCTCTGTACTGGGATCTCTTAACAATCACCGGCCCTGAGCACCCATCTCCTGGGTGAGGGTTTGGGCCAGAAAGATCC
TCTGAGCCAATTCTGGACTCCTTATCTGTGTCCTGTGGTAGAGCAACAGCTCTGTGAGGACAGAGCAAAGCTAGGGGGCCACCGCCG
TGTACCAATATCAGCACAGGAGCTTGGCCCTGGTGGGAAAGCAATGGAAATCCATCTGCGATAGAATGGATGGGTGAAGGAAGAAAC
TTACACCTTCTCAAGCAGGGGCAGGTGGCCCTCATGATTCCAGACTGTGGCTGTGATCTGGGACAAGCTCTCCCCTTTATTTCCAAG
TGAGCTGCTTATATGTCCCATTCTGTAAGAACACAGCCAATGAATCATCACAACAGCACAATGACAATGATGACTTTGGGATCCAAA
ATAAGAAATCAAACTCCCTTACATTCGTTCAGGAATCTACAGTTCAACGAGGCTGTTTCCCATGCATACGTCACACCCTTCGCCTG
CATGTGAGTGGATGAATGTGGCCTCCGTTTACAGGTGAGGCAGCTGAGGTGCTGCCAGGTCAAGGGACCTGCCCAGGACCATGCAGG
CAGCAAAAGGTGGGGTGGCTGCCTCCCAACGTGGGATGCAGAGCTGACAGTTGCCCCAGCACAAAGTCAGTTTCATGCTCAGCTACAG
AACTAATCTCAGTGACCTTTCCTGGGTCCCATGCTCTGCTGCCAGATCTCTTGTGGGATGAGGCCACCATGCTGACCAAGAAGCTCC
AGACCACGATCAGCTGGCTGTCCGCTGTAGGGGCACAGGTCTCCCCACCCCAGGGCACTTGCGGATTCAGGAAGGAGGCAGTGGAGG
CTCTCCTTGCTTTCAGTAGGAGCTAAGCCTTTTCAACTGCACTCTAGGGCATTTCCAGCAAATTCCTACATGACCCTATGCAGGGGA
GGGGACACAACCCTGGTGGCTTCACTTTATGGGTTGAAAGTCTCTGTGGGCTCCAAAAGCGGGTCAGCCTGCCCTCGTTTTCTGGAG
ACTCTGGCTACGCTCTTTGGACAGTTCACAGGGATAATCTCTAGAGGTGCCCTGGCTAGAACACGGAGATGCCTGGCACTGCTGTGC
ATTCATCACAGTGAGTGACAGTGATTTGAGCCCTTGACCACGTGTGACCTAATCCCACAGTATGACCCAATCCCATTTGG
CATTAATGCTGGCTGAGACTGGAGACCACTGACCTCTTTTCCTTTCAGTTTGATCATATAATAAGAACTGTGTTAGTATAAATCATT
ATTTAAAGTGTGCTTTAGGCCAGGCATGGTGGCACATGCCTGTAATCCCAGCACTTTGGGAGACTGAGGCAGGCAGATCACTTGAAG
TCAGGAGTTCGAGACCAGCCTGGCCAACATGGTGAAACCCCATCTCTACTAAAAATACAAAAATTAGCCAGGCATGGTTGTGCATAC
CTATAAACCCAGCTACTTGGGAGGCTGAGTCAGGAGAATGGCTTGAACCCAAGAGGCAGAGGTTGCAGTGAGCCAAGATTGTGCCAC
TGCACTCCAGCCTGGGTGACAGAGTGGACTCTGTCTCAAAAAAAAAAAAAAAAATGCAATAAGTGTCAGTGTTGTGTAATTGACTGTT
AGTTCATAGCAATGGTCTCACCAGTCTGCCCACAGTATAGTGAGGTGGTAGGATGGAAGGCCTGGAACTAGAGACTAGGATTTGAAT
TTTGGCTCCACCACTTCTTAGTTGTGTAACTTCACACACTCTGTAAGTCTCGTGCCCCTAATCTGCAAAGTAGGTACAGTGTCAGTG
CCTACTCATTGAGTTTTTGTGGGGACAAAACCAGAGAATGCTTGTGATGTGTTTGTCACTGTGGTCCCCATTACCCTTAAGAAATGT
CAGCTCTTTTCTTTGCTCTTATTACTACATTAGTTTAAACAATGTCTCCCATAGAGGCTGCTCCTGAATTAGTAGTGTATTCTTGCC
ATAAATTACACTTGGGAATAGCCTGATTAATAGTCAGAGATAAGAGGCCTAGGGTGTAAACTCCACCAAATTGTTCAATGTGTGAGA
CTTTGAGTTAGAAGGCTTGGCGAATGTCCTCGCTATTTGCTGCCTTCATGGGTATAACTAATGTACCTTCTCCTCATCTGTGA
ATGGGGATAATGATGTTCCTGAGCTATGGAGAGGACCAGGCAAGCACTGTGCGCAGAGCACCTGGCATAGGGCCTGGTACAGGTCA
ATGCCCACCTATTTTCTGTCCTTCCTTTCTTCCTGTTTGTTCAAACCAGGTCTGAGGCTAACAGATGTGTCTTGGTGAGTCCAAGAG
GGTGGCTGGGCCATAAGGGTTGGGGGAGTGGAGAAGTGGGGCTACCAGTTGCTGCTGGCAATTTGCAATATCTACCCTGTCCCAGGA
ATTGTACCCTGGCCTCTCGGGATGATTTAGTATGGCAGTCATGGAGCTGAAAGTTACAAATGGAGGCTCCAGTGATGGAGCTGGATT
TCCTGTTCTATTTAGTCCAGAGCCCCATGTGGAGGATCCAAGACAATTAGAGCCAGAGAGTGCTGGAGAAGGCCTCAGTGATCATCAT
CAGACCATCCTTCTTATTGAACAGATGAGGAAACTGAGGCCTAGAGAGAGAAGACACCTAAAAGTCCACACAGCCAAGTTACAGAGAC
TTGCTCAGGCAGAGCCACACCCCTGTGTCCACGCCTCTGGTCCTTCTAGGTCTCTGGGGTCACACTGATCAGAATTCCTCCAGCATC
TGCTATATGCCAAGCTTTGTGTTTCTCATTCAATTCATTTACCAAAAATTGAAAGAGTGCTTACACTGTTCCTGGCACTGGGGACAC
AATAATGAACAGGACAGATCAGATATGATCCCCTCATAGAGCTTCCAGGCTAGTGGGAAGGCACAGGGACAGGCAATAACAAGGAATC
AATCAAATAAGTCCGTGTAAATTCAGATGCAGACAAAGAACGGAATGTGGCAGTGATGGGGGGTCATGGAGGAAGAAATGAATTAGG
GTTGTAGCTTCAAGGGCGACCTCTGAAAGTTACTTTGGTATTGTGAGATTTATTTCTATTAATACTTCCTTTCGCTATGTGACTACAG
TATTATCTCCTCAGGAAAGTGAGGCTCAGAGAGGTTAATTAACTTGCCCAAAATCACACAGCCAGTCAGTGGAGGAGTCAGAATTCG
AACCCTACTCTGCCTGACTCCGAAGTCCAGGTTTTTTCCACCATCCTTGATTATCTACAGAAATTAGGACAACCTACTACAGAATGT
```

```
CAGGTAATATGGTAACCATTAGTATGAGCATTAAGGGACCCCAAGGCCAGACCTGTGAGCCCAGCCTCACTGCTGTCTCTGTCTCCT
GTTCTAGGGGGAGGGACGAGGTGGATATTCTAGATGGTCTTACCCTCTCCTCTCCTTCGGAGACTGGCTTCATTGGGAGTGAGATGA
CACTGTCGCAGGGCTAGCTTTTTGTAGGCCACTAGTTGTCGCAGGCTGCTGATGAAGCCAAGCAGATCACCCCCATGTCCACAAGCA
CACCGGGATTAGATACATTTAAGTCCTAGATCCCAAACGCCTTTTTCATTTCTGGTTTTGCATTTGTTTCCATTCTACTATCTTCAT
TTGGAAAACCCTTTCATTCCTCAAGAAAACAAGCCACACAATCAAAAAAAGATGTGTCTTATCCTCTGGGCGCTGCCTCATTGCCTG
CCCCATTATTGACCAATGCCACCGGAGTGCCTTGCACAGCTTGGCCAGCGTTCGCTGTGCACATCGACCAGTGAGCTGGCATGCTGA
CAGCGGCAGGGGCCCCAGCTTCAGACACTTCTGAACCCAGCTGCTTTACGGACACAATCGGTTGAATCGGTTGAATCATGACTTGT
CTCCCAAACCACAAATTTGTAACTAGTTCACCGTGAGCCTATGCAGCCAGATGAATTGTAACTTGGGCAGTCCCTGCACCTAAATTT
TTTTTTTTACCCAATAAAAAATGATATCTAGACATCTCTGTGTGCATGAAAGCATGTCTGTACGTGCGTGTGTATGTTTGGCACCAAT
GGGAAATACAGAGACGTGGAATGCCAGCTCTGCCCTCAACCTCTTCCCAGACGGGTGGGAGATGATATTCTGTGGACTGTAATTCAA
AGAAGACAGACTAATAACAAAGAGCTATGGAGACACAGGGGAGGGGCAAGAATTCAGATCTGGAGTGGAGACAGGAAAATGGTCCTG
GGAGGCCAGACTCTTGGAGAAATGTGGACCTGGCAATAAGGAATTTATGAGCTCTTTCTCAGTCACTGCTGAGTGTATATCTTCCAAG
GTATTTTCCAGTGGGCATAAATTACCTTTCGATTGCAGTATTCGCCACCTCCACTCATCCCCCAACACCAGGTCTTATGTGGACTGT
TAGGGAAGCCTCTAACTTGTACCCTGCCTCCTCACCCTTTCTGCACCACCTCACCTCATCGTCACAGCTGTGAGGAATCCAACAGAC
ACACACACTGGACCTTGTCCTTGTCTGAAAACTTTTGGCTGCTCCTCCTTGTCCTTAGAATACAGTCTAGACTCCCCAGGGGGGCAG
ATAGAGCCCTCCAGAATTGGCCCTGCCCTGCTCTGCGGCCACATCTGTGCCATTTCCTCCTGGAACACTGTGCCAGTCAACCTGGAG
TCCCCTGTGCCCATTACCCTGGAGTCCCCTGTGCCCCACAGGTCCACGTCTCTGGGCTCTGCATGTACTGCTCCTCCTTCCAAGTG
CTGTTCTCATCTCTTAAGCCTGAGCTGTCCTCCCTGACCCACCCCCACCAACTTTCAGTGCCCCCACGGAGCCTCATGCATACAGAC
GTCTCTTGCTGCACCTCTTATATGCAATATATTCCTCTGTTTACATCACTGCTCATTTACTGAGTTATATGAGGGTGGTCCAGGCCC
GTGCCTCTTTGAGTCCCCATGCCTTATACAGCATCTGACAGAAGGGTGGCTCAGGGAATGTGTGGCACAGCAGTGAATGATGTGCAG
CTTCCACCGTACTTCCAGACATGAATTGATGGTCCTGGAACCTCTCAGAGAAGCCTTACCACGTTGTGATCAGTAGGTGGGGACTCG
ACACTACCACTGCAGAGGTGGCTTTGTGGGGTGGCCCCTGTGTGCGCTCTTATGTTTGCAAAGTGCGTTCTTTCACGCAGTCAGCCT
ACCAATGGGTGACCAGATTCTTTCTTCAGAAGCCAGTCAGAAAAGGGACTTGGCTTCCCAGATTACGCAAAGGCAATCTAATCCCAG
ACCTTCAGTTTTTGGTTAGCCAGCGGCTGCACAAGTGTCACAGACCACAGGGGCTGGGCGACGGGCATGGTGGTCCCTCTGCTTTCC
TGGGCACGTTAGGATGTCATTCTCTCACAGGACTCGTTTCAGAGAATTTTCCGTGTAAATGTGAAAGACCTAAATTTATGCCTGTCT
CAGTAACTAAGTATAGGCTACTTATGGAACCACCCAGCTATGGGAAATCCAGCCATTAACTAAGTACAACTAACTGTGAAGAAATAGCCCCCCTT
ATTACACAGAGCAGACATCCTCAGGGATTGGATGAAAAAGACGCTCTCTAGGAAGTCAGTGAAAACGAGCTCCCTGCCTCCTCAGAGT
ATGCCTGGTCAATGTGTGACAGGCTCGCTACTGGGTTCCCATGCTGCATCCATAACAGACATAACCAATCGATCCCAGCATTCTTTC
TAGATGGGCTCAAAGTCCTTCCTCCCTCCAGTGCCACCTCCATGCACAATGCCTAGCCTCAGGCATACTGCTGCTCCTGCACCTCTG
TGAATGAGCCCTCCACAGAGGTCCACCTTTGGTCCATGGTTGATCCATGGACCAAAATGCCTGTTCCTTGTGGGCCTCAAGACAGCT
GAATAATCTACTAATTCAAAGGGAGACCAGAAAGGCAATAGTCAAAGATAATAAAGAGCATAACAATAGTCTACCCTCTATAATGCA
AGTTTTTTCTCCTCTCAGAGGGTCCCTGATCCCATCAGAGGACTCAGTTCTTCACAAGCAAGACTGATCTTGCAGCAGAGGGCTGAT
GGAGTCATTTACATGGAGAGCCAAAGAGAGTCTCTCCCACCAGGACCTTGAGCTCCTTTGAGGTGTGCCTCATTTTTTCATTCATCA
AGTGTTTATTAAGCATCTATGTTTAAGGGCAAGCTCTGGGGAAAACATGGACATAGAAACAGACATGGTCTCTGTCCTCAAGCAGCT
TATAGCCTCAGAACAGGCCAGACATTAGTCAGATAATTGCACAAATGACTGTAAAATTACAACTGTGGTAAGTACTCCAAGGAGCCT
AGATTGATCCAGTCAGAGGATGAATAGGATTAACTTGACATTGAAGTGAAGGAAGGCAGCATTCTAGGCAGAGGGACAGCATTCGGAA
AGATTGTGTGTCAAGAAGGAGATGAGCACAACAGCCTTGGTGGTCCTGGGAAGAGGATTTGTCTACATTCTACGCACAACAGGAAAC
CACCAGGCAGAACCTCTAGCCCTGGGACAGTGCTGTGCACATTCGAGGGCTGGATGCATCAGTGAGGAACAAAGGCATGCATGGGGG
AAAGGATCCAGTGTGATGGAATGACGGATGCTCTCCCACCCAGATTATGTGCAAACCCTGGAATGAATCCGCCTCCACACTGACTTT
GGAGATCTGGGGCAGGTCGGGTTTCCAACTGCAGGTCTTTAGTATTCCTTGACTAAGTCAGCTTAGTCAACTACGTTAACGAGGTCA
AGTCAAGCTAAGTTAACTACTCCAGCCATTCTTAGAGGTATTTCACTGCAGGCTTTTTGTGTTCTATTTCTGCCTTTCCCCCCACAA
CAGTTGGGTAACAGTCCCCAGCATAGTGAATAAGGTTCTGTAAGATTTAATATTGATTTGTTGGAGTTTTTAAAAATTGAAAGTATAA
TTAATGATTTATTGCTACTTTGCAGTTTGCATTAATCACTGTCATTAATGGTAATCCTTTTAGGTGGTTTATAACTTTTAAATGGAG
TCTGTTAATTCCCAAGCCTGGTGGCTTTGTCTCTAGGCTGCATTGGGGTCTGCCCACCCTCCCATGCCCCCGACATCCCTCCCCATCA
CCCCATTTGCTTTAAGATACAGGGATGAAACTCCAGGTGTAAACTTTTCAGGGTCAGCCTCTTACCAAGGAACAGACATTTGGGTAC
CTTCCTGGCCAAGAGGAGGGGTGGTGCACAAGCTGGCTCTGTGGGCAGAGAGAACCAGGCCACTGCTAGCTCTTCA
ACCCACTACTTCATTTACAACTCTCTGACTTTGTATTCCCTGGTTTGCATGTCTGACACCTCACTTGGCACATGATTTATTACAGCT
TTTCAATAGCCCGTTCTTTCTGATTGACCTGTCCATCTCCATTTGGAGTGGAAAGATTGGCTTCTAGGTGTTTGTGGTGAGAATATT
CCTGAGTGCGATGGTTTGGGGTTAGGAGCTTCCTGCTCTTGGTACAGAACCGAGGACCACAACTAAAGGAACTCCCCTTTCTCTGGC
TGGGAAATGAAAAGGGTGGGGAAATACACACTAGAAGTTGCCTGAGACCTGGCTGAAGGAGAGAAACTTTCTGTTTTGCCAACACCT
TCAGGGATAGAAAAAAAATCAAACTTCTGCCTGCATCAAGGGGAAGCAGCCTCAATGGAAACAGCATGAAAAGAACTTCGGA
GCCCATATAGGTCAATCCCCTGCTTCTGGGGTGGGCATTCTCTCCCACATGAATAGGTCCAGATTTCATTTTGAAAACATTTCCCTG
GGAGGAATCTTCCCTCAAATTCTCTCTCTTTCCGTCTCTACCCTTCTTCAAGGTTTGACAATCTCTCTAACCAAAATCCCTCTTGCT
GTAGTTAGAGCCTCTTAGGGACCAGTTAAACTGGAATAAGGGATGGCCATAACAAAACATGCAAATTCCTTTTCCTTTTAAAGAGAA
ATATGTCTCCTGGATATTACCTGAATAAACCCATCAGCTTGCTGTTACTTTTCTGGGATTATACTTGATGAGGATGGTTGCAGAAAG
TCCTGTAAACATCTTCTATAGTCTGTATGGGGAGGACAAGCTCCACCTGTTTCCAGTGGAACATTCTGATTCCTGTTGTTACTCTAT
ATTTACAGAGTGGGCTCCACTAAGAGCCCACCTCCTTCTGTCACTGGTGGAAAGCTATTTAATATGATCCCAATTAGAGGGAGGAG
GATCTGGGATGGTTGTTGGAACATGGCAGACGGGGGAGACCCGGAGGGAATAAACCACCCCAGGATGCTGTGTGACCACCTGACCCT
CTGCAAAGCAGCATCGTGGCACTCTGTTCCCTTTCAAGGTTCCCACAGTGAGTGCCACCCATTTTTACCACAATGGCAACAGGATTT
ATGCTCCCACCCACTCTCAGTTCTGAACTTTCTATGGCTTTGCCCCTCATTGGTCATAAAACACGTGTCACCTGCCTCTCTGACTTG
GCTTTTCTTGCAATTACATGGTTTCCTTTGCGATCTTCCTGGCCAGTGGAAGCAGAAGGATGCTTTTACAGACAGGGTATTATAGAT
GGGTTGGCAGATGAGGTGATTTCTGAGGGCCTGGCACAATGCCCAAAGGGACGTGAGTCAGCTCTCAGAGGAGGTTTGGTTTTTCCT
TCCAAATTCATACAAACAGCAGTGAAGTTTAAAAGATCCTCAAGTGGATTACAACCCCCATTCATTTGGCAGCAGAGGAGCACAACT
GTCAAAAAGTCCAGGTACTTTAAGCTGTGGAAGAGGCACAGAGTCACTCTCAAGTTGGACAGGGCTTCAAGCTATTTCTAAAATTAA
CATCGGGGCCTTTCGTTCCCTTTCGAGGTTCCTTTGAGGAATGGGTGAAAGCCACCTCTTCTTGACCACAGCAGTGACTGGATTTAT
TTTTGTCCTGTCTTCCTCCTCACATTCTCAGTTCTCGAGGCTTTTATTGTTTTGTTGTTGGTTAGCCAGGCTTGGGCTGGTTGCTTAC
TCTCCGGTGTGCAGGGCTTCTAGAAGTGTTAGCCACAGCCAGCGGGAGGCAGCCGATCACCCATCCCACCTGCTGAGCTGTCGCGGG
CAGACATCTGTTGAGCTCCTCCCCAGCACCAGCCGCAAGAATGTACCTGATGTGGTCTTTGCTCTGCAAGAGCTCTCAGTTTAGAGC
AGTCTTTTTGAGAAAAAAAAAAAAAAAAGTGGTATGATACACGTAATATAAAATACCATCCTAACTTGTTTTTTTTTTTTTTTGA
GATGGAGTCTCACTCTGTCACCCAGGCTGGAGTGCGGTGGTGCGATCTTGACTCGCTGCAACCTCTGCCTCCCAGGTTCAAGTGATT
CTCCTGCCTCAGCCTCCCTAGTAGCTGGGATTACAGACATGCACCACCGCCCAGCTAATTTTTGTATTTTTAGTAGAGACGGGGT
TTTACCATGTTGGCCAGGCTGGTCTCGAACTCCTGACCTCAGGTGATCCACCCACCTCGGCCTCCCAAAGTGCTGGGATTACAGGCG
TGAGCCACCACACTTGGCCTCATCCTAACCATTTTTAAGTATATAGTTCAGTGATGTTATGTTTACTCATATTGATGTGCAAACAAT
CTCCAGAACTCTTGTCTCCTGCAAACCTGAAATTCTGTACAGTATATACATGGGGGATGTGTTCCAAGATCCCTGGTGGATGTCTAA
AACTGCAGATAGTACCGAATCCTACAGAGAGAGATAGTCCCAGATTTAACGATGATTTGACTTAAGATTTGTCGACTTTATGATGGT
GTGAAAGCAACACACATTCAGCAGGAACTGTACTTTGAATTTTGAATTTTGATCTTTTCCTGGGCTAGTGGTATGCGGCATGATATT
```

EP 2 204 376 A2

```
CTCTTGTGATGTTGGGCAGCGGCAGAGAGCCCCGGTCAGCCCTGTGATCACGAGGGTCAGCAACTGATACAGTACTCTACCGTTTAC
AGTGTTCAGTAAATCACATGAGATATTCAATGCTTTATTATAAAATAGGCTTTGTGATAGATGATTTTGCTCAAATGTAGGCTAATG
TAAGTGTTCTGAGCACACAAGGCAGGCTAGGTTAAGCTGTGACATTCGGTAGGTTAGGTGTATTAAATGCATTTTCTACTTACAATA
TTTTCAATTTACAACGGGCTCATCAGGACGTGACCCATCATAAGTTAAGGAGTATCTGTAATGCTGCAATGAACATGGGTGTGCAAA
CATCCCTTTGATACTCTGCTTTTAAAAGCAAGTGTTCTTGGCTGGGCATGGTGGCTCACGCCTGTAATCCCAGCACTTTGGGAGGCC
GAGGCGGGCAGATCACCTGAGATCAGGAGTTCGAGACCAGCCTGGCCAACATATATGTTGCCATATATGTAGCGAATCCTCATTTCT
CTACTAAAAAATGCAAAAAATTAGCTGGGCATTGTAGTGCATGCCTGTAGTCCCAGCCACTTGGGAAGCTGAGGCAGGAGAATTGCT
TGAACCCAGGAGGCAGAGGTTGCAGTGAGCCAAGATCGCACCACTGCACTCCTCCAGCCTGGGCGACAGAGCGAGACTCTGTCTCTC
AAAACAAACAAACAAACAAACAAACAAACAAAAAAAGCAAGCATTCCTAACCTGGGGCCCGTGAACTCCCAGAAACTAGCTGCAATA
CATGGTGTGTTTCTGTAAATGCTTCTTGTTCTAGGGAAAGCAAATTCATAGCTCGCATCAGGTTTTCAGTGGGCTTAGGATGTCAGG
AGGGCTCAGTCTAGAGAGTAACAAAAGCAGCACCTCCTTCGGAATCCCCCAAGGCCCTGCCTGTCCTGAGTGCCGCCTCTCCGATGG
CAGGGCAGGTGTGAAGGAGCAAGTCATTCACTTCTTGGTTCCTGTCCCAGCTGTGCCATTTACTACCTGGGTGACACTTGGCAGTTC
AGCTGACGTCTTGGAGCCCTAGTCTTCAAATCTGTAAAATAGAAATAATGACAACGTCTTCCTAGGATTGTTAGGAAGATTAAACTA
GATTTAGCACAATGGTCAAAAGCATAATGTGGTAGGCTGAATCATAATGCCCCCCCCTGCCCCACCGACCACCACCCTGCCAAAATA
TCCAGCTCCTAATCCCTGGATATCCTGGCAAAAGGGACTTTGCAGGATGTGGTTAAATGAAGGATCTTGAGATGGAGGAGAGTATCC
TGGGTTATCCAGGTGGGATCTAAACGTAATCACAAGTGTCTCTTTGTGAAAAGGGAGGCAGAGGAAGCTACCGAAGAGGAAGCAGGACG
AGTGATGGTGAAGCAGGAGGCTGGAGTGGTGGGGAGAAGAGGCTACGAGGCAGGGAAGGCAGGCCCTCTCTGGAAGCTGGAAAAGCAA
GGAAACGGATTCCCCCGCCTGATCTTCGGGAAGGAGCCAGCCCTGCGGACAACCTCCACGTTAGTCTAGTGAAACTGATTCCACACA
TCTGGCCTCCGGAGCTGTAATATAATAAATCTGTGTTGTTTTAAGTAATGTGTTACAGCAGCAATAAGAAATGAGTGTACGTGGACT
CTGGGGGCCAGACTGCCTGGGTCTGAGTAAACCACTTACCAGCTGTGTGACCTTGGGCATGTCACTTAATCTCTTCATGCCTCAGTTT
CCTCATCTTTCAATAATAAGGCCCACCACCACAAAAGGGCTGCTATGAGGAGTAAAGAAGCTAGTATGTGTAAGTCATTGAGACTAGCAC
CCGGAACCTAGTAAACGCTGTGTTAAGTATTTGTTAAATAAAATAACTAAAAAGCACGTGAAGGTGGCTGGCTCACAGGAGGCTGTG
GGGTGATGCGAGTGGATGCTGACGGGTTTGGCGGCGTGGGCTGTGCTCCGTGGAGTCAGTTCAGGAAGGCAGGGGAGTATTTGGTGC
CTCTCAGGAAGACTATGGTAATCTTCATGAAAATGAACCATGGATGCTTGAAGGAATGAACCCTCCATTGTCTGTTACACTGAATGC
CTCACACCCTGCATTTCCCAGTGTTCCTTGAAATATTAATAGCTGTTACTTCAAAACAAGAGTCAAGGTCAAATAAGTTTGAGAAAC
TGCATTTCAAAGCCGACTTGTTTTCTTTTCTTTAGAAGTCTCAGAAGCTTTAATATGCTAATGAATCTCTGATGAAGCA
TTTTTCAAACATTTGGCCACTCAACCACTTTGTATGGTGCTTCCAACATGGCCCAGCACTCGGCAGAGGCGCCTCAGAGCAAGCCTG
TATACCTGTGCCTGCTACAGAGGTGTGTGAAGGTGGGACAACCCATCTGAGAGGCAGTGTCAGGGGCCTGTTTGCCTGACCTCAGAA
CTAACTAGAAAATGCAGCTGCTGCTGCTCCTGTCATTCATATCCCAGACAGTCAGAAAGGGTTACCGACAGGCCCATCCATCCCTGT
TTCCTTCAGGGCCTGGGCCAGCCTCCAGTGGAGGCGCCTAGACCCTGAGGCCCCAGACCCTAGCATGCCCCTCTCTTGCCTCCCTCC
TGACCCAATCTAAGACCCTGAGGGCACTGTGGATGCCACAGCCCTCAGATCTAAACTTCATCCTGCCCCCTCCTCCAGCTACCCCTT
GGCTGCTTCTCAGGCTTAGGGATGTACCTACCAGCAGCCAGCGTCAGCCTATGGGAGGACAGAGAAAGTGGCACGCGTGGGCCCTGGA
AGCAGGCCTGGGGCCTTTTTGGACAGGGGACTCTGGGCGTCTGGATATCCAAACGGGGGTTTAGAAGGTGGAGGCAGACAAGGCCTC
TTGGCCCTGTGGGCTTCTGACTCCACGGGGTGTGGTGTGACTTAAGGAGGGGTCAAGCAGGGCCCTCTAGTGTGGGGGACCCAGAGC
CAGAGCTCCAGGTGTCTGGATCCAGGGCAGTACCTGTGGGCATTGAACACACAGCAGCACAGACCCCGAATTGCAAATAGTGCCCCC
AGTCACCAAACTGGCCCTCTCCATAGCCATATCTTTGTTTGCTTCTGCGCATCTCTAGCAGACACGCAGTTCTAAGCTCCTGGTTCA
TCAGGAACTTGCATCCTGATCTCCGCTGTACCCCACGGGTCTTGGCATTGTACCTGGCACGCTGAGGCCAGATCCGCAGTGACCTCT
CACCAGCAGCAGTCGCTGTGGTGATGGTGGCTGCCGCATGCCCTGGCCCTGGCCCTGGCCCTGGTCCCTCCCTCCCAAATG
GAGGGAATGGTGGAGCAGGTTTCCTTTCTCAACCGTGGGGAGGGCCACCACTTCCCACAGGGAGCCTAGGCCTCTGCCCTCAGTGGG
GGAGCGGAGTGGGCCACTGACTAACGCGGCTTTCAGTGCCTACGATAGGAATTCACATGGAGAGGTGGCCTTGAAATAAGTGCCTTG
TTTAATCTTCATGCCCTGGAAGTATTTTTCACAAGGTGGCTTAGTTAGAGGGGCTGTGGATCTGGTAATGAGGCTGGCCTTGGAGGG
GGAGACTTGTGAGAGGAGCTCAGCTATGCAACACTTCTAGCAGGGCTGTATTCATAAAATCCTGGGGGCTCCCAGGGGATTTGAAGCT
CTACACAGAAAATTACAGGGCAGGAAAGGACCTGGGCAATTATTTTTCCTCCAAGAATGGCCATGCTTAAAACATCACCATTAGGCG
CCACAAGTGGCTTTCTCCATGACAGCTCTTTTAAAAAATACTTTATTGGGTTTTTTTAAATTACAAAAGCAATATAGGCTTCTTTTA
ACACAGAGGTGTGTTCTCATTATCAAAAGGAAAAAAATCAATAGGTTCCCTGATTCCCATCTCCCTGCGTCCCCAGTAGTACCAGTG
TTAACAATTTAGTGTTTAGCCAGAGGTGATTAATGCACCTTTTGTGTGTGCTTATACAAAAATGTACCAACATGCATACACATATAT
GGGCATTTTTGTTTTTATGTGTTGGTGTTTGCTTTTTTAAAAAAGAGGAATCACACTATACCCATTATGCTGTAAGTTTCTCTTTTT
ACTTAGCAATATATCATGAACATTTTCTAAGTCAATACATATAGATCCACTTCATTTTTAAAAACAGCTACATAATGTTCCCCAGTA
TGAGTACCATAATATATTCAGCTGTTTCCCTATTGATGTGTATTCAGGTTGTTTCCAGGGTTTTTTAAAAAAATTATAATAAGCAGTT
CTTTTAAAATATTCTTTGATTTCTTTTTCTATCTTGTGCATATGTTACAAAAGACTCTTTTGCCTTAGGAGGATTATAAAAAATGGTG
AACAATTAAAATATTTCTTTACAGTCACATTGATATAGTTTTGACACCTTAAACCCTTTATGTATTCATTTGGGTGCCCTGGGCGC
TGTGCCTGGCACTGTGGAGGACAGTGACCAAGGAGATGTCTGTCCTCAAGGACCCCTGCTTTAGTGAGGGTGCTGACAAGCAGGTGG
ACAGGAATGCGGCTCAGATGGACGATCAAGAAGGAGGTTTCGGCCAGAATGGAAACTTAATAGATGAGAGTGTGGCCTCTTGGTTTA
AATCCTGTCTCCACCACTTAGTTGCTATGAGGCTGGGGGATGTTCACTTTTCTTATGGGGCTATTGTGAGACTCAAAGAGTTGATG
TAAAGAAAAGTGCTTAGGAGGCCAGGTGTGGTGGCTCATGCCTGTAATCCCAGCACTTTGGAAGGCCGAGGATGGCTGATCATATGA
GGCTAGGAGTTTGAGACCAGCCTGGCCAACATCGCAAAACCCCACCTCTACTAAAAATACAAAAATTAGCCGGGTGTGGTGGTGCAC
AGCTACTCGTGGTAGCTGGGTACTAATCCCAGCTACTCGGGAGGCTGAGGCAGGATAATTGCTTGAACCCAGGAGGCAGAGGTTGTA
GTGAGCCATAATCGTGCCACTGTACTCTAGCCTGGAAGCAGAGTGAGACTCTTGTCTCAAAAAAAAAAAAAAAAAAAAAAAAAAAAGA
AAAAGAAAAGTGCTTAGGCCCCAGCCAACAAGTTGGACTCACCCAAATGGCTTGTTGCTGTTTGAAGTGCTGCCCCCGCCACTGGGAG
ATCAGGAAAAGCTCCCCCTGAAGGGGTGACAGTGCAGCTGGCCCTTGGGAAGGAGGTGAAGGGCCTTTTAGTCAGTTGGGGAAAAAA
CAAACACTCCCTGCCCACCACCACCACCTGGTCCTGACCTCAGTTCTTCAGCTCCCTAGGCTGTTCAAAGATCCAAAAACCTATTTA
TTTTTAGTTGCTAAAAATATCCCCTAAGCCAGGGTTAAAGAATTTCTCCCTTAGTGGTAAACAGGTGACTTAGAACCAATCAGGGCC
CAGCAGCCCCAGTGATGATGCTGTTCCTCCCCTACCCCCAAACATTCAGCAGGATCCTTTCATGTGTCAAAAGGAAAGAAACTTATC
AGAAACTTCCTGTTGAGAAACGATACCTCTTACCCCAGACTTAGCAGATGCACAGAAACCTTTGACTCCTGGATCTGCCAAGGCAC
ACAAAGAACCAGAAACACCTATTGGCCTGGCCTGGGATAGGCAAACACTGGGGCTCAGCCCAACAAGCCTTCCTAGCACAGCCTGGG
GCAGCACACACAGTAGTCAAGAGAGCTGCCTGCCCTGGCTGTAGAGCCTAGGTCTGCCCCTTATTAACTGTATGACCTGTAGTAAAT
TATTTAGCCTCTCCGAGCCTCAGTTTCCCTGTCTGTAAAATAGAAATAAGAATACCTGTCTCAAAACCCTGTTAAGGACTAAATGAG
ATGGTGCCTGTAAGGCACACAGCACAGGACCTGGTGCACAGTGAGGCTGCAATACATGGCCCCTGCTGTTACCATTGTTAATGGAGA
CCGCCGCCCTGCTCCCTCCGCCCCCTGAAACGGGGGCTAGCACTCCCCAACTGTCTGGCCATCCTTCTCCAGCTGCCATTTGGGGAT
TGGCTCTGTGACAAGCAGGGGGCACAGGGCCTGGGGAATACTGGAGGAAGGCACCCAGCCCCACCTGGGAGTCAGAAGGGACCAGCT
TAGGCCTGTGGAAGGGTGGGACGCTGGATCTCGGAGGTCTCTGGTGAAGAAGCTGAGAGGGGAGTGCTTGTACAGAAGATGGAAGG
GCGCAAGCTCAGGGGTTACTGGTAACTTGGTTTAGCTGGGATGGAGCACCGGGTGTGGCAGGTGATGGGGGATAGAGGGGCCAGCTGG
TGCCAGGTGGGAAAAGGCCCTTTTGGCCAAGTGCAGCTAGGAGGGTGGACACTCTGGGTTGCCACGGAAAGGTTCTAGAAGGGGAGG
TGACATGATTTGGTTTGTATTTTAGAACATTTGCTCTGGCAGCAATGTAAAGATGGCTGGGGTGGAGCACATGATGAGACCAAGGTG
TGGCCACAGCAACCCAAGGATTGGCCGGGGGTCACTTGGCCTTACTGCTTCAAATATAATTGGTTACAAACTCCCACCCCACACAGGA
GGGGACCCGGTGTCTTTCCACTGGTCTCTGGGGGCTTGGAAACTTCCATTTCTCTCTTGACAGCTCATCTGATGCCAAACTCTTTCT
```

110

```
GCTGAATTCTCCACCTCCATCCTGTACTTAGTAACCGCTTTAGGCACCCTTCCTGTTCCTGTTGCTCCTCGAGGGACCCATGACCAA
GGCCTCTAGGTTAGCTGACATTTCCATGGATCTGGAGCAAGGGGAAGGGGTGAGGTGGTGGTGGCACAACAGATAAGACAGCGCTGA
ACGCCACATGCTCCTGATCTTACAGTCTCAGCACTGTCCCTGTCTCATGGGTGGTCAAGTCCTCAGTGGGCTCCGGCCTCCCCCTCT
CATGGGACTGGCCCAAGGTGCTGCTTAGCCAGCTCCCCTCTCCTTCCAGTCCCACAGCTCACAATCCCCCATCTCCAGTGTTCCTGT
TCATTTCTAGGGCACATATATTAAGCCCTGTGTTGGTCTCCTCTGACCCCACCCCAAGCCTGTCCAGAGGAATCTTCTTCATTAGCA
AACATGCTGGGGTGCATAAGTGGGCTCCTAAACTAAGATGGTCTCTTCCTCGCTAGGGACCCCATCACCAGGTCTCCATAGAGGCCT
GTTCACTGGCGGGAAGAGGAGGAAGGGGAAAGATGATAAATGGATATTGTATCTGTGGAAATGAAAGCCGTGGTCTTAGTTCCACAA
CATAGCCCAGTCTAGATTACAGATAACGATGTGGGTTTCCCAGGTGGTGTGGACACCAGGGATAGAACCCAGTTTATTTTAATCATA
TATTGATTCTCTGGTCAGCAAAGATTCTTCTGAGAATGAGCATTTAAAGGGTGGGCTTGAGTTTTCTTTCTTTTCTTTTTTCTTTTT
TTTTTTTTTTGAGATGGAGTCTCGTTCTGTCACCCAGGCTGGAGTACAGTGGTGCGATCTCGACTCACTGCAAGCTCTGCCTCCCGGG
TTCAGGCCATTCTCCTGCCTCAGCCTCCCGAGTAGCTGGGACTACAGGCGCCCACCACCACGCCCGGCTAGTTTTTTTGTATTTTTA
GTAGAGATGGGGTTTCACCGTGTTAGCCAGGATGTTCTCAATCTCCTGACCTCATGATCCGCCCGCCTCGGCCTGCCAAGCGCTGG
GATTACAGGCGTGAGCCTCCGCGCCCAGCCGAGTTTTCTTTAGAAGACAAAGTAAAGCCCAAACTGGCCAGCCCCCAAATAGAGTGC
CTGCTTAGCAGGCTGGCAGGTGAAAGGGCGTGGGACTTGGAAGACACAGAATGCTGCAAGCCCCAGGCAATTGATTGAACCTGTCTT
GGTTTGGGTTTCTTCATCTGCTAAATGGATGTATGACTCCCTTACAGGGACATTATGAGATGGCAATGAAATGGTATGTGCAGAATC
ACCTGGCTCGGCCCACAGTCTGGCTTTTGGGTAATTTTGACACAAAAGGAAATAGCTATGAACTGAATTGTGTCTCCCCAAAACGCA
TTTGTTGAGGCCCTAACCTCCAATGTAACTGCCTTGGAGAAAGGGTCTGTGGGAGGTAATTAAGTTTAAATAAGGTCATAAGGATGA
GGTCTAGTCCTATAGGACTGCAGCCTCTAGGAGAAGAGAAAAAGAGAGATCCCTCTCCCTTTCTCTGCCATGTGAGGACACAGTGAGA
TGGCAGCCACCTGCAAGCCGGGAAGAGAGCCTGACCGTGTTGGCACCCTGATCTCAGACTTCCAGCCTCCAGAACTGTGAAAAAATA
CATTTCTGTTTTTTGAGTTACCCAGTCTATGATATTTTGTTTCGGAGGCCCGAGCTGTGGTGCATATTTAACCTGTGGGCAGTGTGT
TTTCAGAAAGTGGTGACATGGTGCACATGGCCCTGGTGGGAGTGGGTCTGTGGTCTCCTGTCCTTTGAGGAAGCTCCTTCTGTGACA
TCCAAGGTCCCAATGATCTGGGTCCTGCTACCTTCTTGGTCTCACCTGGTATCATCCCTCACTTCTTTCTCACATTGGTCTTCTTTC
TTTTATCTCTCCAACTCTCTAAGCTCCATCCTGCCTTGGGGTTTTTGCACATGCTGTCCCCTCTGTAATGCATACCTTTGCAAGGCT
CACTTTGGATTCAGAGGTCCCTTCCATAGAGAGCCCTCCCTGTCCTGGCTGTCTGGGACTTCTCTCATTTCCTTTGCCCTGTCTTAT
TCTCTTCCTGGCAGGCATCACTCTCTAAAATTAAATGGTTTACCTGTTTAATATCTGTTTTCCTCCCCTGAAATGTAAGCTCTGTGG
GGACAGGGGCCTTGTCTACTTGTCTTGCTATATCCCTAGAGCTTACCAAGGACTGTCACATAATGGGGGCTCAACTCATGCTCAATG
AATGAATGAGTGAATAAGCTGATCGGGGCAAAGCTCCCTGCCTGTGCATCAGAGTGAAAGTTTTTCAGGTTGGTCAGAAAGTGTCTAAGCAGGAATAC
CAGCATCACACCTTGTCTCTCCTTACAACTAGAGACCAGCTGGTCCATCCAAAGGATTTCCAGTCAAAGGGTTCTATGGAGAGCATG
GGGTCCACTACAGAGACCTAGGCTGGAGGAGGAGGTGAGTGAGTTGATCTTTGAATGTTCTTCTCTACCCTTTCAACTACAACAGCT
CTGAATTTGTCTGTTTCCCATGTTGGGCGTACATGAAAAATCTTATGAAAGAAATGGCCCCACAGCCCAGAGAAAATGGAAAACCAC
AAATTGCATCCAAGTCTTCACATTACACTGAGGAATCTGAGGTCCAGAGACTTGCCACACACAGATGGCCAGAGGTGGGATGCTCTA
TCCCTAGTGGTGACGTGCTCAGGGATCAAGAAAATTCCTGCTACGTGAGTAAAAAATAGTGGCTTCGCTCAAATGCCGAAACTTGGCTT
TCTTTCTCTGAGAACTGAGGACAGGGGAAAGGATTTATGATCACCACTGGTCTCTCGTTAGTCCCAAACAAACTTCAGACTCCATAAC
CCCCATGAGTGGTCATTACACACTGCAAGAGACCAAAGTGAGCTCTGGCGAAGCGCTCAGGAGAAGCCTATCCTGGCCGGACCAAA
GAGCCACTGTGGGACATCACACATGGCCCAGGGAGTGGGATGGTTTCAGATGAGGACAATTTCTTCTTAGACACAGAGCGGGGTAGC
GTGGACCCCAACGCCCTAAGCATCTTGTTTATGGTAGAATCATAGACTTCCCTGACAAGGCCTGTTCACTGGCTGGCCTCTTTTAGA
GCCACCATTTTTCCACCTTTGCTTCCCAACCCCCACCCGGCTCTTCTTCCTCTAGTTCCATGGAGCATTTGCTGACTGGTAATT
TTACCAACCATTGCTGCAAACCCAGAAGTTCATTTCGTCACTGGCAATCAGCTCCTGTCCGCAGGGAAACCTAATTAGGTACTACAG.
GATTTTTGATGTTGCTTCCATGGTAACCCCCGTAGTAATGGTGTACATGCAGAATATATGCTCCAGGGTGTTTAAATTTATATATGT
ATATATATATATATAAAATATAAAACATATTGTATATATAAATAATATATCTTAAGAAAATCTGTAATTTTTTCTTCTTGCTTTGTT
TCTGTGTCGTTTTTTTAAATGATGGAAATTTTCCCGGAAGTTCTTCTCCCCACAGAGAGAGAACAGTACACAAAAAGGCTTTTTCCA
TTTTCTCTCCCTTAATTTAGAGAAATTCACAGTTGTGCAAGAGTGAAAGTTTTTCAGGTTGGTCAGAAAGTGTCTAAGCAGGAATAC
GCTCTCCTCCCTGATAGCTGAGAACATTTTAATGACAGATTTTGCTTAAATAAAAAAATCAGAAAGGTCTCAAGTCTTTCAAAATAGA
ATGGGGGGGAAAACGAGTGAATCCAATGAATCGACTCTAAACTTATCCTAAAAAAAAAAAAAAAAAAAAAAAAAAAAAGGAAAAAAAGAAA
AAAGGCAAGCTTCCCTGCATGCCTACCCCTTTGCAGGAAATGTGGGGAGGCCTCGCAAGTCTGAAGCTCGGGCTGTGCCATGTGGCT
GCTGGAGGCACTGGGTAGTGTCCTTGTCTCCTTGTCAAGGCAGCCTGTCCCAGGCCCTGCAGAGATTGCAGCTGAACGGCCCTGTCC
TGAGGTGTCCAGATGGCCTTCAGCGTTGGCCTTGTGGTGAGCTGGTGATGACATCTTTGCTTGGCAGGACACTCTAACCAGGGAAAG
GTCAGACCTGGACTCCCTGAGGTGCAGGTGACAAGGGAGAGGGCCTGTGACACACCACAGCTGAAAGCCAAGGGCTTCTGAAGAAT
CCCTGAATAACTGAAAGTGCTTTCTGTACTATCCCTCGCTGGCCGTCCAAGGATGGAATGCCATGCTGACCTCTTTGTTAGCTGCCA
GCGAATCCATTTACCCTCAGCATTTGCCAACAAGGTCCACTGCTCCCAGACTCCCTGCTGTTTTTCTCCAGCGGCCCCCCTGCCCAT
TAATTCTGTTGTGCCCTTGTTCCCTGATTCAGTCTACCAGCTGACTTCCTCACATCTGAGAAAACACTTTGTATGCAAAACAACTAA
ACTGCTTGCAAATAACTTTTTAAATGCTCTGAGTTATTCTTTGATATCTCAAATCATGTGTCCATGGGGAGAGTGGGGATGGGGGA
ATGGCCATCAAGGAATGGCCCTTGACCCTGATCTCCTCCCCATCCAGTCACCCAGTTACTTGACGAAGGACTGGCCAGCAAATATAAC
TGAGTGGAGTGGGAAGGTGGGGACGGGTGAGGGTTCCTGCTAGGGGCTGTGGCATAAGAATCTAAAAGGAAAATGTTGGGCACAGTA
GGCACATGACCTGGCTTTTCTAATATGTGCCAAATATAAGAATTAGTTTTGTTATATATTAGTTTTGTTGCATAACAAAGCACCTCA
GTCTCACCTTGGGTGAGCTTCTGTCATGCAGCTGGTCTAGGACGGCTTGATGGCTGGCAGCTGGTACTGGCTGGCTGCAGGCTGGGGTG
AAAGGGATAGTGAGGCCATGTGTCTATAGTGAGCCTCATGGCGATGACATTTTTTAAACCTCTGCTGTATCACACTTTTTAATGTTC
CACTGGCCAAAGCAAGGTACATGGCTAAGTTCAGACGGAAGGGATTGAGAAATAGGCTGTACTGGCCAGGCGTGGTGGCTCATACGT
GTAATCCCAGCACTTTGGGAGGCCAAGGTGGGTGGATCACTGGAGGTCAGGAGTTCGAGACCAGCCTGGCCAACATGGTGAAACCCC
ATCTCTACTAAAAATACAAAAATTAGCCAAGTGTGGTTGTGGGTGCCTGTTATCCCAGTTACTCAGGAGGCTGAGGCAGGAGAATCG
CTTGAACCCGGGAGGCAGATGGTGCCATTGCACTCCAGCCTGGGTGACAGAGGAGTGAAACTCTGTCTCAAAAAAAAAAAAAAAAAAA
AAAAAGGACAGAGAGAGAGGGAGAGAAATAGGCTGTACTTCTTGATTGGAAGAGAGGCAAAGTCACATGTACAGAAGTAAGTGA
ATGGGATGGGAGGAATCTGTGGCCTTTTCTGCAAGCTGCCCCCTCTGGTGTGTCTGATGCATGCCTTCCGGGCGCTGCTGAAGCCCT
ATGTAACCCAAGAAGCTTTTCAAATTGCATCCTAATCATGCATGTCTTTTGCTAGAACTACTTGAGATTGTGCACTGATTCATGGGA
CATGTGTTTGATTCAGATTTATATACCCACAATGGCCTAACCTTGTTGATTCTTAATAATTATTATATTGAACTGAGTTGGGTTAGT
TCTCCTTTTTCTCTAATTACAAGCTCCTTAAAGGAAAGTTTGGAATCACTGTTGCATAAAATCTCCCTCCCAGAGCTCTCAGAATTA
TACTAACGGCTGAAAATCCGGCAAGTTCTCAGAACACCAGTTAAGTGGTAGAGGGATTAGGCCTAGAGCCACCTCTGGACAGAGCA
ACTATCTGAGGAAAGAATGGAATTTTCAGGAAACCAGAGACTGTGTACTGACACATTGCATCTTCAGGTCAGTCATACCCTCAATG
AGTGAAATCACTTTTTTAGGCTTAGGAGGCAGAAAGAGTCAGTGACTCGAAGAATGTGGCTTTCCTTTTCCCATTCAGGGAAGCCAG
AGTACAAGGAGCAAACCTATGCCTCATCAGGAAGAGACTCGTTTGTCAGCCCTGGGCCTCTGTGGCTTCTCCTGCTCTGGAACAGGC
CAGAACGACCACTTGAGGGCCCACGGTACTCCCTGCCCACACTCTGTCCACATCACCTCAGTACTTCTCCAGAATCCATC
AGAGGCCAACAGTCAAGCTGGTCTGTAGAATCAGGGCTGGATGACTGAGACTAAGCCCTAGGGGAGGATAGTCCTTTGTGGAGCCACAT
ACACACTCCCCGAGCCCCTCTGGTCCAGTGCTACTCAAGATCCTGGTTCCAGACTTGCTCTAGAACTTTCTAAGGCTTGGAGGTCAC
CTGGGACAGTTTATTCCAGGTTGAACCACCCTTTGGGAATAAGAGGGTGTCCCTTCCCATGGTGTGAGATGAATTGTCAGGTCCTTT
GTGAGTCACAGGCTTAGGAATGGATTTTCAACAGGAATTTCCATTCTGGGTGGTTAACAAGGGCCACAGGCAATCTGTCCTTTTCTT
```

```
CATATGACACAGTCATCCGCCTACACACCCCTTTATTCACCAAGATTCTACTGACTGCCCACTCTGTGCCAGGCAACGTCTCAGGCA
TGGGAGTGGGGTGAGATGAGCCAGGCACGGTTCCTGTCCCTGGAGAGCTCAGGTGCACCGAGAACACAGATGAGGCAGGAGGTGCGA
GCAAGAGGGTCTGTGTAGAACCACAGAATTATGGCTTCTTAGAGCAGGAGGTACCTTGGGGATTGTCTAGTCTATTCCCTCATTCCT
CAGAGGAGGACGCTGAGCCCAAGAGAGGGAAGATTAGTCCAGGGGCACGCAGTAAGTGGGAGTTACTAATTCACTAAATAAGCATTT
ATTGAACACCTACTAAGTGCTAAGATCTGGGCTAGGCTCTGGAGATGCCACAGGGAACAAGGAAGACACAGCGCTTGTCTCTTTGGA
GCTCCAACTGTGACTGGTTGGGGGGTCCCAACCCTGGTTACATTTTAGACTCACCTGGGAGCCTTAAGAAGAACACCCGTGCCAGGG
CTCCACCCCCAGAGATTCTGATTACTTGGCCTGGGGTGGGCCTGGGCCTGGGCATGGGTATTTTTGAACAAGCCCTCCAAGTCATTC
TAATGTGCGACGCAAGTAGGTTGAGAACCATGTCTAAGGGGAGAGACAGCAAAACCTCACACATGCAAATCAATAATTGGGATTGTG
CTTTGTTAGGAATGAATGGGACCCAGAGTGAGGAAACAGCAGGGTTGGGGTATCTGCTACTTGCTCTGAATCGGGAGGGAGCGAGGC
CACACTTGGAGAGCTGTGATTCCAAGGGTCAGAAGGAAGGAAGCATAGAGTGTCCTCACGTGGGACAGCAATGGGATCTTCTGGGAC
ACATCCCACCCTGCAGATGCAGAAAACCTTGCAGCGGGAGCCCTGTCTAGCAGAAGCTTTTAACCTGCAGCCTGAAAACTCTCCCCCA
CCCCCTACTCAAAACACCCGCTCTTTACACACACTGTTTACGAGTGCCCCCAGGCCAGCCAGCTTAATAATCCCTCACATTTTTAGA
GCAGTGAGTAAAGCACTGTCCAACTTTTTATCTGTATTATTGGATCCTAACACTAACCCCAGGAGGCAGGCAGGGCCAAGGTCAGTC
AGTGGTTCCCCTGTTGGAGATGTGGAAACAAGCTCGGAGAAGTCACGCACGGCCTGAGATCACATGGCTAATACCTGGATGTGGGGGA
CTTCAGGCTAGCTCTCCCACCTCCAGCTCCACTACCCCATGTGGCCCCCACCTTCCCTGCACCAGCAAGCCACAACCAAGGCGGGGC
ACCTGAGAGGCAGCTGGGGACCAGCACCCTCCCGTCTGCGGTGCATTCTCCCCTTCAGCTCTTACTTACAAT
CCCACTCCTCATCAGGCAATCAAAGGAATCTGTTACTCGTTGCTTCCATTCCTGCCCAGAGTGAACCCAGTTACTATAGTAACCAGA
TGCGATTCTTTATTACGGAGTAGATGGTTTTAATTTTGTATTTGAAGAGTTTGGCCAGGCAGTCACGTTGCCATGGCTTGGCCTGTC
GGGCGGCAGTTAGAAGCAGAGGGAACCCTTTGAGCAGCCATTAGGGCTTTCTAATTGCTGGGGAAAACCTATGTCTGGAAAAACCCA
GGCTCAGCTGCACTGGGGAAGGAGGAGGGTGAGTCTCCAGCTGGTGCTGTGCTGGGTACTGTCTCCTGTTCAGAGATGACAGCTCCC
TGATTAGAGCCACCAGGTGAGCCTCTCAGCCAGTTGCTCCCTGTAGTTTCCCTTCAGGATCCTCATGGGTGGGTGGGT
GGGGGGCTGGCTGCTCAGAGGGCCATCAACACATTTTTGTTGAATGAATGAATGAATGAACAAATGATCCTTTGTAGTTTATTAAAC
CCTTTCAATCCATTGTCTCCTCTGCTCTCCAGTATGGCTCTGTAAGGGTACGGAGCAGGGAGAAAGAGAAACTGAGGCTCGGCAAGG
TAAGAGACTTGCCTCCTAGTGCTGCCTCCCCTGTCTGTGCCTCTCAGGGCCCATGAATTGCCTGGAATGTTCCCCTTGCTGCAGAAA
CATATCACAGGTAGATGCTAAAAGCAAGGCAAGCATCGCCCGCCCACACCACACCAGGCCTGGTACCATAGGTCCTCTCCTGGGTCC
TCAGTTCTTGCTGAACTGTAAGGAAGGCAGCAAAGTGGCAGTCTGCTTGGTGACTAACTTTGAAGCAGAGCAGAAGCTTCC
TTTGCCCAGACCATCAAGGAGACAGTTGGGTGGACAGAATAAAGGGCTCCTCATTCCCTGATCTCCCTGTGCAAGGGAGGGAAAGGA
AGGCTTCACTCGGCTTTCAGCCAGCCCCCGCAAATCCACGCTGATCCAGTCTCTCTGTTACGCTTTCTGTGCTGAGGGTGTATTTCC
AGGTTAAATAAAACCTCCACCCAACCCTGGCCCCAACATTAATTTCTCCTAACCCAGCCTAGTTTCTTCCGGCCTTGCAGCTCAGGC
CTTCCCAGGATGCATGCATGGCCTGCTCTCCCAGCCAGGAGGTGCTGATGCTGGTACCCTTCATCCTGAGCTCATGTTAGGCCACAG
GACCCTGCTAGCTCCTCCTTAAAGGCAATCATAACCAGCTTTGGAGGCAGCAGCTCTCTGTGCGTGTTGGTGGAGCAGGCAGCATG
TGAGGCTGGAGTCTTGAGTGGGATGTGTTCGTGGATGAGCTGGGACAGGCAACAGCCAGATCTGCTGGAGGGCCAAGGACCCAGTAA
AGAGAGGGCATAGTGTGATGATAAACATTCCACTACCTCCCAGAAGTGAGGTGCCTGCACAGGAGGCCCACACCGAACATATGCCCG
GATCTCAGGCCTCACCCTCCAAGAATCAAATCATCGGAGCTGGATGAGACTTTAGAGGCTTCTCATTTCAGTCTCAGATGAAGACTG
AGGCCCCAGAGGGGAAGGATGTGAAGTCCCTTTCACAGTCATCAAATATGGTGGGACTCCACAGTGGGACTTACCGGAAGACAAAGG
CTGGACACACAGCAGGTGCTCAGGTCTCAATTCTTGTGGACAACGCCATCCTCATGAGGTGACAGGGCATCTTCGTGAAAGTTTA
GGGCCAGAAGGAAGTTTAGACATCACTGAGCCCCAACTGTCATTTTACAGAAAGACCGAGACCAGATCCATTAATAATGCTACAAATA
AGAATAGCTAATAGTTACATAGTGTGCTGGGTGCTAGGCAGGATTCCATGCATGTTGCATATACGAACCCACTGCAGCCTCACAGTG
ACCTCTATGCAGCAGTTATTATCATCATCCCCAGATGAGGTAACTGAGGCACAGGGTGGTTCGGTAATTTGCCCAGGATCATGGAGC
CAGTAGTGTTAGGATCAGGCAATCTGGCTCCAGAGTCCGTGCCGTGAACACTTCACTGCACTGTCTGAGAGCACAGTGCTGCCAGCA
AACACTTGCTGCCCCCTTCTCTGAGTGGGGAGAAGGAATCTGCTGCCAGCCCTTGGTGAGTTCACTCATGCAGGGAAACATGACTTTTTC
ATCTTTACATCCCCTTACAGGGCTCAGCTCAGGTCTGGCAGGGGCTCAGGGATGTGTTTCTAATATAAATGAGTTGATCTTTCTCAC
CATCGTGAAACAGCCAATTTGGCACCTGTGTGCTGGAAGGGACCTTGGGAAGTGGCTCAACATAGAACACTTGTGGTCAGCAAAAAG
GAGGGGGTGGTTCCCACACACCAAGCAATTCTCCAATGGACACTGGCTGAGTGTCCTGCAATATAACTCAAGCCTGATTGTCCGCCT
GGAGTTAGTGTTGGCTCCCACAGGTTGAGGGCTCAGTCCCACCACATTTACCTGACTTCCGATGCTAGTCACCAGCCCCAGGTTGTT
TTATCTGAGCCTCTGACCAAGTGGCTATAAATCAGGGATTCCCAGGACGCCCTTCTCAGGTTTTCATTAATTTTCTAGAGCAGCTCA
CAGAACTCAGGGAAACACATTTACTGGTTTATTATAAAGGACCTTACAAAGGATTCAGATGAGCAGCCATATGGGAGCGAAGCAAAG
GGCAGGGCGCATGGGAAGGGGTGGAGCTTCCATGCCCTCTCTGGGCACGCAACACTCCACGTGTCCAGGGACCTCCATGTGTGCAGC
TATCTGGAAGCTCATCGAAACACAAAGAGGGGAACGACAGACACTGAGGGTGGAGAGTGGGAGGGAGAGGATCAGAAAAAATAACTA
TTGGGTACTGTACCCAATAGTACCTGGGTGACGAAATCATCTGCACAACAAATCTCTGTAACACGAGTTTACCTATATAGCAAACTT
GCACATGTACCCCTGAACCTAAAATAAAAGTTAAAAAAACAAAACAAAACACTGTCTTTTTGGGATTTTGTGGAGGTTTCATTACAC
CGACATAATTGATGACATCATTGGCCATTGGTGATCAACTCAACCTTCGGGCCCCTCTTCCTTCTCAGGAGGTGGTGGGGTGGGGCT
GAAAGTTCCAACCCTCTCATCACCAGATTGGTTCCCCTGGCAACCAGCGCCATCCTGAGGCCACCCTGGATCCCCACCCCGACCGAC
CCCAGCCACTAGTCATCTCATTAGCATACAAATGACACTTATCACTTTGGAAATTCCGAGCGTTTTGGGAACTGTGCCTAAACCAGG
ATAAAGACCAAGTACATTATTTCTTATTATAAATCACTATATCTCAGGCAGCGATTTGCAGAGGAGGAAAGTGAGGCCAAGAGAGTC
AAAGTGTCTTAGCCAGGGCTTCCCAGGTAGACTGAATCCAGCTCTCCCTACTCCACATACAGGGCTCTTGCCACATAGCAAGGCCCG
TAAAGAGCATGCTGAGGTGGTCAGGCTGACCTGAGCCCAGCAGCCTGCCCCAGCCTGCCCCACCTGTCCTGCCTGGACCCTGCTGTG
GGCTTCCTGGTCCTGGCCACACTAATGGACTTTCTGCTCCCACCAGCTCTTCGTGATCGACAACGGCGCGGATGACTGGCGGATAGC
CATGACCTACGAGCGCATCCTCTACATCAGCCTGGAGATGCTGGTGTGCGCCATCCACCCCATTCCTGGCGAGTACAAGTTCTTCTG
GACGGCACGCCTGGCCTTCTCCTACACACCCTCCCGGGCGGAGGCCGATGTGGACATCATCCTGTCTATCCCCATGTTCCTGCGCCT
GTACCTGATCGCCCAGTCATGCTGCTGCACGCAAGCTCTTCACCGATGCCTCGTCCCGCAGCATCGGGGCCCTCAACAAGATCAA
CTTCAACACCCGCTTTGTCATGAAGACGCTCATGACCATCTGCCCTGGCACTGTGCTGCTCGTGTTCAGCATCTCTCTGTGATCAT
TGCTGCCTGGACCGTCCGTGTCTGTGAAAGGTATTCCACATGGGGCTTTCTGAGTACAGAGCCTGTGCTGGGACAGGGAAGGGAGGA
GGAGTAGGGGTGCCAGGAAGTATGTGGTCTGATGGAGGAGACAGGCATGTACCCAGACACCTGAAAGGAGACAATGGGAAAGAGGGG
AGTGCTGAGTTGGGGGTTCAGTATGAGCTAGAATTGCAGCATTGGAACCAAAGTAAGGTGGGAAAAAAGGCACAGAATTTTCCTGATTT
TCTGTTCCTTCTTTTGCAAACCAATATATCAAAGCATTCTGGTTACATCAGTAACTCACATATAACACATTGCAACTTACTCCTCTTG
GGTATTAATAATGCTTATTAGCATTTTAAAGGCTCTGAGAAGTCCTGAAGTAATGAAACCATTTATTTTTGTTTGGTTCAGCCTTTC
TTAATTGTATTTGCCTGCAAATCCTTTTTATTATAAAGCACTATTGGTACCTTGCTACTCAAAGTTTGGTTCACAGACCAGCAGCAC
TGGCATCACTGGGAACTTGTTAGAGTTCCCAGAATCTCAGGCCTTAGCCCAGAATGAATGAATCAGAATTTGCATTTAACAAGGTCA
CCTGGTGATTCATATGCACCTTAGTTTGAGAAGCACTGTACTGATGGAACATACTTTGGGAAACATTGTTTTAGGAGGCTGGTGGTT
GGGAGTCATTCAGAGAAGCTACTCATGGGAAAGAAGGGAGATGTGGCCTGACAGAGATCACCAGGGCTGTAGAACAAAATGCCAACTCC
TTGGAACAGCCATCAACAAGGCTCAAGGGCAGGACTGAAGCCACCTCCTTCCACCACCCTCGAGCTGCCTTTAATGACTCTTCTCCT
TGCAAGGGGTCCTGGATTTTCATTTTATTTATTTATTTATTTATTTATTTATTTATTTATTTATTTTATTTTATCTGAGATGGAGTCTC
GCTCTCTCGCCCAGGCTGGAGTGCAGTGGCATAGTCTCTGCTCACTGCAACCTCTGCCTCCTGTGTTCAAGCAATTCTCCTGCCTCA
GCCTCCCATGTAGCTGAGATTACAGGCATGCGCCACCATACCCAGGCTAATTTTTGTGTTTTTAGTAGAGATGGGGTTTCACCATGTT
GGCCAGGCTGGTCTTGAACTCCTGGCTTCAAGTGATCCTCCTACCTTGGCCTCCCAAAATTCTGGGATTACAGGCGTGAGCCAGCTG
```

```
CACCCAGCCCTGGATATTCATTTTAATCTATTTGACATGTGGAGTCTAAATCTAGGTTAGAAACCGTGATTCTGCTCCTAAGTAGCT
GTGAGACCTAGGGCAAGTTACTTAACCTCTCTGGGCTACAGTTTCCTCATTTTAAAATGAGGATCACTTGAGATAGAATGAAGCTAT
GTTTGCTTGAGATAGATTATTTTTATTATGTTGGGAGGTATGGTGAAGCATCCTGTGTAATTTACACAAATCCTGGTAGTCGCCCTT
AATTTGTCAACACTTACGATTATATTGCCAAATCTTCCATGCACCAGTGTCCTAGCAGAGATCAAACAAGACAAAGAAGTCTGGAGA
GGAGTTAGCCTCAACACACCCTACATGGGTGCCTTGAATCGTATCTCCTACCTATTTTTGAGACATTCTGTTTGCAGACATTTCTTT
CAGCGTTTTGGCCAACAGACACACGGTCAAACTGAGAGGGCATTCTGCTGCCGCAGAACAAGGGTTCAGGCTCCAGGATGCAGGTTC
AGGTTCTGAGAGCCAGTGCAGTGTTGGAAGACCTTTCTCACACACAGAGCCCCAGGGACACACCATGCTCACCTATTCAGAACCTCG
CCATTACTCAGCAGCTGACATTGCAACATCTGCTCTGAGTGGGACCACACTGCACAGGGCTTCATGAGCCTTCTCTGGTAAGCAGAC
ACTCTTCCCCCAATGGAGAAAGCATGTGCTTGGGTTCCGACCCCAAACTCTTCATGGTACTGTTAGCTGAGCAACATTGTGCTGGTC
GCTTCACCTCTCTGGACCTCAATTTTCTTATCTGAAAGCTGGACACAGGATTTATTGTTAGAGTCAAACAAGGTGTATATATGCACT
ATGTACTGCAATCCAATGATGGTGAAAGTTAATAATAATAGTGCAGATCCCTTGATAACTCTTGGGAATTTTGTGCTGCCTTCTTTT
AATTAGAACAGCCCAACTTGTACCATACATGGGCCTATACTCTTCCTGTTTTCCTTTCTAAATGATGGAAGCCATGTTGTCTATTAA
CATAGTAATTGAATTTGCGTAGCCCTTTAAGCTATTTGAAGTCTTTCACTTCCTCAGATGCTCTCTGGCATGTATTTCAGATGGCTG
AACATGCAACAAAGTACCTGCAAGTGAGTTTCGTAGTTTATTGAACCTATCACAAGAAGGGGTGACTATTTTTCACTGGAAAATAAG
CTTGTTTTAAAGAATGTAGTTTGCAAAGTACTTAGATGATGCACCTTATTTGATATTGCATGGCCTTCCATAGCTTCCAAGCTGAGG
CTGGGTTAGTTTGGTCCCTGGTGGGCCACTGGGTCTCTCTCTCTCTGCAGACATGGGTCACAAGCCCCCGCTAAGACCAAGGC
ACAGAGGAACAATGAGCAAATCGGAAATTCTCTGAAAGTTCCCTTCTCTTGCGAAAAGGCCATTTGACCAAAGACTTAGGTATAACA
TGAGGTGTATTAAAGCATGGTGGATGTTATTGTTGCACCCCTGTGCTAGTTCTGTGTAAAATCCATATGTCATCCCAGCTTGCCCTC
ACAATAGATCTATGAGAGAGGTACCTTGCCTGAGGGAAGGAGAGCCCGGGTCCTGGAGGGGCATTTGGGTGAAGCAACATGTCATCC
ACTACATGGATGTGTTTGAATTGGACATGAAGGCTCATGGGAGAGTTGAGGGAAACTGATAGAAAGTAACTGGCCCGAGGTCACAGAG
CTATTAATAATAAGCGTGGAGCCAAAATCTGAACTGGTTTCTGACTCTCCATGTGTGGTGTCACCAACCCAGGCTGAATCCT
GCACATACGAGGAAATCTTGGCCTCTACTCTACTGGCAACTTTCCCAATCTCAGAGTCTTAAGGCCACTGAGACTAACATGTGAATC
CCAGTCTTGCTGTTTTCTCTGCCTCTGCTGCCTATGGTCTTGACCATGTTATCATACTAGCCTCCTGGGCTGCCTTCATGTCCAATT
CAAACACATCCATGTAGTGGATGACATGTTGCTTCATGTCAACATGAAGGGGACAGATGCCCCCTCCAGGACCCGGGCTCTCCTTCC
CTTAGCTGGGGTTGTGACTGAGTCCCTCTCTTCAGGTCAAACCCCTTGCCTGGGGGCAGCAAGCTCCCGAGGGGATTTGTCAGTGTG
GGAGTTCAAAGGCCTAACCCCGTTTGCTTCTAGTAGGACACTCAGCCAGGCTCCAGGGTCCCTGAGGGATCGCCTAAGGCCTCTGTT
ACCACTGCATCCGGGAACTTCCTCTATACCCCAGAGGTATTGATCCTGAGAGCAGCCCCAGTAAAACTCCCACCCCCAAATCTCAGC
ATCTCACAGACTGTTTCCCAGGAATCTGCCCCGTTAATATATATTGTTTCCTAAACAGGCAAAGCACTTCTCTTACCAAGACTAAGCAC
TATTTTTTAGAATCAAGCTTAAATGATCATTCTGTTTCCCAATGTCAAATAATAAGTAAGATTGGCAGAGGCCTTCAGCCAGGACTTA
TTCAGCCAGAGACTGGACAGAAGCTGGAAGCAGGGGAAGCTTTGGGGAACTGAAGCGTCAGACCAGGGTAGTGTGGACAGGATGCCT
TGATCTCTCTCAGCCTGATCTAATGATGATTCTATCACCTTAGGCATCAGAGAGGGTAAGTGACTTGCCCAGGGTCACACAGCAGCA
TGACAGGTTCTAGAAAAATCCACAGCTGCTGATGCCCAGAGCCTGTTCACATTCCAGGTAGCTTTCATCAGCCTGGACTTATCTCTA
GTTGTATACACACACTTCTGAAGTTGCTATGGAAATCTCTCTACAGCAAGATGTATTTTAAAAGACAGTTCTCATATCTTGGGTTGG
TTTTTATGTGACAACAAAGGTTTTTCATATTTCCTCCTCCCAAGGAGAGAAAGTGGGATCATAATTACGCAGAACAGCCGTGCCATG
GGGAGGGCAGCGCCTGGTTGCTGGGAGGGCAGTGACAACAGTGGGCTTGTTTTCCTTGGCCCCCAGAGAGGCATTACATCATGGGTT
CCGAGGCCTGACAGTTGTGGCACTCTGCATTCTCTGGGATTCTCTGGCTGCCTGCCCACCTCCCTCAGGGAGGAACCTCCTGATTT
TACACTGTCAAGTGCCAAGCAGCTTTACTGGTGGAGAGACCATCTGAGGCCCCGGGTCCTCACTGCGGCAGCAGGTTGGAGAGCCAG
CCTTCCTAGCGTGCCTTGAGCAGTGCAGTGGCCTGCCAGCTCACCTTTGTATGTTTATCTCCTTGGGAGGAGCAACAAGGCCTCCCA
TAACATTGATGAAGTGGGGTGAGCCTTGCTGTCACAGCCATGCTTTATAAATGACCTTACTGTCCTCTTGAGTGGATTCCCTTCGGA
CCCTTTGGACCCTTCACTTTGGTCCCTCCTGAGGCTCCAAATTCTGCAACAGGCTGTCTCAGTGTCCCAGGCCTTCCAGTCTGTCAA
CAGGTTTTTTATTTTGCTGGTTAACTTTTGGCATACTGATATTTTAATGAGAAATTTTATTCTGGAGATCTGCTCCTAAAGCAGGA
GAAAAACAATTTGTGGCCCATTCTTTCTCTCATTAAGAGAGCCACCACTCCCATTGTAAGGAGATAAAAAGAGGCTTCCTTGCTGTA
CTCTCTTCTGCCCCTGCCTCAGACACCCAGGCCTGCACTTGCCAGCAGCAGGAAGCAAGACCACAGCTCCCTCCTCCTCCCATCTCC
TTCCCACAACCTCCTACCCCCTCCAAGAAGGGGTACTTCCACTCCAAGAAAACAGTAGGAATGGCCAGGCTCGGTGGCTCACGCCTG
TAATCCCAGCACTTTGGGAGGCCGAGGCGGGCAGATCACGAGGTCAGGAGATCGAGACCATCCTGGCTAACACGGTGAAACCCCCGT
CTCTACTAAAAATACAAAAAAATTAGCCGGGCATGGTGGCACACCTGTAGTCCCAGCTACTCGGGAGGCTGGGGCAGGAGAATGG
CGTGAACCCAGGAGGCGGAGCTTGCAGTGAGCCGAGATCACGCCACTGCACTCCAGCCTGGGTGACAGAGCCAGACTCCATCTCAAA
AAAAAAAAAACAAACCCCAAAACAAAACAGTAGGAAAGTGGTCATTACAGGGATGAAGCACCCTACATGGGGTTGATGGAAGCCTGC
AAGCTAAACCCTGTAGGCTGAGCAGAACTGAGCAGAGCCTGGGAACAGTGGGGCCAGGCACTGTGGTTCCTCATGGACTGCTTTGGT
TTGAGTTATAACCTCCAATGTGGGGCACACGCACAGTGATCCATGGGACTATTGGGAGAAATGATCAGAACTTCTACATATATATAT
ATATATATATATATATATAAAAGCCTAGCTAGTATTTAATAAAAGGCCTAGTATTTTATAATAAATTTATAATAAATATATGT
ATAATGGAGGAGGGCATGCTCAAAAAATTTTTAATTGGTAGGAGCCCATGATTTTAAAAGTTTGCAAACCACTGGTTTATGATATCA
GAGGACTAGTTAGAGAGGATAGGAAAGTAGCCTACCCACATGGGATGAAGCCTGCTAGACCCAGGAACCCTGCCCTCTAGCTGGAGAA
GTGAGAAGTTAGCCTCCTGTAGGGAAGGAGCAGCCTGTAGCGATCCCCTTCTGGTGCCAGGCGGAGAGATAGATGGCTGGGGAGGGG
GAAGGTGGGGAGGCGACAGGGATCCTGCTGGAGGCCTTTGAGAAAAACCTGCAGCATTAAGGAAGAGGAAAATAATCCTGAAGATTC
CTGAAAAAATGTTTAATTATGAAAAACAAAAAGCAACATTAGAAGAGTCTTGGGTGGCCCAGGCACAGTGGCTCACCTGTAATCCCA
ACACTTTGGGAGGTCAAAGCAGGTGGATCGCTTGAGGTCAGGAGTTGGAGCAGCCTGACCAACACAGCAAAAACCCATCTCTGCC
AAAAACAAAACAAAACAAAACAAAAAAAAACCAAAAAAAACATTAGCCGGGAGTGGTGGTGCATCTGTAGACTCAGGAGGCTGAGGT
GGGGGGATCACCTAACCTGGGAGGTCAAGAGTGCAGCGAGCAGAGATTGCGCCACTGCACTCCAGCCTGGGTGACAGAGTGAGACCC
TGTCTCAAAAAAAAAAAAAAAAAGGAAAAAGAAAGAAGAGTCTTGGGCATCTCTAATAGGGTGCCAGAATATATATGATCCTTATG
ATAGAGAAGAGTGGGCAGATGTGAAATGAAAATGTATCTGGTTCACATAAGCACAGAGTTCCAGAAAACTAAAATTGTTTTAGCAAA
ATGAAAATCCACATTGATGGCACTACATTTTTAAAAATGTGGAATGTGGAGACAAACTTAAAAAATTTAAAGCTCTCTCAGAATGCA
GAAGAGATAAGAGAAAGGATGAATAGGACAGAACTGGAGGCTATAGGATCCAGGTGAAATCTAAGAATTAGAGTTATTTCAGAGGAA
GAAATGATAATAATTTGGATAAAAGCATAATCAGAAATATACGCACAGTAAACATTTTCAGAATTGGCCTAGGTAAGGGGATGTTGT
GTGTGGGGATGGTAGATCAGAGTAGGCAGATCGAAACGGATTCCTCTTTTCCAGTTAGAATGTGGGGGACAGGAGGAGCCACCCCTA
GACATACCCCAATACATTCTTTTTCTGGAAAATTTATTGAATCATAAGGTGAAACAATCCTAGAGGCACACAGGCATCCCAGAGCTG
ACTCTCCCTCAGGAACCCTAAATTCAGAGTCATTAGAACAGTTCCACAGTATTTTAAATTTGGATCCACCCTGTCATTTGAATGAGG
CAACCAAAGGGGAACAAAAGTCATTCTTGGAGTTTACTTTTGGGGGGAACTCGTTTGCCAGTTGACCAAAGACTGAATCACAATTAA
GATCTTAATAATGAAATGTTGTAAAGAAAGGCAGACATTGGTTCACACAGGCAAACATGAAATCAAGACTAAACTTACGCGTAGTTG
CAAAACTCCAAATTCTAATGTCAAAGAAAAAGTAGATTCTGGAAAGAGTGGATGTATGAAATTAAAGCAATAATTCAATAATAATTA
GAGGGTTGCAAATCCTCAGATTAAAAATTTGAGAGGAGAAGGCTAGAAGGGATTAGTAAAAGCATGCCAGATGCGTTGTCCTATGTA
GAGGGAGAATTTTAAAATAAAGCCCTATTACTCAGTTTTAGCAATCAGAGAAATATTTTTTTTAAAACCTAAAGGAACCACAGGCAAA
ATTTTATGAGTATAACAAGAATAACAGTAGCATCAGCCATATATTGTTTCACTTAATCCATACCATAGTTCTATGAGACTGTATCTG
CATTCTGTATATGAGGATGTTGAGACACAGAGAGGTTGAGCGATGTGCCTCAGGCCACAGAGCCAGCAGACTTGAGACTCCAACACC
AGCCTGAGAGCCTTTTCCCTACCCTGAAAACATATACCTCCCATGCCACATCAGAGCCCACATAGCTAAGACAGAAAAAATAAAGGA
AGCAGGAAGGAAGAATTTCAATACAAAGTATGAAAGAAGATCAGAGAAAAACAAACTTAAAATTTATAACAATCAACATAATTGTGT
```

EP 2 204 376 A2

```
TTACCTCCTTGTTAAAAGTAAATGTCTCTTAGATGAGATTTAAAAGCAAAATTTAATTGCATGCAATTTACAAAAGAAGTACCTAGA
ACAGAAGGACCCAAAAATAAATGAAGAGCAAAGATTTATTAGGTAAGTACAAATCAGATGGCAAACGAATAGAAATTCTCATTTTAA
ACCAAAATGAATTCAAAACAGAAATCATTAAATTGTCCAAAAGAACCATTTAATATGGATAAAAGTGCTATATACAGTAAAACTGTG
AATTTTATACAAAAAATGAGATTGATAATCTGAATTTTTAAATGTTTAAATTAATTGAGCACTTTGTAGCCTATAAACAAACATCTT
CTTTTTAAGCATCCAGGGAACACTTTAAAAAATTGATCAACTTCTTGGTCACACATTTCAAAACATCAGAAAAGTTGCTCGGGCTGT
ATTCTCTGACCACAAGCCTATAAAACTCCAATTAAAGGACAACTTTTTGGTGGGACTTTTAAAGCAATCTCCTCAATTACAACAGAG
AATTCAAAACTACCGTTAAAGGCTATTAGAAAATTACAACAATGATGACCCTGTACGTAAAAACTCAGGATGTGACCAAAGGTGTAC
TCAGATGAAAAACATCCTTAAAGTTTTTCAGGCACAATGGTGTGCACTGATAGCCCCAGCTACTTGGGAGGCTGAGGTGGGAGGATC
ACTTGAGCCTAGGAGTTCGAGCCAGTAGTGGACTATGATTGTGCCTGTGGATAGCCACTGCCTCAGCCTGGGCAACATAGGGAGACC
CCATTTTTAATAACAAAAACAGAAAAAAATGAACAGAGAAAATAACTGAATCCATTAGAAGAACAAAACAAACCTCAGGAGGGAAGA
TTAAGTTTATAACAAAGGCAAAGATAGGAATTAATGAATTTAAAAACCAGAAAAACATAATTTATAAATCCAAAAGATACCAACTTC
TAAAATATAAATTTAATTGGAGAAAAAGAAACACAAAATTAGAAATTTTAATGGAGAATAACTACTGATTCTTAGGATGTTTTAATA
AATAAGTGAGAATAGCTTAACTTTTTTTGAAAGAGAAGACCATTGAGGGGCTTTCTCTACTAGATATTAAAATGTATTATAAAACTG
AAAGTAATTAAGTCCCTCTGGCATTACTGAAAAATCAATAATGACAGATCAATGAAACCTAATGGAAAACACTGAAATAAACCCTAC
TATGTTAGAACCCAATATACGAGAAAGAAAACCAACTGGGAAGCGATAGATTATTCAATAAATGGTAATGGAGAAATTGGAAGCTTG
GATGAAAAATCAAATTGGAACCCTAGCTCTCACCAGCCACCAAAATAAATTCCAGGTGGATTAAAAAGTCAGATGTAAAAGAAGAAA
CCATGAGCAAGCTCCAGAAAACAAAGTGAATATTTAATCAGCCCTGAGAGGAGAAAGAACTGTTAAGCATAAAATCTCAGAAGAAAC
ATGAAGGAAAAAACTGGTGTGTTTGATTACATCATACACACAATTTTAGATTTCAGTCCCTCAAAAAAATATGAACTTATTTAAAGAG
CAAATGTTATGCTAGAAAAAGTATTTGCAGCAAACATGACAAGAGGTAAATATCCCTATTATACAAAGCAGTCTTAGAAACTAATCA
GAAGAACTTATCTCCCCAGTGAAAAAAATAGGCTGAAGATAAGGCCAACTACAAAGGAAGAAATACAAAAAGCCAATAAAATAAAAC
ACACAACACATATTCAGTCCCTCTAATCAAAGAACTGCAAATTATAACTACAAGGAGAATTACTTTTTCCCCTCTGGAGTTGACAAA
ATTTTAAATAAATATAACTCAGTGCTGGAGAATCTTCTTATTGTTGGCGGTGGGCGGGCATGGGGTTGTCTTACGCTGAGGGCAAGA
AGGAAATTAGACCATCTTTCTGGAAAACAATTTAACAATTTGTCACAGGAGCCTTGAGTTTGTAACTTTGATCCAGGAATTCTCTTT
CTAGGAAGCTATTTTAATAGAATAATCAATTTACATCAAACCTCTTCTGCATGGTGCAGTTTATAATAGAAAAGAATGAGAACCAA
GTAAACATCTAGGATTTCAATGGCTAAACAAGTATGGCACATCCATATGATGGGTAGTTAGCAAGAATTTTAAAAAATCAAATCAAC
GTGTTTGTTTTCGTTTTTGTTTTGTTTGTTTGTTTGTTTACTTCGTTGTTTGTTTGATGCGGAGTCTCATTCTGTCGCCCGGG
CTGGAGTGCAGTGGCGCCATCTCGGCTCACTGCAGCCACCACCTCCCGGGTTCAAGCAATTATCTTGCCTCAGCCTCCCGAGTAGCT
GAAATTACAGGCACCTGCCACAACGCCTGGCTAAGTTTTTGTATTTTTAGTAGAGACGGAGTTTCTCCATATTGGCCAGGCTGGTCT
TGAACTCCTGGCCTCAAGTGATCCACCTGCCTCGGCCTCTCAAAGTGCTGGGATTACAGGCGTGAGCCACCGCGCCCGGCCTAAAAA
TCAATCTTTTGAAAATTACAGTAAGGTAGGAAAATATTCATGATGTAATATTTGAAAAAGGAGCGTACAAAATAGCATTATGTAGGA
TATTACCGATTTATAGATAGGTGCAGAAATACCTGGAAAATACATTAAGTTGATTTCTTTGTGTTACATGTAATATTTA
TTTATTTATGAGATAGAGTCTCGCTCTGTCATCCGGGCTGTAGTGCAGTGGCCAATCTTGGCTCACTGCAACCTCTGCCTCCTGGG
TTCAAACTATTCTCATGCCTCACCCTCCTGAGTAGCTGGGATTACAGGCGTGCGCCACCATGCCCGGCTAATTTTTGTATTTTTAGT
AGAGATGGGTTTTCGCCATGTTGGCCTGGCTGGTGGTCTGCAGCTCCTGGTCTCAGGTGATCCGCCCGCCTCAGCTTCCCAAGGTGT
ACATGTGATTTTCATACATGTAAACATTAAGTTGATTTCTTATGACGTTACATGTGATTTTTTATTACTTTTTATTGTATTTTCAAC
AATGAGAATGAATATACATATATAAATTTTTAATGGAAAGAGGTAGCATTTGTTTTTACACACTTGACGAAGCCCCTCATCTTCCAT
AGGAACTCATGTTTTACATCAACGCGGGGTGGAAAGTAAAGCTGAATGGCTCAGTTTTTACACATAATTTTTCTCTAACAAGCGCAAT
GCAAGTTCCGTCTTGAAGGGCTCTCTTCCGACCCGTCGGCCAGTGGCCTTGGTGGACAGGGCCGCGGGGCGCCTTTGCGGGGCCGGA
GTGCTTCTTCGCGGGCGCGCGCCTCCCTTGGAGCCCCTCTGCGGCCGGCCGCTTATGGGAGCGCCCGAGCCGGCGTGCGGGCAGGTG
GAGCTCCACAGGGGACGTGTGATCGAGGCAGGCTCCACTGGCTCTTCAGTAGCCTCCCGGGCGTCCCCGCGGTGTTGCGGGGTCCGG
TGGGGAGAGGCCGCGAGTCCGCGGGTGGTTCCCGCGGCGGCCGGCGGTCGTGGAGAGGCCGGGGCCCGGGAGAGGCCGAGGCTCGCT
CCGCCAGGGGGCGCAGGGAGGGCCAGGTGGGCGGTGTCCCCACCTGCAGGGACTCGCTGTGGAGGAGCAGAGCGTTCAGGTTTCCTC
TCGCTGTGGCGACAGCCGGAGCGGCTTCAGGGCTGGGCCGGCGGCTCCAGGGCTGCTGCTAGCTCCTGGCTCCTGTCTCGAGTGGGA
TCCCATTCTGATTTCACCTGCGTGATTCACAGTCTCCAGTCCCACTCCGCGGCCGCTTTCTCGTTCTGTGATCTCGTATTCAGCCGT
TCTTTCCTCTTCCTCATTGGCAGTATTTTTGTGCCCTTTGACACTTAAACTGCTCATTCGTGGCTAGTTTTTTTCGTACTTTTATAT
TAAATAATTATACATGACTTAGCTATTATAAGTAATGGCTGATATCTCTTAGAAAAGGAGAATCAAGAAATAGGGAAATTATTAGCCA
GCGTGGTGGCGCGCACCTGTAATCCCAGCTACTCGGGAGACTGAGGCAGGAGAATCGCTTGAACCTGAGAGGCGGAGGTTGCAGTGA
GCCAAGATCGCACCATTGCACTCCAGCTTGGGAGACAAGAGTGAGACTCCGTCTCAAAAAGAAAGAAAGAAGGAAAGAAAGAGAGG
AGAAAGAAAGTGGGAGAGAGAGAGAAAATAAAATAAAAAAGAAACTGGTAAAGCAAGGAAAATTTCCATAAAATTTGCATTCTGAAG
CACTAAGTTTGGGACTGTGGGTGCAAAGTGAAATCCATATTGCATTGAGAAACCATTCCATTTTTTCAGTCAAGTCAACCCCCAAAT
TATTCCAAATTATTTGCTGCATTACACCAAACCTGGGTTTCCCTTCACTCTTCTGCTGGGAAGTCACCAGTGTCTTTTGTCTCCA
GGATTTTTGCCTTAGGGAGACCCAGTTAAATGCCAGGGGTTCCTTCTTTAACTGGGGAGGACTATCTGACCTTTGTCACTGGAATGAA
TTCCTTCCCAAGTCTGCCAAAGATGGTACACAGTGACAGTTAGTGCCATGCTGGGTGTGTAGGAGAGAAGTGACTTCCTTTCCTACA
GCGGATGCCTAAGGGCATGGGGCTTCATTCTCACCCGCTCCCCCACCCACCTCCTTGGAGGGGCTGAGGTAAGGAAAAGGAAGGGGT
TCCTGTAAGTGACGTCAGCCACATCTATTTGTGGGCTGACATTAGTCAAGCAGGAAATCCATATTCTGTCAGCTTTTCCATCCTTCA
ACTTTGGGGTTGTTTTGTTTGCCCCTCGTGATCCCATTGTGCCCTACATTTCCGGTGCAGACCCCTCCCTCAAAGGACGGGCACCTC
AGCTCCACAGGGCCCCTTCTCCATGCAGTTGGTGTCCTAGGCTGGCTCTCAGGAGCGCAGCTCTTCACCCCCCTGTGTTTCCCCTGC
CCTCCACTGCCAGCTTGGGGCCCCGCGCCAGGCTGGGGTAAAGCCCTGCATTCCTGGCTCACATAGCGCAGCTGCTAGAAGGGTTT
TTAAAAATGTGATTTTTTTTGGCCGGGCATGGTGGCTCACACCTGTAATCCCAGCACTTTGGGAGGCCAAGGTGGGCAGATCACCTG
AGGTCAGGAGTTCCATACCACCCTGGCCAACATGGCGAAACGCTGTCTCTATTAAAAATACAAAAATTAGCCCAGTGTGGTGGTGAG
CGCCTGTAATCCCAGCTACTCACGAGGCCGAGGCAAGAGAATCGCTTGAACTCAGGAGGTGGAGGATGCAGTGAGCCGAGATCACAC
CATTGCACTCCAGCCTGGGCAACGAGAGCAAGACTCCATCTCAAAAACAAAAACAAAAACAAAAAACAAAAACAAAAACGATGTTCTCCT
TCCATTTAATACAGACTCCTCTGTTCCCTGAGACTTGGCCCCAGTCCCCAGAGGCTGAGCTGAGAGGGAATATGGGTGAGACATGC
CATATTCCTTGAGTACCCCACCTCGTTCCATTCGGTCTGGATGCCTGCTTGCCCCCCACTGCACAGAGAGTTTAAGGGCAGCCAAAG
TCCCCTGGCTGCCTTTGAGTAGTGGCCCTGGAAGCCTGCTGGCCCTAACACACCTGGTCATTTTGTTTTCCCAAAAGGCGCCTGGGA
GCCGCCTGGTAACACAGCCCCTGCGGGCCCTCTCAGACAGGGCCAGACATGCTGCAACGGGCCCAGAGTGGGCTCTGACACTCCCACA
ATAGAGGGGGCACAGAACTTAGTAAAACTCCTTTTAAATATCTTAAACATGTTTCAGCTGAGCCAAGGGGAAAACACCTAGTACACA
GTGGCAATCGCACCAGCACAGGATCCATCCTGTGATCAGGACTGCCTGAACTCAGGGTGGAAAAACTCGGTTTGACCCCTGGTTCTA
CCATTAACCTTGCACTTGTACTTAACCTGACCAGAGTCATGCAGCCTCTTTTGAACACCCTCTGGGCACTACTTTAAAAGACTATC
CTAACTCAGTTCTCTGGGTCCGCACGAAAGAAGTTCAAGTACCATAGGCTGGCGGTTAAGCCATTGACCTCTGGGAAGATGTGAGCG
TGGAGCCTTGCTTTATGCTCTTACTAGCAGGTGACGTTGGGCCAGGAAAGGGGATAATTCACAAAAAGCCCTTAGCACAGTGTAGAAC
ATTTAGCACACGGTGAGTACCCAGTGAAGCAGCCAGGTATTTCTTTTTCTACTGAAGGTCCCTTGAGTATCCAAAGTCAGGTTGAAT
CTTCTCTGCTCCAACCCAGTGGTCTCCAGTCCCCTCAACCATTGTCACAGGACTTCTGGTCAGACTCCACCCTCTCGCTCACTCTCC
TCCCCTGGTCATCAGTGTCCTCATGAAGCGAAGGCCCCAGAACGAACATAGTGCCCCAAGTGTTAGGCATCCTCCCATTTCCTCGGC
ATTATTTTCCTTTTTAATGCAGCCTGAGAGTGGCCCATATTCTTAGGGCAAAGGGCTCCCAGGCATAATGGCCTGGGAATTAGGGAGC
```

```
GAGACCCAGGGCCCATCTCTGTTTAGATTGGGCACTCTTGACAGTTCCATTCTATAGCCCCATGCTCTATTGTTAACTTTTCTTCAA
TACAATTGTAAGGGCCTTGTTTAGCCTGAATCCTGCAGGGAGCAACTTGCACCCTTGTTCCACATGGCCACTACCAGATAAGAGCCA
CAGCTCAGTAAGGCCAGAGGAGTGGTTCAGCCACGTGGGAACCAGCCAGGACTCAAAGAGACAAGAGGCTCCTGTCCTGTTTCCAGC
TTTAGCAAGGGAGTTGAGGGCTGGCCTGAAGTCCATTTCTCTCTGCCTTAGTTTTCCTGCTGATAAAAGCAATAGTTAGTTTCTCAG
GCTCCCGCAGATTTCTCAACAGATTGTTTGAGCACTGAAGTTGAGATCTCTCCTGCTCTCTGGACACATCTCATACTGATTCCCCCC
ATACCCTATTTCCTACACAGGTCTGTCCTTTACCTGGACATTCTCCCTCCTCTGCCGTTTCCCTTCATCATGCTTGCTCCCGGAGAA
TGCAGGCACTCTCACAGGAATGCATCCATGCATCCCAAGTGGAGCATTGGGCCACGCCTCTCTCTGAGCTGCAGTGCTCTCATGAGA
GACTTGCTAAATAATAATACTGTTATGTATAGGTATGGAGCTCTGCTATTCACCAGGCACTGTGCTAGGAATTAAATATATCTTAAA
GCATAAAACACACATGATTTTTTCCCTCATGGGCTTACAGTCTACCAGGGAAGACAGGTAACAAGTAAGGAAACAAAAAGTAGATAA
TTTCAAATCTATGACATATACTGTGAAAGTGGAAAAAAGAAGTTTGCAATGATTCTTTTTTAAAAAAAAATTTATGGCATTGGATTG
GGATCCTACTCATCTGGGGCCTGCTTGGAGGTGTCTCTGGGGAGGTGATGCTTCAGCTGAGGTCTGAAGGATGAGAGGAAATGAGCC
CTTCACAGACCAAGAGGGTGAGCCTTCTGAGAAGAATGAGAGACTGCACCAAGGCCCTGAGCTGGCAAGGAGCCGGGGGGAAGGAG
TCACTGGAAGGAGAGTAAACAGATGAAGGGGGGTGTGGCAGGGACTGAGCCGCAGAAGGGGGCAGGAGGCTGAAAGTGAAGGGCCCT
GAGTGCCATGTTGTGGGGTTGAACCTAAGTGCAGTGGAAAGCCTTTAAAGAGTTTTATCTTTTTATTTACTTGTTGTTTCTTCTTCT
TCCTCCTCCTCTTCTTCTTTTTCTTCTTCTTCCTCTTCCTCTTCCTCCTCCTCCTCCTTTTCATCGTCGTCGTCTTCTTCTTCTTTG
CCTTTGCCTTCTTGTTCTTCTTGCTCTTCCTCCTCCTCTACCTCCCCAACCCCTCCTCCTCCCCTCCCCATCCTCCTCACCTCTTCTTC
TCTTCTTCTTCTCCTCCTTCTTCTTCCTCCTCTTCCTCCTCCTCATCTTCCTCTTCCTCTTCTTCCTTCTTCCTTCTTTCCCCCTC
CTCCTCCTCCTTCTTCTTCCTCTTCCTCTTCCTCCTCCTCCTCCTTTTCATCATCGTCTTCTTCTTCTTTGCCTTTGGCTTCTTGTT
CTTCTTGCTCTTCCTCCTCCTCCACCTCCCCAACCCCTCCTCCTCCTCCCCTCCCCATCCTCCACCTCTTCTTCTTCTCTTCTTCTT
CTCCTCCTTCTTCTTCCTCCTCTTCCNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNCCCTTCTTTCTTTCTTTCTCTCTCTCTCTTTCTTTCTTTCTTCCTTCCTTCCTTTCCT
TTCTTCTTCTTTCTTTCTTTCTTTCTTTCTTCCTTTCTTTCTCCCTTTCTTCCTTCCTTTCTTCCTTTCTTTCTTGTCTCACTTTGT
CACCCAGACTGGAATGCAATAGCATGATGACAGCTCACTGCAGGCTTTAACTCCTGGGCTCAAGCAATCTTCCCACTTCAGCCCTCT
GAGTAGCTGGGACTACAGGCATAAGCCACCATGCCTGGCTAAATACAAAAATTTTTTGGCGACAGAGTCTCTCTATGTTGCCCAGGC
TGGTCTTAAACTCCCAGCCTCAAGCAATACTCCCACTTCAGGTTCCCAAGTATTAGGATTATAGGTGTGAGCACCATGCCTGGTGC
TTTTGAAGGGTTTTAAATAGGAGAGTAATACAATATGGTTGCTTCTAAAAATATTACTTTGGCATCTTTGTGAGTAAATTGGAGGTG
GCAAGAAAACTACTAGGAGGACTAAAAAGGCTACTGCAGCAGGCCAGGCAACAGACAAAAGTGACTTAGAAGGGCGGTGGTGGCGGT
GTAGAGTTCTTGAATACATCCCTGCACCATGCCAAGTTTTATATACATCTTCTCCTGTACTGATGTCCCACGCATCAAATCTGTGAT
CAAACTCATCATGTTGCCCCTAAATTCTCTCTCTGCTTCTCTGTTTGTGTCTTCTTTTTCTCAGGAACTTTTACCCGTTAGTATTCGT
TTTAGGCTGCAAGTGATAGGAAACCCAAAACAACAATAGCTTAAACAAAACAGAAGTTCTGTCTGACGCCAAAGAAGTCCGGAAAGG
TGTCTATAGCAGGTATGCTGCTCTATGGCATCATCAGGGACGCAGGCTCCTTCTCCCTTGTTGCCCTGCCATCGTTGGTACGTGGCT
TCTTCCTCATACTTCAAGAAAGCTGCTGGAGCTCCAGCCATCCCACCTACCTTTTAGCCATCAGGAAAGAGAAATGGACAAAATAAG
GTTGTTTCTTTCTTTGTTAAGGACATTTCCTCGAAGTAACACATGTCACTTTCATTTACATCCGTTGGTCATTCACATGGCGGGAAAA
TGTAGTCTTTATTATTGGTAATCATTGCTCCTTCCTGGGCGCTCTTACTAATGAAGAAGAGGAGACGTGCTGTTGGGCTGCAACCAT
CTCTCAGGCTCAAATCGTTCCCCATCCTTATGCCACTCCCAGCCCCATTTCCAGCCCTCTATTTAGGCACCAAGCTCTATGGTTCTT
ATTCTGCGGTGTCGCTTGAAGTCATCTCTTCCTGCTACCTCCCTGACACAGATCCCTGTCATCTTACACCAGTTTACAGAAGTAGCT
TTTCTGGCTTCCAGTTACCGTTCCTACTATTACCCCTCAAAAACCTTCAATTGCTCCCTCTAGAAGATTAAGTCCATATTTTTAAAT
TTAGTGACCAAGGCCCTCCATCATGCAATTCCAGTCAACCCATGATAAAACCGGTTTGGTTCAATTTTAGTAACATCAGCAACAATG
GTAATAATGATACTTTGCATTCCTACATGGAAGGTTTTATTGCTCACAGAGGGACAGCACATGGCCCCTGGAGTCAGAAACGTGCG
CTCATAGCCTGGCTTGACAACGGACCAGCTGTGCGATCCTGGGCAAGTCACCTGCCCTCTATTAAATAAGCACCTGCGACTGGCTGG
TTCCTGATAAACCCTGAGCCCTTTCCTTTCCTGGGCCTCTTTCTTGGGATCTGTAGATAGGAAGTAAGCCTGGTCTACCTGCTTCCC
AGGGGATTAGGAAGTTCAAACAAGAGGAGACCTGAACAGCTGGGGGAAACATAAAACACTGGACATCCTCTTCTGTTTTTCACCACG
ACCCTATGAAGTGAGCAGGCAGGCAGGGGAACCCACGGTCCCTATTTCGTGGATGAGGATGTGGTGGCTCAAGGAGGCTCATGATCT
GCCCTGGCCCTGACTTGTAGGGCAGTGGAGGAGGCCTGGGCCCTTGGGACCCCGATCGCCTGTGGCAGGGCTTTTGGGGTATGTGTT
CCACTGGGGAGGTGTGTTACAGAGTGAGTCTGTGTTGAGACAAAAGGAGAAAGAACATAAGACGCAAAGTTTTTCTTTCAGCTTGGA
CTGTGCAGTCATTTTCTAATTTCTTTGAGGTCATGCTGTTTGGTACCTAGGAAAGGAATTGTGATTGGTGTTCTAGAAAAACAGTGA
TTTTTAGGGGGAAGCCACTGCCTATTAGTAAGTACAGGCAGAAGCGGCTCATTGTCCTTGAATGCCATGATGTCTGTTTTCGAGGGG
TGGGGTGGGGTCTGGTGGCTGGACTTTTTTGGAGAAAATGTAAACAAACTTAATAAAACATCTATGTTGTCAAACTTA
CTGAGTTAAGTAGATGTGAATATAGTTAACTGAGTAGTAGCTTTAGAATTATTTTGTCAAGTTTAACATGTGAAATTGTTCCCACTT
AGTTACCAGTATTCTTAAATTGTCTTGTTTATAATTTAAACTGTGCATTTTATTTAGAGAGTAAATGTGTCTGGAGGCTTTTCATTA
TTTCAGAAATTAGAGGATAGATTCCTGAGGTGCCCGGCCTGTCCCTGAGGCCACGTGATGCAGAACTGGAGGCAGGTGAGTCTGAGC
CGGGTGCTCGGAGCCTAGTCAAGGGTGACCACAGGCCGAGGGAGGAGAAACTTCTGACATGCAGTGTCCTCATAGGAATGCTTTGTG
GTGTTATCTCCATTTCCCTGATGGAAAAACTGAGGCCAAGAGCCACTATTTTTGGAGAAAATCTATGTGGAGTTGCTTAATTGCC
TCGCCCCCAGCAGGGGGCATGTCTGTGTGATAATGTCTGCTACAGTGCCCAGCCTCGTAGGAGGCTAGGAATTGTTTTTTAATAAA
TAAGGAGCACTATCTTCGTTCATGGCCATTCATTCTACACAGGTTCTCCTCTGTGGCCCAGCACTCTGTGAAGTAAGCAGGGCAGGA
GCAATTTTCCCATTTTAGAAATTAGGAAATGGAGGCTTTGGGAGGTAAGCGAGCCAGGGTCTCACTGCTGGCCCGGGGCTGCTGCCC
TTCCTTCATCTGCTCAGTGCTTGAAGATCCCAGAGCCCAAGGTCACTTCAGCTAACAAGCTGATGTCCCTATATTTGGGTGAGCTGA
TGGTGCTAATGCAGTGTAGATCTTCGTTTTAGAGGATAATTATGTTTTCTGAAAGTCTGTGGAGAATTGTGCCATTGCTTGAGGGA
TATCTAATAAAAATCACCCACTGGATAAAAGCAGACCAGAGTTCACCTAAGGAATCATCATCGTCATCATTGTCATCATCTTCCTTA
TCAACAGCCTCATCTCTACTCATGACAGATTTGGCCAAAAAGAAAAAAAATAAAGGAGGCCTTTCGAGGACTTAACACCCGGAGGAC
TTTCCTCCATGCCTCCTCTCTCTGTCTGTCTTCCTAATTTCAGTGAGTCAGTTTTTCCTCCCCATCTGTTTGCTTTCTGGCACTTTA
CACAACTGTCTGGGCTCTTTCATTTTGCCTTCTGTGGTTGCTCAACTCCATCATTTCCCTCTCGATGCTTCTCCTTTGTTCCCTGGC
AGGCTCCTCTCATCTGCCAGTTTCCAGTCCTATGAAGAGTCAGAGGTGGGTTTTAGTTTTCAGGCAGCATTTTTGTTGCCTGGCAAC
GTGTAACAAGCATCTTCTGTGGTGGCTGCTTCCCTTAAAACAGAGCCAGGCTGGGGATGGCAGTGGACGAAGGAGTCTCTGGGAGCTT
CTCTGGGGTCATGGGGAGGAGTCAGGGGAGGGGGACAGACAGACAGGTTGGCTTCCATGGTGACTCTGAGTGTTGCCATTCTTACTC
TTTTATAATAGCCTTGGCACTGGCACGCCCTCTCCTGCCCCATTTCAGGCACCCCAGGTGTCCAGTGGTGAAAAGCCTTGGGTTAGG
AGCCATCAGAAGTTCTGTGTAACTTGAGAGGCACCTGCTGCAGCTCCCTGAGGCATGGCCAGCCTGGATGAAGGCTGCTAGGTGGTT
TCCACTGGTGCTGACTCTTGCTTCATCTTGTCTGCACGGGATGCACTGCCTCCCTGGAAAGACACCTGGTTTTGCCTCTGC
AGTCAGCCCAGCTGCTAGTTGTCCCGAGAATGGGAGTGGGAGAGAGAAGGGGGACAGAATGTGATCCAAAAAAAACACCTGACTTAGA
GCTGGAGGGGGCTTTAGAGCTTCTTTAGCCAAATCCTCATATTGTACACACGAGGAAACCAAAGCCCAAAGAAGGGAAGCAACTTGT
CCAGACTCACACCGCGAGTTGGGCGGGTCTGAGCTCCCAGCCAAGGGTCTCTCTCCTGGGACTGGACTCTGCCGAGCTCACACATCT
GATGCCCTGTGGCTAGAAAGAGGCAGCATGTGGTGAGAAGTGCACCAGATGCCCAGTTCTCCGCTTTCTGGCCTCCATTTGCTTATC
```

```
TAAAAAGAATGAGGTGAACTGGGCTAGAGGGAGCCCCAAGACTCAGTGACTCTGATGGTAAAGCTGACACAGCAGGCAGGCCCCTGC
TGCCCAGGCCTTCAGCCACGTGTCCAGGGCTGCTCTCACCGCCCCAGGAAAGCTCAGAAGCTGAGGGGGTCCTGGGCCTCTGAGCTG
ATGCTCCCCAGGAGAATCCCACTGTATGCAGAGACCCCGAGCACAGCCACGACACTTGTCACCATGAGGCTGATTTAGGGCAAGCCT
CCTGGGCATCTGTGTCTTCAAAGATGGTCTCTTAAGACCCTTATCAAGGATCAGGGCTGCAGCTGCCCACTCCCCTACCTAACCAGC
TCTCCATCTAGTGTGGGAAACGGGTAAGAAAACATTCTCTGAGAATTACAGCTCCTTAGAGCTGGAAGGGGCCCTAATGCACATGGA
CCCAGCCCCCACCCTTCCCAGATGAAGAGAGGGAGGCCCAGAGCTGTGAGAGTTGATGCGAAGGGGGTTTTGCTCTATTCCCTTCA
GACCCCACAGGTGAGAAGGCATCTCCAGATGGGTCATCTCTTTATCCTGCTCCTTCCTTTCCTTAAGTAAGAATCTCTCAGTTCAAG
AGGCAGAGAAAAGGATTGCTGAAGGCAATCTGTCTCTTCATATCTCTTTCATTCTGCTCCTGGGGCCGAGGAAATGGGTGAGGAGGG
GTGCAGGAGGCCCTTGGCCTGAGCACCAGGCCTGGAGCCTTCCCTTCCAGCCGCTGCTCTCCAGAGCTTGGGGTGCACCAGCTTCGA
GGCTTTAGAAGCTTCTCCAGCAAGAAAGGCCCTTCTGAGGGAAAAGTGTGTGCGACTGGCTGTCTCCATTCCTTTCTCCATATAGTG
TTGACCTTCGGAATTAGCTTTATTCCTGACGTGTACCAGAATTTTCAGTTAATCATGTAAACCAAGGCTCAGGTGACTTGGCTGTGG
GCAGTTGGGGCCGGACCAGCCCCTGAGAAACTACTCCCATCCCAGACTTCGTGACGCTTCACTTGGCCAGAGCCCACTCCCAGAAAG
CAGGGAAATGTGCCTTTCTGCGTAGCCCAGGCCTATGGAAAGAGGCGAGAGGTCAGGGACAGCTGCACTCCCTGGGCAGGCTGCTTG
TGGTCCGGGAGCATCCCGTAGTCTCCCCAAGCCCCACGGCCGTGGGGACCCCGGGTCAGGGCATTATTGTGCATATCCCTGTCCTCA
GGCCCCTCATGCTCAGGCCAGACCCTCACCCAGGTGCCCTTGCCCTCAGACTCCTCCCCTTCCAGCCATTGTCTCTGTCTCCATCCC
TGTCCCTCTCTTTCTCTATCTTCCTCTCTCTGATTCCCTGGGGGCTACTTTGCAACCTCTGCTGAGTCAGAATTCTTCCCACTCG
CCCTGACGTCCCAGGCTCCTCCTTGGCACCCTGTCTTCTCTGTGGCAGGGAAAGACGCCCAACCAGAGCTCCCACGTCCTGCCCTGG
GAACCCCACTGCTCTGGGAAACAAAAACAAGAAAATTCCTTCCCAGGGTACTTGTTCTGTGCTGTTCCTCTTTGTTCTTGGTCACAG
GGTGATTCCCACGTCCTTCCCACACTAACCCTGCCTAGGTGAGGCTGCAGCTTTGTGTGCCTTCCACAACATTTGCTTGCCTGGGGA
GCTGCCTGCCTTTAATTATCTTTGGAAAGCATAACTGAGAAGAGCAAGACACCCGTCTTTTCTTTAGGGCTCTTGCCAGGAGCACTG
CCTATGCAGCACTTCCCTCCAGGGGGTGCCATTCACATTGAAGTCTTCGTGACTGGCACTCCTTGGAGTTGCACAGTGCGCAACCTA
TGCTGCTGAACAGGGCAGCCCTGGCTCTGTCTTACTCTCCTGGGAAGCATGGCTCATTATAACACAGATCCAGGATGAGATACCTGA
GAGAGAGAGAATCCCCACCAGTCTCTCCACTCATCTCACCTCCCTTTTTAGGTCTCCACCTCCTCCAAGGTGTAATATGGTCAAAGA
CAGGGCTCCTCCAAGGTGCAATATGGTCAAAGACAGGGCTGTGGATGCAGGCAAGTCCAAGCACACACTCGGGCCCTGCCACTCAAT
TGATGTTTTATTTTGTGCAAGTTTCCTAATCTAGTAATTAATCAAGTGATCCTCCCACCTCAGCCTCCAGAATAGCTGGGACTATAG
ACACGTACCACCATGCCCTGCTAATTTTATTCATTTTTTGTAGAGACAGCATCTCACTATGTTGCCCAGTCTGGTCTTGAAATCCTG
GGCTCAAGGGATCCTTCTGTCTCAGCCTCCCAAAGTGCTTGGATTACAGGCATGAGCCACTGCACCTGGTCTAATTTTCCAATTCAG
TAAAATAGGAAGTGACAGTTATGAGTCTTGTTTTTTTGAGGATTAAATTCAGTAATGCGCATAAAGCTTTGGCCCACATGGCACGTGA
TTGGCACTTGAGAAATGATAGTGATTATTGTTATCTCTTCAAATAAGATTAGCATCCAGTCTTACCTACCACACAGAGATAGCCCCA
GAAATAAAATGAAATGAGAAGTAGATCATCTCTAAGATCTTTGAGGAAGTATTTTGCAAGAATCCCAACTACTGTTGTTCTTCTCTC
TGTATATAAAAAACGATGAACTGTGAAGCCCCTTTGCTTCCAAGTCACGTCTGTGACTGATGGCAGATGTCGGGAAAGGCTCAACC
AGGAGAGAGAAAGCTTGCAGGCGCGGGCGGCAGACTGAGGGCCCCGGGCGGGTGGAGCTCAATGAAAACCACAGACAGACAGCAGCT
GTGAGAGCAAGCAGAGACCCAGAATGGCTTTAAACAGAAAGCAGAGGCCCACAGCAGGGAGTGAGATGAGACCAGGTTTAAACGTCA
CTTTTCCCCTAAGTCCTTGTTCATTCACAGCTTCCTTCAGTAGCTCTGACCAGCTTTCTCAGCACCTGCCGACCGACAAACCAACCAA
CCAATGGAGGCTGGTGCTGGGGGGTGGAGATGGCCAAGAGCCGGTGGTGAGAAGTGAGGGGAGAATGGGGAAGAAGGGGCTCCACAGAG
GGCACTTGATCCGGGACGGCTTTCCGGAGGAGGTGATTTCCTAGGCCTAAGGGGCCTCCGGGCAGAGGGGCCAGCATGAGGGGAAGCT
CGGAGGTGGGGGAGCATGGATTGGAGGAGAGAATAAGCAGTTGCTTGCTGGGGTGTCATAAGATGCGGCAGTGCACTCAACGCTACA
GGAGCAGGCACGGGCCTTTTTCACCAGCCTAAGGATTTTGGAATGACTCTTCTATCAGCCAACAGTTTCCAACCTTATTTATGTACT
TGTTTCTTTGATTCCAGCGATGCAGGAGAGATGATATTCAGACAGCCGACTTCTGCTGTGGGTGTGCCCCAGGTTATAAATCATTTC
CATCAGGCTAGCTGGAGTCCCGGCCCTTCCTTTCCACTGAAGAAAGCAAATCAGAGTACCAGATAGCGAGAGTGATATTATCACAGG
ATAGCCTTGAAACTGCCATAACAAAAAAAATAAATCGTTCAACAAATGTCAATCTACTATTAGCCCCAGAGAGACTGTTTG
TCTACCCCAGTGTGTCGCTGTGCAGAGGCTGAGAACGTCCCCTCTCCTCTTCCCACACCTAGGAGAGGGGGAGCCACTGGTAGATTG
GGAGCAGGGGAGGGTCTGGAGTTTCAGAAAGATTTCCCTGGCTGCAGTGTGGAAGGGGGATTTGAGGGGAAGACGGGACACCAGCTG
GGGAGGCTGCTGTAGTAACAGAATGCTGCCCCCACCACCCCTGGGTACAGTGGTGGCTGGGATGAAGAGCAAGGGCAGTTTTGAGAG
AGCTAGAGGAGCCAACCTCCACCGGACTGACTCACTGGATGTGGAGTGATGGGGAGGAAGCTCAGGAACAGTGTCTGGTGTCCTAGT
GTGGCTGGCTGGATAGCTGGTGGGACTGCCCACGAATAGGAGGTGCAGGAGGAAGAGGGCCGGGGTTGCAGGGGACCTGGGCAGCAG
GGAGAGGGCAGGTAATGAGCAGAGTTGGGGATCGGTTGAGCTTAGTTTACCTGCCACCCCTCCAGAGATGTCCCACGTTGGGGGGAC
CCATGCACCTACTCCAGATGTTACCCTCCCCACTTTACAGCACAGGATTCTGCCTGGTGACATTTGATGCCATCTGTATCTGGCCAC
CCAGTGGGTGCAAAATGCAAGTTTGCTGATTAAGCAAAATCAACCTGAAGCAAACAAAGACTGATGTCCCCAAGGGATAGCCAGCTC
CTTGGAGGCAGAAACCCTTTCCCTCTAGGGCAGAGCTTCCTCTGTGGGTCCTCACAGGGGAGGGTGGGAGAGAGACTGAACAAGTGA
AAGGGAAAACAAGAGAGGAGGATGAGGAGGGAGGTGGAGAAGGGCTTTCAAAGGAGAAAAAGAAAAAGGAAGCATGAAAAGGAAGA
AGAAAGAACAGAGAGGGGCAAAGGCAGGAAAAATAGAGGAGAAGGAGGAAGAAGATGGAGGGGTTTGGGGCAAGAGTAACAGTGCAG
GCCAGCTGTGCCTGTGAGTTCACGGGGGTTCTGCTTATTAAACCCCAGCACTGGCAACTTTGAGAAGATGCTCAGAGTTCTTTCCCCG
CCCTGCTTCTCCCTCCAACTCACCTCAGCCTTCTCCTGAGCCTGCCACTCCACCCGCAGCACTGTCATCCCAACCCATACCTGCTCC
TCTGAGATAGCTGTGTGTGTCCCCTGCGCAGTGGCCCCGTGCCCCAACTTGGCGTCACTTGTCTCCATTGTAACGCGGCCCTTTTTGC
CTTGGTGTTGCTTGGTGTCTGCCTTCTTCCTAGAACTCTAAGTTCCCAAGTAGGCACCAAGCCAGGCACACAGGAGGGGCCTCATTC
CTATTGCTGACCAAATGAACTCATTTACTAAGTTCCAAAATTTGTGTCAGGCACTATGCAGAGTTTTATATTTTTCACTAGTCCCGT
GCAAGGTTTTACTGCTTCATTTTACAGAAGTGAAGCCCAAGACACAGAGAGGTAAGCGCCTTCCCTAGGCCACACAGCTAACAGGTG
GCAGGGCTGGGATCCAAACCCCCGTCTGTTGGACTCTCTGCCCATGCCATCTGCCACGCCATCTCCAGATACCGTGGAAGACATG
CTTCCCATGGGAACAGTGCTTCTTCCAAGCATATTATTTTTCTCCATTTTCATGCATTTTATTAAACATTTCCAAATCAGGAAACAT
GGAAATATTTTTTTAATAAGAAGAAAGAAAACATTGCAAATTGAGAAACTGACTCAGAAGGATTTGCCAGATGAGCCATCTGGTTTT
TAATTTCAAAGAGGTTGACAGTGCTTCCCTCTAAGAAGAAAATGGACTGTGAAGGAATGGCAGCTGGCTTCCCTCAAATGGTGCAT
CCATTTGCAAGGTGGAGAAGTTGAGCAAGACCAAGAGACCCAAGAGAAGAGAAACACACTCCAAGAAAAGCTTGGCCAGTGCTGGTG
TTGAAGCTTCCCCGCTGAGGCCTGCAGAGGTTGCATCCAGCTGTGTGACACCAGCCAGCTGTCCCACCTCTTCCAGAGTTTCTCAGA
AGCTTCTACAGGAAACCACTAATGGAGACAGAGATGCCACCACCTCGGTGAGAAAAAGCCACACCAACCAAACTCTCGTTTTCACAG
AGCAAGAACAAGCTGTGGTTGTTTGGCGGCAAGTCGCATTTGTTTGTTGGAGCCATTGTGCTCTGGTGGTGCTGGTTTTCTCTCCTT
TTAAAAATAACTGGAGGCCTTGCACCATGCTGTTAGTTCTTAGGGCTGGAAATAAAAGACTAGATTTAGAGTTGCCTTGGAAAGATC
CAGGGCTGTTACCTTGAATTACCTGGTAGAGGAAAACGTCGTTATTTTCCTAAATTCAAAGCCAACTCTTGAAAAATAGGACACAGA
GGTGAAGTGGATGGAGGGGTGGCTTTATACATGCATGTGGTGTCCTGGGCACGTGGTTTGATAGGCTTGTGATCAGCCCTGAGTCTT
CAAAGCCATTTATTTTACATTTTTAAGATGTTTATAAGGGAAATTGTTTTTGAGAAAAGAACGAACTATTTTTTTTTTTTTTTTGCCT
TAATGTGAATTCACCCACTGGCACAAGCAGTAATAGCCCTGAAGTCACCCTAGGCCATTTATTCCTCAGCTGCCTTTGAATTTCAAT
GGGATTCTTTCACACGCCTCAAGCATTTCTGAAATGCTCCTAAACTAAAGGAACTCATAATACCCTTAAGAAATGATGATTTCACCC
CATTTTCTCCATAAAGTCTGGAATCACATGCCCTAGCATGCTGAAGGTACTTGATAATTAGAAGTTGATTATATTGGGGACCTGGACA
AAGGCAGAGCTGGGAATGGGAATAAAACCCAGGACTCCATGAAAGTCTCTTTCCTTTCATAATAGCTTCTAATTCAGGCTTCTAATA
GCTTCGAATGCCAGTCACATACAGAGCCTCCCGGCCATCACCTACTGGCAGTGGCTGAGCGGGTATTTTGCCAAGCTAGAATGGTTC
CTGCCCAGGCACACGGGTGCTGCCTCCACGCCCCACCCTGTGCCCAGCTCACTACAATTGCCATCTTTAGCAGCCGAAAAAATACAG
```

EP 2 204 376 A2

```
GTTTCAGTCCCAGTCTTTTCGCTTAGTTGCTGTGAGAAGTTAGTTAGGTCAGTTACGCACTGGGAGCTTTGCATTGATTCACTGTAAA
ATCAGGTAACAGCTCTTATCCTGCTGGTCTTGTCCCACTGGCTTGTGTGAGAAAGACGAAGTGGAGCCATTCATTCATTCATTCATTCA
TTGGACAAGTATTTATTGAGTACTAGGGATACAGCAGTAAAGAAAACAGAAAAAGTCCTTTCCTCCATGAACCTTATATTGTAATAG
GGGTTGTCAGTCAATAAATACAAAAATAGGCCACATATCTATTATGTCAGGTGGTGATAAGCGCTGTGGAGAAGAATGACTCGGGAG
AAGCTGGCCTGCTGGGTGTGGGGCAGTAGTGCACTTGGAGACAGGGTGGGCGGGTAACACCTTACTGAGAAGCTGACATTTGTGCAA
AGGCCTGAAATTATCTGGGATAAGAGCCTTCTAAGTAGAGATGTCCCGTGACTTATGATGGGATTACTTCACAATAAACCCACAGTA
AGTGGAAAATATCATAAGTTGAAAATGCACTGAACGTACCTAACCTACTGAGCATCATAGCTTAGCCCAGCCTACCTTAAACGTGCT
GGGAACACTTATGTCAGCCTGCCGCTGAGCAAAATCACCCGGCAGCACAGTGCCCTGTAGAGGACCTTGTAGAGTACTCATGATCAC
CTGGTTAACTGCGAGCTGCCCAGCATCAGGAGAGAGAATTGCATCACAGATCCCTAGGAAAAGATCAACATTCAGAATTTGAAGTAC
GCTTTCCACCGACTGTGTATTGCTTTTGCACCATTGTAAAGTGGCACCATTGTAAGTCAGGGCCATCTCTGAAAAGAACAGCAGGTG
AGGGGCTCTGAGGCAGGAGTAAAGCTGGCTGGGTGAAGGAGCTGCAGAAACCCCAGGTGGCAGGGCCAATGGGCGAAGGTGAGATGG
GCTCAGAGAGGCCCCCAGGTTGGGGGCCCCAGGGGACTTCGGCTTTTTTTTCTGAGGGATACAGGAACTTTTGTCTTACTTTTTTTTAG
AGGGCCTCTCTGACTACTGTAACAGGACTAGCCTGAGGCAAGCGTCGCCCAGGCAGAAGCAGGGAGGCCAGTGAGGCAACTACTGCA
GTGGTCAGGTGAGAGGGCGGTGCTGATCCAGGTGAGAGGGTGGGGCTGGAGCAGGTAAGAGGATGGGACTGGAGCAGGTAAGAGAG
TGGGGCTGGTCCAGGTGAGAGGATGGGGCTGGTCCAGGTGAGAGGGTGGGGCTGGACAAGGTGAGAGGGTGGGGCTGGTCCAGGGTG
GCCACAGACCAGGGATGACAGGCGCTCCGATTCTGGAGATATTTTGAAAGCAGAGGTGACAGAATTTGATGGAGATAAGGGGTTTAA
GCTGCAAGTCACCAAATGAAGAGAAGGGGCTGCAGCCACCCAGAAGACAGCGAAGATTAAAAGGAGAAGCTGGGAGGAGTTTCTGT
TTTCCTACACAGCAGCATCTGCTGCCTGTTCTTTCTCCACATCCGTGCAGCTGCCCTTGTCACTAGGACGAAGCCCCAGGTGAGAGG
CTCCTGCGAGGCTGTGACATACCTCTATCTGTATCACTTGCTGAAGGCTGATGGAGCCCGTGCACATTCACTCCTCACCGCGGCCT
CGCGAGGCAGGCAGGACGGCATCATTACACCCTGCTTCCCGAAGAAGCAGCAGGCCCTGAGTCCGTGGAGTGATCTGCTCAAGGTC
ACAGAGCCATTTGGCATGAAAGGCAGTCTCTGGGCTCCAGATCTTGTGTTCTTTCTGCTATGCCAAGGCTAACCATAGACACACGAG
ATGAAAATAGCACTTTTTTTTAGTGCACCTATGTGCCCGGCACTGCTCTAATCATTTTAGACATATCAGCTCCTTTAACCCTCACAG
TAACTCTCTGTGAGGTGGTACAAGTATTATCCTCTTTTACAGGTGGGAAGACTGAGGATAAGTTCATGCAGACATTAAGGTGCAAAG
CCCGGATTCAAACATAGGTGTTCTGGCCCTGGAACCTGTAGGTTCAGCTATACCCCAGCCAGCTTTGCGGCCTTTCTGCAAGCCAGT
GTGTGGGCAGTAGGTGGATGGTGCTGATGCCACGTGTGGGAGACACAGGCAAAGAGCTGGGGTTGGCTGGGTGGGTCAGGATGGGAT
TTGAAGTGGGTCCTGAAGGGCCAGTTAGAAGGCATCAGGGAGGACAGGGCAGGAGTGACAGGAGCAAGAGAGCCATGTGTGCAAGGC
AAGCTTGGAGCACAGCAACCCTGAAGCGGTGGTCAGAGCATGAAGCCAAGGCACAGTCGAAGGCGGGAGGAAAGCAGGCCAGTG
AGCCGCTGCAGATGCCAGAGGAGGGCAGTGTTCCGAGGAGAGAGGCACCGGTGGGGTCAGAGGCCGCCAGGGGGCCAAGCCAGGTAC
GTGTTGTTGGCTTCCTCATTGGAGAAATTCGCAAGCACAGGGTTCACAGCTCAGTGGGCAGGTCTGACTGTGTGACTCTGAAGATTG
ACCGGAAGGCAAGGACGAAGCAGTCCATTTGGCTGCTTTGGAGTTCTGCAGAGGATCTGGCTCAATGCCTGCAAATTTCCAACAAAC
AAATCAGACCCAGGACAAATGGCTAGAGCCGTCATATCATGTAGATCCATGTGAAAAGAATCCTTTTTTTTTTTTTTTTTTACAAAACT
AGTTTTAAAAAGTATTCAAATTCTTTTGGAGAATAAGGAAGAAGGAGGCAGTCATTTCACTTTGGCTTATTTTCTGTCACCCTAGTGC
TGTAACAGATCACAGCTAGTGTCATGAAGCTGAGTTCTTATTAATAGTGAGAACTGACTCCAAGCTCAAGGAATTTGGCAGCCACCT
TGACAAAGGTTTAATCTGCTGAAGAGAACAGCTGAAATCATTTTAACATGATTCACTGAATTACTGAAGCAAGCCTCACTTTTGTAG
AGGGACAGGAAAAGCAGCATGCAGGCCTTAGTCAGTCCACAAGTATTTTAGTGAGCCCCTATTATGTGCTAGACCCTCAGAAGGTAC
CAAGGAAAAGGCTACAGAGAATACTGTAATATAACTTCAGGGTTAGACAAATAGACCAATGGAACAGAATAGAGAACCAAGAAATAGT
ACCACTTCCACATATAATTTATGAGACAGAGGGAGCATTGAAAGCTAGTAGGGAGAAAGGATGGTCTGCACATTAAGTGAAACTGAGA
TAATTGCTTACCCATGTGAAAAAAATGAAGTTGTATCCCTGCCTCACACCCATATGCAAAATCAATTCCAGGCAAATTGAAGACTTAG
AAAGGCAACATTTTAAAATATGTAAAAATGTTACAATCTCAGGGAGGGAATGATTTCTGCAAAAACCAAATAACTGCTAATTATAAA
TTAAAGGATGATAAAGTCACCTACAGTAAAATTTAAAACTTCTATTCATCAAAAATACTGTAGAGAAAAAGGCAAGGCACACACTGG
AAGAAGATATTTGCAACACACAAACTGACAAAATTCTTATTATCTAGAATATATAAATAACTTTTAGAATCAATGAGAAAAAACAAA
AACAAGCCAATAGAAAAGTGGACAAAAGTCATGAATGACACGCCACAGAAGAGAAAGTATGAATGACAAAGAAACATATGAAAGGCT
ACTTAAGCTTTTATCATGGAAACGTAAAGTCAAGTCTTGATGAGATCCCAATTCACCCACTAGTGTGGCAGAGATTAAAGTCAGACA
ATGGCAAGTGTTAATGAGGATATGGAGAAACAGGAACTCTTATCCACCTCTGGTGGAAGCCTAATTGGCACTGGCATCTTAGAAAAC
AACTTGACATTTCCTAATGAAGTGAAATATGTACATATCCTACAAACCAGCAAACCCAGTCTTAGGTATGTGTCCTAGAGAAACTCT
TATGCAGTGTACCAGGAGCTATGATTAAGAATGTTTGGGCTGGGTGAGGTGGCTCATGCCTGTAATCCCAACATTTCAGGAGGTCAA
GGCAGGAGAATCTCTTGAGCCCAGGAGTTTGAGGCTACAGTAAGCTATGATTGTGCCACTGCAGTCCAGCCTGGGCAACAGAGTAAG
ACCCTGTCTCTAAAAATACAATAACAATAAGTCAATAAAAAATAATGTTTGTAGCAGTATTTCTCATTAGAGGAAAAACCCGTAAGC
TATCCCACTGTCCATCAACAGGTGAATGAATAAATAAATCATGCCATATTCATATGATGAAATACAATACAGGAATAAAAATAGACT
ATAGCTACATATGTCAATATAATATATATGTTGAACAGAAAAGAAGCAATTCATAGAAGAGTTCACTGTATTAGTTATCTGTTGCTA
CATAACAACTTGCTCCAAAATTTAGCAGCTAAAAACAGCAGACATTTACCATCTCAGGGTTGCTGTGGGTCAGGAATTCAGGTGCAG
CTTAAATGGGTACCTCTGCCTCAAGTTCTCTCATGAGATGTGCAGGCTGTCAGCCAGGGCTGTGGTCTCACCTCAAGGCTTGACTTA
AAGAACACCTGCTTCTAAGCTACTCATGTGGTGGTTGGCAGGCTGTGTCCCTTGCCATGTGGCACTCCACGGGCTTGCCTCCCGACA
TGGCCTGTGGATTCTCCCAGGGAGAGTGATCCAAAAGATAACTAGTGAGAGATCACCCAAGACAGAAGCCATGATCTTACCATAACC
TAACTTCAGAAATGAAAGTCTATCACTTCTGCCATACTCTATGTGTTAGAAACCAGTTACTTGGTCCAGCCCACACTCAAAAGGAAA
GGATTGCAAAAGTGTGTGAAGACCTCAAGAGGAGGGGGGGATATCGAGGTCATTTTAGAAGCTTCCTACCATACTCTTGTGGTATGAT
TTCATCCTTATGAAGGTCGGAAATAGGAAAAATGAAATTACATATTGCTTAGGTAATCCTGCCTACATGGAAGCACTATGAAGAAAA
ACGAGGAAATGATAAACATGACATTCAGGGTAATGATGATCCTGGGGTGGGGGGCACTAAGAATGGAGAGAACATACAAGTAGATCC
CACAGGTTGGAGCTGGATGGTGAGTTCATGGTGCTTATTTTCTTTCTGTGTTTCTTGATGTCATTTAATGTTGCATGTAGTTTTGTT
TTTTCTCTTTGGTTTGGTGTTCTGTTGATATGATCAAAGTTACATGTGCATACACTGTAGAGTCAATCCTTCCGTGGTATTTGCTGT
CTTCCACCAGCTCTTCCCATTTACTGTTCTCCAGAGGCAGCTACTTTTACCTTCTTTTGCTAATTAATTTTAGTATTTACCTCCATG
TCTGTAAGTAATATGTGTGTATTGCTACCTTTTGATGCTTTGGTTTATTTAGGTATTATCTATTCCCTTCCCACTCTGGGGAGATGAA
AATTTAGCTCCCTTTCCTCTCCTCCAACTCTTCCTCTCCCTTCATCTTCCTTTTATAATTACATCATAATTTTGGTTAAAGCAGTAA
TCAGTGTTTACATTACTCAGTCAACACTAAATTTGACTCTATTCATAGTTGAGCCTTGTAGTAAAAAAATATGATAACTTTTCCCTTCC
TGCAGAACTTTTCATTTTTCCTGGAGTTGTTAATGATCGTGTTTGTTTGTTTGCTTAGTTTTCTATGTATTCGTCATTAATGCAAGT
GCAAACTCTGGCAGTTGTCTAAACCTCTTTTCAGGATGTTCAGGCACATTAAGTTTCCTATCAATTTCATCTCCTTGGAAGTGTCTC
CCAGAGCCTCTTGACCTGCTCCAGCCCGACGGGTTGTCCTCCGCACCTGGTGTGCACCTGTCCTATGAGGGCCTCCCTGTAGCATC
ACCCTGAGCTCGTTACCCTTTATTCCTTGTCTTCCTCCTTGGTTTACTTCTTTGTTTTGGTGAAGCATACCTCCTGTGTCTTCCTGAG
AGAGGGTGCATGGGAGGCAAACCCGTGACAACCTTACGTATCTGAAAATGTCTTCGTTCCACCCTCAGACTGAACTAATACTTTGGC
CAATTCAGATTTCTGGTCTCTGTTTGGAATTCTGATTTGGAAATAATTTTCCTCCAGAATTTTGAAGGCTTTATTCCATGGCCTTC
TAGCTTCCAGTGTTGCTGTCAAAAAGTCCGAAGCTGTTGTAATTTCTGGTTCTTTGTATTTGGCCTCTTTTATACCCTCCCCCAAAA
CCCCTAAAGCTCTGTAGGATTGTCTCCTTTCCCCTGGTGTTTCACAATAATGTGCCTTGGTGTGCATCTATTTTCATCC
ACTGTGCTGGGGACAGTGAGCCTTTGATCTAGAAACTCATGCCCTTAAGTTCTGGGGATTTTTTGTTTTTGAATGATTTTTAAAATAA
TTACTTCTCTCCTTCTTTTCTCCTTCTTTTTCCCCCCAGTTGTTTCTTTCTGTAACTTCTACTGTTTGAATTGGGAGACTGATTCACTA
ATTTTCTCATATTTTCTTTCCCTCTCTCTCCTTTTAAGAGATGATGGGGGTCTTGCTCTGTCCCCAGGCTGAAGTGCAGGGGCACAA
TCATAGCTCACTGCAGCCTCAACCTCCTGGGCACGAGCAATCCTCCTGCCTCAGCCTCCGAGCAGCTGGGGGATACAGGCACACACC
```

117

```
ACAATGCCTAACTTCATATTTTCTTTCATTTTCATTCTTTGTCTTTTTCTGCTTTTTGGGAGAATTCCTCGATTTATCATCTTCCAG
CCCCTCTAGGAAGACTTTTTTCATACAGCATTCCATTCTTGTCTCGTGGGTCCAGTATCTTATTTTCTCTCTCTTACGTTAATTATA
GGATTTTTCCTTCAAGAGTTCTCTCTGTGAATGGTCTGTATTTGATCCAGAGTTCCTTCTTTTTGTTTGTTTAATTGACTGGTTTGA
TCTGTGTCTTTCATGTTAGAGACTCCTGACTGTCTGGGGATTCCTGGTTGCCTGTTTATTTTTAAAAGTAGGAGACTAACAACTTTG
AGCTGTAAGGGATTTGAGTGAGCAAAAGTAGACATATGTCTCTACCATCTTTGCCAAGTCATACAGTCTTTTGTATCTCTCATTTAT
TTCATGTTAAGAAGTGAAAAAAAATCCATAGTCCACACCCTGAAAGATAAATCAGTAACTGTAATAACTACCCTTGAGCTCAAAGAA
AAATTACATCTTTTCCTCCTCAGCCTTTAAAGAAAAACATGGAGCTATCCCCCCCCCAAATTTTCTATTGCAAAAAATAGCACAAG
ATTAGTATGTGAGTTTGTGACATGTGTAAACCGCTCTAAAATTGTGAATAGGTTTAGAAACCTTTTCTCCCTCCTCTCTACTCCCAC
AGTCCTGAATCACCAGCCCAGCCTTCTGGCTCATCACTTCCTGCTTGGTGAGTTGCATGTATGTCTTATCTCCATGACGAAGCTCCA
AGCCACTGGAGAGCAGGGTGCATGTCCAATTCATCTCAGCATCTTCCGTAGCCCCCACCAGAGAAGCGATCACTGAAGGCTGACAAA
CAACGAAGGACACTCTCAAGATCTTCACTCCTCTCCTCAGCACACACAGTTAGCACGCACAGGAGTTTATTATGGTGAATGAAAAAA
GAAACCCACAAGAACATAATTTATATGTAATTCCACTTTGATCTCTAATCTCTTTACCTTGGCATCCTCCCACATATGAATAGGATG
GTCCATGATCCTAAGGGCAGCCAAACAGCCAGGGAGCACGAGTTTAATCCTCAGGGTTTGCAGGGAAAGGAGGACTGTGGGAGACCCA
CGTGCACAAGAAAATCCATGATGTGCACAGAGGGCAGGCTACAAATTAATCCTAACTTCATGGTTCGGTATTCAAGGCTCTCCATTG
CTTCTCCCTGGACACCTGCAAAGCTTCTAGCAGCACTGCTTATCTGGGGCCTGACCCCTTTAGTCAGTCTTTCATGTAGAAGAATCA
TTTTCAGATACTAAGTATCTCATGTCTTTTCTTTGTCCAAGAACTTACAGTTGATTTCTTTTGTGTCTGTATCAAATAGTTCTCCAG
CCTAAACTTCAAGACATCTTCATGAATGCCTCACCACTTCCACTGCACGTGTAAATCCATGCCCCTGTATTTTTCTGTCTATTGTTTC
TCAGACTGATAACACCTGCGCCCTCTCAGCAACAATAATGCCTGACATTTAGTGAGCACTTAATTTGGGTCACTGTGTCTGGTATTGC
TTGTGGGTTATTTTACTTAATCCTCCCATCATTGGATAAGGAAATTGATTGACAGAGAGGGCACGTCACTTGCCCACAGCCATGGAG
CTACTTAACTCTGGAGCTGGAGCTCCAAGCCAGGCAAGCCCAGCCCAGCCCAAGATCACTGGCTCAGCTCAACTCCTCACTGCTAAA
CTGCCTCAACCACATTTCTTCAGACCTTACCCAAGACTTATCTTCCAGGAAATGTTTTTCGGAAGAACTGAGGACCCTGGCTGTGAC
TGTATTACATTTATGTGTTGCAGGCTTGTGCGTGGAGCTGCCTTTACGTCTTCTCCCTTCTGTGTCCCCTCCCACAGTCACTGAGT
CTTGCTGCTTTAGAGAAGCTAAAGTCTGATTTTCCACCACTGCCTCAGCACAGGGCCTGAGGATAGGGTCTGCCACCATCCCTCCAG
CCAGAAAGCAACCTGCTGGGAAGGGAGGGGTTGCATTGACCTGAGGGCCTAGCAGGGCAGAGTGAGCAGGCCAGGGAGACAGCTGCC
TTTCCAAGGCCACAGCAGAACTGGGAGGGGGCTGATGCGCTGGGTCACGGTCAGGGGTAGACGCAATCCTGGGAATCAGAGTTGGGA
GCCACCAGCCTGTGCTACTCAAGGATAGCCAGGGGAGCAAAGGCAGGCCCAGCCCAGGGAAATGAGGCCCACGCAGCCAGCCAGGGG
CCCCTAGGGTGGTAACAGAGCATCCCTGGGGAGCAAGTATAGCAGATGGAGTGGGCAGGCAAGGCCATCTCATTAGCAGAGCTTTGG
GGAGGGGGTATCTTTTCTTCAGATGTCCCCAGAAGTACCAGACACCCATGTCCTTGTGCAGACAGCCACAAAAAGGCTAATGCTGTG
CCACCCACTGGGTCTCCAGTTGCTGGCCAAGGACTCAAGTGGGGCTGGAGAGCCCACCATTGATGTGCTCGCGGGAATCTGCTAGGG
CCGGGCTCCGGTGGGTGAGAAAGGCTGTGGCCTGCGTCTCTGTCCCAGCTTTGCACACCTGTTTCTTGCCTGGTCACCCCACCTCTG
TGTCCTGTCTCTTCAGATACCCTGCTCGCCACCCGCCGACCATTCTTCCCAGCAAGCTGTGTTATCTTGTCATTCCTGTGCTCAAGA
ACCCAGAATCTCTTCTAAGGCCTCTAAGACAAGATCCAAGCTTCTTCACCCAGAATTCCAGGCCTTCCTAAATCTGACCCCTGGTCT
CTGAAGCCCCCTGGTCACCTGCTACTGGAGCCCAGGTGCTCTCCTCTCACTGAAGCTTCCTCATTATTCCCAAGACACAGCCAGTC
GCTTTTGCCTCTCTGATGTTGAGTGAAACAGTGAAAGGAGCATGGGCTTTGCTTCCTGTGTGTCCTTAGGCAAGTCACCCAGTGTCT
CCGTGCCTCCTGCTGCTTACCTGGGAAATACTTTAAAGACTGTTGTGAGATGAGAATGTTTGCGTACAAAGTGCCAGCCACACAGTA
GGTGCTCAATACATGCACAGTATCCGTCATACCATGCTCTTGCTGCTCCCTGACTTGATGGCCCTGTCCCTTTCTGACTGGCTGAGC
TCCTGTCTCCTTCCAGGCCCATCTCTAGATGCATCTACACATGCAGCCTGCCCTGCACGCCTCACTTAAGGCGCCAAGGGAAGGAAA
AGCAGCATTTGCCTATCACTCGCTGGGCACAGACCCTGTGGGGGCATGGAGATTCCTGATGACGGCAGCGAATCCTTCCAGGGGGCT
TCCTTGTACTAGGCCTGTCCCGGCACGTTGTGTGCATTAACTCACGGAAGCCCCAATGGTAACTGACTTCCATAGATTAAGGTTCCT
AAATGGCTTTTCTGCTCTATCCAGCCTAGTAGGTTTGGTCTCCCTGGCCCTTTGTTCTTAGCTTTCCCCATCCAGTGGCCTCCAAGT
GGTTAAGAGGAGATTTTATCAAGAGGTGAAGCCCTACAGTATAAAATACAGAGTCTATTGTGTGTTGTTCAGGCTGATCCCATTAGA
GAACGGTTTGGAAGAGGACTCACCTTCACCAGCCCTGCCACCCAGCCTGCAACCAGCTCCCTTGCTGCACTTTGTATAAGCCCTGGG
CTTTCATTAGGGTTCTCTGCCCAGAGCACCCGTTCCTCTGGGCCTGCCTGGAAAATGCAAGCCTTGCTCCTCTGTGGCACCTCTGTG
GACCCTCATGTGTTTGGGTCAAGGTCATTGCCCTCTGCCTCCATCCCCAGGGCACCTTGTCCACCCCCTGATTGCACCCCTCTGCAG
ATGTCTGCCTCCTTGGCAAACCCCTCTCTTGGGCTGGGTCCCTGAGGGCAGGGGCTGTGTGGTATTCATCTCTGTATTTCAGTACTT
GGCACAGAGCCTGGCATGCAGCAGGTCTTGGTGTTGGGGATGAAGGGAGGGAGGAAGGGGATGGGGTGGAATAGGCATCCGGGCAGA
GGAAATGGTGCTGATGTTTCCAGTTGGTTCCTCCCAGGGCGGGGCAGCTACAAGCCCCTGTCTGACTCAGAAAGGGAGGGCCTCTGTG
GACACCCCAGAGCCCCCTCTAGAGACAGCTCCACGAGAGCCCCAACCACCACAGCCTTCCCTCCAGACCCCCGGGAGATGCCAGTCTT
TGAGATGCTTCAACTTTCTTAGAGGAAATCGCTGAATATGTTTGGTAATTTTTTTTTGACATTTTCCCTGTTCAGGAGCTTGTGATT
TCAGATTAATCACATCCACATACATTTAGACTGTCGGCTTGGAAGGGAAAATTTGAATTTTATTACTGCATGATTTCATCCAAAGCT
ATCTTACCAACACTAAAAGGATGTCTACAATGAGGTGCAAATCATGACAAAACATTTTATCTCTGGGACCAAGCTCATGGCGGGAGG
CCAACGGGACGGGGGGCTGCAGGTGGGACCTGGGTGGGCACAGTTGGCCACCCCTTCAGTAGCCTAAAGAGATCTGTGTGAGCCACCG
TCACTGCTGCCTCTTCCTCAGCAAAATATCTCATTTCCTTCCCCGCTCTAGGTTTGCCCACAGCCCATTACTCCCAGGAATGGCTGTT
CATCGACTAACTCCTGTCTTCCCATTGGCAGGTACCATGACCAGCAGGACGTAACTAGTAACTTTCTGGGTGCCATGTGGCTCATCT
CCATCACATTCCTTTCCATTGGTTATGGGGACATGGTGCCCCACACATACTGTGGGAAAGGTGTCTGTCTCCTCACTGGCATCATGG
TGAGTACCCGCCTCTCCCCATTCCCTACCCTTAAAAATGTGGCAAAGAGCTGAACTCTTTCTTGCAAGCACATAGGGGCCTTCCTAGC
CGCAGAGCGGGGGGTGGCTGTTAGTTAACCGACCTGCATGTGTGTCCCCTACACATAAAATGTCAGGTGGGAAATCTGCAATGGTTT
CTCACTTTTCAGTTAACTTTTCTAAGGTAAGAATGAGACCTGGACTCCTGGCAGTTAATGGTCTATTCAACAGCTCCCTCAAAATGGG
CTGGGTGAAGCTGGTCCTGGCCCAATGCTCTGATGCCCACCCCACTGCTGTGCAGCCACCCAGTTTGCTCACCTCCAGTGAGCACTG
GAGCTTGCAGTTTATTGTCCTCCTTCCTGTTGCCTTGGTCCCACTGACCTTGGCACCATTTCTGATCATGTTTAGCTGGAACTCACA
GAAGCAGGGACTGGGGAAAGAGCACTGAGTCGGCCTTTGGGACCTGAGTCCTGGACGGGGCTGTCCACATGGCCCTGGGCTGGCTCT
TGCCTTTCCCAGGCTTCTGCTTCCACCTCTGCCTATGGAGGGGGCTGGGCTCTGAGTGCCAAGTGCTTTGCAGCACTGGCATTTGGCA
AGGCTGTGATCCCATCTCCAGGTGCCCAGCAGGTGTGCGGTGCCCTTTCAAGGTGTATCTCTCCTGCCCAGTGCCCTACCACCCACA
CTCGACACTCTCGCGGAGGCACACACTCTCACCTGTCACATGGCCATCACAGTGTGGCTTATGAGAGGGATGTGGGGTCTGAGTTTC
TGCTCACTGTGGACCCTGGGCCAGGCACTCTGCATATTGGACCTGAGCATCCTCCAGGGGAAAGTGGAGATGGAGGGCCTGCCCTGC
CCTCCCCATTGGTTTGTGCAAGGACCAAATGCAATGATTTATGTTAAAGTGCTTTGAAAACGAGAAGCGGCCACACACAGGTCAGTG
TTGGTGGTGTCCTCAGTGTCCTTCAGTTGCAAATTGGTGAAGTGTTCCCTGGGCCTCATTGCACTTTGGGTCCAGGACAGGCAGCTG
CTGCTCTTTGGCCTTCGTCCTGGAATCCTAGAACAGTGTCAGACACAGGGTTGGCACTTTTTTCAGAGGAGGGAAACTGAGGCTGAAA
GAGGAAAGCACATCTAAGCCTGGGTAGTGTTGGGGGAGGTGGCGGGGTGAGAAGTGGTCAGGCTGCTTGTGGTGTGGCTCTGGGTCT
CCATGGCTGGAGACCGCAACCCTCCCAAGGACAGCCCCCCGTCCACAGCCCCCCTCACACCTTTGTGAAAATCCTTCCTTGCAGTGA
TCCCTCAGATTCCATTTGTCACCCCCGTTGCCTCCAGAAGAGATGAAGACCCTCTTTCTGGAAGGGCAGTGACCCCTTTCTGCAGTG
CAAAGGGAGGGCCGCAGGTCTTCCTGCAGGGTCTGAGTGAACGCCTCACAGCGGCTGGGGGCTGCCCCCACTCCCTACCCCCACTCCG
TCTTGTTGGCTTCCTGGGGCTGATATTGGTGGACCAGACGGTCAGACCCAGGCTTGGGGCAGTCAGCCTGCTGAGCAGGAGGGCCA
GGCTCTCTCCTCCTTTCTGACTCCAGTCCAGGGCAGGTCCCTATTAGCAGGCAGGTCCTACGACAGCAGCTAATGACAAAAAGCTTA
GCGCTGGGGTTGTGATAAATCACGGGTAAGTGCAAGGCTTTAGAACAAATACCTCCCATCGGTTAGTAGGAGTCTGTCTTGGCATAG
CACCAACTGTCACGAGGAAACCGGGGAAAGACGCTTCTTACCAGAAAAGGAATGAAATGCCACTGAAGGCCAAATCTTTCCTTCTTAG
```

```
AGTCACTCCTGGGGCTTTGTGCCTCAGAACCAGCTCTCAGCCAGGGCTGGCACAGAGCAGGGTTGCCAGTAGGTGGATGGGCCCTGC
CGGGAGCCTCTGTTATTTTAAAATACCGACCTTTTCCCCTCTCAGCACATAGCTGTCCCCATCCAAGAAGCAGCACCCCAAAGACCT
GGCTTCCTGGTTCCTGGACTCCGCATCTCCTGGCTCTACTCTGGCACAGCTGAGCGGAACAGGCGAGACCCTGGCCCCACCCCTGCC
TCTCTTTGGGGTCCGGAGGCTGTTTGTCTTTCAGCACCCCCTTTTGCTGTCTGGGTGTGCAGTGCTCCCCCAGAGAGAGCGTTCTGT
TCCCTGTGGATTGAGGTGTGATCAAAGTGGAAAGAAGAAACGGACGATGAGGAGCCAATTCCCACCTTCCATATGAAAAATACAGCC
CAGGCCTTTCATCTCTTTTTGTACAAAACCCTGTGCATAAATATACACCTTTCTATATACAGGAACAGGGAGGTTTCCTCTATTTAT
TGTCAGTGTTTTTCACCCAAATTAAAGTCAGTCGCCTCACTCTGGGGTCAGCCACAGTGAGGAACCCTGGTGGCATTTTTCTAACAA
ATGTGATTTTTCTAGCTGAGTCACTCCTAGCCAGGGTTTCACTTTGCTGAGGATCCTTTCCCCTCCTCCTCCCAACCTCCCAACACT
ATGTATAGCTCCCAATTATTATTGCAAGTGCCTTTACTAAAGGTAGGGCTTCCTGTTTGGTTACCATGGACACCAGGAAGGCCAAGG
CAAGGCCTGCTTGGGAAGGGAAAGGAAGGATTTGTGTGTGTGTGGCCTGTAAATAGTCTTAATCATTGTTTTCTCTTGGCAATCCCA
GTAACAATGTAATTATCAACCATTAAGCAGGGTATATAATTGTCTGAACAACTTGCCATGGCTGGCACAGACCTGGTGTGTTTGAGA
GATTTCACCCACAAAAGCAGCTACATTAAAGTCCAGCATCTTTCCATAAGGATTTTTCATGATCATGGTGACAACACAGTAGAGAGA
AAGCTGTCAATATTTAGTATCCAGAATTGGAAATGACAGTGCAACCTAAAAGATGTGCAATGCTGACCTTCTGATTCTGTAGGCATC
TGGAAGACCCCAAAGCCCCCAAGGCTGCTTGGCATAACTTTGTTTCAATCTAGCCTCTTTCTAAAGCTGGACTCTCTCTGTAGACCC
TTCATCAGTTATGGACAAACAGGCAAAAGCTTCTGGATATCTGCAGGGACGCTGAGCTTTTCCCCAATGGCCTCATGAGAGATGCCT
CTGAACTGCCAAGGGAAAGTGATGATGAAGTAAAAGGTAGCACAAATTGAGTCATTTCGGGAGGAGGCAAAAAGAGGGCTCAGCAAG
ACATCTGCTTAATGTCAGTTTTAGAAGATGAGCTTCTCCCGAAACACCTTGGTTTCAAACCAATTGATGTTATTTTAGTTGTTGCAT
GGAATCTGTTGCAGAAACAGAATCCAACTGTAAATCCTCCAGGCTTCCCTCCTCCATTTGTCACTTTCATTTGTGTGTTTTATTTTATT
TTATGCTTCTTTAGTTAAGAACACCTCATACTTTTCCCCTTGGGATACTTGTTTTTCCAGCTTCTAAGGTTTTCCAGCTGGAAAATG
TTTCTTGAAAACAAGAGGTGGGAGGACTGGTGTGGAGGAGTCTCGGTCCAGATTTGGGTCCGAATCTCTAGAGTTAGGCGTGTTCAC
CCCGTCCCAGCCACCCTCTCTCCATGCACTCGGTGCTCCCTCTCGCCCACGTGCCTTGGGAAGTTCCAGCTCATCAGAAGTGGACCC
CATCCACTCCCCTCGCCCCCATGGAACTGGAAGTTGGACAGAGCACATCCAGGGACTCCAGTTGATGTCCCCTGTGGTTCAGGACTTG
GCATATCTGAAAGCAAAAGGCTGAGTCATGGCTCTTCCCCCCAGTCTGACCCCAGGGAAGTCATAGCAGCAGCTGTTTACCCTTTCC
CATCCTGCTTCCTCCCTCCTCACACACCCCTCTACCCACCAGCCATTCAAAGATTCCACAGTTAGCCAGGCCCTCGGTGATTTGT
CTCTTAAAACTAACCTGCATCACTGCCATGTGACACAGCACTGCCAGGCTGCCACTGTGGCCTGTCAGACTATTAACTCACCTAAGT
GAAAGGGCCCAGCATCCCAAAGGGGACCCAGGGGACAGGCAATCCTTATACCACTTGAAGATGGACACAGCAGGGTGGGATCCCTTG
GCACTTCCTTCATGTCCTCTAAAGGGCCAAAATGGACCATTGACTTCTGGCTCAAGCCTTAGGCTCCTTGTTCTTCCTTCCTCTCTC
AATCCCATCAGCCCATCATTTTTTCTTATTCTTCAGCCTCCTCCTTCTCTCTTCCCCCCTTGACCCCTTGCTGTTTAGGATGCAACG
CAACTTGTTCCCCATCTCATTTTCTGCCCAGCTTTACCTCTCCCTGCACCCACCTTCTCTTCTCTTCTCTTCTCTTTTCTCCTCTTC
TCTTCTCTTTTCTCCTCTCTCCTCCCCTCCCCTCCTGTTCTATTGTCTTCTCTCCCTTCTTCATTTTCCCGCCCCGCACCCCGCACA
GTCTGCAGAGGCTCAGCAGTCTAGGCTTCAGGATTCCTCCACCCACTTTATGAAGGCTGCTGATGTCTGGCCCCATCTTTATTAGGTT
GCCTCTTAATGGAAAAGACCAGGTTAGGTGTCATTACTGGTGACTGCCCTAATTACATCCAGTCAATGGAAAAATGAAGCTGAGAGC
ATCCATTTAAGTTGGAATTAAGGGTTAAGAATGCAGCATCCTCTAGAGGAATACTCACAAGGGAGTCATAACATCTCTATGTTGAAA
GAAACTGGGAAGTGTCATCTAGTCCACTGTCCTGCCTCCAGAGAGAAATTTATCTTCATCATGTGAGACAGGTGGTTCATTAGATAA
AACACTCTAGCTTCTCATTAAAAGCCCCCCCTGCAATCAGCCAACTAAAATGTGGGCCATGGCAAGCGCTGTGAAACACCCCATCTA
GACCCTGGAAATTTGGAATGGTGGATGATTGATTGTTGTGATTACATTCCCAAAATAGCATGGAACAGGGAAGCTCCCTCTTCAGGC
CTATTGGACCCCAGCTTTCAGGGGATGATTTAAGTAGAGTTTTGCCTGGGCCTAGCGAGGAGGGGAGAATGGAACCTGGGGAGAACC
CTCTCCATCTGATGTTCCATTTTTAACGCTATGATATAGCCCTCTTTTTGGGGCTTTCAGCCCAAAGTACTTTTGGAACTCCATATAA
AGGAGAGGGATGATACTCCGTCCGGTCGAGGGGAAGGGAGCACACATCTTCCCTCTAGGATGGCTTCTTTTAAGCTATTTTTTGCTT
TTGGAGGCATGGCTAAATTGATTACAGCTACAGACCAGGCTGTGCAACTGCCCAGATCCTTTGAAAATTGGGCACAGAGTCTCCTGT
AAGTCACAGAAGCTGAAAAAACACAGCCGTCCTCGAACAAAAGAGGAAAATGAGGTCAGAAAAGGAAAGTTCACACAGACTTGGTTC
TTTTCACTTTGTGACAAGGCCAACAAATGTACTGAGAAACACTAGATTGTTTTAATGGGGACCCTTGTGGGAAACGAGGAGAAAGCA
TTGTGAACTTCAACCACATTGAAACTTCACACAGGCTAGCCTTTGTCAGCCTGTGGGATAAAGAGAGAAATAGCTTCTAAAAGAGAG
AGTAGGGAATGGATCCACCCATATGCATTCCTTCCAAGTCGGTTATAGATTATAGTACCTTTGAAAAACATTCTTTTCTGCTTCTCA
CACATTTTGTAAGACCTGATGAGCCAAAAATGTTACTGACAGATTCCTTCCATTCTGGGACTCAACTGCTAGACTGGGTTCTGTGG
CAACTAGAATTACTGTTTGACATACAGGATTCGGTTGTTGGCTTTAATAAGTTCATCATCTCTTTCCATGAGTGGTGGTCAGGGCGG
CGCAGGGCACAGCATGCAGGCAGAGGGCGATGGTCTCTGCAGCGGGAGTCCGCTTGCAGAAGGATGGAGCTGACTCCCGCTGCCTGCCG
AAGAGCCAGGGGAGTAGTGAGTCTCTGAATTCCCCCTATGCTCACAAGCCAACCAAGAAGCCTAGCGAAGAGGGATTTTAAAATGCAT
TGGGAGAATGTTGGAATGTGCAGGGAATTCCCTGCTTATTTCCCAGACAGTCAAGTATGCACCCAGAGGATTAGGAGAGAGAAAGTG
AGTCCCACCATTCTGGGGGCTCAGCAGAGCCTGCTTCCTTGCCCACCCTCCCCCGGGCTCCCCTGGCACGGACCTACCGAGCAGACT
AGGATCGCTGTTCCAGAAAATCCCAAGGGTCTGCTGCGGGCTCACTTAAAATCCCGGTTTTGATCCAGCAGAAGTGGTGTTGTCTTT
AAAATCTCTGCATGACGGTGAATTCATGAGCCTCCTGGTGAAGGCAGGAAGGAACCGATTCCTTGTGCTGAGTCATAGCGTAAACCT
TGAGGTGAAAAAGCCCAAGTGTAGAGCAGGCACAGTCACTGAGTGCACGTGTTCGTGCCAACCTGGGTGCCCTCTGTGAAACAAGCC
CTGGGGAAAGGTATTGCCTCTTCATTCGATTATTTTCAGGAACACTCTCAGCCACTGGCAGGTGTTCCATCTGCACCCCAGCTGATG
GCCTACCTTCTTTCCTCCCAGGCCTGCTGGTCTAGAGCTGGCAGTGGGATGGAGACCTGCCTGAGGGAGAGAGGGCCCGTGTGTGCT
GGCCCCTCTGTCTTCCGCTGCACAGCTTGGCAGCTGACCAGGTGCTTAGTTGTTGGTTAAACCCCCCAAAGCCTCGGCGCCACCCAG
CATCCTTCTCCATTGAAACTGGCTAGCTCAGCCTCCCAGAGGTCAGCTGCAGAGCACAACCCAAGAGGCACTGCAGACCCCTCTGTG
GGTCAGGACATCAGAGTCCTGCGACAGATTTCAACAGATTTTTGTGCGTTGGTCGTGCACCAGCCAGCAGAGAGCTCAAGTTCCCACTCCACT
CCCAGGAAATTACCCCCGTGGCCGCCAGGCGTGTTGAGTCTCTCATCTTGCCTCTTAATGATATGTTCTTTCCCCTCTGGTCCTCTT
GATGTCCCCCGCAGGGTGCAGGCTGCACTGCCCTTGTGGTGGCCGTGGTGGCCCGAAAGCTGGAACTCACCAAAGCGGAGAAGCAC
GTTCATAACTTCATGATGGACACTCAGCTCACCAAGCGGGTAAGATGCCACACAGCCATGTCTTATGGGGGACTTAAGGCCACAGTG
GAAGCCAATCAGAAAGCAAATGGTCCAACGGGTGCAGTGGCTCACGCCTGTAATCCCAACACTTTAGGAGGCTGAGGAGGGTCGATC
ACAAGGTCAGGAGATCAAGACCATCCTGGCTAATACGTTGAAACCCATCTCTACTAAAAATACAGAAAATTAGCTGGGCATGGTGG
CACACACCTGTAGTCCCAGCTACTCAGGAGGCTGAGGCAAGAGAATTGCTTGAACCTGGGAGGCGGAGGTTGCAGTGAGCCGAGATC
GCACTACTGCACTCCAGCCTGGGTGACAGAGAGAGATTCCATCTCAAAAAAAAAAAAAAAAAGAAGGAAGGAAGGAAGCAAATGGTCA
TGTCGTATGGGTTTGCACCAGGCTTTTAAAGGGTGTGTTTGAGGGAACCCCAGACACAAAATGGAAACAGCACCTTCATACGCCGGG
TTCCTGTCTTCTGTTCGATCACTGTGCATGGTTCAGGCAGCCAAATAATTTTCCTGGTAATTTAATTAATAGCCTACAATGGTATAA
CCTGCCCCTTTGCCTGATGACCAAATAAATCACGGTATGAGGCAATGTTTTTTGTTTATGCTCAAATATTTTCAGGATTCTCATT
AGGAAGGGCAAAACTATATTGAAAAATATACCTAAAACTATAAAAGTATATTGAAAATGTACCTAAGTCTAATCCTTTAAAAAAAATC
ATTTAAAGAATCATTCTATGCTGGGAGCGGCGGCTCACGCCTGTAATCCCAGCACTTTGGGAGGCCGAGGCGGGCGGAGCATGAGGT
CAAGAGATCGAGACCATCCTGGCCAACATGTGAAACCCCGTCTCTACTAAAAATACAAAAATTAGCTGGGTGTGGTGGCAGTCACCT
GTAATCCCAGCTACTCAGGAGGCTGAGGCAGGAGAATCACTTGAACCCAGGAAGCGGAGGTTGCAGTGAACCGAGATCGCGCCACTG
CACTCCAGCCTGGGCGACAAGAGCAAGACTCCATCTCAAAAAAAAAAAAAAAAGAATCATTCTAGTAGAGCCCACATCCCTAAATAAAA
TTGTTCATCAAAACATCATCATCCAATTTTTCCCTTTTGGGTTTTTCCGGGTAATGTTTCTCACTTGAACCATTATGGAAGTGGTTAT
GGAAGATGAAAATCTGAAAATATGCAGGAATGGGTAGCACATTTAGCTGCTGAAAGTTGGACACCCCACTGAATTGTCTGAGATGAA
AAGACCCATTTAGAATAGACAGATACAAGCCTTCCAGAATAACAGCAGGCCAAATGGGCAAGGCAGGAGCTGTTTATTCAAGGACAG
```

```
CTTTTATGGAAAATTCTTTAGAACTACAGCCTGAAGGAAGGTCAGTAAGATGATTTCCTGCAAGACTGTCCTCTTTCTGAGACGAGA
CAGAATTAAAACCCTGTGCCCGTGTCTCGGCCTCCCATAGTCTTTTTCATTTAGGACTGAAGTAGGTTAAGGGACCCATGAGCAACCA
AATTTTTGATTTGGGTTTTTGAAAGCTAGAGAAAATATTCAGTGATTAATGTTGCACAGCAGCAAAAATGTGTTAGAAATAGAGACAA
CGGCAGGAAGGAAGAACAAAAAGAGAGAGAGAGAGAATGACAGAGGGAGCTGCGAGCGAGCTCTTGGGGAAGGCAGTGGGAGAGTGT
GCAGCGGCCAAGGCCTCTCCAGTTTCTCAGCAGAATTATTTATGGTGTTTGATTGTTTGGTCTTTGATTACATTTATTGGATTCTGT
AAAGAGCCTTAACAGACATTAAAAGGAACTGGTACGGGCAAATATTCTTATGTTGTTGACATTCAAGTGTTTGTACTTGATACATCA
TGGCCCTGAGCTATCAGCTGCACCAAAAAAAGCCATGATTTGTCACATCACGATGGCAGTGATTGAAGGCTGTTATCACAGTAAGGC
CTGCCGGGAACCCTGCCACTTGGCCACTCTCTTTCAGCCCCCTCTAATAATGGGTGCAAAAGTCATGCTCAGTATTGAGGGAAATTAT
TCAAGTTATTGAAACACCTAAATGCAAATCCACTTGCATCTATTTTCCTGAAGCAATTTACTGAGGTTTCACTGGATTGAAACTACG
TGCCGGAGTGAGGGCAGGGAGATGTTTTGCCTCCAAAGCCTTCTCATAAGCTGTTTACCAAGAGTAGAGACATTAGCAGTCTCTCTG
TTTATTCTGGTTTTCTTTTTTAAGCCAAGAATATTCTTTGTATTTGGTGTTATTCCTTTGTCCTCTCCATTTTTCACCAAGTAGATC
AGACGCTAGAGGAAAATAAAGCTGAGGAAAGCCAGCTCCTTTGTATGCCAGGAAAATAAACAATTTTTCTGCAATATAATTGTAGAA
GATGAGACTGTGTGGTTGCTAATTTTTAATCTTTCAAAAATGATTCCCTAACATCCTCCTCAATTCCACTTCTTGAAAAGTAGGACTGAA
AATCAAATCAACAAATATTTATTGCTCACCCATGAATCTAAGGTAGCTTTTTAATCGAATAACATCTCACTTTTCTGAGAACACTTA
AATCTGTTGAAATGTCTCTTTAATATCTAATATCTTTTCTCAAGGAAATAAACAACACAGAAGGGATGGTTCTTACGAGAGAAGGT
TTTCAAAGCTGGCAGGACTAGAACTTCTGAAGGTTCTTTTACATAACCTTAATTGAAATGAAACTGGATGTGTGCATCCTTGGCCTG
CTCTGGTTTGAGCTGACTTTGACCTGCTCACAAGAGAACCCCACCTCTCTTCCCCTGGGGATGCCTGGCCTGCCTTCTGTCCTCTTG
AAATGGTCCATTGTATCAAGCTGGAGTGGCTCATTGGTCAAGAAGATGCTAAGCAGCCGGGTGCGGTGGCTCACGCCTGTAATCCCA
TCACTTTGGGAGGCCGAGGTGGGTGGATCATGAGGTCAGGAGTTCGAGACTAGCCTGACCAACATGGTGAAACCCTGACTCTACTAA
AAATACAAAAATTAGTGGGGCGTGGTGGCACACACCTGTAATCCCAGCTACTCAGGAGGCTGAGGCAGGAGAATCGCTTGAATCTGG
GAGGCAGAGTTTACAGTGAGCCGAGATCCCACCACTGCACTCCAGCCTGGGCAACAGAGCAAGACTCTGTCAAAAAAAAAGAAAAAAA
AAAAAGAAATACTCACTCTCAGACATGATCCTTAGAAGCTCTTGGCCGGGCGCGGTGGCTCATGCCTGTAATGCCAGCACTCTGGG
AGGCCGAGGCAGATGGCGTCAGGAGGTCAGGAGATTGAGACCTTCCTGGCTAACACAGTGAAACGCTGTCTCTACTAAAAAAAAATCC
AAAAAAAATTAGCCGGGCGTAGTGGCACGTGCCTGTAGCCCCAGGTACTCGGGAGACTGAGGCAGGAGAATCGCTTCAACCTGGGAGG
CGAAAGTTGCAGTGAGCCGAGATCGTGCCACTGCACTCCAGCCTGGGCAACTGAGCGAGACTCCATCTCAAAAAAAAAAAAAAAAAA
ATGCTAAGCTCAGTCTATTCATAACATCTCAGTGAATTCTCAGAGAAAGATCCAACATGAAGGAGTTGACTGTGGCATAATTGCAAC
CTCTTCGGAGTGGGCAATTAGTAGATTTGACAGATAAAAGCAGTCTCTTACCGGTATATGCAACCTTGATGCTTCAAAGACCTACGC
AAAACGAAAAAAGGAATTGTAGCTATTAATCATAGAAAATACTCAAATTGTTCTAGTATTTATTCATCCTGTCTTGCTCTAAAGACT
GCAGCCTAGCCTTTGCCATGAGAGCCAGCACTATTTTTATTTATTTGTTTGTAGGGAGGGAAGAGGGTCTGATAGCACTGGGTAAGA
ACTCAATTTCTGCTTTTCTTGAGTCCCATGCTTATCTTCTTTCCTCGTATGACTTATCAACTCTGGTGGCAACGTCATTTCTGTATT
TAATTCAATCAGATCTTCCCACACTGACTTCGCAGTGTCACTGCCATTGTTATTCTGTAATTATAACTTATTGCTAATCTCTGGGCT
ACTAGATAATATTTAAGTACATGATTCACAGAGGGACTGGAAGAAAGATACTAGAGCCCCATCTTTTCTGCCACTTAGTGTTTTCAT
GTCCCCATCTTTCTGTTTTATGTAGCTAAACAGATCCATCACAGAGGCCACTCCCTGGGTGCCACACTCTGTACTCTGGGGGCACCA
AGATGGGTAAGAGGTGGTCCTCACCTCCATCCCTAACCTCTGGTGGCTGACTGCCCTCAGAGAGCACTTCTTCTTATAAGAGGTAGA
GTGGGCCGGGTGCGGTGGCTCACGCCTGTAATCCCAGCACTTTGCGAGGCCGACGTGGGCGGATCACTTGAGGTCAGGAGTTCAAGA
CTAGCCTGGCGAACATGGTGAAACCCCGTCTCTACTAAAAATACAAAAAATTAGCCGGGCATGGTGGTGGGTGCCTGTAATCCCAGC
TTCTCGGGAGGCTGAGGCAGGAGAATTGCTTGAACCCAGGAGGCGGAGGTTACAGTGAGCTGAGATCGTGCCACTGCACTCCAGCCT
GGGTGACAGAGTGAGACTCCATCTCAAAAAAAAAAAAAAAAAAGGAAAGAAAAAGAAAAAAAAAGGCAGAGTGGCTGCTATGGACACAC
TTGGCTTGGGAGGCACCATGGACCGGAGATATTTTCTTTGCGGTACCTACTCTCCACATCAAGCCACAGTGAGCAGAACCTTCAGAC
AGCACAGTGTGCTAAGGATCCTATCTCCTTCTTGCTGCAGGGGTTTCTTGCCCCAAGTAGCTCACCCAAGTACACTTCCCCATGAAT
TCAATGAATAAGACCAAAATTAAACCCACAACTTCCCTAAACTATTTCCTTCTCCTAACTTCCCTGGTGTGCTCCTGACATCGTGTT
CTTCCAGACGCCAGGCCTAAAGACTTAGCCCCATTTCTGACTCCTCTGGACCCTGGACCCCACGAGGGTCTGAGCACTTTCTGTGTC
TGATTTCACCTTGCCGTGGCTGTTCACGCATGCCCGCCTCTCCAGGGCCTCGGCCCCGCCACTCTTGAAGCCGCCATCTTCTGTCTC
GCACTATCACAGCAGCCTGCTACCTGCTCTCCTCACTTCGGCGTCCCTTACCCCAGTCATCCTGCACTCTGCTTCCCCATGGATCTC
CTTAGACTCTCAGGCTTAGCTCTGAACAAATTACTTCCTGCTCAAAAACCTTTAAGTAGTTTCCCCACCTACTATCTCCTGAATGAC
ACTGAAGAAAACTTTTCTTTAATTACAAGCATATGCCAAAGTTTGATCATAATCTTGCTAGAAGAAAATTTTCTTCAGCAGGCTAAT
TCAAAGATTACTCTTAGTAGTGGTATTTCGTAGGAAAAACAATGGTAGTCATTGTTATGCCAGATTATAGAAGGTTTAGCTAAAGAC
TGCTAATGGACAGCTGGTTGGGCTCAACAGTGGATTTCCAGAACAAGTCTTCAAGTCTTTACAATGGATCAGATAATCAATAGGGCA
GTTATCAGGAATGCTAATTCTCCCCAGAAGAGGATTATAAATTACATCACGCACAAGGTTCTGTCCCAGACAGATTGGGTGCAGGTT
TTCTTTAGCAATAGTCACAGAAACAATGTGCGTTTAAGCTGGAAGGACCCTTAGGGAAGAGCCACTCCAACCCACATGAGAAGACGA
AGGCCCAGAAAGGGGCGAGGGTGGGCCCCCGGCCTATGCAAGATCCACTTTGCTCTCAGACCATCAGGCAGGCAGCTTTAGGGCGGT
CAGTTCCTTGGAACTCAGCATGGGGGTTAGGGTCCCAGACCTGCTGAAAAGAAAAGCCAGCTTAACTCCACCCTAACTGAGTATCACA
TAAATAGCATTATAGAATTATCCACCACCATTACCCATTACCATATTGCTACAGGGAGAACCTCAAGCTCCAACTCTGAACAACGGAAATACA
CCTCCTCTCTCTCTACCCAATTTCCTGCCAGCCAGAGAGGTGGAGCTCCCAGTTCTAAGTGGTCAGTGAGGGCCTCTGGGTGAGGCA
GGCAGAGAAGCCTGTAAAGGGAGTGTGGAATAGAACAGGGGGAGGAAAAAGAGGAGGTGCAAGCAGGGTGATGAAAGAGAGGGCAGC
CGGGGGAAGCCAGAGCAAGTCTGCAGGGTGGTGATGAGGGGAGAGTGGGCTTGGCAAATGGGAAAGAGGAACCAGCCCCCTTCTCTAG
TCCTGTGTGTAATGGACACCTTCAGTCAAGGGCTGGCTTGACGGTTCTCCGATAGTGATCTGATCATCACTCAGCCTAATTTCAACC
TGTGTAACTCCTTGAAGTTACAGTCATGCACTGCATAACCAAGTTTCAGTCAATGATGGTGCACATATACCAAGGTGGTCTCAGAAGG
CTATAACAGAGCTGAAAAATTCCTCTCCCCTAGTGATTTGTGTTACAGTGGCCTACAGTACAGGAATGTGCCATACAGGCTTGTAGC
CTAGGAGCGATAGGCTATACCATACAGCCTAGGTGTGTAGTAGGCTGTCCCATCTAGGTTTGTTTAAGTACACTCTGTGATGTTCAC
ACACTGAAGAAATTGTCCAAGATGTGTGTATTTAAATCACACCCACCGGCCAGGCCTCCCTTTCCCCGTCTCCAACCTCCAAGTAGA
ATACTTCCCTGGCAAGGGTTCCTGGCAGCATAATGGGCATGCTAGTGATTTCTTCTCTGGGGGGCTCTGCTAATGGGACCCCTGGAA
TTTCCCAGGGCTCCTGGGGCTGCTGAGGCTTAGGGTGCCCAAGAAAGGTGCTCCTTTCATCCCCCTTGTACTCCTGCCCCAAGCAT
TCTTGCCGCAACCGACAGCCCCTCTGTAATAGCAAGTAGCAGGTTACCTGGGCAGAGAGGTGGCCAGACGAAGTCATTGACATGGGA
TGCTGCGGCCAAGAGCTCAAGGAAGAGGTGTGTGTTCTGTTGTATGTTTTCTTGCTGGCTGGGGAAGTTAGAGGGAAGGAAGCTGAC
ATTTATTGGAAGGAGAGAATATTAACAAAGGTACCATGTATTGAGGCATATGGCCAGGATCTGTGCTAATGTTTTAAGTATATTCTC
ATTTAATCAACACAGTAATCCCATTTCACAGATGCAGGTAGTACTTGCATCACTCTCTAGCCCTGGACCCTGCTTTGTTGTTGTGGT
TTTTTAAGCGCTTGTACCTGATGCTATCATACAAACTGCATACCGTCTTCCGCAGGATTGTAAGTTCCGTAAGCGCAGCAATGT
TATTGATTTGTTCACTGTGGTACCGTCACTTGCTACAACAATGTCTGCCCATCCATAGGTGCTCATTAATTGTTCCTCAAATGTATG
AACAAAGAGAGTCTCAGAGAGTTCAGCAGTGGGTCCAAGGTGGAGCTGATGATCACGTCTATTTATCAAGTTTTTTCTGAGGGTTAAT
GAACCAGTGCAGACATATGTGCTTAGCACAAAGCCTGGCACGTAGTAAGCACTCAGTATATGTTAGCTGTCACCATAATTTGCAAGG
TCTTCATTGGACCACTTTCTTGCCCAGGCCCATTAATCTTAGAGCCTTTCCTAAAGTAGATCATAGAGCCCATTGTCTGGCCCCAAT
CAGAGTGAAAGCAATTTTTTGGTAGAGAGTTTTTGGGCTTGCAGAAAACAATCAGCCAATTTGTTTATGGGTTTTGGAGGCAGGACA
GCTGGACTTCCATTCACAAAGGGTCCTCTCCTAACCATCCCCAAACCCATCCGATTGTGGCCAACCTTGAGCCACTCTTTCTGGCTT
GCTGCTTTCATCTTAAGCCCTGCCCTCTCGCACTGCTGCACCCTGAGAGCATCTTTTGGCCCTGGTGCCTCTGCCCACCAGGAGAGG
TCAGGTGGTACCCAAGCCTCCCTCAAGGAAGGGTGCATGGAGCCCTTTGGAGGCTCGGCAAATCTGAGGAAGGGCACATGACCTCAT
```

120

```
GTGCTCCCGACCCCAACAATGCTTAGATCAATCAGCCAGGTGAACTGGAGGGGAGACCCTGGCCACAATAGCATTTACTGTCCACCTG
GGAAAGCACAGTGCAGTTAAAAGCCTATTTCTTTTTCTCTCGCAGATATTGATCTACTTAGCTTTGTGCCAGGCTGTTTGCTGGTAA
TTAATTTCAGAGTCAGTGCAAGGGCTGGCAGTTAACATCACTTTCCCGAATGCACGGAGGCCTCCCTCCAAACTCATAAAGATGATG
TGACTGCCTCTCTCAGCTACCCGGGCCGTGCTGTGACTTCAGTATCTTACATTTGGGCTGACGTTAAGTGCAGGACCTGGACTCCGT
GTTCTGTCACATTGCTAAGTGAGGGCCATGATGATTAAAAAAAAAAAAAAATCCAGTGCTCTTTCCAGTTCCTTGATCCTGGAAAAC
AGGAAATGATTTTGTTTGTCTTTCCTTCAGTCCACATGAAAACCTTCTGCTCTTCCTGGACGTGGCTCTTGTTTTGACTTTGCCATT
AGCTAGCTGTGTGATTCTGGCTCTCACATCCCATCTCTGAAGTGGTTTCCTCGTATGTTCAATGAGGGGTTTGAGCTAGATAATATT
AACTAATATCTGTATGGTACTTTACATTTTATGTAGCATTCACACCCACACTGACTCAGTTTGCCATTAATAATTGTCTAGTCTTGG
TCTGTGTCTTGGTTTCTTCATCTGTAAAATGTGGATAATGTTAGCACCTCCCTGATGGGGTTGTGGTGAGGATTAAAGGAGCTTAGA
CATATGAAGTACTCAGACCTGTGGCTGCACCTGGTAGATGTTAAGTGTTGCCCCCAGCACCATCATCACTGCCTCATAGCAGCCCTC
TTTTATTTTTCCCATCTTACAGATAATGAAGCATTTGCTCCTAAACTGCTTAATAATTGACAGAGCAGGGATTCACCTGCAGACTTTC
CAGTTTCCTTGAGGCTTTCCTAAGAGGCTAAGTGCGTAGGGAAACTGAAGAAATGACACATCCAATTGCCTCCTCTCCCTGCAAGAG
GAGGGCAGTAGTATTAAATGTGTGCTCTGCAGAGAGCAAGGCTTCCCTTCCGTGCCGCCTTTTTTGCCAGGAGGCTGCTGTTCACTC
TAGTTCCCTGAGCAGAAGCCCAGAGACCCACAGTGTCCTCTCCTGGACATCCACCGTTTCCAACTTTCCACCACAACCTGCTTCCTTT
CAGGCAAGAAAGTACTAGAGGTGGGCAGTTAAGTCATTTAAAACTATTTTCACAGCAGATCTTTACTGAATTTTTAAAAAGCAATAT
TTATGTCCCTTAGACATTTCTGAGAAGACTTAGCTGTTCTTTTAAAAACATCACTTCAGCGCCAATGAAAAATATAATCCAGCCTTA
GTAATAAATTTTTAAAACATACACACACAAAAGTTATCAGCCTGCAAAACAATGTTTCCTAGGCTTCCCACACATCTCAGTATTTTA
ACAACATCCCATCTGTTGATGTCAGTCTGAAGAAGCAGGTCTATATTCTCTAATTTTATTAAAAATCCTTTCTGGCTAGTAACCTGAT
TTCTATTAGGCTGGCCTACTTATTTCCAGGCCCCAGGCCTGTGGCACATGCCAGTCTGCATGGCTGACAGAAATGGGGATGGTTAA
TCAAAAATCTGCTTAGCATGTGCTTAATAAGGAATGTTTTCTGTTGCTCTTGTTACTAATTGCTGAAGATGTCTGTGTTTGGATTAA
AACTTTGTCCCCTGCCATCTGGATGCCATTGATATGTCTCAGCTCAGTCATGAGCCTGGTGATATCAGTGGTTTCCATGTCAATGGA
TGACAGCGTCATATGTTGAAACGTGTGATTGCACAGTGAAGGAGCCAGCCCTGGGGAAGAGGAAGCTCTGGTTTCACACCTA
AATATCTTCACTGATTTTCAGACATTGCTCTGAAATTATTTCTTTTTTGTGCCAATTACATTTACATGTACAATTGCAGTGCAAATG
TTTTTGGCCATTTGGGAAAGGAGACAATGGGCTCGTTGGTACCTATAGTTCCAATCAGTGGGAAAGACATTTTCTCAAAGAAGTCTT
TTAAAATCTCCAGCTGATAGGAAATCTGTTTACTCTCTGAACCCAAGCAATGTACCGTATACACGAAGTGGCAATCTCAGGCAAAGC
TCGGAAGTCTGCATGTAAGGGCAGCAATGGTATATCGCTAGCTATTTTCCAAGGGGACCCCGGGACTCAAGGTCTCGCTCATGCAGC
CTGTCTAAATATAGCATCAGCAACAGCAGTCAAGAGATAGTATTGAAATAATCAAGAAGGAAGAAAGAAAGAAGGAAGGAAGAAGG
AGAAAGAAAGAGAGAAAGAAAGAAAAAGAAAGAAAGAAAGGAAGAAAGAAAGAGAAAGAAAGAAAGAAAGACCCTCCAACTTCAAGC
TCTGGTCTGCAGTGTCCAACACAAGATTAAGAACAGATGAATTTTAATCACCTTTTTTTTTTCCTTTTTTTTTTTTTTTTTTTGAGATGG
AGTTTCACTCTTGTTGCCAAGGCTGGAGTATGGCGTGATCTCAGCTCACAGCAACCTCTGCCTCCCAGGTTCAAGCGATTCTCTTGC
CTCAGTCTCCAAAGTAGCTGGTACTACAGGCATGTGCCACCATACCTGGCTAAGTTTGTATTTTTTGTAGAGACGGGTTTCACCATG
TTGTCCAGGCTGGTCTCGAACTCCTGACCTCAGGTGATCCACCCGCCTCGGCCTCCCAAAGTTCTGAGATTACAGGCGGGAGCCACC
GTGCCCGGCCTTAATCATCTTTTCTAGGAGTTCCGTGATTTTGGTTAAGAAAATAGATCATTGCAAAATCGTTTCCTTATGAGGTAG
ACTACAGTAGCAACCACAAAAATGAACCTAAGAAATGAACCTGATAGCAGCCTACTTTATTTATTCTTCCTTAGATCAAGAATGCT
GCAGCCAATGTCCTTCGGGAAACATGGTTAATCTATAAACACACAAAGCTGCTAAAGAAGATTGACCATGCCAAAGTGAGGGAAACAC
CAGAGGAAGTTCCTCCAAGCTATCCACCAGTGAGTATGCCAGAGCGGTTCAGCCATCAGATGACCAGACTGGGAGTGGCGGGACTCC
TACAACTCTGTCAGTAGCACAGAAGAAATCCATTCTGTTCCACTCAACAAGTGGAGTGGTGAACTGGGTCTGATCACTCACTGACTG
CACCAGCCACATGCCAGGAATTGGGCAGGCTGCTATGGAAGGTAAAAAACTAGAGTAAGAACCTCTAAGAGCTTTCACATTCTACACA
CACACACACACACACACACACACACACACACACACCATGCATTGCAACTCAAATCAAAACAAACCGTGGCTGATGCTTTCTGTGA
TGGAGGAGTTTTTAAAAGTATAGTGGAAAAGCAAAGGATGAAAACATTCATTCTGATGGAGAGGTTTGGGGAATCCCTGAAAAGTTG
AGAATAAGGCACTCAACATGGAAGGTGCAGCCCAGGCATAGAGGCAGGGAAATGATCTGGGAATAGCAAGAAACCAGTGACTGAAGG
TGAGCATGCGTGGAGACAAGAGTTTGCTTTCTGGGGGGATGTGACAGCCCCTGGAAAGTAAAAGCCTCAGTTGTTCCCCAGGTTCA
CGGTCCCCATGCTGGGTTCCAAAATCAGAGGCAGTGGTAGGGCTGGAGGATCTGCTGGTGGGGCTAAACCTTTGCATGGACTTGAAG
GGCTTGCCTGTTACCATTCTTCCCCACAGGTTGAGGAGCGTCAAGATGGAACAGAGGAAGCTGAGTGACCAAGCCAACACTCTGGTG
GACCTTTCCAAGGTGAGTGGCAGTGTCTGGAGACATCCCCTAGAACACGCAGACACTGAGTCTTCTCAGGCTCACCTGTGAGTCCTG
GCTGGGTTCGCACTGGACAGCGCGGGACCAGGCAAGCAAAGAAAAATTTCTGCTTGGTTCAAAAATACCTAGGCTCAAGTTTGTTTC
ATTTGCAATCTACCGGTATGTCTTGTGTGGCTTTTGAAACTTGGTGTTTGTTTCTTAAGTGAAATCGCTCAGGTGTGTCTCTGCGTGA
AATTAATGAGCTGACTAAACCTTTTCCGTGTCATTTTATGAGCTGCACATTGAGGGACTCCTGAGGTTGGGCTTGAGTTTGCACGGC
TGTGAAGCATGCTAGCAAGGACCAGACGGTAACGATGGCCATGGTAACTGCCCAGGTCACACCGGTTTGCTGTAATACTTGGAAGTT
CATTTTTTGTACATCCTCCAGTCCTTAACCAACTAGTTTTGCTGGCCTCAAATTGGCGGCAGTGGTGTGTGTGGGGGGGTGCATTTCT
TTACTTCTCAAGAGGCAGATAGCCTACAAGGAACATGAAGATACCCAAAAAGAGAGCCCTCTTTTGAGAGCCCTAGGGTGTAAGGCT
CGCCATCACTCTGCCCACTCTGTTTCTGGCCCTGAGCTGCCATAAATACTGTAAGGAAACTCCTCTTGCAAATCACCCAGGTGAGGGA
GCCGAAGGCCACACTGCTGGCAGAGCTGAGAGTCGGTGCCACTGAAGAAAGGGCCTCCCCCTCCTCAGCCCCCTCTCCTCATTGCCT
CTTGATAGGAATTGTATATTAAGTGTACAGCCCAGGGCCAGGCTGGCAGAACATTCCAGCGATGTCAATTATCATAATGGAACACA
TTCCCTGGGTAAGCAAGAGAAACTCCCTCAGGCACATTAAGCACCCTTTGAAAAGAGTGCAATCAGAGAAATCACTAGTTGTCGGGC
CACGTGCAGGTGCCTTGTTTAATAAAGGGATTGAACCAAAGAAAGGGCCTTCTAAACCTGTGAGGACTTCAATCGGGGAGTAAGTGG
CTTGGCACATTTGGGGAGGAAGCCTCCAGGCCCGCGTGCCTTTGGAGGGAGGCGTTCACGTGGAGTGCAGGGTGGTTCCTAGGAGCCT
GGGGTGGGAGTGGAACGGCATGCAGGAGGAGAACTCCCTCAGGTTTTTGGGGTGTGTTGGTTTAATATCAGAAACCCGCTGGGAC
TAGGTTTAAAAATGGAGAAATCTATTCTAAGGATATAAGGGAGTCTCATTCCAGGCAAAACCTCAAAGGAGACATGTGGCCAGACTG
CAGGAAGGAACTGACTCGAACTAGACCTGGGAAGCCCCCACAACCAGGAGGTCTCTCACCATTTCTTCTCTGTCCTTCTGTTTAAGT
GACTGCTTCAGTTCTCTCTCCCTCTCCCTGTCTCTCTCTTCCTCTCCCTTTCCCTGTCTCTCTCTTCCTCTCCGTCTCCCTGTCTCT
CTTCCTCTCCGTCTCCCTGTCTCCCTGTCTTCCTCTCCCTTTCCCTGTCTCTCTTCCTCTCCTTTCCCTGTCTCTCTCTTCCTCTC
CCTCTCTCTTCCTCTCTTTCTCTCCCTCTCTGACCTCCTGCCCCGCGACAGACTGTAACAGGCTCCAATTCAGAGAGTACATATAAT
AGTTTTTAGCCACAGGTAAAGACTAATTGGCCCCCTACTAGTCTTAATTCTAAATTCTCAGAATAGAAGAAATGATTGGCCAATTTGG
GCCACGTATCCACCCTGGGACCAATCAGCATTGGCCAGGAAGAGGGTCATATGGAGCAAACAGGGCTAGGGCCCACCTGTGAGCTT
CAGGAATAGCCCAACAAACTGCCCCAGGATGTTTCTTCCATGAGTAGCAGTATTTCCTGTTTCTCCTCTCACCTTGCAGGACATGCT
GTGACTGATGTTCCCTGGGGACATGAGGCAAACCAAACTTTCTTTTTTTTTTTTTTTAGTTTTATTATTATTACACTTTAAGTTTTAG
GGTACATGTGCACAATGTGCAGGTTTGTTACATATGTATACATGTGCCATGTTGGTGTGCTGCACCCATTAACTCGTCATTTAACAT
TAGGTATATCTCCTAATGCTATCCCTCCCCCCTCCCCCACCCCACAACAGTCCCTGGAGTGTGATGTTCCCCTTCCTGTGTCCATG
TGTTCTCATTGTTCAATTCCCACCTATGAGTGAGAACATGCGGTGTTTGGTTTTTTGTCCTTGCGATAGTTTGCTGAGAATGATGGT
TTCCAGTTTCATCCATGTCCCTACAAAGGACATGAACTCATCATTTTTTATGGCTGCATAGTATTCCATGGTGTATATGTGCCACAT
TTTCTTAATCCAGTCTATCATTGTCAGACATTTGGGTTGGTTCCAAGTCTTTTGCTATTGTGAATAGTGCCACAATAAACATACGTGT
GCATGTGTCTTTATCAGCAGCATGATTTATAATCCTCTGGGTATATACCCAGTAGGGATGGCTGGGTCAAATGGTATTTCTAGTTC
TAGATCCCTGAGGAATCTCCACAACTGACTTCCACAATGGTTGAACTAGTTTACAGTCCCACCAACAGTGTAAAAGTGTTCCTATTTC
TCCACATCCTCTCCAGCACCTGTTGTTTCCTGACTTTTTTAATGATTGCCATTCTAACTGGTATGAGATGGTATCTCATTGTGGTTTT
GATTTGCATTTCTCTGATGGCCAGTGATCATGAGCATTTTTTCATGTGTTTTTTGGCTGCATAAATGTCTTCTTTTTGAGAAGTGTCT
```

```
GTTCATATCCTTCGCCCACTTTTGGGCAAACCAAACTTTCAAGCCTGCATTTCTGCAGTGCCACCCACGGCCTCTTGGAGAGATGGG
GCCCACGGAGCAGACCGGGTGAAGCGAACCCCTCAGTGAACTGGAAAGACACTCCTGTGAATTGGGTGGGGGAACTAAGCCGTAAAT
AACCCAGGAGCAAGTTACCTGGAAAGAAAGAGTGTGCAGGAAAGGGTTAAGGGAAAAATCTGCAGAACCCCCGTGGGGAGATCTGGG
GAGGAGATGAACTCTGACATTCGCCCAGTCTTTGTCTTTTCCTCCCAGGAGACTCACCCAAGGTCCTGGCAGCAGCTTAGCACACTT
CACACTGGTCCATATTCCCCCTGGCATCCTGTCACCAGGCAACGGAACAGGGGGAGACAATAACGCTGTGTTCTCTCTCTTTTCTGT
CTTTGTGCTGTAGCCTCCCAAGTCAGCGCCTTCCCACTCCTGCCCTTTGGTCCTCTCTAAATTAAGAATATGGCATGTGGCCTCCAC
ATATTACGTCTGTATTGAATTGCTGTCCTATAGGCTGGCAGAGCTGGGAGGGCTTGGAGTCTTTCCCCGATTGCTTCAATTACTTCA
ATTGAAATATAAAAATAATTCCTACCATCGGAGGCTCAGGTGATCGAATAAGATGATATCTGTGAAAATGCTTTGTAAACTATAAAG
GTTTTGCAATTGGTAGTTCTGATTATCAATATAGATGAGGAAACCAAGATCCAAGGGAACAAAGTGACCATCCCACGGTCAGGCAGT
TGGTGAGCACCAGCGTGGGGGCCACAACCCAGGGGCCTGGACTCTGAGTCCAGAGCTCATCAGGGTGCCCCTGCGGTCAAATGAAAT
GGGTGATTTTGCACATTCCGGGAAGAATGTTTGCCTCGAGGAGGTTCTGTCATGTTGATTAATAAAGGGAGTACTTTCACTTCTAAA
GTCACCCTGTCCTCCAAGGGCATCCACGACACGGTGTCCTGCTCTGGCAACAGCAGAGCTCATGGGAGCGGGGCCATCTCCATGCCA
CCTTGTGACCTTTCCTTATGCTGCCCCAGTGAATTTCCTCATGGGGCCTGTAGTCTGCTGGGGAGGCCTCACTGCATTTTGTGCCCC
TGCTCCCCTTCATTCTTCTCTGGCTTCTCACATCTTCTTTTACTCTTCTTTTTTCCTCTCACCTTACTTGCTGCTGCTTTGGTCCCT
GGGGCATAAATCATGTCCATATCTTCCTAGAGTAACCTGAAATTTCACGTGAAATAGGATCGGGGTGTCACTGGCAGATCATCAGAA
ACCCAAAGAGCCACAGTACCTCAGGGATGACCAAGCAACCACCGGCCTGGCCCTCTGCATTGCCGCCCCAGGAGCCTCACTCCCTGA
CACGTGGGAGGCTGAAGATGCTTGTGAATCTTGTTCCCAAGTCTGATGTACACAGCCAAGATGGCATTTGCTAATTTGCTGGGGAGAA
AAATATCTGTTGCATAGACTGGGACTCTGCAGACCAACAAGGGGGCCACAGGATGTTGGGGAAGGAGCACTGGTAATCGGCCGGCAC
CCCTGGAACACGGGGAGTCAGTGCTGAGTGCAGGAGGGGTACTGGGGAGCTCTCAGGGGAGCACCCTCAGGACCCCTGCCAAGGAGT
GAGGGAAGCAGGATTGGTCAGTGGGAGGGTTTGAACTGTGATACAGTCAACAAAGACCTCAGCCCTTCAGAGTGGCCCTTCAGGGAT
GTCCCACCCTGAGGCATTGTGGATCAGGCAGGCGAAGGGCCACACCTCAGCAAGGCAGCCTCCTTTGGCTAAGCACTGTGCCTGAGA
GTTAATTCACTTTACATCCTGTCTGCTGGGTTAAAATAGAGGTTGGGAGATGTGCAATAACTCCTAACTGTGTACAGTTATTATTAA
CTCCAATTTTATAAGGCAACCGAGATTGAGAGAGGCCAGGCGCCTGGCAGAGGGCACACTGTTAGTGAGCTGCAGATGTGTACTTGA
GCCCTGGCCTACTAATTATACACCCACTGCACTGTCCATTCCCCACCAGCGTCTTCTCTGGAATCATTAGATGCCAGCAGCCATGTG
AGCTGAGAGGTTGTGGCCCTGTGAGAACATGAGACAGTTTAGGACTCACTTTATTTAAGAAAAAAAAAGGGCCATTTTTTCCCC
TTGGTGCATATTTTCTAATAAAATGCTGTCATTAATACTCATTACTTTTGTAATAAGAAAACAAAAATGCACACTTCAGCATCCC
CATCCCCAGGAAAGAAGCCTGTATCCTTCCTTGACCAGATCAGCTGGTGTGGAAGTGGCTCCTGTCATACTGTGGAGGGCGTTTCAT
TTCTCCCATTGCAGATTCCTCTGTGATGACCTGTCATCTCCCTGTCTCTCGCCTCTCTCTCCACACAGATGCAGAATGTCATGTA
TGACTTAATCACAGAACTCAATGACCGGAGCGAAGACCTGGAGAAGCAGATTGGCAGCCTGGAGTCGAAGCTGGAGCATCTCACCGC
CAGCTTCAACTCCCTGCCGCTGCTCATCGCCGACAGCTCCGCCGACGCAGCAGCAGCTCCTGTCTGCTGCCATCATCGAGGCCCGGG
TGTCAGCGTGGCAGTGGGCACCACCCACACCCCAATCTCCGATAGCCCCATTGGGGTCAGCTCCACCTCCTTCCCGACCCCGTACAC
AAGTTCAAGCAGTTGCTAAATAAATCTCCCCACTCCAGAAGCATTACCCATAGGTCTTAAGATGCAAATCAACTCTCTCCTGGTCGC
TTTGCCATCAAGAAACATTCAGACCAGGGAACGGAAAGAAGAGAGACCGAGCTAATTAACTAACTCATGTTCATTCAGCGTGCTTGG
TCCGACATGCCTTGAAACCAGAAATCTAATCTCTGTTTAGGTGCCTCTACTTGGGAGCGGGAAGAGGAGATGACAGGAAGCGACGCC
TCTGCCAGGGCCCTTGCTGCAGAGTTGGTGGAGAACAGAAATCCACGCTCAATCTCAGGTCTTCACGCGGGGGGTGGGGGTCAGATG
CACTGAAGTAGCCAACAGCGAAGCCAGTCCAGAAGAGGGGTCCGCTGGGAGGGAGGGTTGTGTCAGGCTTGGGGGATGGGCTCTTCG
CCATGGGGGTCTTTGAACACACCTCTCTCCTTTCCTTTTGTCTACGGAAGCCTCTGGGTGACAAAAGTAAAAGAGAGCTGCCCACAA
CTTGCCAAAACAGATATACTCGAATCAGACTGAAAAAAAAAAAAAAAA
```

HUMAN mRNA SEQUENCE : hR27-009.1 (Seq ID No: 27)

```
CTTACAATGTAACAGTGGCAGGAGGAGGCGAGCGAAGCTATTGAGCCAGCGAGGAGTGAAGCTGAGCCTGGCCTCACACGCTCCTAG
AGGACCACCTCCTGAGAGAGTTCTTTCACCCCCTCTTCTTTCTCCAAGCTCCCCTCCTGCTCTCCCTCCCTGCCCAATACAATGCAT
TCTTGAGTGGCAGCGTCTGGACTCCAGGCAGCCCCAGAGAACCGAAGCAAGCCAAAGAGAGGACTGGAGCCAAGATACTGGTGGGGG
AGATTGGATGCCTGGCTTTCTTTGAGGACATCTTTGGAGCGAGGGTGGCTTTGGGGTGGGGGCTTGTGCTGCAGGGAATACAGCCAG
GCCCCAAGATGGACACTTCTGGGCACTTCCATGACTCGGGGGTGGGGGACTTGGATGAAGACCCCAAGTGCCCCTGTCCATCCTCTG
GGGATGAGCAGCAGCAGCAGCAGCAGCAACAGCAGCAGCACCACCGCCAGCGCCACCAGCAGCCCCCAGCAGCCCCTG
GACCCTCGCTGCAGCCTCAGCCTCCGCAGCTTCAGCAGCAGCAGCAGCAGCAGCAGCAGCAGCAGCAGCAGCAGCAGCAGC
CACCGCATCCCCTGTCTCAGCTCGCCCAACTCCAGAGCCAGCCCGTCCACCCTGGCCTGCTGCACTCCTCTCCCACCGCTTTCAGGG
CCCCCCCTTCGTCCAACTCCACCGCCATCCTCCACCCTTCCTCCAGGCAAGGCAGCCAGCTCAATCTCAATGACCACTTGCTTGGCC
ACTCTCCAAGTTCCACAGCTACAAGTGGGCCTGGCGGAGCGAGCCGGCCACCGACAGGCCAGCCCCCTGGTGCACCGGCGGGACAGCA
ACCCCTTCACGGAGATCGCCATGAGCTCCTGCAAGTATAGCGGTGGGGTCATGAAGCCCCTCAGCCGCCTCAGCGCCTCCCGGAGGA
ACCTCATCGAGGCCGAGACTGAGGGCCAACCCCTCCAGCTTTTCAGCCCTAGCAACCCCCCGGAGATCGTCATCTCCTCCCGGGAGG
ACAACCATGCCCACCAGACCCTGCTCCATCACCCTAATGCCACCCACAACCACCAGCATGCCGGCACCACCGCCAGCAGCACCACCT
TCCCCAAAGCCAACAAGCGGAAAAACCAAAACATTGGCTATAAGCTGGGACACAGGAGGGCCCTGTTTGAAAAGAGAAAGCGACTGA
GTGACTATGCTCTGATTTTTGGGATGTTTGGAATTGTTGTTATGGTGATAGAACCCGAGACCGAGCTCTCTTGGGGTTTGTACTCAAAGGTAG
GGGCTGTGGTTTCTCTTTATACCTTGAACAAAAGGAATATGTAGGTAGCAAGAGAGGGATTGAGAGAGGGGGATTGTGAGAGAGAGA
GATTGAGAGAGAGAGAGAGAGAGAGAGAGAGAGGAGACTGGGAGAGAGAGGTGGTGGTGGTGGTGAGAGGCGCTTGCTCAGTTAT
ATTCAACACTCTAACTTCCCGTGGTTTTCCTTCTTGATCCCGGGAGAATTCATTCCTAGCTTCCTCTGGGGGGTAGGGGACATCCCC
ACACACTGTGTCTAATTTGCAGACTCTCTGTTAGGGAGGATTCAATAATCCCTTCTGAGAAATCGCTTTTCCTCCTGGCTCACTCGT
ACTAAAGCATAACCCAGCAGCTTAACGCACCAATTAATTACACTCCGCCTTCATGTGTTTGCCAGTTCCTGCGACTTCCCCCTTCTTT
CCTCCCCTCCCTGCTCCACTCCATTTTCCTGAGGATAAAAAAGAAATAAATGTCTGGGTGGGGGGTGGGGATTCCGTACCATTGCCC
AAGTCCCTTCTCTCCCTGTCTTCTCCATCTCTTCCCAAGTTTCTGTTTCCCTCCTCTTCTCAGAGAGTCTCGGGCAAAATAGAAAAC
CATCCATGACCTGGGTCTCCTGAGGGCATGGGGTAAAAATATAGGTGGAGGAAATCAGTGGTCCTTTCCCTCAGGAGAGAGGACTCA
GAATAAACCTGCTGCTGCTTCGT
```

HUMAN PROTEIN SEQUENCE : hP27-009.1 (Seq ID No: 28)

```
MDTSGHFHDSGVGDLDEDPKCPCPSSGDEQQQQQQQQQQQQQQPPPPAPPAAPQQPLGPSLQPQPPQLQQQQQQQQQQQQQQQQQPPH
PLSQLAQLQSQPVHPGLLHSSPTAFRAPPSSNSTAILHPSSRQGSQLNLNDHLLGHSPSSTATSGPGGGSRHRQASPLVHRRDSNPF
TEIAMSSCKYSGGVMKPLSRLSASRRNLIEAETEGQPLQLFSPSNPPEIVISSREDNHAHQTLLHHPNATHNHQHAGTTASSTTFPK
ANKRKNQNIGYKLGHRRALFEKRKRLSDYALIFGMFGIVVMVIETELSWGLYSKVGAVVSLYTLNKRNM*
```

HUMAN mRNA SEQUENCE : hR27-009.2 (Seq ID No: 29)

```
GCCTCACACGCTCCTAGAGGACCACCTCCTGAGAGAGTTCTTTCACCCCCTCTTCTTTCTCCAAGCTCCCCTCCTGCTCTCCCTCCC
TGCCCAATACAATGCATTCTTGAGTGGCAGCGTCTGGACTCCAGGCAGCCCCAGAGAACCGAAGCAAGCCAAAGAGAGGACTGGAGC
CAAGATACTGGTGGGGGAGATTGGATGCCTGGCTTTCTTTGAGGACATCTTTGGAGCGAGGGTGGCTTTGGGGTGGGGGCTTGTGCT
GCAGGGAATACAGCCAGGCCCCAAGATGGACACTTCTGGGCACTTCCATGACTCGGGGGTGGGGGACTTGGATGAAGACCCCAAGTG
```

```
CCCCTGTCCATCCTCTGGGGATGAGCAGCAGCAGCAGCAGCAGCAGCAACAGCAGCAGCAGCCACCACCGCCAGCGCCACCAGCAGC
CCCCCAGCAGCCCCTGGGACCCTCGCTGCAGCCTCAGCCTCCGCAGCTTCAGCAGCAGCAGCAGCAGCAGCAGCAGCAGCAGCA
GCAGCAGCAGCAGCAGCCACCGCATCCCCTGTCTCAGCTCGCCCAACTCCAGAGCCAGCCCGTCCACCCTGGCCTGCTGCACTCCTC
TCCCACCGCTTTCAGGGCCCCCCCTTCGTCCAACTCCACCGCCATCCTCCACCCTTCCTCCAGGCAAGGCAGCCAGCTCAATCTCAA
TGACCACTTGCTTGGCCACTCTCCAAGTTCCACAGCTACAAGTGGGCCTGGCGGAGGCAGCCGGCACCGACAGGCCAGCCCCTGGT
GCACCGGCGGGACAGCAACCCCTTCACGGAGATCGCCATGAGCTCCTGCAAGTATAGCGGTGGGGTCATGAAGCCCCTCAGCCGCCT
CAGCGCCTCCCGGAGGAACCTCATCGAGGCCGAGACTGAGGGCCAACCCCTCCAGCTTTTCAGCCCTAGCAACCCCCCGGAGATCGT
CATCTCCTCCCGGGAGGACAACCATGCCCACCAGACCCTGCTCCATCACCCTAATGCCACCCACAACCACCAGCATGCCGGCACCAC
CGCCAGCAGCACCACCTTCCCCAAAGCCAACAAGCGGAAAAAACCAAACATTGGCTATAAGCTGGGACACAGGAGGGCCCTGTTTGA
AAAGAGAAAGCGACTGAGTGACTATGCTCTGATTTTTGGGATGTTTGGAATTGTTGTTATGGTGATAGAGACCGAGCTCTCTTGGGG
TTTGTACTCAAAGGACTCCATGTTTTCGTTGGCCCTGAAATGCCTTATCAGTCTGTCCACCATCATCCTTTTGGGCTTGATCATCGC
CTACCACACACGTGAAGTCCAGCTCTTCGTGATCGACAACGGCGCGGATGACTGGCGGATAGCCATGACCTACGAGCGCATCCTCTA
CATCAGCCTGGAGATGCTGGTGTGCGCCATCCACCCCATTCCTGGCGAGTACAAGTTCTTCTGGACGGCACGCCTGGCCTTCTCCTA
CACACCCTCCCGGGCGGAGGCCGATGTGGACATCATCCTGTCTATCCCCATGTTCCTGCGCCTGTACCTGATCGCCCGAGTCATGCT
GCTGCACAGCAAGCTCTTCACCGATGCCTCGTCCCGCAGCATCGGGGCCCTCAACAAGATCAACTTCAACACCCGCTTTGTCATGAA
GACGCTCATGACCATCTGCCCTGGCACTGTGCTGCTCGTGTTCAGCATCTCTCTGTGGATCATTGCTGCCTGGACCGTCCGTGTCTG
TGAAAGGTACCATGACCAGCAGGACGTAACTAGTAACTTTCTGGGTGCCATGTGGCTCATCTCCATCACATTCCTTTCCATTGGTTA
TGGGGACATGGTGCCCCACACATACTGTGGGAAAGGTGTCTGTCTCCTCACTGGCATCATGGGTGCAGGCTGCACTGCCCTTGTGGT
GGCCGTGGTGGCCCGAAAGCTGGAACTCACCAAAGCGGAGAAGCACGTTCATAACTTCATGATGGACACTCAGCTCACCAAGCGGAT
CAAGAATGCTGCAGCCAATGTCCTTCGGGAAACATGGTTAATCTATAAACACACAAAGCTGCTAAAGAAGATTGACCATGCCAAAGT
GAGGAAACACCAGAGGAAGTTCCTCCAAGCTATCCACCAGTTGAGGAGCGTCAAGATGGAACAGAGGAAGCTGAGTGACCAAGCCAA
CACTCTGGTGGACCTTTCCAAGATGCAGAATGTCATGTATGACTTAATCACAGAACTCAATGACCGGAGCGAAGACCTGGAGAAGCA
GATTGGCAGCCTGGAGTCGAAGCTGGAGCATCTCACCGCCAGCTTCAACTCCCTGCCGCTGCTCATCGCCGACACCCTGCGCCAGCA
GCAGCAGCAGCTCCTGTCTGCCATCATCGAGGCCCGGGGTGTCAGCGTGGCAGTGGGCACCACCCACACCCCAATCTCCGATAGCCC
CATTGGGGTCAGCTCCACCTCCTTCCCGACCCCGTACACAAGTTCAAGCAGTTGCTAAATAAATCTCCCCACTCCAGAAGCATTACC
CATAGGTCTTAAGATGCAAATCAACTCTCTCCTGGTCGCTTTGCCATCAAGAAACATTCAGACCAGGGAACGGAAAGAAGAGAGACC
GAGCTAATTAACTAACTCATGTTCATTCAGCGTGCTTGGTCCGACATGCCTTGAAACCAGAAATCTAATCTCTGTTTAGGTGCCTCT
ACTTGGGAGCGGGAAGAGGAGATGACAGGAAGCGACGCCTCTGGCAGGGCCCTTGCTGCGAGTTGGTGGAGAACAGAAATCCACGC
TCAATCTCAGGTCTTCACGCGGGGGGTGGGGGTCAGATGCACTGAAGTAGCCAACAGCGAAGCCAGTCCAGAAGAGGGGTCCGCTGG
GAGGGAGGGTTGTGTCAGGCTTGGGGGATGGGCTCTTCGCCATGGGGGTCTTTGAACACACCTCTCTCCTTTCCTTTTGTCTACGGA
AGCCTCTGGGTGACAAAAGTAAAAGAGAGCTGCCCACAACTTGCCAAAACAGATATACTCGAATCAGACTGAAAAAAAAAAAAAAAA
```

HUMAN PROTEIN SEQUENCE : hP27-009.2 (Seq ID No: 30)

MDTSGHFHDSGVGDLDEDPKCPCPSSGDEQQQQQQQQQQQQPPPPAPPAAPQQPLGPSLQPQPPQLQQQQQQ
QQQQQQQQQQQQPPHPLSQLAQLQSQPVHPGLLHSSPTAFRAPPSSNSTAILHPSSRQGSQLNLNDHLLGHSPSSTATSGPGGGSRH
RQASPLVHRRDSNPFTEIAMSSCKYSGGVMKPLSRLSASRRNLIEAETEGQPLQLFSPSNPPEIVISSREDNHAHQTLLHHPNATHN
HQHAGTTASSTTFPKANKRKNQNIGYKLGHRRALFEKRKRLSDYALIFGMFGIVVMVIETELSWGLYSKDSMFSLALKCLISLSTII
LLGLIIAYHTREVQLFVIDNGADDWRIAMTYERILYISLEMLVCAIHPIPGEYKFFWTARLAFSYTPSRAEADVDIILSIPMFLRLY
LIARVMLLHSKLFTDASSRSIGALNKINFNTRFVMKTLMTICPGTVLLVFSISLWIIAAWTVRVCERYHDQQDVTSNFLGAMWLISI
TFLSIGYGDMVPHTYCGKGVCLLTGIMGAGCTALVVAVVARKLELTKAEKHVHNFMMDTQLTKRIKNAAANVLRETWLIYKHTKLLK
KIDHAKVRKHQRKFLQAIHQLRSVKMEQRKLSDQANTLVDLSKMQNVMYDLITELNDRSEDLEKQIGSLESKLEHLTASFNSLPLLI
ADTLRQQQQQLLSAIIEARGVSVAVGTTHTPISDSPIGVSSTSFPTPYTSSSSC*

## Table 4

MOUSE GENOMIC SEQUENCE : mD27-014 (Seq ID No: 31)

```
AGCTCGGCCAGGGGAAAGTGAAAGTTTGCCTCGGTGCTCTCGGTGTCGCTGCGGCTCTCTGCATCCCAGGACAGCGGCGTGGCCCTC
GACCGGGGCGCGGGCTCTTCAGCCACTAGCGAGCAAGGGAGCGAGCGAACCAGGGCGGCCAACACGCCGTGCCGGGACCCAGCTGCC
CGTATGACCGCGCGGGGCGCCGCGGGGCGCTGCCCTTCTTCGGTAAGCTGCAACCGTGGCGCGCGGGGCCCGGGCCGGGCTGGGGCA
GGAGCTCTGCAGCAAGCAGCAGGCGGCTCTGCGGGGCCACTTGGAGCGGACAGCCCCTTCTCGCCAGCTGCCCAGGCTTCTCGGCCC
TGGGTTCTGGCTTCCTTACTGCTTCTAAGCTACGGCTGAGGCCCGCGGTATTTCAATAGCTGCTCCTGGGGCTGCAGCGCTTTGCGA
GGTAAAGAGAAGGCTGCTCATCCCATTGCACGGAGGAGAACACTGAGCTACCCACAGGCTGGAGAGAGAGATGGGGTCTGGGGGGGG
GGGGGTATCGCAGGGATTGGCACAGGCCCAATGTCTGCCAGAATGGTCAACCAGGTCTCCAGGTTCCGAGTGACACAAGATCCGAGT
GTGGCATCATCCCCAGTAGATATCTTTCTTTGGTTGTTCACATGTTTTAGGCCCCAGTTGGGAGTGGGCCTTCAGGGTAGGGAAAA
TTCAGGCTCACTCAGCTTTACACCAGGACCTGTGCTGAGCAACCTGTTGGCAGAGAGAGAGAGAGAGAGAGAGAGAGAGAGAGAGAG
ATGGGACTGCTGCTACTTGTTCAATGGATGTGTAGGAACTATTAGGGACTTGGGTGGCTCTTTGAGGGGAGCTGGACCCGAATCTTT
TCTTGAAGAAAATGAGATGGGGCTATTCTAGACTCTGCGGCTGACTGGGAAAGAAAGTGCAAGGAGAGTTCAGAGCTGCAGCTGAT
AATGGGCTGCTCAGAAGCTGTCTCCAGTAATGGCAGCAGATGATAGCATGGCTCTTACAGAGTGAAGGATGGTACTTTCAATCCACA
GCAAGTGGGTTTGCTTCAGAAGAGAATGAGAGGGAAAACCTGGAGGGTAGGAACCGAACTTGTGATGGGCAGCGCGTTGCAGGTGTC
TGGGAGGAGAGAAAAGTAGAACAGCAGAGAGAGGGGCTCAGTGAGAGGCAGAAGGTGGGGTTAGGAGGAGGGGAAGGGAGGAAGTTA
TCCCACATGCAACAAGCATAAAGAGAGAGAGGGGAAGAAAAGAAAGACTACAAGGAGACATAAGAGGAAAGAGAGGAGGCCGAGGTT
CAGCCAGGCAGTCAGGCAGGCTCTGTCTGCAGACTGTACTGATTGGAAAATGGAACATGATGTCTTGAAGTGCTCAGAGACTGCC
GCAGTTTCCAGGCTGCTCTCATAGTCAGAGGCTGAGGCAGTGGGTGGGAGGAAGAAGCAAGCCAGGAAGAGCCTGTCCAGCCAGATG
CAGGCTAGAGTATTAACCCTTGGGTGTCCATCTGTATCCCTGTCTATGGCAGCATTGGGGTTACAGAGCCAGATCCAGGAGTGGCTG
GTCATCTTTGAAGCCAGCAGCTGTGCATTATTTCTGTGCATGCTTGAAATGCCACCAAAAGCCAGGTAGCCCTGAAGTAATGTCTTT
ATCTTGCTATCTCCAAACATCAATGGCTTGAAATTCCTGGGTTGAGATATTTCTGAACATCTCCATTCCCGGGTCCTTTCCTTCATA
CCATACACACATGGACTGACAGGCTGAGTGATACCAGCCTGGGCCAGAGTCATCTTCTGCAGTCATTTCCTTTAACAGGTTCTGAGA
```

```
CTGGGGGTTCAGGAGACCCGGGCTGCCACTCCCACTCAGGACTAACTTTCCAATTGCCCAACCATGCCCAATGCAGTTTATCAACCCA
TAGCTGGCTGCACCCTCTCCCCCACCTCTTTCTCCCTGTCTCTCTGTGTCTGTCACTCTGTGTCTCTGACTGTCTGTCTCTGACTGT
CTGTCTCTGACTCTCTGTCTCTCTCTCTCTAAGGTCAGGCAAGGAACCTAACAGGTCCTGTGCGTAATGGCATTAGGCAACCCTG
TGCCTTAACACCCATCAGCATCTTCCACCCTTTAAGGCCTCACTCTTCCCTCCGCATGGCCCAGCCAAAGCTGCCTTTCCTCCCTGG
TACTCACTGGCAAGGCTTTGGGCTTTTAGATGTAACCCAGTGTTGTTATGGACTGTTTCATAGCCTCCCTGTGTTTTTCCAATGCGC
TCCTATTTGCAACATTTGTCTCATCATCTGGACTATTGGGGCAGAGCCAGATCCAAGAAGGTGCCAGTCATCACTCCACCCTGCCAG
CATTTCCCTGAATTGGGGGATATCAGCAGCCAGAATGCTCAGAATGGCAGCATCAGGCCAGGGAAAGAAAGCCCAAGAGTCCCTCTG
CCCCAGGCTGGCATCTCCCGGGATACCCTATTGGAGCCCGAAGGGGTGGAAGGATTTTAGAGTAGAGGAGGAGGAAGGAAGAAAGGA
GAAAGGAAGGACCCAGTCAGGTGTCAGGAGCCAGACCTAGGACTCTAGGGATGAGCAGCTGCAGGGCCAGGTGGGTGCTGACGGAAA
GGAGAGAACTTTCTTGCTGTGGCAAGAGTTTGCTGACTAGATGAAGGGCCTGATGGGTCTCAGATAAACAGATGCCAGCAGGGAAAC
ACAGCATTGTCCTTGATTAGTCAGCTGGACTCCTCTTGAAATGCAAGTCCAAGGCTCTAGGAGGTCTCCCACTGGAGCTGAGTTCCC
AGTGTGTTGCATCCTGGTATTTTCCCCTGTAGAACCAGACCTGTGTAACCAGGTGGGAAGGAAGGAATGAAGGAAGGAAGGAAGGTGCC
AGGGGTAATGAGTGTTTCTGGCTTGCAATCTGAAAGCTAGTGACTGACCACAAGTTTGAAGGCAGCTTACAAGGTATCTATTTGTCT
CAGTGTTAAAAGTTAAAAGGATAGAAAATCTTACCCTTGAGTCTCTCAGTCACTAAAACTTATACTGGTTGGCACACCACCAGTATT
ATCATTAGACATTCTCTGTAAGATGGAAAGGTGGCTCATCATTTAAAAACACTTGTTGCTCTTGCAGTGGATCCAGGTTCAAATCCC
AGAATCTAGATGATGGTTCACAACCATTCATACCTCCAGGTCCAGGAGATTCCAAGAAGGAAGCACATAGAGTGTGTGCATGCAAGC
AGGCAAAATATTCATAAACACAAAACAAAAACAGTAACAGTTTTAAAATTGTCTTTTAAAGTACTCTGTATTGTTGAGCTGCTCTGG
GTTAACCTCAGAAGAGTTCTTGGAGAAGATGGCATTTGGGCTATTACCAAGGGAAGAGTGAGGTGGTGCCCTGGCTGATAAGAATCT
AATGGTGCTGCAGCATGGGAATGGGGCGTTGTTTCTTTGCCCTTAGTGGGACATGGGGGTAGCTAGGGAGAGGAAGTGCTGGAAAGA
CATGACTCCCTTCTGTTATAGACATGGCTGGGCTCCCGGCTGCTGCTGGTCTGTCTCCTCATGAGCAGGAGTATTGCCAAGGAGGTG
TCAGAACACTGTAGCCACATGATTGGGAATGGACACCTGAAGGTCCTGCAGCAGTTGGTGAGTATGAGACCATGCCTTCTCCTCACT
GAGGAAACTGAGGTAGCAAGCCAGAAGGCAGAGTCAAGGAGGCTTCCAGAAAACAGTCAAGTGGATAATGGCTCTCCTGGTACTT
CTCTCATTGATCTTTTTAGGTCCTCCCAACCCCTTAGTCTGGGTCACCTGTAGTGACTAGGGTGGGCTTCTAACTGCTGCCTGGGTT
AATGGTTGTCCAGGAATGTCCACTATGGATTCTAAACTGCTCCTTGACCCTGCTCTGACTCAGACAATGCCTCCTTTGGGAAGTCAG
CCCAGTCTGCCTGCTGGGTCCAGATGGTTCATCCTGCTCCTGGATAGCCAGATCCCTGAGCGCCTGGCACCAGGGCCCCACCCAAAT
TTAGATGGGAGGCTCTAGCCTAACTCTGCGCCTTCTGCCTTCATTCCCCTGATCCTTGCCCCTGAGGTTGGAATGCCCTGCTCATCT
GGGCAGGAAGGAACCCAGGTCCCATTCTAATAGGCTTAAGCTTCAACAATTCTAAATGTCATCTAGCCAGGCAGGATCCAG
TTGTTGTCATGGTTCCTGCTCCTCTCACTAAAGAATTTATTCCACAAGCCCGGCAGGGTGGGGCACCAGTGGGTAAGGCCATGTGCA
GAGCCTGAACTTGGGCCCCCGAGCTGACCTTCCCAATCTCCCTTCTTAACAGCACCCTATGCATGCAGCTTCCATGGCAGGCCCAAG
GCCATGAGTGCTGTCGTGGTCTCTGCTCCAGGGAGAGGCAAGGGCTAGAGACCCAGACTCCCCAGAGCTCAGCCAGCCTCTCCCTGA
GGCTTTTCATGATAGAGTGCTTACCACCTTCCAGAAAAGATTACATCCTATATTTTCCTAGAGATAATTCTTCCATATACAGACTAG
ATTGTGCAATTTCATAGGTCAAAGTAGAAGTGTCTGTTGTTGGCTTTTAAGTACTCTGGGTATTTGGAAGATACAGCTTTTCCCCAG
CTGTGGCTCTGTCATTTTGGCATTGTTTTAGGGAGGTAAAATTAAAACATCATTCACCCCAAAACCTGGGTTCCCCATCCTGAAGCT
GAGTGGACCTGTAGGTACTATGTAACCACTGGCCTGTTCTATCTCACAGATCGACAGTCAAATGGAGACTTCATGCCAGATTGCCTT
TGAATTTGTAGACCAGGAACAGCTGGTGAGTAGCAAGTGTCTGTAAGGTGCTTAAGTGAATATTCACGGCCCGCTCTGTGTTGGCAT
GGAACAGTATGAAGGCAGCAGAAAACCTAGAGCAGACAGGATGAGGGTCCCTGGGTGCTCAAGTGTCAAGTTCCAGCTTGCAGGGCT
GGGTAGGGTCCAGAGCTTAAGTCCTTCTGTTTCCTTACTTTGACCTGGTATAAATCAGGTACTTGCTAAATGCTAGGCACATTCCCC
AATTAATCCTAACAATACAATTCTGAGGTCCACAAATATTGCTTGTTACCCATGCAGAGATTAAGGAGCATGCTTAGCCAAGTGATA
GAGGCCTAATTTGAGCCCAGGAATGTTATACCTTGTAAAGTAAAAAATGTGCTTTATTTTTTAGATCACATCTTCTCCAATCCACTG
GCCAGAGACAGGAAGGCAGTCTAGCCTGGCTTTCTACCCCACACCTAACTGACCAGCCACCCAATGCAGCCAATACTGCCTCTCTGC
ACCTTAGGGTGCTGTCCCTCTCTCCTCCCCAGTCCCTTGGAAATTTTCAGGGGCTTCTTGACTTCCCTGATTCTAGGGCACTCTCCA
TCTTACCACTAGGGGGAGCCCTCTGAAACCCCAAAGCCTTCACTTATTTCGCCTTACTTGTCAGGAGCCCCATGGCTCCAGGAGAAA
GCACAGTCTAGTTCCAAGTCTGCAGTACCTACCACACCCTACGTTCATTTCCCTCCTCTGCTTCATTCTGCTCCCCAGCTTTTGCAT
CAGCTCTTTGTGATGTCTTTCCATCCTTTCCATGTGCCCCGCTGATCCCTGTGCTACACACCTCCCTATCTTCAAGCCTCAAGAGAA
CAGTCAGGCTACCCAGAATTCCTTCTATCTCTGAGCTCTCATGCACTAGACCCATGCATCTCTGTGAACATGCCATGGCCTGCATAG
TCTCAGGGTTTCTTCCATCCATTCTATTGCTCCTTCACTTGCACCATGGCACCTATTTATTGGATCAGGCACTAGAGATACAGAGAT
AAAAGTAGTTTCTGTCTTTGAACCAACTACTGTATCCTTGGAGACAAAACCCAAGGCTACACTTTTGACAATGAGGTAGTCCTCCCT
CAGTATTTTTTATATTTTGGAGGTTTTGGGGGCCCATATGGCAAAAAAAAAAAAAAGTGGAAGTAAGCTGAAAGGAGAGAGGAGAGA
GAGAGAGAGAGAAAATGCTAAGGGGCAGCAAGGCTGAAACCCACCCTCCCCTGTGTCTCCTGAATAAAGCTGCATTGCAGAAAAGCT
CTCTCCAACCAGAATCCACCTTTCCTTCACGCCAGCCCAACTCTCCATCATGGTTGATTTAAAGAAAGAAAACAAAACACAGGCCAA
GGGTATCACCATCCTCATCACCAGGGTGGTGTGTCCCTTCCTCAGATTACAGGGTGACAGGCTACAGCCCTCTGTCTGCCTAGGGTG
TGATCGTCTCATATAAAGCCTGGGTTTCCAGGCCACCATGGCCCAGGTGCTCAACCTCCTGGTTCCTGTATTCCAGGATGATCCTGTTT
GCTACCTAAAGAAGGCCTTTTTTCTGGTACAAGACATAATAGATGAGACCATGCGCTTTAAAGACAACACCCCCAATGCTAACGCCA
CCGAGAGGCTCCAGGAACTCTCCAATAACCTGAACAGCTGCTTCACCAAGGACTATGAGGAGCAGAACAAGGTAGGGTGGATTCTGG
GGCTGGAGCACCAGAGAAGGGCAGAGGGACGCTACAGAGGTCCCACCCTGCCTCATATGCTCACCGAGCCTGAACCTTACTAACTTA
CTCCCTTGCCCATTCATTTCTTATGCCATCCGCCTTTATCAAGCATTCATTAGACAGAAGGCACAGTGCTGGACCCTGAGGATATCC
TTGGAACAAGAAATTCCCCTTCCCACCCTTACAGGGCTTATAGCCAAGTGAGGTAGCAGCTGTTAAATGATGACACCTATAAATAT
GATCAGAAGGGCTCTTCATTCATTCAGTAAAACCTTACTGAGTGACCATCAAGGGATGTTCTGAACACCAGAAATGAGCTGTGATTT
TAAAAAGGGGGGGGGGCAAAAATCCCCACAATCATGACGAGAGGAGGGGTGCTGAGGCAGCTAAAATGAGCAAGTATGTGAATGGACA
ATGATGCTAACAGCATTGGAGAAGTGTAGAGCAGCAGGTGAGGGGCGCCCTGGGTGACAGTGAGAGGAACAGGAACCCGAGACTCTG
AGTGTGCGGTGAGGAGCTAATGTAATGAGGATGCAGAGGGGAAGAGGCAAAGGCAGCTTCTAAGCAAGAAAACTTTAGCCGGCGCTC
CAGCGTGAGGAGGAGCAGTAAGAGCCACAGACAGAAACAGAGTGGGGAGTGCTATGCTCAAGCCCTAGGGTAACCAGAAAAGGTTT
GCATTCAGAAATCCAAAGCGGCTCACCATGGCCCAGGCATGGGGTGTGGACAGGAGGAAGCAAAGTCAGGAAGCACTGCATTCATGC
ATGGTGTCAAAGAGGTGGGCTTTTTGTCTAAATTTGAAAGAAGCCACCATAAGCTAGTGAATGACACGATGTCATATATGATGGAAG
ACCATCTGGTGGTGAGGACGCTGTGGAGAGCAGGAATGAAGGGCAGGAGGCCAGCAGGAGGTTCAGCCAAGCTGGGCATGGTGGTGA
GTTGAATCCGAGGGGAGCAGGGGAAATGGAGGTGAGTGGGTGTGATCCCTCCAGACAGACAGGACTCCATGCTTCCTGGCAGAAGGG
GAAGGGTGAGTGAGGATGCTCTCATTTTGAGTCCCTGGAGTTCTCACTGATGTCAAAATAAGGATGACCTCAGGCAGAGTAGGTTTG
AGAGAGAGAGCTCATTTTGGTGTGAGGCACATCTGACAGCTGCAGGCGCGGCTCATGACTGTGAATGCTGGACATACAGATGCTGTTT
GCAGAGCCACAGAACTGCACAGCCCTGCCCATCCTTCTCACCCTGGTGAGAAGAAGGGGATCCAGGATCCTGAATCCTGAATGCA
ATGCAGCCTGAGGTAGGAGGAAAACCTAGAGAGGGCAGGGCTTCCAAATGGAAGGAGCCAGAAGCTTTATCAAAGGAGGCTGAGAAG
TCAATTAAGGTAGTGTCCATTGGTCTGTCTCACTTGGCAGCATGGAGATTGCTGTTGGAGGCAGGTGGGGAGAAAAGAAAGAGAGGA
AACAGAGCCACAAAGTAGAGGAGCTTCGCTCCAAAGGGGCAAGGAAGCAGATGTTCTGGGGTGGCTTTCTGGTGAGGAAAGGCT
GGTTCTTTACCATGCAGAGATTCACCCGGAGAGCTTATTTAAAAATGCAGAGTCCCAGCATCACTGCTACGGACTCTACTTTCCTGAG
CTCATAGGTGAGCCTGGGAATCTGCATTTTGCGAAGCTGCCCCATCAGTGGTTGAGATAGAAGCCCCTCAGTCTTTCCTGGGCCGGT
GACCATTCACTCAGTGCATGCTGTGGGGTGGCTCAGGGGAAAGACAGTGTAAGCCCAAGGAGCCAAGTCTAGTCACATGGCTCAGAA
AAGGAGTATGAGCTCCGGAACCAGCAGGCTCCACACTGAGAAAGCTTGGGTGTCAGGTTCTAGGGGAGGATGCCTGGTTTCTTCTAC
```

EP 2 204 376 A2

```
AGCATATAAGGCAGGTTTGAGGGAAACAGCTTTTATAAAGACACATGCAGGACAGAGCCAGCTGGCCTGACTTAACAGCTCCTGGGAA
AGGCTGGCTGCTGTTGCTCTAACATCACAAACAAGGAGATGAATCCACAGAAAGCCGGTCTATTCTCACCTAGCTGCTGAGTGATGA
ATCCAAGCCCAGGTCATTTCATTCATTATTTGCCTAGTAAGTGTTTATGAGCCTGCTATGGGCGGGCCAGAGAGATGCAGTGATGTA
ATCAAGGGAGCTATAGTAGCCGTAGAAAACCTATTGTTCTTCCCAGAACAGCTCAACTTCAAGGGGGAAAGTTGGCATTCTGGAAAT
AAGCCGTAACACATATAGCTTGCTGTGAAAGGTGAGGTGGACCTAGGGCAGAACCCTGCAGCACTGCAGTATTTAGAAGGAACTCTC
TGGCACAGAGCCTGAAGGAGCAGCTGGTGAGGAAGGAAGAACTCTGGAGAGCCTGGCTTCTCCGAGCTGCATGGGGTGTGAGTGTGA
GCACCTGGGCCCAAGGCAGAGGAGAAAAACCACTCAACAGGCAGGCCAGGGAGTCCTCAGCTCTTCCTCCAAAATGTGGCACCAGCC
AAAGACCTCATCTCCTCTGATCTCCCCAGGTCTGCTCTCTACACCGTACCAAGATGATTTTCCTAAAATACAGGTTTTATACCATGG
TTTTAAACCTCCCCAGCAGGTTTTCAGTTTCCGGTTGGCTCTGTGAATTACAAGTTCCTGGCAGAGTCAACAAGTCCTTGGCCTTGG
CAAAGCCCTTTCAGCTGCCACTTTCTTCCCACGTGGCAAAGCTGCACCCGCTCCAGCCTTCTTTCACTCTTTTGCTGACTCATGCTT
TCCTGCCCCAGGGGCCCTTGCATCCTCTATCATCTCTGGCCCCACTTCCTTCTCTTCCCTCGCTCATCTTTTAGCTGTTCCTTTGGAG
ATCTTCTCCCTGGGCCCTCATCTATCGCCCATTTGTTACACACCTTCTGATGGTTCAGGACACTCTCTTGAGAGTCACTGTGACTTG
TAACAATGTGTCTGCTGGACTCAGTGCCCAGCTTGGCCAGGAACCACTTCTCTTTTGTTCATTGCATGATGCCCAGGTAAGTTACCA
TATATCTGGCACTTAAACGTGTGTTAGATGGATCTCTGACTAAATGACGCATCTCTCAACCTCAAGACTGATGCTGCTGCAAAGCAG
AGAAGGAAGGAAACAAAAGGGGGAAAGGAAATGCCTGTGAGAAACCCCAAGAGAGTTTGACAAAAATACCAGTGAGAGGTAGCCCAA
TGTTCATGCTTACCAAAGGGGGCTCCACTCTCCTCCAGGCCTGTGTCCGAACTTTCCATGAGACTCCTCTCCAGCTGCTGGAGAAGA
TCAAGAACTTCTTTAATGAAACAAAGAATCTCCTTGAAAAGGACTGGAACATTTTTACCAAGAACTGCAACAACAGCTTTGCTAAGT
GCTCTAGCCGAGGTAAGCACAGAAGGGGTCAGCTACAGGGAGGGTGTGTGCAAGGTGAGACGGGGCTAGGGGAATCCCAGAGGCAGC
CTGGGTGCTAAGTGTGTGTTTCTGTGTTTTGTGTACTCGTGTGTGGCTTCATGTACCTTAAGGTCCTGGGCACATGTCTCTGCTGACATGT
GTGTGCATGTACACTGACTTATGCCTGCATGTACTGTCTCACTGCCAAACTTCATGTGGCAGCATCAAGAAGGCCCCTGCTGGTAAC
TGTGATGGAAGTGAATGGGTAAGGGGGACCTTTCTTAGAAAGCCATATAGGCATGGTGGCGTCCCCCGGGCTCTGCAGGCTGGCTGAA
TAAATGGGGGTTCCGGAAAGACCAGGTGCTCCACCTGGCCACGTGTGTCCTTGCTCCAGGAGCTCTGCTGCAAAACACCAGGATAAC
CTGGCTCCAGAAGTCCCCAGGATGCCTTGGATGTTCCCTCAAGGGATAAGGCTGGATTTGAAGTCTGAACATCATTCAAACTCTCTC
TACCTGTGCACTCTGAGTGTACTCGTCTCTGCATCCTGTGCCACGTGTGTCCTGCGTTCAGCCTCGCTCCCCAAGGCAGGGGCTGAG
GTCCTGCAACCAGACAATAGCCACATCCAGCCTTAGTGCTTCAATTCCAAAACCCTGAACTTTGGCTGCTTTCCAGCCCTGCCTTGC
CCTGCCTGTGGCAGTGGCTGAGGTGGTTCCACTCCTGGCTGTGCATGAAACCAGTGTCTTGGAGGCTTCTGAGTGTCTTGTGTTAAT
CCAGGAGCTATTGCTCAGCCCTGTGGCCTGGGTGCTGCTAACCCCTGGCTTGGCTTCTGGCCAGCTAGAGCCCCAGTACACTGTCCT
GTGGTCTTCCTCACCTCAGTCCTTCTCTGTCTGTCTGTACCTTGCTTTTGGTTCCTAGTCCATATCCCCTGCGTATTGATCCCTCCT
GCCTGAGTTCTGTGTCATGATCGTTTATTACTGTCCTGCCCTCTCCACCGCCCCAGGGCTGTGTTAGAAGAGGGACCCCCTTCCCAT
GCCCCAGGGCCATATGCCCCTCTTAGCCCCTCTGGGGGAGCTGTGCTGGAGGCTGGTGACTCTATCTCCTCCCCATCTTTCTCTCTC
CCCCTCTCTGTGGTTCTTTCAGATGTGGTGACCAAGCCTGATTGCAACTGCCTGTACCCTAAAGCCACCCCTAGCAGTGACCCGGCC
TCTGCCTCCCCTCACCAGCCCCCCGCCCCCTCCATGGCCCCTCTGGCTGGCTTGGCTTGGGATGATTCTCAGAGGACAGAGGGCAGC
TCCCTCTTGCCCAGTGAGCTTCCCCTTTCGCATAGAGGACCCAGGCAGTGCCAAGCAGCGACCCACCCAGGAGTACCTGCCAGACCCTC
GAGTCAACAGAGCAACCAAACCATGGGGACAGACTCACTGAGGACTCACAACCTCATCCTTCTGCGGGGGGGGCCCGTCCCTGGGGTG
GAAGACATTCTTGAATCTTCACTGGGCACTAACTGGGTCCTAGAAGAAGCTTCTGGAGAGGCTAGTGAGGGATTTTTGACCCAGGAA
GCAAAGTTTTCCCCCTCCACGCCTGTAGGGGCAGCATCCAGGCAGAGACTGACAGACCCAGGGCCCTCTCAGCATCTCCATTCCCT
AAATCAACAGAGGACCAAAAGCCAGTGGATATAACAGACAGGCCTTGACAGAGGTGAACCCTATGAGACCCATTGGCCAGACACAG
AATAATACTCCTGAGAAGACTGATGGTACATCCACGCTGCGTGAAGACCACCAGGAGCCAGGCTCTCCCCATATTGCGACACCGAAT
CCCCAACGAGTCAGCAACTCAGCCACCCCGTTGCTCAGTTACTGCTTCCCAAAAGCCACTCTTGGGGCATTGTGCTGCCCCTTGGG
GAGCTTGAGGGCAAGAGAAGTACCAGGGATCGAAGGAGCCCCGCAGAGCTGGAAGGAGGATCAGCAAGTGAGGGGGCAGCCAGGCCT
GTGGCCCGTTTTAATTCCATTCCTTTGACTGACACAGGCCATGTGGAGCAGCATGAGGGATCCTCTGACCCCCAGATCCCTGAGTCT
GTCTTCCACCTGCTGGTGCCGGGCATCATCCTCTAGTCTTGCTGACTGTTGGGGGCCTCCTGTTCTACAAGTGGAAGTGGAGGGTAAGG
AGGACCCCAAGAGAGGAAGACATCCTATGGGGCAGAACAAAGGCTGGGTAGGGTACAGGCTGGAGGCTAACCAGGTTCCAATACGTT
ATGCAGTTGCTGGAAGGTGGGGATGACAGCCCGAACGGTAGACGGTAGTGATGGAGTGTGGCTTCACACAGGAGCTGCCAGGTTTGG
TGGTGTGCATTTAACCCTAGCAATCCAGAGGTACAGGCAGGCCTCTGATCTACTGGTCAACAAAGCAAGTTCTAGGCTAGCCGAAGC
TACATAGATCCTGTCTGAGAAAGTAATTAATCAATTAATTAATGAGGTCTCCAGCCTCAAGATCATTAAGAAAGGATGAGTTCTAGG
CTAGTCAGAGCTTCATAGAGAGATTCTGTCAAATAAATAAATATATAAATAAATAAATTTAAGAAGTTTCCAGACTCCTTGAGATTA
TTGAGAAAGGATGAGAACAGAATCCTGTAGAAAGGAGAATTCTTGGCTGGGAACTCAGGAGGCAGAGAGGCAGAGAGAGAGGCAGAG
AGAGAGGCAGAGAGGCAGAGAGGCAGAGAGGCAGAGAAGCAGAAAGGCAGAGAGGCAGAGGCAGGTGGATCTCTGTGAGTTTGAGGT
CAGCATGATCTACAGAGTACCAGGGCAGCCAGGGTTGTTATGCAAAGAAACCCTGTCTTGAAAAAAATGAGAGAGAGGGAGAGGGAG
AAGGGATGGGAGAGGGAGAGGGAGAGAGGGAGAGAGAGGGGGGATATTCTTAAACAACCAAGTGGGAGAGGGAAGGAAAGGGGAAGA
GCTTGAGGGAGCTCTGGGAGCTTGAGGGCCTATAGGTGGTATTCTACATGGGAGGGACAGGAGAAGGCGCACCCATCCTCAGAGGGC
CCAGGAATCTCCCCCAGCTGTGCATGCTTTGGGGTAGTGGGAGGAATTCTAGCAAGTCTCTCAGTGATGCTCAGGGGCATGCCAGCG
CCTCAGTGAGATAAATCTTTCTTGTTCTCAGAGCCATCGAGACCCTCAGACATTGGATTCTTCTGTGGGGCGACCAGAGGACAGGTG
AGCAGTTGGAGTGGGGCTATAGGAATCACTGGATGTCTGTCTTGGGACCTGCTTCCCCTCAAGACTGAAAGAGAGGCTTCTCCCCCC
TTGCCCTAAGGAGATAAAGCTGCAGAGCAGGGTGGGTACCAAGAAAGAAGGGCCCCTCTTCTTCCCATGACAGTGGAGCTTTCTCCT
GATTTCTTCCTGGAGTTGGGCAGTTCTGGGTCTTCTAAATCTCACATAGATGGAATCCACGTGCAGGGTTGGGGGTTAAGCTAGGAGG
TCCCCTCAGTCCCATGCTCCTGCTGAAGTCTTATGTCAGCCCTGTGTTCTGCCTGCAGCTCCCTGACCCAGGATGAGGACAGACAGG
TGGAACTGCCAGTATAGAAAGGATTCTATGGTAAGGTTCTGATTTTGATATCTCTCTATCCCTTAAAAAGAACTATCTCCAGGTACC
CAGTCCTGTTGTAGAAACCACCTGATGCCCAAGAGGCCCACACATTTCAACTTCCTCTCCCGTCCCACTCCCAAGCCCATACATTC
ATCATCACGTCTAGCTAGGCTCCTCTCTTCCAGGCCTCAGGTCTCCTCTTGCTCCTGAATCCCTGCCCTGGACCAGTTACTGCAACC
TCTGCATTATGTTTCTATTCTTTGCTGTTGCTACATCTGATCCCAAAGCAACCACATTCTTCCTCCTCTAATGAGGCTTTTTTACAAGG
TCTTTACCACCCTGGAGAATTTTCATTAGCCTTTGCATGGCCGCCAGCCCCCTTCAAATACGTGCATTTCCAGCTTGGCTGCTGAGCC
CTGACCTCCTCAGCATCCTCTTCTTGCCAAATTGTGCCTTATCTGGTCCTACTCCCAAGCCAAGGTTATTGCTGCCTCCCTGATTTA
GGATCATTGTGCCATAGAAATTTAGTCTTAGGAGAGCTGTCGAGAGGTCATTTGGCCCAGTGTCCCAGCCTAAAAGGGTTACTCTGC
ATCCCCCACAGGGAGTCAGCCAGCTTCCTGCACACACACACACACTCCCAGTTCCAGGCAAGTTCTCTGCATGCCAGTCTGCTCA
ACTGGGGTCACAACATTGGGGGTCACAACACAACACTGGGGGAGTTCCTTCCTTATGTAGGCAAAGCTGCCTTCCTTTCTTCCCTT
CCCAGACCAATCCTCTCTCATCTTCTCATGAGGCCTGATATTTAAAGTCAGCTATTCATGTCACCTTTTAAAATTCTCTGTGTTAAG
TGTGGCCAAGCCCCTCAAACATTCCTTATATGATATGGTTTTCAGACCCCTCACCATCCTGGACACACTCGTTTGTCAATGTCCCTC
TGAAAATGTGGCGCCCAGCCCTGGACACAGTACTCCAGATGTTGTCTGACCAGCTCAGAGTACAGTGGGACGGTTGTCTTCCTTGAT
CTGGACAGTACTCTTCTACTCGTGCAGATTAAGATCACATTAGTTTTAACAGCTGCATCATATATTGTCATATGTTGAGCTTGTAGT
CTATTAAAAACCCCAGTTCTATTTCCTGTGAACCTTTGTCCAGTAGACCGTCGCCATCCCATCTCCCATACTTGGACACAACCGTT
TTAGCCAAAGTGTAGCTGGTGCTCACCTTTGTTAAAAACTCCTTGTTGTTTTCTGCCCATCCCCTGCACCTACTGAAAGTGTTTTAGT
TCCTAATTTGGTCACTTTATAACTCCTGGTTGGGTCCCCTGCACATTAATGCGTGTCTTTTGTTGTCCTTGCCCACGCTATTGGTGG
AGATGTGACCTGTATTCTTCCTCTACACCCACCCACACCAGCCTATCCAGAAGCCTTCCCTTCCTAAAATATTCCACATCCCTCTGG
TTGATCTTTTAATTCATTCTGCTCTGTTGTATATGGCTGATGCTATATTAATCCACATACATTTGGATGTTGTTTTATGTATTTTAA
```

```
ATCATATTTTATGTATATATGTAAATTTTGAACTACTAAGAAGATTGTAAGCCCCTAGAGGGCAGGGACTATCTTCCTTTTTATTTT
TTTTCCCCTTCACTTCATTAATTCTAGTACAGACAGTGCCCCTCTATCCTTGTCAAAAACTTAGTAAATGGTGGTAGCTGTTTTCA
GGTCTGAATCACCAACTAACCCCGGCCAGATTTCTCTAACTGACCAGCCTTCTGGCTGACCAATTAACTAGCCAACTAATCAACTCA
TCTTACCAGCTAGTTGGCTAACTGACTACTTGGCCAACTAGCTGGTAACTACTTAACTGACTAGTTCTTGATTGATGAGCTGATTGG
CTAACTAGCTGGCAGAGTGCTTAGTTGATTTGCTGACTGACTGGATGGCAAACTAGCTGTGTGATCGGTTGAAGTTTCCAGTTATAC
CCCTTGCCACATGTAAGCAAACTGTCCCATGTGTAGGATGATGTGTCTAAGCCCCTTTCCCAAAGGAAATATCACAGATGCTCAGAG
AGCCAGGGAGATTTATTTGAATTAAAGCAAGCAGCAACCCATATCTAGAATCCCGGGGACCCACTCAGCAGTAATGTGGGTGGAGAC
GGGTGAAATGAATTAGCTGGCTCACTGGAACAGCTACAAGGACACAGAGTCCCTCTGACCTGAATCAGAGAGCAAAGGTTGCTGGGG
ATTTGGGGGCTCAGTGCTGTGTCACCTTACCAAGGGCTACCAGGGTTCTTCCTCCCTGCCACCTGTATGACCTTGTCCTCGGCCCTG
GTTTCTCTGTACCCAGTTGTCCTTCCCATGCCTCCTAACTCTCAGACCTGCCCAAGATTTTACGTTCCTCACCTAAGGATTTCAAGA
TTTCACTCAGTCAGCGCAGATCTAACCTGATCCCCATTCCAGGCTCAAGGAGCCTATGGGAACTGAATGTCTGTGTCCTGATTGACC
AATACTCTGACAAGGAGATGAGTGGGGCACACAGAAGTGGGGACTAAGAAAAGGTCACCAAGTTGTAGAAAACTCAGGCCCTCGGCC
CCAGGAAACTGGGAGTTGGGTTAGAGGCTAAGGTCATGCCCTCTGGGGCCTTCTTCCCGATGGGCCCTGCCAGAGCCTGTGGCCTGG
TTGTACCAGTATGGCTCATGCCCAGTTTATAGTTTCTTCTTGTTTCCTATCCAAAGCATGGGAATAGGCTGGTGGCCAAAGGCTGTA
GGTCTCATTTTCTTTCATACCCATCACAGGCTTGACCAGTGGGGCTATGGACATTACTGGGGAGGCGCCATACAGGGTCCAAAGTCA
AACTGTTGGCTGAAGTCCAGGGGATTTCTGCTATGGAACAAAGAGTTCTGAGGATACTGGACAAGGGGACAAAGATGGGGGTGGGGT
GGGGAGAGGGCAGGGCCCTGGCTCCCATGGATGACAAGAGGCAAGTCCCTCATCCCATCTGGGAGGAGGCTAGGCTGCTTGGGGTGA
GCATGCCTGTTGACAGGGCAAGCCCTCCCTTTTCCTGGCATTGTCTCTACCCTCTGCCCCTCAAGCCTGAGTTCTTTCTATTCAAAT
GCCTTTGCCCAAGTGTCTTCTGTGCAGATACCCTCTAAGGGATGGTATGAGAGTTGGCAGAGATCACCTTAGGTCATTGGTCTAAGA
CTACTGAGATGGCCTTACCTGAGAACTAGCCTAGTAGGGGCATTCAAGAGCTTGTTCCCTGCTCCCATCTGCTAGAGCAATAGCCCT
CTCTCTCTCTTGGCCTCGACCTCCTCCAGTCTTAGTCCCTCATTTTGTGGTCACTACTCCCCCAGGAGGAGAGGTAGGAGGTGAGGC
CAGGCTATCCAAAGGCTTGGACTGCTGCCAGGCAAAGCTGCCTGGTCTCTCTGTGGTGTCTGGGGTCGCCTAGAGGGCCAGGAAAAT
GACCAGAGAGGAAGTCTTGCCTTAAGTCAGCCAACAACCCACAGGAATGTCACACCTCCCCCAACTATGTCCTTAGCGACCCTCCCCA
CACTCCCCTGCTGACCAGCCTTCTTCCCTTCTTTCCCCTACAGCTGGGCACACAGGACTATCTCTTTATGGAAGGAGACATATGGGA
ACATCCACCACTACCCTCTCCTACCATCTTCCTGGGAATGTGGCCTACCACTACCAGAGCTCCTGCCTACCAAGACTGGATGAAAGA
AGCAGCTTTGATGGGGTCTTTCCATCCTCACCCTTAGACTCTCAACCAAAGAGAAAGGGCTGGAGGATGCCCCCACATACTGCCAC
TATTTATTGTGGGCCCTGGAGGCTCCCTGCATTGGAGGAAGGGCAGCTCAGCAGCTCAGGACCCTTTCCCTTAGGGGCTGCTTCCTC
CCCTCAAAACCAGAACCTGGCAAGGGACTCACTAGCCTGGATGGCCCATGGGAGACCAGGACAGATGAGAAGGAGCAGAAGAGCCCT
GTGCCCAGAAGACCCAACTGGTGCCAAGGAATCCCAGCATGGACAGGCAGGGACCTGTTTCCCAAGAAGAGAGCCTGATATTCAAAG
GGTGGGACAGCATCTGCCCGACTTCCCGTAAAGGCATAAAGGCACGCAGCCCAAAAGACGGGAAGAGGAGGCCTTTGGCTGCTTGTG
TTGACAGCTTAAAGGGGTCTACACCCTCAACTTGCTTAAGTGCCCTCTGCTGATAGCCAGGAAGGAGGGAGACCAGCCCTGCCCCTC
AGGACCTGACCTGGCTCATGATGCCAAGAGGAAGACAGAGCTCTAGCCTCGTCTTCTCCTGCCCACAGCCCCTGCCAGAGTTCTTTT
GCCCAGCAGAGGCACCCCTCATGAAGGAAGCCATTGCACTGTGAATACTGAACCTGCCTGCTGAACAGCCTGTCCCATCCATCCCTA
TGAGTGACCATCCGTCCGAATGTTCTCCCACTTCCTTCAGCCTCTCCTCGGCTTCTTGCACTGAGCTGGCCTCACGTGTTGACTGAG
GGAGCCCCTGAGCCCCAACCTTCCCCTGCCTCAGCCTTTGATTGTCCAGGGTGAAGCTGTGGGAGAACCGCCTGGGCTACCAGTCAG
AGCTGGTCTTTGGGCTGTGTTCCTTGCCCAGGTTTCTGCATCTTGCACTTTGACATTCCCAGGAGGGAAGTGACTAGTGGAAGGGAG
AGAGGAAGGGGAGGCAGAGACAAAGGCCACAGGCAGAGCTATGAATGAGAATGGGTCTTGAAAATATGTGTGCACCCCTAAGCTTGA
AATTGATCTCTATACTCTAGCCCCTCAGCCAGCCTCCTTCCTGTTGTCTGAAACCTGGAGCTAAGCAGGTTGCCTGTCACAAGCTC
TGGGGACTGAGCTCCATGCTCCAACCCCACCCTCTTCTGACCTTTGTTCTCCAGACCTGACCCAGGTAGGCAAGGGTACCCTCCCAG
TCTCACCTACCATACTGTGCCATCTCTAGCCAAGCAAGCCAGGTTTAGAGAAGGGTCAAAAAAAAAAAAAAGGGGTTGTTTACTTCCA
ACTTGTTCTGATGCCCTCTGTTTCCCAGGCCAGGCTTGTCTGTGGTGACCTGGGCATGGGTGACAGGGCTCTCATTTGCCCCTTGGT
CTCTTTATGCTGCTGAGTCCCCCTTTCCTGCCCTCCCTGGCTACTGGGTCAATAATCTTTCAGGCCATGAATCTGGGAGGAGAGTGG
TCTGTAAGCTCCATCAGCCCTGTCCTGAGACAGCAGGGGGGAAGGACACTGGAGACTTTCTTGTGGGGACTTTACTTAGCCTTCTGGTT
ACAGACTATTTCCATGCTAGAAAATACATATTTTAAAATAGAAGGAAAAACACAGAAACAAAACAAAACAAGGCATTCTCTACCCCT
CCACCTTAAACATATATTATTAAAGACAGAAGAGAAAATCCAACCCATTGCAAGAAGCTCTTTGTGGGTGCCTGGTTACATCGGAGC
AGGGGAGCCTCAAATCCACCTTTGGAGCCGCCCCTGTGTGCATTAGGAACCCTTCTCTCCTCTGAGAAAGCTCAGAGGGAGCACTGC
CTCACAAACTGTGAGACTGCGTTTTTTATACTTGGAAGTGGTGAATTATTTTATATAAGGTCATTTAAATATCTATTTAAAAAATAG
GAAGCTGCTTTTATATTTAATAATAAAAGAAGTGCACAAGCTGC
```

MOUSE mRNA SEQUENCE : mR27-014.1 (Seq ID No: 32)

```
GGGAAAGTGAAAGTTTGCCTCGGTGCTCTCGGTGTCGCTGCGGCTCTCTGCATCCCAGGACAGCGGCGTGGCCCTCGACCGGGGCGC
GGGCTCTTCAGCCACTAGCGAGCAAGGGAGCGAGCGAACCAGGGCGGCCAACACGCCGTGCCGGGACCCAGCTGCCCGTATGACCGC
GCGGGGCGCCCGCGGGGCGCTGCCCTTCTTCGACATGGCTGGCTGGGCTGCTGGCTGCCTCCCTCCCTCCCTCCCTCCTGGCTACTGGGGTCAATAA
TCTTTCAGGCCATGAATCTGGGAGGAGAGTGGTCTGTAAGCTCCATCAGCCCTGTCCTGAGACAGCAGGGGGGAAGGACACTGGAGA
CTTTCTTGTGGGGCTTACTTAGCCTTCTGGTTACAGACTATTTCCATGCTAGAAAATACATATTTTAAAATAGAAGGAAAAACACAG
AAACAAAACAAAACAAGGCATTCTCTACCCCTCCACCTTAAACATATATTATTAAAGACAGAAGAGAAAATCCAACCCATTGCAAGA
AGCTCTTTGTGGGTGCCTGGTTACATCGGAGCAGGGGAGCCTCAAATCCACCTTTGGAGCCGCC
```

MOUSE PROTEIN SEQUENCE : mP27-014.1 (Seq ID No: 33)

MTARGAAGRCPSSTWLGSRLLLVSLPGYWVNNLSGHESGRRVVCKLHQPCPETAGGKDTGDFLVGLT*

MOUSE PANTHER CLASSIFICATIONS
        FAMILY (SUBFAMILY)
                MACROPHAGE COLONY STIMULATING FACTOR-1(MACROPHAGE COLONY STIMULATING
FACTOR-1)
        BIOLOGICAL PROCESS
                Immunity and defense(2.16.00.00.00) > Macrophage-mediated
immunity(2.16.05.00.00)
                Immunity and defense(2.16.00.00.00) > Granulocyte-mediated
immunity(2.16.04.00.00)
                Developmental processes(2.23.00.00.00) > Mesoderm
development(2.23.10.00.00) > Hematopoesis(2.23.10.06.00)
        MOLECULAR FUNCTIONS
                Signaling molecule(1.02.00.00.00) > Cytokine(1.02.01.00.00) > Other
cytokine(1.02.01.99.00)

MOUSE GENE ONTOLOGY
                No Gene Ontology
MOUSE PROTEIN DOMAINS (INTERPRO SIGNATURES)
        No Domain Hit


MOUSE mRNA SEQUENCE : mR27-014.2 (Seq ID No: 34)
AGCTCGGCCAGGGGAAAGTGAAAGTTTGCCTCGGTGCTCTCGGTGTCGCTGCGGCTCTCTGCATCCCAGGACAGCGGCGTGGCCCTC
GACCGGGGCGCGGGCTCTTCAGCCACTAGCGAGCAAGGGAGCGAGCGAACCAGGGCGGCCAACACGCCGTGCCGGGACCCAGCTGCC
CGTATGACCGCGCGGGGCGCCGCGGGGCGCTGCCCTTCTTCGACATGGCTGGGCTCCCGGCTGCTGCTGGTCTGTCTCCTCATGAGC
AGGAGTATTGCCAAGGAGGTGTCAGAACACTGTAGCCACATGATTGGGAATGGACACCTGAAGGTCCTGCAGCAGTTGATCGACAGT
CAAATGGAGACTTCATGCCAGATTGCCTTTGAATTTGTAGACCAGGAACAGCTGGATGATCCTGTTTGCTACCTAAAGAAGGCCTTT
TTTCTGGTACAAGACATAATAGATGAGACCATGCGCTTTAAAGACAACACCCCAATGCTAACGCCACCGAGAGGCTCCAGGAACTC
TCCAATAACCTGAACAGCTGCTTCACCAAGGACTATGAGGAGCAGAACAAGGCCTGTGTCCGAACTTTCCATGAGACTCCTCTCCAG
CTGCTGGAGAAGATCAAGAACTTCTTTAATGAAACAAAGAATCTCCTTGAAAAGGACTGGAACATTTTTACCAAGAACTGCAACAAC
AGCTTTGCTAAGTGCTCTAGCCGAGATGTGGTGACCAAGCCTGATTGCAACTGCCTGTACCCTAAAGCCACCCCTAGCAGTGACCCG
GCCTCTGCCTCCCCTCACCAGCCCCCCGCCCCCTCCATGGCCCCTCTGGCTGGCTTGGCTTGGGATGATTCTCAGAGGACAGAGGCG
AGCTCCCTCTTGCCCAGTGAGCTTCCCCTTCGCATAGAGGACCCAGGCAGTGCCAAGCAGCGACCACCCAGGAGTACCTGCCAGACC
CTCGAGTCAACAGAGCAACCAAACCATGGGGACAGACTCACTGAGGACTCACAACCTCATCCTTCTGCGGGGGGGCCCGTCCCTGGG
GTGGAAGACATTCTTGAATCTTCACTGGGCACTAACTGGGTCCTAGAAGAAGCTTCTGGAGAGGCTAGTGAGGGATTTTTGACCCAG
GAAGCAAAGTTTTCCCCCTCCACGCCTGTAGGGGGGCAGCATCCAGGCAGAGACTGACAGACCCAGGGCCCTCTCAGCATCTCCATTC
CCTAAATCAACAGAGGACCAAAAGCCAGTGGATATAACAGACAGGCCGTTGACAGAGGTGAACCCTATGAGACCCATTGGCCAGACA
CAGAATAATACTCCTGAAGAACTGATGGTACATCCACGCTGCGTGAAGACCACCAGGAGCCAGGCTCTCCCCATATTGCGACACCG
AATCCCCAACGAGTCAGCAACTCAGCCACCCCCGTTGCTCAGTTACTGCTTCCCAAAAGCCACTCTTGGGGCATTGTGCTGCCCCTT
GGGGAGCTTGAGGGCAAGAGAAGTACCAGGGATCGAAGGAGCCCCGCAGAGCTGGAAGGAGGATCAGCAAGTGAGGGGGCAGCCAGG
CCTGTGGCCCGTTTTAATTCCATTCCTTTGACTGACACAGGCCATGTGGAGCAGCATGAGGGATCCTCTGACCCCCAGATCCCTGAG
TCTGTCTTCCACCTGCTGGTGCCGGGCATCATCCTAGTCTTGCTGACTGTTGGGGGCCTCCTGTTCTACAAGTGGAAGTGGAGGAGC
CATCGAGACCCTCAGACATTGGATTCTTCTCTGTGGGGCGACCAGAGGACAGCTCCTGACCCCAGGATGAGGACAGACAGCAGGTGGAACTG
CCAGTATAGAAAGGATTCTATGACCCCTCACCATCCTGGACACACTCGTTTGTCAATGTCCCTCTGAAAATGTGGCGCCCAGCCCTG
GACACAGTACTCCAGATGTTGTCTGACCAGCTCAGAGTACAGTGGGACGGTTGTCTTCCTTGATCTGGACAGTACTCTTCTACTCGT
GCAGATTAAGATCACATTAGTTTTAACAGCTGCATCATATATTGTCATATGTTGAGCTTGTAGTCTATTAAAAACCCCAGTTCTATT
TCCTGTG


MOUSE PROTEIN SEQUENCE : mP27-014.2 (Seq ID No: 35)
MTARGAAGRCPSSTWLGSRLLLVCLLMSRSIAKEVSEHCSHMIGNGHLKVLQQLIDSQMETSCQIAFEFVDQEQLDDPVCYLKKAFF
LVQDIIDETMRFKDNTPNANATERLQELSNNLNSCFTKDYEEQNKACVRTFHETPLQLLEKIKNFFNETKNLLEKDWNIFTKNCNNS
FAKCSSRDVVTKPDCNCLYPKATPSSDPASASPHQPPAPSMAPLAGLAWDDSQRTEGSSLLPSELPLRIEDPGSAKQRPPRSTCQTL
ESTEQPNHGDRLTEDSQPHPSAGGPVPGVEDILESSLGTNWVLEEASGEASEGFLTQEAKFSPSTPVGGSIQAETDRPRALSASPFP
KSTEDQKPVDITDRPLTEVNPMRPIGQTQNNTPEKTDGTSTLREDHQEPGSPHIATPNPQRVSNSATPVAQLLLPKSHSWGIVLPLG
ELEGKRSTRDRRSPAELEGGSASEGAARPVARFNSIPLTDTGHVEQHEGSSDPQIPESVFHLLVPGIILVLLTVGGLLFYKWKWRSH
RDPQTLDSSVGRPEDSSLTQDEDRQVELPV*


MOUSE PANTHER CLASSIFICATIONS
        FAMILY (SUBFAMILY)
                MACROPHAGE COLONY STIMULATING FACTOR-1(MACROPHAGE COLONY STIMULATING
FACTOR-1)
        BIOLOGICAL PROCESS
                Immunity and defense(2.16.00.00.00) > Macrophage-mediated
immunity(2.16.05.00.00)
                Immunity and defense(2.16.00.00.00) > Granulocyte-mediated
immunity(2.16.04.00.00)
                Developmental processes(2.23.00.00.00) > Mesoderm
development(2.23.10.00.00) > Hematopoesis(2.23.10.06.00)
        MOLECULAR FUNCTIONS
                Signaling molecule(1.02.00.00.00) > Cytokine(1.02.01.00.00) > Other
cytokine(1.02.01.99.00)


MOUSE GENE ONTOLOGY
                No Gene Ontology
MOUSE PROTEIN DOMAINS (INTERPRO SIGNATURES)
        No Domain Hit


MOUSE mRNA SEQUENCE : mR27-014.3 (Seq ID No: 36)
GAAAGTTTGCCTCGGTGCTCTCGGTGTCGCTGCGGCTCTCTGCATCCCAGGACAGCGGCGTGGCCCTCGACCGGGGCGCGGGCTCTT
CAGCCACTAGCGAGCAAGGGAGCGAGCGAACCAGGGCGGCCAACACGCCGTGCCGGGACCCAGCTGCCCGTATGACCGCGCGGGGCG
CCGCGGGGCGCTGCCCTTCTTCGTGGATCCAGGTTCAAATCCCAGAATCTAGATGATGGTTCACAACCATTCATACCTCCAGGTCCA
GGAGATTCCAAGAAGGAAGCACATAGAGTGTGTGCATGCAAGCAGGCAAAATATTCATAAACACAAAACAAAAACAACATGGCTGGG
CTCCCGGCTGCTGCTGGTCTGTCTCCTCATGAGCAGGAGTATTGCCAAGGAGGTGTCAGAACACTGTAGCCACATGATTGGGAATGG
ACACCTGAAGGTCCTGCAGCAGTTGATCGACAGTCAAATGGAGACTTCATGCCAGATTGCCTTTGAATTGTAGACCAGGAACAGCT
GGATGATCCTGTTTGCTACCTAAAGAAGGCCTTTTTTTCTGGTACAAGACATAATAGATGAGACCATGCGCTTTAAAGACAACACCCC
CAATGCTAACGCCACCGAGAGGCTCCAGGAACTCTCCAATAACCTGAACAGCTGCTTCACCAAGGACTATGAGGAGCAGAACAAGGC
CTGTGTCCGAACTTTCCATGAGACTCCTCTCCAGCTGCTGGAGAAGATCAAGAACTTCTTTAATGAAACAAAGAATCTCCTTGAAAA
GGACTGGAACATTTTTACCAAGAACTGCAACAACAGCTTTGCTAAGTGCTCTAGCCGAGGCCATGTGGAGCAGCATGAGGGATCCTC
TGACCCCCAGATCCCTGAGTCTGTCTTCCACCTGCTGGTGCCGGGCATCATCCTAGTCTTGCTGACTGTTGGGGGCCTCCTGTTCTA
CAAGTGGAAGTGGAGGAGCCATCGAGACCCTCAGACATTGGATTCTTCTGTGGGGCGACCAGAGGACAGCTCCCTGACCCAGGATGA
GGACAGACAGGTGGAACTGCCAGTATAGAAAGGATTCTATGCTGGGCACACAGGACTATCTCTTTATGGAAGGAGACATATGGGAAC
ATCCACCACTACCCTCTCCTACCATCTTCCTGGGAATGTGGCCTACCACTACCAGAGCTCCTGCCTACCAAGACTGGATGAAAGAAG

```
CAGCTTTGATGGGGTCTTTCCATCCTCACCCTTAGACTCTCAACCAAAGAGAAAGGGCTGGAGGATGCCCCCCACATACTGCCACTA
TTTATTGTGGGCCCTGGAGGCTCCCTGCATTGGAGGAAGGGCAGCTCAGCAGCTCAGGACCCTTTCCCTTAGGGGCTGCTTCCTCCC
CTCAAAACCAGAACCTGGCAAGGGACTCACTAGCCTGGATGGCCCATGGGAGACCAGGACAGATGAGAAGGAGCAGAAGAGCCCTGT
GCCCAGAAGACCCAACTGGTGCCAAGGAATCCCAGCATGGACAGGCAGGGACCTGTTTCCCAAGAAGAGAGCCTGATATTCAAAGGG
TGGGACAGCATCTGC


MOUSE PROTEIN SEQUENCE : mP27-014.3 (Seq ID No: 37)
MMVHNHSYLQVQEIPRRKHIECVHASRQNIHKHKTKTTWLGSRLLLVCLLMSRSIAKEVSEHCSHMIGNGHLKVLQQLIDSQMETSC
QIAFEFVDQEQLDDPVCYLKKAFFLVQDIIDETMRFKDNTPNANATERLQELSNNLNSCFTKDYEEQNKACVRTFHETPLQLLEKIK
NFFNETKNLLEKDWNIFTKNCNNSFAKCSSRGHVEQHEGSSDPQIPESVFHLLVPGIILVLLTVGGLLFYKWKWRSHRDPQTLDSSV
GRPEDSSLTQDEDRQVELPV*


MOUSE PANTHER CLASSIFICATIONS·
        FAMILY (SUBFAMILY)
            MACROPHAGE COLONY STIMULATING FACTOR-1(MACROPHAGE COLONY STIMULATING
FACTOR-1)
        BIOLOGICAL PROCESS
            Immunity and defense(2.16.00.00.00) > Macrophage-mediated
immunity(2.16.05.00.00)
            Immunity and defense(2.16.00.00.00) > Granulocyte-mediated
immunity(2.16.04.00.00)
            ·  Developmental processes(2.23.00.00.00) > Mesoderm
development(2.23.10.00.00) > Hematopoesis(2.23.10.06.00)
        MOLECULAR FUNCTIONS
            Signaling molecule(1.02.00.00.00) > Cytokine(1.02.01.00.00) > Other
cytokine(1.02.01.99.00)


MOUSE GENE ONTOLOGY
            No Gene Ontology
MOUSE PROTEIN DOMAINS (INTERPRO SIGNATURES)
        No Domain Hit


MOUSE mRNA SEQUENCE : mR27-014.4 (Seq ID No: 38)
TGAAAGTTTGCCTCGGTGCTCTCGGTGTCGCTGCGGCTCTCTGCATCCCAGGACAGCGGCGTGGCCCTCGACCGGGGCGCGGGCTCT
TCAGCCACTAGCGAGCAAGGGAGCGAGCGAACCAGGGCGGCCAACACGCCGTGCCGGGACCCAGCTGCCCGTATGACCGCGCGGGGC
GCCGCGGGGCGCTGCCCTTCTTCGACATGGCTGGGCTCCCGGCTGCTGCTGGTCGTGTCTCCTCATGAGCAGGAGTATTGCCAAGGAG
GTGTCAGAACACTGTAGCCACATGATTGGGAATGGACACCTGAAGGTCCTGCAGCAGTTGATCGACAGTCAAATGGAGACTTCATGC
CAGATTGCCTTTGAATTTGTAGACCAGGAACAGCTGGATGATCCTGTTTGCTACCTAAAGAAGGCCTTTTTTCTGGTACAAGACATA
ATAGATGAGACCATGCGCTTTAAAGACAACACCCCCAATGCTAACGCCACCGAGAGGCTCCAGGAACTCTCCAATAACCTGAACAGC
TGCTTCACCAAGGACTATGAGGAGCAGAACAAGGCCTGTGTCCGAACTTTCCATGAGACTCCTCTCCAGCTGCTGGAGAAGATCAAG
AACTTCTTTAATGAAACAAAGAATCTCCTTGAAAAGGACTGGAACATTTTTACCAAGAACTGCAACAACAGCTTTGCTAAGTGCTCT
AGCCGAGATGTGGTGACCAAGCCTGATTGCAACTGCCTGTACCTAAAGCCACCCCTAGCAGTGACCCGGCCTCTGCCTCCCCTCAC
CAGCCCCCCGCCCCCTCCATGGCCCCTCTGGCTGGCTTGGCTTGGGATGATTCTCAGAGGACAGAGGGCAGCTCCCTCTTGCCCAGT
GAGCTTCCCCTTCGCATAGAGGACCCAGGCAGTGCCAAGCAGCGACCACCCAGGAGTACCTGCCAGACCCTCGAGTCAACAGAGCAA
CCAAACCATGGGGACAGACTCACTGAGGACTCACAACCTCATCCTTCTGCGGGGGGGGCCCGTCCCTGGGGTGGAAGACATTCTTGAA
TCTTCACTGGGCACTAACTGGGTCCTAGAAGAAGCTTCTGGAGAGGCTAGTGAGGGATTTTTGACCCAGGAAGCAAAGTTTTCCCCC
TCCACGCCTGTAGGGGGCAGCATCCAGGCAGAGACTGACAGACCCAGGGCCCTCTCAGCATCTCCATTCCCTAAATCAACAGAGGAC
CAAAAGCCAGTGGATATAACAGACAGGCCGTTGACAGAGGTGAACCCTATGAGACCCATTGGCCAGACACAGAATAATACTCCTGAG
AAGACTGATGGTACATCCACGCTGCGTGAAGACCACCAGGAGCCAGGCTCTCCCCATATTGCGACACCGAATCCCCAACGAGTCAGC
AACTCAGCCACCCCCGTTGCTCAGTTACTGCTTCCCAAAAGCCACTCTTGGGGCATTGTGCTGCCCCTTGGGGAGCTTGAGGGCAAG
AGAAGTACCAGGGATCGAAGGAGCCCCGCAGAGCTGGAAGGAGGATCAGCAAGTGAGGGGGCAGCCAGGCCTGTGGCCCGTTTTAAT
TCCATTCCTTTGACTGACACAGGCCATGTGGAGCAGCATGAGGATCGTCTAGACCCCCAGATCCCTGAGTCTGTCTTCCACCTGCTG
GTGCCGGGCATCATCCTAGTCTTGCTGACTGTTGGGGGCGTCCTCCTGTTCTACAAGTGGAAGTGGAGGAGCCATCGAGACCCTCAGACA
TTGGATTCTTCTGTGGGGCGACCAGAGGACAGCTCCCTGACCCAGGATGAGGACAGACAGGTGGAACTGCCAGTATAGAAAGGATTC
TATGCTGGGCACACAGGACTATCTCTTTATGGAAGGAGACATATGGGAACATCCACCACTACCCTCTCCTACCATCTTCCTGGGAAT
GTGGCCTACCACTACCAGAGCTCCTGCCTACCAAGACTGGATGAAAGAAGCAGCTTTGATGGGGTCTTTCCATCCTCACCCTTAGAC
TCTCAACCAAAGAGAAAGGGCTGGAGGATGCCCCCCACATACTGCCACTATTTATTGTGGGCCCTGGAGGCTCCCTGCATTGGAGGA
AGGGCAGCTCAGCAGCTCAGGACCCTTTCCCTTAGGGGCTGCTTCCTCCCCTCAAAACCAGAACCTGGCAAGGGACTCACTAGCCTG
GATGGCCCATGGGAGACCAGGACAGATGAGAAGGAGCAGAAGAGCCCTGTGCCCAGAAGACCCAACTGGTGCCAAGGAATCCCAGCA
TGGACAGGCAGGGACCTGTTTCCCAAGAAGAGAGCCTGATATTCAAAGGGTGGGACAGCATCTGCCCGACTTCCCGTAAAGGCATAA
AGGCACGCAGCCCAAAAGACGGGAAGAGGAGGCCTTTGGCTGCTTGTGTTGACAGCTTAAAGGGGTCTACACCCTCAACTTGCTTAA
GTGCCCTCTGCTGATAGCCAGGAAGGAGGGAGACCAGCCCTGCCCCTCAGGACCTGACCTGGCTCATGATGCCAAGAGGAAGACAGA
GCTCTAGCCTCGTCTTCTCCTGCCCACAGCCCCTGCCAGAGTTCTTTTGCCCAGCAGAGGCACCCCTCATGAAGGAAGCCATTGCAC
TGTGAATACTGAACCTGCCTGCTGAACAGCCTGTCCCATCCATCCCTATGAGTGACCATCCGTCCGAATGTTCTCCCACTTCCTTCA
GCCTCTCCTCGGCTTCTTGCACTGAGCTGGCCTCACGTGTTGACTGAGGGGAGCCCCTGAGCCCCAACCTTCCCCTGCCTCAGCCTTT
GATTGTCCAGGGTGAAGCTGTGGGAGAACCGCCTGGGCTACCAGTCAGAGCTGGTCTTTGGGCTGTGTTCCTTGCCCAGGTTTCTGC
ATCTTGCACTTTGACATTCCCAGGAGGGAAGTGACTAGTGGAAGGGAGGAAGGGGACAGAGACAAAAGGCCACAGGCAGAGC
TATGAATGAGAATGGGTCTTGAAAATATGTGTGCACCCCTAAGCTTGAAATTGATCTCTATACTCTAGCCCCTCAGCCAGCCTCCTT
CCTGTTGTCTGAAACCTGGAGCTAAGCAGGTTGTCCTGTCACAAGCTCTGGGGACTGAGCTCCATGCTCCAACCCCACCCTCTTCTG
ACCTTTGTTCTCCAGACCTGACCCAGGTAGGCAAGGGTACCCTCCCAGTCTCACCTACCATACTGTGCCATCTCTAGCCAAGCAAGC
CAGGTTTAGAGAAGGGTCAAAAAAAAAAAAAAGGGTTGTTTACTTCCAACTTGTTCTGATGCCCTCTGTTTCCCAGGCCAGGCTTGT
CTGTGGTGACCTGGGCATGGGTGACAGGGCTCTCATTTGCCCCTTGGTCTCTTTATGCTGCTGAGTCCCCCTTTCCTGCCCTCCCTG
GCTACTGGGTCAATAATCTTTCAGGCCATGAATCTGGGAGGAGAGTGGTCTGTAAGCTCCATCAGCCCTGTCCTGAGACAGCAGGGG
GGAAGGACACTGGAGACTTTCTTGTGGGGCTTACTTAGCCTTCTGGTTACAGACTATTTCCATGCTAGAAAATACATATTTTAAAAT
AGAAGGAAAAACACAGAAACAAAACAAAACAAGGCATTCTCTACCCCTCCACCTTAAACATATATTATTAAAGACAGAAGAGAAAT
CCAACCCATTGCAAGAAGCTCTTTGTGGGTGCCTGGTTACATCGGAGCAGGGGAGCCTCAAATCCACCTTTGGAGCCGCCCCTGTGT
```

```
GCATTAGGAACCCTTCTCTCCTCTGAGAAAGCTCAGAGGGAGCACTGCCTCACAAACTGTGAGACTGCGTTTTTTATACTTGGAAGT
GGTGAATTATTTTATATAAGGTCATTTAAATATCTATTTAAAAAAATAGGAAGCTGCTTTTATATTTAATAATAAAAGAAGTGCACAA
GCTGC
```

MOUSE PROTEIN SEQUENCE : mP27-014.4 (Seq ID No: 39)

```
MTARGAAGRCPSSTWLGSRLLLVCLLMSRSIAKEVSEHCSHMIGNGHLKVLQQLIDSQMETSCQIAFEFVDQEQLDDPVCYLKKAFF
LVQDIIDETMRFKDNTPNANATERLQELSNNLNSCFTKDYEEQNKACVRTFHETPLQLLEKIKNFFNETKNLLEKDWNIFTKNCNNS
FAKCSSRDVVTKPDCNCLYPKATPSSDPASASPHQPPAPSMAPLAGLAWDDSQRTEGSSLLPSELPLRIEDPGSAKQRPPRSTCQTL
ESTEQPNHGDRLTEDSQPHPSAGGPVPGVEDILESSLGTNWVLEEASGEASEGFLTQEAKFSPSTPVGGSIQAETDRPRALSASPFP
KSTEDQKPVDITDRPLTEVNPMRPIGQTQNNTPEKTDGTSTLREDHQEPGSPHIATPNPQRVSNSATPVAQLLLPKSHSWGIVLPLG
ELEGKRSTRDRRSPAELEGGSASEGAARPVARFNSIPLTDTGHVEQHEGSSDPQIPESVFHLLVPGIILVLLTVGGLLFYKWKWRSH
RDPQTLDSSVGRPEDSSLTQDEDRQVELPV*
```

MOUSE PANTHER CLASSIFICATIONS
       FAMILY (SUBFAMILY)
              MACROPHAGE COLONY STIMULATING FACTOR-1(MACROPHAGE COLONY STIMULATING
FACTOR-1)
       BIOLOGICAL PROCESS
              Immunity and defense(2.16.00.00.00) > Macrophage-mediated
immunity(2.16.05.00.00)
              Immunity and defense(2.16.00.00.00) > Granulocyte-mediated
immunity(2.16.04.00.00)
              Developmental processes(2.23.00.00.00) > Mesoderm
development(2.23.10.00.00) > Hematopoesis(2.23.10.06.00)
       MOLECULAR FUNCTIONS
              Signaling molecule(1.02.00.00.00) > Cytokine(1.02.01.00.00) > Other
cytokine(1.02.01.99.00)

MOUSE GENE ONTOLOGY
          No Gene Ontology
MOUSE PROTEIN DOMAINS (INTERPRO SIGNATURES)
       No Domain Hit

HUMAN GENOMIC SEQUENCE : hD27-014 (Seq ID No: 40)

```
TTGGGACGATCATAGAGCGCTAGCACTGAATCAGCCTGGAGAGCGCGGAAGGAAAGGGTCGGTCCGCAGAGGGCGCGGGGAAGGCAG
GGTGGGGACGCGGTGGAGCCCGCGCTCGTTTGCTGAAGGCTTGGAAGTGCAGCGCAGAAGACAGAGGGTGACTAGGAAGACGCGCGA
GCGGGGCTGGCCGGCCGGCGGGTGGGGGGAGGGGAGGGGCGGGGGAAGGCGGCTGAGTGGGCCTCTGGGAGTGTGTGTGTCTGTGTCAGTG
TGTGTGTGTGTGTGTGTATGTGTGTCTGGCGCCTGGCCAGGGTGATTTCCCATAAACCACATGCCCCCCAGTCCTCTCTTAAAAG
GCTGTGCCGAGGGCTGGCCAGTGAGGCTCGGCCCGGGGAAAGTGAAAGTTTGCCTGGGTCCTCTCGGCGCCAGAGCCGCTCTCCGCA
TCCCAGGACAGCGGTGCGGCCCTCGGCCGGGGCGCCCACTCCGCAGCAGCCAGCGAGCGAGCGAGCGAGCGAGGGCGGCCGACGCGC
CCGGCCGGGACCCAGCTGCCCGTATGACCGCGCCGGGCGCCGCCGGGCGCTGCCCTCCCACGGTAAGCGACGGCCGCGGCGCTGGGC
CCGGGACGGGCTGGGGCGGGGCGGGTCGGCGGCCAGGCGGCCGGGAGCGGCCCCTCGGAGCCGACAGCCTGGGCGCGCCGGCTGCACTG
GCCCGGCGTTCCCGCCGCGGACGAACCGCCTTTGCGCACGGACTGGGCCCTGGCGCCAGGGCGGAGGCCGGTGGCGGCCCTGCAGCT
GCACCGGGACTGCCAGGCTTCCCTGGGAAACGGAGGGCTGCTCACCCCATTGCACGGAGGGGAACACTGAGCCACCTGCAGGCAGGA
GCCCCCGCTCAGCCAGGGTGCCCTGCCTCTCTCCCTANNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNGCTGC
TTGGGGCTAGCTCAGAATCTCTTTTTCTTGCAAATTCTGGTTTCATCGGTTGGGGAGATTCTGGGCTTTGTGGTGGTTTATGGTGGT
TTGCAGTGCTTGAGGAAAATGGAAAGAGGGTTTGGTGTTGCAGAGCTGTGTCCAGGGGCAGGGTCTGCTCCTGACTGCGTAAGGGAC
GCCCTGCCCCAACTGTGGCGGTAGCAGATGCAGCCATAGAAAGTGAGGGTGGCACTGTTCATGACCAGTCAGTGGGCTCATAATAGA
TTTGCTTCTGAGGAGTCCTGGAAGAAAATGAGAGGGGAAACCTGGGGGGAAAAGAGCAGAAAATTATTTGGGGGTAGAGGAGGAGCGG
GCTGCAGGTGAGGTGAGAGAGAGAAAGGGAAGAGTGGGTCCACAGAAAGAAAGTGCCTCACTGAGAGAGGAGGAGATAGAGTTGAGAGGG
GAAGGGAGAAGGGTTAGCCATAGGCAGAAAGCAAAGCTTCGGGGCTGAAGGGGGAGACCATGAGGAAAGAAGAGAAAGACAAGGGGGC
TGTGTTTAGACAGGGGGAAGTCCAAGGGCCCGGTTCTGCCAGGTCTCTCCAGAGGCCTTGTTGAGGGGGAAGATGAAATGGAATAGGA
TAGGTGGCAGAGGAGGCTGGACAGCTGGACTGAGGTGTCCTAGCATCTCTGAGCCTGCAGCAGAGATTCCTTCTGAGGGCTGCTCCC
CAGGCAGAAGCCAGGATGAGTTCCGGGGTGGGGAGGAAGAGGCAGTCAGGAGGAGAGCCTGCCCAGCCAGATGCAAGCCAGAGTGTTAACCCCC
CACTGTCTGTCCAGATCCTCCTGAGGCAGAATGGAGGCCCCAGGTCCAGGGGTATCCCCTTTGGCCAGCTTCGAAGTCAGCCTCTCA
TGTAGTGGCTGCACTTTCCCCACACTGATGCCTCAAATGTCATCAAAGCCAGACAGCCCTTGGGGTTAAGCATTGCTCCCACCTTGC
TCTCCTCCAGGGAGGTTGTCTTCCTGAGCGGTCCCTTTCATCCATACCATTCACTCCAGACCTTGAGTGGCAGCCAGTCTGGGGTAG
ATACACCGTCTAGTGGCCCAGTTTCCTCCAATCATTCCTGAACCTAAGGTTTCTGAGTACCTGGGCCATTATTCCCACCCAGGGCAA
CCTCCCAACAGAGCCTGAAGTCCCCCCAGCCCCCGTGACTGCCGTCCCCTCTTCCCACACTTTCCCTCCCTCAGATCAGCCAGGAT
CCTCACAGTCCCTGTGCTGAGTGGTATGAAACTATCCTTTGGCTCTTCCTTCCATGGCTCCCACCCCTCTCCTGCCATCACCTTCTC
CCCATCTTTCAAGGCCTCACTGAGGTCCCTGCTCTCTGCAGTCTCCCTGGTCACCCAGCCAGAGCTGCCTTCTCCTCCCCAGAAGAG
CTGGCAAGCATTTGGGCTTTTGGTGATATGTAGATTAGCACATTGTTGTGGGCTGTTTCATAGCCTCGTTGTGTTTTTCCAGTGAGC
AACTTATTTGCAGTATTTGTCTCAACATCTGGATTATTGAGGCAAAACCCAGAGTCCAGGATAGTGTCAACCTTCACCCTACCCTGC
CAGCATTTCCCTGAATCTGGGGGCATCAGCCGCCCAGGAGGCTTGGGAGGCTCTCAGGAGGTGACCTCAAGCTGGAGAAAGAAAGCCCA
AGAGTCTGTCTGCTTTTGGCTGAAGAAGGAGAGACCTAGGGCCAGGGGTAGGGATGAGGAGACCCCAGTGAAGAGAAGGAAGAGAGG
AAGGAAGGGGAAAGACAGGGCATCAGGAGCCAGTCCCATCACTCTGGGGCTGAGCAACTACTGGGCAGGTAGGTGCTGGGAGAGAA
GAGGGGTCTCAGAAAACTTCTCTGCCATGATAAGAGGTGGCTGGCTGGAGGCAAAGCCTGATGGTGCTCAAATAGGCAGGTGCCAGG
CAAGGGATGGCCCATTACTGGCACTGGTGCCTCTGATTAATCAGCTGGGCTCCTTTTGAAACACACACCCAGGGCTCCAAGATGTCT
CTGGCAAGGGATGAGTTCCAGTGAAACTGAGTTTGATCCCACAGTTTTTCCAGTGGCAGAGCCAGATCTGCATGGAACCAGGTGGGA
GGAAATGCTGGGCCCAGGGGAGCTGTCTCTGGCTTGCCATCTGAAAGCCGGTGCATGAACATGAACTTGAGGGCAGTTCAAAAAGTG
CTTGGCTCAGTGTTGATGTTGCAGAGACAGAAAAAAAATGGGTAGATGCAAGTCCTGCTCTCGAGTTTCCTGTGTCATTAAAATTTAG
GCTGGTAGGCACACCATCTGTGTCAGTATTAGTTCAATGTATAGTTAGGTTTGGCTGGCTTAATCTGGGAAGGCTTCTTGTAGAAGG
```

EP 2 204 376 A2

```
TGACATCTGGGTTGTTTTCATGGGAGAACAAGGTTGTGCTGTGGCTGCTAGAAATCCTGGGAAAGCTGGATTTGAGGGATGCTGTAT
CCTGAGGTAAGGGGCAGAGCCTGTAGCATTGTAGATATGAGGCCTTTGTTTTTCTGCGTTGAGCAGGGCATGGGGATAACTGGGGAG
AGTGAGACCTGGGGAGAAATGACACCCTCTCTGTCACAGACATGGCTGGGCTCCCTGCTGTTGTTGGTCTGTCTCCTGGCGAGCAGG
AGTATCACCGAGGAGGTGTCGGAGTACTGTAGCCACATGATTGGGAGTGGACACCTGCAGTCTCTGCAGCGGCTGGTGAGTGTGTGG
CCATGCTGTATTCTACCTTCTCCCCACTGGGGAAATGAAGGCAGGAGCCAGGGAGCAGGTCAAAGAGAGCAGTTGCAGGCAGGAAAA
TAGGGCAGTGCGGGACATTGCTTGTGGTTCCCACTAGCTCCACCAGTGATACCCTTCACTAACCTTCCCAAAGTTAGGACCTCTGGT
CTCCCCAGCTCGAAGCCCTCTCTGACTGCCCTGCCAGGCAGTGGATGCTGTGGGCTTCCAGCTGCTTGCCTGGGTTAGTGATTGCCCA
GGAACATCAACCACTGATTCTGAAAAGGCTTCTGAGGTCTGCTGTCCCTCAGTGGGATGCCTCCTCTGGGAAGCTAGCCCAGGCGGC
CTGCTGTGTCCAGATGTTGCATCTAGCTCCTGGACTCTCATATGTGGCGCCAGTCTGGCATCAGAGCCCCACCCAGATTTGGGAGGGG
AAGCGCTTGCCTAACTCCCAGCCTTCCACACTCACTTCCCTGGCTCTTGCCCATACCCTGAGGCTGGGATGGCCTGCTCATCTGGGC
CATGAAGGAGCCTGTATCTCATTCTAATCAGACCCATACGTGGTCCAGCCAGGCAGAATCAGGTTGCTGTCATGGCTGCTGTCCTGC
TCAGTGGGAATTTACCATTCCTCTGGCCAGCCAGGCAGGGTGGGGCATCACTGGCTAACGCCAGCTCCAGGGCCCTGAGCTTGGGGC
CTGTGGGCTGTGCCTTCCGCCTCTCTTGCCCCAGCACTACTTCTCCTGTATGTAGTTGCTGTAGCAATCCAAGGTAGACCAAGAGCC
CCAGCATTCTCTGAGGCTTAAAATCCAGAACTGCTGCTCTGGGGCTAAAGAGGGCTTTAAGGGCATCCAGCTCCAACCCCTACAGGT
GTTCAATCCCCAGAGCTTGTCCAGCCTCTGCTTGAATTCCTACCATGACAGGGTGCTCACTGCCTCCAGGGAAGATTATATCCTATA
TTCTTCTATAGACATCTCTTCCTTAAAACAAATGGGCATTTGTCAGATTTCGTGGGGTGGTAGAAGGAAAGAAGAGACTTCTTGTTC
TTCTACAGCCTTCCCCTGGGCATCTGGAAGGCACTGATCTTCTCCTAGACTTGACTCTGTCTTTCCACGTGTGGTTGGCAGGGATGA
AGTTCAAACCCCAATCCACTCAGAAGCTAAGGTCCCCGTTTTGAAGAAGGCTGAAGGCTGAGTTGAGCTGTAGGTTACCCTGCAATC
GTTGGCCTGCTCTCTCTTACAGATTGACAGTCAGATGGAGACCTCGTGCCCAAATTACATTTGAGTTTGTAGACCAGGAACAGTTGGT
GAGTGATGGCTTTTTACAAAATCCATGCACCAGCCTGCATGCAACTCCCAGGGTGGGGTGTGTGGGGGAGCATGAAAGCGGCAGAAT
GCCTACTGCTGGAAAGGGTGAGAGTGTGAGGATCCATGGGTGCTCAACTCTGGGGTGCCAGGATCCAGGGCTCAAGTCCCCTGCCAT
TCCCTTCTCCTGGCCTGATACATAACAAGCGCTCACTAGGTACCAAGCACTTTGCTAATGTAGTTCTGACAGTACCACTATGTGGTA
CACAAATACAGTTTATTATTCCACAGAGAGGTGAAAGGAGCATAGTAGGTAGGTGCTAGAGGCCTGATTTGAATCCAGGAAGGTTGGC
TGTAGGGCTTGAGGCAAATCAATACTTCTTCCAGGTCACAAGCTTTGCTTAAGGATGTCACTTCTCTTCACCAGGCCAACCAGCAGG
CAGGTAGGCAGTCCAGCCCTTCCTGGACTTGCCCCACACCCAACCAGCCACCAAGTGCTGCTGGTTCTGCCTCTCAGCATCTCTGGG
TTCTGTCTCCTCCATCCCCACCTTCACCCCTCAATCAGTTGCTTGGACAATTTCAAGAGCCTCCTAACTGGTGTCTCCCTGCTTTCT
AGTTCACTCCTCATCTTACCACTGGGGGAAATTTTCTGAAACGCCAACCTGCTGCCTTTACCTCCTTCTCCCAAAAACATCAGGGCC
CCCATCACTCCAGAAGAAAGCCAAGGTTGTCTAGTCTGGGTTACACCAGGTCTAGCCCATTCTCCACCACAAGACCTCTATACCACC
TCCCCATTCCCTGCTTCTTCCTGCCTCCCATCTTTGCACAAGCTCTTTCCAATGACTGTCATGCCTTTCCACCTGCCCCCCCAATCAT
TATTCCACATCTATTCATCTTTATGCCTCATGCAAAACTGCCTTGATACCCAGAAGTTGCCCCTGTTTCTGAGCTTCCACACACCTG
ACCCATATGCACCTCTGTTAGGACTAGAGATCACCTCATGGTCCAAAGAATAGCCTCAGTATTTTATGCACTCACTCGCTTGCTCTA
GCACTCATGCCTGAACTAGCATTTGCTAGGAACCTACTGGACTAGATGCTGGGGGTAGAGAGCTAAAGCAGAGTGGGTCTTCAAGGC
AACTACCATCTCTTTAGGGAGACAAAACACAGAGGTTATTCTTTGGACCATGAGATAGTCTTTCAGCACCCCTATGTCCTGGAGGATT
TGAAGGGGCCATATGGTAAAAGATATTTGTGAAAGCAAGACAAGAGGAAGACAGAAGAAAGGCAGGGGAAGGTTCCCTGGGGACCCCA
CCTTCCCTATATACATCCATCGTGATTAAAGCCTCATATTCCAGAAACCCAGAGAACTCTCTAATCAGAATCCACATTTCCTTCACT
CTAGCCCAGTCTCCAGTGGCGCTAGATCCCAAAGAGAGAGAAAGACAGGCAAGCTGAGGGCGGCATTCTCCTAGTTGCTGGCATGGT
GTGGTCCCTCCCCTGGGGGAAGGGGGAGAGCAAGCTGCAACCCTCCATCTGCCTGGGGTTGGGGGTTCCCAGGCCACAATGGCCAGG
CCATAAGCTCCAGGTTCCTGTTTTTCAGAAAGATCCAGTGTGTGTACCTTAAGAAGGCATTTCTCCTGGTACAAGACATAATGGAGGA
CACCATGCGCTTCAGAGATAACACCCCCAATGCCATCGCCATTGTGCAGCTGCAGGAACTCTCTTTGAGGCTGAAGAGCTGCTTCAC
CAAGGATTATGAAGAGCATGACAAGGTAGGAAGCCCTGAGGCCTGGAGCACTGAGTGAGGGCAGAGGGTGGCTGTGGAGGCGCCGCT
CTATCCACAGGCACAGAGTACATTCGCTCACCTCGCCTCACGCCATTGCTTGCTCACTCTCATTTATTTGTCATCCCACCAACCTTT
ACCAGGCAGGAGGCAGGAGGGATCATGCTGGGCCCTGACAATGTCATCATGAGCAACAAATACATGGTCCCACCCCATGCAGCTTAT
AGTCAAGTGAGCTTGGCAGCTGTTAAACAAACCATGACACCAGTAAATAGAATTACAAACTATAAGGACTGTTCATTCCAACAGCAA
GCAGTTATTGTGTGTCTGTTGTGTACCAGACACTGTTCTAGACACCAGGAAACAGCTGTGATGAAAACAGACAAAAATCCCCACATT
CCTGGAACTTACATTTGGGGGTGATGGTAGTGGTGGTGAGACAGTTAACAAAATATGTAAGTAAAATATACAACATGTAGCATGGCA
ATAACAGCACTGGAGAAGAGTAAACCAGGGATGAGACGACCCGAAGGAAGTGGAGGAGTGAGCTGGGAACAAGAACCTGAAGGGCTC
AAGGAGAGCAACAGGGAACTCAGACTTCCATGGAGTTGGGGAAGCAGAGACAAGAAAGGCTTCTGAGTAAGCAAATATTGATTTGAG
CTCCAAAGATGAGTAAGAAATTGAAAGCAGTAAGGCTAGAAGAACATTCTGGAACAGGAAAAGCTATGCACAAAGCCCTAAGGTAAAC
AAACAGGAAAGTGCACATTCAGGGACCCAAAGGGTTATCAGCATGGCCAGAGCATGGGAAGTGAAGGAGGGGGAGGAGAGAAGTCAG
GAGCCAGATCACATGTGGTTTTAGAGATGTGGGATTTTGTCTTAAGGGTGAGGGAAGCTGTTGTGAACAGGCATGTGGCTTAATCTC
AGGTTATAAGACTGACCTGGTTGCTATGTGGAGGATAGACCACAGGGGGCAGGAATGGAGGCTGGGAGACCAGCAGAAGGTGTGAGT
CCAGGCTAGACACATTGGCGGCTTGGACCAAGGTGGGACAGTGAAGGTGGAGGCAAGTGGACTGGACATGTTCCCGTGGGTTTAGGG
GCAGAATCAACAAGACTTGGTACGTGCAGTGGATGTGATGTGATGAAAAAGCCAAGGATGGTTTTTGTCTTGAGCACCT
GGATATTCCAATGATGCTGAAACGGGGATGATCCATGGGCAGAGCAGGTTTGAGGGAAGCAGTTCAGTCTGGGGCACAGCAGACGT
GAGGTGCTCATGAAATGGTGAGTGGAGACGTTGAGGAGGCAGCTAGGTGTGCGGGTCTTGAGTGTGACCAGCAATGCACAGATCTAT
GAGGCATGGGGGTTCGGCTTATCTGTGAAGCCATGGGACTGGACAGCACTGCCACTGTGAGAAGAGAAGCAGGCCCAGGAACCCAGC
ATTTGGAGGGCAAGCAGAGGATGGGGGACCTGGCAAGGGGCAGCCTGAAGGTGGGAGGAAAAGAGGGAGAGTGCAGAGTCGAGGCAC
TGCATTCCAAGATGGATAAGGGATGGAAATTCATAAAAAAGCGTTGAGAAATCAAGCAAGGTGAGGGCTAAAATTGTCCATTGGATT
TGGCAACGTGTCACACATGCTGGATCAGGGTGAACAGAAAGAGAGGAAGTAGAGACACCACTGTGGAAGAACTTGGATCCGAATGGG
GGCAGGAAAAGAGATGTAGCTGGAGGGAGTGTGTAATTGAGGGAGGACTTTTTGTTCTGTTTTTGTTGTCGTTGTTTTGAGACAGGG
TCTCATTCTGTCACCCAAGCTGAAGTTCAGCGGCGCAATCACGACTCACTGCAGCCCAACCTCCCAGGCTCAATTGATCCTCTGACT
TCAGTCTCCTGAGTAGCTGGGACAACAGGCACACCCCACTATGCCCAGCTATTTTTTGTAGAGATAGGGTTTCGCAATGTTGCCCAG
GCTGGTCTCAAACTCCCAGGCTCAAGCAATCTGTCTGCCTTGGCCTCCCAGAGTTGCTGGGATTACAAATGTGAGTTACTGTGCCTGG
CCAGGAAAACATTTTTAATAAGAGAGGTATTAAATCTGCGGTTTTCCAACATTAAGATTCCTAAGAATCACTTGGGGAATGTGTTGA
AAATGCATATTCCTAGGACACACCCCTAGAGAGTCTGATACACTGGGTTTAAGAGTGGCTCCAGGGCCGGGCGCGGTGGTTCATGCC
TCTAATCCTGGCACTTTGGGAGGCCGAGACAGTGGATCACCTGAGGTCAGGAGTTCAAGACCAGCCTGCCCAACAGGGCGAAACCCC
CCATCTTACTAAAAATACAAAAATTAGCAGCTGGTGGTGGCGCATGCCTGTAATCCAAGCTACTCGGGAGGCTGAGGCAGTAGAATC
GCTTGACCCAGGGTGGCGGAGGTTGCAGTGAGCTGAGATCGAGATCCATCTCAAAATAAATAAATAAATAAATAAATAAATAAATAAATA
AATAAATAAATAAATAAAAGACTGAACCCAGGAATCTGCATCTTAAGCAGCTGTCGCACTGATGATTATGACCCCCTTGGATATTCA
CTTGTAGACATGTGTGAATGCTGAGGGGCAGGCTCCAAGAGAGAGGGTGGGTGAAGCTGGAGGAGCTAGCTCCCAGCCAAGTCTCTGG
AGAAGACAGTGGGAGGGATTCAGAGCTCCTGAGGGAGAGTGAGAGTGAGACTTCATCTCAAAAAAAAAAAAAATGTTTACCAGATAG
ACGTACGAATGAGAAGACTGGGCTCAGCTCCATCACTTACCAGCTAGGTGGCAGTGACCAAGTAAACTGCCGGAGTCTCACTCTCTC
CCAGGCTGGAGTGCAGTAGTGCCATCTCAGCTCACTGCAACCTTTGCCTCCCGGGTTCAAGCAATTCTCCTGCCTCAGCCTCCCGAG
TAGCTGGGATTACAGGCACGCACCACCATGCCCAGCTAATTTTTGTCTTTTTAGTAGAGATTACAGGCACGTGCCACCATGCCTGGC
TAATTTTTGTCTTTTTAGTAAAGACAAGGTTTCACCATGTTGGTCAGGCTGATCTCGAACTCCTGACCTCGTGATCAGCCTGCCTCG
GCCTCCCAACATGCTGGGATTACAGGTGTGAGCCACCGCACCCGGCCTATCTGGTAAACATTTCTTAAGGGCTGACTGCATAGGCCA
```

130

EP 2 204 376 A2

```
GGGAGATGCGGCGATGCCTTCAAGGACTTCACAGCCTAGAAAAGCCCATGTTTCTTCCCTGTGAAGCTCAGTTTAAGGAAAAACTTG
GTATCTGTGATTCAGTGGCATTTAGGCCCTGGATGAAGCTGTGAGCATGCATGGCATGGCCTTGTGTGAAAGGAGAGGTGGGCCCGG
GAGTGAACCCTGAGGGATGGCAGCATTTGGATATCAGGAGGAGAAGGGGAGCTTGGTAAGGAGACAGAAAGAGCAGCTTGAAAGGTG
GGAGAAGACCCAGGATGATGCAGCTTCTCAGAGCTCCAGTGGGTGTGGGAAGCCGGGGCCCCTTGAGCAGCACAGAAGACAGGGCTG
GGACCTCCTCAGCCCTCCCTCTAAGACACACCAATGCCAGCAAAGGACCCATATGCCTGGCGGCTGCCCCGTTCCCTTCTCCACACC
ACAGCTCAATGATTTTTCTCAAACTCAACTCTTACCATGCCACTGCCCACCCCTAACACCCCTTCAGATGTTTTCCCTTTGCTCTGT
GGACAAAACTAAAAGTTCTGGTCTTGGCCCACGAGGCCCTCAAGACCTGGCCCTTGCTAACCTCTCTCCATGTGCGTCTCTTGCCAC
GCTCCTCCTAGATGGTAGTGCTGCAGCCACACCAGCCTTCTTTTAGTCTTTGGGCTTTTCATGCATTTTCTTGCCTCAGGACTTTTG
CACGGTCTGTTCTTGCAGCCTGGACAGCTCTCTGGGATCCCATCTCTTTCCTGGTTTCTCTTGCTCATCTTTTAGTTGATTCCTTGG
GGAAATTCTCCCTGAACACTTGTTTAGTTCCCCTTGTTGCACACCATCGGATGGCCCAGTACTTATTCATAGGACTCATTGCACTTG
TAACAATGTGTTTAGTCCCTGCTGGATTGCTGGACTCAGTGTCCAACTCAGCCAGGAACCACCTCTCTCTTGTTCGTTGCATGATTC
CCAGGTAATTACCATATGCCTGGCACATAAACATATGTTGAATGGGTCTGTGAATAAATGACATATCTCATAACCTCAGGACTCATT
CTGCTGCAAACCAGGGGAAAGGGGAGCAAGGAAACAAAAGGGAAAAAGAAGACAGATGTGAGAAAGGCCAAGGGAATTTGACAAATA
AGATGGAGACATGGGGCCAATGGTCATGCTCACAAAAGGGGGCCCTGATCTCCTTCCAGGCCTGCGTCCGAACTTTCTATGAGACAC
CTCTCCAGTTGCTGGAGAAGGTCAAGAATGTCTTTAATGAAACAAAGAATCTCCTTGACAAGGACTGGAATATTTTCAGCAAGAACT
GCAACAACAGCTTTGCTGAATGCTCCAGCCAAGGTAAGCATGGCAGGGGCCAGCAAGTGTGTGGGGGTGGTAGCATATGGAATGGGG
ATTGGGAGGTGAGATGTGAAGCTGGGGGGACCCCTGGGGAGGCAGCCTGGCTGCTACTCGTGTTCCCGTGTGCATGAATGTGCTTGT
TCTGTGTATATATGTGGCTTCACGTACCTCTGGTCCTGGGCACATGTCTTTTCTGACATGTGTGTGCATGCACACCTACATATGTCT
GCATGTGGCAGCGTCAAGAAGGATCATGCTGGTGCCTTGAGATTAGGGAGTGGAAATGGGAGCCTGTCTTGGGAGCCCTGTAGGTAT
GGAAGCTTTCCCCACTGGCTCTGCAGGCTGGCTGAACGTGTGGGGGGTTCTGTGGAGACAGCTGACACCCATCTGGGAGTCAGGGCC
CTTGCTCCAGGAGCTCTGCTGCGAATCACCATGATACCCTGGCTCCAGAAGTCCCCAGGATGCCTTGGGTGTTTCCTCAAGGGACAA
GGCTGGATTTGAAGTCTGAGCATCATTGGAACTCCCAGGGCTGGCTCAGCTGCATACATGTGTACACTGCGTGTCCTCGTCTCTGCG
TCTCGTGCCACACGTGCCCTCCTACATTCAACCTTGCTTCCCAAGGCAGGGTCTAGGGCCCTGCAACCTGCCAGTGGCCAAGTCCAG
TCTCGTTGCTTCAATCCCAAGTCCTCAAGCCTTGGCTGCATTCTAGCCCCAGCCTGTTCCTGCCTGTGGCTGTGGCTGTGGCTGTGG
CTGTGTGGCTCCTGGCTATGCATGAAACCAGTGTCTCTGGGGCTTGAAGTTGTCTTGTATTGGTCTGGAAGGCAACCGTTCAGCCTC
CTGACCTGACTGCTGCTCACCCCTAGCTTGGCCTGTGGCCAGTGGGAACCCCTGCATGGGCTGTTCTCTTATCTTCCTTCTCCCCAA
CCCCCAGTGTGTGCATCTCAACCCTATTCTTTGTCACTGCTCATGAGACCCTGCATACGGCACCTTCCCTGTGTCATGAGCACCCAC
TCTAGTCCCATCCTCTTCTCAGCCCCAGGGCTGAGCTAGGAGATGAGGGCCCCCCAGACTCACATTCCCCTCTTGCCCCGCTCTGGG
AAAGCTGTGCTGGAGGCTAGTGACTCTATCTCCTCCCCATCTTTCTCTCTCCTTCTCTCTGTGGTTCTTTCAGATGTGGTGACCAAG
CCTGATTGCAACTGCCTGTACCCCAAAGCCATCCCTAGCAGTGACCCGGCCTCTGTCTCCCCTCATCAGCCCCTCGCCCCCTCCATG
GCCCCTGTGGCTGGCTTGACCTGGGAGGACTCTGAGGGAACTGAGGGCAGCTCCCTCTTGCCTGGTGAGCAGCCCCTGCACACAGTG
GATCCAGGCAGTGCCAAGCAGCGGCCACCCAGGCAGCACCTGCCAGAGCTTTGAGCCGCCAGGACCCCAGTTGTCAAGGACAGCACC
ATCGGTGGCTCACCACAGCCTCGCCCCTCTGTCGGGGCCTTCAACCCCGGGATGGAGGATATTCTTGACTCTGCAATGGGCACTAAT
TGGGTCCCAGAAGAAGCCTCTGGAGAGGCCAGTGAGATTCCCGTACCCCAAGGGACAGAGCTTTCCCCCTCCAGGCCAGGAGGGGGC
AGCATGCAGACAGAGCCCGCCAGACCCAGCAACTTCCTCTCAGCATCTTCTCCACTCCCTGCATCAGCAAAGGGCCAACAGCCGGCA
GATGTAACTGGTACCGCCTTGCCCAGGGTGGGCCCCGTGAGGCCCACTGGCCAGGACTGGAATCACACCCCCCAGAAGACAGACCAT
CCATCTGCCCTGCTCAGAGACCCCCCGGAGCCAGGCTCTCCCAGGATCTCATCACTGCGCCCCCAGGGCCTCAGCAACCCCTCCACC
CTCTCTGCTCAGCCACAGCTTTCCAGAAGCCACTCCTCGGGCACGTGCTGCCCCTTGGGGAGCTGGAGGGCAGGAGGGAGCACCAGG
GATCGGAGGGAGCCCCGCAGAGCCAGAAGGAGGACCAGCAAGTGAAGGGGCAGCCAGGCCCCTGCCCCGTTTTAACTCCGTTCCTTTG
ACTGACACAGGCCATGAGAGGCAGTCCGAGGGATCCTCCAGCCCGCAGCTCCAGGAGTCTGTCTTCCACCTGCTGGTGCCCAGTGTC
ATCCTGGTCTTGCTGGCCGTCGGAGGCCTCTTGTTCTACAGGTGGAGGCGGCGGGTGAGTAGATCCCCATGAGGAAGAAGAGCACGT
CCCTTAGGGCAGGGGCAGAGCCTGGCGGGGGTGCAGGTGGGGGGACAGCTTGGGTGCCTGGCTGAGTTCTTCAGACACAGAGAGATAG
GGGCTGGCTCAGCACAGAGGAGTGAGAGGTGGAAATGGAGGATTACTTCGAGAATTGGGAAGGTTCAAGCTATAAGGTCAATGGGAAG
GGACTGGAGCAGAAGGGAGCGGGAGAGAATATTCATGGGCTGACAGCAGGATGATATCCAGACGGGCAGGGAGCTGGAGGAGGAGAG
CAATGCTGTGTGAGCGTGTCAGGGCAGGCGTACACTGGAAGCTCTGCAGAGCCTGGGGCAGGAGGACCCATAGGGAACAGCCTGCAA
GGGTGTGGGGAGAGGAGAGGGGACACCATCAGCAGAGAGACCTCACAAATCTCCCTTGGGCCTCTGGCTGATCCCCACTTTTGGGAA
GTTGGGAGGACTCTTGCAACTCTCTCATAAATACCTGGGGCAGCCCTTACTGGGGATGGGCACCGCCCCCCACACTCCCCCAGCT
CCTCAGTGAGATGCTCTTTCCTGTCCTCAGAGCCATCAAGAGCCTCAGAGAGCGGATTCTCCCTTGGAGCAACCAGAGGGCAGGTGA
GAGCTTGAGGTGGGGCTCTGGGAGGCACTGGGGGCCTGGCTTGGGATCTGTTTCCCCTCTTCGTGGTGGTGGGGCTCTCCTGCTTGC
TCTGAGGAAATGGAGCTGCAGAACAGGATGGGGGAGAGAAGAAGGGCCTCTGTCCTTTCCCAGATATCTGGGCTTTTTCCTGATTTC
TCCGTAGAGTTAGACAGTTCTGGGTCTTTTCAATCTGAGAGGGCCTAGAGCATGTCTGGGGCTTAGGGGTAAACTTACGGAGTCCCC
TTATTCCCCTGCTCCTGCTGATGTCTAATGCCAGCCCTGTGCTCTGCAGCCCCCTGACTCAGGATGACAGACAGGTGGAACTG
CCAGTGTAGAGGGAAATTCTAAGGTAAGATTCTGACTTTGATCTCCACTCCTAAAACTTACCTATACCCTTTTCCAGTCACGTTCACC
TGTTGGTGACACCAATTCCCCTGAGGCCCCCACACTGACTCCCATGCCTCTCTCCAGTTCCCTTTCCATACATCGCCAGCCAGGTCC
AGCCACCCTCTCCTTTCCAGGTCCTCAGCTATCCCCCTGCCCCTCCATTCCATCCACCCCCGCTGAGGCCAGGGACTATTACCTCTG
CATTGGGCTTCTAGTGTTGCTGCCACCTGTGACCTCAGAGCAACTAAACCCCCCTTTTTCAAATGTGGCCTTTATAAGGTCTTTACC
CTCCTGGAGAATCTTCAGTAGCTCTGTGTGGCAGCCACTGCAAGACCCTATATTCTTGGCTTGGCTGCCAAGCCCCTTCGACAGCAGC
TGCTTGCCAGATCGCCACCCCCGCCACCAGTTAACCCTCCTCCCCAGCTGAGGTCAGGTTCTGCTTCCTTGCTTTTAGGATCATGTTGA
CATAGAACCTGAGTCTTGGAAGGGCTCTCAGGGGTCATTTGGCCCTGGTTCCCACCCCAAAGGGACTGCCCCTCTCCATGTCCCCAA
CAGGGAGCCAGCCAGCTGGCTGTCACACACTCCCAGATCCAGCCCAGCTCCCTGCCTAGCACAGCCCATGCCATCTTCCAGTCAGA
CTGGGAGAAACGTCTTCCTCATGCGAGGCAAAACTGGCTTCCCTGCCCCCTCCATACTGCTCCTCCCTCCTGTTCTCATGAAAACCC
TCAGATATTTCAAGTCAGGTATCCATGTCCCCTTTGAGGAATTCCTCAAGCTAAGTATGGCCAAGTCCCTCAAACGTTCCTTATATGA
TATGGTTTTCAGACCCCTCACCATCCTGGACACACTCGTTTGTCAATGTCCCTCTGAAAATGTGACGCCCAGCCCCGGACACAGTAC
TCCAGATGTTGTCTGACCAGCTCAGAGAGTACAGTGGGACTGTTACCTTCCTTGATATGGACAGTATTCTTCTATTTGTGCAGAT
TAAGATTGCATTAGTTTTTTTCTTAACAACTGCATCATACTGTTGTCATATGTTGAGCCTGTGGTCTATTAAAACCCCTAGTTCCAT
TTCCCATAAACTTCTGTCAAGCCAGACCATCTCTACCCTGTACTTGGACAACTTAACTTTTTTAACCAAAGTGCAGTTTATGTTCAC
CTTTGTTAAAGCCACCTTGTTGGTTTCTGCCCATCACCTGAACCTACTGAAGTTGTGTGAAATCCTAATTCTGTCATCTCCGTAGCC
CTCCCAGTTGTGTGCCTCCTGCACATTGATGAGTGCCTGCTGTCTTGTCATGTTGTTGATGTAGCTGTGACCCTATTGTTCCTC
ACCCCTGCCCCCCGCCAACCCCAGCTGGCCCACCTCTTCCCCCTCCCACCCAAGCCCACAGCCAGCCCATCAGGAAGCCTTCCTGGC
TTCTCCACAACCTTCTGACTGCTCTTTTCAGTCATGCCCCTCCTGCTCTTTTGTATTTGGCTAATAGTATATCAATTTGCACTTATT
TGGATGTTGTTTTCATATTTTTTAAAATCTTATTTTATATGTATTGTACATTCCACATCCCTAAGATTGTAAGCCCCTTGAGGGCAG
GGACTGTCTTCCACTTTTTTTTGTGTTTCTTTTTCATCCTGGGTTCTACTAAGAGAGGGCCTCTCCACATTTGCCAAACATGCAAT
AAATGTTAACTGACTGGCTTCAGGGCTGACTCACGGACTGACCCTGGCCAGCTGTCTCTAACTGACCAGCCTCTAGCTGACCAGTTA
ACCAGCTAACTGACTGACTCTGACTGACTGGCTGACCAGCTGGCTGGGTGAATGACTAACCAACCAACTATCTTGGTAACTGCCTAA
CTGACCAGCTGTTGCTTGGGGAGCCCATTGAGCAACTGACTAGCTGATTGCCTGACTAATTGGCTGGTTGGCTGACTAGCTGGGGTG
ACCCAGTCAAGTTTCCAGTCACATCCTTTACCAAGTGCAAGCCAGTGCCGCAGGGCTCTCCCATGTGTGGAGTGACAGGTCTGGGCC
```

131

```
CCTTCTAGAGAGATAGGGAATTTGGCAAATGCTCAAAACCAGGGGAACCTATTTAGACAAGAACCAGCAGTAACAACATATCTGGAA
TCCTGGGGTCTACCTTGTCAGTGGGGTGGGCTAAGATGGGTGAGTGGGTCAGCTGGGGCACTGGCAAGACTGTGAAATGACCCAGGC
CTCCTCAGAACTGGTTGCTGGTGGCTCAGTGCCACAACACCCTTCCCACTGGTCGTCTGTCCCCAAACCCCTCGCCGTGGTCTTGGC
CCTCTGTCCCACCCCTCTACACGCCCACCCTCCTTTCCCCACTTGCTTATTCTCAGGCCCGTCCCAAGCTTTCCTCTTCCCCCTCAA
GATTTCACTTGGAGAGGGAATCCAGGGCAGGGGCTACATCCAGATCCCACTTGGCCCGGCAGGTCCAGCCCTAAGGTGGTCTCCAGG
AGGCAGAGACTGGCCTTGGAGAGATTGAGTGCCAGCCCCTAGAGTGACTGCTGCTGTGATTTGAGAAGTGAGAGAGAGAGAAACATAG
AGGCAGAGACTGGCCTTGGAGAGATTGAGTGCCAGCCCCTAGAGTGACTGCTGCTGTGATTTGAGAAGTGAGAGAGAGAGAAACATAG
AGGCAGAGACTCAGAGATAGGGACTCAGAGCCCACAGAAAATGCAGCACCAGCACCTCAGCCCTAAGCAGCTAGAAGATGGGGCAGCAG
GCTGGAACTCGCTCACTGGGTCCTTCTCCCCTCCTGGGCCCTAGCTGGGCCTGTGGCCTGGCTGTACCCAGCATGGCTCATGTCCAG
TTTATGGTTCCCCTTCTTTCCCTCTAGAGCATGGGAATGGGCAGGGAGCCAGAGGCTGTGGTTCCATCTTCCCTCCTTACCCAGTAG
GGCCTATGGATGTTAGGGAGGGAGGCTCCACACAAGGTCCAAAGTCAAACTGTTGGATGGGGTCCAGGGGATTTCTGCTCTGGGAAC
AAAGCGTCCTGAGGATGCTGGGCAAGAGGACAAGGATGGGGGTGGAAGGCGAGAGGACAAGGCCCTGACTCCCATGGATGATGCGGG
AGGCAAGGCCCTCGGCCCATCCAGGAGGGGCTGGGTTGCTTAGGGGTCGCGTGCCAAATGACAGTGTGGGAATTTCCTTGTCCTGGC
ACTGCCTCTAGACTTTTCCTCTCTGGCCCAAGTTGTTCCTCTTCAGCTAGCTCTGCCCTACTGTCTTGAACATCATATGTGATGCCC
CTCTGATGAAATGAAAGATTCTTCTTTGCTAGTGTTAGCAGGAATCACCCTTTGTTCACTGGACCAAGGCCAATGAGATGACCTCGC
CTGAGGAGGGGTCCAAAGACATTCAAGTGCCCGCTTCGCTCCTTGTCAGAGCCCTCTGCTTGAGCAGTAACCCCTCTTCCCAGCCCT
GCCCAGACCTCTCCCGGGCCCCCGTTATCTTCCACTGAGGCCCTGATTCCCCAGGAGGAGGTAGGAGGTGAGGCCAGTTGGGCCAGAG
GCCTGGGCTGGTGCCAGAGAAAGCTGCCTGGTCTCTCTGGTGGTGTCTGGAAAGGCCAGGGAAGGGAGGAAAGGAAGTCCTGCCTGCA
GCACCGCCAGCAGCCCACAGGAATGCCACACCTCCCAATCCATGTCCTCAACGACCCTCCCCGCATTCCCACTCTGCTGACTAGCCT
TTCCTTCTCTCCCACAGCTGGACGCACAGAACAGTCTCTTCCGTGGGAGGAGACATTATGGGGCGTCCACCACCACCCCTCCCTGGCC
ATCCTCCTGGAATGTGGTCTGCCCTCCACCAGAGCTCCTGCCTGCCAGGACTGGACCAGAGCAGCCAGGCTGGGGCCCCTCTGTCTC
AACCCGCAGACCCTTGACTGAATGAGAGAGGCCAGAGGATGCTCCCCATGCTGCCACTATTTATTGTGAGCCCTGGAGGCTCCCATG
TGCTTGAGGAAGGCTGGTGAGCCCGGCTCAGGACCCTCCTCCTTTCCCTCAGGGGCTGCACCCTCCTCTCACTCCCTTCCATGCCGGAACC
AGGCCAGGGACCCACCGGCCTGTGGTTTGTGGGAAAGCAGGGTGGACGCTGAGGAGTGAAAGAACCCTGCACCCAGAGGGCCTGCCT
GGTGCCAAGGTATCCCAGCCTGGACAGGCATGGACCTGTCTCCAGAGAGAGGAGCCTGAAGTTCGTGGGGCGGGACAGCGTCGGCCT
GATTTCCCGTAAAGGTGTGCAGCCTGAGAGACGGGAAGAGGAGGCCTCTGGACCTGCTGGTCTGCACTGACAGCCTGAAGGGTCTAC
ACCCTCGGCTCACCTAAGTGCCCTGTGCTGGTTGCCAGGCGCAGAGGGGAGGCCAGCCCTGCCCTCAGGACCTGCCTGACCTGCCAG
TGATGCCAAGAGGGGATCAAGCACTGGCCTCTGCCCCTCCTCCTTCCAGCACCTGCCAGAGCTTCTCCAGGAGGCCAAGCAGCAGAGCC
TCCCCTCATGAAGGAAGCCATTGCACTGTGAACACTGTACCTGCCTGCTGAACAGCCTGCCCCGTCCATCCATGAGCCAGCATCCG
TCCGTCCTCCACTCTCCAGCCTCTCCCCAGCCTCCTGCACTGAGCTGGCCTCACCAGTCGACTGAGGGGAGCCCCTCAGCCCTGACCT
TCTCCTGACCTGGCCTTTGACTCCCCGGAGTGGAGTGGGGTGGGAGAACCTCCTGGGCCGCCAGCCAGAGCCGGTCTTTAGGCTGTG
TTGTTCGCCCAGGTTTCTGCATCTTGCACTTTGACATTCCCAAGAGGGAAGGGACTAGTGGGAGAGAGCAAGGGAGGGGAGGGCACA
GACAGAGAGGCTACAGGGCGAGCTCTGACTGAAGATGGGCCTTTGAAATATAGGTATGCACCTGAGGTTGGGGGAGGGTCTGCACTC
CCAAACCCCAGCGCAGTGTCCTTTCCCTGCTGCCGACAGGAACCTGGGGCTGAGCAGGTTATCCCTGTCAGGAGCCCTGGACTGGGC
TGCATCTCAGCCCCACCTGCATGGTATCCAGCTCCCATCCACTTCTCACCCTTCTTTCCTCCTGACCTTGGTCAGCAGTGATGACCT
CCAACTCTCACCCACCCCCTCTACCATCACCTCTAACCAGGCAAGCCAGGGTGGGAGAGCAATCAGGAGAGCCAGGCCTCAGCTTCC
AATGCCTGGAGGGCCTCCACTTTGTGGCCAGCCTGTGGTGGTGGCTCTGAGGCCTAGGCAACGAGCGACAGGGCTGCCAGTTGCCCC
TGGGTTCCTTTGTGCTGCTGTGTGCCTCCTCTCCGCCCTTTGTCCTCCGCTAAGAGACCCTGCCCTACCTGGCCGCTGGGCCC
CGTGACTTTCCCTTCCTGCCCAGGAAAGTGAGGGTCGGCTGGCCCCACCTTCCCTGTCCTGATGCCGACAGCTTAGGGAAGGGCAGT
GAACTTGCATATGGGGCTTAGCCTTCTAGTCACAGCCTCTATATTTGATGCTAGAAAACACATATTTTTAAATGGAAGAAAAATAAA
AAGGCATTCCCCCTTCATCCCCCTACCTTAAACATATAATATTTTAAAGGTCAAAAAAGCAATCCAACCCACTGCAGAAGCTCTTTT
TGAGCACTTGGTGGCATCAGAGCAGGAGGAGCCCCAGAGCCACCTCTGGTGTCCCCCCAGGCTACCTGCTCAGGAACCCCTTCTGTT
CTCTGAGAAGTCAAGAGAGGACATTGGCTCACGCACTGTGAGATTTTGTTTTTATACTTGGAAGTGGTGAATTATTTTATATAAAGT
CATTTAAATATCTATTTAAAAGATAGGAAGCTGCTTATATATTTAATAATAAAAGAAGTGCACAAGCTGCCATGTGAGT
```

HUMAN mRNA SEQUENCE : hR27-014.1 (Seq ID No: 41)

```
CCTGGGTCCTCTCGGCGCCAGAGCCGCTCTCCGCATCCCAGGACAGCGGTGCGGCCCTCGGCCGGGGCGCCCACTCCGCAGCAGCCA
GCGAGCGACTGCCCGTATGACCGCGCCGGGCGCCGCCGGGGCGCTGCCCTCCCACGACATGGCTGGGCTCCCTGCTGTTGTTGGTCTG
TCTCCTGGCGAGCAGGAGTATCACCGAGGAGGTGTCGGAGTACTGTAGCCACATGATTGGGAGTGGACACCTGCAGTCTCTGCAGCG
GCTGATTGACAGTCAGATGGAGACCTCGTGCCAAATTACATTTGAGTTTGTAGACCAGGAACAGTTGAAAGATCCAGTGTGCTACCT
TAAGAAGGCATTTCTCCTGGTACAAGACATAATGGAGGACACCATGCGCTTCAGAGATAACACCCCCAATGCCATCGCCATTGTGCA
GCTGCAGGAACTCTCTTTGAGGCTGAAGAGCTGCTTCACCAAGGATTATGAAGAGCATGACAAGGCCTGCGTCCGAACTTTCTATGA
GACACCTCTCAGTTGCTGGAGAAGGTCAAGAAGTGTCTTTAATGAAACAAAGAAATCTCCTTGACAAGGACTGGAATATTTTCAGCAG
GAACTGCAACAACAGCTTTGCTGAATGCTCCAGCCAAGATGTGGTGACCAAGCCTGATTGCAACTGCCTGTACCCCAAAGCCATCCC
TAGCAGTGACCCGGCCTCTGTCTCCCCTCATCAGCCCCTCGCCCCCTCCATGGCCCCTGTGGCTGGCTTGACCTGGGAGGACTCTGA
GGGAACTGAGGGCAGCTCCCTCTTGCCTGGTGAGCAGCCCCTGCACACAGTGGATCCAGGCAGTGCCAAGCAGCGGCCACCCAGGAG
CACCTGCCAGAGCTTTGAGCCGCCAGAGACCCCAGTTGTCAAGGACAGCACCATCGGTGGCTCACCACAGCCTCGCCCCTCTGTCGG
GGCCTTCAACCCCGGGATGGAGGATATTCTTGACTCTGCAATGGGCACTAATTGGGTCCCAGAAGAAGCCTCTGGAGAGGGCCAGTGA
GATTCCCGTACCCCAAGGGACAGAGCTTTCCCCCTCCAGGCCAGGAGGGGCAGCATGCAGACAGAGACCGCCAGACCCAGCAACTT
CCTCTCAGCATCTTCTCCACTCCCTGCATCAGCAAAGGGCCAACAGCCGGCAGATGTAACTGGTACCGCCTTGCCCAGGGTGGGCCC
CGTGAGGCCCACTGGCCAGGACTGGAATCACACCCCCCAGAAGACAGACCATCCATCTGCCCTGCTCAGAGACCCCCCGGAGCCAGG
CTCTCCCAGGATCTCATCACTGCGCCCCCAGGGCCTCAGCAACCCCTCCACCCTCTCTGCTCAGCCACAGCTTTCCAGAAGCCACTC
CTCGGGCAGCGTGCTGCCCCTTGGGGGAGCTGGAGGGCAGGAGGAGCACCAGGGATCGGAGGGAGCCCCGCAGAGCCAGAAGGAGGACC
AGCAAGTGAAGGGGCAGCCAGGCCCCTGCCCCGTTTTAACTCCGTTCCTTTGACTGACACAGGCCATGAGAGGCAGTCCGAGGGATC
CTCCAGCCCGCAGCTCCAGGAGTCTGTCTTCCACCTGCTGGTGCCCAGTGTCATCCTGGTCTTGCTGGCCGTCGGAGGCCTCTTGTT
CTACAGGTGGAGGCGGCGGAGCCATCAAGAGCCTCAGAGAGCGGATTCTCCCTTGGAGCAACCAGAGGGCAGCCCCCTGACTCAGGA
TGACAGACAGGTGGAACTGCCAGTGTAGAGGGGAATTCTAAGCTGGACGCACAGAACAGTCTCTCCGTGGGAGGAGACATTATGGGGC
GTCCACCACCACCCCTCCCTGGCCATCCTCCTGGAATGTGGTCTGCCCTCCACCAGAGCTCCTGCCAGGACTGGACCAGAGCA
GCCAGGCTGGGGCCCCTCTGTCTCAACCCGCAGACCCTTGACTGAATGAGAGAGGCCAGAGGATGCTCCCCATGCTGCCACTATTTA
TTGTGAGCCCTGGAGGCTCCCATGTGCTTGAGGAAGGCTGGTGAGCCCGGCTCAGGACCCTCTTCCCTCAGGGGCTGCACCCTCCTC
TCACTCCCTTCCATGCCGGAACCCAGGCCAGGGACCCACCGGCCTGTGGTTTGTGGGAAAGCAGGGTGGACGCTGAGGAGTGAAAGA
ACCCTGCACCCAGAGGGCCTGCCTGGTGCCAAGGTATCCCAGCCTGGACAGGCATGGACCTGTCTCCAGAGAGAGGAGCCTGAAGTT
CGTGGGGCGGGACAGCGTCGGCCTGATTTCCCGTAAAGGTGTGCAGCCTGAGAGACGGGAAGAGGAGGCCTCTGGACCTGCTGGTCT
GCACTGACAGCCTGAAGGGTCTACACCCTCGGCTCACCTAAGTGCCCTGTGCTGGTTGCCAGGCGCAGAGGGGAGGCCAGCCCTGCC
CTCAGGACCTGCCTGACCTGCCAGTGATGCCAAGAGGGGGATCAAGCACTGGCCTCTGCCCCTCCTCCTTCCAGCACCTGCCAGAGC
TTCTCCAGGAGGCCAAGCAGAGGCTCCCCTCATGAAGGAAGCCATTGCACTGTGAACACTGTACCTGCCTGCTGAACAGCCTGCCCC
CGTCCATCCATGAGCCAGCATCCGTCCGTCCTCCACTCTCCAGCCTCTCCCCAGCCTCCTGCACTGAGCTGGCCTCACCAGTCGACT
```

# EP 2 204 376 A2

```
GAGGGAGCCCCTCAGCCCTGACCTTCTCCTGACCTGGCCTTTGACTCCCCGGAGTGGAGTGGGGTGGGAGAACCTCCTGGGCCGCCA
GCCAGAGCCGGTCTTTAGGCTGTGTTGTTCGCCCAGGTTTCTGCATCTTGCACTTTGACATTCCCAAGAGGGAAGGGACTAGTGGGA
GAGAGCAAGGGAGGGGAGGGCACAGACAGAGAGGCTACAGGGCGAGCTCTGACTGAAGATGGGCCTTTGAAATATAGGTATGCACCT
GAGGTTGGGGGAGGGTCTGCACTCCCAAACCCCAGCGCAGTGTCCTTTCCCTGCTGCCGACAGGAACCTGGGGCTGAGCAGGTTATC
CCTGTCAGGAGCCCTGGACTGGGCTGCATCTCAGCCCCACCTGCATGGTATCCAGCTCCCATCCACTTCTCACCCTTCTTTCCTCCT
GACCTTGGTCAGCAGTGATGACCTCCAACTCTCACCCACCCCCTCTACCATCACCTCTAACCAGGCAAGCCAGGGTGGGAGAGCAAT
CAGGAGAGCCAGGCCTCAGCTTCCAATGCCTGGAGGGCCTCCACTTTGTGGCCAGCCTGTGGTGGTGGCCTGAGGCCTAGGCAACG
AGCGACAGGGCTGCCAGTTGCCCCTGGGTTCCTTTGTGCTGCTGTGTGCCTCCTCTCCTGCCGCCCTTTGTCCTCCGCTAAGAGACC
CTGCCCTACCTGGCCGCTGGGCCCCGTGACTTTCCCTTCCTGCCCAGGAAAGTGAGGGTCGGCTGGCCCCACCTTCCCTGTCCTGAT
GCCGACAGCTTAGGGAAGGGCAGTGAACTTGCATATGGGGCTTAGCCTTCTAGTCACAGCCTCTATATTTGATGCTAGAAAACACAT
ATTTTTAAATGGAAGAAAAATAAAAAGGCATTCCCCCTTCATCCCCCTACCTTAAACATATAATAATTTTAAAGGTCAAAAAAGCAAT
CCAACCCACTGCAGAAGCTCTTTTTGAGCACTTGGTGGCATCAGAGCAGGAGGAGCCCCAGAGCCACCTCTGGTGTCCCCCCAGGCT
ACCTGCTCAGGAACCCCTTCTGTTCTCTGAGAAGTCAAGAGAGGACATTGGCTCACGCACTGTGAGATTTTGTTTTTATACTTGGAA
GTGGTGAATTATTTTATATAAAGTCATTTAAATATCTATTTAAAAGATAGGAAGCTGCTTATATATTTAATAATAAAAGAAGTGCAC
AAGCTGCCATGTGAGT


HUMAN PROTEIN SEQUENCE : hP27-014.1 (Seq ID No: 42)
MTAPGAAGRCPPTTWLGSLLLLVCLLASRSITEEVSEYCSHMIGSGHLQSLQRLIDSQMETSCQITFEFVDQEQLKDPVCYLKKAFL
LVQDIMEDTMRFRDNTPNAIAIVQLQELSLRLKSCPTKDYEEHDKACVRTFYETPLQLLEKVKNVFNETKNLLDKDWNIFSKNCNNS
FAECSSQDVVTKPDCNCLYPKAIPSSDPASVSPHQPLAPSMAPVAGLTWEDSEGTEGSSLLPGEQPLHTVDPGSAKQRPPRSTCQSF
EPPETPVVKDSTIGGSPQPRPSVGAFNPGMEDILDSAMGTNWVPEEASGEASEIPVPQGTELSPSRPGGGSMQTEPARPSNFLSASS
PLPASAKGQQPADVTGTALPRVGPVRPTGQDWNHTPQKTDHPSALLRDPPEPGSPRISSLRPQGLSNPSTLSAQPQLSRSHSSGSVL
PLGELEGRRSTRDRRSPAEPEGGPASEGAARPLPRFNSVPLTDTGHERQSEGSSSPQLQESVFHLLVPSVILVLLAVGGLLFYRWRR
RSHQEPQRADSPLEQPEGSPLTQDDRQVELPV*


HUMAN mRNA SEQUENCE : hR27-014.2 (Seq ID No: 43)
TTGGGACGATCATAGAGCGCTAGCACTGAATCAGCCTGGAGGCGGAAGGAAAGGGTCGGTCCGCAGAGGGCGCGGGGAAGGCAG
GGTGGGGACGCGGTGGAGCCCGCGCTCGTTTGCTGAAGGCTTGGAAGTGCAGCGCAGAAGACAGAGGGTGACTAGGAAGACGCGCGA
GCGGGGCTGGCCGGCCGGCGGGTGGGGGAGGGGAGGCGGGGGAAGGCGGCTGAGTGGGCCTCTGGAGTGTGTGTGTCTGTGTCAGTG
TGTGTGTGTGTGTGTGTATGTGTGTGTCTGGCGCCTGGCCAGGGTGATTTCCCATAAACCACATGCCCCCCAGTCCTCTCTTAAAAG
GCTGTGCCGAGGGCTGGCCAGTGAGGCTCGGCCCGGGGAAAGTGAAAGTTTGCCTGGGTCCTCTCGGCGCCAGAGCCGCTCTCCGCA
TCCCAGGACAGCGGTGCGGCCCTCGGCCGGGGCGCCCACTCCGCAGCAGCCGAGCGAGCGGCAGGCGGAGGGCGGCCGCGACGCGC
CCGGCCGGGACCCAGCTGCCCGTATGACCGCGCGGGCGCCGCCGGGCGCTGCCCTCCCACGACATGGCTGGGCTCCCTGCTGTTGT
TGGTCTGTCTCCTGGCGAGCAGGAGTATCACCGAGGAGGTGTCGGAGTACTGTAGCCACATGATTGGGAGTGGACACCTGCAGTCTC
TGCAGCGGCTGATTGACAGTCAGATGGAGACCTCGTGCCAAATTACATTTGAGTTTGTAGACCAGGAACAGTTGAAAGATCCAGTGT
GCTACCTTAAGAAGGCATTTCTCCTGGTACAAGACATAATGGAGGACACCATGCGCTTCAGAGATAACACCCCCAATGCCATCGCCA
TTGTGCAGCTGCAGGAACTCTCTTTGAGGCTGAAGAGCTGCTTCACCAAGGATTATGAAGAGCATGACAAGGCCTGCGTCCGACTT
TCTATGAGACACCTCTCCAGTTGCTGGAGAAGGTCAAGAATGTCTTTAATGAAACAAAGAATCTCCTTGACAAGGACTGGAATATTT
TCAGCAAGAACTGCAACAACAGCTTTGCTGAATGCTCCAGCCAAGATGTGGTGACCAAGCCTGATTGCAACTGCCTGTACCCCAAAG
CCATCCCTAGCAGTGACCCGGCCTCTGTCTCCCCTCATCAGCCCCTCGCCCCCTCCATGGCCCCTGTGGCTGGCTTGACCTGGGAGG
ACTCTGAGGGAACTGAGGGCAGCTCCCTCTTGCCTGGTGAGCAGCCCCTGCACACAGTGGATCCAGGCAGTGCCAAGCAGCGGCCAC
CCAGGAGCACCTGCCAGAGCTTTGAGCCGCCAGAGACCCCAGTTGTCAAGGACAGCACCATCGGTGGCTCACCACAGCCTCGCCCCT
CTGTCGGGGCCTTCAACCCCGGGATGGAGGATATTCTTGACTCTGCAATGGGCACTAATTGGGTCCCAGAAGAAGCCTCTGGAGAGG
CCAGTGAGATTCCCGTACCCCAAGGGACAGAGCTTTCCCCCTCCAGGCCAGGAGGGGGCAGCATGCAGACAGAGCCCGCCAGACCCA
GCAACTTCCTCTCAGCATCTTCTCCACTCCCTGCATCAGCAAAGGGCCAACAGCCGGCAGATGTAACTGGCCATGAGAGGCAGTCCG
AGGGATCCTCCAGCCCGCAGCTCCAGGAGTCTGTCTTCCACCTGCTGGTGCCCAGTGTCATCCTGGTCTTGCTGGCCGTCGGAGGCC
TCTTGTTCTACAGGTGGAGGCGGCGGAGCCATCAAGAGCCTCAGGAGCGGATTTCTCCCTTGGCAACCAGAGGGCAGCCCCCTGA
CTCAGGATGACAGACAGGTGGAACTGCCAGTGTAGAGGGAATTCTAAGACCCCTCACCATCCTGGACACACTCGTTTGTCAATGTCC
CTCTGAAAATGTGACGCCCAGCCCCGGACACAGTACTCCAGATGTTGTCTGACCAGCTCAGAGAGAGTACAGTGGGACTGTTACCTT
CCTTGATATGGACAGTATTCTTCTATTTGTGCAGATTAAGATTGCATTAGTTTTTTTCTTAACAACTGCATCATACTGTTGTCATAT
GTTGAGCCTGTGGTCTATTAAAACCCCTAGTTCCATTTCCCATAAACTTCTGTCAAGCCAGACCATCTCTACCCTGTACTTGGACAA
CTTAACTTTTTTTAACCAAAGTGCAGTTTATGTTCACCTTTGTTAAAGCCACCTTGTTGGTTTCTGCCCATCACCTGAACCTACTGAA
GTTGTGTGAAATCCTAATTCTGTCATCTCCGTAGCCCTCCCAGTTGTGCCTCCTGCACATTGATGAGTGCCTGCTGTTGTCTTTGCC
CATGTTGTTGATGTAGCTGTGACCCTATTGTTCCTCACCCCTGCCCCCCGCCAACCCCAGCTGGCCCACCTCTTCCCCCTCCCACCC
AAGCCCACAGCCAGCCCATCAGGAAGCCTTCCTGGCTTCTCCACAACCTTCTGACTGCTCTTTTCAGTCATGCCCCTCCTGCTCTTT
TGTATTTGGCTAATAGTATATCAATTTGCACTTATCTGGACGCACAGAACAGTCTCTCCGTGGGAGGAGACATTATGGGGCGTCCAC
CACCCACCCCTCCCTGGCCATCCTCCTGGAATGTGGTCTGCCCTCCACGAGACTCCTGCCTGCCAGGACTGGACCAGAGCAGCCAGG
CTGGGGCCCCTCTGTCTCAACCCGCAGACCCTTGACTGAATGAGAGAGGCCAGAGGATGCTCCCCATGCTGCCACTATTTATTGTGA
GCCCTGGAGGCTCCCATGTGCTTGAGGAAGGCTGGTGAGCCCGGCTCAGGACCCTCTTCCCTCAGGGGCTGCACCCTCCTCTCACTC
CCTTCCATGCCGGAACCCAGGCCAGGGACCCACCGGCCTGTGGTTTGTGGGAAAGCAGGGTGGACGCTGAGGAGTGAAAGAACCCTG
CACCCAGAGGGCCTGCCTGGTGCCAAGGTATCCCAGCCTGGACAGGCATGGACCTGTCTCCAGAGAGAGGAGCCTGAAGTTCGTGGG
GCGGGACAGCGTCGGCCTGATTTCCCGTAAAGGTGTGCAGCCTGAGAGACGGGAAGAGGAGGCCTCTGGACCTGCTGGTCTGCACTG
ACAGCCTGAAGGGTCTACACCCTCGGCTCACCTAAGTGCCCTGTGCTGGTTGCCAGGCGCAGAGGGGAGGCCAGCCCTGCCCTCAGG
ACCTGCCTGACCTGCCAGTGATGCCAAGAGGGGGATCAAGCACTGGCCTCTGCCCCTCCTCCTTCCAGCACCTGCCAGAGCTTCTCC
AGGAGGCCAAGCAGAGGCTCCCCTCATGAAGGAAGCCATTGCACTGTGAACACTGTACCTGCCTGCTGAACAGCCTGCCCCCGTCCA
TCCATGAGCCAGCATCCGTCCGTCCTCCACTCTCCAGCCTCTCCCCAGCCTCCTGCACTGAGCTGGCCTCACCAGTCGACTGAGGGA
GCCCCTCAGCCCTGACCTTCTCCTGACCTGGCCTTTGACTCCCCGGAGTGGAGTGGGGTGGGAGAACCTCCTGGGCCGCCAGCCAGAG
GCCGGTCTTTAGGCTGTGTTGTTCGCCCAGGTTTCTGCATCTTGCACTTTGACATTCCCAAGAGGGAAGGGACTAGTGGGAGAGAGC
AAGGGAGGGGAGGGCACAGACAGAGAGGCTACAGGGCGAGCTCTGACTGAAGATGGGCCTTTGAAATATAGGTATGCACCTGAGGTT
GGGGGAGGGTCTGCACTCCCAAACCCCAGCGCAGTGTCCTTTCCCTGCTGCCGACAGGAACCTGGGGCTGAGCAGGTTATCCCTGTC
AGGAGCCCTGGACTGGGCTGCATCTCAGCCCCACCTGCATGGTATCCAGCTCCCATCCACTTCTCACCCTTCTTTCCTCCTGACCTT
GGTCAGCAGTGATGACCTCCAACTCTCACCCACCCCCTCTACCATCACCTCTAACCAGGCAAGCCAGGGTGGGAGAGCAATCAGGAG
AGCCAGGCCTCAGCTTCCAATGCCTGGAGGGCCTCCACTTTGTGGCCAGCCTGTGGTGGTGGCCTGAGGCCTAGGCAACGAGCGAC
AGGGCTGCCAGTTGCCCCTGGGTTCCTTTGTGCTGCTGTGTGCCTCCTCTCCTGCCGCCCTTTGTCCTCCGCTAAGAGACCCTGCCC
TACCTGGCCGCTGGGCCCCGTGACTTTCCCTTCCTGCCCAGGAAAGTGAGGGTCGGCTGGCCCCACCTTCCCTGTCCTGATGCCGAC
AGCTTAGGGAAGGGCAGTGAACTTGCATATGGGGCTTAGCCTTCTAGTCACAGCCTCTATATTTGATGCTAGAAAACACATATTTTT
```

133

```
AAATGGAAGAAAAATAAAAAAGGCATTCCCCCTTCATCCCCCTACCTTAAACATATAATATTTTAAAGGTCAAAAAAGCAATCCAACC
CACTGCAGAAGCTCTTTTTGAGCACTTGGTGGCATCAGAGCAGGAGGAGCCCCAGAGCCACCTCTGGTGTCCCCCCAGGCTACCTGC
TCAGGAACCCCTTCTGTTCTCTGAGAAGTCAAGAGAGGACATTGGCTCACGCACTGTGAGATTTTGTTTTTATACTTGGAAGTGGTG
AATTATTTTATATAAAGTCATTTAAATATCTATTTAAAAGATAGGAAGCTGCTTATATATTTAATAATAAAAGAAGTGCACAAGCTG
CCATGTGA
```

HUMAN PROTEIN SEQUENCE : hP27-014.2 (Seq ID No: 44)
```
MPPSPLLKGCAEGWPVRLGPGKVKVCLGPLGARAALRIPGQRCGPRPGRPLRSSQRASERARAADAPGRDPAARMTAPGAAGRCPPT
TWLGSLLLLVCLLASRSITEEVSEYCSHMIGSGHLQSLQRLIDSQMETSCQITFEFVDQEQLKDPVCYLKKAFLLVQDIMEDTMRFR
DNTPNAIAIVQLQELSLRLKSCFTKDYEEHDKACVRTFYETPLQLLEKVKNVFNETKNLLDKDWNIFSKNCNNSFAECSSQDVVTKP
DCNCLYPKAIPSSDPASVSPHQPLAPSMAPVAGLTWEDSEGTEGSSLLPGEQPLHTVDPGSAKQRPPRSTCQSFEPPETPVVKDSTI
GGSPQPRPSVGAFNPGMEDILDSAMGTNWVPEEASGEASEIPVPQGTELSPSRPGGGSMQTEPARPSNFLSASSPLPASAKGQQPAD
VTGHERQSEGSSSPQLQESVFHLLVPSVILVLLAVGGLLFYRWRRRSHQEPQRADSPLEQPEGSPLTQDDRQVELPV*
```

HUMAN mRNA SEQUENCE : hR27-014.3 (Seq ID No: 45)
```
TTGGGACGATCATAGAGCGCTAGCACTGAATCAGCCTGGAGAGCGCGGAAGGAAAGGGTCGGTCCGCAGAGGGCGCGGGGAAGGCAG
GGTGGGGACGCGGTGGAGCCCGCGCTCGTTTGCTGAAGGCTTGGAAGTGCAGCGCAGAAGACAGAGGGTGACTAGGAAGACGCGCGA
GCGGGGCTGGCCGGCCGGCGGGTGGGGGAGGGGAGGCGGGGGAAGGCGGCTGAGTGGGCCTCTGGAGTGTGTGTGTCTGTGTCAGTG
TGTGTGTGTGTGTGTATGTGTGTGTCTGGCGCCTGGCCAGGGTGATTTCCCATAAACCACATGCCCCCCAGTCCTCTCTTAAAAG
GCTGTGCCGAGGGCTGGCCAGTGAGGCTCGGCCCGGGGAAAGTGAAAGTTTGCCTGGGTCCTCTCGGCGCCAGAGCCGCTCTCCGCA
TCCCAGGACAGCGGTGCGGCCCTCGGCCGGGGCGCCCACTCCGCAGCAGCCAGCGAGCGAGCGAGCGAGCGAGGGCGGCCGACGCGC
CCGGCCGGGACCCAGCTGCCCGTATGACCGCCGGGCGCCGCCGGCGCTGCCCTCCCACGACATGGCTGGGCTCCCTGCTGTTGT
TGGTCTGTCTCCTGGCGAGCAGGAGTATCACCGAGGAGGTGTCGGAGTACTGTAGCCACATGATTGGGAGTGGACACCTGCAGTCTC
TGCAGCGGCTGATTGACAGTCAGATGGAGACCTCGTGCCAAATTACATTTGAGTTTGTAGACCAGGAACAGTTGAAAGATCCAGTGT
GCTACCTTAAGAAGGCATTTCTCCTGGTACAAGACATAATGGAGGACACCATGCGCTTCAGAGATAACACCCCCAATGCCATCGCCA
TTGTGCAGCTGCAGGAACTCTCTTTGAGGCTGAAGAGCTGCTTCACCAAGGATTATGAAGAGCATGACAAGGCCTGCGTCCGAACTT
TCTATGAGACACCTCTCAGTTGCTGGAGAAGGTCAAGAACTTCTTTAATGAAACAAAGAATCTCCTTGACAAGGACTGGAATATTT
TCAGCAAGAACTGCAACAACAGCTTTGCTGAATGCTCCAGCCAAGGCCATGGAGGCAGTCCGAGGGATCCTCCAGCCGCAGCTCC
AGGAGTCTGTCTTCCACCTGCTGGTGCCCAGTGTCATCCTGGTCTTGCTGGCCGTCGGAGGCCTCTTGTTCTACAGGTGGAGGCGGC
GGAGCCATCAAGAGCCTCAGAGAGCGGATTCTCCCTTGGAGCAACCAGAGGGCAGCCCCCTGACTCAGGATGACAGACAGGTGGAAC
TGCCAGTGTAGAGGGAATTCTAAGACCCCTCACCATCCTGGACACACTCGTTTGTCAATGTCCCTCTGAAAATGTGACGCCCAGCCC
CGGACACAGTACTCCAGATGTTGTCTGACCAGCTCAGAGAGGTACAGTGGGACTGTTACCTTCCTTGATATGGACAGTATTCTTCT
ATTTGTGCAGATTAAGATTGCATTAGTTTTTTTTCTTAACAACTGCATCATACTGTTGTCATATGTTGAGCCTGTGGTCTATTAAAAC
CCCTAGTTCCATTTCCCATAAACTTCTGTCAAGCCAGACCATCTCTACCCTGTACTTGGACAACTTAACTTTTTTTAACCAAAGTGCA
GTTTATGTTCACCTTTGTTAAAGCCACCTTGTTGGTTTCTGCCCATCACCTGAACCTACTGAAGTTGTGTGAAATCCTAATTCTGTC
ATCTCCGTAGCCCTCCCAGTTGTGCCTCCTGCACATTGATGAGTGCCTGCTGTTGTCTTTGCCCATGTTGTTGATGTAGCTGTGACC
CTATTGTTCCTCACCCCTGCCCCCCGCCAACCCCAGCCTGGCCCACCTCTTCCCCCTCCCACCCAAGCCCACAGCCCAGCCCATCAGGA
AGCCTTCCTGGCTTCTCCACAACCTTCTGACTGCTCTTTTCAGTCATGCCCCTCCTGCTCTTTTGTATTTGGCTAATAGTATATCAA
TTTGCACTTATCTGGACGCACAGAACAGTCTCTCCGTGGGAGGAGACATTATGGGGCGTCCACCACCACCCCTCCCTGGCCATCCTC
CTGGAATGTGGTCTGCCCTCCACCAGAGCTCCTGCCTGCCAGGACTGGACCAGAGCAGCCAGGCTGGGGCCCCTCTGTCTCAACCCG
CAGACCCTTGACTGAATGAGAGAGGCCAGAGGATGCTCCCCATGCTGCCACTATTTATTGTGAGCCCTGGAGGCTCCCATGTGCTTG
AGGAAGGCTGGTGAGCCCGGCTCAGGACCCTCTTCCCTCAGGACCCTCCACCCTCCTCTCACTCCCTTCCATGCCGGAACCCAGGCCA
GGGACCCACCGGCCTGTGGTTTGTGGGAAAGCAGGGTGGACGCTGAGGAGTGAAAGAACCCTGCACCCAGAGGGCCTGCCTGGTGCC
AAGGTATCCCAGCCTGGACAGGCATGGACCTGTCTCCAGAGAGAGGGAGCCTGAAGTTCGTGGGGCGGGACAGCGTCGGCCTGATTTC
CCGTAAAGGTGTGCAGCCTGAGAGACGGGAAGAGGAGGCCTCTGGACCTGCTGGTCTGCACTGACAGCCTGAAGGGTCTACACCCTC
GGCTCACCTAAGTGCCCTGTGCTGGTTGCCAGGCGCAGAGGGGAGGCCAGCCCTGCCCTCAGGACCTGCCTGACCTGCCAGTGATGC
CAAGAGGGGGATCAAGCACTGGCCTCTGCCCCTCCTCCTTCCAGCACCTGCCAGAGCTTCTCCAGGAGGCCAAGCAGAGGCTCCCCT
CATGAAGGAAGCCATTGCACTGTGAACACTGTACCTGCCTGCTGAACAGCCTGCCCCCGTCCATCCATGAGCCAGCATCCGTCCGTC
CTCCACTCTCCAGCCTCTCCCCAGCCTCCTGCACTGAGCTGGCCTCACCAGTCGACTGAGGGGAGCCCCTCAGCCCTGACCTTCTCCT
GACCTGGCCTTTGACTCCCCGGAGTGGAGTGGGGTGGGAGAACCTCCTGGGCCGCCAGCCAGAGCCGGTCTTTAGGCTGTGTTGTTC
GCCCAGGTTTCTGCATCTTGCACTTTGACATTCCCAAGAGGGAAGGGACTAGTGGGAGAGAGCAAGGGAGGGGAGGGCACAGACAGA
GAGGCTACAGGGCGAGCTCTGACTGAAGATGGGCCTTTGAAATATAGGTATGCACCTGAGGTTGGGGGAGGGTCTGCACTCCCAAAC
CCCAGCGCAGTGTCCTTTCCCTGCTGCCGACAGGAACCTGGGGCTGAGCAGGTTATCCCTGTCAGGAGCCCTGGACTGGGCTGCATC
TCAGCCCCACCTGCATGGTATCCAGCTCCCATCCACTTCTCACCCTTCTTTCCTCCTGACCTTGGTCAGCAGTGATGACCTCCAACT
CTCACCCACCCCCTCTACCATCACCTCTAACCAGGCAAGCCAGGGTGGGAGAGCAATCAGGAGAGCCAGGCCTCAGCTTCCAATGCC
TGGAGGGCCTCCACTTTGTGGCCAGCCTGTGGTGGTGGCTCTGAGGCCTAGGCAACGAGCGACAGGGCTGCCAGTTGCCCCTGGGTT
CCTTTGTGCTGCTGTGTGCCTCCTCTCCTGCCGCCCTTTGCTCCGCTAAGAGACCCTGCCCTTACCTGGCCGCTGGGCCCCGTGAC
TTTCCCTTCCTGCCCAGGCAAAGTGAGGGTCGGCTGGCCCCACCTTCCCTGTCCTGATGCCGCGACAGCTTAGGGAAGGGCAGTGAACTT
GCATATGGGGCTTAGCCTTCTAGTCACAGCCTCTATATTTGATGCTAGAAAACACATATTTTTAAATGGAAGAAAAATAAAAAGGCA
TTCCCCCTTCATCCCCCTACCTTAAACATATAATATTTTAAAGGTCAAAAAAGCAATCCAACCCACTGCAGAAGCTCTTTTTGAGCA
CTTGGTGGCATCAGAGCAGGAGGAGCCCCAGAGCCACCTCTGGTGTCCCCCCAGGCTACCTGCTCAGGAACCCCTTCTGTTCTCTGA
GAAGTCAAGAGAGGACATTGGCTCACGCACTGTGAGATTTTGTTTTTATACTTGGAAGTGGTGAATTATTTTATATAAAGTCATTTA
AATATCTATTTAAAAGATAGGAAGCTGCTTATATATTTAATAATAAAAGAAGTGCACAAGCTGCCATGTGA
```

HUMAN PROTEIN SEQUENCE : hP27-014.3 (Seq ID No: 46)
```
MPPSPLLKGCAEGWPVRLGPGKVKVCLGPLGARAALRIPGQRCGPRPGRPLRSSQRASERARAADAPGRDPAARMTAPGAAGRCPPT
TWLGSLLLLVCLLASRSITEEVSEYCSHMIGSGHLQSLQRLIDSQMETSCQITFEFVDQEQLKDPVCYLKKAFLLVQDIMEDTMRFR
DNTPNAIAIVQLQELSLRLKSCFTKDYEEHDKACVRTFYETPLQLLEKVKNVFNETKNLLDKDWNIFSKNCNNSFAECSSQGHERQS
EGSSSPQLQESVFHLLVPSVILVLLAVGGLLFYRWRRRSHQEPQRADSPLEQPEGSPLTQDDRQVELPV*
```

HUMAN mRNA SEQUENCE : hR27-014.4 (Seq ID No: 47)
```
TTGGGACGATCATAGAGCGCTAGCACTGAATCAGCCTGGAGAGCGCGGAAGGAAAGGGTCGGTCCGCAGAGGGCGCGGGGAAGGCAG
GGTGGGGACGCGGTGGAGCCCGCGCTCGTTTGCTGAAGGCTTGGAAGTGCAGCGCAGAAGACAGAGGGTGACTAGGAAGACGCGCGA
GCGGGGCTGGCCGGCCGGCGGGTGGGGGAGGGGAGGCGGGGGAAGGCGGCTGAGTGGGCCTCTGGAGTGTGTGTGTCTGTGTCAGTG
TGTGTGTGTGTGTGTGTATGTGTGTGTCTGGCGCCTGGCCAGGGTGATTTCCCATAAACCACATGCCCCCCAGTCCTCTCTTAAAAG
GCTGTGCCGAGGGCTGGCCAGTGAGGCTCGGCCCGGGGAAAGTGAAAGTTTGCCTGGGTCCTCTCGGCGCCAGAGCCGCTCTCCGCA
```

134

```
TCCCAGGACAGCGGTGCGGCCCTCGGCCGGGGCGCCCACTCCGCAGCAGCCAGCGAGCGAGCGAGCGAGGGCGGCCGACGCGC
CCGGCCGGGACCCAGCTGCCCGTATGACCGCGCCGGGCGCCGCCGGGCGCTGCCCTCCCACGACATGGCTGGGCTCCCTGCTGTTGT
TGGTCTGTCTCCTGGCGAGCAGGAGTATCACCGAGGAGGTGTCGGAGTACTGTAGCCACATGATTGGGAGTGGACACCTGCAGTCTC
TGCAGCGGCTGATTGACAGTCAGATGGAGACCTCGTGCCAAATTACATTTGAGTTTGTAGACCAGGAACAGTTGAAAGATCCAGTGT
GCTACCTTAAGAAGGCATTTCTCCTGGTACAAGACATAATGGAGGACACCATGCGCTTCAGAGATAACACCCCCAATGCCATCGCCA
TTGTGCAGCTGCAGGAACTCTCTTTGAGGCTGAAGAGCTGCTTCACCAAGGATTATGAAGAGCATGACAAGGCCTGCGTCCGAACTT
TCTATGAGACACCTCTCCAGTTGCTGGAGAAGGTCAAGAATGTCTTTAATGAAACAAAGAATCTCCTTGACAAGGACTGGAATATTT
TCAGCAAGAACTGCAACAACAGCTTTGCTGAATGCTCCAGCCAAGATGTGGTGACCAAGCCTGATTGCAACTGCCTGTACCCCAAAG
CCATCCCTAGCAGTGACCCGGCCTCTGTCTCCCCTCATCAGCCCCTCGCCCCCTCCATGGCCCCTGTGGCTGGCTTGACCTGGGAGG
ACTCTGAGGGAACTGAGGGCAGCTCCCTCTTGCCTGGTGAGCAGCCCCTGCACACAGTGGATCCAGGCAGTGCCAAGCAGCGGCCAC
CCAGGAGCACCTGCCAGAGCTTTGAGCCGCCAGAGACCCCAGTTGTCAAGGACAGCACCATCGGTGGCTCACCACAGCCTCGCCCCT
CTGTCGGGGCCTTCAACCCCGGGATGGAGGATATTCTTGACTCTGCAATGGGCACTAATTGGGTCCCAGAAGAAGCCTCTGGAGAGG
CCAGTGAGAGTCCCGTACCCCAAGGGACAGAGCTTTCCCCCTCCAGGCCAGGAGGGGGGCAGCATGCAGACAGAGCCCGCCAGACCCA
GCAACTTCCTCTCAGCATCTTCTCCACTCCCTGCATCAGCAAAGGGCCAACAGCCGGCAGATGTAACTGGTACCGCCTTGCCCAGGG
TGGGCCCCGTGAGGCCCACTGGCCAGGACTGGAATCACACCCCCCAGAAGACAGACCATCCATCTGCCCTGCTCAGAGACCCCCCGG
AGCCAGGCTCTCCCAGGATCTCATCACTGCGCCCCCAGGGCCTCAGCAACCCCTCCACCCTCTCTGCTCAGCCACAGCTTTCAGAA
GCCACTCCTCGGGCAGCGTGCTGCCCCTTGGGGAGCTGGAGGGCAGGAGGAGCACCAGGGATCGGAGGAGCCCCGCAGAGCCAGAAG
GAGGACCAGCAAGTGAAGGGGCAGCCAGGCCCCTGCCCCGTTTTAACTCCGTTCCTTTGACTGACACAGGCCATGAGAGGCAGTCCG
AGGGATCCTCCAGCCCGCAGCTCCAGGAGTCTGTCTTCCACCTGCTGGTGCCCAGTGTCATCCTGGTCTTGCTGGCCGTCGGAGGCC
TCTTGTTCTACAGGTGGAGGCGGCGGAGCCATCAAGAGCCTCAGAGACGGGATTCTCCCTTGGAGCAACCAGAGGGCAGCCCCCTGA
CTCAGGATGACAGACAGGTGGAACTGCCAGTGTAGAGGGAATTCTAAGACCCCTCACCATCCTGGACACACTCGTTTGTCAATGTCC
CTCTGAAAATGTGACGCCCAGCCCCGGACACAGTACTCCAGATGTTGTCTGACCAGCTCAGAGAGAGTACAGTGGGACTGTTACCTT
CCTTGATATGGACAGTATTCTTCTATTTGTGCAGATTAAGATTGCATTAGTTTTTTTCTTAACAACTGCATCATACTGTTGTCATAT
GTTGAGCCTGTGGTCTATTAAAACCCCTAGTTCCATTTCCCATAAACTTCTGTCAAGCCAGACCATCTCTACCCTGTACTTGGACAA
CTTAACTTTTTTAACCAAAGTGCAGTTTATGTTCACCTTTGTTAAAGCCACCTTGTTGGTTTCTGCCCATCACCTGAACCTACTGAA
GTTGTGTGAAATCCTAATTCTGTCATCTCCGTAGCCCTCCCAGTTGTGCCTCCTGCACATTGATGAGTGCCTGCTGTTGTCTTTGCC
CATGTTGTTGATGTAGCTGTGACCCTATTGTTCCTCACCCCTGCCCCCCGCCAACCCCAGCTGGCCCACCTCTTCCCCCTCCCACCC
AAGCCCACAGCCAGCCCATCAGGAAGCCTTCCTGGCTTCTCCACAACCTTCTGACTGCTCTTTTCAGTCATGCCCCTCCTGCTCTTT
TGTATTTGGCTAATAGTATATCAATTTGCACTTATCTGGACGCACAGAACAGTCTCTCCGTGGGAGGAGACATTATGGGGCGTCCAC
CACCACCCCTCCCTGGCCATCCTCCTGGAATGTGGTCTGCCCTCCACCAGAGCTCCTGCCTGCCAGGACTGGACCAGAGCAGCCAGG
CTGGGGCCCCTCTGTCTCAACCCGCAGACCCTTGACTGAATGAGAGGCCAGAGGATGCTCCCCATGCTGCCACTATTTATTGTGA
GCCCTGGAGGCTCCCATGTGCTTGAGGAAGGCTGGTGAGCCCGGCTCAGGACCCTCTTCCCTCAGGGGCTGCACCCTCCTCTCACTC
CCTTCCATGCCGGAACCCAGGCCAGGGACCCACCGGCCTGTGGTTTGTGGGAAAGCAGGGTGGACGCTGAGGAGTGAAAGAACCCTG
CACCCAGAGGGCCTGCCTGGTGCCAAGGTATCCCAGCCTGGACAGGCATGGACCTGTCTCCAGAGAGAGGAGCCTGAAGTTCGTGGG
GCGGGACAGCGTCGGCCTGATTTCCCGTAAAGGTGTGCAGCCTGAGAGACGGGAAGAGGAGGCCTCTGGACCTGCTGGTCTGCACTG
ACAGCCTGAAGGGTCTACACCCTCGGCTCACCTAAGTGCCCTGGTTGGTGCCAGGCGCAGAGGGGAGGCCAGCCCTGCCCTCAGG
ACCTGCCTGACCTGCCAGTGATGCCAAGAGGGGGATCAAGCACTGGCCTCTGCCCCTCCTCCTTCCAGCACCTGCCAGAGCTTCTCC
AGGAGGCCAAGCAGAGGCTCCCCTCATGAAGGAAGCCATTGCACTGTGAACACTGTACCTGCCTGCTGAACAGCCTGCCCCCGTCCA
TCCATGAGCCAGCATCCGTCCGTCCTCCACTCTCCAGCCTCTCCCCAGCCTCCTGCACTGAGCTGGCCTCACCAGTCGACTGAGGGA
GCCCCTCAGCCCTGACCTTCTCCTGACCTGGCCTTTGACTCCCCGGAGTGGAGTGGGGTGGGAGAACCTCCTGGGCCGCCAGCCAGA
GCCGGTCTTTAGGCTGTGTTGTTCGCCCCAGGTTTCTGCATCTTGACATTCCCAAGAGGGAAGGGACTAGTGGGAGAGAGGC
AAGGGAGGGGAGGGCACAGACAGAGAGGCTACAGGGCGAGCTCTGACTGAAGATGGGCCTTTGAAATATAGGTATGCACCTGAGGTT
GGGGGAGGGGTCTGCACTCCCAAACCCCAGCGCAGTGTCCTTTCCCTGCTGCCGACAGGAACCTGGGGCTGAGCAGGTTATCCCTGTC
AGGAGCCCTGGACTGGGCTGCATCTCAGCCCCACCTGCATGGTATCCAGCTCCCATCCACTTCTCACCCTTCTTTCCTCCTGACCTT
GGTCAGCAGTGATGACCTCCAACTCTCACCCACCCCCTCTACCATCACCTCTAACCAGGCAAGCCAGGGTGGGAGAGCAATCAGGAG
AGCCAGGCCTCAGCTTCCAATGCCTGGAGGGCCTCCACTTTGTGGCCAGCCTGTGGTGGTGGCTCTGAGGCCTAGGCAACGAGCGAC
AGGGCTGCCAGTTGCCCCTGGGTTCCTTTGTGCTGCTGTGTGACCTCCTCTCCTGCGCCCTTTGTCCTCCGCTAAGAGACCCTGCCC
TACCTGGCCGCTGGGCCCCGTGACTTTCCCTTCCTGCCCAGGAAAGTGAGGGTCGGCTGGCCCCACCTTCCCTGTCCTGATGCCGAC
AGCTTAGGGAAGGGCAGTGAACTTGCATATGGGGCTTAGCCTTCTAGTCACAGCCTCTATATTTGATGCTAGAAAACACATATTTTT
AAATGGAAGAAAAATAAAAAGGCATTCCCCCTTCATCCCCCTACCTTAAACATATAATATTTTAAAGGTCAAAAAAGCAATCCAACC
CACTGCAGAAGCTCTTTTTGAGCACTTGGTGGCATCAGAGCAGGAGGAGCCCCAGAGCCACCTCTGGTGTCCCCCCAGGCTACCTGC
TCAGGAACCCCTTCTGTTCTCTGAGAAGTCAAGAGAGGACATTGGCTCACGCACTGTGAGATTTTGTTTTTATACTTGGAAGTGGTG
AATTATTTTATATAAAGTCATTTAAATATCTATTTAAAAGATAGGAAGCTGCTTATATATTTAATAATAAAAGAAGTGCACAAGCTG
CCATGTGA
```

HUMAN PROTEIN SEQUENCE : hP27-014.4 (Seq ID No: 48)

```
             MPPSPLLKGCAEGWPVRLGPGKVKVCLGPLGARAALRIPGQRCGPRPGRPLRSSQRASERARAADAPGRDPA
ARMTAPGAAGRCPPTTWLGSLLLLVCLLASRSITEEVSEYCSHMIGSGHLQSLQRLIDSQMETSCQITFEFVDQEQLKDPVCYLKKA
FLLVQDIMEDTMRFRDNTPNAIAIVQLQELSLRLKSCFTKDYEEHDKACVRTFYETPLQLLEKVKNVFNETKNLLDKDWNIFSKNCN
NSFAECSSQDVVTKPDCNCLYPKAIPSSDPASVSPHQPLAPSMAPVAGLTWEDSEGTEGSSLLPGEQPLHTVDPGSAKQRPPRSTCQ
SFEPPETPVVKDSTIGGSPQPRPSVGAFNPGMEDILDSAMGTNWVPEEASGEASEIPVPQGTELSPSRPGGGSMQTEPARPSNFLSA
SSPLPASAKGQQPADVTGTALPRVGPVRPTGQDWNHTPQKTDHPSALLRDPPEPGSPRISSLRPQGLSNPSTLSAQPQLSRSHSSGS
VLPLGELEGRRSTRDRRSPAEPEGGPASEGAARPLPRFNSVPLTDTGHERQSEGSSSPQLQESVFHLLVPSVILVLLAVGGLLFYRW
RRRSHQEPQRADSPLEQPEGSPLTQDDRQVELPV*
```

# Table 5

```
MOUSE GENOMIC SEQUENCE : mD28-002 (Seq ID No: 49)
```

```
CGCGGCATGAGCCCGCGTGCCCCACTGCTCGCCGGGCTGCTCCGAGCCGGGGTGCTCCGGGGCTCCAAACTCGGGCTGGCGGGGCA
AGTGTCTTCATGAACCCAGAGGATGTCCGGGAAGCACTACAAGGGTCCTGAAGTCAGTTGTTGCATCAAATACTTCATTTTTGGCTT
CAATGTCATATTTTGGGTAAGTTGGAGCGCTCCTGGGGTTTCCAAGTGCTGTGGCGGATGGGTGGGCGACGGTCCCCGCCGTGGTCG
CTGCTTTACTTTTCGCCGCCCCCGGGAGCTCCTGCCCCAGTCGCGGCGAGAGGCGTCTACCTTCCGCCTTCCCGGCTCGAGCGCGGGG
GAGAGAAACACTTCATCCTTTCCAGGACTCCGAGGAGGTGAAAAGAGGAACCAAGCCGGGCAGGGGGCAGCCAGGCCTCGGGGTGGC
TTTTTTCTTTCTTTTCTTTCTTTTCTTTCTTTTTTTTTTAAGGGGGTGGGGGGCGCGAATAGCTGGGGTACAAATGGGAGGACGGCTC
GGCAGTTTGAGAAACGAGGGAGCACACGGATTAGATTTGCTTCCCGTGGGATCTGGATGTTGGGCGAGAGCGTGGTGTCATAATGGGG
CAGGCTAATCGGGCAGAGCCGACCCCGAGATCCACCTTCCAGCGCCGATCTGATTGGCCGAGGGCTTGGCTCGGTGTTGCCGCGGCT
GGTCTGTGCCGTGCTCAGCGTGTATCTCCTTGCACCATCTCCGGCTTTGTGTAAGATGCAGATGCCGTGGTGTGTGGCTCTGTAATG
TGCACGTAAATAACACGTCCTGCATGCTCCTCTCCGGCAGTAAAGCGCTCCCTTTGCATAATGGACTGGCCAAGTGGGCGTGCAGGG
TGGAGGGATTTATTTCTCTGTGTATCCTTGTGGAATAACTAGTCATCAGTCAACATATGTTTCGGTGTGCGCGTGCCTCGCGTCTTC
CCCTTTTAAAGGGGTGACACACAGGTTTTCGGAACCCTTGGAGACATGCAAAGGAGAAGGTGTGTAAGACCTGTTTTGCTGGCAGAG
CAGGTGAGCCGCGGCTTGCAAACCGGCTCCCAACCGTGCTCTGCGTGACACTCCCTCGTGCCGGGTGCCTATTGTAATCAGGCTGGG
ACCCTACTCTTTGTTGTGATTCTTTTCTGAGACCTTCCAAACGTGTTCAGCAACCAGACAAAGAGTTCTCTAGGACAAGAGGGGAA
ATGGTTTGTTGGTTGGTTGGTTGGTTGGCATTGGTTGGCAGACCGTGTTATTTTTCAGCCCACCTATTTCCATCCGGGGTCGCCTTC
CTCCCTGGCACCTCTCTCTCCATGGGAGGGAAAGATGGTGGCGGACATCCCCAAGTTTCTGCGAGGTAAACAATGATC
CAGTGGTCTGAATCCAGGACATTTTAATTTGGACTCCCCTTAATTGTGGCTGTTTGCTGTAGACATAAACATCTGTGAGAATAGTTG
TAGTTATGCTGATCTTCTGCCCACCCCCTCACTTACCAGCTTATTTTATTTTCTCTCAGAGTAGCTATGTGTGTTTGTGAAAGGAA
AGGAAATGGACTTTGTGTCAGCATGTCATCTGTCCTCAGGACTGGACCAGATTGATGAGGGAGTGTGGGAACAAATACCATATACAG
CCATTCACGAGTAGTTTTGTTTTTTTTCTGTCAGTGATAATATATGGTTTCTGGGAGCAACTCCCCACCCCTGCCCCCTGCTGCAAA
CAGACGAAGAGGAAATGCGTTTCTTTCTTTCCTTCTTTTTTTTTTTTTTGCTTCTCCTGGACCCTTTCTGGAAATGTTT
AAAAGTGGTGGCTAGGCATGCTTCCCATTGAAGTATTCTGATTGCCTTGAACTATGAGAGGCAAGAGAGGCAAGGGCCGTCACATGG
GGCTGCAGGGGTGGTTCGCCCGGGCCTCTTCTTTCTGCTCCAACACTTCCAAAAGTTGCACATTTCAGCTACCAGGCAACAAAAAGC
ACCCACGGGCTTTCTCTGCAGTTTCAGAGCGGGGAGTGAGAGGGAAATAAAAAGGAATGGGGAAATGTTTCCCTGGCAGTTTGGTGT
AATTTTGTAGCGAGCACAAAGGCAAATTTAGCCCTGTGTCTGGATTAGGCTGCAGCAAACCCCCTGGACTGTGGAGAGCTTCAACAA
AATTGCAGATTGCCTGCATGGAGAGTTTCAGAATAGGACCCACATCTTGTCTGATTTTTGAAGTATTACTGTGGGCGGGCAGTAGGT
AGGCTTGTGGCAAGTCAGAGAACGGTCTGAGGCCCTGAAACTGAAGGAGCTGCCCTGGGCAGCATTGTTAAGTAGGTGTCTACTTCA
GAGAGTCATTTGCTCTCCCTGTCCTTCCAGTGCAGAGGGCCACCTCTAGTTCGTTTTCTATACTGCTCCTGTCTCCAAATGACCTCA
TATTTAAATGTCCTATTTTATCAGGGCCAGTGGGCTCTAGCAGAAATTAGCAGGAAAACATCCACGTTATCATTAAAAGGAATACAC
ACCAAAATATTAGTGACCGGAGATCTGGCAGCAGCTGCCAAAAACATGCACACTTCTGTCTTCGGCCACCAACCTGCCCGTGACTGT
CCAAGATAGCAGAGTCAACACTTCAGTTCAGTTGTACGGTTCTGCAGGAGGAATTGGTAACAGAGACCCATGGGCTCCCGGTCTTTCA
TCTTCACATTGACGTGCAGAACTAAATTCGAAATTCATTGAAGACATTTGAACGTAATAGTTCTTGTAAAATAAGAGGGAGCGCACA
GTGGCAGCTGTGACAGCTCGGATCTCTTGCTAATCAGCACTCTGTCTGTGTTAGTCAGTTATCTGCCCTGGGCCTTCGTTACTCAGC
TATTCGAATGAAGAATTTGGTTGAATTGTGAGGTTTTTCTCTGTCTCTAACAAAGACTTTGTTGAGTTCTAGTACGCCAGCCAGGCA
AGATCGAGTCTCAGGGATGAGCAGTAGGCTCTAATGATGGACTGGAGACTTGCCTATAGTTATAGATGGTCCTTTAGTAGACATGCT
CACGGACTAATTTGTTTTTCATTCTCTCCTCAATGATCTTTTTTCACTTAAAGAAAGAAACAAAACAACAAACCTTTCACTGAATTT
TCCTTGCTTGTTGAGTTATTGGTATGGCACGCATATTTCCCTATCTCTCCCCTGAATCTCAAGTGTGACGCTCTGAGTCATTAGCTG
TATTTTTATTATTAGAATCACAGCTTCATGTGGAGGATCCTAATTCTTTTATATAACAGCAGTGCTGATATTGTGAGTAAGAAAGTC
AACATCTTGTACAGATTCCTTTCTTGTGAGTTAGCACAGGCGGTGGGGGGGAAAGCCTTGTTTTTCTTGCTCTATATTGGAAGAAC
TCAAGCCAGCAGGAATTGATTTTATTGAGGGTGTCACCAGAGGAAGACATGTATCAGAAACACTGAGTTGTCTTTGGGGCGGAGGAG
AGAAGAGAAGAAGGTTTGTTTTAAGGTCAGTCCTTTGGGGAGTTAATTTCTCTGTCCTGTCACTGACCGTTTATAGATGTTTTGTTC
TGGTTCTTGACACTTAACTGTTTCTCAGAAAGTGAACGTCAACATCCTGGCTACTTATTGCTGCACAGCAAGCTCCCAAACTTTAGA
ATTTCAAAACGAAACCTCATCATGATAGTTCATAATTCTGTGATTTGACTCCTCGGGGGGCTACTTCTAATGTGGGACCTCCGTGCA
GTTGCTGGGAGTTTCTTGCTGTCTCCTTTGGGTGGGGGAACCCCGGGCTGGTGGTCACCTGTGATTGGTGATGCTTACCCATGGCTC
AGTGTCGCTGTCAGCTGTGGCACAGCAGGTGGATTTTTCTGAGGTTCGAAGCAGCTTCCAAGCAGTTTAACTGTACTGTCACTTGGC
ACATTTAGTCTAAGTCCTCATGGAGTCTGCTCAATTGAAGGTTCCATCTTTGGATAGGGAAGTGTCAGGGTCACTTTGTAGAAGGTT
ATGTGGTGGGCTGGCTAGTTTTATATCCACTTGACACAAGCTACAGTTATCTTAAAGGAGAGCATCTCAACTGAAGAAATGCCCCCA
TAAGGGCCGGCTGGAGGGCATTTTCTTAATTAGTGATTGATATGGGAGGGACCAGCCCCACTGTGGGTGGTGCTGTTCTTGGGCTAG
TGGTCCTGGGTGCTATACGAATCAGGTTGAGCAAGCCATGATGAGCAAGCCATGATGAGCAAGTCATAGTTTCTGTATCAGCTCCCG
CCTCCAGGTTCCTGCCCTGCTTGAGTTCCTGTCTTCACTGCTTTGATGAACGGTTTATGTAACTGCAAGATAAGTAAATTCTTCTCT
TCTACCCCTGGTTGCTTTTGGTCATGGTGTTTTATCACAGCAATAGTAACCCTAAGGCATGCGGATTTCAGCCGTCATTGGAAAATG
ATTTCTTAGAAACCATTGATGCGCTGGTCAGCAGAAGCCTCGAGTTGGATGGTGGGACCAACTTGATATTTTGTTCTCTGTGCTGTCC
CAGGACTAGAACAGCTGTTGGCTGAAGCCAGATGTCTCTTTGGTGTGTGTTTTCAGATATTGCTTTGTGTTGCAGGTTGGAGGTTTATC
CAGAGTAAATGGCGGAATGTTGTATATGCTTTGTTCCCACAGGCCAAGAGGAGGGAGATTCAAGGAATCTTGGGTGGTGGGGTGGGG
CGCCAAGTAGGATGTCTTATCTTATGATTCAGTATAAAATTTCTAGATGTTCTGGAATGATGGTACATGAGCCGTTTATAAGTCATAA
TTTTTGTTTTAATTTGCTAGACATCTGTTAGCTTCGATGGCAACCATATGGGAGTCTAAATTGCCTTTTGAATTTCAGGGAGAGGGT
TGGAAGAAAGCTAAATGTGGCTATGGAAGTGTCAGGTAGGCATGATGTGTGCAGACCTCAGAGGGGAGAGTCAGCGATGCACCCAGTGTC
TTGCCTCTTCCAGCAGTTCACTGTGGGGCTGATGCGTTCTCAATGGACGATGCTCTTGAAGGTTGAAATGGACGCAGTAGCAAAAGT
CGGAAAACCAGATAACATTCCGCCCTGCATAGAAGTTATGAAAAGGGTGAAATAAACACTTCTGCAGTGGGACTTGGAGTAATCTGA
GGGACATTTTATATGCTTTTAAAAAATCAGAATTTAATTGGGATGCCAGATTTCTAGCAACTTGCATCTTTAGTTTCCCAAATAATCT
GGAAGTTAGCATTTGATAAATGATTTTTTTAAGAAAAAAAAAAAAAAAAAAGAAGTTGCATTTGGGAGCTCATTTAGTTGGATCTCTC
TGTCCCAACATTCAGATTGGCATGCCTTTCCATACAACAGGGAAAGCAATGTGTTCTTTTGATTTCTACATGGGCCTTGCTCTTT
AACCCAGTGTGGTCTCTAATTTTGGCTCCTGTGCTACTCCTCCATCTCTCACTAGCGGCTGAGGCTTTTAGCTATAATCTGCTGTG
CCTAGAACCGTGTGTGTTTCAAAAACCCCACAGCTTTACCATTGTGATGTCCTTTTTAAAAGCCTGTAAAAGTATCTGACAAAGAC
CTATGGTGATATTTTTGTGACCAGTAAAAGAAATTGGAGCACAGAGATGTGAAATGGCTCTCGTGGTGTTGGAGCGCTTGCAGGTTC
GCTTGGGGAGTGTTAAGTCGACGCTGTAGCTAGAGCCCCAGTTTCTCAGACTGAAACTTATTTTAGAAGATGCTTGTTATTCTAAAA
TTAAACATCAAGTAAGTCATTTGCATCTGTTCATAATTGAAAAGTGATAAAGTTTTTGCATATGCCACAATTAACACCACCCACCAAG
CACCCACAAAAAAAGTTTAGGATGAAATGTTCTTAAAGAGAATGCTAGGATATAATAAATTATTATGTAGTCTGTTGAAAAATTCAT
AAATGACAATTAGAGTATTAATGAGGGTATTTTTTTTTTGAGTCACATGTTTATTTCTACGCGTAATACCAACTGCCTTAAGGTGAC
TAACCTCAGGATTCTAACTTACTGTTCAGTACGGGATGATAAAGGGCCTCCTGTTTGTGAACAACCATCTCCCAATCGTTTCCTTAAG
CCTCGTGTGTCATGTGTTGTCTCGGAACACATAGGCATCTAATAACTGCTTGTTGAATGGATGAGCTTCCGAATGTTGAGAAAGCCCA
TGGGGTTTATCCTTCAAGCCAAGGAAGCCTTGCTTTTCACAAGCACAGGCAGTTAGCACTCAAGGGGTGTTTGTCTTTGGAACATTTAGT
TAGAGAGCTGCGTCTGTCTTCAAACCAGATGTAAGGTCTCTCTGAAAATTTCAGCCTCCTAGTTTACTGTTGATTTTTCTTTTTTAA
TTAAAAATATTTTAAAAATATTATGTATATGGGTGCTTTGCCTGCACATATGTCTGTGTCACCACATGCATTCCTGGTTCTTGCAGAA
GCCAGAGGAGGATGTTGGATCCCCTGGAACTAGAGTTACAGATGGTTGTGGGCCACTGTGTGGTTGCCAGTGCCCTGAAACACAGAG
CCACCTCTCCAGCCCTCCTGGTGCTGACTTCTCACTTGAATTTCTTTTCTTTTCTTTCTTTTTTTATTTTTGGTTTTTCGAGATGGA
```

```
GTTTCTCTGTGTAGCCTTGGCTGTCCAGGAACTCACTCTGTAGACCAGACTGGCCTCCAACTCAGAAATCCACCTGCCTCTGCCTCC
CAAGTGCTGGGATTAAAGGCATTCACCACCACTGCCCAGCCTCACTTGAATTTTAACTATATATCTCAAACCTACAGGAAATGTTAGA
GGAAACAAATGATAAAGTTTGCTACTTCAGACATGTTATTCATATATCTTTAAGTTTTCCAACTTGTTTTTATCATGGTTAAGGACA
AAGTTGTCTGCCTGATGGCTCTCACAAACACAGTGTGGATTTCGTCTCGAAGGGTTCTCTCCTTGATAGCCACAGTGCCACCATCAA
AGCTAGGAAATTACTCCCATCCACTTCCCAGGCCTTGGGTCTCCTTCACTCTCCAATCTCAATAATTCCATTTAAAGCAAAGGAAT
TCAGGCCAGAACGGCACAATATGTGGGGGTTCTGTTAAGGCTCGTCCAGTCCCTCACTATCAAGAGCGTGTGACTGGCATTGCTGGCT
GCCCTCCCTGTGGGTTGCCTGTGGGCTCCTCAGGCGCAGGTTCTGGCTGTGTGTTCTGGGGTGGCAGCGGCCAGAAGGGACGCTTTG
CTTTCATCCGAATCCCATACGGTGGCAGTGGTTGTTGCTTTAGGCGTTTTCTCTGCCAAGTGTCTCCGATCATGTTACTACTTTTGT
TGCTTTGGCAACTTGTAACTGCTTTTTGGCAAGCCACTTCGAAGCTATAGAACTGCCCATCTCTCCTGGACTTCCTATCAGTAATGA
ATGTCTGTCAGCAGCGTTTCATGGCTTCCTCCTTTTCCATCTTGGTATGTTTCAACAACGAATCCAAATTAACCCCTTTTCTCTTAAA
GTTGCTTCTGTAAGGCATTGGGTCACAGTGATAAGAAAAGTAAGCAAACCCTTTGCTCTCTTTAAAAAGTTAATTTATTAATTACAT
CCTAATTGCTGCCCCCCAGAGCTCCCCTTCACAGGGTCCCTTCTCCCCTCCTCCTCTGAGAGGGTGGGGCCCCCCCTGGATATCCCC
CCTTCCCGACCCTGGTACATCAAGTCTCTGCGGTGTTAGGCACATCCTCTCCCACTGAGGGCAAACAAGGCAACCCTGTTAGGGAAT
GGTCTCCACAGCCAGGCAACAGCTTTAGGGATAGCCCCTGCTCTAGTTATTGGGGGATCCTCATGAAGACTGAGCTGCACATCTGCT
ACATATGTACTGGGGGCCCTGGTCTAGCCCATGTATGCTCTTTGGTTGTGGCTCAGTCTCTGAGAGCTCCTAAGGATCCAGGTTAGT
TGACTCTGTTGGTCTTCCTGTGGAGTTCCTATCCCCTTCAGGGCCTTCAATCCTTCCCCCAACTCTTCCACAAGAGTCACCAGTGGA
CAACTTTGTTTAGCTGTGGATATCTGTATCTGTTTCAGTCAGAGGACAGTTATGCTAGGTTCCTGTCTGCAAACATAACAGAGTATC
ATTAATAGTGTCAGGAATTGGTGTTTACCAATGGAATGAGCCTCAAGTCGGGTCAGTTAATGGTTGGCCATTCCTTCAGTCTCTGCT
CCGTCTTTGTCCCTGAATTTCTTTTAGACAGGACAAATTTTGGGCCGACATTTGTGGGTTGGGTTGGTGTCTTTACCCCTCCTCTGG
TGGTCCTGTCTGACTACAGGAGGTGGCCTCTTCAGGTTCCATATCCCGTGTTATGTGTCTTGGCTAAGGTCATCCAGATTGAC
TCCTGGGAGCCTCCCCCATCCCAGATCGTGTGGGACTTCCTAGAAATTTTCCCCAGCCCCCAACCCCAGCAGCTGCAGATTTACATTT
ATTTACATTTATTCTCCTGGCCCTCAGGCCCTCTCTCCTGTCTCTCCCAACACCTGATCCTGCCCCTCAACCCTTTTCTCTTACCTG
CCAGTGCACTGGCATCCTGGACCACAGCCCTTCATCCTTATGGGACATCCCTCTTTCTATGGTATGAGTTCTAGGCTCTTGCAGCAT
CCTTCTGCTGCCCTGCTTGGTCCTCTCCAGACACTCATATTCCTCCCCCATGGTCCTTCCTTATTTAAAAATAGCCTTGATGCCATT
TCTCAAACCATTTCTCATCTCTGGAGGATCCCACACCCTCCTTGTGTCTCATGGATAGGCTGTGCTCTGGCCTGATCATAGCCTCTG
GTCATAGTGTTCTGCCCAAGGTCTTATTTGCATGCACTTTCCTTAATGTAGGCACCAGAATGGGACACGTTTTCCATAAGTTCTTCA
GTCCCTCTAGAGGGCTGTTACCTTTGAGCTCTCCCCTTCTACACAGAGCTGCATCTTGAGCCAGGCTGTGCTGGTCTGGCCTGGAAG
AAATTACTATCACATTAGAGAGTGATGTTTAGTTACTCATCAGAAAGGCTGGGCAACACATTTCCAGAGTCGTCTTCCTGTATTAAA
TGAGATTATACATGTGAAAGCACTTGGTAAGAGGCAAATCTCCCTGTGTAAACATTAGTTATTAACCCAGGCTAAGCTGTACTCATA
TCCCAACAGCCAGGGAATGTCGCTGGCTCAGGTTGAATTTCTGGCGAGTTTAACCTTCCGTCTTCTTTCAAATGAACTTTGGCTAAG
TCTATCCATCCAGGGTGGACACTAATCTCTGAAGCCTTAACTTTTACCCTACAGGTTTCAGTTTGGACCTGTCATTGCATAGATCTC
CTGCTTACGTGGGTATTTCTGGCCCATTGCTTTTCAAACTTAGCTGCACCCANNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNGGGGGAGAGAGGGGAGGAAGGAGGGGGGAGAAGAAGTAGAAGAAGAAGAGGAG
GAGGAGGAGGAGGGAGAAGAGGAGGAGGAGAAAGCATTATAACCTTTAGAAGTCCATGGGACTCATCTTTCCAGCCATGGAAGCCTA
AATGTCTAAGGTATGTTTTGAAAGCTTGCTGCTGGTGCTGAGGCTGTAGCGCACTGATGGCATTCTTGCTCAGTATGCACAGAGTCC
TGGCTTGTATCCCCTTCAGTGACAAAAATCAAAATGATCGGCAGGTGGTAGATGGTTAGATGACAGGGAGTTAGCAAGTACGTACAT
AAATTAAGCAGCAGTGCTCAGATGGGCTTTCCTCTTGTAGTCTTCACCATGGGTGAGGAGACTTCTGGAGGATATGATGATTGGCAA
TTTTACCTCCCTTTGGACAGCAGCAAGCTAGAAAGCCTCAAACTTTTCATTGAGGTGTGTGTGTCTGGGGGGGCATCTGGTTCAGTAG
GTCCAGACAGGGTATAATTTGGTGGAATGAGTGTTATATTAAAAAAAAAAAAAGTCTTACAGCTGACTCGAAAACCCAGGCTGTGGTT
TATTTTTTTCAGGGTGGGAGAGATGTGTCTTGACCCTTCCTTCCGTTTTTGAGGGAGGCAGATGGGCGGTGTGCAAATACTTGTGAAG
GGTGATTGATTGCACCAGCACCGGAAGCTGCTGCTATTCACTTACGGGGATAAAAAAAATGAAAAATGTAGCCCAGTTAATCCCGCA
AGGAATTTCTTATTAATCCAAAAGTGCCAGGTTATTTTTAAAACCCGTGGGCCTTAAAAAACAAAATGAGAGCATGTCTTTAAAGAGAG
CGTGTGCCATAGTGGGTATGCCAACAGGCAGGGCCGGAGAGGGAAGGAAGCCAGTTAGAGCAGGCAGGGTGGGAAGCTCTGCACCCG
CCTGTGGGATCTGCCTGTGTCACCGCAGATGGAGAGCTGATTACCACACACGGCCTCCCTCTGGAGCACCCACAGTGCTTCCTGACA
ACTGTCCTATCCTGGGCTGCCCAGACCTCTGACCCTGGGCACTCTCTTCAGAGGGAGGCCTTGACAGAGGTGACTGCTGCAGAGCAG
GGAAGGAACATTCCTTCCTCCCAGATCCAGGTCATCAATGTAGCTGCCAGGACAGGTAACAGCAAAGCAAACGTGGGAGACAAATAGG
ATGTCTCAGTTTTCTTTGCAAAGAAGTTTCAGTGTATAATATCCAAAGCTTTCTGCATAGAGCAGGCTCCCTCCCTCTTTCCTTTCC
TTTCCTTTCCTTTCCTTTCCTTTCCTTTCCTTTCCTTTCCTTTCCTTTCCTTTCCTTTCCTTTCCTTTCCTCTTTCTTTGCC
ATTCTTCTACCTCCTAGTCTCTCTCTTCCTTCCCCCAGCCCCTTAAAAATTACATATTTTGATTATAGCCAGGTACCCAAAGTGGAG
ATATTTTGAAAAAGTTGAAATTTTGAATAAAAATATCCATTATCGGAGACATTTCACTGGGGCTAGACCATCTATCTCCTAGAATGG
AAAACAAAACGAAAAGAAATAAAAAGCTTAAAACCAGAGTGGCATCAAAAAGCAATGTTATCCCTGTGGTACTATCCGTATGTGTGT
AGAGGATTGCTAATTATGAATTGTAAGGTGTAATTTAAAAATGTATAATCACCAACCTTATTAGCAGAGTAGTTTCAGGAGTGCGTG
GTAGGACAGTTTGCCACCTCGCTGCAGGCTTGGCGATGCTCTGCCCGCTTCCAGCAAGTGTTTACTGTCTAGATGTAGAATTCATGA
CATAGGCTCATCTACTTGCCCAACGTTCTTTCTAAATTACTCTTGGCACCAAGGCTTGAATTTCTAAAAATCTGGAGGGAACAGCTG
GTGCTCTAATAAATATTCAGGCCAGCTATTTTAATGCTTTGTACTCAATTAAGTAGTCCTAATAAAAGAGGAGCCCTTGCCCTAAGC
ATTTCTCAAAGAATAATGTTTTGCTTCTAACAAAAAACTTTCTCCCAGTGTTAGTTAATATTTCTCCCAGGCAACTTTAAAACTTCG
TTGCATCCAGTGAGTCTTTCTGTGACGAGGCTCAGTCCTGAAGTCTGCAGTTTCTTTAACTGACGACGATATCTTTTAACTGACGAC
GACCATAGGCCACTCATTCACTTTCTAGGGAAATTCTCGGGCTTTGTTTTTTGAGATGGTCTGTTCAAGACTGTCCTTCTGACTGGC
CTTGAACTCATGACTCTTCAGCCTTAGCATCGCAAGTGCTGAGATCGCTGACATGTGCTACCACATCCAGGGTTTACCTGACATAGC
TTTACACAGTAACCCATCGTGCAGCTTGGCTTAGGCCTCTGGTGCTCTTGGGTGATATGGAGTCTGCTGTTAAGCATTTGGGCTTCA
AGAGTCGGCAAGTTTTCTTCTACACCATTCTAGCCTTGGGACCTTAGGCAAATTATTTAGTTGCTGGTGAAGTCTTCTTTTGCAAA
ATGGTACGTTTAGTAGTAGTTTTAATATTATTATTTTGTTAGGACCTGCATTTGAATGTGTCATTTTTACAAGTACCAAACAAGCAAA
CAAGCAAGCAATAGTCTTACCATCAAGATACATCCCCAGCAATTTATATTCTCCTTCACCTCTATTTTGAGACACAGTTTTACTGTG
TATCTCTGTGTATTTTGAAACTTACTATGTAGACCATACTGGGCTCGAACAGTCAGAAATCTGCCTGCCTCTGCCTCCTGTGTGCTG
GGATTAAAGACGTGGAGCACCACTGGTTAATTACTATTCTGTTTTTCTTTTTAAAGATTTATTTTATGTATATGAGTCCACTGTAGG
TGTGTTCAGACACACCAGAAGAGGGCATCGGATCCCATTAGAGATGGTTGTGAGCCACCATGTGGTTGCTGGGAATTGAACTCAGGA
CCTCTGGAAGAGCTGTCAGTGTTCTTAACTACTGAGCCATCTCTCCAGCCCTAATTAATATTCTTAACGGGACTATTAAAACCTAGA
```

137

```
ATAATCTATCTCAGTTGTGAGGATGAGAGGAAAGATGTGTAAGCTGCCTGAATGTCCCATCTGTATTGGCAGTTACTACTGGTTTCT
CTACCCCATCATGTCTTTCGTTAGCTTTCCATCCCTTCTTCAGGACCGTTAAGATAATGGTACCATGTAACATACACTTTCTGCTCT
CTGCGCAAGTGTATTCTCAGAATAACTGAAGAAAAGTAGATCCAGCAACTGTTCTCAGTGCTCTTCAGACTGTCCTGGATAGAACAG
AATTCCTATCATGAAGACCAGGCCATGAATTCATAGCTATTGTTTATTTATTGTTATTCTCTTACTTCCAGTACCCAGTGATTTGTT
CAGTGCCGTGACCAAATACCGGTGAGAGACAGTGAAAGGGAGAGAGATTACTTTTGCTCACCTTTCAGAGGTATAGCCTGTCATG
CTGGGGAAGGGATGCCGTGTTTGTGGCTGGGTGGTTGTATGGTTAGAGCTCCTCACATTTTGATAGGCCAAGATGTGGAGAAAGTGG
AATGCCGGCTCTTGTGTTTTCTAGGTGGTCAAGTCAACTTGACAATGAGACGAACCATCACAGCTGGTTAGAGTGAATCTTTTTTTT
TTTTTTTTTTTTTTTTTTGCAAAACCATACTGCCCCATATTAGAATTTTGTACCAGGAAGTCAGGTTTCAGAAGCTCAGCTCTGCT
ACTAAGCCTGACATTTAGTATCAGGGTGTAGGATGGGGATTGAAAGTACTTTATTAGCGTCTCCTAAATGTGGGGATTCTAAAGCTC
TCTAGGTGGCCTGACTATAGTTCTAGCTTTTTATTGTAATGTAAACACTACATATCAGTGCCATGTTTTATCACACACACACATACA
CATACACACACACACACACACACACACACACACACACACACACACTGCATTCTTCACGTCATGAGAATGAACACCCTGCCAGAGATA
GCAATGACTTGTGAGTTAACCCTCATGTGCCCTTTCAAGTGACGCCCAGTTAGGCTTTGGCCTGTACCACCCACATCACCGAAGGAT
TGGGCCACTCATCTCTGTGAATGAGTGATGGACTGACTGGCTTCTCTGGTACTTAAATGAGATGTGCTGTGCACTTGTATCTCAATA
TGGCTGGCTGACATGGAAGGCACTGGCTGCCATAGCTCACCAGCTCACCACGGTCTCCCTTAACCCTGGCAGAAAAGGCATCACAGT
TCATGCAAGGCTGGAGGCATAAAGAACCTTGCTCCTGGGTTGCCATGTAGAAGGTGTGCCGAAGACCCTGTACTTTGTCCTCTGGAA
CCCTATCATCTCTAGGAATCTTACTCCCTATGATTCCAAGTGCAACCTCCAAGGGCCAATCATTGTAAGAGTAGCATGGCCTTGGAC
AAACTTGTGTCTTCACAGAGTCTTTCAGGTGCCTGCATGGTGTATGAGACTCCCGTAACCCATACTTGGGTTTGGTAGTCAAGAAAG
GGGCCTGTGATGTGGCATGTACCTTGAGCAATCTCCCCCTTGATGCTTCCTGTTTGATAATGACATTTTGAGTCACAAGGATCCGCC
CATTTCAAGTTTTTTTTTCTTTTAAGTCAATTCTGTGCTTAAACATTAAATATACTATTTAAGAAGGGGAAAATTAAAAAAAAATATC
TCTACTCAGAGGCACACAATGACAAAAACATGGCCTATTCACTTGTAGGTAGCTGTTGGGAACTGCTGGCAGGCCGAGGCCCAGGCT
TGCTTCCTTTGTTATAGAGGAAGTTAACAGGTGTAAGAGTATTTGGCAGTTTGGCACCAGTTGAAGAATATCTCCTTTACCAGAGCA
AAGAATGAATAAAACGGAGAGGAAAGAGCATCCCCACTCAAGGTAATTTATGGCCATGGTTTGAAGTTGTCTCCTCTGGAACCCCAC
ATCTCTCAGAATCTTCCTTTGGGAAATTCCATGTTTAAGCAGTTTGCAAGGCCATATGTGTTTATGAGTTGAAAGTCACAGTTTCTG
GAGGTGCCCTGTTTGTAGAATGTGTTAACATGTTTGTCTTTCGCGTATTCTGTTTCTTTAGAGTGCTGGGCTTGGGAAGCAGAAGTC
TCCTCGACTTCTGAAAACTTGTGAACCCTTGGTCACTGAGTAGTTAGAGTTGAGGTACAAAGGGAAAATAATTTGGGGGTGTATCTC
AGACTCACAGGAAGTCAACCTTAAATGTTTCTGCAGTCTGTCTTAGGAGGTCCACCTTCTCCTGTTTACTTTATGGTTGAGTTCAA
CATGGCTTAAATCTTGTTATTTGATGCACTTTTAAGGAACAGGATAATTATTGTGTGCTTTTCTATTGGGAAAGCAATATAAAAACA
TGGATAGAATTTTGAAGTAAGGATGCTCATAATCCTAGTAATACTTTCATTGGGTTCAACTGGAACTGGAAGCTTCTATCCAAATGC
TGAAAGCAGCAAACAACTGCACAAAACCAACCAACTACCCAGCCATCCAGCCAACCCACCATCCATCCACCCATCCAGACAACTATG
AACCATCCATCTATCCATCCATCCATCCATCCATCATCCATCCATCCATCCATCCAGACAACTATGAACCATCCATCTATCCATCCATCC
ATCCATCCATCCATCCATCCATCCATCCACCCATCCATCCAGACAACTATGAACCATCCATCCATCCATCCATCCATCCATCCATCCAT
TCCATCCATCCAACCCACCATCTATCCATCCATCCAGACAATTATGATCCACCATCCATCCATCCATCCATCCATCCATCCAT
CTATCCATCCAGCGAGCCAGCTAGCCAGCCAGTCATCTTAGAGTTTCTCTCTAGTCATCCGTGAGGCTATGACTTTAGCCTCACTTT
GAAGTGGATACTGCTACATCACTACCCTTTAAGCGTTTGTTTTGCCTTTACTAGCGTCTTCCCCCTCAGGACAACATTGAGAATAAG
TGTTTTCAGCATACAGAGCAATGCCTTGTGAATAAACACTGGTTTCAGTCAACCAGAAGAGGGAAAGAAAAGGAAAATCAATAAACC
TCCTGCAGGCATTTGAGAAACTGTGTACTTTTAAGAGAAGGAATGGCCAGTGATAAGGAGCTTTGCAAAACAATAGCTCTCATCTCT
TCCTTTTTTGAGCCGCTGACTATGACTTTCTGTGCTGAACCTTGTAGCTGGGATTTCCTAGTTCTCTGTTGCCTTGGTTGTGCCCCA
CTTAGCTGGGGAATGACAGCCACTGTCCCTGTGACAGCTCAAGGGGAGCAGCTCTACAGCCTTCCTAGATTTTGAATGTCATAGCAG
GAATCCTAAGGAGACCAGTGCTCCTTTGGTTCTGGCTCCTTTTGCTTTGCCTGCTGGTCTTGAGAATCAAGAGTGAAGAGCTGCCTG
GGTGTATATGGCATCTTCATTTTTCCTTAGGTCTGCAAAATTTAACCATTCTTGGTATTCTCGCTGCATGCTTAGAGTGTTCTTCAA
TTGCTATCTGGTGAGAGTTTGAAACAGCTGTATAAGAGTTTGAAATAGTGTTTACTTCTATTTTTCCTTAAGATGAAGAATCAACA
TTGTAGAAATGAGTGGAAGGCACCAAAGCGACCTGGAAATATCTCTTACACTTTTGTAAAATATATGATTATTTAGTTTAAATACAG
ACATGTGTCACTGATGGCTGTTAAATAGCAATTTCAATATTTGGATTGTCTTAAATATAGATATGTGCTGTCTTTTCGTAGATATGT.
ACAGTAATGATCTGAATGACTGATATGTGACCTTTAAATATTTTTATCCATGTTAAGAGAATAAAATGTCTTTATCATAGCATACTA
TAACATTTCAAACTATATGGGACGTTTGTTTATTTTTTTGAGACAAGTTAGCTCAGGCTGGTCTCTTAACTCATGGCAGTCTTTGT
GTCACAGCCTTCCCAGTGACGGAATTATAGGCATGAGCTGCCAAGTCCCCGTTCTCTCACTCCATTTGGCTCGACTTGA
GATCAGCTTCGATATTTACAGTGCACACAGGAAGAACCCTGTTGTGTTTCTCATTTGTAGATGGGTCAGAAAGTATATGTATGATAT
GTATAATCATCTCACCCTGAACTGTGGGAGATTGTTGCGTCACAGCTGGGGATAACAGCCCATTCTTTATTGCACCTTTCAAAACAC
AGTTTTAATTTTGAAGTTCCTTCCCATGGAATTTTCCTTCTCTTGCCCCGTTCTTTCTTTGACTTTAACTCCAGGATCGAGGAGGCA
GGGAATCTTCTTAAGGGAGGCTAAACCCCTCTTAGTCTTCAAAATAAAAAGGCTAGCCTGCTGCAGACAGAATAGAGGATTGGGATT
CTTTTGAACAGAAATGGCTGTGCAAATATTTAATCCGTGTTCTTTAGTTGGTCTATTTAGGGCAGATACTGGAAGGAACCGTGCT
TTAATTGGCCTTCTGCAGACCTGATTATGTAACCTTAAGACTTGCATGTTGAAAAGGACGTGAAAAATCCTCTAGTCTCTGTTACCT
ACCAGTCATCTTTCTGCAATCTCCTCTTAAGGATGGATTTTATTCTCCGGCTGTAAGTTATAAAACAGGGGACAGAGCTCTTTTTTT
CTGGCAGCTTTTCATTTTATAGGTCTGAGATGCCCTCAGCCTTACTTGTAGGGGCAGTGTGCAGAGAGGACAGTCAGAGTCCCTGTG
GGTTCGCTCACGGGTCAGGTTTTAGCCTGGCCAGTGGTGAGGATGTCTGTATACCATTTACACACTGATCTGGGGTGAAGGTGGCAT
TTGGCAGTGCTATTGGTAGGATGCCTGAGCGCCGAATCACCCGAGCTGCCAAGTCCCCGTTCTCTCACTCCATTTGGCTCGACTTGA
CAGTTTTGGCTCAGTCATTTTGATTAACATCAACCTTGCTTATGTTAAGTAACTATTCAGGTTATTTATTATGTATTACTCTCATGC
TTTGGAATGAGCGGCCAAGAAACTTCTGCCTCATATGGACTCCATATGCAGCTGTTAAGTTAGCAGAAACACAATTAGTTGGCGCCT
AAGCTGCATATTGAGTAGATTAGGCCGGATCGCACAGGCGTTGTTTGAGATTGTTGTCTCCAATATTTAAGAAAGCTATTAGGTGTT
TGAGGATGTGGGGTGATAAAGCATCTCTGAGTTCACATATGTCCTGTTTTGTATTTTTATTATGGAGTGCAAATATCTGTGGAATCT
TGGTTGTTTGTTTTGCACTGCTGAGGTCCAGAGAGGATTAAAAACATAGTCTGTGAAAAACAGACATCTCTTTATTTCCATTGTTCC
CTCCCCATCCCAGGCTAGGCCGAGAATGTTGCAGTGGGTAAAAGACAGCCTGGGCTCAGATGTAGGCCCTGACACTCCTACCTGTGT
GAGTATGTATAGGCAACATCTTTAGGGTCACCCCAGACACCAGCATGCTAAGGAGTGCAGGTGAACAGGGACTTCCAGAGAGTACCC
CACTGTCACGGTGTGCTCTGGGTCTCTGTCCCTTGAGAAGGGTGTTGTCAGGTTTAGGATGTGTTCCCTGAAGGATGGAATCTGTCA
CTTTTCAAGCTTGCTTTCCATTGTCCTGCTCCCACGGAAGGATCTTGGAAGTCCATCCTTTTCCTTGGCTAGTAGATGGTAAGGTTT
GGCCTGGGGACAGCTGCCTCTTACCGTGTCCATGTGTTGCAGTGCTCGTTCTGTGCTGTCACCGAGCTTTCCTCTTACG
TGGGGACCCTGTGTGTGTGCGCATTGTGTGTGTGTAGGTGCCCCGGCGTGCATGGGTGTGACGTGGACGTCATATCTCAGACGCCGTCCAC
CTTGTTTTCTGAGACTGGGTCTCTCCCTGGCATGGAACTCACCAGTTAGGTTGCACTGGCATACAAGACATCTCCTGTCTCTGCCTT
GTGAGTACTGGGATTGCAAATGTGTACCACTGTACTTAGCTTTTTTTTTTCTTTGTTTTTTTAAAGTGGCTTTTAGGGAATCAAACTT
AGGTTCCCTATGTTTGTGTAGCAAGCGTTTTACCAACTAAATTATTTTCCCAGCTAGGGGGGTCCTTTTGAGGCCATTTATATCCGCA
TTATAATTACAATTAAATATACATAAGTATGCCTAAAAACTGCTTAAAATATACTGAGTAATAATTATCCATTGAAATAATTTAATT
AAATTTGAAGTAATTTTTTTTTTTCATTAAGGAACAAAATAAGCATCCATTTGGCCCCCACTACAAAAAAATTGCTATACACAAATAG
GTATGCTTATGAAATTACTTAGTTTATTAGCATATAATTCATCTCAGATTATTACATTCTCTTAATTGAATTTACACTTGTATGCTTA
TAATTTTAAAAATAGTTTTAAACAAGAGGTGTCTACGAGCTGCCGAACATTAAGGGCTCTGGTATTTTTAGTTGTCTGAGAGGTAAA
ATTCTAGGTCTAAAGGGAAAACATGGATGCTATAGTAAAAGATAACTCTCCTGCTCTGTGCTGTGAGCATCTGCAGCAGAGTTTGGA
TTGATTTTCACCTTCTGAGCTGTCATAGACGATTTCTGTTCCTTCCTTGACATAGGAGATTCCTGTAGCTCAACCCCTTACCTAGTCT
```

```
GAGGCCATCCTCTGCCTCTTCACAAAAGTTCTAAAGGTGAAAAAGTGGGCTGGGGAGGGAGATGAGAAGCCAAAGCTGAGGTTGGTT
ACCACACCGAGGGACGTGCTTGCTTCTTGGAGTTGTGTCTTGTGGATGTCAAAGCCCCTTTCCAGGAAGGGCCTTACTGTTTGCTGC
CGTGATGGGAAGAGTGTATTGAGGCTCGTGAGAAGCAATGGCTGAGATTGGGAGCTGCAGAGGGATACTCATCTATGTTTGGAAGCC
CACACAGGCATTGGGTGATTTCTTGGACAGCGTTCCTGGCGGTTGTAGTGACAGTGAGCTTTACCTGGGCCATTGCTCTGGTTGTTC
ATAGGGAGCAGCCTTCATCCTTCAGACATGATGTAATGTCTTTCTCCAGACAAAGAGCAGCCTCTGTGGACAGTGGGGCCCCAGAAT
CCCAAACATTGCTCCTCAGCTATGATGCCTACCCACTGCATGGACAGCATTCACCTTTGACCTTTAGAGCAAAGAGAACCCAGGCCA
GAATGATGTTCCTGCTGCCTCCTGTGCTCTGTATGGTAAGTCCTTAGTGCCTGGTCCAGAAATCTTTGTCGTCCTCTTCTTCTTCTC
CTTCTCCTCCTCCTCCTCCTCCTCCTTCTCCTCCTTCTTCTTCTTCTTTTTTTTTTCCTGAGACAGGGTTTCTCTGTGTAGCCCGGG
CTGTCCTGGAACTTACTTTGTAGACCAGGCTGGCCTCGAACTCAGAAATCCGCCTGCCTCTGCCTCCCGAATGCTGGGATTAAAGGC
GTGTGCCACCAACGCCCAGCTTCTTTCAGGGTTCAAAAAGCAGACGCAAAGGCGAGGGTTGGTAGTGCCTCATAGCTGGTCAGGTTT
CTTAGCGGCAAGGATGTCATCTTAGATACTTCTCTGTGTGTCAGTGTGCCTCCAGGCAAGCAAAGGAGACAGACCACAGTACCTTCA
GGAATTGCGTCCTGTGTTGGATGAGATTGAAGCTGAGTCCAAGTGTGATTGATTCTTGGTCGTAGAAAAGGAGTCTGACACCAGGAG
TGTGGGCAGCCCCTTCAGGGACAAGCTGTAGCCCATCTGAGGCAAAAATGCAGGGTTAGCCAGATGCCTGGCCCTTAAGAACAGATG
GGAGTGTCCTAGCAAGGTGCTCCTACCACGAGGCAGCAACGGCCTGTCTTGACAGCAGTGGGCCTCTGATGAAACAGGGCCAGAGAG
ATCTCGAAATCTGTGTCTGGTATTTGTAAGTTGTTTTCAGGCACTCAGCCATGCTGTTGGAGAAGGCCTCAGAGCTCACCTCAGAGC
TCACCTGAGGCACGTTAGGGACGAGTTGATGATTGATTCTCAGCTCGCTCAGCTGGGCGGAGCCAAGGCTAGTCCTTGGTTCAGAGCA
ATTTTGAGTGCATTTCTTTGTGAGTTCTACATGCACTTGCCATCTGTACTGGGACTCTTCACCCCCCCCCCCCACTCGCCGAGAGGG
ATAATATATAATACAGGTTTAAAATGAAGACTTTAGATGCTAGATGGTAAGAGGTGCCCAGGTAGGAAACCTGATGTGGAATCTTAG
TCCTATGATTTCCAGGATGAATCTTACAGGCATCTAGTCAGACCATAAACAGAAATGACATTTCGTTCACTTAATGATAGGTGAAAA
ATTATTCACTGGCTGTGGCTGCTAATTGAGGTCCAGCATGAGTCAGGCTAGAACTTAGGAAGTCAGGGACAGGGTAAAAAATAAGCA
TGGATCAGGAAGTGGATTTTAATAAGATATCCATGGGAGGGGGACTCTTGCTATGTGAGGGTTAGATAAATATGATGATTTCCACTG
GTGTCGTGCTGCTGTGTCTTTGGAAGTGGGAGGGGTGGGGAAGGGGAGTCAGGGCTCCTAAAGTGCCACTCTCCAAGCTTTAGCTGACA
TAGAAAGCAGGCGGTGCTTGTAAAGTCAAATGCTTTTTGGCATTTCTGGTGGTGTAAATTCCATCGTGTCTCTACCATTTTGAAGAA
CATCTTGGTGTGGTAGGAGTGAGGGGCTTCTTGTGGTAGGGCTCCCTGCTAAAGGAGAGAGGTGGCTCAGCAAGATGGCTGAGGCAT
GGGGACTTTGCAGAAGGGCCCAAGACAACACCCACTCTGAGTACTTCTCAGCCAGTCCCCACAGGGAACAACACCAAACACTGTGGT
TGCTGAAGCTGTGCCCTTGAAGCTAAGGGAGCATGGAATGCTAGTGTGAATCAGAGTGGGTGTGGCCATTCAGGGACAAAATTCCCA
TCCGTTGGCTGTGGTTCTTTTTATCTTTAGTTAATGATTTCATTCTTTAAAAATTAAAAACAAAACAAAACAAAAACTAACCA
CGTCAATTTTCACATGTTTATGGTGTTGTTTGTGGATTAGTGGTCTGTAATTACCTCAACTCCTGGGCACGTATATTTGTTATCAC
AGTGAAGAGGGCGTACCAATTATTTTAAAAAGCAATTTCTCTTTTAATTTATGCACGTGCGATATTAGCTAGCTTAAATGCATATGC
ATAGAAAGATACGTAGTCTCTCTACATTGACCTGGCTGTCCTAGAACTCAGTACGTCGATGAGGCTGGCCTCAAACTCACAGAGCTC
CACCTGCCTCTGCCTCCCAAGTTCTGGGAACATAGGTATGTGCAACCATGCCTGGAGAAAGATAATTATATATAGCTATTTATTATA
TAGCTATTTTATTGCAAATAATTAAAGGAAAGAAAGACTTGAATATTAACATACTTTTCATTGCCATTGAACATAAATTCTAAAATG
CTGAAGTTTTTTTTCCAGGGGTTTAGAGAGGACTGAAAAGACACTTAAAGTAGGGCTGTTCCCAACTTCACATCCAGGTACCCATAGTT
GCCTACTGTCAGCGCCTATTTCTGGGTTACCATTTGACGTGCGACACTGCCCATGCTAACTTCTCCTAGTCCCTTGGCTCCTGTCAA
AGGAGCTACGTCAGTGCATCAGATGCCTCCCTAGGAGCCTAGAGGACCTGTCCTGTTTGTCACCTGCTTTCCCTCTGCCTCTCTACA
GACACACAGGGCTTGTATTTGACAGATTTGATAGTGGGAGTCAGAAGGTTTTGGTATCAGAGGGTTGGAGTTTGAATCTGTCTGTCT
GTCTGTCTCTGATGGTGGTGCTGGGATTCCCAGCCATCATATCTCTTGTTTATTTAATAGGAGAGTTATTTTGCCTCCTGTTTAAATG
TTCGAGTCTTGGTTTCCTTGAGGATCAAGGGTGTGTTGGGAGACAGTTCTTTTAGGTCACTTGTGTTTTTGCATGGGGTTGGTTAG
GGTGTTGGTTGCTTTTGATCTGAAGGATGTCTCTATAAAAGAAGCCTAGTAAGGTTGGTGACAGTCACATAACATTTGGGCTCCTT
AAGCTCAGGAATCCTCAGTTACTCTGTGAGCCCTCGGCAATGTCCTCTGGCAGTCTGGGCTTGGGGAACCAGCACTTGACTTCTGGG
AGCTAAGGCCTGCCTGAACCCTGGATGAAACCCCATCATTTAGGTCTTTTCCTACAAGGACCCAGACAGCCATAGTAAAATGGGCCT
CTGAGTCAGTGCCATTGGGGGGGTCATTATGACAGGGAACTATGGATCCGTGTAAGGTGGAAAGAAGGGGAGAGATCTGATGCCTGAACGTTTT
CCTGACTGCTATATGTCACACAACTCATCCCCCCCCTCCTCTCTTGCACACACACACACACACACACACACACACATTTGTCATA
TATAATACTGAGGAATGTGGGTCAGTGTGTAATGTGTGCTTAGCCGTGGGTCCAGTATGTAACATCACAAAAACAAAACAAAATTCT
AAATTTGTTGTAAAATTTGAAGGGACAGTTAATGTAACTTTTCCTTTTGGTGAGTTTGGCACATGGGGTGTGGATAAGATCTCCCAG
TCAGAAGTGCCACTCGAGGCCCTGTGATTTCTTTAGAGTGGCTGGCTCAGGGCAGCCTTCTTTGTTGTTTTGGAGTCTGCCCTTTCT
TTTCTCTCCCTGAACCCCCATGTTCTACCTGATGATCTTTTGAGTCACGGCAGATTGTTTTTTAAGGGATGTATCAAAGTTGCCTTTAA
ATGGTTGACTTACAAGCTGGCTGTTAAAGCGAGTTTTACAGGCTACCAGATGAGATGGAGTCAGTGGTCTGGCTAGGTGTACTGTTG
CAACAGTTTTAAAAGACAGTGCCAAGATGAAGACATCCATCAGCAACCCATTGCGTTACATAGGGGTCCCGTGGTAGATGGTACCAT
CACAGTTCCTTGTCATGATGCCCCCCACCCCCCAAAGGTGTCTCACTTAGATCTTTCTGGTCCGTGTGGGAGAAGATCTAGATGAGA
GACTTTCATCCTAGGCTTCGGCAAGGCTTGGCTCCCAGAAGGCATTCCATAAATTGTTTTGTACGCCTGAGTGACTAAAAAAACATG
AAAGTGCTTAGTTAACTGTAAAGTGTTAGTTGCATGTGAATCTATCTATCTCCCCCATCCCTGTCTGCAAAGTAGACCGATGCTCAGGTG
TGGCAGAGGAGAGCCAAAGGAGGAGATCTGGGGTGATGGAAAGTGGCCTCCACACTAGCCTTCCTCTCCTGAAAGGTTGACAGGCTA
GGCTGTGAAGCAGTTTCCTCCCTCCCTCCCTCTCCTCCCTTCCCTCCCCTCTCCTCCNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NCCCCAAAGGTGGAACAGTTTCTAAGGAGCAGATGGTCCCAGCTCCTGTTATCCATTTATAACAACCCCAGAGATCTTTCTAAAGGA
TGGGTGTCGTTCATCTCTTTTACCACTTTCATCTTATAGACTTTAATTATTTCTACCAAAATTCTCCCCATCACGTTTCTGTCTTCA
TCTGTAGGGATTTTATCTGGCACCATTTTCTTGTGTCCAAAATATCTTTTCTCCCAGAGCTATAGGCTCCTTTCATGATTCTTGGGT
GCTTTCTGGAACCCTCTGCTGAGTGAGCTGGAATATATCTGGTAGGATAAGGGCAAGGTCACTATGTGCTTTTAGAGTCTGTCCTTG
GGACAGAAGGACTACCAAGGAACTCCTTCCATGTGTGAGGGTGATTTCTGCAGCAACCACTTACAAGCGGCACAGGTGAAACCATTCA
TTTAACGAGTGGAATTTATAACAAAGGAGAAAGGTACATTTTCATGCATGTCACTTGATAGTATTTGAAATAAAAGTTGTCATTAGT
TTCTACTGTCATGATGATTTTGTTTTAATTAACAAATTGTTGAGTGTGTTTGTGTGAGTGCCTACATGTTAGCGGGAGGATCAGAGC
AGCGCTTCCAAAGGCCAGATCATTTCTTCCACTGCGGTTCCAGCGACTGAAGCCAGGCTGTCAGGTTTCTGCAACAAGTATCTTTAC
CTGATGGGTTCTGTCACCAGCTCTGGTTGTTCTGTAAGTGTCTGGGAATCCTTGCTTTTCTATGTAGACCAGAGAGTCTAGATGCTG
TTAGGATGTATTTCTTCATATAGCACCATATAACTTCGGAGGTACTGGTTCAGGGTGGCTCCACCGCTGTAACCTTTGCGGCTCCTT
TATCGGATTTCTCTGCAAGGATGACGTGTCAATCATTGTGACTTGTTGTTTGAGAAACAAAGTTCTTCCTCAAAATGATGAAAGTAAGC
GTGGACTGGATGTGATTTTAAGATTTTTCAATTTGGAAAAGCACCCAGGGTTCTGGTGCTCCGTCTGTCTATAGAGGAAGGCTGATGT
CATCACTCAGCGTCACTCAGCAACTTTGTCCCTGGCAGTGCTACTGGGTGGCATTCTAAACAGGTCACCACATACATGGAGAATTAC
TGAGTAGTTTAGGGGGCCGCGTCTTGTTAGGATCGCTGAACAGCGCGGTGATTTATGGATGTGGAGAGCTGTGGCAGGGTAATTTTT
AGCATTTACATTTCATTTTACCACAATCTCGAGGGAATTTTGAGGCTGCGTGAACAAGATTACAAGGCCACCCTCAGGCAATTTAGG
ATTATATAAGGAATCTTCTTTGCTCCCTCACACCCAGGCTCCGTCTTTCTAGCCCACCCAGAATCCTGGTAACCTTACTCCCTCCCA
GATTGGGCGTGGGTCTGTTAGTCCTTCTCTATTGACACCACCCTGGTCCAGCTTCCAGCCTCTCCCTGAAACTGTTACCTACATCCT
GATTTTCTTCTATGGTCCCTGTAACCAACACCTGTGTCAGTTTGTCCTTGCCCCAAAAGTGACCTGTCTCCCTTCCCCAGAGTTACC
TTGAGGTCTTATGCCTTTCTTCCTTCCTTTCCTTTTCTTCCTTCCTTCCTTCCTTCCTTCCTTCCTTCCTTTCTTTCTTTCTT
```

```
TCTTTCTTTCTTTCTTTCTTTCTTTCTCATTCCAGCAATTCCCCTGCCTCCCAGTTAGGTTAACCCTTAGAGGATTCTGTCATACTT
CGCATGCCATTTCCTTCCAAGTATCTTATCCTAACTGGGTCAGATCACTGTTACATGTTCTGGTGATTAATTGGGAACTAAGCGTTC
AGTTTGAAGCGGACGGAGCCATTAATAGTTATATTCCACGCCCACAGAGGGCCTTAGATAAGTGCTCTGGGAGGTTTGGTTTTCAAT
GATCATGTGGTTACAGCCGTAATCCCTGCAAAATTCTTAATACTGACCCCTTTGACAGTTAGTAATTTGAGGTGACAGTATGTATG
GTGATCTTTTTTAAAGTCCCAAGGTGTGAAGCCGTTGAAGTAAAGGACGGAGTTCTCAGGCTTCCAGAGATGGAGAACGGGCTTGTA
GGTGATGCGGTTTTATTTCACTCCTGACTGAAGATACATTTTGCTCTAAAATCTGGCTTCGTATGCAGATGCAGAGGCTTTTTACAT
TTTGAGGAGGGAAGAATATTCTCTGTGCACCTGGGATTTTGGAATGTGATGACGAGGGGCCCTGGGCTGTTACAGAAATGGCAGATA
AACAGAGGGTGGTGAGGGAGGCCCTTCTGCCAGTCAGAGGCTGACAGATTTGATCAGAAGGGGTGGAGGCTGCTGCTTGTATTTTGG
GTAGATTTTCTCGTTCCAAAGGGACTTGGTCTCTAGGTGGCCATTAGTGAGGGGAGTTATGTGATCAGTGGGACCATCTGATAGTGT
GTGTGTGTGTGTGTGTGTGTGTGTGATTAGTGGGGGTGGCATGTGATCAGTGGGGGCTTGTTACTAGCTGGGCCACACGATTAGAGA
GACTGATCATGAAGGCCATGTAGTGAGTGGCGGAGGGGGGGTGCGGGGGGGGGAGCTGTTTGGTTCATTGGGCCATTGATGAGGGTG
GGTCATGTGATCAGTAGGAGCTAGGTGGTTAGTGGTGTGCCAAGGGGACTCGGGCTTCCTTTTCTGTGCCCATGTATACTCAGAAGG
TAGGGTAGACAGTGCCCAGTGAGCTTGATGAATCAGAAAGAACTTGAGGCCCTGGTTTCTAAGACCTGCTCTCAGGACATCCCTGGA
GACTCAGTTCCTCCCTTTGACTCCTGCTGACATTGGCCTGCCTCAGGGAAGGCTCTGAAGAAAGGAGGCACAGCCATGCTTTGCAAGG
TACTTTCAAATTGCTGGGTCCCTTCAGGGGAAAAGAGCCTTTTGTGACACTGGTCTAAAGATAATTTTTATCAGATTCTGTAGCCTG
AGGTCTCAATAACAGCAAAAAGCCCTTCCAGGGCCTGGCTTCCATGGGGGTTGGACCTGATTGAAGTAGCGGATTCCTTCATGCTGG
CCCTGCAGCAGGCCCAGGTACCCCAGGAGTGTTTCCCTGAGAGAACTGTACAGTCAGAAGGAGGTGCCAGAAAATGAGATGAACGTG
CGAGCCCTGGCAGGTGGACTGTGCTTGCTAAGGGCAGGTGTGTGCAGTTCTCACATCACGTATCTATCTCCACAGGGTCAGGGTGGA
ATATGCTCATGACAGGTCACATGCAAATGAGGTTTAGAAGGACGGGCTCTGTTTCATTCCTTTCTGAGTATCAAAGTCATGGGACAG
GGTGTGCTTCACTTGCTGTGGGAGAATAGAGCCACTCTAGCGTTCGAAGCATGGGGGATTCACAGGGCTCATGATGATTTGCCAGCTAG
ATGATTCAGCAGAGTCTTTGCAAGGTTTTGCTAATGGTTAAATATCTACAGCAGGCTGAATTTGGATTGGCTGAGGGACAAGGTGAA
ATAAGGTAATCATAGTTACATGATTTTTTTTTTTCTTCAAGTTCTGGCATCTTGTGTTCTCTGCCAAGCAGAAATATTCCGGCAGAAT
CGTGGTAATGCCTCTCATCTGCAGGCTTCCAGATGGCAGCTTCTCTGCCAGCGAGCAGAAGAGAGCTTTGGAAGCCCAGGGGTATCT
CAGTCCTTTCCTCGGTGTGACCCAGGGAACTCTTGTTCCCTGTTTGTACAGGGTGCCGGTGTCTCTTCAAATGCCTTGTATGAGGCC
TGGTGTGGTAGTGCACAACTGTTATTCCAGCATTTGGGAAGCTGAGGCCAGGAAGATTCTTAGTTCGAGGCCAGCCAAGCCAACATAT
TGAGATATTTTGTCCCCACTGTGAAAAAAAAAAAAAAAAAAACCAGATAAAAAAGCCATCAGATCTTTAAATTTCAAAGTTGAAGGGTGCCATT
TTCCCAATAGCAAATGACCACCTGAGTAGAGCCTGTCAAAGGCCTTCTCAAGAGAACTGTCATCTAAGTGAGCCTTGTTGTTACAG
ACAGAGGCACTCCCTCCTGTGGGCAGCACAGTACACACTGCAATGCTCCATGAACACTGTTTTTCTAGCTGTGACTCACCCTGTGTC
ACGCCCTCCCCAGGCAAGCATTCACTTTAGTGATGTTTATAGATGCGTTTGTCTGGGGCTATGAGATAATTGGGTTACATCTTCTAC
TGGGGGAGCCCATGGGGAGGGGAGGAAAGACACACACAGAGAAAGTGGTGTGTAAGCCTGAGGGTGGGGAGAAGAGTGAGCATTTAA
GGTTGTGCCAGTTGAAGGATTTTTAGGGATTTGCTGCACACAGCGTTGGCCATATTTCCCTATGTGTTCCAGCAGCCTCTAATAGCT
ACCGGCCTTCTCTTGAGGGTCTTGGTTTCGGTCTTCAGCGTTCTTCCCCTTCAGTCCCTTTGAGAACCTTTACAGACTACCCTGGGC
GTGCCCTACGGAAGCTCTTACACTGTTCTGTGTAGATACTCTGAAGCAACGGTTATTTTCCTTGCTAGCCTCGACTGTAACTCACTG
AAGGCAGGCAGTCACATCAAATGCAGTTAGTACAAATGGCAGGCTCGGGCAGTATTAGGGTCAGATAGACCGTGGTGACAGACCTTC
AACACCACTCTTAAATATAGCAGTGTGTGTAGTTTTGAAAACATGGTGCATTCTGTAGGCCTGGGGAGATGGCTTCCGCCAATAAAAT
ACTTGCCTAGCAAGCAAGAGGATGGGAGTTAAGCTCCTGCTGCTCACATGAAAAGGCCCAGTATGGTGGCATGTCCCTGCTATCCCAG
TGCTAAGAATTAGAGACCGGGGATCCCTGGAGTGTAGTGGCCAGCCACGCTAGCCAAATCATGAACTCCAGGTTCAGTGTGAGAACT
TAGCTCAGAGAATAGGTGGCGAGCAAAAGAGTAAGGCACCCAACCTCAACCCCTGGTCTCTACACACACACACACACACACACACAC
ACACACTCCTTATGTGCATACACACCATTGTTTTTGTTTGAGAAAAGCACAAACTTGTAGGCTGCCCATCATCATTTACTTGATGTC
TGTTCATTCATCCATTCTTCATTCATTCTTCATTCATTCACAGGCTATACCTTTTTTTTTTTTTTATTATAAGACCAGATTTTTC
TCTTCAGTGATATTTTTTGAATCTTGGATATTTTTACTGCTCTGGGGATTTTCTTTGAACTTGATAATTATTTTGATAAATCTTTAT
CACTTAGATGGTAGCTTAGAGTTAATTCTTTCTATTAAGTACTTTTTAGAAGATAAACCTTTGGAGAAATCACCACATTTTATTAAT
GACTTGTGAGACTTAGCAGCGACTTACGTTGATAATAATGGAATGCGCTTTGGGTAGAAAAATTCTTCTTGCTGACACACCAAGGTG
GAGATGAAGATGTGTGCAGATTGTTAATTTAGGCCAGTGTGTCAAGATAACAGAAGGCTGGCAGTAGTCTCTGTTGATTTTTCTCTT
GTAGCACCTAAGAGGCAATGGGCACAAGCTTGTGCATGCCAACTTGGACAGCTCTCCATGAAGTGCCTGCGGTATTCTTCATGTGGC
ATGGAGAGGGCCTGCTGGGGTATGGAAAAGTTATGGGGCTTGCCCATGGCCATGGTTTCGTGCCCACAGATAACCCCTCCAAAACTG
GAGGTTTCTGGTTATACAGTATAGATTTACAGTTGGAAGAGTTTGTAATTGTTAAACAATAAATATTCTTTTGCCAGTTGATGCTCA
ATGCTTCTTTTAAACAATATATATATATATATATTTATGATTATTTAGCATTGCCTTTCTTAGTTGTGCTAAGCTGTACATAGCTGT
GACAGTTAATTTCGAACCCATTTGAAAACAATGTTGTCACGTTTTTCATTGCTGAGAGACGTTCAAAGCATCATCATTTCCAAAGCAA
AGGATGGGCTTTGCCTTTGCCACTGGTGAGGAGAATGTCACGTGCAGAATGTTAAAGTCTTCTTTAACAGTAAAAGCTTCTACTCAA
TACCAGCCAACGTGTGCTCTCCGAAGCCTCCTCTAAGACAGGATGTGTAAGATACAGATGCTGGTCAGGGCCTCGTAAGATCTGGGGT
TGTAGGCTGCTGGGGGTAGGCAGTCTCGGGGATGCAGTGTGGAATGCTGGGTATGTATTGTGGAGCTCTGGAGTTCATACAACTCCT
CTGCCTCATAGAGGGATGGTACTTCCCTCTAGTTGGTAGTTGATCTGGAAATGTGTGTATGTGTTGCCAAATCTAACCATCTTCCAA
TAGTAGCTGGAACTCCACATACTTTCAGTTCAGAATCTATTAATTTTAAATCTCAAGAAGAAAGAAAATTCAAATTAAAACAAACAT
TAGACAGCCCTGGGGGTGGAGGTTGACTTTGGAACCTTACTGTATCAGATCTGTGGCCTGTCCTGTTGGCACCATGGTCACGTGCCT
GCTTTCATTGTCCTTTGAAGACTTGGCGATACTCTGTCAGCTATGCGTTCCCATGACTGCCTGCAAGTTCATTTTTTTTCCCCCTG
AAAAATGTAAGTGTTCTCTTCCACCGGATAGAAGACTGGGGGGGCCTTTCCTGAGTCTCAGTTCAAGTGAGTAATCACTCGGGATGTT
TCTCTCGTGCGTTTGTGCTTTTCTGTTCTGCTAGGTAACACATACAGCTTGAGTTAAGGGGTCGTTATCTGGGCCTGCTCTACATTA
CACAGGGATTCTTATAGCTGTGGTGTTTGTTGTAATGAGATTTGACCTGCCAGGGGAGCTGTAGATGCTTGGGCGAGTGTTCAGTCC
TTAATATTGGCATCTAAATTCTAGGCATGTTGCCTGCAACTTCCAGTTTCAAAAAAAAAAAAATCTATGTCAATCTGTTCTTTTATAA
CCTAGTGCAATTGCTTCCCCCCTTTTCCCCCCAACACCCTGTTTTATATTTAATCATTCTCTGCGGCTCGCAACACCAGACCAGTCC
AAAGATTTACTTTTTTCTATTTCCAACCCCATCATTAATTTTATGTAAAAAGTATGTTTTGAGTTAGACAATTGGTGGGGGATCAGGC
TTGGACTCTGTGGGGAGAGGAGAGATGTCATTTACAACAGAGTTTATACAGATGAAATGTTCGCTACGTGCTGAGTTCCACTGTAA
TCCAGGGGGTTTGTTTGCATTTACACTGAAAGAAAAAAGATGACCATATTTGGGCTGACTCTAAGCCAACATGATATAAGCCCCAAA
TTTGCATTTCATTCAGAATATTTCATGCGTGACTCAAGTTTTATAGCCTCCTTTTTTTTTTTCTTTTGGAAACAATGTCAAACCGAG
AGGGAAGTTTCAGAGAAAAACGGAGCTCTGTGTCCCTCCGTATGACTCGACTTCCCAGGTATGTCCTCACGGTGGCCGGTGCGCACC
TCACACTCCCATCTCCTGCCTTACCTTGGTTTTTGTGAATCCTTCCTTCCTTCATCTCTCATGCCAGTGACTGTTTTGATTTCTGAG
GTGAGGCCTTAGTCTTCAGCCCAGGCTGGGTTGCAGCTCCCTGCAGCCTTCCTCAGCCCCTTAAGTGCTGGTTTATGGTATGAGTCA
CCATACCTGGCCTTGGTTGTGACATCTTTTTTTTTTTTTTCTTTTGGATGATGATGGTGTCAGTTGATGGCACAGAATATCCTTCC
TTTCCTCAGAGGATGTGTTCTGTTCTCTTCCCGTACTTACTACAGTTGACTGGCTGGACGCTTGTGTTCCATTCAGTGGGAAAGGCAGGGTGTGT
TCATGAAGACCTGTGAGTTTCTCGCCTCTGGTATTACTACAGTTGATTCCTTAGTCAGATATTCTTTATATGGGTATTTATTTATGTGAT
GTTTATTCTTTCACCTAGTCAAAACACACACACACACACACACACACAGACACATGAGTTTTGAATTCATGGGTTCAATTAATGG
ATCAAAATATTTTGAAAAACGTTTATAGCAGGGTGAATGGCAAGTACCTTTAGTCCCAGCTCTGGGAGGCAGAGGTCATTGGATCTC
TATTACTTTGAGGCCAGCCTGGTCTAAATATTGAGCTCCTGGCCACCCGGGACTCCATAGTAAGGCCCTGTCTCAGAATGCACACTC
ATATTTATTGATTATTTACAGACCGTGTGTTATATCATTTCCTAAACAGCATGATGTAGTGGATATTTCCATCCAATTTACATTTCC
```

```
TGGGAATAATACATAATCTAGAGAAGATCTAAAGCGTATAACGGATACACTTAGGTCCTGTGTGCAAACAAGATACCATTTTATGGAAG
AGGCATAACTATCCAAGGACTTGGTGTTCTGGGGAGCCCCAGAACCAGTTCTGTGAGGAAGCTGAGGGACAGCTGTGCTCTGGAACT
CCTGTGTGCCACGTGGCCTATACTCAGGAGGAAAGCTGGTTAGTAATAAAATCCTGCCCTCGTGCAGCTTAGGTTTTAATGGGGCTA
ACAAATGAATAAGTCGTAAATAAGGTAGATCATGATAAATGCTGGAAGAGAGTCAAGTCAGGGAGGGGAGGAGGGAAGGGAGGAGGG
AGGGGAGAAACAGACAGAGACCGATGTTCTTGATAAGAGGGCAGGGAAGGTCTCCCATGAATTTGATTATGGAGCAAGGATCCCAGG
GATAAGGCCTGGGCAGGGAAGCCTCTGGAGTGAAGGCTTGTTGAGAGGCATCCTGGTAGCTGGAGCCTGGAGGGAAGAGAAAGGTGT
TGTTCCAGTGTTGATGTAAGGGAGATGAGAGACCCTGGGAGAGTTTTGATCCCGGAAGCTGTAAGATCTGACTTGTTGTGCCTTACA
GAGAACACCTTTTGTTATGTAGAGCAGAGCTGGTGAGGGGCAAGGTTAAAGGCAGAAGAGAGAAGAATTAAAGCAGGGTGGGGTAAA
CTGCATAGTGGCATGTCCTCTTGGGACATCTGAGGCACTCAGGGGCCATCAGACTTCTTCATCATCACCACCACCACCATCATCATC
ACCACCACCACCATCTCCACTATCATTACCATCACCATCATCACCACCATCTTTTTTGAGTGTGCACCACCATCCTTGGCTGAGACT
CGAACCTAGAACCAGGCAAACATGCGGCTCTCCAGTTCTTCCCGCCCTCTCTCAAAGTTCAGAGCACACCGGGCTTTCCTGGGCTATC
TGTAAGCAGTGCTGGCTGGCTGCTTTGCCAGGAACCTGGAAGTCTTTCGGTCTCACCTGTGTTAGTGTGAGAGCGGTCCTTGAAGCTT
ATGTTGATAGACACAGGAGCGAGTTGTTTTGGGATGAGTGAGTGATTGGGTTCTGGAAAGTTCTTTAGAGACACTTAGCTATGAAGT
GTTTTGCACTCTTACTGTTTTCGTTTTCCAAGAACACATTTCTTAGCTTAAAAAAATGTGGTCCTATGAATTTTCTCTCAATATTTATC
TGGTACAGAAGGCTATCACTAGGCACAGCATTTTTTGGTTCTTTTTTGAGAGAGAGAAGTTACGTATTTTATGAACTGAACACAAGA
CTCTGACGTCGTCGTTTTCTTTTTCTTCCCCTGGACACTTGGTTTTCAGGCAGAATAAATAAATGCTACCAACAGGACCCACCCACA
GAGAAGCTATTAAATGTTTCAGTAGGAAAGTCGGACTCAGGGGGTAGGTTAAATGAAGCTATTAAAGATGGATAAGAGAGAGAACAT
GATGTTGGGCCCAGTGGGCCAGGTTGCCTGCCCCTTGCATTACATCAGAACCTCAAGGAAGGAGAGGGTTTGGCCCTCTGACCAAAG
GCTGTTAGGGCCAAAGCTTTGAGGGACATTTGGTTCTTTTGTGAATTAACTTCCATTTTGCCTGAATAATGTTGGGTCAATTAAGAA
AAATGAGCCGCGCTTTACCCGGGCGATGCTAAGGTCAAGGGTACACATCCTTTGGAACCGAACTGGATTTGAGTCCCGTTCTGGTGG
GCAAGGCGTGTTACCTTGGCCTGCCTCCAAGGGAAGAACAATCAGTACCCGCTTCCTAAGTTCACTGAGTTAACGTCCGTGCGGCAC
CGTCGCGTCTGACATAAACCCTCAGCAATTAGCCTTTCCTCCTCTCCTTCCTGCTAGTACCATTGTTACCGTCATGAGCATTTTTCA
GTGACTTCAAGTCTATATCTGCTTTCTGGAGTACAGAACACTCTCAGTTATTGCTCTTTGGGGTAAGGGTAGCCTGCGTTGGTCATG
CCTGTTGTATTCCTGCATCCTCTGGTCACAGGAGGTGTGCAGTGTTTGGTATTGTTAATCCCGTGTTTACGGCCAGCCATGGCTGGA
AGCAGCCTGCTGCCCGTGTACGTCTTTTGCCTTTGTCTCTCCCTGTCAAAGAGGGATGTTCCAACTTCCTTCAGGGACATGTGGTGA
AAATTAATGAATAACTTAATGTTTGCGACTCTAGTCCATTAAGTACTAATGAGCATCCGTTACTATGCTGACAGTCTCTGAGCGCTA
GCATCGAACTGCTCTCCGAAGCAGGGGCATTGGTTGAAAGGGCTTTCCGTTATCTGGTGTGGGGGAAGCTTTTAAAACATGTCCCAT
TGCTTTATGTGACTACCGTTCTCCATACCCCAAATTCTAGACCTCCCTTGCCGGAGAAGAAAGAACTCTAATGGAGCAACTTCTCCA
AGGGTTCCGGTAAGAAAGTGCTGTCTCACGGTAATTAATTATGTTTGGGGACCAGCGCCTTTCACTTTTGACACTTACACTTGGTCA
TCTTATGTGAACTGCTTGTGTCTGAACTCCCCAACAGCCTTGTAAATTTCTTGTCACAGATTTATGATGGTCTTGGGTAGCCAAGAC
TCTGTGTGTGTGTGTGTGTGTGTGTGTGTACACCTATTAAGTTTGTATTAAGTGTCGTATTAAGTCTTTGTATTAAGCTTG
TGCTGTAGAGGCAAGATTAGGAGATGTTAATATCTACTATTTGGGGATAGCTATTATTAAATGTAGCCAATACAGTGATTTTATTAT
TATTATCTTAGTGTGACTCTATTTGATGATCTATGCAGCCAAAGAAGGTGTTTCTTTTGGGTCTTAGTATTGTAATGAGATTTTTGC
CCAACGTAGAGTTATCTTAAATAACTGATTTCCAGTGGACACCTCTTCCACCCATTGATGAGATCTTTTAGAGAATGTGAATGTATG
TGTGATATTCAAATTAGGTAATTTAGTGGGCTGCTGTTTTACTCTTCCGGGTTTGGCATGGACATTGTACCACTAGGGAAGTACAGG
GTAGCTCTGGATGGGTCTGGTTCCTGAGAGGTCCCAGGACTGAGGCCCCTCCCTGGACTAAAGCTGATTCCTGCTTT
CTTCTCGAAGCAGTGTTTGTTTAATCCCCAATGTTCCCATGATTACTAAATTACAATTCAATCTCTTGAATCTAAAGGATTAGTTTA
CTCTTGATTGATGCATAAAACAAGTTTTCTAATTTCTGGAACTGTGTCTAAGGAAAACCTCTGAACTGTAATTTTGCGAAAGGACAG
AGGATGTTGTTATAGATTTGGTCTTAGGAGCCCCGTCTTTCGACTGGACGCTGTAATTAAGTAACCTGCTCATCAGTGGGGAGGGTG
ATAATAGTCACCTGCGCTAATCTCTGCTAAGCTCAGTGATCCTTTGAAGTGGGGTCCAGCTGTCTCCTCCCGGTTTCAGGTCCTCGG
GAGAAACAACAGAACTCGCTAAGGACCCGAGAGGACCCTGTGCACGCAGCTCACCAGGAAAAGACAGTTTGAGGGTGGATAGGGTT
GGCAGCTGCACTGAGGCGTGTTTGTTCGCCAGTTCTGATAGCAGGTCAAAGTAGTTTAACCTCTTACACAGCAGTGAGAGGCACCAC
TTCCCATTCTGGCATCACTGACCTCCCTCTGAAAAGGGACAGGGAAACATAGAGGAACGGGAGGGCGCTTATCTTTCAGGTATCAGA
ATTCAGACCAGTTTCAGATCGCCTGGTTTTTAGTAGTTCCATGACTCTCGTTGTTGCTGTTTCCCTCTACTCCAGCACTCCGTTTTG
GAGCTCCTAAATGCTTAGGTCAAGCATGCTCACCCACCCCAATTCTTATTTTTGTTTGAGACATTTTTTTTTTCCTACCGAATCCCG
GATGACCTCATTTCTAAGCCAGAGTAACTGTCTTACCCAGCACACCCATTGAATGTCTTAAAAGGCTTTTCAGATCCATCCATATAA
AACCCTCCACTTGCTCCTTCATGGAAGCCGACCTGCTGTGGCCTCACACAAGCACAGCTGTGCTGGTGGGGGTTGGAAAAGGTGTCGTC
ATTCTAGGTCCATTTAGACAGGCTTCTGAACAGCTAGACAGACATACAATTACTGTAAGAACCAGCACTCCATGTGTATATTCCCAA
GAGAATGGAAAAAGCAGGCTTCAGATGAAATGTATACATGTGTGTTCAGTGTCAGCATTATTCCAAGTATTGCAAAAGGAAAGGAAA
AAAAAATCCCAGCTGTCCATCAGTGATGAGTCTGCATAAACAAATTGTGATATTCTCGTACAGTGCAATGAAAAGGAGACAGTAATA
GTGTATGCCAACAACATGAATGTACTTAGAAAAAGTTATAGTAAATTATAGCAGCCATCTGCAAAAGGATGCAAACCCATAGAAACAG
GGAGAATATCAGGCAGGGGCAGGGGGTAGGGGAGAGAGACAGGGAGTGTATATCAGAGTGCAGGGGAGAGAGACAGAGTATATCAGA
GTGCAGGGGAGAGAGACAGGGAGAGTATATCAGAGTGCAGGGGAGAGAGACAGAGTATAACAGAGTGCAGGGGAGAGAGACAGGGAA
TGTATATCAGAGTGCAGGGGAGAGAGACGGAGAGTGTCTCAGGGGGCAGGAGAGAGAGACAGAGTATATCAGAGTGCAGGAGAGAGG
AATGGGAGTTTTTTGGAGGGATTTGAAAATGTTTTATCATTGGAAAGATATATTGTGAAGCTACCAAAATCTATGAGTTGGCCACTT
AAAAATAGTCTAAGTGTTGATTACAGGAGTCCCCAGGGACAGCTGCAATAAAGTGACACAGGCTGTGAGGCAGGAACAACAGAAATC
TGTGTCTTACCAGTTTGAGGAGGTGGGAAGGTGGAGGTGGAGGTGGAGGTGGAAGAGGCCAGATTCCCCTTAAGGACTGAGGGTAGGC
TGTTCCAGGTCTCTCTGCTGCTCCTGGTGTGAACACTCAGGCAGACAGTGGGTTGGAGACCCTTCACCCCATCTCTGGCTTCATTGT
CATGTGACATTCTCCCCGTGTGTGTGTGTGTTTGTCAAGATTCCCCTTCTCATTAGGACATTGTCATAGTGGAGTAGTGTTCACCCT
GGGTGACTTCAATTTAACCAATTATACCTGCATTGACTCTGTTCCCAAATGCACTGTGGAGTGCTGCGGTATCACTTTAACATATGA
CTTGGGAGGGGGTATTCAATTTAGTCTATAATGGTGTCTATTATCTAGTGTGATTTACTTCAATTTTTTTAAAAAAGAAAAGTGGCT
GGAGCGATGGCTTGGCAGTTAAAGAGAACTCGTTGCTTACAGAGGACCCACATTTGATTCCCAGCGCATAGAACTCCGGTCCCAGGG
GATTCAACACTCTCCTCTGGCCTCCTGGGCACCAAGCATGCAAGCAATACATAATCACACATGTGGGCAAAGCCCCAACACACACAC
ACACACACACGCACACACACACACACACACACACACACAGTGACAGCGCAAGTTCAAAGTCAAAGAGCAAAAATA
AGAAGGACGTAAAGACATAAAGACATAGAAAACAGTTGAACCCTCCCTCCGTGAACTTCAGACATCTGAGCGGCTCAACCCCTTATG
GTCTGACATCATGGCCTCTGCTTGCCTGATTCCTCTGCCAGGCAGCTGGTTTGTGTCCACAGCCTGATTCGCTGTTCCGACTGATGG
AAGTCTCTTCACCCTTAGAGTGCAGGCCCAGTGTCACAGGATACTGTCCCCTGTGCGACCTCTCAGTGTAAGACTGCTGCCT
CTTGGTCACATGCAGCACTGTGTGGGTGACCTGCCACCGTGTGCAGAAGCACGGGTCAAACAGCCACCTTTCCTTTACAGATTGTGT
GGGTCAGGAGGCCAGGCAGGGCAGTTGGGTGACCCCTCTCTCTGGTATAGAGCCTTGATGGGAAGATGAGGACACTGGGGAGGGGGC
GGGGGCAGTTCCAGGTGTGTGTCTGCACTGGCAGGACTTGAGGTGACACTCAGCACTTCTGCCCTGGGGCCTACGTGTAGCTGAGAT
GCCTTCACAGCAGTCGGACTTCAGGGTGTTCACACTTCTCTCAGGGCTTTTAGAGCTCCAAGAACCAGAATTCCAGGCACGAAAATA
AGCAAGAAAATGCATGGCCTCTTCAATCCTCAAAGTCACATGACATCATTTTCCACCATAGGCCTGCCCAGATGCACAAAGAA
TGGCCTTAGGTCTCAGTTCTCCGTGAACAGAGAGTCTGAGGCCGCCTTTATTAACACTCAGTGCTACCTTGCCCCAGAGTCTGAAGC
CCCTCCCCCACGAAGTCATATGTTGAGACCTATTCCCCACTCCTATAATACTGTACCCTGGGGCTTTGATGATACGGGATGCCTGCC
CCCAATAAAGGAAGCCCAGGGTACCATCTAAGAAGTGGCGAGCGGTTTCACATCCTCAGGTCCTGTGACCCTGGATCTCTCAGCCTC
CAGACTGTGAGACGAAATGTCTGTTGGTTTTTAGTTACTCCTCCCTGGTAATTTCACTTAGCAGTCACCTGACAGACAGCCGCTAAT
```

141

```
CAATCTGTGCTCTGTCAGGTCGCTCCTTGGTAAACCGCTCACTTCTTTGCATCATTATTTAATTGATTTTTGCTTTTCCAGTGAGAG
GGTAAGCTGGTTTGTTGTTGTTGTTGTTGTTGTTGTTGTTGTTGTTGTTGTTTTCTGTTGGATCCTGAGCTTCTTGGTGGCTGGCAC
AGAGAGCAGGAGCTTGGTGAACTGATGGAAGGTGAAAAACATAGAACCAGAAAAGCCATAAGCATGATAAACCTGTAAGGCAGGCTC
AGTATGTGTGACCCACGGCTTGGTTGGATCATCTTCCTGGTCTTGATTCTGCAGTCAATCTGAGGCTGCCTCGTCGTGAGCTGCGGAGTC
TTGTTGACGCCCGCCATTAGACACAGCAACGTAAGTAAAAGTGAGGCCGGCTGTTTGCTGAGTGCTTTCTACAAAAGCGGATAAACA
GAATTAAAAATTTAAGGTTTAAATAGGCCACCGCTAATAGGGCTCATGCGCTCATGGATTTGTGTTTATTGAGTAGAGGGAAGGTAG
GATGTGGTACAGTTTTTTAGTTTTAACAGCCTGCCTTTGTCAGAGCTTTATGGGGCTGATCATGTTTATTTAACACTCCCTGTGGGG
CTTAGCATGAGAGTCTCTCATCTGTTGGGCTTCTCATTTCTTTTAAAAATTCTACTTGGAGGTAGAGGCGAGTGTAACCCTGCAAGA
CATCGTCCGCTTCTTCCCCGGTTTCTGCACAGCTGCCTGACAGCTGCCTTGGACTTGTTAGGTGAGTGCCAGTCTCCTTTTTTTTTT
TTTTTTTTTTTTTTTTCCTGTAGAATTCCAAGGCAGGGTTTGCACAGGACCCCTTAAAAAATGCAGGGTGAGGCGATTCTGTGATC
GAATGTGCAGTTTTTGTTTTCTCCTATGCAGCAAAGACCCCCACCCAACTCCCCAAGGCAGTGCAGGCAGAGACAAAAAGCACTGA
CTACAGAATTGCTTGATCCCAAGTGTGAACTCTGACCCTATCACCTTTTATTGGTGTAACCTCAGGCGAGTTAGGCACCTTCCATAT
GTCTTCCCTGAGCCCTCCATGACAAGGAAGGGGGATGTCTGTGAAGATCCAACCGCAGCCTGTCGTGTGGAGAATGTTTGGGGGAGT
CCTTGAATGGCTTTTAAACACTGGTCTTATTCTGGCTTTGTTCCCATTGGAAGGAAAAGCCTATGCTGTTTTAGGCTTGGAATATTA
GTATATAACTGAGCTCCTTGAGGATTTTGCTTTGAAAAGCTTCTATTAAAGAATAAACATGGGCCGGGAGATGTTGGCTCAGTGGTT
AAAGTACGTACTGCTTAACCATAAGGACTAGAGTTTAAATCCTTAGCACCCATGACAGAAAGCCATAGGCATGGTCACTGCCTATAA
TCCCAGCACTCAGTAGGCAGAGATGGGTTCCCTGGAGCAAACCAGTGAGTGAGACATGTCAAATCCATGAGCTCTGCATTCGATTGA
GAGAGCCTGCCTCATAAATAAAGTGGAGAGCAATCGAGGAAGGGCCTTCATATGTACGCCCTTCCCACCCCACCGCCCATATACACA
GGCACACCACAGAATGAGGACGTTTGGAACCCGAATGTACTTTTCTACATTTCTCTACCATCTGTGACAGCTAGCTCATGCTGCCAG
GTAGAATGTGTGACTTGTTTGTTTCCTAACATTGGATTTAAAGTTGATGGCTCTTCCCCTTCAGGGCCAGGTCTGAGTGAGCCCTG
TGGACGAGAGCCCCATACCCTCTGTTCAGAAGACCAAAGATGGCGGCAGATGCTGTGGCTGGTGAAGTGACGTCAGAGGAGGGAGGA
GGGACGAGGGACGAGGGCTTTATAACAGATGGTTCTTGGATATCTGGGGCTTGTCCAGTTCTAAGCTTTGAAAACGATGGGCCAGA
CCCTGAACTGGCACAGACCCAGGCATCGTTGCCAGAACAGGAAGTGAGGTCCTAGAGTGATGTCTTTGGGACAGCTGCTGCTGCTCA
GCTGGTGGGCTGGCACAGTTGGCATTGGCTTCCTGGGGGTTTAGTGAGGTGAGTGTGTGTGTGTGCGCGCGCGCGCTACAATGGCA
ACAACGAGATGGGAAGCTGAGAGGGTCGTGACTGAGTGAGGTGTATCCTCTGATTTAGGAGAGGTGTCAGGTGCGGTACCATGTGATA
GTTTTGCCGACGTTGGCATAGATGGAAAGGTGTGACTGTAGTAGAAACCAAAGATCCGTGCATCCCACGCTCATCAGCCTGGAGTTCAGG
CTTGTGTGGAGCACAGAGTGATGCTCTGTGGGAGGGCTTGGCTGTCCTCTTGACATTAGCCTCCAAAGACTAGGAGCCTATCCCAGA
CATTGCTAACATCCTCTAATACCCTCTTGGAGTAGTTAAACCATTCTTGGCTGGATTCTAGTATTTATCCTGCCTCTCATTAGCGGC
ATGTCCTTAGGCAAATTATTTAATCTCTCTATGCCTGGCTAGTTTTGTCTATACAATGAAAACCTGGACTGGATGATAGATTGCTTA
TGTCCAAATATAATTTAATAATATTTGATTTGTAAAAAATATATTTTAGTGTTTTCTTTTAAATGTGAGCTTCATGAATTTTGTTTT
AAACACACACCTTTCTTAATTTTTTTTTCTCTCTTAAGGATTTAGAGAGTGCTTTTCAATAACTAGTGTTGACGTGTTTGGGAATTAG
AGTTTACTATCATCATGTTAAGGATCTATATTTTCCCTGTTTCTGTTTCTATATTGTCTTCTTCCTTTCATTGTTGGGAATAACACT
AGTTGCTATCCCTCTTGGAGAGCAAAGGCCCAGAAAACAGGCCAGAGAACCATCAGGGCTTTTCCCTGGAACTTAGAGGCTACCTGG
TCAGTCGTGATGCTGAGAAGAGGGAGATACTGAGGCCCTAGAAGGGTTTGTTTTTTTTTTTGTGAACAAGACTGTAAAGCATAGACTT
CTTGACATCTTGACTCTTTAGCTTGCTTTTCTCTGCTGTTTCGTGGCATTTGGCAGAGTGATCTGAAAACGATCTTTCACACCGGCT
CTCACCTGACAGTAGGAATGGTGTCTGGTCCTCACCCAGGTCCAGCAGCATTAGGGAGGGCTGCTGTCTGGTCCTCACCCAAATACA
GTAGCATCATTTTAAGGTAGAACTAACTTAGGGGCTCCAGGTTCCTCTTGAGCATGCTACCTCTCAGGGACCGAGCTCAGGGCTTAG
GTGTCTTGACATGTGAGTGGGGAATAGGGGGCATTCTATGGAGTGTGAGAAAGCCGGGCTAAGGGACTTGTGTTCACATGAAGCTCA
GAGACCATGGTGCAGTCCTTGCTCACATAACTGGTGTCAGTAGATGGCTTGCTGGCCAAGTTCTGTGGAGTTGGAAACTGTACTGGC
TGGTTTCATCTGTCAACTCTGACACAGGCTGGGTTATCAGAGAAAGGAGCTTCAGTTGGGAAGTGCCTCCATGAGATCCAGTCCGT
GGGATATTTTCTCAATTAGTGATCAAGGGGGAAAGGCCCCTTGTGGGTGGGACCATCTCTGGGCTGGCAGTCTTGGGTTCTATAAGA
GAGCAGGCTGAGCAAGCCAGGGGAAGCAAGCCAGTAAGGAACATCCCTCCATGGCCTCTGCATCAGCTCCTGCTTCCCGACCTGCTT
GAGTTCTAGTCCTGACTTCCTTCAGTGATGAACAGCAATGTGGAAGTGTAAGCCAAATAAACCCTTTTTTCCCCAACTTGCTTCTTG
CTTGCAAGTAACCCTTCTGCTTCGCAGCCTTCCTGCACCCTCCCAACACCCGTAACAGTTCACACAGCTCTGCAGCCAGCCCCATT
CCGTTGTTTCTCCTTCAGCCTCTTCTTCCTCACTGGACATTTTCTTCATGTTAAAACATGAGTTTATTAGTAAATTTAAATGTCTGA
AATTCCAGCTAACCTCAGTGCCCTTGTTAGCCTAGACTTGACACAGCCCAGAGTCAATTGAGTCACTAACTGGCTTTGTCAGTTTGG
TCTGTGGACAAGACTGTCAGGGTTTGTGTCATGATTATTAATTAATGCGGAGAGTTTGGGATGGCCCAGCCCACTATGAGCATCACC
ATCCCTAGGCAGGTGGTCCTGTCGACATGAGTAGAGCTGGCACAGCATGGGCCTGAGAACAAGCTGGTAAGTACCATCCGCCATGAA
ATCTGCTTCAGATACCTGCCTTGGATTCCAGCCATGATCTCTCATCATGATGGACCCTGTTAAGGTTGAAGATGAAATAAATTCC
TTCCTCCCCAGTTGCTTTTTGGTCTGACTCTGTTTTATCACCATAACAGAAAGGAAACTAGAATATTTAAGAATCAGTTTGTCTATT
TGATGTAGTCATTTTCTTTTCACTGAAACCACTGTTAGGAAGCCTTCCGAAAATCTGAAAGACCAATAGGTTGAAGATGAGATTCTG
TCAACAGGTGGAAGGTGAGGAATTCAGAGAGGGAGAGGAGAGAGAGGCCAGCCGTTTGCCATACAGGACAATTTAAGTGAGGGAGAGA
CTCACTGTGTGTTATCAGGTTCCACATCTGAGTATTCTGATAATTCCATCATACTGATAATGCGATCATTCTGATAATACTATAATAAC
ATCCCTGATAACTGCAGATTTTATTTTAATTTCTGTTTTTAATTTGTATCTGTTCTGGACACACATAAGCTTCTTTCTGGGGTCCTGT
AGTATACAGAACTGTACTGCAGCTATTGCAGGAGTGATGGCTCTTCATTGAGTAAATAACCTAGAGATGATTTCAAGTCTACCAGGG
GATATGTAGAGCTTATATGCAAACCATGACGTTCCGTTCAAAGGACTTGAACACTAGAGGGTCCTGGAGTCAGACCCTCACAGCTGT
CCAGGGACGATTGACCCGAGGGTTTCAGGCTGGTGTAACTTACTCAACAGCAGCCTTTCAAAGATTGGTGAGAAAGTTTCGACAGTA
GCTAGGTTTCAAGGGGCACAGACTGCCCAGCAGGGATGCTGTTGTTGCAGATTGCACATGGCAAAGGATAGGAGCCTTGAGGGTAGC
TAAGGATAGGAAGGGTGGTTCTTGGCTAGGTCTCATGGATACTGGACTGGCAGATGTTTCTAGATCCACAGCAATACTGGGAAATAG
TTTATGACTAATAATTGATCAAAGTCTCTGCCAAGCTGACGGTGATGGGCCAGGAACCATCATTTCTGTGTATGTGGAACTTGAGGT
ACATGGTTACGGGCTGTTGGGCATCAAGGTGTCGTCTGCATATGCCTGTGGTCGGAGAGAACAGGTGGAAGTGGGCTGTGGGGAAGC
TGGAGCTCAGACCTCACGCCCACGTGCTTTCCCATGATCCTCCCGACATTTAGTCACAGAGACAAGCTGGCAGGATGAAACGAATGG
TAGCATTCATCATCCAAATTTAAATTTGTTTCTCATTAACAGACATTCTGCCTGTAGTGCACCATGAATTAATATGTTTTATAACTC
TTGTAACTGTCTCGTGAGCATTAACATTAGTCATGAGTGTTGGGTGTTAGAAATGTTAATCTGTTCAATGTAGGCATTGAAATATT
GCGTGCGTTTCTTTATTTTTTAACATCTCTGAGCGCTCTAAGGAGATGAGAGTGGCCGTGGCCTCTCTTGACCTCTGGGCGGCCTGCT
TTGACCCGGGGTTGTGAATCAGCTTCTGCCTTCTGGAAATGCTACCCAAGAGCACAGGGGGTGTGGAGAGGGAGGGAGGNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNACAGCAGAATTCTGATTCAGTCCCGAGGCTTTGAAGTTTGCAACTCCGTTCCTTTAATGCA
TGCGTAGAGTGCGGGTAGATCACGCAATCCCTGGGGACCCTGCCAATCACGTCCATCCGAAAGATCAAGCCACTGCTAAGCTGCTTC
CTTAGAGTGAAGTGATTGTTCAGGCTGTTGGTTGTTGTCTCAGAAACGCTACAGAATAGACAACTATAAGGGGAGGGAAAAAAAAAA
AAAAGCAAAACAATTCCAGAAACGAGGAGCGGATAAAGAGATGCAAGGGGCCGTTTAGTCAGCTAGATGAAGACTGGAAGTGATAAT
TGGCGGTGTTTAGGGAGGGTTAAAATTGGAAAGCAACGCGAAAACGCAGTTTAAGCCACTGTGTGTGTGTGGGGGGGGGGGGGGGT
TCAAAGTAAACACTTGATATTTGGTAGTTACAGACACAAATTTTAAATCGACACAGCACCCTCCAGTGCTCTATTCCCCCAAGTTT
CTCTTTATAACTTGTAATAATTTATTCCACAACCACCCTCAGAAGATAGCTTAGGCTGGGCTGGACCGCATCCGATACCATGTTTGT
```

142

EP 2 204 376 A2

```
TGTTTGGCCAAAACTTTGATGTTTACGGAAGGGGACCAGAGGCCTTTATAGGAAAAAACACAAAAAACCTGGCGTGTTGGCAGATGT
TGAAGTAAGCAGTCCTGGACCCTGCTGTGTTATGAAGGGAGGGGATTTTGGACCTCAGTGTTCTGCAGAGACTACATTTGCGTTATTA
GAAAACACAGCACCATGGATAAAATGTTGGCAGCTCCTACAATAGCCTGGCAATGTGGTTTTCTTGTTCATTCACAAAGTCTCCTTG
TGCTTACTGAGACCAGAACGCACCGCACCGGGTGGGTGGGGCTTACTCCTGGACACCTTGAGATGCCTCAGGAGGGTGGAGCACAGG
CATGGCCTGAGAACCTGCTTTGCTTCTCTGTGTCCTGACAGTGCTCACCAGAAATTGTAACATTATTGTGGATTTTTCTTGAGACCC
TTTCTATCGAGCTATTTACTGGACCTTGTTATTATATTTCAGTATATTATTTTGCCAGGAAAATGTCAAGTCCATCGACTCACTCAT
GCAGCTCAAAACGAAGCAGGTTTTGGGCCTCCTCCTGACCAGTTGAGACTGAACCCTCCATGCTGGGCCTAGGCCCTATGCTGCAGT
GTCTGTATGCAGTGCGTATTTGTGGAGGCCATAGAGCAGCATCCTGTGTCACCCCTTATATATTTTTGGAGACAAGGGTCTCTCACT
GAACATGAAGCTCACAATCTGCAAGACTAGCTGACTGGTCAGCAAGCTCCAGAGGGTCTTGTCTCCACCTTTCCAGTGCTAGGATGA
CAACATTTGGATCTATCATGGTTGTTCTCACCTGGAAACTGGGGATTTGAAGTCTGGTCCTTATGATTACAGCAAACATTTGACTGA
CTAAACCCTGAGTACTGAACCTCAAATTCTTTTGTCTCTTCTATTTTCCTGTGTTCTTTAAGTGACTGAATCCTGAGGTTGGTGGTT
GTCACTGTCACTAAAATGTTGGAACAAAAAAAAAAGACAAACCAATTGTGGTGGTTTGTATATGCTTGGCCCAGGGAGTGGCACTAT
TAGGAGGTGTGGCCCTGTTGGTGTAGGCGTGGCACTGTGGTGGTGGGCTTTAAGACCCTCTTCCCAGCTGCCCAGAAGCCAGTTTTT
TGCTAGCAGCCTTCAGATGAAGATGTAGAACTCTTAGCTCCTCCTGCACCATGCCTGCCTGGATGCTGCTATGCTCCCGCCTTGATG
ACAATGGACTGAACCTCTGAACCTGTAAGCCAGCCCCAGTTGAATGTTGTCCTTTATAAGAGTTGCATTGGTCATGGTGTCTGCTCA
CAGGCGTAAAACCCTAACTAAGACACCAGTTCTTGTGTGCATGGTGATATCTGTCCATCTGTCTTAGAGCTTGATAATAAATATGTA
AATACTTCAGAGGGAAGGAAAAGTCTCATAGTTATACATTAGAGTTATTGGTGCCAGGAAAACTAGGGCCCGGGAGTGGGGCAAGGT
GAGCGAGTGAGAGGAAGGAAGTCTAGGTGAGGAAGAAGATGCAGTATAAACTGAGTGCTGGAGAAGAGCTAGGCGTGTGTGTGTGTG
TGTGTGTGTGTGTGTGTGTGTGTGTGTGTAGGCCAAGGCTGCTTTTACGTGGGACAGTGTCCCAGAGGGCCTGATGAAGGAAAGTGG
AGGTGGGGTGCTTGAAGAGCTAGGCTTATGGCTTTGAGTACCATGTGTGAAGGGACTGTGACCCTGGGAGAGGTAGCCTTGGTCACT
CTGTGCTGGTGTCTCCCCGGTTTCCTTGGTCCTGTGCTTTCATAGCGACTTGTCAGCTTTAGCTCTCTCCCTGCTAAGGAAAATCTC
TCGGCTGTTGGCTTTGTTTGAGGAGCTGTAAATGGACTTCACCTGAAACACATTACAGGCACCGGAAGATTAGTTTTAAGCAAAAGA
ATTTGATTGAAGGCCGCATCTTGTTCTTGCAAATCCTTTGATGTTTGGCCATTGTCTTGTGGGCTTTTCATTTTGCTCATTGGCAGC
TCCGTGCAGTGCTGGGCTGTAAAAGCCTTCACCTCCAGCTTGTCTTTGTGGAGTCTGGAGGTGGCTTCCTCCTCCCTCTCCCCCTCC
TGGGACAGCAGAAAGCTCACCCAGTTAATTCTTTATAACAAACTATTCTCTCTTTCCAGGGAAGGGGTTCTGGGGCATTTATTGGTA
GGTTGTGGTTATTCCCTGGTCTGGAGTCAGGATCCTAGGGAATCATTGGGTCGAGCCACATGCAGTATGGGACAGAGGTATGTGTGT
GACCTGGTCAGGAGGTTCTAGTGCTCTTAGCTGAAGACAACACAGGCAGAATCCTCAACAGTGGAGCCACTCACCACCCTCCAAAGG
TCCAGACCAGGACTTGGGGCTCGGTCAGCCCAGAAGAAGCGGGTGTCTTCTATTTGTTAGAAAACCACTCCAGCCACTCAGCCTGGA
GAGTTCTAGATAGCTCTGCCTGGGAGACAACCTCACTCAGTAAGCAGTGGGGATCCCAAGAAGGATCTGTTCTTACTTGGTGGAGTT
GGGGCTGGCAAGCAGTCTTTGCCTCCTGTGACAGAGGCATCCCTGAATAGAGTGGAGACTCAGATCATGGCAGAGTGAGTAGCCTGG
ACCTGGACGAGGAGCTGAGTCTTCCTACGGACTACCCTGATGACTACATTTGTCACGGTGTCTTAGCCAGTAATATAAGTGACTTGT
TTCTTTAAGAGAGTGACTTCAAGGTCAGGGGTATGGCTCTGTGGGGGAGCATTTGTTCCTTCTGTATAGGATCATGGGTTTGATGCT
CAATACTGTCCCTCTCCCCAACACACAGAGAGAGGGTGACCTCAAAGATAGGTGCCTCATGTCAAGTCATATCCTACAGATTACAGC
AGTATCCAGGGGCCTTGGCAAAAGTGCTCCTCCCCAGGGATCGGGGCTATATGCCAGGCTTCTTCACCTATGGGTGTTTCAAGAGCT
AATTAATTTGCGCTGACCTTGAACTCCTTGCCCAAAGCAGTCCCCCTGTCTCCACCTTGGAAGCCATACCAGGCAGCACTGAGGGAT
ATAGTTCTAAAAGGGTCAAGAAAACTCTGGGGGCCTAGCTGCCACATGGGTCCTTGCTCTTCTCTGACCTGACATCAAGGTGGCAAG
GGACCCCAGGTGAGGTGTTAGCCAATAAAGACAGAGTTTCAGGCCTGCCACTGTCATATTACCATTTGTTGACAACCGTCCTTCATT
AACCAACCTGTTCTTCACACATTGGTTCGAATGTGCTTCTCGGATTTACTTCATTCAGTTCACCAATATGCGGTAAGCACTGAGGGG
CCCAGTAATTGGAAGTGTCTGTCGATGGCCTCTGCCAACCTGGCTCATGATGAAAGTCCTACTTTTATAACTCAGTGTCAAGCTCAA
TAAGAGGGAGGCCGGTTCAGCTATTGTCAAATCTCATTGTTTTTCCTACTGGGAGGACCACAGTGGGTTCAACTGGAGACAGTTGCT
GTCTGAGCCCAAGGTCCTTCTTTGTAGCTGGGCTGAGGACTGCAGGTCTCTTTAGCCTGTACACATGGACAGAGGGCCCTCCTGCTC
TGCATACCAAGAAGCCATGCTGGCTGTCTCTCCCAAGCTCTTCTCTGGCCCACTCTCCTTCCCCCCACATCTGGTGTTTGGAGGAGGT
TCTCCTTTCTGAGGGCAAAGGGTACATAGGGGCATTAGTCCCCTACTCGGAGAGGTGAAGGTAAATGGCGAAGAAATTGCCAGAAGC
ATCCTTTTAACATCCTCCGTGAGCAAAGCTCGGCCTGTGGAGTGGACTGGTACCTAGACTCCAAATCACGGAGGAGTGGCTACAGCT
GGAATCGGGATTGTCTTTCCTTGGCCACCTCTCTTGTCTCCAGCTGTCCACCATTGCTACTGAAAAATGGATTTTGAAGACACCCAT
GTTGGGTGACTGTGTGTACCCGGAAGGACCAGTGGAAAGGGGTACACAGAGACCACATCTTAGTTTGCCTCCACAGCTGAACGGGCC
CTGCCTGCTTTAGTCTGGATTGTTTTTCTTCTGCAAGTGGAGTGAGCAGGACTGCTTTCATGTCCTGTAGCCTGAACCT
GCCCTGCTATGTCCCACTCTGCGTGGTGGTGGTCATCTGTGGGGCTTGATCAGCTGCATTTCTAACTAAATGGTTTTTTTTTTTTTTTTTT
TTTTTTTTTTAGTTGAAGTCATTGTGGCACCTGGACTTGAACATCTTGCTCTTGTGTTCATTGTTGTTTTTTTTCTCCCTCTCCATTCC
TGTCACCAGCCTGTAGACCACCTTGAATTCACTCCAACTTGCTGTATAAACCCCTCTAGCATTCCAGTGATAGAGAACTTTCTTAAA
AACAGAAATACACACAGTTTATCAATATCTGTGTTTTTTTTCTCCCTGGAGGAGCAAAAAAAATCAAATCACCACCACCACCACCTCC
AAGAAACCAAAACCAACCAACCAACCAAGCACCAAAACAAACCAAAACAAAAACCAATTTAGATTCAGGAGCGGGAGAAAGGAAAGAAAATG
TTTTTGATGATGTTAAAGTTTTGGACTTAGAGGCAAAAGTCCTCGAACTATTAGAACCAGAAAACACTGCATACTGAATAATGTACA
CCCTGAATGCATACCTGTGGTTCAAAGGTGTGGGTCTAAATGGCTAGTTTCCTCTCTAGCAGCCCGGAGGTGTTGCATTCATTCCTT
TTGGAGAGAGACATATCACTTTTAAAAAGTGGCTAAAAATTGCTGGAGACCCATTTGGAGACAGGATATTTGGACTTCATTTGGCTG
CTGGGAAAACTGTGGGCCACCAAGACTCCATTTCAATGCGAACTTATTCTACCAAGAGCTGCTGTAGCAAGAGGGAGGAACCTAGAT
GGAGCAGGGGTCTCTGAATGCTGCAGGCTTACGTGTTAGCATCTGGAGGGCAAGAAACACCAAACTTTAGCAGAGAAGTTCAGTGAG
TTTAAAAACCATAGATTCAAGGAGCTTAAGGAAGAAAATAGTTCCTGTTGTATGGGTCCATATAGGTCCCTGTCACAGGTATCCTAG
GAGAGAGGGAATATAGGTCCCTGTCACGATATCCTTGGAGAAGAAAGGGCATATGGTTCCTTGTAACAGATATCCTTGGAGAGAGGG
TACATGGGTCCTCGTCACAGGTATTTGGTGATGGGGAACATATAGGTTCTTGTTACAGGTAGCCTTGTGTGTGTGTGGGTGGTGGTG
GTGGTGGTGGTGGTGTAAGGATTCATGTCACAAATAGCCAGGTGGAGGACAAATGGGTTCCTGTCACAGGTATGGGGGAGAGACAC
ATTGGTTCCTGTCTCAGGTATCCTTGAGAGCAGACATGGGTTCCTGTTACCGGGATCTTTGGGGACTACATGGATTCATGTCACAGA
CATCCTTGTGGGTGTGGGTGTGTGTCTGCACCACAGTCTAGAATCCTGTGAGTTCAAACAGACGTGGTTCATTCACACTCCTGCAGC
GTGTCTCGCATGTTACGCTATAATGAATCAGGTTCCAAGTTAAAACTGTTTTTCGTGAAATCCTGTGGGGAAAAGTGTCAATTTATG
GATAGGTGCGGTGTATATAGATTTCATGTAGAGCATACCAAATGGTTTTCTTTTTTTAAAAACCATGCGCTCTAATTACAGATGGAAA
GTGTCTAAGGAAGTTGGACTAAAACAACACACACAGACGTATATATTATATAATAAAGATGGACATGTGTAAATGATAAAATGCTGC
ATACAGGAAAGGGGAAGCTGCCTTAGACATCAAACCCATTACGATGAGCATGATACTTACATGAGAGGCCTCCTGGAAACCAAGGC
AAAAAGAAAAACATGGTGGATGATAGGTCTCTAAGTATCCCTCCTCCTCCTTCTTCATCTTCATCCTCCCCCCCCCCTTTTTTTTTG
TACTGAAAAAAGAAAAGACCGAGAACCTTGTTACATTCCTTAGGTTGGCACAGATGTATTCTGTAGCCTAGGCTAGATTTGAACTTA
CCATCTGTCCAACTGACCGACCATCCTTCTATCCATCCATCCATCCATCCATTCATCCATCCATCCATCCATCCATCCACGCACCCACCCT
TCCGTCCGTCCGTCTGTCCATCCGTCCATCCACCCACCCATCCATCCATCCACCCACCCATCATCACCGTCCATCCATCCATACACCCATCC
GTCCACCCACCCACCCATCCACCCACCCACCCATCCATCCATCCATCCATCCATCCATCCATCCTAACCTAATTAGCTGAGATTAGA
GGCCTGTACCCACTAAGCCTTCCTAGAAATCTTTCTTCAAAAAACAAAACAAAACGCTACACACCTATGTCATGCCATGAGGTGAGAA
ACTCTGTTGCTAACCTCTGAGATTTTACAGCCTGTCGCTGATATGCTTTCATTCTGGCTGGCTTTACCAGACATCCAGAGGTACCAC
ATTCTCTGACAGCTTCCCAGCTGCAGTTGGGTTTTCATGGGAGTCCTGAGAGGTTCCTCTCCTGGGCCTCAGCTCTGGCTCCTCAGT
```

143

```
GAGCTTAGTTCTCTTCCCTTAGCTTTCAGACTTACTTGTGGGGCTGCCAGGAGCTGGACAGGCTGCACTGAGGAAATACAGGGAGCT
GGGGATGGTGGGGGTGATGGTTATTCTACTCAGAAAATGGCAGCTCAGAAGCATGTGATGGGTCTGACCGTGAAGGAGGGGCCCAGC
CTTCCTACCTGAGGGAGCTGAGCTGAGGGTGGGAAGCCTTGCTCCAGCATCCCCTTTTGCTTCTCCATGAAGTCCTGTGCCCGCCAT
CTTCCTCCAAATCAGTTGCCCAGCGATTATTCAGAAACGCCTTGGAAAGATGAAAGTGTATTTTTAACACTGGAATGAGAACACAGT
AACGCTTTTGAGCTTCAAATTGGATTGCTTTACCGAGCTGAAGAGTTCTCTTTCTTCCTTTAAAAATACTCGCAAAATTTAAAGAAT
TAAAGACTCTGGGGCTAGGAATGTAGCCCAGTTGGGAGTATGCTTGCTACTATGCTGAAGCCATGGGCTCTTAACACAGCACAGCAT
TAGCATAGAGGGGTGTTGCACACTGGCAACGCCAGCAGGAGGAGGATCAAGAGTGCAAAGCTAGCCTTGGCTACATGAGATAGATAC
TGTTTCAAAAACAGCATCAAGAACAACTCAGATACCCCGAAGGGCAGGGAATATGTGCTCAGTACATGGTTTCTTTACCTTTCCTCC
TCCTTTGGAATTTGTAGTTGGAAACCTTTAAGTAGATTTTATATATGTGTGTGTGTGTGTGTGTGTGTGTGTATAATTTAAAA
CCACACTCTGCTCTGTCTAAATCTCATAAGCAAGCACTAACTACTCCAGCTCATTCCAGTTTGTGTTCTCACACATGGAACTATGGA
GTGCATATAGTATTACAGTTATACTTTGTGCTGTGGAGTGCTTTGTGGCTTCTTCTCATGGATGGGTCTATGCAGGGCAAGCCGTTT
GATAAGAAGGTCTGTGCTGCTTCCTCTCGGTTCCTGGATTGGTGGCAGAGATTTAGTGGCTCTGTCCTCGTGATAAGGCAGGGTAGA
ACAGCATTCTGTGTGTGAGTCTGCTCAGCATCACCAAACATTCATCCTTCTAAGCCTTTAGAAAGCTCAAGAACTCATAGAAATAAC
CCTGTTGTGTTGTTTGGTACCAAGATGAAACATAGCCAAAGCTTGGGGGATTAGGACTGGGAAGGTTCCCAGAAGTAAATCTCAGAC
TTTCTGGATGATATTCCACCATGGCTTTTTGACAAAAGAGCAAGGCCTCAATGTAAGGCTCAATGATGACGTGTGTGTGTGTGTGT
TGTGTGTGTGTGTACATATCTATCTACCCACTCACCCACCCATCCATCCATCCATCCATCCATCCATCCATCTATCTATCCAT
TCATCTACCTAGTGTTCTGTCTGTCTATCTGTCTGTCTGTCACCTCCCCCTTTTTAATCCAGACTCAATGTGTTTTCACACCTCTCC
AGGTGAGCATCAGGCAGGGACAAGAAGTAGCAGACAGAGTGGCAGCTCAAAATGACCTAAGTAATTGTGGGTGGTCAGGAAAGTAAC
TGGTTTTTAAGTTCCTTCTGGGATTGGGCCACCTGAGAGAGTGCATGGCTGGGTGGGGGTTGTGTGGCTGGCTTTGCCATGCTCTGG
GGGCTGGATCCCACGGCCTGTTGCAGAGCCATTAGTGGGTTCAAGGGGAGGTGCATGGGTGGGGATGAAGTCAGAATTGTGGAGCAG
GTGATGCAAGCACTTCCCTGAAGAGCCATCCCACTGCCTGGCCGCCTACCTGATGACACCACCCAGCGTGGCTTGTGAACGTCACGT
CAGTTCATTGCCGCACAGACACTTTTAATTTGCACAAGCCAGAAGATTTACACTGGGGTTGAGATAGCAGAAGACGAAAAACCCAGT
GGGTTAGTTCAGAGCTCTGACTTTGTTTCTGTTCCTCACCCTGGTGTGAGATGGCTAAGCAACAACAGGGCTAGAGTTCAGATCTAT
TGACGGCATACTTGCCTTGGTACCAGAAATTTCTTCCTTTTGAAGTAGTTTAAAGTTTAAATTTTCTTCTTTCTATGTATGGGTGTTT
TGCCTGCGTCTGTGTCTGTGCTGATAGAGATATCAGATCCCCTGAAACCAGAGTTACAGACTCTGGGGCCGGGAATGGAGTAGTACA
TTAAGAATGCCAGTGCTGGGAACTGAGCCTGGGTCCTCTGGAAGAACAGCTAATGCTTTAAAGCACAGAGTTGTCTCTCTGGCCGCT
CCGTGGCTTGAGCTTCTGAAACGGGGTTTTTCCTAGAGGTCCATGGAGAATCTCCTTCAGCCACACCGTGGACCTCTGTGGTACTGA
AGATGCGGCACTGAGGAGCGGTCTGCAGCCAAGCTGTGTATATTTAGTCCTAGGAGTGAGGATGCTAGACAAAGAGCTAGGTAGAGTGC
AGTGTGGAGGAACTGGAAGGTAGCACGGAACAGTCTTGGTGTAGATGACAACCTGTAGTTTATGCATGGCATCCTGCTGTTACTACC
GTGGTGCAAACGACCCACGAGATGGTATAAAAACCTGTGAGTTCTGAGGTGCAGAAGGAGGAAAGAAACTAAAGAAGCATTAATGGA
GGGAGGAGGCAGAGGCTATGATGAGGCAGGTCTAGCCTGTAAATCCAGTGAACTTCCCACCCTAAACTGACCTGAGTTACCCTGCAA
CAAGCATCCCCACCTCCTGCGATTTCAAGGCACCCATATGTCGCTGTGTGTCGCCTGTCTCACTTCATCTGGAAGGAAGAGCATCCATGT
GGAACATCAGTGGCACCTGTCTTGAAAACTCAGGGCCACTGTTGGTGAGGGGACACCAGTTCTTTGCATAGGGTACCCTCTGGTCAG
ATGGCCTTCCCAATCCGTGCAAGTAGTTTAGCAGGGGAAGGTGTTTTGATTTGGGGCAGGGTAGACAACATGAAGAATGTGGTTTTG
GGATTGGGACACCAAATGGCCACATTCTGGGACTTTTTATCTGGGTAAGGTTGGGCTGGTGTGAGCATTGTGTATCAGGCATTTACA
GAGACCAGGGAAAGGAGAGAGGTATGAGTAGAGCTTTGTCAACAAGAAACCTTGACACAGCCAAAATGGATAAGGAGCTTTTCTCAG
TGTCTCCTGTACCATAAACTCATTCCCTTATCCCACTAGTGTTGAGAATATCAGGTACTCTGTAGGCTAAAGGACACTTGTAGGTCA
GCCTTTAATTATTACATTAATCACAGTATGGAGTAGTACATTAGCTTCTGAAAAGCATGGAGGAATTACTCCAAATCATTTAAAACG·
CGTATCGGAAGAAACACATTTGTGAATATAAATAAAAGCATTATTTATAATATCACAAAAACCTGTAATTGATCCAAGCATCCCTCG
CGTGGTGAATGGTGGAATACTACTCAGCCACAGAAAGGAGTGGAATTCTGAATGTATATAGCAGCATGCATGGGCATGCAGAAGCA
TTGTGTACATCCAATTAGATGATACTCCAGTGCAGACAAAAGTAGTCTGTGCTTATACAAAGCAGATGGATGAATGTCTGGGGCTCA
GCATGTAGGGGTCAACAAGGGAACCTTTGTGGCAATCGTGTACTGACCTTGACTGTAGAGATGGTTGAATGGTTGTATACACTTGTCA
ACACACTTAACTCTACATGCAACAGGAGTGGATTTTAAGTGAGTTGTATATCAAAGTATTTTTTTAAAAGGCTCATGTTTAAATGTAA
GGCAACTCCTTATAAATTTTGTTATCAATTATAGCTTTTTTTTATCGGTAGGAAGGAGTTCAAGTTTCAAAGCATGTAAAAGTGACGT
TTTAGATTATAATCTGTGCAAAACGGGGAACTGTTCATATGCTCAGGCTATCACTCCATTTTAATTCTCTCTAAACAAACTTCTGGA
TTCTATTATTTTCAGAGGGCTTTGGAGACCATTTTAAAGTGCATGTTTTCCCAGGATTTTCCTGTACCTCTTTGGCAAGCTTCATAC
TTACATTGTTTCAAAAGTATAAATAACAGCAGAAGAGAGCTCTGTTTTTGGCATACTCCTTTGTCCTTTCCTGTTGTCCTGATTCTT
TTATGTTAAATCAGCCTGCAGTTCCCGTCCCTTCTGCCTTTGAACTTAGAACATGGGCTCCCGGTCCTTTGCAGGCAGACCCAATGA
AGAGAAAGCATACTGTGCATGGCAGAAACTGGGTAGGACAGAATTCGGGTGCCTGCCTGCCCAGAGCGAGGGTAGTGAAGTGTGGGA
GCAAATTGCTACTTGATAAGCACTTTGGGTTTGGAAATCTGGTGTCACCCACCTGTGTCCCGTGTGGTAGCGCCTATTCTTTATCCT
CCCCCCCCCGCCCCCTCGCTCCTCCTCCTCCAGGTCCTGTGGAGTCAGCTACTTAAACTCATGGTCTAAGACCTCAAAATTTCACAG
TGATCTGTCGTCACAACAAATCTAAAATAATAAAAGGTAATTATGTTTCTGAAGGTACCAGTCTTTTGCATGCTTAGGACTGAGTGT
GCATTAAAGTGGCTATTGAGTTTTTGAGGGGAGGGGACTGGGGTGTGATAGTGTGTTCGGGTCTAGGGCCCAGGCTGTATGTGGTCT
ACCCTACGGTTGGTGGCCTGGACTTGCCCTTTTCCTAGAAAAAGCAGATCAGAAAGAACAGGCAGTTCTGGCCCAGCTTCTTGACGA
TCACCTTTAACCCCTCAGTGCTGACATCTGGGCTGATGGAGCCCGGGCCCTCTTGGGGATGTGGTGTAGGTCAGGAGGCTTGGGTG
TTCTGCCCATCAGGGCCCTGAGGAACCCTCTGTTTTTGGGGAGATAGCTTATTTTGTGAGCTTGAGGAACTCAGCTCTGAGTTGAGA
ATGCCATTTCACAGGGTGTGATACTTAGCCATGTCAACTTCCATGAGGTTTACGGTCGCCTAGGCGACAAATCTCTGGGTGTGTCTG
TGAGGGGTTTTCTGGGTGAGGTTTACTAAGATGGGAAGACCCACCTAACAGTCAATAAAAAGGAGACCGTGAACTGAGAGGGAGCAA
GTCTCTTTATGCTTCCTGACAGCAGATGCAACCAGGCCAGCTGCCTCGGGCTTCTGCCGCTGTGACTCTCTGATAGAATGGACATCA
CCCAGTATCTGTGAGCCAAAATAAGGGCCCCCTTCCTTAAGTTGCTTTATCAGGTACTTTGTCACAGCCAGAGGGCAAGTAATCACT
GCAGAGGGATGTCTAGAACATTAGATGTGATGAGGGAGTGTGGTAGGCCTTTGTAAACCCTTCTCTGTTGGGAAGCTGGCTTTGGAC
CAGCTGCCGTGGAGCCGAGCCGACTGCCTGGTAACGCCTTGTGATGTCTGGTAACGCCCTGTTCATTTTGTAAATCCAGGGGACGCG
GAGGGAACAAGGCCAGAATAATTTGCAGTCACAGGTGCTGCTTTGTTAGCAGCACAGCCCTTTTTGAGATGGGGACAGTGTCCCTTCA
TTTCCTGGTTTGCTCTGCACGTCAGTTTAATACACAGGCATGTGACTAGAATCACAGCCCATGGCTGCCAGGCTCCGAGTAAATGCG
CTCACTGGAGGCTCATTACCTGTGATTGCAGACACAGGGAGGAGATTTGACTGTGGAAATGGAGCTTGATGTGCTATTGAAATAACT
TGGCAGGTCGGTAGGAAAGGCGTGGGGGGTGAGTAGAAGGTGGGGGGGGGCTCCCACAGTGATATCCCCTCCACACATCGGTAGCAA
GCAATATTGTCCCATCTGATGAGACATGCACTGTATTTTGTTCTGCTCTACAAATAAGAAAACTCCTATCTATACTTATCACAAAGT
GATGAAATTTTGGGGGATCATGATAAGTATTTTTTAAAAGCTTAATTGTATTATTGGTGTTCCAGCTTCGGACTTTATTTCTGGTGCC
CTATAAGTGGCCAGCAGCCTGGTCCCTGGGTGCAGATCTAGGCAGTGGGAGAAAGGAAACAATTATCCAGCAAATGCGAAGAAATCC
TGCCAAGGAGCAAGAAAGACACCCCTCACCCCCTGGCCAATTACAGAAAATTGGTGTTTATAGGGAATTAAGTTGTCTGGTAATGAT
GCTTTCTCTTCCTTTGTCTGGTAGAGCCATCGTCAGAGCCATAAGTGAAAGAAAGCCAGAGTGTTTCCGGCCTCTGTGCTGAGGCGG
TGACCCTGCCTGTAAGCACCCACCTTTGGGGGCTCAGCCGCATTGGGAGCAGGATTGAGCTTACTTCCAGGAGCCTAGTTGACCCTA
AAATACAGTGACGTGAAGCGAGTGAGGTGTGTTTATTCCAGCTTGCTGGCTCTGCTTGCTATGCGGGGAACTTGGTGTTCTGGTACAGCTG
TCTGGTAGCTAAAGATGACTTGGGTCTGGGTTGCAGCCCGTGTCGATCCTGGGAAACTCTCTTCCTATGTTGGGAACATTGAGAGGGT
CCTGGGGTGTGACTCTAGTCTCAACAGCGCTGCCTTCTTACATCACTCTACCTGTCCTGGATCTGCTTTCCCACGGGAACAATGTCA
CGGTACTTTCTAGGCACCGTCTACAGTTTGTAATCTATCTTCTCACCCGAAGAATGGTATGGCACGTGACAGACATGAAGACATCTT
```

```
TAGAAAACAAAGCTCTCCACGAGTGTAAACTGTGACTGATGACACACTCAGTGACCTTATCTGAGAGCTCATATTAGAGAGACAGTA
TTTGCATCTTTTAGGGGCCCATGTACCAAGGTAAAGCCGTGGCTTCCCTCCTTGAAGCCCTTTAGGGCACATCAAGATGCCTCAGGG
GACTGAATCTAAGCATAACTGGATTTTATTTGCTTTCCACTTCTCCTTTGTTTAATCTGCTCTGTTCTTTATTTCCGAGTACTGGGA
AGGATAGGAAATATGTAAGATTTTTTCATATCAGTAAACAGAAATAAATGGGAGCCCCGTGGAAAGAGAGAAGAGACTATATTAAACA
TATTCAAAGCAAGAGGCAGGAGAAGGGAGTCTGTGAAAGTTGGCAGCCGGCAACCCCAAGGTTCGTCTTAAGAAATGACAGGCTTGG
AGAGTGAAGCGCGGGGCTGTAGCATTTTTGGAAAATTGAAGTTAACACCAACGCTGCCCTTTCTTTGCTCGGCGAGACATATGGCAG
GGACAGATTCCTGTGATCCTCTGCAGTCGTGTGACATTGGCGCATTTGGCTCGTTTTTTTTTAAAAAGGCTCAGCAAAGTCTTAGGA
GACAGAACGCCTCTTTACCTCTTTCATCCTGTGGGCCCACGAGGAAGTTAATTCCTTTTTAACTTTTTGAGACATACTGGTTTCACA
AAACAGGAAAATAAAGGGTCTCTCAAAGGACTGAGATAGACTAACCAGTCTGCAGAGTAGGCAGGAGCAGGCTGAGCGACAGGACAA
GACAGGATGGCTGGCAAAGGGTGTGGTGAGGGCATGGCGGTGGTGCTGCATGCCTTGAGGCCTTGCATCGGATCCCCACCGTATGAG
CATAGGATCACCTGCGGGAGGACACTGCAGAGGACTAGAAAGCTTATGGCCCATGTCAGAGCACCGGGGTGATTCTGTACTGTACCT
GTCCTGCGTACTGCAGAGTTCCTGGTCAGGATTCACTCACATGTGAGTATAGCAGTATGTGCTTGCTTTCCTGGAGCCAAGGAAAAG
GCAGACAGCTTAAGAAGAGACTGAGTAATTAGGTTTATTTAGAAGGTGAGAGACAAATTGGCAGTGTGGATATCCAGGAGAACACAA
ATAGGTCTTTGTTTGTACCCCAGACCTTTAGAAATCATGGAGTCTGGGTGCTCCTAGATTATTCTGTAGCCACAGGAGATTTGACCG
GCTTGTGCCAGACAGTCTCTATAATTGAATTCTTGGTGCCCACTGCATACCACCTCCTTCCCAGCATCCTCGGCTCCATTTGGTTTT
GTGTCTGCCATGTCACGACTGGACCACGTTTTCCCTTCTCTGGCTTCTTCACGTTTCTTGGAGGACGGCTTGGGAGCAGCCCCTTGG
CACACAGTGGTGTGCTCTGGGTGCTAGCCACGGCAGGACATCCGCTACAGGAAGAACTGCTATCCCTCTGCCCCCTCACCCTTGGCT
GGTGGCCGTGCCCCCTTCCAGCTGCCAGGGGGTTCAGTTCCTTGCCTTTGTCTATAGGGTGGGGGAGGGGGGAGCACTACCTCCTTG
GCTCCCCATCTTCATTTGGAAGCCAAAGTCGCTCCTCCGACGGGGCACTGTGTTGCCTAGGCATGCAGGTGGTCCATGGGAAGATTG
GAAAAGGGTTGCTGCTACTTGGTGTGTGGTGAACTGCCAGGGGAGTGTGGAGTGCCATTGCTTGTGGCTATGAGGGAGCTGTAGAAAG
AGCATCTATAGTTAGACCCTGTGCTGAAGAGCCCCTTTCCACGTAGTCTGTGTAGACCCTGGAGACTATAGAAGCCGCTTCTCTCTG
GATTAACTGTCGCAGCAGCCTCCTCTGGAGGATCATTGGCAAGTGTTGTAAAAGCCTCAGCTTCCTCCTGGTGTAAGTGAAGAAGAG
CTTATCTGCCTTCTTGCCTGCTTTATGAAATAACTAGAGGGTCACCCGTTCATCTACATAGTCAGTGTCCTACACCTATCTCTGCTTT
TTTTTTGAAAAACTTGTTACCTTGGAAACAGTGCCCCAGCTTAGAAATTACATAAGGCTAGCTGTCTCGGTTTTTTGTTTTTTTTTTT
TTTTTTTTGTATTTTGTATTTTGTATTTTTGTTTTTCTCCCCACAGGAAAAGCAGAGGAACAGGGGAAAGGTTATAGATTTTTTTTT
TCTAAAATATCTTCAGGGGTTAGGAGAACAGGGTGGGGAAGGAAAAGGGAGAGATGTGTTCTTCTGGTAACATTTGTAGAATACTCAG
CTGAAGTTCTTTAAAGATACGATTTGGTTGGTAGCCAGTGGCCTCTAGCGGCCCATCTTCTCAGCGGAGGACTTTGCCTTCACATCT
GGATGGAATGAGGACACTTCCATGGTTCTGAAAGTCCTTTGCTCCACGAGTGAGGAGCCTGGCATCCAGAGGTGACATGAGTCACAC
AAGGGCATTCAGAATCTTCTGTAGAATTCACCATTCCCGACTCAGATGCTGTCAAGGCCGAGGGAGGGAGGTGTCTGAGAATATTAT
TGTCTGGAAGTCAAGTAGCAGTCAGCTTGCTTGCTTGGTGAAGAAACAGAGCAACCTGGCCTCACGTTTTCAGTCCCTTGTTCCTGG
CAATAAAAGGATGCTGTGCACGGAAATTGTCAAAAACATGCCTTCCCATCCAAAGCCTGATCGGTCCTCTTGGTAACTGTTTTGTCT
TTTTGGTTTTAGTTTTTGTGAAGTTCCTCTCACTTTGCTTTTCTATAGTGATCTACCTACGACATGAGGAAACACACTGACTTTTATA
TGTTTTCTTCTTAAAAATGTATATGGAACATGTTAGCCCAAGGGAAGTACAGAAACTTAGAAAGAGTTTTGAGGAAAGCGGTAAGTT
TTAAAACTCAGCACAGTGTTCCAGTTGTGCCGTTGAGGCAAATGTTTGGTGTAGTAGTGGGGACTGTGTAGAATTTCTTTCTGAATGT
TGTTGTGTATTCATCCTGCATTGGCAAATATATCTTCGTACCCCCCAGCTCCAGCATAGGCAGGCAGTGTATTTATTTATTCGGTGA
TACAGCAGTTGCTTAGCTGACCTTAAGTGCTTGCCGTACACTGTGCTGGGAAGCATAGTTGGATACTGTTCATAACCTCTGCTAAAG
CTTGGACATTCATGTTGAATTGGTTTCCGGACATTTTAGATAAAAAGAGGGGTGTGTTTGGGTGTTGGTGGTAGGTGCATG
TGTGGGTGTATTTATGTGTGTATGTGTGTGTATATGTGTGTGGTGTGGGTGTATATGTGTGTGGTGTGTGGTGTATAGGTGAT
GTGTGGTGTATATATGTGTGTGTAGTGGGTGTATATGTGTGTGTGGTATGTGTGGTGTATAGGTGATGTGTGTGTATATATGTGTGTG
TATATGTGTGTGGTGTGATGTGTATGTGTGTTGGGTGTGATAGATGTATGTGTGGGAGTATATATGTGTGTGGTGTGGGTGTATATA
TGTGTGTATGTGTGTGTATGTGTGTGGTGTGGGTGTATATGTGTGTGAGTATATGTGTGTGTGGTGTGGGTGTATATATGTGGTG
TGTGTGTGTGTGTGTGTGTATGTGTGTGGTGTATTGCTGGAAGTGGCCCATGTGTGTGTGAATGCCAGGTGGCTTCCTCC
ATCATTGTTCACCATTTGTGTCAGACAAGGTCTCTGATGGCACCTAAGCTCACAGTTAGACTCACCAGCCAGTGAACCCCTGAATCTT
CCTGTCTCCCCCTCCCAGCATGGGGATTATAAGAGCACTGCACCTCGACAACTAGACTTTTTATGTGAATGCTTGGAACCTGAACTT
AGGTCCTCCAGCACTCATGATATTCACTGCACTATCTCACAGCCTCTGAGGGGAACATTTTTGTTGCTTGCATATATTGGGAATGAT
TGGTTTTTTTCATACAGTGGTATTAGTCCTACTGTTCTTACAGTAAGAATAATTCCTGACATCTCCCTGCTGTGTCTGAGGGTAGGGT
AGGGATCCGTGAACTTCGGCATAGGGGTAGTTTCTGGACGATGGGACGAGGAGCTGGGCAGAGTGGAGCCGAGTCATGGAAATGTGCG
AGCCAGTACCTCGTGCTGGTACTGGCTGCACAGTAGAGAGGGTTGGAGGAGCAGTGACCTGGGAGTAGAAGATTCTGCAGATGCGTG
GCTGGAACTTCCTAGCAGGTATTCAGTGTACAGTTTTGCCATTATTCCCTAGGTCCCATGTGAGAGTGATCTAGTGGTGGTCTCTCT
CGTCACCAGTGGAGATGAGCTCCTGGACACTCCTATAACCCAGCTATGAGCTAGTATCTCACCCTGGTCTCCAGGCTGCAGGAAAGC
CTGTTGGCCAGCTCTTGCCTCTACCCACCATGACCTCTCTCTCTCTAAGGCAAATCACTGGCTCCACAGAGTATTTAGAAATATT
TCTATTTTTTTAAAGGTTTTTAAGAAGGTTGAACTGAAGACAAAGGAATGGTCTAATGAACAGCAAAATATAATGACTCAGTTATA
AAATGTCAAGATGATAATACAATAAATAAAAAGTTAATCCATTTTGGACTCCACTGTGTTGCTCTTGTTGTGGAGAACTGTCATTG
TGGCAGAGTAATTAAAACCCTACAAGAGGGTTAGGAGTCAAGAAGGAGAGAAAAGAGGCAAGGGGGAGCTGCCTAGAAGACAGGAGA
CTTTCTAGAGTGCTGAGGCATGCAGTTCCGAGAGGTGATATTTTGCTTCTCTGACTGCACATTTCACATCTATGAGGGCTAAGCACC
TGGGTTACACAGGTGACCCGAATTGGAAGTGATCACGATCTGAGATGGACCTGCGCGTTGCTCTGTAAATCGCATGGTTGCCAACTAA
GCCACGAGCCCTGCCTCACACTGCCTTTACTGCATTTTCAAAAGACTTTCTTATTTCTAGACCTTACTAGGTCTTGCTCACTGTTTGT
GCTAACACACGCCAAAGAAGATGAAAGCCAGAGCGGTATGTTACCCTCTGCCGTTGAGCTCCTGTTTGTGTCAGCCTGGTCAGTC
TTCAAGTGCCAACAAGTCGTTCTGGTTCATTTTTCTTGCCCTTCTTTATTGGCTCTGAGGTTTTCAGTGGTCAGCCTGTGCTTATAG
CTTCAAGTCATTAACTTTGATCAAAACAGGAGGGATTTGAGAAGTCAAAGGACAGAGGAAAAAATCCAGAGCTAGCCAGAATCTTTG
GTTTTCTATAGAAACGAGTGAGAGAGAGGGAACGTTCCAGGAATCAATATACTGGCTCTGCCCTTCCCACCCTGGCCTAGTTAAAGT
GGCTTCTTCCTATACCATTGCACGTCTGGCTGGCATTGGGAATTATTCTTAGGTTGTCAGGTTCCATTCTGGAATTAAAAATGTATAA
TTATCTTTTTAAACATTTTTGTGTATTAGCTTTAAAATCAAATAACATAAATGAATCAGATGTACTTTTTGACTGTCTTTTGGTTTC
TTGCTGCCAAGGACACCCCACCGTGGCATTTGAGTCCAGCTGGCAGCGATACTAGACTCAGCACAGGTCACCTTTGGGTTGCTCTGC
TATTTCAGTTCTGTTGGAAGTGTTGTGCACGGGGGGCCCTTGCCTGCCTGGGACGTGTGAGCCTGAGGTGAGTTTGCCCACACAGCC
TTTCTCTGGTTTGCAAGAGAGTCACAGGGGTCAGCTGGCATCTGCGAGTCTAGCTGAGGTGATTGTCTATGTGAATGTTTTCTCAGAA
CCAGATAAACCTGTGTCACTTGCAACACTACCACTACACACATCGGCACTAGCTTGGTGACAGTGGATGGTTCTACTCTAAAATGGT
TTTTCAGAGACTCGACTGTGTGAAGCCTACATAGAAATGAGTATGATTGTAATTATGATGAGTAAGAGCTTATTTTCTTTTAAAGAA
GGTAATTGAAAATTTAATTTCAACTGACATGTCTAATCTCTTCTATAAACAGATAGAGTTTTATTTTGAGAAAGGAGCCTGGATAGT
CCAGGGTGTCCTTGGCCTCGTTAATGAGACAGAGATGACTGTGACATGTTTGTTAGCCCTTGTGTGAGTGGTGATATGACTGGAGAG
CACTTCCACATGCAGCTTCTATGGCATTGGACAGGTACTGGGGAAGCACTCTACCAACCGAGCTATAGTCCTGGCCCTTTAAAAGTC
TGCAGGAGTTTGTTACATGGCCCAAGCTGGCCTAGAACACACTATTCTGCCTCTGACTCTCAAATGCTGATATTACAGCTATGCTTG
CTACCGTGGCTGGACAAAATACGAAAGTGGCCGGGCGTGGTGGCGCACGCCTTTAATCCCAGCACTTAGGAGGCAGAGGCAGGCGGATTT
CTGAGTTCGAGGCCAGCCTGGTCTACAAAGTGAGTTCCAGGACAGCCAGGGCTATACAGAGAAACCCTGTCTTAAAAAAAAACAAAAC
AACAACAAAAGAAATACGAAGAAAGTGTATTCATTCCGTAGACAAATCACTAATAAAAGAATCTCACAGTGTCCTACCTAGCCAACT
GTCACATACTGTGGATACTCAGATCTTCGTAAAGGTGGGTGGGAACTTTCATTCATTTTGATTGGGGTTTGTCGAGTAACACGGACC
```

145

```
TGCAGAGGATGTCGTCATTGAGTAACACGGACCTGCAGAGAGTATTGTCATCATTTTCTGATTTGTAGATGGCCGCCCTTTACACTG
CTGATGAAGCTCAGGGTTGACTAAACACACCTCCGCAGCCTTTGCCTTCTACATAGGCCTGAACTCGAGAGAAAGCTGGGCGTAGAG
CAGCCCCTGAGAAGCCTAATAGGGAGAGAGCTGGGCGTGAAAGCCGCCATGCTGAACTACCCCACATAACACAGTCTTCTTGGCTGC
CTTGTTTTCTTAATGGTTTTAGTGGGGAAACCAATAACATGTTCGTTTGAAGAAGCAGTTAAAAAATAATCAAACTTAACTGAAAAC
GGAGCCCCCGCCTTTGTTAAACATCCGTTTATTTGGCTTGCTTTCTGTCACGTCTCTCTGAAAACAGAGCCATGAGTCCTTTCTGTT
TAACTGATCTGCTCGGTGCTAACCCAGTGCCCTTGATAGCTCAAGTCTAGGGTCTGAGGTCTGTGTGAAAGTGCTGTGCCTACACCA
GGTTTGCCATGGCAGGCCGGGAACTGGTTTTCGTGTGCAGTGACGTTGTGTTGGTCTTGAAGCAGGGGAAGAGGGCTTGAGGATGGTG
GAAGAGGTCAGAGCGAAGTAAGGGAATGGCATAGCCAGCTTGGGGCCTGGAGCAGGGGCAGGGCGTGGATGCTGGCGATTGAGAGTT
GGGCATGGGGGCGGGGTGCAGGTGCCAGTGTAGAGGAGTCTCTCCTCCCTGCCCAAGCCTCATTGGTCTTTATTTCCTTGCCATCAA
TAATGACATCAGGGCTGGAACAGAGGATACATTCAGTGTTTCGGATGCACCCACAAGTGAGAAGCTGCCATTGCCACCTGCCTGAAG
CTTCCAGCCGGCATATGGAGGGTCCCAGCCGGCATATGGAGGGTCCGTGCACTTCTTCAGGAAGCATCAGACCTTTGGCTCAGCAGA
AGGGATTCCTGAGCAGGTGTCTGGGTGGGAGGTGAGGCAGGAAGGCAGGCTGATGTTACTACCTAACGGAGAACTGCGGCGTCTTTT
TCCCTTTCTTCTGTAGTCAAGTGGCTCCTAGTTCAAGGGCAAAAGTTGGGAAGAGTGTTCAGCTATGCGGGCCGGCCATACTCTGTT
CTTCCTTGGAAGAGACAGCCGCAGATGGCCCATCCCTTTGCTGTAGCTCACCAGTCTTATCTGCTCAGCATTAGGCTCAGGGAACTT
TCCAAGCACAAAGGAACCATTCTCGGGTTCCAAGACTGGAAAGGCCTTGGAACTGCGTTTTTAAAATTTCACTTTATTTATTATTTA
TTGTTTTAAACATCTTATCCCTGAAGCCCAGCACAGGGGCTGGCAGACTATAAATGCTAATAGAAAAAAAGAAAAAAAGAAAAAAAAAA
GGCAGTTCAGGCCTGGTTTCTTGGAACAGAGCTGAGAGGAGAATTCGCCTTAGACTAGATGAGCCGAGAGTCACTTTAGGATTATTT
CCTCTTATCGAAGTGTAAGTATTTTGGGGGTGGGATGTGAGTGAACTCCCTCACGGCTGTCGGAGATAAAGTGTGGGGTTTCTGCT
CACGTTGCAAGTTCCCGTCTCAGGAGGAGGTTTTGTGAGACTTGGTCTGCCTCCTGACCGTACAACTTTGCATAATTGATTAACCAA
GTATCGCTGTTTGCATTCCCTTCCTAAAATAAATTCCACTTTTTAATTAACTCTCCAGAATTAATAAGTGCCAAAAACGCAGGATGT
GTGTTGAATCTTGTGATTCGATGTGATGAAGGCAGCTCCCAGGGAAAACAAGTCTAACAAATGATTTTGAGTCCGTGAAGGTATTCT
TAGTTCCTGAGCATGGTAAACAGCTCTCTAAAAGGAGTTAATGCCCGCTGGCCTTCGTGTGGGCCAAGCGCCATGCTATGCCTAGGG
CATCTCGGCTACTCCCTTTGGTTAGCTCTTTATGAACCCAGTACCTAGCTGAGTGGCAGAGCCTTTGCCTATAACCGAGGGATGGGT
TGGGATAGGGAATTGGGTTCCATCCGCAGAACCTCAGGAACACACCTGCCACATGAACTGGGTGTGGCGGCGCACACCTGTGAACCC
AGTTCGTGGGAGCGGAAGCAGGAAGATTGAAAGCAGAGGTTCAAGGCTATCCTCAGCCAGCCTGGATTATACAACTCTAAGATTAGC
TTTCTGATGATCCGTATCTCCACAGAGAAGCCGTTTGCCAAATTGACGCTGCCAGTGTCTAGCAGCCCTGAATTTGATCTGCAAGGC
CAGACTCTCAAACAGTGTTCCTCCAAATCAGGCCACTCCCCTCCGCTACAACCCCCTATTAGCCCCTTTGTATCTTCAGGTTGTCT
TTACTGCAGCATCTTCTCTCAGGTCCACTGTGGACTGTCTGGTCAGACGTGCTAAACATGGTGAGTTTGTTGGTATTGGAAATGTCC
CAAATACATGGCTCCTAATATAGAACCCAACTTAAGGAAGTCTTTAGGCAAATGACACCACTTGTATAATGGTAGGTCACAGCCCTC
TAATGACTTGGAGTCAATGCTGGGTCATTCTGTGCCTGTGTGTTTCACAGAAGCAGAAACCTAGAGCCGCAAAACCAAGCAGGGAGG
TTACTGGAGCCTCACGGGGAGAACACAGCTAAGGATTCTGGTGGGCAGGCTCTTGGGAACAGGAAGAGCGGAAAACATTGCAAATG
AGTAGCACCCCCATAGCTTCCAAAGGAGAGGTGAAGTCTTCACCGGAGTCCAAAACTGGCGATCCGGCGGCTGTCCTCTGAATGAGC
AGAAATCCGAAAAAGGCAGCCGGAGTATGGTTTTTCAAATTGGACTTTAATTTTTTACTTTCAAATAGACTTTAAAGCAGTGGTTTT
CTATAGCTTTATGAATGAGGAGGATCCAGTGAAGTGTCGTATGTGAATAAACAGGTGTTAGACATGACTGTGTGGCCATCATGGATG
GCCTACTGTGATTAATGCACGCCTAGGTTATTTTCATTAAAAAGTAAAATGTGTTCGTTCCTATTGTAACAAGAAAGGCTAAGTATT
AGGGGCAGAGGAAGGACGGAGTGCAGGTAACCGACGACGGTTATAAAAGAGGATTCAAAGCTAATTGCTTTGCTAGTCCTAAACC
CTGTGGTAGATTCTCTTCTTTTCAGTGGTAAGTGTATGACATGGATTTGATGCTGAAGTCTTAATAACACTGGAAAAGTCGATCGTG
CCTATAAGTCTTGAGACAAAGAAGAAAGCAATACTTTCTGATGTTTTTTTAAATCCTCTTGTAAATGATGTGAGCATAAAGAAGGTG
ATAAAACACTGCAGCCCTTCTGAGACGGAGAGGAAATACAATAAGCTGATACTTGAATGTAGCACTGAGTATCAGAGAGTTGTAGTT
TCTGTGGAAACAGAAAAAGTTTTCAGAATATCGTGTAGCTGTGTAATATGTTGATTATTTAAGGGAGGGGGAGCCTCGAAGGAGATT
TTTATTTAGCTTTAATGGTTTTCTTTATTTTCCTTTCGTTTTGTTTTTGGTTTTGTTTTTATGAGAGATTGTTATTGGTTGTGAATTAA
GAAAGAAAACTGGTGAGATATTATTCCTGATCCTCTGGACCAATCTAAGGAAACAAAGGAATTACTGCCCCACCCCCACCCCCCTTGT
GGTCCTGACTGGCCAGGACAGACCTTAGCATGAGAAAACAAGTAGGAGAACTAGAGACCTGGTCCCAAGGCTCTCACACTGCCCCC
ACCTGCATCTGTCGAGGGCTCAGGACTGAGGGATTAGTGGTGATAGTGTCTCCTTGGACGGACTGACAGTGACGAGCTGTTTCTTCT
CTAAAAATGAACTCCCCAGCCAGCTGTGGATATACACTCAAGTTTTTAGTGACATTTTAGAAAATCTGCATATATGTCCAAGAAAGG
CCTGTGAAATGTTTGAGAGTTTAAGATTATTCTGAAGCTTTATAAAAGTGTTTTCTGTGATTTTTTGTTTTTCTGGATATTTGTATCCC
TGCCTGTGTGCTATCATGGTTAATTTGCACTGTATCACTATGCCTGAGAAGCCTCTATTAGTGACTGTCCCTGTCATCCTGTGAGCT
GTGTCTGTCACCGCTCTGTGGACAGGAGTGTTCCCAGGAGACCCGGTCTGAAATGCTTGGACTTAGGAAGGCCCCTGGGAGAGAGTT
CAAGATCTATGAAGGATGACTATTTAAGACTGGCCAGGAATGAGCCCAACCCATTCACTGCACAGAGAGACCATTGCTGCTGGTGCA
AGAGTGCAGTTCAACTTCAGTGAGGAGGCAGAAATACAGGCTACACCTTTAAAGGTTTGAGAGGTTGGCTGTTCAGGGTGACAAGGA
TTACCTTAGGGATTAATGAATTAAGAGAGGTGGAAAGCTAAAGCCGTCCTTTCTGCACCATTCAGTTCCATTGGACGCATACTTTCC
CAGGCTGAGTGGACCATTTGCCTTGGCGGAAACTCACGGCCTTTGAAAATGATGACATCGGGCCAGTTCTGTAACTATGATTTATGC
TCGAGTCTGTACACCGTGGGTCAGATTAATTCAGTGCTGCCCTAAGGGACCCAGTTGTTGAAGAACTGCTATCTCCACACCCAGAAG
CTGTGTTAATGGTCCACTGATGCCCCGAGAGCCCGTGTTCTTAAGGTACCAAAGTGCTTTTGTGTTTGCTGCAGGATTATTTTAAGT
CACAGGCGCACTTTGGAGATGATCACCACTCACATTTTGCTTAAAAGAGTAAGACAAGCCAGCCTGAGGTCCAGAATGGTCGGTACT
GATGCTGCACCTTGGTCGTTGGTGGGAGGATGAGATCAGTATTTAACAGTTGGAGAATTCTCAGTCTTGTCAATCAAAGCATATTA
CATGGGTGTTTCCACACCATTTGAAGTGATGATTTAAATTCATTTCTAGTTGTGAGCTGTGCGAGAGTGGACGCCCTGGGCTTTGGA
AGCTGCTCTTTCTCCACGTGGCCCTTTTAAGCTGCGGTAGATGTTGGTGAGGAAAGGCGTTCCTGTGATGTTTTGTCTTTTCTTTTTT
TCAACAAGTGGTTCCCCCCACCCCTGCTCCCACGAGAGGCAGGGTTTCTCTGTGTAGCCCTGGCTGTCCTGGAACTCCCTCTGTAGA
CCAGGCTGGCCTCGAACTCAGAAATCCACCTGCCTCTGCCTCCCAAGTGCTGGGATTAAAGGCGTGCGCCACCGCTGCCTTTCGAGA
TGTATTTTCTTAAGGGTGTTTTTCCTTGTACTTCCAGGTCAGACCTTACGAGGACAAACTCAATTGTGGATTTAATGTCTGTGTACT
CCTCAGCGTCTCCAGGTCCCCTTCAGAACTTTGTAGAAATTGTTAGTGTTGTTTGGGTTGATAGCCATGGCCAACCCAGGGACAGAA
CAAAGGAGACATCTCTAATGAAAGGAAAGAGGGTAGAGGCGCATTGGTGTGTATCCTCCGAGACTCCTTCCCTCACAGGAAAGAATG
TGTATCCTTTCCTCTGGGGGAAGGGTGGGTGACTGGATCAGCCCAGACAAAAACAACATATGGTTAGTTTTCGGAGGACTTGTAGAC
AGATGGCAACCTATAGGCTAATTATGCAGTGGGCGACATTCTTTAAACAAATCACTGCTTTTGTGGCATGGTTCTTACTCACAAGGT
GACAATCTGAACTGTTTATTGTCACACCAGGTAAACATGTGCCAGGTGAATGCAGGGAAGGTGCTACCCGAGGGAACATAGTTATATT
GAGTTTCCATTTGCATCTTGATCTTCCAGGCCAAAAGAACAAAGAACGGCAGGAAATTCACAGAATAAGACAAACCTTCAGGTCAGG
GTAGGGTGTAGCCCAGTGGCACACTGGAGGGCTAGTGTGTGTAAGGCTCTGGATCCAGCCCCCTAGCAGCTAACAGATCACCAAAGT
CGTCATGCTAGGGGCTGTTGCCTTGGGGGAGATGACCTGTGTCAACCTTCTTGTACTCACTCGGAATGGGATGTTTCCCGTGTCTCT
GCCATCAGAAGCAGGTTGTCAGAGCACTGTGTCAGGCTTTGGATGAGCAAGGTGGCAGGTCACCCCTCAGAGTTCTGCCCTGGCT
CCTTCCTGTCCAATCCTGTGTCAGCATGTTCAAGGTCGCCGTCACCCACTCCTCCTTCATGCTTCCTCTTTCTCATTTAGATTTTTC
TTCACTGGCCTTTTAACTATCTGCAGTACAGGTTTTGTTTTTAAGTTTCATTATTGAGTGTCTCCTTCCACGAGGGTTGCTTCACAAG
GACGAGGGGCCCAGTCTGTTCTCTGCCTGTGACAGGGCTTTGTGTTTGCAGAGGTAAGGGAGGGGTCCATTAAAACTGCAACCCAAC
CTTTTTCCTCCCCAGTTTGTAGGCATGCCACGTGGGCCCGGTGTGCATTGGTCATGGGCTCTGATGGCTGGTCCCACCCTGTCCTTG
AGAACAGATTAGGGGCTCATTGTAATGTTTGCAATGTCTCCAGTCCCACTCTCTGATGTAGACTTGTTTTTGAACTGAATGAGCCAA
```

EP 2 204 376 A2

```
CTGACTCAGCTGAGGAGATGGTCAGGAGCCAGGCTTGTTCCTGGCCAGCAGCCTGAATGGTGGGTGGTTTCCTGTCCATCCTGTTAC
ATAGGTTCAACCTAAGGGGTGTGGCCATGGTTTAAATTTAGGTCCGATTGGTGTTCAGCTTGGCTCTACCAGGAGTGCTTTACACAC
AGGATTCCTGTTTCAGTGTTAACTTTCTTACATCTCACAAAGCCACAGTGCATGTATGTTTTTACTTATATGATTGAGAAACTATGA
AAAGGTGCTACTAATAGCTTAATAGTGCTTTTAGAATAGTTTTACTAATATTATAAATTTGAGAAATAGCCCAGCACAGTGGGTTGG
AGTCCTTCTCTGGAGTCGTATTTGCCAGCCCTACAAGGGCTCTGATGGCCCATGGCCACCTCTAAGACACTGTGACCTCCGTCTCTG
GAAAGCTCCCCTCTTCATGCAATATATGCTATCTCATCTCTCCCCTGGTTAGCTGGCTAACTTAGATTTTGTTGGGACTGTCTGGCC
AGATTGCTTTTTATACTTAAGTTTGTTGCTTTGTAAAGAGCCCCTGGTTTGAAAGTAGTCATCAGACTAGAATCTGTTAGAATTAGG
GATGTGGGCAGATGCATTCTGACCGAGCTGCAGGAACTTTCTATATGCTGGTTGGTTGGTTTAGATGGTTTGGTTCCAGGCCTTGAG
AGGCCAGGTCCTTGGTCTTCTCAAGGATTCATTAGGTGAGGAAGTGCATGTATCCCTCACAGGGAAGTTGGGCTCAGTTCTTAGGAG
ATGGGGTATGACTGTGACAAGGTGGGAGGTCTATACAAGAGCAAAGGTGCCCTGGTGAGAGGTCTTAAACCTGATTGAGGGCACAGG
TAGTAGCACTGAGGACTCACAGAAAGATTAGTTCCTTGGTGCTGTTCCCTGTATCCTCATCTCTGATTGGGTATTTTGTTGTTGTTG
GTTCATTGTATTTAGTAGATTGGGTTAATTCAAAAAGGGAAGGTAGAAGAGCAAAAAGACTTTTTTTTGTATTCATTTTTAAAAAGG
AATACACAGTCAAGTTCTTACTACAAAATAAATGATCCTTTTGAGGGGAGTCAGAGGGCCTTGTAAAGTGGATACTTTGTAATATAT
ACAGAATAAAGTGGATACTTTGTAATATATTTATATATTATATATGGAATAAAGTAGATACTTTGTAATATATATGGAATAATGTGG
ATGCTTTGTAATATATTTATATATTATATGTGGAATAAAGTGGATACTTTGTAATACATATGGAATAAAGTAGATGCTTTGTAATAT
ATATAGAATACAATAAAGTAAGAACCATCTTTAGCCAAATTCCCATAGTAACTATAGTAAGAAAATGTTTCAGGCAGACCCATGGTA
ACCCTCAGTATGGACCACTGAAGTGTGTGCTCAACAAGCTGCACACCAGAGGCCAAGGGTCTGAGGAGCATAGTTGAAATATTTTCT
CCCAATCTTCAAGTCTTCTGCGGAGACAGCAGGGAAATTATACTGAGATAAGTGGTGAATAAGGGTGAGAGAGAAAGATGTAGGCTA
GACATAGCTCTCCAATCAAGACTGTGTTTCTACTTCTTGCTGTTTGTCTGCATTTTTAAAATACCCACCATGCAGTGCTCTCAGCTC
TATTTTCCCTACTGTTTTTCCTACCCAGTGATTGAGGGGAGATTATGCTCTATCAAGAATTCACAGTTTTGGCAGTCTCATGTCGTC
TCTGTCATCTGTTCACTTGACCTCATTCTTGCAAAGGGCATTTGGGTGTGTCCTGGAATTGTCTTAAACTTTCAGTGAATAGAGAAC
TCAGATCACATGGATTGCAGTTTGGAAATCTGCTGTCAGCTGATGAAGCTGAATGAATGTGTGGTCTCGGTGTGGGCACGGCCCTAG
GGTGTGAGTTGTGGCCGAGTATTAGCTGTGTGCCTGGCATGTGCTGTCACAGGTCTGCACATGTGCCTCTGTTTCATTTCTGGGAGCT
TCTCCACGTGCTGCCTTCACTGAGCAAATGAGATTAGATTTTTCTAGATCTCTTGGCATGTCTTTGTCACCACCTTTATGACTAAAG
GACATAAAGTTAAGATATGATTGATGTCTAATTGAAAAATAGCCACTTCTGTGGATTGAAACCTTCAAAGAAGCGGACTGGAGAGAT
GGCTCAGAGGTTAGGAATGCTCATTGTTCTTGCAGAGGACTGGGGTTTGATCTCCAGAACACCTACATGGTAGCTCACACTACTCTT
ACCTCCAGTTTCAGAGAATTCAAAACCCTCTTCTGACATCTGCATCTCGGTACAGGTACCCTCTAGAATGCATTGATATAATCAGCCA
GTCAATCAATAGCCAGTCAATCAATAGCCAGTCAATCATCAATCAGTCAGTAGTCTCGGTGCTCACTGTTCAATCATTAGGACTCAA
GTTGGATTCCTCAGGAACCCAGGTAGCATGCCTCCTTAATCCCATCATTCCTAGAGAGACGGGGGGGGGGGGGCATGGACAGAATCC
CCAGAAGTACTCAGGCTCGCTCACATGGCATACACTAGACAAGAGACCCTGTCTCAAATGAGACCAAAGGTAAGGACTAACTCTGGA
GGTTGTCCTCTGGCCTCCCCACATGCTCGGTGACATGTGCACACCAGTATTCATGCACATAAACATGTACGCACATAGATACATGAC
AGGTGTCTCACATACACCTCAAAAAGATGAAAAGAAAAAAAAAGTCATTGGCATGAAGGAAAGGCGTTGCTGAATGACAGAATGCCC
TGTTTTCCACCAGCCGTTGGAGTAGACAGCTACCGAGGGTAGCCTCTCAAACAGACTCTTTGCAAATCAACTCCAAGTTCAGCAAAG
CTTCCCCGGTGGCATAGACAGGAACTTGCTCTGAGTTTCGGAGACCCTGAATCTCTCAGGGTCATTTTTGTATCTCTTACCCCCTGT
GATGGTTAATATTGATGGTCAAAGTGCTAGGATCTGGTGGCACATCTACAAGGAATTTTTTTTTTTTAAGATTAGGTTTGAGGTGGG
GAGAATGACCATAAATGTGGACAGAACTGTTTTATGGGCCAGGGTCCTAGACTGAATCAAAAGAAGAAAAGCAACTGAGCAGCAGTG
TCTTCCTGCTCTGTTTCCTGGAGGTGTATGCCCCACGACCCAGCTGCCTCACTTCCTCCTACTGCCCTATCATCCTCGCCATGAGGGA
TGGTGCCCTCTATGAGCCAAAAAAATCCAGGTATTTTTTTCACAGCAACAAGAAATGAAGTGAATACACTTCCCTAAGGCTTCTCTTG
TGTTTTCCTCTTTTTCCTCTATTCCTACCATTACTGCTTGTCCTTGGTACCTTGTGCCCCTCAGCCGTCTAAGCTCTACTCTGAACA
TAAAAAGGGCTGATTTCTTCATCCTGATAACCCCCTCAGGACATGGCTGCATAAAACCAGATCCTAGCCTGACTCAGGCCAGGCTCA
TGCTGTCTCTAGTTCCCTTCTGTGGCCTGGGAGTCTTGGTGCAGGAATGACAAATCAGTACACAGTCTCCTACTGTTTGCCAGGTCC
CTGGAGGAGCCAGATCAAAGTCAAGATCAGATGCTCTGTTGGCCCCTGAGAATAAAGATAGAGAAGTCACAGGGTCAGTAGGCATCCAGCTTAA
GTCCACAGTGTCTCCTTCATGACCAGGAGGCCTCCAGTGTGGCACTGTGCAAGTTGGCTTTGGCTGCTGTCAGTGTCCACTGTGGAT
CCTGATGTTGCCCATGACCCTTTCTCTAGGAATGCTAGTCTGAGCGTAAGGCCATTGTTCTTCTCTGTCTCTCTTTGCTTAAGAAAG
GCCTCCGGGCAGTGGTGGCGCACGCCTTTAATCCCAGCACATGGGAGGCAGAGGCAGGTGAATTTCTGAGTTCCAGGCCAGCCTGGT
CTACAGAGTTAGTTCCAGGACAGCCAGGGCTACACAGAGAAACCCTGTCTCGAAAACAAACAAACAAACAAACAAACAAAAAACCAA
AACAAAAAAAAAAAAGAAAGAAAAGAAAGAAAGAAAGGCTAAGCCCCCTTTTTTTACCCTTAGACTTAGAACTTTTAACTTGATAGGTGT
GTTGAGAGGGGTTGTTGCACATCTGGTTCTCAGAGTTCATCTCTGAGGAGCAGAAGTCTTTTTTTTTTTTTTTCTCATTTCAAAACCC
CACTGTGCCCTGTTCCCCTATCTTATTTCCTTAAAAGCTGTTAAGGCCATAATTTTAGAGATGGCATTCCATTTTTCTTTGCCTGAT
TTTCTTGGTCAGCCTTTCAGAGTCAGCCAGCCTTTTTTTTTTTTCTTCCCTCTACTCTTCTCCATTTTAGAAAGGGGCCTGGTCCCA
TCACAATCTACCATCTGTCTCTGATGAGGTGTCCTGAGCTCTAAAAACCCATCTGGACACTTTCCACCGTCCTTAAAGGTACAGGAT
GCTCCCGGGTCACAGCTTTATCCTGAAGTCTGAGCACTTTGTACGCGCTTCATTTAGTCGTCTTAGAGAGGGACATTGAAATCTGAG
GCTCCTTGGATTTTCAGGTTAGGGTTATTCAGTCCATAAAGTCTATGCAAGAATTCTGAAATCAAAAGAATTTCCGAGTTCAAGCTA
CCACTGCTCTCAACTGCTTCATATGCAACGCTGTGTTGTTAGCCCTGCTTTACAGGTGAGGATGGTGAAGCCCAGAATCTCAGTCTG
GTTAGAACCGCCTGGGTACCCTTTTCCTCTGCTCTGAATGCCCAGTGCCCTCTAGTGCACCCAGCTACACTCCTGGTTGACCTGCCT
GTCCTCTTGGTGTGAGCCCAGCCCGTTTCTGCATCTCTGTCTCTCTGCCCCAGGCGGTACTTTCCTTCACGCCCTCTCCCTGCCTGT
CATCTCTGAACTCCCGAGGTACTCAAGGATATTCTAGAAGCAGACCCATCTGTTGTGACCCAAGAGCCCATGCTGTGTTGCTTGGCAC
AAGTTGGCTAAACTCTTTGGCTCCCTGCAAAACAGGCTTCATAAGGTTCCTAGAGGATTGTATAAAAAAATTCTTTGTAAACAGATC
CCCCTCCCCTGCCATTCTCAACACATCAGCTATTATTCAGACTTCTTACTACGTCTTACAGCAGCAGGATGGATATATAGGAAGGTG
TTTCTACCAGCATGGATGCTCTGAGAAATGAATAGTTACGTGAGTTCACTGTTCAAACACAGTGCACTTACGTAAACCAAGATGGCC
CCATTGCCACTAGACGGTGGGCCCCTCAGACTACTTTTGTATATTTGGCCAGCTGACAGAAAGATTATTATATAATACATGACTCCT
CTTTAATATTGTGACTCAGAGGCTGTGAGCACGCCCGGTTTTGTCAGGACATTTTCTTCTTAGTTTGTGTGTGTGTATGTGTGTGT
TTGCTATAAACAATGCTGGTTTGTGTTGTTGTTAATTGATGGATGACTAGCCACTGTCAGAGGCCACCCACAGTGACCTGAATGGCACT
AAGCCCCTTGANNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNGGCAAACTCACCTCAGGCTCACACGTCCCAGGCAGGCAAGGGCCC
CCCGTGCACAACACACACACACACACACACACACACACGCGCTCACACGACACGTGCACACTCATATGAAGCTAGAAT
TTGCATATGGGAACATGTTTTTGTCTTTCTGAGCCTGGGTTACAGTATTTAATATAGTAATTTCCAGTTCCATCTATTTTCTCTTTC
ACATCCACAATTTCTTCTTCCCGGTGATTGCATAAATTCCATTGTTTCTACGCTCCATGTTGTCATTTTCCACTCTTCAGTGGATGG
ACACCCGGGCAGTGGTGGCTCCTCTTCATTCTCCTGCACCCTCATGGCCCTAGCCAGTGATGGAGACTCAGCTGGTTCTCCTTGTTG
GAACTTTGGTCTGGGAGTGAAGAGACTCAGAGGACAGATTTCTGCTATACATAAATCATTTGCCAGTCACTCATTTTTCAAGGGCTT
GTCACTGTCAGGGAGGTCCTGGGGATAACACTGTTTCTGCCCCAGCCAGGAGCCCACAGGTTCCCTGCACTTGGTACCAGCTGCACA
CAGCTGTCTCTACAGCAGCCCTCTGCAGAGTTTGGCCTCTGCTGGAAGCCAAGGCATGCTTTATTGGTCTTCAGGGAATATATTCCTC
AGGCTTGCGTCTGCCATCCAGTGTGCTCTGTGCCTGACAGAGGTCATACCTCCCTCCGGTCCCTGATACCACAGCTTCTTAGTCTGA
GCTGCCCTGCCCCTGCTGGCCTGGTAGTAACAGAATCCTAGGCTTTGAGAGGAAGGTGCAGTGCACACATGCTGCCTGTCTGCCTCC
CAGCAGCTGGGCTTCCCCTGTAAAAGCCTCTAACATCCTCAGCTCCCAGGAGGAAACAAGCCTGCTGCTTGTGACACCAGAGCACCA
```

147

EP 2 204 376 A2

```
ATTTGTCACCCTCTTCACTGGCTCTGGCCCTCACCTCCTCCTCTCCTCTATGGCATCTTTCTTTCCAGGGCCTGCTGCAGCTGGGCT
CCAGCCCGACACTGAAGCCCCACGTGGCTGTAGCCTCTGCTGTGGTCTGTCCATGGGGTTGTGGCTACTGCCTTATTCCTAAGCCAA
GGTCTGTCCGTTGAGCATGTCGTTCTCCTGGGAAGCTTGCTTGGGCTGATCCTCGCCCCTCCTTTACCACTGTGCCCAGCCACTTGA
GAAACATGCAGGGCCCACTCAGCACAGAATGCAGTTAGTACCTGTAGCTCTCGCTCCGCTGCAGTTCTGTGAGCCGCCTGCCTCCGC
TGACCCTAGAAAGAGTTCCGCATTGCAAGTGTAGATTTCAGGAACCAGAGTGTGGCTGATGCTTCTCTTAATTTTTTTTTTTTTAATT
ACATTTAGCATGTATGGCAAGGTTGTAAAGGTCAGAGGACAACTTGCATGAGAGCATGTCCCCTGTTATCATATATGGGTACTGGTC
AGGCTGTCAGTGAGTGTTTTTGTCTTTCCATCTCCCACCCTCCAGCCAGCTCCCTGAACTGTGAGGCTTTCTCTCCATTTCCCCAGA
GCAGACCCCAGCCCCCCACTCTCCATCTTTTCCTTTGACCCAGGCCCTGCCCTGTCTTTCTCATTGAAACCTAAGTGCTTTTCTGG
GTTTCCCTTCGGCCACTGAAGCCCACTTGCTTTCTCTCCAGGACTCTGTGTTCAGTCGTATAGTGGGTTTCTGGAAAGCCTTGGAGG
TGGGGCTCATGGTCTGGTGAGTATTTGGGGCCAGGACTGCTGTGTGACTACATTACTTTCTGTCTGCAAGGGACACACACGGGTGCC
CTGGGCCACCCAAGAGGGAGTATGAGCATGAGGTCTCCTCTGAGAGATCTCGGGGTTTTCTTAACTGGGACCTGTTGCGAGTGTCAT
TTTTCATATGCTGGGGGATCATGGCTGCATGTGACAAAAGGCACAGTCGACGACTTCAGGAGAGGCCAGCAGAATTTCTACATGACA
GTTCGCTCAACAAATTTCCTTCTAGAGGATTAAGTGAGTTTAAGAGCTCTGTGTAGAGCCGGATGTGGTGACGCACGCCTTTAATCC
CAGCACTCGGGAGGCAGAGGCAGGTGGATTTCTGAGTTTGAGGCCAGCCTGGTCTATAAAGTGAGCTCCAGGACAGCCAAGGCTATA
CAGAGAAACCCTGTCTCAAAAAAACCAAAAAAAAAAAAAAAAAAAAAGCTCTATGTAGGTTTTTCCAGGTAGCAAACATGTGTTCATA
GGGTCTCACCATGTTGTCCAGACCAACCTTGAATTCACCATCCTCCTGCCTCAGCCTCCTGTGTCATCACATCTAGCCTCAAATTTT
CATAGACACCCTCCCCTGCAAAGGGGACTGAAAGGTTGTTCAAAAGTTTGTGTATAAAATTCCGACAGCAGGAGAGCTGAGAGGCTG
GGGGCCGCGCTAAGCAGTGATCAGCTAAGTATGGTTTCAAGAGTCTCGCTAAAACACTCAACGGTTACACGAAGCATGCGAGTGTAT
TTTACATTTCAGCTGACATGTGGGAATTGGATGCGACTGTGATTTTAATAAAATAAATTTCATCATGCAGGGAGAAGAACCAGCCTA
TCTCTGAAAGGAAAGTGCCGTCTATCTCCTCATACCAAAGGTTCCCTTGCTCAACAACTGTCACAGATGCTATGACAAGCTGTATTG
TGCAGGGTGTGGGGTTCGTGAGACGCAGCTTTGTGAGGTGGCAGGTATTGAGCTGTGATTTATGTCCCATGAAAATCCATCAGCCAT
TGTCCCACTTCCAGGGCCGCCGACACTTAGGGTAGTAGCATTGCCTCTGTCCAGGGCAGGCCAGAAAGGCTTCATTCAGCATGGAG
GTGCGGTGGTCGGTCGGGGGCAAACATAGCCCATTTCTTTTCTCAGGGAAGGTGGGAGGATGAAGGTCTGGGGCTTTTGAGAGAAAC
TTTTTTTTGTTGTATTTAGTGTGGTGACAGTACAAATGAAAGAGCCTTACAGTTTTAAGGCACCATTAATTTTAATGTGGGGATCTGA
GCAAGTGACAACATGAAGCTAGATGCCTCATTAATAAATGTGTGCGAGGTATTTACCCTCGTTAAGGTTTTGAGAATGGGGATGAAT
CTCACTTCGTTCTGTTGAAGCTCTTCATTAGTCTCAAGTGAACTGGCAGAATATTAATATGTACATATTAATAGGCTGAAGAACAAT
GATCAAAGCGGCCCTGTTTATATCTCATAGTAGTCGGTGGGCATTGCTATTGTTTATGTGATCCTTGGAAATAATAGCTTTTGTTCT
TCCTAAAGACTGTCTTCGGGATGACAATTTGACAGAGACGATAGACTTTGCTTCATAGGCAAGCACTAAGTTGTACCCTTGAACGCA
GAGTGGCTGTGATTATCTGTGCAAGCTGTCAACAACATTGGGGCTACTGACTCTACCCCTCCCTCAGGATTTATGGGGTTTTCTTCA
GTGGTGTACCTATGCACTAGTAAACAGTCCTAATGAAACTTGTGGGGGTCGATCTCTCTCTCTCTCTCTCTCTCTCTCTCTCTCTCT
AACACACACACACACACACACACACACACACACTCACTCTCACTCTAGTTTGTCCTATTTCACCGATTGTCCCTTCGTTCATTAGGGCC
TAGGCTTCAGTTTCTAAGTACTTCCCCCTTTCTGTGCATCACTTCTTCAACCTAGGTGTCCTTGAAAGGATGAGATCTTGCTGGGCA
GTGATGGCACATGCTTTTAACCCCAGTACAGGAGGCAGAGCAACCAGGGCTACAAAGAAAAACCTTATCTTGACCCCCACCCCCCTT
GTCTCTGTGGCTGGAGACAAAGGGACTGTGATTAAGAGCATTTGCTGCTCTTCCAGGGATCCAAGATCAGAACACCGTGTTAGATTAG
ATGGCTCATAACTGCTTCTGTCTCCACTGGACTGATACCTTCTTCACGCCTTTGTTGGCACCTGCACACACAAAGCACGCATATACA
AGCATACACACAAACGAGACCAGAATGCTTTTAATAGACAGAAGAAGGCACAGCTCGCTAACCTCCCTGTTCCCCCGGTGGAGGATA
AGAGGCCTTCTGCTGCTGTGTGCTCTTTGTGCCTCTCCAGCCTGATTTTATCCACTCTTCCTCAGTCTCCGAACATTCCCAGCCTGC
TGAGCCCAGGGCCTGGTCTAGGCAGAAAGAATCAATGGTGAAGGACATGAGTCCTTGTCCTTCCTGGAGCCACTTTCAGGGGGCTGG
ACTCATCGTTCCCTGAACTCAAATGCTCTTCTTGCCTTAGCCCGGGGACCTGCCCACACAGAGTCTGTTTGAGTTTTAAGTCAGGAC
TGGGCAATGGTGTCCTGGCCCTATAGAATTAGTTCTCCATGTTTTGTGCTGTGCAGCTTACCATTTAGGTCTGCCCTCAGATGAGT
CTGTTGTGTGAAGTGTCTGTCTCTAAGGCTGCCTTTTAGTACCCAAGTAATGCCCCACTGCTAAATGGTCACTCATAAATGCCTAGCG
GCAATGGGAGTGGCTTGAGGTTGCTATCCATCCAAGGGTGTCTTGTTTTCTTCTTCTGGTGCTGGGAACACACACCCCAGTACTTTAC
GCGTTGCTTCTAAGTGACTTACTCCATTCTCAAAAAGAGCCCTGTCATCATACATGCATTTTGACAATTGGACACAGGGAGGCATTT
TGATGAAAAAAAAAAAAAAACCAAAAAACAAAAAAACAAAAAAAAAACAAAAACAAAGCCAGACCCTATTCAGACGGGGTTCTAGTTCAGTTT
GTTAGTGAGAACTGCCGTTGCGGGTTTACTAGAAACAAACATTGACCAGCCATGTCCTGTGGATACATGCAGATAAGGTCAGGATCA
ATAGTTGAGACTTGCTTTACGAAGTGTCCTCTGACCACAGTGACAGTGGCACACTGTGATGGCATTACCCCCCTGTATGTTGGTTC
AGTGTCATCTGTATTGTGACAGTGAAACTGTGTTGGGCGGTCACCGTCACATGACTTTTAGTAAGAAATCCTTACCAGGGGGATACC
TTGGACTCTGTTTTTATAACCTACTCAGCCATGACCTCAAACAGGCTCTGACTGTGGACTTCAGCAAGCTGTCAGCAGAGCTTCGGT
TCATTCAATTGTAAATGTTAGTTCATTTGTCTTCTGTGAGATCATTGACTTAAAAAAAAAAGTCCTTATCATGCACTCTTGCAGCCCC
CTGCCCTTTCCTTGGGTTCCTATTGACAGATCTGTCTGCTCCGTTGCTATTTCAGTGGCATTACCGAGTAAGTGCGGATCGTAACTGC
ATTTAGCTAGATTCTAAAGCCGTGACTATTGAAAAGACCCAACCCTTGTCTCAAGGGAGCCCATTTTGTTACTGTTTTCTGCCTGTT
TGGTCAGTGGATAGTGTTGTTGTAAATATTTAAATTCAATCTGGGTTTCTACCCAGCCTTTGATCATTCAGTTCCCAGATAAAAGAC
ACACAACTTTTATATTTATAATGAACCTTAATCAGCACTAAAGCTTGGCAGATGTCTACCTTTTCTATGCCATTATTCTACTTCCCT
GCCAATAACCCCAAGATATGACTAGCCATGTTCCACCTGGGCTGCTCTTACTCCAGTTGTCCAGCCCTCATGGCCATGTTTTCATGA
CTCATCTGCCCCATGGGGTCTTCTCCTTCTCTTTATCTTTTCTCCATTCTCCTCCTTAATGCCTGTCTCAGACCCCAAACCTAGGAACT
GAAACCCCACCTACCTCTCAGCTATAGGCTGTTGGCATCTTTATTCAGCTAATAGTTTTAAATTAAGGAGCAATTATGTGGCACATC
ACTTGTTCTCTGTCCCTGGGGCCGCCAGGCCTTGGGGTCTAACATTACAATACATAGCAGAAGACCAAACCTCAGCAGAATAGCTAT
CTTCCCTGCTGTGACTGAGGGACCCAGGCACTTCTCTGGTGTAGTTCTGTTAGTAATGTTGGGCTCTGTTTTCTGTAGAAGGGGGAA
GAGGAGAGTGTTCATTTGCCTCTTGCTGACCATAGACAAAAAACAGTTACAAGGCCTTTCAGACTTCAAGAGGATCTGAGGCATGGG
CTCACTGGGTACCCCCTAAGGGGCAAACAGCTTTGGTGGCACTAAACAAACCCTCTGAACTAGATGTAGCATATAAGTAAT
AACCTCTGTGTAGACATTTCCATCACACGAAGGCATCTGCAGAGTTTGCACTGTCACATGACCTAAATCCCGTCTCATCTGAGAGTT
TGCTGAGCTTTCGACCCCATTGCACCGTCCTTTCCAAAAGCTGCTGCAGCTCACTCTCTGTCATATAGTTTTATTTTATCTACTCGA
CTAGAGCGTGATACAAGCAGACAAGATTTGACTGTCTGACGTTGAGCCTAGAATCATGTCTGGCACTTGTCTTCAAGGATTCTTGAA
TTTTTCCAGTGGTAGATTTTATTTGTCATCTCTGTGTCAGTAACTCCTTGCTACCCAGCCCTTGTCATAGGTAAGTTACAGCTACCT
GCTGGGTCTGGTGCGAAGACCCTTGCTGTCCTTAGCACGCACCTCTGTTCTGTAACCCATTCAATGGTATTTCTGTGCCGCTTGCTTG
TAGTGTGAAAGAACTAGTAAGTAGCATACTTCAAAGTCTCCTTGTAAGTAAAATGTATTACTCAGTTATAGGAAATCATGATAACGTA
AGGGAAATATTCTTTGAGATCATGGAGGAAGTTTTTGAGAAAATAATCTCTACTCCACTTGTAGCCTTTGAAAAACTCTGCTTATGA
GTAAGGCAGTCTCTTCATTGCTAATAATAATGGCACTGGGGGAGCTGGAAGGAAGAGACATTTGTCATCCAATGCTAAGTTACCAGC
TTGTCTTGTTGTTTGGGTGGAGCGCTAGTCGTTGGGGAGCAGAAAGCTCAATCTAAACATCTTCTTGGGAGAAAAAAAAAAAAAAAG
AAAAATTCAGTGGCATGTTGGTGGAAATTCCTCAGGGAAATAATGTCTCTGGAGGTGGAAACATGGAACTACTGCATTCGGGA
GGTCATAGTACACTGAGGGGAAATAAAGTGATCAGTGGTTATCTTAGGATCTTATTGCTGTGAAGAGGCACCATGACCAAGGCAACT
CTTTTTTTTTTTTTTCTTCCTGGTGCTGGTATTTTATTTCTTTGTGTACATTAGGCCATGAATTCGTATGGAATGGGCTCCAACAG
CTCAGGCTCTTTTCCGTTAGTCCTCACAAAGTGTGCTTCTCTGGGTGGCACAGGCTGGCGCTTCAGCTGCACCCAAGTGCCCTTCTC
TTTGGCTTCCTTTTTCTTCTGGTCGTTCTCCTTCACCCACTTCAGGAAGCTGTCTCTGCTCTTTGAGTGCTTGATGTGCTCAATCCG
```

148

```
CACATTGATCCTCTTGGCCAGAATTTTGCCCTTAACCTGCTTGTTGACAGTGATGCCCACGGCATGCTGGATAACATTGTAGACTCT
TACGGTTTTGCCATGGTAGCACTTATGGGGCATTCCTTTTTTAACAGTGCGCATTCCCTTGATGTCTACAATATCACCCTTCTTGTA
GATCCGCATGTATGTGGCCAAAGGAACAACGCCATGTTTCCTAAAAGGCCTAGAGAACATGTACCGGGTGCCTCTCCTCTTTCCCTT
TGTGTTCCGTCATTTTGGCGAGTTACTGGAAGATGGCTGCCCCAAGGCAACTCTTATACATAAAACATTTCATTGGGGCTGGCTTAC
AATTTCAGAGGTTCAGTGCCTTATCCTCATGGAGGGGAGCATGGCAGCATGCTGGCTGACATGGCACTGCAGGAGCCAAGAGTTCTG
CATCTTGATCCACAGGTAGCCAGGAAGAAACTATCATCCCCACTGGGTGGCCCTGAGCATATAGACCTTAAAGCCACGCCTCTAC
AGTGAAACATTTACTTCCACAAGGCCACACCTCCTAATAGTGCCATTTCTCATGGACCAAACACTCAAACATTAGTCTAAGGGGGCC
AAACCTATTCAAACCACTACGTTGGTGTTCTGTGGGCTGGGGTGGCTGGCTGAGTCAAATTTCTGCTTTCTACCAAGTGTAGAGACT
TCATGTGAGCAGAGTGGACTCTGTCTGAAGACAGACCCAGCGGGAAGATGTGTTGGTTTGGTGTTTGGGGTGTTTGCAGATGCAGAA
GACCATCAGGAATCTCCCTGTGTTGTAAAGTATTTCCATCTACTGCCTTTTAAAGGGGTTTCAGGCAGAAAACAGAGCCTAATGTTA
GTAAAATGGGAAAAAAGCTGCCTTAAAGTATCATTCAAGAAAATAGAGTGCACACTTAACGTTTTTGCATATATAAAGGGGTTATAAA
TCCTCTGGGATCTCCTAGTTGCTAATTTATTATCCCATGCTTACTCATTGATAAATTTTTCTTCTTGCAAAAAGATAGATGAATAAG
GGAGAATACCAATTTTCCACCTCATAATAAAACTAACTCATTAAATTATTGCGTGTGGGCTTTTGCTTTGTTTGTTTTCTTTTTCTT
TTTTCTTTTTTCTTTTTTTTTTTTCTTCTCATGTTCTGGTTGGAAGCTCAGGCCCAATACATACTAGACAACAATGAAACTTTTCCA
CCCCCTGGAAAGTTAGCTGAAGTATCCATCGTGAATATACAGTCCTATTTAGCCACTTTTATAGTTTAAATTATTCTTTAAGATTTC
CAAGTTTGGAAGGTTGGAAAGAGAGAGTCTGATGGCTTAGCCCCATCCCTGCCATTTTGCTACAAAAACTAAAAAGAGACCTTGACT
TTCTGTGGTCTCAGATTGTTCCATCTGCACGTGCGTGAGTGAGTGAGAGTGTGCGTGCGTGGGTAGGTGCGTGTGTGCGTGGGTTCG
GTGCAGGATAAACTCGGAAACATATCAATCAAGCCAGAAAGAAAGCAGAGCCCTGGGTAGGGTTCTGCCGTTCCACGCTAATGGGAA
ATTGGTGCTTTCTCTTTGCTCATTCTGTGTCATTCTTACTAAAGTGTGGGTATTTGCAGGGCTGTTTACCCTACTTTGCCCACGGCT
GCAGATAAATTATTATTTTGAATGTGTTGTCTCGGAATCAGAGAAATCCTGCTGCCTCACATTTGGACTGTGGCCCCTGCACAGTGT
CATGGACTTGACTGTGGCCCCTGCACAGTCTGATGGACTTGCCGCACTGGGCATCTCTGCTTTGCTGAGACTCTCTGACATATGCA
GGTGCCTGTGGATGTGGGAGACTGATGGAGTAGCCAGAAAAGCAGAATAGAGACCAGAGGAACCGCCAGCTGGAATGTGGAGTTGGT
AGAAGGAAGTTCTCAGAGCATTTTGATGCTGTAGATGTGTCAACCAGGAGTTGCAAAAGCGGCTCACCTATAAAGGGCCAGCAGACT
GAAACCCCCAGTGAGGGCAGAGGGTGTCTGCATGCAGTCCTTTCCAATGGCTGCTCGGAGCCTCTTTCCACTTCCTAACACTAATCT
TAGTTCTGAACTCTTGTTAAAGCAGAAGATTGGAAGCTGGGCGTGGTGGCGCATGCCTTTAATCCCAGCACTCGGAGGTAGAGGCAG
GCGGATTTCTGAGTTCCAGGCCAGCCTGGTCTACAAAGTGAGCTTCAGGACAGCCAGGGCTACACAGAGAAACCCTGTCTCGAAAAA
ACAAACAAACAAAAAAAAAAAAGCAGGAGATTGGGTAGCAGAAGGTTAGAGTACCCAACTATGAATTATAGAGAAAATGTTTTATTTTA
TTCCTTATCACTCTGCAGTTGAGCCTAGGGCCTCATGTATTGTAGGCAAGTGCTCTTTCTGTGAACTCCAGCCCAGCCCATTTGTTC
TCTTGGATTTGGGATGGGAAGTTCTTCAGGCGCATATACCTTTTATCTTTGTCAGCTTGCTTTTAACCGAAGTGGTTGAAAGTAAGT
ATCTGAGTGATTGACAAAATGGCCCAAGCCCAAACGATTCTAAGGGAGGGTTCTGTGCCCTCTTGTGGGTTTCAGCCAACTAAGTTT
TGATTCAATAAAATCCTCACCCATAGATGAGTGGCATATTATACTATAACAAACATCACTAGCTAGCATGGGTAGGACTGGGGAATC
TTTTGCCGTCATTCAACTTAAAAATTTTGGTTTAAAAATCAACTTAAAATTTTGGTCTGCTGTGGACATGTCACACTTGTGAGCATGCA
TACTTGAAGGTTTAAGCTTGAGTGTTTAATGACGAGATTTAAAAAGTGAAGAAATTTAATTTTTCTCATGTGTGGGTATTAATTTGT
ATGCATGCTTGCACACACATGGGCACTCGTGGGTGAATGTGGGTAAGTATGCCTCCATATGGACATGGATATAGGGCCTTAAGTTTG
CCGTTGGTATTTGCTTTCTGTAGTGTCCCTCAGTCGAACCCAGAGTTTGTCAGTATAGCTAGTCTTGCTAGCCAGCTTGTTCCCATG
GGCCTCTGCCTCTGCACTGGAATTACAGGAGTCACCATGCCCCACCCCCATTATGTGGTGCTAGGGGTTCGGCTCTGGTCCTCTTACT
TGTGTAGCAAGCAATTGAACTACTGAGTTCTCTCTCCAGACCCGGACTGAACCAACTTTGAGTTAGAAACCCTTTTATGTTCATCGTG
ACCTTGTTTGCGTCCCTTTACCTGCTCTGGTTGAGTTATCAGAAGTAACAGCAGCCCAAAAAAATCCATTGCTCTCTTACAGTCTCC
ATGAACGCCAGACATACACATTGTACCTAGACATATGTGCAGAAAATAAAAACATACACATTAAATAAATAAATAGTATTATTTAAA
TGAAGATCGGTCTGAAGGCTGTTGCAGGTCCTCTGTGTTTGCCGAGGACTTGTGCTGTCTTCATTGATTATCATATTCACAGTTTCT
GAAGAAATTACCCCAATATGCTGTAGCTCTTCCTTACAAGTTAAAGCCAGCCACCAAAGCACAGTGGGTGCTGAGCAGTGAAAAAGT
ATCTTCTCTGGAGTCTGAGTCCCGCATCCTTTTCGTCGACTGCACGTCCTTCAACCCTCACCTTCTCTTTTAGAACATTGAACAACT
CTAGTGTGAGACGCGTGCTTCCTGTGTCAGTAACTAAACACGACTTGGCAACTACGGTAGGTAGTACCAAGTACCACAGACCCTGCT
ACTCCCGCACAGATGCGGGTGATGTAGAAAAGGTATGCTTCTTCTGAGCGCTGAGAGCGGGGAGCCAGCATCCTGACTGTCCTGACC
TTTTCTAAAACTCTGTTGTGAAACTGCAGCTCTGCAGAGCCTCATGTTTGTATGATGGTTTGCATCATGTCTGCACACAGGTTTGTA
TCATGACACATACATTCATACTTACATACCGGAACATGCATCCCTGCCCCCTCACCCCCCCCACTGGGCGGAGGAAAAAAAATCCC
AGATAAAGTACCTTTTTTTTTTTTTGTCCTTTTTATGACATTTAGTTGGCATGAGTTACCGCCGTATAATCATCTGTCTAGTAGAACCTA
ACATGTTTCTTGGCTCGAAGTCCCTCTTTTGTCCTCTCCTCCTTTCTGCACTGTGTTCTTAGAGGGAAGTCACTGTGCACACATAGA
CTTAATGAATGGGAACTGCCCCCTAACCAACTTGGGGTTCTCAATATAATTACTTGGAATTCTGTTATATTTTATGTATGTGGCTTTA
GCAATAACTTTGATAGCAGAATGCTGCTGTAACTGTTGTAGTTATAAACTCACTGCGGTGGCCATTTTATTCTAATTTGAAGGGAAC
TAGTAGCAGCCAGACAGCCCTGAGCAGAGACCGTGGAAGCAGCAGAACTTCTACCAGGGATTGTCTTACTGTGCCAAACATGCCAGATGC
TGTCCACGCCTGTTCTGGGTCAAGACCCAGGTCTGTTTGTGTGCTCTCTTCTGCTTCAGCAGGCTCACTGGCATTTCTGGAGACCAC
AGCTTGTGTGTCCTATCTGCATTTCAGCGGCTATGTATCCTGTTTAGCAAATGCTAGTAGGAAAACATATGCCATTTTGACATGTTT
AGGTATTGCAGGGACATAGTTGTTAATGGGTATCTTTTCATATCCATTAATTGCCTCTGACTTGAAGTTACTCCTCATAAGAGTATT
AGGATATAATTAAAGATCTTCAAATGTAGTCAAATATTTAATTACTTCATATTCAGCACCGTGTGTGCAGTTCTTGGGTTGTTTTCC
TTTTTGTTTTCTTTTTTTGAAAATATTCCAATAATTTTTTTACAAATTTATTAATTAAGGATTATATTTCACTCACTTTTGGTGAGC
ATATATAGTCAGTGAGAATTAGGTATATTTTGAAAGCATAGAAGTCTACGTATTTTGCCACTGGCTTCAGCAGAGAATTAGTAGCGA
AGTCACGGTTTTGCCCTTGCTCAGTGTTTCATGGATGCGCTTCTAACCCTGCCGTCAGCCACTGAATATGAGTGGTGATTGTTCTCA
GCCTCAAGAAGAAACGGAGGTTAATAGAAGTAAATTTCCCAGTTTTAAGATTTTTCTCCTATCAATTTTCCTTAAACTCTTGGCATC
TGACCCTACATCCCTGGTGGTGAACATGACTAAAGCAAATGAGAAGTAATGTAAAAGTTGTTAGCAGAAGAATTCCAAGGCAGCAGG
TAATTCAATAACTCCCATTATCTTCTTGACAACACAAGTTTATACTGTGGGGGTTTGGGTTTGCCCTTTATATATTTTTCATATAC
TGTCATTTATGGGAAAGAATCTCTATTTAATAGACAGACACAGAACTATCGCCATGTTCCCATGTTCCTCGATCCTCCTCCAGGCTG
GCTTGGTATCCAGGTGACTCATGCTCTGAGCAAGGTTTGAACGCCGCCCCTGCTGAATGGAAGGCAGCTAGGAACCCCCGAGCTTCC
TGAAACAGGGAGGCCATCGTCCAGCGGTGATGTAATTGTCATCTGTACATCCTTCTGCGGGAAGGATGGGTTCACTTTTCCACCCAC
AGCGTTGGCACACAACTCCCCTGCGTCTCAGGCCAACACTGTGCAACAATTATGAGGTCCTAGAGCATTCCCGGCAAGGCCTGTGTG
TACATCTGTGTGCAGGACACACATGTGGCTGTAAATGTAGTGTGCTTGTGCCCAGTAAGTAAGTCACCAGTCTTACTTACTGTATGG
GAAGGATGAATTGATAGCATTTCTGTTTATGATAGAATTAATTAGAATAATTAATCACCGTCCTCCAAGGATGTGGTAGGTCTTTTC
TATTTAAAGGGGAGGATCGGTTTTCCAAGTTTTTTTGCTAGGTTTCTGTTTGTGTTTGTTTTGACAAGGTCTTACTCCATTGCCT
TCCAACCTCAGTTCCCAAATAGGTAGGACAGGACTTGTGTGCCACTGAGCATGGCGGATAAGGTGTCGTAAGTGGCCAGAAAAGGCT
TATTTGAGGCACTACGTGAAATGAACCTCAATTAGTGAGACTAGCCCGTTCTTCTAGGAGAAGATCAGTGGATGAGAAGTGGGAGAG
TGAAGGAATGAAGACTAGACCTGGATGACTACAGAGACACGGAGGTTGTGAGGCTGTGTAGGTAGCAGGGCAGAGGTCCAGGGACCC
ATGTGTTGTCTCCTCAGAAAAGACATGTGTGACATGTTTTCTCACACTGAAAAGACTCATGAAGCCAACACCTAGGTCAGGAGGCA
GCATTTCTGCTACCATGGGGGCCTTGCTGGATGCCACACCTGTTTCGGTCTTTGTAGGTCAAGTTCACTTACAATCTTGCCTCAGGG
AAAGTTCTGGAGCTTGCAGTCTGTTCCTTACAACCTCTGTGATCTAAAACGTAGTGCAGCATGCAGGGTATTTCCTGTGGTCTTTTC
CCCAATTCTCGCCTCAATCCGATGCCTGGAAGTACGTGGTAAATGTTTATTTGTTTCTATATGTGTTTGTCATGTGTTTTCTCTTGC
TTAGAAAGCAAGACTTGTCCTGTGTATCCTTCCTTCATGTCTAAGCCAAAACTTTTGTCCCATTTTCATGCATATTTAATACTCATT
```

```
TTTGACACAGTTTATATTTACTCTTTGATAACAGAATACTTCAGGATGTGTCGTTCATTAAATCCAGAATTTTTGTATAAAAAGTCTG
TAGGCACCTAACCAAATTTAAACTGTAAATAAACTAGAGGTTATACAGCAATTTCAGACAGCTTTGTATTTAAGATGGGACTTTTAA
AGGTAGTCCATAATGAATCTTTTTTTTTTTTTTATTTTTCAGAGAGGGTCTCATGTAATTTAAGCAGATCTCAAACTCACTGTATAG
CTGAAGATGACCTTAAACTTAAAAAAAAAAAAATTGTTGGGGCTGGAGAGATGGCTCAGTGGTTAAGCACACTGATGGCTCTTCTAGA
GGTCCTAAGTTCAAATCCCTGCAACTACATGGTGGTTCACAACCATCTATAATGGGATCCTATGCCCTCTTCTGGTATGTCTAAAGA
CAGTTAGTTACAGTGTATTCACATACATAAAATAAATAAGTCCTTTGAAAAATTGTTTGTGTGCTTGTGTGTTTGTGTGTTTGTGTG
CTTGTGTGTGTGTGTTTGTGTGCTTGTGTGTGTTTGTGATTGTGTGTGTATGCATGCATACACAGTGTATGTGTAGTAAGTATCAGAGG
ACAACTTGCAGGAGTCTGTTCTCTTCTTCCCAGAGATGAAACTCGGTTTGTCAGGCTGGCCACTGCAGCATCGTTGACATCCAGGCTT
GACTTTCAGATCCCTGCTGTTACCCTCTTGAGTACTGGGATGAGAAGTGTGTGGGTACCATGGCACCCACACAGTGCTGGGGACCAA
GCCCAGGGCTTTATGACTGCTAGGAAAGTGATCTTCTGGCTAAGCTGACTCCCCAGCCCTAGAGTGAACTTTGATGATGATGATGAT
GATGACGATGACGACCACGACGACGACCTGTACAGTGGTGTTTACCTGGGGGCAGCAACTCAGGTTGTGTAGCTGGTGTGGGAGAGA
ATATAAGCACTTTCCTGTCTGCTTGCTCCCTGTTGTTTCACACCCGGAACAAAGAGAGTGCAGCTAAGTCAGTAGGTTCAGTAGGCT
CATATTGCAAGCCCAATGAATTTCAAATAAAAAAAAAGTTACTAAAATGCTATATGCTTGGCTTGGAACCAGATACCTTAAGTCTTT
TTAGGGAGTTGGGGCAAGGTGCTTCTTTTTATAAGAGAATTTTACACTAGAAAAAAAATCAAGTGTAACTAGTGGTCTATTTTGAGA
CCAACCCTTTAGAAAACGCTTTAGGCGTTTTCTTTGGAAGATCGGATCTGATGTTGTCTTGCCTAAGCTGCTGTTGTTTATTTTGGC
CTTCTGAACTGATGATCGTCCTTAGAAGCAACTTGGGGCCGCCTCTGAAAATGCCAAGATGTGTATTTTGCCACTGAGACATTCCTC
CCATAAAAGCGTGTGAATAATTTTTTATGGAGCGAGTCCATTCTTTACGATGTCTTAAAGGGGGAAGGATATCATTTTCAATCTAGAT
CATTACCTTGTTTCTTATTTCTGAATGCTCATGCCCCCCCCCCCCCCACACACACACACATTATCTAGATCGTGTGATTTAGATGGACC
ATTTGGAAAAGCTCTCTCCCTGCCTTCTCAATTGTTTTTCTAATTTTTGAAATAGTGCCACCTAGGGATAACAGTTTTAAGCCACCT
AACATTTAAAATGCATTTTAGTGTTTTTGGCCTCACCTTTTAATTTTCTTGCTTCTTGGAGAGAAAGAGTTGACTCTAAAATGCTAT
TTTATCGGTTCAGCTTTTATTTAATTTACTCTAGTACGTGTAGATGGAAAATTAGGCGATTTAATACTCACAATGTTATCTGGCAGT
TATTTTACTTACTGTTCCGTTTTAAGATGTTAGTTCGTAAAATACAGCCTTGAACTTATCTTTCACATGGATGCTGAAAATGTGTA
GTGTTAATTAAATGGGCTCCGTTGTCGGTTAACCGGCATGTGGGTACATCAGGAAAGGTAGCAATGGACAGGTCACGGATCATTTTA
CATCCACCGCATACGTGAGAACGCCACATGCTCCAGCTGCTCTTGGGGAGGTGAGGAATCAAGAGAGCAGAGGTGACTCTGTTCTCG
GTGCATTCTGGGAGATGGAGGATACAGGCTCACATGCTAGAAATGCCGTATGCCGTGGTCTGTCTGAGTTCTAATGCTCTGTCCATT
CCTTGCCTTTGCTTTCTCCTGATTAGCTTCCTTCTTCGCCTCCCCTGGCATCCCGACAGTTTAACATCTCTAACAGGCAGGTGTAAT
ACTTGACTAAGAATCTCTGGGTTTGGGGAAAATCCTGGAACGCTTGTGAAAAAGCCAAGCTTATAAGCCTGCGTGGGGAGGTGGAGC
TATGCGCCTGTGTGGGGAGGTGGAGCTACGCAGAGTCCTGGTACTTGCCTGCTTAGCTTATTTGGAGAGCCCCAGGCCAGTGAGAGG
TATTGTCTCAACTAAGCACCCTGGAAGATGCCTTCTAAGAACTCCAGAGATGATCCTTTGCCTCTTGACCCATACAAACATGCATAC
CCACCCCTTACACACACAAACACACACACCACACGTGCACACACATACTTAAGCATTTGTACTTACAAGAATCTCAGGTTGGCCTCT
CACTGCCTTGGTATTCCCAAGCCGAGCCCTGTAGCACGGTAGGTAAGAGGCAGTGATCACTAGGTCTGGGTCGTAGAGCTGCCATTT
CATGCTGCATCTGAGCCGCCAGCATCATGCTGCCCATCAAGAGACAGGAAAAAGGGAAACCGACTTGTAGAACCTACCAAGCTTCAG
TGTCCAGAGAGAGGATCGACAAGGGATGAGAACTGATTCATTAAATTATTAGCTGGACCATTTAATATAGCATATATACTATATCAC
ACACATAGTTGTGTGTGTGTGTGTGTATATATATATATATACTTACATATGTATACGTGTGTGTATATATACATATGTATATATGTA
TATATACATATGTATATGTATGTATATATAGTATATATATACACATATATACATGTATGTGTATATATGTATATATGAAGTATATAT
GCCTCTTGGCACCATCCTATGCCAAATTCTAAATAATTTGGACTTTAAATACTTAACTGGTCATTTCTGAAGGGTAATAGACTTAAA
AATAGCTTTTTTTCCCTTTGTTTTGCTTTAGTATCATTGAGAGAAAATAACTTCATAAGTGGAAAGACATTTTAACCCCCATGAGTAT
GTTTAACATTGTTATACATGACACGGAGCAGGGCATACAGCGCAGTGAGGGAGCACCTGCCTGCTATCCATGAGGCCTTGCAATCCA
TCCTCCACACCATCTAACAAACAGCCACAAGTAAAACTGAAATGGGTTATGGATTAGAACCCCTTTTCTAAGGGGTTATGAAGGGTT
TCTGGCCACGTGTCTCTTAGGAATCCTACCAAGACCCTCTCACATGCTCACACCTGTGAGGACCTTTCCTGAGAGAGGTGCTGATCT
GTCATTACATGTACAAAGAGCCATCCCACAATGGTATCCAACCACCGATGGCTAATGCCTTTTGTCGCTAGAGAGGAAGTGGTTCC
CCTGTCTTCCGGGAGTTAAATAACTCTCAGGTGGGTGTGATGCCAGTTAGAAGAGATTGAGCCTGAGGATGTACTATTGGCTTTGT
TAATGAATTTGGTTCATACGAGATTAGAACGTAATGTATTATGGGATGAAGCTGTAATGAAATGGGCTTCCTTGGCCCTTCTAGTTTC
ACGGGCAATGGCTGGAAAATCACTGCCAGTTGATCTTGGCTCAGAGCTCGGACGCAGGCTCAGACCCACCTCTTGCTGTTTGGAAGA
GAAAGGAAACATGATAAAATATATGGCTTTACTCTCAGAAGCAATTTATGACGGCCATTTAATAAAAAATTTCAGGAGCTTCCTTTTC
TTCCGAGGAGGAGCCAGGGAGTCCTGGTGGGACCTTAAGCTTAAGGTCCCGTGTGAAGGCCCTCCACAGCCTCTAGCCTTCCCAA
CTTCCTGCTTGGCTACACAGGAAAAGATGGAGGGATGGGTGGGGTGGGGGGTGGGGGCTGAAGACCTTTAACTCCATCAAGGTTCCA
GTTCTGCTTATACATCTTGACCCCCCCCCCCCCCACTCTGAACTCAAGCCTTCCTGGTGAGTTCTGACTTCAGCTTTGTGGGTGTGGG
AGGGGGCTACTGAGAGCCCCACTCTTATACAAACTGACACGTGTCCAGCTTGTGTCCAGCCCACTCTGTCTCGTCCCGTGGTTCTTT
TTCAGCCTTGAGTGAAGCAGTATTTATCATTCAGGATCTCCATGATCAGCAGATACCAAGGACCTGGAAGGACCTGAAAGGACCTTT
GGTCTCTAATGTTACTTACAGCAAAGGGCCCTCAGGAAATTCTTAGGTGCGTGAGTTTAAGGCTTTCTCCTGGCAAGCGGGTTGCA
GTGTGGCTCAGTTCTGCCATTCCTCTTAACCTGGGAGGATGGTTTGTTCTGGCATCCACTGCCTGTGCCCGGTAACCATGGTTTAGAC
AGTTTTACAACCAAAATTCACACAGCTTCCTCTTCTGCCTTGTATCCTTCAAATGCTTCCTGACAATGACCTATAATGAAATATGCC
TTCTAGCATGGCACTCAGTAAGCATAGACATATGTACATTTCACACAGTACCCTTATAAGAAATATACATACCCATATTCTGAGAATA
TATCTCCTTCTGTGACTAGCTCATGTTTGCAACCTGCAGTGGGAAAACTGCCCCCAGGCACTCCCCCACCCCCCAGAGATGCTCTGT
ACACTGCGGTCTTTTAGGTCATTGTAGTAGAGGATGGCTTAGGGCTAAACGCTGAGCATGGCAGAGCCAAGTGTGTGGCCTTTCAGT
GGCAGCCGAATGTTCTGGAAGAGTGGCATCGAGTGTCCAAGAGCAAAAGTTCTGGCTGTCGTGTTCTGTTCCATCTGGGCCCTGACC
TTCCCATCTGCTGTGAGTCTCTCAACCTCCCTGAGTCACTTCCCCCTCATGTACAATGAAGGCATAAAACTTTTTACTCTCAAATGT
TGGGAGGATTGATATATCATCCCTATATTAATGCACAGATATTAACAGCATTTATAATACTAATAGTCAACATTTATTTAACGTCAT
GTATAGCTTTTCTAGGTTAGCAGACACTTGCTGTTATAATGATTAATGCTGTATCTTAATTTTAGACACATCTCTAGTTGGATGGCA
TTTGCAACCCATTAAGAGTAGGTGGCTTACGTGTAGGGATAATCCCCGTGTCCCAAAGCGTCAACTCTAATCTAATGTGATTAAATGG
AAACAATAAGTTGCACAAGGTATTTACAGTGTCTAAGCTGGTTGAAACTCCCTGTTACATTCTGCACACTGACGGACTTATAGACTAAC
AGAAATCTCTTAGTTCCATGATGGAAGATTCCGTGAGCATTTCATTTGCTATTGGCCTTATTCAAGTGCCTCGGTAATGTTAGAAAT
CTTTCTCATCATTTCTTTTGCTGCCTTTCTATGTTGGTTTCATTTCTAGGCAAGCTTGTCCCCAGGTAGTGCTAGAAACAGCTCTCA
GCTACTCAGTTTTACATTCTCCAAGCAGAAAAACAAAGCCCCAACATGCTAGTTTTAGCTTAGTCCTTTGATGGGCAGAGTGATCAG
CTAGATGAAGGCTACCCCTTGCTGGCCTTTTCAGACACTTGGCTAAGAGAGAACAGTGGCTGTTGGACACCATGCAGCATCAGCATATATCCCC
CCTGGGGGCTCAGCCCATACTCAGAAGCACGGAGTACAAATGGAGCGCACCACTCTGTGGGGATGTCCCAGCAGCACCCGTGGTGC
ATCAGGCATCTCCTTCAGTTAAGCTTAACTTTCTTCATAAGATAATCAGCTTTGAATAACCCCCAACTGGGCAACACTAACACCAGA
GACCTTATTGCAAACCACCCTCACTTTCATTCTTGATGTACACTGCGGTAGTGCCTGAAGATGTGTCATCCCTTCTTTAACTTCCAG
CCCATCTTTGTGTCGTCGTGATTTAGAAGTTATCCCTTTAATAGTGGTTGCTTTCATTATGAACCACAAAACTCAGAGGTGGACTAC
CCCACCTCCTTGGTTGCTCTCCCTTTCATTTTGTGATCCAGCTAGCCCCAGCACACGACGCAGCCCTGCTTTATGCAGTTTTCCTTTCATCCAC
GGGCTGAGCATAAGTGTAGTCTCTCATTTATTAGTGTCCCTTGCACCCATACATCACATATGTCTCTCTCTCTCTCTCTCTCTCTC
TCTCTCTCTCACACACACACACACACACACACACACACACACACACACACACACGGCAGTGAGTGCTTTGGGTTTAGTGGG
GCCTGCAGATGGTACAATATGGTTGACAGGGAATTTAAGAGAATTATGAGTCTAGAGCCAGAAAAGTTCTGACAACACCTTAGAGCC
TTTTATCTGCCATGTGAGATAATTAATTAATTAGATTAAATTTGGAACAAAGCAAGCATACCTTTATTCCTAAGACAATATTATGTT
GCATTATTTAAAAACTGTATAGAATTGATAAGTCAAATATTGCAATGTTTTATATATAAGCAAAGCTATGAATTCAGATAGTTTATGT
```

EP 2 204 376 A2

```
TATTTTGGGGAGATATTGAAAATTCTCTGAAGATATTAAAAATTTCAAGTCCTGGAAATATTATATGGTCAGGTTCACGCATGCATA
TACATGCATCACATAAGAAGTGCCAGGTGAGCCAGGCAGTGAGGGTGTATGCCTTTAATCCTAGCACTCAGGTGGCACGGGCAGGTG
GAGCTCTGAGTCCGAGCAGCTTGGCCTACAGAGCAAGTTTCAGGGTACCTAGAGCTATACAGAGAAACCCTGTCTTGAAAAAAATCA
ACCAGCCAGGGGGAAAAAATGCCAGGTGAGACACATGTGTCAGTTATGCTTTGTTTTCTTATGGGAGCTTGGGGGCCTAATAGTCAT
TCTATACAGCTAGAGTTGAAGTACAGTTGTTACACTGCAATTACGACAGGAAATGGGTGAGTTAAAGTGGTGAGGCCACATTTAAAA
TCAAAGCCATATGATACATGTTTATTTTAAAAGTTCCATGTTGATTAGCATTTGTTTTCCCTATTACTGTCCTGTAAATGGAGACC
TGGGTGTTCCTTCTCCCAGTGATCCGCCACTTGCTATAAAATTATAAGCACCAGGAGTGTTTGGATGTAGGTCTGTGTGGCCCCTGA
TTCAGTCAATGCTTCCTGTATAAGCTTTAGCACTAACTTATCCACCACAGTGTCCATTTACAAGTCTACAACGTGGGTCTAGACCTA
AGAAGCCTTTATAATAAGCTGAAGATTTTCCCTGCATTTTCCTCTAGTTCTTTATGGTTGACTCTCACACCTGATTCAACGGCAGAC
TGTTTTCATGGGATCTTTTATAGTCAACAAAAGCGGTAGTGGACCAACTTATTTGAGTGGGAAAGGTAGACCAGGCTGGCTGTGTTT
AGACATCAGTAACATCAAGGATTCCTCAGAAAGAATGGTGTTCAGTTTGCTGTTATATGTCCACATCTGCACTGTAGCATCTAAAGC
CCCGTTGACATGGTTTTCAGCCCAGGAATAAAGCAACATTAAGAGAAAGCCACCACTTTTTCACAGTTTAGCCCTTCCTTGACCTGCA
TCATGATTTGATTAGACGCGTCCTGGACAGGCTGTGTTGGACACTTGGTCCTCAGCTGGGGTGCTGTTTTGTAGGCCATGGTGCCA
TTGGGGGGGTAGGGCCTAGCTGGAGGAAGTAGACTGCTGGGTGCTGTATCTGTCTTCAGAATTGACTCCTGCTTCCTGGTGTACCCA
GCCCTGAGCTGCCTGCACTGCCAACAGGATTCCAGCTACTGTGGCTCTCTGCCTCAGTGGACAGCATCCCCTCAAACAAGGAGCCAA
GACAAATGCCCCACCCTCTCACTGGTTCTCTTAGATGCTTGTTACAGTGTGAAAAATGCAGCCCAGCTGGGAGGTCAAAGACTAAGT
CCACAGAGAATGAAGTTTATAAAGCCACTCCATCTGGTTTCTTGTTTGCAGACGTAACTCACGCTGTCCTAGCTAGAGAAAGCCGAG
AGAAGAATGTGAATATTGATATGTATTAGTTACCTATCACTGTCTTCCAGATCACCCAAACCCTTGCACTGTAAAGCAAACTTAAGG
TCTCACATGGTTTGAGAATGAGAAGTCAGGGAATGGCCTAGCGGGCTGGTCTCACAAAGCTGCAGCCAGGCTGTGGGCAGAGGCTCT
CTAGTCCTAGGGTGTGGGGAATCTGCCTTCAGAGTTGCTCTGAGAGATGCTGGCTGGACACTTTGTTCTTACACGTAGACCTCGCCC
AAGGGTTGGACAGCATGGCTTCCCTTAGTGCCGTGTGACAATGACCAAAAGAGACTTGATAAGCTCATTTCACATTGACTGTCCATC
CCATGTTCAGTGTTCTGCTGGCCACACACTTGGACTGAGTATGTCATCTTGGCATCGGGAAGTGGGGTGTTACTACCATACATCATC
ATTATCAGTGACCAGCAGTGCCTCTTTGTGAAGGTTGCCAGCCACAGTGTACAAATTATAATTTTGCAATGGCAACTTAGCTTTGTTC
CTAGCGTCCCCCCCTCTTCCTCCCAAAGTTTTGCAAACAGTAAAAGTCCACCAGGAGATCACAGTGATGGGAACACAGTGATAAGTG
GTAGGGGCCTCCAAGGAACTATTGACTGCTGTCCTCTGCTTGAGAACTGAGAAGCCTCTGATTGCCTTTGAAGTCTGTGGTGTACAA
AGAGTGCGGCCTAGGAAAGCGGAGAGTAGCGGCAGTCCAGTGAATGAGCGGATCTGGGTCCACCTTGGTTTTGCCTTCATCCGTCCT
TTGAGGGACCCCACACTCTGCCTCAGAGATGGCAGGGGGTGTTTGGGATTCAGTCCAGTGGAAACTTGTGAGAGGCTCATGACTCCT
TAACCTCTTCCTTTCTTGGATGGAGAGAGACTTTCTTTGTGTTTGTTTGTTTGTTTGGTTTGAGTTCTTTATATATGGTAGACACCA
ACCCTCTGTTCGAGCTGTAGCTGGTAAAGATTTTTATTTGCTCTCAGCACGCCGCCTCTTCGCCATCTATCACGATGGCATCCTTTG
CTGTCTAGAAGTTTTTTTAGTATCGTGAGGTCCCATTTGTCAGTTGTTGACCTTAATGCCTATGCTATTGGGATCTTCTTGAGAAAGC
CCCTTCTTATGCCCTTCTTGTGATCCTCCTGCCTCTGCCTCCTTCAGCAAATCCTACCGGCGTGCGCCACCACTACCGACTCTTATG
CCCTTCTTATCCTCTAAGCTAAAGACTATTTTCCACCTTCCTCCTGGAGACTCCAGTTTTACTTTGAGGTCCTTGATCC
GTTCAGAGCTGAACATTGGGCAGGTTGGTATTAGGAGCTAGCCTCATTCTTCCATGTGTAGCTATTCCGTTTGATCAGCACCATTGG
GTAAAGATGCTGTGTTTTTTCCCAAATTATATTTTTTGGCCAAGAAAACAAACAAACAAATAAACAAAAAACAATTACAAATTTGGG
CAAGAAGAAATACAAATTGCCGAGAAATATTCTTAAAGCATTCAGTGTTCTTAGCTATTAAAGAAATGTAAATTACAACTGGTGAGA
TTTCCTCTTAGTCAGAATGGCCAGAATCACATGTATGTAGAAAACAAACCCTGGAGCGGATGCGAAGGGCAGGAAGCATTTAACCGT
TGCTGATAGCAGTGCAAACTGGTTTGACCAATATGGAAATGAGTGCAGAGGTTCCTAAAGAAATTAGAAACAGATCTATCGTGTGAG
CCAACTGTAAACTCCTGGGCATATCCCCAAGGACTCTGTATCCTACCACAGAGATACAGATTCATCCATTCATTGCTGTTCTACTCA
CAGTAGCCTGAAAATGAAAAGAGCTTAGCTGTATAGTACCGATGGGTAGGGAAAATGTGGTACATAGCCACAATGGAATATTATATA
CCTGCTAAGGGGAATGAAATAAAGACAGGTAAGTGGACGAAGCTAGAAATGAACATTCTAAGAAAGATACCAGCCCCGGAATGTTCT
TTCTTACGGGTGAATGCTGACATTGAATCCTAAGATTCAATGTGTGTGCTTACACTGGAATACATAGAGGAATCATAAAACTAGTAA
GAGACGAAGAAGATTTCAGGGGAAGGGAGATAAAATGCAGGAACATGAATGGGGACAGGGGAATAATGAAGCAGGAGGGGTTAAATT
GGGTGGTGAATGGGAAGGCCAGGGTACGGGGCGTGGGGTGTGTGTGTGTGTGTGTGTGTGTGTGTGTGTGTGGAAGGAAGCCATCA
TCCATGACTTAACCCTAACCCTGAGTACAACAAACCTTTGAAAAAGCACATGGAGACCTGCTTTTGCAGTTGCCTCTTGTTGGCCAG
TAGGAGTCCTGTAGGTGTACCCAAACATTATTGTGCTCTTATCTTTGGTCATCCTCCAGGACTTGATGGTTAGAGTCTATTGCAGAA
GGCATCATACAAGTGAGTCATGTATGGAGAAATCAGGCTGGTACCCATTTGGAAACATCACGCCTACTAGTTACCTTTTATACTGCT
GGAAGATTCTGTACATACTACTGGGGAAGAAAATAATTAACCATGTTGCCCAGCTATGAACCCTGTGAGCTACATAGTAATAACTAG
CCTGGCAAGATATTCTCACTGATGCAGTAGGGATACCAGTGTTAGGGGTATAACCAACCACTTTCTGCTTTGATTAAAAGCCTGCTG
CACAAGATGGAACCCATACCTTGTATCATTAATTTGGCCAAAAATCTGTGGCCTGCTGGGTCATAGTCTCTAGGGAAAAAGCGTAAA
ACTGTTTATTCTGCTAAAAGGACATAGTTTTAAACTTCCACCTAAGCCCTTATCTTTATAAGCATACATCAGTGCATCTCTCAACCT
AACATTCAATGGGAAATTTATATCATACTTAGTGTTCCCAAGGCTCAGGAGTCATCTTGGGAAATGGATTTATTTTATAAGAGCAGT
ATTACTGGACATCTGTAGTGTAATAGTATTTACCAGACATGACAGGTCCTCTACACACATGATCACACAGCAGCTGTGACTGCATGC
ACCAGACTTGCAGAAGATGAAGCCAGCCAATATCCCAACATAGATGGGAGATGGGCTCATTAAGTCCCATCCACTGGCTGTGGATGA
CAATTGGCAGTGTGACTGCAGGTGGGAAGGAGAGTCATTTCTCTTCATGGATCTGACACCATGCTCCAGTAGATGTGCCCACTGCTG
TGCACCCAACAGGTGTCACTCTGTGGATGGGGTGAGTTCACAGCATGTGGAGTTGGGAGGGGGAAATAGTGGAGGGGTGGGGAAAGTC
GCAGATGTGAGGATAAAGTTGGATTTGCTCAAAACAGTTTCATGTATTTCAAGTTTCAAACAACGAAAAAATAAATTAGAG
GGGATAATACGCAGAACTTTGGTGTAGTGTTTGCTTAGCATGTGCAGAGTCCTGGATAGGTTCCCTAACGCTTCACCTACACCTACA
CATATACACGATATAGGTTTCTAAAATGCATTCCAAATTTGTATCTATTTTAGTATTTTTTTATTACACTACACGTAAGTATAGTG
CCCATGGAGGCCAGATTCTCTGGACTGGAATTACAGGAGGCTGTGAGCCACCTAATGTGGGTTCTGAAACCCAACTATGGATGCCT
GGCAGAGCAGCAAATGCTCTTTCTGCTGAGCTCCCTCTCCAACCCCAACCCCAACCCCATTCCCAATTTTAGGATAATTCTTTGAGG
GAAAATATTTATGTACTTCAAAAAAAAGAAAGTATTGAAAGTAGAACTTCCCCCTCCCCCTTTGTGAAGAGAGAAACATCTTGCTTT
TGCAAATGCTGTGTCCTGGGACAGGGTGGGAAGTCACAGTGACTATTCTACAGCCCCCTCCCTCGACTCTTAAGTCGTAGACAGTAG
AGGAAACTTACGGGTGAACAGTGACTGATGTCATTTACAGGTAGACGATGTTTTTTGTCAAACATCCTCCGAGCTTTTAAAAACAAA
CATATACTTTGGTGAATAATGGTAATAAAAAGGAAATTGTAATATGAAATAAATTTGAATGAATGCTCCTTTAGCTCATACAGCCAG
TTGATAAATATTTCACCTAAACTGATACAAAGTATATCAAAAGTATTAACTTTAACTTTGAAAGGAATTTCTGTGTTTTGCATTCAATAATA
TTTCGCCAAATAGCACACACATATCAGGCAATGGGGGTAATAAAACTAATTTTCACTAGGTGGCTCACCCTAGTTGATGATCCTGTC
TCTGCCATTGCCTGTGAGTCACTCACCAAGTAGATTCCAGACATCCTGACCCTGTCCTCTGCGCCTGCCTGTATCATGAACTCTCTT
TCAGACTAAGGAGGCAGCGTGATAAACTAGTGTCTGAGTGGCCTCTTGTTTTTTGGCCCTGCCTCTCTCAGCCAGAGAGACCCAAAG
ATTTCTCCAGCTCTAAAAAGATCCACTAATGATGGAGATAGAAGAACTGACAGGGGGACCAAAGAGGTACTGGTCCCTGTCCCTGGC
AGAGTGTTCAAGTCACTTACGTGTTTTCAGGGTGGAACCACTTTATAAGCATCCATTACAAAACCTTGTCTTTGAGGACTGGCTCCC
ATAGTCTGTTTCCCATGCTGATCTGGATGCTTCTCTTTGCTCATGAGTGGGGACCTGGAGTCTCTTGCTGGGGGAGGTAGAAGAGGGT
GCCACGACATGACAAAAGGTAGCATCTCGGATGGTGGTCTTCCTATCTCGGAGCACCGGACAGTGCCTCTCATAAGGACCACATCTT
CCAGCCCAGGGCACCGGAGGTACCCACAGAGGCCAGAAGATGATGTTGGGTTTTCTAAAGCTGGAACTGTGAGCCACATATCGTAGG
TACTTGGAAACGATCTCTGGACCGCTGGAGACCAACAAGTTTTTTAACAGCCACCCCTAAGCCCCATGTTCACGGATCTTCTCTAAG
```

151

EP 2 204 376 A2

```
CTAGAGAGGTTGAAGAGTCAGGTGATTTGGAGCTGGGTAGAATATAGGTTCTCTACTCACTGGATAGGCAGCTGGGATGGCACCTCA
TAAGCTCGCCGTCCCTAATCGGACAGTGGATGGAGTTTACCTCACTTTCAGGAAATATATTTTTCTTTTCTTCTGTGGCACTGGGGA
CCGAACCTGTAGCCTCATGCATGACAGGCAGGTACTCTGCCATTGAGCTATAATCTGGTTCTTTTACTTTTACCTTTTCTCAAGGCT
GGGGATTTGAACTTGGGACCTCATGCTTTCTAGGCAAGTGCTCTACCACTGAGCTACAGCCATAGACCTGTGTTTGAGACCAGTGAGA
TGATCAAGCCAGATGTTTAAGTAGACCAGGTCATGATAAATAAGACTAGTGCTATCTTCTTTCTTTTCCCCTCCTTCTCCCCTCCCC
CTCCCCCCTCCCCCTCTCCTCCCTTCATCCTTTCCTTACTATCTTTTTCATTTTTATTTCTAGGGAGTATATTGTTTTTGAACTACA
TGACTGCTTCCTGCAGTTGTTTTGCCTTTTAGAAATAGCTGTGATTGGTTTCAAATTTGAATCTGACCCTCTACTTTGAATGAGACA
GTCTCCAATTTCTTTGACCTTTGAGGGTGGTGGGTACCTGTGTTAATTGAACATATGGGGGTTCTCTAGTAGACAGGTAAAAATACTC
GTGTCTTTGGGATCTGGGGCCATGCTCATTATAAGTAAATATTTGGGTAGAGGGCAGCTCCGTATCTGTGTGTGTGCTCTCAGTGTG
TGTGTGTGTGTGTTTGTGTAGGGGGGGGGTGTAACATTGTAGCATGCTACACCAGTTAAGAATTGCAGTGGTCAGGAGGCTTCTATGA
TTTGGAACTTGGGTGGTAGAGTCAGGCTTTTGGGTCTGGGTCTTGCCGCTGGACTTTACCACTGTGTGACCTACCCCCCCCCATGAC
TGCTGCCAAGACGGAGAGAACTGAAGGTTGATTTGCAGCATGCTCACTCTTAAGTGGGTAGCATGCTTCCGTTTCCAGCAGAATGGC
GGACACCCTTGTAGGTAGGCATCGCAGAACTGACCATTAGTCGCCATGCATCTGGTACATAGACGAGGAGAAAGCTTCTTTCTCGTT
CAGACTGGATTACACCCACATCAGCCTGAATCTCACTACCGCTGCCATGCTCAGTCTTCAGATGAATCCAAGACTTGCCCTGTGACT
CCTTGTTTTAATTAGCTGGCTTGGGTTAAGTACAAGGGCTAATTGCTCCAGTCTCGGCCCCTGGGTTTGGTTGTTTTGTTCAATTTAC
TTATGTAAGCAGGCGTTCTCTATGCAAACATTTGTGGAAGTCACAATCCCAAAACAGAGTAAATACAGAAGCAGGCTGCACACTGCA
GCAGTGTTTTAGCGACAGTGAGAAAACATCATCTGTTCCTTCTCCTACTTGGGGCTAGCGTGAGCTCTGTTGGTTTGCTAATGTTCT
TTGCAGTTGATTGATGGATTCATCGGGGATTTTGTACCACTCCCTGCATCTGTGCATTTAAGGGCTATTTTATTGACATGTCCACAT
CGTGGCTCTCTTTCTTCTTTGTAAAGAGGTGCCCTGGGCTGGATAGCCGAGAATGTGGGGGATAGGAGAAACAGCTGCTTTTCTGGT
TGGTGGCAGAACTGGTACCCAGCTGGTGTCCTGGCTCCTCTGGCAGTTGGGTATGTGGAAGTGCTGCTTTGACTCTTTAAAGTGTTG
TTTTTGGTAGAAACAGGGGAAAACTGATTCCCTTTTGTTGCTCAGCAGCCTTACCTCCCTCATGCTATTCATCAGAAATAATGACTT
CCAATTTTTTTCTCCTTTAAATTAGATACACCATTGTTCCATCATCTATCAATATTTCCTTTACATTTTTTTTTTCTGTTGAGAGTGTT
GGCATGTTCCCAGCTATCCTGAGTGCTGGTGTCTGGATTATCAATAGCCCTGGCTTTTCCAGGCTCTGTGTGGCTTACACCCTGAGT
CCTCAGGTGTTCCTGGGGTGGGCAGGTTCAGACAGGATGCCCGAGGGTGAGAACAGAGGCTGCACTGAGAGAATCTGGGGAAGGCAG
TGGGTTCCCTCTTTCATCAGGATTTCTGCTTCAGGCTGCCTTTTGCATTTTCACCTATTTTTATATTCTCTTCAAAGCAGTTTTGAA
GTGATAACAGTCATTAACTTCTTTGCTGGCAAATGAACTGAGAGGGATTTAATGCAGCCAGTGAGAAATGAGAACCTCCCTTCAAA
CCTTTCTGGGGGAGTTGCTAAGAAAGAACAGTTGAACTCCCAAGTTTTTATGTAATTTTGGGGGCGGGGGCTGGGATTTGGGAAAGTA
TTAAATTGATCTCATATTCTGAAATTGCGTGATCAAAAGAAACTCAAGAAGAGCAGTCATGTGACGCCGTCAGGTAGCGTTGTCTAC
TTGCATTAGTCCTTTGAAGGTGCAAGATTAAAAATTCCTGAAGCCCCAGCTCTGATTACTCCCTAATTTTGTCCTTTTGGCTTTTCTG
AGCTTTGGATTCAGAGACATGGCCTCATTTTGTGGGCTGGTTGTTGGATCAGTCTCGCTGTCGTGGATGTAGTCAGTACTACCTTGA
AGGATGGAGCTTGCAATTAAGCAGAAGCAGCCCAGGGGTGAACCGCTAAGCACTGCAGGTGTCAATGAAGCATAGGGTGGAAGAGAT
GAAAAGCTAAAAGCCCCCCCATGACCTCTTTGCACACACCGATTATCCAATCTGAAGTCATTGGTGCCATTTTGCACATAAGGGCCGGG
ATTGCCCTTATGACCACGTCCTTTTCTAGCTAGCTAGGTAACTTCCCTTTTCATTTTTCAAGCCAAGGCCCCTCTGTGCTTCATTGT
AACAGTGAATGGGGAAACATCTTGAAGTAGTCCTTAATCTCCCCACACTCAGTAACATTGTATTCCATTTCATTAAGGATGAAAGCG
CTGGTCCTGACCTATTAAAGGGACAGCAGGGGGGTTGTGACCTGCTATTTGAAAAAGAGTGGTCCAGAGGCTCTCCATCCTCGGTTC
TCAGCATCCTCTGCCCTCCGTGGAGACTTGGCCCCTTCATGAAAACCTCCCTCCTTACTGACCATCTGGTGGCCTCCTCTGACCTTATCA
CTTTTCTCAATCTCATTTACAGCATTTACTGCAAATTGGCTCTTATGCTAATTACTGATTTCAACTTATCTCAGTGGCTTGATGGT
GAGTGCCTGGTCAGGGCATGAGCTCCGTGCTGACCTCATCCATCAACACACACACACACACACACACACACGCGCGCGCGCGCCC
CAGAGTGTCCTGCCACCTGAGCCCTTGCAAAGTGTGCAGTGCTTGGATGCGCCTCTAGCTCAGTGCTGACACTTGAAAGCCAGGAC
ATCACACTGCCACTGTTACAGCTCCAGGGGCATGGCTATCAGCTGGGCTGCCTTGATTTGTTACATATCTGAAAAAGAATGAGATTA
GGTTGGGGTTGCAGGTCTTTAAACCTTTGACCTTTGCAGGTAAACAGGTAATTACTTCAGCCTACAAGGCCTGCCTTTTACAGCATG
AAGTGACTTTTCATGTGTGTTTTGTGATGATTTGTGGATCCTTGACGGGATGGTATGCAGATGGGGCGTTTTACTTATTTATATTGGAG
CCATTTGTCTACAATGTGGAAACCTCACACAGAGGAGAAGGAATACAGATACCTTAATCATACGCCGTCTCTCACATCTGGAGCTCA
GTTTTGTGCTTCTAATGATGGCGTTCAGAGAAGAGCAGGACCCATTTTACGTTGACACAAATTATCTACCCAAAGGCTGACCTTATT
TAAAGGAAGTGATTCAATTTAGAAAGGTTGTCCCCATTCAATCTAATTTCAGAAACTTGACTGTTAAAACCTGTCCCTATACCATCT
TGACTAACAGATGTAGCCATAGCCCCATCCATCTTGGGGAAGTGCCTGATTAAAATATATACCATCCCTGGACTTCCCAGACTCTAT
ACATTTGAGAAAGAAATGTATGCTGTTTATAACGTTCTTAAAAACGGTCTGCTAGAAATACCTAGGAAGTCACTGCTGAAGAAAGAG
CTAGTCAGTGATGCGTACTTTTAGGGGCTGGGCAGTTTGCATTATTCACAGTGGATTTCAGTAGGACATTTTCATACATGAGTGTTT
CTTCATGTTCACTCTCTCTCTCCTACCCCAGCTACTCCTCTGAACTCTTCCCAACTGCCCCCTCCCAATAACTTTCATCTCTCTCCC
TTTCCTGGCAATCATCAACTATGCACAAACGGTTCGTGCTTCTTATAATGGCTTCTTTTCTGGGCTTGGTGGACATCTCAGCATATT
TAGCAAATCTGCTCATACATAGGCAGATTTCGCTTTTGACCATCAAACCCTCACGGAGACTTTGACTGGGTTTAGTTTAATTCTGC
ACTCAGCGAATTTGCAGACAACTTGCCTTATTTGGACTCCTTCCCACCCCGCCTGCCACTTACCCAAGCTAAAATGTATGTCAAAGG
AAGCGGGGCTCATCTTAAAGGAGATTTGGAGAAAATCTTCCTTCAATATTTAAAGTGCTTGACAAAGTCATGTTCACATGGAAGTTG
GTTTTAAGATCAGGAAGTTAAAATCGGGCGGCTTATTTACATTACTGTCAGAAAGCTGTTCTAATAGGATTGACATGGGGCACTAAT
TAATCACCCAGGGTCTCTCCATTCACTAATCATATTGCGTGAAACTTCCCTGCTGGCCGAGGCTCAAGGGCAAACCTTGGGCTACCC
GCCAGCTTATTTTTCATAGGCATTATGCTATGATTGAACAGGTAATTCTTGTCAGTGGTTTATTCTCCCCATGGC
GATCTTCTCTGGTTAAACAGCTTTAAATGAAATTTTATATTAAGTATGAAAAGAACTTTTCTTTCAGATGAGGGAAAGGGGATTAAA
AAAATCATCTTTGTAATCTTTTTCCCCCACAGTCTATACTCTTTCAGTTCTGGTAAAAATCTGCTAGATTAATTGAATAAGGCACAG
ATGACATGTTAGTTTCTTGCCATTACCTGAGAAGTGACCTTATATTAAGCCTAGGAAACGTTCCCTATTACATTTTAATTGCAGCAG
CAATGTGTGCTTATTAAGTATCTACTGCACACAAAACGTCTCAGCCACTTCTTCTTATGTCTGCCTTTGCAAATACAGTAACTGTAT
CAATGCGAACCCTAGTCTGTTAGGCTGCCTAGGGTCACATTTGGTAAATGCTGGAGCCAGGTTTGAACCAGAGCTTGTCCATTCAAG
TTCTCCCTTATCGGGCCATGTTACTGTTTATCATTTGTACAGTTCCCCCATACTGTCATTTTACCTTGGGACGGTCCAGTGGTCTTA
ATCTTCAATAATGAAATGTCTATTTCCACATTGACTCATTACATTCATCTGAGTCATTTCTGAGGCATGTTGGTTTGGGGTACATAA
TTTCATCTCTCACTTCACCCTCACGGTGCCTTATTCAGAGAACAAGAAGTTAGCGGCATCTTTGAACCAGAAATAAACATAGCTTAT
TCATCTCCCTCATGCTGTTCTTCATCGCTCTCAAGAAGAAGAAAAAAATCCTAATAGAAAAGGAAAATTCCAGAGTGTATTCACATG
GAGCGCTCCCAAGGCAGTGCCCGAGAGCCTCAGCTTCCCATCTTCAAGCTGCCTGTGCAATGGCGACAGCTTGTCCTGTATGCAA
ATGAATTCCCAAGGAGGCTGGGAAATAAACTCTTACATAAAAGAACATATAAATGGCTGACGTGTCAAAACCCATTCCTTAAGAGAGC
AGGACGGTACTTGGTGGCACAAGCGGGGTTTCCCCGTGAAGCTCGCTCTCCTTAGTATGCTGAGCTGATGTTCTTGGCTGAAAGAGA
TCCATCCCGTTTCTTTTTATAGTGAGCGTCATGAAAAGTTGGAAAGGGGGAAAAACTGTGCTGAACATACAGAATAAATAAGTAACT
GCTGCAGGCCAGGCAGGCTGCCTTTTGTTTTCAGACATCCACTCCTTCCCCCTTTGTCCATCAGGGATGCCTGGTGTATAGAGCGCT
TCTTATGGAGGTTGTTTGTCATCCACAACAAAGGGCTAATTGCATCTGTGGGCCTCCTTTCACATGATAAATCAATGGATCTAGATCTCC
TCTCCCTATCTTATGTTCATCCTCGTCCTTATTTTATTTATTTTAGGTTTTGATTGAATGTAAAGGATCAGAGAGTGACGCCCACCTA
GTAAACGCATGAAGGGAATTTTAAACGTGGGCACGGAAAGGAAGGCACCTGCTACCACTTTTCTTGTGATTGATTTGAACAGAATTA
TAACTTAGAATAGCTGTTGAAAAGACTCCGTGGTGTTCCTAGGCACTGATGCTCTTCTCTGAGGACCATTTCAAAAAGGCCCTCTGT
GAGGAAACTCAGTCCCTGTTAGAAGAGGGGACCCAAGGTGCTTTGAGGCTACTTGGGGTGCTCTATGTACATTCATTTCTGAAGTGTT
CTAATTTTATTCTAATTCTAATGAGAAGTGAGTCTGTGTTCAACATGGGTTTCTTTTTTGTAGCTTTGCCAGAACATACCATTGTGTA
```

152

EP 2 204 376 A2

```
TGTGTGAGGCATGTATGTGGCTTTACGTTTGAGTGTCAGAGTAAGCTAACTAGATGAAAATCTCTCCATCTACGGAGCTTATGCTAC
CATAAGTGCCCCCATTTTGTGTATTTGGAAACTAAGGAACAGGAGGTTGAATTACTTGACCAGGCTCACATGTTTGTGACTCAGACC
TAACTCAAGATAGAAAGACAGGGAAATATTTGGAGATCCCGACAAGGGAAAACAACACTTCTCCACCAGGAGTCTAGTTGATGCCAG
CCACCCAGCGGCATCAAAGCAAGAATTTCAGAGCTATTAATAACTGCCTAAGGCCTTCCTCCCTTCTCAGCTATAAACCTACAGTTT
GCTAGGAATTTCCCCAGGAACCCCGTTTACAGGGAAAGGACGGATACTAACTTACTACCCTTGTGTAACCCTAGTAACTTCTGGGTT
ATCATATTATAACCCAGCCAAGGCATCTCCAGAGCCTCCCTATAATCTCACATGTCAAAAGAATGAAGGGAGAAGAAGGCATCTTCT
TAGCAGTCTTTTGAGATGTAATTTGAGAGCCACCAAAATGGTATATATGGCTGATATCAAGACAATTGTGACTTTCCCTTGAAAAGC
TCAGCACACACACACACACACACACACACACACACACACATATATTTTTCTTCCTTTGTGACTCTTAATGAATCCCAGTCATGCACT
TGGGTCTGTCTTATTTTCCTGTGAGCTCCCCCCTTTCTTTTAATCTTTATTTTAGAGCCCATTTTAAAATAATCCAATTTTACTTTA
TGAACTGATAGTCCTGAAATTCTTTTCTGCCTTGAAGCCAAACTTGAGGCCCATGGAAAGAGTCTCCAGACCATGAGTATGGTGACG
TGGAACTCTGTCTCAATCCCCTTCACCACCGACAAACCTAGGCCCATTTACCTAAGGGTCCATAGCTGTCACCGTTCACCCTTCTGC
TTGTTGTCACAAAGTCCTCTGGCCTTATGGCTTCTCATAGTCTTCGCGCAGATTTTCCTGCTCCTTCCCGCTTTGGCTGGTGTCTTT
CTCACCAGGATGGCTGCAATGGCCTCCCAGCTAGCCTAACTACTAACAGCTTGGCCTCTTTCCCTTTGTTCTCTGTACTGTAGTGAA
TAAGTTGTCCAAGCTGCAAACCTAGCCCCCCCCCCCCCCCCGTCCTGCTTGGAGCTCTCACTTGGTCTCCCATGGCTTGGTGGTCCC
TGGGTGTACACACCACACTGGTGTGCTAACCATGCCCTAGCTTAACCCTCTGGCCTCTAGGTTCTCAAGCTTAAAACATCACCTGC
CTGGGATTGAAGAGATGGCTCAGCTGTTAACAGCACCTCTTGTTCTTACAGAAGACTCAGGTTTGCTTTCCAGTATCTTCCTAGTAC
CTTGTAACCCAGTTCTAGGTGCCTCCTTCTGTCCTTTCTGTCCTTCGCACACAAGGCACACATATGACCACGCTCGTACCTGTAGGCAAAGCACT
CATTCACATAAAATAAAAACCAACCTTTTTCTTTAAAAAAAGCCACGCATCTCGGTGACTGTGCAGGCAGGTTACGGAGAGAGCTGGC
TGCCTTCAGCCAGTTTTCATGGGGCGGGATGAGGGATGCCTGAGATGGTGCATTTCTGATAAGTCCCCTGGTGCAGAGCCATCCTGT
GAGGACCACTGCTGGCCTGAGCCTTGTCGCACGCACGCATACTTGCAGACCCCTCAAGGGACCCACCATGCCACACTTTGATCCTTC
TGCATCTACTGTTGCCTGTGCAGGAGTGAGGCTCCCTTTGTCTCCCCTTTCAAACACAGCTAGAATGTTGCTTCCTAGGAAGTTTCC
TCTGTCAGCACCCCCACCCCAATGCCTCTGTTTCTATGGCAACTTGTCTATGCCTCCAGGAAGACTCCATTCCCTGCACCTAAGTTG
TGATTTTATTTATGTGCCCCCATTAGCTCTCTAATAAAAGACATCATCTGTTGTTGTTTTTTTCCCTTTTGGGGGAGGGGCGGGCAA
GGAGGATGGCATTCAAGACAGGGTTTCCCTGTATAGCCTTGGCTGTTCTGGAACTGGCATTGTAGACCAGGTTGGCCTGGAACTCAG
AGATTCATCTGCCCCTGCCTCTAAGTGCTGGAATTAAAGGTGTGTGCCACCCCTGCCCGGCTTTCTCATTCTCCTTTCTATCCCTGG
CACCTAGCTCCGTGCCTGCTCAGTCCTGAACATATGAAACATGTTGTATGATTTAATCCTTCTTCCACCATTACAAAGAAAAATCCT
ATTTTGAATTGTATTTGTGTTAACTATTTGTTTTGTTTGTTTTAGCTCTTAAAACATGTCTGTGGTATTTTGCTTTTAAGTTAACAAG
ATCTTTGAGTACAAAAGCTGTGCATCTAAGAGTACTAAACTTCTAGACCGAGTTGGTGAGTGCTCTGCATCTTGCTGCTTAACTCAA
ACAGTGAGAGCCTCTTCCCACAGAGGTTCATGCTTGCCTACAGATAACTGACTTTTTACGCCTGTAGTCTGACATACTTCCAGTCCA
TTTCAGCCTTCAGTGCGCCCCTCTGGTGTGTTTTCAGCCTTGGCTTTGTCTAATGGCTGGGGGAGGGGTAAGCAATAGAGCAGCCCA
TCAGGGCAAGCCTTTTTTAAATTTTATTTATTTATTTTTTTAACTGTAAGACAAGCAAAGATTAGTGAGCTGTAAGCTTGCAACTTC
CACGTTTCTATCCAGAAGTGGATAGCTAATCCCTCATGTTCCTTTTTTATATGCTTGCATTGCCTGGGTAATTTCCAAGAATATGGA
TTTTATTGCTTGCGTAGCCGTCCCCCTCCCCATCTCAGATTACAAGCTCTTGCTCAAAATGTCAAGGTCCAAGTTCTAACAAAGAGC
TCTCTAGACCAGACTGCCTCAGACTGTGGGCATTTCTTTAAGGCCTTCTCCTGGAACAGTGTGCATTCAAAGGGAAAAGCCCTTTCA
TCGTCTCTGGGAAGATACTGCGGGCTCTTGGTGCTACGCAGTTCCAAAGAAGTCCCCCTCCTGGCCCCTTTTTCCTTTCCCAGGCTC
TGTGTGATGGACTGCTCCAGATCCTGTTCTTGGCTTCGAGCCCCAGCAGTCTGTGTCCGCAATCCCATAAACAAGCAGAGGACTAGC
CCTTCAGGTGGATGGTGGAGACTGAATTAAGTATTCTTGCCTCTCGTGCCCGGTCACAACAGCTGGCCCAACAGTTTAGAACATCTG
GGGTTTTCATTGAGCTGCCCTGAAAAGAGATCACAGCTTCGTACAAGTCAGAGGAAGCCCCCAGCACTGTGGTTGTAGGCTTCTCTG
AATGTTTTGCTCATGCAGCATGAGAATTATGTGGATCCCCTAAAACTGTGGGTGTAGGTATTTTATAGCCATTCATGTAAAGGAGCA
GAATAGATCTCAAACTAGAGTCCGTTAGTGTCTCTTAAATATATAACTATTTGGAGCACAGTTCTGGAAGGATGCTCAAGCCGGTGA
CAGAAGAAGGAGAATTAAACAGCACTCAGGGGCCGTCCGTCACCTTTGCTTTATCCATGCACGTGCTCAGTGATTAGTATAGGCCCT
GACTGTCCTACTGTCTGACGTGAGGTGTTGTCATTCTTTCCAGTCCTGTGTACACATGTAGGCAGGACAAGGCCATACCTGGTATGG
CAGGGAAGCTCTGAGGACAAGGGGGCCTGAGTTCATCTTCTACCAGGCGTTTTCTGACTCTGAAAGGGGAGCTGCCTGTTAAGGTGG
CCACTGTGGGAGGAGTTGGGGAGCAGGCAGGGTAATGTCTGAGGAGCGATGGGGGGCAGGGAATAATCTGCACAGACTCCTTGCTTA
CAGTTGGCTTTCATGTGGTTTCGTGGACCCCACAGTTTGCAGCAGTTAGTAACATCAGTCATTGGTTAGATCAGGCAAGGCCTGCAG
GCTCTGGGAAGCCAAAGAACCCTTCAGGGATTCCTGCCCCATCTGAGGACACAGTGTAGTCTTCTCAGTGGCCTGTGCAGCCTGACA
CAGCCTGGCTACTGTGAGCCTCTGAGCTGTGCTTCTCCGTTCCTGGCCACTTTGTGTCCCGCTTTGTCCGCCATCTGTCCGGGGCCTCA
GTCCAGATGTCTGGCTTCGGCTAGGGTGCTGTTCGGACATCCGCCAGTCAGCTCTTCCTTCCTTCATGGTGCCCAATGAACATGTAT
TTTTTTTCCCCTTAATATTTATTTTGTTTTTATTTATGTGTCTCTCTGTGTGTATGCTCCAAAGAGGATGTTTAATCCCCTGAACTAG
TGGGGCTTGAACTAGTGGTTGTGAGCTGCCCAACAGGGGGTACTCAGACCCCATGTCTAGTCCTCTGTAACAGCAGCAAACTCTTAA
CTAATGGAGTCATCAGATACTAATCCCTGTCCCTCTCGTGGGTTTGCTCTAGTCACTCTGCAGTACCTGCAGTTGCCTTCCACTTGG
CTTTCTCTCCTCACTCTGCCCTCCTCCCTGGCAGCAGCCCTGAGCAGCCCGCTCATCTGTGACTTTCTATTGCTTGTCTTTCCTTGC
GCTGCCCTGCTAGCGTGTCAGTGCTACAAAGCAGGCACTTGTCCGCTCAGTTCCCTGACTTGTCCGAAGCCCTCCAGTGGCTGCATT
GCCGGTTGTAGTTGCCCAGCAGGCTCTGCCTTTCCCGAGTTGTATTATGGTACTGTGCAGCACCCAGGGCTAGAGGTCCCTAGGCAAT
ATGTACTGGGGCCACAATTAGATGGCCTTGTGACTATGTAAAGGGAGAGACATTGGCCCTGGCCTCCATGGGAATTGTCCTGTTTAC
AGAGTCGAGTTTTGTCTGTCATGCCATCTTATCAAGACCATTCTGGACAGTTGTAAACTGGGACATCAGAGCCCAGTGGCAAGAGTA
GGATTGATTCTCTGTTGGTTGAATTTTCTGCCAGCTCATACTTTGGCAGGAATTGTACTCAGGGACTTTTTTTTCTCCTGAAATAACT
CCCCACCCTACCAAGACAGGTGTTGTATTAGTGGCTGTACATAGGATACCTGTATAAGCTGGCTACCCATCTGCCAGAGCTGCAGTG
TTGGCTCAGAAAAAAGGAGAGTGAGAACAAGTGAGCTTCTCTGCTGGCTCATTGGTCTGTCCAGGGAAGGCGGTAGGTGCGCCTCGC
CTGCCTGTTCCAGCGGAGGTTTACATTCTCAGAACGTTAGTCCGCTGTTGGTGAGGCTGTTGGTGAGGCTCGGCTTCCTGCAGTGCT
GGTCGGGAACTTTGTTTCAGTCTGGACAGATCGCAGGTGAAATTGCTACATCTTAAGCAAACAGGAGTAAGGAGTGCTCGTTTAGAA
GCCGCTTAAGAACTTGGATGGCTGGGCCCTGGTATGTAATTGCTTCTTTTTAGGAAATACAAGTAGCAGAGGTGACGAAATATATGA
TGATGGTAAAATAATTTAGTTTTCAAAAATACAAAACGTGAGATTTGCTTCCCAGAGAATTCAGTTAGCAGCCAAATACTCAAACAA
GAAAACATCCTTCAGAATTTCTGCTAGGTCCAGCCAGCCCTGAGAACAGCACCCAGAAGTCGATTCTGTCTGCAGAGCAGTAGGTT
TTATTTCCCCTGTACTGGGAAGTTGAAGACGGGGCCCAGTGTGTGCTTGGAAAGCACTAACCACTGAGCAGCAACCCAGACCCACAT
TGGAATGTTGAAGGGCCATAGTTTCCAGGTATAAGACCAATTACCCTTCACACCTCCATCCTGTCATTACCGGAAAACACTTCTCAG
TAGTTCTCAGTTCTGCCCACGTTTCCTCCATATCCTGGACACGATCAGCTCCTGCCTGCTCCTCCCTCATCTTCCTTTGGATCCCTG
TGGATCCAGACGTCTGCAACCCACATGGTGATTCTTCCTCCAGTGCCAGCCTGTCCCACCCACTCCCACCCACCACTCAGTCTCAC
AGATGGTCCCTGAGAATCCTCCTGTCACCAGGGTGATGAATGGGCGCCTTACAAGGTCCAAAAGTGCCGTGCCACACTTGCGTCTCT
GATTGCATGCCAGCCCCTCCCATCTGCTGTTGTTAGCCACACAGATTTGGTGACTTGTAATTCTCTCCTTACCACGGGGCAGTTTTA
TTCTTTCGTGACCTGAAAGTCCTCTGAACTGTAAGAGCTATGTCTGTTTTTTCTTATCTCCTTTTATCTGAGCAGTGTGTAAGGTCAA
GTATAAAGTTGGGACTCAAAGCGACTTGGAATACTGGAAAGGTGGCTTTTTGAAGTGTCCATTTCGTGCCCAGTTGGGATGGGACACT
GTTCCAGTCCAAAACAATGGAGTCTGCCAGGATGAAAATAACTGGATATGGAAGAAGAGCTTTTCCACTTGACAGTGGGGCAGATGC
ATCCTTCTGGAATCACCCTGTGACACCCGAGACTATTTTCCTTTCCAGTTTTGCCAAAGCATCAGTTGTGCTGGCTCTGGAATCCAG
GTCTAGCTTCCGTCACTGTACCACTCTTGGTTCCCATGGGGCCATTCTCAGTTCTGGCAGAAGAATGCACCTTGGATGCCTAGACAT
CAGAGGAGCAACTTAGAGTATTAGGGCAAGCACTTCCCAGTGTTTCCACTGTGAGCTGGTCTATATAGAGAGGGCGTTTTAGACATT
```

153

EP 2 204 376 A2

```
AGGGGACACACAGGATGAGCATGCTCAATCTGAAAAACCATAGGTTTGTAAGTACCGCTCTAATGGCTGAAAGGTTTTGGATTTTCA
TGCTAAAGCTGTTTAACTGTAGTAACCAGCAAAGTCTATGCAAATATACCCCAATTCAGTAAACTCTGAAATATGAAATACTCCGTC
CGGTTCCAAGCATTTTAGTAAGGGTAATATGCAGGGCTGGAGAGATGGCTCAGTGGTTAAGAGGGGCTGGAGAGATGGCTCAGTGGT
TAGGAGCTCTGGCTGCTCTTCCAGAGGACGTGGGTTCAGTTGCCATGTATACATCCATGTGGCAGTATATATCTATAACTCAGTTCC
AGAGGATTGGATGATCTCTTCTGGCTTGTATGGGCACTGCAAGCAAAATGCCCACACACATAAAAATAAGTAAAATATTTTAAAAAA
ATCTTTTAAAAGGGAATATTCAGTAGTACCTATTCCCTGAGTGACACAACTCAAGCACAGGCCTGTGCAGTGCTGGGAGAAACATTG
TTTCTAAATGTATTCTGCTATCTGTTAAATAAGAGTGATGTCAAGTATCTATCTACTGCATGGAATTGCTGTGGGATACTTCAGGTG
AACATTATTCTGCACAGATGCAATTTAATGCAGGCTTATATTTTCCAGGAGTTATGCTTTAAAAAAAAGATAAAATTAATTATAAGTT
GAAATTTGCCAAATAAAATCTTAACATTTCAAAGCAGAATAAATAACGAAGCACCAGCTAATGGAATGGCCTATATCCTTTTCATTC
CTCCTAGCTCTCGGAACCTGGCTGGTGCGCATGGAACAAGCGCTTGCCTTTGCACCTCAGTATGAGCTTGTTGCCTACCAGGTGATC
ACTGGCTACCACACTGTTAGTACTGGTTTTGGAATGGGGCCAGACATGGTGGCACATGCCTTTAGTTCCAGTACTTGGGAGGTAGAG
GCAGATAAACCTTTGTGAATTTAAGGCCAGCCTAAGTCTGCAAAGCAAGTTCCAGGCCAACCAGGAGATGAGACCTGGTGAGATATG
TCTCAAAACAACAAGTGTCCTTAGTAGGACCTTCTTGCTGGAGATGACTTTGCTTGCAGGACTCAGAAACCAAACTGGAACTGACCT
GGAATCGTCTTTACTCATTAATGCTTGGCTTTCATAGTGACCAAAGTACTAGCTGGGCTGCTGGGAGGAAAAGGCATCCATGAGCTT
ACCCAGTGGTGAGCCCTGCATGCTGCAGTACCAACCTTAGAGGGAAAACGTGCCAGCCAGAAGTCATGGCTTGTCAAAGGCCAGTGG
CTGACATGGTCGCAGGTCCTTGGAGGCAACGTGCTCCTGTTGTTCTGGGAAACGGACATGTTACCAGGTTTTCTTGTAAATATGTGT
TTTTATTCCACTAGGTTAGTGTTACTCTCAGTCTTGATCTTTTTGTATTAAGCGATGGTCAGTAAAAATTCTTATTTTTAGTCAGGC
GGTGGTGGTGCACACCTTTAATTCCAGCACTTGGGAGGCAGAGGCAGGTGGATTTCTGAGTTCCAGGACAGCCAGGGTTATACAGAG
AAACCCTGTCTTGAAAATCAAAAACAAAACAAACAAACAAAAAAAGATTCTTATTTTTTTTGAGAGAATGTTTTATTTATTTTTGAA
TTATTTATTCATTTTATGTATATGAGTACACTGTAGCTGTCTTCAGACACACTAGAAGAGGGCATCAGATCCCATTAGAGATGGTTG
TGAGCCACCACGTGGTTGCTGGGAATTGAACTCAGGACCTCTGAAAGAGTAGTCAGTGCTCTTAACCAATGAGCCATCTCTTCAGTC
CCAGTGGCAGCAGTGGTTATCTGCTTAACACGTACACAAGATCGTGCCAGTCTTATAATTACAGCAGGGGGGAGGGGAGGGGTGGGG
AAGGGGCTCATGATGATCCACCCCTACCTGAGGGCTGTTGGCTTAGGAGGAGTCAGTTTGTCTTTGGGAATGTGGCCCATCCTCAGG
CATACAGCACTAACTGGACTCACTGAGGTAATTGTTAGTGAAAGAAAGAGAGAGGACGTGGAGATGAGGGGGACATATGTTTGGGGA
GCCTGTGGGAGAAAGAGGGAGGTTCCTCATGGATACAGATCATTGTATAAATGTATGATAATTTAAAAATTTAGTATCTTGGGGCTG
AGAGTATAGCTTAGTGATAGACACCTGCTTAGCTGTTTAAGAGGCCCAGAACTGGCCCTAGTACCAAAACCAGAAATGAAAATAGCG
ACTTAGCTCATATTTAGCATCCAACTAAAGTATTGTTACTCATTTCAACCTCAACACTTTGATGGACAGACATTCGTGCCAACAT
TTGCATACACTGCTGTGTGGGAAGAACGTGGGGTTCGGTTTTGATTCTATCTCAGTAGAGTAACTGGTCACCATGACGAGTTCCCAG
CGTTCCTCTGCACCCCTCCCCACTGGGTTTCACAGTGAGGTCACTGTGGGGTTTCTGGGCATCTTTCCTTTGGGAAGGGGAAACTGC
TTCAAGTGCTGCGTGCCTGTCTCTGGGTTTTGGCTCTTCACTGTTAATTATTTGGAGCTGGAACAACTTGATTTTAAAACAAAACAT
ATGGGCTGAGAGCAGTCGGGAATACATTTGCTGCTGATGTAGTGCGGAAGCTATGCAGCAAGCCGAGGTCCCGCCCTGTTCTAGTTA
CTCGCATAGGTATGGAATGGTTCCCGTAAATTTCAAGTCATGCCCCTTTCTCCAAATTCTTAAACTCCCTTTCGGAAAGACACAAGT
CAAAGCAGGAAACACTGAAGCAGATTGTAAAGAAAATTGTGAATCTCTCCTCTCTCCCCCAACCCCTACTTTCCTCTTTCTCCTCTG
TGGAAAACCTTATCTTTTCAATTTTTTAAAATTCTGATTCTTAAAAGAAAAAAAAAAAAATTCCCAAAGTGCCAAGAGGAGAAAGG
AGAACTGTGTATATGTAAAGGGAAGAAAGCCCTCTCCACACCCCCAGCCAAATTTCTCACTTTCTCCCTGGTCAACACTAGAACGGA
GAATGTCCCTTTGACGATCAGCACTAAAGCGAAGGCGACATTTTGTCCCTTTCTTGAGATAGTCTCTTGTAGCCAAAGCTGTACTGG
AATTCCAGATCCTCTTGTGTCTTTGGGTTGAGGTTGGTAGGTAGGCACCCATCTCTTACTTCACCTTCTCTGCTTGCTGCCGTCAAGC
CATGCCGTGTCCTGGTCATGTCTCACAGAATTCTGGCTCAAATGCCATCCCTTTGCCATCCCGGGTTGGGGGTGGAAATTCATCAGT
GAGGACTCTTGCTTGCCCTTTATCAGATCTCCATTCCTCCTCATAGCACCTGTGAGTTCTACAGAAGAACCTACCCATAGTTATGTC
CCCCTTTGCTAAAAAGGAGGCCATCACAGTGCCAGTTTCAAAGTCAAACTCTACTGTGAGGCTCAGAGAAGACACTCCCTCCCACTC
AGGAAGCAGAGAGCCCGGCAAATATGGAAAGCAGAGTTGCAGGAGCGACTGGCAGGGACAGGCATGCACCCAGCCTGCTCAGCAGCA
GTGAGACAGGGACTTGACCATGATACCATCTGTTCCATCCCATCCACTGCCAACAGTGATACCGTGGCTGAACTCTGTGTCAGGAGC
CGTGACTGTTGTCCACAGAGAGCTTTAGCCTTAGAAGGTCGGACATTTCCCCCTGGGGTGCATTTGATTTTAGTTGCATGCAGCAAA
GCCTGAACTCTGCCCTATAGAGCGTCCCTGATCTGACAGAGTCCTGTGTATTCTCTTCTGTCTGTCAGCTATGACTGTATTTTGTCT
AGAACCTATAAATACAGTACTAATACGCCAGGTTGCTTGGTTCCTGGACCTTTTTCTTGTTTTATATCAGGAACATAGCACACTGAC
CAAGGAAGTAGAAAGGGCTGACCATAGTTTTTTCATAGGCATTGAAAACGAGAAATCTTTTTTTTTTTTTCCTCCAAGCAGATTTTCT
CTGTATAGCCCTGGCTGTCCTGGAACTCACTCTATAGACCAGACTGGCCTGGCCTCAAACTCAGAAATCCGCCTGCCTCTGTCTCCT
GAGTGCTGGGATTACAGGCTTGCGTCACCACGCCCAGCCCTCTTTTTCTTCTTTTAAAAAGATTTATTTATCTTACACTGTACACTG
TTGCTGTCAGACAGACCAGAAGATGGCATCAGATCCCATTACATATGGTTGTAAGCCACCATGTAGTTGCTGGGAATTGAACTCAGG
ACCTCTGGAAGAGCAGTCAGTGCTCTTAACCTCTGAGCCATCTCTCAGCCCCACAAGAAATCTTTCTAAGCACGTATGCTGGCAGGT
TTTATGTCAGCTTGATATAAGCTAGAGCCATTTGGGACTGGGGACTCTGGGACTCTGTGTCTACACGTTTGGTCTGTGGGCAAACTT
AGGCAGACAAGTCTGGGGAGGCCATTTCCTTAATTAATGATTGCTGTGTGGAGGGTCCAGCTCACTGTGGGTGGTGCCTGCCTGGGCA
GGTGATGGTCCTAGGTTGTATAAAGAGGAGCAGACAAAGCATCCCTCCTTGGCCTCTGCATCAGTTCCTGCCCTGAGTTCCTGCCCT
GTCTTCAGTGATGGGCTGTGATGTGGAAGTGGCAGGGAAATAAACCCTCACTCCCCGGGTTATTTTTCCTCACTGTGTTCTGTCACA
GCAATAGTAAACTAACCGTGACAGAACTTTTAAAGTAGCTCCCTTGGAGCAAGGGTAGGGAACATAGAGGCTTATTTTTTTTTCCTT
TCTCTCTCAAACTGAAGACATACTGAAGGTTTCTGTGGGTACTTAAAGTATTTTGTACTTTATCTTATTTTTTTTATTATTTTTGTTT
GTTTGTTTGGTTTTTGGTTTTTTGTTTTTTCGAGACAGGGTTTCTCTGTACAGCCCTGGCTGTCCTGGAACTCACTATGTAGACCAGG
CTGGCCTCGAATTCAGAAATCTGCCTGCCTCTGCCTCCCAAGTGCTGGGAATAAAGGTGTGCGCACCATGCCCGGCTTTATTTTAT
TATTATTATTATTATTATTGAGCTCTGGGAATTTTTATTCTTATAAATAGGACCATATTTCTGAGTGACTGATCTTGTACTGTCTCC
ATTTGGATTTCTTGTCCCCTGCCTCTAAAGTGTCAGAACTCCCTCCTATGAATGGCAAGATCTTGCTGAGTGGACAGCATCCTCAGA
CTCCCCTGAAGCTGGGGAGTAACCACCCCTCACTGCTGTGCTCTTCAGACACCCTCTCAGCAGAGAGGTCTTCTGACATTTAACTTA
ATCAAGATGTTAGTAAAATAAACAGTGTTCTTACACCGTCGGACGGAAACCGCTTGTCACCCCACCCCCACCCACCACCACTTCAGTGTT
TGGAAAGGAAACTCGGAGTCGCTCTGAGTCCAGTCACGCTCAGTCCTGGCCCTGGTTTCTTCCTGGGGTGGGCAGCACTGTCTGCTTG
CCACCCAATCTTCAGTGGCCCAGTTAGGAGTTTTCAGTACTGTCGGGGGGGCGGGGGGGATGATACCATGCCTTTTAAACCACATTT
CAGTTTCGGGGATGTTAAAATGTAAAGGGGGAAAAGTGTGTCTCAGAGCAGACAGGGTCTAGTGAGAACCAATTAAAGTTGGAAGCC
CATCCAGTCCCGTTGCTGTTTGCATCATGACCGCCCCTCCCCCCATCCTCAGTGCGGCTGACAGCGCCTCAAGGAAGTCTGCCTTCA
TTCACTGTGCATGTCATGTGTGTGCCAAGTTTTACATGGACGTGTGGACATGCTGTAGACTAGGAAGAGTAATGGGGCCCTCGGAA
CCACAGTTTTAGTGTCTTTAAAAAAAAAAAACAGTCATGAGGATTATTTGGGTCACGTGGATCACAGGGAGCATAGGATTCTCGCTGCC
TTTGAAAGAGTGACGAAGGGTTGGTCAGAGGACATGGGGCTCCTGTGTTCTCTCTCTTACCTGGTCCACTCGAAATGGTTCAGTGGG
ACAGAGATCGCTGCCTCACTAATGTGTGCCCTAAATGTTCACATCTAATGAACAGTGTCGTAGGTACTTGGACTCAGCCACTCCACT
CTGAATATTAGTTCATCTCATTCAAGAGTCTACCTAAAGATTATAAGTCACCATTTGAATGACTTTCAGGGTTCCTACCCCATCTCA
ATGTTACAATATTTTAAGCATGATTGGCAGTGGTAGTTCTTTTCCTCGTTAACGCAGATGTTAAAGAAAGCTTTTTTGGTTTTGTTTGG
TTTGTTTTTTTTTTTTTTTTTGTCTTGTTTTTCTGATCAAGTAATGTAATTAGACAACCAATTGAAACCATATTTTCGAGAGTAAAC
ACATGGTGCTTTCTCCCTAACGCTGACCCCGTCTCCTCTTCTCTTTCAGTTTTTGGGAATAACGTTTCTTGGAATCGGACTGTGGGC
GTGGAATGAAAAAGTAAGTGGAACTCCTCGGAGAGGAACAGGTTTGGTTCAAGGCCGTGTCTGGTCTCGAGTGTTTCCCCTTGCTCT
TTGATCAGCACAGGGGACAAACAAACCGTTGTCTGATGAGAGGAAGCATTCTTCACGACTGGGTGTCTCAAGTGCTCACGACATTAA
```

154

```
AATAATCAGCAGAGCCACAGTGTAGTGAGTGTGGACCGCGGAGGATGTGACTGGGGTTTGAGTCCAAGGCACAGCTCCACCCAGCCA
CGATGACCTCAGTCGGCTTATAAATGTCACCACGTGACCTGTAACCTTTCATCTCCCTCTCTCGTCTTTTGAATCCTTTTGGAAGAT
GACTTAAATGATAAAAAATGGCTTTTCAGTAGGCCGTGTGGTGGGATGGAAGCCAAATATAAACTTATGTGTAGATATCAGATAAAT
TCATCACCAATGCTGAGGGACATGAACAGGCAGCCACTGGTCATAAATATCAAGAAATGCTCTGAGTCCGTGGTCAGGGATTGCATT
GAAAAAGGAAAATCTAGGCATATCACGTTACATATTCCTAGTCTCAGTGATGACTGGGTTCTGGGCAGTTCCTGTCCTAAGCGGTGC
TCCCAGCTGCATGTTTTGATTTTTCTCTGAATTTCCCAGTTTACCACCGATATCCAAAAGTGGGAATCACCCCCTCTCTCCTCCCTG
TTAGGGGGTGAAATCACTCAAAGCATGTTTTGGCTACACAGTGCTCTTACCTTGTGAGAGTGAGCCCTGTTGGAATTTGGTCAGTGG
TCTGGCAAACCCAGGAAGGAGTTAGCTTATTCTTCAGGAAGGACTTTCTCTTAATCTCCTGAGTATTTGTCCTTCTGAGCCTGGCTT
TATTTCAAGGCAAAGGCTCTTTTGAAGTGTTAAATGTATTAAGAGCTGTTACCAGTAAATAGAAAGGAGACCTAACCAGGGCTTCAG
CCAAATTACAGACTTTGAGGTGGCAATGAGAGTTTGACAGTAGCGGCTCTTCCAGACAGACCCAGGAAACACACTCGGAGTGAAACC
TCAGAGCATGCGTGTGTTATGCAGTGGAAGAGCATTGTCTGGAGTCAGCCCCCAACACTGCATAAAAGGAGGACATGGGTTCCGTTG
GTAAAATGCTTGCCACATGAACATGTGGACCTCAGTCCATCCCCACACTGATGCAAAAAGCCAAGCGTGGCGGCACATATTTTTAAT
TCTATGCTGGAGAGATGGTGACAGGATCCCGGAGCCTGGCTAGTGAGCTTCAGGTACCCATGAGAGACCCTGTCTGAAAAACATGAA
CTGGATGGCAACTAAGAAATGACACCTGAGTAATCTCTGGGCGCCACATGCATGCACATGCATGTGAATGCAGACTTGCACAGCACA
CTGAATATGTGAATGCAAATGCACACCCCTCCGAACACACACACACTGCATAAATGTAATGATATATTAAAGAATAAGGAGAAAAAG
GCACCACTCCTTATATTTATGGAATGCTTCTATAATATATTGACTAGATTATGGAAAATGTACCATTGCCCAGCAAACAGTAACCTT
TTGAGACAAAGCCTTCCTTATACAAAGCAAGACAGCTTAGTTCCCTGAACAGATAGGTTCATCCCGCGAAACGTCTCTGTTGAATA
CCAATCTTCTCATGGATTGTGGTTAGCTGCTGCTTCATCCCAGTTAAGCCTTGGGATTTGTCCTGGTGGAATCTCAGAAATTCCCCT
TTTAGAGCTCTCTGTCTGGTTAAATGCCAGATAACACGGCTTCTTGCAACTCCTTTATTAAGAGTCCATTTCTGGCAGCTTTGACAG
AGATACCAGAGGGCATTAAATGAACTAGTTGTCTATTCTTGTAATAAACAGGGAGCTGGTCATTTTGAAGATTTTTTTTCCTCTTTA
TGAAATAGAATTACGTTTCTATTTCGTTGGTCTCCTCACATGTCTTTTCTGATGAAATTTAAAAAAAAAAAATTCCAACATTGGCAG
CAGCTCCCAAATACCCGGGCTGCCACCATGTCAGAAAAGGATGGCATCTCAGTTCCTTTATCAGGGCCCTGCCAGTAAGTGGGACGT
TTTTCTTGAGAACTGAAGTTACATGGCCCAAGTCATTCAGCAGAACTTGATGAGCAAGAGGAGGAGGGGCTAAAAGTATCCAGCCAT
ACAGTAGCCCAGCCCGTGGCCCACAGCTCATGGGTCTGGACCTCTGACACAGGGCTATAGCAAGGAGTGCTGCAGAGTCCCTGCCTC
AGCCGGCTTACATTCTAGGAGAGACACTTGCTGGAAGGAGGTCACTGGCCAGGAACTGCATCCAAACACCAGCAAGAAGCATTGAGT
ACTGGCTATTTAGAGAGTGGCAGGAGACCAGTGTCTAGCATTCTACAGTTTGATTGTTGTAGTGAGGACTGAACCCAGGGCTTTGTG
GCTGCTAGGCAAATGCTCTGTTACTGAGCCCTAACCCTGAGATGTCCCATTTTGGAGCTGAGAACCTTCCGGAGGAAGTGTCATTAGA
CTCTATGGAGTAAGAGAGATCTGCACAGGAACAGGCTGAGGACATTCCAGGAGGAGGATGGCGAGGACCTTGCTGACTCCGCCTGCC
TAACCGCAGACGCCCCTTGTCTGAGACCTTGGAGTCTTCAGCAGGCCAGATCTCTGGTTCAGAGTGAACCCTAGTCAGGTGGAGGGT
GGAAAGTGGCTGAATCTGCTGGGTTAACATCAGTGTTAGTCAGTAAACCCAGACAAGGCTGTGGCGTTTAGTTTGAAGGAGGAGGCA
AGGACTTTGAACTGCGTCAAAGCTGGAGTGATGTTAAATCCACGGTGGGGATCAGATTTGCTTAATGTGAAAAACTTGAAGCACACT
TAGGTGGAGCACAGGAGCAGTGGAGGCTAAAGTAACTACCCTTCAAAGTTAGTATCTCTCAGTCCGGGTTAATAGAGCAGGTGAATG
CTAGAAAGAATAAATTTTATGTAAATTCCAGTTGCCCTTGAAGATACAAAGGAATGGCTGCCTCTCCAGGGGAAGCTGGTCTGCATTTT
GTTTTCTGAGATCACAAGATCTGTTCCTTAGAAGGAAACTTAGCAGTTACATAGCGTGAATCAGGTCTCCAACACCCTTGGTGACCT
GTAGCCGTAGAGAACTCATTACTTCTGATAACATTATTGAGCACATAGCCTGTGACGGAGCCTGATACTGAATCTAGGAAGATGAAG
CAAAGAGTTAGAGTTCTCAGATGCAGGAAGGGTATGTGTATACTCACAAAAGACATGCATATGAGAATAACTGCATTCAATGGTGGT
CGGAGTGGTTGTGCATTGGAGAGATAAACACGAGAATAATTGTACTGCATAATGCTGGTTGGGGTAGTTGTGCATTTCAGAG
TTCCCACAGTGCCCTACCCTCCCCGTTTCTGTATTTGGGATACTTTTACTATTCAAATCACTATGGATTTTCACTCTGTGTTGGATC
TCTCCTCTTAGCTCTTGGGGATTTTTAGGATAGAGCTCTGCTTCCTTTGGGGTATGCAGGTAGAGCATCTGTAAGAAACACATAGCC
TCAGAGGACATTTTTCCCAAAGCTGTGGGAATTGCCAGGCCGGGGGCCACCATGGCTGTGTTCCCGCCTTCATTACTATGGAGGACG
CGATGGATAAAGAATACCTCACGCCCAACACCCTAGTCCCCGGTGGGCAACACCGTGGACCTGCCCATGGCCCTAGATAACTATGAC
AACCAGGCCATAGAGAGCACATATGTGTGTGCAGATGATAATGAAGCCCATGGAATAATGTGTGGAACAACCTAGTTGTAATCGGCT
GAATTTAGCTTAAAAAACTCCGGTCTGGCATGGCAAAATCACTCACCTCCTGAGTGCCCAGCGGAGAAACTTCCAGCCGAGAAATAAG
TCTGGTAGACCCCACGCAGACACGTTCATCTGCTGCTTAGAGAGCAAAGCCTACAGGACTCAGCTCAGGTCCAGAGGCTCCCAAGTA
AGGAACATGAGTCTCGCCTTACCTGCGCACTGCCACTGGGAGCTAAGGAAACACTTGCCAAACTGGAAAGGTTAGTGAGAGAGTGTT
TATGTTAACGAACGAATGTGGGCCCCTTGTTACCATTTATTTGCTTTTTGACATTTACGAGTAAATAAAAGTACTAAAACCTTTTCCA
CCAGGTTTTGAGGAATTTGTCTCTGCCACGCTTTACAGCCCAAATCGCGATAGCATCCTTTCTCTGGGTGGTCCTCCCATGCTATGG
GCTGGAACCCCACCACCATCCTCTTGGACATGCACCTCTGGCCTCCCTATAGCCTACCCTTTCCACCACTGAGGGGACAATTGAGGA
GCCATAGAAAGTATGCCTATGGCCTGGAGAGGTGGCTTAGCAGTTAACAGCACTTGCTATTCTTGCAGAAGGCCTGGGTTCCTTTTT
CCAGCACTTACAAGGAAGTTCACGTCTTTCTGTAACTACAGTTCCAGGAGATCGTATGCAGGCACTTGATATACATGGTGCAATACA
TACATACATGCACAGGCATAAAATATAAATAAATAAACCTTTAACATTTAAAAATATGTATATATTTAATATTAATTTTATATAAGA
ATATATTTAATATAACTATATTATATATACATACAATATATAAAAGGAAATATGTACTATGTTTGGGTATCCATGGAGTTTG
AGATCCACTGTCCATCCCTGAGTCCACAGCTTGCTGATAGTTCCAGCTTGTTATCAGCCCCAGCTGCCTTACCACGTAACCCACACA
TCTATCTAGGTTTGGGGCTCAGTGGACAATTCTAGGCATGCCTACCATCACATCCACCTTTTCTACTTCTAGAGCCCAAACCGTTCC
TCTAGAAGGACACCCTTCCGCTCAGTGTGGATTCGGCCACACACCTGGTCAGTGTCCTTCACTTCCTCCTCCTCCTCCTCACTC
AAGCCTTCCTCCTTCAGCCTTCCTTCTCCCTCTGTGAATATGAAGCTGTAACTTGAAAAGGGCTGGAGTGGTCCCGTGAACACTGGC
TGCACAGGCCTCAATCGATCGTTCCAGTGGCTGTCACTAATGAAGGCCACTGGAGGAGTGTTGATTGGGTGAAGGACGCAAGCTCAAG
TCACACCGTTAGAATTCTCCTTTGAGATCGACAGGCACTCGTTCAGGTGATGTGTACCCTGGGGTCGGGCATCCTCTCCGTCATCGG
CAGCATCCTTTCCTTTGTCATCCCTCCATGTCTTTGCGAGCCCGGGAGCTTAGTTGGACCTTCTGTTTCCTTACTGAGCCCTTGCTA
GGTGTCCCCACATAGTTCAATCTCATGCACAAACCCAGTGTGGTTTCTCATCCATGGGGCTCTCAGAAACCCATCCCAGCTCCGGGT
TGCAGTGCCTCACTCTCTCTTCTGCTCACTTGATCCTAGGAACTCATACTCTGCTGCTTTTCTTCCCTCATAGACCTTGCCAATTCCA
CCTCTGCCCTGAGACCCACCACTGCAGGTATCTGCAGAGCCCAGAAGAGGCCCTTGGATGCCCTAGAAATGGAGTATGGATTAGCTGC
TTGATGTGGGTGCCGAGAACTGTACCCAGGTCCTCTGCAGAACAGTCTGTGCTCTTTACTGCCAAGGCCTCTCTCCTGTCCCTGAAT
GGCACCTGTTAGTGTGATGATCGAGAAATGCTCTCCAGGGGCTGGAGATGTAGCTCGGGTGGCATAGTGGTTCCCTGGGGTTCTCAA
GTCCCTGGGGGCCATCTCTGGTACATAATCCTGATATGGTAATCTATACCTTTATTCCTATCACTCTGGAGGTGGATTTGGGAGGGC
CATGAGTTAAAGGCAGTTCTTGGCTTTGTAGTTGAGGCTACATGAAATCCCATCTCAAAGAAAGAAAAAGTGGGAAATGGGGAAACA
AGAAAGAAAACATGTTGTTTACACAGGGCATCACAGGGTGGTGGCTGCCTGTAATTCCAGCACCTGGCAGGGTAGTGTAAGAAAGA
TGGTTATAAATTTGAGGTCATCTGAGGCCACATAACAAGACCCAGTATTAAATGCAGTGATAAGAAATATGATCTCTGAACCATTCA
TCAGTGAGGAGATGAACATTATTTTAAGCCCTGGCTATTCCATGGCCTCTCTCATTGAGCAACAGAAGTCAGTAGTTCCCTAAGATT
TCTCGTGAATGGACTACTTTCACATTTCTTACTACATAAAAGGAGGTAAATTACACCTGGTTTGTTTCTGATATCTGGTATGAGTGA
CACAGCCTTTGAAATAGCAGGCTTGGAAGTTCTGGGAAATGGCCTCTCGTTTCTCTTGTTAAGAAGGGGACTCCGTATGATACCTTG
TTTTGTTAGAGAATTAGGTGTTTTCCCTCGCCTGCTTTTTTCTGCTCATTATTACTTAGTTCCCGGCAGGCAGGGCAAG
GGGAGGAGTGGCCTCCCTAACTGTTGAAAGGGAGAGTTTTATGGCTGTTAACAGGGGATGAGAGGACTCCCACTTCCCAGAATAGGAA
AGAGCCCTTCCTTGATCCTGCCTGTGCCTTTTGGATTTCCAGTCTCCCCTGCCTTCTCTAACACAGCCTCTGCTACTAGGCAGACAA
CTTCTGGGGCCGTGCTTGTTATTCAGTAGGGAATACCAAGGGGACAGGACTTACAGCTCAGTGGCCATGTGACCTAGATAGACATCC
CTACAGGGGAAGGGAGGTTTCTCCCTTCAGTGATTTGGCAGGTTGACCTCTCTCACTTTCTGTCTGTTTCCCCACCTGGAGTACCCA
```

EP 2 204 376 A2

```
GGTTATAGGTTTGTCATGAGAGTAAAGTAATGCAGGTCTCAGCAGGGTGGCTCTCAGGACTGGGCCTTCTCCACAACTGGCCCACCA
GCTGCGTCCGTGGTGCCTAGCCACTGCCTACATGGGAGCTTCCTGGTACAGAAGTGCCTGGGCTCTCCTCTCCTGAGCATCCGCAGGG
CAGGTCCAGTCCCATCCAGACACCCTATTCTGTCTTCGTCATTTGTCACATCCAGAGACCCTGTTGACCAAGTTGTTTCTGCGTCTC
AAGTCTGCTGACATGGCCTTCCATCTCCGAGCACATGGCAGCACTCTTGGATAGCTGCCCATAGCCTCTCCCTGTGGACAGGCTGTA
GGCTGTCACTGGATCTTACTCTAAATGGCCAAGACTGATGGAGTCCTTGGCATCTCAACTTCAGACTGCAGACTAAGACAAGATACT
ACACTGTGGACTAAATGACGAATGATAGCCCTTGGTCCCTCACAGCCCTGAGAAGGATGGCCGTCTACATCAAGGCGCTGGCTGGTT
CCATTCCAAGAGGAAGACTGTCTGTCGTCCTAGTCTGCAGATGGGTGAATCCGCACTGCATCTGCACAGGGCAGGATGAGAGAGGCCA
GGCTCTGCTCTCTTCCTCTTCTTACAAGGACCCCTGTCAGGGCATTGGGGTTCTAGTCTCATGGCCTTCTCCTAGCCTAATATCCTT
CAAGATATCATCTTCAACAATTGGTGGTGATTATGTTTCAACATACTAATGGGCTGGAGAGGCGTTCAGTCCTAAACAGAGCTTCTA
TTGCTTTTTCTGTTTAAATATTCTGTTTAAGACTAGAATGTTCCTAAGTATATTTTCTGCCTTCTAAATAGATACATGAAAGAATCC
AGCAAGCGGCAAGGAACACAGGGACCTTAGCATGTGGGGACACTTCTGTGTGGAGCTGTGTCTGCGGGACAGTCACTCACTCGTGGG
GGCGGGGACTGTGTCTTCCCTACCTGTGAGCAATGCCAGCCTGTGGAACTTGGCCCAGCTGTCCTCCCAACAGCTGCTTGACTGCTA
GATTCTGTATAGGCTTAAAAGATGATCGTAACCCTCTTCATCTCTTCAAAGATACCTCATGACGATTTTTTTTTTTTAATTTTGCAA
GAAAGGATTACTTGAGTCACTCTGATGAACTTGCTTGACAAGGTAAAATGGAAGGGATCTTGTGCCTTTGAAGCAGTTGGCTTTCCT
GCTTGTGAGCTTTTCTACTAGGTGTGAGCTAGGCTGCTCTCGCCCCATCACATCTCCTGACACGCTCTCCTGGCACTCTTCATCCCC
TACCCCCTCTGGCCTCTGCGCAGTTCATCCATCCCGTAGATGGAGACTTGGGTTTTTACAAAATGACTGCCATGGCACATTCTTGGA
GGGCCTTTGATGTGTTTCCATAGTCCCCTGCCTGCCTTGCGGACATCCTTCAGAAACACATTTCCAATTCTCCAGGACAACCC
CCTCTATGGGGAGCTGCATGTTCTTTGAAATGTCGCACCCGCCATTGTCAGCAATGGAAATGGCTCCTTCCCTCGTATCCTGGGGAG
GTTTTCAGGCCCACCATTACGGTAAGGCCTGGTGTGTGGGGCATAGGTGAATCAGGACCCAGTGTCAGAAATATCTCAGCGGAATTC
AACTGGTCTGGGTTTGTACACATTTCATATGCCTGCCTCTCTGTTTCTCGTCCAGCCGTTGGAAGGAATCTGCTTGTTTATGAAAAG
ACAGGAGAGCCCCAGACCCCGGGGTGCGTTCCTGCTATTTTTTTGCAGTTCTTCTCTGTGTGGGTTGACAGAAGAAACTGCAGCCTG
CCAGGTGGCACACAGAGGCTGCTCTGTGCTGGCGCTGAGGAAAACCACAGACACTCCTGAAGTGTATTGGCTTGGAACACACCGCAG
CTTTGTTTGCCTTTTTTTGTTTCTGGCGCTTGCAAAAAGCAACTTCTTTGAAGAAGAAATTGTTACCCAGAACAGACTCCTCTCTTG
CGGGAACCGTGAAGAAGATGCTGCTTCAGCAGCAGAGATGGCTGGGCGGATGAGGTGCTGGTGACGCGGTGGGGACGAAGGTTGGCG
GATGTTTCCACCCTCTTACCGGAGCTTATGCGGACAGACTGGAGTTGAGCAACCATAAGAAGGTCACTGCAGAGTTCTGAGTCCAAC
CGCCACCATGTGCTGTCAGCTCCCTGGCTGCAGAAGGATCCAGCTGCTGCTTGGGCAGGGTCCCCTTGAGGCTGTGACTTCTCCGTC
TTCTTCCTCCCGCTGTGCCGCTTCTGGGTACCCATAGAAGCTTTTACCTAGTCCTGCTTTTGCTCCCTCCTGGTTCTCCCCTCTGCC
CTCCTCTGTACAAGCTTCTATTATTTTTTACCCCCTACCCTGTCCCGCAAAGATCCAATTAGCGAGCTCCAGATACTTTTGCCTCACT
TTGTGAAATCAACAGAGTGGCCTCAGATTTCCTTAGGCACCAGGCAGGGGAAGTACTTGGAGCAAGAGATTCCTCTCTGGTAGCGCG
TGCGTGCGTGCGTATGTGTATACGTGCGAGTGAGAGCGAGTGTCTTAGGGTTTCTTTTGCTGAGACAAAATAGCATGACCAAAAAGC
AAGTTGGGCAGGAAAGGGTTTATTCAGCTTCCACTTCCACATTGCTACTTATCACCAGAGGAAGTCAGGGCAGGAACTGAAGCAGGA
TCTTGGAAACAGGAGCTGATACAGAGGCCATGGAGGTGCACCGCTTACTGGCTTCCTGCTTCCTTGCTTCCTTGCTGTGGGCTTGATC
ACCCTGCTTTCTTACAGAACCCAGGACCGCTAGCCCAGGGGATGCCAACTCCCACGTTGATCACTAAAGGAGGGAAATGCCTTACAGC
TGGATCTCCTGGAGTCATTTCCTCAGCTGGCTAACTGGGCTCCTTCCTCTCTGCTCACTCCAGCTTGTGTCGAGTTGACACACAAAC
CCAGCCAGAGCATCCTTCCTCTCAGATGTACCCGTGGATGTGGTGGCATCCCCTTGTCCTAACGTAATAGAGATGTGGATGATAGAG
CTAAGCTGTCACTGGATTCTTTTCTGTGCCAGGTATTTCGCGCAGAGTGTTTGCATTTACTTGATCTATCTCGTTCTCATAATCACC
TTATGGCATATGCTATAATTTCACGGATGTGTAAATTGAGGCTTGAGGCAGGTGAGGCGGCTTGCCTACAATCACATAGGCAGTAAGC
AGAGAAACTGAGATGCAAAACAATTTTGCCAGATGAAAGATAATAACAAGTGCTAATGAAGGTGTGGAAAGTGAACCCTTGCACAGT
GTTGGTAAAAATATAAATTAGAAGAGCCGTTATGAAGAAAGGAATAGAGGTCCCTCAAAAAATGAATTAATCTATAAAGCTAGGAGC
CATGTGAAATAGTATATAGAGATAATCCAAACATCTAAGAGGCTACAACAGGAGGATCACACTGAGTTCAAGCCCTGCCTGGATTGC
ACGTTGACTTTGAGGCCAACCTGAACTACATACTGACATTGCTTCAAATGAAGAATTAAAAACAGAATTGCCGTGTCTGGGGAGGGA
GGTGGGTCAGAGGGCAAAGTGGTTACTGTGCACCGGCAAGGACCTAAATCCAGATCCCCAGCGTCCGTTAAGATCAGGACAAGATGG
CACACACTTTTAAGTCTGGCACTGGGAGGTCACAAGATGCCAGGATCTCACTAGCAGTAGAGCCAAAATGGTAAACTCCAGGTTCA
GGAAAGGACTCTGTCTCTAAAAACAAACAAACAAAAATGATGGAGAAGCAGTTGACAAAAAAAAAAAAAAAAGTCTAGTGTCAACGC
CTGTCTTCTACATACTTCCCACACATCAGGTTGGCACACATACATGCATACGTATACATAGCTCTGAAGAAAAAAAAAAAGACAAAA
TAACAAAAGCTGGTTGTCCAGTTTCTGGAGTGGCGATTGTTGAAAACACTTGTTGCTTTCACGTTTGTAATGGACTGAGCTGTATC
TCGTGTTTAGAGCAGCGCTTTATGTTATGAGTGATTCGGCTTTCTCTTGCTTGCTTGGTGGGACAGCCGTAATCTGAGCTGGATTGCA
GCATGAATATGAGGCAAGTTTAGGAATGTGTGCTAATTTACATCAGCTCCTATTATTATAATCTTAATATTCTTAATCGCACACACA
CACACACACACACACACACACCACCATTATTTTCCCTTATTCTCATCCCTTCTCTTTCTGCATCTGCTTCCCCCCGACCCCCC
AAGGGGGTCAGAATCTCCCTTCCTTGTCACCCTAAGCACAGGTGACCAGTCACTCCACTCTCCCTCCACCTAGACTCAGCATCTCAG
AGTGGCATTTTCTGACTTCTGCCAAGGTCAACATCATTGGCCTCACTACCCCCTCCATGCCTGGAGCATACAGGTAGGAGAAGGATA
AAAACTTGTCACATTCCAGTGCTAGGCACATGTGTGTGCTGTGCTCTGGCTCATGTCAGCTCTGGCTATTGGAGGGACAGTCGCCAGAGGACAGG
TTTGTGCTCGTTTTTAAAAAAATAAGTTTCAAGATTTAAAAGTTAGTGTTTAAACTCCAAAGCTAAGATAGTCAACTTTACGTGCAGA
ATTAGGACGTAAGCCGTGGTAGTATTGCTGGCCAGTGATGTCGCTACCAGAGTCCCCAGGTTCCATCCAGACTGCTCATCTCTTTGC
TAATGCTGAGTTGAAAGCCAGTTCTCATGAATGAGCCAGGACCAGCCTTAATTGCTGCTGACATGGGTCACCAGCTAGCCTGGAGAA
GGCATCAGTTGAGACGTAGTACTTCTTTCACTCTTCTTCTCTGATGTCCCTAAACAGCCCTTCCCTCTTTCCAGCATGAAGTGTGAG
CTTTAGTTTTAGACAGGCTCTGTTAAAATCTTCAGGAGCATCAAAGCAAGACTCTCTGGGTCCAGATTAAAAACTCAAGTAGAACTT
TCTCTTTTAGTCCTTGTTAGTTTTCTTTCCACCTTGATATTTTGTTTCGAGTTTCCTGTATGGCCTGGCTTTGAATTCACTGTGTAG
CTAAGGATGGCCGTGAACTTCCACTCCTTGCACCTCCATCCCAAAGGGCGGGGGCCACCAGCTTGTATGCCCAGGAAATAGCTGGAA
TCCCCCCTGCTCTTGTGCTGCTTTTCTTTGTCTTTTTTAAGATTTATTTATTTTATGTACATGAGTACACTGTAACTGTCTACAGAC
ACACCAGAAGAGAACATCGGATCCCATTACAGACGTTTGTGAGCCACCATGTGGTTGCTGGGATTTGAACTCAGGACCTCTGGAAGA
GCAGTCAGTGCTCTTGACCTCTGAGCCATCTCTCTAGCCCCCATGGTGGTGTTTTTCTTAAGCACCACCTTTGTATTTCTCCATGTGG
TAAAAATTAGCCCGACACATGGTTGTACGTGGCTCCGTGGCTCTCGGTGTGCACTTTCCTAGCTCTGGTCCCCAGGAGGAAGAGCCG
GTCATAAATGTCTGCATCCCAGCGGGGAGGACTTGAGACTTCCCTGCTCTCTGGGGTGGGGAGGGGCTCTGGAGAAGCTGGGGAGCAC
TGACAGGGATGAAGAGGATGAAGAGGAGGAGTCCCCACAGAAGAGGGTGGGCGCCACAGGTTTCTGGTAACCAGGTAACTGCTGTTT
TCTCATCACAGGCTCCTCAGGATTCACTGCAGATCTAAGCACCTACTCTACACTTGGCTGACTTACCTGTGACCTTAGAGTTGTAGG
TTTTTTTTTTTTTAAGCTTGCATTATCATTATTATTATCAGCAGGCATGGCACGTAGCACTTCTTCCTAATTTGTCATATTCGGCTCCCCA
GAATTAAAAGTCCAGGGAGAGCGCCTCACGCTTCTGACACCAGAAAAATGACGCCTTGGCTCCCATCACTTTTGTAATCTAAAAAAT
GAGGAATGTATGCGGTTTGTTTTGAAGTCCTGTGTAATCAGAAATAGACCAGCTCTCTGAAGCAGTACTGACAGATGGGAGCATCAG
CCTGCTTTCCCTCCGCATCGAGATTCTAACCTCATTCCAGGTGACAGAGAGCATACTGAATATACTGTATGCTTGAAGAGTACCGAG
CGTGGGTGTGCTATATTCTCCCCAGCAGAAAGTATAACTGTGGGGTAACGAGTGTTATATGGATTTATTCATTCCAACACACACACA
TATACACACACACTAAGAACATCCAACTTATTAGGTAATGTAATATTTTATTTGCTGTTTATATTTTGGGAACGCTCCTCATTG
TCTCTCTCTCTGTCTCTGTCTCTGTCTGTCTGTCTCTTGCTCACTCGCTCTCTCTTGCTCTGTGTGTGTGTGTGTGTGTCTGTGTGT
GTGTGTGTGTGTGTAGCCCCAGGCTAGTTTCAATGTCTCTAGCCTAGGCTGGTCTCCAACTTGAGATGTCTTGCTTTCACCCTGAAT
GCTGGGACTGTAGACATGTACCACCACAATGGAAGTGTATGTAAGCTCATCTGTTCCTATGGTATAAGGTTTTCTTTTACCCCACTC
TGGGGAAAAAAGAAGTCTGAATTAATTTACAACTGATAACAACTTGGGTGTGCTCTGAACCCATCAAAAGTAAAGTCCTCAGGGGTC
```

156

```
CTCCCTGTGTTGCATGCACAGAGCCCCTGGCACTTGGTGGTACCTGTCTCCAGCACTGTCTTTTCCTGGAATTTAGAATTAGATAAG
GAAACTAGGCTCTCAAATTATCATCGGTTTCCATGGAAACAGTTGAAAGCCCCTCAATTAAAAGTGTCCCTGCAGGCTAGGGAGAGT
ATTCAAATGAGCTGCTCTGAGAAGCCAAGTTATCCTCAAAGTGAAGCCAGAGAGGACGGAGGAGCAAGGAGTCTGCTAGAGCACCAG
TTGCGATCTCTATTATTTTTTTTCCATTCAGGGAAGAATTTTATCTTGGTATTTCATTTGTAGCATGTTTGTTGGCTTTGGCAGAGC
CACCTAGATAATTGTCCTGTTATCTACTAAGTTTTTTATGCTCGCCCATGGATCACGGAAGTGTTTGAGAACGCTCTGAGCTTTGCT
CACACATGAATATGAGTCACAGATAAAAAGCCCATTTAAAAAAATTGATGATATCCTAGAAGAATACTAAGTTGGAAAGAGTCTGTG
GGTCATTAAGAACTAACTTCCCACCCATTTGGGATTCCTTGCTAGAGTGCAGTAACAGAGGTTGTTCAAGGTATACTTCAAACAAAA
AAAAAAGAATTTATTTATTTCAAACGTATGAGCGAGTTACATGCATGTATGTCTGTTTGCCGCATGCATGATGCATGGTGTCCTCAG
AAGTCAAAAGTGGACATTGGATTCCCTGGAAGTAGAATTATGGGTGACTGCACTATGTAGGTGCTGGGTTCTCTACAAGAACAGCAA
GTGCTAACCACGGAGCCATCTCTTCAGTCCCCTCACATTATACTACTAAGGAAAGCAGTGTGTGTGTGTGTGTGTGTGTGTGTGTGT
GTGTGTGTGTTTCATGGTAAGTGCTGGAGAGATGGCCCAGTGGTTGAGAGCACTTGTTGCTCTTCCAGAGGTCCTGTGTTTAGTTCC
TAGCACCAACACGGCAGTTCACTACCACTTGTGACTCCAGTTCCAGGGCTCTGACATTGTCTTCTGACCTCCATGGGCACTAGGCAT
GCATGTGCTACCCATAAATGCAGCCCAAACACTCGTGTACATTATAAAAGAAGATTTAAAAAGACAGTAATTGTATACCTGTCAAGC
CAGGTGTGAGATCTCTGCTTGGAAGTTGGCAGTAGAGACTGCAAACTCAGGGCCTTGCTAAACCAGCACCTCATGGTTCCAGAAGTC
ACATAGAAAAACTGGATGTAGCAGCACACGCCTGTACTTAATACTAGGGAGGCAGAGATGACATAACCTTAGGGCTTGTTGGCCAGC
CAGCCTAGCGGAATGTATGAGCCAGTTCACTGAGAGGCAGTTCGCAAAAGCTAAGATGGATCATGATTGAAGTTCAACACTCTAGTC
CACAAACACAGTTGCAGACTCATCACACGTTTGACACACACACACAAACACATAAACTCACATGCGCACGCATCTGTTTGTTGTCC
CAAGAAACACACTTTATCATCAGAGACAAAAACCTGAAGATGAAAGGTTTTTCAGCCCTGTGGAACTGGAAAGAAATCAGGGTATAT
CAGAAATATCTGACAAGATAGACTTTAAACCACTTAGATGGAAGAGATAAAAAAAAAGTTACTTAGTACTAATTAAAAGAAAATCAC
AATTTGAAACATGTGTATACCAGACATGTGTGTATCTAATTTTATAAAACAAACACTGTTGGATTCAAAGAGGCAGATTAACCCCCA
ACCCAGTAATAGTTAGTAATGCCAGCACTCTACTCTTACCAATAGGCAGTCCAACAAAACCTAGAAACATCTAAGTTAAATGACACA
GGTCAGATGGACCTAAGAGACATCTGTGGAATGGTTCACTTAAACCCTGTAGAGTCCACATTCTTTTCGAAGCTCAAGGAACCATTG
TATACTGTCTGATGACAGTGGAGTGAACCTTGAGATGAACAGCAAGAGGAACTGCAGAGTGTAGAGCTGAGGACCCAGGACACATTC
CTGGATGAAAGCAGTCCTAAGCAGTTAATTCATAGCTACAAGTATGTACACTTAGAAATTAGAGCTCAGGGTTGAAGAGATCGCTCA
GCAGCTAAGAGAAGAGCCCTGGCTGCTCTTCCAGGGACCTGGGTTTTAATTTCCAGCACGCTCATGGCGGACTGCAACTGTAACTCC
ATGTCTGGAGAGATCTGTCTGACACGTCTTCTGGCCTCTGTGGCACAAATGTAGGGCACAGACAAGCATTCAAACTGAACACTTGTA
CACATCAGAAACATTTAAAAGTTATTTTTAAAGAAGAAACCAGCTAGTGATACATGTCAGGGCCTTGGTAAACAAGAACAAGCCAAA
CATAAAACCAACAGATGGGAACAAAGACTCAGGATCAGGGCAGAAATGAGCGTCATGAAACACTCAAATGACAAAGAATCAGTGAAA
CAGGGCTTTGGAAAAATAAACAAGATTCACATGCTCTTAGCCAAACCAAAAGGAAATCCATGCAAATTAATAAAATTAGAGATAAAA
AGGGAGGCATTGTAAAAGACACCAAATGAAAGTCGGAAAATCAATAGGACCCAGCCCTAAAACTTTTGGAAATGAATTTCTAGACTC
ATATGGCCTTCCAAAACTAAACTAAGTTTAGATAAATAACTTAGATCTATTTTTAAATATTTAAACAACAACAATGACAATAATAAT
AATAAACAACTGAATTATAATTAATAATAATAATAATAATAATAATAGAAAACAACTCTGAATAAAGTAAAATAAAATAAAGGC
CAGGCCCAGATGAGTTCGTTCCGTTCTACCAAGCCTTTAAATAAGCATACTATTCCAAATTATTCCATAACATAGAAGAGGAGGGAA
TACTGTCATTTCACGAAGTCCCATTACTCAGGTACCAAATCCTCATAAAGGTACAGAAACAAGAAGACAAGAGAGAAAGAAAGGGAG
AGAGAAGGAAAGGAAGAAAGACCGTAGACTAATCTCCCTAACGAACGCATTCTCAGTGAAGTCCGTGCAAAGCAAGCGCAAGAACAC
GTCCAAAAGGCCGTCGTCCACATAATCAAGTTGGCTCCGTTTGAGAGATGCAACGGTGGTTAACCATACATACGTCAGTAAATGTAG
TCATCACAGGCATGGCCTCGAGGGTGGAAATCACGTGATCATCACAGTACATACGGATTGGCAAAGCCCCCATCCCTTCATGATAAA
AGCTCTCGAGGAGTCCAGAAAGATGGCTCAACCATTTAGAGCATGTACAGCCTTGCAGAAGACAGGTCAGTTCCTAATACCCATATC
AGGCTTGCTGCCTCTAACTCCAGTTCTGGAGCAGTCTGACATGAGTGGCCTCTGTGGGCACCTGCAAGCACTTGTGTACACACACAC
ACACACACACACACACACACACACAAACGATTTAAGGAACTCAGTAGAAGGACCATAGCTCAACAATGAAGGCTGTATGTGAGAA
ACCTATAGCTAGCATTATCCTAAATGGACAAAAGCTAGTGTCTTCCACTGAAATCAGGAACAAACCAGGAGCGCCTGCCTTCTCCA
CCCTGCGGTTTGGGAGTGCGCACACACGTGTGCGGTATACATATGCAGAGAACAGAGTTGTACACACGTGTGCAGTATGTGTATGCA
GAGAAGAGAGCTTCATTTTCCCTTAGATTTCGAGAGAGGGTACCCAATGAAATGGGCAGCTTATCTTTGTAGTCAATCTGGCCACCA
AGCCAAATCTACTTGTTCCCACCCACCCCCACTCCCATTTTAGAGTTAGAGTCACACAACTCGCTTTTATGTGCATGCCAGAGAGCTCC
CTCACTCAGTTAAATATAGTACTTAAAAGTCTTAGCTAGGACAAATTTAAGATAAGGAAATACAAACAAGAAATCAGTTTCCTTA
TTTACAGCTGATCCGATCTATACACGAGAGGCTCTGAAAACTCCAACAGGAAACTCAGAGCCGATGAACACTTCCATCAAAGTAAAA
GGATACAAAAAATTCACATACAACATCAACTGCCTTCCTCTATACCATGGCAAACGTACTGAGAGGGTAGACAGAGAAACAGCCTCA
TTCCCAGCCATCCAAACAAGTACCAAGAATAAATGTAACCACAGCGGTGAAAGACCTCTACCATGAGAACTTTAACACACAAAGAAA
GATGGGAGAGACCTCCCATGCTCATGGATTGGAAACGTTAATATTGCGAATACGCTACCAGAGGCAATCACCAGAATTCAGTACCTGT
CAAAATTCCAACACTATTTTTTTCTCACAGAAATAGAAGAAAAAAAATTAAAGTCATCTGGAAGCACAAAAGATCTCAGATAGACAA
TAGAATTTTCAGGAAAAAAGAATTTCAGGTTGATTCATAGCCTATTTTAGGGACGACAGGGCGGTAGTAACAAAACAGCATGGTACT
GCACAGAAACAGACGTCTATATCAGTCGAACAGAATAGAGGAGCTGGGAGCAGAGTCAAGCAGTTACAGCTGCCAACATTTTTGACA
AGATTTTAGTACAATATAGTGGAGAAAAGTCTCTTTTCAACAAATGGTGGAAGATCGAAGCTAGATCCTTATCTTTTGCTGTGCACA
AAAAAATCCATTCCAAATGGATCCAAGACCTTAGCATAAGCCCTGAAAACTCGGAAACCATTGGGGAAGAAAACATTTCCAGACATT
GGCATGGGGTGGGAGGCGGCTTAGTGGGTGAGAGTGCTTCCTGTTCTTTCCAAAGGACTGGAGTTTGGTTCCCAGCACACACGTCT
GGCAGCTCACATGGAACTCCAGCTCCAAGGGCTTTGAATCCTCTTCTGGCCTCGTCAGGTGCCTGCGTATGTGCATATACACACATG
TAAAGTTAAAAAAAAATCTTAAAAAAAAAGATATTGACATCCGCCAACACTCAGTAAGGACTCAGAAAGCAATTGACAGATGGGATTT
CATGAAATGACCAAACTTCTGCACAGCCAAGGAAACAGCAGAGTGAAGCACACGGCAACATGGGAGAGAATCTTTGCCGTCCCATGGA
AGACTAAGCTCTAGAATATACAAAGAAATAGAAAATGCTACATGGGGCTGGCACGATGGCTCAGCGGGTAAGAGCATTGACTGCTCT
TCCAAAGGCCCTGAGTTCAAATCCCAGCAACCACATGATGGCTCACAACCATTTGTAATGGGATCTGACACCCTCTTCTGGTGTGTC
TGAAGACAGCAACAGTGTACTTATCTATAATAATAAATAAATCTTTAATGCCAAATGAACCAAACAATAAATGGGCTGGTGGACAGA
CAGTTACCAAAAGAGGGAAATACAAATGGCCAATAAACATGAAAAATTGTTCAACTGGCCATCAGAGAACTGTAAATAAAAGCTAAAT
TGAGATCCTATGAGAGACAGAGTAGCAGTCATTGAGACAACAATACCAGACAGTGGTTTGTATGGGAACAAAGGAGAGCTCATATGC
CATGCTAGTGGGATATGAACTAATCAGGCCACTGAGACTCAGTGTGGAGTGTCCTCAAAAGACAGAAAAATGAGACTGCCATATGCC
TTAGCTGGCTACCTGTCCAATCCACACAGGACAAGGACACTTGCATGGCAGTGTTCACTACGGCACTGTTCTCCATAACCAAGGCAT
GGAGCCAAGTTCACCAGTATAGAAATGCATAAAGAGAATGCAGTGCGTACACACAATGGATTGCTTTCGTCCATGAAGAATGATGAA
ATTGTCATTTGCAGGAAATGAGTGATGGCGATCATCCAGTTAGGCAGATGAGTTCAGCCTCAGAATGACGAATTCCAATGTTCTCTC
TCTCTTATGGGTATTAGACATTGCATATACATAAAATCATGTGTGTACAGACGATGTGAAAGTGAAGGTCAAACTGTCTAGGGAAAC
TGAGCGGACTAGAGGGATGGGGAGAGCCAGGAAGAGGACGTTAGCAGGATTGGGAGTCAGAGGTGGAATTCTAGCTCAATGTACATT
ATGTAGTTATACTTGCCTGATGTAACCAAATACAATTTAAGATAGGCTCAGTGAGTAGCTCAGTGAGTAAGATGCTTGGTGG
ACAAGCATGCGGGCCTCAGCATTCCACCCTTGTCATTCATGTAAGAAGACAAACATGTGCCTTCTGTTTGTAATCCAGAGCTGGGGA
GTTGGGAGACAAACAGAACCCTGGGGCCTTTGGCCCAGACATGCCAGGCAAATCATCCAGCCCCAAGCTCCAGGGAGGTAACCTTGTC
TCAAAAGATAAATAAAATTAAAAACTAAATGAAGAGGATGGTGCCTGAAGAATAACAAACACCCTCTGGTTTACCTCTGGTGTACAC
ACACACACACACACACACAGGCACACACACACACCACTGCTCATATACACATACATAATGCACATATGTACACACAGAGACAC
TGTAAACACTTAATACATTTAAGACAGCTGAAAAGGAATGACAAATTGCCAATGTTGATGTTATGCTAATTACTTCAATCTGAACAC
```

CACACACTGCATGTAGCCACCGACTGAAATTTGACGTTGTACCTCCACAGTACCATGTGCAATTCCAATACATCAATTTAAAATATT
TAATTAAAAATTGTGAGGAAAGAGCCGGGCGTGGTGGCGTACGCCTTTAATCCCAGCACTCAGAGGCAGGGGCAGGCGGATTTCTGA
GTTCGAGGCCAGCCTGGTCTACAAAGTGAGTTCCAGGACAGCCAGAGCTATACAGAGAAACCCTGTCTGAAAAACAAAACAAAACA
AACAAACAAACAAAAAAATTGTGAGGAAAAATAAACATGTTTTCCTCTCCCCTCCACCTCTTTGTCTTTCATACCAAGCAAATCAAT
ACTTCTCCAATAATGAACTTTTCAGATTGCATCATGTGCACTCAAAGTTTGCTTGGCCACTGACAGAACAAGGTGTGAAGAATTCAC
ACCTGTGGCAAGCCGAGGGGCTGCCATCCTTCCAGCTGAGCCTTGATAACCACTGGCTACATTCTATCTGATTTCTCTGGCAACATT
TTTTCTCTGCAGCGTTTATGTAAGTGTAGACTTGTTTGGTGGTTTCTGTGTTTTCTGTTCAGTTTCTCCAACCAGGGTAAATAAATG
TCCAAGTTCTAAGCTTAAACACAAAACTGGGCTTAGCCCTGGGTCTGTGCCTGCAGTGCCCTGCAGGGTGCAGCACATCTTGGGGCC
CACTCCCCTCCTCCCTGCTTTGAAACTGCCCAGCCTTCCTCTCTGACTTTATTTAAGGCCGCCATCATTTCTACTAAATTAAAATGT
TATACTTTAGTGTTGTAGCTTTTTTTTTTTTTTTTGTCAAACACATTTTGTTGTTTTTTAAGTTGACACTCCTAAAAAGAAACAACT
CAAAAGCTCTAAAATGCTGTTCCCCAAATGTTTCAACAACTTTCCAATGGATTGAAAGTTAAACCTCCTACACTCTGGTTTGATTGG
CACTGCCTGGGAAGCACACTGCGGCAACTGCCTGGCCTGCTTGATCAGGAAGTGCTGAGATAGACATCTGCTCCAGTTCTAAAATAA
CCCATGACCTTCAGCCCGTCACCAAACTGCTCCAAATTCGATTTTCCTCATCCAAAATCAGATGTGTCAGCCCTGTTCCAGGTCGGT
GGCAGTGCTGTGTAAAAAGACATTCACTATCTTTTTTAGATTGGATGGTAAACCATACTTCAATTAAGTGATTTGATTTGAGGTTTC
AAATGTAATAAGAAAATCAAATAGTGATGATTCTGGGTGTTTCAGGTTCTCAGTTCCCAGAACACTGTCGCTGGTCTCCAGAGACCA
AATCCAAGGGTGTCCTCTGCATTTTAGAGAATCAGTATCATAATTCCTGGCAGGCTTTGACATGTTCTCACACTCCAGAAAGGAGTT
CTCTTAAATGGGTATTGCATGTGGAGAAATAAAATCACGTTGGATTCCTTATGTAGTAGAAGAATAAAGGAATTGAAAGTAAGGGAG
CGACTTGCTTGTTTTTAATCTCAGCCCCTGAAGTGTGGCGAATGTCTCCACCACTGAGCACGTGAGCAGACCTGTGGGATCCTTGGG
ACCTGAACCTGACGGGGTTGTCCGGGAGGCTCAAGTGTGGAGTCTGGGAGAGCGCTTTACCTTGGGGAGGCCTGTGAGCGCTTGCAGA
CAAGCCTGCAAAGTTCACTCACAAGCTCCTCGCTCCCCCACCCCACCATATTTCTGGAAACATGCCAAGGGCTTGCTTGCCAGTGGT
CCCGGGGGACTTAAAATGACTTCTTTCTGTCCAAGGTAGTGTATTTGTTGTGACATCTACTTTTTACTCAAAACAGTATGTATTTGA
GGGCACTGAAACAAAGATACCATCTATAGTTGCTCCACCCAGAGCCTGATAATGGTGTTTTTATTAGTGTTGGTTGGCTATTTTATC
ATTTTTACCAAAAATACAAGCACGGTTGATCCTGCAAAGAAATCTATTTCCCTCCCTCCCTTCCTTCCTCCTTTTCTCCCTCCTTCCT
GCTTTCTCCCTCCTCTCCCTCCTTCTCTCTCTCTCTCCCCCTTCCTTTCTTTCTCTCTCCCTCCCTTTCTTTCTTCCCCTCCCT
CCCTNNNNNNNNNNNNNNNNNNNNNNCCCTCCCTCCCTGCCTCCCTCCCTCCCTCCCTCCCTCCCTGACTCCATCCTGGTCCTCCAGCC
TTTCTCTTCCTACTTCCCCTTTTCCTATTCCTTTTCCCCCCTTTCCCTTCACTTACCTGGTCTCTTGACTACAGTCATCCCAGATTG
CCAGCAGATGACTGCTGAATATGGCCAGATAACTCCTGTGGGAGAGAATGGCACTTGCTGGCAGCTTGGCCTGGTGTCCTGAGTTTT
GTTTTTTTGTTTTTTTCCATCTCCAGGGTGTCCTCTCCAACATCTCGTCCATCACCGACCTCGGTGGCTTTGACCCAGTGTGGCTTTT
CCTTGTGGTGGGAGGAGTGATGTTCATTCTGGGGTTTGCAGGGTGCATCGGAGCACTTCGGGAAAACACCTTTCTTCTCAAGTTTGT
AAGTATTGCAGACTCTGCTCTCAGCCTGAGATCTAGGCTCTTGTTTTACACCAAGACGACAGTCTTAGCTGCAGCCAGGATAGTAAG
CGTGCCAAAGACCTGGGCAAAACATGCAGCCTAGTCCACTTTCATTTCAAGAGCGCTCCTCAGTCCTGCCCGCTCTTCCCTTGCTCT
AGACATGCCTCCTAGGCAGTATACATACCACCTAGTCTTGCCATGCATGGAGACTTTGTACATATATGCAACGCATTTGACTTCAAT
CCCTAAAGTAGTTTTAGGAAGGAATGAGATGTTGGGGGCGGGGCGAGTAGTTTGTTGGATGTTGAATGTGCATGTGTTGGTGTGTTG
GTGGTGAATATTGTAAATAGAAAACCTAGGGTAACAAGGCACTAGCCCAGCTTCTATAGCCTGTGCAGCCTGCACAGTTTGAATAAA
CATGGCGATACAGTAAAGGTGAAAGACCTTCTTAGTTTCACCAGCTGTATTTGTAAGGGGGGGGGGGACTTTAGCAGGAGGTATGAA
ATATTCAGAGCTCGTCCTAGAGAATGGGCAATGAAAGCCTTCCCTTAGGGCTCCTTTCCAACAGGACAGCGGCATGGGCCACATATC
AGTATCTGAAGTCAGAAGAATGGTCAATGTGAAATGCAAATACAGCTTCCGTGATGGCTGATGTCATCAAATGTAACTTCAGGTTGC
TTTTCTGTATCTTTGGAAAGAGAGGACTTAGCTCACAGGGGCCCCGAACCTGTCAGTTTCTGCCAGATACTTGGTATGAACTGAGTTT
GGCCTCTTTGTGTGTGGAGGCTGGCATTTTAATGATTGCTGTAGGAAAAGTATTCACTCTCCTCTCCAAGAACAGGGAAAGGATCAG
AAGGACCCACTGTCCCATGATGGGGGAGGACAGGGGAATTTCTAGAGAACAACTTTGTTGGCTATGCCTCTTATTAGAGCCATTCGC
ACCAGAGCCCATCCTAGAACAAGTCTGAGCAGCCACACGGTGTGTGATTCTCCGACCCCACCATGTCTTCTAGAATGTTCTCTGGCG
TTTTTTCATGAGCATCTCTAAGAGATACTCCCATGCCATTCTAGGGTAACCCATTTCCAATTGGCCCAGCTCTGGCTCCCTGAGA
GCCTTGGAGGCTGTAATCCTAAATCTTTTCATGTGGCTCACACCTATATGGAAGACTGGGGTGGGGTCATTGCTCTCAGATAGCACC
CTTTTTCCTGTGCATGGGAGTAGCTAGTTTCCCATGTGTAGAGCTGCCTTGATCTTCCAGATTTGCCCCTTTCTACACAAGCTTGTGT
GATCTGTCCCTTCTTCCCTTCTCCCTCTTCGCTGAGTCAAAGACAGATGGAATCCCTCAGAAGGATATTTGTCCTTGATCATGACCT
GCCAGTCTTATGAGGCATCTCGAAGTGGGGACTTGGTTCCACTCAGCTTTGCTGTGACCCATTATCTGCCGTGTGACCTAACACTTT
GAAACCTCCATGCTTCCAAATACGAGGTCCCTTTAATATAGCTTTCTTGAACATGGTTTTACTTATTATAGTAATGCCCTTCCTAAC
GTTTCAGATTTGAAAAGAATCATGTTCTTAGCTTTCTATCTTGGAGACTATTTAAATGGCCTTTCTTTATTTAGAACTAACAATAGA
ACCTGGCCTAGAAGTCTCTCTGAATGGGGGCTGTGAGGTAAGGAGGGAAACAATGTCACCGTGCCTCCAAAAAAACCTCAAAGATCAGA
TTGTCCTAAGACAAGGAGAGTTTCTGGGGGAAATCCAGGAGGCAGGACTGTGGAATAGAAGTGGCATTTCCTAGCTGACCGATAGCA
TTGAACAAAGCTTTGGGTCACTTTCTGCACCTTTGAAATGGGGGTGTTAGTTCACAGAGCCGGGCATGTAGGCCTCTGCCAAATAT
TTGATATTGAGTTTGAACTCAATATTTGAGATGTATATCATGTAACTTGAGGGAATTTCCGAGGGCACCTGGGAAGTGATCGTCTCT
ATCCTTCATGGAGGTGAGAGCAGAAATCTACAGTCTCTGTGTGACTGCCCAGCTGTTTCACGTACATGCATGCCCACCCCCGTCCAG
GGTCTATTCTTTCTGTGGTGTGAGGACCCTATGTGTCCATGTTTGGGGAGGTACCCAGTACAGGTAAAGGGGTTGCAGGGGGCACAA
ATACTGGTGTGCTCCTTCTTTTTCTAAGAAAACGTAAATTTGAAAAGAATCGTAATTTGAAATTATTGATACCACTCATAATTAAGAAC
TTAACATTTTATTTACAGCTGCAAATAATTGGTACAGTCAGTAAACAAGAACTTTACATTTTATGTCCTCACTGAGGAAAGATAAAC
AGGAGCCCTAGGGGAGCTTGGAAGTGGAGTTTGTACCTCAGCCTCAGCATCAGCATCCTAGACTGTCAGGCACTGGTGTGGTCCTTC
TGACTCTCTCCCCTCACTCCCCCACAAAGACGCATCCATCACTCAGTGCCACGTGAATGGCCACACCCCACTGTCCACCCACCATGG
CCCCCCATTTTTGCACCTCAAGATCACAAAACAGTTTGCAGAGGGTAAAGATGTGGGAGGAAGGAGATGTGGAAATGGTGGTGGGTG
CAGCCGGCAGCCAGGATGACTAAAATGAATCTCTTTGAAGTGTCTGGGTGGAAGGGAAATTTCCCCAGGGTTCTGAGCAGTTACCCC
AGGGCCTCATGCTCTGAAGCCGTATACCTTGTTATCTGCCTTAGCCAGAAGTCCCTTCTGATTGCACTGTGGTGTTATTTAAAGTGT
TTCCTGGTCTTGGTGTTTCACAGGCAGGAAGAAAGAGAGCCCAGAGTTTCATTTCCATGATTGGAAATGGTTTTCTTGCCTCAGTCA
CAGCCCCTCCCACTTCCCCTGCTGACCTCTTTAAGATGAGCGGATCTATTTCACAGGGCTGGGAGGTGGAGGGAGGTGTTAAAACAG
TGACTCCAACATCAACACGATAATGTAGTGTGTGTGTGTAGTGTGTGCGTGTAGTGTGTGTGGAGTCAAGCCACGGTTGCAAGGGAC
AGGTGATTATAAAACCCACGATAATCTACCTTTCAAGTTAGGAAAGAGGCTTCCTGGAGGTCAGGCAGAACATTGTTCACACGTCTG
GCAGTGCATTAATCACGAGCAATTCATTAGGCCTTAATGTGGATCTAATGAGAAATTCCTATTACTCAATGTTTGATTTGGGCTTTG
TTGGTTAATTATCAAATGGAACACCAGAGACTCCAAAATGTGATTATGAAGTGATCTTTAATTTTTAAAAGTCCTTTCTTGGGGTGT
AATATTCCATTAACAAACCTTAAGAATCAGAAGGCCCCAGTTTGGAGATGTGTAGCAACTGAATGTAATTACAGCCCCTTTCAGAGA
AAAGCCTCTCGCTTCCCTGTGATCTAAGGCCTTGTAACCCGTTCCCTGTTGTGTGTCTCTTTTGTCTTAGTTTTCTGTGTTCCTGGG
GATTATTTTCTTCCTGGAACTCACTGCTGGGGTGTTGGCATTTGTTTTCAAAGACTGGATCAAAGACCAGCTGTATTTCTTTATTAA
CAACAACATCAGAGATGACATTGATCTACAGAACCTCATAGACTTCACCCAGAAATATGTAAGTTCCAAGCAGGAGCA
TCTTTTCTCCCATAATGCCTTGTGTGCCTTTGCCAGCTGGAGACGGCCAACTGACGTTTCTGCCCAGTTTCTGGCTGTTTGCTTGTT
GGTTTGTTTCCTTTTCCATGTTCCACCGCTCCTGTTTCATCTCAAGGAGACATGTTCCGAGTTCACTTGCTCCAACTTGTGTTAAAT
TAGAGGAAGATATAATTCTTAACTAAAAAGGCGATTACCGCACCGTCCCGTTGTCAGCTTTTGAAGAAAATTGCTTTTATATATGTT
TAGAGTTTTATGTGATTAAATAAATTAAACGAGTTTTCCTTGCTCCTTCCCCTGTGCTATCAATAATAAAATTATCAGGGGGATTTG
TTTNNNNNNNNNNNNNNNNNNNNNNGAACTCACTGCTGGGGGTGTTGGCATTTGTTTTCAAAGACTGGATCAAAGGACCAGCTGTATTT

EP 2 204 376 A2

```
CTTTATTAACAACAACATCAGAGCCTACAGAGATGACATTGATCTACAGAACCTCATAGACTTCACCCAGGAATATGTAAGTTCCAA
GCAGGAGCATCTTTTCTCCCATAATGCCTTGTGTGCCTTTGCCAGCTGGAGACGGCCAACTGACGTTTCTGCCCAGTTTCTGGCTGT
TTGCTTGTTGGTTTGTTTCCTTTTCCATGTTCCACCGCTCCTGTTTCATCTCAAGGAGACATGTTCCGAGTTCACTTGTTCCAACTT
GTGTTAAATTAGAGAAAGATATAACTCTTAACTAAAAAGGCGATTACCGCACCGTCCCGTTGTCAGCTTTTGAAGAAAATTGCTTTT
ATATATGTTTAGAGTTTTATGTGATTAAATAAATTAAACGAGTTTTCCTTGCTCCTTCCCCTGTGCTCTCAATAATAAAATTATCAG
GGGATTTGTTTGTAAAGTTCCTCAGATATGCTCATCCAGATACCACATGCATGTAGTCCGAACCAAAAGTTCAGAGATTCGTAAAAC
CTGCTGCCCTCTAACAAGTGAAAAAGAAGCAGACCCCGCCTGCACCACAGGTGTCTGAACGTGACTGTGTGACTGACTGTTCCCATA
CTGTAAACAGGCAGCTGTCATGTCACTCAAAATGCTCGAGGAGCCAAGCGACACTGCCTCATTGACCATGGACTGACAGAAACTTGA
ACTCTTTCCCAAGAGTAAAAGGAGAATAAAACTGAGTTACTATCTAGTACGTATGGCTGTTGGGACAGTGGTCTCACCTTAAGCCCT
AGATGTTACCAGACTAAAGATTAGCAGGTCCTCACACACCTGCCCTGGAAAAAAATGAGTAAGCCGAGCGACCTTGGTTCCATGGTA
TCCATGGCTTTAATATTTAGAACAAGATAGGAGACTACACAGAGGCTCTGTGGACCAAGGTCTAGCTCTGAGTTGGGTAGGTTGCTC
TGGGGATCACATTTACCTTCAGTCAAGAAGGCCACACTGCATCAGCATTGAGTGGGTGTTGAGGTAGGCCATTCTTCTCTAGGTGCTC
AGCACTGGGCTGAGGTCTTAGTTTCACTCAGTGGCTTCTATCTAGAGCAAGCATCCTCAGGTGGTGTGTGAAATTGAGCCCTGTCTCAC
GTGCTCAGGCGATTTCCCTGTGCTGTTAGGTCATAATGGATGCAAGCTCTCAGCTTCCCGGGTATTCTCCCTGACTAGCTGGCATGA
CAGAGCCGGCGAGGCTTTACAGGAAACCTGTGGTGTCCAGCCCAAGCCCAGAGGAGCAGCTGAGCCTCCAGAACCTGTGTTTGCACG
GTAGCTGTTGGTCCTGAAAGCCTTAAAATGGCCACAAATTGGTTTTATTTAACTTAAGAAGCCAAGATGATCCAGTACAGAAGGAGG
CCATAGTCAGTCCCCATTTAAGATTTGAGTTGAACTGAATTTAAGAACTGAACTATAGGCTGGAGAGATGGTTCCTTGGTTAAGAGC
ACTGACTGCTCTTCCAGAGGTCTTGAGTTCAATTCCGAGCAACCACCTGGTGGCTCACAACCATCTGTAATGGGGTCCAATGCCCTC
TTCTGGTGTGTCTGAAGGCAGCTACAGTGTACTCACATACATAAAATAAATAAATCTTTTTAAAAAAATCTTTTAAAAAGAACTGAA
TTGAGCATAAAAATAAGTTCTTTTTAAAAATTTAGTGTAGAGATGAATATAATGAATAGTTAGGCCTCTAGTATTATAGAGAGGTGG
GTCTGCCTCATCCGTGTTTGACCACTGTAGCAATGGACATCAGCCCTGACGTTCACTGTCCTGTGAAGTGGCTACTGTTCTACTGTG
TTACCCACAGACCAGACCTCATCAAAGTGTCTACTGTTTACCTGATGCCACTTAAACACAGTGAAACTGTTTACGAACCATTAAAAT
AAGATCAAAACTTAAGCACAACCACAGTTGCCTCGTTATACGTTACCGTTGTTGCTAGTGTCTTGTTTTTCCTTCTAGAATGTTCTG
GTTTATCCTAGAACGTGACCGAGCCAAATTTATCGTGAGCCCTGCTGTACAATGTCCCATGCCAAACCATCTCTAGGAACCTTCAGC
CTACATCCCTTTGGAAGGCTTTCCAGGGCCCGTTTAGGTTAATTTTAACTTAAAACTAAATGTTGAGGCCCCGGACCTGTCTGGCAG
GGGATGGGTGTCATGGGGGTGGGGTGCAGGTGGCTGGTCACCCCACCTGAGATGTCCTTTCTAACTCTCTCCTGTTTGCTCTAGTGG
CAGTGCTGTGGGGCTTTTGGGACTGATGATTGGAACCTAAATATTTACTTCAATTGCACAGATTCCAATGCAAGCCGAGAGCGATGC
GGTGTGCCATTTTCCTGCTGCACTAAAGACCCCGCGGTAAGAGCGACTGCTCACTGCTGCCTGCCCGCCGTGTGCCCGTGTGACGG
CAGAGCCATTTCTGTCTCAAAACTCTCTTCTCACAAGCTCAGACCTGACTGTAGGTCCTAGAGGAGGCTGATACGCTCCAGTGTTCC
TAGTCAGGACAGGATGGCAGGAATTAATTTAGCCAGTGCTTGTTGTCTCCTATTCTGAGCCCTGGTGTTGTGTCTCACGGGAGGTGA
GAAAGGGAAGTCCCTTTCTGGGGAAGAAAAATAAAACCCACCAAGGCCTATGGAGTACAAATGATTTCTCCCAAATAACAGGGCTGAC
AAGGGACAGAGCCGGGTGACTGTGTCTCTTGTCTCTCCTTATAATGTATGAGCCACCTCTCTGCAGTAGCTTGGATTTAGTAAGCCT
TTTCTTCTCCTCTGGTTTCTGGGAAGGGACTCAGGAGTAAAGAAGGCAGATGAGGATGGGGCGCTGCTTGAGGACCCACAGTGGCCC
GGAGGGTGGCATGGGGGTGGCTGCAGCGGCTGCCTTTTGCGGTTTTACTTTAGAAAGAAAACACGGAGTGCGTCCACAAGCTACTCC
TCTTTCCTACACTTCACATCGTGTGCATGGCTCACAGAATGAAAATGTGAACCGTCTGTATTGTAAGTGGAAAGCCAGAACTGAGTG
GTAGTGTGCCCCGTGTGGGGAGTGGCTACTGCCCTTCTGGGACAAGCCTGCCCATTGAGCACAGTGGCGACAGTGGGTGCAGCGTC
CCACAGGCTCCCCCTCCTGCAAGGTTGGGGCTATGCGGCTCTCTCGCGAGGCTTGGGTTTTAGGATGTTGAGCTGCAGGCTGTGCAG
GCTGCTCTTCTGGAGGGCGCAACAGCCTGGGCTGAGCGTCACTGAGAACTGTGTCATCCTGCCTGTGACTCCTTCCTAAACTTCCTA
GCTTGCCTTCCTCCTCTGAGGCGGCTCAAGCTGCAGCAGGATATCCAGCCTGGAAGCCATGGTACCCAGCATAATGTGCTTGCTGGT
GGGGAATGTTTTACTATATACTTTTGAAGCTGCTCTAGTAAAAGCATGGCATTTAATAATAATAATTGTTAAATAATATTTGATAAT
TAATTTAATAAATAATGTTTAAATACTGTGCTTCCAGTGCCCATCTTAGCTGGGCTTTCTGCTGAACAAGAATGAGTTTCAGTTTTCTC
TTCCTTTCTTCCCCAGGAAGATGTCATCAACACTCAGTGTGGCTATGATGCCAGGCAGAAACCAGTAAGTGAAACCCGCCCCATGTC
TTAGCACATACGGATTTTTTGGAGGAGCGTTTGCGCATGCGTAAGGCTGTATCTATGACATTCTGCTCTCTTCCCTTGTAAAGGAAG
TTGACCAACAGATTGTAATCTACACAAAAGGCTGTGTGCCCCAGTTTGAGAAGTGGCTACAGGACAATTTAACCATCGTGGCTGGTA
TTTTCATAGGCATTGCATTGCTACAGGTAAGATGTGGGTCCTCCGTGCTAGCTCAGCAGGTGGTGCCCAGAAAGACCTCCCCAGTCC
AAGATCTTTGCTTTGTCTCCTCGATGCCTTAGCTTCTGAGCACACTGTAGGTCCTAGAATCAGACTGGAACTATTGGGTTTGTGTT
TATTGAAGGCCTGAGTCTAGAGACAGAAATACCTTAGGAAGTCTTGCCTCCTTTCACCACAGCGGTGTTGATGGGTCCCAGGTTGTT
ATCTGGCATCCCATTGTTTCAAGGATTCTTAAAGGATTGTGTTGTGACACTTGGGGCAGACAAGGCTGCTCAGAGTGGGGACCCCAG
CAGCAAGGCCAAGCTCTGTGGAGTTGCACAAGGACGTGCGATATCTTTTGTCTCTCCAGACCAGCTCACCCAAGTGCTGGTGAGGAC
AGTCCCAGGAGCTGACTCTTCCTGTTCATAAGATGCTTTCTGTGGAGGGCCACGGATGGATACATGGTTGAAGGAAATTGAAGAGGT
CTTAAGTAAATAGGAAGGCAAGCTGTGTCACAGATTATAAACTCAGTGTCGCTCAAGTGGCATCAGCAACTCATGGTGATCTAGAGA
TTCTGTGCTCTCCGTCTCTGTTCCAGCCTCCTCATTTAGTCATTGACTAGCTGGGCCTGAGTTCACAGGGAAAAATAAGAGCCCAGA
GTAGTCAAACACACTGAAAGAGAATGGCAGGATGAAGAATTCTGGTTTCCCAGTTAAAGCATACTACAAAGTAACAGGGTTCAAGGC
AGGCTTGTACGGTTGTAAAGTTAGCCATTTTGATAGATGAGATCGGATTACAGAGAGAACTTTTGTGAGCTTTGTTTTGATAAGGGA
ATTGAAACTTTTCAATGGGGAAGGAAAAAAGAACTTTCAAGGATGAAGGAAAACAAACAAAAACAAAAAAAAAACAAAAAAACAGCT
TAGTGGTTCAGAGCACTGGCTGTTCTTCCAGAGGACCAGGGTTCAATTCCCAACCCCTACATGATAATTAACAACTGTCTGTGACTG
TAGTTCCAAGGGACCTGATGCCCTCTTCTGGCCCCCATCCACACTTTATGCATGTGGTGCACAGTCATGCCTGTAGGCAAAACACCT
ATACACATTTCAAAACAGGGGGAAATTTTTTTAAAAAGTTTTTTGACAGATGGTGCAAGAATACTGATTTTTCTGTCTAGAAAGAA
AGGTGTTTAGACATCTATCCCATACCATATATAAACATAAACATAAACTAGATCAAAGACCCAAATCTAAGTGTGGAGTTCTGTATG
CCTGTAATGTAAGCCATTAAAAAAGGTTGTAGCAGGATAGCAGGATCGTCACTTAAGAGTACACAGCCAGCCTGGCCTTCACACTTA
GACCCTGTCTTGGTGTCAAATAATTAAACAAAGCTCTCTCAATAAAAAGCCAAAGCTGTGGGCTGGAGAGATGGCTCAGTGGTTAAG
AGCACTGACTGCTCTTCTAGAGGTCCTGAGTTCCATTCCCAGCAACCACATGGTGGCTCACAACCATCGTAATGGGATCTGATGCC
CTCTTCTGGTGTGTCTGAAGATAGTGATAGTGTACTCACATTCATAAAATAAATAAATATTTTTTTAAAAAATGCCAAGTTGTGACA
GTCTAGAAGAAACATGGTATAAATTGGTGATTCCCTTTGTTTTGTCTTGCACAAAGAGTTTCAGGGGAAAAAAAAAAACCATCAGAT
GAAGGAGAAATAATGGCACTTCATCAAAATATAAAACTGAGGTTACCCACAATAGGAGAAAGTATTTTTGAGGAGTTACGTGTGTGT
GTGTATGCATGTATACATAATATATATATATATATATATATATATATATATATATATATATATATATATATATATCTTTATAGGAAGCAAGAAA
AAAGGCTAGCATGGGCTATCTACCAAGGTCCGGTTTAAAAAATGAACAGAGTACTTAGACATTTCTCCAGCAGAGACAGGAATGTCC
CATGAAAGATACAGCATCACTTGCCATCAGAGAGCTCGCCAGAGCTGCAGTGAGGTGACCATTGCACACGTACACCCACTCGGACA
GCGGGTGTTCACGAGGATGGAGGATGGATCACCCTCAGCTTGCTAGAGTGGGGGAACAGAAAGGGCCCGGCTCTTTGGCAGATAACA
TAGTTACCATGTGACCTTACAGTTTTTCCACAAATGCCAAAGGAATGAAAAGCGACACTTACCAAAACATTGGCACACACGTGCACA
TAGCAACAAGAACTGGAGCACGCTGAAGAATCCATCCATGGATTTTTAAAGACTGTAGAACGGCCTTACGGTAGGGTCGGTAGTCAGC
CATCAAAAATGCAGGCATGGGCTGTGCTGCGCATGCCTGCAGTCTCAGCCCCAGGGAGGCAGAAGCGAGGCAGCAGAAGAACTTAG
ACTGTACAGGAAGGCTCTGCCTCAGAAGCAAGCAGACACCACAAATAGAACAGACTGGCCAGGTGTTTGTTTGCAAGTCACCACAGT
TACTTTCTTTAGCTGCTTTTGTATAGAACCCAGAGCTTGTGTCCCTTGCTGGCGTCCTGCACCACATCTCCCCCACTTCCATCTGAG
TCAGAACTCTGAATCAGCCACTCACCGCTTCTTCACAGTGCCCTGAGGGTGCCCACTTCTCAGCACAGCCTGAGTCCCTGACTCAGA
```

159

```
AAAATCACCCACCTAGTCAGCCTGGAGCTCTGCCTCCCCCCCAACCCCCACTGTGACCTTGCTGATGGGGAGAGGGAGGAAGCTGCA
GAGCCAGTGAAGTGATGGAGCCTTCCCTGAAGGTCACAGACCCAGCCTCAGCAGGGAGAACAGGGTGCTGAATGGATCAGTTGGTAC
CCAAGCCAGAAAAGCTGGTCACCAACAAAAATGTTCCCTGCATGTGTGATTTTATTAAGGCTTATTACCTGGCAGAGCCCATGAGAT
CCATACAAGGAGTGGGTTGTGACCCACCTGTGCAAGATAAGAGCCCAGCTGGGCTTCAGCCTGACATTTCCACAGACACGTGTGGTT
TCCCTGGTCATGAACACAGCGCATGCGTGTTAAGGTTACCTCAGCTCTCCTTATCTTGTGTAAAATGTTCACTAAAATTCTTTGATT
TGTACCCTGTAAATAAAGAAAACTCATAGTTGGGTCCTGAAGGGGTTGTAGCGCTGTGATTGAAACCTTGCAGAGGAAAGAAAGAGAG
GTTTGTTCAGACCCGTGGTGGGGGAAGGCATAGAGGTCATAGCAGCTCGGAGACAGATAACTCCTTTGACAGCTGTCCCTAGCAACA
CACACGTCTCAAATGTCCTACAACCTCCCAAAACATTGCCACTCACTGGAGACTGGGCGTGCACACCATGAGATTGCGTGGGCTTTT
CACATTTGAGCGGTCACCGTAGCAATGGACGAACCCAGAGATGGTTTTGCTCCTTGAGAGAAGTCACAGGAACCACATGTTGTCCGA
AGGCATTTTCATGAAATGTGCAGATCGGGCAGATCCATAGAGGGAGCAGGGAGGTGAGAGCTTTCTAGGCCATAGGGAGGGTTGGAA
ATGACTTCTCGTGGGATAGGGTTAGCTTAGCGGGGTGAGCAGCTCCTGCATTTAGTATTGAAAATATAAAGTCATGCTAAATCTCTG
GATTTTTTATTTTCTATCTCTCTTTCGCCACTCTTCCCCCCCCCCCAGCCTTTTTTTAAAGACAGGTCTCATGTATCCCAAGCTGGC
CTCAAACTTGCTCTGTAGCCAAGGATGGCTTTTTACACTCCTGCTTCTTCCCACCCAAGGGTCGGGATTATAGATGTGTACCACCAT
GCCTGGCTTTCTATGGCGCTGGGGGGTAGAACCCAAGGTTTTATGTATGTGAGGCAAACGCTACCGACTGAGCTACATCTCCAGCCC
CCAAATATCCATTTAAGAATCAGAGGGGGTGGACCTAGTGGCCAAGGAAAAGCCAATAGCATTTGAATTCTGCTGTCTAACTTGGTT
TCCTTTCCCCCTTTTTTTTTTTAGATTTTTGGGATATGCCTGGCACAGAATTTGGTGAGTGACATTGAAGCTGTCAGGGCTAGCTGGT
AGACCCCTGCGACCACTGCTGTCAGACACTGGACTGATCCCCCCTCACTGACTGACCCTCTGTGCGCCGGCCGGCTGATGGCGGGCT
GTAGAGGCCTGGTCACAGGCCTACAGTCTCACCTGATGGAGCTGCCAAGAACTTAACTTTCTGTGGGGGTAGAACTGGGAAATGCTG
CTCACAGACGGAACTTGGAAAAAGAAAAACAAAACAAGTGCGCAACTTGCTGAGTCAACGTGCAGTGAATCTCTACTGTAGCCGTGG
ATTGGTGGTCGGATGGATGCTACCAGAAGGGGGAAGGGGGGAGGTTGGGCACGGCATGTACTTGAATTTTTGGAGAATACAGTGCTG
TACACATCTTGGCTAAAACTTGGGCCCTTAGCCGTCAGGGCCAGGTGTCTTGAAATCTCTCAAACCCTTTGCATCTGTCCTCCAGAG
AAGAAACCAGCTGCCCCCTGTGTTCAGATTCTGAGAGAGGTGGAGCGGGTCACGATACAGCATGCGCCCTTCATCCTGAGTTTGCCC
TTAAGAGAACTGGGCGTTTGCAGTAAACTAGGGTGCCAGAGGCTGAGCTCCACATTTCCACAGGACTGCTCTTTCCTCTGAGTGGCC
AGCCCGAGCCTGAGCTCTGTCAATGACATCCAAGGAGAAAATGAGGTTAATGAGAGACATTAATTAAACACTCCCTCACCCCACCGC
ACCAAACCAGTTGGGTTCTTCTGATATTCTGGAATACTCTGGGCTATGTTTTATGTTTTATTTCTTTTTTAATCGGTTGTTATTTTG
GTTCTTTTTTTTTTTCTTTCTTTCTTTTTTCTTTTTGTCTCCCAAAGGAGGCTATGACACAGTTCTCTTGGTTTCTTATTCAGCTTG
AATAGTAAGTTTTTGTAGACTTGTCACCTGCATGTACTGTAACCCCACCCCCAAATCCACCATGTTCTTTTCAGAGAGACA
TTTGAGCGTGTTCCTTAGTGTTTATGTCCTAGGTATCCAGCCTTGAGTGGTTGTGGAGTGTTGTCCTAGCCGCATCTTTGTATGCCC
CTCAGGCTCTCTGTCTCTCCTGGCATCCAATTCAGCTTCTGTCCATTCGCCATTGAGTGGTAGAAAAGCCCAGCTCCAAAGCAGGGT
TGACACAGTCAAAAAGCATTTCATGAGGTTTTTCTTTCTTTTCTTTTTCCTCCCTTTTTGCAACTTTAGTTTTGTCGTATATTAAAT
AAAAAACCAAGCCTAGTATAGTGCATGTGCTCGCTGAGGTGACCTCGGGGTGGTTACAAAGCACAAATATTTGAGGGGCTGCGCCGA
AGCCCAGCCAGGGTTCAGTGACGCTAGGTGGAAAACCACCTTCAGGTTCTTCTCCTGCCCACTGGGAGGGGTGGACTTTAG
CCCCAGCCTCTGCGAAAAGCAGCTCCCCATTGGCCAGCCAGCAGTCTGCGTCATGCAGCCCGGTTTTCCATTTCCGCAGCACGTGT
GCGTTGCCGTGGCTGATGGGCGCTGTCTGCTCAGAGGGAGGTCAGGCACTCCTTGGGTTTACGTGGCTCTGCATGTTTGTATCGGTG
TCTGTTTTTAGGTAGAGATAGTCATTCCATACGTGGATGCGAGGTCACGTGACTTCACCCCTGTCCATACCACAGCCGTCGCTTGTG
ATGTCCTTCAACATGGACACTCGTGCTCTCGGATTGCTCGGATTCGTCTTCTTTATTACCTCATTCTCCAGTGAACTGCATCTGGCCA
ATTGATTCAGAATCAGACAGGATCCCCACCCCGTGGCACATCCAGTGAAAGCCAAAACACAAGCCCAAGCGAGTTTTAAATTTTAAT
CACCCTTTATTTTTTACACTCGAGAGTGATTGTAATAAAAGCTGTCATTAATAAACTCGGTTCTACCT


MOUSE mRNA SEQUENCE : mR28-002.1 (Seq ID No: 50)
CGCGGCATGAGCCCCGCGTGCCCCACTGCTCGCCGGGCTGCTCCGGGCTGGGGTGCTCCGGGGCTCCAAACTCGGGCTGGCGGGGCA
AGTGTCTTCATGAACCCAGAGGATGTCCGGGAAGCACTACAAGGGTCCTGAAGTCAGTTGTTGCATCAAATACTTCATTTTTGGCTT
CAATGTCATATTTTGGTTTTTGGGAATAACGTTTCTTGGAATCGGACTGTGGGCGTGGAATGAAAAAGGTGTCCTCTCCAACATCTC
GTCCATCACCGACCTCGGTGGCTTTGACCCAGTGTGGCTTTTCCTTGTGGTGGGAGGAGTGATGTTCATTCTGGGGTTTGCAGGGTG
CATCGGAGCACTTCGGGAAAACACCTTTCTTCTCAAGTTTTTTTTCTGTGTTCCTGGGGATTATTTTCTTCCTGGAACTCACTGCTGG
GGTGTTGGCATTTGTTTTCAAAGACTGGATCAAAGACCAGCTGTATTTCTTTATTAACAACAACATCAGACAGATGGACAGAGATGACAT
TGATCTACAGAACCTCATAGACTTCACCCAGGAATATTGGCAGTGCTGTGGGGCTTTTGGAGCTGATGATTGGAACCTAAATATTTA
CTTCAATTGCACAGATTCCAATGCAAGCCGAGAGCGATGCGGTGTGCCATTTTCCTGCTGCACTAAAGACCCCGCGGAAGATGTCAT
CAACACTCAGTGTGGCTATGATGCCAGGCAGAAACCAGAAGTTGACCAACAGATTGTAATCTACACAAAAGGCTGTGTGCCCCAGTT
TGAGAAGTGGCTACAGGACAATTTAACCATCGTGGCTGGTATTTTCATAGGCATTGCATTGCTACAGATTTTTGGGATATGCCTGGC
ACAGAATTTGGTGAGTGACAATTGAAGCTGTCAGGGCTAGCTGGTAGACCCCTGCGACCACTGCTGTCAGACACTGGACTGATCCCCC
CTCACTGACTGACCCTCTGTGCGCCGGCCGGCTGATGGCGGGCTGTAGAGGCCTGGTCACAGGCCTACAGTCTCACCTGATGGAGCT
GCCAAGAACTTAACTTTCTGTGGGGGTAGAACTGGGAAATGCTGCTCACAGACGGAACTTGGAAAAAGAAAAACAAAACAAGTGCGC
AACTTGCTGAGTCAACGTGCAGTGAATCTCTACTGTAGCCGTGGATTGGTGGTCGGATGGATGCTACCAGAAGGGGGAAGGGGGGAG
GGTGTCTTGAAATCTCTCAAACCCTTTGCATCTGTCCTCCAGAGAAGAAACCAGCTGCCCCCTGTGTTCAGATTCTGAGAGAGGTGG
AGCGGGTCACGATACAGCATGCGCCCTTCATCCTGAGTTGTCCCTTAAGAGAACTGGGCGTTTGCAGTAAACTAGGGTGCCAGAGGC
TGAGCTCCACATTTCCACAGGACTGCTCTTTCCTCTGAGTGGCCAGCCCGAGCCTGAGCTCTGTCAATGACATCCAAGGAGAAAATG
AGGTTAATGAGAGACATTAATTAAACACTCCCTCACCCCACCGCACCAAACCAGTTGGGTTCTTCTGATATTCTGGAATACTCTGGG
CTATGTTTTATGTTTTATTTCTTTTTTAATCGGTTGTTATTTTGGTTCTTTTTTTTTTTCTTTCTTTCTTTTTTCTTTTTGTCTCCC
AAAGGAGGCTATGACACAGTTCTCTTGGTTTCTTATTCAGCTTGAATAGTAAGTTTTTGTAGACTTGTCACCTGCATGTACTGTAAC
CCCACCCCCACCCCCAAATCCACCATGTTCTTTTCAGAGAGACATTTGAGCGTGTTCCTTAGTGTTTATGTCCTAGGTATCCAGCCT
TGAGTGGTTGTGGAGTGTTGTCCTAGCCGCATCTTTGTATGCCCCTCAGGCTCTCTGTCTCTCCTGGCATCCAATTCAGCTTCTGTC
CATTCGCCATTGAGTGGTAGAAAAGCCCAGCTCCAAAGCAGGGTTGACACAGTCAAAAAGCATTTCATGAGGTTTTTCTTTCTTTTC
TTTTTCCTCCCTTTTTGCAACTTTAGTTTTGTCGTATATTAAATAAAAAAACCAAGCCTAGTATAGTGCATGTGCTCGCTGAGGTGAC
CTCGGGGTGGTTACAAAGCACAAATATTTGAGGGGCTGCGCCGAAGCCCAGCCAGGGTTCAGTGACGCTAGGTGGAAAACCACCTTC
AGGTTCTTCTCCTGCCCGTGCCCACTGGGAGGGGTGGACTTTAGCCCCAGATCCCCCCACCCCCAGCTTGTGATAACCCCTGGATGT
TGCTTTCACGGGGAGCAGATTTAGTTTGTGGGTTTGCTGGGAAGGCCCCAGCCTCTGCGAAAAGCAGCTCCCCATTGGCCAGCCAGC
AGTCTGCGTCATGCAGCCCGGTTTTCCATTTCCGCAGCACGTGTGCGTTGCCGTGGCTGATGGGCGCTGTCTGCTCAGAGGGAGGTC
AGGCACTCCTTGGGTTTACGTGGCTCTGCATGTTTGTATCGGTGTCTGTTTTTAGGTAGAGATAGTCATTCCATACGTGGATGCGAG
GTCACGTGACTTCACCCCTGTCCATACCACAGCCGTCGCTTGTGATGTCCTTCAACATGGACACTCGTGCTCTCGGATTGGCACTTC
TGTCTTCTTTATTACCTCATTCTCCAGTGAACTGCATCTGGCCAATTGATTCAGAATCAGACAGGATCCCCACCCCGTGGCACATCC
AGTGAAAGCCAAAACACAAGCCCAAGCGAGTTTTAAATTTTAATCACCCTTTATTTTTTACACTCGAGAGTGATTGTAATAAAAGCT
GTCATTAATAAACTCGGTTCTACCT
```

160

```
MOUSE PROTEIN SEQUENCE : mP28-002.1 (Seq ID No: 51)
MSGKHYKGPEVSCCIKYFIFGFNVIFWPLGITFLGIGLWAWNEKGVLSNISSITDLGGFDPVWLFLVVGGVMFILGFAGCIGALREN
TFLLKFFSVFLGIIFFLELTAGVLAFVFPKDWIKDQLYFFINNNIRAYRDDIDLQNLIDFTQEYWQCCGAFGADDWNLNIYFNCTDSN
ASRERCGVPFSCCTKDPAEDVINTQCGYDARQKPEVDQQIVIYTKGCVPQFEKWLQDNLTIVAGIFIGIALLQIFGICLAQNLVSDI
EAVRASW*


MOUSE PANTHER CLASSIFICATIONS
        FAMILY (SUBFAMILY)
                TETRASPANIN-RELATED(TETRASPANIN TSPAN-5)
        BIOLOGICAL PROCESS
                Cell adhesion(2.29.00.00.00)
        MOLECULAR FUNCTIONS                          .
                Cell adhesion molecule(1.05.00.00.00) > Other cell adhesion
molecule(1.05.99.00.00)


MOUSE GENE ONTOLOGY
                No Gene Ontology
MOUSE PROTEIN DOMAINS (INTERPRO SIGNATURES)
                IPR000301 (TMFOUR)
                IPR000301 (TMFOUR)
                IPR000301 (transmembrane4)
                IPR000301 (transmembrane4)
                IPR000301 (TM4 2)
                IPR000301 (TM4 2)


HUMAN GENOMIC SEQUENCE : hD28-002 (Seq ID No: 52)
CGCACGGACGCGGGCGCTCGCTTTGTGTTCGGGGCTAGCGTCGGCGAGGCTTGAGCTTGCAGCGCGCGGCTTCCCTGCTTTCTCGCG
GCCACCCCGGCTCCGGCGGCCTCGGCGCGCGAGGGGCTGGAGGTGCGGGAGCCGCTCTCCGCCGGTCGGTCCCCGCGCGGCTGAGCC
CAGGCCGCCAGCGCCGCGGCCCCGTGCGGTGTCCCTGAGCTCCTGCTCCCCGCCGGGCTGCTCCGAGCAACGGTGCTTCGGAGCTCC
AAACTCGGGCTGCCGGGGCAAGTGTCTTCATGAACCCAGAGGATGTCCGGGAAGCACTACAAGGGTCCTGAAGTCAGTTGTTGCATC
AAATACTTCATATTTGGCTTCAATGTCATATTTTGGCTAAGTTGGAGCGCTCCTGGGATTCCAACTATTGTGGCCGATCGATTCGCG
ACGATTTCCCCCACGTTGTTCGTTGCCTTTACTTTTCGCAGCCCCGCAGGCGCTTGCCGGAGCAGCATAGAGGCATTCGCCTTCCTC
CTTTCCAGCTTGCGCGTTGGACGAGATAAACATTTAATCCTTTCGAGGAATGGGAAGAGGTGAAAAGAGAAATCGAGGCGGAAAGGGG
GCACCGAGGCCTTCTGGTGGCTTTTTGAGGAACGAATAGCAGGGATGCAGATAAAAGAAAGGGTTCGGCAGTTTGAGAAATGAGCAA
CCACACGAATTGGATTTGCTCTCGGTGGGATATAGATGTTGGGCAGAGCGTGGTGTCATAATAGGGAAGGCTAATCGGGCACGGCCG
ACCCTGAGGTCCACCTTCTAACAACGATCTGATTGGACGAGGGCTTGGCTCCGTGTTGCCGCGGCTGGTCTGTGCCATGCTCAGCGT
GTATCTTCTTGCACCATCTCGGGCTTTGTGTAGGATGCAGATGCCGTGGTATATGGTCCTGTAATTGCACGTAAATAACGTTTCCTG
CACGCTCTCCTCTTCAGCTGGAAAGCGCTCCCTGTGCGTAATGGACTGGTCAGTGGGGGTGCGGGGTAGAGGGATTTATTTGTATGT
ATATATCATCGTCAGGATAATAAGTCACCGTCAACATATATTTCAGTGTGTTCCTGCCCCACGTCCTCATTTTTAAAGGAGTGACAC
AGAGCGGGCCGTTTGGGGGGAACCTTTGTAGAAATGCAAAGGAGAAGGTGTGCGAGGCATGTTTTATTGGGGAACCGAGAGAGCAAAT
GCTTTAACACAAGATTCAAATGATGCTCTGCTTGGGACTGCAATGTGTTGGGAACCTATTGTAATTAGGCTGGGACGCCTACTCTTT
TTGTTTGTTTGTTTGTTTTGATTCTTTTCCTTGATCTGGCAAACGTGTTCAGCAGCCAGACAAAGAGTTCTCCAGGACAAGGGGAAA
ACGTTGGTTCAAAGACCATGTTATTTTTCTTCCCCTTCTCATCCCATCTCATCCTCTTCCTCAGTGGGCACCCGCATTTCCATAGAA
AGTAAAGATGGTGGCTGTCTGGGTAGGGGGTTCTCTAAGTTGCTGTTCAGTGTGAGGTAAACAATGCTCCAACTGCAGGATCAATAA
TTATCCACAGCAGTCTGAACACGGAATGTATTTATTTGAATTCTCCTTTAATTTGGGCTGTTTGCTGTACGTATATACATCTGTGAG
AATAGTTGTCAATACGGCTCTTCATATTCTTTTTTTGTACCGCACTCTTACCAGGTTATTTTATTTTCTGTTTTCTATTATAATACC
TTGCATTGGTCATGTATGTTTTGTGAGGGGAAATGCAAGGGATTGGAGACCACTTTCCGTGTCAGCATGTCATCTGTCTTCAGGACT
AATAAAGATAATGAGGGGACACATGAATGGATGTCATATCCGATCATTCCACAGGCAGTTTTTTGCCAGTGTTGTGCATGATTCCCG
GGAGCAGTCACCCACCTCCCTGCAATTAAACACTGCTACAAGTACAGGAAGAAGGAATTATTTTCTTTCTTTGCCCCTTCTCCATTT
GTGAAAATGTTTAAAAGTGGTGGGTAGCCACGGTTTTGAGTAAGATATTCTGATTTGATTGGAACTTGAAACTTTGTGACTTGGAGA
GGCACGGACTACCGAACTGGGACTGGTTCTTTTTGGGGTAGTGAGTTCAGGCCTCTTGTTTCTACTTGAACATTTCTAAAAGTTGCA
CATTTTAGCTACCCAGCAACAAAAAGCACCCAGGGACTTTCTCTGCCGTTTCCTAGCAGGGGGTGGTAAGGAAATAAAAGGATTTGG
GTCTGTTTCCCTGACAGTTTGCTGTAATTCTGTGGAGTGCATAGAGACAAATTTAGCCCTTTCTATGGATTAGGTTGCAGCAAAACC
CCTGGAATTGTAGAGAGCTTCAAAGAATTGCAGAGTGCTTGCTTGGAGAGGTCCAGAACAGAGCCGCATGTTTTCTGATTGTGAAGG
TCTTCATAGGTGGGTGGGTGAGTATGGGAAGCCTGAAAGGAGACCTAGGCTATGGGACTAAACTAGCTGCCCCAGGCAGAATTGTTA
AGTGAGTGCCTTCCTTTATGAGGGCCCTTCACTTACCCTGCCTCTTGCCTTCCCTTCCTCTCCTCCTCTGTGCCCAAAGCCTTCCTCTC
AGATACTCCCTCATACCTTTCCTATCCTGCTCTCTGCCTCAAAATTACCCCATATTTAAATTTCCTGCTTTATTGTACCATGGTCTC
TAGTGATGATCAACAGAAAGGCAACCATATGGTTTTGGTTAAAGTAATTCAGACTAAGATGTTAGTGACTGAAAAGCTGGGGTCAGT
AGCTTGGCAGCTTGCTTCTGAAAAGTTACACACCTCTGCTCTCTACAGAGTCACCAACCTGTCTGTGATTGCCAAAAACAGCAGACT
CGGTGCTACTCCCATACAATTATGAAGAAGAGATTGCCAACAGCATGGTTTCCTAATTTTCATTTTCACCACTGAACTGCTAGACTAC
ATTTGACATACACTGGTGACATTCAAAGGTATAGTTCTGGTAAAATAAAATTGAACATATGGTGGCACCAGCACTGAGAGCTTGGTT
CTTTTTCCTGATCAGCAGTTTGGCTCTTCATCAGTTAACTGCCTGGGCCTCAGTTTCTCAGCTGTTAAATTGAAGGAGGTGGATGAGT
TATAACGTTCTTTCTAGTTCTTACACAGAATGAGTTTCTTGAGTTCCAATATGCTGGAGAAGAAAAATAGAGAGTTTGGCCACTAA
TTTATAACAGAAGTAGTATATACCAGGACACGTGATAAATTATAGACATTTTCTGTTAGGGAGACTTGTCTGAAGACTAGTTTTATT
ACTTTCATTTCTTCCTCAAAGATCCTTTCATAAAAAACAAACAAACAAAAAACAAAAAACAAAAACAAAAAAAAAACAAGGCTGGGC
GCGGTGGCTCACGCCTGCGATCCCAGCACTTTGGGAGGCTGAGGCACAAGTTCAGGAAATCAAGACCATCCTGGCCAA
CATGGTGAAACTCTGTCCCTACTGAAATACAAAAAACTAGCCAGGTGTGTTGGCGGGTGCCTGTAGTCCCAGCTACTCGGGAGGCTG
AGGCAGGGGAATCACTTGAACCCGGGAGGTGGAGATTGCAGTGAGGCAAGATCACACCACTGTACTCCAGCCTGGCAACAGAGCAAG
ACTCCGTCTCAAAAAAAACAAAACAAAGCAAAACAAAAAACCTTTCACTAATTTCTTCTTGGCTTGTTCAGTTCCTGGTATAGTGTC
CGTGCTTCTCTCTCCCCACTGTGAATCTCAAATGTAGTCCTGTGAGTCATTAAATATATTTTTATTATTAGAATTACAGCTTCATGT
ATAAAATCTGAATCCTTTTATATAACAGTTGTAATGACATTCTGAGTAACAAAGTCAACATCTTGTACAGATTCCTTTCTCATTAGT
TATCACAGGCTGGTGGGGGAAAACTTGTTTCCTCTGTTATATATTGGACAATTCAAGACAGAAGGGAATTGATTTTATTGAGGGTG
TCACCGTACTGGGACATTTCTCAGAAAACCCTGAGACTGCTTTGGGTTTGGGGGAGGGAGAAGAAAGAGGATTTTGTTTTAAAGTCA
TTTCTTTGGGGTACCGAACACCACATCCCATCACTCATCATTAATAGATGTTTTGCCCTTTTGGATTCTGGCTTTTAACTGTTTTGG
ATTCTGGATACTTAACTGTTCCCAGGCAAGTAAATTGATCTCAATATTCTGGTTATTTATTGCTACATAACCTCCAAGCTTAGTTGT
TTAAAAACACAGTTGTTTATTATTATTATAGTTCACAGTTCTGTGATTTGGCCAGGCTCATCTGGACAGTTCTCACTTGGGGTCTCATACA
```

EP 2 204 376 A2

```
GTTGTGGTGAGATGTAGTTGGAGTTGGACTTACCGGAAGGCCCTCTGGGCAGGATGCCTGAGATGGTATACTCATATGACTGGAAGC
TGATGCTTGCTGGGAGCTTAGCCAGAGTTGTCACTGGAGCACCTACCTACACGTGGCTTCTTCCTGTCTCTAAGTCTTAAAGAAGCT
TCCAGCCAGTTAAGGTTCCACTTAGAACTGGCAGTGCCACTTTTATCTATTCCATTGGTTAGAGCACTCATGCAGACTGCTGAATTT
CAAAGGGTGAAGAATTAGAAGCCACCTTTTGATGGAGAAGTGTCAAGGTCACATTGCAGAAAGTCATGTAGGTTGTGGCCATCTTTG
AAAAATATAGTCTACTGTATTAAGGCACTGAGCCTTAATTTCTTGTTTATAAAGGAAAGATAACATTAATGGTGTTGTAGTTTTAAA
GATTCAGTGAGATAATGTTTTATTCGGCATAATATCTCAATATTTAACAAATAGTGAATATCATTACTGTTGTTGTCTCCAGATTTT
ATACTGGTTCCATACAGGGCTATAGAACTTCTAGAATGCCTTAGTCTAGTACTTTGGCATCATTACTACTAGGTAATGATAATACCC
TTAAGATCTGCCAAGAAGATGAGGCAAATGGATTCAATAGATTTTATAGTGACTTGTTAGGAAAGAACTCAATGGAAACGGTGGAAT
GATTTGGGAGAACCAAGTCATTTTTCTGTTTTCTTTGCTACAAAGGATTAGAAAAGCCTCAGTTACTTGAGGCCAACTTTCTCTTTT
GATATGCTTTCAGATATTACCTTGTGTCTTTAAGTGCAAGTTTATCCAGGGTTGATGGCAGGATCTTGCCTCTAATTTGCTTCCACA
GGGCTTAATTCAGAGAGGAGGGGAGACTCAGGAAACCTTGGGGCGGTGGGGGAGGGGGGGCATCAAGTAGGAAGCTTTATCTTATGAT
TCACTATAAATTGCAAGATGTTCTGGAATGATGATACATCAGCAGTTTATAAGTCATAATTTTTGTTTTAATTTGCTAGACATCTGT
TAGCTTCGATGGCAACCATATGGGAATCTAAATTGCGTTTTGAATTACAGGGAGATGGTAGGAAGAAAGCTAAATATGGTTATGAAA
GTGTCATATGGGAAAGTGTAAGAAAGGTATAAAGTGGGGAAAGTAATTGAATATAGGCTTGCTTAGTGTGGCCACTCCCTCTGTGG
GGCAGTATGAAGGCGCAGAAGCATTCTCACTTGATATACTCCGCAGATTGAAATGAAGCCAAGTGGGGAGAAGTTGGAAAACCAAAT
CATGTTTCAACCACCATAAACTTGCCAAAATGTTGCACTCATTTAGATGGGTCACTTTGTTTTAATGTCCACAGTAATAGGTAGTCC
CTGGCAAAAGGTGAAAGCAATCTGCATTTTTAACAGAACTTTTCACTAATGATGTTTTTCTTGTAAGCACCCCAAAGAGTCTTCCAA
GAATATTATATCTTTGTGACAGATGAAGAAATTGGAGTACAGAGATGTGGAGTAACTTTTGAGGTGTTGAAGAGCATGTCAAGGTTC
AGTTTCAGAGTGTTAAGTCTCTTCCGTAATGATAGCCCCAGCTTTTTGGGTGGAGACTTATTTTAGAAGATGCTTGTTATTCTAAAA
TAAAACATCAAGTAAGTCATTTACATCTGTTCATAATCGAAAAGTGATAAAGTTTTTGCATATGCCACAATTAACACCACCCACAAG
CACAAAAAAGTTAGGATGAAAATGTTCTTCAAGAATATGGTGGAATAATTGTAGCCTATTGAAAAATTCATAAATGAAAATTGGAGTA
TTGTAGACAGCATTCTTGAATCACATGTTTGTTTCTGTGTGTAATACTGATGACTGTCTTAAAATTTCTAACTTCCAAATAAATCAT
ACTCCACTGGATTATAAGGGCTTCAAGTGTGGGGGGTCGTGTCTCACTTTCATAAGCCCAGTGTCTTGCGCATTGTCTGAAACACAGTAG
ATGTTTTGATAAACGTTTGGGCCAGGCTTGGGGGCTTACACCTGTAATCCCAACACTTTAGGAGGCTGAGGCTGGAGGATGGCTTGT
GCCCAAGAGTTTGAGACCAGCCTGGGTAACATAGTGAGACCCTATCTCTACAAAAAAAAAATTAAAAATTAAAAATTAGCTGGGTGTG
GTGATGCGTGCCTGTAGTCCCAGCCACTTGGGAGGCTGAGGTGGGAGGATCACTTGAGCCTGGGAGGTTGAGGCTGCAACTGCACTC
CAGGGTGACAGAACAAGACCCTGTCTCAAAAAAAAAAAAAAAAAAAAAAAGTATGTTGAAGGAAAGAATTACAAAATCATCAGA
AAGTACGTAGGAATCATCTCCTCTAACTTCCTAGTCAGTGAAGCATTCTCCATTGTATCGTGCCTACCTTTCTAAGCATGGATCTGT
TTTTGGACCACTTTAATTGTAGAGTTTCATCTGTTCAAACTAGATGTTATCTGTCTCCCCATCATTTCCATCCTCCTAATTTACTGT
TCATTTAAAATTTTTATTTGGAAACATTAAACCAATAGGAAAATTGCAAGTAGTATAGAGAACCTGTTTTTATCCTGTTTGAGAAAA
AGTTGCCAGCATGTTGGCTATCACACAAACAGTTGAGTATGTATTTTCTGTAAACTGGACTATTCTCCTAGATCACCACAGTACAAC
CATGAAAGCCAGGAAATTACCAATGATACATTAACACCATTTAATCCTTAGATTTCATCCAAGTTTCCCAGTTGCTCCCATAATGTT
TTAAAGCAAAAGAATTCAGGTCAGAACCACACTTTGTATTTAGTTACATGTTTTTAGTCTCTTTCAGTCCTTCACTATCATGCTCTT
GATACTTTTGACAATTATAAGCCAGTTATTTTGCAGAATGTCCCTCAGTTTGTGTTTGTGCCATTTTCACAAGCTTAGATTCAGGCT
GTGCATCTTTGGTAGGAATAGTACAGAAATGGTGCTGCGTTCTCATTGTACCCTATCAGGGTGGCATTGATTTCTGTTTGTCCCATT
ACTGATGATGGTCACTTGATCAAGGTGTTGTCTGCCAGGCTTCTCTACCATATAGTTACTCTTTTCTCATTGTAATTTATAAATGT
TTTATGGCAAATTACATTGAAACCATGTAAATAGCCATTATGTCATTCCTAAACATTCTGTTTTCCACCTTTAAAAGCTCCTAGTTCCTCC
ATGTGTTTACACTAATGATGTTTTTTCTTGTAAGCATCTCAAAGAGTCTTCCAAACATATTATATCTTTGTGACAGATGAAGAAATTG
GAGTACAGAGATGTGGAGTAACTTTTGAGATGTTGAAGAGCATGTCAGGGTTCGGTTTTAGAGTGTTAGGTCTACATATACTGTTTC
CAGATTGTTCTTTGCCCTGGTCACGGTGCTCTGCCTAGGGTCCCATTTGGACACACCTCTATTAATGCAGCAACCAGAATGAAACAC
GTTGTTCACAGGCTTTTCTAACCATCCGACAGGACGCAGCAGGCTCTTGAACTCTCCCTTCAGTCCCTAGGCTGTGCTGTGCCTGGTGGC
AGAAGCCTCTGCTACTGTCTGGGAATTGACTTTGAGTAAGTTACTTTCACAGATTAGTTCCTCAGTTGCTCATCTAAAAAAACCAGGA
AAATAATTTTCTGCAAAGTTGTTATCCAGTATTATGTGAGATCATGCATATCAACGTGCTTCATAAATGGTAAATCTCTAAATACAA
ATACTAGTTATTAACCCAGGCTAAACTGCACTTGCTTTCCCTGGCAGCCAACGAACACTTTGGCTTGGATTGACTTTTTAGTGAAAT
AAAACCTTCACTTTTTCAAGTGAACTCTATTAAACCATCCCCCTAAGCTATATGCTAATCAGAAGCCCTAACCCTCCAGTTAATTAT
TTGAATTTAGATTTAGTTCTGTTTATTTCTCCCATTTAAATGTCTTATTCTGGCCCATTGGATTTCAAACCTGCTACCAGCAATCCT
AGGCCTCCTTGAGGCGTATGTGGGTGGGTGGGTCTGTGTCTGGTGGGGTGTGGACTGAGCAGTACCTCTCTTTCCTCCAGAAAAGCTT
GGTTATTAGTTTGATATTTTGGGATACTAAGAAAGATTTTGCTTGAAAAAAGGGGTCCCACCACTTAAGAAAGAAAAGAACCAGTAT
TCTAGCCTTTAGATGTCTTTGAATCTTGATGGCCAAAGTACATTTTTAAAAAAAATTACTCCTCGTGTGGGTTTTTATCTCTTGGAAT
CTCTCCATGCATGGGAAGTCCCTCTCACTAACTAGGGGTCACTTCTGTAGGATTAGTTGATATTTCACCCCTTTATGCATGTAGTGT
GGCAGCCTGATTAGTAAGGAAATTTCAGTCTTCCCACTGGGAGATCTGGTTCAGTAGGTCCATCCAGGATGGGGTCCTGGAATTTCT
GTTAAGAAAAAAATCAAAACTTCCTGCAGGTCATTGTGATGCCCAGCTGAGTTTGAGAAGCTTTAGCTTGTTTTTCTTGGGAAGATG
TGGCCTCTCCTTCCCGTTGCTCCCTGGAGGGGATGTGTGGAGAGCTGGGCGTGGGTGGTGTAGAGCGGTGACCATTTCACCAGCAC
ATGAAGCTAGTGTTCTTTGCTTACGAGAAAAAAAAGGTGGCCTATTTTATCCCTCATGGAATTTTTTATTAATCCAAAAACGCCAGT
TTATTTTTAAACCTGAAAGCCCTAAAAACAAAATGAGAAAAATCTCTTGAAAGAGAACCTCTGGTGCTGCAACTAGTTTGCTAGCAG
TTAGCATCATATAGGGCTAGAAGCTAGCCATTTATTCGATAGGCACAGTGAAGAGCTAGACTTCTGGTTCCTTTGCCCTGCTTGTCC
TGGACCACTCTGCGTGTGTCAGTTGAGTGAGTCTAACTACAGACACTTAACAAGGGCAGACCACCTTCTCCTTCAAAAAAAAA
TCAGCCTCCCCATCCACTGTTGCTGTTCCTTTCAGCCTCCCTTTTCTGGGGCTTTCTCGTTGGCCTCCAGCCCTGACTATGCTCAGT
TTGTAGGAGGGGAGTTGCTTTCTGCGTCAAGGTCTGCATGACAGGTTCATTGAATTCCCTCTGCTGTAATGCTGTGGAGGGGACATT
TCTTTTCCCATATCCAAACTTGCAGCACTGTCACAAAGACAGTTGACAATAAAGCAAATACAGAAGACCCAGACATGATATTTGCCA
GTGTTCTTTAGAAAGCTTCAATGTGGAATATCCGGGTTATGCCTAGTGTTTTCCTGTTATTTTCTTAACTTTCTTTTTTCCTTCACC
CCCTTTGAAAGTTAGATCTTTGATTACGGCTGGGTTCCTAAAGTAGAAATCTTGAAAAATAACATTTTGAATAAAACTCTGTATTAT
TTCTCTGAGCTGTTTTACTGGGTCTAACACCACTCCTTTTTTTTTTTTTTAAACAAAACAAAACAGAAAAGCCAACCAAACAAAACC
AAACCAGAATGGTATGCAAACCCAATGGTTAGCACTGTGATATTATCCATATGTACATAGAGGATTGTTAGTTATGAATCACAAGGT
ATAATTTTTAAGTAATATAATCACTAAGCCCAATTACAAAATGGTGTTTTTGTGGATGGCAAAACATCTACAAATGGCAAAACATTT
TGGCTCTCTTCATTGGAAGGGAAAAGTGATGTTCTATACCACTTCCAGCTACTATTTACTATTTTGATAAGTATTAGAAGGCATAGG
```

162

```
GCTCTTTTTACTTGTTGGAAAATTTTTCTAAATTACACTTGGCACTAAGGCTTGAATTTCTAAAAATCTGGAGGGAACAGCTGGTGC
TCTAATAAATATTCAGGCCAGCTATTTTAATGCATTGTACTCAATTAAGTAGTCCTAATAAAAGAGGAATACTTGACTTAAGCATTT
CTCAAAGCATAATGTTGTGCTTCCAACAAAAATTTCCTCCAACTGTTGATATTTCCCCCCAAGCAGCCTTAACATTTAATTCTGCCA
AGTAAGTAATCTAATCATGGATGATGCATTTAATGCCAAAGTTAGTTCTCTTTTTAAGGTATAGTTGTATCAATGTGGCCTAGCCCT
TTAACTGGAGACTCCATAGGTCACTGACCTTTTAGAAGAATTCTTCGTCTGGTTCAGAGTGAGAGAGTATTGAAAGATTCATCCTAA
CTTGGGCATTCTGGTACCCTTGTGGTGGGGAGGGCAGAAAGTCTGCTGTTAAGGAGTTTAGACTTTGAGTGTGTCTGGATTTTTACCC
AGCTATGTTCCCAAGCTTGTGACCTTAGACGTTACTTGCCCACCTTGCAGTTAAGTCTCCCCTTCATCTGCAAAATGGCACGTTTAG
TAGTATTAATATTATTAATAGTAGTATTAAGATAACGTCTATCTCGGAATCCTGAGGATTTGAGGAGTTAATATGTGTAAACTGCTT
GGAATAGTCAATAGGTATTAGCAATTATTATACTGTTTTCCTCATAATTTATTTTGATTGTTTTCCCTTCTTTCAGAATCATTAAAA
TAATCTTACATTGTAACATATATTTTTTGTTCTCTGTAATATTCTGCCTACATAAGTGCATTCTCAAAATAACTTAAAAGGAAAAAA
AGATCTAACAAATGTTCACAGTGTTCTTTTCATTGTAATGAGTGGCACGCAGTTCTTATCGTGGATACCAGACCATGAATTCGCTTC
TATTGTTTGTTGTTAATTTTCCCTTTTTTCTTTACCCAGTTACATTCACTATTTTCTTTGCAAAATCAAAGTGTCTCCTATTAGTAT
TTTGTACTGGGAAGTTGAGTTTCTGATGGATAGCTGTGGTGGTGTTAGGCCTAACACCTGGGTGGGGTGGGGAGGATAGATACCCTT
TGTCAACATCTGTTAAATCTGGGCATTCTGGAACTATCTAGATGGCTGTAACTAAGTGTCTTTAGTTCTTTATGGTATCATAAATCC
TGCATATTTTGGTCATTTTTTTCCTCTATTTCAGACAAATTCTTGAGGTCATGAAGGTGAAACTTGAGTCAGTGATCCTGATAACTT
GGGTTTTCCCCTTATATGTCCTTCCAGCTGATGCAGAGCTAGGATTTTGGATTGAACTACCTGGATCATTGACTAATCATCTTGGTG
AATGAGAGACTCACTGGCTTCTCCTCTAGGGCATAATGGGATGCGATGAGAGCTTGTGCTTTTTTTTTTTGCCACCTGTACTGTCCCC
TGACACAGGAGACATGGGCTGTCACATTTCCAAGTTGCCTCTTCCATATAACTGGGCTCAGCTCTGAGAGGAAGGCCAGGGCGTGCT
AAATTAGTAGGTGCCTGTCACTCATTCCCATGCAGAAGGCATGCTGAAAACCCCAGCCCTTCAAGGGGAAGCAGGGGATAAAGGGAT
AAGTCTAAGTAGAATTTTTACTGTAGTTTTTATTAGTAAAAAAGAATGATATATGTAGCACAACAGGAACAGAAGCGTATTTAAAGT
CATCTCTAGCTTACCAGAAACACCCCTAAAACTGCTGGTTTAGATTCATTAATCAACCTCATGGTCCTCAGATGGGTTGTGACTCCC
TGTTGCGTAAACACGAACCATGTTACTTCCTGGTCCTCCTGACCTCCACTAGCTGATTTCAGCGAGGATCAGGAGTTAGGTCGCCAG
CTGATCTAAAGCAGCGAGTCCCAAAGTTCAGCCATTTGTAAACCACCTGCATGGTTTGGACCAACTCTTGTGTCACCCCATAGTTTT
CTCAGATTTTAATATGCATACAAGTGGCATGGGATATGTTGTTCAAATGCGGATTCTGGTTCAGTAGGAGTGAGAGGGGACAAAGAT
CCAGTACCTTTAACAAATGCCCAGGTGATGCTGATGGCTCTGGCCCACTGATCTCACTTTGAATCTCAAAGATATAGTCCATCTGAA
ACAATCTTTTCTTTTAAATTGACTCTGATTTTAATCTTAAATACATATATTTAAAAAGGAATGTAAAAAGTATCACTATTCAAATGC
ATATAATGACAATAATATGACTTACTAACTTCTAGGTAGCTATTATTGGTGCCTGTTGGTAAGCCTGAGTCAGGAGTCCTCTTTTCTT
TTGTTATAATGGAGGTTAACAAGTGTAGCAAGTGTTCGACATTCTGGCATCAAATGGAGACTTTCTCCTTAACTAGACCAGAGGATG
AAAAGATAGTTGAAAACCAGTGACTTTATATTGAAGGATATTTAATGCCACGTTCTAAAACTTCTCTTCTGGTACCCCAAGTCACCA
GGGAATCGCACTTTGGGGAACACTTGTGCTTAAGTAATTTGCAGCAAGGCATGCATTTATAAACTGAAATCTATTAGTGAGGTGGAT
GGATCCCTAGAGGTAAAATGCTTGGGGAATGTGTTTAAATGTTTGCATACTCCATGTTCTGTTTTCTGTTGAGAGTGTGGGAGAGAA
GAGGGGCATCCCCCATTTCTGAAAAGTTGCTAACCCTAGGCCCAGTGAGTAGTTACATCTTAGGTATGAGAAGGAAAATGCTTTGGG
AAATTGTATCTCAGGCCAGCAGGAAGTCAACTTTAAATGCTTAGGAACTGTATCTCAGTGGGCTTCCTCCTCTCCTCTTTCAAGTTA
CTATAGGTATTGCTTATGGTCAGCTTTGGAATGAGTCTATTTACATAATGCATTTTAAAAATAGGGTGCTTTTCATTGTTCAATGAC
GAGTTAGTGGGTGCAGCGCACCAGCATGTCACATGTATACATATGTAACCTGCACATTGTGCACATGTACCCTAAAACTTAAAGTAT
AATAATTTAAAAAAAAAAGGGTGCTTTTTTATTCCAAAAATACGGGAACATCAATATTGCTTTTTTAAAGGAAATTTACTACAATAA
TATTTTCTTTCGGTTCAACTGTATTAAAAAGTGGAAACTCTTCGTCAGGTGCTCCAGAACAGTGAACAAACACTTCAAATGAAAAAA
ATATTTTAGAGTTTCAGTTGTATTTCCCTATGAGGCCTTGAATTTAGCAAACCTTAGTTAATTAACTTTTTAAAGGAAAAATGTGCT
CTTACATTAGTGGCTTTTAAGCATTTCTTCCGACTTTACCGGCCTTTCCCCCTTAGGACAACATTTAAAATAAGTGTTTTCAACAAA
CAGAGCAAATACCTTGTGAATAAACATCAATTTCACACAACCAGAAAAAAAAATAAATCAATAAATCTCCTTTAGGCATTTGAAAA
ACTCTATACTTTTTAAAAAGGGAGGAGTTGTAATTCAGACAGGAAAACTAAACCCCAAGAAACCAGGCTGTTAGTTTTTAGATGTT
TTTCAGCATGACCTTATGGAGGCAGAAGACGGGCTGGGAACTTCCTGAGCACCTGTTTCGTTATTGGTGCCCTACTTATCCAAGGAT
GGGCAACAACTGCCCACAGGGCAACTCCTGTGAGAAAGCCTACTCTGTTTTTGTTTTTAATCTCATAACTGGAATCCTAGGGAAAAT
AGTGTTTTCTTTGGTACCAAGTTCTTTTGGCTTCATCTATTGGGTTTTGAAAGAATAAACTGAATGAAGAGCTGTCTAGATGATACA
ACTATATGTGGCATTTTCATATTTTTTCCTGACCCTGCAAGATTAATAATCCCTAATATTCTCACTATATGCTTTGAGAGTTTTGCT
GTCCAGTGAAGATTTGAAACAATTGCACAAAAATTTGAAAACGGTGTTTTACTTGTCATGTTTAAAGATACAGAGTCACTATTTTAG
AAGTGAAGGGATAGGGGTCTCAAAACAAATTGAAATATATTTTGTGAAATATATACATATGTATTCCACTTAAATATAAAATATATA
GTACCAATATATATCCTTGATGCTTATTAAATACTGATCTATATCCTTGGACTTTTTAAAAAAAGTACAGTCATGTATCGCTTAACA
ACAGGGGTATGTTCTGAGAAATGAGTCATGGGATTTCATCATTGTGCAAACATTGTAGAGTGTACAACACAAACATAGACGGTGTGT
AGCCTACTACACACCTAGGCTGTATGGCATAACCTGTTGCTCCTGGACCACAAACTTGTATAGCATGTTATGGTAATGAATACTGTA
GGCATTTATAACACGATGGTATTTGTGTATCTAAACATACACAAGGTACAGTAAAAATATAAAAGATAAAAAAGTGGTACACCAATG
TAGGGCACTTACCACGAATGGAGCTTGCAGGACTGGAAGTTGCTCTGGGTGAGTCAGTGAGTGAGTGGTGAGTGAAAGGGAAGGCCT
AGGACATTACTGTACACAAATGCAGACTACATTAACACTATACTTAGGCTACATTAAATTTACAAAAAATATCTGTCTTCAATAATT
AACCTCAGCTTACTGTCACATTTTATTTTATTAACCTTTTTTTAGCTTTTTTACTCTTTTGTAATAACACTTAGCTTAACAGATTGT
ACAGCTGTACAGAAATATTTTCTTTATATCCTTAGTCTATAAGCCTTTTTTTAAAAATTATTTTTTATCTTTAAAACTTTTTTGTTA
AAAATGAAGACAAAAACACACAATTAGCCTAGACCTATACAGGATCAAAGTCATCAATACATCACTAGACAATAGGAATTTTTCAG
TTTCATTAGAATCTTATGGGACCACAGTTATTCCGTCATTGATTAAAATGTCGTTATGTAGTGTGGGACTGTAGATATGTGCTCTCT
TTGGAGAAAAAAAAAGAGAAACTACCTAAATGACCGTATCACCTCTAAATATTTTCATTCATAAGAGACTAAAATTTCTTTACTGTA
GTATACTATAAATATGCAAACTAAATGAAACTTGTTTTAAAGTTAAAAGCTGGAGATCCATTTTAGAAACTAACACATAAGCAGAAA
AAAAATTTTGTGGTTATTAGTTGGAGGCTAGAAAAGAAACTCTTTGAACTATGAAGTTACTAAGTCTTAGCTAGAGACCCCAACAAA
TGATTAATTATTGCTAAGAATCTTTTAAAATCCAGTTTTAAAGTTTAAGTGTTGTTTCTGTGGAATTTTTCCCTCACCTGCCCCCTT
CTTTCTGTGATTCTAATTCCAAAATTGAGAAGACAGAGCTTTTTACTTTTAGGGAAGCTAAACCTCTTTCTTGCTCTAAGAAGGTTG
ACCTGCTGATGAAAGAACAGAAGGTTGGAATTCTTTTGAGCAGGAATAGCTGTGCAAATGCTTATTTCATTGTTCTTTGGCCAATCT
TTTTAGCACTCAAGTATTAGAACTATGCTTTAATTGGTCTTTTGCAGCCCTGATTGTATAATCTTAGGATTTATATGTTGAAAGAGA
CCTTACAAATAATTTGGGTCCTAGTTTTTACTACTCACTGTTCAACACTTTGCCTACCCTTTTCATAGTGGAATCCTTTGTTTTCCT
AACCAAAATTTTAAGCAAAGACCCCAGTTTATAAAACAAAGAACAGAGCTTCTCTTCTGGGAGCCCAGCATCTCAGGTGCCTCAGCT
GTGTTCTTGGTATCACTGTGTAGGAACAGTGTGCAGAGGGGACAGTGGAGTCCTGGTGGATTTTACCCATGGGTGGAGTTTGAGCT
GAGACTGGTGGAGAAGGCATCTGCTTACCGTTTATATGTTGAATTAGGATGAAGTCGGCACTTGGCCTGTGTTCTTTGTAGGATGTG
TGCACGCTGAATTGTCCAAGTTCCCAAGTCCCAGCACTTTGACTGTATTGGTTCCACTAAATGGTTTTGGCGCAGTCGTTTTGATT
AACATCAACCTTGCTTATGTTAAGTAACTATTCAGGTTATTTATTATGCATTACTCTCATGCTTTGGAATGAGCAGACTCAAAAACT
AGTAAGTTAAGCTTGATGGCATAGGCATTATTTGAAATTCTTTGTCATCTTTAATATTTTTATATTTCATTTAAACAAGTTATTAGG
TGTTTGAAGGTTCTGTGGTGACGAACATAACCTACACATTCACATATGTCCTGTTTGGTTTTTTTGTTACAGAATGCAGGTATCCAT
GGAATTTATTTTATTGTTTTATACTGATGAGCTCCAGATAGTAGGAGATTATATAAAAAGAGGGGATATTTACTTTTTTATTTCTGT
GATTTAACTTTTTCTCCCCTCCTCTTCCCAATGCTAGGCAGAGAATATTACAACATGTAAGACAGTGGTTAAGGGTGAGACTGCCTG
GATTCAAACATGGGCCTTGATATGTCCAAGCTGTGTGACCATGGATGGTTACTTCATCACTTTGAACTACTCCTTTTGGGCAAAAAG
```

```
TGGGTAATATTAGTGGCGACCTCTAGGATCATTGTGAAGATTCCATGAGATGATGTACATCCAACCCTGAGCACTCAGTGAATGCTG
ACGTGATAATTGAGGGACCAGTAGGTGACCCACTCAGGGGGAGTTGCGGGCTTTTGGTTCACTGTCACCTGGGAAAAGGTGTTGTCAG
TGTCCGGACCCTCAACTTTCCTTGTTAGAAAAGTGTACCCTGTATAATAGAACTTGTCACTAATGCCAGGCTTTGTTTCCTTTGTCC
CCCACCCATTGGAGGAGAGTGTATCTCCATGCTTTCCTTTGGCAACTGGATCTGAGGGTTTTCTCATCAGAGCAGCTGCCATGTTCC
TCTCCTCCTGGGCTGTAATTTCCTCTCCTGTGTAGCCATAGCTGTCCACCACGTCCATTCCTGACATGGCCTGGCCAGTCCTTCCTT
TCTGGATAACAACGCCAACATTTCTTATGTCTTACCTCAGGGATCATTTTCAGGCCACAGACCTACCTGTTTTGTTTTGTTTTTTTA
AAATGGTAGTATCTACAGAACATTACAGTTTCCGTAAAATCATGTATAATAAATATGCCTAGAAAACTACCTGAAATATAGCAGCTAT
CCCATAATTACTAATTGAAATACGTTAGTTGAATCTGAAATAAATTTCTTAGCCTAGGAACAAAGTTAGCATCCATCTGGGTCTCTG
CAAAAAATTTGTAATGTGTAAAAATAGGCATGTTTAGGAAGTTGCTTAATTTATTAATATATACTTCATTTTTATGGCAGTTACAGAT
TCTCTAATTGAATTTATATTTGCATGATTACAGTTTTTTTTTTGTTGCAAACGATTTTTATGGGCTGCTGAAGTTTAAGGAGTCTGG
CATTTTAAATTGTTCTTCACTGTCTGAGAAGTAAAAATATGTCTTAAAAAATCATTGATGTTATAGTAAAGGAAAGCTCCCTCCCTT
CCCTAAGTTAGTGCCTTTCAGAATGTGAGCATTTGTCAAGTGGAATTCTGTCTGATCCTTTTCATTTTCTGAGCTATTATAAGTGC
TTTCTTTTCCTTTAACTGTCATAAATGGTTTTTTCTTCCCTTAACTAAGTAAAGATTTCTGGAACAATGTTTTGACCAGATTGAAACC
AGCTTCTGTCCTATCTACTAGTCATTCATTTCTTGATTTCTCGTGCATTTCTAAACATAGATAAGTTTCTAATGGTGAAGAAAGTGA
AACAGAAAGGAAGGAAAGAAGCAAAAGCTGGGTTGGGTCACCATATTGAGCGAAGGATATGCTTGTGGGGAATCCTACACTGTGATA
TTGGAGTAAACAGTTACATTATGGGGTGAGACCAGGTGAGATGGCTTATGCCTGTGATCCCAGCACTTTGAGAAGCTGAGGTGGGAG
GATTGCTTTAGGCCAGGAGTTCAAGACCAGGCTGGGCAACATAGACCCTGTCTGTACAAAAAAAAAAAAAAAAATTAGCCAG
TCACAGTGGGGTGTGCCTGTTTTCTCAGCTACTCTGGATGCTGAGGCAGGAGGATTACTTGAGCCCAGAAGTTCAAGGCTGCAGTAA
GTTATGATTTTGCCACTGCACTCTGGCCTGGGCGACAGAGCAAAACCTTGTCTCAGATTAGATTAGATTAGATTAGAATAGATAGAT
AACATAGATAAGATCAGTGTTTCAAAATCCTTTTCCAGTGAGAGGCATGTACTGCTGCTTTTGTAACCCATGACATTGAGAGTGTAG
CAGGGGGTTCATGTGGAGGAGTTGCTGAGACCAAGAACCCCGTAAGGATAGTCATCTAAGTTTGGGAATACACACAAATTGTTGGGA
GACACTTCTCCATGGGTCTCTTGTGCTCCTGCATGTCTTACTGAGTATACCAAGAACGTTCTTTACCTGGGCCATTCTTCGTGTTG
TGTTTGCAATGAGCAGCCTTGAGGGATGAAGTAGTGTCTCTTTCTGGGACAAAGACTGACCTACTCTCACTTACTGCTTACTATAAA
GCAGCAGGTTCCCAAGCATAGGTTCCTCAGCTGGATGCACGCTCACTGCATGTGCAGCTGCCATCTGGGCCCCTCAACGCTATTTC
CATTGGACTGATGTAAACATGATGCTGGTGTTGTCTGAATCATGCTGTGAATAATAAAGTCCTTTGTCCTTGACCCGGGAATCTCAT
GTCTTCTGCCAGCTTCTATGAAACAGTAACAGGCTAACTTATTACCTTGCGAGTAGGGTAAAATCAAACAAGACCAGATACAGGTGG
AGCTTCCAGATGAGCTGAACATGTGGAGGTTCCTGTAGGGTGGTGCTCTAGAAAGGGCATGGAAGCGCCATGTCCCTTGCCCCATAC
CTTTACCTGTGAATCTCTTCATCTGTATCCATTGTAATTTTTTAAAATAATAAACTAGTAAATGTAAAATAACCAAAACAAAGAAAC
CAAATGCCGGTGTATTACATATAAGTTGATACCTAGCCAGTTAAATGCGTTCAGATGTGTCTTGTTTAGGAGGTAATCACCCGGGCC
TCCCCCACAAAGTATGGTTCAAAGGGTCATGGTGGGTGGCTCCTCAGAGCATCTCAGCTCCCTTAGACCTGATGAAGGCTTCACAGG
CATTTCATATCTGTGCCTCTGGCCAGTATATCTCCAGGGAACTGAGTGCCAAGGGAGACCAAGGCATCTTTACTGACATTGTTGTAC
CTTAAGTGAGGCAACATGTAAAACTGTGACACATGGGAGTGCTTGATTTTTGAGCGTGCGGACAACAGGGTCCAGTGTTATGGTGG
CAGGGGCAGGAGAACTCATGGCATCAGGAGCGTAGACAGCCCCACTGGGCACGAATTATAACCCAGGCAGAGGCAAGGCGCAAGGGT
GGTCAGACATCTGGCCCTGAAATAAGAAGAGACAGGTGGGAAAGTCCAGGCCAAGACCGTGGAGCAGAGTTAAGAAGGAGCAGGAAG
AGCCGATCTCAGAAACAGGCTGGCTCTGTTGAAATTGGACTGAGGCAGATAATTTCTTTCTTTCTTTTTCTTTTCACCTTTCAGCAC
TGTTGGACATGAAGGAAAGATAATTTCTAAGTTTACTTTTTGATATTTATAGATAAAATGCTTTCAAGCAGTTAACCAGCCTATTGG
AGAGTGACACTTAGATCATCTTGAAGTTGGTTTGGGCGATTTAGAGAAACATTTATAATTAAGTTATGCAGTTAATGAAAGCCAAGT
GGGGACATTTGTTCAGAACAATTGTTTTTGTACCTTTTCTTTGTGATAGAGCTGTAAAAGTGTATTTACTTGTAAACTGCAACAAGT
TTTTGCTCCTTCCCCCATCAAAAGAATAATATATCTATATAATAATTAGGCCTGAAATTAAGATTCTAGATTTAAAGAGGACTCCCA
GGGCTCAAATATAGGAGATAAGATGTTGAAACACCACTCCTGTTGTTTGCAAAATGAATATTAATGACATTTAGTCAAAACCAAATT
CTAAATATTTAATAATATATGTGAAAATTATTAACTGCCTGTGGTCATCAGTTGAGACCCATAGTTACTGCTGGAATCCAGGGATACAG
GGATTAAAAAAATATCATGGATCAGAAAGTAGATGTTAATGGGGAAGTATCTGTGGGAGAGTGACCTCTTATCCCTGAGGGCCGCCATG
TACAGCTACACAGGAGGCGAACTGCACAATTCCCTGGAATGTGCGTGATGCTCCCTGGAGTTGTGCAGTGTGGCAGTGCTGTGTGCA
TCAAGGGCAGGAGCAACGGCAGTCCTTCAAAGGCTTAAGTAAGTGGCATAGAAAGTGGACAGTCCTTTTAAGTTGCATGGCTTCAGA
CACTTCTGTTAGTGTGATAACTACTGCCTGTGCCTCTCACTCTTTGAAGAGGAACTTGCTGGTGATGGGGCTTCTCATGGGGAAGCT
GGGAAGCTCCCTACCAAGGGGGAGGGTTCCTAAGCCTGACAAGGACTGCTCATCAGGTGCTTTGGTATCCTGTAGACTGGGCTAAGA
AACCTCACACCAGGGTCCACTCTCCAGCTGGTCTCCACTGGGCCTAGGGAGACCAAAAGACCCTGCAGTTGCTGAACCTCTCTTATTG
TACCTGTGAAGCCAAGTAAACCATTTGTTCACTTATGTTAAAGAATAAGCTTGATCATTCATGTTCAAAATAGTTAACTCTTTTTTG
TGAATTAATAGTCTGGCTATAATTACATTTGACATTTCAGTCCAGTATATTTGTCATCCAGATGAAGAAGCTGCCAGCCGCATTTTT
TTCCAGCAGTTTTACTTGACTTTCTTTTAATTTTATGTACAAGGTGTTATTTAAGTGCACAGATTAATAGAGATAATTTTTATATGT
AAAATTATTTTATTGCATACCTCTATGAGTAATTTCTCACTTTGCCATTGTTATAATACCTCCCATCCTTCCCTAAAGCGCCACTCTC
CCCACTCTACTGCAGGAGATTTCTCTAGCAGCAATTCTGATTATGTCATACTCCCCTGCTTTAAAATAGCTAAATATTCTTAATTTGG
AAAAGATTAAAGCTAGGCACCATTATAGGAACCTTGCCTACTTGTCACCTCTCATATTTTCTCATCCATATTCTGTTCCACAGTCTT
ACCTGCATGGAGCAAAGGTGTAGGGCATATTTGATGTCCATTATAAAATATTCAGGCTCCCTCAGTTCAAGGGTCCTCCACTGTAAA
GCAGTCCACTATGTATGCAGGTGTCAGTCCTTTTTCCACCACCTTGTGGGAATTGGGACTTGGAGAACTGCCACATTATGCCCTTAT
TTACATTTCTTGACTCTGTAGCTTTGCGCTTACCTCTGCTTTAAATGATATTTCTGCCATTTCTGTCTACCTTCCATCAAAGGTATG
GTGTCTACCTTTACAATAAGATTGTAAAGAATAAAGAGGTAATGAGTACAAAGCACTTAGCTCAGGGCTTGATTAGCTGCTGCTGCT
ATTATTGTTACTCTTGCAAAATTGGTTCCAGAATTATACCCTTTGGGTGCTCTTTCCTAAACTTCTTCTATCCCTACCCTATTATTT
AGGGAGACCCTATTTAGCAGTTCAGTCTTTATATATTCCTTATAGCTCTTTGTTGGCAATATTTTTAAATCATAAAGAATACCTAAA
TCTATAATGATACTAAAAAGAGTATTTGTCTAATTTTCTTCTGGCCAGGTGGATGGCATCTATGAAGAGGGGGCTGTGGAACGCTCT
CAGCCAAAGAGTGCTAGTCTGGCATGTTGTGGCTTTTTCAGTTTGAATGGCTCTGGGCAATCTGTTTTGCTGTAGTCTCTTTTTTCC
CCCGTTCAGCACCTTATTTTCCACCTGATGGTCTTTGATTCACAGCAAATTGTTTGTCTTCTAAAGGATAAATCGAAGGTGGGTTTA
AATGGTTCAAATACAAGCTGGCTATTAAACTGTGAGTTTTACAAGCTGTCATGTCATATGGGCTGAGTTCTATCTAGCTAGATCCAC
CATTGGAACTATTCTAAGCCATGGTGAAAAGATAAAAGACATCAGCCAATAAATCCCTTCCTCATGTAGGGGATTCATTGTTGGGAT
AGTACCTCCACAGGGTGTACTATCAGGCCCAGGCTTGTCACCATGACCTCTTTGGTGCTAAGTCAGTTAGAGGACCCATTTCTCACT
GGTCTTTGAGTTCCCCATGGGATAGGACCTTAAGGGGGTTTTATCCCAAGCTTTGCCAGGGCCTGGCTTCTGGAAGGCATTCCATAA
ATTGTTTTGAATGCATGAATGACTAAATAAGAAAAATGGAGAGTGCTTCGTTAACTGTAAAATTAGCTAAATGTCAACTAATATTTT
TTCCCTTCTGTGATGTGGAAAGATATGCAGGTGCAGGAGCAGGCAGCTGATGGTGGAGACTGGGGCGAATGGAAAGTGCCTGTTATA
AATGGCAGCCTCCTTACCTGTGCTTCTGTCCAGCTCTCTTGCCTCTCACCCTGAAAGGCTGTGGTCTGTCAAATCACTCACAGGTCT
GTCTGTGGAGAAGTCCTAAGTAGTATAGGGTCCCCACTTCTGTGATCCATTTCAGTTCCAACCCAAAGGACTTTCCAAAGAATGGAC
ATCTTTTGTCTCTTCTCCCACTCTGTCCAGTTGGGTTTATAATCCCTTTCATTATCTTCCTCATTCTCCCCACTCTGTTTCTGGTC
TTCATCTGTAGAAATTTATCTGTCACCATTTTGGGTGCTCAGGATGCTATCGTTTCTCAGATCCTTCCTTCGTGTTTCATGGATACC
TCCTGGAACCCTTTTTTCAGTGAGCTGGGAATATGTCTTTCAAGAAAAGGAGGTGGTATATATTTATAAAAAATTTAATTTGGGACAA
AGCATCTGACAAAGCGTTTTTCCCTCCTTATGTGTGGACTTACAGCCAAGTGTTTTATAATAGCATGCAAAGTAAACCACATTTGATG
GGTTTTATTGAAAATAATACATTTACCCCAAAAGGGGAAAAAGTACATTTTTGTGCATGTCAATTGACAGTATTTGAAATGCAAATT
TTTCTAGAAAATTTGGGAGGTAACAGTCTCAGGAAAGGAAACTTTATATGCTATAATCCAGACATTGGTCACAGAGAGATTTCCTTT
```

```
GGTGGCTTAGGTAGGGCAAGGCAAAAGGCCCCTTGTACCCATCTGCTGTAGAGATAGCATATGGAAGAGCCCTGCCACTCCCTGCCA
GTCAAGCCCTTGTGATAAATCTTGATACTCCCATGTATATTTGCCTGCTTGTGCTGCCAAGGCCACTGAAAAAGGTCAGTGCTTTGT
GAAATGGAGAGTATGATATAAACATAGAAGTTGTTAGTAATTTTTATTGTGATTGATCACTTTTTTTTTCTTTTCTTTCCTTTTTTT
TTTGAGACAGAGTTTCGCTCTTGTTGCCCAGGCTGGAGTGCAGTGGTGCCATCTCCACTCACCGCAACCTCTGCCTCCCGGGTTCAA
GCGATTCTCCTGCCTCATCTTCCTGAGTAGCTGGGATTACAGGCGTGTGCCACCACGCCTGGCTAATTTTGTATTTTTAGTAGAGAC
GAGGTTTCTCCATGTTGGTCAGGCTGGTCTTGAACTCCCGACCTCAGGTGATCCTCCCGTCTCGGCCTCCCAAACTGCTGGTATTAC
AGGCGTCAGCCATCGTGCCCGGCCGTGATTGATCACTTTTAAGTACATGAGAATTCTTGGCATGACATACAAAAGGATGGGCAGA
GTTCCTTTTCCTGTCTGTGATATATTTCATGACGATCCAGTCTTTGCGGGCAGTGGTTCAGGGTAGTGCAGCCATTGTAACCTAAAC
CATTCCTGTGTGTTATTTATCAGATTTTCTCAAAGGGTGAGTGCTGATCATTTTAAATTCTGTTTAAAAATAAAATTGTTCATCGA
AGTGATGAAATAAATAGGGGTCAGATATGATTTTAGAGATTTCGAATCCTGAAGAGGGTTTGAGATTTGAATACCATCTCTGTACAC
TCAAGGCAAACTTCCTGTATCACTCAGGACTCATAACTTCATCTGCAATTTTATTATTGGGTGACATAACTGAACAGGTCGCTGCAA
ACACTGAGAATTATTGAGTTGTTTAGGTGAGTCTCTCTTGTTTAAACTGTTGAAAGGTTCATTGATTTGTGGGTGAGGGAAAACTGT
TGTAGCGTAGTTTTAGTGTTTGCATTTCATTTTAACATTACCTTGCAAGGGATTTTTCATGCTGTACTAAGGAGATTACAAGGCCAC
CCTAAGGCATTTTAGGGTTATATAAGGAACTGGCTTTGCTGTTTTATGTACTGGCCCTTTTATTTTCTGGCTAAGCCAGAATCTTGG
TAACCCTCTCACCTATCACCTTTGGGTATTACCTTTTCAGCTCTCCCTTTGCCCTCATGAGTCCAACCAGTGCCCTGGTCTGAGCCT
CCATCCTCCCAACTGCCCTGGCTTCCTGCTGTCTGCCTTCCGTGTTCCTGCCTACGGTTCATGCTTTGCCCTGGACCTCAGTGGTCC
TTCATGTCTGTCTGATCACGACACCTCATGCTATGAGTGACCTCCACAGCTTCTGCTGCTTTTGGTGCAGCATCAAATCCTCCCGGT
CTGTGCTTTCTCCTTCAGGGCCCACCTGCAGCCTTATTCCCTTCTCCTCTTCCTTCTCTTCCACTCCAGTCCTCCTCCCCATCCCCA
GTCAAGTTAACCCTTGGAATATTCTGTCATACCACCCAGGACTTTTCCTTCAGAGCATACATCATAATTAGGTGAGGTCATTATTGC
ATATTTGGGTGATTATTTGATTGTCTGTATCTTGCGCTAGGCAGTCAACTTCCAGAGGGAAGTGGCTGTCCTTTTAATGCAGCATTG
ATGGTAGCACCCACACTCAGGGCAGGGTTTTCGAAGTGCTCCATAGGTCCTGGTTTTCCGTGACAGTGCTGGTATACCCCTGAAGTC
CCCATGTAATTTATTAATAGTGTCCCCTTTCACTTTCAAAGTTACTCTCAATTTGAATGACAGCATATATGATGATCCCTTCTAAAG
CCCAGTGATATGTAGTCATTCAGGTAAAAGGTTTTATTGTCAGGCTCCCAGAGTGGGAGAGCCTGGTTCCAGGGTCGGGGGGTCAGA
GGGGAGCTGTTGCTGAATTTTGATTCTGGCTGAAGATAATTTATCTTCTTGAAACTCTGGCAGCATATACAGATGTGCCTGTTGTTA
ACATTTTAAGAGGTAAGAACACTCTCTGTGCACCTGGGATTTTGGAATATGATAGTGGGAGGCACCAAGACTGTTACAGAAATGGAA
AATGAACACAGGGTGGCAGAGGGTAGGTACTTCTGCCAGTCAGAGGCAGACAGACTTTCAACTGGAAGGGGTCAAGGACACTATTGC
ATTGTGTTTGGCCAAACAAACAAaAATGCATTCTAGGGGAGAGCTGGTTTTAGTCGTGTGTTTAGCGGGGGCTCAGTGAGAGGCACAC
AGATGATCTGTGCATCCATTTCTCTACTGATGTAAAGACTCAAAGAAGGTAGTGTGCACGGTGCCAGGGGAATCTTGTTGAATTACT
AACAGTGAAAAACTAAAAGTGGCCTAAGATCTTGGTTTCTAAGGCCTATTATGGCCGAGTCCCCAGAGACAAAGTTTCCTCCTGTC
TGACTTGGAGCAGATGCTCGTCTGCCTCAGGAAAGGCTCTGAAGAATCCAGGAGAACATGCTTTGCAAGGCACTTTCAAATTGCTGG
GTCCCTTCAGGGGAAAAGGGGTTTTTATGCACACCACTAGTCTGAAGATAATTTTATCAGATCCTATAGCCTGAGGTCTTAAAACAG
AACAACACTAAAGAGCCCCTCTCAGGCCTGCTTCCTAGCTGGATGGGACCTTGAAGTGGTGGCTCCCTTCATGTTGGCCC
TGCAGGGTCAGGCATCTAAGACTCGTGGAGGCCCAGGCAGTCTGGAGGGATACCAGATGACATAGCAAATGCCAAAAAAAAAAGATC
GAGTGCTATGTTTGCCTGTGTGTGGGTGTGTTTGTTTCACAGGTACAGGTGCACCCACCCGGAGTCAGGGATGGAACATGTGCAGTG
AAGGCTATATGCTTCCCAGATATTGGGGTGAACTTGATGAGAGTCTGGTGGTGGACTCAGGGACCTGGAATTGTCTTCCTCACTTTC
TTGCCACTTCTCAGTGTGAATGTTGTCTGTAGGGGGTGCTCTCCTTGCCTGGGTCTCAGAGCTACCAGAGCATTAGAATGTA
GGAGATGCGCAGGCCTTGTGACGTTTTGCCAGTCGAGCGATTCAGCAAAGTAATTGCCAGGTTTCTCTAATTGTTAAATATCTACA
ACAGGCTGAATTTGGATTGGCTGAAAAGAAAGGTGAAATGAGGTCATCAGAATTACATGATTTTTTTCAAGTTCTGGCATCTTGTGT
TCTTTGCCAAGCAGAAATATTCCAGTAGAACTGTGATAATGCCTTTCATCTATCTGCTGAGCCTTCCAGATGGCAGTTTCTCAGCCA
GTGAGCAAAAGAAAGGGTGTTGAGATTTATGAGTAAGTTGATTCCCTTCGTTTGTACTTCCCAGTGAACTCTCATTCGCTATTTGTG
CTAAGCACCAGTGGCAAGTTATCCTCACATCTTTAGCTCTGCAGGGCCCTTAAGCCAGTAGGCCTTACTTCAAGGGGAGGAGGGCTT
CAAATTCAGAGTAATAAATAGCTGTCTAAATAGAGCCCTTCACATACTTTCTCAGAGAACTTTTTGTGATCTAAGTGGACTTAAGTT
TCTTGAGGACAGGGGCACTCCTTTCTCCTGGGTAGCACCAATGCACAGGAAGCTTGGCGTTGGATTCCGTGGGCCCCTCCCTTTCTA
GTTATGTGCACAGTCTCCCGGGTTGAGAGAATCCCTCCCTTTGCTAAGTCATCTTCATTCAATCCAGGGATGTTAGGGAATATGCG
TGCCTCGGATGACAATACAAGATGGATTAAGGTGATTCTTGTGCTTTGAGAGCTCTCAGTCCAATGGAGGAAAAACATATAAATAAA
TACACACATAAACAGAAGTGGTGTATAAGTCTAAAGATCAGGTAATGAGGGAATGTTTTAGAGGAGGTGGTGCTGCTGTATGTATT
GGAGGCGGGTAGGGATTTGCTGAGCAGAACATGTGCCATGTTGTTTTCTCCATCAGGCCTCATGGCTGGAACAGCTGCAGTGCTTTT
CGTTTGGTGCCTCCTCTGCTTTGCCTTTGTCCTGTTCTTGTTCACTTTGAGCACCCTTGCAGCCTACCCTGGGCATAACCCTCCAGG
AACTCTGATGCTGTTTAGTAGATACTCAGAGGTTACCACTTAATCCTTGTTGGTTAGTTTTGTTCCCCAACTCGAGCGTGAATTCAG
GACAGGGCCCATCTCTGACCACACCAAATGCAGTCAGTGCATATGTTAAGATTTAGGGAAAGCGTTTGGGTTGGTTAGTCGAGTATT
TTCATAGATTATTATTACCTGGTTATGTAAGTATGACAAACAGTTTAACAATGACACAAATATAAGCAGAGGATCTGATTAAAAAT
ACTTTTTTTTGAAAGGGAAGCATAAGTTTGTGGTCAGCAGCAAATAACCCATTTGATCTTCATTCATTTATTCTTCCATTTATTCAC
TCACTAACAGTCTGTACCTTTGTGAATAGCAACAGTCTTTTTCTTCATTGGTGTTTTTTGAATGCTGGGTTTTTACTGCCCTGGGGA
TTTTCTTTAAACCTGATAGTTGTTTTGATAAATCATCTGGATACCAGCTTTGAGCTAATTCTTTTCTATTAAGTACCTCTTAGAGTG
GCTGACCATAAACCATTTGAGAAATCACTACATTTTATTAATGACTTTTGAGATTTAGCAGCAACTTAAATTAATAATACTGGAGTG
TGCTTTGGATAAAAGACCAGGAGACTGCTTCATTCTGCAAACCAGAGCAGAGATGAAATGTTTGCATTAGGAGAACGTAATTTATC
AGCATTTTAGGCTAGTGTGTCGATTGATCAAGGTTGGGCAATAGTGGTAGGCAGTGTTATAAGTGCTGTTTGTATGTATCAGCTCA
TTTAATCCTCCCAGTAACCCTTTAAGATGTCTGCACTATTATTTTTCACATTATTCTGATGAGGACATCGAGGCACAGAGGGGTTGT
GTGACTTGCCTGCAATCTTGGTTGTGTGCTCAGAAATAGTCTTTTTGAGACTGAAGATATTCTGGTTATGTGTAATACACAGTACAA
CTCAAAGACTGTGAAATAGTTCATAGACAAATTATCCATTCTTTGCCACTTTAGAGTAATTATGCTTATTGTTTCTGTTTCAGGATA
ATATGTGTGTTTATAAGCATTTTTTCCTTTTTATTTAATTGCAGTAACATATACATAAATAACTATGATGGCTAGTTTTGAACTC
ATTTAAAAACAAGTAAATTTATGCTTTGATAAGCTAAGGTACATTCAAAGCATTCCCATTTCCAAAGCAAAGTGTTTTCAGTTAAAG
ATTATAGATGTTGCCTTTTGCCAGGGATGCTTGTGAGAATGTCATTTAGAGAAATCTTAAAGTAATTTATCCATAATATTAACTTAG
CATGCCTTCTCTCTGCAATGTACAATGTATCATAGGACAATGTATGATACAGCTCAGTTTTGATATATCAGCAAGTTGAGTAGTAAT
TTTTGTTGTTTGTTTTAGTTGTTTTAAAAGCTGGTgTTCAGATTCCTATGCTTAGAAGGACCAGGCAGAAAATAAGCCATTAAGG
GTGCTGGGAATGCAATAGGGAGTGATGGGGATGGTGTGTAAAGGGGTCAGCAATGCTTCTGCCATGCCTATCAGGGAGCTGGTTTCCA
TTAGCTGTTGGTGACCCAGGAATCTGTACCTGTGTTGCCAGATCTAACCATTTTCCAGGAGAGGCTGGAACTTCATATTTAAAAAAA
TGAGAAATTTGTCAACTTTAATAATTTGGAAGAAAATTCAAATTAAACCAATACCAGGCAGTGTGGTGTACCCCACATTACAGAGGAA
AGTAGGGGACTTGGGAGGTACATGAGTCAATCTATAGATGCTCCTGTTGTCATGTTAAACTTGTGCTTGCTTTCAGTTGCTGTTAAC
ATTTTGGGTCCACTGTATCTAGACATGTCCTTCAAAAGTTGCATGTAAGTTAATTTTTTTTTCTGTATTTAAAAAAATGGCCCCACAA
ACACTAGTCTAGATGTTGAAAAAAACCCTTATACCTTATTATGGACGTTCTGATGAGTCCTTTTGTACAGTGAACAATCACTTGCT
GTATTTACGTTTGTACTTTTGTGTTTAAAATGGGGAATGGTGTTAGATATTGCATACTGCTTGAGTTAAGGAGGTCGTTAACCAGGA
ATGCTTTGGATTACTCAGATTATTATTGCACTGGTGTTTGTTGTAATGGAATTTGACCTGTCAGGGGAGCTGTAGATGTTTGGATGA
GTGTTCAGTCCTTAATACTGTTATCTAAATTCTAGGCATGTTGCCTGTAACTTCCAGTTTCAAAAAATCTATGTCAATCTGTTCTTT
TATAACCTAGTGCTGTTGTTTTTTTCCCTCTTGTTTTATATTTAATCATTCTCTTTGGCTCACAACAGCAGTCCAGTCCAAAGATTT
ACTTTTTCTATTTTCAACCCCATCATTATTTTTGTGTAAAAAGTATGTTTTGAGTTACAGCAATTCATGGAGATCAAGCTTCTACTC
```

```
TGTAGGGAGAAGAGAGATGTCATTTACTACAGAGTTTATACACACAAATGTTCTGCTGCATGCTGTGTTCCCACTGAAATCCAGGGG
GTTTATTTGCATTTATACTTAAAGAAGAATGACCGTATTTGGCTGACTCTAAGCCAAGATGATACAGGCCCAAAGTTTTTCATGGAG
GAATGGGGGAGTAAGGGAAATAGGAACATTTGGAAGAAATAGTTGTTGTTTTTCCCTGTTTTATTCAGTGGGCATTTTATTCACAGA
TCATTTGATGCATTGCATTTACTCGTGTTATTCTGGTTCCTTTTGTGTTTTTTTTTTCCTTTTGAAACAACTTCACACTTACAGGAA
AATGGCAAAAATACTAAAATAAGAACTCCTATAAACCTTCACTGATAAAATCCCTTCAGTTTTCTCCAGTTATCCCAGTAACGTCCT
TTATAGCAAAAGGATCCGGTTCAGGATCACTCATCACGCTGTCATATCTTTCCAGTCTCCTTCATTCTAGAAGCATTCTTCATTC
TTCCTTCATCTTTCATGACACTGACACTTTTAATTACAGGCCAGCTGATGTGTAGAATGTCCTTCCTTTTCTTAGGAGATGTTTCT
GTCCTTCCTTGAAAACCCCACTAGTGTTACCTGCCACGAGGAGAGCTAGGCGCTCTGCCCCCTACCCCAAGCTGGTTGCTCTCTCTT
ACAATGCTTCATTAATCAAGTACCCTTTTTTGGAGCATTGATGATATTCATTCATCCTTTTAGCCAGTAAATATTTTTGATGTCTA
TTACGTGCCAAATGGGATACATTTAAGAACAAAGCAGACCAAAAAAAAAAAAAAAAAAAAACCTGCTCTCATGAAGCTTACATTTT
AGTGGAGGGTAACAGATAACCAACAAGAAAAAATAAGTAAAACAGCAAGTACATTAGATCATTAGATAGTGATAAACGCTAAAGAGG
ATCGAATCAGGCAGGCAGGAAAAGGGATGGGCAGGTTCTTGATAAAGTGGCCAGGGAAGGCCTCCCACACTAGTGATTGTGGAGCAGGAAC
CCCAAGGAAGGGAAGGAACAGGCTGTGGGGCTAGCTGCAGGAAAGCGGTCCCGGCAGAGGGAGCCTGTAGGGGTGAAGGTTCTGCTG
CACCAGGTGGGCCAGGGGTGAGGAGGAGGCGGTCTGGTGGCTGGAGCCAAGCAGGAGGGGAGAGGAGAGTGGTAGGAAATGAGTCAG
GATCTGACTTATGCCTTAAAAAATCCTTTTGTTGTTTACGGAATAAGCAGGTATGTAGGGATGGGGTCAAGGTTAGAAGCAGAGAGAT
CAATTTAAGATCAATTATGGGAGGTTATGATAAGCCCTTTGTGTATCTGTTTGTCCTATTAGCACATAAAGGGGACTCAGTGGTCTT
CAACCTTTAGAATACATAAGATGTACCTGGGCTGTTCGTGAGTGATGCAGATTTCTTCACTGGGAAACCAGCATGTACCTGGGCTGT
TCGTGAGTGATGCAGATTTCTTCACTGGGAAACCAGCATTCATTAGATCTCACCTGTGTTGTCAACAGCCAGCCCCAGGTGATTCTC
ATGTGGATGGTCCTTGAACAACATGTTGATGTTGATAAATATAGTAACAAGTGAGGAATGAGTGAATAATTGGTTTATGGAAAATCC
TTTAGAGATACTTAGTTATGAAGTGTTTCCACTCATTGCTTTATTTCTCATAAACATAGTACTCAACTTAAAAATATGTGGTAGTAT
AAATTTTCTCTTAAAACTTACCTGGCACAGAAAGATCTAATAAGCAAAGTGATTTTTTTTTTTTCCAAGAGAAGATGTGTATTTGATG
AAATAAACATACTACTCTGACATACTATTTTCTTGCTTTTAAACGTGGTTTTCAGAAGGAATGAATAAATGCTACTAATAGGGTTCA
TTCATAAGAAGCAATTAAAATGTATTCAAGAGGAAATTGGTTTCAGGGAGTAGGTCGGATGAAGCCATTTAAAGATAGTAATAGCAA
GTGAGTCGTATTTTAGAGTGGACCAGATCTGGCTTGACATTTCATTGCACCAGAGGTTCACAGGAAGGAAGGGGTTTGGCCCTCTGG
CCCTGGAAATATGTAAAGGGCAAAACATAACAGACTTTTGTTTTTTCTCTTTGGTGAAGTAACGTCAATCTTGCATGGGTAATCTTG
AGTTGAACATGAAAATAAGTCTTACCTTGATTAACTGGGTAAAATCTTGGTCAGTTTCATGGGCTTTGGAACCAAAGTGGCTCCAAA
TTCTGCTCGGAACTATCAGTGTGACTGGGCAACTCACATCACCTTCATTTCCTTATAAGCGGGAAATAATACCTGCTTCACAAGCTC
ATTGTAAGGATTGAGAGTTAATCCATTTATGTCTGAGGTTGCAATTTTTTGAATTTTTGCAATCAGACCTTGGCAACCACCTTGAGC
AGAAGGGTATAAAAAACTCCCACATGCTTAGCGTTCCAGTAATGGAACACTAGGCATAAATAACTACTGCCCATAGGGCTCTTACAG
AATTGTCTGGTGTATTATAAGCACTCAACAATTATTAGCTTTTTCTCCTCCTCCCTTTCCTATTATTGTTGTTGTTATTATCATTAT
TATGTTTCAGTGCTTTCAAATCGTATTTTGCCTTTTTGGAATCCTACAAATTTTCAATGATGGTTTTTTTGGGATTGGTTGTGTCTAA
AAATACGTTCATTAATGCCTCTCTGTATAGGAGGAATCTAAAGTCTTAAGTGTGGCGTTAGTCTTGTGTATTTATGATCACTTCCTG
GAAGTAGGCTGCCATCACTTATGTAACATTGACTTTCTTTCCCTCTATCAAAGAGAAGCTCAGACTTGCTTCAGGGAAGTATGTTGA
GAATTAATGAATAAGTTAATGTTTGTGGCTCTACCCCATTAACTACTAATTAATGTAAGTTACTGCTCTTATAGTTCTCCAAGTACT
ATCGTCATACTGATTCTCTGAACTAGGGACAGGGACATTGAGAAGACTTTTTCTTATGTACTGTGGGGAAGCAGTGTTTTGTAAAAT
TTTTGCTCTTTTACAACAGAGTATCACTCCGTATCTAAAATTTTACACCTCCTCTACAAAACAAACAAACAAAAAAACACAGAA
AAACCCAACCTAGATTAAATCAAGTAACTTCTTATGGAGCTCTTAGTAATGAAATGTTACTTATGTTAATACGTATTTTTTTTTCCT
CTGGGAAACCATTACTTTCATTCTTGACTTTATCTCCTCATCATTATGCTGCACTTAGTCTGTACTTGGCAATAATGTTCTAAAAGC
TCTCATAACAGTTCCATGAAGGCATAGTGTGGTAGAGGCAGTCCTAGCGTTGTGGTTTGTTTTTTTTTTTAAAGGTTTGTATTTCAA
TTCTGTGTTTCTAAAGGTAAGATTTGGATCTTCTGCCCCTGAACTTTGTCTGTAAAACAGGGATGGTAATAACTTCTATTTGGGTT
TTTGTAAGGCTTTGGAATAACATATATGTCAAATACCAGGCCTGAATCCAGCAAATGTGGCTGATTGTGACTAATGCAATGATTTT
ATTACTTTTTTTTCAATATGATTCAATTTGGTAATATAATTAGAACCAATTTCATAGTTTCATATAAAGCAAAAGAAGGTATTTATAG
GGTCCTAACATCAATCATATTTCCACCTGACATAGGCTTACCTTAAATAACTAATATCCAAACTTACTCTGGGAGGACACCTCTTCT
GTAAATGGATAAAATTGAACTTTTCAAAGTACGTGAATACAGAGGAAACATGGAATTGTATATATGTATGTGTGCATGTGTTGTTTT
CAAATAAAGTAACTCACTGTAATGAATTACAGTGAATTTTAAATTTCCCAATTTTGTATTAATGTTGAAAGCCACCAAAGGAGTAAG
GGATGGGTTGTATCTCAAAAAGGCCACCGGCCTGAGCTAAAACTGTTTCCTATACTCTTCTAAAAGGCAATGTTTATTTAATCCCAAG
AGTCCTTTGTTCTCATGATTACAAAGTTAAAATTTAATTATTTGCATTGTAGACTTGAAAGGATTAGCTACACTCGTGATTGATATG
TAAAGAGAGTTTTATAATCTCTGGAACTATGGCTTTTGAGGAACAAAATAATTTAAGCTATAATTTTGAAGTGGGACAGAGGATATT
ATTACAGGTTTATTCATAGGATTCTCATCTTTTGATTGGACTCTAATTAAATACCTTCTTGTCAGCAGGGAGGGTGTGTGGAGGAAT
TATGATTCTCCTGCTTTAATCGTTGTTGATTTCTTAGTGAATCGTTGAAGTGGCATCCAGTCGTTCTTCTCCAGCTTTCAGACCCTTT
GATGAAACAATAGAAACTTGTTAAGTACATGACCAGATTCTTTTCATGCAGTTCCCTTGGGGGAGGTCTATTTTAAATTAGATAGGT
TTGATGTCCAGTCTGAAAAGCATTTATTCACCAGTTCTGATAACAGGTCAAGTAGTTTAACCTCTTACACATAAGACTAAGGGAATT
AAAACCTTTCCTTTCCACCACCACTACCCTCCCCTCCCCTAAAAAAAGGAAAGGAATTTTAAAAAGGAAAAATGTAGAAGAAGTAAA
ATGGTGTTTATCCTTTTTTGTTAAAAATGTCACAATTTAGACCATTTACAAATCTGTTTCTTATTACAAAATGATGAGTCTCTTTTGT
CTTTGCTCAGTCTCTTCCTCTTAGTCCACCAATATTATAATTTTGTTAAATTCTGCTTATCACACTCCTTCCTGCAGACACGTTTT
CTTCTGTCCCACCACTCATTTTCAGAGCTCCTAAAAGTTCAGAAGCCCCTCTTTTCTATACTTTGCACTTTCTCTCATGGAATCTCT
TCCATGCCCATGACCTGCTGAGTCTCAAAGTTTAACTCCAGAGCTGACTGACCTCATTTTTAAGCCTCAGACTGACTGTCCTACCTG
ACATGTCCTCTTGAATGTTTTAAAAGTCATGTCAGACCCATCGGTTCAAAACTGAACTCATGTTCTCTCATGAATGCTGGTTTCTAT
GACTTTTTTTCTAAGCACAACTCTATATCTCATTTGCTTGAAGCCCTCCAGAGAACCCTCAGACATTGCTGATGGGAATGAAAATGG
CGCAACCACTGTGGAAAGTGTTTTGGTGGACCCCCAAAAAAGTTAAAGAATTACCATATGACTCATCAGTTTTACCCCTAGGTATAC
ATTTCCAAAAGAATTGAAAACAGGTGTTCAAATAAAATGTGTGCATGCTGGATGCAGTGGTTTATGCCTGTAATCCCAGCACTT
TGGGGATCTGAGGCGGGTGGATCACTTGAGGCCAGGAGTTCGAGACCAGTCTGGGCAACAGGCTAATTTGTCTCTATGAAAAATACA
GAAATTAGCCAGGCATAGTAGTGGGCACCTGTAATCCCAGCTACTTGTGAGGCTGAGGCACGAGAATCATTTGAACCTGGGAGGCAG
AGGTTGCAGTGAGCCAAGATCGTGCCACTGCATTCCAGCCTGGGCAACACAGTGAGACTCTGTCTCAAAAAAAGAAAGTGTGCATAA
TGTTCAATAGCAGCATTACTCATAATAGCCCCAAAGTAGAAACAGCTCAGATAATTGTCCCTGATGAATGGGTAAACAAATTGTGGT
ATACCCATACAATGGTATAGAAAGGGAATGAAGTAATGATACATGCTACAACATGGATGAACGTGGAAAACATTATGCTAAGTGACAA
GCCGTACACAAAAGGCCACATATTGTGTATGTTTCTATTTATATGAAACGTACAGAATAGGCAAAAATCATAGAGACAGAAAGCAGATT
AATGGTTGCCAGGGGCTGCGGGGGGGGTGGGAATGAGGCTCTCCTTTTGGGTATGTGAAAATGTTCTACAATCAGATAGTGGTGCTGGC
TGCACTATATTGTGAATTTACTAAAAGCCACTGAACTGTGCACTTTAAAATGGTGACATCTGGCCGGGCGCAGTGGCTCACGCCTGT
TATCCTAGCACTTTGGGAGGCCTGAGGCGGGCGGATCACGAGGTCAGGAGATCGAGACCATCCTGGTGAACATGGTGAAACCCCGTCT
CTACTAAAAATACAAAAAATTAGCCGGGACGTGGTGGCGGGTTGCCTGTAGTCCCAGCTACTCGGGAGGCTGAGGCAGGAGGATGGTA
TGAACCTGGGGGGCAGAGCTTGCAGTAAGCAGAGATCGCGCCACTGCATTCCAGCCTGGGAGACAGAGCGAGACTCTGTCTCAAAAA
AAAAAAAAAAAAAAAGTGACATATTGGTTCCCTAGGGGTTGCTGTAATGAAGTTCTAGAAACTGGGTGGCATAAACTACAGAAATGT
ATTGCCCCACAACTCTGGAGGCTGCAGCTCTGAGATGAAGGTGTCACAGGGGTTGGTTTCTACTGATGGTTGTAGGGAGAATCAGCTC
CGTGGCTCTCCCCTAGCTTATAGTGGTTTGCCAGCCATCTTTGTCATTCCGTGGTGTATAAACCTGTCACCTGACCTCTGTCTTCAT
```

EP 2 204 376 A2

```
TTTCACATGGTGGTCTCCTTGTGTGTGTGTCTGTGGTCCAAATTTTGTCTTTTTATAAGGACACAGTCATACTGGATTAGGATCTAC
CCTGAACGACCTCTTAATTAAGTATACCTGCAATGATTCTATTTCCAAATAAAGTCACGTTTTGAGATGCTGGTAATGACTTCAATA
TGTGAATTTTGGGGATATGCAGTTCAACCCATAACAGTGACTTTTATGTTACGTGAATTTTCAATTAAAAAAATGTATATAGACAAC
CCCTCCAGGGACTTCTAAGTGCTGTCAAGCACCAGACCAGCAAAGCACCCAATGTGGTCTGACCCCTGCCTACCTTGTCAGCCTTTT
TACTCTCCTCTCACTCTGTGCTGTTTTTTCTGTGCCAGCCACACTCCCTCTGATTGCAGTGCCTGTGCCTACAGCCTGCTTCTCCCT
CTCTTTCCATTAATGGAGGTCTTTTCACCCTTCAGATCGCAGCCCTAATGCTACTTTCATGGAGAGCGCTCGCATGACTTCCTGGCA
CAAGGCAGCCTCTGTTATATGCAGCACCTTTGTTCTTATTGTCGACTGCTGTGTGACAAAGCACTTCTAAACACTGTGGTGTTAAAC
AGCAACCACTCTTAGGCTTGTGGACTCTGCAGGTCAGGAATAGAGACGGCCTAGTGAGGAAGGCTTATCTCTGCTCCATGATGGGAG
AATGTGAACAGCTGGAGGCCAGAATCATAAGGGAGCTTCCTCAGGTGTGTATGCCTGCTGTCTGGACTGGGATGACTCCAAGACCAG
CCTCAGCGGGGACTGTTGACCATGGCTCCTACTGTGGTTTCTTCATGTGGCTTAGACTTCCTCACGACTGGGTGGCCTCCGGGTGGT
CAGACTTCTCACAGGGTAGCTCAGGGTTCCAAGAGTGAGCTAAAGAAAGCTGCATGGCCTCTTATGACCTAGCTTTGTTACATAGCGT
CATTTCCAACATGTTCTATTGGTTAAGCTGTCATAAGGCTGCCCATATTCATGAGTGAGGGGACATAGACCTCACCTCTCAGTGGGA
GGAGTTTCAGAATTTGCAGCCATGTTTAAAAACTGCCCAAATTCTCCATGCTACGTCTAAATGTTTGTGTCTCCCAGAATTCATGTG
TTGAAACCTAGTACTCAATGCAATAATATGAGGCTTTTAGGAGGTAATTAGATCCTAAGGGTGAAATTGGATTAGTGCCCTTATAAC
TGAGGCTGGGGAAGCTTGTTTGCCCCTTCCAACACGTGAGGACGCAGTGAGAGTGCACCATCTGAAAAGCAGAGATGAGCCTCGCCA
GACGCTGAATCTGCTGGAGCCTTGATTTTGGACCTCCCCACCTCCAGAACTGTGAGAAATACATTTCTATTGTTTATAAGTTACACA
GTTTATAGTGTTTTTTATTATAGCAGCCTGAACAGATGGACACTCGTCAATCTGTGTTCCTTCACGGTGCTCTTTTCTAATTACTCAC
TCCTTTGCATCATTATTTGATTGACTTCTGCCTTTTTAAATAGGCAGTAAGCTTATTTTGCTCACTACAGTATTGCCAGCAGCTAGC
ACAGTAGCTGGCACATAGATGGTTTGTAAGACTTTGAAATAAAAGTTGACTGGGAAGAATTAGAAAAGGTAGAGCAAGGAAAGCCAT
TTGCTTATATCATAAACATCACAAGCTTGTGCAGCTGATCCAAGCATGATCAACAGCAAATATGCCCAGTGGCCTGTAGGTTTATTG
TTTCATGCTCATGATCTCAGTTTAGCAGTAAATCTGAGGCTGCCTTGTGAATATTGATACTGCACGAGATGTCTGAGAGCTGCCACC
CAAGAGACAGTCATATTGACGCCTGCAATAAAATAACAGCAACAGAAGTATAAGCAAAGCCAAATGTCTCCTGAATGAATTTCCCAA
AAGAGATAAGCAGAATTAAAAATTTAAGGTTTAAATAGGCTAATACTGATAGAGTTTGTGTGTGTGCTCGTGGATTTTTATACTTGT
TGGGTGAAGAATGAGAGGTAGTAGGAGATACAGTTTTTTATTTTTAATGACTGACTTTATCAGAGCTTTATGGGGCTGATCATGTTT
ATTTAATACTCTCTGTAGAATTTAGAAAAAGATTATCTTTCCCTTCTCCTTGGCTCCCCAGTTTAAAAAAAAATTTCAAGTCTGAGA
CAGAGGCACGTAAAGCCTTGCCTAGCACACGATTCACTTGTCCCCCGGCTTCTGCACAGCTGCTTTACAGCTATGCTATAGACTTGT
TAGATGAGTGCCAATTTCCCTGTCTTTGCCTGTAAATTTTCAGGGCAGTCAGTTACACAGAACCTCTTAAAATGGAAGGTGATTGCT
GCTTACTAAATTAAAATGCAGTTTTAGTTTTTGCTCCTACACACTGAAGACCCCAAGGCAGTGTGGTATAATATGAAAAACACTGGC
TTTAGAGTCCCGTGGACCTAGGTTTGAACCTTGACTCTGCCCCTTTTATTGATATGACCCTAGGCAAGTTACTTATCCTTCATATGC
CTCCTTTGCTTAAAAAAAGGAGGAGGTAATAACACAGAGGTGTTAGAAGAATTTAGTGAATCGTACATATAAAGAGCCTAATGCAGTTT
GCCACGTAGTGAAGCTCAATCAATATTAGTTATTACGACTGCTATTATACAAATGTAATTGCTTGGGGTTTACTTAGAATTTGTATA
TAGTAATACTTGAATGGCTTTCAAAGAATGCTTGATTTTTATTCTGGTTTTGCTCTTAATTGGAAAGTAAGTTCACAGTGCCTTAGGT-
CTGAGGTATTAGTACATTAATAGTGTGATTATTCCTACTTTGTCTTTGTTTTATACTGGTGACTTTTTTTTTTGAAAAGCATTTATT
AAAGAATAAATATAAATTGAAAAGGGGAAATGGTCCAGAATTCTAGGACTTTAGAAAATTGAATGCACTTTTCTACATTTTTCTAAC
ATCTGTGGGAGTTAAAGCTGCCAGATAGAATGCATGGACCTGTTTGCTTCAGGATGTTTGATTTAAAACAGTGGTTCTTCCCTTTCC
GGACAGGGTCAGAATGACCTGGGTTCTCTCTCCAAGGTTGTGTACAAGAGCTCCACACCTTCTGTTCAGAAGACCAAGGACAGTGGCAG
ATGCCATGGCCTGGTTGTGAAGTGAAGTTGGAGGAGGGAGAATTCTAGAACAGATGGTTTCTTGGATATCTGGGGCCTGTCCAGCTCT
AGCTTTGAAAATGATGGGCCAGACCTTGAACTGGCATGGATACAGGCTTAAGTGCCAGAACAGGAAGTGAGGTCCTAGGGTGATGTC
TTTGGGGCAGCTGCTGCTACTCAGCTGGTGGGCTGGCACCGCTAGCTTTGGCTTCCTATGGTTTGGTGAGGTGATTGTGTGTGGTAC
AATAACAACAGTGAGGTGGGAAACTGGGCCTGCAATCTGAGAAGGCCATAACCACTTTGGAGCTGTTGGCCTATTTTAGAAGGGGTG
TCAGCTACTGTGCCTTGCAGGACATGTGCTACCTTTGGCATGCATGACGGAAATGCATGAATGTGGGAGCCAAGGATACATGCATCCCCTA
CACTGATCAGCCTGAGTTCTGCCTTGTGTAGAGCAGTAAGCAGTGCTTTGTGGGAGGGCTTGGCTGTCCCCTTTGACATTAGCCTCC
AAAGACTAGGAGCTTATCACAGACTTTGCTAACATCCTCTGATACCTTTTTGGAATAATTAAATCATTCTTGGCTAGGTTCTGCTGT
TTGTCCTGCCTCTCACTAGCTGGGTGTCCTTGGGCGAATTACTTAATCTCTCTGTGCCTAGCTTTCTTTATGTATAAAATGAAGAGG
CCAGACTAGATGATACCATAGATGTGAGATGATCTCTGATGTCCAAATCTGATTAAATAATTTTTAGGTATACATGTATTTTGGTAT
AAAGATTTCTGTGAGCTCTAATCCCAGTATTTTGGGAGGCCAAGGCAGGTGATTGTTTGAGCTCAGGAGTTCAAGACTAGACTGGGC
AACATGGTGAAACCCAGTCTCTACAAAAAATAAAAAGAATTAGCCAGGCATGGTGGTGCACACTTGTAGTCCCAGCTACTCGGGAGG
TCGAGGTGGGAGGATGGCTTGAGCCCAGTAGGTCAAGGCTGCAGAGCAAGACTCTGTTTCAAAAAAAAAAAAAATTTACGTGGAGCT
TCTCAATATCTAGTATTCAAGAATTTGGAAATTGGAGTGTATCGTTATTTTATGGAGAGAAGGACCCATTAATTCCCTCTCTCGTAA
ATGATCTTGTCCCATTTGTTGTTGGAACAATACTGGTTGCAACTGGTTGTTTGAGAGGCAAAGTATGAAAAGCATGGTGTCGAATTAT
AGAAACATATTATTACAGTGGCACTAGAGGCTCTTCATACTAAGAGGAGGAGATTGAGGCCCAAAAATGTTTCAGGGTGAGTCTGAG
GCTGTGCGCTATGCTCCCCTTGAGAGTGGGAGGCTGTGCTTTGTTCATCTTGCCATCCAGCGCCTCTTGGGCTGGTTTTGTTTTATTT
TGTTTTGTTTTGTTTTTAAACAGCTCTTGTCAGAGTGACACTAGGTAAAGATCTCTCTTTAACTTCCCCTTGACCTGTTCGATGCTG
AAATGCCGTCACCCATATTTCTAAGCATTATTTCAAGGTAGCACAAACTCCAGGAGCCGTGGGTTTCCTTTTCAGCATGGTCTGTGT
AAACATGGGCTGTTTCTCAGAGGCTGGGCTGAGGCCCCAGAAGTTTCTTCATAAGGGGTCACCTCTTACCTCTGCCCCTACAATTC
CTTTTCTATGAGGTTTAATCTCCAAGAGGGAAGCAGAGGTATGGTTCTGAGGAGTGATGTGGATTCTGGGGAATGCAAGGGAAGCA
AGGGCCAAGTGGGTTGAGCTAACGTGGGGACTGAGAAACCATAGTGAGGGAAAAGGACAAGCAGAAGGAAGGGCCTTGTAGAACCAC
CTAGTGCTTGGATAAGATTCTTCTGCAGAGCCCTGGGAAATTGGAAACACTCTCCGGGGTGGTGTGTGCACTGGCAGTTGAATCTTA
GCATCTTCGTTGTTTCCTTTTGATTTCACTTGTTCTGCTTCTTCTCTTGCTGTAGTTGCCACGTGCCCTGACATTTCGCAGAACTTT
GTTGCCATCCATCCAGATTTGGCCATTTCTCCTTTAATTCCACCTGTTGCCCCTCATTGTAAGTGTGACACACTTATCTTCTTTGGT
TTATTCTAAGGCATTAACTAAACATGTCTGAAATTCCGACGAGTCTAAGAATCAATATGTGTGTGTGTATACAAAAAGTAATTCTGC
CTCAGCCTCCTGAGTAGCTGGGATTACAGGCATACATTACCACGCCCTGCTAATTTTGGTATTTTTAGTAGAGACAGGGTTTCGCCA
CGTTGGCCAGGCTGGTCTTGAACTCCTGACCTCAAGTGATCTGCCTGCCTTGGCCTCCCAAAGTGCTAGAATTACAGGCATGAGCCA
CCATGCGCCCGGCCATCAACGTCTGTGTTTAATGCCAGGTTTTTTCTTTTCCCTAAAAGCATTGTTCGGAAATCTCTGCATTTGAGA
ATCTAGAAATAATGATAGTCAAACACGGGACATTTTTAGTGAAGAGAAGGGAAAAGTACTGTATTGTCATTGTCCCTGAGGTTTCCAGGCTTGTG
GATGACAACCTTAAAGGTGGGAACAATTTGCTAAGACGAGAGGGAAAAGTACTGTATTGTCATTGTCCCTGAGGTTTCCAGGCTTGTG
TGAATTTTGAACAGGGGCAGCCTGTCCAGTTGTAGGAAGGTTGTGGCATTAGGCTCTCTTGGTCCCAAGGCACAAAAACTCTGTAGC
AGGGGTTTTCATAGCAGGTTACACGTGGCAAGGCAGATGGGAATCTTGAGAATATCTGAGGATATGAACTGTGGCTGGGCCACACAG
GTATGGGCTGTCACGTGGTTCCCGATCCAAGGCAGCACTGGGGGGTTGGTGACTCTTTCCTCCTTGAAATTTCTGCTACGGTGACTG
AGTGTATATATCAGGGAGGATGGCAGCAGCTGTTGGCTGTGATGTGGCATTTAGCTTGGGCCTGACATTCATGAAGGGCCTCAGATTTA
TTACTTCATGTTGTCTCCACATGAACTTGTACGTTTAGTCACAGAGACAAACTGGCAGGATTGAAAGAAGATGGCATTCGTCATCCA
GATTTAAATATGTTTCTCATTAATAGACATTCTGCCTGTAGTGCACTATGAATTAATACATTTTATAAATCTTGTAACTGCCTTATG
AAGAGCATAATTAGTTATAAGTGTTTGGATGTTAGAAATGTTAATTTGTTCAATATAGGCGTTAAAATGTTTTGTCATTTTCTTATT
TTTAATGTTATTGGGAACTTTAAAAAAACAAGAGTGGCTCAAGCCCCTCTTGACCTCTTGGAAGGCCCTGCTGAGCTGGGTTTGCATC
```

167

```
GGCTGTTCTGGGTCATATGTTCACTTTGGGTCCAGTTGGCTGGGCCTAGGGTTTCACAAGGTGTGGAAACCTTTGTCCCCCATATGT
AAAATAGCCTGGCTCCATTTCTGTCAGAAATGCAGCCCACTCTACCCGCCACAGCCCAGGCAAGGGGATGGAGCTGGGTCAGTGGGT
GTGACAGGCTGATGACGGAATTGAAGAGGGGGAGCCTGTGAGTCTGACTTACGGGACTAAAGAATAAGGCCCCAGGCAGAATTCTTG
ATTTAGACATAAGACTCCAAAGTTTTAGGCAGTTCATTTCAGTTTTCTGCATCTACACTTATAAACTACAGATGAATAACACCCAAAT
GACTTTTGAAAACTCTGAAGACTGTAAAACTCATTTGTGCTTTGAGATACCAAGCCACTGCTAAACTACTTTCTTGAAAATGAAATC
ATTGTTTAGGCAGTTGGTGGTTGTTTGGAAAAATTTCAGAGTGAAGGGCTCTAGGAGAAAAAGAGTTCCAGAAATAAGGAGAGGATA
AAGTGATGCAAGGGACCCATTTAGCCGGCTAGATAAAGGCTGGAAGTGATAATTGGTTGTGCTTAGGGAGGGATAAAATTGGAAAGCA
ATGTCAAAATGCAGCGTGAGTCATTTTGGGGGGGGAATCTTTGAAATGAAAAACTGGTATTTAGTAGTAACAGCGACCATGCATTTT
AAAATCCATCAACCAGCATCTCTATTCCCCACCCTCAATATCAACCTGTAATAATTTATGCTGTAACCACCACCGAGAGATAGCTTA
GGACTGGATTACATCAGTCACTTTTTTTTTTCTTGGTTGTATGGCAGATATTTTGATGTTACAATAGGGACTAGAGAATTTTATGG
GGAAAGGTTGGCATATTAACAGTCATTGAAGTAAGCAGTCTGGGACCCTGTTGTGTTATAAGGGAAGGGATTTTGGACCTTGGTGTT
TTGCAGAGGCCATATTTGCATAATTAGAGAGCACAGGATTATGGGTAAAATGGTTGCAACTACTAAACTAGCCTAGTTTATATGGAT
TCTTGTTCATTTGCAAAATACCTTTTCTGTGTGCTTGTTGAAACCATAATATCCTGGGTAGGTTTTATTACAATAACTTATGGTCTA
GTAAACATATCTGGAAATACCATAAGAGAAAGCATAGGCAACATTTGTAAGCCTGCTGCTTTATGAAGAGATTGTGCTTATCAGAAA
TTGTAGCATTACTGTAAATTTTTTCTTTCATATGGAGTGATTTACTTGGTATTTCAGTATATTAATTTGCTAGGAAAATGTCAAGTT
AGTACTTCTCTCAAAAGGGCATTGTGCATCTGAATCACCCGTGGAATTAAAAAAAAAAAAAAAAAAGGTTTTGCCCTCAACCCAGATG
TGTTGTGACTTAATCTCCAGTTCTCGGGCCTGGGCACCCATCTAAAATGGTGAAGAGGACAGAAATGATTCTTGACTTCTTGGTGTGT
ACGTCTGTGAGGAAGCCAGATGATAAACACTGCAGGGGGAAAAAACCATAATTGTAAATTGTGATAAGTGCTACGGTGAAAAAATAA
AACACGGGGTAAGAGACTGGCAGAAAGAATGTAGTTTTGATGGGCGGGAAGGCCGCTCTGAGGAAGTGAGACCGCAGCTGAAAACTC
AGGGATGGAGAGGTTAGCCCTGTGGGGGGGAGGGAGGAAGGAGCATTTCAGGCTGAGGGGACAGAGTAAGGGAGGCCTGATGTGGAGA
GGGCTTGGTGGGTTTGAAGAGCTGAAGATGATTGGGGACTTGTGGCCCAGAGATAGGGGAGCGGGGGGAGCCCCCGCGGGGAGCGGGAGAG
GATGGTGCTCCTGGCAGAGATGGCAGGTGGCCTGCTGTTTCTATTCTGTGCTCCTTAGTGTCTTGGTTATGAAATGACTTGCCAAAA
TGTCCCTTTAAAAGAAAAGGGGTCCTTGCCAGTTACAGCAGTGTCACTGTTTAGGTCTGCTAAGCAAATTCTAGGGTGTCTGATGGC
TTTGTGTTGGGAGTTGTAAATGGATTTCATTTGAGAACATGACAAGAACTGAAAGATTAGTTTAAGCAAAAGAATTTAATTGAAGGA
TACATCATTGTTCTTGTAAATCCTTTGATATTTGGCTGTTGTCTTCCAGCCTTTTCATTCCCTGCATCCTGTCTGCTGCTTGCAGGA
CTGGGCTGTAAGCCTTCATTCACCTACAACCCTTTTGTATTGCTTGGAACTTGCTTGCTTCTTCCTCCTGCTCCTCTTGTTCCCCAC
TCCTCTTCTCCCTTCACCTCCCCATCCTCCTCCCCCTCCTCTTCCTCCTGCTTCTCATCTCTTCAGATGGAAGAGAGATGGATGGAAAGA
GCATTCACTTAGTTATTCTTTGGAATAAAATATACGTCAGTTTCCAGCGAGGGAATTCTGGCAGATTTACTGACAGGTGACAGTGTC
TGTGTAGAATTAGGATTCCTAGGAAACATTAGTTCTAACTACATTATTGATAACAATGCCATGTGCATTGTAAATTTGGGACCAGGC
CCTGCATGCAGTTGTGTGGTGGTTCCCTACACAAGGGCTCCTGGCTGAAGGGCAAATGAGGGTGGAATCCACTGAAATGCAGCCCAC
TCGCCACCCTCCACGGACCCCAGGGCAGGGATGGAACTGGGTCAGTCTGGAGGAAGAGGGTGTTCTTTTCTGTTTCTTACGCAAGCAA
ATCACCAGGCCCATGTCTTGACTCCTTCAGAGGTGCCAAAAGGGCTGGTGATAGCCCTGCCTGGGACAAACCTCAGTGACTTCTGGG
TCGCACATTCTTGCCTGGGAAGACCCTGTTCTTACCTGGTAAGGCACAATTGAAACTATGATTGGTCCACTTCACAGACGAGGCAGC
TTGTTTTCTCCCCTGGTGTGTTATTAGTTTTAATGGACATATTTGTGCAATCAGTGTGTACAGTTCATGTTGGTAAGCATCTGGCAT
ATTTTCATTATTTTAGAATAATGCTATGCTGAGCTCACATTTCAGCTTCTTGTCAAAAATAGTAATATTCTGGTATGGCAGATTTCC
TTCTGGGGCAGTACTTCCCACCAGTGTGTGCTGCCTGGATATAGTTTCATTCACAGAGGCTGCTTCACTCATGAAGGGCCTACTTATGTAGATAA
GTGCTTTAATAGTTGATTCTTAGTATTAATAAAAATTGACCGGGTGCTTTCATCTCCAGTTTATAAAGGAGGAAATTGTGCAAGGGGG
AGGCTAACTCACTTGCCCTAGACAAATTAGAGGCTTGTTTCAGGGTTGGAAAACAGGTTCTCATATTCCTTCTGGAATATGTTAAGG
TGTGGACTAATCTCTTCTGTGACAAGGACATCCCTGAGTAGTACAGTGTGTCCTCAGATCGTAGTCCAGAGTTAAGAAGTCAGGGTT
GGAGGAGTTCCTCTGCCTTCCTCCATGCCTGGCTGTCCAGATGGCTCCGTTTGTCACCATGTCTTAGCCAGTGGTGCAAGTGGCTGT
GTCTTTAAGAGGGTGACCTCAAAGTTGGACACATTACATCGAGTCATATCACATCACGGTATAGTCACAGGAACAAACCTAGTTCCC
AGGGAGCCTAGGAAATATAGTCTATACCTGGGGAGCGGGGTCAGGGGGAATCACATGCCTGACTAGAGCTAGGGGAGTTCAGTACTA
AAAAGAGTAGCGGGGAGGATGCCGGGGCACTGACTACCACATTAGTCCTCGTTCCTCCCTGAACAGCCTTAGGGTGGGTGAGCAGCC
CTAAGTGAGTTGTTAGCTAACAGAGATGTATAATGAAGGGGCACCACAGAGCATTTTATATTCCTACTGTAGCCCTAAATGAATAGG
CAGCTCTTTGTACATACGCTTGAATATGCTTCTTGGAACCAGTTCATTCACTCGACAGATACGTGCTGAGCGCCAGTTCTTTGCCTG
GGTGCTGTGTGGTCTAGTAATTGAAGGTACCTGTAAGATGGCCAGGGCCAGCCCTGCTCATCATGAAGGGCCTACTTATGTAGATAA
TTTTGAACCTCAAATAGGAGGGAAGTTGGAGTTGATTTACCTGTCATTAAGTTGTTAAAGAAAGATTTTTCCTACTGGTAGCAACTG
AATGGAGCTCTACTATGGTTTGAATGTGTCCCCCAAAGAGCATATATTGGAAACTTAGTCCCCAGTGTAACCGTGATGGGAGGTGGA
GCCTAATGGGAGGTGTTTAGGTCATCAGGGCTCCCCCTCATGAGGGGATTAGTGCTGATTATAAAAGGGCTTGAGGCTGAGTTCGAT
CTCTTGCTGCCCCTCATCTTTTGCATTCCACTATGGGATGATGCAACAAGAAGGCCCTTTGCAAATGCCAGCTCCTCGGTATTGTGT
TTCCTAGCCTTCGTAACTGTGAGAAATGAGTTTATTTCTTTACAAATTACCCAGTCTGTGGTATTCTGTCATAGTGACACAGAATGG
ACTAAGATGAGCTCAGAGGGAGAGAAGGCTGTCTAGGCCCGGGGCCTTTGCTGTACAGCAGCTGGGCTAATGCCTCTGCGAATTATA
TTAGCCTGTTTGTGTTAAATGAGGAGCCCTCCTGCTTGAAAGAGGCAAGGCATTCTGCTTATGAAGCCAGAGAAATCAGTCAGGCAG
CCTCTGACTTACCTTTGCCCTCCCCTTCTGCTCAGAATCCTTCAACTCACTGTCCGTCAGGCTCAGCCCTGTCTCCCCTGGTCTCTC
GGGACATTACCCCTGAACTGAGAGGGTGGTCGTGATGAAGAAGATTAATTAAGCACCCAGAGACATTGTCCTTCAATACATTGCAAAGGAAA
GCTTGCTCAGAGTAGACCTGCACCTGGACACGAGAGGCTGGAGACTGTATTCAGGACAGTGCTCGTGGGGAGCTTCCTTCACTGCTC
CACTCTTGTGTCCTGTGTTACAGGGGCTGCTGGCTGTTGCCACTGTTTTTCAGAGAATGGGCTGCCTGTACACACCTGACTTGGGTG
ATCATGTCTACCCAGGAGGACCAGCAAAAAGGGACAAGCAGTGATGCCATCTGGTTTTACCTCCAGCATTCCGAGCACTGCCCTGGC
CCCAGACCTCACCCCAGCCAGTGGTCGGGGGTTGGGTGCGCCCCACTCTGGCTGCACAATAGAGTAACGCAGGAGCTTTAAAAACTCCCAACA
GTTGAATTTAAATGTTACAGTGGGGATTGAAAATCTTGGTCTAGTGGTGGTAGCTGTTGGGTTTTTTTTCTTTTCATTTTTTTTGCA
TCCATTCCTGTTACTACCCTCTAGACCACTGTGATTTCTTAGTCTAATCCTGACTTGCTACCTGAAACCCTCTAAATGTAAGTCTCG
TGCAAGAGAGTGAGAGGTAAAGTTTGGTTTTTATGATAAGAGATACCTTGCTCAGTAAGCAGAAATAAGTGCCATGTGTTAGTAACT
ACAGTTTTGTTTTGTTTTGTTTCCTGTGGGAGCGACAGAAAAGAGTTTACAGGGTGTGGTGGGGAAAAGAAGAAACGAACATCTCTC
TGAGGGCGAGTATAAGGTTGTGGACTTAGTGGGAAAAGGTCCTCAACTTCAAGTCAGAGAGAAACTGTCGAGGAGGAGACTGATGAAG
AAGACACAGTGAGGTTTTTAGTGGTTAAAAACACAGCACTGCGTTTACATAGTCTATTTCCCCCCTAACAGTGGCAACATGTGTGG
ATTTTCTTTTTTTTTCTTTTTCCTGGAGAGAAGCATGCTGCCTCTGAACAGTGGAGAGGAGGAAAATCATCAGCATCCTCATTGGGG
TCAGGTGACCTGGGCCTCGTTCTTCCGCTGCCACCTGCCAGGGTTGGGATTGTCACAGAAGACTGTTCCTCTCCGTGGTGACTGATT
GCGGAGAGATAAGGGGGTTAGGAACATGCAGCTTGCTGTGCTGGGCTCAGAAGGGACTCCATGTGATTGACTCCAGTGGAGACTTAT
TTTACCTGCCATTTCTGTAGCAAGAAGGAACCCAGATGGGGCTGTGAATGCCTGGAAATGTATATGTCAAACATTGTGTG
AGGGTACACATCCTTTTCTGGGGAAGAAGTTCCATGGCTTTTAAAAATCAGAGTCCTAAAGGATCCCAGTGACTAAAAAAAGGAAGT
GAAGAATTCCTATCATAAGTATCTTTATCTGTGATAGGGAACGGGAATGTCTGCCTGATGTAGTCTAGAATCCTGTGAATTCAAACA
GATACTTTGTTCGTTCACACTCCCGCAGCTTATGTCTAGCGTGTTATGCTATAACGAATAGGGTTCTAACATTAAACTTATTTTTTG
AGAAATCTTCAGGAAAAAATACCATCTTATGAATAGGGTATAGTATATATAAATTTCATGGCCCGGCGCAGTGGCTCATGCCTGTAAT
CCCAGCACTTTGGGAGCCCGAGGCAGGCGGATCACTTGCGGTCAGGAGTTTGAGACCAGCCTGGGCAATATGGTGAAACTCCCTCTC
```

```
TACTAAAAATACAAACATTAGCTGGGCATGGTGGCGGGCACTTGTAATCCCAGCTACTCAGGAGGCTGAGGCAGGAGAATCGCTTGA
ACCTGGGAGGCGGAGGTTGCAGTGAGCTGAGATGGCGCCATTGCACTCCAGCCTGGGTGACAAGAGTGAGACTCCATCTCAAAAAAA
AAAAAAAATTTCATTTAAGGCATCACAGATTGTTTCTTTTTTTTCCCAACTGTGTACTCTAAATACAGGCAAAAAGCATGTAAGGAA
GGTTGTAACAAAATGTATATGTAGGCAATAGGACTGATAAAATACAAATAAATCACAAAAACTATATGGAGGAAGGGGGAAATCATG
CCAAATAACTTAGGCTACTGAGGTTATTTGAGCATTACATTTATAATGAGCTTCCTGGAAACCAAAGTAAAAAGAGAAACACAGTGG
ATGTTAGAATTTCTACTTAAAGATAGCAAATTCTTTCGTTAGGTAAGACAGTCTTTCTGGGGTTCATGTCTAAGACTTTATCACATG
TCGCATTCCTCAACACTTTCACCAAACATACAGAAATGCCTCTCTTGTGATTGTTTCCACTTCCACAAAACTAGTTGTGTGGGATA
CCCATCTCCGGATGTGTGGTTCTTTGATGAACTCTCTCCCCTGTTTTTCTAAAAACTCAGTTACCTTAGCTGCTGGGAATCGCAGTG
CAAGATTACACTCTGTGGAACTGCCCAGAGCGGGGAAAATGAAGAGGGTGACAGGTGCTTTCCTTCAGGAAATGACAACTTAACAAG
CACAGTGATGGTTTTTTGCTATGGAAATGGAGGATGCTGACCAGAATGTCTGCTGGGATGGCAGGTCCCCGGCTGCTCCCTTCCCC
CCACTGCCTCACCACCTCCCCCTGCCACACCACCCCACTATGGGGAGTTGAGCTGAGGAGGCCCTTTGCAGCCATCCGTCTGCACCT
CGCCTCTTTAGGAAGTACTCTTTCTTTCACCTTCTTGCACATAATTTACTTTGTGACTACTCATAAATGCCCCCTAAAGATGAAAAC
ATATTTTTAAAGTTGGAGTGAGAATCACCGTAAAGCCTTTATAGAACCAAATGACGTTTCTTTACCCAAACTGCAGAGCTTTCTTTTC
TGCTCCTTTTCTTCAAAAAGAGTCACAAAAATTCAGGAATTTTAAAAACCTCTGCATTTAATTCCTCATTGCCTTTCATATATGTTC
AATAATTGCATAAGCTGTCTCACTTAAGCTGTTGCTAATAATAGCTTTTTTGTCGTTTTGGAAAACTATAACCAAGAACCCTAATAC
ATAAATACTTCAAAGTATGTTTTCTTAATAATGGTGTTTGATGTAGGGTGGAGGATGAGGGGAAGTCAAATATTTAGACACTTACAT
AGTTTATGTTTAAGAATTATCTACCTGTATATCCATTATAGTTTTATCCAGAAATAGCATGTAGTGTGGAAAGATGTTTCCGTAACC
TTCCTGGAGTAGCTCATAAAGCAAACCTAAGAATGCTTTGGAAGTCTTTATGGTATGAGTGTTTTGTGTTTGTCCTGTTTTCTTTTG
AAAAGTACAGATCCAAGGGCAAACCTTTCAGAATGGGGTAAGACAACAATTGAACTTGATGAGTTGCCTTATGTAGCAAAGATGGCT
TCAAAGATAAAGGTAATCTGCAGAAATAAAATCAAGCTTCTTTTGCTCTGTGACTGCATCACATGAAAATGGCTAGGTAGATTTTCA
CTCGATTGGAGTTTGTTTAGGGTGGTCTGATTTAAAATGTAGGCTCTTGTGATGTGCATAATTTTAGATGGTCTTGATTGCCCAGAG
CAGCTGTTACTGCTGTGCAGAAACAAGTGTTCAGTGTCCCTTTGGAGAAATGTGCCGTGTGCATACAGATGCCATAGACAGAGAAA
ACAAATGGTGGTGTCAACATGAGCCCCACCTCAGGTAACAAGGACAAGCTGGATTCCAGAGGCAGGGTTGTGTGGGACAACTCCGT
GAAGTCCACACAGCAGAGAGGAGCTACAGGGAGGTGTTTATTTCACAATGGTTCGTGATTTTTTTGACCAATGCTGGATGGGCAGCT
AAGATATAGTATCAAAAACACATTAACTATAAATGGGTGAATTTTAAATAGCCACATGTACACCTCCATTTTTAGACTGTTGAGAAG
CCATTAAAAATTAGTTCCTCCTGTGATTATTCACAGGGATGAAGTATTCCGAGGGATTGACAGTTTGTGGCTAAGCCATTTAAAAGG
CTGTATAAACTGGGTATTTTAAATTAGTCTTATAAGTGAACTTCAGTATTACTATGGAGTATTTTTCTTAAAAATTGTATGCTTGTCA
TCTTTTTGGACATTCTTTGGGTCAGAGACTCAAGTGATTCATGCAGGAGGTCACAGTGAGTAGTGACTGCTGAATTAGGAGGTAGGGC
AGGGCAGAGGTATGTCTCTGCTCTTCTCAGCTGACCGGTCGTGTGTTGGTTACTTTGTCCACCAAACAGTTCTGGCACCAACTTTGG
AAGGGTGATGCGATATTATGCTCAGTGAACTGATTAGTGCCCCCACAATTGAACCTTAGCCAAGTTGTTGGTTGATAATTGGCACAT
TTTTTCTGATATGCTGATAATCCAGTTACTGCTTCAAGTTTATATTAAAATGTTTGACTCTTTCAAACCTTGCGGTTTTTGATGATG
TCAGTAGGTCACCAGACTTTCTGAAAATAGTTCTTTGTTTCAACAACAACAACAACAAAAAAAGAGAAAAAGAGGATAACACATAAGCAA
TAACTTACCATTATTGTTTTTTAAACCTTCACAAACTGACTTCTTTCATGGAAATACTGAAATATTTATTTTGTATTTAATTTATTA
TATAATTTAAAATGTAGAGCGAGTGAATTCAGTTTGGGATCTACTGAGTCACAGTGCCTGGTAGCAGTTGGATGTTGTAAGAGTGAA
GTCTGAGTGGGAGAAAAGGGTTGAGGAATCTCAGCTGAGTGTCCCTGAGCAAGCTGCATAACATCTCCAAGCCTTGGTTTTCACATC
TGACAAATGGCATGAGACCTGCTCTTTAGGGTTGTATTAGAGGATTAAATAGTCACATAAGGAGTACTGAGACTGTAGTATGTGCTCA
GTAAATATTCTCTTTTCTCTCTCCCCTCTTTTTGGGTATGAAGGTAGTTGTAGTTGTAAAATTTTCTTCCACACTTAGGAACAGATAT
TTCTCTAAATGTAAGCCCCCTGGGCTTATTCTAGTTTGTTCGTACATCTTAAAAATGGAAACCTCAGACTAAATATAGTATTCAAGT
ATAAATCCCACTACTGCCTAGATAATGGTAAAGACATTACTTCATAGTCTTTGCGTGCCTTGTTCCCATTAATACATATCATGTCAT
GTTAGCTGTTTTATAAGACAGTTCATTGCTGATTCATCTCACCAATTCAGTTCCAGAACAGCACCGTGTCAAGGATGCAGTGGCTCT
GTCATTTTGATGACGAGATGTTAGAACAGTTTTCAGTGTAAGAATCTGCTTAGCATTACAAAGCATTCATTTCTGTGGAAAGAAGTC
TTCACAAAGCCCGGGAATTGACTAAAATAACCACCCCTTTTTTAGGGGTTTGGTGTAAAGAATAGAACATATTTGAAGCGTGGGTGA
TTGGTGATGGGGAAAGTTACATGAACTGAGTTTTGGCTTCATTTCCCGGAGTGATGATAATGAGTTTACTGGGTGCTGCAGTACTAC
GAGATCACAGAGTGCCAAGACCTCAGTGCAGTGCTGCGTGATTGAAATGACTGATACTCTCTTTTTTTTTTCATGCTAGTTCAGCCT
GTTTTCATACACTCCAGAGGTTCAGTAAGCATCAAACAGGGAAAAGGAAATGGCAGTCCAATAGAGCGGTCGTTCAAAATGACCAAA
GTAATTCCAGAAATTCAAGAAAGTAGTTGTTTCTCAAGTGTCCATTAGGATCGGGCTGCTCTTAGAGTCTGCATAACTCGGAAGTGAT
TAGAGGGGTTTTGTGGCTTTTCTGTTCTCTGATGACTGGAGCTGGTTCAGCCTTCAGAGTAAGTAGCCAGAGAGTAAGATAAGCA
TGCTGAGGAGCTGAAGTGGGCACCCAATTTTATTTTCCATTGTGAAGGAGATGAGCATCTGTAACCCACAGCAAGATCAACCACCACA
TTAAATTTATATTGTTTGAGACAGTTCTGATCCACTTGAGTGTGTGTGTTCCACTAACACTCTGAGATGGTTGCATAGGCCACAACT
GTTCTGTCCAGTTCCTGCCTGATAGGTAAGCTCTCTGGGTAACTTTATTCCCATGAATTGAGATAGCAGAAGACAAACACCACAGTG
GCATTGGTGTATCCAAGGCACTGATTTTGTTTTTCTTCCATGGCTGCCTAGTTTGAGCTGGCCAACTAACATCATGGTATTAGCCTCAAAAT
GTATTGACAACAGATTTGTTTTGATAGCAAAATTGCTTTTATACTTGAGAAATCTGATCTTTTATAAAAGGCCCAGGGTAAATGTCC
TTTAGCAACATCATGAACATGTGTGATATCAGTGATATGGTGGGGAGATGTTCCTCAGCAAAGGATGTCTGTATTTGTTCCTAGGAT
TGACAAAGCTGTAAAGCAAAGTGCCTGGTAGGGGGGGAGTCTAAAGGAACTAGTAGGTAAAATGATTAGAGAGCTTACTGTAGTACAG
ATGGCGATATGCAGTCTACAAATGACATCTCCTTGCCACCACTGTTACTATGGGTTCTAATGAAAAGAACCTGAGACTTTTGAGAAT
TTTTGAGTTCAAAGGTATAGAAGAAGGAAAGAAAGCTGTGAAAAGCAAACATTGAGGGACAGAGAATTTGGCTAGGTAGGTCT
AGACTGTAAATACAGTAAAACTTCCCACACTACAGTGGTAAAGTAAAAATTTTCCCCACTTTACAGCATACAGCCACCTTCTCGCCATA
TTCCAAACTTTTTCATTGCTATTATACCTGGTATTATTTCACTTCACTTTATTGCATTGCTGTATGTCATATGTGTTAGGAGCCTGT
TTGGAAGAAGAGCAGCATTGTAGAATTATGGAGACAGCCATCTCTTGCACCCGTGTTTGAGCATCTTCATTTAAAGGGGTTACCAGT
CCCCTTGCATAAATGTCTATTTAATCAGCTTGCTTTCCTAATCTTCTGCATGAGCAATTCTGTCGCAGGGCAAAAGCGTTGATTGTAG
GGGAAGAAGAGAGGGAAAGGAGAAAATGTAGTTTTTGGAAACAGAACATCAGTGCCCATTTTTCAAATGAGGGTAGGCCAGGTTCTGTAGCTTCCTCAG
GGATTTAGAGTAGGGTTGATATGAGAGCTGTGAGTACCTAGGCATCTGTGGAGAAGAGGGAGTGGAAGAGAGGTAAGTGAGGCTTTG
TCAAAAAGAAATTTTGAAATGAGTAAAGTGTTTCTGGTTTTGCCAAAAAAGGAGAGGAAATTCTCAATAACCTTCTTATACCACAGG
CTTCCTCCTTATCAGACCAATAGTGATCGTATCAGACATGCTGCAGATTAAAAAGACATTTGTGGGTCAACCTTTAATTTTTACCAT
TAATCACAGGAATGCTTTGTTTTATTGTGCTTCACTTTATTGCTCTTCACATATATTATGTTTTTGGTTTTTGGGTTTTTGCTTCCT
AACAAATGCAAGGTTTGTGGCAACCCTTCATGGAGCAAGAGTATCAGTGCCCATTTTTCCAATCGCATGTCTCACTTGTCTCTCTG
TGTCACATTTTGGTAATTCTCACGACATTCCATACTTTTTCATTGTTACTATATCTGTTATGGTGATCTGTGATCAGTGATCTTTGA
TGTTACTATTGTAATTGTTTTGGTGTGACATGAACCACGCCCATGTAAGAAGGCAAACTTAATAAATGTGTGTGTTCTCACTGCTCC
ACCGACTGGCCATTCTTGTCTCTCCTCCTCAGGCCTCCGTATTCCCTGAGACACAACAATATTGAAATTAGGCTGATGAGTAATGGT
TTCTCAGTGTTTAAGTGAAAGGAAGAGTCACATGTCTCTCACTTTAAATCAAAAGCTACAAATGATTAAGCTTAGTGAGGAAGGCAT
GGTGAGAACTGAGATAGACCAAAAAACTTGGGCCAAACCATTAGCCAGTTGTGAATACAAGGAAAAGTTATTGAAGGAAATTGAA
AGTGCTATTCCAGTGAACCCATGAATGATAAGAAAGCAAAACAGTCTTATTGCTGATATGGAGAAAGTTTTAAATAGAAGATCAAAC
CAGCCACAACACTCCTTAAACAAGAGCCTAATCCAGAGCAAGGCTCTAACTGTCTTCAATTCTGTGAAGGTTGAGAGAGGAGAAAAA
AACTACAGAAGAAAAGCCGGAGTCTAGCCAAAGTTGGTTCATGAGGTTTAAGGAAAGCAGCCATCTTCATAACATAAAAGCGCAAGG
TGAAGCAGCACGTGCTCATGTAGAAGCTTCAGCAAGTTATCCAGAAGATCTAGCTAAGATAATTGATGAAGGTGGCTACACTAAACA
ACAAATTTCTGATGTAGATGAAACAGCCTGCTATTGGAAGAAGATGCCATCCAAGACTTTCATAGCTAGAGAGGAGAAGTCAGTGCC
```

```
TGGCTTCAAATGACAGGCTGACTCCCTTGTTAGGGGCTAATGCAGCTGGTGTCTTTAAGTTGAAGCCAATGATCATTTGCCATTCCG
AAAATCCTAGGGGCCTTTAAGAATGATGCTGACTCTATAAATGGAACAACAAAGCCCAGATGACAGCACATCTGTTTATAGCCTGGTT
TACTGAATATTTTAAGGCCACTGTTGAGACTTATTGCTTATAAAAAAGATCCTTTCCAAAATATTACTGTTCCAGTGTGATAATGTA
CCTGGTTACCCAAGAGCTCTTATAGTACAAGGAGATTAATTTGCTTTCATGCTTGCTAACAAAACATCCATTCTGTAGTCCATGAAT
CAAGGAGGAATTTGACTTTCAAGTCTTATTATTTAAGAAATACATTTTCTAATGCTGCCATAGACTATATAGTGATTCCTCTGATTG
ATATGGGCAAAGGAAATTGAAAACCAGGAAAGAAGTCATCATTCTAGATGCCATTAAGGTCCTTCATGATCCATGGGAGGAGGTACA
AATATAAATATTAACTGGTGTTGGGGAGAAGTTGATTCCAACCCTCATGGTGTCTTTGAGGGATCCAAGACTTTAGTGGATGTGGT
ACAGCAGATGTGATCAGATAGCAAGAGAACTAGAATTAGAAGTGGAACCTGAAGATGGGCCTGATTGCCACAATCTCATGATAAAA
CTTGAATGGATGAGGAGTTGCATCTTATGGAGGAACAAAGAAAGTGGTTTCCTGAGATGAAGTCTACTCCTGGTGAAGATGCTGTGA
ACATTGTTGAAACGACAGCAAAGGATTTAGAATATTCCATAAACTTAGTTGATAAAACAGGGTTTGAGAAGATTGACTCCAGTTTCA
AAAGACGTTCTACTGTGAGTAAAATGCCATCTGACAGCATAACATGTTACAGAGAAATCTTTTGTGAAAGGAAGAGTCAATCAATGC
TGCAAACTTCATTGTTGTCTTATTTTAAGAAATTGCCACAGCCACCCCAACCTTCAGCGCCACCACCGTCATCAGTCAGCAGCCATC
AACATTGAGGCAAGGCCCCCCACCAGCAAAAAGATTACGATGTGCTAAAGGCTTAAATGATTTTCAGCATTTTTGGCTATAAAGTAT
TTTTAAATTAAGGTATGTACATTTTTTAGACATGATGGCATTGCACACTTACTAGATGCAGTATAGTATAAATATAACTTTTATGTG
TACTGAGAAAAAAATTGTGTGACTCGCTTTATTGCAATATTTGCCTTAGTACTGTGGTCTAGAATGGAACCTGCATATCTCCAAGAT
ATATCTGTAGTGTGATTATTTTGGAAAACCATTTAGTAGTTACCCAAGATAAGTTAAAACATGTCCACAAAAAAGACTTGTATGTGA
ATGTTCATAGCAACATTATTCACAGTGGCCAAAAACTGGAAACAACCCAAGAATCCATCACCTAGTGAATGGTTAAACAAATTGTGG
TATATCCATAAAATGGAATACTACTCAGCACAAAAAGGGAACAAATTTACAGATACATACAACAGCATGCATGAATTCCAGAAAAAT
TGTGCAGTCAAAGGAATAAAACTCAGAAGACCACATACAATGTTCATTCATATGAAGTTCTGGAACAGGCAAAACTAGTCTGTAGGT
ACAGAAAGCAAATCAGTGGTTTTCTGGAGTTTGACATGGGGGTACTAACAAGGGAACTTTTTGGGGTGGTGGGAATGCTGTATATTT
TGATTGTGGGGATGGTTACATGGTTGGATGCATTTGTCAAAACACACTTAATGGTAATGCAAAATGAATATATTTTATTTTATGTAA
ATTATACCTCAAAGTTGAATTTTTTTAAAAAGTTTCTTTTAAAAAGTAAAGACATTTGTGGTGCTTCTTGTAAATTTTACTGCTGAT
TATGACCTTGTTTCTTTAGATTTGACTTTCCAAGTTTGAAAAGACCAGTTTAAAAATGACTTTTGCTGGGCATGGTGGCATGTGCAC
GTAGTCTTACCTACTCAGGAGGCTGACGCAGGAGGGTCCCTTGAGCCCAGGAGTTGGAGGCTACAGTGAGCTATGATTATGCCACTG
CACTCCAGCTTGGGTGAAACTGTGAGACCCTGTCTCTAAAAACAAAAATAAAAAAAAATTAAAATGGCTTTTTAGATTATAATCTAT
ATGAAATTGGGGGTTATTTCGCACTTTTATATTATTTGTCCATTTTGCTTCTTTCTAAATAGCCTCTGGATTGTGTGTTATTTTCAG
AAAGCTCTTGGAGATTTTTTGAAGTGTATGTTTTAACAGGATTTTCCTGTTGCTCTTTGGCAAACTTTGTCCTTTTATTGTTTAAGG
AGTATAAATAACAACAGAACAGAATTACTTTCTGGTGCATTTTCCTCTTCTCCTGAGTCTTTTATGTTCAATAACTCTGAAGTTCCT
GTTATTCCTGCCTTTGAACGTAGAAAATGAGACTCTCTCTCAAATACCTGAAACTAAGGCACATAAAAAATTTAGGAGGGGCCAGGC
ACGGTGGCTCATGCCTGTAATCCCAGTACTTTGGGAGATTGAGGCTGAGGTGGATGGATCGCTTGAGCTTGGCAGTTCAAGACCAGC
CTGGGCAACATGGTGAAACCCCATCTCTACAAAAACAAAAATTAGCTGGGTATAGTGGCACGCACCTGTAGTGCCAGCTACTTGGGA
GGCTGAGGTGGGAGAATTGCTTGAGCCCGGGAGGTGGAGGTTGCAGTGAGCCACAGACCGCGCCACTGCACTCCAGCCTGGGTGACAG
AAGTGAAAAGAAACACTGTCTCAAAAAAAAAACAGGAGGGGAGGAGCCAAGATGGCCGAATAGGAACAGCTCCGGTCTACAGCTCCCA
GCGTGAGCGACGCAGAAGACGGGTGATTTCTGCATTTCCATCTGAGGTACCGGGTTCATCTCACTAGGGAGTGCCAGACAGTGGGCG
CAGGCCAGTGTGTGTGCGCACCGTGCGCGAGCCGAAGCAGGGCGAGGCATTGCCTCACCTGGGAAGCGCAAGGGGTCAGGGAGTTCC
CTTTCCGAGTCAAAGAAAAGGGGTGACGGACGCACCTGGAAAATCGGGTCACTCCCACCCGAATATTGCGCTTTTCAGACTGGCTTAA
GAAATGGCGCACCACGAGACTATATCCCACACCTGGCTCAGAGGGTCCTACGCCCACGGAATCTCGCTGATTGCTAGCACAGCAGTC
TGAGATCAAACTGCAAGGCGGCAACGAGGCTGGGGGAGGGGCGCCCGCCATTGCCCAGGCTTGCTTAGGTAAACAAAGCAGCCAGTA
AGCTCGAACTGGGTGGAGCCCACCACAGCTCAAGGAGGCCTGCCTGCCTCTGTAGGCTCCACCTCTGGGGGCAGGGCACAGACAAAC
AAAAAGACAGCAGTAACCTCTGCAGACTTAAGTGTCCCTGTCTGACAGCTTTGAAGAGAGCAGTGGTTCTCCCAGCACGCAGCTGGA
GATCTGAGAACGGGCAGACTGCCTCCTCAAGTGGGTCCCTGGCCCCTGACCCCCGAGCAGCCTAACTGGGAGGCACCCCCCAGCAGG
GGCACACTGACACCTCACACGGCAGGGTATTCCAACAGACCTGCAGCTGAGGGTCCTGTCTGTTAGAAGGAAAACTAACAACCAGAA
AGGACATCTACACCGAAAACCCATATGTACATCACCATCATCAAAGACCAAAGTAGATAAAACCACAAAGATGGGGAAAAAACAGA
ACAGAAAAACTGGAAACTCTAAAACGCAGAGCGCCTCTCCTCCTCCAAAGGAACGCAGTTCCTCACCAGCAACAGAACAAAGCTGGA
TGGAGAATGATTTTGACGAGCTGAGAGAAGAAGCCTTCAGACGATCAAATTACTCTGAGCTACGGGAGGACATTCAAACCAAAGGCA
AAGAAGTTGAAAACTTTGAAAAAAATTTAGAAGAATGTATAACTAGAATAACCAATACAGAGAAGTGCTTAAAGGAGCTGATGGAGC
TGAAAACCAAGGCTCGAGAACTACGTGAAGAAGTACGGCAGAAGCCTCAGGAGCCGATGCGATCAACTGGAAGAAAGGGTATCAGCAATGG
AAGATGAAATGAATGAAATGAAGCGAGAAGGGAAGTTTAGAGAAAAAAGAATAAAAAGAAATGAGCAAAGCCTCCAAGAAATATGGG
ACTATGTGAAAAGACCAAATCTACGTCTGATTGGTGTACCTGAAAGTGATGTGGAGAATGGAACCAAGTTGGAAAACACTCTGCAGG
ATATTATCCAGGAGAACTTCCCCAATCTAGCAAGGCAGGCCAACGTTCAGATTCAGGAAATACAGAGAACGCCACAAAGATACTCCT
CGAGAAGAGCTAACTCCAAGCACATAATTGTCAGATTCACCAAAGTTGAAGATGAAAGAAAAAAAATGTTAAGGGCAGCCAGAGAAAG
GTCGGGTTACCCTCAAAGGAAAGCCCATCAGACTAACAGCGGATCTCTCGGCAGAAACCCTACAAGCCAGAAGAGAGTGGGGGCCAA
TATTCAACATTCTTAAAGAAAAGAATTTTCAACCCAGAATTTCATATCCGGCCAAACTAAGCTTCATAAGTGAAGGAGAAATAAAAT
ACTTTATAGACAAGCAAATGCTGAGAGATTTTGTCACCACCAGGCCTGCCCTAAAAGAGCTCCTGAAGGAAGCACTAAACATGGAAA
GGAACAACCGGTACCACCCGCTGCAAAATCATGCCAAAATGTAAAGACCATCGAGACTAGGAAGAAACTGCATCAACTAATGAGCAA
AATCACCAGCTAACATCATAATGACAGGATCAAATTCAACAATATTAACTTTAAATATAAATGGACTAAATTCTGCAATTA
AAAGACACAGACTGGCAAGTTGGATAAAGAGTCAAGACCCATCAGTGTGCTGTATTCAGGAAACCCATCTCACGTGCAGAGACACAC
ATAGGCTCAAAATAAAAGGATGGAGGAAGATCTACCAAGCCAATGGAAAACAAAAAAAGGCAGGGGTTGCAATCCTAGTCTCTGATA
AAACAGACTTTAAACCAACAAAGATCAAAAGAGACAAAGAAGGCCATTACATAATGGTAAAGGGATCAATTCAACAAGAGGAGCTAA
CTATCCTAAATATTTATGCACCCAATACAGGAGCACCCAGATTCATAAAGCAAGTCCTGAGTGACCTACAAAGAGACTTAGACTCCC
TGAACTCAGCTCTGCACCAAGCAGACCTAATAGACATCTACAGAACTCTCCACCCCAAATCAACAGAATATACATTTTTTTCAGCAC
CACACCACACCTATTCCAAAATTGACCACATAGTTGGAAGTAAAGCTCTCCTCAGCAAATGTAAAAGAACAGAAATTATAACAAACT
ATCTCTCAGACCACAGTGCAATCAAACTAGAACTCAGGATTAAGAATCTCACTCAAAGCCGCTCAACTACATGGAAACTGAACAACC
TGCTCCTGAATGACTACTGGGTACATAACGAAATGAAGGCAGAAATAAAGATGTTCTTCGAAACCAACGAGAGCAAAGACACCACAT
ACCAGAATCTCTGGGACGCATTCAAAGCAGTGTGTAGAGGGAAATTTATAGCACTAAATGCCTACAAGAGAAAGCAGGAAAGATCCA
AAATTGACACCCTAACATCACAATTAAATGAACTAGAAAAGCAAGAGCAAACACATTCAAAAGCTAGCAGAAGGCAAGAAATAACTA
AAATCAGAGCAGAACTGAAGGAAATAGAGACACAAAAAACCCTTCAAAAAATCAATGAATCCAGGAGCTGGTTTTTTGAAAGGATCA
ACAAAATTGATAGACCGCTAGCAAGACTAATAAAAAAAGAGAGAAGAATCAAATAGACACAATAAAAAATGATAAAGGGGATATCAC
CACCGATCCCACAGAAATACAAACTACCATCAGAGAATACTACAAACACCTCTACGCAAATAAACTAGAAAATCTAGAAGAAATGGA
TACATTCCTTGACACATACACTCTCCCAAGACAAAACCAGGAAGATTGGAATAGACCAATAAGACAGGCTCTGAAATTGT
GGCAATAATCAATAGTTTACCAACCAAAAAGAGTCCAGGACCAGATGGATTCACAGCCGAATTCTACCAGAGGTACAGGAGGAACT
GGTACCATTCCTTCTGAAACTATTCCAATCAATAGAAAAAGAGGGGAATCCTCCCTAACTCATTTTATGAGGCCAGCATCATTCTGAT
ACCAAAGCCGGGCAGAGACACAACCAAAAAAGAGAATTTTAGACCAATATCCTTGATGAACATTGATGCAAAAATCCTCAATAAAAT
ACTGGCAAACCAAATCCAGCAGCACATCAAAAAGCTTATCCACCATGATCAAGTGGGCTTCATCCCTGGGATGCAAGGCTGGTTCAA
TATACGCAAATCAATAAATGTAATCCAGCATATAAACAGAGCCAAAGACAAAAACCACATGATTATCTCAATAGATGCAGAAAAAGC
```

EP 2 204 376 A2

```
CTTTGACAAAATTCAACAACCCTTCATGCTAAAAACTCTCAATAAATTAGGTATTGATGGGACGTATTTCAAAATATAAGAGCTATC
TATGACAAACCCACAGCCAATATCATACTGAATGGGCAAAAACTGGAAGCATTCCCTTTGAAAACTGGCACAAGACGGGGATGCCCT
CTCTCACGGCTCCTATTCAACATAGTGTTGGAAGTTCTGGCCAGGGCAATCAGGCAGGAGAAGGAAATAAAGGGTATTCAATTAGGA
AAAGAGGAAGTCAAATTGTCCCTGTTTGCAGACGACATGATTGTTTATCTAGAAAACCCCATCGTCTCAGCCCAAAATCTCCTTAAG
CTGATAAGCAACTTCAGCAAAGTCTCAGGATACAAAATCAATGTACAAAAATCACAAGCATTCTTATACACCAACAACAGACAAACA
GAGAGCCAAATCATAGGTGAACTCCCATTCACAATTACTTCAAAGAGAATAAAATACCTAGGAATCCAACTTACAAGGGATGTGAAG
GACCTCTTCAAGGAGAACTACAAACCACTGCTCAAGGAAATAAAAGAGGACACAAACAAATGGAAGAACATTCCATGCTCATGGGTA
GGAAGAATCAATATTGTGAAAATGGCCATACTGCCCCAAGGTAATTTACAGATTCAATGCCATCCCCATCAAGCTACCAATGACTTTC
TTCACAGAATTGTAAAAAACTACTTTCAAGTTCATATGGAATCAAAAAAGAGCCCACATTGCCAAGTCAATCCTAAGCCAAAAGAAC
AAAGCTGGAGGCATCACACTACCTGACTTCAAACTATGCTACAAGGCTACAGTAACCAAAACAGCATGGTACTGGTACCAAAACAGA
GATATAGATCAATGGAACAGAACAGAGCCCTCAGAAATAATGCCGCATATCTACAACTATCTGATCTTTGACAAACCTGAGAAAAAC
AAGCAATGGGGAAAGGATTCCCTATTTAATAAATGGTGCTGGGAAAACTGGCTAGCCATATGTAGAAAGCTGAAACTGGATCCCTTC
CTTACACCTTATACAAAAATCAATTCAAGATGGATTAAAGATTTAAACGTTAAACCTAAAACCATAAAAACCTAGAAGAAAACCTA
GGCATTACCATTCAGGACATAGGCGTGGGCAAGGACTTCATGTCCAAAACACCAAAAGCAATGGCAACAAAAGACAAAATTGACAAA
TGGGATCTAATTAAACTAAAGAGCTTCTGCACAGCAAAAGAAACTACCATCAGAGTGAACAGGCAACCTACAACATGGGAGAAAATT
TTCGCAACCTACTCATCTGACAAAGGGCTAATATCCAGAATCTACAATGAACTCAAACAAATTTACAAGAAAAAAACAAACAACCCC
ATCAAAAAGTGGGCGAAGGACATGAACAGACACTTCTCAAAAGAAGACATTTATGCAGCCAAAAAACACATGAAAAAATGCTCATCA
TCACTGGCCATCAGAGAAATGCAAATCAAAACCACTATGGAATCATCTCACACCAGTTAGAATGGCAATCATTAAAAAGTCAGGA
AACAACAGGTGCTGGAGAGGATGTGGAGAAATAGGAACACTTTTACACTGTTGGTGGGACTGTAAACTAGTTCAACCATTGTGGAAG
TCAGTGTGGCGATTCCTCAGGGATCTAGAACTAGAAATACCATTTGACCCAGCCATCCCATTACTGGGTATATACCCAAATGAGTAT
AAATCATGCTGCTATAAAGACACATGCACACGTATGTTTATTGCGGCACTATTCACAATAGCAAAGTCTTGGAACCAACCCAGATGT
CCAACAATGATAGACTGGATTAAGAAAATGTGGCACATATACACCATGGAATACTGTGCAGCCATAAAAAATGATGAGTTCATATCC
TTTGTAGGGACATGGATGAAATTGGAAACCATCATTCTCAGTAAACTATCGCAAGAACAAAAAAACCAAACACCGCATATTCTCACT
CATAGGTGGGAATTGAACAATGAGATCACATGGACACAGGAGGGGAATATCACACTCTGGGGACTGTGGTGGGGTCGGGGGAGGGG
GGAGGGATAGCATTGGGAGATATACCTAATGCTAGACGACACATTAGTGGGTGCAGCGCACCAGCATGGCACATGTATACATATGTA
ACTAACCTGCACAATGTGCACATGTACCCTAAAACTTAGAGTATAATAAAAAAAAAAAAAAAAAAAACAAAAAACAGAGATCCCTTAGT
TAGGTAAACCAGGCCCTTTCTGATTGGTTGCTGTGAATCCTCCTGGTTTTTTGAATACTGGCTCATTTTGAAGTTCAGTTTCATTATG
TGGCACCGAACAGGAGCGATTCCATACTGGTTCTGTCTGGTCTTTTGGGGCCTAGAGCACAGCCTCAGCCCTAAACAATGGCTTCTC
TTCCACTTTGTTTAACAGTTCTCTGAACTCTTTCAAATTAACGTTTCCGTATGGCATGAGATAAAGGTAATGGTTCATTTGTTCTCC
ATGTGGCTATCTAGTTGTTATAACACTATTTATTGAAAGTGTATCTTTTCTTTATTGAATTACTATCATATCTTGTTAAAAATCGA
GGATATGTGTGTAGGTCAATTTCTGGATTCTCCATTCTGTTCCATTGATCTCTTGGCCATATACCACACTGTCTTGATTACTGTAGC
ATTATAATAAATCTTTAAATAAGGGAGTATAAGTCCTTCAACATTGTTCTTTTTAAAAATTCTTTTGGCTATTTTTGGTTCTTACCA
TTTTNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNTTTTT
TGTACTAAGTCATTGAAATCTGGTGTGTTTTTACACTTACAGCACACCTTAGTCACCTTAGTCTGAATTAAGATTCAGACTAGCCAC
ATATCAAGTGTTTAGTAGCCTCATGTGGCTCATGGATACCATACTGAACAAAATAACTATAGATCAATTTGGAGATAATTGTCAAAT
AAATAGTATTTAGTATTCTTAACAAAAAATGAATTTAAAAGTGTATTATTAAGTGTCTTATTTATCTAAACAGGTATTAGTGTACAG
TTCTTGCATGTACTTTGTTAAATTTATTCCTAAGTACTTCAGGTTTTTGCGTGCTGTTATTAATGGTATTGTTTTTCATGTTTTAGT
TTTTAATTGTTCATTGCAACTTATATAAATAATTGAATTTTGCGTATTGGCCTGGTATATTCTGCAACTTATTGAAAGTGTTTACTA
GCTCTGGTATGTTATTTTGTTGACACTTTAGAACTTCCTACCTAAAAAAAATATGTCTGTAAATAAAAGCAGTTTTCACTTATTAAA
AAAAAAAAACAAAAAAACAAAAAAAAGGAAAAGCATGGCAGGGCAAAGTTGCTCATTTCTATTGGGCTATGACTGGGAACCAAGAGT
TAGGGAAGTTGGTTTCCTACTTGATGTACACACCAGTCTTTGAGGTTTACTGATTACAGGTACCCAAAGTGTGTGTTCCTGCTTGCC
TTCTCAGGTTCCTGTGTGGATGGATGGTTATCTTAATGCTGGTAATTAGGCTAAAGGAGCCATTTCACCCTTGTGGGGTCTTAGTGA
GCTGATAATAGACTAGTCTTGGAGTTCAGTGACCTGTGTTCAGAACATGACACTGATCAGCACTGTGACCTCGGACAAGTTAATTC
ATCTCTCTCTCTCTCTTTTTTTTTTTTTTTGAGACAGTCTCGCTCTGTCGCCCAGGCTGGAGTGCAGTGATGCAACCTCGGCTCAC
TGCAACCTCTGCCTCCCGGATTCAAGTGATTCTCCTGCCTCAGCCTCCTGAGTAGCTGGGACTACAGGCACATGCCACCACACCTGG
CTAATTTTTGTATTTTTAGTAGAGACGGGGTTTCAGCGTGTTAGCCAGGATGGTCTCGATCTCCTGACCTCGTGATCTGCCCACCT
CGGCCTCCCAAAGTGCTGGGATTACAGGAGTGAGCAACCACGCCTGGCCAATTCATCTCTTGAGCCTTAGTGTCCTCACCTGTAAGA
TGGGAATATCCACTTTACAGAGTTTTCTGGAAAATTAGATGAGATGAAATAAGTGAGAGTCATAGGTGTTGTGCCTGGCACATCATA
GGCACTTGGTAAACCTGAGTTCATTTCCTTCCTTTATTGGGCAGCTTCCTGGCTCAGGTCCCTATTTCACATAAATTGTATGCCTAG
CAGCACCCTATTCATTTTATAAATTGAATATGAGAATCAGATGATAAGAGTTTGGGACCATCTTTAGCAGATGGTTTTTGTTAAAGG
TACCTTTTGAAGTGGGGGTAGTGCCGTTTCATTTCATGGTCTGTGTTGTGTACCTTTGCAGCATGTCTTTTGTGATATGCAGTGATGT
GACTGGAATTTCGTACAGTAGGATGGGATACTGAAAGTAAATCTGATCATTGGTAGCACCTTTCCTGTAATTACAGCACAGGAGAGA
GGTAGTGTGACTATGTAAATAGAGCTAGAAGTGCTATTGAAATAACTCAGCAGATTCTTTAAAGAGAAAAAGGGGAGAAAAACCCCA
CCACCTTCCACAGAGACATTCCCATGCTAGGCCTGCACTGACACTGCAACACCAGATAGACGATTCAGTTTATTTTGCTCCAACATT
TTACGCGTTGGTCAGAGAAAATGGTTTTCATTAACACAAGGTAATGAAAAATTTTGTGCCATGGTATCATGTTTGTAAAAAGCTTAA
TTACATTGTTGCTGTTTCTGTTTTTGAACCTAATTCTGGTAGTCTGCGCGGTAGCTGGCGTGCCAGTTCCGGGTTGGGGTCTAGGCAG
TTGGAGAAAAGAAGCAATTATCCAGTTAATGTAAAGAATCCTGCTAAGGAGGAAAGAAAGGTGCCCCTCATGCCCTCATCAGTCACT
CCAATTGGTGTTTATAGGGAATTAAGTTCTCTGGTAATTGCCCTCCTTCCCTTTCCTTTGTCTGCTAGGGCCATCATCACAGGCCTT
AAGTGAACAACTCTGGGAGTATTTCCGGCCTCTTCTCTAAGTCAGGCCAGTAATGCAGCTGTTAAGCACTGACATCTGGGTCTCAGT
CATGCTTCCAGACCAGGATTGTTCTTAGTTCCAGGAGATTAATTGACCCATAAGAAATATTATAATGCAAATATATGTTTGCGCCAA
TTTGCTGACCAGTGCTTGCTATCCTGGAACTTCACAGTGTGGCAGAATACAACTGTCTGGCATTAAGGATAACCTAGGTCTTGTTTCC
ACTTGTTTCCAGCATGTACTAATCTAGGGTGAACTTTCTTCACACGTAGAGAGCATCAAAGAGGAAGGTGTTGAACAGGAGCCCTG
GATTAGGGATCAGGAGTGTGGAGTCTGGTTCCAACACTGTCACATTTCTGCTGTGTGTCCCTTTCCAGGCCCTTAACCTGTCTGGGG
CCTCAGTTTTCTCATGTATAGAATGCAGTAATCATAGCTTAATTTCTAGCCAGCTTTCTAGGGTTGTTAATCTATCCTTTTATCAAA
AGGATAAAATGATATGTTATAAATGAAGACACCTTAAAAAACAAAGCACTCTGCAAACGTAAGATGTTATTAAATGTAAACTCAATG
ACATTATTTCAACATTCACATTAGACAGAAACAACATTTGAAGTCTTCTAGGCCACACACAAGATTATTGTTAGAGCTTAAATTTCTCTTT
GGTGAAGTTCTTTTAGGACAGATAAATATGCCTCAGGGCCAGCTTATCTCTTTGAACATCGTAACTGGATATTCTTTGGCTTCCTTT
TTCCATTATTTAATCTTACTAAATTTCTCTTTTATTTTGAAGCATTGGGAATAATGAGAAAACTGGAAGATTTCAACAATAATGAGT
GGAAATAATATGAAGGACCTATGGGAAGAGAGAATAAATTATATTAAATATATTCAAAGCAAAAGATGAGAAAAGACATTCTTTAAA
AAATAGTGATAATGTTAAAATATCTTTTTTAGAAGTAATAGACTTGGGAACAGAAAAGGGATGTTGTTAACTTTTTTTTTTTTTTTGAA
CCGAAGTTAACACCCAGCACTGTCCTTTCTTTACTTAGCGAAACATACGGTGGAAACAGATTTCTGTGATCTTCTGCGGTCTGTTT
ACTGGCCACCCCATTCATGTGACATCGGCACACTTGGCTCATTTAAAAGGCTTCTCAGTGATGTCTTGAAAGCCTAATCCTTTTTCA
TTTTTATCTCTTTCATCCTGCAGGCCATGATGAAGTTAATTCGTTTTAACTTTCCGAGACACAACCACTTTTACAAAACAGGAAAATG
AAAGGACTCTGAAAAGTGTGACACAGAATATCTAGTTTGCAGAATAGACTGTGCAGGGCGGGAGGATGGCCGACGACGGGTTTCATT
```

171

EP 2 204 376 A2

```
TAGGAACTCAAAGTGAAAGGACAGGGCAAGAAGTGTAGTGAACACCTTCAGGGAAATGCCGAAGAGACCAACTGTTATTTGAAAACT
TGGATAGGTTTTGGGGCCCCAGATCCAAGCCACCTTGTTTTGTTGTTGTTGTTGTTGCTGTTTTTGAGACGGAGTCTTGTTCTGT
CACCAGGCTGGAGTGCAGTGGCATGATCTTGGCTCACTGCTGCCTTCGCCTCCTCAGTTCAAGCGATTCCCCTGCCCCAGCCTCCTG
AGAAGCTGGGACTACAGGCACGCACCACCATGCCCAGCTAATTTTTTTGTATTTTAGTAGAGACAGGGTTTCACCATATTGGTCAGG
ATGGTCTCAATCTCCTGACCTCGTGATCTGCCCGCCTCAGCCTCCTAAAGTGCTGGGATTACAGGCATGAGCCACTGCGCCCGGCCA
GCACCTTGGTGTTTATGGAGATGGCGATGTGCCTTGCTTCACTGGGGTATGGATGGGCGGAGTTTCTGTTCCAGACTTGCTTGTCTT
CCCCAACCCTGCCTACCTTCTTTAGAGGAGGAAATATATGTTCAGTCTTCATGGAGCTAAGAAAAAGCCAGACAGCCGAGGAAGAAG
CTAATAATTAGGCTTCTTTGGAAGGTATGAGACAAATTGGCAGCATGGTCACCCGGGAAAAACACAGTGGGGCTTTGTCTGCAACTAA
GAACTCTAGAAATCATGGTGTCTGGATACCACGGGGTTTAAGCTGTGGAAGCCTCAGCCTGCGTGTGGAAGCTGGGCTTTCTAATTG
GATCACTGGCACACACAGTGCTGTTGCTTCTTCTCTGCCCATGTCCCCCAGAGCATCTATCTGCTTTTGTTACTGCATTGTTATGAA
TGCCCTTCTTTGGCGCCCTCCACTTTTCCTGAAGGCAGGCTGGGAAGCAGCCCTTGGCATGTGTGATGGCTGCGCTGCATGCTGGCC
ATCGAAGGATGTCCATCCAAGGAAAAGCTGCTCTCCCTCAGCCCTCACCCTTGGCTTTTGCCCCACCCTGCCTCCCAACTGCCGGTG
CATTCAATTCCCTGCCTTTGTCCAAGGGGCAGGACAGGGCACGACCACTTTGGCTCCCCTTTTCCCTTTGGAAGCCAAAGTCACTCC
TCCAGCTTCAGAACTGTGAGTTTCTTGTGCATGCCGCTGGCTTGGTGGGAATATGAGAATGGGAACTCTAACTCTCACTGTGTGAAG
AGTTGGCAAGGGTGCATGTGCACGCTATTGTGTGTTATCTGTAAGAGAACTGCAAAAACAAGTGTCTAGAATTAGATTCAGTGCTTC
AGGACCCCCTCTCCTTTCTCCATTTAGATTCCTCAACAGAAGCTGCTTTTCGCTTGATTAACTGTAACAAACTCTCTTTGGAGTTTT
GTATTTTGGAAACTGCATTGCCAAAGGAAAATCATTATGTCTGCTGTAAAAGCCCCAAATAAATTCCTCCTAGGGTAAATGGAGGAA
AAAGAATAGAAGGGTTTAATCTATCATCTTGCCTATTAGTGAAATACTTAGTGACCTAGTTCATTTACATATTAAATGTGCTAGACC
TATTTGTGCCTTCTTTTTTTTTAACTTGTTACCTTGGAAACCATGCCCTGGCTCAGAAATTACACAAGGCCAACAGCAAATCTGCAG
TCTGGTTTCTTCACACAGACAAGTCAGTTAATGGGGAAAGGTTAGATTGTCCCAAAATGTCTTCAGTCGTTAGTAGAAAAAGGAGAG
GAAGGAGAAAAAGTGAGAAACATGTATTATTTTGGTAACATTTTCAAAATACTACTTTTAAACTAATTGGAAGGGATTTTTACCTAGA
CTTAAGAAGAAAATAATAGTGTGGACTCTTAAAACTTCAGTCTGCTTTTAAAGAACTTTCCTGGTACATTTGGCAGCACTGGCATGA
AAAGGATCAGCTATCAATTGACATTGAATTCCAGACCTACATTTAAGGAATCTTGACTCTCTCATTGCTTTGTTGTAATAGCACTGG
TTCTTCCAGATTGTTTGGTGGTGTCTTATGTTCTCAGGCCAACTTTTCAAGAGTCTGAGAAGCAGAATTTAAAAATACGATTTAGTT
AGCAGCTAGAGAGCAAAATTTCAGCCCATCTTCCCACCTGAATATGTAGCCTGCAAGTTCTGGGAGAACAAGGAACTTTACTGATCT
TCTACAGTGTCTCATTTCACAGTGGAGGAGATTGGCACCCAGAGAGGTGAAATAAATTGCCAAGGGCATCCATCTGGTTTGGGGCAG
TCTAGACTAGAAAACAGGCTTCTTAACACATCTTGCAGCATCATGCGATGGAACTGGAGGCTATTATCCTAAGTGAAATAACTCAGAA
ACAGAAAGTAAAATACTACATGTTCCCACTTACAAGTGGAAGCTAAACAATGGATACACATGGACATAGAGAGTGGAATAATAGGCT
GGGCGCAGTGGCTCACGCCTTTAATCTCAGCACTTTGGGAGGCTGAGGCAGGTGGATCACTTGAGGTCAGGAGTTTGAGACCAGCCT
TGCCAACATGCTGAAAACCTATCTCTACTGAAAATACAAAAAATGGCCAGGTGTGGCGGTGCATGCCTGTAATCCTAGCTACTTGGG
AGGCTGAGGCAGGAGAATTGCTTGAAACCCGGGAGGCAGAGGTTGCAGTAAGCCAAGATTGCGCCACTGCACTCCAGTCTGTGTGACA
GAGCAAGACTGCCTCAAAAAAAAAAAGAGAGAGTGGAATAATAGACATTGGAGACTACCAAAGGTGGGAGGCTGAACGTTGAAGTA
TTGCTTGTTTGTTACAGTGTTCACCATTCGGGTAATGGGTACACTACAAGCCTAGAGTTTGTTGCTATGCAATATATGCACACCAGA
AAATATGCACTTGTACCCCCTAAATGTATAAGAATTTTGAAAAAACTTTTTAAAAATTAGGTTTTTTGACTGCAGATCTAGTGCTCT
TTCAACATTAGTCCTGACACAGATAAGTCACTTGCTTTGAGTTCTGTCAGAGGTAAAGCTGGGTGAAGAAATTGTGAGAAGAATGCT
ATTCTCTTTAAGCCAGCCACCAGCCAGCTTACTTGGGAAGCGGTGGAAGGAGGACACAGTGTATCCTCAAATGTTAAATCCCTTCTT
AGTGGTATTTGTTGTGGATAGAAGGAATTAGATTGAATGTGGAACTTTGAGCACTCTGAGGATAAATTGTGCCTTCTCATTCAAGAC
ACATTCAATTCTCTTGGTCACTCCATCTTTTCTGAAGTTCCTTTTACCATGCTTTGCAGTGGCGATAGGCCTATTGCATGAAGAGAT
AAAATGTCTTTTAGGCTTTTTCCTCTTAAAAACTTGGGATAAGAACAAGTTAGCCTGAGGCAATCACAGAGTCTTTATAAAGAGACT
TTTAGCAAGGAAATGATTTTTCAAATACAGTACAGGATTTTGGGCATGACTTTGAACTGGAAAGTCTACATATATAAGTAGAAGATG
GTGTGTAATATCTTTGCAAATACTTTTGTGTATTCATTCCTCATTTGTAAATGTTTTCTTACCTGCTGACTCAAGCACTGACAGTGT
ATTTGTTTGCTCATTCAACTATGGACTGTGAACATGCTTGGGAAACAGAGTTGAGTATTGTACGCAATCTCTGACCTCAGTAATACT
TAGGCATATATGCTTATTCAGTAACTGGACATTTTAAAATAAAAAGAAATGAGTATTTTCACTGCTTGCATATAATGGGAATGATTG
AATAACTTTCCCATAAGATGGCATTAGTTCAAGTGTCCTTATTTTAGTAAGAGTATTTCAGGCCAGGTGTGGTGGCACACACCTGTA
GTCCCAGCTACTCAGGAGGCAGAGGCAGGAGGATCCTTTGAGCCCAGGAGCTAGACTCCAGCCTGGGTAACAAAGAAAGACCGCATC
TCTTTGAAAAAATAATTTGGAAGCTCTCCGTAAAAATCTCACCTCTTATTTTCCTCAAAACAAGCTCTGGTGCATTAGGGTATATAG
TAGGGTCCCCTGGCCTTTGGAATGGGGGGTTATTTGAGACTGGAGGTGGGAGTGGGAGAGGGGAGGGGGAGCCAGGGACAAGCATGG
CAAAGTTCCAGATTCTCTGTATTTGTGTGAAAAATCACAAAACCCTACATTGACTGTGTAGTGGATGTCAGATGGGAAGAGGGAGTG
GGGAAGCAATGAATAATAGCAATCACAGGTGCAGGAGCCACGTCTCCTCCTGGGCACTTTACATGCGTTCTCTCACTTATTCTTCTT
TCTCACCCTGTGAGGCTTGGTCCAGTTGCTATTCCCAAATCACCCATGAGGAACCTGATGCTGTGAAGATCAGCAGCATGGCCAAGG
CCGTGTAGTGAAGTGATGAGCAGGAGTTAGTCCCGGTCTTTCTGTTGGTGAGCTTACCAGCTCTTGCCTTCACACAGTGCTCTCTC
CTCTCTTATCAAGTTGAACGTGGGTCAGGACCCATGAAGTTAATTTTCTTCCTCCTTTTAATCATCAAAATGGAATTTCTGTTTTGCC
TGTTAGAGATTATTGATATAAGAAAGGCTGGGCGTTAAATGCTGAGAACACATGGACACATAGAGGGGAACAATCACACCCTGGGGC
CTGGTGGAGGGTGGAGGGTGGGAGGAGGGAGGGGGATTAAGAAACAACGAATGGGTACTAGGCTGAATACCTGAGTGATTAAACAGTC
TGTACAACAGACCTCCATGACACAAGTTTACCTGTGTAACAAACCTGCATTTTAAAAGTTAAAAAAATGAAAGGCTTTATATCAATA
AATAAGTGGGCCTAAAAAAGGGAGACAAAATGAAGGAAAATTATTCTGGATTCTTAAGAATAAAAGTGAAAATATCGGACTAAACTG
ATAAAATGAGGTAGAAAATAGAGGAATCACATACAGAGTTTTGACTTCCAACCCACTTTGCCTTAAGTTATTTCAGTTGACAAGTCG
AAGTTGGGTTGAGGGGATGAGGGTAGGAGGTGAGTGATGTATGGATAAATACAACGTTCTATTTTCTAATGGTAAGTATATCTGCCT
TCCCTCCCTCCAGTGCTTTTATACGGTACCAAGGAATATGTTTTGCTTTTTGTCTGACAAAGGGAATTCACAACTCATAACAGCTAT
TTGACATCTTTTGTGGGTACCAAGCTCACTTTTCTTCTTTCCAGTGATAAGTGCCTTAATAGAACAAAGTCAACATAATCCTTGCCT
CCAGGGAGCTTGCAGTTAATGAGGGGGGCACATGTTTACAATATAGTGGGGTCAGTGCTGGGGAGGCATTGAGGAAGAACTTAGTG
TATGAATCCATCTCCGAACTTCAGTGATGTGGAAAATAAGCTGTTAGAAAGATATCTTGGACTTCATTTTTCAAGCAGCTTGAACTC
CTCAAACAATTGTTCTGCAGACTTATTTGCCACGAGATTTGTGACTTGAAGTCTAGTGTTTAGATCACAGTGAGCTTTTTGGAGATG
AGATTTGTGCTCACATCTGTGGCAAGTCAACGTGTGGCAAATGCCTGTTTTGACTGTCAAAACGGAAAGCAAATCTCTTTGCGTGAT
TTTCTATGTCATTTTCTTTATTAGTGCACATGTCATGGGCGTTTCTTTTCCTTGGCGTTTTGTGTGAACTTAATCCTAGACAGAACC
TCAGTTTGAGTGGTTAAGGCTCAGCATCCTGGTCAGCGTCTGGTGTGTCCTGCTGGGTCCCTGTTGTGCCGTCAGCTCCAGTGGG
GATGGCACCAGGTTCAGGGAGGCTGAAGAAGTGACCCAGAGCCAGCAAACGCAACATGCGGTTGCGTCGGGGGCGTTACATACAGGGGA
GAGGGCCCAGCGGCTATGCGCTGGACAAGAGAACCGCAATCACTTGCAAAAGCTATGCAGTTTATATTGCATTTTCACTTAGTACTC
TGTTTAACGATTCCACCTGGCAACCTTCATTCATGCCAAAACTCGGGGCCTCCATTTCCCTACAGCCCATGTCCCACAAGAGATGGG
CCGGGGAGCTCAGATGTTCCTCATAGACAAGGAACAGATCTCTGGGGTGGCCACTTCCAGATCCCTAGCTCAGAACTCTGAACACATT
CGGGGGCATCTGCCATACAGAATCATTCTTAAGGATGCCTTAAGTTACTGCTATCAGGTGCATTTACTGTATACCCCTAAGTGACT
TCAACTTCTAGTCAGTCTAATGAAATTTGATTTCATGCAGAGGGTCTCTTAGACAAAAATTACATCTATAAGCAAATTACTGGCTGC
GCAGGTTACTTTGAAATATTTCTGGTGTTTTTTTAAAAAAAATCAGTTAAGCTGATCAGAGACAAAGGGAAGAAATTACCCTTTTCTT
TTTAATGTGCAGCAAAATGTGTACTAATGGTGCAATCACAGAATATTAGGTTGTTAATACAATCAATAAAAAGTTAATCCGTTTTGG
ATTTCATTGTATTATGTTTTTCTTGTTTGTGGAATAACGTAATTGTGACAGAGTAATTAATACCCTGCAGAGGGATTAGCCGTCAAG
ATTGAGATAAAAGAACAAGGAGACAAGAGCAGAGTTGCCTAGAAGACAGGGGCGTTTAGAAAGTGCTGAGGCATAGAGTTTCCTTAA
```

172

```
GGTGATTTTTTACCTTTCCTGACTCTAATTATACATTTTTGCATCTAGTAGTGATACAGCTGGGTTGGTAAAACATATTATTAATAG
AAGTGAGGGAGTTCTGAGGTGGATCAGATTTTTCCCTTTAAGCTTGGTTTCCAGCCAAGCCACCGGTCCTCAATTGGTATTTTGCTG
TGTGTTCAGAATGGCTTTTTCACTTCTGTAATTTACTTATTTCTTATTATTTGTTATTCAAAGATCTTTTGGTCATTTATTCTGCAC
AAATCCTAGAAAATGAAAGCCAAATAAGGAGGGATTCCCCCAAATGAGTACTAGTTTTTATATCAGTGCATGGTGGGCCTCTCAGTG
CCAACAGATTGTTCCCGTTAATTTTTTTTTCCCCTTTATATTGACTCTGGGGTTTCTAATGGTCAGCCTGTGCTTTCAGATTGGAGT
TATTAACTTTGACCAAAACAGGAGTGGTTTGAGAAGAGGCAGTAGAGAGGAGAAAGTGGAGATCTAGCCAGAATCTTTTGTTTTCCA
CAGAAATTAGAGAGGGAAAAGACTATAGGAATCAGACTCTGTCAGCCTTGTCCTTTGTAGCCCCTACCCAGGCAGCAAAGAGGAAAC
TGTCTTCTTCCTGTACGGGTCTCCAATTGGCTGACATTATAAATAGTCTCAGTGGCAGGTTTCTCATTTTTGAATAAAAAAACAGGC
TTTCATGCTAAATTATTAAACATTCTGTGGTTTGGCTTTAAAATCAAATAAATAACTCTCTTTATGAATGCATTAGACACCCTCCCT
AGTTTCCTTTTAATTGTTTGTTCTCCCCTGTTCCTCATCTTGGACTTCAAGGCCATCATCTTGGTATTCTGGTGTCTGCTCTTCTCT
TGCTGACTTCTCTCTGGGAAACCTTTTCCAGGTCGGCTTGTTCCACTATCACCCACCTCCCTGTGAAGGAAGATTGAAGCTGGTGTA
GATGGGGCTTCCTGCCTGCCCTGGAAGTCTGAGCCAAGAGTAATTTTACCCTCATCTTTCCACAGTGTGTATAAGAGTAAAAAAGGT
CAGCTGGACAACTTGCCTCGCTGGGATAGAGCAGTCAGAGTCAGATGATGTTTCTATGAACTGTTTTGTTGGGGTCAGATGGACTCA
ACTGTGCAGTCCACATCATCTGCCCCCATCTGCACTGACCTGATATGGTGGGTGGCCACATCCTAAAATAGTTTCTCAGAGACTTTG
TGTGTGAAACATACATATAAATGTGTTCATGTTTATAATGTTGGTGTGCAAGAGTTTATTTTGCTTAAAGAAGATAATGGAAATTTT
GGTTTCAACTAACATATCTATTTTCTCTCCACTAATGAGTATGGAAGGTTTCTCCTAGATGAAAACATGGAGAGAATCACTAATAA
GAAGAGAAAGCTGTTGAGTACTGTTAAAAATATCACAAAACAGTTACTATTATAGTCATATATATGATACGTTCATTCTTTTAGAGGA
TAATTTAAACATTATTTTGGGTATATAGGGTTTTAAAAAATCTATTCAGCATCTAATATGAATGTGTGAAAAATTTCACATAAATTT
TCATATAGTAATTGGGACCAAATTATTTGCTGTGTCTTTTGTCTCTTTCACATTGTTTATGAAGATCTGGCTTGAATAAAGGAGTCAGG
AGCTTTCGCCTTCCATAGCTAAGCCCAACTGTCCTAATGCTGCATAAGACAGCTGGGATTAGAGAAACTCTTGAAAAGCAGGATTGG
TAATGGGAAGTGATAGCTGTGTGTGAAAGCCTTAATGCTGAAGTGCATTCTTGTAACGCAATTTTCTTGTCTGCCTTGTTATCTTAC
AATTTTAGTGGGAAAAATAATAACAATGTTATTTCAAAGAAATGGTTAAAAAAATAGTCAAACTTAATAGAAAGCAGAGCCCTGTCCT
TTGGTTAAACACTCATTTACTCAGCTCTCTCTCTCTTTTTAAAAAACAGAACCATGAATCCTTCCTGTACAGTTGCATGGGTTGATA
TTAACTCTCTCTCAGCTTATTCACTGTCCACCTAAAAGAATAGTACAGGGCTCCAGAAATGTACCTTGCTCTGGTTTATAGGTAAAA
TGCTGCACTTGGGCATAGGTTTGTGGAGGGTGAGTATTGTTTCTCTTCCTGTGATGAAGATTGATGGCATTCCTGATAAAGGAAGAA
GAGCGTGTTTGAGGATGCTGGAAGATGCTGAAGTGACGCTGAGAACGTGACGGCCAGCCTGGGCTCTTGGGGGTGGAGGATGGTTGT
GGATGCTGTGTTGCAGTTGACTATGCCCTTTCTGGAGGTGTACGGGTAGCACCTGTTCTGCTCTAACAGCTTTGTGGGGAGGACAGC
CACCCACCAGAGCCCCCCATCCAGCACGTCTTCCTATTTCCTTGGTATTAGTAACTACATGGGGGATATTTATCTATGTTTTAATGC
ACACAGGCTAGTGCAGTCAAGGGTGTGGGGCATACAAAGGTAAAAAATAGATCATTGTTACCCTATAGAAGCTTTCAGTTGGCATGT
GTAGTGGGAGGTGTGCTTTAGGGAGGTGTCATATGTAACTTAGAGGAAAGAATTCCCAAGGTGGCTGAGAGGACTGGGGAGGTGGGA
GAGTCTGCTTTGGATCTGGAGGAAAAGTGGCACTGTCTACCTAAGGAATAATAGTGCCAAAGACAATTTGCACAGAGTTTGGCATATAA
TAAGTACTAATAGGCTAGGCACAGGTGGCTCATGCCTGTAATCCCAGCACTTTGGGAGGCCGAGGTGGGCAGATCACTCGAGGTCAG
GAGTTTGAGAACAGCCTGGCCAATATGGCAAAACCCCATCTTTACTACAAATACAAAAATTAGCTGGGCGTGGTGGTGTGTGCCTGT
AATCCCAGGGGTAGCTGAGACAGGAGAATCTCTTGAACCCAGGAGACGGAGGTTGCAGTGAGCCGAGATTGTGCCACTGCACTCCAG
CCTGGGCGACAGAGCAAGACTCTGTCTCAAAAAAATAAAATAAATACTAATAAATACTTAATGAAAGAAGGAGAAAAGTCTAGGGAA
GTTATTATTTGGGGAAGAGCTAAGAGGAATTCATCTGTGAATAGACAAACTGAATATCATTCCACGATTACTTTCACTTTAAATGTA
AGTATTTAAGGGTACCGTGAATGAATTCCTTCACAGATGTGTTGGCTCTGCAGGGATAAAGTGATGGGGTTTCCAGTTCATGTTGC
AAGTTCATGTCTCAAGGGGGAGGTTCTGTGAGACTTGGGCTGTGTCCTGACCACACAGCTTTGCATAATTGATTAATTCTGCATAACT
GCTTGCATTCCTTTCCTAAAATAACTTTCACTTTCTAATTAGCTCTCCAGAATTAATAAGTGCCAAAAAATAGGATGTGTGTTGAAT
CTTGTGATCTAATGTGATGCTGCCAACTCACACAGATTTAAGGTATAATTAATAATGCTGAATCTCTGAGGGAATTCTTAATTCCTG
AATTGGTAGACAGATCTCTAAAAAGTAGCTAATCTTATTGTGTTTACCATGTGCCGGGCACTGTGCTGAGGACTTTACCCAGTTTA
CCTCCTTGATCTGTTTTCATTATCACAAACACCCTAAGGATTAGTATTATGGGTATCTCCATTTCTAGAGAGGTAAAGTCACTCACA
CAGCCAATTGACACAGCCAGTGAATAGCAGAACTGGATTTGGGCTGAAAGAGTCAGGCTCTGGAGCCAGTGCTCCTAAAGGCCATGT
GACATTGCTTCTCTTTTCTTTCTCTGGGGTGTTCCAGTTTTCCTCAGGGCTTCGTCATTGCTGCTTTCTACTTGCTGACACTTTTGT
CAAATGTGATAAACAGCAAAAATTGATACAGTACAGGTCCAAAATAAACGGACCCTGATAGGTACCACAGGTAAAGTTGTTGGCAAA
TGACATCACTTATGTAATGGTAGGTCCTTCACTGGAGGTCAAATTATGGACGATGCTGTGAGGGAAAGGTAGGTAGATGTCTCTGGG
CTGGGAGAAGGCAAATGATGAGGGGACTTGGAGTTTCAGGGGGGGAAACAATAAAGATTCTGGTTGGCAAGTTTCTTGGCAAATTTT
AAGGGTGGTAAACATTTTAAATCAGTATGTCAGTAACTTCTGCATGAGAAGTGCCAGGTACTTCACAAAACTTTAATATAATGTGTG
AGCCTTAGGGTTGAAAGTAGGGATGGCACTGCTTCTGGCTTCTCTGTTAACAGAAATATGAGAGAGAAGTATGAATAATTTTTTCTC
AAATTGAGTTTTAGATTGGGGTTTCATTTTTCAGCAGAATATAAAACAGTTATTTTCTATTTCCATGAATACCAGGAGGGGAGACTT
AATAAAATGCAGTAAATATGGCCGGGTGCAGTGGCTCACACCTGCAATCCCAGCACTTTGGGAGGCCGAGGTGGGCAGGATCACCTGA
GGTCAGGAGTTCAAGATCAGCCTGGTCAACATGGCAAAACCCCATTTCTACTAAAAATACAAAAAATTAGCCAGATGTGGTGGCGGG
TACCTGTAGTCCCAGCTACTCAGGAGGCTGAGGCAGGAGAATCACTTGAACACGGGAGGTGGAGGTTGCAGTGAGCTGAGATCGCAC
CACTGCATTCCAGCCTGGGCGACAGAGCAAGACACTGTCTCAACACAAACAAACAGAATAAAGTAATAAATAGGTGTATGCTGAAAG
AAAAATCATTCTTAGTCCTGGCCTTTTTTTGAGACAGGGTCTTGCTTTGTTGCCTAGGTATCATCACAGCTCACTGCAGCCTGGACT
TCCTGGGCCCAAGTGACCCTCCATCCTCAGTTTTCCAAGTAGCTGGGACTATAGGCACATGCCACCACGCCTGGCTAATTTTTTAGT
TTTTTGCCCAAGCTGGTCTCGAACTTCTGACCTCAAGCAATCCTCCCATCTCGACCTCCCAAAGTATGTTGTCTGGGATTACAGGTG
TGAGCCACTGCTCATGGCCCCATGACATTTTTTAAATCAAAATATCTAGATAAAAATCAACATATTCAGACAATCTAATTTGGTTGA
TATACAACTATGTTTTTAAAAATTTTCCCTTTTTGAAAAAGGCAATATTTGTGGTGATTGTAGACTTAGCAATGTAAGTTTAAGTTT
TGTAACATTGCAGGTTGATTTTGAGTTAATTGAAACATTTGAAGTTTAATATGTTGGCAAAGTGGATTGTGTACATGAATTGAGGGG
GAAAGAGGGAAATTAATACTGTTTGATTTTTTTTTTTAATTTCTTGTAAATAATGTGAATATCCAGAAAGCAACAGAATAACCTAAGT
TATCAGAGGCTGAGAAAGCACATAAAATAACCTTTATTATTATTATTGTTTTTTTAGATGGAGTCTCGCTCTGTCCACCAGGCTGGA
GTGCAGTGGTGCCATCTCAGCTTACTGCAATCTGTGCCTCCCAGGTTCAAGCAATTCTCCTGCCTCAGCCTCCCAAGTAGCTGGGAT
TACAGGCATGCACCACCACACCCAGCTATTTTTTGTATTTTTAGTAGAGATGGGGTTTCACCATGTTGGCCAGATGGTCTCGATCTC
CTGACCTTGTGATCTGCCTGTCTCAGCCTCCCAAAGTGCTGGGATTACAGGTGTGAGCCACCACGCCCAGCCACTTTTTTTTTTTTTT
TTGTGGGGTGGGGGCGCAGATGGATGGGAATGGCATTGGAATACAATATTCAGGATATTGTAGATTAAAGAGGAAAAGTTTCTAGAA
TATTATGTGACTGTTGTGGGAGGTGTTGAATATTATAAATAAGGAGATTTTGCTGCTGTTGTTTTTGTTTCTGAGGAAAAGAAACCC
TTGGATTAGCCCCAGTGGTTCTGGCTGGTGAAATTACCCAGTAAATAACCACAGACGTCTCGTTAGCAGAAAGAGAGAAGGGAATCA
GCAGAAAGAGCTGGGGAACAAGGTTCACTGGGGGCTTTCAAACTTCTCAGGTAGTATGTGTATTGAAGAGCTTAGATCTGAAAGATT
AGGAGTTATATTGTCTCATTGAAAAGAGACTGACAGTATGGGCTTTAAAAAAAAAAAAAAAGAGCCCGCCAGCCTGTTAGAGATAT
ACTATGTGCTCAAAACTTTTGTGAAAAATTCAGAAAGCCTATGTATAAATCTGAGAGAGAATTGTGTGTGGAGTAGCGATTATCTC
TGATGACTTACATATTTTAAATAATTTTCTATATTTTTTGTTTTCCAGATATTTGTATATATGGATCCATTTTGTTCATGTTGAGTT
TGTACTTTATTTTTAAAAAATTTGCCACTACATTGTAAGCTTTCCCTGTGTCATGAGGTGTTCCTTGCAGAGGGGATTCACAAGACTC
TGTTTCCTTCTCCACTCAGCGTGCTGGCTCTGGCTCTGGCTCTGGCCCTTGTCTGCACCTCTCAGCTCCTACACTCCGGAGTCTGTG
CTTTTCCTGTGGCCCAGAACATTCCAGCTGCCACCCTCTTCACCTGGCTAGCTCTTAATCTTCGTGGTGCCCTTTTCTTTTTTAGGA
GGACTTAAATGCATTGGACAGTGGACAAAGACCATTTAAATGGATTACAAAAAGCAGACTTCACCAGGCAGAGCGGCCTCTAACTTC
```

173

EP 2 204 376 A2

```
TTCAGTAATGTTGCCATTTACCATGTGTCTGTTGGGGATCAGGTCCGTCGCTAGGCACCCTTCGTGCTTTTAGGTAATAGTCATAGC
TACCCTGAATAAGGGTGAGGAGATGGCATTCCCATTTAGAAAAGTTGAAACCAGGATTGCAAGAAGTGTATGAGCCGGGATGGGAAG
CCAGGTCTGTCTCTCTGCCCTTAGATCACGCTGCTGTTACTATTCTTTGTGACACCCTAGGACATTGCCGTGGAAGGAAGGGGAGAA
ACCTTGATAGGGCCCTTGGATGAAGCACTGCAGCTTATGCTAAGAAGATCAAGGCCCTGTTAGACTGGAAACATTCATTGTAAGACC
TAGTGCCATAAGGTAAAGAGGTCTCTAGGAGAATGCTCAAGATCTCTGAGGGATAACTGTTAAATATCCTGTAAGAATTAGGCTTAA
ATTATACACACGTGAGAAAGGTAAGTGCTCTCAGTGCAGGGGGTAAAATAAACCTTTAGTGAAACTCTATGACGAGGAACCCTGCCC
ATCAACTGTTGGTAAGTTTAGAAAAAATGAAGAGATGCAATTCAAGGTCAGTAAGTAGGTGAAATATGATGTGCGCATAAACCTTTA
AAGGTTTGAGAGGTTGGCTGTTTTTGGTAACGGGTTAACTTGAGGATTAATGAGTCAGGATATATGGAAAGTTAAAATTCCCTTCTC
TGCACCATTCAATTCAGTTCAACAAATACTTTCCCAGGCCAAATGGGATCATTAGCCTTGTAGGAAACTCTTGGCCTTTTTAAAATG
ATGACATTGTGGCCAGTTTTGTAAATACAGTTGATGCGTGAATCTGTAGACCAAGGATCAGATTAATTTTGTAATGCCCTCAGGGAA
GGCAGTTGATGAACTGGTTTCTCTGCATCATAAGAGCAGTGTTAATGATCCACTTGTACCATGAGATTCTAACATTCCTGAGTTACC
TAAATGCTTCAGTGTTCCCTGCCATGTAATTTTAAGCTGTATGTACATTTTTGGGATAATTTTTATCCATATTTTGTTTAAAAAAATG
AACTAAACCAAACTAAAATTCAGAACATTTTGTACTTCTGCTTCTTATTAAGATTGTTTAGTCCTTGGTGGGTGATGTGAGAAGATC
AGTACTTAACAGTTTGAGAATTCTTGCTCTCATAAATCAAAATAGTATTCGATGACAGTTTTCAAACAGTTTCAAGTGATGATTTAG
ATTCTCTTGTAGTTGTTATCTACTTTAGAATAGTGTTACTATGATTTAAAAGTTGGTATTTCGGCTGGGCCATGGTGGCTCAAGCCT
GTAATCCTAAAACTTTGGGAGGCCAAGATGGGAGGATCACTTGAGCGCAGGAGTTCGAGACCAGCCTGGGCAACATAGACTCTATCT
GTACAAAATACAAAATTTAAAAAGTTGTTATTTCATGAGTTTGCATTTTTAAATGCAATGGACAATAGATAGAACTGTTTATAAGAT
ATATTTGCTTGTATGTTTTTCTCTTCATATTACGAGGTCAAACCACAGAGGAAAAATAAAATTTGGAGGCCCTAAGCCTATTTACCC
AATGTCTCCTAGAAGAGCCCCCTTCAGAAACTTCTAGCAATTGTTAGTGTTGCTTGGGTTAATAGCCATGGTCAAAGCATGAACAGA
ACAAAATAAAAATTTTCTAAATATAAAGGAAATATGGTAATAACTGAGAAGCCAAGCATTAATAACTGGGAAGCCAAGCATTCTCTC
AATATCCTCTTTCTCCATAAGAACGTGTAGCATTTTCCTTGGAGAAGGGTCTATGTTACTTTTGTTCAGCCCTTGATGAAAACAAC
ATATGGTTAGTTTTATGAGGACTTAGAGACAGATGGTGACCTACAGGCTAATTATGCAGTGGGCAAGATTCTTTAAACAAATCACTG
TGATTGTGACATGGCCCTTTCTCATGAGTTAGACAATCTGGACTGTTCATTGTCACACTAGGTAAACATGTTCCAGGTGAATGAGGA
AGAGGTGCTACGTGAGGGAAATGTATTTACAACATCTTTTCATCTCCATATTTACTTTAATGGGTATAAGAGAAAAGGAAGTCAAGA
AGGTAAAAGGGGAGCACACTAAAACCTCAGCTAGTGCTAGGATTGTGTTATTTTGGACAGCTAACTTTTCAGATAGCTGAGGGGTTC
CCCCATTAAGCATAAACGTTTTTCTTTTAATCACTTTGAATGAACTCTTTCTGAAGTCTTCCTTGCCCTCTGAAAACAGAATGTCTG
AGACGTAGTAGGCTTTTATAGTGTTTATGATCTTACTTTTCCTGCTTTCCCAAACGTAAGCTTCATGAAGGTAGCGGTCTCAGTCTGT
TCTGTGCTGTGACCCTGGCCCCTAGAACAGTGCTGGTACACAGCCACATTAAATATTTGCTGAAGTAAGTGGGAATTAATTAAAGCT
CTTTAATCTTCACAACTGTGTCACTGTCCAGGCTTTGTTCTCCCTGTTTGTAGATTGGTCAGGGACCCTAGAGTGCATTGTGCCTAT
GCTTACGGTCTTAATGTCCCTCTGCCCCAGTGTGGTGGCTCATGGCTGTAATCCCAGCACTTTTGGAGGCCAAGACCAGCGGATCAC
TTGAGATCAGGAGTTCGAGACTAGCCTGGAAAAAATTGTTAACGGCGAAACCCCGTCTCTACTAAAAATACAAAAATTAGCCAGGTG
TGGTGGTAATCGCAGCTACTTGGGAGGCTGAGGCATGAGAATCACTTGAACCCGGGAGGCAGAGGTTGCAGTGAGCCAGGATCGCAC
CACTGTACTCCAGCCTGGGTGACAGAGTTGAGACTGTGTCTCTAAATAAATAAATAAATAAATAAATAAAATGTCTCTCTCATTATA
TTGTAATTGCCTTTTCTTGTCTCTCTCCACCACCGGACTGTAATATCATTGACAGCAGAAATTTTGGAGGTTCACTGTTATATCCAC
AATGTCTGGTAGTACATATTGTTACTTGTTTCTTGAACGTGTGAACAAACTAATTCACCAAAGGAAATGGTCACTGCCCTGGGTCCT
TTCCTTTACAAAATCGTATGATGTAGATAGTTTGATGCCCATTTTACATACACAGCTGAGAGGAGCAGCCGTGATTTGCATCCAG
GTTTGTCAGCCTCAAGGCTAACCTTCCACCGTATGGGTAGCTCCTCACCTTTACACACACAAGTGCCCCCTTTCTGATGTCAAATTT
TCCTAACCTTGCACAGTATGTGGTTTTTTTTTTACTTACCTCACCCAGAATGGTATAATAAAAATGCTGTTATCAGTTTAGTAGTGCT
TTTAGTTACTGTTTTTAGTCATTATACTAACATTGTAAATTTGAAAAAAAAAAAAAAAAGCCAGAACGGTTTGTGGGAGAACTTCTT
GTTGGTTGCAATGCTCCAGGCTGCCTGGGGGTTTGCCAGCCACCCCCTGCAGTAACCCTGTGCACTTCCCCTTTCCCAGACTCCCT
CCTCCCCGCCAGCAGCCCCTTCTGCGCTCCCCCGTGCAGCCTGCCCTCCATGAGTGGCCACGGACTTCAGGGTCCACGCGGTTCTTC
ACACCGCCCTTTATGTGATGTGTGTCCCCTCCTTGGCTGTCTGGTTTTGCTTTTGTCAGTAGGGCTAGTGGCCAGCCCATCTCCATT
TTGAATTTGCTGTTTTATAATAAGCCTTTGATTTGAAAGCCAGATGATCAGGGCTGCTAGAACTTGCTAGAATGAGGAAAATAGACA
AGTGGTCCATTGACCTCTGAGTGAGTTACAGTAACTTTTACTACATGGATTTCTTTTTTTTTTTTGAGATAGAGTCTTGCTCTGTCGC
CCATATTGGAGCGTAGTGGCGCGATCTCGGCTCGCTGCAACCTCCACCTCTCAGGCACCCTGAGAGGCCCTGTTCTCAGGCCTTCTC
AGGGTACTGGTAGGTGAGGCCCCAGCACGCGTTGCTCAGAGCCAGTTTGGCTCAGGTCTGTAGGAGGTGCGATGTGGACATAAGTGG
GGTGGAGTATGAAAGAGGAGAGATGCGCAAAATGGGAGAGCGTTTACCTGGTCAAGGGCTTAGGTCACATTAGGTTCTCAAAGGCAAA
TATGGCTTCATGTGGATACTTTCTTGAGTCCATCTCTCTCTGTTTCTGTTCCTCCCTCTGCCTAACCTATCCTGGCTCTAAGCTGGC
TCAGACACTGCGTTTGTGTCTTTTCACGTGGCATTTTTCTTTTTTCCCTCCACCTTGCTAAGTGCTGAAATTTGCAGTCAGCATTTA
TATATGCCCTATAGACAAGGCACATTTCTCTAGTTTTACCTTTCTTGTCATTTGTGATCTTTTAAATTTTCAGTCAATGGAAAGTAA
TTGTTAATATGAATTGCAGTTTGGAAACTTTGTGCCAGATGATGAAACTGAATGAATCGTGGTCTTTACGTAGGCAGTGCCTTACG
TATGGTTTGGTTTTTGGCCCATGTACTAGATGTTTGCCTTTCTGTGGGCTGTCACAGATCTGGCATGTACCTTTATTTTTTGGGAG
TTTCTACATGTGTTGCTGCAATCTCTCAGCTAAGAGTATTTTTCAGTCCTCTTGCTATTTGTTTTTTACCACTTATAGGGTGAAAGG
AAGTAAAATTAGATAGGATTTTGATGTTTGATTTAAAAATCAACATTTCCATGAATTCAAAACTTCAAGAAGATGAAAGGATAACAT
TGATTCTTCTGTTTAAAGAAAATTGATTTCCTTTGGCTGAAAAAGTTTTGTTCTCTCTCTCTTGGTGTTCATTATTTTAGATGTTCC
CTTTGGACTCTGTGCACATCCCCATCATAGAACCTACTATATTTACTATGGTATAAATTGACCTCTCTGCCAACACCACTGGACCA
TGTAAGTTCCTTGAAGGCAGGGGTCCCTAGTGACAGGCAGAGTGAATATCTGGTACATTGTAGACACTCAGTAAATGTTTGTGAAATG
AATAGAGTGACCAAGCAATCTAATGAATATGGTCCTATCATGGTCCCAACCAGTCATTGGACTGAGTATAGTTTTGATGGTGTCATC
TCAAATGAACATATCTCAAATCACATCCAGGTTCAGCAAATTTTCTTCTCTGTCTTGCCAAGGTGGGGGCCCCACTCTGAGTCCCAG
AGATAGAAACTCCAGTCATCTTTGCATTTCTTTTACTTCCTTAGTGTCTCTTGTATTTTCCTCTGTTTTTTCTCCATTCCTACTGCCT
CTACTCTAGTTTACACTGTTGGTCACCTTATGTCTGTAGACCTGTGAGCTAGCTTGCTGACACGAGCACCCCCAGCCATCCTGCACTC
TGCCGCAGCCAGATCAGGCTGCCTCCCACATCCCTTCTAATAGGACGTCCCTGCAGATGGTCAGAGTGGGTTTTCCCAAGGAGTTCT
GCAGGGCGGTGTGTTCCAGTGGACATCCTTGGTCCCCTGAGGACCAAGGCTTAGAAACTGATTTTATGAGCCTAGCCAGAGCTAATC
TCTCCATCGTGTTATCCCAGTTCAGGAAGTGGATATATAAAACCAGATTGTCTGCTTACTGCCTTAAAATGAGAATAGAACTGTTTC
TATTTCCATTTCATGGCCTGGGATTCTGGTGTGGGAACATCTAGTACACATTTTCTTCCTGTAGTCAGGCCTTCGTGGGAAGCCAAA
TCATAATCAGATGCTTACTTGGCTGGTGGTATGGAATAAAAACAAAGAGAAGTGAAGTGTCAGATGGCATCAAGGTTAAGTCCGAGGCAT
CCATTTCACAGTCAGGAGGCTTGCCAGTGGGAATACCCCAAGTGAGTTGGCTTCAGCTGCCTGCATCCTTCTCCACAGTGAGGCTGA
CTTTGCCAGTGGGTCCTGACCCTCCAAGGCTCAGGAGGTTGCCTCAGGCCCTTTCTCACTACATATTGCTGGGAGTGGCAAGGCCAT
TGCTGTTCTCTTTGACTATCTGCTTTCCTCCACTTTCAAATCTGACCAAGCCCTTCTGATTGACATTCTTACCCTTAGATATAATAC
TTTCAATTCTTTAGGGCTTGGAGCTGTTTCTCCAGGATGATTTTGCATGTCTTGGCTTTTTAAAGTTTAACTCTAAGAGCAGTATGC
TATCTTTATTTCAAAGCCTTTATATCTTCTCCCTCACTCACCATCTCCTTTCCTCAAAACCCTTTTAAGCCCTAACTGTTATTGG
TGGGAGTCTAAATTTCTTTGCCTAGCCTCTGAGGTCCTCTGTCTGCCTCCCCTGAGTGATCCAGACTTCTTTTCTTTTTTTTTGTGGTG
GGTTGGTGGTGGGCTGGGGGTGGGGACTGTCGCCCAGGCTGGAGTGCAGTGGAGTGATCTCGGGTCACTGCAACCTCTGCCTTCTG
GGTTCAAACGATTCTCCTGCCTCAGTCTCCCAAGTAGCTGGGACTACAGGCACGTGCCACCACGCCCAGCTAATTTTTTGTACTTTT
AGTAGAGATAGGGTTTCACCTTGCTAGCCAGGATGGTCTCGATCTCCTGACCTCTTGATCCGCCTGCCTTGGCCTCCCAAAGTGCTG
GGATTACAGGCGGGAACCACCGTGCCTGGCCTCCAGACTTATTTTCTACTTGTTTCCCTGTGGAATAAGAGGACTGGGTCAGAAGCC
```

174

EP 2 204 376 A2

```
CCAGCATAAGTCATTCTGTCTGTCTGCTGGAGTTTCCTGAGCTCTAAAAAACCCATCTTGCCTATCTCATAGGATTGTTGTGAGGGCC
AAATACAGTGACAAGTGTTCATTTTCTCTATTGGTGCAAGATTCTGCGTAGGTGATGTGACTCCTTTTTTTTTAAGCTATCATATATT
GAGCCCAAGCAGTGTGCATGTATTATTTCATTTAGTCCTTTCAAGTAGATCCTGTTGTCATCCCCATTTTACAGATGAAGAAAGTGA
AACTCAAAGAGGCTAGAGAAGTCTCAGGACTGATGGAGACCAGATGAGTCTGTTTAATCCCTTTAGTCTGCTCTGAACTTCCAGTAC
CCACGGCTATATTGGACTGAACACTCGCCTGACCAGGCCCCCGCCCCGCCACCACCGCCAACAACTAACTGAACTCGTTTCTACTT
CTCTGACCCACTGCCCTGGCCAAAGTCCTCCCTGCCTTTCCAGGCCCCTTTCAGGTTTCCTCTCTCTCCTTTTGTGTCTAGCCCTCA
TTGCTCACCATCATCTTCTCTGACCTCCTGTGGTACTTGAGGACATACTGGAGGCAGACCCATCTGCGATTACATCCCAACCGTGCT
GTGTTGCTTTGAACAAGGCAGCCACATCTCCGAACCACTGAAAAAAGGCATAATAATGATAGCTGGATCCTGGGATTTCTGGATTCT
GTAAAACCAATTGTGTAAAAGTTCTTTGTAAATGGTTAGATGTGCCTGGTTCTTCAGTAATCAGTTATTTTTGAAATTATTTTTAA
TTTGTCCCATAGTAAGGAATATATTTTAATGTTGTGATGGGCACATACATTTTAATTAAGAGTTCCACAGGAACATTTTCTTCTTTT
TTTGTGTGTTAAAAACAGTGTTTATTTTGACCCATTTAATTGATTTTGACTATCCACTGTAGGAGGCTACCCACAATTTGGAAGG
AACCATCGGGCCTAGATGACAATAAACCCTTTGAGGGCAGGGAATGTATTTTGCACTTCTGCGTCCTCATAGGCCCAACAGGTTACG
AGAAGATCCAGTCTGTTGATGGATTTTGTGCTGGGAGTAGGCAGGATGGAGAGACTTATTCAAATTTCACATTATTTATTGAGTGCC
CCTTTGCAAGAATTGGCCAGTTAAACCCTGGGGGGCAGCTTTGTAGCTGCACTACTCGGGAGCCTGCACCCTCTTGAAGACATCATG
CCAGCGTCTCCTGCACTTGGCCGTGTAGGCTGCAGCACAGCTGTTTCTGCTACAATCAGCTCCAGTATTTGTATTCCTCCTCTTGAG
GCCGAGGCTTGTTCGTTGGCCCCAGGGAATGTGTTGTGTTGGGGTTTGAATCTCCTGTCCTGCGTGCGGTGCCCCTGCCAAGGGCTG
TGCACCCACCTGTCCCAGATGGGTCTTGCGTCATGTTTTCCCTGCTATGCTGGTGGTGACAGCATCCTGCTGTCTCAGAGTAAAGCT
CAGCTGCATATATGCCACCCGCCCTCCTCCACAGCATCCAGGCTTCCAGTGTAAAAGCCTCTTACAACATCAGGCGGGAGAAAACAA
GCCTGCCGCTTGTGGTGCCTTAGCACCAATTTGTCACCCACTTCACTCAGCCCTGGCCCCCTGCTTCTCCTCTGTATGGTATCTTTT
TTTCCCATGGCTGCTCCAGCTGGGCTCTGGCCTGACACTGGAATCACATGTTTTGGCTCTAGCCTTTGGTATTATTCTCGAGGTTGT
CAGCATACATCCCCACTGCCATCTTTTTCTAATGCAAAGGTCTTTTCATTAATGGATATTTTTTCCTGTGAGACTTGTTTAGGGGAG
AGATGCATCCCTCATTTCCCCACCCCCCACCCGCCATAAGGTTTTTACAAGCCAAGCTAGAGATTGCTTTCCAGCCACTTTCAGAAAA
ATGCTGGGGCTCCACTTTGTGTTTAGCACAATCAGCACACATAATTCTTGTTCTGCTTGGGCTCTGTTCAATGTCACTTGCTCCTCC
TGACCCTAGAAAGAGTTGAGAATTATAAGCACGGATTATAAGGACTCATAATCTCAGTTGCCAGAGTAAGGGAAATAACGTTTTCAC
CGGTTCCCTGAAATAGACCTGGCGGACAGCTGTTGCCTCCCCTCCCCTTTCCTCTCATTTCCCCTGGAGATGTACTCTGCCTGTTTC
TTCTCCTCGCTAAAACTAAGGAACTTTCCCTTGTGTTTGTTGGCTTCTAGATTGTTCACTTGGCTTTTTATCTAAGCTTGTATT
AAATTGCTTCATGATCTCGTGCCTGCAGAGGCTTTGGGCATAACATGGCTCACAGAGAAGTGTTGTTTGTTGTGGTTGGACCACTTTGT
TGCAGCAGCATCTGTTCTTGTCTGAATGGGGCATGCAGGTGAAGCATGTACTCTAGGCCACTGAAAAAGGAGGGAACACAGGCACCT
GGACTGACCATTGGCAGCTGTGATCTACTTATGTGACTCTCAGCAGCCTCTCAGCTGCTTGTGATAAAGGCTACAAAATTCCAGGC
AGCTTTTTGGTCCCCATAACAAGTATATATAACTCCTACTTTAAGCAGTCCAAATTTAATACAGAGCAATGGGATGAGATGTATGTA
GGTGGTGTCACATCCACAAGAATATCTTTAATCATTATTCTTTTCAAGATTCAGTTTCAAGAGGAATAAGTGAATTTAGGGAAATTT
GTATATGGGAAAACATTGTCAATCAGTAAATGCGTGGTCTACTGTGTATATTTTCTCCTCAGTTCTTTAAGGCCCCCAAAATGGGAT
GAAAATTTGCTCAAAAGCTCTCATGCAAAATTATAAGAATATGGAGATATATTAGGAAATGGACAGATAAATATGCTTTCAAGAATC
TTATTAAAGTGCTCAACAGTTATATGAAACATGCAAGTATAAGTTGAAAATTTAACTGACATGTGGACATTGAATGTTTCTGTGATT
TTAATGAAAAAAAATTCAAGGAACAAGGGGGAAAAACACCAACCGTCAAAAGGAAAAATGCCCTCTATCTACTCATGCAAATAGTCC
CCTTGTTCATAGCAGTCACAAAGGATATGACGAGCCATATTGTGCAGATCTCAAGGTTCATGAGAAGTAGGTTTGTAGGTGGCAGGT
AGTGGGAAAGTGATTTATGTCCCATGAAAACCCATTCAGCTGTTGTAGCACTTCCAGGGCCTGTGACGTGTGAGGGAGCCATTGTCT
TAACACTCAGGGTGAGGACAAAAAGGCTTCAGTTGCTGAAGGTGCTGGTTTGAGATGGTGATATCCTGTCTCTTTTTCCAGCAGAA
GCAGGAACATGATGTCTAGGGTTTTTAAAAGAAAAAGCTTTTGTTCTTTTTTATTTAGTGACAGTAGAAATTAAAGTAGGGCCTTAA
AAATTAGAAACACACGTGAACCCCAGGGGGCGAAGCCTGCAGTGAGCCGAGATCATGCCACTGCACTCCAGCCTGGGTGACAGCAAG
ACTCCATCTCAAAAAAGAAAAAAAAAAAAAATTTAGAAACACAGTCAATTTTAATGTCAAGACCCAAGCCTCAGCAGTTAAATCGG
ATGCCTCCTTAAAAAATGTGTGAAAGTTATTTACCCTCATTTCAATTTGGGGCAACAAATGAATGGCTACTTAGGTTAGGTGTGGGA
AGCATTAATTAATAATGGGATAAAAGGACTCAGTGAGATTAACTTAGATCACCTATATTTAGCTTCTGATTTTACTTCATTCTGTTT
GCTTCTTTCATTAATCTAAATTAACTGCCAGAATTGCATTTATTAGTATGTAGGAGAACAATAATCAAGAAGTGTTTTTATTTATAT
TCGTAGGAGGCTATGTTGTTATTCATGTGATTCTTAGAAAATAATGGATTTTACACAGGAAAAGATGTTTATACTTTTCAAAGATCT
TACTCATTTGGGGTAAGAATTTGATAGAAATGCTAGGATTTGGTGTATAGAAAAGCATGTAAAGCTGTATGTATCATTTAGGTTTCTA
ATGCCTGTTATTGGCTTTCATGTAATTTGGATTGGGAGTCTAGAAGGAAGGCAGTGATAACAGGAGGGGCTGGGGAAAAGTGAAATT
GTTGCCCATTTCTTCAATATAGTGTGATAACTGTCTCCCATTTAATTTTGTCTCATCACACGTTTTTTGTGTGTGTGTCTGCTTTTT
GCCAAAAAGTACTTACTGGTTCTAGGCCTCAAGGAGCTTATGATTTAGCACAGATTTTCTCAGCCTCAGCACTGTTGACGTAGTGGG
CCAGGTAATTCTTTGTGGTGGGGACTGTCTTGTATATTATAGAACATCTAGAGACTTCCCCAACCTCTACCCACTAGATGCCAGTGC
ACCCTTCCCCAATAGGGTGACAACCAAGATGTCTCCACAGTGTATCAAGTGCCTCTCCAGGGAGGCCCAGAATTGCCTCCATTTTGG
AACCACTGGTTTGACACAGCACGTCTTGCTATAGAAACTCACTAGCATTCTTTGCCATTACAGCTGTTCAGTTACTTATGCTTATTT
CTAGTTTTGGAAAAGCCATTTGAAAATGCCCTGAAGTGATTTTTATTCCATTCTCATAATTCTTGAAAAGTGTGTGGAAAGCTAGAG
GAGAACTGTTAACTGTGTCTGTGTGCATGTGTATGTGTAGGGGTGTTTTCATCATTGTCATCGTTATTTTATGAAGAAAATGGAGGC
CTCAAATGTATGAGCCAAAGGACAAACACTGAGATGTCCATGAGAAAGCAGGGCGAGGACTGCTTTCAGGAAGTTCCTGATATTTCC
TGTATTAACACAAATCTGTTAAGACCCTGAAAAGCTTAGGGCTTAATAAAATCGTTGAGATCTTAAATGTAGAGGATGTAAGATTTTTT
ATAGATCAGCTTTAAAATGAAGCCTAGGCAAAAATGAAGTAGATTTGCCCCTCTTCACCCTCACCTGCCTTCTCCCGGTTTCTTCTC
TTTCCCTCCTCTCATCTCTGCTTGCTCTGGCAGTTCCTTTAACAAACATATTGTTATGTGATGACTTTGATGAATTACAAACAGGTA
CCTCTCTTTCAAGATACATTATATTCATCTACCAAAAAATGGTCGTAATAGCTGTACAAAGCTGCAGTGACCCACGGTAACTGGAGC
CCCAGGAGTTGTGCAGTGCACAGCTTGCACAGCAGTAAGTGGTGGCTATGTTTGGGCAGTTTCATTCCCTCCCATGACATTAGCTTT
CACCTGAATGCTGTTCTGTTTTTGACCCTAGACCAAACCTTTCTGGGAACATCATGCCTATGTAAGCTACCTTTTGGACACATTCT
TCCTAGAATACTCTAAAGCATCCCAAGTGTAACATATGCAAAATTGAATTACTGTTTTCCCCAAGCCTGGTTTTTCTTAAATTTAGG
AGTCGTCTTAGTCTCTCTTTTTATATACTTATATACACACTATACACATACACATGTATGTATCACAAGCCTTCTCAGTCCTCCC
CGTATAGTTCTTAAATCCTGTTTTTTTGTTTGTTTTGTTTTGTTTTGTTTTGTTTTTTGAGACAGAGTCTCGCTGTGTTGC
CAGGCTGGAGTGCAGTGGCGCGATCTCGGCTCTCTGCAACCTCCACCTCCCAGGTTCAGGCAGTTCTCCTGCCTCAGCCTCCTGAGT
AGCTGGGACTACAGGCATGCCCCACCACGCCCAACTAATTTTTGTATTTTTAGTAGAGATGGGGTTTCACCATGTTGTACAGGATGG
TCTTGATCTCCTGACCTTGTGATCCGCCTGCCTTGGCCTCCCAAAGTGCTGGGATTACAGGTGTGAGCCACCGCACCCGGCCTGTGA
TTTTCTTTCTACCCCTGCTTTCCTCCTGTTGTCTTATTTCAGTTTTTCATCTCCTCTCTCCTGAGGCCTGGGCCATTGCAGTCATCTA
CTACCTGCTTTTCCTGTCCAGGTATCACACCCCTTCAAGCATGTTTCAACTAGAATGCTCTTGTAAAACACAAATCTGGCCTTACTG
TTTCCCATCAGAGCTCTTTGAATTCCTTCTGTTATGCCATAGTTTATGCCTCCTTTATAGCCTTATTTATAACCTCATCCCCTCCCC
TCAAATGTCTAGGCATTCAAAATCTGCAAAGTGCAGGGCCTGTTCTAGGCACTGGAGACTCAGGATTGAATAAAACAAAGTCATTGC
CATCATGGAGCCTACTTTTGAGGGGGCTGGACTATTCGCAGTTCTGTGAGCTGCCATGCTGTTTCGTGTACCTTTGCTGGAATGTGCCT
GCCCACAGCTAATCTTCCTGGACAGTGTCTGCTGGAATTTCAGGACGCAGCGACGGTGTAGTGACCCCACTAGCATTACCACTCTTC
TTTTTATGTCTTCCTTGTGTTGTGTGCTTCAAGCTTCCTCTTCACCAGGGCCTGGCCTTACCAGGCTTTGTGTTTCCAGCACCTATA
GTAGTGCTCAGCGCATAACAGATGCTTCTAAATGTTTATTGAATGAAAAGCACACAAGCATGGAAACACTTTGGGATCCGTGTCCCT
CAAATGTGTCATGTGATTTTTCTTCCTTTCTTTGCTTATACGCTTTCCACTGCCAAGAATATCCACCCCAACCATCCTACCAGGCCCTT
```

175

```
ATAAGCCACTTGCCCCATTCTTTAAAGTGTCGCTGTCATAAACCCCTGCTGAACCCTTGCCTCAATTACTTATTGACCTACTTGCTT
TTTTCATGCTTTCAGTAGCATTTATTCAATAAATACTGAATATCTACCATTTTCCAGGATTTGTGCCAGGTCCTAGGGATATGACAA
TGGAAAAAGATCTGCTAATATTGTATTTTTAGTAGAGACAAGGTTTCTCCATGTTGGGTCAGGCTGGTCTTGAACTCCCGACCTCAG
GTGATCCGCCCACCTTGGCCTCCCAAAGTGCTGGGATTATAGGCGTGAGCCTGGATGACTGTCCTTTTTGCAGTGATGTATAGGCCC
AGGCCCTTTCCACGTTATGGCTCTGCTAGCCCCATTGTGTTCCGTTTTATGTGAAAGGGTAAAGAAGAGAGCATGGAGTGGATATAT
TTGTTTCTTAAAGCCTTGCCTCAGAAGTGACACCTACCACTTCTCTCACAGTCCATTGGTAAAGACAAGTCACAAAGCCACTCAAAC
TGCAAGGGCAGCTGAGACATGTGGTCTAGCTGGGTGACCAGAAAAGACAAAATGGAGTTTTGGTAGACAGCTACCAAACTCGGCCACA
TACCTCATTATACTAGCTGCAAACTGTGTTATCGTTAGTTGCCTGCATATTAATTAGTTCCTTCTGCTTAGAACACTTTTTATCTAGA
ATATTTGCAACATTTGCCACTCACTTCATTTAAATCTCAGCTCATCTGAAACTTCTTCCAAGTGTAAGACCACTCAGTCTGGAAAAG
CTCCCCATCTTCCTACACTGCCTTTTTTACTTTAGAGCCCCTGTCACACATTATATTATATATATAGTTTAGTTTATTGTGCTCCCC
TGCACAGGAATGTAAGTGTCATGATGGCACAGCATTTGTTTTGCTCATTGTTCTATCTACAGAATCTAGAAGAGTGCCTGGCACTTG
TCTTCAAGGATTATCCACATCCTGAAGGCAGAGCTTTGTTGTTCACATTTCAGTGAATATTTGCTGAGCGGAGCCTCATGCTAGACA
GGCCTTGGAGGCACGTCCTAAACAAGGAAAACACGCCCCCTGCCTCCCTCAATTGAAGATATTAGTTCTCTTCAATCTCATTCAATTT
TATTTCTCTGTGCTTAGCATTTAGAAGCAATTAAATATGTTTGTGGAATGAACAAACTAATTAACAGCATATTTAGAAAATCTCCCT
GTAGTAAATATATTAAAGTTTTAGAAAATCATGATAATCTAAGTGAAATATTTTACAGATTATGGGGAAAACATGAGGGATGAAAAT
TTACCCTGCTCTGCTTTAGCTTTTGAAAAAGTCTGCTTATGAGCCACTCTCTTCATTGCTAATAATAACACCAGTGGGGGAAACTTG
AAGAAAGAGGTATTTGTCACCAAATGGTAAGTTAGCAGCTTGTCTTGTTATCTGGGTTGGAACACAAAGTCATTAAGTGGCATTTGG
ACTAAATCTGATTGGAATTTAGAATCTACTTGGAGACAAACAAAATATACTTCTGCTTAGAAAAATTACATTTTCCACTTTAAGATG
TTGTGGATTAGAAAATGATAAGAATTAATTTTAAGAGAACGCCTGTTTTTTGAAAGGATTGGTTATATGTTGGTGGAAATTCTTCAG
GGAAGTGACATCTCTGGAGGGGGAAACACAAAAACAGCTCAATACAGTCAGTCAATTTATTCTACACTTAAGGAAAAAATAACTAAG
TCTGCTTGCTGGTTTTCTGTGGGCTGTTAACAGCTGGTTGACTCAAATTTCTGCTTTCTACCAATTGTGAAAATTAAGCTTTAATTT
AGAGGAGCACCTGGATGTTGAGACTCTGTCTGAAGATACAGGGGCTAAAAATGTTCAGATTCTGTCTGAAGATACAGGGGCTATGGC
TTGTCTATGGTTACAGTTTTTGCAAATGCAGAAAAGCTTTTCGAGCCTACTTATGTTATGGAGTATTTCCAATAGTGGTTATTAAAG
TAATTCAAGGCAAAATCAGTAACTAATGATAATAAAAAAGTTGCCTTAAAATAGTGTTCAAGAAAATAGAGTGTGTATGTCATATTTT
AGCACATAGAAAGGCTTAAACCCTCTTGTGAACTCCTACTTAACCATTTCTTTATTATACTCTTCTTACTCAGATTTATAAATTTTC
CTTCCTTTCTGCAAAAAGACAGAGATAAATAAAGATACTAACTTTTCCCCGTCATTATATCACCCCATTACCCTGTTATGACAGTAA
TATTCATGTTGAATATCCGGTCATGTTTAGCCATTTGTATTGTGTTTAAAGTTATTTTTTATGATTTCCAAATTTGAAATACTGGAA
AGGAAGATCCCAGCCTGATGGAAATTTTTACCAGAATAGTGATTCCTTTTTGCTGTAGAAAACATGGGAGGGAAATTGTCTTAGTGT
CGTTACTGTCTAGTAATACTGTTTGAATTCTTGAAAGGCTTGTTTCCCACGGTGTTTTACTGCATTATATTCATGGAAGGGAACATG
CAAGCCAGAGACAGTACAGGTTTTAGGCAAAGAGGCATGGGTTAGGATTCTGCTCTGCCACTCTGAAGGGAGTTATTTCTTCTGGCT
TATTCTGTGTCTTTGTTATTTAAGTGAGGGCATTTACAGGACCTACATCATAGAGTGCTGGGAGATTAAACAAGAAGACATTCACAG
GTTATGCCCTGCTGCTAAAGAAAAGGAATTACCATTTTCAATTTGTTGTTGTCTAAAGACCATCTGAAAATCTTGCTGTCCCTCAGTTAG
ACTCTGGCCTATCCACAGTAGGTCAGCTTGCCCACATGGGTTTCAAGCAACTCCACTCTGCTCTCAGGGCTCGCTAAGGGTGAACAGG
TGATCATGAATGTGGAAGGCTGAAGAAGAAACTGGAAAAAGCTGAAACTGAATATCAGCATTTAGAAACTGCATTTGGAGTGCTGAG
ATAATAGAAAGCTTTCAAGGCACTTTGGAACTCTACATATGTTAATCAAGAGCCTCAAAAGCAGCCCATCGATTGGCAAACCAATCT
CTTCAAGTGCCACCACATTAAAGTCCCTGGGGAAGCTGTGGATATTAGCTAGCAGCGGTCACAAGCCGTTTACCCTCCCTTCGCTG
CAACTTTAAAAACTTTTTGCCTAATAATCTCTGTTCTAAATTCAGTATGGATTTACTTAGCCAGCAGATTGAGATCCAGGGGGACAG
TGTGAGACTTGGAAGAGTTTTATATTTACAGGAAAGTTGCAAAGATAGTACAGTTTCCATTTACTGTGCACCCATTTTCCCCTGTTA
CTAACACCTTATGTTAGAAATGCTTGTTCCCTGGTGCTGCAGAGAAATAAATGTATGAGCATTCGAACATACATTTAATAAAAATGG
CCTTGCTAAGATGATTAAATTTATGTTCGAGTGCTGTTTCTTTGCGTCACCGGGGAACAAGTATTTCTAACACCTTACATGTTAATA
TACTGCATTTGTTACAACTAATGAGCCAATATTGATATATTATTATTAACTAAAGTTTGTACCTTATTCAGACTTCCTTAGGTTTTA
CCTAATGTCCTTTTCTGTTCCAGGATCTCACCCTGGGCACTACATTACGTTGTTCATGGTTTCTTAGGCACCTCTTGGCTGTGACAG
TTTCGTAGACCTTTTTGTTTTTTGATGATCTTGACAGTTTTGAGGAATAGTGTTTAGGTATTTGTGGAATGTTCCTCAGATTTGTCT
GTTGTTTTTTCTCATGATTAGACTAGGGGAAGACCCACAGAGGTTAAGTGCCATTTTTGTTGTATCAGGGGTACATCCTGTCAACATG
ACTTATCACTGATGTGATCATGTCAACATAATCTTGATCACGTGGCCGATGCAATCCGCTGCCAGATTTCTCCACTGTAAAGTGACT
CGGCCCACCTCCTTTGGAAGGAGGCCACTACGTACAGCCCTTGCATAAGGAATGGGGAATTTTGCCCTTCCCAGTTGTGGGCAGAAT
ATTTACATAAATTATTTGGAATTCTCAGTTGTATTTCTAAGTGTGTGATTTTAAAAACTACTTGGATGTTAAAATGGTTACTGTTGT
ATTCATTATTGAGTGAATACTTACAAATGTGGTCTGATGAACATTTTATTCTACCTAAGGGAAAAAAACAGCTTCCCAAATTGCAGC
CACCCTGCCTAGTTCAGAGACTGTAGAATTTGTATTCCTCCCCGAGTGTGTTCTTAGTAGCACGCCAGAAGTGCCAGATGCTACCCT
CTCTTTTTCTGTGTCATGATTTAATACCATTTGTGCATTTTCTTCTGCTTCAGTTGATTTAGTGTCATTTTTGGAGACCACAGACCT
ATTTGTATTTTATATGTTACTATACAGTGTGTATTCTGTTTAGAAAAATAAAAACGCTAGTATGATAAAATGTACTATTTTATTATC
TTTATTTTTAAAGTGTTACTATCAAAGACATCCTTTGGTGTCTCATAAATTGGCTTTGACTTTGTTGCTCTCAATAAGAGTAATATGAT
TCCAGATTTTCTGTTTAGTCAATGTTTAAACTACTTCATGTTTAACATTTATGCACATTATAATACAATGTGGTAATAAGGGTCATT
TGGCTTCTTGGCTCTTTCAAAATATTGGTATAATTTTTAACAAATTTATTTTGAAATAATCATTTCACTTTTTGATGAGTATAAACT
TGGTGAGAATTAGAAACTTCTTCAAAATATAGAAGTCTATTTTCCTTATCTTTGGCAAAGAATGATAATAATTGGACTTCAAAAGAC
CCAGCCCAGTAGTCCCCACTGCTAAAAATGAATAATGGCCTATCTCAATCCACAGAAGGAAAATAAAATGGGCTAATAGAAACTTTT
TCAGTTTCATGTTTTTCTCCTGTCAACATTTCATAAGTGTTTGGCATTTGCTACCACATCTCTTTTGGTGAGTATTAGTGAAAATGA
ATGAATAAGTAGTAAAAGAATGTTAACAGAAAAATTTGGACAACCAGTTAGTTTAATAACTCTATTATTTTATTGAGAACACGCA
AGTTTAGGGTATGGTGTTTTTTTATTCTTTCTTAGGGTTGAAATAATCTCCCCTGCCAGGTACGTATAAGCTATGGTTTTAGATGGC
ATTCATATACTGCCATTTCTGCCAGAAAAATTATTCCTCGTTCATAAAAAGAAGCAAAGTATCTCAGTGTTTCATATTTTTCCTCAA
GGGGAGCTTCCTGAGACAGGAAAGCATGGGTCATCCTAAAGTTGTATAACAATCATCTGTGTATCCTTCTACAGGAAGGATGGAATTG
ACTTTTCCACCCACAGATTTGGCATAAAATGTCTCTGCATCTGGGCCAAGACAGTTGTAAGGATTGTGAAGTCCCAGTGGTTTGG
GTAGTAGGTGAAGATGCCAGTTCAGTGCTGAAGAGCCATTCCTACCTGTGTGTGTGTGTGTGTGTGGGGGGTGCTGTGTGTGTGT
GGGGTGTGTGCTGTGCACGTGTGTGTGTGCGCACGCACGTGTGTGTGCGCGCGCGTGTGTGGAGTGATTTTTCAGTCTTTTTGCAGG
GAGGAGAAAAGAATGAATTAGTAGCTTTGCTTTTGCTAAGGATATCCTAACAGTGTGGTGGATCCTTTTTGTTTAACAGGGTAGGTC
AGATTACTGAGGAGCTGTTGGTTTATTGTTTTGTCATTAGTGCCATGAAGCTGCTACCGCTGGTTGTGGGTAAAGCAAGGCACATGA
AAGCAGTCTGAAAATTTGTATTCAAGCTAATTTTTAATTTTTAACTACTTGTTTACTTATTTCTATATTTACCCTTTTAGCATAATA
CCTGAAAACTTAAGGGGTTGGGAAATATCATTCATACAGCCAATTTTTTTTAATACCTTCTGTATTCAAGGCACTGTGTCTGGGGAAA
CACAAGTGGATAAGATGGACATGGTCTCCATTCTCATGATGCATACAGCCTACTAGGGGAGCAGCTGAAAAACAACTACAGATATA
ATTAGAAGGAATAATTTCAGGAAGTGTGGTTAGTAATATGAGGGGGGAATAAATTCGCAAAGGTGGCCAGAAAAGACTTTACTGAGT
TATCATCTGAAACAAACCCTTAGGGTAGGAGTACCCTGTCTTTATAAAGGAGAGAAGAAAGAGATGCAATTAGCAGGATGATTGACG
TGAAAAATTGCTTGATATAGTCAAGGAATAGAAACGTAGGCCTGTGTGACTGGAGAGAGTGGTATGATGTGAAATTGGAGAGTTGGTA
GAAAGGAGAGCACAGAGATCCTTAGGGATTATGAAGTATCGTATGGTGCACATACTGATGGATTTTCACAAACTGAAGAAACTGTGT
AACCAGCACCCAGATCACGAAGTTGGACATGTTCAGTACCATTGGGGCTGCTGGAAGCAGTAACCACCCACGCTGTTAGCTCTCTTG
TGTTCACATTTCAGGGCCTTGCTTTAGGAAAACTTCTGGTGTTCTCAGGCTATTTCTTCTATTACCATCCAAGGCCTAACACTTCTA
```

```
GCTCCAAGCAGAGTATCTTTCTGTGGGATTTTTATACTCATGTCACTTCCTCTCCCCAACCCCCACCTTGAACATGTATTATACATG
GAATTCTTTGGTAAATAGAATGCAAATTTGTTTTCAAAGCTTGTTTGGCACATACTCTCTCCTTTAGAAAGCAAAGGGTGTCCTTTC
AGAAGGTTCTTATAATATTTGTGGATCTGGCCTTCAGGTCTAAGCTGAAGCTTTTGTTTTCATTTGGACTTTATGCATGTATTAACA
GTTTATATTTTCTGTGGTAATGGAATATTTTAGGGGTTGTTTCTTCATTATATTTAGATTCTTATATTAAAAAGCGTGCAAAGGCAT
TTAACCAAATTTAATCTAGAAATAAAGATAATATGGTAATTTCAAATAGCTTTTTGTTCAAGATGAGATTTTAAAAGTTATTATTCC
ACAGTGAATTTTAATGATGATTGTGTACAATGGCATTTATGGATGTGCAGCAATTGAATGAGTATGATTAGCGCGTTAGATTATGGA
TTTGTAATGTGAAATGAGAACTTTTTAATAAGCTTTCTCCATAATGGTTCACGCTGTGAACAAACAAAGTATGAGACACTGAGCTTC
ACTAAATCATTGCAGGGCTTTGGAATCAGTAGGTTCATGTTTTCAAATAGTTTCCTATTTTTAAAAACACCGCTAAAAAGTTAAGAG
GCAGCTACATAACTAGATATCTTATATTTTGAGGAGTTGGAAGAACATGTTTCTTTTTATAATAAAAGGAAAATGTTATTGTTGAAG
TATATTTATTGTTCAAGTATAAAAATGGAATTGAAGTTATGCTTTGAGAAAAACTTGATTGCAAGACCTTATTAGGATTTAACACCTC
ATCAAGACTGCTTTTTACATTTTTTTTTTTTTATTTATTTTTTTTTTTAGATAGAGTCTCGCTCTGTCACCCAGGATGGAGCGCAATGGC
ATGATCTCGGCTCACTGCAACTTCCGCCTCCCCAGTTCAAGCGATTCTCCTGCCTCAGCCTCCTGAGTAGCTGGGATTACAGGCGCC
TGCCATCACGCCCAGCTAATTTTTGTATTTTTAGTAGAGACAGGGTTTCGTCATGTGGGCCAGGCTGGTCTCGAACTTCTGACCTCA
AGTGATCCACTTGCCTCAGCCTCCTAAAGTGCTAGGATTACAGGCGTGAGCCACCGTGCCCAGCTGCTTTTGACATTTTCTTTGGGA
GGTTTCTGGGGTACAATTAGCTGAAATCTGAAGTTTGGTTTTGCATGGTCTTGCCCAAGCTGGTGGTTGGTCATTGTGGCCTTCTGA
TCTGGTGATCCTAAGAAACATTCTGAAAACATACCCAAAACACATAAAAGGCTACCTTACCATTTAAAGGTTCTTTCAGTAAAGG
CCTATGAGTAATTTTTCATGGAGGGAGCCCATTCTTACGAAGTTTTAATGGACAAACATATCATTATCTTTAAATTGTATTATTACG
TTGTCTTTTATTTTTAAAAGCTCATTTTTCCCCATTATCAAGATAACATGTTCTAGACAGACAATTTAGGAAATGCATATGATTATT
TTTGTTCTAATTTTCTGTAATACCACCACATAGAGACAGTAGTTTTTAAAAACTGTGTGATATTTAATGTATTGTTTTCATTTCCTT
TAAGATGCAATTTAGAGTTTTAGCCTTGAACTTTAGTTTTCCTTCCTCTTGGAGATTTAGTTTGATGCTAAAGTGCTATCTTCTTGT
TTCAACTTTTGCTTAATTTACTCCAACACTTTTAGACAGCAAATTAGGTAACTTTATACTAGTGTTATCTAACAATGTTTTGCTAAC
TTCCATTTTAAGATGTTAGCTCACAAAAAAAGCTCTTAATTTAAATACATATAAAATTTTGAAAATGTGTGACTTTAATTAAAGACC
ATTGTTGGCTAGCATCATGGAGGTGTCAAGAAAATTAATAGTAAATGGGCCAGTCATCATTTCAAAACTTTCATGTTACATGACAAC
TGAAATTCCAGATACCCTTTAAGAGGTAAACTAGTTATGGAGAAGGGATGGAGAAGCACAGAATCCTAAATTTACTGTGGTTAGTCA
TGCACTCTGTGAGATGGAGGTTATTAGCCTCATATACCTAAAAATATATCCAGTGACCAAACCATGTCATAGGGCTCTGTCCATTTC
TTGCCTTTGCTTTCTCTTGGACTGGCTTCATTCTTAGGGTATCCTCATGTGGTTTCAAGGTAGCTACTAGCAGCTCCTATTGTCTTT
CCCATGAGTTTAATAACTGTAGTAAATGAGTCTCTCTTTGAAAATACTTGAATAGAAATCCCAGGATGAGTCTTACTGGACAAGTCT
TAAGCACCCACTAGTCCCAAACCAATTGTTAATGTGAGATAGGTGGAATGCTGTGATTGCCAGGATTGGGTCACATCCTTACCATCT
TACTCTAGAACACCAGCTTATTGCCCAAAGAAAGACCAAGGTGTTGTAATTGGAAAAAGGGAGGCAGACTACAAGCACACAGGAAAA
CTTCCTGGGTGTTCACTAAAAAGGTCGATGAAGAATGAAGCATAATTCCTTGAATGAAAATGAAAATACAGCATAGTGAAACCTGTG
AGATACTGCAAAAGCAGTACTAAGAGGGAAGTTTATACGATCTAAGGGCTACATCAAAACAGTAGGTCACAAGTTACCTAACATCAC
ACTTGAAGGAACTAGAAAAATAAGAACAAACCAAACTCAAAGTTACAAGAAAATAAGCGACAAAGATCAGAGCAAAACTACATGAAC
TAGAGACCAAAAAACAATACAAAGGATGAGTGAAATGAAAAGTTGGTTTTTTGAAAAGATAAACAAAATTGATAAACCACTAGCTAG
ACTAACCAAGAAAAGAAGAGAGAAGATCAAAATAAACACAATCAGAAATGAAAAAGGAGACATTACAACTGATCATGGAAATATAAG
ATCATCAGAGACTATTATTATTAACAACTATATGCTCACAAACTAGAAAAGCTGGAAGAAATGGATAAACTCCTGGGACACAACCT
CCTGAGATTGAAGAAATAGAAGCCCTGCACAGACCAATGATGAGTAGTGAAATTGAATCAGTAATAAAAATCTCTCAACAAAAAAG
CCCAGGACCAGATGCAGTCACAGGCAAATTCTTTCAAACATACAAAGGAGGCCAGGTGCAGTGGCTCATGCCTGTAATCCCAGCACT
TTGGGAGGCCGTGGTGTGTGGATCACGAGGTCAGGAGATCGAGACTATCCTGGCTAACACTGTGAAACCCCATCTCTACTAAAAATA
CAAAAAAAAAAAAAAAATTAGCTGGGCGTGATGGTACCCACCTGTAGTCCTAGCTACTTGGGAGGCTGAGGCAGGAGAATCCCTTGAAC
CCGGGAGGCGGAGGTTGCAGTGAGCCAAGATCGCGCCACTGCACTCCAGCCTGGGTGACAGAGCAAGACTCCATCTCAAAAAAAAAA
AAAAAAGAAAGAAAATAGAAACCCCAAAGAACCATACAAAGAAGGAATTATAACCAATCCTCCTGAAACTATTCCAAAAAAAAAAAACGAGAGG
ATTCTCCCCACCTCATTCTACAAGGCTAGTTATAAACCCTGATACCAAAACCAGACAAGGATACAACAAAAAAGAAAACTAGAGGCCA
ATATCCCTGATGAAAATATATGTAAAAATTCTCAACAAAATACTAGCAAATTGAATCCAGCAGCTCATCAAAAAGATACTATACCGT
GATCAGGTGGGATTTATCCTAAGGATGCAAGGATGATTCAACATGTGCAAATGAAGAAATGTGATACATCATGCAGACAGAATTAAG
GACAAAAACATATGATCGTCTCAATAGATGCAGAAAAAAGCATTCAGTAAAAATTCAGCATCTCTTCATGATAAAAGTCTTCAACATA
CGAGACACAGAAGGAATTACTTCAACATAATAAAGGCCATATATGCAAACCTACAGCTTAAATGGTAGCAAAAACCATACTTAATAGGAAAAAGTTG
AAAACATTCCCCTTAAGAACTGGAACAAGAAGCATGCCCACTTTCATGACTCTTATTCAACATGGCACTGGAAGTTCTCACCAGA
GCAATCAGGCAAGAGAGAATAATAAAAGACATCCAAATTGGAAAGGAGGAAGTCAAATTATCCCTGTTCACTGATGATATGATCTTA
TATCTAGAAAACCTTAAAGACTCCACCAAAAAACTCTTAGATTTGATAAATCAATTCAGTAAGGTTTCAGGATACTAAATTAACATA
TAGAAATCAGTAGCTTTTCTGTACACTAGTACAGTAATGATCTAGCCGAGGACCAAATCAAAAAGTCAACCCCATTTATAATAGCTA
CCAAAAAAAAAAAAAAAACCCCAAAGAACCCAGCTGGGAATATATTTAAACAGGAGGTGAACTACAAAACACCGATGAAAGTAATCATAGACAAC
ACAGACAAATGGAAAAAATATCCCATGCTCATGGGTTAGAAGAATCAACATTGTTAAAATGACCATCTCCCCAGAGAAATCTACAGAT
TCAATATAATCCTTATCAAACTTACCAACATTATTTTTCACAGAATTAAAAAACCCAATCCTAAAATTCATATGGAACCAAAAAAGC
CAGAATAGCCAAAGCAATCCTAAGCAAAAAGAAAAAATCTAGAGGCGTCACGTTACCTGACTTGAAATTACACTACAAAGCTATAGT
AACCAAAACAACATGGTACTGGTATAAAAATAGACACATAGATCAGTGGAATAGAATAGAGAAGCCAAAAATAAAGCCACATATCTA
CAACTGATTTTCAACAAAATTCAACAAAAATGTACACTGGGAATAGACACCCTATTCAATAAATGGTGCTGGGAAAATTGGATGTAT
GTTGTAGAAGAATGAAACTGAACCCATACCTCTTATCATATACAAAAATTAACTCAGGATGGATTAAAGACCTAAACGTAAGACCTG
AAACTATAAAAATCCTAGAAGAAACCTAGGAAAAACTCTTCTGGACATTGGCCCAGGCAAAGAATTTATGACCAAGTCCTTAAAACC
AAGCACAATGAAAATAAAAATGGAGAAATGGCACTTAATTAAATTAAATTAAAAAGATTCTGCACAGCAAAAGAAAAAAATCAGGAG
AGTAAACCTACAGAATGAGGAAAAAAAAAATTCCCAAAGTATGCATCCAACTAAGGGCTGACATGCAGAATCTACAAGAAACTCAAAC
AACTTAACAAGAAAAAAACCCCAAAGAACCCATTAAAAAGTGAGCAAAAAAACATGAACACACACATTTTTCAAAAGAAGACATACAAG
TGGCCAACAAACATGAAAAAAATGCTCAACATCACTAATCATCAGAGAAATGCAAATTAAAATCACAACGAGGCCAGGTGCAGTGGC
TCATACCTGTAACCCCAGTACTTTGGGAGACTGAGGTGGGCAGATCACAAGGTCAGGAGTTTGAGACCAGTCTGGCCAATATGGTGA
AACCCTGTCTCTACTAAAAATACAAAAAACACTAGCTGGGCGTAGGGCACCTGTAGTCCCAGTTACTCAGGAGGCTGAGGCAGGAGA
ATTGCTTAAACCCGAGAGGCAGAGGTTGCAGTGAGCCGAAATCACACCACTGCACTCCATCCTGGGTGACAGAGTGAGACTCTGCCT
CAAAAAAAAAAAAAAAACAAAACAAAACAAACAAACACAACAAATAGGAACAACCACAATGGATCCTTCTCACACCAGTCAGAATGGT
TGTTATTCAACAGACTAAAAATAACAGATGTTGGTGAGCATGTGGAGAAAAGGGAACGCTTATACACTGTTGGTGGGAATATACGTT
AGTACAACCTCAGTGGAAAATGGTATGGAGATTTCTCAACTAAAAATAGAACTACCGTTGGATCTAGCAATCCCATTTCCGGTATCT
AACCAAAGGAAAAGGAATTATTATATTTAAAAACACCTCAAGTCTATGTTCACCACACTGCTGTTCACAATAGCAAAGATATGAAA
TCAACCTAAGTGTCCATCAGTGGAGGATCATTGGATACAGAAAATGTGGGTTACACACACACACACACACACACACACACACACACC
TTGGAATACTATGCAGCGATAAAAAGGAATGAAATCTTGTCTTTTGGAGCAACATGGATGGAACTGGAGGCCTTTATCATCAGTAAA
ATAACTCAGAAACAGAAAGGCAAATGCCACACATTCTCACTTGTAAACGGGGGCAAATGATGGGTACAACATGGACAGAGAATAA
AATAATGGACATTGAAGACTAAAAATAGTGGGAGGATGGGAGGGAGGTGAGGGTTGAAAAATTATGATTGGGTAGAAAGTTTACTAT
TTAGGTGATGGGTACATTAAAAGCCCAGACTTTACCACTAGGCAATATATGCATGTAAGAAATCTGCACTTGAACTCCCTACATGTA
TTTTTTAAATTTTAAAAATTAAAAAAAAAAATTGCCACTGTGCCATTAGCTGGTACCATGTAATTTAGCATATAACTGTGTACGGATCT
CTTTTTCTCCATCATTGCTTCATCACGTTAATTTTGTTATGATGAGGGACTGTCTTTTACTTTTTTTATATTCCTCTTAGCACTAAGC
```

EP 2 204 376 A2

```
AAATAACAGGACCCTAAACACTTAACTGATTTTTCATTTCTGAAGTACAATAAAGATAAGTAGGAAATGTTAATATCATTGTTTTGT
TTGTTTGCTGGTTTTGTTTTTCAGTAGGTGTTTGAGAGAACACTTAACTCTCTAAGTGAACTAATTTTTTGCCATGAATGTGTTCG
GCACTAGGAAAGGTGATACATACTAGAACTGAGGAACTTAACTGGGGGTTCATATGCTTTCTAAGAGACCGTGGGAGAGTTTCTGGG
CATCTCTGCACCCCTAAAATCCTAGCAAAATTGTGTATGTGTGCACGCTTTTGTGTTTTTTTCTTTTTCTTTTTTCTTTTTTTTTTTT
TTTTGAGACAGAGTCTCGCTCTGTCGCCCAGGCTGGAGTGTAGTGGCATGATCTCGGCTGTCAGCTCACTGCAGCCTCTACCTCCAG
GGTTCAAGGGATTCTCCTGCCTCAGCCTCCTGAGTAGCTGGGATTACAGGCACCCACCACCACGCCAGCGAATTTTTATATTTTAGT
AGAGACGGGGTTTCACCGTGTTGGCCAGGCTGGTCTCAAACCCCTGGACTCAAGTGATCCGTCTACCTCGGCTTCCCAAAGTGCTGG
GATTACAGGCGTGAGCCACCACACCTGGCTTCTTTGTGCATTTTTCCTGAGTAAGGATGTGTAGCTTTCATTAGATGCTCAAAGGAG
ATCCTTAATCCACAGAAGGTTCAGAGCCACTGATAGGAAATATTTAACACTGACAGAAAATAGTTCTGAACTTTGCGATTAAATAAT
TTATTGTGAGTGGGAGGAGCTAAAAGAGTGATGCCACTTAGGAGAAGCACAAAGGAATCTGAGCTCGAGGATGAATTGTTAATCTAT
GTTAATGAATTCAGTCTGTACAGGGTTCAAACAAAATATATTATGGATGAGCCTCTAATAAAATGGACTTCCTCTGCTCTGCCAGTC
CGACAGTTAATGATGGCTGGCAATTTTCTGCCGGCTGATTTTGGCTCAGTGCTTTGGAGCAGGCACAGGCCCAGTTCTCGCCACTTG
GAAGGAAAGGGAAAGTGATAGTTTGTTACTTTGCTCTCATAAGCAATTTATGACAACAGTTTAATAAAAGCTTAAGAAGTGTCCTCT
TCCTGGGCGGGGAGAGGAGCCGAGGAGCCTCAGTGGGAGCACCGCGTGGGATGTTAAGCCCCTCAGCATCTCCTGGGGCCGCCTGAC
GCTGCCACACAAGAGAAGAGCGAGGGGGAGCCAGCGAGGTGTTTCTTTTAGCCCAGGCAAGGCTACTGTTATGCTTGCCAATTTGAC
TTTTCCCCAGCAAACTCAAGCTGTCCTGTGAGTTTCCACATCAGCCGTGTGCCTGGGGAGCTGCTCCGAGCTTGGGCTCTGGTGCTG
TCTGCTGCATGGACCTCATTAGCGCTCACCCAGCAGATTGCTTTCATATCGCTGCTTCACCCTTTTCAGTTTTGAATTACCACATAA
ACATCAGATATTGGGGGTTTTGTGAAAGGATCCTTAGTCTCTAATATTTAGTTAAGGAAAAAAGCCCTCTTAGGGAATCAGATAGAT
TTGCTTAGAGGATTTCCTCAGGAAAATACATTGCAGTATGGGTCAGTTCTAATTTTCCTCAATTCCTGAGGCCATTTAGTTTTCACT
TCTCTCTCTCTCTCTCCCCACCTCAACCCTGTGAGTCCAGGTTTAGGGGATTTTACAGCCAGCCTTCACACAACTTTCTGCTCTACA
TTTGTGGCCTTAAAACTTTATGATTTATAGGAAAGTATATTTCATGTCATGAACTGGGATATAAATATGTATGTATATATGTATAAG
CAAAAAGGTTTCACAAGGCAATATTTGCCCTTATATACCTAGTCTGATATTTTCTATTCTGTCGGGGAATACTGCTGGTCTTCACT
CATTAAATCATTTTCATGGCTCACCAATGGTTTGCACCCTGCAGTTGGAAAACACCTCTCAGGGATACTCAGGGATCCGTCCTTTCT
ATCCACTGTGTTGAGGGATGGCTCAGGGCACCGTGTAGTGGCTGAACCTCTTTATGTGGTACAGCAGAACCAACTTTATGTATTTTCA
GTAACACTTCTGATATTCTGGAGGGAGTAGAGCATAGTGATTTAAGAGCGAATGTTCTAGTTAGAATATGCTTGCTTGGTCCTGGGT
CTCTGACCTACCACCTGCTCTGGTGTTGGGCAAGTTTCTTAACCTCTCTGAATCAGTTTGCCCTTCTTTAAAATAGATATAATTTGA
ATACTTATAGCGAAACATTGTGAAGATTGAGATGTATATTATTAATATCACATTATTAATATATCCTAATGCACATCTATTAAGACACAA
TACAGAATTAATATTAATAGCAAACATTTGTTCAATGTTACTATGTGTCTGTGGTAAATTTAACAAATGTTTGCTATTAACATTATT
AATACTGTATCTTAATATTTATGTGTATCTCCAGTTGGATTACATTTTCCTTCTGCCCTTTGAAATAACTTATGTTGAGCCTATTAA
GAATATGTGGGTTGGGTGTGCTGGCTCACGCCTGTAATCCCAGCACTTTGGGAGGTTGAGGTGGGCATATCGCTTGAGGCCAGGAGT
TCTAGACCAGCCTAGGCAACATGGTGAAACCCTGCCTCTACAAAAAATACAAAGAAATTAGCCAGGTGTGGTGGCACACGCCAGTAG
TCACAGCTACTTGAGAGGCTGAGGTGGGAGGATCACCTGAGCCTGAGGCAGGAGGTTGTAATGAGCCGATATCACACCACTGGACT
CCAGCCTGGGTGACAGAGCAAGACCCTGTCTCAAAAAAAAAAAGTCTTATGTGTAGAAATAATCCCTATGTTCAAAAGCATGAACA
GTAAGCAGTGTGGTTAAATGAAAAAATATATATATTGCAGCTAGATAGTTACATTTTGAATTAGGACATTTTCAGCTCTAAGTGACA
GAAACCCCAATCAAACTGGCTCAGGCAAAAATAGGAATTTACTGATTGACGTAACAGAAACATCTAGGGATATTGCTTTTGGCATTG
GCCATTTCCAGTTGCTCTGAAGGTATCAGCAGTTTTTCTCTTCATTTCTTGCTCTGCCTTTCTCTGTTGGCTTCTTTTTCAGGCAAG
CTTGCCCCCAAGCAGTGGCAGAAACAGCCACCAGCTGCTCTAGGTTTCTGTTTCACTAGCAGGAAGAAAAACCCTCTTCTCTGGTAA
TTTCAGCTTAGTCTTGCATCTGATGGACATGGACTTTGGTGGGCTAGGCTCAGCTCACCCCTCCTGACTCCTTGCCACTGTCGGTCA
CGGAGCCATGAGAGGCACTGGTCATTAGATGTCCTGGGTCATGTACTTGCCTTGTAGGCTCAGCCCTGTTCAAGCCACAAGGTCAGA
ATGGAGGAGGGCTGCTCCATGGGGGCATCTCAGCAGATGCCCATCACACACTTCCACCATCCCTCCCCTGAACATAAGACTTCCTAC
ATAGATGTTTTCTTTGAATAATTCAACTGAGAGTAGTGTTAACACCTAGATACTTCTTATTCCAAGATATACCTCATTTCCACTCTT
AATTTAAATTATAGCTATGTTCAAAGATGTGTCATCCCTTCCTTCCTGATCACTGCTAAGATGTTCTTTTTAGCCACCCGTCTTTG
TATTGCCACTTAAAAGTCATCATTTCCTTCTATCTCATTTTATGCCTCCCTCTGCTTTCGCACACCTTTAGTACTTGTTGCCGTCGT
TATGAACAGCAAAATACAGACATGTGACACCCCACCTCCTCAGCAGTCTATCCTTTTCATTTTGTGCTCTAGCCAGCCCAGGGGTCA
GAACTGCATTCTGCAGCTTTTCTTTCATTCTCTGGCTGTGAATGAGGGTCCCTGATTTACCAGACTACATGAGGTTGGGACTGTGAG
TTGTTGGACAGGCCATGGCTTCCCCTATACACACACACACAGACACACAGATGAAATGGTTGACAGTGGACAGTTTGGTTGCAGT
AGGGACTCCAGATGGTGAAGTGTGTCTGCCAGGGAGTTAAAGACAATTGTGGGTCTGGAGCCAGTGAAATTGAGGGCACATTCCAGA
GCTTACGTCTGCCATGTGCAAGATATGTTTAGCTTGCTAATTAAAATTAGGAACAAGGTAAGCACTTGTTTGTTCATTCATTCATTCA
TTCATTCAGAGACAGGGTCTTGCTCTGTCACTCAGGCTGGAGTGCAGTGGTGTGATCATAGCTCACTGCAGCCCTGAACTCCTGGGC
TTAAGGAATCCTTCTGCCTCAGCCTCCCGAGTAGCTAGGACTACAGGCGTGCACTACCACACTTGGCTAATTTTTAAAAATTTCTTG
TAGAGACAAGATCTCACTATGTTGCCCACTTTGGTCTCGAACTCCTGGCCCCAGACAATCCTCCTGCCTCGGCCTCCCAAAGTATTT
GGGATTACAGGTGTGTGCCACTGCACCTAGCTGACATTTTTTAAGCCCAATATTGTGATCTAATATTCTATAACTGTATAGGATGGA
TAAATCAAATGTCGTGAAGTTCACATATAGAAAAAGGTATGGATTCAGGTAGTCTCAAGGTATGCATTATTTGGGGGAAAATTAGAA
AATTCCCTAAAGACATTTAAAAAATTCTAAAGTAGGCTGGGTGCAGTGGCTCACACCTGTAATCCCAGCACTTTGGGAGGCCAAGGCA
GGTAGATCACTTGAGGTCAGGAGTTCAAGACCAACCTGGCCAACATGGTGAAACCCTGTCTCTGCTAAAAAATACAAAAAAAAAATTAG
CCTGGCGTGGTGTCGGGAGCCTGTAATCCCAGCTACTTGGGAGGCTGAGGCAGGAGAATCGCTTCAACCAGGGAGGCGGAGGTTGTG
GTGAGCCGAGATCACGCCACTGCACTCCAGCCTGGGCGACAGAGGGAGACTTCATCTCAAAAAAAAAAAAAATTCTAAAGTAGCAGA
ATTGTTATGTATGTATTTATGGATGGGTGTATATATATGTATATTAAGAAATGCTAGTCAAAGTGCAGATATGATTTACACATTATC
TAATGGAAGCTTTCTAACCCCCGACCCCATAGGCACATCCACGATGCGAGAACACAGGCTCATGCAGAGTTGAAACGCTGTTGCCAA
ACTAAATCAAGACTGATAATTGGCCAGTTAGCTCAAAGAGTCAATGAAAATGATGAGGGTGTGTGTAAAATGACACATACTTTACAC
ATTTTATTTTAAATGAAAATACGATTTTGAAGTAGTATATTGTCTAGCATTTTGGTTTTTTCCCTGCTCGTGCTGTGTGCATGGAGA
CTCTGATATTTTTCCCCCAGTGGTATGTCACTTGCTGTAATATTTTGAGTACTAGGAAATGTTTTGAGATAGGCCCAAGGTCTTTTTC
TTGTCTAAGTGTCCTCTGACTGAAGTATCTTCCTTGTGTAAAACTTCAGCCATAATTTGTCAGCCACAACTTCCATTTACATTTTTTT
AAGGTGGACCTAGAACTCAGCAATACTTGCAAAGTAATCTAAGTCGGGAGGTTTTTCCAGAATTTTTCCAGTGTTATGCAGTTGTCT
CCGTCACACCTCATTCAACAGCAAACTTTTGTCATGGTTTGCCTTCATGGAGTCTTTTTTTTTTTTTTTTTTTTTTTTAAAGAGATGAG
GTCTCACTGTCACCCAGGCTAAAGTGCAGTGGCACGATCATAGCTAATTGTAACCTTGAGCTCCTGGGCTCAAACGATCCTCCTGCC
TCAGTCTTCTGAGTAGCTAGGACTATAGGCATGTACCACTGTGCCTGGCGAATTTTTAAGTCTTTTTGTAGAGAATGGGTGCTTGC
TGTTTTACCTCGGCTGGTCTTGAACTCCTGGCCTTAAGCGATCCTGCCACCTCTGCCTCCCAAAGTGCTGGGATGACAGGCATGAGC
CACTATGCCCAGCCTGCCTTCATGGAGTCTTTACAAGGCATTTAGCTTAGTTTTTACAGAAACAACTATTTCTTGACTTCCATATTT
AATTGATTTTATATAATGCTTGCACAGTATGCATGCAAAGCAAGTACACTTGGCACAAAAGATTTGAAAGGAAACATAGCTCACCT
GGAATAAGCACACAGCTCCATGGGTGGAACGGATGTGAGCAGATGTGTTGGCACTAGGACCACTGGTAGCTCACACTTAGGTGGGGC
TGTGTGTTCTAAGTTCTTTTGTATACATTGACTCATTTCACATATGCTCACAGAACCCCGTCAGGTAAGATATTATTATCCCTTTACACAGA
TGAGGGAAGTTATGTACAGAGAGGACAAGACAGATTTGCTTCAGAGTGGCCTACTAGCTGCCAGCATTCAGCCTGCATTGGGCTTTAGA
CAATATGTTTTGCGAAGGAAATGAGAGCATTTTGTGTGGGGGGTAAATTAAGAGCACAGAGACACTATATAGAGATAATCCCTTAATA
AAACCCACCATAACACATAAAGACTTCTGAAATACTCTCCATATCTCTCTAAAATTAGAGTCTAGGCTTTTAGAGGTAGAGATGACC
TTAACGTATCCCCTGCATATCCCAACAGGATAACCAAGACCCAGATGGGTGAGGTGATTTGCTTCACAGGCACCCAAGAAAGTGTGG
```

178

CATAAAGTTTAGTTTCCTGATTTAACCCCCATTTTCCTTCTCTCTCTCTCTCTCTCTTTTTTTTTTTTTTTTTTTTTTTTGAGACAGAGT
CTTGCTCTGTCACCCAGGCTGGAGTGTAGTGGCATGATCTCGGCTCACTGCACCCTCCCCCTCCTGGGTTCAAGCAATTCTCATGCC
TCAGCCTCCTGAGTAGCTGGGATTATAGGCATGCCACCATGCCCAGCTAATTTCTTTGTATTTTTTAGTACAGATGGGGGTTTTTCCAT
GTTGCCCAGGCTAGTCTCGATCTCCTGGCCTCAAGTGATCTGCCTGCCTCAGCCTCCCAAAGTGCTGGAATTACAGGCGTGAGCCAC
CATGCCCAGCCTATCCCACATTTTCTTATGGAAGAAAAGGATGCATTGTAGTGTATATGTAATCTTCTGAGAGAAACATTCAGGAAA
TGAGCCTCTCAGAGTTTGCCAAGAGAGCAGGAAGGTTGGTGGATAAGGACCACCTTAGTGTTATCAAATCCACATCTCTCCTGCAGT
CAGAGTAAAGCCTGTACCAGGTAATCTTAGGACTAGAAAAGGGTCTAGCGATGTGGTTTTATCCACCCCTGCTAGAGAAGGAGGGAA
TGAGGCCAGCTGAGCACCAACAAGGTAGATAGGTTTTGTCACTATTTTATGGGTAAGGAAGTTGAGCTTCAAAGAGAGAAAATGATTT
ATCCAAAATCACACTGCTAGAAGGAAATGGAACCAGGACACAAGCCTGGTCTTGCTGAAGCTAAGGCCCGTGTTCTTTATGCCGTGC
TAATTGTTCATCCTGTGCAGTTGTTTAACCACAGTTTTCTTGGCTAGAAGTTCTTCCTAAAGTTAACCCTAAATATCTCTTGAATTT
AAATGTGTATCCTTCTGTAGTCCTCAGTCAACAAAAGAAGCGATTGATCAGCCTATTTGAGAGGTTATAAGAAGACACAGATTGGCA
GTGTTGAATGTCAGTAGTACTCATCAAATAATCCCAGGATAGAAGGCATTTGTTCAACTCCCCATTATCCCTGCTCACATTTGCT
CTGTAGCATCCCCACGAAGCCTGTGCTGGGTAGTTTTCAGTCCGGGAATTAAGAGATATAAGAAAGAGGGGGCTGGGCACGGTGGCT
CACGTCTGTAATCCCAGGACTTTGGGAGGCTGAAGCAGGCAGATCACCTGAGGTCAGGAGTTCAAGACCAGCCTGGCCAACATGGCG
AAACCCCATCTCTACTAAAAGTACAAAAAATTAGCTGGGCATGGTGGCACGTGCCTGTAATCCCAGCCACTCAGGAGACTGAGACAGG
AGAATAGCTTGAACCTGGGAGGCGGAGGTTGCAGTGAGCCGAGATTGCGCCACTGCACTCCAGCCTGGGCTACAACAGTGAGACTCC
ATCTCAAAAAAAAAAGGAAGAGGGGAAGTCACCACCTTAGCCCATCCCATCTTCTTCCCAAGAGGTCATACACACATATGCCGTCTT
TGATTTGTATTTGCAAAAGTAATTCATGTTTATAGTAGAAACTTGGATCACTGAAGAAAGCTCAAGAAAGAATTTGAAAATCTATGT
GTATGAGTTATCTATTGCTATATAACAAGTTACCCCAGAATTTAGCAGCTTAAAACAAACTTTTATCTCCCATAGTTTGAGAGTCAT
TAGTCTGGGGAGTGGCTTAGCTGGGTAGTTCTCTCATGGAGTTGCAGTCAAGCTGTGAGTTGAGGCTCTAGTCACCTGGAGCTTTGTC
TGGGGCTGCAGGGTCTACTTTCAAACTCACGTGAGTGTTGGCAGGAGACTTCAGTTCTTGCCATATGGGCCGCCTCATGGAGCTGCC
CACAGCATGGCTTCCCACAGACCCATGTTGATGAGAGAGAGAGAACACGCACTAGGAGCAACCAAGACACAGCCCACAATCTTTT
CTACGCTGATCTTAGAAAGGATGTTCCTGCACTTCTGTTGTATTCTGTTGGTCACACAACCAACCCCGGTATAATATTATAGGAGGG
GACTACACAAAGGTGTGAGGACCAGGGATGGGAATCATTGAAGGCCATATCGAAGGTTGCCTACCACAGCATGTAAATTAAAATTAT
GTTGCAATAAATTACATAAGGTTTTTGCCCACAGTTCTCCTTTTCTTCCCCCTAGAGTATTGCAAGATACAAAAGATCACCAGGAAAA
AAAAAATCTAAAGGGAATGCAATGTTGAGGCATGAATGTCCCAGTAAGCTGTTTATTCCTTTCTCATTCTAGAGGAGCTTCTTGATT
TTATTCCAAGTCTGCCATGTCAAAATTGTGGTTTGGGGAAGAGGGGAGTAAGGGCAATAGAGTGGATAGAAGCATGTGGATGCGCCT
TGGTCCTGACATTTGTGTTTTGGTGAACCTGCTGCCTCTGAGAAAAGATAAGGAGTTTAGGATTTGGTCCAGGCTGAAACCTGGGAG
AAGCTCATGGCTCCTGGACCTCCTTATTCTGCTTTTAGATGCAAATAAGAAACAGAGATTTGTAAATGATATTCTAATTTTTACAAA
TAATTATTTGAGGAAAAAGATTTGACTACTTCAAACATACAAGCATTGAAAGTAACATAAAAGGTTTTTTGGTTTTTTTTTTTTTTG
GTGGGGGTGAGGGGGAGAAGCATTTTGTTTATGCAAATACAGGGTACAGAGATCCAGAAGGCAGGATCATAACATCTGGACAAGCAGTA
GCATTCCCCAGCCTCCCACCCCCAACTGTTACAGATAACAGAGGGAAACTTGTGGGTAAATAAGTAGTAGGTAATACAGGCATTTATA
GATAGGGGACGTTTTCATATCAGACACGGCAGAGCTTTTTTTTGAAAAGCACATACCTGTATGATATTTTGATGAATAAAGGTAATGC
AGAAGCAAACTGTAATATCAAATAAATTTGAGTGAATTCTTCTACAGTACCTACAGTCAAAGGAAATTTATCAGTGAAAATCAGTAT
ACAACAAACAGCAAAATGGAATAAATTTGAATAAATTCCTGCACAGGCAGTCACAGGACATGTTGATAAATATTTCAGTATAAACTG
ATACAAAGTATAGCATAAAATATTAATTTTGAAGGAATTCCTATGTCATGCCTTGAATAAAAGCTAATATTTCACTGAATAGTGTGT
ATGTACCAAGCAACGGGAAGAAAACTGAATTAGTTTTCACAAAATGGCTCTAACTTAATGTGTATAACCATTTTTCAGCTGACTTTG
AGCCACAGGTTGCAGTAGTCATATGGATGTGACTCTTAGATGTCAGAGGAATGGCCCTGCTCTGGTGGCGGGGTGGCTGTTGGCCTC
TGTCCCTGTCCATCCTGTGGACCTTTTTGTGGACTAAGGAGCTGATGTAGTGAACTGGTACTGCTGGTGTGTGGCTTTCTGGACCTG
CCTCCCTCAGCCAGAGGAGACCCTGGATTTCTCCATCCTAAGCCAGACTTACTGATGGCCAAGAGTGAGGAGCAGGCAGAACAGCC
ACAAAGTAACTGCTTTGCTCTTTGGCTATGCCCTAAAGTGTCCAAAGTCATTGTTCTAAAGTGGAGCAACTACATAACTGACAGTTA
ACCTCTAGAGCCCTGTACAAATCCTTCCTTTTAGTCCAGATGCTATATGAGTTTGGGGAACCTGAGCCTTCCTGTGTGCACGTATTC
CCCTATTGCTGCTGATGCAGGGAAAACTGTGCCCAGTAGTGACACCATCCAGCACCTCAGGTGCCAAAGTCCCCAGGCCGTGATCAC
AGAGCATGGGGCTGAGCCCCTCATAGGTGGTCACATCACCCTCTTTGTCCTGCTGTCGGCTGGAATGTGTCCAGCCTAGCTCAGCAT
GGAAGAAGGTGAACCCCACCACCTACCATGAGCAGCACCTCAAATGACAATGCCACAGAGAAAATGGAGCAAAACCAGGATGACCTT
GCCAGCTCTTCATTGTAGGATCAATGCATTTAGGTGCAGAGGGGTGTGGATGGAAGCCATGGCCTAGCTTGGCTTGTCGCAGACCTC
TGTGCCAGGCCCTTGCCACCTCCTAATGCAGACAGCAAATACTGGAGGCCTGAGCATCAGCTCTTGAAGTAGCTCTCTGGGAGGGCG
GTGAGGCACATGTTGAGGCATGGGATTTGCCAGCAGAAAGATCTTCAGTGAATTACCTAAGCTCATTACACCTTAGAGTCCTCATC
TGTAAAATGGGGCACTTGCCTAATGAGGGTGTTATGAAAATTAGCATATTTAAAGGTACTAGGGCTGTTTCTGGCACATTGTCAGGG
TTCAGTAATTCTAGTTAAAAATAGAAAAGTGGCTTCTGGCTGGCAAGATGGCCGAATAGGAACAGCTCCGGTCTGTAGCTCCCAGCG
AGATCGACTCAGAAGGCGGGTGATTTCTGCATCTCCAACTGAGGTGCCCGGTTCATCTCACTGGGACTGGTTGGACAGCGGGTGCAG
CCCACAGAGGGCAAGCCGAAGCAGGGTGGGGCGTCACCTCACCTCACCCGGGAAGCGCAAGGGGTCGCGGAATTTCGTCCTCTAGCCAAAGG
AAGCCGTGAGGGACTGAGCCTGAGGAACTCCGGCATAGATACTGCGCTTGTTCCACAGTCTTCGCAACCCACAAACCAGGAGATTCC
CTCCGGTGCCCACCCCACCAGGGCCCTGGGTTTCAAGCACAAATCTGGGTGCCATTTGGGCAGACGCCGAACTAGCTGCAGGAGTT
CTTTTTTTTTCCATACCCCAGTAGCACCTGGAACGCCAACGAGACAACCATTCACTCTCCTGGAAAGGGGTGCTGAAGCCAGGGATCCA
AGTGGTCTGGCTCGGCGGGTCCCACCCCCACAGAGCCCAGCCAAACTAAGATCCACTGGCTTGAAATCCTCGCTGCCAGCACGGCAGC
AGTCTGAGCTCCACCTGGGGCACTAGAGCTTGGTCGGGGGAGGGGCTCCGCCATTGCTGAGGCTTGAGTAGGTGGTTTTACATTCA
CAGTTTAAACAAAGGCACTGGGAAGTTCGAACTGGGCAGAGCCCACTGCAGCTCAGAAAGGCTGCTGGGGCCAGACCGCCAGATTTC
TTTTCTCTGGGCAGAGGATCTCTGACAAAAAGGTAGCAGCCCAGTCAGGGACTTATAGATAAAACCTCCATCTCCCTGGGACAGAG
CACCCGGGGAAAGGGGTGGCTATGGGCGCAGCTTCAGCAGACTTAAATGTCCCTGCCTGACAGCTCTGAAGAGAGCAGCGGACCTCC
CAGCACAGTGTTTGAGCTCTGCTGAGGGTCAGCCTGCCTCCTCAAGTGGGTCCCTGACCCCTGTGTATCTGGGAGCACCTCCCCAGT
AGGGGCCGACGGACACCTCATACAGGAGAGCTCTGGCTGGCATCTGGCAGGTGCTCCTCTGGGACAAAGCTTCCTGAGGAAAGATCA
GGCAGGAATCTTTGCTTCCGCTGGTGATACCCAGGCAACAGGGCTGAGAGTGGATCTCTAGCAAACTCCAGCAGACCGGCAGCAGA
AGGGCCTGACTGCCAAAAGGAAAACTAACAAACAGAAAGGAATAGCACATCTGCTTAAAGACCCCATCTGAAGGTCACCAACATTAA
AGACCAAAGATGGATAAATCCACAAAGATGGGGAGAAACCAGCTGAAAAAGGCTGAAAATACCAAAAACAAGAACACCTCTTCTCCT
CCAAGGGATTACAACTCCTCACCAGCAAGGGAACAAAACTGGATGGAGAATGAGTTTGACGAATTGACAGAAGTAGGCTTCAGAAGG
TGGGTAATAACAGACTTGAGCTAAACGAGCATGCTCTAACCCAATGCAAGGAAGCTAAGACCTTGAAAAAAGGTTAGACGAATTGC
TAACTAGAATAACCAGTTTAAAGAAGAACATCATGACCTGATGGCACTGAAAAAACAGCACAAGAACTTCGTGAAGCATATACAAG
TATCAACAGCCGAATCAATCAAGCAAAAGAAAGGATAGCAATGATTGAAGATCAACTTAATGAAATAAAGAAAGAAGACAAGATTAG
AGAAAAAAGAATAAAAAGGAACGAGCAAAGCCTCCAACAAATATGGGACTATGTGAAAAGACCAAATCTGTGTTTGATTGGTGTACC
TGAAAGTGACAAGGAGAATGGAACCAAGTTGGAAAAACACTCTCCAGGATATTATCCAGGAGAACTTCCCCAAATTAGCAAGACAGAC
CAACATTCAAATTCAGGAAATACAGAGAACACCACAAAGATACTCCTTGAAGAGCAATCCCAAGACACATAATCTTCAGATTCAC
CAAGGTTGAAATGAAGGAAAAAATGTTAAGGCAGCCATAGAGAAAGGTCAGGTTACCCACAAAGGGAAGCCCATCAGACTAACAGT
GGATCTCTCTGCAGAAACCCTACAAGCCAGAAGAGAGTGGGGGCAATACTCAACATTCTTAAAGAAAAGAATTTTTCAACCCAGAAT
TTCATATCCAGCCAAACTAAGCTTCATAAGCGAAGGAGAAATAAAATCCTTTACAGACAAGCAAATGCTGAGAGATTTTGTCACCAC
CAGGCGTGCCTTACAGGAGCTCCTGAAGGAAGCACTAAACATGGACAGGAACAGCCGGTACCAGCCACTGCAAAAACATACCAAATT

```
GTAAAGACCATTGATGTTATAAAGAAACTGCATCAACTACCAGGCAAAATAACCAGCTAGCATCATAATGACAGGATCAAATTCACA
CATAACAATATTAACCTTAAATGTAAACATGCTAAATGCCCCAATTAAAAGACACAGACTGGCAAATTGGATAAAGAGTCAAGACCC
ATCAGTGTGCTCTATTCAGGAGATCCAACTCACATGCAAAGACACACATAGGCTCAAAATAAAGGGATGGAGGAATATTTACCAAGC
AAATGGAAAGCCAAAACCAAACAAACAAACAAAAAACAGGAGTTGCAATCCTAACCTCTGATAAAAAAGACTTTAAACCAACAAAGA
TCAAAAGAGAAAAGAAGGGCATTACATAGTGATAAAGGGATCAATGCAGCAAGAAGAGCTAACTATCCTAAATATATATGCATCCAA
TACAGGAGCACCCAGATTCATAAAGCAAGTTCTTAGAGACCTACAAAGAGGATTAGACTCCCACACAATAATAGTTGGAGACTTTAA
CACCCCACTGTCAATATTAGACAGATCAATGAGACAGAAAATTAACAAGGATATTCAGGACTTGAACTCAGCTCTGGACCAAGCGGA
CCTAATAGACATCTACAGAACTCTCCACCCTAAATCAACAGAATATACATTCTTCTCAGCAACTCATCACACTTATTCTAAAATTGA
CCACATAATTGGAAGTAAAACCCTCCTCAGCAAATGCTAAAGAATGAAAATCACAACAAACAGTCTGTCAGACCACAGTGCAGTCAA
ATTAGAACTCAGGCTTAAGAAACTCACTGAAAACCACACAACTACATGGAAACTGAACAACCTGGTCCTGAATGACTACAGGGTACA
TAACGAAATTAAAGCAGAAATAAAGATGTTCTTTGAAACCAATGAGAACAAAGACACAACATACCAGACTCTCTGGGACACATTTAA
AGCAGTGTGCAGAGGGAAATTTATAGCACTAAATGCCCACAAGAGAAAGCAGGAAAGATCTAAAATTGACATCCTAACAACAAAATT
AAAAGAACTAGAGAAGCACAGCAAACAAATTCAAAAGCTAGCAGAAGACAAGAAATAACTAAGATCAGACAGAACTGAAGGAGATA
GAGACACGAAAAACTCTTCAAAAAAATCAATGAATCCAGGAGCTGGTTTTTTTTTTTTTTTTTTTTTTTAAGATCAACAAAATGGAT
AGACCACTAGCCAGACTAATAAAGAAGAAAAGAGAGAAGAATCAAATAGATGCAATAAAAAATGATATAGGGGGCCGGGTGCTGTGG
CTCACACCTGTAATCCCAGCACTTTGGGAGGCCGAGGCGGGCGGATCACGAGGTCAGGCGATCGAGACCATCCTGGCTAACATGGTG
AAACCCTGTCTCTACTAAAAATACAAAAAATTAGCCAGGCTTGGTGGCGGGTGCCTGTAGTCCCAGCTACTTGGGAGGCTGAGGCAG
GAGAATAGTGTGAACCCGGGAGGCAGAGCTTGCAGTGAGCCAAGATCGCGCCACTGCACCCCAGCCTGGGCGACAGAGCAAAACTCT
GTCTCAAGAAAAAAAAAAAAAAAAAAAAAAAAAGATATAGGGGATATCACCACCAATCCCGCAGAAGTACAGACTACCATCAGAGAATAC
TATAAACACCTCTATGCAAATAAATGAGAAAACCTGTAATAATGGATAAATCCTGGACACATAACACCCCCACAAGTCTAAACCAG
GAAGAAGTTGAATCCCTGAATAGACCAATAACAAGTTCTGAAATTGAGGCAGTAATTAATAGCCTACCAACAAAATGTCCAGGACCA
GATGGATTCACAGCCGAATTCTACCAGAGGTACAAACAGGAGATGATACCATTCCTTCTGAAACTATTCCAAACTATAGAAAAGAG
GGAATCCTCCCTAACTCATTTTATGAGGCTAGCATCATCCGTGTTACCAAAACCTGGCAGAGACACCACAAAAAAGGAAAATTTCAGG
CCAATATCTCTGATGAACATTGATGCAGAAATCCTCAATAAAATACTGGCAAACAGAATCCAGCAGCACATGAAAAAGCTTATCCAC
CACGATGAAGTCAGCTTCATCCCTGGGATGCAAGGCTGGTTCAACATACACAAATCAATAAACATAATCCATCACATAAACAGAACC
AGTGACAAAAACCGCATGATTATCTCAATAGATGCAGAAAAGGCCTTTGACAAAATTCAACACTCCTTCATGCTAAAAACTCAATAA
ACTAGGTATCAATGGAACATATCTCAAAATAATAAGACTATTTATGACAAACCCACAGCCAATATCATACTGAATGAGGAAACACT
GGAAGCATTCCCTTTGAAAATCTGGCACAAGACAGGGATGCCCTCTCTCACCACTCCTATTCAACATAGTATTGGAAGTTCTGGCCAG
GGCACTCAGGCAAGAGAAAGAAATAAAAATAAAGGTTATTCAATTAGGAAGAGAGGAAGTCAAATTGTCCCTGTTTTCAGATGACAG
ATATTTAGAAAACCCCATCGTCTCAGCCCAAAATCTCCTTAAGTTGATAAGCAACTTCAGCAAAGTCTCAGAATACAAAATCAATGT
ACAAAAATCACAAGCATTGCTATACACCAATAACAGACAAACAGAGAGCCAAATCATGAGTGAACTCCCATTCACAATTGCTTCAAA
GAGAATAAAATACCTAGGAATCCAACTTACAAGGGATGTGAAGGACCTCTTCAAGGAGAACTACAAACCACTGCTCAAGGAAATAAG
AGAGGACACAAACAAATGCAAAGACATTCCATGCTGATGGATAGGATGAATCAATATTGTCAAAATGGCCATACTGCCCAAGGTAAT
TATAGATTCAATGCTATCCCCATCAAGCTACCAATGACTTTCTTCACAGAATTGGAAAAAACTACTTTAAACTTCATATGGAACCAT
AAAAATCCTAGAAGAAAACCTGGGCAGTACCATTCAGGACATAGGCATGGGCAAAGACTTCCTGACTAAAACACTAAAAGCAATGGC
AACAAAAGCCAAAATAGACAAATGGGATCTAATTAAACTAAAGAGCTTCTGCACAGCGAAAGAAACTATCATCAGAGTGAACAGGCA
ACCTACAAGGTGGGAGAAAATTTTTGCAATCTGTCCATCTGACAAAGGGCTAACATCCAGAATCTACAAAGAACTAAACAAATTTA
CAAGAAAAAAACAACCCCATCAAAACGTGGGCAAAGGATATGAACGACACTTCTCAAAAGAAGACATTTATGCAGCCAACAAACAT
ATGAAAAAAAGCTCATCATTATTGGTCATTAGACCACAAATCAAAACCACAATGAGATACCATCTCACACCAGTTAGAATGGCGATC
ATTAAAAAGTCAGGAAACAACAGATGCTGGAGAGGATGTGGAGAAACAGGAATGCTTTTACACTGTTGGTGGGAGTGTAAATTAGTT
CAACTACTGTGGAAGACAGTGTGGCGATTCCTCAAGGATCTAGAACTAGAAATACCATTTGACCCAACAGTGCCATTACTGGGTATA
TACCCAAAGGATTATAAATCATTCTATAAAGACACATGTATGTATGTTTATTGCAGCACTATTCACAATAGCAAGACTTGGAACCA
ACCCAAATGTCCATCAGTGATAGACTGGATGAAGAAAAATGTGGCACATATACACCATGGAATGCTATGCAGCCATAAAAAAGGATGA
GTTCATGTCCTTTGCAGGGACATGGATGAAGCTGGAAATCCTCTTTCTCAGCAAACTATCACAAGAACAGAAAACCAAACACTGCAT
GTTCTCACTCATAAGTGGGAGTTGAACAAGGAGAACACACGGACACAGGGAAGGGAACATCACATACTGGGGCCTGTCGTGGGGTAG
GGGGTAGGGGAGGGATAGCATTAGGAGAAATACCTAATGTAGGTGACAGGTTGATGGGTGCAGCAAACCACCATGGCATGTGTATAC
CTATGTAATGAAACTGCAAGTTCTGCACATGTACTCCGGAACTTAAAGTATAATTTAAAAAAGAATAAATAAAAATAGAGAAGTGTG
CCTGTAATCCCAGCTACTCAGAAGGCTGAGGCAGAAAGATTATATAATACCAGGAGTTTGAGGCAGCAGTGAGGTATGGTCATGCCA
CTATACTCCAGCCTGGGCGACATAGTGAGACCGTCTTTTAAAAAAAATTTTAACATTTAGCCGGGTATGGTGGCACATGCCCCTAGTC
CCAGCTACTCAGGAGGATGAAGCAGGAGGATGGCTTGAGCTCAGGAGTTTGTGGCAGCAGTGAGCTATGGTCTTGCCACTGCATTCC
AGTCTGGACAATGGAGTAAGATCCTGTTTCTAAATATGTGTGTGTGTGTGTGTGTGTGTATAATATGTGTATATGTTGTGTAATC
TTAAGTGAATCTAATAACTGCTCTAGTTTACTCTGCAAATGAAAAAACTAGACTAGACAACCTCTAAAATCTTTGTTGATTCTAAAT
ATCTGTGTCTCAGTTTAGCTCCCATTCCCTTATTCCCAAGTCCAATGCACATCCCATCACTTTGTTTCAACTTTCATGGCCGAAGTG
CAGGTGGGGTGGAGTGTGCCTCTAGGAAAGTGGAAACGGCTGTGGCTTTTCCACTGTGGCTTCAGGGAGCCCTGGCCTTAATCAAGC
TGCTTTCTAGAGCTGTGTGTTGCCTCTGAGCCTGTTTCCTCATTTGTATGATGGGGGATATCATTGTCTCCTTCACAGTGTGATGACAGT
TAGTCATGTACAGAAGGCACCTATAGCTCCTGGCACAGGCTGATGAGCAACAAAAGTTCACCATTATCACTTTCTCAGTGTCTAGGG
GACATTTCATCCCTGAGCATCTCTGGCCATTTCAGGATGTGCTCACTGTCTCTCATTTGCTCTGGTCCCAGTGACCTTGTTAGCTCC
TGGCAATGTGTGTGCAGGCCCTTCTGCCTACCCAGTCAGGTGATGGCCTGGTGAACAACAGCAGAGTAGGCCCATGCCTGATGCTCA
GTCATTTCACCATCTTTTTCCTGCCAGGTGCCATGTCCAAGTACAACACCTACATCAAGGAACTCAGAGTCTGAAGTCAAGGCTGC
AGAAACTGTGGTCTAGCTTTCTGACCAAAGCTGGGTTTGTGCTTCTATGGCAGTGTAGATTCTTTTACTTGAACATACTCAAAATC
TTAGTTTTTAATTGGTGATTCTTTTTAGGATTACAGCAGTTTTGGTCTCAAAGCCAAAAGATTCTGAATTATTACAGATCTTGTGT
TATTTTGTGGCTGTATAACATCTCTTAACCAACCAAACAAGATGAGTAGGTTAGGATTAAACTGGCTGCAGAAGTCTAAATGAAAC
TGAATCCCCAAGTTCTGAGACGCAGTGAGGAGAAACTGTATTGCCAATAGATCTTCTCATCCTGCATTTGGAGTGGTCGGGATTCTG
GAGTCAGATGCCTGGGTTTGAATTGGGGGTTTTCCCAGTCACTAGGTAGGTAGCAACTCTAGTCCAGGCTACTTAATGTCTCGTAAGC
TCAGTTTCCACAGTTGGAAAGTGACGATATGGTGTTTACTTCAGTGGTTGTCCTGAAACTTTATTGAAATGACACAAGTTCGTAACT
GTGCCAGTTCCATTGTAAGGATGCAATAAATGTTAGCTCTTGTTTTTATTAATGACGAGGATATAATGTTTAAACTCAATGCCCACT
TCGGGCAGTTTTTACCCTGTAGAAGCAGTTGTAATCATTTTCAAATCAGGCTTGCTACACCCTTCCAGCTGGAAAGAAAAGTCTCT
AATTTCATTGATTTTCGAGGGCACATGCTTTAGTTGAATGAGTAGTAACCAAATGGAGATATGTAGTAGGCGGTTAGAAGTGTTAGC
ATCTTTGGGATCATGTGGTCAACCACATTATAAATGAATGCTTGGATGTAAGGGCAGACCTGCATCTGTGTGTGTGTTTTCAGTGGG
TGGATGGGGCCATGTGGTATCACAGCGTGCCACACAGGGATTTACGGTGGTCAAGAAGCATTTGTGGTACAGAGCTTGGAGTTTGGA
GTAAGGCTTCCTTGTTTTGCATCCTGGCTCTACCACTGTCAATCTGTGTGACCTTGGGTGAGTCACTTAACTGTTTCCTCACCTGTT
AAATGTGAATAATAACAGTACCTGTCTCCCCCATTTCCAGCAGAGTGGTGGGCAGGTCTAGCTAGCCCTTGTACATACACCTTGCAGAATAGTT
TATAGTAGTGAGCATAGCTGGTAAATGTACAATAAAAAAAGTTCCTGTTTTTCATTTCTACCTGATTGAATGAGACACCTATTTAAT
GCAAATTTCAATAATGCTCTGATACTAACTCTGTTCAAGTTAGTAAGAGAGCTTTGTAGAGTTATTGTGACACCTGGGTTAAGTATG
GCTGACTTTGGGTTAAGTATGAGGTCTAATCATCCCAGTCTGTCCTCTAGATTTTTTGCTGTTTTGTTCAATATAATTATGTAAACA
```

```
GGTATTCTCTATGTAAACATTTGTGAAAGTTAAAATACCAAAGCAGATTAAATATAGAACTAGACTGGATGCCTACATCAGTGTTTT
AGTGACAGCAAAAAAAAAAAAAAAAAATCATCTGTTACATTTAATTATTTTGGCTAGTTTCAGTTCTGTTGGTTTGATAGTATTCTTT
TTAGTTGATATATAAATTTGCTTGGGGTTTTATACCAGTCTTCTATTTCTGCATATTTAAGGAATATTTTAATAAATAATAACTTAT
GTCAACCTTGAGGATCTATGACTTTTTTCTTTTAAAGAGAGTTCATATATTAGTCCATAACCATTGAATAGATCGTTCAGAGTGTAT
GAAGAAGAGCATAATCCTGTGCACAGCTGGCTTTTCTGATTTGCAGGGTATTTAGTGTACATTTCTCTATCCAGCTCATATGCCGGA
CAGAGGAACACTTTACCCTTCTGGTCTCTCTGCCTGTTGGGCAATTGGGTTATTCTGGGTATGTGGAGGAGCTTTTTTGATTCTTGT
TGTGAGGGATGCTTTCAACAGAAAAGAGAGGAAGAATATTTGTCTTTCATTGCTCAATACCCAGATATTTCATATTCATCAGAAATA
GCTACTTCTGAATTCTTTTTTCCTCTTTAAATTAGACACAACATTATCCCGTCTACTGTTATTTCTTTTTTACATTTTTATATTAGC
AGTCGACTGTTGGCATGTTCTCTCAGCTGCCTCTAGTGATGACATCCGGGCTTTTAACAGCCATGCTTTTCCAGGGAGCCCTGTGCA
CTTCATAGATTACACCGTTAGTCCTCACAGTTGTTCCCGGAGGTAGGTAGGATCAGACAGAGCACGTAGGGGTGAGAACAGAGGGCT
GGGGCGAGTCAGTCTGAGAAAAGCAGTGGGTTACTTCTTTTATGTTAGCATTTCTGCTTTAGGCAGAGTTCTGCATTTCCACCTATT
TTCATATTTTCTTCAAAGCATTTTTCAAATGATAAGTCATTAGCTTCATGGCTGCAGAATGAAGTAAGGGGATATTAATATAGCTAT
TGAGAAATAAGAACCTTCACTGAAATATTTTTAGGGTCGTTAAGAGCAGTTTTAACTCTTGGTTTGGGTGTGATTTTTGCCTAGAAT
TTGGAGTGTATTTAAATTGATTTTATATCTAAATTGAGTGATCAAAAGAAACCTCTCTAATGATATAGTTGTCTAATGTCTATTCAG
TGTAACTCTTTGAAGGTGCAAGATTAATATTCCTAAAGCCCAGCTCTGATCATTTCATAATTTTCTTTTGTGTCCTTTTTAACATTA
TGAGCTTTGGATTCAAAAGACCTGGCTTCATTTTATGGGCTGGTTATATAACCTGTCTGATTCTCACTTGCCTCCTCTATAATGGAG
GTAATAATACCCAACTCAAAGAGTGGTGGTTACAATTAAGTGAGATCATGTGTGTGAGATACTAAGCACAGTCGATGTCAATAAAGC
TTTAAAAAGGCAAAAAACAGTACAAAATCCAGATTCTTTGATGCTATGTTTTAGATGCTGCCTTGGAATCACCTCATAACTGAGTTC
TTATTTTCCAGCCAAATACAAAACCTCACTCTTCATTTTTCATGAAGCAGAACAACCAATACAATTTACATGAGGCTAAAAAGCTTC
ATGAAATAGTACTTTATCTTGCCACATGCTATAGCATTCTATTCCATTTCATTGAGGAAGAAAATGGCCATTAAACTGTGATATCAA
GCAAGTTGCAACCTGTTTAAAAAACAGTGGTCTAGAAGGCCTTCCATCTCCATTTCTTAGAATTCTATCCTCCACCCCCCTTGAGAC
CTGTACCCCCTCATGAAATCTTCCCTCATCTTGGCATCTGGTGGCCTTCTCTGAACTTATTTTTTCTCAGTCTCATTTATGGCATTT
ACTGCAAATTGGCATTTAGGTTCATTATTGATTCATAACTTATCTCTATGGTGAGATGATAAGTACCATCAGGGCATGGAACTCCATT
GTGCCATCATTTATCAACCTGTGAACATAGCAAGATGTCCTGCCCACTGAAGCCCTCAGAGGTATTTGTTAGGAAATTGCCTGGTGT
CTTGGATGCTTCCTTACCTTTGTGCTGACATTTGAAAGCCGTAGAATTAGAGTTTGTGTGTTTACCCTTGGGAGCGGGTTGCCTCTC
GCCATGACTACTATCTAGTTCAGATGTTTTCACCTGGCCTGCCTTGGATTTCTTGTGTATGTGAAAAAGAATGACATTAGGTTGAGA
TTGGTATTCTGGCTTCTAAATGGAGATTCTCAATTTTCCTGACTTGACTTTGTTGGGCCTCAGAGGTTCCTTTCAGTTCT
AACGGTGTTTGAGCAAAAATCAGCCTCCTTTCTGATGAGACCCAGCCTGATATCTTTAAACCTTGGACCTTTGCAGCATGTAACAGT
TAATGATTTTAGCCTATGAGACCTGCACTTTATAGCCTAGGACACCTTTGATGTTTCTGTGTATGTACACGGTGATCTGTAGATTCT
ATATGTGAGTATATTTATATGCAGTATTTTATTCATATAGAGCCTTTCATCTACAATGAGAAAATAGTATACAGACAAGAATGGAAT
ACTTATATCTTAATTATACAGATACAGTAAGTCCTCACTTAAGATCATCTATTTGGAAACTGTGACTTTAAGCAAAATGACATGTAT
GTATAAGAAATCTAATTTTACCATAGGCTACTTGATACAGACAAAGTTGAAGTTGCTAGGACATATTTCTGGTCACAAAAACATCA
GCAAACTAAGTAAAGATTCAAAACACTTTTAATATTAAACATTGAAATAAATGTGAGCTATATATACAGAACTCAGGAGGTGGAGGT
TGCAGTGAGCCGAGATCTCACCACTGCACTCCAGCCTGGGGGACAGAGCAAGACTCTGTCTCGAAAACAAGAGAAAGGATTAATAAA
AACAAAATAATTATTTACTCAATGATTCCATGATTTTAGTTCAGGGTTGAGAGTGGCTGGAGTCTGTCCTGGCATATCAGGGCACAA
GGCAGGGACCCACCCTAGACAGGACACCATTCCATCACACATCCACACTCACTCAGACAGGGACCGTGTGGCACTTCAGTTCGGTT
CACCTAATGCACACATCTTTGGGATGTGGGAGGAAACCAGAGTACCCAAAGAAAACCCATGCAGACGTGGGGCAAATGTGCAAACTC
TGCACAATGGCCCCGGCCTCTCATCAATGTTACTGAAGGAAACGACATTGAACAAAATGACATTTCTCGAAGACCTGCTGTACACCA
CATCTCACATTTGGAGCCCAGTTTTGAGAACCTAGTGATAAGATTTTAGGAAGAGCAGGACCCATTTTATGTTTACACAAATTACCT
ACTCAAGGGTCAACCTCATTTAAAGGAACTGATTTAATTTAGGAAAGCTGTTTCTTTCCCTTTTAAATCTAATTTAAGAAGCTTGAC
TGAAATAGTAATAGATACCAGAGAAGTGACTGTGCAGCTACGTAAACTAACAGACCTGGCCATAGAAACATCTCTCTTGGGACAATA
CCTTGATAAAATACATATCAGAAATATCTAGGAAATTATAGATAAAACAGATCATTAACAATTTAATGACACACAGTAAAAAGCAT
TTTTAAAAACATAACTGCATAGTAAAGTCTGGGAAAAGTTACTGACTGGTACACAGGTGTAGGCAGTAAATCTATTCATAGTCAGAT
TTTTCTCTTTTGAGCATTGAACAGTTAATAGGGACTTATGAAATGAGTTTTGTTAATTCAGTACTCAGAATTCTCAGACAAGCTTCC
TTATTTAGATTCTTTCCCATCTAGTCTGCCATTTACCAGAGCTAAAGTGTGTGTCAAAGGAAGCAGGATTTATCTTAAAGGAGATTT
GGAGGAAATCTTCCTTCAATATTTAAGTGTTTGACAAAGTCATGCTCACATGGACGTCAGTTTTAAGAGGAGGAAGTTTAAAATCAG
GTGGCTTATTTACGTTACTGGCTGAAAGCCTGTCTGATAGGATTGACATGGAGCACTAATTAATCACCTAGGGTCTCCTCATTTACT
AATCATATTGCACAAAACTTCCCTGCTGACTGAGGCTCAAGGCAAACTGTGGGCTTCCAGCCAGCTTATTTTTCATAAGCATTATG
CTACCACTGTAAATTAATGTGAACCTAATATATTCTGTCAGCGGTTTACTTTCCCATGGCGACCCTCTCTGGTTAAACAGCTTTAAA
TGAAATCTTGTATCAAAAATGAAAAGAAGTTTCTTGCAAAAGGAAAAAAGGATTTTAGGTCCTCTTTGCAATCTTTTTTCCCCATAG
TATATGCTATTTCGGTTCTGATACAAATCTGCTAGATTAACTGAATAATGCACAGATGATGTGATAGTTTCTTTTTGTTATCTGAAA
AGTGACCTTATATTGTGCTTAGGTAAGAGACTTTTTAATTACACTTTTAATGGCAATAATAGTGTGTTTATTGAGTGCCTACTATAC
ATAAAACACATACCTTAACTCATTGATCCTCATATCAATGCTATGTGAATGCTAGGCAATAGTCTGTATTTATGGGAGAGAAAATTG
AGAGTGTCAGCTTGCCCAGGCCACATCCATTAAATGCTGGGGCCCAGATTTGAACCAAGGTTTGTCATCAGAGGTCAGGCTCATTTG
GCTGTGTCACTATTTATAATTTGTATGGTCCCTTTATACTTTCTGTTTTAACTTCAGGAAATTAGCAGTCCTCTTAATTTTTAAAGA
TAAAAGCTGGGCACGGTGGCTTATGCCTGTAATCCCAGCACTTTGGGAGGCCGAGGCAGGTGGATCACTTGAGGCCAAGAGTTCATG
TTGACCAGCCTGGCCAACATGGCGAAACCTTGTCTCTACTAAAAATACAAAAATTAGCCAGGTGTGGTGGCGCACCTGTAATCC
CAGCTATTCAAGAGGCTGAGGCAAAAGAATCACTTGAACCCGGGAGGCAGAGGTTGCAGTGAGCCGAGCACGCGCCTGCACTCCA
GCCTGGGCAACAGACGGAGACTCTGTCTCAAAAAAAAACCATAATTTTTATTTCCATGTTGATTTTTTAATATTCATCTGAATTAGT
AAATCATTTTAGAAGCTTGCTGATCTCTAGCACATGCTTTTATATCTTCAACTCACTTTAATGTCATGCCACTTTTATTCAGAGAAC
AAAAAGTTATGGATGTCTTTAAACCAGAGGAAATAAGGGACGCTTATTCACATCCCTCATGCTGTTGTTCATCAGGAGACTCTTCCA
AACAATTCTAACAGAAAAGGAAAATTCCAGGTGCACACATGGCACACACATGGACAGTGTTTCCAAGGCAGTGCTTGTTGAGAACCCAGCCTTCTCT
CCTGAAGCTGTGAATAGTTTGTCTTATATGTAAATTAATTTCCAAGAGTCTGTGCATTAAAGTCTTATATAAAAGAACATATAAAAG
AATGATTTGTCAAAACCCATTTTCCTTGTAAAAAATACCTAACTAAGGAGAGAGGATGATATTCGGTGGCTTCTGTGGGCTCTGTGT
TAAGCCAGTACTTTACTTCTTAGTATGCAGTGCTGATGTTCTTGGTTGAAAGGAATCCATTCCATTTCTTTTTATAGTATCATGAAA
AGTTGGAAAGAGGAAAAACTGAGCTGAACATACAGAATAAATAACTAAATGGTGTCGTCAGGCTGCCCTTTTGTTTTCAGACATCCC
CCCTCCTTCCTCCTTTGTGAGTCACTGGCAGCAGTAAGATGGATCAAAGATGCCTCAGTGTATAGAACATTTTATGTGGAGGGTATTT
GTCATCCACAACAAAGGACTAATTGCCTCTGTGCCTTCTTTCACATGATAATCAATGGATTGGGATCTCCTGATTATGCCCCATGCA
TCCTGCATTCATCTCTCAAGATGTTTTAGGTTTTGATTGAATTTATAAAGCATCAGAAAGATGACGTCAGTTCAGTGCAACTATTAA
GGGGATTTTAAACATGGGTATGGAAATATGAAAACAGAGCTTGAAGTGAAGGCACCTGTCATCATTCTTTTTGTAAATGGTTGATTT
GATCGGAGGTATAACTTCTATGATAGCTATTAAGAAGGGTCCTGGCATTTTTAAGATGGAGCGTTTATTTCAAAAGGCCCACCAGGA
GAAAATTCACTTCCTGTTTCAGCGTGGCATGAGGTTCTCAGAGATGACTTCTTGGATGACTTTGTTGCTAATGTTATCAATCAGGT
TTCATTTTTAAAGACTTTCTGAATTCTAACAAGAAGTCAGTGTACTCTGAGTATAGTTTTCTGTAGTGGATTTGCCTAATGTGGGG
GTTGGGCACATAGGCCACGGGAGCGTGTAAGTGTAGGAAACACCATTCAGCTCATTCTGTACAATGTCTGAAGCTTTGAAGTTACAT
TCTCCTGGCATTTCAATTATTTCAAAGGCAAGATGTTTACTAAAATGTACCTGAAACATGGAGTTATTTCTTAGGAACTTTTAATAT
TTACAGTTAAAATTAGAACACTTTAAAATGAAAGAATGATATATTGACTGTAGCACAGACATCTGTTTAATAACAGTCACTTTTCCTC
```

EP 2 204 376 A2

```
TCTTTAAATGTATAAATGTCCTGTGTTTCTTAGAAAAGAAAGATTTCATCAGTGAAACAAACAAAAAAAAACAGAACCCACAAAAACA
AAATAAAATTCAAAAACACACACAGATATATAGGCATAGAGAGAGCATTTGTGATAAGTGTTTTAAAAATAGCTATTAATATCTCAG
CAGCCTCCTCAGCTCTGATGGTGCTGACATCCCACAGGGCAAGCTGAAGCAAGCCAGGGGGCTGGAGAACAATCTAAGACACCAAAC
TCTTCTGTGGAACAATAACACTGAGTGTCAAGGACTGTGAAGGGTCTGAGATTTCACCCTGCATGCCAGCAAACAAGTTATCCTGCC
ACAGTGTCACAAATCCTGGCAGAAGGCATCACACTGCTGGGGCAGACACATAGGACTTTATTAATCACAGCAATGCCAGGAACCAGA
GGATCAATATTTTTGTACACTTTCCCTGAGCCCTAATTCTTACAGGGTGGTGCAGAGAGGGCCAGGTGATACATAGTGAGTTGTATT
CTAGGAGAGGAACCCTGAACTTAGGGAACTCCGATCTTTTTATAATGGGCAGTAAGTCTCCCTGACCCTTGCTCTGTAAAGAAACACT
GAATCTTTCAAGGCTATCTGCTGTTCAAATTTTCTTGAAAAGATAGTAACGAAGGACTCACTTGTAATGTCAGTGCCTTGCTGATAA
GGTGGCTCACACCTGTAATCTCAGTGCTTTGGGAAGCCGAGGCAGGTGGATCACGAAGTCAGGAGTTCAAGACCCGCCTGACCAACA
TGGTGAAACCCTGTCTCTACTAAAAATACAAAAATTAGCCAGGCGTGGTGGCACGCACCTGTAATCCCAGCTACTCAGGAGGCTGAG
GCAGGAGAATCGCTTGAACCGGGGAGGTGGAGGTTGCAGTGAGCCGAGATTGCGCCACTGCACTCCAGCCTGGGCGACAGAGCGAGA
CTCTATCCCCCCACCCTGCCCCCCAAAAAAGTGTAGATGTGAGAATTGTCTCCCACACAAACTCTTCTGGAAATAGGCACCAGAT
AAGTACCGATTCCATGAATGGCTAATCTGTGCTGCCTTCTAAAGGCAGTGCAGGTACCAGATGACACAGCCATAACTGCAGCGTTAT
ATCTCTGTTATTTTAAGAAATGGATAAAAGGTGGTATTTTAATTTATCCTGTATCTATGCAACATATTCTTTTCCAAATGTCTTCCC
TGTGTTTGACAGTCACAGTCTTGTGACATTGCAGCAGGTGTTATTACCCATTTACACTTGAGGAGACTATCATTCAGTGACTTGACC
AAGGTCACACACAGTTACTCTGCAGCTCGCCACTACACCTGGGGGCTCCTGACTCCCCGTCTGATGGTCTTTGGAACACATTGTGTA
ACACTGCCTCCCTTTTGTCTCCTACGTAAGGTAACGGATATTTCCAGATATTACAGAGGTGCCCCCAGGCCAGTCCTGAGTTCCTTT
TTGGCAGTTTCCGTGCCTCCCAACTCATCTTACCTAGCCACACAATGCCCAGTGTCTGGTGGTCCATGCTGGACTCATTTGAGTTGAG
GGAAGTTACTTTTTTAATGCTTTCTTGTTGCTCTATGTTTTAATTTTCACTTAACGGAACACAAACAGTCTCCACTGTTGCCACCCC
TTTGCTTAGTAGAGCTGGCCCCCTTCCCCTACTTAATGTGTAATTGCTCTATGGGCTTTGGTGGCACCTCTTTCTTGGTTTCCTGCTG
CCCTTTTATCTAATTCTTCTGAATCTCCCCTTTTGGTCCCCTCTGTACACTGCCCCATACATCTTCATGCTCTCTAAGACAATGCCT
TGATCCTCTTTCCTCCAGACTATATGTAGCCTTAATGATAACCACAGTCAGTTATTTATTGAGCCAGGCATATGCCAGGCACCTTTT
CATGTGCCACACACTTTAAGCACGTTATTTGAGCACATGCGTTATCTCATTTAGTCTTCCCAATGACCCCACGAGATAGATTCCACT
TGTCTCTGTGTTTTGTAGAGGAAGAAGTAGAGGACTGACAAGTTAAATTACTTGTCTGAGGTCACACAGCCAGTAATGGTGGAACCT
GAATGCTAAGCTAAGTTTGACTCTAGAGCCAACTGCTGTCCAGCCCCCAGATTTCCTTGTGCAAGAGTCAGAATCTTTCCACATCAG
TGTCTGACATCTGTCTTGGTAATAACGCCTAAAACTGAAATCTAAAACTGATATTTCTCTCCAAATATTCTCCACTTCTAGTATTCC
TAACTTTGTAAATGACACCAAAAGCCTGCCAAGTTGTTTAAGCTAAAAACTGGGACGTTTTCTTAGACTGCCACTCTTTTCTTCACCT
CCCAGGACATTCCCCTTCATCCATTCATTTTTTTCCTTGCCTTTCCTCTTGTTTTTCCTTTCCTTTCCTTTCTAACAGACTCGCTGT
CTCACTCAGGCTAGAATGCAGTGACGTGATCATACCTCAATGTAACCTTGAACTCCTGGGCTCAAGCGATCCTCCCACTCAGCCTCC
CAAGTAGCTGTGACCACAGACACATACCACCATGCCCGGCTAATCTTTTTAATTTTATTTTCATATTTATTTTTTATATTCACTGTG
TTGCCCAGGCTGGTCTCAAACTCCTGGACTCAAGCAATCCTCCCACATTGGCCTCCCAAAGTGCTGGGATTACAGACTGAGCCATTG
CACCTGGACCTAAAGGCCCATAATTTTAACCTTGTACCCTGCTGCTTCCTGCCACTGAGTCCATAGAGTCTCTGCCTTTTCATCTGC
TGTCTCCATCTTGCTGCGGCTGCCTCTTCCTTTTTTCAGCTGATGTCATTCTCACCAAATTAGTAGCCTCCCAATTTGTCTTGCTACC
TGCAGTCTCATCTCTCTTCAATTCATTCTTTGTACTGTGCTGGAAAGATGTTTCTGAAATGCAAATCTGATAACTTGTTTTGCTTAA
AATCCTTGGTTGGCTTCCCATGGCTTGCTGGTCCCTTGGTACCCATAACACATACAGCTATGCTGATCAGGCCTTAACCCTTCTAGC
CCAGAGGTTCTCAAACTTCACACATGTCACAATTACCTGCAGGGCTTGGTTAAGAAGACAGAACTTGCTGGGCCCTGCCCCCAGAGT
TTCTGATGTAGTAGGTCTCGAGCTGCCTCAGAATTTGCATTTCTGACAAGTTCCCTGGTGCTACCACTGGTGCGGGACCCATAGCCTG
CCTAGTGCCTTGTCTGGCTCTCTCTGCTCCAGCCCTGGAACCACTTGCAGGTTCCTGAAGGGAGATGCTGTTCAGGCTTTGATCCT
TTTGGATAAGCTGTTGCCTCTACAGGAGATAGTCTTTCTATCGCCGTCTCTTTAAAATACAGTTCAAGTGTCACTTCCTAGGAAGCT
TCCTCTGACCCCTGCAGTGATCTCATAGTTTCTGTGGCAACCCATGTGCATCTCTGGGAATGCTCCATTTCTGTAATTGAGTTGTCA
TTTTATTTATGTATCTCCCACCCACTTCCACCACCAGACCATTAGCTCTTTAAGAAAAGATATCATTCATTTTCTTTTTCACCCTTCTAT
CCCTGGCACCTAGCATGGTGGCTGGTATATATCGAAGATATTCATTAAATAAGTTGAATGATATAATCCTACTTTTACCTTAAAGAA
TAATTCTATTTTGGTTTTTATTTGCATTAACCTTTATCATACCTTGTTTTTAAATGTCAGGATAGACTGGGTGTGGTGGCTCACG
CCTGTAATCCCAGCACTTTTGGAGGCTAAAGGGGGGAGGATCACTTGAGCCTAGGAAAAGTTAAAAAAAAAAAAAAAAAAAGTCAAGA
TGTTTCAACATAAAAACAGTGTGCATCTGACAGTATTAAAACTTTCGAAGCTACCATTAGAGAGAGCTTATAACTCAGCTGTCTAATG
CAAACCGCAGTTTATTCCTACAAAGGCTGATGTTTGTCTATAGAAACTTACTTCTCATGCCTCTGATTTGAAGTGCTTGAGTTATT
CCTCAGTCTTTCTCAGCCTTCAGAACATTTCTCTGGTATGTTTTTCAGCCTTGGATTTATCTAATCAGGGAGTTACAGATGGAATAG
TGCAAGCCTTTCTTTTACTGAGACAAGCAAAGATTAGTGGGCTGTAAACTTGCAGCCTCCATGTTTCCATCCAAAAGAGGATGGCTC
ATCCCTCACTTTCCCTTTTACGTGCCTGCATTGCTTGGGTAATTTCCAAGAAGAGTATCAGTTTGATTGCTTGCACAGCCCTCCCTT
CTCTCCCCTCCCCATCTCAGATGACTAGTCCTTGCCAAAATGTCAAGTTCCAAACCCTAACAAAAGAGTTTGCAAACAAGACTGTCC
CTGGATTGTAGACATTTCTTCAAGGGGTGCCTGGCCTTCTCCTGGTGTAGTGCAAGTTCAAAGGCAAAGAGCCCTTTCATCCTCTGG
GAAAACAGAGAACACAGTTCCAAAAATGCTTGGATACCTCCAAATAGACCTCCTCCCCTGCCTCACCCCTTTCCCAGGCTCTGCTAT
GTGGTGTTTTTCAGGAACCATTCTGTGGATTTTAGCCATATCATTCTGCTTCCATAGTTCAACAAGCAAGCAGCAGACAGCTCTTTC
CTGCAAGTGGATGTGTGGCTGAATTATATATTCCTTACACAGCAAGTAACAACATCTTGCCCAGTAGTTCAGAACAACTGGTATCTC
CATTCAATTATTTGGAAAGAAATTTCCCTCTGCCAGAGCATGATGATTAGTCTCTGAGCACCATCAGTCTCATGCCTGTTTTGCATATCT
TGTAGCTTACTGTAAGCTTGTCACAGGATTAAAGCATGAAAGCAATTGATCTGGTCTCTTATTTATGTCTTAGGACCAGTCTTAATC
TGTTTTGTGGTGTTGCTACAATAGAATACCCGAGCTGGGTGATTTATAAAGAAGAGACATTTACTTAGCTCATGGTTCTGCAGGCT
GGGAAGTTCAAGGGCATGGCCCTGGTTTCTGGCAAGGGCTTCCATGCTGCATCATAACATGGTGAGATGCACAAAGGGGAGACAGAC
ACGTGCAAAGAGGGATCAAACAGGAGAAGGAATCTCACTTAATGCCAACCAACTCTTGTGGGAAATAATTCATTCCCAGAGAACTAA
TGCCCCCATGACCCAAACACCTCCCACTAAGCCCCACTAGCTCTGACACCACCACATTGGGGATCAAATTTCAACATTCATTTTGGTGA
GGGCAGACAAACCGTGCTCAAACCATAGCAGAATGCTTTTGGCTCATGTAACAGAATGCCCCCAAAGTAGCTTTAGCTAAGATGAG
AGGCGCTTACTTAAAATAGGAGCCCAGGGGATTGCATGGAACCCAGCATAAGGAGTGCAACCACAGGTCCCGGGATAAGAAGCTGGA
AGCAGTCAGGAACATAAGCTACTCTGGGTCTCTTTTCTACTTTCTATATCTACATAATATCCCCCAGTCTATTCTAGCATGAGGGAG
ACTAGGCTGAGAAAAATCACTTTGTCCAATACCAGACTCTTGGGAAATGGGAGACTCTGGCCCAGCCTGGTATTCAGTGGAACAGCT
CTTCCTGAGTCATATGTCTGACCCTAGTGACTCATCTGTCCAGAGAGTGGGAGAAGGCCCATCCTCCACAAATATGCGTACAGGAGC
CCCTGTTGGGGCAGGGCCAGTTTTCAGAAAGGGGGATGTAGATGGAGGAGCTAACTCGATTAATATTGGTTATCATTTGTATCCCAC
GTTCTTCTGAAAGGATTCTGTAAAAAAATAGGCACAATGAAACCAAACCCATTCACACAGGATATCATGCAAAAACCAGGAGCAATA
AGGTTACGGAGAGGAAAGATGCATCTAGAAGCCCTGATGGAGAGAAACTTCTTTAATGCAGAAATTAAATTAATCAAAATTAGTGGA
TTTAAAAGTTCTGGGGCTAGTCATCCAGAATTCATGAACTAGGTTGCTAAGAAAAGCAATGGAAAAGATGTGAAATTAAGTACCATA
TGAGTATCTGACTAGAGTGTGTTAGTTTGTTTTAAATGCATGGGATACTGTATACATTTTTTCCTGGAATGACCCTCAAGCTGAAAT
TGAGGAGAAGAAGATAAATGGGAGGCCGGATGCTGCCACTTCCTGTGAGCACATGCTCTCTGGTTAGGACAGGCCCCATCACTGATCT
CTGTTATCTAATCCCAGGTCACATCACTCCTCCATAATCCTCTGTGTTTGGGTGTGTGTGTGTGTAAATGTCAAATAAGGGCACATG
CAGCAAGTGATACAGAAGTTCAGGAGAGGGAGAGAGAGAGAGAGAGAGAGCCTCTGATCACTGTGAACTAGTATATTTTCTTGTA
TTTGGAGGAGGCAGGACGTGAAGCTGCATTTCATTTTGACCAGTTGTAGATGGCAGGGTTGGAGGGGCAGGTTGAGAAATGTGTCAG
GAGGGGGGCAGGAACTGATATTAATGACAATACTTGCCAAGCACTCAGTGCTCACGTGGTCACATCAACACACAATTGCTTTGTG
```

182

```
CAGTTCTGAGGTGCCCTTCTTATCTGCATGTGACAGGTAAGGACACCAGAGCTCTAAGAAGTCAAGAAACCCTCCAGTGATTCTTTG
TCTCAGGAGAAACCAGAGTCCTCACCACAGCCTGCAAAGCCCAACACAACCTGGCTCCTATCATCCTCTGTGCTGTGTCTTGCAGCC
ACTTGGTGGCTGTGCCTCCAGACTTTCCCTGGAATGCTGTTCCCCCGACACCTGCCATTCACTCCTTCACCTCTTTTGTGTTTTTGC
TTATATGTCACTTTCTTAGTGAAGACCTCTGTTATTTGCTCTGCTTTCATATTGCAGTGCCTCCTCTGTCTTCTGCCACATGACAAG
CCCGTGACACTGATCTCTTTCCACAGCACTTCCCATTTTAAAAAAATTCCCTGCATCTACTCACTTATGTTTCTTTATATTGCCTTTC
TCTTCCTGCACACTTCAACTAGAATGTAAACCCCACAAGACCAGTCACTTTGTTTTGTTTCCCAGTTTATCCAAAGCCCCTAGATGA
CCTCCTTGCTCATGGTAGGTGCTCAATAAATATTTGCCAAATGAATGAAGACCCTTTCCCAAGATCATGAAACTACGTAGCACTGGA
GTCAGGGTTCAGCAGGCAGAGTGCACTCGGGCCACACTGAAAGGAGGCCATCAGGATGAAGTGACATGAGAGATGGTGACCCTGGCT
GGATGGGACATGTCCTGTTTACAGTTTGAGCTTTATCAGTCACGCCATCTTACCATGAGGACTCCGGACAGTTTGAATGTACAAACT
GGGACACGGGGACCCAATGGGAAAACTGGGATTGACTTTCTGCATATTGAATTTTCCACCAGCCCACAGTTACTCCTTTGACATGGC
CAGAATTATCCCCAGAGGTTTTTTTATTCCTGAAATAAGTCCCTCCTCTATAAAGACATTTATTTTGTTATTATAAGTATTTAGCAC
ATTTGTGTGGACCCTGCTAAAACGAGTGTTGTTCAGACTAAAGTGTCAGAAAATATATAATACAGAGTACAGGTGCTGCAGCTTCAG
GAAAATACAACTTTCCTAATGGCACTTCTCCCCTTGCCTCCCTTCTATGCTAAATTTTTAAATGCATTTCTCCTGTTTATTCAGGCA
GGGATTAACATTTTCTTAAAGACTTCATCAGAATGTTGGGTTTTTTTCCATTCATTGTTATTTAGGATGATATTTTTGTGACTCCAA
TTTAGGTAAGAAGATTGACTTGATCTTTATTTTCCTCTATCATATTTACCTTGTGTGCTGTTATTTTGAGTCTGTTCACAGGATCCT
GGGCAGATGCCATCAATGTAATTGCTATGTTTTTAAGCAAAGAGGATTGAAAGTGTCAATTTAAGAATTGTCTAGGAACTTGATATA
TACTATCTATCTATCTATCTATCTATCTATCTATCTATTTGCTTTTTATGAATAAAAGTAGCAGGGGTGTTGGAA
ATATATGTATATTTTTAAATAATTTATTTTTTAAAAGTATACAAACCGAAATTTATTTCAAAGAGAATTCAGATAGCAGCCAAAATA
CACAGAAAAGAAAACTGTCCTTCAGAAGTGCCACTAGATCAAGCTAGTTATGAAATTGGCACCTGAGAATAACTTCTGTCAGAGGGG
ATGTAAGTCTTTGGAATTTTGAAGAGCCATAGTCTCTGTAGAAGATGCCAGAAATGTAGGCATTATCCTTAACACTTCCTTTTCCTT
CCCCCTTCCTCCCACCCCCACTGAAAATGTCAGTTTTACTTCTCAGTAGCTCTCAATTCCGACCGTTTCACCGTATTTCAGCCCAGGA
CATGATCACATCTTGCCTGTATTACCAAAGTCACCTCCTTTTGTGATCACTGTAGATGCACTCAGACTCTAATCTTTTCTGCGGCCCAG
ATCCTCATTTTTCCAAAGCCCAGATCTGCAAAGACCTGCCTGCCTACCCCACCCCTGCTCACCACACATAGCTGGCCTTTAAATGGC
TTCCTGGCACTTGTAGGAGAATGACTGGATTCCTTAAGGTGGGCTACAAGGCCTGCATGGGCTGGTCCATGCTCGTGTCTCTAATTG
CAAACCTGCTGTACCTTCCCCACTTCCCCCATTCTCTGTGCACCAGCCACAGCAATTGCCTTTTAGACCTGTTTACTTGTCATGCTT
CTCTCTCTACTGGGACTTTGTACATTCTGCTCTGCCTGAAACACTCTTCCTCCTTTCTTTGCTCATTAACTCCTTCTCATCCTTTGG
ACTACATGTACGCATTTCTCACTCAGGGAAACCTTCAGATGCCCCTCCAGGTCAAATCCCCCTGTTATAGTTTCTTTGGCCTTATTT
GTTTCTCCTTTGTAGCACCATTAGGAATGATTTTACGTTTTACTCTTTATTTGAGGACTCTCTCTCTCTCTTACTGGATGTATGCT
CATTGGAGGCAGGAACTGTTTCTGTTTTCTTACCTTCATTTATCCCAAGAGAAAAGTAGTGTCTAGTACAAAATAGGAACTCAAGAA
TATTTGTTGCAGATTATAAATGAATGGATGAATGGCCTCTTGAAGTATCCATTTTTATACCAGTTAGAATAAGGCACTGTACCAATT
TCAAAATGTTGGAGGTTCAGCAAGGAAGAAAACCACCAGATAGGGAGGAAAGGATCTTCCTTTTGTAGAATTCAGAGAAAAGTGGGG
CAGATGTGTCCCTCCAGAGGTAACTAATCGCCCTCATCACATGCTGGGGCCATTTTCTTCTCCAGCTGTTCCCAAGGGTCAGCTCTG
CCGGCTGTCCTTCTGCTATGCCTGAAGGCTTCCATCAGCCTGCCTGAGGGCTGTTCTCATTCCTAGGTGGGAAAACATACCTTTGAT
GCCTAGAAATTAAAAGAGTAATTTAGAGACTTAGGGCATGAGTTTCATTACAGTTCTCCGTCCCTGAGCTTGCTCTGTGGACAAACA
TTCTGTGCATATTAGGAAAGTATAATAGTTTTGGTCTTGGGCTAAGGATTCAGATTTTCTGAGCAATGTAGCTCAGGCACCTTTATG
ATCTTGGGAGGAATACTTAAAAATTTCTTTTTTTTTTTCTTTTTTGTTTGAGACGGAGTCTTGCTCTGTCGCCCAGGCTGGAGTGCAGTG
GCGCGATCTCAGCTCACTGCAACCTCCGCCTCCCAGGTTCAAGCGATTCTCCTGCCTCAGCCTCCCAAGTAGCTGGGACTACAGGTG
TGCACCACCACGTCCAGCTAATTTTTGTATTTTTAGTAGAGACGGGGTTTTACCATGTTGGCCAGGATGGTCTCGATCTCTTGACCT
CATGATCCGCCCACCTTGGCCTCCCAAAGTGCTGGGATTACAGGTGTGAACCCCTGCACCTGGCCTTAAAATTTCTAAGCCTCAGTT
TCCTTATCTATAAAATGGGGATTAAAACAATATCTAGTGCCTGGGATATTGTTATGGGATACTTTGTACCAACATTATTCTATAAAA
ATATAATGCAAACCACCTATGTAGTTTACGTTTTCCATAGCTATCTTTAAAAAGTGAAATGAAATTAATTTAAATATTTGACCCAGT
ATATCCAAAATGTTATCACGTCAAGATGTAATCGATAATAAATATTAATGAGATATTTTACATTCCTTTTATTCATATTAAGTTTTC
AAAATTCAGCCTGCACTTTACACTTACCAGTGCAGCTCAGTTTGAAACAGTAGTCATGTGCCATATAATGATGTTTCAGTTAACAGC
CAACTGCATATACAATGGTGGTCCCATAAGATTATTATATTGTGTTTTTACTATGCCTTTTCTATGTTTAGATACGTTTGGATGCCA
GATACTTACCATTGTATTACAGTTGCCTACAGTATTCAGTACAGTGACATGCAGTATAGGTTTGTAGCCTAGGAGCAATAGGCTATA
CCATATAGCCTAGGTGCGTCATAGGCTGTACCATCTAGGCTTGTGTAAGTACACTATAGTTTTGCATGACTACACATTTCTCAGAA
CGTATTCCCTTTATTAAGCAACACGTGGCTGTAGTTTAGCCATACTTAGCATTCAACAAATGCCGGCAAAATTATTGTTATTTATTT
TAAAACATCAAAATTTGGGTTGACAAACATTTGTACTGTACATTTGCATATATTGCCATGTGGGAAGATGCCTCAGATAAGATGCCT
CAGATATATTTTTTTCCATTTCAAAAGAGTAAAGGGTTGACTTTAAGACTTCTCAGCTATTTCCTGCAAGTGGGCCTCCCTGGGTTT
CAGAATGAGGTCAGTTTCGTGGTTTATGGTTGTCTTTTCTTTGGGAGGTGGGAAGGGGAAACTGAATGTGCCTCAAGTATTGCATGC
CTGTCTCTGTTTTTTGGCTCTTCTCTGTTTAATTATTGGGAACTGGAACAACTTGATTTTAAAACAAAACATATGGCTGAGAATAA
TTGGGAGGACATTTGCTAGTGATGTAGCACTGAAGGTATGTATCAAGTGAGGCCCAGGCTTGCACCACTTGCTTGCTTAGGTATTG
AGTGTTTCCCTTAACTTCAAATTGTGTTCTTTCCCAGTTCTTAAACCCTTGTTTGGAAAGTCACAGTCAAAGCAGGAAATATGGGAG
CAAGATTATAAAGGAAATTGTCTGTGAGTCTTCTTTAAATCCCTACTTTTTTTCTCTCTCCTCTTTATGGAAAACCATTGGTTCCA
AGCTATTTATATGGCTCGTGAATGAAAAAAGTTGTCTCAAAGTGCCAAGAGGAGAGACGAAAACTTTATTTAAAAAGGAGAATGTGG
TGAAGGCAAGGTTTTTATCTCTCTTTTACCTTTTCCACACCCCTTTGCTTGCTGGGGGAGGAATATATGATATGCTGATCATGTACCA
TGTAATTCCAGCCTGAATGCCTCAACTTTTTATATCATGGGAGCAAAGGGGAATTTATCAGGACTGCTAAGAACATGTATTTCTCTT
GTTCAAGTCTCTGCTCCTCTGCTTAGAAGCTATGTGCCTCTGAGCAAGTTATTTAAACTCTCTAAACCTCATTTTCCTCTTTCCTAA
AAAGGGGATAATAAGAGCGCCTATTTCAGAGTAAAATTGTTGTGAAGCTTTAAAGAGACATTGCACACAAAGCACTAAGCATGATGC
CTGGAAAGTAATAAAAACAAATCTTAGCGATCAAGCAGAGGCCTTCGGCAGGAACATGTCCACACCATCAGCAGCAGGAGGGACAGT
GAATACTGCGTCAGGGAAAATAACCAAACATTTGTCCATGAGGGAGCTTCAGCATTGGAAAGTCAGCCATTTCACTGGGTTGTGTTTG
ATGTCAATGGGTTGAAGTCAAAGCCTTAACTCTGTGCTTCTGTGCTTCCTAGGTCTCATGGGAGCCTGTACACTCACTTGTCAGTGG
TCGTTCTCTTTTGCCCAGCACCTATAAATATGAGAGTAACATGCTGAATTGTTTTACTTTTGTGCTACAGAGTCCTAAGTACCTTTG
AGTCTTTTGGAGATTCACTTGTTTTGGATCTGAAATGTAAAATGAAATAGCTCTCTGACCAGGGCTGAGCACGGTTTTTCAGTTGGG
ATAGTAAAACGAGAAATCTTTCTGAACACTTTCCAAGGAGTCCCCTATAAGAGCAAGGGGTAGGAAGAAAGGGGTCTTCAGTTCTTG
CCAAATCAAGGACATGATTTTCTGTATAGGGCTCAGAATGTATTGTGTGTTGTCTTGCTTCAATGTCAGATGAGTGTCGAGAGCTTC
GGGAAGGTCTGCTTTTTAAAAAACACCACTATGTGTTTCGGAGCAGCTAATTGTCTTTGTCAGTCCCCTTTTCTTTCTGCTCCTGCCA
TCTGTCCAAGTCCCGTTTGGATTTATTAGTGATTGCTTACAAAGTATCAGGAACTCCCTGCTGAGAACAGTGAAGCCCTTGCCCAGT
GGGCAGGTCTCGGATTTCCCTGGAACTGTAAAAAAATAACCACTCTCACTCATTTTTTTCTCTGCATACCCCTCTCAGTGGACCACTT
AAAGGAAATTGAGAGTTGCTATTGATAATAAAGATGAAGCATATCTTTACTCATGGCCAATTACACACAGTCCTGATCATTGGATTT
TTAGTGGGTGGACAGCTCTGTACAATGTGTCATCAAGTCTGTGTCGCCCCAGTTACAAAACACTCCATTGTTTTCTATATCAAGGGG
GAAAAAATTATGAGATACCATGGATTTTAAGATACATCCCAATTTCAGATGTGTTCAAATGTGGGGAAAATATATGTATCTTATAGC
AGATGGAATACAGTGAGAATTAATTAAAATGGAAAGCCCTAGTCCGGGAGTGGTGGCTCACACCTATAATCTCAGCATTTTGAGAGG
CCGAGGCGGGAGGATTGCTTGAGCCCAGGAGTGTGAGACTAGCCTGGGCAATATAGTGAGGCCTTGTCTCTACAAATAATTGAAAAA
```

```
TTAGCCAGGCATGGTGGCATGCGCCTATGGTCCCAGCTACTTGGAAGGCTGAGGCAGGAGGCTCGGTTAAGCCTGGGTAGTTGAGGC
TGCAGTGAACTGTGATTGAGCCACTGCACTCCAGCCTGTGTGAGAGAGTAAAACCCCATCTCAAAAAAAAAAAAAAAAAAAAAGAAAA
ACAGTAAAATGGAAAGCCCTCCTGGTTATCACCTGTTCCTCTCCTGGCATTCCCCCAGTCCTCAACACAGCTAATTGACAGAACGGT
GAGATTTGTGGGTATGTATGAGATGTGCCTCTGCCTTAGGGCTGAATGCGTAACTCAGTCCCATGTGTTGTATACTCTGTGTATGCA
TGAAGTTGTGGAACTGAGTCAAAACCAAGAAGGGTGGTGGGAGGTCCTCTGAACTAAAAATTTACACAATCGAAGTTTCTGAAAACAG
AACTTAGGAATTTTCTTTGGGTTACATTGATAAGATTCTTGTTACCTTTGGAAGGCATGATCAAAGAGGGACATCCAAGGCTCTACA
TGCCCCTCGCTGACCTGGCCCATTTGTAATGGCTCAATACTGAAGAGAAGATCCCAGTTCCTCTAAGTTAATGCCTCTGCTTATCCA
TTTTCTAGTGACCACAGGCCTCAGATCCATGGGGAATCCGTTTCCTCCCCTACCCTAAAAGGCAGCATATTAAATGTTATTGATAAT
TTCAGTGTACTCAGGATTCTACCTAATTATTACTCTTCATAATCTGAATGACTTCTAACCAACTGTGTGTCTCCAGCCCTTCTTAAT
GCTGTAAAAATTTTAAGTAGGGGGATTATGGTATTTTATTTTCCTGGTAAAGATAAGTGCTCACACTTTAGGAAAAGCTATTTCCCAG
CCAGGTAAAGTGACAAAAGAATTGATTGAAAAGTTTCTTCTTTATATTAGCGGCATTTTCCCTTATATTTACACTGCTTTCTTTCTT
TTTCAGTTTTTGGGAATAACATTTCTTGGAATTGGACTGTGGGCATGGAATGAAAAAGTAAGTTGAATATCATGACAATGCACACAT
CGTGGTTGTTAGAGGCTGCACATCATATTATGTATTATCCCCCTTTCTCTTTGACAACCACAGAGTATAAACATGGCTCTTTAACCAT
GAAAAAGTGTTGGTGGAAGATTAATGTCATACTCTCAAAGACACTTTAGGAATTGTGCCTAAATTGATGACTGCATCAAAATAATTA
CTGAAGTAACAATACACGTGCTCAACAGAGAATATACCTTGGAATTTGATCAGTTAGCCAAAGATCGATGTTCCTATGGGATATTTT
CTCACTCAGGCACAAGAATTCCAGTAATGATTATAAATATCACTAAACCACCTGTAGCCTTTTTTTTCTCTTCTGCTTTTTTTCTTCA
ACTCTTTGGAAGATAACTCAAGATAAAAACGATTTTTTTTAGTGTGTTATGTGGTAAACAACTATTAGCATGTGGATTGAAGGAAGT
GGGATATAACTTCATGTTTGAGTATCAAATACACTCATCATTAATGTAAATAATGGGAATGTTTGGCTGCTGGTGGCAAGTATTAAG
AAATATTCAGTTTATGATCAAATACAGTATAATAAAAGGAAAATCTGGAAATACAGAGTAATCTATGTGACAGTGAAGATACAATGT
TCTGGGCAGTTTCTGCTCTAAGCAACGCACTCATGCAGACACCCCCTGTGTCCACACCTCTCCTTCCCATCGCACCTTCTAATTTTT
CTCTACAGCTCCCCCTACTGAGGCCACCAGTGGAATTCAACAGTGAATATAAACACCCGCCCCCTCCTCCCAGCTTTTTTCCTTCCT
TCTTTGCAAGTGAAATCACCTGCAGCATTTCATCTATCCTTGTATCTGGCTATCACAAAAGCTTTATTATCTCCCACGTAAGGACA
AGGGGATGTATAGAATGGTTAAAAGCTGCCATAAACTAAAATCTAGCTTAAAATTTCCCAGAAGACCCATTCTTTGATCCCGTAGGTG
TTTCACCCTCTAGCCTGACCCTGGCCTGAAGCTGAGAGTTCCTTTGAAGTGGTAAATGCATTAAGAAAGGAGAGAAAACTGGGAAG
GTCCAGTCAACAAAAGCCCCTCAGAGGCCAGTAGGCATTTCCACAACTAAAACAATGTTCAGACAGACCATATAAAACAAAACTCAA
AGCAAATCCTGAGTTTTGCAAATGAACCATCCTGTATTTATTTACAAAATTCTTCTACAGAATGTAAAATGTTTTATGGAAAACAAA
CTACTTTTTAGCACAAGAGTAACTGAGGCTACCTGGAGTTTTGAGGCAGGGCAGTGATTATGTATGAGGGGAGACAAATTAAGTTC
CCAAAACAGAGATAATTTCATCTCTAGAAACTTTTACATTGAATATCGGTTGATCTTGTCTTGGATTATGGTTAACTTTTGCCTCAA
GCCTATTGTGAACTTGGAAATTTGTGGAATCTGGGAAGTTGGTTAGATGCCAGATAACACAGCTTTTTATAACTCCTTTAATAAGAG
TCCATTTCTATCTGCTTTGAAAAAAATGCTGGAAGGCATTAAATGAACTAATTATCTGTTCTAGCAATAAACAGCAGTTGGTAATTTT
GAAGTTTTTTAATTTTCTCTTTATGAAATATAATTATCTTTTTGTTTCATCAGTTGCCTCAAATGTCTCTTCTCTGATAAAATCACA
AACTTCTAAAACTGGCAGTGTCATACAAATATCAGAGCAAGGGGTTGATGCGGTCTTTAGGGAGGGATGAAATAGCAGTTCCTTTTT
TGTAAGGGCCACCATCAAGATTTCTCTTGAGATCCGAAGTTAACCTGGCCAAGGTCACCCAGCTCCATGTAGCAGAACTCCATAAGC
AGGGAACAAATAGAGGCAGCAGAGGGTGAAGCGGGTGTTAGCCGTGCACCAGCCCAGTTTGGTGAGTGCTGGCCATGTGTCTGGGGC
CTGAGTGTGGGGAATACGACAATGAGGGATGCAGAGTCCATGCCTCATGGAGCTTGCATTCTGGTGGGTCACCAATGAGAAGCAGTT
ACTGCCCAAGTATGTTTGTCCAGATACTGGTAAGTTCTGGCAGGAAACATCAAGTTAGAGAGTGGTGGGAAAAGGAGTCTCTTTTAG
ATGAAGTAGTTGCCAACAGCCTCTCTAAGGAGGTGGCATGGGAGCAGAGACCTGGATGAAGGAAGGAAGGATCTGCAGGGTATGGGG
AAGAACATTCTAGGAAGAGGGGAAGAGCAAGTGCAGTGGCCCTGAGGCAGGGACTTTGGTGACTCCAGGGAACAACAAGAAGGCCAGT
AGGCCTAGAAGGGAGAAAGCAAGGACAAGAAGAGGTGGGTTGTGTTGCCAGGTAGAGGCCAGACTGGGCAGAGTCCTGCAAGCAAGTT
TGGATATATTCTAGGTACAAGGGGAAGCCACTGGAGCATTTTGAAAAGTCTAATATGACTTTTTTTTTTAAGATCACTCTGGTTGCTG
GGTGGGAAAAGAGCACAGCTTTGTGGCTTTGCTATGGACTGAATATTTGTGTCTCCCCACCCTGCCAAATTCATATGCTGAGGCCCT
CATCCCCAATGTAATGATATTTGGAGGTGAAGCTTTGGCGGGGAGGTAATTAGGTCATGAGGGCANNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNATGTTTGTTGTTTAAGT
CACCCAGTTTATGATCAGTGTTATACAGCCGACTAAGACAGGGGTAAACAGACCTGAGTTCAAATACTGGCTCCTCCAGTATTTGAC
ATGTGTGGAATACACACATCTCATCTGTGTGATGTTGGCCAACTGAGTTAACTTCTCTGAGATGCCATTTCCTTGTTTGGCAATAGG
TATACTGTTCTCACCTTATAGAATTGTTGTGAAGATTAAATGAGACCTCCCTCAGCACAGTGTCTGCTCTAAGGCAGATGCCCACTG
GAGACATGGCTCCCTCCCCTGGACACATGACCATAGGCTGTCCACTCTTAATATGGGGCAAGAGCACAAAGGTAGTTTTGAAAGGCA
GCCTCAAATTTTAGGAGAGTTTCAGGCTCCAATCCATGGACCAGAGAATTGGGTTTGTTTGTGGTCTCTTGAGGAGGATGTGAAGT
GTGTTGATCTCCTTGGTAACTGCAGAGACTGCTCTCTCAAAGCCCTTTGGGTTAATTTCTGAGTTCGGCCAGGCCTCTGCTGGAGTA
TCCCCCTAGACCCAGATGTGGCGACATTTACTTGTAAGAGGCCTGGAGGGTAAATCCCCAAACTGGATCATAGCTGGAAGGATGAGAT
TTCCACTGGAAGAAACACATTTTCTTAGCAGATAGGACTTTCAGCATATTCATGTGAAGTACAGAAGCATTGAAAGCAAAAATGATT
ACCCCATACTTAATATCAGTCTGAGTTTTAAACCTAGGGCTAATATTGCAGTTCAACATTAGCATAATAATTTAAAGAATTCATTT
TGTCTGAGTTTTAAATCAACTGTAAAAATGCAAAGGATGAGTGTCTCCAAGGGAAGCTGATTTATGTGCAATTGTTCAGAGATCATA
GGATCTGCTGTTTGGAAGGAACATTAACAATTATGTGACATGAATCACCTCCACAACACCCTTGACAACATCCAGTGACAGTCAACT
CATTCATTCAGATAACATTTATTGAGCACATAGCATGGCCAGGGCTTGCTGAATTCTAGGGAAATGTGGATGAAGTGACCTAGTT
TCAGTCTTCATGGAGTTTATCCAGTTGTAGGAAGGGTGTGTGCATATACCTAAAATAATACACTGCAATAAATGCTACTTAGGGGTG
TATATATGTTTAATGGTTCCCACAGCAAAGTGCTCAGCATTTCTGTCTCTTTAGGTTGCCCTTCCTGTTGGAAAGACCTTCATTCCA
CCGAATTAGAATCATGTCCCACTAGTTTTCACTCATTGATTCTGGATCTACCCTGTTGGATCCCACAGAATAAGTCTTTCCACATGT
TAGCCCGGGAGGTATGTCAGGGTGAGGGCCTGGTTCCTTCTGAGTTTCCAGGCTAAACATCTGCTTCTCTCTAGGTGGTCCTCACAG
GATGTGGGCTGGAGCCCCTTGTCCATTTTGTTGGTGCTCATCTGGCCCCGCTCTGGTGTCTTTTCTGCACCCATGGAGGAAGCTTT
AGGGGCTGTAGAGAGTGTGGCTGGATTGCGTGCCTTTCTCCCCTCATCAATCTAGCTTGGGATCCACTGTAGATCACGGTGTCTGCAG
CTCACTGAGAGTCCCAAGTAGGTTGCAGACCCAACTGGTTACCCCATCCACCTCACCTATGGCTGATTTTCATAGGCAGTTGCAGGA
TTCCACAATGACACAGTGATATTTCAGCCCACCTGTCCACCTGCTGAGATCACTCCAGAGCCTCTATCTAGCATCTTCCCCATTCCG
CACCTGGGAAAATTCTGATTCAGTAGGTCCAGGATAGACACTGCGATGCTCTATTTTAACAGGCCTACTAGATTGTTGTGACTGCAG
TCAAGTTTAGTTATCATTGATAAAAGCCAGTGACATGCTGTTGAGCAGGCCAAGGACAGAGCCCAATGACATGTCACTAGAAATTTC
CTTTGGGGTTGACACTCATTCACTTATGTGTGCCCTTGGGTGTCATCAGTCAAGGAGTTTCTCATCCATCTACTTGTCATTACATCA
GACTATATTTCTGCATCTTGTCTTTGTAGATAGATGGTATCTTTCACTTTTTCACTGGTTAACTGGCCTAGGCACTTAACTGAACCT
AGCACTTTCTTATTGAACCCATGCTGGGTCCTGAAGCACATCATTTTCTCATCCAGGTACTTGGGAACCTTTATCAGCAAAGCAAGG
ACTAAGGCATAATACATCACTCCTCAATTCGAGAAACGTTTTCCTCACTTGGCTTCCAGGATATCACACTCTCCTGGTTTCCATACC
TCACAGGTGGTGCCTTCTCACATGTGTTTGAGTAGTTCCACCTCATTTCCCTGACCTCTAAATTTTGGAGTGCCCAAGGCTCAGTCTG
AGCTTTGACTCGTCTTAAGAAATCTCATCTAGACTTGGGGCTTTACATGCCATCTGTCACTGATGACTTCCAAATGAATATTTTTGC
TTCCAACCTCATCTGAATTCTAGACTTGCATAGCCAGCATCCACACTGAAATGCCTGGTAGGCTTCTGAAACTTAACACATTCAAAA
CAACACAAGTGATTCTCATGCCCTCCCAACCTGCTCTTCCCAATTGCGGTAAAGAAACAACTCCACTCTTCTACTTGCTCATTCCTG
```

```
ACACTCTGCTTTCTTTTCTCCTCATACCATATTCAGCCCATCAGCAAATACTGTCAGCTCTACTTTGAAACTATATTGAAAATCCGA
TTTCTTCCACCACCTCCGCTGTTATACCCTGTTCTAAGTGGCCACCACATCTTGCCTGCATTTGTGCAGAAGCCTCCAATTATCTCC
CTGCCTCTGCTCTTGCACCCTGACCCTCACCCTAGGGCTGTCTCTGTGCAGCAGCTGGAGTGATCCTTTGGAAATGTAAGACAGATC
CTTTTACTTCTCTGCTTCTAGCCCTCCAGTGAGCTTATCTTCTCACTCAAAGCCATATATGGTGCCCATGGCCTGTGTGACATTTGC
TTTCATACTGTGTGCTTCCTCACTGTGTCCTATGGTCCTACTTATCAACTTGCTATTCCTCCCGCATCCCAAACATACCCCCACAAC
CCCAGGGCCTTTGCACTTGCCCCTGACTGGCATGCTCTTCCCCAGAAAACCACATGAGTGCTATCTTCTTCTACTTCCTGGTTTCTTCTC
AAATGTCACCTTTGACCTAGCCCTTTCTTCCCCTTCCTTGTGTTTTTCCTTCTCATGCGACTCATCACTTAGCACGCTAACAAGTTTA
TTGCCTGGACTTTTTATTTATTACCACACTCCTAGTACCCTAGAATGTTAGATGGCACATAGCAAGTATTCAAGAGGTATTGCTTATGA
AAGAATGAATGAGCAAATGAGCAATGACAGCATTCTTTAAAACACTGGCTATGTCATGGAGTTATAGAAGTCAGTACTTGCCTCACA
TCTCCAGTAAGCATACTATATTAGCGATTCTTACGAATAAAAGGAGGTAAATACGTGTGGTTTCTGAAGTCTGATTTGAGAATAAT
ACGGGGTTAGAAACAAAATATATATATTGTCTATATCAGAAAAGAAGAAAATTACTTCTCTCGTAAAAGTGGAAATCTATATATGAT
TATCTCATTTCATGGAAAAATTAGGGATGCCATTCCTTTTCCGTTTTGCTCAACTCTTATGCTTATTACTTAGTTCTTAAGTGGGAG
CAAGAGGGAGAGTGATCACTCACAATTAGAAATAAAGGTATTGGGGCGAGGGAGGACTCAGCTTTTCAGAGGAGGGAAAATGCCTTT
CCCTGGTCCTTAGTGTGAATTGAATGTTCCCATTCTGCCCCTGCCTCCCTAATACAGCACCTAGCCCACTAAGCAGAGTGGATACCT
ACTCCTGGCTTGTCTGGCCTGGAAACTCCTTGAGGACAGGAGCCTGTCTTTGCCCTTTATTCTCAGTTCCTACCACAATGCCTCCTA
CACAATGGATGCCCAGAAGCTGTTTGTGACTAAGCCAATGAGAACATGCAAAAAGAGCTTTACACGTTGTAAAATGCTACACAGGAA
AAAAATACCACTTTTTATAAAGGAGTTGCATTTGCACTTCCCTGACTTCTGGATCCCCTCAAGTTAACTTTAAGAGAATGACATTCT
ATTTCATAGGTGGGGAAACCAAGGCACAGAGATGTCAAACTGGAAGTCTGGCCCTCTCCCTCCCAGTCCTCTGGCCAGTCCCCTGTC
ATCCTCCATTGCACTCACTACGCTGGGTTCTCTAGGGGTGATTGGAAGGAAAGAACTGGTGCCCAGTGCTGATCCCAGTAGGGAGAA
GGGGGAATTAGAGGAGCTCTAGCCCCCATATTGCTTTTCTCCTTGGCTTTGCTGTAGATGATGTTTCTTTTTCCTGGAGGGTTTTTC
CCAGCCCACTGTCTGTGATTTTAGGTCTCAATGCAGAACACTCCAGTTCTTCCTGTCACAATTATACAGGAGACTCACCTTGGAAAA
CCTTTCCCTATTTGCCGAGGAAGGAAAATTCAGTGAGATTCATTGATTCAGGAATGTCATAGCAGTACAGGCCAGTTTCTGTTGCCT
GAGGGAGGCAATAGAGGAAAATGGTTACTCAGATGACTTGAGCAGAAAAAAATAGATTTCTTAACATTGAAACTTCTTTAAGGAGAC
ATTACTGAGATGCTGAAAATACTTCTATGAAGACTTGGTTTTATTGACTGAAAGTAGATTGTGATTCTTTTCCTCAATTATCCCAAT
GATTCCCTCAAGTTCTCTGTTGTATCTAAAGAGCTGGAAACCGGCCGGGCGCGGTGGCTCACACCTGTAATCCCAGCACTTTGGGAG
GCCGAGGCGAGCAGATCACGAGGTCAGGAGATCGAAACCATGCTGGCTAACACGGTGAAACCCCATCTCTACTAAAAATACAAAAAA
TCAGCCAGGCATGGTGGCAGGCGCCTGTAGTCCCAGCTACTTGGGAGGCTGAGGCAGGAGAATGGTATGAACCCAGGAGGCGGAGCT
TGCAGTGAGCCCAGATCACGCCACTGCACTCCAGCCTGGGCAGCAGAGTGAGACTCCATCTCAAAAAAAAAAAAAAAAAAAAAAGAGC
TGGAAACCAAACAGCTCATGGTCAGTACCTGGAAGCTATGTGATCGATGTGCTCTAATCAGTAATGTTTCACACACACATGAAGTA
TATGGGAAGAAAATTTAATGTTTCTCTATGTATTCCCCTTTAAACTGTATTCAGTGGGGTTTCTTAGGGATGAAAATTTAGTTGGGT
CAAAAAATTCAATGACTTTGTTGTTCACTCTCAGATATCACAGTCCTTCTCTCACTTTGAGGCACAGATCCAGGCCCAGTGATTAGAC
AGGGCTGGATAATAAAAACTGCAAAACCTAACTTTGCATTAAAATGTTCTCCCTGCCGAGCCCATCAGCCTTGGCCCCTGAGGAGGGAA
GGGAACATGGGCCCTTGCCCTCTGCATCCTCCTTCTGGCCCCTGGCCCTGCCCAGATGCCTCTGCCCACCGCCTGCCCGGGCAGAAG
CTGGGGTGGGCTGTGCTCTCTGGGCCTGGTGGAAGTAAAGAGGAGAATATCCTGGTCTTTGTGAAGCTCCCTGCTGAGTCCCTGT
GCCTAGTGATTTCCTTGAGCTTGGTAGCCATCTCAATTCCAGGTGACCACCTGGCAGCCCTGCTCATCCCTGGCCACCTTCCTCAGC
CAGTGCACCTCTGAGCTCTGCCCTCTGAGGTCACTTCTTCACACGCCTGCAGGCAGCCCTATCTGACAGCGTCCAGGATGACCTCAG
GCCACCTCTTCCGTGCTGCTCCCCGTGTTTTCTCCAGGACATACTCATCTGGGACGGCGAGGTCAGTGCCCCCAGAACAGCCCATTGCCT
GTCCTCACCTACTTCTGATACCAGAGCAGCCAGCCGGCCCTTCTCTCTCTAGGTTGCAAGGGCAGGAGGCAGCCCACAGCCCCTTCC
TAGGCAGAGTCGGGCCCTAATGGCTGTGCGGGTCAAGTGCAGAGAACCACTTCAGAGCTCCCGGGAGGTGCCCTTGGAACTCCTCCT
AAGGTTTAGGGTGAGAAAGAGGCTATCCACACTCCACCCCCAGGCCCTGGGCAGTGGTGTCACAGCACAGCAACTGCTTTCTCCAGA
GAAACCTTGCCTCTCAGTCCTCCTCTGACCCCTTTTGTCCCTGCCAGATTTCACCGAGGCGCACAAAAGAAAGATGTCCTTTGTCGG
CGGTGGTGGAGGTGGGGTGTGGTTGGGGAGGTCTTTGTCTCCTACTCACTTTGGTTTTCAGATAGTGATCAGTGAAATCAGAGGCAAGA
ATGTTATAACATATTGTGTGTATATTTTTCTTAAAAATTTTAGAAACATATGTATGATGTTACGTTGGAAATAAGAATCCTCCTGCCAGC
TGCTACCCCAACTTCCAGCAATAGATCACAGGGAGTGGTGTGAACCCTGCTCTTGGGTCAGGCGGCCCAGGCTCAGAACCCTGCTCT
GTCACTTCCTGACCTGGGCTACCTGGGCATGTTAGTTCCTCTCTGTGTCTGTCTCCTCACATGGGTAAGAGTGCCTGCCTGGCTGGT
TTCTGGTGAGGGTGAAGTAGTCCAGGTAGCCCAGTGCCTGGCTGGAGTCACTGCTGAGAAGTGTAGTTCTTTTGGTGCTGCTTTTAT
TCAGGCCTTCAGCAGATCTCACCTGGATCACCCCCAGAGCCTGCTAGCTGCTTCCACAGCACGTGACTGCCACCTGAGCGGGCTTCTA
AATTCAGAAATGGCCTGGAGCTTCCTCTGCTCCAAATGCCCTCAGATACAAACCCAGCCTCGCCCAACCAGCCTCTTCATCTCTTCT
CACCGCTTGTCACATAGAGGCCCCACTGGGCCAGATCCTGTCGCTTTGTCTGCACTCTTACTGATAGGACCTTCTGCCCCTCCCCAT
GCACCCCCATAACACCCACTGCTTGTGCCTCTGCTCCATGCACTCTGTTCTTAACCGTCTCCCCTCTAGACTCAGCTCCTGAGGGTG
TCACTGGATGTTTTGCTAGAAATACCCTTGACTGACATAATAACCTGGCAACTCACAGGTGATCCTCATGAAAGTATTTGCTCAATAA
ATGAACGAGTCTCATATTTTTGAGGTCCCAGAATAGAGGGTCACCTGCCGCTAGTCCAGACTTCACCTTTTTTTCACTTGAGTTTAGG
GGCACCATGATCAGAGCCTGTTTTAGCTCAGGCTGCTGTAACAAAATACTATAGACTGGGTGGCTTAAATAACAAACATTTCTCTCT
CACAGTTCGCCAGGCTGGGAAGTCCAAGATCAAGGTGGTGACAGATTCAGTTCCTGGCGAGGGCCCTCGGTTTGCAGCCGCCTTTTT
GCTGTGTGCTGGCATGGCAGAGTGAGAGAGGGAGGGCCCTGGTCTCTTCCTTTTCTTTTTTTTTTTTTTTTTTTTGAGATGGAGTCTC
TCTGTGTCACCCAAGCTGGAGTGCAGTGGCACGATCTCGGCTCACTGCAACCTCCGCCTCCTGGATTCAAGCAGTTCTCCTGCTCAG
CCTTCCGAGTAGCTGGGACTACAAGGCGCACCTGGCTGATTTTTGTATTTTTAGTAGAGACGGGGTTTCACTGTGTTGGCCAGGC
TGGTCTCAAACTCCTGACCTCATGATCTGCCCGCCTCAGTCTCCCAAAGTGCTGGGATTACAGGTGTGAGCCACTGTGCCCAGCCTC
TTCCTTTTCTTATAAAGACACTAATCCCATCATGGGGTCCCACCCTCATGACATCTTCCAAACCTAATTACCTCCCAAAGGCCCCA
CCTCAAAATACTACCACACGGGGGCTTAGGGTATCAGAATGTGAATTTGGGGGAGTCACGAACATTTAGTCCATACCAGAACCTCTA
TTAAATCTTTCAGCTTAAACTTTACACCTAGGCTGGAATGTTTCTAGGTATGTCTCCCGTCTTCTGAATGTAAATGAAAATATCTGG
GAATGGCTGAGAACATGGGTGATTTCTGAAAGGCTGCGCTTTTGCATGTGGCTGTTTCCACGCTGATGGGGCAGTGGCTCTCACCAG
AGTGTGTAGAGATGGTAGTTTCCCTGCCTGTGAGCAGTGCCACCTGATGGAACTTGGTACCGCTGGCCTCCCCAGCAGCTACTTGAT
TGCAGGATTGTATGGAGACCTAAAAGATGACTACCCCTCCTTCTCCTCTTCTCCTCAAAGGCAGGTCATGGCCACTTTTTAATCTGC
AAAAAAAGGATTACTTGAGTTTCACTAATGACCTTTTTTGACAAGAAAAAATGGGAAGGAGCCATGCACCTTTGAAGCAGCTAGTCG
TCTTTTATCCTTGTATCTCTTTGCCAGGCATGAACTAAGCTGCTCTTGCTCCCTTACATCTCCCTGGACTGTTCAGGGCCTGACAGG
CTTTCTTCTCATTCCTAGTCGTCACTCTGGCCTCTGCGCAGTTCGTTAGATAGATGGATGTTTGGAGTTTTACAGAGTGGCGGAGG
ATCTTTGACAAGTTTTTATAGGGGCAGGTTCCTCTGTGGCCCTGTGGTCTCTATTTGTTCTTACTGTGCATACTGTTTAGAACCACA
TTTCTAACTCTCCAGGATCACCCCTCGCTGAGTAGCTGTATTTTCTTTGAAATGTCATTCCTCGTTACCCTTTCTCACTAGGGAAAT
CCTTCTCTATCTCTTACTCTAGGAAAACTGTTGTCAGTAGGATCTGGTTTATGGAGGGGTTGGTGAATCAAGAGCTAGTCTCATAAA
GTGCTTGTATATGCAAAAGCAGAAGAGATCCCAGACCTTGGGGTTTATTAATGCTATTTTTGTATTTCTTTTCTGTGTGGGGTTGCGAG
AAGAAGCTTGCCAGGTGGCACAGAGAGGCTCCTCTGTGCTGGTGATGAAAAAAAAAAACACAGACACTCCTGAAGTTTATTGGTGTG
GAACCCTTCTGGAGTTTTGTTTGCCTTTTCGTTTCTGCATGTGCATAGAAACCTCTTTCAAGAAGAAATAGTTACTCAGAACATATT
CCTTTATTGCAGAAGCATGGGGAAGAAGCTGCTTAAATTCCAGAGACAGCTGAATGAATAAAGGCACTAATGAGGAGAGGGAAGGTTA
GCTGGTTTGTCCTTTTTTTTTTTTTTTTTGAGATGGAGGCTCTCTCTGTCTCCCAGGCTGTAGTGCAGTTTCGCAGTCTCAGCTCAC
```

```
TGGCGTGGGCAAGGTCCGCCTCCCGGGTTCATGCCATTCTCCTGCCTCAGCCTCCCGAGTAGCTGGGACTACTCCCACCACCACACC
TGGCTAATTTTTTGTATTTTTTAGTAGAGACAGGATTTCACCACGTTAGCCAGGATGGTCTCGATCTCCTGACCTCTTGATCCACCCG
CCTCGGCCTCCCAGAGTGCTGGGATTACAGGCATGAGCCACTGCGCCTGGCCGGTTGGCTGGTTTTTCACATTCTTACTGAGACATA
CTGTGTTCATCAAACCACTGAACAGCCAGATCTAACGGGGATAGAACATAGTTCAGCCACTGGAAGGTCACTCCAGAGTGGTGACTG
ACAACCAGTGTGCCAGTCAGCACCCCAGCAACAGAGGTATCCTTGCTCCTGGTTCCATGTGCCACCATCGTGGCAGCACCACTGCTG
TCTTACCGCAGTCACTGACTCCTCCTCCCTGCTTTCTTCTCTGCTGCACCACTTCTCAGTACCCTAAGCTTCCACCACTCATGGCTTCTG
CCTACTCCTGGCTTCAGCTTCCTCATGGCCTCCCTCCTCTTCTGCCTTTTCTCTCCATGTCTTAGATTATTTCCAATTCCCCCCAAAAGA
AGATCTTATTAACAAGTTAGTGACTGTCACCTCTGTTAGGCATGATCATCAACTGTGGCCATGGCTTTCCTTAGAGGGGGGTCAGGCA
GGGCAAGTACCCAGAGCAAAACAGTTCTTCTCCAGTGCAGTTGGGTTAGACACTCCTCCTGGGTGCCTCTACAGTGTCTGGTGTCT
TCTTCTTGTTCTAATAATTATATTATTAATATAATAGTGATAACAGTGATTATGGCAATATACTTTTATTTAATTCTTATTATATTC
GAAGCACTTCACATCGTGTTTGGATTTACTTGGATTGTTTCCATCATCCTCATAATAACCTTAGGACATAGAGTACCATTTTCACAG
ATACAGAANNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNTTAGCATTGGGATTCAAGACAAGCTAGTCTGTATTAGTGTCTGTC
TGTTTGTCTGTCTCCCCCATTGTCCAGGCTACCCCACTTTCTGAGTTTTGGATTTACTTGGATCCCAGGCGATATTGGAGGTGAGCC
TCATTGGCTTAAGTACAGTTCAGGATGGTTTGTTCAGGAGGAGGTTGATTCTGGAATGCTCTCTCTGCCCCACTCTAGAGCAGGCTA
GTCTAAGTGGCTGGAATATCAGAGTCTCCCTTGTACCAAGATGGGTGCAAATGGATACATTGTTGCTTGTTGGCTCTCTAAACTTCT
GTTACAATCCTATTGCCTCAGACATGGACCACTAATGTGTCTTATTTCTTCTTAGACATATACTGGTCTCTTTGCTAAGTTATGAGA
GATGCTGTCTGGTACCTAGTGGATAATGGAAATCTCCTATTGCAGAATCATCTCTTAAAGGCTTCAAGTGGATATGCCAGAGTTAGT
TGTCCAAAAGCTAATTTGCAGAATGGCCAATTTGTTGAATATATTTTTTTCTTTCAACTTTTTAAATTGACTGCTATTCATTTTGTC
TTTATAGCAGCACTTTATGTTATGGTCAATTTGTTTTTTGCCTCTGCTGCCTGCTGCAACAACAGCTGTAGACCTCAGAGGCAGGAG
AAGAGAAGATTAAGGATCCTAAGAAGAATATTGACATAGCATATAGTCTGCATACGAAGAATTCTTACGAATATATTCATTTATATC
AGTTCTTACTAATAAAATTTTAATGTATTAATAAGATATATGTAGAGGAATTCTTCCCACATAGCATTCTGAATCGATCATCTTGAA
TATATTTTTCTCATCACCTGCAGTCTTGGTCTCTGGCTCTCTCTCATTCTGAATCTTTCTGTTTCTGCTACTACTGTTCAGAATCTC
ACTTTCATATGTCAACTAAAACGGATGGAAATAACTGTGTGTGGCCCGGTTACTCTGCTTCTCACTCATTTATCCTTGTTCCAGTTAG
GTTCAGCTTCATGGGATATTTTCTGATGTTATCTGCGAAGTCTAGTACCCTTGACATCTTTAGCCCCCAGGGCTTGCTATGCTTGTA
GGATTCAGGTAGGAGAAGGATGAGAACTTCCTATGGAATATAGATGCCAAGCTCTGTGTGTGCACTTCTGGCATAAACAGGTACAGA
AGAGAGTAGTCAAATAACATTTTGTATTTATTTACTTATTTATGTTTTATTACTTTTTGAGATGTGATTTCACTCTGTTGCCCAGGC
TGGAGTGCAGTGATGTGATCATGGCTCACTGCAGCCTCCAACTCGTGGGCTCAAGTGATCTTTCTGCCTCAGCCTCCCAAGTAGCTA
GGACTACAGGCACACACCAGTGTACCTGACTTATGTTTAAATTTTTTCTAGAGATGGGGTCTTGTTTTATTGCCCAGGCTGGTCTCA
ACTTCTGGCTTCAGGCAATCCCCCTACCTCGGCCTCCCAATGTATTGATTTTTTTCCGTGTTTGAGGTTTTAAAAGTTGGTATTTAA
ACTCAAGGGCTGAACATTCAAGTTTACATGCAGAATTGGAGGTTAAACCATGTTATGCATTATTTGTCAGTAACATAGACAAATCAC
CTAAAAGTTCATAAGAGTTCCACAGAACTGCTCATTTCATTGATGATGCTGAATTCATAGTGAGTTAACATGAATGAGACTGTTTA
CACCGGCTTAATTACTACGTTGACTTGAAATATCAGCCAGCTGGGGAAAATGACCATTTAAAACCTGGCACATCCCTTTGCTCCCTA
TACCCACATTTTTTCCCAGCTTCGACCACGTGCGTGTGTCTGTGTGTCTATGTGCATGTCTGTGTGTGCGTTTACGTGCATGGAGGA
CTGAAGCATAGTGTATTCAAAGCTTTTGTGTTGTGCAGGACTTCGCAGATGCAAATGAAGACCCAAAGAGAACCAGCATAAGCAATC
AAGGGAGTGTATCTAAGGCTCCTGGAGTGACACATAGAGCTCTAGGGCAGGCCTCAGGAACCACTGGACTGGGGATTTATAATGCTG
TCTGGATTTTCCCTCCCTACTCCTCTTTGTTTCTTTGTTTCTTCTTGAGCGCGGTTTTCTGCTTCTCACTGCTGTCTGACTTTCTCCACTTG
GTAGGAAATATGACATGCTTCACAAGGCTCTGGAGTTTGGCATTTTAGAGTCTCAGCCACTAGAGAAAGGTGCCTGTTCTAAATCCT
GGCACTTTAGGAAGGGAATCCAGGCTTCTCAGCCCTCACCAGGCCAGCAGGATTGTATGAGATCTTGGCAGCTCTGAGGGGTCATGG
AGTGAAAGGAGGGGCGAAGGCATCCTTACAAGGAGGAGGTGGTGCTGTTCCCAGAAGAGGCGGGGGCCTCAGGTTCCCAGAAGACTA
GGTGTCTGCTGGTGGTGTTCTTGCACATTCCTAAGGATTTACTGTGAATCTGGTGCCTGTTTTAAATTGCTTGGCAGACTTAACTGT
AATCTTAGATTTTTTTTCTTACAGCTTCTTGTCAACTACAAGGTTTAATTATAATTACTCTTCAGCAGGCATATACTAGCACTTCTT
ACTCATCTGTCAAATTGAGCTGAATGAAATTAAAAAGTCCAGAAAGAGACCCCATCTTTAGGACAACAGAAGACTGAACAGTCAACT
CCCTATCATTTTGTAATCCAAATCACTGTTGTCAGGATTATGTTCCCCCCCACCTTTTTTTTTTTTTTAGACAGAGTCTTGCTCTG
TCACCAGGCTGGAGTGCAGTGGCTCAATCTCAGCTCACTGCAACCTCTGCCTCCTGAGTTCAAGCGATTCTCCTGCCTCAGCCTCTC
GAGTAGCTGGGATTACAGGCAGGTACCACCACGTGCAGCTCATTTGTGTATTTTTAGTTGAGACTGGGGTTTCACTGTGTTGGCCAGG
ATGGTCTCGATTTCTTGACCTCATGATCTGTCCGCCTTGGCCTCCCAATCCCTCTGTGTAGTCAAAAGCAATAAAAACTGCTGCTCTT
CAGTGAATGTTGGGAAATTGTGCACAGATGAAAGAATAAGTCTGTTTTTTTTCTCCTGCTAGGTAAGTAATCCCTAATTTTCCCTAA
TCCCAAGGGGAAAAATACTGGCCATTGGGCATTGACTTCCACATTCACTTCTTGTGTTTCAAGATTTGAGGTAGTGATGATGGCTAA
TAATATATTCTTGAAAAATGCCGAGTGGATGTAAAGTGTTCTCACCACAAAAATGATAACTATGTGAGGTAATGCACATGCTAACTA
GCCAGATTTAGTCATTCCACTATTTATATATACACGCACCTCAAAACATGGACATGTACAATACATACAATTTTTTTTTTTTCTGAG
GCAGAGTCTCGNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNCCAGGCTGGAGTGCAGTGGTGCGATATCGGCTCACTGCAAGCT
CCGCCTCCCGGGTTCACGCCATTCTTCTGCCTCAGCCTCCCGAGTTGCTGGAACTACAGGCGCGTGCTGCCACGCCCGGCCTAATTTT
TTGCATTTTTAGTAGAGACGGGGGTTTCACCATGTTAGCCAGGATGGTCTCGATTTCCTGACCTCGTGATCTGCCCGCCTCGGCCT
CCCAAAGTGCTGGGATTACAGGCGTGAGCCATCAAGCCGGGCCAATACATACAATTTTATGTCCATTTCAGTTTTTTAAAATTTTAA
AATAATTTTTAAATAAGTAGATAAAGGATATATTCTTTTCACCCAGGAAAAAAAAGATTTGAGTTAGTTTTCAACTGATAACTACGTG
GGTATATTCTGAATCCATTAAAAATAAAGTTCTTGGTAGTCTTTCCTATGCTTTGTGGGAGATTAGTTCCCCCCAGCACGCTGTAGT
ATCTGTTTGCAGCTCTATCTTTGCCAGAATTTAGGATTAAATAAGTAGAATCTGCAGTCCAGCTCAGCCCCCCAATGATTGTTGTTT
CCGTGGAAACAGTTGAAGGCTCTCAATTAAAACTGCCTCTGAAGGCTGGAGAGGTAAGTATTAAAATGAGTTTGTCTGAGAAGCCA
ATTTTTATCCTCCAAATTAAACCCAGAGAGTGCAGATGATCTAGGAATCTCCTGACAGTTGGGATCATACTTCTTTGTCTTTGGGGG
AGGGATTATTTTGCTATTTCGGGCATAGGTATGTTTGCTAGCTTGTTGCTTGCTTTGCTGAGTTGCTCAGATCATCGTCTTTGTTAC
CCACTGAGCTCTTTATGTACAACCATGGATCATGGGCAAGGGTTTGGGAGCAATCTGATCTTTGTTCAGACTTGTGAGCCGCAACTA
AAAAGCCCATTAAAAATGGATGAAATCATAGAACGGTCTCAAAGTTGAAAGGAATCCTAAAGGTCATTAAGGCCTAATTTCCCATCC
ACTTTGGGAATCTTTCCTAGAATGCTGTTGACAAAGGTTCACAGCATCAGCAACCCTGTTGGGGAATCCCGTATTTCTCAGGGCAGC
CTTCTTTGGTTCATTGAACAGACTTGGTGGTATTGTTACCGGGGGTCCTTGTTCTTAGAGCTCCCAAGATGGTGGCGGGCCGCTTCC
AAGGTGGCGGCAAGCCTCTTGTTCTCTGACCTGGGGTTCTTGGCCTCACGGATTCCAAGGAATGGAATCTTGGGCCATGCCATGCGGT
GAGTGTTATAGCTCTATCCAGCTCGATTAGGATGAACCCTGGGCACTTAGACCGTGCAGGAACAATGGCGAGCCTCTAGCCCGATTA
AACGACGGCAATCAGCAGTGGTGGACGGCGAGTGAAAGCTCAGTTCGAACCAGAACAAACACAGACCAGAGAGTGTGCAGTTTGCAA
GATTTAATAGAGTGAAAACAGAGCTCCCATACAACGGGAGGGGACCCAAAGGGGTTGCCCACTCCCTGCTTGAATGCCTGAGTTTAT
ATCTCGATCATTGTCCCTCCCCCTGTGCTCTCAGGCGATATATGATTTGACAATTTCTTTACCTCCTGCTTTAGCCTAATTTGTATT
TTAGTGAGCCTATTTACTACCTGATTGGTTGGGTGTGAGCTGAGTAGTTACAAGCCCCATGTTTAAAAGTGGGTGCGGTCACCTTTC
CCAGCTAGGCTTAGGAATTCTTATTCGGCCTGGGAAATCCAGCTAGTCCTGTCTCTCAGTATTAGAAATGCCTCTTTATGATGGAAC
```

EP 2 204 376 A2

```
TGACATTTCTCTCTGTCAATTCTGCTACTGCTACCTCTTCTTTCTGGTGCCAAGTTAGCTGTGTCTTCTTCTCCCATAATTAAGCTT
AAAGTCTTTTAAGAATGTCTTCATGTATCCCCAAAGTCTTCTCTCTCTTCCGGTGGCTGAGAGGTCCATAACCTGAAAGCTTCAGG
TACTAACATCTGTGGTTAAAGGCATTGTCAGAGGAGAGGCTGGTTTCCTAACAACCCTGGGCCTTTAGCACTGTTGCTTGGATTTTT
TATTTTAAGTTTATTTTTGTAGAGAAAGGGTCTCGCTATACTGCCCAGGCTGGTCTCAAACTCCTGGCCTCAAGCGATCCTCCCACT
TCAGCCTCCCAAAGTGTTGGGATTACAGGTGTGAGCCACTCCACCCAGCCTGTTGCTTGGATTTTGTCCGACTTCTCTGGCAAGTTT
TTTCTCAGCATCATTTGTATAGGTGGAGAATTTGGTTTGAGTATTTCTGTTTTCCTTCAACCTTTTCCCCCGGAGTAAATATTCATA
GTTCACTGCTTGGAAACCCAAGAAACAAAATTGGATCAAACCTTTGGTCTATGCAGACTTGTGAAGGGTGTGGTTGCCTTCTTTGAT
GTCACCTCAGAAGTTAATGAATACAACCTCAATCCTAACTGCTTTAATAGCCTTTCAACCATTTGTCTGTGACATTAAACCTTAGAC
TTTTTAAAATTAGGTTTAAGTTTTACATTATAAGAGTTCTTTATTTCCCCATATACATTCTGTTCTTAATATCCTATTATTGTTCGT
AAGTTGACACCCCCCTAAAGGAAGAAACAGACAGAAAAATAGAACAAACGAAATACGCTTCTCCAAATGTTCCTACCTACAACCTTT
AAATGGATTAAAACTTAAAACTCCTAAGTTCAGATGTTGGTTAGTGCTGACTGGGAAGTACTGGGAAGCAAGTGTGTGGCCCCTGCT
TGGTACTGAAATAGACATTGTGTACTGGTTTCAAAGAAGCGATGGCGTCTGTCACCTTCAGCCCATCACCAAACTGCTCTGAGTTCC
ATTTTCCTCAAGTATAAAATCAGATGATTGCTTCCTGTTCTTCAGGTTATTGGGAGTGCTTTGAAAACAATTTTTTTTTATTATTA
AGGCTTTACAGCAAACTATACTGTGATTCTCTGGAGGTATTTTGATTTGAGAGTACCAAACAACTTCAATAAAATTAAAATATTGGT
GATATTGGCTGTTTAAGGCCCATGCACATCCACAGTTTCAAAATAATCTTGTAACATAGGTTCTGGGTGGTATTTAATATGGTATCA
CACATAAGGGAAGAGGGGATTTCAGTAAAGAAATGTCATTTTTTGTGGAAAAGTGAAATTACTCGAGGTCCGTTTGGTGTTCTTGTG
GAAGAAGAATAAGAGTTGAAAGTGAGGAAGTAACCTTGATTGTAAATCATTTAAATTTCAACTCGAAGGGGGTAAGAAATACCCC
AACAGCAAGCCACAGAACAGCCACCAGAGCAAACCACACAAGGCTTTTGGGATCTCAGCCTAATAAAGGTCTCCCATGGAGCTCAAG
AGTAGATCCTGAGGTACGATATTTTCTTGGAAAAAAAAAACTTGTAGGTGTTTGTAGCCGGACCGGGTGCTGTGTTAGTTTTCAGCT
TCTAGCTCGATCCTATTTCCTGAGAAATGCCAAGGGCTTTGCAATAGGCATTTGTCTAACTTCTGCGTGAATTGGGTCTTACAGAAG
ATGACCTCCCCCCTCTCTGGGGGTCAGGGGGTGTGTGTGTTTTTTATACATCTGTCTTTTTTTGTTTGTTTGTTTTTACTGAAGACC
TCTTATATTTGCAGACACTGAAACCAAAGGTAACACCACGTCTTCTCCTTACAGGGTCTAATGACAGTTTCTTGGGCTCTTCTTTTC
TTTTTTTTTTTTTTTTCTCTACATATAGTTTTCCCAAAAATATGAACACGTTTAGATGTTGGAAAGAAAGCAGTTTCCCTCCCTCCT
TCCCTCCATCCCCCCTCCCTCCCTTCCTCGCTTCCTCCCTTCCTCCCTTTTGTTTCTCTCTTTTCTCTTTCTCTCTCTTTCTCTTT
CTTTCTCACTCTTTCTTTCTCTTTCTCTTTCTCTCTCTCTTTCTTTCCTCTCTTAAGTATTAATCACATACCTGATAATCAGATGCC
CTCTGAAGGTTTTCTTTGGGAGGGGAAAGGACAAATGCAATAATCTTTACATGTTGGAAAACTGACCCCACATCCCCGTGCCTTGTT
GTGTGTTCTTTTCTGCTCCCAGGGAGTTCTGTCCAACATCTTCCAACACCGATCTCGGCGGCTTTGACCCAGTTTGGCTCTTCCT
TGTGGTGGGAGGAGTGATGTTCATTTTGGGATTTGCAGGGTGCATTGGAGCGCTACGGGAAAACACTTTCCTTCTCAAGTTTGTAAG
TATTCCACTCTCTACTCTCAGATTGAGAGCCAAAACTTTACCTTCCATCAGGAGGTCACCCTATCTACAGGCAAGCAGTGAGCTGT
ACCAAAGGCACTGGCCAAAGTAGGGCCCAGGTCATTATTTTTCTTTTGAGAGAGCTCTCTAGCCCTGATTCCCAGTTGTGCCTCCTTA
ATAAGTACAACACCACCCCCACCACCCCACCATGCCATCCATGGAGGTTTTGTGCATATATATCACAGACTTGGATTCCATTCCCTA
AGATATCTTTAGGACTAAGGTAGGTATATATATATATTTTCTTTAATGATTCCTTCCCCACCGTTTTAATGCACATAGTAAGTGGG
GAGCTGTGCAGGCTGTTGGTTTGGAGAAACCAGGCAAAAGCACAGTGACTGTGGCCTGTCCAGATTAAATCTGTTAAGCAGTAGGTT
TTGCTAAATATGGAGAAACAGTAAAAGGTAAAGCTTCTTTGTCCTTAGCTGGTACAAAGTTCTCCTTTGTAATTTTTAGATGTGGTG
CGGTTGGCGTGATCACTGTCTGAGTGAATGTTTGTAGAGGAGAGGTTTCACCAGGGGGTTCACTGGTATTTAAAGTTAAAATAGAGA
ATGAAAAGTGAAAGCCTTTGCGTTGGAGACTCGTTTCAAAGGAGAAGAAAATGGCATGTGCCGTTTATGTATCTTATATAGGGTTGT
TGAATGTAGCCAAATGTGCCTGTCAGCATGGAGCTTGGCACTCTGAATCATAGCTCTCTATACGAAGCATCCATTC
CCCTTTCTAAGAACAGAGCAAAGGATCCAAAGCACCCACCTTCATTTGCTGAGGAAGGATGGGGATGGTGGAGATTTCTAGAGAACAG
TTGTAGTGTAATATTTCCTGTTACCACAGTTATTCCCAACTTTTGTTGACACATCATCATCCAGGGACATTCTCATTAAAAAAAAAA
AAAAAAAAAAAAAGCATATCCAAGAAGTAGCCCAATAGTCATGTAATGTGTGATTCTCTTCCCCAGCATTTCTGCCACATCATCCTC
CAGCGTCTTTTCTTAAACATCCCTGAGAAGAGGGTACACGGTGTGCCCCACCCAGGGCAGCCCATTTCTGAGCAGCTCTGTCTCGGA
GAAGTTTTTGGAGGCTATTTAACCTGAAAATTAGCATGACTTTATTCTAACAAAGAGAACCGAGGGGTACCTGGTCTTACAGATAAGT
TCACAAGCAGCTGGTTCTCTCTTGTGGATACATGGGACCACAGTGGCTGTAGCTAGTTTCCTAAGACTCCATCTGCCTTATCTGCCA
GATCCTTCCTTTGCCACACACACACACAGTGTCTCTCTCTCTCTCCCTCCCTCTCTCATACACACACAACCAAATGCTTCATTCT
TCACCTAGTCAGAAACCTGGGGAGCCCCCAGGAGAGCTCTGTCCCTGATTGCCACCTGCCAACCTGCCAGTCTTCTTGGCTTGAGGC
ATCTCAGTGCTCCAGTGCTCAGCTCACCTTGCCAGCTTTCCTGTAGCCTGATTGGGTCCTGGTCATACTCATCTGTTGCCACTATTT
CATCAGTTTCGGCCTCCATGCCCCAAAATGTGGGATCCTTTTTTTTTTTTAGATGGAGTTTCACTCTTGTTGCCCAGGCTGGAGTG
CAATGGTGTGATCTCGGCTCACTGCAACCTCTGCCTCCTGGATTCAAGCGATTCTCCTGCCTCAGCCTCCCGGGTAGCTGGGATTAC
AGGGGCCTACCACCGTGCCTGGCTAATTTTTTCATTTTTAGTAGAGATGGGGTTTCACCATGTTGGCCAGGCTGGTCTCAAACTCCC
GACCTCAGGTGATCCACCCGCCTCAGCCTCCCAAAGTGCTGGGATTACAGGCGTGAGCCACCATACCTGGCTGGGATCCTCTGAATT
GTATTTTTTTTTTTTAAGTAAAGATGGGGTTTCACCATGTTGGCCAGGCTGGTCTTGAACTCCGGACCTCAAGTGATCCACCTTGCT
CAGCCTCCCAGAATGCTGGGATTACAGGCATGAGCCACTGCACCTGCTCAGTCCCCTTAATTGTGATTTACTTTAATATAGTTTCAT
TTATAGTGGTGGCCCCTTTAATATAGTTTATCATTTCTTACAGTTGAAGAGTCCAGTGTTAACTGAACAGGCAGAAAACTACACATT
TCTCTAAATCAGCACCTTAATTTATAAAGTCTCATGTTGGAAATGAAGAGAATTGTGTTGTTTCTTGGCTTTTAAGACTTGGAAAAT
ATCTTAAATGGCCTCTCTCCATTTAGGACAAACAATAAAAACTTGCCTGGAATAATCTTCGAACAGAAGGTATGAGATAAGTAGTAA
GTAAACGTCCACAGGACTCCAAATAGCTCTAAAGATCTGATTCCCCTAGGAGACTTGCTGAGGGAAAATCTAGGAACTAAGATACTG
CAAAATGGAAATAGACCTTCTTGGTTGACCGAGGCTCTAGGGTGAAGCTGTGAGTCAGTGCTGCACCTTTGGAAATAGAGGAAAT
TAGCTCAACAGGGCTTGGGGCATGTAGGTATCTGCCAAATATTTTATATGAATTGAATTTGGACTCAATTTTTGGTATGTATAATAT
GTAACATGAAGAGAAAATTTACCAGGGGAACTGGAAAATGCACTTAACTTTTATGGAGGTGAGAACAAAAACTGAATTAAAATCTTT
CCCTGACTTGGTGTTTCCTGTGTATGCTCATGCATGCTAGCTATAATTTTGTCATTTCTGTACTGTGATAATTCATAATTCATATTT
ACATGCTTAAGGTGGGGCCTAATACAGGGCAGGGCGTTATTTACACTGTAGGGTAGGCATTGAGCAGAAGTGGTGTGATATTTTTTC
TGTTCATTTTTCTAGATTGGGGAAAGTTTAAATTGAAATTATTGGCATCACTGATGATAATTAAGAACTGTTTAAATTCCATTTCTG
GCGAATTCCTGAAGGATATATAATCAGGAACTCTAGGGAAACTGAAGGGCTGAGCTGGAGGGTCTAGAATTGGGGCCTTAACCTGG
GAGTCCAAAAACTTTGCCAAACACTTATTTCCACCTTCCACTCCTCCTCCCGTGCCCACCCTCACAGAGCAGTACTTGGCACCCTAG
ACAAATACTGCCAGATCTACAGACATCTAATGTCTGCAGATACCCACTCCCTGCCTAGCCTCCCTAATCACATAAGACTGGGACATC
TTCGAAGGCAAGGATACCCCTTGTTCTTACTCATCCTTGTTTCTGCAAAGCCCACCTACCTCAGGGCCTGACTCATTGTGGGTCATT
CAGTAGATGTTTGCTGAATGAATAAACATTGTTTGTCTTTTAATGAACTGCTGTTTCCTTTGCACCCCTCCCCAAT
ACATTTTTCTCTTGGCTGGCAGCTCCTTGTCTGCTTGCTGAAGATAGAACCGCAGACCACCTGAGCACCCCCTGCCTCCTCAGCTGC
TCACTGTTCTTCTGCATGTGGCCTTCAGGACCTCTGCCCAGCAGTTGTGGCCCATGTCACCCTCTCTGCTGTTGTCACCAGAAATTT
ACCCGCACTGTACCCTCTGGTCCTTCACAGCTCATTTCTCATAATCTATTTTAACAACAAAGTTTGAGAAGGTTAAGAATACAGGAT
GGTAGGAGGTGGAAAAAACAGTGGGCGTGAGCAGTAAGTCGATATGACTAAGCTGAGACTCTTCTGAGTCTTCTAGATGGAAGGGGA
ATTTCTCCAGAGTTACGGGCAGTTACCACGTTGCCTGCTCATGGTCTTGAGCCCCTCTTTTAAAACCCCTGTTGTCTACATTAAGCGGAA
GTCTCCCCTTGGCTGCATTTTGATGTCACTTAAAATGTTGCTGGGGCCCATGCTTCCTAGAGGAAGGCAAGCACAGCTAGCATTCCC
AGGAAAGCCCTCAACTCAGGGGTTTCATTTTGTTTTTATGATGGGAAATAACTTTCATTTCGCGGTCACTGCCCACCCCACTACTTC
CTCTTTGACCCCTTCATCTAAGATGAACACATCTGTTATTTTACCCTGTATAGCAGGTGCAAAAGAGAATTAAAATGGTGTCTGAAT
CCATAAAAGGAATATATTGCATAGAGAATCAAGGCCACAGTAACAAGTGACAGGCGATCTTAAAAATATTGGAATAATCTATCTTTCA
```

187

```
AGTTACAGAAGCTTACTAGAGATAGGGGCAGGTCATTGTTACAAGCATCTGGCAGTACATTAATCATGAGAAATTAATTAGGCCTTA
ATGTGGGTCTAATGAGAAATCCTTATTATCCATGTTTGCTTATGGGCTTTGTTGGTTAATTATCAAATGGAACACTAGAGACCCTAA
AATGTGATTATAAAATGATTTTTAAAAGTCCTTTCTTGGAGTGTAATATTCTATTAATGAATCAGCAGAAACTGAAGATTATCTGCT
TTGGAGATGTGTAACAATCCAATGTAATTACAAACCTGTTTAGAGAGGAACCATTTAGCTATATATGATCAAAATAGCCCTTATAAC
TGTTCTCTGTGTATGTGTCTTTTATCTTAGTTTTCTGTGTTCCTGGGAATTATTTTCTTCCTGGAGCTCACTGCCGGAGTTCTAGCA
TTTGTTTTCAAAGACTGGATCAAAGACCAGCTGTATTTCTTTTATAAACAACAACATCAGAGCATATCGGGGATGACATTGATTTGCAA
AACCTCATAGACTTCACCCAGGAATATGTAAGTTTAAAGTGGTTTGTTTTTCAAGTTGAAAGTTCTCTGAACGATGACCCCAGTGAAT
TTCATCCCTCCTGGTTTCTGCATCTGCCAGCTGGAAACTACCTGACTATTCTGCATGGGTGTTTGTTTGTTGTTTATTATTTTCCG
ACTTGCTCTGTGACTTACAGGATTTGTATTCTAAATGGAAATTTCCTATTTTCACTTGTTTCAACTTCTGTTAAGTTAGAGGAACAA
AGAAGAAAATGCTTGAGCAAAACGTTATAATCATGGCTGTCCTTTCCTATTTGCATTTGAGGAACATTGTTTTGCTCCGGGTTTAAA
GTTTTCTTTGGTTATTAAATATGGATTCTTTCTTATCCCTTGCCCCCTTTTTTGGTAATCCTTGATATTTCACAGCTCATAATAATC
TACTTATCAACAGAATAATTTGCCACAAAGTTCCTAAAATGTGCCTATCCAAATATTATTATTGCACATGCAGTTAAAAGCAAAAGC
TTAGAAACTCCTAAGTCCTAGTGGCTTCTAACAAGTGAAGAATCTGTGCAGTGGAATCCGGGCCACTCTCTCCTATCCCTGAGCATT
GGAGTCCAGGCCACTCCCTCCCGTCTCTGAGCATGCCTGTGAAATGGCTCTTTCAGTGCCAGCAAAGCAGCAGATGTCCCGTTTCAC
TCAGAATGCAAGGGACCAAAAGACACAGGTCTAGTGCCAAAATCTGATAAGGGACCCTGGGCTCACAGGACCTTGACCTATTTCCCC
AATAGCAAAATGGAAATAATACTAGCTACTGTCTAACTGTCTAACTGTACTGTACACTAATGAGACATCACATGTTTTTTATATTAGGC
TAAGGTGTTAGGGTCCTACAAACTTGCAGGTCTTTATATACCTGCACTGAAAAGTCGGGTAGGCTGAGCTTGGACTTGGACAGCGGT
GTCTGTGGCATTCATGTATATGGCAGGACCGGAGGGCAAGCAGAAGCCCAGTGAACGGTAGCCTTCTTGGGGGTTGGATAGTTGTCTG
CCTGTGATGAAGGGAGGCAGCAGCCATGTTGACACTCTTTATGAGGGAGGCCATCCCCTTCCTTAGGCACTCAGGACTGAGCAGGGT
TATGTGGTTTTTGTTCAGTGGCAGCAACATTTCGTCCTGGAGTAAGCTGCCCCTTAAGTGGGAAATTAAGCCCTGATTCATGTCCTCA
GCCGACTTCACTGTTTTATTAGGTCCTAATGGGTGCAGGCTCCCTAATAATCCCTTTCCTGGCATTATTCCTGACTAGTTTGCATGA
CACATTGATGGAGCTTTGTGGACACACCTCGAAATGCATGACCCAAGCCCTGGAAGGACCCATGTTGTCTTGGGATGCTGTGTGCTTT
AAACGGAAATACTACAGTTTCATGTGATTTTCTTTTTGCATCATGGAATGTAATGTTTAGAAGTAGCTGTTGGTCCGGAAGGTCTTC
AAATGGTTGAGACTTGATATTTATTTAACTCAGAGAAGTAGAGAAAGTGCATCCCAGGGAAGTGAGGAAGGGGCTTGTGTAGTAGCC
TTTGACTCCGGCCCCATTTAAGAACCACTGAATTTAACCGCTTGTTTTAATATATTTTAATTGGAAGACTGACTTTTTAAAGTTGAA
AATGTAAAACACAATTGTTTTTAAACAAACTCAGATTGATGATGAAAAATATGATGAAAAGTTAACCTCTAAAATTTTTGGTGGTA
ATGGATATATCAACTATATATCCATTGATTGTGGTGACATTTTTGTGATGTATACAGATGTCAAAATTTATCAAATTGTATACTTTA
AAATGTGCAATTTATTGTATGTCAGTTATACCTCCATTATTGCGGGATCTGGCCAGCAGCCTGCAATGCAACAGGGATCTCTCTGTT
CCCAGGCGGATCGGCAGGTCGAGAAATAATAGACACACACAAGATAGTGAAAGCTGGGTCCAGGGGGGTCACCGCCTTCTGGTCACG
CAGTGCCAACAATGCACTGGATATACCAGAATTTATTATTAAGTTTAGTGAGGGTGGGGGTAGGTTAGTGAGGGATTTAGGTCATTT
GATTATGAGGTGAGATGGTCACATGGGGATGAAGTAATTCTTTAACATAACATCTGTATGCAAAGTACAGTATACAGGGATAAGAA
TTTACAATATAGTGTGTGCTTCAGTAATTTCTAACAGAGCCTTAAAACAGAAACACAGTCTTTCCACAACCTATGATTAGCAAGATA
TTAGTCAGCAGTAACAGTTGCAGCAAAAGCCAGTTACAAACAATCCATAGAAACAGGACGTGAAGCTACACAACCAGTTAGACCAGA
AATTCTCAGAAGGGAGTATGCCTTAACCCTAAAGAGGTCTAGAAGAGCCGTGGCAAGATGAGGGCATTTATAGCCCTGTCTTATACA
TATGGACAGGTGCCCCTCATGTGTCTGTTTATAGGCTCTCCACAAAGGTCGCATTCTATTCCCAGAGCTATGAACATCTGCTTTTCT
GGGATAGGAATCTTGGTGATGTGAAACCTCCCTGACTGCAGCTCCATTCATAGGCTCTCTGCAGGGGAAGCACATCACGCACTGTT
GGCTCATTCTGGCAGTCCAACCTGGCATTGTCTTTACACAATCCTGCATGCAATTTTGTATTTCCAGTAATCAGGAGCATTTCATCT
TTTATTCCATAGCAATAGTTTCAGGGGGTCTCCCTGCGCTCCATAAAACAAAACAGATCTTTAAAAAAACTATTAGAATGTAGAGAT
CAAAACTTTGAAAAAGTAAGCTATAGTTAGCCCACCCATAACCCCAGTTGCCTCCTTCAGTCACTACCACTGTCGTTAGTGTCTTAT
TTACCCTTCTAGAATGTTCTTGTTTCTCCTTCTAGAATGACGTGCCCAAGCCAAACAGATCTTGTTCTAACTGAGCCCTGCTGTACA
GCTGCTCTGCCCTTCTTCCACCTAACACCACAGGTCTCATCAGTTGTTTAAATCACGTGCCAACACATCCCTACAAACCTTCAGCCC
TAATTAATTTGGAAGATTTTATATGGGCCATTTAGGTTCAGTTTGGCTTAAAAATAAAGTCTAGGACAGAAAAGAAGGTCAGAGCTG
GCAGAAGCTGGTGTCGTTCGGCGGGTGTGAGTGTAGGTGACTGCTTGCCTCCAAGTGGGATGTTCTAATTCTTTCCTGTTTGCTCT
AGTGGCAGTGCTGTGGGGCTTTTGGAGCTGATGATTGGAACCTAAATATTTACTTCAATTGCACAGATTCCAATGCAAGTCGAGAGC
GATGTGGCGTTCCATTCTCCTGCTGCACTAAAGATCCCGCAGTAAGTGAATGCCAGCGAACTATTGGCCTACTCTGTACATCATGCC
GAGAGTGTGCATGGGACGACGAGCAGAGTGATTTTTTGGTTGAACGTCTTCTTACCTGGTTACTCGTATTTGTAGCTCCTGTAGGGGGT
TATGGTGCTCAAATCTTCCTAGTAGGAGTAATAATTGCTGGCATTAATTTAGCAGTGTGTGCTGGTTCCTATTCTAAGCCTTTATGT
GTGTTGGCTCATTTAAGTTGGGCACATTCCTACTGGCAGATACCACCATTACCCTCCCTTTTCAGGGGAAGAAATCAAGGCACATGA
GGTGTAAGTGACTTTCCCAAGCTTGCACTGCTAGCAGGCAATGGAGTGGAATATGAGACCCAGGCAACTTGCCTGTATCATCTGTTA
GCCTTTCTTCATGGAAGGGCAGGCTCATATGAGAGCTCTCTGCATTGGTTTGGGGGTTAGTAACCCTTTTCTTTTTGGCTTAGTTCT
AGGGGAACTATTAGGAGTAGGAGGAGGAGAGTTTGAGACAAGATAGGCCTGGACTGCTGTGTAGGAAGACACAGCTGCCCAGACTCCTG
AATGTCATCATGACTATAGTCATGCAGTTTGCATTTTATTATAAAAAAGAATAAAATATAAAATCTATTTGTAAGTCACTCACTTTT
GCCACAGGTTTGATTATATTTTGTGTCAAATCGTGCCTGGTATATAGAATGACTTAAAATGCAATTCTTAAGCCAGGCATAGTAGTT
CACACCTGTAATCCCAGCACTTTGGCAGGCTGAGGTGGGAGGATCCCTTGAGCCCAGGAGTTAAAGACCAGCCTGAGCAACATAGTG
AGACCCCATCTCTACAAAAAAATTAAAAAAATTAGCCATGTGTGGTGGCACACACCCGTGGTCCCATCTACTCTGGAGGCTGAAACAG
GATCACTTGAGCCCAGGAGTTTGAGGCTGCAGTGAGCCATGATCATGCCACTGCCTCCATCCTGGTGACAGAGTGAGACCACAATT
CTTAAAAAAAAAAAAAAAGGTAACTCTCTAGTGTTAGAATGACAAACATGAGAGGTTACTCCAGCATCTCTGTGTGCTCCTAGCT
GCAGAACCCTTCTGGGACTCCTCAACTGTTCTCTGGCCCTGCAGTCTCCAGATGTACTCCCAAGGCTCGCCCTTACCCCTCAGAAG
CAGAGCCGAGTGGGTGCTGATGAGCTCAGTGGGGATGCTCCTTCTTATGGTGGAGGCTGTGGGGCTCTTAAAAGGAGCTATGGTCTT
CTAAGGACTGTGCTGCCCCGCCAGCTGCCCTTCCACCAGCTCAAACCTCCTCTCCCTTCTGAGGAGAGCCACATGGCAGCAGAGTG
CCCACTCAGAGCTTGAGGGGCTCATCCTGGTGGCCTTGCACTGGCCAGCGGGTGCCACAGTGCGCCTCCTGTCGCCAGTTCCTGT
CCCACATTAACACTCCCTTGGGAGAATTCTGTATTCTCAGAGGCATTAATGCTTCCCCTTTCACCTACAGATCCTCTGGGCAGAGCT
AGCTCATTCCTTGCGCTCTAAGCTAATAAAATCTGCCCCACACCCCGCAGAGAAGGAAAATAAACAACCCGAAAAAGTGGAACCATG
TTTCTGATCAGACCCTGTGAGACGTCAGGTTGTCTGTGTGATTATGTGGTCGGGGCATTGTTTTGTTTTTTAATTATTAAAAGCTCA
TTGAAAAACAATTTAAGTAACATGGAAGTACATAGATTAAAAAGTAGCAGTCCATCCAAAAGAGCATACATGAAGATTCACTGTGCC
CTTTATGTTTTTTATCTGTGTCTCCAGTTCCCTAAATCCCATAAGCTTCTTGAGGTTGGCACCACTTTGAATCTTGAGCCTCCTGTG
TGATAGACTTGCTGTCTTTTCTGATATTCCCCAAGCCTTAGTTTTCCTTATTTGTAAAATAGGGCATATTTTAATACCTACTTCTGA
AGATTGTCGCAATAATGACATGGGATCATGTATATAAAGTGTTTAACATAGTGCTGCAGTCTTTGTGCCCAGATACACATTTTATTA
CTAATGCGTAACAGAAGTCTTCTGCTCATTTCTAGGTGGGCTTTCTGTCTAATGAGTAAATTTCACTTTTCTCTTTCCTTTCTTCAC
CAGGAAGATGTCATCAACCACTCAGTGTGGCTATGATGCCAGGCAAAAACCAGTAAGTGGAATCTCATGTTGTTGCTTAGCACGAAGG
GTGTTGGAGGAGCTTTGGCCTGTGCCTGGTGCTCTATCTGTGACAATGTGCTCTCCTCCTTCTCTTGTGAAGGAAGTTGACCAGCAGA
TTGTAATCTACACGAAAGGCTGTGTGCCCCAGTTTGAGAAGTGGTTGCAGGACAATTTAACCATCGTTGCTGGTATTTTCATAGGCA
TTGCATTGCTGCAGGTAAGATAAGGGTCCTTTATGGATATCACAGGGAAATAATGCCCTGGGAGAGCCCTTGCCAGGATCGGAGCTT
TGCTTAAAAACCTTGATTGTCATAGTTTTTAAGCACATTGGAGGGCTGAGGAACAGACCTAAACGATTTGGTTTGTGTTTTATTTT
ATTCATTTATTTATTTATTTTATTTATTAATTTTTTTTTTGAGATGGAGTCTTGCTCTGTCGCCCAGGCTAGAGTGCAGTGGCATGAT
```

```
CTTGGCTCACTGAAAGCTCCGCCTCTTGGGTTCATGCCATTCTCCTGCCTCAGCCTCCCGAGTAGCTGGGACTACAGGTGCCCGCCA
CTACGCCCGGCTAATTTTTTTGTATTTTTAATAGAGACGGGGTTTCACCGTGTTAGCTAGGATGGTCTCGATCTCCTGACCTCGTGA
TCCGCCCATCTCGGCCTCCCAAAGTGCTGGGATTACAGGAGTGATCCACCGTGCCTGGCCTGATTTGTGTTTTATTAAAGCTCTGAG
TTTAGATGCTACTATCATGAAATACTTTAGGAAACCTTGTCTTTTTCTAAGATGGAGACATTAATTAGATAGTGGTTGCCTACTGGC
ATTCCATCGTTTACATGGGCTCAAAAATACTGCATTGTGGCAACTGAGTCAAACATGCATAGTTAGAAGTAGATCTCAGTGGTGACG
TTAAGGTTTTGGGAAGTTTCACAGAGATGTCATACCCCTTTCTCTCCCTAAACTACCTTGCCAAAGTGCTAGTGAAGGAAGGGAGCA
CCCACTTTTCCTCCTGGCCAGTCTTAAGAGCTGGACTCTTCATGTTGTTGGTGTGATACTTTCCAAAGAGCACATAGTTGAAACAAA
TTAAAGAAGACCTAAGTAAATATAAAGATATCCTGTGTTCACAATTGCAAAACTTCATATTATTAAGGTAACAACACTCATCAAGTT
GATCTACAGATTCAGTGTAATCCCTATCAAAATTGTAACCACCTTTTTAGCAAAAATGGACAAGCTGATCCTAAAATTCATATGGAA
AAGCAAGGGACCCATAATAGCCAAAACAAATTTGCAAAAGTAAAACAAAGTTGGAGAATTCACACTACCCAGTTCAAAACTTAGTAC
AAAGCTATATAGTAATCAACATAGTGTGTACTTGAGTAAGATTAGCCATATAGAGAAGTGGAATAAAATTGAAAAATCAAGAAATAA
AACCAGGCCAGGTGTGGTAGCTCATGGATGTAATCCCAGCACTTTGAGAGACCAAGGCAGGAGGATGGCTTGAGCCCAGGAGTTCAA
GACCAGCCTGAGCAACATAGCAAGTCCTTGTCTCTACAAAAAGTAAAAATAAAAAAATTAGCCAAATGTAATGGCACATGCCTGTGG
TCCTAGCTACTCAGCAGGCTGAAGTGGGAGGATTGCTTGAGCCCAGAAGGTTGAAGCTGCAGTGAGTCGTGATTATGCAACTGCACT
CTAACCTGGGTGACAGAGCAAGACCTTGTCTCAAAAAAATAAGAAAAAAATATATATGATTAAAAAGAAGAAATAAGGCCGGGTGCA
GTGGCTCATGCCTGTAATTCCAGAACTTTGGGAGGCTGAGTCAGGTGGATCACTTGAGCTCAGGAGTTTCAAAACAGCCTGGGCAAC
ATGACAAAACCCTGTCTCTACAAAATATACAAAAGTTAGCTGGGCATGATGGCGCATGCCTGTGGTCCCAGCTACTTGGGGGGCTGA
GGAGGGAGGATCACTTGAGCCTGGGAGGTTGCAGTGAGCTGAGATCATGCCATTGCACTCCAGCCTGGGTGACAGAGCAAGATCCTA
TCTCAAAAAAGAAGATGAAATAAGCTCGTACGTATATGGTCAGTTGATTTTTGACAAGGGAACCAAAACCATTCAATAGGGAAAGAG
CAGTCATTTCAACAAATGGTACTGGGAAAACTAGATATGCAAAATAATGAATTTAGACTAACTCACACCATATGTAAAAATTAACTC
AAAATAAAGCAAAGACCTAAATATAAGAGCTAATGCTATAGAACTCCAGAAGAAAACATAGGCATAAATATTTGTGACTTTGGATTA
AGCAGTGTCTTCTTAGATGACATCAAAAGCATAAGCAATGAAACAAATGTAAATAAATTGGACTTCAAAATTTAAAACGTGTGCTG
AAAATGATACCATCAAGACAGTGAAAAGATAACTCCTAGTATCTAGAAAAATAAAGAACTTTAAAAAGTCAACAATAAAAAGACAAA·
CAACTTAAAAATGGGCAGCCAGGCATGGTGACTTGTCCCTGTAATCCCAGCTGCTTGGGAAGCAGAGGCAGGAGCCTGCACTGAGCT
ATGATCACGCCACTGTACTCCAGCCTGGGCAACAGAGCAAAACCCAGTCCCTTAAATTATTAAAAAACAAACAAACAAACAAAAATA
CGGGCAGAGGATCTGAACAGACATTTCTCTAAAGATTAAAATTGCTAATAAAGGCCGAGGCGGGTGGATCATGAGGTCAGGAGATCG
AGACCATCTCTGGCTAACAAGGTGAAACCCCGTCTCTACTAAAAATACAAAAAATTAGCCGGGCGCGGTGGCGGGCGCCTGTAGTCCC
AGCTACTCGGGAGGCTGAGGCAGGAGAATGGCGTGAACCCGGGAAGCGGAGCTTGCAGTGAGCCGAGATTGCGCCACTGCAGTCCGC
AGTCCGGCCTGGGCGACAGAGCGAGACTCCGTCTCAAAAAAAAAAAAAAAAAATGGCTAATAAGCACATGAAAAGATGCCAACGTCA
GTAGTCATTAAGGAAATGCAATTCAAAGCCACATATTTCATACTCGTTAAAGCGGTTAGAATAAAAAAGACAGACAATAACAAGTAT
CGGCAAGGATGTGGAGAAAACGAAACCCTCATATGTTGCTAATGGAAATGAAAATGTTGCAACCTCTTTGGGAAACAATTTAGCAGT
TTCTCAAAAGTTAAAATATAGTGCTACCATATGAGTCAGCAATTCTACTCCTAGAGGAGTACCCAAAAGAATTGAGAAAGTGTTTACC
CAAAAACTTGTATGCTCATGTTCATAGCAGAATTATTCATAATTGCCAAAATGTGAGAGCAATGCAAATACACATCAACTGAGGAGT
AGATAAACAAAATGGGGCATATCCATGCCATGGAATATCATTCAACCATCAAAAGGAATAAATTAATAGCACATGTATATGAACCTT
GAAAATATTTTGCTACATGAAAAAGACAGCCACAAAAGCCCCATATTTATATGATTCCCTTGATATGAAATGTCCAGAAGAGGCAAAT
CCACAGAGGCAGGAGTAGATTAGTGGTTGCTAGGTCTGTAGGGATGGTGGGATGGGAATGACTGCTCACAGGTACAGGGTTTCTTT
TTGGGGTAATGAAATGTTCTGTGATTAGATAGTGGTGATAGTTGCATAGCTTTGTGAATATACTAAAAGCCCCTGAATTATACACAT
TTCAAGGATGCTTTTTACCACATATGAATTACATCTTAAATTTCAAAAATCAATGATTTAAAAAAATGGGACACAGTGGCCAGGGAA
ACAAGTTACAAGTGTCACTGTTTCACTCACATTTTTCTTCCTTTCTCAGATATTTGGGATATGCCTGGCCCAGAATTTGGTTAGCGAT
ATCGAAGCTGTCAGGGCGAGCTGGTAGACCCCCTGCAACCGCTGCTGCAAGACACTGGACAGACCCAGCTTTCGGGACCCTCCCGCG
TGCCGAACTGATCTTCGAGCTGCATGGACCTAATCACAGATGCAGCCTGCAGTCTCGCCTAATGGAGCTGCCATTAGGGGAGTGTAA
AACTGGGAAATGCTGCTCACTGACAGAATTAAAAAAAAAAAATAACCAGTATGAAAGTCGTTGCACCGTGAATCTCTACTGTAGCCAT
GAATTTATGGACAGTTAGATGCTTACCAAAAAAGAAAAAAAGGGAGGGTAGGGGACCCAGATGTACTTGAATGTGCAGAAAATACAT
TCTTGTCCTCATCTTCCGTAATTGGAGGGCTGGGAGAGGCAGCTTTGCTCTTCACCACACCTTGGACGGACCACCTTCTTTCTGTTC
CATGGCCTGAAGGAGTGCATCTCCTCAAAGACTCAGCCCCTCACCTGGGAGGGCAGTGGTTTGTGGGCATCCCTCCATGTACATTTT
AGGAAACACTTGCAACTCTCATCTGAAGAAGAAAACAACTCATCTTTGGGTTCAGATTTTGTGATGGTATTCAGCAAGTCACTTGGG
CGAGCACACTTGGTCTATCCTGGAAAGTCTCCTTATAAGAGAAGTTGTGTATTTCATGTGCACCGAGCAAGGGCATTGGAAGACGTC
ATGAGGCTGTATTTTAGCAGGACTGATCGTTTTTCTAAGTAGACCTGAGCTTTGTTTATCAGTGAAATTCAAGGAGAAAATGAGGTT
AATGAAGAGGTATCAGTTAAATATCCCCTTCTTCTCACCCTGCCAAAATTAGCAGTTGGATTTTTGGAAACTCTGGAATATTCTGGG
TCATTTTGTTTTGTATGTTTGTTGTTTTTCGTCTTCCAAAGGTGAAAGCTATGATACAGTTCCACTTAAATTTTAGTGTTTTCTTAC
TCAGCTCAAGCATTAATTTTTGATTAAGTCTTAATCTGCATGACCTGTGAAATCTGAATCCATCATCTCCCTTTCCTGCCAGCTTTTC
TACAAACATTGAAATATGTTATTTGGTCAGCACTTTATTTCCTAGGTTCACAGCCTTGGGAGGTTGTGCATGTCCTCCCAGTCTGGC
TGGGAAGAGACCAGCTGTACCATCCAAATGCTTCCCTGGTCTTGATGATCTCTTCCAGAGTCGATCTGAGTGGCCTTTTCTGCACCC
TCCCCTTCTTTCTCTTTGAATGGAATTAAACCCAATTTGGAAACAACATTGACCCAGTCAAAAGCTTCTAATGGTTTCTTTTTCTTC
CTCCAGTTTTAGTTTGCTTTTATTAAAAAAAGAAAATAGTGCATGGCCATAGCTCCTTCAGTTCTCTTATTGCAGACTAACCATCAG
GATGGTATCAAAGCACAAATACTTTGGAGGGGAATGCGTTGAACTGGGGCAAGTACTCTGTAACACAAAGTGGGAAACCACTTCCTG
GTGCTGCCGCTCCTGCCCCCACTTTAGGTGGGAGGGACGAGTTTTGCCCTCTAGATTTTAATCCAGCTGGTGTCCACCGGATGTTGC
CCTCCTGGGGAGCAGATATCAGTCTGTGGAACTCTGGGAAAACCACAGGCACATTTTTCGGTGCGGACAGATTTGCCAGCACATAAC
TGGGCAGCCAGCTAGAATACTTTGTGGAAATTAAGCGAGGTTTTCCATTTCAGCCCCATGGTGCATGGTGGTGGCCGATGAATGTGT
CAGTCTGCTCAGAGAAAGGACAAAAAGGAAATTATTTTCAAAACTGTGTTCACTGTTTGGGTGTGTGTATGGCTCTGCATGTGTGTG
TTTTTGTCTCTGTATAGGTAGAGGTATTCACATCTTACTCCGACTGTAAGGTTGTCTTTACTTCATCTCTGCCCCCACCACAGTTGCC
ATTTTGTAATGTCCTTCCAACATGGAGAAGACACGAGCTCTCTCCAGTTGGCATCATTTGTCTTTTTTGTTGATTGCCTCATTCTCC
AGTGAACTCCATCTGGCCAATTGATTCAGAATCAGGCAAGATCCCTGCCCTTTGGCACATCCACTGAAAGGCCAAACAGCAAGTCCG
AGTGAGTTTTAAATATTAATTAATCACCCTTTATTTTTTACACTTGAGAGTGATTGTAATAAAGGCTGTCATTAATAAACTTGGTTC
TACCT

HUMAN mRNA SEQUENCE : hR28-002.1 (Seq ID No: 53)
CGCACGGACGCGGGCGCTCGCTTTGTGTTCGGGGCTAGCGTCGGCGAGGCTTGAGCTTGCAGCGCGCGGCTTCCCTGCTTTCTCGCG
GCCACCCCGGCTCCGGCGGCCTCGGCGCGCGAGGGGCTGGAGGTGCGGGAGCCGCTCTCCGCCGGTCGGTCCCCGCGCGGCTGAGCC
CAGGCCGCCAGCGCCGCGGCCCCGTGCGGTGTCCCTGAGCTCCTGCTCCCCGCCGGGCTGCTCCGAGCAACGGTGCTTCGGAGCTCC
AAACTCGGGCTGCCGGGGCAAGTGCTTCTTCATGAACCCAGAGGATGTCCGGGAAGCACTACAAGGGTCCTGAAGTCAGTTGTTGCATC
AAATACTTCATATTTGGCTTCAATGTCTCATATTTTGGTTTTTGGGAATAACATTTCTTGGAATTGGACTGTGGGCATGGAATGAAAAA
GGAGTTCTGTCCAACATCTCTTCCATCACCGATCTCGGCGGCTTTGACCCAGTTTGGCTCTTCCTTGTGGTGGGAGGAGTGGATGTTC
ATTTTTGGGATTTGCAGGGTGCATTGGAGCGCTACGGGAAAAACACTTTCCTTCTCAAGTTTTTTTCTGTGTTCCTGGGAATTATTTC
TTCCTGGAGCTCACTGCCGGAGTTCTAGCATTTGTGTTTCAAAGACTGGATCAAAGACCAGCTGTATTTCTTTATAAACAACAACATC
AGAGCATATCGGGATGACATTGATTTGCAAAACCTCATAGACTTCACCCAGGAATATTGGCAGTGCTGTGGGGCTTTTGGAGCTGAT
```

```
GATTGGAACCTAAATATTTACTTCAATTGCACAGATTCCAATGCAAGTCGAGAGCGATGTGGCGTTCCATTCTCCTGCTGCACTAAA
GATCCCGCAGAAGATGTCATCAACACTCAGTGTGGCTATGATGCCAGGCAAAAACCAGAAGTTGACCAGCAGATTGTAATCTACACG
AAAGGCTGTGTGCCCCAGTTTGAGAAGTGGTTGCAGGACAATTTAACCATCGTTGCTGGTATTTTCATAGGCATTGCATTGCTGCAG
ATATTTGGGATATGCCTGGCCCAGAATTTGGTTAGCGATATCGAAGCTGTCAGGGCGAGCTGGTAGACCCCCTGCAACCGCTGCTGC
AAGACACTGGACAGACCCCAGCTTTCGGGACCCTCCCGCGTGCCGAACTGATCTTCGAGCTGCATGGACCTAATCACAGATGCAGCCT
GCAGTCTCGCCTAATGGAGCTGCCATTAGGGGAGTGTAAAACTGGGAAATGCTGCTCACTGACAGAATTAAAAAAAAAAATAACCAG
TATGAAAGTCGTTGCGCCGTGAATCTCTACTGTAGCCATGAATTTATGGACAGTTAGATGCTTACCAAAAAAGAAAAAAAGGGAGGG
TAGGGGACCCAGATGTACTTGAATGTGCAGAAAATACATTCTTGTCCTCATCTTCCGTAATTGGAGGGCTGGGAGAGGCAGCTTTGC
TCTTCACCACACCTTGGACGGACCACCTTCTTTCTGTTCCATGGCCTGAAGGAGTGCATCTCCTCAAAGACTCAGCCCCTCACCTGG
GAGGGCAGTGGTTTGTGGGCATCCCTCCATGTACATTTTAGGAAACACTTGCAACTCTCATCTGAAGAAGAAAACAACTCATCTTTG
GGTTCAGATTTTGTGATGGTATTCAGCAAGTCACTTGGGCGAGCACACTTGGTCTATCCTGGAAAGTCTCCTTATAAGAGAAGTTGT
GTATTTCATGTGCACCGAGCAAGGGCATTGGAAGACGTCATGAGGCTGTATTTTAGCAGGACTGATCGTTTTTCTAAGTAGACCTGA
GCTTTGTTTATCAGTGAAATTCAAGGAGAAAATGAGGTTAATGAAGAGGTATCAGTTAAATATCCCCTTCTTCTCACCCTGCCAAAA
TTAGCAGTTGGATTTTTGGAAACTCTGGAATATTCTGGGTCATTTTGTTTTGTATGTTTGTTGTTTTTCGTCTTCCAAAGGTGAAAG
CTATGATACAGTTCCACTTAAATTTTAGTGTTTTCTTACTCAGCTCAAGCATTAATTTTTGATTAAGTCTTAATCTGCATGACCTGT
GAATCTGAATCCATCATCTCCCTTTCCTGCCAGCGTTTTCTACAAACATTGAAATATGTTATTTGGTCAGCACTTATTTCCTAGGTTC
ACAGCCTTGGGAGGTTGTGGCATGTCCTCCCAGTCTGGCTGGGAAGAGACCAGCTGTACCATCCAAATGCTTCCCTGGTCTTGATGA
TCTCTTCCAGAGTCGATCTGAGTGGCCTTTTCTGCACCCTCCCCTTCTTTCTCTTTGAATGGAATTAAACCCAATTTGGAAACAACA
TTGACCCAGTCAAAAGCTTCTAATGGTTTCTTTTTCTTCCTCCAGTTTTAGTTTGCTTTTATTAAAAAAAGAAAATAGTGCATGGCC
ATAGCTCCTTCAGTTCTCTTATTGCAGACTAACCATCAGGATGGTATCAAAGCACAAATACTTTGGAGGGGAATGCGTTGAACTGGG
GCAAGTACTCTGTAACACAAAGTGGGAAACCACTTCCTGGTGCTGCCGCTCCTGCCCCCACTTTAGGTGGGAGGGACGAGTTTTGCC
CTCTAGATTTTAATCCAGCTGGTGTCCACCGGATGTTGCCCTCCTGGGGAGCAGATATCAGTCTGTGGAACTCTGGGAAAACCACAG
GCACATTTTTCGGTGCGGACAGATTTGCCAGCACATAACTGGGCAGCCAGCTAGAATACTTTGTGGAAATTAAGCGAGGTTTTCCAT
TTCAGCCCCATGGTGCATGGTGGTGGCCGATGAATGTGTCAGTCTGCTCAGAGAAAGGACAAAAAGGAAATTATTTTCAAAACTGTG
TTCACTGTTTGGGTGTGTGTATGGCTCTGCATGTGTGTGTTTTTGTCTCTGTATAGGTAGAGGTATTCACATCTTACTCCGACTGTA
AGGTTGTCTTACTTCATCTCTGCCCCCACCACAGTTGCCATTTTGTAATGTCCTTCCAACATGGAGAAGACACGAGCTCTCTCCAGT
TGGCATCATTTGTCTTTTTTGTTGATTGCCTCATTCTCCAGTGAACTCCATCTGGCCAATTGATTCAGAATCAGGCAAGATCCCTGC
CCTTTGGCACTCCACTGAAGACCCAAACAGCAAGCAAGTCCGAGTGAGTTTTAAATATTAATTAATCACCCTTTATTTTTTACACTTGAG
AGTGATTGTAATAAAGGCTGTCATTAATAAACTTGGTTCTACCT

HUMAN PROTEIN SEQUENCE : hP28-002.1 (Seq ID No: 54)

              MSGKHYKGPEVSCCIKYFIFGFNVIFWFLGITFLGIGLWAWNEKGVLSNISSITDLGGFDPVWLFLVVGGVM

FILGFAGCIGALRENTFLLKFFSVFLGIIFFLELTAGVLAFVFKDWIKDQLYFFINNNIRAYRDDIDLQNLIDFTQEYWQCCGAFGA

DDWNLNIYFNCTDSNASRERCGVPFSCCTKDPAEDVINTQCGYDARQKPEVDQQIVIYTKGCVPQFEKWLQDNLTIVAGIFIGIALL

QIFGICLAQNLVSDIEAVRASW*
```

## Table 6

```
MOUSE GENOMIC SEQUENCE : mD28-010 (Seq ID No: 55)
GCATTTCTTACTTACTGAAAGGTAAGGGTGAATTGTCCTCAGTATTCTTCCAGAAGTTTGTAAGAATGATAACTTAGATGATGGGTG
GATGGCTTAAGAAGGGCTGATAAATGTATCCCTTGAGGCCAAGTGTGTACAGCTACAAAAGGTGCTTGTCCCTGGTGTCCTTAGCTT
TTGGGGAGGACACAGTTCCTTTCTCTAATAGTTATTTCTCATGAGTCTGTATTTCAGACACTCACTGGATGAAAGTGGCTTTTGGTC
GAACTGAACTTGGAGTGGAACTAAGGGAACATTGGGTTCTCTGAGGTGTAAATGAGTGTGAGCACCTGTCATTAGGGATCAGCATCT
AAAACTGGTCTGAGAACATTTCCAGAACTATGTTGTAGGAATTGGAGTTAGAATCCTAGTCTCATAAGTTGTGGTGTCTCTCACATG
TCTTTAGAGATCATTCAGTCTACTTTTTGGAGGAGACAAATCCAGTGTTGGATTCTTAACACTGTCTGGAACGTGGAAGGGTTGGCA
CAGAGGAGAAAGGAGACTTTTAAAGAAAGCACTAGAAGAAGAGAGCGAAAGGGAGACCAGTCCCACCTTGCAGGTGGGAAAGGGAAGTT
GCTGTGGGGGTGGGGTGGGGTGCTTCTGGTTCCCTTCATAGCAGATGAACTGTCTGCTAATGAGGGATTCTCAAGATATCATATGCG
CACTGGGAATCTTCATAGTTTTGGCCATTGCCAGTGTGTTTTGATTACAGGTGACTTTATAGAAATGACACAGATGCTCCCTGTAGT
AGGGTGTACCAAGCAAACTGTTTGGGCAATACTTTCTTTCATTTTCTATAAAAATGTAAGTAGCAAGTAAGGCATTGTTTTAAAA
GAAGAAAGTGCACACTGGGATAACAAGAAAAACAGTCAACATTGAGGTGTTCTAAAGAAATAAAAGGACTCCTTTTCTGGAGAGTCT
ACAGTTAACTGGGGGACATGCATTTTATGAGTCTTTGGGGTTGGTCAGTTGGGTTGGGCCCATTTCATCATTGATTGGCTAGAGCAC
ATGAGGCTGTGCACTTATGTTCTCTGAACCTTAATCTTTTTCATTTAGATAACAGATAATCTGCCCAAGTGGGTGACCATGAGGAAC
AAATGCATAGGTAAAGTGCTTGCTGTAATGGTAGCCCCTCAGTGAAGGTTGGCTTTCTTTTTCAGGAGAAAGTCCAGATACTGAGTC
AGGTCAAGCTGAGGTGTTACTGTTTGCTCTGTAAGAAAACAAAAACAAAAACAAAAACACCCGCCTTGTGCTTATGATTCTCCTGCA
TGTTAGGATTATATGTGTGTACCACCACAGCCTTCTAGAAAGATCATTNNNNNNNNNNNNNNNNNNNNNNNNCATCCCCCCAAGCTATGTT
AAGAACATGGGATTGGACATGATAATAAGAATGATAAGATGATTGGGTTCTGATGAATAATATTCAAAAGTCAAAAGTCCCCCAGAC
AGTTTCCATATAAGTCTTCAGGTTTGAAGATCTGAGGGAAGATCTGAGCTCTGAGGGAAGTCATTGCTGTTGAACATAAATACTGCA
TTTGCAGTATTTTCTTCTTACCAAGGCCAAAGTTTGTCCTGTGTCCTACTGTCAAAGGCAGGATCCAGAAAGAGAACCTGCTACACT
TTTGTGTTGGGAGAGGACTCTGGGAAGCAACCCTGTGCATCCTTTTGTTCCTGTCTCAGAAGGTCCACGAATAAATGCACATATGGG
TGTGGAAACACTTAGTCATTTGTTGTCATAAAATTGTGTCATTCACGAAACTTCTGGTGTTTAACTTAGAATGGGTCTAGCCTTTCT
GTGGGCCAACTCATTGATTCTTAAACAATGGAAGATATGATGAATTTTCTTACGTGTCTGACCCATCTGTCATTTGTAAGTTGTGATA
ATGAACATGAATATTTACTTCAATTTGAGGCATATTAACTTACTACAATAAGACAGTCACCCAGTGACTCAATTATTAATTGTATTA
AAATATATATTTTCATGTACATAACACTTAACAGATTAAGCTATATTTCCTAGTTGCATAGGTGGTAAAGAAAACCTTAACTTTTAA
AAAAAAATTCTCTAATAATGACAATATGAATTTATCTAAGATATCTAGATTCGATACTTTCTTACCATATTTAAAAGGTTATCACTTG
TTGGTATGATTAAACCCAGGGCCACAATCATGGTAGGCAAGTGCTACATGCAGCTCTCGGAGTGAAGAGAATCAGAACTCTAGGTCTG
TGTTTTGGAAGATGTTTAAAGATGAACATGTTCCTCACATGCATGCTGGCTACACTGTGCTTCCGTCTTTGATCTCTGATAGAATTC
AGTCAGTAAAGCTTTTTCCTGGGCTTAGTTTCCTGCCTTGTATGATAAGGGGATCCACACCACAGGATTGTTGAGTGTAAATGAATA
GATTTTCTAAGGTGCTTAGCTCTAGCAAATGTTTAGTAAATGCAAGCTTGGAAAGACATTCGGTTCATTATACACCTTTAAGGGATA
AGGTGCCCTTTATTTGGTAGAATTAAGGTAAGTTCATTTTTGCGTCCTGTCCTCTGATGCATAGTCATCATCAAGGTTTAATTACAG
GAAGGAGAACGTTAACTTCTTGTTCTTCAGCAAGATTGTGATTACAATACTGCTTCAAAATGACCAGTGTCATAGCAAATGGTGACT
```

```
TTTTTTTTTTTTTTTTTTTTTTTTTTTTTACATCCTTCAATGTCTCAGTCCTGTGGTATAGAATGGAATTTAGATTGATCTCCTATTGAATTT
CTGGGGTTAGCAAGTGTGATCATATTATCTCCATGGTATGTTTAACTTTACATCTTCATGTACATGAAGAAATACTACTCCATTCCC
AAAGAGACTGTCACAAAATTTTTAATCCACCTTTTATGAAAAACAGCAATCCTTATCCTGCTCTTTAGGTCAGAGAGGACCCTCATG
GTAGTGGTGTGCTATATTTGGAGTCCTCCTGCATCTCTCCCAGGGCACCTGGGCTGTGAGTCTGCTCACATCAGGGAATGCAGACCT
AAATCCTGTCTCCTGGGGTAAGAAAGGTGAGAAGCTGCTGCTATGCCCATATCAAAGCCCCCAGCCCTCTGAGGACTTGGGGCATTT
GTCCTTTTGGGGCTAGAATATTAGATACTTTATACATCAGTTACTTTTCTGTGATAATATATCATGGCCAAGACAGCTCAGAAGGCA
GGGTTTATTTGGGTTTATGGTTTCAGAGGGAGGGTCCATAGTGTGTGTTGGGGGGGGGCACATCTTCAAATGCAACCATGAGCCAGAG
AGTAAACCGGAAGTAGGGCAAGGCTGTGAACCCGTAAATCCCACTCTGGTGTGCTTCCTCCATCAAGGTTGTATCACCTTCCCAAAT
TATACACCAACTGGGGATCAAATGTTTGGATACCCAGAGCTCTGGGTTTCTCCTTCAAATCACTACAGCTAGCCATCCTGTGAGCCT
CTGAGTGGAGGGCATTCCTTCCCAGTCTGCCCAATCCTTGTCCAGTGTGTGGGAGTGCTAGGTAGAATGAAAGCCAACCAGGAAAGA
ACATGACAGTGGTTCTTAAAAGATTCCCAAGAGCTCCTTCTTTTTCTTTAAAAAAGATAATTATGTTGATAATTGCTATTGCAATTA
ATTATACAAAGCTAAATAACTTTTAAGTTATCCAGGGGAACTATGTATCCTCTTGTGAATTCATGTATCCCATCATGAACGTACTGAT
AAAATAAAACCCTTCAATTTTGGATGCATAAATCCAGATGCTCGTATGCTTTTCCTCTGCACCAAGAAGCTTATATTTTTGGTGGGG
GGCCTGGGGTAGATGTGTGGCTGTCGGTTCCCTTTCCTTCCTCTTCTTTTCTTTTCCTTAGCTTTAAGAACACCAGACTGCTTCTCA
CCTCAGGGGGAGACATCAGTCTGCAGTGTGAGGAAGCAGACCTCATTTTCTGCCTGGGTGCTTGTTTCTAGCTTATTAAGAGCTGCT
TTAATTGGGTCCCTAATTTGTCAGGAGTACAAATTGGTCAGCTCCTACTTAAGCAATGTAATTGGTGCCAGTGATTTGTGAGCCAGA
GCAGAAGGATGGGCTTGGGTGACCTCGGCATGGCTTGGTGAATAATGGTGTGAGCCAATTTTTAATCTGATCTTTATTCATATCTGG
CCTTGCCTTAGAATTTATGCTGCTCTTAGCAATATACAATGCCGCTGTAAAATGAATGACACACAGACTATGAGGCACTGCTTAGTC
CTGGTGACATTGTGAGTCCAGTCACATCCTTTCTTGCCCACACTACCCTGTAATAAACTAATTTTGTGTCCTTCTTCTCCACCTCTC
GTGCTTTGTGCATGGCTCATTCCTTGGTGTTGAATGAGTTATTTCAAAGCCCATTGGTGTCCCTCTTATTTCCATATATATATATAT
ATATATATGTGTGTGTGTATGTATGTAGAGACGCACAGGCCTGGGAAGGAACATGGCTATGAGACCTTGATTGGCCTCGTCTCTTAAC
TTCTCTGACTTTCGCTTTTTGATATTTCCATCTCAAATGCATGTGCTGTTTGCTTCACAGAGCTCCTCTGGGGGGGGGGGGGGAATCAT
AAGATAATTCAATGAAAGGCTCAGCACTGTGTGGCTTGTTTCCAGTAAGTGCCGCTTGACTGCCTTTGTGCTGTGATCCTGTACAGT
CTGCTGGGACATCTGTGACTTTCAGGCTATGGTGGACTGAGTGAGGGCACCTTCCCAAGAGAGTTCCACATTTGAGGTTAAGGTCCT
GATAAGCCACCTGCTGGGTAAGGTGACTGAGGTCTTCTCTTCAAAACTCATTAAGAGCTCAGAGCCTTTGATCTTTGTCACTACAGG
TAGCTTGGCCTTTCAGATAGTCACTATAGGAACAGCCAGAATTGCTTCTTTGTACCTGAGGGAGATGCTGACAAAAAGCAAAATTTCT
AAGCAGATGGTTGGGTCAACCCTTCCAGGAATCTCAGAGGAGAGAGTATGGAGTTCTCTGAAATGGTCAACTGAGACCTGTCCAGGT
AGTGTGTCTCCAAGTTGTCCTGTGACGCAGGTGTCCGGTGGCTTTAGTCAGCTTAAGTTTTAAACAGTGGTTTCCAGTCAGAGTCCTCA
TGACCCGTGGCCAGATACTTACCTAGTGAGTCATGTTTCCATGAAGAGGAAGTGAAAGCAAGACCTGTCTGAGAGGGGCATAACTGT
ACCTAGCATTGGTGAAGTTTGCTCTGAATCAAGGCTTTGCCTTGCTGTTACAGTTGTGTGTGTGAGTGGGGGGTGGGGGGGGGTGGGG
TGCTGTCAGAAAGTGGGCTGAAAGGAGACATCCAGAACAATACTTTAAGTTCTGACTGAGAAAGTGCAAATTGTGGCTAGTACATGC
TGTCTGATATTAACGTCACTCTCAAAGATGGATACTCTTCGCTGCCTTTTGCTTTAAGGAGAGTGACCAGGCCCCTAAGTCAACACA
GAAGCCTGGCCCCAAACCATCAATCTTCAGGGTCCATGCTTCTTACAACTTCATAGTGTGGAAGATTGCCCTCAAACAAGTCATGGT
ACCCTTTCAGAAGATGTGTCAAGTGGTTATTGTTATTTCCCACAGGAAGCCAGCCTATGAGTGAAAAGACCATAGGCACATGACAAA
GAATTGGTGAGACCATGGAGTTCTCTGAAATGGTCAACTGAGACCTGTCCAGGTAGTATCTCCAAGTTGTCCTGTGACACAGGTGTC
CGGTGGCTTTAGTCAGCTTAAGTTTTAAACAGTGGTTTCTGGTCAGAGACCTCCTGACCCAGATACTCACCTAGTAAGTCATGTTTC
CATGAAGAGGAAGTGAAAGCAAGTTTTGATTACTCTGTGTTTGAACCCCTGACATAGACCTTCATTTTGTTAATGTTGATTTCTCTG
TCATTTTAACTTTTAATTCTTTTTTAATTAGATATTTTCTTTATTTGCATTTCAAATGTTATCCCTTTCCTAGTTTCTCCTCCAAAA
ATCCCCTATCCCCTCCCCTGCTCTCCAACCTACCCACTCCTGCTTCCTGGCCCTGACATATTCCAACTGTTTCTCAATCATTTATT
GGAGCAGGACTCTTACTGCATTTAACAGTGAAATAATCCCTTCCATCAATTAAAAGAAAGAATCAAACTTTCAAGATTAGGCTTTAA
ATCTCAAATTAGTGAATAGTCATTTTATGAGAGTAATATCTATTCTGAAAGCTTATAGATGCGAACCAATTACTCGGCTTTCCATCC
ATATTAGATGATGGTGGATGTAGAGCTAACAAGCATCTGTTGGATCTTAATACACGTGAGCTATGGTATGGCCGATTCTGGGGAGGT
TAGAAAGTAACGAGGCCTCTATTCCACAGGGGTTTTTAGTTTTTTAGTAGAGACAGTAACTCTGATAAAGATAAGTAAATAAGTAAGTAA
AGGATAAAAAATAAAAGATAAATAATAAAAATTAGGAAGTTCAAAAGGAAGAGGCTCTGTTTGGAGATCAAGGATGACTTCCTGCAGG
AGGCTGGGACAGGGTCTAGCTCTGCAGTGGCAGACCTGTGCTTCTGTTTGGGAGCTGAGTTCCTGTCTGTTTAATGTAAGAAGTTTC
TAAGTAACTGGGTGAGCTGATGGGTGGAAAGCAGAAGCAGTTGTGAGAGAAGAGCTCCCAGCAAGGCTTTGTTAAACAAATACCTGC
TGCTGTGCCCCAGGCCTGTGGCAGCTGGGGGAGAGGCCACTAGTACAAGACACAGATCCAGTGATCTGGGCAAAGGGCACAGTGGCCG
GGTGTGGGGAGGGAGTGGCAGATAAGCAAACCTAAGAGAGTTGGCAGAGCTTGTGAAGATCCTGGATGCTGAGGAATTTGGGGAGTG
GGACCACCAACGGCTCCGTGCTGCTCTTTTATTTGGAATAAGAAACTATAGGCTGATAACTGATCATGTGGATTTCGCTCGTTAGAA
AGGTAGTTAAGCCTAAGCATAGACATTCCTCTGTGCACTTGAGGATCCTGCAGTGTCCAGGACATCTACTGATACCGGCTCTGTGCT
GCTCAAGGCCCTTAGGTAAAAGGGTATGAACCGCATGCACCCACTCATCTCCTCATGCATGCTCTGAACCAGCTATAGATTACATAG
CTGCATGATTGGTTTTAATTTCAAAGTAATTAAGATGTAAGGTTAGCTCCGGCTTCCTCCTTCTCCCTGTGCGTTTCCTCCCC
CGCTTTCCCATTCTGTGTATGACTTTGCTCTATCGCCCACATCCCTCAGGATATCTTTCTTCCCCTCATAATTGTCTTTTCAATTTC
CTGACTTGTGAACACAGATACAGAAACACGTACGTATATATTGAATTTTGAGGCTATGTTCAGCATAGAAAAGCAAACATGCAGCAT
TTGTCTTCTAAGCCCTCTGTACTTGGTTTTGCATAGTGATCTCTGGGCATCCTTTCATGGTAATCTTACCTATCGTTCAGTACCACA
CCATCCTCCTGAGCTGTCTCACCAGCTTAAATACCTAGTTCATTGTAAATGCCACAGGAATCAATATCATCTTTTCTTGTTTGGGGA
ATGATAAGAAATAAATGCCCCATGTGTTTAGTGCTGACAATCCTCCTGTCCTTATCCCTCATCACACACACACACACACACACACACA
CACACACACACACACACACACAGTGTGTGTGTGTGTATATATATATATATATATATATATTCAGTGGCTTGATGTAGCTACAGATTA
GATTTTGTGGCTACATGGGGCTGCTCGTATTTCCTCTCACAATATTCTAGAATATTCGATGGCTTGTAACTTGTTTCAGTGTTTTCC
CAGAGCAAATGAGCGGTGCTCAATAGTAGAACACATGTCTCATTTGTATTTAATTATAATTAGTTTAATCTAAAATTATATTTTACA
GGTCTGCCAAATTTATTCTGTTATTATAAAAACTGAATTTTAAGCAGCCAAGGAATTAATGAATCTAGGGCTTTAATTCTAGCATTT
CCTTAAACCAGCCATGGATGATTGGAGTGAGCTGTTCAATTTAGATGTCATATGTGTGAGAAGTGTCCTCCTTGTCCTTCTACCTATGTGG
CTATGACTGTATCCTGCTACCGTCACATCACATAGACATAAAAGCTGAAAGAAGCTTTGAAGTTTTGAGTCCATCTGCCTTGTGAG
ATGTCTGACCATGCCACCTCTTAGCCAAGGGCCTTTAGGCAATTTGCTCAACTTTGAATCTAACTTCCTTATCTGTGACACCGCAGT
GACAATTCCCTATGACTCCTTATTACATGTTTATTATTTAGTAATCACTAGGCATTTTTTCAAATAAGGACACTGGGGTGTGGAGCA
ACGAAATTCTTCACTTATGGTTGAAACAGGGCCGGCTGGGCTTCCTGGCTCTGTGGCACTGTCTGCTCTGCTCTGTGGTGGCACTTT
AAGGGCTTTTCAACCAGGGGGTCTGAAGGTGACACATGCTGTTTGTCTCCCAGGGTTTTTGTGAGGCTAAAGTGAATATCACGTA
CTTCTTATTAAGGGCATTTAAAAGTATTAAAGTTTTAATTATTTTTCTATGCTATTGACATTAGTGATACATATTCAATGTAGAGAA
CTTTAAACAAATGAAAGAATGAACTAAGGCGGGGCCCTCCATAATCCCTAAGGATAATAATAAACTGGCTTGGAATTTTAGTGAAAT
GAATACATGCTTTGGTTCCCTGTTTGTTCTTTTTAAATTTTATTTATTTTGACTTTGGCAGCTGCATCGCCTTACAGGCCAAGTGTG
AATTTTATTTACATACTGGAATCAGTCATGACACATGCCAGGATGAAAGTTAAAATCTCTGTGAAATGTAAAGCCTTAAAGGGGCGG
TCAAAGGCTAGGAATAGTGTGAGTTCTGGGGCATTCAAAAGCACAGGCTGTAATAATACTCAGAAGACCAAGGGGCAGCTGGCGAGGTC
GAACCTTGAAAATGATCTGACCTAGCTTTGCTGGCATCTACCACAACCTATGGGAACAACTAAAAATCATAAAGCAAAACGCAGAGC
AAAGATGACCCAAAGAGATCTGCTCTGCCTTTGTTCCAAGGCTCTGAAGTGAGTTGATCACAGATACTGCAGAGCTCGTTTGGAGAT
GCTGACTGGTGCGTTGCTATAGATGGGGAGCGAGAAGCTTCTGGAAGCTCAGGCATTCCACACTGTCTACCCAATAGCTGGGGCCAA
```

```
TAGACAGGGGAGAGTTGGCCTCTAAGGACTCTATTACATTAGGTTTGTGACTAGAGGTCTTCCAAAGATTCTGACTGTTGGGAAGAT
GTGACTTAGCAGGCTGGTAACTAACACGCTTTATCTCTGGTTCACTCACAGGAGTCCAAGTGAAGAGAGCAAGATGGTGTCTGCCCA
GCTTCACTTCCTGTGTCTTCTCACACTTTATTTGGTAAGCACAGAGGGTCTCTGCAGGATATTTCCTGACAGCCTTCCACTCCATAC
CATGAAAAGAACATTTATTACCACTTTTTTAAAAGGTGGTTTATCAGAATTGTACAAAAAGATGTGACGTGTTCTCGTATGATGTAT
CCAGTGAAGCGACCACAAGCCCCACGGTTGCCATCGGCTGAAGGGTGCACTTTATCCTCAGCCCTTAGAAGCTAATACGCCGTGGAA
TGGCAAACTGAGCTCTATGGTTATAGCTTTACTTGTTTCTGTCTCTACCGTCTGTTACCTACACCCCAGACCTTGTACATAGACAATC
ACTCTGCCACAGAGTTACAGCCAGTACTGAACAAACCCACCTTTCACCTGTAGCTTCTGGCCATAGAAAAGAATTACAATGATTTAT
CTGTAAGCCTCTATAACCCAGCTTTAAACCCACAAAAGCACCAAGAAACTATTTTTTCCCTGTTTTCACCTTTCTATGCTCTGTATT
TTTATTTTCATAAATGGCTTGGAGGTTCCTAGTTAAATTTGTCATTAGCCACATCTTCTATTGTTGCTATTGTCTGACAGGCTTTCA
GCACGGAACAGAATGGAAACTGGGCCCCAGCTATTGGGTAGACAGTGGGGTACCATCTCTACTTTACTCACAAGGCTCCTGTGGTAC
AGAAGGATGTCCAAAGTTTCAATGTCTTATGGACCAAGATCTCAGGTGTGCTTCTGCTTGTTTCCATAAAAGGAAGTTTTAGATGGC
TGTACCTAAAATCTTGCTTCCTTTTTTCTTGTACCCTAAAACTACACACCAAATTTTAAGTCCTCAAAGTCCTCCAAGGGCCTTATA
GTTTAAAAAGGTTTAATGTTTTTTATTAAAATACATTTTATATGTAATGCATATGTACACCTTAACACATTACAAAACGTACTTTTA
TCACCCTACCCTCTGTATCATAGAACTGAACCAAGATCTCTGGAGCTTCCATCAAGGCTAAGGAGGAGACTGCTGTTCAGATCGTAG
GAGAATTCTCACAGCCTGGGTCTGAATGAGGACAGTGCTCTCCCTGTCTTTCCTGCCTACGTTATCATAGGAGGACAGTAACGTCAG
TGCCAGGTTCTAGTTTGTGGCTACAACTTGCTCATGATAGGAAATGGACTGAGTAGCTTTAAGCTTTAAATCTCAGTTTTCTGTCCT
TTGGGCTGGGTCTCATCCTAGCATTTGACATAGAGGAACGTTTGCATCAAGGAGTAGCACAGCCTAAGCTCTGAATACATAAAAGCC
ATTAGTAGTCTATATTTCAAATGTCCAGCCCCTATATCCATATAGACCTGCCCTCGATAATGAATGAGGTCACCTTAACAGGCTTTT
CTCAGCACCTAGCATAGAGATAGGAGGTGATCACATGATCTCAATTAGGAAGCAGCGTTGAGATCAGAGAGAATGAACATTTAGCTG
TGGTACTTCGCTGGTGGTGGGCTCATTTTCTTCTTGTCTGAGGTGACTTGCCTTCCTTTCTGTAGATGGAAGCTGTCTTTCTGCCAC
CAACACCCTTCTCAGCTCTGGCCGTGGTGGTTTGGTGACTCATATTGTTCACATTCAATTTGAAATCTTTATAGCCCCAATAGTCAT
GTTAATGGGAGGGAAGAAAGATTTGTTGCTCGATACCAGGAGGAGGAGGATGTAACTTTGAACCCTGGAACTGACTCTCCCTCCCTG
CATCTCGACAGCTTGTCTGTATGCATAAAATGTAGCCTTGTCTGTGAGGTCTGTGGGCTAAGGAAGTTTCCATTGGTGTGGATAATT
GCGAGGCTGATTGCTCACTGGGTGGTCTCAGAGCCGAGCCTTGGAGCTCAGCTCTCTGGTAGCACTGCAGAAAACACTGTCCTTGTC
TTAGATCCAGCTACACTGGAGGCCTGTGCTCAGCTCTCCTGCCTCACCTTCTAGAAGTAATGGAGCTGGAGTAGACCTGAGAAGAAG
GAAGGAAAGGCTCTTGAAGGAAGGCAAACTCAGAGAGACGGTGGCTAGAAGTCTGGAAAATTATTGTGGAAGTTAAAAATGCTTTTA
GCTGCAAATAACAGGAAATCGGGATTTGGAGCAACTTAAATAAATGAAGGGTTATTTCTCAGCGATAACAGGAAGCCCGAATGATAA
ACTTAGGGCAGGGCTAACCATTCTGTGATATATCTGAGGGGGGATGCTTTTGTCTTTGGACTCCTCACTGCTCTGGGTGACTGCTCC
AGCTGCACATACAAAGCCCGTGGCCTTTCTAAGAACAACAGGGAGGGGTAGAAATGCCAGCCATAGCTGCTCTTTCCCTTTAGGAAG
GAAAGGCCAGCATTCAGATTATGACATTGACTTCTGGTTCTGGGTTTAGACGCCTATTCACCTGTTCTACTCCCTTTGGGAGTGGTA
GTAAGAGTCAGTAGAGATGGCAGCCATTGGCTATGGAGTATTCCCCAGGGGTTGGAATGGTGGGAGCCACACCTTCCGGCACCTCCC
TATTACAGCCAACTTTCCTTCATCACTTTTGCATCCCCTGGTGGACAATAACGCCTGGGGTTGAGGAGGTTGGTCTCCAGAGATGCAG
GTTCAAATCCCGCTTCTAGTTACTCTGAGGGGGAAATAACTTTGCTATGCCTTAGTCTCAATGGGGCTAGCTGTACTATACTTTCCA
TAGTATGTCGCAGAGTTATTGCTAGGATTCAGTATGCTTAGGAAAGATTCCCATGAATACCAGCTTAGCAATTATAAACTTTCCTTA
CCACCCTGGTCTGGATTCGAGTAACTGCTGAGATGCTTAGATTTCTAAGGTCTTTAGGCTTTAGAACCACGTGAGACCACTGCTTGA
AGTAAACACTTTATGATTTTAATTTATAAAGTGTTGGTCAGTTGTGAGGTATATATGCTGAACTTCTAGTTTGACCGGAATTTACCA
CGTGATTGTGTGCCAAGTAAGCATTGTGCTAAACTATCTACATATTCTTTCTCCCTCTGATAAATTCAACCGCCTTTGAGGTGACTA
TAGACTCCAGGATGCTGGGGACGTGTCACAAATGTCACAGAACATTTTAAGTGGTGGGGGTATTTCAAAAAAGCTGGTATAACCATG
TATAATTTATAAGGCAGAACGGCTCTGAGAGGCTGCAGGATTTTGTTTAGTTAAGACAGAGCATGTGCTGTCCCTGCTTGTCACACC
CATGGTTTACTCCGGGTTTCACCCTCAGAAGTCTCCTTTGTAACATGGGAGTTAAGATTCTTCCTATGGGATGGTAGCACTCATGGC
TAAATGAGAAAAAGCAATTTGAAACTCATGGCAGACTACTTTGGCAAGTAGGAGGTCTCTCAATAAACACTAGGCCAGTTTGCTT
TCTGACCAGGTCCAACACTATTTTTGGCCCATCATGGAACCCTCCACTCCGTACATTGTTTCTGCAAGTGACACCCATCCATTCACACA
CGCTCTCTTTATTAACAGACCTGTGGATATGGCGAAGAAGGGAAGTTCAGCGGTCCCCTGAAGCCCATGACATTCTCCATTTTTGAA
GGCCAAGAACCGAGTCAAGTCATATTCCAGGTAAAGACTGATCTTTGTTATCTTGATTTCTGTTCGTAGTGGACCTTTGCTCTCATC
TCTACTGAGCAGCAGGGAGTAACACGAAGGCGGGCAGTTTTTATCCTCTGTGACTTTGATGAAGCTGAGTTGCAGAGCAGTGTGGGT
GTAGCATAGGAGGAGGAGCTAGCATTCTCACACAGGCTCCCTTAATCACGATTGTGGCCAGTGCTTCAGTGCTTCAGTCCTCTCAGAGGAGGTCG
GCATTATGGACTCCACCTCTGTCCTGAGTGCAAAAGCTGCTGATTTCTTCGGGAGGTGCTGGCTTTCTGGTCATTAGGGCATCCGCT
CCTCACCTCGCTTGGCCTGTGAGGTGTTTGAAGTGGGTGGAGGAGCTTCTCTGTGAGACCACAGCCTGACTTCCTGCCTGCCTCCTC
TGAATCCTTGTCGATGTTCTTGCCTATGGGTTGCCCAAGTCATGTTGGACATGTTAATCACCATTTCCATTACTGTGATGATTTTAG
TTTTATTTTCGGTCGCTGTGATAAATACCCTGACAACAATCAACTTACGGGAGGAAGGGTTTCCTCTGGCTCACGGTACCGGAGGAT
ACAGTCGGGCATGGTATGGAAGGCAGGCAGGAGGAGTGAGACACTGCTCAAATTCCGTACATGCTCTGTAAGCAGAGAGAAAACAG
GAAGTAAGGGTCAGGCTCTAAAACCTCAAATCCTCTTTCTAGTGACCCTGTGATGTGCGTCCTCCAGTGAGCTCCACCTCCGAAAAA
ATTCCAAAACTGGGCCACCAGCTGGGGCCCAAACATTCAAATACAGGAATCTGTGGGTGGCATTTCACTTTCAAACAACAATAATTG
CAGACTACTGGGTGTGGAAGGAGAACGACTCAGAGAGAAAAAAGTTTGACTCATGACTTCCATCCATGGTAACTAGGCTCCATTTTC
TGAACCTTTGCTGAGGCAGGGCACGGTGGAGGAAAGCCAAGGCCTGTGGTAGTAAGAAGCAGAGAGAGCAGGAAAGGTCCAGAGATA
AGCTATGTATTCTAGAGGTACTCTCCTCTAGAGTGACCCATTCCTCTAACAGCAGTTCTCAATCTGTGGGTTGGGTCCCCTTTGGGG
GTCCAACGATCCTTTCACAAGGTTCACCTAAGACCCATGGGCAAATACAGATATTTACCATTATGATTCATAACAGCAGGAAATTGCAT
TTATGAAACAGAAACAAAAATATTTTATGGTTGGGAGGTCACCACAATATGAGGAGCTATATTAAAGGGTCGAAGCGTTAAGAAGAT
TGGGAACAACTGCTCTAAGTAGCCCCCACTTCCTATGATCCCATTCAGCAAATGAAGTCATTAAAGCATTTGTCTCCTGATGATGTT
CACACTCTTACAATCCAATTCCCTTAACGGCATCACCAGATGGGGACAGGACCTCCGATACATGAACCTTATTGGAGGCAGACAACT
CATATCCCAGCCATAATCTTGGCAGGTAGTTAGGTACCCATGAAAAGCAATAGGGGCTGGGGAGATGGCTCAGTTGATAAAGTGCTT
GCCACACCAAGCGTGAGGACGTGAGTTTGGATCCTCTGCGACCACATATATGAAACACTAGATAAAGTGGCTTGTACCTATAATGCC
ATCACTGGAGATACTGAAGCAGGAGGACCCCAAGGGCTTGCTGGCCCAGCTCTTCTTGCCCAGTCAATGAATTCTACATTCAGTGTT
ATATTTCTGCCTTGTAAAACAGAATGGGGAAGACCTATGAGGGTAGCTCAGTGGGTAAGGAAACTTGTTCTGCAAGAACAAAGACTG
AATTAGAATCCTAAGCACCCACGTAAAAGTTGGGTATTGGTGCTTGTAACCTTATCACTGAGGGAAGAGGTAGTCAGATCCTGAGAT
TGCACTGATTGGCCAGCCTACTCCAAATAGCAGGCTCTGTATCAGTGAGAGTCTCTGTCCCATGGCAAATAGGCAGAGAGTAATAAT
GGGCAAAGACACCCAATGCTCTGTTGTGACTTCCTCGTGTGTGTGCAGGGAAATTCAAGCTGTGTACTCACACTCGTGTACCCATAC
CACATACAACATGCATCACATGAAATAAATGTGGAGATTAATACAGACTTCTGATGTTAAACTCTTACCACATACACACTCACACAC
TTGAGAACGTGTACACACGCATACACAGAACACATAAATATGTGTATGCCATACTTTTGAAGGTGGAGTGAAGAACCTTGGCTCTCC
CCCAGGGGGCCACTGACATGCATCAGAAGGAGATGACATTCCCTACAAATGTACCCGAGGGTCCAGGCAGAGCATCCTCTCTACATG
TGTGCATCTGCCTTCTCTTCCCACCGCAGGCATCTTTATAACATGGGACAAACGGCAGTGACCTTGCGTGGGAATAGTTTGTCTCTT
AGCTGCTCTCAAGGACACAGTGCTTTTTCTCCTGTGCTGTTTTGCTCCAACAACATGCCTTAGAGGACACTTGAAATATCCAAATTG
AAACCTGGGACAGGAGACCTCCTTGCTCCCAGCCCATGTTTGACAAGCTTATACATCACAGACCAAGTCACCATTGTACAGTACA
AATGGGCTTGACTTAAGTTTCTGGTCAGAAAGATCTGTCCTTTGACTGAAAGACAGACAGCTAGGGAGATTGCAGGGTCCTGGCTAG
GGGGCTGCAGTACAAGTCTTTCTCCTTTTCTGCCCTGCCAGGGAGCTCATGTCAAAGAGTCTCCCTGATATTTTTTAAAAATCTATT
TCCTTAGAACATTATACTTTAAAATCTGATATGAAGTTCACACACTAGGCATCACATCTATTTATTTAATTTATTTATTTAGCATAA
```

```
ACAGAATGCCTACTGTGAGGTACAACCAATTTAAACTTATCCACGACTGGCTTACAAGTAAATGAGACTCAGATTATGGTTATTTTA
TTTGGCTTTGACAATTACTGGGGGGTCACCCCTAATCGACTCTTCTAACTGCTGCCCTGGCTACCTCCCCAGCCGTGTACCCCAGAT
ACTTGCTGTTTCTCCTGGCCATGTGCTTTCCATTCATCTCCCCTCATGGGTGCTTCTACTCTTTTCTTCCTCCTTCTCCACTGGTCT
CTCCTCCAACCAGGTCACAGATACCCCAAGAACCTCTAATCCCTTCAGTAACGGAGCTGGTGACCATCAGAAGTCTGTCTGCTGCTC
AGCTCAGAATAGAGCAGATCACCGCTTACTCTGTGCTCCCCAGTAGCTCCACTGGCTTTCAGTAAGACATGGACTCCCTTCCCCTCA
CCTCCACACCATACCATTTCTGTCTCTCATTAGAAAACAAACAGGCTTCTAGGGAATAATAATAAAATAAGATAAAACGAAAACTAA
CACACCAGAATTGGACAAAATAACCACAAGGAAAAGAGCCCAAGAGAGACATAAGAAACAGAGACCCACTCATGGGCACGTTCAGGA
AGCCCATGGGAACACTAGGCTGGAAAACTGGAAGCCATCATATATGTGCAAAGGATCTGTGGGGTAGAAAGAAGAAAAATTATAAAA
AATACAATAAATCTTCTTTGAAGGTCTGATAGAACTCTGCAGTAAACCCGTCTGGTCCTGGGCTTTTTTTGCTTGGGAGACTATTAT
TGGCTGCTTCTATTTCTTTAGGGGATATAGGACTGTTTAGATCATTAACCTGATCTTGATTTAACTTTGGTACCTGGTACCTGTCTA
GAAACTTGTCCATTTCATCCAGGTTCTCCAGTTTTGTTGAGTATAGCCTTTTGTAGAAGGATCTGATGGTGTTTTGGATTTCTTCAG
GATCTGTTGTTATGTCTCCCTTTTCATTTCTGATTTTGTTAATTAGGATGCTGTCCCTGTGCCCTCTAGTGAGTCTGGCTAAGGGAG
TATTCTGTTGGTATAGCTTCAAGTTTGTCTGTGGTGTTATTCCAAGCTTTCTTTCCTTATTGGTTCTTGATTTGCTCTATAATTATT
GGAAGTGATGTCTTGAAATTGTCAGTAATGACAGTTTCATGATCATATTTCTGTCCACTCTCCCAGTCTTTGATGCACTTTAGAGGC
TGTCTCTAACATTTAGGTGTGCAGATGTTTGCAGTAGCTATAGTTTCCTGGCATGCTAACCCTGCTATATCTTGTCTTTCCTTTTGA
ATGGGATCTCATTCTGTAGCTCAAGCTATTAACTCATTCTACAGCCCAGGCTAGCAAGAATTTTTCCCCTTAGTTTCCCAAGAGCAG
AGATTACAGGGAGGAGGCCACCGGCCTAACTCTTACTTTTTATCTATAACAAGTTTGCCTGAAGCTCTGTTTTTGTCTGGTTCTAGTTT
AACTTCACCCTCACTTTGGGCAACTGTTTTATTGGATTAGAAGTCTTCCTCATCTCACCAACTGTAGCACTTGGGATGTGTTATTTC
GTAGCCCTTGGCCTTACTGAGAAACCTCTTCATTTTATTGAAAAGCTCTGTGTGTCCTGATTCACTAGAGTCCTTCCTGCTATTTTT
CAGGAAGCTCCGTCTTTCAGATGTTTGATTGTGCTGCATTTGGGATCATGTGTCTGTGCCTTCTGCAGTTCACTGAGCTTCATGGAT
GGGTCTGCGAGTGTCTTCTTCACATTTGGGGAGTTTTAGGCATTCTGTCTTCAAATCATCTTTTCTCTTCATTCTTCCTCCCCTATG
GGAATTTTACAGTACAGGTATGCATTTGCTTGATGGTGCCTTTCAGGCCTTCATTTTGTTGTCGGTAGTTCATTTTTCTTTTTTTT
TTCCATTTTATTAGGTATTTAGCTCTTTTACATTTCCAATGCTATGCCAAAAGTCCCCCATACCCACCCACCCCACTCCCCTACCC
ACCCACTTCCCCTTTTTGGCCCTGGCGTTCCCCTGTACTGGGGCATATAAAGTTTGCAAGTCCAATGGGCCTCTCTTTCCAGTGATG
GCCGACTAGGCCATCTTTTGATACATATGCAGCTAGAGTCAAGAGCTCCGGGGTACTGGTTAGTTCATAATGTTGTTCCACCTATAG
GGTTGCAGATCCCTTCAGCTCCTTGGGTACTTTCTCTAGCTCCTCCATTGGGAGCCCGGTAGTTCATTTTTCAAGTTCTCTGGTTTT
CCACCAGCTCAAATATTTTTTCAAGTTCTCTCAAATGTTTTATTTTAACTAATATTCTTTCAACTCCAGAAATCCTACTTAAAAAAAA
AAAAACAACAAAATTTTTATGTCTGTATTCTCTATTTGGCATGACTTATTTTTATGCTTTCCCTAGTCTTCTAGAGAAGGCTTCCTT
TAATCCTGGATCTATTTAAAATAGATCATTTAGGATCTTTAGCAAGTTCAACATCAGGGCTTTCTTAGGAACAGTTTTTGTATCTAC
AATGTACGTTAGTTACTTATTTAGCTGTATGTCACACAGACATTTATTGAAGAGTGGACATTTTAAATGATGTGATATGGTAATCTA
GGCATCCTGTTGCTTCCTTCCTTAGAGCATATTGCTTCTACTTCTTGCCTAGTGGCTTTTATGGACTAAGTCTGAAAAGTCAGTATT
CATTGTTATGTTCAGCCACTGAAGTGCCTGATCACCTTGGTGCTCAGTTAATGATGGGACAGAGATTATGTTAGGTGCATTGGATCA
GAAAGTCTCAGCCTTCACCAACATTGGAGCATGTTGGAGCACATTGGAGCATGCCACTAGCAGTGTGGTGGAGAGTTTACAGATTTG
GCTTTCCCTTCATTTCATACGTGTGTTGAGCCTCAACATCAGCTGGGGTGAGGGGTAGAGCCTTCTCTGACCCTTCCTGCACATGC
TGGCAGCTCGGTTAAATGGATGATGCTCCAAGGACTAACACACGAGGTTTGCCGAGTCCCCTGTGGGCAGCTTATTCCCCCAGCCTT
TCCTTTAAATGTGTGACCAGTTTCTCATTTGCTTCAGCTATCATTCCTGCCTTAAGCTCATCAGTATCCATTGCTGACAGGTCTGAA
GATTACTTATTTTAAAAACATTTTTATTTATTCTTGGAAAATGTTACATGTATCATGTATGCAATGTATCTTAATCATATCCAGCTC
CCACTCCCCATTCCAGTTTCCCCATCCCAGACCCTTCCCAATGCATCTCCCAAACAATTTCATGTCTTCTTTTTAAATGTTTATTTT
TAACCCCTGACTCCAAGTAGAGCTGCATATATGTGTGCATAGTATAAGGTCATCCATTGGGCATGGTGGTTCCAGTGGCCACACCTCT
CCTAAGTACAGTGACTCTCCCTCCCTGCAGCAACCATCAACTGCAATACTTCCAAAGTGAGGGCTGGGCCATCGTAGCCCTGCCCCT
ACCCATGGTGGAATTTTAACTTAGTTCATGCGTACAACATCCATGTCTTGTTCAAAAGACAGCATGCACGCGCGCGCGCGCGCGCAC
ACACACACACACACACACACACACACACCACACACACACACACACACACACACACACACACACACACACACACACACACACACACAC
ACACATTTAGTCTTTCTTCAGTGATGTACCCTGTTCCCTAAGCCTTTGGGAGATATTTTATTCACAACAGGGACTTGCTATATAGAA
CTTGGGTTGGCCCGGTGCTATATCCTAAGCTGACCTCAAACTTACAGCAATCCTCCTGCCTCAAACTCCCAGGTGCTGAGATCTATG
TGTGAAGTACTATGCCCACCAGTTTTGCATGGGTCTTTTCAGTAGAAGTCCGTGGACATTAATAGTGATAAGAACTGACTCAGATTA
AATAGACACCAGCAGGTTACACAATGACGGTTCTCTGGCAATGGCTCTGGGAGTTACTGTCTTCTCTGCCCACTGCCACACTGTGCT
GGTGAGTCTGTCCTGTCCCTTCTATCCTCTTTCACACTGAGCTGGAAGGTCAGTGGGGTAAGGCAAATTAAAACAGCACAGGTTGGT
GCTTATTGACATCCAGGAGGGTTTTGGTCAGTTCTGATGCTGCAAAGTTGATAAATTGGTAGTTGCCACTATCTACCAACATT
TCATGCTTTACTGGAGGAATAGCAGGGTCTCACTCCATCTCTCCCATTGACGTCATACTCGTCTTAAAACACTAACAGTGGCACTGT
TTATTAATAGCCGTAATATCGACAAGAGGATTTGTAAAGCCCGTCTAGCCATGCTGTTCTCACCTTGTTTGTACACACTGCTGACA
TGCGCACTTCTGCTTCAGAGGCAACCATGGCCTGGCTTTACTTGTGTGTACTTTTGTTACTTTATTGTATTGTGTCTTTTGCCCTTT
ATAATGAAGCTTAAAGGATCAATATTGATCATGTTCTCTGCTTTAAGAATTCATGAATTCATAGATATCTGAGTTTTTAACTTTGCT
AACATAACAAACATTTCCTCTTGCACTCTAAGAATGTAATTCACAAATTTTTATATTATTGTTTACTATTAAAG
ATGGTACTTTATACCTCAAGCCATTTCCAGATGGGTCATATAAATTATATCCTATCAACAAAATATCTATTTCTTTGCAGCCTAATA
TCAATGAAATGTACTTAAAATTATTAGTGAAGTTTAACACCTTTTATATATTCTTAGTTTCATGCAGAATTCCTAATTTTTCACTGT
TGTCTTATAAGGAGCTGCATCTACCTCGTGAAGAGTTGTTGATTAGTTTTTCTATGTTGTAGAAAGTCACCATGAAGATTATCATTC
AGGAATATGTGATGTTGAAGGCACTTGTAGACTATTTTCTTTAACATTAACTTGTGTCTTCATCTTTGTCACAAGTATTAAAGTACT
GAGTGACATCTTTTGGTTTTGGCAATAAAGCTAGAAATCTTAGTGATGGTGGTAACATCATGGCTCTCACATATTCACT
GAAAACTATCTTTTTTAATATAATTTTTTCTTAGGTATTTTCTTATTTTACATTTCAAATGCTATCCCCAAAGTCCCCCATACCCCC
CATTCCCTACCCACCCATCCCACTTTTTGGCCCTGGTGTTCCCCTGAACTGAGGCATATAAAGTTTGCAAGACCAAGGGGCCTCTC
TTCATTGGATGGCTGACTAGGCCATCTTCTGATACATATGCAGCTAGAGACACGAGCTCTGGGGGGGGGGGGGGGTACTGGTTAGTTC
ATAATGTTGTTCTACCTATAGGGGTTGCAGATCCCTTTAGCTCCTTGGGTACTTTCTCTAGCTCCTCCATTGGAGGCCCTGTGATCTA
TCCAATAGATGACTGTGAGCATCCACTTCTGTGTTTGCCAGGCACCGGCATAGCCTCAAAGGACCAGCTATATCAGGGTCCTTTCA
GCAAAATCTTGCTGGCGTATGCAATGGTGTCTGTGTTTAAAAGGCTGATTATGGGATGGATCCCCGGAGGTGTGGCAGTCTCTAGATGGT
CCATCCTTTCATCTCAGCTCCAAACTTTGTCTCTATAACTCCTTCCAAAAACTATCTTTTAGCATTAGCTACAGTTTAAAGCACCAC
AGCCTTTTCTCGGCATATTGGATTGTATGATGCTTTGTTTATTGAGAGAAATGTGTTCTGAATGCTACATAAAGGATGCACAGACAA
GATTTTAGCATTGCCACCATAAAGGGCTCAAGGTTTCTTATTCTGCGATGTACAAATAAAAAAAATGGAAATAGGCATCTTTCTACAG
ATCTACAGCATTGTGCTTTCCTCAGAACCTGATGTGATGGTAATTCTTTTTTCTTTTTCTCAGTTTAAGACCAACCCTCCAGCTGTG
ACTTTCGAGCTAACGGGAGAGACAGATGGTATATTTAAGATAGAAAAGGATGGACTTCTGTGTCACACAAGAGCCCTGGACAGAGAA
ACAAGAGCGGTTCATCATCTGCAGGTGAGCTGAGACAGTGGGATGACACAGTGGGATGACACTCAGTGGGATGACATGCAGTGGG
ACAACACAGTGGGATGACACACAGGCTTCTGTAGCACGCTCAGTTCCCGAATCCCTGGGACCATTATCCCTTGCTTCTTGTTCA
GTTCTCCTGGCATTAGTACAAAACTGCAGTCCTCATATGACCTCCCTCCCTCCCTCCNNNNNNNNNNNNNNNNNNNNNNNNCCTCCCTCCC
TCCCTCCCTCCCTCCCTCCTCCTTTCCCCCCTTTCCTTTTCCCCCTCATTCTCCTCGCCTCTTTGAAACCGACTACAGAGTGATTTT
TGACCTAGTAATCCAATTTACATGATCAAAGTCCTCAGATGGCCATACTCATTTGGGGCTGGGGCCACTGTGGGCTGCGCCCCCAGAA
CTGGTGTGCCCTGGTGCTTGGTATGTGTGCCTCACACCCACTATGAAGGCAGCATCAGTGCTGGCAGAGCCACTGCGGCCCCAGAA
TGCAGAAGGAACTCATGGTCCTACTGGAAATGCTCTTCCTTTCCGTTTCCCCATAGCTTCCTAGTAGGTTTCTGAATAGCTAGAAAG
```

```
GCAGAGCGCTAACTGAAAAAGAGCAAGCTGAAACCATCTTCACTTTCTGCCATGGGCTCTGTAGACTGACCACTGTGCCAGCTCTGA
GGAGTCATAGTACACCATCACCTGAGTAATGTTCTGCATCCAGTACCCTGAACATCTAGAAAGCTACTGCCATCATCAGGGTAGCTT
AACTGATTGGACCCTTATTTTAGCAGTATATAATTTGGGCTTTTGTGGGTTCTTCATCTCCTGCAGTCATCAGGTCATTGGTCTTTT
GAATTTTAAGTTCCTATAAGTGACTTTGGGGCATGTATCTTAAGTCCAGAGTCACAAGGTAGCTTTCTTTTCTTCAACAACAGCTTG
CAGCCTTGGATTCTCATGGAGCTATAGTGGACGGTCCAGTCCCCATCACCATAGAAGTCAAGGACATCAATGACAACCGACCCACGT
TTCTCCAGTCAAAATATGAAGGCTCAGTGAGGCAGAACTCTCGCCCAGGTAGAACAGTCAGTCTGAGTATGTCTGTGGGGTGATGAA
AGAGTGGAATTGTGTGGCTGACAGTTGTCACTATAGCTCCACAAGACTGACTATCTAATGTAAATTTCATAGCGATCTGAATGCTGT
TTTATTGTGCCAATTGTTTTGGACCAAAGTCTCTCATCCTTGGCCCTGTTTAGTCTTGGGTAATGTAGGATGTTTGGAAATATCTCA
GACCTCTATCTATGAGATGCCAGGGACACTCCTGTCTCTAAACTGTGACAATAAAAAATGTCTCTAGGAGATCTAGAGAGAAGGCCT
AGCAAGAGCCTTTCTGCTCTTCAGAGGATCTGAGTTCAGTTCTGAGCACTCTTGCTGAGTATCTCATAAATTGCCTTTGACTTTGGC
TTTAAGGAATTCATTGGTTTTGTTTTTGTTTTTTTTTTTTTTTGTTGTTGTTGTTTTGCCTCTGTGGACACCCACATTTGTGTGCC
CATACTCCTCCCCTCAATTAAAAATGGAGTAAATCATTGAAAGAGTCTCTAGATCTTGAGAAATGTACAGTGTAAGTCAAAACTATG
GTTCTCTAGTGAACTTTCACTAAATACCACACAGCTTGAGGACATCGAAGGACTGTCTGGAAAAATGAGCTTGCTCTCCAATATCCT
TGTGGGATACTTAGAGTTACTGTTGGCTCCTTTCCTTATTGCTGTTCCCCAGCTGTGGCCTGTGCCTTCTCTATGTTGACCTACTCT
CTATTTCTAGATCCTTGATCTTTTCATCAACTTATAACAAGGAATCATTTGTGTTATTCAACCCCTTTCTTTTCTCAGTCTCCAGTA
GGCAAGTAACCATTCAGGGATGGCTGCACTGTCCCTGTCCCCTGTCCCTAGTGGCATCCTTGTCAGTGTTTAGTATCTGCTTCTCCT
GGGTGAGTGGGAAGAAGTGTGGTTTGTTCCCTCTGCAAGCTTTCATGGTATAACTATTTACCATGGCCTATTTCAAATTACTAGTGA
TATTAATACATGACTTTATAGACTTTGGGTCTGGTTGGTTTTAGCATATGGTGTTTTTATTTGTATGCTTTTCTTGTAATCAAATTT
ATACAAACTTTAGAAAAATAACTGACTGAAAAATTTCTGGAAATTCAAAATTGTGCTCTCCCAAGGTGAGTCCAGTGTAGCACTGGC
TAACATCGTAGAAAGTAAAACACTGACTTAGTGAATAATTTAAAAGAGCACAGAGTGTTTAGTGAAAAGTATATTTTTCAAGACAAA
AATAGTTAATGAGAAAAATGACATTCATTGGATTCTTTGCAAGTTTCTTAAATGTCTGGATGAATAAAAACAATTGGGATAAAGGTC
TGTGTCTGGATCCATGATGTTCCCACATGCTGTTTTTGTTGAAGTAGTATGTGACGAAAATTAGTCTCACACAGATCTGTAGCTTAG
GAAATGAGGAATATTTTAACAGCATCTTCAGATATTATGGACATTCTTTCATATACAACCAAAGCTTGAAAAATGCATTTTTCTTGC
AGATGGTAGCAATATAATAATGGTATATTTTGTACTATTTTACTTTAAAATCCACTAAATTATCTTGTTCTTAAATGGACCTTTTCT
AGATGTGATTTCAAACACCATAACTTTTTGAAATATTCCTGAGGACTCCTATAGGATACAAAATAAAGATTAATTTTTCCAGTTAGG
AACTGAGGCAATTTCCTCCACTCTCTTTGCATCTCCTGAGGCAGACATGGATACAGTTGCTTTAAGTTTCAACTCTCTTCTGCGTAT
GCTTTGTCACCAGGAAAGCCTTTCATGTATGTCAATGCTACAGATCTGGATGATCCGGCTACTCCCAATGGCCAGCTTTTTTATCAA
ATTGTCATCCAACTTCCCCAAATCAACGATGTTATGTACTTTCAAATCGACAGCAAAACAGGGGCAATATCGCTTACCCCAGAAGGT
AAGTCAAGGCAAGCCCTCACAAACCTCAGCCAGAGGAAGGTCTGTCACCTGTGGGCACATGTCTTTTCTCCTTCTCCTCCCCAACTT
CTACAGGATCCCAGGAATTGGATCCAGTGAAGAATCCTTCCTACAACCTGGTGGTCTCGGTGAAGGACATGGGGGGCCAGAGTGAGA
ATTCCTTCAGTGACTACTACATATGTAGATATTTCTATTAGAGAGAAATCTGGAAAGCACCAGAACCCGTGGAGATTAGAGAAAACT
CGACTGATCCTCACCCCATCAAAATCACTCAGGTAGTGTCCAAGTGACATTTATTGCTAGTTCGGATCCCATACCTTGATATGTAGAG
ACATATTTTGTGGTCTTCACTAATTGTTTTACTAGATAGAGTTTTGCATCATATAATTACAAAGCAAACTAAGTAATCTCCAAAGAT
TATCATCACAAGCAGAAAACTTGGCCCATGTGCACAAACGCGCGCACACACACACACACACACACACACACACACACACACACACTT
TATTGTGGTGTTTTATTGTTTTCATTCAGCAGTGTGAAATATAGCTGCTTTATAGTAGGTTGAATGGCATGTGGTATACCATAAATA
TATGTTGTTAGTAATAATATCAGAGAAACCAGGCCTTGTCTATGTATCAGGCCTTACTTTTGAATATTTTTTGTTGTTGTTTGGAGG
ATAACTAGGGGTCAGTAGTTATTTTTATTTCTGAGATCTTTCAGCCTTTAATATTTGAGGGAGTACCCCTCATCATGTGTCTCAACT
AAATGAATGAATGAATGAGTCTAAGTCAGTTGTTCTCAACCTTTGGGGGTTGAATGACCCTTTCATAGGGGTCACATTGGATATCCT
GCTTATCAGATATTTACATTGCGATCCATAGCAGTAACAAAATTACAGCTATTTAAGTAGCAATAAGAATAATTTTATGGTTGGGGT
TACCAAAACATGAGGAATTGTATTAAAGGGTCTCAGCATTAGGAAGGTTTAGAACTACCTCTTTAAGGAATCCTTTATTGCTCTGGA
TTCATCTTAATTTTGGATTCTAGAAGCTCATCATACATCATATGAAGTGCCTTTTTTCTTGATAAATTGATGTTATACCCAGTCAGA
GTTCTAGAAGAAAACAACCTGTGGGCTCTTTGTGAGTTTATAAAGATTGCTTTAAGGAAAGAAATTCCCTTGTCTGCTTTTAAGG
TTAGTTTAGGTTGAGAATTTTAAAATATGTTACTCAGTTGCCTTAAGAAACTGCTCATGTGCAGACAGAGTTGTGAGAAATTCCAAAG
TGAGAATATAGAAGCTGTTGATCACCTTTTCAGTCTGTTAATTCTTGTGTTTTTTCCCACCACTCGCTTTGGCCGTGCAGGTCAGT
GGAATGACCCAGGGGCCCAGTATTCCTTAGTCAACAAGGAGAAGCTGTCGCCGTTCCCATTCTCGATCGACCAAGAAGGAAATATTT
ATGTGACTCAGGCCTTGGACCGGGAGGAAAAGAACTCAGTGAGTGAGACGGGATGTTCCGTGGGCCACAAGACAAATCCTATGTCTA
CTTACAATTGAGGACAAATTAATTGTAAATTATTAAAAAAAATGACAACAAACTGTGTTCTAGATCCTGGATGTTTGTGCATTCTC
AAAGACACAGTGATTCGATAATTTCCCCACAAGTGTAGCCAACATACTTAGAATGGAGAATTGAGGGGGGAGTAAGTATGTCTGTGG
GATGATGAAATTCACTCCCCCCCCCTTCATTTCTCAATTCTAAACATGTTGACACTAAGGTGAGAGATGGTTAGCAAAACAGTTATTT
TCCTCCACCTGTTAAAGTGAAGGCATCTTCTTACCTTTGACCTTCACATTGCAGCATGTTTTCTTTGCAACTGCCAAGGATGAGAAT
GGAAAACCACTTGCATATCCACTGGAAATTTATGTGAAAGTTATTGACATTAATGACAACCCACCGACATGTCTGTCTCCAGTGACT
GTATTTGAAGTCCAGGAGAATGAACCATTGGGTAAGAAATGAGAGCTGTTTGATGTCATGAACCTGCCTTCCTCAGCTGCTCCTGTT
ATCCCTTTCTAGCCTCTGGGGGGTCAGGTAGACCTATTCTGCAGTCCATTGTCCTTGTCACAGAGCTTGAAGGTATCATGAAAGTCA
TATAGGTACAACACTTTGAAATGAACACTGTCTCTGGTCCTCTTAAGTTTGATGCTTTTATAACTGCCACCACCACCACTGTTTCAA
TGACAGTCACTGCAGAGCAGCATCTTGTGTACAAGTCCAGAAGGGTCCTTGTTGCAAGATTTTCCCTTCAGATATCTTATCATGCTT
CTAAACAAAATACTTTCAAGAGATAAAACCTCATCCAGCATGCTAAAGAACTTGATCCTTTACATCCACAGCCAGATATCCCACAAT
AAGTAGACAATACCAAGTTCAATGTAACCCTGTGGTTCCCATTTGCGGAGACCAAAGATAACAGAAAAACCTGGCAAGTGAAGTTAA
GCCTCAAGCATGCTTGTCCTTTTGATCTTTCTATGTTTGAGAAGAATGTATCTCCTGATAGCTTTACATTTGTATAGGGACGACACT
GTAAGAATGTTAATGACTTGGGGGGGGGTGTTGTCTCTCTCCTGGATTTGAAGCTTTTGTCTATTTCCATCTTGGAGGTGAGCCCAT
TGAGTAGGCTGATGTGTCTTTTGTGTTTGGGTTATGTACTTTTTAAGCTTGTGAGATCAGCATCTTTGTGGTCCCCATGGGTATGGC
CTACCCATCTAGGGATGGACATTTTCTTTGATCCTTCTGCTCTGGCTGCAACTCAGCTTTGACACAGCAACTGCTTTCCTGGTGGCT
TGGTGGGTTTCCTCCCATGTAATCATTCTTTGCCTCCGTGGTTAGGTGTCAGTCACACAGAGGTTGAATATTCTCTGTGTGTCCT
GCACTGGTGTTCTTCATTTTATTTATCTAAATTCCTGCNNNNNNNNNNNNNNNNNNNNNNNNNNNNNNNNNNNNNTATTGTTGTTCCACCT
ATAGGGTTGCAGATCCCTTTAGCTCCTTGGGTACTTTCTCTAGCTCCTCCATTGGGGGCCCTGTGTTCCATCCAATAGCTGACTGTG
AGCATCCACTTCTGTGTTTGCTAGGCCCCTGCATAGTCTCACAAGAGACAGCTATATCTGGGTCATTTCAGCAAAATCTTGCTAGTG
TATGCAATGGTGTCAGCGTTTGGAGGCTGATTATGGGATGGATCCCCGGGTATGGCAGTCTCTAGATGGCCCATCCTTTTGTCTCAG
CTCCAAACTTTGTCTCTGTAACTCCTTCCATGGGTGTTTTTTCCAATTCTAAGAAGGGGCAAAGTGTCCACACTTTGATCTTCGTT
CTTCTTGAGTTTCATGTGTTTTGGAAATTGTAACTTATATCTTGAGTATTCTAAGTTTCTAGGCTAATATCCACTTACCAGTGAGTA
CATATCATTTGAGTTCTTTTATGATTGGGTTACCTCACTCAGGATAATGCCCTCCAGGTCCAACCATTTGCCTAGGAATTTCATAAA
TTCATTCTTTTTAATAGCTGAGTAGTACTCCATTGTGTAAATGTACCACATTTTTTTGTATCCATTTCTCTGTTGAGGGGCATCTGG
ATATATGCCCAAGAGAGGTATTGCAGGATCCTCCAGTAGTACCATGTCCAATTTTCTGAGGAACCGCCAGACTGATTTCCAGAGTGG
TTGTACGAGCTGCAATCCCACCACCAAATGGATGAGTAGTGTTCCTCTTTCTCCACATCCTCACCAGCATCTGCTGTCACCTGAATTTTT
GATCTTAGACATTCTGACTGGTGTGAGATGGAATCTCAGGGTTGTGTTTGATTTGCATTTCCCTGATGATTAAGGATGCTGAACATTT
CTCAGGTGTTTCTCAGCCATTCAGTATTCCTCAGGTGAGAATTCTTTGTTTAGCTCTGAGCCCCATTTTTTAATGGGGTTATTTGAT
TTTCTGGAGTCTACCTTCTTTATATATATTGGATATTAGTCCTCTCTCTGATTTAGGATAGGTAAAAATCCTTTCCCAACCTGTTGG
```

EP 2 204 376 A2

```
TGGCCTTTTTGTCTTATTGACAGTGTCTTTTGCCTTACAGAAGTTTTGCAGTTTCATGAGGTCCCATTTGTCAATTCTCCATCTTAT
AGCACAAGCCATTGCTGTTCCATTCAGGAATTTTTCCCCTGTGCCAATATCTTCGAGGCTTTTCCCCACTTTCTCCTCTATAAGTTT
CAGTGTCTCTGGTTTTACGTGGAGCTCCTTGATCCACTTAGATTTGATCTTAGTACAAGGAGATAGGAATGGATAGATTCGCATTCT
TCTACATGATAACCACCAGTTGTGCCAGCACCATTTGTTGAAAATGCTGTTTTTTTTTCTACTGGATGATTTTAGCTCCCTTGTCAA
ACATCAAGTGACCATAGGTGTGAGGGTTCATTTCTGGGTCTTCAATTCTATTCCATTGGTCTACTTGTTGGTCGCTATACCAGTACC
ATGCAGTTTTAATCACAATTGCTCTGTAGTAAAGCTTTAGGTCAGGCATGGTGATTCCACCAGAGGTTCTTTTTATCCTTGAGAAGAG
TTTTTGCTATCCTCGGTTTTTTTGTTATTCCAGATGAATTTGCAAATTGCTCTTTCTAATTCGTTGAAGAATTGAGTTGGAATTTTAA
TGGGGATTGCATTGAATCTGTAGATTGCTTTTGGCAAGATAGCCATTTTTACAATGTTGGTCCTGCCAATCCATGAGCATGGGAGAT
CTTTCCATCTTCTGAGATCTTCTTTAATTTCTTTCTTCAGGGACTTGAAGTTTTTATCATACAGATCTTTCACTTCCTTAGTTAGAG
TCATGCCAAGATATTTTATATTATTTGTGACTATTGAGAAGGGTGTTGTTTCCCTAGTTTCTTTCTCAGCCTGTTTATTCTTTGTGT
AGAGAAAGGCCATTGACTTGTTTGAGTTAATTTTATATCCTGCTACTTCACCGAAGCTGTTTATCAGGTTTAGGAGTTCTCTGGTGG
AATTTTTTAGGGTCACATATATACTATCATATCATCTGCAAAAAGATATATTTTGACTTCTTCTTTTCCAATTTGTATCCCCTTGATC
TCCTTTTGTTGTCGAATTGCTCTGGCTAGGACTTCAAGTACAATGTTGAATAGGTAGGGAGAAAGTGGGCAGCCTTGTCTAGTCTCT
GATTTTAGCAGGATTGCTTCCAACTTCTCACCATTTACTTTGATGTTGGCTACTGTTTTGCTGTATATTGCTTTTATTATGTTTAGG
TATGGGCCTTGAATTCCTGATCTTTCCAAGACTTTTATCATGAATGGGTGTTGGATTTTGTCAAATGCTTTCTCCCCATCTAAAGAG
ATGATCATGTGGTTTTTGTCTTTGAGTTTGTTTATATACTGGATTACGTTGATGGATTTCTGTATATTAAACCATCCCTGCATCCCT
GGGATAAGACCTACTTGGTCAGGATGGATGATTGTTTTAATGTGTTCTTGGATTCAGTTAGTGAGAATTTTATTGAGTATTTTTGCA
TCGATTTTCATAAGGGAAATTGGTCTGAAGTTCTCTATCTTTGTAGGATCTTTCTGTGGTTTAGGTATCAGAGTAATTGTGGCTTCA
TAGAATGAGTTGGGTAGAGTACCTTCTACTTCTATTTTGTGGAATAGTTTGTGCAGAACTGGAATTAGAACTCTGCACTAAACCCGT
CTGGTCCTGGGCTTTTTTTGGTTGGGAGACTATTAATGACTGCTTCTATTTCTTTAGGGAATATGGGACTGTTTAGATTGTTAACTT
GATCCTGGTTTAACTTTGGTACCTGGTATCTGTCTCTAGAAAATTGTCCATATTGTCCAAGTTTTCCAGTTTTGTTCAGTATAGCTTT
TGTAGAAGGATCTGATGGTGTTTTGGATTTCTTCAGGATCTGTTGTTATGTCTCCCTTTTCATTTCTGATTTTGTTAATTAGGATGC
TGTCCCTGTGCCCTCTAGTGAGTCTGGCTAATGGTTTAATCTATCTTGTTGATTTTCTCAAAGAACCAGCTCCTCGTTTGGTTGATT
CTTTGAATACTTCTTGTTTCCACTTGGTTGATTTCACCCCTGAGTTTCATTATTTCCTGGCGTCTACTTCTCTTGGGTGAATTTGCT
TCCTTTTGTTCTAGAGCTTTTAGGTGTGTTGTCAAGCTACTAGTGTGTGCTCTCTCTAGTTTCTTTTTGGAGGCACTCAGAGCAATG
AGTTTCCCTCTTAGAAATGCTTTTATAGTGTCCCATAAGTTTGGGTATGTTGGCTTGTCATTTTCATTAAACTCTGAAAAGTCTTTA
ATTTCTTTCTGTATTCCTTCCTTGACCAAGGTATCATTGAGAAGAGTGTTGTTCATTTTCCACGTGAATGTTGGCTTTCCATTATTT
ATGTTTTTATTGAAGATCAGCCTTAGTTCTTGGTGGTCTGATAGGATGCATGGGATGATTTCAATATTTTTGTATCTCGTTGAGGCCT
GTTTTTGTGACCAATTATATGGTCAGTTTTGAAGAAGGTACCATGAGGTGCTGAGAAGAAGGTATATCCTTTTGTTTTAGGATAAAAT
GTTCTGTAGATATCTATTAAGTCCATTTGTTTCATCACTTCTGTTAGTTTCACTGTGTCCCTGTTTAGTTTCTGTTTCCATAATCTG
TCCATTGATGAAAGTGGTGTGTTGAAGTCTCCCACTATTATTGTGTTGATTTGATGGATTTCTGAGTTTACTAAAGTGTCTTTA
ATGAATGTGGCTACCCTTGCATTTGGAGCATAGATATTCAGAATTGAGAGTTCCTCTTGGAGGATTTTACCTTTGATGAGTATGAAG
TTTCCCTCCTTGTCTTTTTTGATAACTTTGGGTTGGAAGTCAATTTTATTCGATATTAGAATGGATACTCAAGTTGTTTCTTCAGA
CCATTTGTTTGGAAAATTTTTTTCCAGCCTTTCACTCTGAGGTAGTGTCTGTTTTTTTCCCTGAGATGGGTTTCCTGTAAGCAGCAG
AATGTTGGGTCCTGTTTGTGTAGCCAGTCTGTTAGTCTATGTCTTTTTATTGGGGAATTGAGTCCATTGATATTAAGAGATATTAAG
GAAAAGTAATTGTTGCTTCCTATTATTTTTGTTTTTAGAGTTTGGAGTTCGTAGGCTTTTTGTATGTTCATGGGCATCTCT
TTACTTTCTTGCTTTTTCTAGGGCGTGATTTCTGTCCTTGTATTGGTTTTTTTCTGTTATTATCCTTTGAAGGGCTGGATTCCTGGA
AAGATAATGTGTGAATTTGGTTCTGTCGTGGAATACTTTGGTTTCTCCATCTATGGTAATTAAGAGTTTAGCCGGGTATAGTAGCCT
GGGCTGGCATTTGTGTTCTCTTAGTGTCTGTATAACATCTGTCCAGGATCTTCTGGCTTTCATAGTCTCTGGTGAAAAGTCTGGTGT
AAATCTGATAGGCCTGCCTTTATATGTTACTTGACCTTTTTCCCTTACTGCTTTTAATATTCTATCTTTATTTAGTGTATTTGTTGT
TCAGATTATTATGTGTCGGGAGGAATTTTTTTCTGGTCCAGTCTATTTTGGAGTTCTGTAGGCTTTTTGTATGTTCATGGGCATCTCT
TTCTTTAGATTTGGGAAGTTTTCTTCTATAATTTTGTTGAAGATATTTGTTGGGCCCTTTAAGTTGAAAATCTTCATTCTCATCTACT
CCTATTATCTGTAGGTTTGGTCTTCTTATTGTATCCTGGATTTCCTGGATGTTTTGAGTTAGGATCTTTTTGCATTTTGTATTTTCT
TTGATTGTTGTGCCCATGTTCTCTATGGAATCTTCTGCACCTGAGATTCTCTCTTCCATCTCTGTATTCTGTTGCTGATGCTTGCAT
CTATGGTTCCAGATTTCTTTCCTAGGTTTTCTATCTCCAGCGTTGCCTCACTTTTGGTTTTCTTTATTGTGTCTACTTCCCTTTTTA
GGTCTTAGATGGTTTTATTCGATTCCATCACCTGTTTGGTGTTTTCCTGCAATTCTTTAAGGGATTTTTGTGTTTCCTCTTTAA
GGTCTTCTACCTGTTTAGCAGTGTTCTCCTGTATTTCTTTAAGTGAGTTATTAAAGTCCTTCTTGATGTCCTCTACCATCATCATGA
GATATGCTTTTAAATAGGGGACTATCTTTTCAGGTGTGTTGAGGTGCCCAGGATTGGGTGGGTTGGGAGTGCTGTGTTCTGATGATG
GGGAGTGGTCTTGGTTTCTGTTAGTAAGATTCTTATGCTTGCCTTTCGCCATCTGGTAATCTCTGGAGTTAGTTGTTGTAGTTGTCT
CTGGTTAGATCTTGTTCCTCGGGTATTTATGTTAGCCTCTATCTGCAAACCTGGAAGACTAGCTCTCTCCTGAGTTTCATTGTTCAG
AGTACTCTCTGCAGGCAAGCTCTCCTCTTGCAGGGAAGGTGCCCAGAATATCTGGTGTTCAAACCTGCTTCCTGGCAGAAGTTGTGAT
CTACTCACAGAGGTCTTACGATCCCGTGGAGGGTCCTATGTGTACCATGCAGGTGTCTGCAGACTACGTGCCCAAGGTACCTCGGTG
CTGGCGTGGACCAGAAGGGATTTGTGCCCCTGGTCAGGCCGGGTTTTTTGCTTCCCCAATTAATGCAGTCTCAGGTCCAGCACGATT
GGATTGGAGTAGCAGCTGTGTTCCACTCACCAGAGGTCTTAAGATCCTGTGGAGGGTCTTGTGGGTAGCCTGCGGGTGTCCACAGAC
TCCGCGCCCAAGCTACCCCAGTGCTGGTGGGGCACTTCATGAATTCTTTAAAGGAAGAAAAATTCTATTCAACTTGTTCCATATGCA
GCTGTGGGAGGTACAGACAATGCATCTCCTTCTTCTTGAGGGCTTGCTGCTTAGCTAGCTATATAAAACATACACAAGTAGAAATGTTAAC
ACATGAAGTACTGGTTCGTTCCTCTTAAGTTTATACTGTCCTCTTTTAGGAAAAAGAGGAAAAAGAAAAGTTTCCTCAATTATGATACA
TTTAGAAAGAAGCAGCCGCTGTTACATGAGTACAAATTCCAAATGGCTCTGAGCCACATGCCTCATGGGTGGATCTATGTCTATCTA
TCTATCTATCTATCTATCTATCTATCTATCTATAGATCTATAGATCTATAGATCTATAGATATAGAGAGACAGAGAGAGGC
AGAGAGACAGAGAGAGAGAGACAGGAGAGACAGACATAGTGATAGAGATAGAGATATTTCTTCCCTTTTCCATCCTTTAAAACCCAA
CTCCTGTAAGTGTCTGTATAAGGCCTTGGCTGCAAACTGATATAAAATGAAGAGTGTAATTCAATTCAAATAGTGGCATCTGAATAAA
TCTCAGGTGGGATTCTGACCGCATGAAAGGAAACTCACACTTCCCATTTTATAGATCTGGATGACCACAGATTCTATCAGAACTTGC
TCAAAGTTGGTAGGACACAGAATCACATCCACTGACCTCAGGAGCTCCTGGGCCAAAATACACACAGGCACAGTATACATATGGCCA
TGGCATGTGGTATGTGTGTGTGTGTGTGTGTGTGTGTATGTGTGCGTGTGTGACTATTCTTTATGTGTTATCTTTGAGTGGAGTC
AGTCTCTACAGTTGTTCACAAAGCTCTCAGAAGTAGCCCAAGATTGGCTTCTTCTAGTTTAGCTGTGACATTTGTCCAGTGTGTTAG
TGTCTTGGGGCTATAGTAATAAAGCACCAGAAATGTGAACTTCTCAGTTCTGCTGATGGGGAATCTGAAATTATGGTGACAGGATCT
TGTGGGAAAGCCTGTTGCCTCTTCTGACTCTTGACTTAAGCTAGCTGCTGACAACACTCTGCAGATCTCTTTCCTCCTCTTCTTCTTT
ACCTCCCTCTGTGTGTGGATGATCTCACCCCAGTAATGTAGTTGCTTATATCTCTGATGACCCTATTTTCAAATAGGGCCCCCCACC
AAGATTAGAGGTGGAAGTAGATTCCTTTGGGTGGGTGAGGAGCAGGAGGTACACCATGCTGAACTGTCACTCCCCACCAAATCTGTA
AGTTGATGTCCTGCTCCTTGTGTGGGGTAAATTGTTCTTCCTCCCCAGCTCATAGGATAAAGTTCTAAGTCTTGGTACTTCAGAATA
TGGCTATACAGATGTGTAGATGTTTTAAAGAGGGAAATTGGGTAGGATGAGTGCTGGGTAGGATGTGGGACTGGGTAGGATGAGTGCTGG
GTAGGATGTGGGACTGGGTAGGATGTAGGGCTGGGTAGGATGTGGGCTGGGTAGGATGTGGGACTGGGTAGGATGTGGGGTTGGGTAGG
ATGTGGGACTGGGTAGGATGTGGAACTGGGTAGGATGTGGGACTGGGTAGGATGTGGAACTGGGTACGATGAAGGATTAGGAAGATAGC
TCAGTCAGTACATTTTTTTACCTTGCCTCTATAAGGACCTGGGTTAGATCCCCTGATGCATACACACAAAGCCAGGCATGGCAGTACA
TTTGTAATCCCAGTGCTGGGGAGGCAGAGACAGGTAGATTACTAGGGTTCACTGGCCAGACAACTTAAATATACTTAGCAAGATTTT
```

195

```
GTAGAGAGAGAGTGCTAGAGACCCTGTTTAAAAAAAAAAAACTAAATAAAACAAAACAAAAACCAAACAACAACAACAAGAACAACAA
AACCAAACAAAAGGAAAAACAACAAGAACAACAACAAAAAGGTGGGTAGCTTCTACGGAATGCCGCTGAGGTTGCCCTCTGACTCCC
ACATTCACATGTATGTATGTGTATACCCACAAACACCCAGCTGGTGGGCCTTAATCTAATCTGACCAGAGTTCCTAGGATAAATTTG
GGTGCTAAGGTATAAAGAGGAGGTTACATAGTGAGGAGGAAGAAATAGCCAGGGAGAGACGCTTAGAGGAAAGCCGTTTTGTCAACA
CCTTGATTTCATGTGTCCAGGGTCTAGGTCTCCCAGAAGGCTGATTTTTGTTTAAGTCTCTCAATTAACAATACTGTGTGGCAGGCA
GTCTTGGCTGCCACTATGTTCAATCAAGCATACCATGAAAAAATGCAAGGAGCCAAAGTAAATCTAAATGTCTTTGCTCACCACCCC
AGACAGTTCCCGTGTTTCCTAAGAAGCCACTTGATGAACTGATGTGGTTGTTGTTCTTTCTTCTCCAAAGGTAACAGCATCGGGATC
TTTGAGGCCCATGATATGGACGAAGCTAACAACATCAACAGTATTTTGAAATACAAGCTTGTAGACCAAACACCCAAAGTTCCCTCA
GATGGACTTTTTCTCATTGGTGAATATGAGGGAAAGGTTCAGTTAAGTAAACAGTCCTTGAAGAAGCAAGACAGTCCTCAGTACAAC
TTAAGTATCGAGGTGTCTGACGTAGGTAAGCACTGTACCCGATGTGGTGAGTTTACATGTCTGTTTTCCTTTTACTAAGGATTTGTG
AGGCCCTTCCCATGCCAGTAGCTTTCTCTGCTATCTTTGTGACTGTGAGGAAGCCAGATGTTTTCTCAAGAGCTAGAGGTGCAGATT
CGGAGACATCGCATTGTCTTTGGTCCTCTGAGAACTAGGTGTCAAGAGGAATTCAGTGACCATCTTAAAGAGAATGCCCTTAAGAAA
AAATGGAAAGGAAGCTTCGAAGTCTAGGGGATCCCCAGACAGCAGTTCTGGCTTGATCAAGATGGAAGGAGTTTTGATAACAATGTT
CTAGACCCACTGAGCAGTCTAAGAAGAATTCCAAATGGTTATCGGGAAGGTCTGATGCAGGGCATTGTCTCTGTGGATAGGCCTGTCT
TTGCATCACTACTTGCTCAGTAACCATCTGGGGGCAGCAAATAGAAGATGAGGTACAAGTGTACTGTTGGTCACAGCATAACACTGC
AGCTACCAGGTTATCAGTCCAGCTGTTAAAGTCACCAGGCGCAGTCTCACAGCTACTATAATTAAAATTCAGGAAAAATTCTTTCCT
CATCCTTGCTTCCAGACTAAAAGTATCTGTGTATGAAAGCAGTAGAGAAATTAGTCTTTGCTGTTCTTGAAGGCAGAGAGAGTTCTG
CCCTTTCTTGCCACCTTCGTATTGCCCACTAGACAACAATAGGAAACCTTTCCCCTTAATCCTTGTATACTATCTAAGTGGGTTCTG
CTTAACCTCTCCTCTCCTTAGGGCAGAGGAATCCTTAGAATCTTATGGCAACTTCCCATTCTTAAACACATCTATACATCCACAGAC
AAATTCGCTGCTATCTCTAGGGTACTCAGAGTCCTAGGAGGTAACTGGCTCATGAAATGATTCTGTAATTGTTTCTGAGGAACACA
GTTTACATAGGTGTGGAAAAGAAAGGACCAGTGGTTCACATGTATTTCTTATTTTTTTCCCCTTTCCCATGGACCCTGGGGAGAACT
TGAGAACTTGAGTTAAACAGGTTCCAGTGCAAACTGCCTGTCAGGTTCTCAGGTAGCTAGTGAACTGACACACGATGAAGGTCTGAT
GCTGTTTAGCACTATGTTAAAAGATCCAGTGAGATAAATGTCAATCATGCTATCCATGGCAAAGGGGCAGACTCTCCCTCACATTGG
CCACCCACACATTCAGTATTTAATCATTTGCCTTGCTAAGGTATCAAAGTATTAGGCATGCACAGATGAACTGGACCCAGTGTTTGT
CCCTAGGGAGTACCCCTTTTGTCAAGAAAGATGGACACACATGTTAACAACTTGTAGGGACATACTATCTACTGTCATTGTGGTGCT
CTTCGTAAACACTGTCCCACAACTCATCTGCAGGCCACACTCATCTGCAATCTAGGATCCACTTGCTGGTTCCAATTAGTCCTAGAA
TTGTCAAACCACCCTCTAAGCCTGGACCATTTCTGTGAGCTACCTTAGCCTCCCTTGGAGGTGAATTATGCCACTTTATTCCTTACT
TGGAGAAGAGGAAGCAAGTGACCTTGAACCTCTCTACCATGCTGGGTAGACACATGCAGAGAGGGAAAGGCAGCCTGTGGGGTGTTC
AGCAGAATTGTGCAAAGGGACAGTTTTGCTTTGGGCATCAGAGGTTATATAACATTCACCTGTCAGGTAAGGGAGCTGCTTCAGAGA
AAAGCAGCCATCCTGGTTGTTGAAAAGCCTTCCCAAGGATAGGGCAGAGGGATGCTCAGGAGAGACTCTCAGGGCCCAGTCTGGCTT
CCACTTCCTCTTGTTGACCTGCTTGTGGATGAGGTGGTGGTGGTTGGGGTGGGGGGCAGAGCAGCCTCCATTCACAGGAACTGTAAA
GTTATAGCAGCAGCTACTGTGTCACTTGTCAATGTTTCCTTACCAAATCCTGATGCCAATGCATGATTTCCAGTGAGAATTCTGTGG
TGTGACTTTGGTTTTCAGATTTCAAGACTCTCTGTTATATTCAAGTCAACGTTATTGATATCAATGATCAGATTCCCATCTTTGAAA
CATCAAATGTGAGTAGATATCACTATTTGTTTTATTTGGATCATATAATTGAACCCTTATTTAAATAAAAACACAGACAATGTGCCA
GGTAGCCTGCTCAATGCATCAATAAAAATCAGGCACCATCTTCACGTGAGTCCTCAGGGGCCCTAAGGTGTACTGAGAGTAAGGAGG
GATGTCTATTTATAAACTCTTGCCAACAGCTCATTTATTCTAAATGAGGCTTAAAGGCTACAACCACAATCCTTTTCAGATTGAAAA
TTAAATGGCTGCTGGTGGCTTGTTTATAGATTTTGCATACAATGGACAGTAGCTAGCTACAAGATCTATGCCAAGCATTTGGGATT
TAAAAAATTAGCATCAGAAATACTGGTGACTTTAACTCATTTAGTATTTAACCAAGTAAGAGGAAAGGAAAGGGGAAAAATATTTCT
GGCTTAAGCCTAAGAGAAATGTGCTCCCTATATTTGCAAGAATTAGCGTTGGACGTAGAGTAAGTGTCAGGGCAGAAAGGACGTCAG
GGAAAATGGCTGTGGTCACAGTGTGGTGTTATCATTGTACCCTTAAAAGGAATGTCTTGGTTTATTTAGCTCCAGGGAAAGAGAAGC
CCACTTGACTAGATTGTTATGTTAGAAGTCAGTTTTGAGGCAGCCCAGAGCCTCCTGATGATGTCCATAAATCAAAACAGAGGCAAG
GCTGTAACAAAGAGGGAATTAATTTACAGTAGAGCTCAGAAGCTGATGGAACGTATCTTGTCTAACAGCCACTGAG
CTTAAGTAAGCATGAAATAAAGTATTCATTTTTTTCTCAGCCTCTATCCTCTGAGGCATTTTTCACTTCCTAGTATTGTGTGTCCCC
TGCATTTCAGCAGACAGTGGTCAAGTAGATTGAGCAGAAGAATTCTGCAGTGGGGAGTTCAAAAGCTTGGAGATGCAAACCTGGCTG
GAGAGAAAGTGGGCTTGCTTAGTCCATTGCCAGCGTCTTCTCTTGGATGCTGGCTGGTACCATTTTTGAGGTCATGATGTGGGGGAA
GAGTGGGTCATGGGCTTCAGGGGAGTTAGAATGCTTTGTGGGGTGTGAGGAACACAGGCTTTGGTGGTTTGGGTTCAATCATACAA
GCTGTTAGCCTGGTGTGTGTGTGGGGTTGACTATGCTAGTTCTTGGCTCCTCTGTCAAATGTCACTGACATGTTTGTAGCCCTCTAG
TGTACTTTGCTGCTCATCTTTCATAAGGGCTGTATACCAGCCATTCAGAGGCTCTGCATGCATTATCCCCTAATGCACCACAGGTTGC
ATTCTCTTAGTTGACAAGTGACAGAGCCCATGTTTAAAGAAATGAGATAACTGTATCATTCAACTAACAAACAGCAAGTTTAGGGTT
AGTAGCCAACTCTGACTTCAATGTTTTGGGTTCCTAATGTCCATCACTTGGTTGACTCAAGAATTAGAAGTATCTGTCACATCATAA
GGACTTCTCATGTTATGACTACCTATATCATTATTTTTCAAATAATTATAGTTATTAAACATGTGGATATTGGAGGGGAACTCTATT
TTAGAATCCTTGTAGAATGTCTAAGTTCTATAAACTTAGTTTAGTTGGTATAAATTTCGGTTCATGAGTACAAATGATGTCAAACCC
GACTGTCACATGTTCATCAAGAGGAAAACCATGCTGGGTATTAAAGCCAGCTGTGACCTCTGTCCTGAACCCAGAGACGTTTGCCTT
TTGTCCAGTAAACTCAATGCCATGGACCTTCTGGTTCCTTTCTTGTTTCTTCCTCAGTATGGAAGCAAGACTCTGTCTGAAGACACC
GCCATCGGATCCACCATCCTAATCATCCAAGCTACAGATGCTGATGAGCCCTTTACAGGGAGCTCTAAAATCCTGTACAAGATTGTA
CAGGGAGACACGGAGGGAAGACTAGAAGTTGTCACAGATCCCACGACCAATGCAGGATATGTCAAGATCAAAAAGGTAGATATCAGC
CGGGCAGTGGTGGCACACACTTTTAAGCCCAGTGCTTTGGAGGCAGAGGCAGGTGGATCTCTGAGTTCAAGGCCAGCCCTGGTCTACA
GAGTGAGTTCTAGGACAGCCAGGGCTACACAGAGAAACCCTGTCTCAAAAAACAAACAAACAAACAAACAAACAAACAAACAGCTAT
CCCACATAATAATTGTTAACAAAATAAATAAAATAGATTATTCTACTCACAATTATGTCAGGACCAATAGGCAGAGGTAACATGTGC
ACATGTACACAGAAGTTATCTGATGTGGATACTGGGAACCAAACTATGATCTCTGCAAATGTTCAACTGCTAAGCCACCTCTCTAGC
TCCCAAGATGAGACTACAGACAGATATTCCTTATGTGAGTAAAATGGCCATGCATAGAATTAATCAGGAAGGTGAGCCCAAGTTTTA
AGACAGAGGACAGATGTGAAATGAAGGAAGGTGACAGACTTTTATCCTGATTTTAGTTAGCTTATGAGTCTGAGTGAGGGGCCAG
TGCTTTTCACCACAAGGAATTATGAAGAACTGCAACTCCTTCCTTTACCATGTTTCTAAGCCCAGTTGGCTCTTTTCTCCTTTCTAT
AGACTAGAGAGTTTGGAGATACAGTATTAACTCATAGTCTCGTCTTCCTCCAGGGTCTCTGCAACTTTCCCCCAGCCTTTTCTCATC
CCTCACTAGGTACCTGAGTCTCAAGTTTTCTCTTCTGTAACTGCATAATTTGTGTTAGAGCTCACCATGGGATTACCAGATAACTCA
TTTGGAACCAACACCACCCAATGGGTGTTGAGTTAAAGCACTTCCCATGGGGAGAGTGCAAAGCCTTCCAGGTGAACAAGGAGAGGT
TCAACAGAGAATTGGTTGGAATGCTGCTTGTTGTTGGGGCTGAAAAAAAAAAGGCCCAGGAAATGTTTTTAACTAGAGCAATGGA
AAGTTGAAAATAAAGAATAAAAGTGGAGGCCAAGTGAGTGGTTGCACAGAAACAAAAAAGAGCCAAGGAAAAAAAATAACGCCAGC
CTGGAACCAGCCATTGAATTGCATCAGCTATTTGGATGCGAACTGTTGCCCATAGTCTCATGTGTTTGGACACTTGGTCTCAAGCTG
GTGGTGCTGTTTTGTAAGGCTATAGATCCTCCAGGAGGTGAAGCTTTGCCTGAGAAACTATGGCCAGAGCTAAGAAGTTTAATGGAC
CAGCCTTGCTTCCTATCCATTCATTTGCTTTCTGATAGCAGACACAGTGTGATCAGCTATCCCTTTCTTGCTGAGATGGGCTGTGTC
TCTTCATAAGCTGTGAGCCAGAGTAAACTCTTCCTCCCTTAGCTGCCTCTTGTCAGGTATTTAGTGACAACAAGAAAAGATGATGT
ACTGTCCCCTGTACCACATCTGACAAGGGTGCTTAATGGGTTCAGCAGAGTTCAGTGTATTACACCCATAACCTTCTGTGGAAGCCT
GATATTTGGAGAACATATGATCACAGCTTCTTCTTCTTCTATACTTGGCTATTTACATGAAGAAAGAACATGGGAAGGATGGAGGAA
GGGGTGGCTTTTCATAATTGGGAGTGGGTAGTCTGCAAAGCATGAGTCTTTGCTTCAGGGCCGGGACATCTCTCTCCTCCCATCACTC
TAGGGACTCAGAGAAGATAGGAAGATGTCTGTCCCAGTAAACAACTGCTGCATGTCATTTTTTTCTTTGTGGTAGCTTATCATGTAG
```

```
CATCCTCAGCTTACCTGACCATGTGTGGTGATTGGATCCTTTAGGGAGATGATGGTAGTGGAGAACTATAGCTGTACTTCCACATAA
GTCATATATAAGGTACCATTATAGTGGTGCCAGGCCACTCACTGAACACTTGCCATCCTTGTTTCATGTATTGCTGTATTTACAGTG
TCTAAAATATTGCTTGACACATGGCAGATGAAGATATTGAGTATTGCTTGACTGAATGAATAATTGAAATTTGTTTCATGTCTAATT
TCTAATAATCACATAGCAATAGTATAACCTCTGTATTAGGAACCTGAAGTCTCAGCTCTCCGTTCCCCAGGCACTGTGACTACAGAA
CTTGAGTGTCAAAATTTGGCAGGCTGATCATTGGAAGGTAATCTCAGAACACAGCTGGAGTCCTGGAATTCTGAAGGTCACAAGCTG
TACCAGAGGCTTACACTACTATTGACAATGACACTGTGTTTTTAGCCACACTAAGTACTTAACAGTCTGTCCTCCCATAACACCAAG
CATATCTCTGCAAGAGCATTTTGGTAACGGACATACACTGTGCTGATGGGCAGAGTAGCTGGAGATTTCAGTGGCTCCTTCTGGAGT
GTGGACCTCAGATTAGGCTTTTTGAAGTGCAACAACCAACCCTCAGGGATGACTTTTAGCTCACTGGATGCAGAAAAAGATGTCATC
ATATACATATACATACATACACATACACATACACATACACATATACACATACATCACAAATATATATATCACAGATATATATATATA
TCACAAATATATATATAATATTTATATATTATTTATATAGTATTTGAGTGTGGACTTGGCATCAGTGTATGGTTTATACATGTGTGG
GTGAATTCAGGAGGCCATGGAAGGATGCTGTATATTCTCTTGTATTACAATTTTCTTTCCATCGAAACTCTCCAGGATGAAGCCTAT
GTACCCTCTAACTGATGTACTCTGGGTACTGTTTGTCAGGAAGGAGATAAAAAGTGAGGTGACTATGCCCCAGTCCTTCCTTTCAAC
AGGAGTCTGAGGGTTATACTAGACATAGCTTGAGCATATATAAGACCTCAAAACCCACCCCTACAGTGACACATTTCCTTCAACAAG
GCAATACCTACTTCAACAAGGTCAAGCATTCAAATACATGAGTCTGTGGGGGCCATTCCTATTCAAACCACCACAATATTCTTTTGG
GCTTTGCATTGGTTCTGTTTAGTTTTTGATGTTTTAGGTCCTTTTAAGAATCTGACAACAACAACAACAACAACAACAACAACAACA
ACAACAACAACAACAACACTACCTGACTATTTGACTGGGACTGGCAAGATGGCTCAGTGGATGAAGGAACTTACTCCATAAGCCT
GACCACCTGAGTTTTATCCACATGGTAAAAGGAGAGAGCTAACCCCCACAACGTAGCCTTTCATCTTCGTACATGTACTATGGGATA
CATGTACCAACATACATATACATACACATACAAATAAATAATGGAATTTTAAAAGTCTACGTGCCTCCATCTTAAAATAACAATAAT
ACACACACACACATGCACACACACACACGGAATGTTTAGATTTTAGTTCACAGGGGTTCAGAGTCCTATGGGATTCTTGAGTCTT
GAGATGGCAGCCTACACTGCAGGAGCTATCATTTCTTGGTCTCTCGTCTCCTATAGAACAATAAATTCCTGCTTAGAGGGGGATATG
AGGTCTCATAGGGCCAGTTGCTCATAGGATAGATACATCTTTCTGTTCTGTTGCATATAGTTAAGCATATCCTTGAAAAGTTACAGA
ATTTGGAATGAATTGTTTTCTGAACCAACTCTTACCTTCCTTAGGTAGCCTGGTTGTTAAATTCTCCCTCATACAGAGCCAGAAACA
ATGCCTTGTTCTTATTACTGGTCCTGTCTATCCAAGTGGACCAAAAACACATCCTCCTGCCGGCATGGCCTCTGCTCACCTCTCCCA
GAGTCTCATGTTTGGGGTCTTGGGTTCTGAAATCACTGGAAAGTGATCCTCCATACCATGTGAACATCTCTGTAGTACAAATATTTT
CCTAGATGCCATTGTCTGAGGAGAATCGAGATTTTTCTGAGAAGACATGTAGGGTGTACCAGGGTTAAATGAATCTGAGGAGGGCTG
TGTTAGAGTCTCTCTTCCTAGACAGCCCCATGTACGTGAAACCACTTAATGCCCTGATGGCACCTGAAATAAAGTTGTGTTATGGATT
TCTTGATATGAAGGTTTCAACTAAGATTTATGAATTTTTTAAAAAATAAATCCTTTAAGGGGGCTATTCACCCCAAGATTAATTTCC
CCTTGTTTTCTCTCCCTCCACAGCCTCTTGATTTTGAAACTCAACCAGTTAGCAGCATTGTGTTCCAAGCGGAAAATCCTGAGCCAC
TGGTGAAAGGCATAGAGTACAATGCTAGTTCTTTTGCCTCCTTCGAGCTGATTGTGACGGATGTGAATGAAGTGCCTGTGTTCCCCC
AGCGAATATTCCAAGCAAACGTCAGCGAGGATGCTGCTGTGGGCAGTAGAGTGGGCAACGTGACTGCCAGGGATCCTGAAGGCCTGA
CTGTGAGGTACAGGGCAGCAGGGCTGGACAGGCCTACTTGCATGCTGCAACCATAACCAGAAGGAGGGCTGGCAAGGCAGAGAAGCA
GCGTGCCCTGACTTCAGGACTGGGGCAATGAAGCAGACAGCAGAAGCAGGGTCTCTGGCCTTGGTTCAGTGTGTCAGCACAACATGGCT
TCAGGTGCCAATTCTGTCTCCTTGCTGCAAGCTTCATTTGAGATCTGGTGAAGGTAGTAGGTGGGAACCCCACTCCCTGAGATTGGG
TGAGCCATGGCTAGGCCCTAGGTTTGAACTATTGGGGTTCTAGAGAAGCTTCCTGGGGAAAAAAGACTGGAGTTGTAGAGAGAGAAA
CACCCAGAAGTAGAGAAAGCCACCCTAGAGGCCAAAGGGAATGTTTCAGAAGTAGGGATTCTGGGCTTCTCCTCTCTGGAGAGCT
AGTCTGGGGCTAGTCTTCCCCCTCATTCCCAGAGGGTTAGTGCTCAAGGTGAGATGCTAAGACAGCATTTCTGTTTTAGCCATTCTC
ATTCCTTCTTTTTCGATGCTACTGTCCCCACACATATTTGCCTCAAAAATGTACAATGTACATGATTAATTCATTTACTATATACACATATAC
ACATTTGTATAATACCTAAATGATTTTAAAGATCACATAGTTAGTAAACATAGATAGGACTTGAACTCAGACCTTTTGTTTGTCTTA
TACAATATTCTCCAGAATATAAACAAATAATTCCACATGACTTAGCTGGTACAAGTATTTCCAAGTCTTTAAGTTTCTCTGTATACT
TGAGTGAAGTTGTATCTCCTATCGAGCTGTCCTATAGACAGTTGGGGTTCAATTATATGGGCTTTACTGATTTTCTTGCATGAAATAT
ATTATTAGTTTGCTTACTATAAAAAGAGTGTGAAGCAGAGAAACTTTATAAAGAAAACCAGTTTATTCAGTTCATAGCTCTGGACAT
TTATGGGCATAGCACCAGTACAGGCTGGGTCTGTTGAAGGTCAGTGTGATTATTAACAACAGCAACACAAGCAAAGGGAAAAGAT
CACATTGTAGGCAGGAAGCTGTAGCCACTGCATTCTCACAACCCCTGCAAGGGAGTGTCCCTAAGTGATGCAAGACATCCCACTAGA
CCCTACCTTCAACGGTCCTGTATTCAGCAAATTTACCATCTGAGAAGGAGGCTTCTAACACGAGAACAGTTTTTGTGGGGGAGGGGG
AACACTCAAATCATGTCCATATCATACTGAGGAGTAATGTTCCCACTAGTGAGGATTATATTGAATTATTCAGTGTAGAAACAATGC
TATACATCCAGGTCACAAGTCATGATGGAATTCAGAGCCCATTACTATCTAACCAATGCCTCTGTATTCTCTGGGCGAATGCTATAA
AAATTTGCTGATTGTAATGAAAAGATAAAATAAATCAGTAAAATTACCTGGCCAGAGGTATGTGCACGTTAGTATGATAGAAACAGAAG
CATTTAAGGAAATTCCTCTGTTGTCTCAAAAACTGGTATAGAGCATAAAACCAGAGTTACCATCAGATGAGACGGGCAAGGGAGACT
GAGAAAGGGCATTCTGAGAAGAGGCATTCTATAATGTCTGTTTCTGGAAGCACGTGAATGAGGAGTAATCAAGGCAGCCAGGGGCAC
ATGACAGAGTAAGAGCTAACTTTATGGACTCTGACATTGTGGACTGGGAAATTTAACTCTCCAGCTGGGTAAACCTAGAGAGTCGAT
CAGCAGATTCAAATCCAACCCACCTTCACATATTATAGAGCTCATTGCAGGCCACAGATCTAGAAGCCACACCTTTCTGACTACTTC
CCCTGTGGTCCACAGCACTGCCATCTCTGTTGTGGACCACCTCCTTCCCACACTCCATATCTCATTTGAGTCAACTGTGCACTGCA
GGCAATGAGAGCCTTTAAGATGAAGTAAGCTGACTGTCCAGCTCCTCCTTCTGAGCTTGCCCTGGGATTCTTGCTTCGTCTCAGGGTTG
GATCCCAAGTACTTAGGAGCTCAATTTATCATTCACACAAAATCATCAATACAGTGCTTGACATATGCAACATGCTCAAAATATACA
TGGGAAAATTAATTTGACTAAATATAATTTTAATCATTCATATTCCTGGACATACAAACTAACAGAATTTTAGAAAATTGTAGGCAG
TCTTTTGAAGTCACACACACACACACACACACACAATTCCTTGACTCCTATTAAATATTTATTGAGGTGACATTTCCAGGACT
GGAAATAAGTGGCTTGCAACTATAGACATCTTTACAGACATATGTAGGCTTTACAAAAAGGCTAAATTTACTGGCTTTTGTCAGAC
TTGTTTCTCTCAAATTTATCTTTCCTACACCCACCAATGAATTCTGTTGAGGACACCATTTCAGCCAGGTCACCTTTCCACTCAGCA
TAGCTCGATGGCTACCTCTGGCTTGTATGATCTGGCTCTCCCAGTCTCTGTGGCCACATCTTTTCCACTTACTGCTGCCCAGACATA
TCCATGTACTGTGCACACACCAGGATGCTCCTGGCCTCTCTGCTGGTTTGTGTATCTTTGTGGCACCCTTCTTGTCGTGTCAACAT
CTTCAGAAAGGCTTTTCTAAGTCTAAGTACTGGATTATCTGTGTATCTAAAGGCTTCATTCTTCAATTCTTGTCTCACCCCTGTCTG
CTGGAGTTGATGCTCTTTTAGTGAAGTTTTGCCAGGCCACCAGCACCTGCCTATGTCTCCAACATTACTTGCTCAGGTTTCCTTAGCAGG
GTGGCATACTGACAGCTTTCAGAAGACATATACTTCAATATGAAATCTTGATTGCCAGGAAAGCATAACTTTTCTCCCAGAGCTGGT
CACTTCGCCATACATAATAAAACAAGCATAGGACCATGTTCTAAGGCAGTGGTTCTCAGCCTTCCTAATGCTGCCACTCTTTAATTT
AGTTCCACGCGATGTATGACCCTCAACCATACAATTATTTTCATTGCTACTTCATAACTGTAATTTTGCTACTGATATGAATCATAA
CATAAATATCTGTGTTTTCAGATGGTCTTAGGTAACCCTGTGAAAGAATTCTTTGATCCCCAAAGGGGTCACAACCCATAACTTGAG
AACCACTGTTGTAAAGACAGTCTCTTTTCCAAAGAGAGATGTTGATTAGATGTTGATTCTGTGATTCTGCGATTCTGCTCCATAG
GACACCCAAATGATGATTCTATGTCGGTGTATAGAAGCACAAAATGTTTTCATGTCAAGTAACACAGGTTTTTTTTTTCTGCTCTCCC
CTCATGAAGTTATTCACTGAAAGGCAATATGAGAGGCTGGCTTAAAATCGACTCTGTCACTGGTGAGATATTTAGTGCTGCTCCGCT
GGATAGGGAAACAGAAAGTGTGTATCGAGTACAAGTGGTGGCCACTGAAGTAGGTGAGCAAAACAAAGCACAACCAGATCACAGATT
ATCAGGGGTGGGGGGGACTTCAATAAAAATGAGTGGGAGGGCATGAGAAGGTTAAAAAATCGTTGCTGAGTATGACCATAAAGCCATGG
GAAGCAGGCACAAAAGGGAGGGGAGGGAGGGAGTGTGAATATGTGAGTGAGTGTGAGTGTATCACTTGTGTGGAGTGTGTATGTGTA
TGAGTGTGTATGTATGTCGAGTGTGAATGTGTGTGTGTACATGTGTGTGAGTGTGCTTGTGTTTGTGTATGTGTATAAGTGTGAAT
GTGTATGTGAATATGTGTGTGAGTGTGCAAGTGTGTATGAGAGTATGTATGAGTGTGTGAGTTTGTGTGAGTGTGTGTGTCTATGTG
TGTGAGTGTATATATGAGAGAGAGAGAGAGAGAGAGAGAGAAAGAGAGAGAGGGAGGGAGAGTTTGTGTTTTCCATCCCTCGTGGTT
GTGTTAGAGAACACTTTGAGCCAATCTGTGCTTCAACTAGGCAGAGTTTCCCCAAGGCTGGAGGGTTATCAAGGTTGTTATCTGCTC
```

```
TGGTTTTCTGGAGAAACTAGAGCCTCTTCTTGGAATGTTCTGGAGTCCCTAGCTAACCTCCCTCTCTACTTCCTCAAAGGGGATTGG
GGGCTTCTCCTAAACCAGACACCAATGGTCCTCAGAGACCCTTGGTGCTCATGCCTTTTTAAACACTTGTGCATTAATGAAACTGGG
AGTTTCATTTCCTTGGGGGTTCTGATTCAGCAGGTATCTGAACCCGAGTATCTGCCACTTCGATGAGGCAAGATAAACTTTGAAAGG
GAAGACAATTGCACAGATATGCCAGAATGTTTGGACCAGCTTTGCAAAGGCCTTGCATTCTATTTAATTCACTTGTGATACAAGTTG
TCTACTGGCTCTGAACACAGAGTTCCAGGAAAATTATCAGATTAAAATAAAGATAAACTCACAGGAATGATATGGTATCCAGGGATA
AGGCTGGTAATTAAAACACTGAGTGTCTCTTTAAATGGAATTTTCCATCTTGCAGTAGAAATTCTGTGGATCTTGTGCTGATCAAGGAC
TCTCATTAGCTAGTAATATATCTGCTTGACTTATAAAAAAAAAGATGAGAGCAGAACTAATTACTATCAAACAAATACAGTTTTAGA
CTAAAAAATAGTAAACTTTTAAAATACAAAGCCCCAAATTAGAGGCAGACACAGGGAACAGAGAAAGGAATCATAAAAATGTATGGG
AAAAAAATGGAGAAAACCAAAATGACCATAGTTGAGTTCAACTTTAGCAAAGTTGTTTATTATTGGAAGGGATGTACAAATAGATGA
AGCTTTGCTGAGACACGGACAGCTCAGTCTCTGCCGCCTGCTACCCATTCTGCTATCAGGATGTATATATTTTAACTGGTGATTTAA
AAGCTCCCAGCCAAGTCCAAGGGATGGATGCTGTGTCTTCTCCTTGATCCCATGACAATCAGCATTTCACTTTGCACAAGTTTCTAAG
TGGTTTTCATGTTTTAGAAGTCACATGGATTATGACTGGGAGCCAAAAACCTGTTCCATTTTCAGTTGTTGGCACCAAAATAATAAT
AATGTTAATAGACAAAACAATTTCAAGGACATCAGTGGACTCATCTTTGATTTAAGAAAAATCAGAGACATAAAAGTTGCTGTAAAG
AAAATGAAGAAAACAAAGCTTCCTGGAATCAAAGCAGGAGCCATAACAGTTTACAGGAACACAAGTAGTGACCAACCATCAAAGCAG
TGTCTGCGTGAGGTCAAGCAGGGACAGTCAGGGTGCGATTGTACCACACTGGACGCCTCCTGTGCACTTCTGTTATTGACCATGCTTT
GTGCACAAAGAAACCAAGGCATTGAGAAATTGATTTGCCCAAGAGTGCACAGTTACCATGGGTCATAATGAAGAGGACATGGTCTTC
ATCACTATAGCATGACAGGACATTTGTGCAACCATTTGCTGTTTTCTCTTTGTATGTGAGATATCCACAATGATCTTCACATAGCCA
TGTGCTTCTCAGCTGAGGAAATGAAGCTTAGAAGACTGAGTTAGGATGTAGAAGGTAGTGGAAAGTGATCAAGGGTGTGGCTCATAG
GGACCTCTCCACTGTGCACTAGCTCTGTGAACTTGGCTAGGTCACACAGCTTTTGAGTGTCATTCTTCTCCTGTGTAAAGTTCAAAG
TTCTTTTGAAGATATACACATACACACACACTCACCTGGTTTAGTATTATCATTTAAACAATTAAAACATTTTTTTTGTGTTTATTTGT
GTGTGTGTGCCCATGTGAGTGTGCGTGTGTGCATGCATGAGTGTGTGTGTATGTGTGTGTGTGTATACATAAGTGCATCAAAGAA
TAATGTATGGGAGTCAGTTCTCCTTCCTCCATGTGGCTTCCAGGGATCGAAGCCATGTTGCCAAGTTTGGTAGCAAGTGTCTTTACT
CACAGAGTCATCTTGTTGGTTCCATTTAAACATTTTAAGCAGTACTGAAATTAAGTCTAATTCTAAAAACTTCCTTCTATCACTTTA
ATAGACCCAACTACTTAAATATTGATATTTTTGAGGAAAGAGAAACATGATTCCACGTGAAGGATGATCATAATACTTAGAATATAA
TTCTCACCCTTCAATTTCTTAGGTGGGTCCTCTCTGAGTTCTACGGCAGATTTCCACCTGGTCCTCACAGATGTGAATGACAACCCC
CCTCGCCTAGCTAAGGACTACACAGGCTTGTTCTTCTTGCCATCCCCTCAGTGCCCCTGGGAGCCTCATCTTTGAGGTCACTGACGAT
GACCAGCAGTCACTTCGGAGGCCCAAGTTTTACATTCGCCCTTGGCAGAGAAGGCTTACAAAGTGACTGGGAAGTTTCAAAAATCAAT
GGTGAGTTATCAAATGTACAATGAGAAAAATCAATGGTGGATGAAGGAATTACTTTGTGTGTGTATGTGCTAAAAACTTGAAGCATT
TTCACTAAAATCAGGAATAAACCAGGAGTGCCCCACCCTCCCTGACACCTGTTCAGTATAATAACTTGTAACATTAGATAAAAAATA
ACTCTGCAGCCAGTCCCACAGCACCCAGAGGAAGTCCACTCACAGGCACTCTAACAATCCCAGGATCATAGGACCAGAGGTGAAGA
GGACAACATCTACCCCAACATTGGAGGTAACTGGGACCAGCCGGACACAGGCATCCAGGGAGCTCTGCCCACATGTGACACAGGTT
ACTTCCTCCCTGGGCTGGTGTCCTGAGCAGAATTTTGGTTCAAACTCCCCAGCCAGTTTCACAACACCCAGAGGAAGCTCCACTCCC
AAGGGCTCTGGCTCTCCCAGGATCATAGGATCAGAGGTGCCCTGAGCAGACCTTGGGTGTGAACTCCGGAGCCAGTACTACAACACC
CAGAAGTTTGACTCCCAGGCACTGTAACACCCCCAGGACCACAAGATCCCAGGATCCCAGGATCCTAGGAGCTTGATCACACTAGGA
TCTTAGGGTCCCAGAGGCAGCTTGACTCCCTGGAGCTCTGACGAACCTAGGATCTCAGGATAACAGGATCTCAGAATCACAGAGACA
GCTGAACTCTGAGGAGTTCTGACACAACAAGGATCACAGAGGACAGGATCCAGTCAGTATATGAGAGCAGGATAGCACTAGAGAT
AATCAGATGGCAGGAGGTAAGCATAAGCAATAAGCAACAGAAACAAAGGTTACTTGGCATTATCAGAACCTAATTCTCCCACCATA
GCAAGTCTATACCATCACACCAGAAAAGCAAGATTCGGATCTAAAATCACTTCTCATGTTGGTGATAGAAGGGCATAAATAACTCCC
TTAAAGACATACAGAAGAACACAGGTAAACAACTAGAAGCCCTTAAAGAGGAAACCAAAAAAAAAAAAAATCTCTTGAATTCTAGGA
AAACACAATCAAACAGCAAAGGAAATGAACAAAACCATTGAAGATCTAAAAATGGAAATAGAAAACAATAAAGACATCACAAAGAGA
GACAACTCTGGAGTTAGAAAACAGGAAGACGATCAGGAACCATAGATGCAAGCATCACCAACAGAATACAAGAGCTAGAAAAAGAG
AATCTCAGGTGCAGAAGACACCCATAGAAAACATTAACCAACAGTCAAAGAAAATACAAAAAGCAAAAAGCTCCTAACCCAAAACAT
CCAGGGAATCCAGGACACTATGAGAAGATGAAACCTAGGGATAATAGGTATAGAAGAAGGTTTCCAACTTAAAGGGCCAGTAAATAT
CTTCAACAAAATTATAGATGAAAACTTCCCTAACCTAAAGAAAGAGATGCCCATGAACATACAAGAAATCTACAGGACTACAAATAG
ACTGGGCCAGAAAAAAAATTCCTCTCATCACATAATAATCAAAATACCAAATTCACTAAACAAAGAAAGAATATTAAAACAGTAAAG
GAAAAGGGTCAAGTAACATATAAAGGCAGACCTATTAGAATTACAACCAGATTTCTCACCAGACATTATAAAATCCAGAAGACCCTGG
GCAGATGTCATACGACCCTAAGAGAACACAAATGCCAGCCCAGGCTACTATACCCAGCAAAACTCACATTACCATAGATGAAGAAC
CAAGATATTCTAATACAAAACCAAATTTACACAATATCTTCCCATAAATCCAGCTCTACAAAGGATAATAGATGGAAAACATCAACA
CAAGCAGGGAAACTACACCCTAGAAAAAGCAAAAAGTACTCAACTTTCAAAAAACCTGAAAGAAGATAGCCACACAAACATAATTC
TACCTCTAACAACAAAAATAACAGAAAGTAACAATCACTTTTCCTTAATATCTCTTAACATCAATGGGCTCAATTCCCCAATAAAAA
GACATAGACCAACAGACTGGCTACATAAACAGGACCCATTTTGCTGCATACAGGAAACCCACCTCAGGAACAAAGACAGACACTACC
TCAGAGTAAAAGGTTGGAAAACAATTTTCTAAGCAAATGGTCCCAAGAAACAAGCTGGAGTAGCCATTCTAATACTGAATAAAATCA
ACTTTCAACCAAAAGTCATCAAAAAGATGAGGAAGGACACTTCATACTGGTCAAAGGAAAAGTCTACCAAAATGAACTCTCAATTG
TGAACATGTATGCTCCAAATGCAAGGGCACCCACATTCATAAAAGAAACTTTACTACAGCTTAAACCACACATCTCACCCAACAATA
TAATAGTGAAAGACTTCAACACTCTCATCAATGGACAGATCATGGAAACAGAATCTAACCAGAGACACAGTGAAACTAAACAAAAGT
TATGAGCCAAAAGGATCTAACAGATATTTATAGAAATATTCATCTAACCAAAAGAATATACCTTCTTCTCAGCACCTCTCAGTAAC
TTCTCCAAAACTGACCATATACTTGATCCTAAAACATGACATACAAGACATACAAGAAGATTGAAATAATCCCATGCACCCTATTAG
ATTACCATAGACTAAGTCTGGTCTAGAATACCAACAAAAACGATGGAAAGCACACATACACATACAAGCTGAACAATGCTCTACTCA
ATGAAAACTTGGTCAAGGAAGAAATAAAGGAAGAAATTAAAGGCTTTTTAGAACTTAATGAAAATGAAGCACATCACACCAAAACT
TAGGGGACACAATGAAAGCAGTGGTAACAGGAAAACTCATAGCTCTGAGTGCCTCCAAAGAAAAACTGGAGAGAGCTTACCCCAGTA
GCTTGACAGCACACCTGAAAACTCCAGAACAAAAAGAGACAAAATACACCCAGGAGCAGTGGAAGGCAGAAAAATAATCAAACTCAGGG
CTGAAATTAACCAAGTAGCAACAAAAAGAACTATATAAATAATCAACAAAAGCAGGAGTGGGTTCGTGGTGGGCCCATGCCTTTATTC
CCAGCACTTGGGAGGCAGAGGCAGGCAGATTTCTGAGTTCGAGGCCAGCCTGGTCTACAGAGTGAGTTCCAGGCAGTCAGGGCTAC
ACAGAGAAACCTGTCTCAAAAAACAAAAAGAAAAAAAAAAAGAAAGAAACTCAACAAGTAGATAAACCCTTAGCCAAACTAACC
AGAGGGCACAGAGATAGTATCCAAATTAACAAAATCAGAAATAAGAAAGGAGACATAACAACTAAACATATCTTTAATTATTCTGTT
TGTTGAATATTAACATTTGAAAACATTAAAATCATGTTCTACACACATCATGGAAGTATTATTGACAATTTTTCGCACTGAGCTTGA
ATTAGCATTTTCTTAATGTGTAACTTCAAAGGGTTTTTGCTATTTTGAAATTTTTAAAATATACTTACTGATAAAATAATTTCTCTC
CTAGAGACACTGGTCTTTTCTAAGTAAACTTATAGTTAGACAATGTACACAGATATATAAAGCATTTTAAATACTCTCTCACTATGT
CAGGTGGTATCATATAAGGGCTTTTGAATGTATTTCTTGAGTCATTTTTAATATTTGTGTTCTTTTATTGTGCTTAATTCCAATAT
TTTGTATGTTTTGAAACAATTTAGTATTCCACATTAGATATAGGATCCTCAATTATGGATAGAATCAAATATTCATTAACAATATTT
TTAGGGTATGAAAGGATATGAATATAAAAGTTGTACAATTTTTTATGTATTATTTGATTCTCAAAATACTCAATATTATTAATATGT
TTGATGTATAAAATGCATTTAAATAATAAATATTTAAGGGAAAAACTAAAACAAAAACAAAACAAGGAGATACAGGAGATAGCA
AATAGACAAGGAAGAAGTTAAAAGGATGTGTTAGCCCATTTTTTTTGGTAAGACATGGAAAAATATGTTAAATATGTTAAATTAT
TCAAACTATCACCTATATCAGACTATTTTTCAATTTACTTAATTATATACAATGCAAAAATGAGCTTTATTTCTGCTGAGTGAAGGT
TCTAGAGTTGTATTATCACGTGATTCATTTTCTCCCTGTTCTGACAGGTACACATGCTAGACTCTCCACCAGGCACACACGCTTTGA
GGAACAAGTTTATAACATTCCAATCCGCATCAATGATGGGGGGCAGCCACCCATGGAAGGGACTGTCTTCTTACCAGGTAGGTACCG
```

```
TGTAAAGGTGATGTAGGATATAGGGTACCATGTGCACTTTAAGAAATGCTTCTTTAAGTTGGGATTTTGCAACTATTCACTTAAAAA
CGTAATCTATGGAGCCCTGTTCTGCTTTTTAGTTACTTTCTGCCAGTGTGTGGAAGGAAGCTGTTTCCGGCCAGCAGGCAGACAGGA
TGGGATACCCACTGTGGGCATGGCAGTTGGTATACTTCTGACCACATTTCTGGTCATTGGTAAGTATGACTTTTCAGAGCCAAGGTT
TCCTGCCCCCAGCCTCAGATGTGAGCTCATCATGCCATCTATAGGGGTGCTCTGGGGGTTTCTTGCCTTGGGTATCAACCAAGAATA
CAAGTCCCCAGTCAATCACATCAAAGTATCCTGGTTGAGAAATACTGTGTCAAAGCTCCCATCTTCCTGTCATTCCTTGCTTGGAT
TTTCATGCTGACATTTATACCAGAAGGCTCTTTATTAACCAGCCATTCTTTATTGTAGCAAATATCTAAGACAATCAACATTTTAAA
AAAGTTTCTATTTGGCTCATGGCTTCAGTAGTTTCAGAGGTCCACGATTGACTGGCCTGTTGCTTGGGGGTCCACAGGAAGATAGTA
CTTTATTGAGGAAAGCTGCTCACCTCCCTTTGGGAACAAATGAGAGAGGAAGCGGGAAGGGGCTCAGGTCCTACTGTTCCCTTGCAG
GGTACAGTCTTCAAACTGTCTAGGGGAGGTCGTCTGTTGATAATGCTGAATTGGGAACATTTGAAGGCCATTTAAGATTGTAACCAT
ATCAAACTTTACACACAAATTGCTGCATTTTGGGCCGAGTTACCAAGCTCTTATGAAGGAGAAAGAAATTAGTCTCTGTGACCTGCT
GTGTCGGGCACTGAGTTTATTTAGAACTGATATCATGATGGGCAAGGCCTGCATTGACCCTCTACCCGTGTTGGAATAGGGAGGGGTA
GGCTGTGAAATGAAGATTCTGTGACACCTGAGATGCTGCCGAGTTTCCCTTTGCGTTATTTTCATAACTCTGGCCTCTGCGTCTTTA
CAACCTGGAGTAATTTCACAGCATCATGGCTTTGACTCTTCCCCTAGCTGGAGAGCTGTCCTCTCTCATGCACGGAGAGCAGCTGGC
CTCCTTGAGAACGCTGTCTGCTGCATGTGAGCGGCCCTTCTTTTGAGAGGGACTTGCTTGTAGTCCAGACTGGCCTTGAACTCAGG
CAAGCTCCCAGCCTCAGAGCTAGATTTTGAGATACTGTTTACTAAATCCTTTGGCCTTTATGCCTTTTATTGTTTTTCCTACCCACC
TTGAGAAGATTGAGTTCATGCGCCACACTGAAATTTTTCACAGCCTCATTCCCATGATCCTACTCTAAGTAAAGAATGGGCAGT
TTTGAGGATATATAGAAATAGAATTGACAGGGCTATTTTTGTTTTGTACCCTTATTCTTTTCGAATGGGGGCATGTGAATAATCACA
AGATTGTCAGCAAGTGTCTGAATGTCTAGTCTTCTTGGCTATGGTGCCTTCTGTAAGCTTATTGATGAGCAATTTCCTTATAGTAAC
TCTACACTTTTCCTAGTGGGCTCTGACAGTTGAGATTAGTGCTACAAAGCACAGCGTAGTCCTGTTAGAGTTCAGAGAAGTAAGAGA
AACCAGAATGAGTAGGCAGGCCACTGAAGGTCCCTCAGGAGAGGCTGGTACTGCGGGGAAGGTCCCCAAGCTGCGCATCTGACAACT
GCTCTACTAGTAAGCTGCATGCTGAGCCCTTACTGTGAAGGTGTGCTTGTTGACCCTGAGTTTTCTCTTTTCAGGTATAATTTTAGCA
GGTAGATACAATGAAAGTAGACTCTAGGCAGGTGGGCAGGTCTAGATAACTCCCTAGTTATGATTAGACTTTTTTCTCTAGGGAAGA
AAAACTAGCATCAATGTTTGGGTATATACGTGGTTCAGACAGACTTATTTCCACATTCTTTTCCCTCCGAGGAATTATAGCACTGGC
TCCTCTATTCCCTATTGCCAGCCTCCAGGTTAGTTATAACTTCGGGCTTCCTATATACTCATAACAAATGACCTCTTTACTTATCCA
AGAGAGCCAATGCCCAGAGCTCTATACTCACTAGAACAACGAAGCACAGTTTAATCACACAAATGGTGGCTCACAAGTCATCCCCAC
TCCTACCCTATAAGTGACTGTGTTAGGACATCTGAAGACTGTGCTTGTTTGACCCTGAGTTTTCTCTTTTCAGGTATAATTTTAGCA
GTTGTCTTCATCCGCATGAGGAAAGATAAAGTTGAAAATCCGCAGTCCCCTGAGAATAAGCCTCTGAGAAGCTGAATTTGAAAAGAA
ATGGTCAAGTCTGTGCACCAAGCACTATATCAATGCCTCAGCTGTTTAATTTCATCCAAAATGTGAACTATAATTGGTAGAGTTTAT
GCATCATGGGCTATATCTGGTCCCCAGCTGTTTAATCTCATAATTTCATCCAAAATGTGAACTGTAATTTCTTTGATGGCATGCCCT
TCTTGTCCTGCAACATTTTATGCTACTTAGATATTTTATGTTCTGCAGACATTAGAGTTATTCTCCATTTCCCCATAACATTTTCCT
CCTTTACAAAAGCCTTAGCTTTATAACTTGAACCTTCCCCTCTGGGCAGGAAGGAGGAGCTGAGTACACACCCTGTGTTGTTGCTTCA
GAGGACGTAGCTGTTATCTATCACCCAAATTTCCTAGGTTCCACTTATCCTTTCTGTTTCAGCCTGTGCTCCTTCATCTCTTAGCATT
ACACTGAATTTAAAATGTGTCAAACAGAAGAACAAAGTGAAGTCCTGGTTGGGGGTTGGGGGGTAAGTGGGAGCCTTGTTCTTTAAC
ATGGGCAACAGCTTCTCTGTGCTGTTAATGTTGTTAATGGCGGTCCTCCAACTTGGCTATGGCACAGACGCTGATGAGGTTATCAAA
TTCAACTGCTCTATTTCAAGAAAGATCTACTTCCTACGGCACAGGAACTGGTGAGCTGTCTGGTTTACTCACTACCCACATGCTTTCA
TACCTGCTGTACACGTTTTATTTGTATATTGAAGTTTTGTTATATATTTATCATGTGGAGGAAACAAGAAAGTATAAAGTGGTAAAG
AAATC

MOUSE mRNA SEQUENCE : mR28-010.1 (Seq ID No: 56)
AGGGAGAGGCTAGAGGAGCAGAACAGGTTGAGGAAGTGTCTCTTAGGGTTCTTCAGCCACAGCAAGTGTCTCCACGGCTGAATGGAC
GACAAAAACTTCTAAGCAGCATTTCTTACTTACTGAAAGGAGTCCAAGTGAAGAGAGCAAGATGGTGTCTGCCCAGCTTCACTTCCT
GTGTCTTCTCACACTTTATTTATGGGATATGGCAAGAAGGGAAGTTCAGCGGTCCCATGACCCCATGACATTCTCCATTTT
TGAAGGCCAAGAACCGAGTCAAGTCATATTCCAGTTTAAGACCAACCCTCCAGCTGTGACTTTCGAGCTAACGGGAGAGACAGATGG
TATATTTAAGATAGAAAAGGATGGACTTCTGTATCACACAAGAGCCCTGGACAGAGAAACAAGAGCGGTTCATCATCTGCAGCTTGC
AGCCTTGGATTCTCATGGAGCTATAGTGGACGGTCCAGTCCCCATCACCATAGAAGTCAAGGACATCAATGACAACCGACCCACGTT
TCTCCAGTCAAAATATGAAGGCTCAGTGAGGCAGAACTCTCGCCCAGGAAAGCCTTTCATGTATGTCAATGCTACAGATCTGGATGA
TCCGGCTACTCCCAATGGCCAGCTTTTTTATCAAATTGTCATCCAACTTCCCCAAATCAACGATGTTATGTACTTTCAAATCGACAG
CAAAACAGGGGCAATATCGCTTACCCCAGAAGGATCCCAGGAATTGGATCCCAGTGAAGAATCCTTCCTACAACCTGGTGGTCTCGGT
GAAGGACATGGGGGGCCAGAGTGAGAATTCCTTCAGTGATACTACATATGTAGATATTTCTATTAGAGAGAATATCTGGAAAGCACC
AGAACCCGTGGAGATTAGAGAAAACTCGACTGATCCTCACCCCATCAAAATCACTCAGGTGCAGTGGAATGACCCAGGGGCCCAGTA
TTCCTTAGTCAACAAGGAGAAGCTGTCGCCGTTCCCATTCTCGATCGACCAAGAAGGAAATATTTATGTGACTCAGGCCTTGGACCG
GGAGGAAAAGAACTCACATGTTTTCTTTGCAACTGCCAACTGAGAATGGAAAACCACTTGCATATCCACTGGAAATTTATGTGAA
AGTTATTGACATTAATGACAACCCACCGACATGTCTGTCTCCAGTGACTGTATTTGAAGTCAGGAGAATGAACCATTGGGTAACAG
CATCGGGATCTTTGAGGCCCATGATATGGACGAAGCTAACAACATCAACAGTATTTTGAAATACAAGCTTGTAGACCAAACACCCAA
AGTTCCCTCAGATGGACTTTTTCTCATTGGTGAATATGAGGGAAAGGTTCAGTTAAGTAAACAGTCCTTGAAGAAGCAAGACAGTCC
TCAGTACAACTTAAGTATCGAGGTGTCTGACGTAGATTTCAAGACTCTCTGTTATATTCAAGTCAACGTTATTGATATCAATGATCA
GATTCCCATCTTTGAAACATCAAATTATGGAAGCAAGACTCTGTGAAGCACCCATCGGATCCAACATCCTAATCATCCAAGC
TACAGATGCTGATGAGCCCTTTACAGGGAGCTCTAAAATCCTGTACAAGATTGTACAGGGAGACACGAGGGAAGACTAGAAGTTGT
CACAGATCCCACGACCAATGCAGGATATGTCAAGATCAAAAAGCCTCTTGATTTTGAAACTCAACCAGTTAGCAGCATTGTGTTCCA
AGCGGAAATCCTGAGCCACTGGTGAAAGGCATAGAGTACAATGCTAGTTCTTTTGCCTCCTTCGAGCTGATTGTGACGGATGTGAA
TGAAGTGCCTGTGTTCCCCCAGCGAATATTCCAAGCAAACGTCAGCGAGGATGCTGCTGTGGGCAGTAGAGTGGGCAACGTGACTGC
CAGGGATCCTGAAGGCCTGACTGTGAGTTATTCACTGAAAGATGGCAATATGAAGGTGGCTGGCATATCGACTCTGTCTCACTGGTGAGAT
ATTTAGTGCTGCTCCGCTGGGATAGGGAAACAGAAAGTGTGTATCGAGTACAAGTGGTGGCCACTGAAGTAGGTGGGTCCTCTCTGAG
TTCTACGGCAGATTTCCACCTGGTCCTCACAGATGTGAATGACAACCCCCTCGCCTAGCTAAGGACTACACAGGCTTGTTCTTCTG
CCATCCCCTCAGTGCCCCTGGGAGCCTCATCTTTGAGGTCACTGACGATGACCAGCAGTCACTTCGGAGGCCCAAGTTTACATTCGC
GGATGAAGGAATTACTTTGTGTGTGTATGTGCTAAAAACTTGGATGTGCATTTTCACTAAAATCAGGAATAAACCAGGAGTGCCCCACCC
TCCCTGACACCTGTTCAGTATAATAACTTGTAACATTAGATAAAAAATAACTCTGCAGCCAGTCCCACAGCACCCAGAGGAAGCTCC
ACTCACAGGCACTCTAACAATCCCAGGATCATAGGACCAGAGGTGAAGAGGACACAACATCTACCCCAACATTGGAGGTAACTGGGA
CCAGCCGGACACAGGCATCCAGGAGCTCTGCCCACATGTGACACAGGTTACTTCCTCCCTGGGCTGTGTCCTGAGCAGAATTTTGG
GCCCTGAGCGGACCTTGGGTGTGAACTCCGGAGCCAGTACTACACCACCGAAGTTTGACTCCCCAGGCACTGTAACACCCCCAGGA
CCACAAGATCCCAGGATCCCAGGATCCTAGGAGCTTGATCACACTAGGATCTTAGGGTCCCAGAGGCAGCTTGACTCCCCTGGAGCTC
TGACGAACCTAGGATCTCAGGATAACAGGATCTCAGAATCACAGAGACAGCTGAACTCTGAGGAGTTCTGACACAACAAGGATCACA
AGAGGACAGGATCCAGTCAGATATATGAGAGCAGATAGCACTAGAGATAATCAGATGGCAGGAGGTAAGCATAAGACAATAAGCAA
CAGAAACAAAGGTTACTTGGCATTATCAGAACCTAATTCTCCCACCATAGCAAGTCTATACCATCACACCAGAAAAGCAAGATTCGG
```

```
ATCTAAAATCACTTCTCATGTTGGTGATAGAAGGGCATAAATAACTCCCTTAAAGACATACAGAAGAACACAGGTAAACAACTAGAA
GCCCTTAAAGAGGAAACC
```

MOUSE PROTEIN SEQUENCE : mP28-010.1 (Seq ID No: 57)

```
MVSAQLHFLCLLTLYLTCGYGEEGKFSGPLKPMTFSIFEGQEPSQVIFQFKTNPPAVTFELTGETDGIFKIEKDGLLYHTRALDRET
RAVHHLQLAALDSHGAIVDGPVPITIEVKDINDNRPTFLQSKYEGSVRQNSRPGKPFMYVNATDLDDPATPNGQLFYQIVIQLPQIN
DVMYFQIDSKTGAISLTPEGSQELDPVKNPSYNLVVSVKDMGGQSENSFSDTTYVDISIRENIWKAPEPVEIRENSTDPHPIKITQV
QWNDPGAQYSLVNKEKLSPFPFSIDQEGNIYVTQALDREEKNSHVFFATAKDENGKPLAYPLEIYVKVIDINDNPPTCLSPVTVFEV
QENEPLGNSIGIFEAHDMDEANNINSILKYKLVDQTPKVPSDGLFLIGEYEGKVQLSKQSLKKQDSPQYNLSIEVSDVDFKTLCYIQ
VNVIDINDQIPIFETSNYGSKTLSEDTAIGSTILIIQATDADEPFTGSSKILYKIVQGDTEGRLEVVTDPTTNAGYVKIKKPLDFET
QPVSSIVFQAENPEPLVKGIEYNASSFASFELIVTDVNEVPVFPQRIFQANVSEDAAVGSRVGNVTARDPEGLTVSYSLKGNMRGWL
KIDSVTGEIFSAAPLDRETESVYRVQVVATEVGGSSLSSTADFHLVLTDVNDNPPRLAKDYTGLFFCHPLSAPGSLIFEVTDDDQQS
LRRPKFTFALGREGLQSDWEVSKINGELSNVQ*
```

MOUSE PANTHER CLASSIFICATIONS
        FAMILY (SUBFAMILY)
                CADHERIN(LIVER-INTESTINE-CADHERIN)
        BIOLOGICAL PROCESS
                Signal transduction(2.11.00.00.00) > Cell communication(2.11.03.00.00) >
Cell adhesion-mediated signaling(2.11.03.01.00)
                Cell adhesion(2.29.00.00.00)
        MOLECULAR FUNCTIONS
                Cell adhesion molecule(1.05.00.00.00) > Cadherin(1.05.02.00.00)

MOUSE GENE ONTOLOGY
        BIOLOGICAL PROCESS
                cell adhesion > homophilic cell adhesion
        MOLECULAR FUNCTION
                ligand binding or carrier > calcium binding
                cell adhesion > calcium-dependent cell adhesion
        CELL COMPONENT
                cell > membrane fraction
MOUSE PROTEIN DOMAINS (INTERPRO SIGNATURES)
                IPR002126 (CADHERIN)
                IPR002126 (CA)
                IPR002126 (cadherin)
                IPR002126 (CADHERIN 2 5)

MOUSE mRNA SEQUENCE : mR28-010.2 (Seq ID No: 58)

```
AGGGAGAGGCTAGAGGAGCAGAACAGGTTGAGGAAGTGTCTCTTAGGGTTCTTCAGCCACAGCAAGTGTCTCCACGGCTGAATGGAC
GACAAAAACTTCTAAGCAGCATTTCTTACTTACTGAAAGGAGTCCAAGTGAAGAGAGCAAGATGGTGTCTGCCCAGCTTCACTTCCT
GTGTCTTCTCACACTTTATTTGACCTGTGGATATGGCGAAGAAGGGAAGTTCAGCGGTCCCCTGAAGCCCATGACATTCTCCATTTTT
TGAAGGCCAAGAACCGAGTCAAGTCATATTCCAGTTTAAGACCAACCCTCCAGCTGTGACTTTCGAGCTAACGGGAGAGACAGATGG
TATATTTAAGATAGAAAAGGATGGACTTCTGTATCACACAAGAGCCCTGGACAGAGAAACAAGAGCGGTTCATCATCTGCAGCTTGC
AGCCTTGGATTCTCATGGAGCTATAGTGGACGGTCCAGTCCCCATCACCATAGAAGTCAAGGACATCAATGACAACCGACCCACGTT
TCTCCAGTCAAAATATGAAGGCTCAGTGAGGCAGAACTCTCGCCCAGGAAAGCCTTTCATGTATGTCAATGCTACAGATCTGGATGA
TCCGGCTACTCCCAATGGCCAGCTTTTTTATCAAATTGTCATCCAACTTCCCCAAATCAACGATGTTATGTACTTTCAAATCGACAG
CAAAACAGGGGCAATATCGCTTACCCCAGAAGGATCCCAGGAATTGGATCCAGTGAAGAATCCTTCCTACAACCTGGTGGTCTCGGT
GAAGGACATGGGGGGCCAGAGTGAGAATTCCTTCAGTGATACTACATATGTAGATATTTCTATTAGAGAGAATATCTGGAAAGCACC
AGAACCCGTGGAGATTAGAGAAAACTCGACTGATCCTCACCCCATCAAAATCACTCAGGTGCAGTGGAATGACCCAGGGGCCCAGTA
TTCCTTAGTCAACAAGGAGAAGCTGTCGCCGTTCCCATTCTCGATCGACCAAGAAGGAAATATTTATGTGACTCAGGCCTTGGACCG
GGAGGAAAAGAACTCACATGTTTTCTTTGCAACTGCCAAGGATGAGAATGGAAAACCACTTGCATATCCACTGGAAATTTATGTGAA
AGTTATTGACATTAATGACAACCCACCGACATGTCTGTCTCCAGTGACTGTATTTGAAGTCCAGGAGAATGAACCATTGGGTAACAG
CATCGGGATCTTTGAGGCCCATGATATGGACGAAGCTAACAACATCAACAGTATTTTGAAATACAAGCTTGTAGACCAAACACCCAA
AGTTCCCTCAGATGGACTTTTTCTCATTGGTGAATATGAGGGAAAGGTTCAGTTAAGTAAACAGTCCTTGAAGAAGCAAGACAGTCC
TCAGTACAACTTAAGTATCGAGGTGTCTGACGTAGATTTCAAGACTCTCTGTTATATTCAAGTCAACGTTATTGATATCAATGATCA
GATTCCCATCTTTGAAACATCAAATTATGGAAGCAAGACTCTGTCTGAAGACACCGCCATCGGATCCACCATCCTAATCATCCAAGC
TACAGATGCTGATGAGCCCTTTACAGGGAGCTCTAAAATCCTGTACAAGATTGTACAGGGAGACACGGAGGGAAGACTAGAAGTTGT
CACAGATCCCACGACCAATGCAGGATATGTCAAGATCAAAAAGCCTCTTGATTTTGAAACTCAACCAGTTAGCAGCATTGTGTTCCA
AGCGGAAAATCCTGAGCCACTGGTGAAAGGCATAGAGTACAATGCTAGTTCTTTTGCCTCCTTCGAGCTGATTGTGACGGATGTGAA
TGAAGTGCCTGTGTTCCCCCAGCGAATATTCCAAGCAAACGTCAGCGAGGACTGCTGCTGGGCAGTAGAGTGGGCAACGTGACTGC
CAGGGATCCTGAAGGCCTGACTGTGAGTTATTCACTGAAAGGCAATATGAGAGGCTGGCTTAAAATCGACTCTGTCACTGGTGAGAT
ATTTAGTGCTGCTCCGCTGGATAGGGAAACAGAAAGTGTGTATCGAGTACAAGTGGTGGCCACTGAAGTAGGTGGGTCCTCTCTGAG
TTCTACGGCAGATTTCCACCTGGTCCTCACAGATGTGAATGACAACCCCCCTCGCCTAGCTAAGGACTACACAGGCTTGTTCTTCTG
CCATCCCCTCAGTGCCCCTGGGAGCCTCATCTTTGAGGTCACTGACGATGACCAGCAGTCACTTCGGAGGCCCAAGTTTACATTCGC
CCTTGGCAGAGAAGGCTTACAAAGTGACTGGGAAGTTTCAAAAATCAATGGTACACATGCTAGACTCTCCACCAGGCACACACGCTT
TGAGGAACAAGTTTATAACATTCCAATCCGCATCAATGATGGGGGCAGCCACCCATGGAAGGGACTGTCTTCTTACCAGTTACTTT
CTGCCAGTGTGTGGAAGGAAGCTGTTTCCGGCCAGCAGGCAGACAGGATGGGATACCCACTGTGGGCATGGCAGTTGGTATACTTCT
GACCACATTTCTGGTCATTGGTATAATTTTAGCAGTTGTCTTCATCCGCATGAGGAAAGATAAAGTTGAAAATCCGCAGTCCCCTGA
GAATAAGCCTCTGAGAAGCTGAATTTGAAAAGAAATGGTCAAGTCTGTGCACCAAGCACTATATCAATGCCTCAGCTGTTTAATTTC
ATCCAAAATGTGAACTATAATTGGTAGAGTTTATGCATCATGGGCTATATCTGGTCCCCAGCTGTTTAATCTCATAATTTCATCCAA
AATGTGAACTGTAATTTCTTTGATGGCATGCCCTTCTTGTCCTGCAACATTTTATGCTACTTAGATATTTTATGTTCTGCAGACATT
AGAGTTATTCTCCATTTCCCCATAACATTTTCCTCCTTTACAAAAGCCTTAGCTTTATAACTTGAACCTTCCCCTCTGGGCAGGAAG
AGGAGCTGAGTACACACCCTGTGTTGTTGCTTCAGAGGACGTAGCTGTTATCTATCACCAAATTTCCTAGGTTCCACTTATCCTTTC
TGTTTCAGCCTGTGCTCCTTCATCTCTTAGCATTACACTGAATTTAAAATGTGTCAAACAGAAGAACAAAGTGAAGTCCTGGTTGGG
GGTTGGGGGGTAAGTGGGAGCCTTGTTCTTTAACATGGGCAACAGCTTCTCTGTGCTGTTAATGTTGTTAATGGCGGTCCTCCAACT
```

```
TGGCTATGGCACAGACGCTGATGAGGTTATCAAATTCAACTGCTCTATTTCAAGAAAGATCTACTTCCTACGGCACAGGAACTGGTG
AGCTGTCTGGTTTACTCACTACCACATGCTTTCATACCTGCTGTACACGTTTTATTTGTATATTGAAGTTTTGTTATATATTTATCA
TGTGGAGGAAACAAGAAAGTATAAAGTGGTAAAGAAATCGGAAAAAAAATGTTGGTTTCAATT


MOUSE PROTEIN SEQUENCE : mP28-010.2 (Seq ID No: 59)
MVSAQLHFLCLLTLYLTCGYGEEGKFSGPLKPMTFSIFEGQEPSQVIFQFKTNPPAVTFELTGETDGIFKIEKDGLLYHTRALDRET
RAVHHLQLAALDSHGAIVDGPVPITIEVKDINDNRPTFLQSKYEGSVRQNSRPGKPFMYVNATDLDDPATPNGQLFYQIVIQLPQIN
DVMYFQIDSKTGAISLTPEGSQELDPVKNPSYNLVVSVKDMGGQSENSFSDTTYVDISIRENIWKAPEPVEIRENSTDPHPIKITQV
QWNDPGAQYSLVNKEKLSPFPFSIDQEGNIYVTQALDREEKNSHVFFATAKDENGKPLAYPLEIYVKVIDINDNPPTCLSPVTVFEV
QENEPLGNSIGIFEAHDMDEANNINSILKYKLVDQTPKVPSDGLFLIGEYEGKVQLSKQSLKKQDSPQYNLSIEVSDVDFKTLCYIQ
VNVIDINDQIPIFETSNYGSKTLSEDTAIGSTILIIQATDADEPFTGSSKILYKIVQGDTEGRLEVVTDPTTNAGYVKIKKPLDFET
QPVSSIVFQAENPEPLVKGIEYNASSFASFELIVTDVNEVPVFPQRIFQANVSEDAAVGSRVGNVTARDPEGLTVSYSLKGNMRGWL
KIDSVTGEIFSAAPLDRETESVYRVQVVATEVGGSSLSSTADFHLVLTDVNDNPPRLAKDYTGLFFCHPLSAPGSLIFEVTDDDQQS
LRRPKFTPALGREGLQSDWEVSKINGTHARLSTRHTRFEEQVYNIPIRINDGGQPPMEGTVFLPVTFCQCVEGSCFRPAGRQDGIPT
VGMAVGILLTTPLVIGIILAVVFIRMRKDKVENPQSPENKPLRS*


MOUSE PANTHER CLASSIFICATIONS
        FAMILY (SUBFAMILY)
                CADHERIN(LIVER-INTESTINE-CADHERIN)
        BIOLOGICAL PROCESS
                Signal transduction(2.11.00.00.00) > Cell communication(2.11.03.00.00) >
Cell adhesion-mediated signaling(2.11.03.01.00)
                Cell adhesion(2.29.00.00.00)
        MOLECULAR FUNCTIONS
                Cell adhesion molecule(1.05.00.00.00) > Cadherin(1.05.02.00.00)


MOUSE GENE ONTOLOGY
        BIOLOGICAL PROCESS
                cell adhesion > homophilic cell adhesion
        MOLECULAR FUNCTION
                ligand binding or carrier > calcium binding
                cell adhesion > calcium-dependent cell adhesion
        CELL COMPONENT
                cell > membrane fraction
MOUSE PROTEIN DOMAINS (INTERPRO SIGNATURES)
                IPR002126 (CADHERIN)
                IPR002126 (CA)
                IPR002126 (cadherin)
                IPR002126 (CADHERIN 2 5)


HUMAN GENOMIC SEQUENCE : hD28-010 (Seq ID No: 60)
GGAAGAGGGAGTGTTCCCGGGGGAGATACTCCAGTCGTAGCAAGAGTCTCGACCACTGAATGGAAGAAAAGGACTTTTAACCACCAT
TTTGTGACTTACAGAAAGGTAAGGGCTGACATGTCTTAACTGTGTCAGTAACGTATTTATTCCAGAAGGACAAAGTAGATGGAGGGG
GAGGGCACTTAAAAAGTTGCTTGACAAATTTGTTCCTTGAGGTGGGCTATGCAGATGCGGAGAAGGGAAATTTGTTGCTGGCATGAC
AAGTTTTGCTTATGGAGCACAATTTTTTTTTTGTAGTTTCTTTTCATTGGTCCATATTTTATAAATTCATTTGATGAAGTGGCTGCAG
TGAAATCAAGCTTGGCAGTGGAATTAAAGGGGAAATTGAGTTCTCTGATGTTTAAACAAGTATCACATCCATTCTTTGTGATGAGTA
TATAAAAGTCCCTGGTCTTAAGATGCTCTAGGGCTCTTGTATTCTAGAAATTGGGGTTGGAATCACATTCTCAAAAAGCATGTCATT
CTATCTACTTCTCAGAGAATGAGATTCAATTTGGGATTCTTAGAATTGTCTAGGACACAGCAAGGGAGGGAACATGCAGAGAAGAAG
AAAAGTCTTTTTGTAAAAGTACTAGAATAAGAATTGAAGACAGACAGACCTGCCTTCACAAGTGAGAGAAGAGGCTGATGTGCGTGT
TAATCAGCTTCCTGGGCTGCTTTTGGCCTTGGTGGTGGGAGTTACCACCTGCTAATGTGTGGTTATTTTTATGTTAAGCCACACACAG
CTTATTGGTATCTTCAATGGTCCGATATTTGCCTCTGTCTTGTCTTGTCTTTATGACAGGTGACTTTATAGGGACAGATGCCCTCTGTGG
TAGGGGATGCCAAGCAGCAGATTGGAAGGACAGTGCCTTCTCTAATATTTTCTTGTCTTTATTTTTCTTCAAAAGCTTAAATAGCAA
ATGAGGCATTATTTGAAAGAAGAAGAAAAAAACACATGTTTAGATGAAAGGAAGATGAATGCTATTCAGCTCTCACCAGGAAACAAAG
CGAGTCCTTTACTGAGAGAGGGTTTCTAGTGAGTTACGGGGCCATATGAGTCTTCAGGGTTGGCCAAATGGGTTAGGCCAGTTTCAT
CATTTTTCCAACCTGTGGGCACAGAACAGAGAGCTGCCACTAGAGACTTGCCTAACTAGGTAGTTGTGAGAAGTAAATTAGATAAGTA
GGTAAAGAGCTTACTATGTTGCTTGGTGCATGGTACACATCCAGTGAATGTTAGCGCTCTGTTCCATGAGAAAGTTCAAGTAGGTGA
CTCAGGATGAATTGAGAAATTTTTGTTTCATTCTTTAGAAAAATAAGAAAAAAATCAGTGATGCCATAAAAAATAGCATCTACCATTA
TGCAAGAGAGGAACTAATTCTATGTTCAGGAAGTAGGATTCAAAAATATGAGAATAATAAAACTATTGGTTTTGCTATATATATAGC
ATTTTAAAGCCCCTCTCATGGTTCCCATATAAGTCTTTGGGTGAGGTGCAGGTGGGCATCCTTTGAAACTCACCCAGATGACCCCT
GAAAAAAGTCATCACTGTTCAAGGTGAAGGTTACATTTCCGGTACCTCCTCCTTTCTCATACAAAAGGCAAAATATGGCTGGGTGTG
GTGGGTCATTCCTGTAATTCCAGTACTTTGAGAAGGTGAGGAGGGAGAACCGCTTGTGCTGGAGTTCAAGACCAGCCTGGAAGGTCT
GAACATAGGGAGATCCAGACTGTACAAGAAAATTAAAAATTAGCCAGGCATGATGGCACTTACCTATAGTCCTAGCGACTTGGGAGG
CTGAGGTGAGAGGATTCCTTAAGCCCAGGAGGTTGAGGCTGCAGTGAGTCATGATTGTGCCACTGCATTCCAGCCTGGGTGACAGAG
TGAGAACCTGTCTCAAAAAAAAAAAAAAAAAAAAATCTGGATGATGTGCAAAATATATCCTGGCAGAAACTCTACTTCTCGTTCCATT
GTCAAAAAAAATGTCTAGAAGGATGTGTCCCTCATTTTTCTTCCACATTAACAGCAACAGCTCTGAGGAGGAAAGCTGGGCATTGTC
ATGCTCCTTGCTCTGGAGGCCGACAAACAGTGCTTGAACAGAAGCTGGTTCACATGGGTAGAGATGTATAGATTTGACATAAAATTG
CATTGATTCAGAAATCTCTGGTATCAGATTTAGAATGGGGCTAGTTGTTCTGTAACTGCTCGTTGATTATCCAAAGAAAGACACACG
GAATTTTAAGGGAATACCTATCTACTTATTTGTTACTTGTAAGAACAGACAGGAAGAAGAAGGAATGTTTACTTAGGTACAAGAAAT
ATTAACTTAATAAAAGCAGATATTCCCCTAGTGGCTGAGGTATTAACTACACTAAAGTTGATTTTTTTTTTTCACTTAAGCAATACG
TAACAAGCTAAGCTATGCTTGATTTTTGTGTATGTGGTACAAAGAAAATTTCTCTTCAAATATTCTGTAAGACTGACATGGAATTCT
CTCATTCATGATATTTCTGAGTTATTCATTTTTGACCATATACTTATTACTTAAAAATATTAGGACTTGTTCTTAAGTGACGGATGA
GGAGAATGGAAATGGTAGATTTGTTCCCTTAACATCTAAGCTAAAGATGAGTTCTCTTCCTCACCAAGAGGCCATCTAGCAGAGTGG
CTTGGTGCTTGGCCCCTGCATTCACACCACTTCCTCAAGCGCTCTAAGCATCAGTTTCCTGCTCGTGTGGGATAACAACAGGACCCAC
ATTATATGATTGTGTTGTTCAAGTAAGTTGTCCAAAGTGTTTAACTTAGCTTGCATTTGTTGAGCATTTGCTATTTTTTAAAACAAT
TCCAATGATTAAAAAAAAAAAACAATTCATGAATGAGATTTCATCGTGGTCTGGCACCTTTTATTAAGCAGAATTAAATTTATTTTCC
CTTCCTCTCCTACCCATCCCAGTTGTCAGTTACGATTTAATTACAGAAATTGGAAAGTGAACCTGCCATACTTAAGCATGACTGCAC
```

```
ATTCTTGGCATTAAAATTCCTGCCTTGGGTCACTTTGCAAATGAACTGGTATTAGAGTATTTTTCTTTCTCTTCTCTTCTCTTTTCT
TTCTTTCTTTCGATCCTTCAGTGTCTAAATCCTATGGGATGGAACCTAATTTAGATTTACTTCCTACTGAATTTCTAAGGCCAGGAT
GTGTTGCTTTATTATCTTGACAAAATGCTCATCCGTAAACTTTATATTACATAAAGAAATGTTGTGTAATACATAAAGAAACACTTT
GTCAAAAAAAAGTCAAGCCACCCTTTATGAGAAATAGTGATTCCAGTCCTCCTCTTTAGTCCAGGGAAGATGCTTGTGATATTGTTC
TGCTAGATCTGGGGGAGAAAGGGTCTCCCCCTGCCACCTTCTAGGGTTTTTGCGGGTATATAGCTGCACACGTGAGGGAAGTCAATG
CTGAAATTCTTCCTGGCTACCTGCTTAAAACCCATTTATGCCTAGTGTTCCATTATTGGAACACTAAGCATGTGGGAGTTATTTATA
TCCTTCTGCACAAGGTCATCGTCAAGGTCTGATTGCAAAAATTCAAAAAATTGCAACCTCAGGTATAAATGGGTTACAACATCAGGC
AAGTGCCCTGGTGGGACAGTCACTGTTGTCCCTAGGTCAGTGGCTGTCTCCCTTTCCTGTGACTGCTCTGGCCCCCCTTCCATTCAATGCT
GGGATGTTTTGGGAGACTTGGAGGAGAGGCCTCTTCTCCCCAGCTGATCCTTCCCTTGCTCTGTCTTGGGGAGCACTGGGGGAACAA
ACGCCAACCAGGAACAACATGACACCTCTCTCAAATATTTCTTGGAACTTGTTATTCTTTTCTTTAATAAGAATAATTATGCTAGTA
ATCCCATTACTGGGTATATACCCAAAGGATTATAAATCATTCTACTATAAAGACACATGTACACGTGTGTTTATTGCAGCACTATTT
ACAATAGCAAAGACTTGGAGCCAACCAAAATGCCCATCAATGATAGACTGGATTAAGAAAATGTGGCACATATACACCATGGAATAC
TATGCAGCCATAAAAAAGAATGATTTCATGTCCTTTTCAGGGACATGGATGAAGCTGGGAACCATCATTCTCAGCAAACTAACACAG
GAACAGAAAACCAAACTCTGCATGTTCTCACTCATAAGGGGGAGTTTAACAATGAGAACACATGACACAGGGGAACATCACACATCGG
GGCCTGTCCTGGGGTGGGAGGCAAGCGGAGGGAGAGTATTAGGACAAATCCCTAATGCATGTGGGGCTAAAACCTAAATGACGGGTT
GATGGGTGCAGCAAACCACCATGGCACGTCTATACCTATATAACAAACCTGCATGTTCTGCACATGTATCCCAGAACTTAAAGTAAA
ATTTTTAAAAAAAGAAAAAGTGAGACAAACATTTTTTAAAAAAAGAATAATTATGCTAGTAATGTGTATTATAATTGATTATATCAA
TCTAAACCATTTTAAGTTCTCCAGGGGACCATTTAGCCTCCCGTGAGCCCATCTTCCCTATCATGAACCTATTGGTAGAACAAATCA
TCACCTCTAATTTTGCATGTGTGTAAATCCAGATTTTCATATACTGAAAGATTGGCATATCTATATTACAGAGTTTAGTGACAGAAA
GCTGGGCTTATGTTTATGTATGTATGTTCATCCCTCCCTCCCTCCTTTCCTTCCTTCTTCCCTGTCTTCCTCTCTGCTCTAAAAACA
AATCATCCAGCTGCCCATTCCCTCCCCATGTGGAGTAATCTGTTTGAAGTGTGAGGCAGAAGCCCAGATTTATACCTGTCTGCTTGT
TTCTAGCTTATTAATGGCCACTTTAATTGGGTCCTTAATTTGTCAAAAGTGCAAATTGGTCAGATGCTTCTGCGGTTCTCTAAACCT
TGAGTGATGCAATTGGTGCCAGAGATCTGTGAGCCAGACCAAAAGATGGGCGTGGCAGATCTGAACATTAATCGATGAAAAATGGT
TTGGGCGAGATTTTCATCTGATCTTTATATCTATCTGGCCTTTGCTCCTTTATTTATGCCGCTCTTAGCAATATACAGTGAGGAGGAA
AAATAAATGGCTCACAGGATATTTGGCACTGTTTGGTCCTGGTGGTGTTATGAATTCCAGTCACATCCCTTCCTGCCCACACTACCT
TAGGATATTTGAAAACAAATTCTGTAGCTCTCTCCTTCTGCAACTTCTTTGTTCTTTGAAGCTTCATGGCTCAGCCTTAGTGCTAGG
TGAATTATTTTAATTCAAAGCCCTTCATGCTTTTTAAAAAATAAATAAATAAGAGATGACTGACTCTAAGATTTGTCTATCACTTAG
CTTTTTTCACGGGGGACAAAAATCAATGCTTATGTGTTGACTTGTAAATCTGGAGATCTGGGACGTAGAAACTCCTTGTCCTTACTC
AAATAAATGGGTAAATGACAAGAGCGTCTAAACATCACCTTCCCATCTTGCAAGGTGGTGGGTGAAAAGTTGAGAAGCCGGATACT
AAGGAGAAGAAACCTGAAGCTAACAAGGCTTTGCCAGTGGCAAGGTGAAAAGGGGAACCTCAGAGCTAAAAAGCCCAAGAAGGGGAA
GCCCAAAATCCTGTCCTCATTGGAGGAATTGGCAGATATTCCTGATCTGCTATGTATTCCAGAAAGGCCATGTACGAGAGGAGGTAC
TTAGCTGCTAAACCCAAGATTGAAAAGAATCTCGCAACCGTTACAAACCCAGTTGAGGTGACAAGAATGGTGATGCCTGGCTATTTA
AACTTCACAAAATGCCTTGATATTATCCAACAGAGATGTGCCTCAAAAGCTGTCCAGCCATCGCAAGAAACTCTGCAGTCAGCATGT
GAGAAAACTTGGAGCCAGCACGGCCCTCGAGACCATTCTGCTCATCTCCCTTGGTGCCACAGGCAGGAGGGTGGTTTTCCTGGA
GCAGCTGGGCAGTAGCTTGTTACTTGGCTGGACCTCTGGTCTTCCATGGAGTTCCTCTGTGAAGGACACACCAGAAATTTGTCAT
CACCACCTCCATAAAAATTGATATCAGGACTGGGTGCAGTGGCTCACACCTATAATCCCAGCACTCTGGGAGGTCAAGGTGGGTGGA
TCACTTGAGGCCAGGAGTTCGAGACCAACCTGGCCAACATGGCGAAACCCCCGTCTCCATTAAAAATACAAAAATTAGCCGGGTATG
GTGGCACACACCTGTAATCCCAGCTACTTGAGAGGCTGAGGCATGAGAATCACTTGAACCTGGGAGCTGGAGGTTGCAGTGAGCTGA
GATGGCGTCATTGCACTCCAGCCTGGGCATCAGAGCAAGACTGTCTCAAAAAAAAAAAAAAAATCGATACCAGCAGTGTGAGAACC
CAAAACATCTTACCAATGCCTACTTTAAGAAGCAGTTGTGGAAGCCTAGTCAGTAGGAGGGTGAGATCTTTGACAGAAAAAGAGAAA
TGTAAGACTACAAAGCAGTGCAAGGCTGATCAGAAACCATGGACTGGCAAATTTTACCAAAACTCAAAGCTATTCCTCAGCTCCAG
GGCTACCTGCAATCTGTGTGTGCCCTGGTGAATGGAATTTATCCTCACAAATTGGTGTTCTAAATTTCTTAAGAAGAACCTAATTAT
GTAACTAAAAAAAAATAAATGAATGCCGTTAATCCCCTTGGGAGCTTGGAGCCTGGTGGGAGAATCAAGGAAGCCTTGGGTGGGCTG
CTGGGATGCGGGGGGTCCCTAGGTGTCTAATGACTGAGCAGACCCCCGTGAGGCCACCTCTGTCCTGGGACAGCAGCACAGGCTA
AACAAAAGTTCCATGGGGTCTTCATTTGCTAGGGCTGCCTTAACAAAATACCACAGAACGGGAGGCTTAAACAGCAGATATTTACCA
TCTCAGTTCTGGTGACCAGAAGTCCAAAATCAAGAGGGCAGTAGGGTTGGTTCCTCCTGAGAGCTGTGAAGGAAGGATCTTCTGTGC
ACCTCTCTCCTTGGCTTGTAGATGGCTGTCTGTACATTCACACAGTGTTCTTTCTTTGTGGATGAGCCTGTCTCCAAATCTCTTCTT
AGAAGGACATCAGTCATTGTGGATTAGAGCCCACTCTAACAACCTCATTTTAACTTCGTTACCTCTGTAAAGACTCCAAATAAAGTC
ACATTCTGAGGTGCTTGGAAGTTAGGACTCCAACATATGCTTTTTGAGGGGGAGGGGAGCACAATTCTACCCATAGCATTTGGATTG
ATTTAAAAAAGGGGAAGATTCCTAAGGTGAAGCAACAGAAATGAGGAGAAATAGTGAGACACAGAGATACATTTGCAGTAGAAACAAAA
CTTCCCAGACCAAGGCAGCCTTTTCCTCCAGGCAGGTCGGGGCAGGTCATGTCAGCATCTGAAAAAAAGAGCAGCTGCAAAGTAGGG
GGAAAAAACCTTTTCAACATTTTGGAAAGATCTGTAGTGGATTAAATGATACTTGGTGTCTCATCATCAGGATATTTGTGTTTCAGA
AAGAGGTATATTATAATAGCATCTGCTCCATAGAGTTGTTCTGAGAAATCAGCAAGATAGTTTTTCACCACCAAGTCTTATCGGTGC
ATGCAGCAAATTGTTTTCAGTCAATGTTTGCTGCCTTTGTCGTTATCCGGGCTGGATGCAGATCCAGGCCGCGCGGGATTCCCATG
TCTTCTGTTGTGTGGTAGATTAAAAATGAGGCACTGTTCCTGCAGAGCAAGCCCAGCAGCCCTGGTTCCCGTCTGCAAGCAGGTGCCC
TGGTCTACTGTTGAGCAAGTGGTGGTCATGGTGGTGCCCATTGGGTGAGGGGCAGCCACAGTTTGGGAACCGAGGCCTTCTTTTCAA
AACTCCTTTTTTTGTAAAAAAAAAAAAAAAAAAAAAAAAAAAAAAAAACAAAGATAATAATAATAACCCTCTGATCTATGTTGCAAAG
GGTGTGCTTGGTCTCTCAAATAATTGCTGTAGTAACTGTCTGAATTTAGTCCTTGTATCTGAGGGAAATCCTGACAAACGGCAAAAA
TTCCACGCAGACGGTTGGCCAATTATTACAGGTAGTTCAGATAAAATGCCACAGAATCCTCTGGAAATCTATTTCAATTCTTTTCCA
AGTAGTGTTTTTGAGGGAAACCTAGGTGTCTGGCTTGGTGGTTTCGGGCAATTTCAGTGATGAAGGATGGCTTCCCATCAGCACCATT
GCGACCCAGGGTACCCCACACTTGAATGGTATTGTGTCCATGGAAGGAGGAAGTGTAGGTGGGACCTGTTTGAGAGACCAAAGTCCT
CTTTTGACATAATCCTAGCACTAGTGTAGTTTGCCTTGAATCATTCGGGTAAAGGCTTTGCCTTGCTGCCTCATTGGCTGTGAGGA
TGTTCCTGTGTAGGCTGTTTTTAGAATATTTTCATTAGTGGGCCACCAAAAGTTGGGAATTGTGAAGAGAAATGTCCTGTTGGACAA
TGTTTACAGTTTTGACTAAGCAAACGTGATGATATCCCTGGCAAAATGAATTTTTGCCAACAGTATTCCAAATATTAACTCGCTGAATCTCTTA
ACTCTCGAAGGTAGGTATTATTATTATTATCCCTGCTTTATAGATGAGTAGATAGGGCCACGAGTCAGCCGGTGGAGGAGTTAAGAC
CCAAGCCAGCCAGTCTAACTCCTGGGTCTATGCTCCTAACAACCGCATGGTGTGGTAGATTTGTCTTCAAATAAATCATGGCACCCT
ACGTGTGGCAATGGAAAGCAGTGACTTCTACTTCCCACTGGAAACCCTCGGTGAGGGCAGGCCTAAATGTAAATGACAAAATAAAG
GATAAGTACATTTCTCCTTCTACTTCCTAGGTTCCATTGAAACATCCCAGACAGTGATCTCTCTGAGCATCTGACCAAAATAAGGCA
TTCCTTTTATTTTTATTTGCTTTTCTATCATTTAACTTGTTTTTTCTGGAAGACTGAAGTGTTTAAAAAAAAAAAAAATTAGTTCAA
GAAGAGATGATCTAGTTCGACCAGTTCTGGGTTATTTACTGAGCAGAAATCTGTTAGTGTATTCAAAGGAGAAACAACTCTTTTCAT
CAACAATAAAAATGTATTACACAATAAAGCTTTGAATGTTGAATGAGTTTCCTTTAATATATCATGTTTATTGAAAGCTCTTACAGG
TGAGAACAAATTAATAAGATATCCATGTTAGGTAATCATAAATGTTTTAGAATTTACCCAACATCTGATGATGGCTTGATATATGCA
AGGGATAGTATGAAGAGACCTGATAAGTTAAAAATAAGGCCTCTGCCCTTAAGGATTTTATTTAATAGTGGCAGTAATGCTAACAGA
CAAATGATAAAAAAAAAAAATATATATATATATATATATATATATATATATATATATATATCAATCAGAACTGGCTCTGAGAGGAA
GAGACTGGCTGGAAATCAAGGAAGATTTCATGAAGGAAGTAGGGGTTTAGGTCCTAGCTCCATGAAGATAGACCCTGTACCCCTTATT
```

```
TTGGTACCCCAGTTCTTGGCACGGTATCTGGGACATACTAGAAGTGGTGTAGTTCATTGCTATTTAAATAATTAAGTTGAACTTAGA
TGGTAAGATGTGAGAAGCAGAAATGAGGGAAACATCTCCATCCAACCTCATTAAGCAAATATCCATTGTCCATTTTCTCTGGGCCAGG
CCTTGTGCCAGGCAGGGAGAGCAGCAGGGGTGAGAGCTTGGCTGCCAGAGAGCTCAGGGTGCAAAGTCCATCTGGGTGGAGGTGTGA
CTGGGTGTGGGAAGGGAGTGGCAGATAAGGCTGGAAGAGTTGGGAGCAGCATTTGAAAGATCTTGAATGCTGAGGAGTTTAAGGAGA
AAGATCTCCCAATGTGTCATTATTGCCTATATTTTTACTTGTATTAAGCAAATACAGGTTGATAGCTGACCGTGTACATTTTGCTAA
TATTACAGAAAACTAGTTTGTTAGACTTAATTTATTCATTTATAATTGATAATATCATCTTGCAATATGCTAGAATCTGAGGTTGTT
ACTTGTTTCTATGTGTTTCCAAACCAAATAAGTACTCTAAAGCAGTAATGTCTTGTTTAACTATGAGTTAATTCTAAAATGATGTTT
AGATATGTCAACCAAATTAATTATGTTGCAAAAGCTAATTTTTAAACATGCAAGGAATTTATGAATCTTGGAGTCCTGAAGGGTTTT
AATCCTAGCTTTTCCTTAAAATAGTTGTGGAATTTCAGGGCAAGTTGCTTAATTTATATAGCATGTATAAATATATATACATACATA
CATGATATTTGTATATCTCTACAGCTCTATCTCTGTCCATGCTCTATGTCATACATCTACATCTAGGTAGCTATCAAACTATATAAA
GCACAGTAGCAAGAGTTGAAATTCCAGAGTTTAAACTCTGGAATTGATAATCTGGTTGGAGGCAAGGTAATGTCACTTCCTAGCTGT
ATGTCTTTAGGCAAATTGCTTGACTTTTAACCTCAATTTTCTCACCTACAAAATGAGAAGAACAATTCCCAGCTCATTATATCTGAG
AAAATCATAGGAGCACAAACTTTTTCTTTTTTTCTTTTTTTTTTTTTTTTTTTTAGACAGAGTCTCGTTCCATCACCCAGGCTGGAGT
ACAATGGCCCAGTCTCAACTCACTGCAACCTCTGCCTTCCAGGTTCAAGCAATTCTCCTGCCTCAGCCTCCCAAGTAGCTGGGACTA
CAGGCACCTACTGCCACTTCTGGCTAATTTTTGTATTTTTAGTAGAGACGGGGTTTCACTATGTTGACCAGGCTTGTCTCGAACTGC
TGACCTTGTGATCTGCCTGCCTCAGCCTCCCAAAGTGCTGGGATTACAGGCGTGAGCCACCGTGCCTGGCCAAGAGGACAAACTTAT
TATTGAGCAATGGCTATGTGTTTTCCATGTGAGGAGACTAAGGCACCGAAAGATGAAGTAGTATATCCATAGCCACATAGCTATAGA
GAGCAGTGCTGCGTTGCTAGGCCAGCTTTCTGACTCCAAGCCTACCTGCCATACCACTCTGTTACTGTTTTTATTTTTAATTTTAAT
TTATTATTTATTTTTATTTTTTGAGACAGGGTCTCGCTCTGTCACCCAGGCTGCAGTGCAGTGGCGCAATCATGGCTGGCTGCAACC
TCTGCCTCCTGGGCTCAAGTGATCCTCCCACCTCAGCCTCCCAGGTGAAGTGGCATCATTGTCTGGGATAAATACCCGAGGTCATCA
ACTCAGGGTAGAGAAATAGAAAACCTGGGCAAACAAGAAGTGAGTTTAAGAGCAGAGGTTTAATAGGCAAAAGAAAGAAGAAAGCGGA
TTAGCTCTATCTCCTGCAGATAGAGGGGGGTGCCCGAGTGGGTCTTCCGGTTTTGTGGTGAAATGGCACAGGGTTTTATATATGAAC
TTGAGGAGGCGGTATCTGATTTACACAGGGCCGGAGAGATTGGTTGGACCAGGTGTGATGTTTGCATAGTGCTTGAAGAAGCTGGCC
TAATCTTTTATTATGCAGATGGATTCTCTACCTGGCTAGCACCATATAGTCTGTTGCTTACTGTACACGTGGTTACAAAGAAAAGGG
AAGATGGAGCCGCCGTGTTGAACATGCCCGGCCCCCAGATAGCCTTTTCCTATTGGCCGAGCTGCTGGCATTCACCTGTGGAAACTT
CCAGCTTGCTTATCTATGTCTGCAGCTGGATTCTACAGGCTGCTCTTTGTTAGAAATGATTTTGGGGCTGCTTTTTGTTAAAAGGAA
ACCTTACTGAGGACTCTCTTACCCTTACTATCTGCCTAAATAATTTATTTCCAGCTCCTGTATCACAAGTAGCTGGGATTACAGGCA
ATGAGTCACCCACCTGGCTAATTTTTGTGTTTTTAGTAGAGATGGGGGTCTCGCCATGTTGGCCAGGCTGGTCTCAAACTCCTGAGC
TCAAGCAACCCACCTGCCTCGGTCTCCCAAAGTGCTGGGCTTCTGGATGTGAACCATTGTTTCCCGCCTGTTACTGTTTTTAAAGAC
CTCTCTGCAAATCTACATCCTGGGTGATGCTTAATAAAGGATTGTTTCTTTATCTCCCAGAACATTGGTGAGGCTAAAATGAAATAG
CATTTTTACCCTTTAAAGTTCAAATATAAGTTATGCAAATGTAGGGCATGTAAAATTTTCAGGATCTAAATTTTTAGAGTGGTTAAA
ATGTTAGTGAAATTTTTTCCATGCTGGTGATATTAGTGATATGTGTTCAATGTAGAAAAACTTTAAAGAATAAGATGATGAAGTAAA
CCAAGATTATTCCTAATCTCTAGAGATAATAAAGTGATTGGAATTTTCGTGAAATAAATACATTGAAAAAATTTTCTTGACTCAGCC
AGGTGTGGTGGCTCACGCCTGTAATCCCAGCACTTTGGGAGGCCGACGCAGGCAGATCACGAGGTCGGGAGATTGAGACCATCGTGG
CTAACACGGCAAAACCCCATCTCTACTAAAAATACAAAAAATTAGCCAGGCGTGGTGGCAGGCGCCTGTAGTCCCAGCTACTTGGGA
GGCTGAGGCAGGAGAATGGTGTAAAACCTGGGAGGCGGAGCTTGCAATAAGCTGAGATCGTGCCACTGCACTCCAGCCTGGGCGACAG
AGCAGGACTCTGTCTCCAAAAAAAAAAAAAAAAAATTTCCTTGCATGTCTTTTTGGGGAGGGGCAGTCCAAACTTATGACAGGTAACAAC
ACTGTTTTCCAGGCCATGTGGAAAATTTCTCTCACATCTTAAAATCAATTAGTCATGACATGTCTCATAGTGAAAGTCACAATCTCT
GTGAAATGGGAAGCCTTAAGGAGGAAGGGAAAAATAATATCGGACAGGAGACAATAAGGACAGAGTTCAGATTTTTGTTTGATCTCT
TTTGGAATAGTAAGAAATTAATTTTTTCTTAGGTTTATTGTGTAGGTAATGACAATATCTAGAAGATCAAAGAGGCAGCTGGTGACA
TCTTTGAATGAAGAATTGTGAGCATTCTTTAGTTCTTTTCACTTGCGGAATCTCATATGTTTGTGGCTTTGAATCAAAATGTTTAGT
TATCCTGGAATCTGCCAAAATACATCTTCTCCATCTGTATAATAGGAGTGAAAGGAATACTCATTGCTGGCTGGGCATCTGTAATCC
CAGCACTTTGGGAGGCCGACGCAGGCAGATCACCTGAAGTCAGGAATTCGAAACTAGCTTGGTCACCATGGTGAAATCCTGTCTCTA
CTAAAAATACAAAAAATTAGCCAGGCGTGGTGATGGGCATTTGTAATCCCAGCTACTTGGGAAGCTAAGGTGGGAGAACTGCTTGAA
CCCAGGAGGTGGAGGTTGCAGTGAGATGAGATTGCACCACTGCACTACAGCCTGGATGACAAAGTGAGACTGCGTTTCCAAAAACCC
CAAAACAAACACTCATTGCCCATCATTTAAGACGCTGAAATGAAGAAGATCATAACAAAGCAAAGCAGTTAGATAAATGGAATTATT
GTGAGCATGTTTTGAGATGTTGGCTTGCATATTGCTATGGATGATGCTATGAAGCTTCTGGAAGACTGGGCTGCTCCATATAGAGG
AGAGGCTGGCCTTTTGGGATACCCATTTTTACTAGATTTATAACTGGATATCATCCATTGATTCTTACTGATTGGAAAGACATGATTTT
AACAGACTGGTAACTAACCATTTTTTTTATCTGGTTCCTTCACAGGAATTTGAATTAAAGAAAACTATGATACTTCAGGCCCATCTTCA
CTCCCTGTGTCTTCTTATGCTTTATTTGGTAAGAGAAGAGGGTGTTCTTCTCTATTTGTTTACTAGAATATTTTCTGCTATCTTTCT
ACCCCACACCAGGGAAAGAATGGCTATTATTACTTAAAAAAAAGATGACCTATCAAAATTAAGTTGTTGTATAGAATGGAAACATAGC
TTGTTCCACTGGATATTTTCGATGAAATGGCTACAAATCACATGTCTGCCCTCGACTAGGGAATGTATTTTTCTTTCCAGCTCTTAA
AAGCCAACACCAGTTATGGGTAATGTTGAAAACAAATCTATCTATAGCTCAGGGAATCTGAAATGACATTGCACTGATACATGAATT
AGTCTGTGTCCCTTAATCTTGACTTTCTTTTTTAAACTTTGCGCATGAAAACTTACCACCAAAACATCAAGAAAAGCATTTTCTCTA
TTTTCAGCCTTGCTCTGCTCTATACTTTTCTTCTCATCAAGGGTTTGGAATTTTCTGGTTGTCTTTAACCATTTGTTGGGTTAACCC
TATTGACTGACAGGCTTCTAGAGCTCGGAGCTGGATAGAAACTGGGGAGTCCTCACCCTTGACCTTGGGGTAAACAGTAGGTGCCTT
TTTTTAAAATCTACGTGACTGCTGAGGTTCATAAAGGTTGCCCCAAAATTGCTGCATTAAGAATTTGGTGAGTTACCTAAGCTTTG
AACCTCAGTTTTCTGCCCTGTGAAGTGGATCTAATAATAGTACTTGTCTTATGGGATTTTGTGGCAATTCAATGAGATACTGCATTT
ACAAAGCTCTTGGATCAGCGACTGGTGGTTAAACACACAATAAACATTAGCTACTATAATTACTACAGCATTTTAAGTGTTCAGTGT
GTACGTCCACATGGACTCTGACCTCCATGAAAAATGGACTGTGCTTTTATCATCTTTGCAGATTCAGCACTGAGCACACTGCTAAAT
TATTTAATAATAATATCTAATTATTAGGCCTTATTTGCTTCTTGTCTGAGAAGGGAACATGCATTTTTATTCCTGGATGAAAGGTGA
AAATAAGGTTTCCTTCTCTCTACTCTCACCCCAGTGGGATTTCTATCTAGAGTGAAAATACTGCCCAACCACTGCTGGCAGCAGTGT
CTTACCCAGGTGAAAGCCACAAGCACAGCTGTCTGGGTGAAGTCAGGGAGTGGGATTTCAGGGGTGTTGGGAGGGGAGCCACTTCT
CTCTCCTACCTCCTCCCAAGGCCTCTTCTATGAAGACTGGAAGTTTGCTTGAAGAATCTTGCCTTCTGCATAGGCGTTTATTCAACA
GAAGCACTAATTTACTCCAGACTGCGAGTAGATCTTCTGTTCTTTTCCAAGAGATAGATTTATCAGAGGGTTAGAAACACAAATACG
GCATTGATGTACTTCAGGGGCACCAAAGAGAGAGTAAATAAGTGATCCAGGGTTTGGGAAATGGTACTGAACAGGGAATCGGAGGAA
ACCAAGGCAGAGCCATCTGTTTTGGGATTATGCCTGGGCCTTCCAGAAGGAAGATGAGTTTGGTACAAATACTACATAATCACTGCA
CTGAGCTCTTGATGGAGCTACCAAGTAGGTGAACCTGGCAATTGCACATGTCCTTTGACCTGGAAGGCAGTAACAATGTGGTTAAGA
GTTGGGGGCTTGGTGGTCCAATGGCGCTGGGTCTGGTCTGAGGTAGCTCTGTCTCTGAATGCTTTGCTTCTACTGTGGGACTTCTCT
GGAGATACTTTCTTCATAGCATTGGTCAGAAGAAATGATGTCTTGTGGTGATCAAGTCCCATTACTGCCACATAAAAGATGCTAAAA
ATGACTTTAAGATGGCAGTTCCATTCTCTTCCTTTCACCTCTTTGTGCAAGATGGAAAACGCTATTCCCTCCAACTCCTGCATAAAG
```

```
CCGCCATCTTGTCCTTTCACAGATTTCCACCATACCATCTTTCCCACTTCTGGGGAAAGCGGCTTGATGATTCACAGTGTCCACATG
CACTTTGAAATCTTTATAGCCCCAATAGTCATGTTTATTGGCAGGAAGAGAGATTTGTTGCCCCAATACAAGCACGCAACTTTGAAC
CCTGGAACCAAGACTTGATTCTCATTTTCTGATGGGTGAGCCAGCTTGTCCTTGTTGGAATAGTGCAGCCTTGTTTGTGTGTGGCTA
ATGTAGTTTTCACTGGTGTGACTAACTGAAAGACTGCTTGCTGACATGAGTGCAAAGCTGATAAGCAGCAGCTCCACAGAAAGCACT
GACCTCACTTAAGCAACCATGGTGGTACCTGGACATCTGTCCTGCACACCCAGTTCTTATGCCCTGCCTTCTAGAGGAGCTGATGGA
ACAGGAAAAGATGTGGAAAAGGAGTGAAAGGGATGAAGACCTCCCAAAGGAAGATAAACTTAGAAAGGAGGAGATGGGCTAGAAGTC
TGGCAACATTACTTTGTGAGTTAAGGATGCATTTTCTTGGCCGGGCATGGTTGCTCATGCCTGTAATCCCAGCACTTTGGGAGGCTG
AGGCGGGTGGATCACCTGAGGTCAGGAGTTCGAGACCAGCCTGTCCAACATGGTGAAACCCCATTTCTACTAAAAATATAAAAAATT
AGTCGGGCATGGTGGGGGGGTGCCTGTAATGCCAGCTATTCAGGAGGCTGAGGCAGGCGAATCGCTTGAACCTGGGAGGCAGATGTT
GCAGGGAGCCAAGATCGTGCCACTGCACTCCAGCCTAGGCAACAAAAGTGAAACTCTGTCTCAAAAAAAAAAAAAAAAAAAAAGCATTT
TCTTGCAAGTAACAGGAAATTTGACTTATTTGACTTTAGAGCATTTTAAATAAATAGGGGTTTATTTTCCTCAGATAACAAGAAGTC
TGAAGTAGGCAATCCAGGGCAGGTTTAGCAATTCCATGATGTTGCAGAGACTTGGGCTACTTTTCTTTGCTACACCATTTTTTGAGC
TGGTTTGTCCTCTCAGGGTTCATCACAGCTGCCCCAACTGCAGACACAAGGTACATTGTCTCAGCAAGAGTCACAGGAAAGGGCAAT
GGCAGCCACAACTACCCTTTTTTTTTAAAATCAGGAATATGGAAGCTTGACCACTGCCAGTGGACTTCCCCTCTCATTTCATGAACCA
GAACTGGGCCACTTGGCCACCTTCCTAGCTGCAAGTGAGCCTGGGAAAGGGAACTTGGTTTACCGGTCTCTGTGGTGGGAGGAAGAA
AAGGAGAGGGCTTGGCAGTGGCCCAGGGGCAGCAAATCTAAGGCCCTACCACAAATCTGAGAACAGAAGACCAAACTTCAAAGCCCC
TGTGTTCTCATTCATTATTGCTTTTTTTGCCAGACATCCAGCATCCAACTTAGTCAAGGTTCCCCAGACTGAGGGATCTAGCATAAGA
CCTTTCTCTGATCACTGGCATATTGATGGCTTTTGGGTATTCCCAGCAGGGAGCAGTTTCATGGCAAGAGTGTGCTTGCTCTCCCT
AAATAACTGGAAAATAAATGGTTTTCCCCCAACCTAGTGGCACCTGGACATCTGTCCTGCATACCCAGTTCAGGGAGTGAGAAGACT
TTTTACTCTTTTATTTTTATTGTCTAGCTCCAAAAACCATTGGCTTGCCACATCATGCTTCTGATTTCAATCTCTTTCACCCCTGAT
TTCCACAAATGGGAAGACCCAAAGTCGTGCCCAGGAAGGAGGAAGGCCAGCGTGGGGATGAGGCACTAAATCCCTGGTCCTAGAGGG
TGCCCCAACGTGGCTGCATGACTGGCTGTCCCTTCCCGTGGGTGCCCACTCCATCTGCCTTCAGAGGTGCTGTTGTGGTGAGGGCTA
CAGTGATGCTCCCCATGTTCTGAGACTGGGAATGGTGGGAGTCACACTCTCATGCACATCTTCAGTGCAGCCTGCCAGTTTTCCCTC
CAGGTTGCCTTGGCTGTTTCACCCGCTCTGGTGGGTGCTGGTGCATAGAGGCTGAGAGGGCTGGTTCCAGAGTTGCAGGTTCAAATC
TTGCTTCCAGCCACTACCTGGGCAAATAATTTAACCTCTTTGGGTCTTAGTGTCATCACCTGTTAAACAAGGGTAACAATAGTAACT
TTCTGCCAGAGTTGTTGTGTCAGGATTAAATGTGCTTAGGACAGAGCTCCACATGTACTAACTTAGCAAGTGTGAGCCACTCTTCTTAC
CATCCCAGCCACATTCACTACTACTCCAGTCTTGACTGGGTGTCTGCAGAGCCTGCTTAGCTTTCAAGTTTAGGGTCTTTGGATTT
TGGAACCATTGTGGGGCTACTGCTTAAAGTAAACATTTTGTACTTTTATTTTTTTCTAAATGAATGTAAAGAGCTAATCAAATGTTA
AAATAGTAAGCTTTGCTTAAAGTTATAGTTCAAATTCAGATTCAAATTTGGCTAAAACTTACCGTGTAACTGTGCCAAGCAAACTTT
GTACTAAATACGTCTATATTTTCTTTCTCCCCCTTATAAATTCAAAACACTAGAGTGCTGGCAATCTGCTGTAAATGTCTTTATAAA
ATAAAAAAGACGTGGAGGGAGGTGTTTTATGAAGCTGGAGTAAAAACATATATAATTTAGAAGACAGTGTGGGATTGTGGAAGACCATG
GGCTTTAGTTCAGTTAGGCCTGAGCATGTGTGCTGTGTTAACCTTTTTAAGCTTCATTTGTAATATGGGGATGTTAACATTCTTCTC
ATAGAGTGATTCTTCTCATCTCACATTCTGCTCAGATGTTTAAGAAAAGCAATGGAAAGGTTTGTAACAAGCTACTTTGACGTGGTA
GAGCTTCAACAGCTATTACACTCTTTCCTGTTTTGATCAGATTTCAGCCATGATAGAATCCTATGTTTTGTATATTGGTGCTGGAAG
CCCTAATAAAATCAATGAGTGACACAGACACTTCATACAAGATGCTAATTTTCTCTTTCTTTTTTAACAGGCAACTGGATATGGCCA
AGAGGGGAAGTTTAGTGGACCCCTGAAACCCATGACATTTTCTATTTATGAAGGCCAAGAACCGAGTCAAATTATATTCAGGTAAA
AGCTACCCTTGATCACTGTCCTCTTGATTTTGTATGCTCTCTTGGTGGACTTTTACTTCCTTATCTATTGTATAGCAGGAAATAAAA
TAAAGGCATGGCATTCTCATTATCAAGAATGATTTCAGAAGCTAAGTTAAACAGAGAGATGAGTGGCCTCAGTATCATAAGAATTGT
ACCTGACATTCTATTTGGACTTCCTCAATCATCTCTGGGGCGAGTGCTCCCAGCCCTCTGCAGGGAGCTTGGTGGCTGCAGGCTCTG
CCTCTCCCCTAGAGTAGAAAGGGAACCTGTCTCTGGAGAGGGGAATGACCATCCACCCTCGAGGCTGTCTGATCAATGAGCTTGCTG
CTTCAAGTACTGGAAGATACGGCTTCTGGAGTCAGTGCTAGAATGAGCTTTCTGGCCAGAGGACTGCCCTTTTCCTTACCACCTTC
TCTGAGCCCCCATGTGGGCCATTGTCAAGGTGCACTGGGAGGTGGACGTTTTATTTAGTTAGATAACCATGAACAGTATCAGATGGA
GGGGCAGTCTGCAGGTGGGAATAAGGGAAGGGACCTAAGTCCCATCTTAAGTGCTATTAAAGACACAGAAATAATCCTGGAAATGTA
GTGGAGAAGGGGAAAACACTACTGTATTTACCAGACATCCCCTGAGAAAGTCACTTGGCCCTGAAGTGATTGGAGGATGCTGATGAG
CCTTCTTGGGGGTGCCCGTGACCCCTCCCCGTCCCCTGAACCAGGGAAGTGGCAGAGGTTGCTGAGCTACCTACCTTCTAAGGCAAG
TGCCTTCCATTAACTGCCAAAAATTTACAGACGGCCCTCTGTATCTGTGGGTTCATGCATCAGCAAATTCAACCAACAAAGAATCAA
ATATATTTGGGAAAAACAATAAAAAATGATACAAATTTAAAAATACAGTATAATAATCATTTACATAGCATTTACATGGTATTAGGTA
TTATAAATAATCTAGAGATGGTTTAATGTATATGGGAGAATGCGAGTAGGTTCCATGACCATAATACACCACTTTATATAAGGGCCT
TCAGTATCTGTGGATTTTGGTATCTGCGTGGGTCCAGGAATGAGCCCCCACCAATGCCAAGGGACAACTGTATTGTGCTGGACAGTT
CCCCTCCACAGAGGAGAGGCTGAAAGCTATCTCTCTTCCCCTTTCTTTCATTGGTACCAGCTTAAGGCAAAGGGCCCCAGAGTAAGA
GCTGGCAGGGGCATGCTTGCATGTCTATGCAGCCCGGTGAGGTGAGGTGTTATTAGGGGCCTCCACCTCTCCAGTCAGGGAAGTCAG
GGCCTGAAGAAGCAGTGAATGTGTTGAGAGCTGGAGGTTGATGGGAATTAACTTGGTGAGGAAGGGAGGGAAGAAAGCTTAGAAGAG
GCCCGGCAGGGATGGCTGCCCTCTGCCAGGAGAGGGCAGGAGACATACCAAGGACTAGCAGAAGGCCATGCAGGTAGGGAGGATGGA
GCCAGGAGGAGCAGGTGGGAGAGGAGGCCACAGAGACAGGGAGGACCCTGACTCCTAAGGATCTTGAGTTTTTCTCAAAAGCAATGG
ATCTGAATCAAGACTCCATTGAGAAAAGGTCACTCTGGCTACAAACTAAAGGAGGAAAGAGAGGATTCAGGGAACTGGCTAGGGGAC
TCTGGCAGGGGCTCACACAGAGTTGAATTACTTACTTTTTCTCACAGACTTTCTTTTATTAACCATGTACGGAGTGACTACTATATA
TACCAGCTGCTCTGCTGGGTACTGGGGATATGGGATGACCCCGACTCCATCCCTGCCGGTGAGCTTACCAGCTAGTAAGAGAAAA
CTAACAAGGAAACAGACACATGCACTGAAGCTTCTCATGGGAGGCTGGTGGGATGTGGAGGGGGCAGAGGGTAAACTAAGAAAGAGT
GACTGATTTTTATTTGTTTATGTTTATTTTTAATTGACAAAATTGCACATATTGTGTACAATGTGTTTTGAAGTATAGAGAGAAATG
ACTAAATGAAGCTAATTAACATATGCATTATCTCACTTATCTCTTTTTTGTGGTGAGAACACTTAAAATCTAAGAATGATCAATTTC
AAATAAAGATGGTAGTGAGTGAACAGAAGAGGTTTCATTGACTCCTAAACTGAGTACTCAAAAACGAGCAGGTGCTCACAGTCAGGA
AGCAGGTGCTGAGTACAGGATGGGAAAAAGCATGGGGGTGTGCACTTGTCAAGAAAATGACAGCTGGCATGTGGGCCAGAGTATAGA
GTGGGAGACAGGATGCAGTCAAAGGTCTGCATGCCTGCGGAGGTCAGCACGGGCTGGACTAGCCCTTCGTCCTATAGCAGGCTGCAG
GGTGCTGCTGGAGGGTTTCAACAAGGAAAACCGTAACTAGATTTGTGCTGTAGAAGCTTACTGTGTGATCACTCAGAGCATGAGCTG
CAAGGGAGCGAGCCTAGACGTTAAAAGGATTTTAGAAAACTGTTTTCAATAGTTCAACGGGAAGTGAGGGAGGCCTGGTTTGAGTAA
CTATAGAGGTCTGAATGTTGGTGTCCCCACAAAGTTCATATGTTGAAGCCTAATCTCCAATGTAATAATATCAAGAGTTAGGGTCTT
TGGTGGGTGATTAGATCATGAGGGCTCTACCTCTATGAATAGGATTAGTGCCCTTATAAAAGACGCTTGGGGGTGCCTGTTTGCCTC
TTCTGCCATGTAAGGACACATGAAAGGTGCAGTCTGTGAGGAGTGGGCCCTCACCAGACACTGAATCTGCTGGTTGGATTTCTCAGC
CTTCAGAACTATGAGCGATAAACTTCTGTTGTTTATGTTTCTGAATGAGCACAGGGGATGAGGGAACAGAAAGAAGGACAACTCTTA
GGTCTCTGGCTTGCTTGACTGGGTAGTTAGTGGTGTCACTGAGTGAGAGAGAGAGAGAGAGTACAGCAAAAGATTTAGAGAAAAGTG
AATGAGTTTTAGGCTTATTGATTTTAAGGTTATCTGTGGGCAGCCAGGTGGAGAGGTCTCAGACAGTGTTAATATGGGTCAGGAGA
TACAGATTGGGGACTTCCTTATATAGGTGCCAATAGAGTGCTTGGATATGACATAGATACCTGTCTGTGGTGAGTTATCTCAACT
GCTCTTAGGGGCTAGCTGAGTGATAAATGAAGCCATATGGAAAAAAGAAAAACAACAGCCAAACAACAACAAAAAACAGATATG
AGAAAAAAACCCAACTGCACAAATATGAAATCAAACGCTTTCTCATACTTGTCTTTAGTATTTGGGACAAGAGGGGAGTAGTGGGGAC
TGTGGCAAACTGAAAGTAAGTCAAAAGGGGATAGGCTTAATTTAGCTCCAGACGATTTTCACGAGTTAGAATTTGGGTACTACTCTA
CTAGATTTTATAATTTTCCTAGAAAAGGCATAAAATGTTATATTTATATAAAATTCACTGATGTAAAAAAATTGGCATAGTTTTTTT
```

```
TTAATTACAAGCTAAATAAAACATATTTGTGAAACAAAGTTTGTGGCTACTTCTGAGATTTCTGTATGAAAAGACCCAAGGTCAAAA
TAGAGTAGGTGTTCTGTGAGTACAGGTCCACACTCCATTGAAGACAATCAGGATTGTACATAGATGCAATCTTCAACTTACAAGCTT
TACAACGGACAAACATCCAGACCTAGAAACTGTTCAGTAGGATCATTCCAGAGTCTCTGGGGATCCTAGCCAGTAAAAGTCATGCCT
TGGGCTGCTCCACTTTTCTTTTTTTGTTTTGTTTTAGTTTTTCTGTTAGTCATGCTGCAAAGTTATCCCTTCTTAGCAGGAAATGAT
AGCCTGGTTATCATGGTTGAATGTTTCTTCTTTACAGGGGTGTGTGTGTGTGTGTGTGTGTGTGTGTGTGTGTGTATTGGGGTAG
GGTGTGGGGTTGGGATGACAGAGTAGTGAGAGGAAGATGAAGGGGATTTTCTTTTTAGAATCACAGAAGCTTCATGCTAGAAAAACAC
ACTGGGGAGTTTTGGTACAACCGCTTGTTTTAAAAATGGGGAAACTGAGGCTTAAGTTAGTTAATGGCAGAGCCAACAATAGAACAC
AGGTCTCCTGACTTCTAGTCAAGCACATTTTGCTCTCTCCATCTTTGTACGTGGAAAGAATGCAGCAAACCCAGCCTTGGTAAGATG
TGCTCGGAGCTCTTTGCTTCAGTGGTGGGGAGGAGGTAGGTGTACAGATCCTCTCATCCAGCACCTGGACCTACAGAGGCATGAAGA
ACACTCATTGCCCCATGAGGTTGGTTCTTGGGTTTTTAATATGTCCTCAAAAATAAAATGAACTGAACCACCATACACTTCTTTCAT
TGTGGATTTTCAACTAGTGCCAGCTCTGTACTCACCAGGGGCTTCCATATCATCAGACAGTGTGATATAAATCTGAGAGAGGTCTTT
TTTTTTTCTCATTTTAAATAAGATGTAACCCTAAAAATTCATGCCTCTAAAATTCAGTAGTTTTTAGTATCTAAAAAGACGTGCAAAT
ATCACTGCTATCTAATTACAGAACATTTCCATCAGCCTCCAGAGAAATCCTATGCCTATTAATAGTCACTCTCCAGCCTCCACTCCC
CTCAGCCTTGGGCAACCACTAATCTACTCTTGATCTCTATAGATTTGCCTGTTCTAGACATTTCATGTAAATGGAATCATACAACAT
ATGGCCTTTTATGTATGACTTCTTTTATGTACCATAGTATTTTAAACATTCATCCATGTTGGTAACATGTACCAGAACTCCATTCCT
TTTTATGACTGAGTGATATTCCATCATCTGGATATATCACATTTTGGATATCCATGTGTCAATTGATGGACATTTTTTTTCATTTAT
GGCTATAATGAATAATGCTGCTATGGCTATTCATGTACCATTTTTATGAGGACATGTTTTTGATTCTCTTGGACACACACACACAC
AAACACACACACACACTCAGTAGCAGGACTGCTGGGTCAAAGTTAATTCTGTTTAAACTTTTGAGAAACCACAAACTGTTCTCCAAA
GCAGCTGAACCATTTTACATTTTCACCAGTAATGTATGAGTTCCAATTTCTCCATATCCTTAGCACTTGTTATTATCTGTCTTTTTT
TTTTTTTTAATTATAGCTATCCTAGTGGTTGTGAAGTGGTATTTCTTTGTGGTTTTGGTTTATATTTCCCTAATGACTGATGTTGAG
TATTTTTTTCATGGGGCAATTGACTATTTATATATCTTCTTGGGAGAGATGTCTATTCAAATCCTTTGCCTACTTTTAATCAGGTTAT
TTGTCTTTTTATTGTTGTAAGAATTATTTACATATTCCGTATACAAGTTCTCTCCCTTATCAGATATGATTTGCAGCAAGTATTTTC
TCACATTCTATAAGTTATCTTTTCACTTTCTTGGTGGTGTCCTTTGGAGCACAAATGCTTTGAATTTCGATGAAGTCCAATTTATAT
TGTTATTGTTATTTGTACTTTTGATGTCATATCTAAGAAACCATTTCCTCATTCGGGGTCATAAAGATTTATGTCTATGCTTTCTTC
TCAGAGTTTTATAGTTTTAGTTTTTCTATTTAGGTCTTTGATCCATTTTGAGTTAGTTTTGTGTATGGTATGGGTAAGGGTCCAAC
TTCATTCTTTTGCATGTGGATATCCAGTTGTTCTAGCACCATTTATTGAAAACACCATTCTTTCCCCCACTGAATTTAGCATCCTCA
TCAGAAATCAATTGAATTGTTTCTGGCCTCCCACTATGATTGTTCCATTGATCTATATATGTTTATGCCAGTACTGCCATTATCTGGAACT
ACACCTTTGTAGTAAATTTTGAAATTAGAGAATGTTAAGTCTTCCTACTTTGTTGTTGGCTATTCTGAGTCCCCTGTAGTTCCATAT
GAATTTTAGGATCAGCCTGGAAATTCTACAAACAAGGCAGCTGTAATTTTGATAGGGATTGTATTGAATCAGTAGATAAACTTGGGA
AGTATTGCCATCTTAATAGCATTAGATTTTTAAATCTGTAAACACTAGGTGTCTTTCCATTTATTTAGAACTTGTTTAATTTCTTTC
AACAGTGTTTTGTAGTTTCCATTTTATAAGCTTCATATTTCTTTGCTTAAGTTTATTCTGAGATATGTTATTCTTTTTGTTGTGGTT
GTAAATGGAATTTTAAAAATATCATTTTTGGGTTGCTATTTGCTAATGGTTAGAAAGATTGATTTTTGTTTATTGATCTTTTATCCT
GAAACCTTATAATACTTGTTTATTAGCTGTAATAGTTTTTTTAGGTTCCTTAGAATTTTCCCTATATAAAATCATGGCAACTGTGAA
TAGTGATAGTTTTATTTTTTTATTTCCAATCTTTATGCCTTTTATTTCTTGTTCTTGCCTAATTCCCCTTGCTAGAATCTCTAGTAC
AATGTTAAATAGAAGTGGCAAGATTGAGTATCCTTGTCTTGTTTCTGATCTTATGGGGAAAGCTTTCAGTCTTTCACCATTAAGTAC
AATGTTAGCTGTGGGGCTTACTTCGGTGTCCTTATTAGGCTGAGAAGTCACCTTCTATTCCTGGTGTGTTGAGTGTTTTTATCATGA
AAGGATGACGATTTTGTCAAATGATTTGCTGCCTCTATTGAAATATGTGGTTTTATCCTTGATTCTATCAATATGGGGTACTACC
TTAATTGACCTTCATAGATTGAACCAGCTTTGTATTCTGGTATAATTTCCACTTGGTAATGGTATATAATACTTTTCATATGTTGCT
GGATTCAGTTTGCTAGTATTTCATTGGAAAATTTTGTGCCTATATTCAAAAGAGACATTAGTCTGAAGTTTTCTTGCATTGACTTTT
TTCTGGTTTGGGTATCAGGATATTGGCCTCATAGAATGAATTGAATAAGTGTTCCTTACTCTTCTAGTTTTGGAAGAATTTCTGAAG
GTTTGATGTTAATTCTTCTTTACATGTTAGGTACAATTCACTAGTGAAGCCATCTGGTTCTGGGCTTTTTTGTGTGTGGAAATTTTT
TGATTACTAATACAATCCCTTGTTATTGTTCTATTCAGATTTTCTATCAGTTTTTGTTCTTGAATCAGTTTTAGTAGTTTTGTCTGTCTAGA
AATTTGTCCATTTGATCTAGGTTGTCTAATTTGTGGGAAAACAATTGTTCACAATATTCTCTTTAATACTTTTTGTTTCTATAAGGT
TGGTAGTAGTCCTCTTATATTCCTGATTCTAGTTATTTGAATCTTACCTCATTTTTCTTGATCAGTCCAGCCAAAAGTTTGTCACTT
TTGTGGATCTTTTCAAATAATCAACTCTGGTTTTGTTGACTTTTCTCCTGTGCTCTTATATTCTCCATTTCATTAATTTCCACTCTG
ATCTTTGTTATTTCTTCTGCTTGCTTTGCTATTCTTCTTTTTCTTGTTTTTCTTTTCTTTTTTTTTTCTAAGTAGAAGATTAGGTT
CTTTGGGATATTTCATCTTTGTAAAGTATGAACATTTACAGTAGATGATTTTCCTCTAAGTACTGCTTTATCTGCATCCCATAAGAT
TTGGCATGCTGTGTTTCATTTTCATTTACATCAAAGTATTTTCTAATTTCCCTTGATTTCTTCTTTGACCTTTGGTTTTTAGAGTAT
ATTGTTATTTTACATATTAGTGATTTTCCCAAATTTCCTTATATTGTTTTTAATTGTATTCAATTGTCAAATACTTTGTATAACTTC
AATCCCTTTAAATTTATTGAGACTCGCTTATGGTCTAACATATAACCTCTCCTGGAGAATGTTCCATGTGACTGGCTTTTGTTGGAT
GGAGTGTTGGGTGTTTGTTAGCTTCTAATTGGTTTATAGTATTGTTCAAGTCTTCTGTTTCCTTATTGATTTTCTATTCTATTATTT
AAAGTAAAGTCTTTGAAATGCTTCAATTATTACTGTTGAATTATTTAATTTCCTTTCCATTCTGTCAGCTTTTCTTTCATGTGTTTTGA
GGTCCATTGTTAGGTGCATATATGTTTATAATTGCTATGGTTTCTTGATGGATTGACCCTTATATCATTATAGAATATCCCATTTTG
TCTCTGAAAATTTTGTTTTAAAGTCTGTTTTGTCTGATAGTAGTATAGGTTAATTTTATCCTCATTTTGAAGGATAGTTTTGTAGGA
TTTACAGTTCTTACTTGGCCACTCTTTTTCTTTCAGCACTTTGTTTTTTTTTTTTTTTTGGTTTGTTGTTTGTTTGTTTGCTTGT
TCCGCCTTCTGGGTTCACGCTATTCTCCTGCCTCAGCCTCCCAAGTAGCTGGGACTACAGATGCCCACCACCATGCCCGGCTAATT
TTTGTGTTTTTTAGTAGAGATGGGGTTTCACCGTGTTAGCCAGGAAGGTCTCGATCTCCTAATCTCGTGACTCGCCCACCTCAGCCTCC
CAAAGTGCTGGGATTACAGGCATGAGCCACTGCACCCGGCCTCTTTTTAGCACTTTGAATGTGTCATTCCACTGCCTTCTGATCTCCA
TGGAAAATTAGCTGTTAATTTTATTGAGGATCCTTTACATGTGATGAGTCTATGCACTTTTAAGATTCTCTATCTTTCAAATGTTTA
TGATGTGACTAAATTTGGATTTCTTTGAGTTTATTCTATTTGGAGTTCATAGTGATTGTTGGATATATAGATTCATGTTTTTCTTAA
ATTTGTGAAGTTTTGGGTCATTCTGTCTTCAAATAATCTTTTTTCCCCCTTTTTCCTTTCCTTCTGGAACTTTTATTATGTATGTGT
TTTTCTGCTTGATGGTTTGTTTCTTCTCTGGGAATTCTCTGGAAATCTGCTTTTGAACACCCTCTTATGAATTTTTCCTTTCAGTTATCATCCTT
GTCAACTGTAGAATTTCTATTTTAAAAAATTTCTATTTTTAATCAATATTCTATATCTGATGAGGCATTGTTTTCAAACTTTAATTCT
TTAGAGATGATTTCCTTTAGTTTTTGAATCTATCTAAAATAGATTTTTAAAAATCTTTAGTAAATCCAACATCTGGGCTTCCTCAGG
GACAGTTTCTATTGACTTTTTAATATTTTTGTACATTTCATAATTTGTTGAAAACCAAACATTTTAAATAATGTGATGTGGCAACTC
TAGAAATCATATATCTTTCTTTTCCTGGAGTTGGTTGTTGTTGATCTGTTTGTTTGTTTGCTTAGTGACTTTTCCATAATGAATTCTGTAAAGT
CTGTATTCTTTGTCATGTCTGGCCACTGAAGTCTGCTCAGTCAGCTTAGTGTTCAGCTAATGACTAGATAGAGATTTCCTTAAATGC
CTTAAGCCAGTAAGTCTCCTGGTGTTTACCAAGGGGCACTGTGTGCATGTTGGGACACAACCTTCAACTATGTGGCAGGCAGTTTAC
AACTCTGCCTTCGCCTTCACTTTCTGCTTATGCAGAGCCTCCAAGTCAGCCAGAGGTGAGGGATTAGGGCCTTCTCAGGTCTTTCCC
AGGCATGCACACAGCCCTGCTCATGATAGTGGCCTTGTAGATTGCCAGGAATATATTAGAGATTTTCAAAGCCCCTTATGGACATCT
CATTTCCCAGCTTTCCCTTTTTAAGTGTTTTTGGTCAGCTGCTTCTTTGCTTCAACTGTTATCACAGCCTCAGGCAGCTGCAATGTTGA
ACAATTGCTAATGGTTGTTTTCAACAAATGCCCCAGGGGAAAAATTGTGTGGTGGTATGAATTCTGAGTCCGGTCAAATCGAAGATA
AGTGTTGTAAGTAGATGTTCCCAGGGAAATGCCAACAGGATAGATAATGATAGTTCTCTGGTGATGGGAGCTTTTGGGAAGCTACAAA
CGCTTCTCACCCTCTCCATGGTACTGTGGTGTGAGGCTGTTGCTTTCAAGGCTACTTCAGATCTTAGGAAAGGACCATGGGATTAGG
```

```
GTGCATTAAAACACCACAAACCTTAGTTTTTTTTTTTTTTTTTTTTAATAAACATTCCTTGGATTTTGCAAGCATCTGGTTAATTTCCC
ATATTCTTAAAAAGATGATATTTACAATTTTTGCCAGTGTTCTCATTGCTTTTATGGAGGAGCAGTTTTCTGTAGGCCCTTACTCCA
CCTTTCTCACTGATGTCCAGGTCTTTTTCTGAAAACACAAAATAGCAACATGTTCATTTATTATATAAACAATATAATATCAACAAG
CAAACATGCACAAAAACTTTTTAACTTACAATCTTACCCATTTAACACATCAACTCTTCTTATATTTTAAATTCCATTTCTTACCTA
CTGACATGAATTCTTTTGGTACCCATAACAGTCTAGATTTACTCTCAAATTTACTTCTGTTACTTTACATCATTGTTTCTTATGTTTC
TTCATAATTTGTAGAATGAACATTTTAATGATTAATATTCTGTCAAGTTCATGTGCCATAATAATTTCACTGTCCTTTTTTTTTGAC
CATTTAGGAGTTTTCAATCTTACTGGTATTATAAATAAAGTTACTTTCACTATAAGCATGTACTTGTTTGAAGAAATATTTCCTTAG
GGTAAATTATGTATAGTAATGTTTGCTATCAAAGGGTACAAACATTTTATGCCTCAAGTTATTTCCAAAAGGATTATAGGAATTTAT
ATTTCCATCAACAATGTTTCTTTATATCCATACTAACTTTGATATTGCTTTTAATTTAATAAAAATATATAAAAGTATACTAAAACT
TAAGGTTGAACAGCTTTTATGTTTTCTTAGTTTTTATGTGAATTTTCTGTTGCTGTGGTCTTAAAAGTGATATAGCCATATCTATGAA
TATGGTGGTCGGTATTTTCTCCACTTCTGAAAAAGTAATAGATTATCATATAGCATTTGCATTAAGTTAAAGCCACTTATAAATTCT
TTTCCTTAACATTAACGTGTCTATTAATCTTTGTCACAATTTTTAGAAGTGCTAAATGACATATTTTATCCTAGAGAGAAAGCTACC
CTTTTAATGCTTCTTAGAAGCTAGTAATGTCATAGATTTCAGATATTACCAAAAAGTCTCTTTTAACCCCAGCTATAATTTAAAGCA
TCAATGACTTTTGTCAATGTATTGAATAATAGTATACAGTCTCTTGAGGAAAATGTATGTTAAATGTTACACAGTTATTGTGCAAAC
AAACTTTTTAGAAGGTACCACTTTAAAAGTTGAGGATTTCTTATTTAGTAATTGAACCAATGAAAATTTACCTGAAATTGGTGACTT
TCTACAAATTCCACAACATTGTTTTTTTCCCTTACAACTTAATCTGTTTTTTGCTTTTTTCCCCAGTTTAAGGCCAATCCTCCTGCTGT
GACTTTTGAACTAACTGGGGAGACAGACAACATATTTGTGATAGAACGGGAGGGACTTCTGTATTACAACAGAGCCTTGGACAGGGA
AACAAGATCTACTCACAATCTCCAGGTAAATTGATACCAGCTGAAGGGATCTAACTCCAACCTCTCTGAGCACCCTTTGTTCTGGAA
GTATGGCTGGCACCTTCCTTCCTTGCACAGAATCCAGTTACAGCTTTCCTTACATTAATGGGACGCTTTGCAATCCTCACATGTCCA
TTTCTTACCAACTACAGAGGGGTGTGGCAAACATATCCTTTTCCTTAGGCTTTTTAACCAACGTAACCCCACTTACATTGCCAGGAC
CCTCTGATGGCATTACTTATCCAAGTAAGACCATGCTTAGGTACTTGGATCAAAAGTCAGCACATCTCTGATACTTCAATGGTATCT
GCCTGTGGCTGTTGCATTGGCTTGGGACCCATGAAGAAGGCTGTATCGGTGCTGGGATGGAGCTCCTTTGGGCGTTGGAAGAAGCAG
AGGGAACCAGTAGCCAGGAGCTGACTAAAAAGACCCGTCTCCCTCTCTTTCTCCTTTAGTTTTCACCGTTGGTATCTGAATACCTAG
GAAAGTCAGAGAGCCTACTATGGAAAAAAGAGTGAGATAAAAGCTTTCTGTCTGGTACTTCTGCTGCAGGCCTTGCAGGCTGACCGC
CATAATGGCTCTGGAAGAGAACACAACACTTTCATATTCTCAGGGGTCACAGTGCTCTTGTTTCATGGACTTCAGCCATCCAGAACA
GATGCATGTTCAGTGTCCAGAACACCCTGCTGATAGAAAGTCACCATCATGATTAGCCAGGCTTAACTGATACAATCCCTGTTTTAG
CATTTCCCAATTTGGCCTTTTGTGAGTTCTTTCTCTCTACAGTCACCAGGCCATTGGTTCTTTGAGTTCTAATTTCCTTCAAGTGA
TTTTTGGGCATGTGACATGAAGTTCAGTCTCACCAACATGGTTTCCTTTTCCTCAACATCAGGTTGCAGCCCTGGACGCTAATGGAA
TTATAGTGGAGGGTCCAGTCCCTATCACCATAGAAGTGAAGGACATCAACGACAATCGACCCACGTTTCTCCAGTCAAAGTACGAAG
GCTCAGTAAGGCAGAACTCTCGCCCAGGTAACAGGCAGGGGCCAGAGATATTTATGGAAAGATGGAAGGGTGTCAGGTGGAAGGGGC
AGACTTGTGTGGCTAATAGCTGCAGCTGTCACTCAATAAGCACTATCTTACCTACACATTTTCACACTGATTTGGGAGTTGTTTCACTAT
CTCTATTGCTCCAGAGCATGGTCTCTCAATGCAGCACTATGTATTGACATCTTGGGTCTGATAATTCTTTTTTTGGTCAGAGGCTAT
TCTGTGCCTTGTAGGATGATTGGCAGCATCCCCCTGACCTCTATCCACTATGTGCCCCCCTGCCAAGTTGTGGCAATAAAAATGTCT
CCAGGCATTGCCAAATATCCTCTGGTGGGTGGAGGGCACAAAATCCCAGTACTTTGGGAGGCTGAGGTGGGAGGATCACTTCAGCCA
GGAGTTTGAGAACAGCCTGGGAGTTTGAGACCAGCCTGGGCAACAAAGTGAGATCCTGTCTCTACAAAAAATAATAGTTTGCCAGGT
GTGGTGGTGCATGCCTGTAGTCCTAGCTACTTGGGAGGCTGAGGTGGCAGAATCACTTGGTCCCAGGAGATTGAGGCTGCAGTAAGT
TGTGATCACACTATTGCACTCGGTCTGGGCAACAGAGTGAGATTCTGTCTCAAAAATTACCCCCAGTTGAAAAACGCTTTTCTAGGG
TGATCTCTAAGATACCTCAGAGCTCTAGAACACTCTAGAAAGGTAAGCTTACTCCCCTGTATCTTCTTAGGAACCTCCATTGATATT
GTCACTGGTGGTTTCCAGCTCTTTTCTTCTCTTACATCCCATCTGTGGCTCAGCCCTTCTCTATGCTGCATTGACCTTCTACCTTTC
ACTTCCACAAAAGCCCTTCATTAAGCCCTTCATTAAGGCCTTCATTAAGGCGTGAGTGTGTCCAGCAGGCACCTAGGAAGGAGCATC
ACCTCCTAACAGTTGCACACAACATAAATGAATATTAGACTTCAGAGTTCTTAGAAATATGGTTTATCTATTAACATTTACTGTATT
AGAAATTTTAAAGATATTCATTTAAAATAATACTAATCAAGACTTGGGGCCATTTGCATCATCCCACCATCAAGCTTCTTCCTTCTC
TTTCTTCAATGCCTGCCTCCGAGAATTCCAGGAAAAAAATTCACTTCAGTTTATTATGTGGAGTGATGTCCCTGTCCCTGAAGCTGCA
TACCAAAGACTCACACAGACTCTGCTAAATGTGTAGGCTCCAGGCCGTACCTTTTCTCTCTTCACTAATAGAATCTGCTAGAGAAAT
GGCTCTCAAACTTTTTGGTTTCAGGACTTGTATATTCTCTTAAAAATTTACTGAGGACTCCAGAGAACTTTTAGACATGTGGGTTATA
TCCAGTGGTGTCCTGGTAAAGGCTTAACAGCTATTTTTCTGGAGGCTAAAGCTCTGATTTGTAGAATTTTCTAGTTTCCATGTTGC
AAATACTCCTACCATGGCCTATTTCAAGTGACCATTGGTATTAATAATTGACACACAAAATTTCTGAAAATTCAACAACTGAGTCTC
ATGAGCTGTCTCTAGTACATCACTGGTTATATCTATCAACATTTACTATATTAGAAATTTTAAAAAATTATTAATTCATTTAAAATA
ACACTAATCAACATGTTTTAATGAAAAATAATTGATTTTAAAGACAAAAATAGTAAGAAAAATGGCATTGTGTTAATCTTTGCAAAT
CTCTTTAATGTCTGGATTAATAGAAAACGCTTGGATTCTCATGTCTGTTTCTACATTCATTCTCTTGCCATGTGCTAGTTTGGTTGA
AATATATGAAGGAAAATTAGCCTCACACAGATATGCAGTTGGACAGAGGAGGAATATTTTAATAGTATTTTCAGATAACTGGACATT
CTTTAGTACTATACCCAAACTCAATAAATTGTGGTTTCTTAAAGATTAGTTGCAAAGTGAAATCAAAATTAATACCATATTTTCCAT
TCTTTTACACTACAATTCATTAAATTATCTTGTACTTTAAATGGATCTTTTACAGAAACATGACCTTTAGCTTTACAGAGCACTTGA
ACAGTTTCAGGGACCCTTAGAGGTTCTTGGACCAGACCTTGAGAAACCTTATTTTAGGGAAGGGGGGAGTGGGATAAATATTTATTT
CCCCACTGGTGTGAATTGTCATACCTTTCCCTATTATATCTCCACTTTTTAGATGGAAAAAGTAGCTATGGTTGGTTTGGGTTTCAA
TTAATATCTTTTTTTTTTTTTTTTTCTGGGTACGTCTTTGGTTAAACAGGAAAGCCCTTCTTGTATGTCAATGCCACAGACCTGGATGATC
CGGCCACTCCCAATGGCCAGCTTTATTACCAGATTGTCATCCAGCTTCCCATGATCAACAATGTCATGTACTTTCGATCAACAACA
AAACGGGAGCCATCTCTCTTACCCGAGAGGGTAAGTTCAGGGGTGCCCTGGTAGGACTGTCTCGGTCAGAAAAAGGGGAGTTTGGTCT
GTCTCTTCCAGAGACACTTCCTATTCCCTTCTCCCCTACTTCTCACAGGATCTCAGGAATTGAATCCTGCTAAGAATCCTTCCTATA
ATCTGGTGATCTCAGTGAAGGACATGGGAGGCCAGAGTGAGAATTCCTTCAGTGATACCACATCTGTGGATATCATAGTGACAGAGA
ATATTTGGAAAGCACCAAAACCTGTGGAGATGGTGGAAAACTCAACTGATCCTCACCCCATCAAAATCACTCAGGTAGTACCCAAGC
CCAAGTAACACTGAGAGCCAACTAGATAAATGGCTTCACTTTGTAGAGACCCACCCTCACAGTCTTCACTGATACACTTATTAGATA
CCTTTTTTCATAGCATGATTATAAAGCAAATTATATAGCCTCTGGCCCCGAGTCACTGGACTTGACCATGCATCATGGGCAGGCAACT
TGGATGCAGATATTATGTTAGGCCGTTTTTGTATTGCTATAAAGAAATACCTGAGGTGGGGAATTTGTAAAGAAAAGTTTAATTGGT
GCATCGTTTTGCAGGCTGTTCAAGCGTGGCTCCAGCATCTGCTTCTGTTGAGGGCCTTGGGAAAATTACGATCATGGCAGAAGGCAA
AGGGGGAGCAGGAGCATCATATGACGACAATGGGAGCAAGAGAGTTAGGTGGGAGGTGCCACACACATTTAAGCACCAGGTCTCATG
TGAACTCAGAGTGAGAACTCACTTACCACCAAGGGCGTGGTGCTCAGCCATTCACGAGGGATCTGCCCCCATGATCCCACCTCCCAC
TGGGCCTCACCTCCAATACTGAGGATTACGTTTCAACATGAGATTTGGAGGGGACAAACATCCAAACGACATCAGATATATACTTTT
GCTTTCAGAGAGAACTTCACTCACCCTCACCTTAAAAGTAGACAGCAGTGATGTTTGTTGTTCCCTGTTAGCAGTGTGGGATATGGC
AGCCCTGCAGTGGGTCATTTATGGTGCACTATGTATGGACATGGTTTGATTTGGGGGTTGCAAATAGATTTGAAAGGCTGCTGCTTC
TTATTGTGGAAAAAATCTTGACCCAATAGACTTGCCCATCTTAGGCTGAGGGACGGAGATAGGACTGGCTAGGGGTAAAAGGATCAT
AGTAGGGAACCTTTCCCCTTGGAAACTCCCTCACCCCTGGCACGTTCCTGTGTGTGCCTCTCCCCTCCCGTTCAGATGCTCCTA
CTTCCCCACCTCTGTGGCACGTGCTCTTACCCTGGGCCCCCTCCTACCTTAGCTCCTAGAAATTCACTCTGGACTCACCCAGATTT
GATATTTTCTGGAAGCCCAGGAAATTCCTCCACATGAAAGCTGTGGGTTAGTGGGAGATCCCTGGATTTGGAAGCAGATGAACAAAA
TCATATTCCTGTTCCATCACTTGAATGACTGATCATAAACAAGTCATTTAACTATTCTGAGCCCCAATGTCTCATCAATAAAATAGG
GATAATACTTCAGTGTTTTGTGAAGATTAACTGAGACAGTATCTGTAAAAGTAGATAAAAATATTAGATATAAAAGGTATTATTATG
```

```
AAGAAGTACTAGTCTGTAGTTCCCCTAATTTTTTTTTTAATGCTACTATTGTTAAGGGGCCCTGGAATGGCCTATTCCATCATATTTA
TTGGGATGGGCCCAGGGACATTTATTTATTGAATGTTGAATTAATGAACTTCAACTTCAGGAGGGGAAAAAAAGGAAGAACAGCAGG
AAGAATTCTGATTTCCCAGTTAGAAAGAAAGCATGTCAGCACTCATTACCATGAGAATTTCTGATTGGGGGCCTAGACTAAAGTCTT
CATAGCTATTATTCAGGTTACTCCCAAAGGGATTGGTAAATGCTGCAAAGAACTCATGCTTAGTTGCCTTGGGGCTGCCAAGGGAAA
ATTCTAGAGTGCCTACCCAATTTTCAGGTCTGGAAAAAAATGCTCTGGGTGTTCTGAAACCCAAAAGGAGAGAGGGAGAAAGAGAGAG
GGGGAGAGGGGGAAGGAGGAGCGGGAAGGGGAGGGAGGAGGGGGAGGAGGGGGAGGGGGAGATGGGAAGAGGGAACTCAGACTGAAT
TTTTAGAACACAAGAGGAGCCCACTTTAGAACCAAGAAGGTAGTCTGCAGGGACAATGGGTGATGCTTAAATAATTAGCAAGGGACA
AAGTTGCTGCTGTGATATCTTCCCAGGCTGCTCTGAGAATAGTTTACTGTGGAAATTTCAACTAGCAGTAGGACCTTGGGCAAGTCG
CTTAAATTTTTTGTGCCCTAGTTTCTTCATTATAAAATAGAGATAACAGTAGTCTGTACGTCAGAGGACTGTTTGGAGATTAAATGA
GATATAACACATAAATATGTTTAGAATTGTGTCTGGCATGTGGCAAACTCAGTAAACATTATTAATATATCAGACCAGGCTCTGTCT
ATATAGCAAGTAGACTGGTCTTACTTGTGTTTATTTTTTATTTTTGCCAGGTTGTTTACAAACAACCCTAGGAGTTGGCAGTTATTC
TCATCCCTGAAGTCTCTCAGGCTAAATATTTAGGAAAGGAATGTCCTTATTTTTGTATCATTTGTCTTACTTAATGGCATGGAGAAG
ACTTCAGAGTGGCCCCTATTGCCCCTGTTCATCTTCATTGGTGAGTATTAGATGCTCACCATACATTGTGGCTTAGTCCTCAGACAC
TGCCAGCCACAAAGTACATTTTGTTTCTTAAATGGATGGTTCGTGGATAGAAAGCTTTCACAGTCAGACTTCCAAAAGAAAGCAGCC
CAGAAACCCTGTAGAGGGTTTGTGAAGAATTGCTTTAGAGAAGAAACATTTCCTGGTGTGCTTTTTTCGAGCAATATAACTTGGTGG
GGGGAAACAGGCTTATTTTAGGTTAAAAGTGAAAAACGTAATTCAGTTGTTACAAGAAAACCACAAACATACAAGGTTGTCAGAGATT
TCAAATTGAGAACTAGAAAATTATTTCCTTTCCAGTTCTCTCAGTCCAAATACAGCTTTCCTCACAACTGACTTTGGGCCCTGTAGG
TGCCGGTGGAATGATCCCGGTGCACAATATTCCTTAGTTGACAAAGAGAAGCTGCCAAGATTCCCATTTTCAATTGACCAGGAAGGAG
ATATTTACGTGACTCAGCCCTTGGACCGAGAAGAAAAGGATGCAGTGAGTAAAAGAGAAGAGTTTCACAGGCGACAAGACAAAGTCC
TATGCCCATCTACAGTTGAGAGCAAAATGGATTTTACTTTAAAGAAAAAAAGACACACATTTCAGACTCCAGCATATTATTGGTTT
TCAAAGACATGATAATTTTCACATAATTCTGACCCATCCTCAGTTTTTAAAAATTTTCCAATTCTAAGTGAATTAAAAATAAGGGAGTA
AATGACATAGAAATGAAACAACGATTTTCTCTGCTTTTGACAGAGGGTGGTGGGTGAGTCTTGCCTTAACTCTGACTTTCCTGTTGT
AGTATGTTTTTTATGCAGTTGCAAAGGATGAGTACGGAAAACCACTTTCATATCCGCTGGAAATTCATGTAAAAGTTAAAGATATTA
ATGATAATCCACCTACATGTCCGTCACCAGTAACCGTATTTGAGGTCCAGGAGAATGAACGACTGGGTAAGAAATGAGAGCTGCCTA
ATGCTGTGTAACTGCCCTCCATTTCTCTCCTCTGCTGGGTAAAAGGTGAGCAGGAGACCTTACTGGGAGTCAGTAATCCATGTCTTC
CACAGCATAGAGGTTGACTGTACCTTGGGAGTCACGTAAGGACCGTATTTGGGATCAAACAGTATCTGGATAGTATTCTTAAATTGG
GGCTTTTATAGCTACCATTACCGCTACCATTATCACTATCATTATCAACCTTACTACTATCATTAGTATCACAGCCACCTACAC
TAGTATCTTATATACATGTATATAGGGATATCTCATTTGCAGGATTTCCCCATACGTTATTTGACTTCTAATAATCTCATTAAAGAG
ATAATCTCATCACATTTGCCAAAGAATTTAAACATTTACACCCAAAGTCAGACAGACACCCTGCAATAAGTAAGCAACACCAATGGA
AGTTGACTTTTGACCCTTGGGCCCCCCTTTCCAAGAAGCTGATAATATAATTCAAGAGGCTGCTAGATGAAGTCAAATCCCATCTAT
TTTTATCATTTTTGCTTTGTCTTGCTATGTTTTTCTTTTACTGTGTTGGTATCCTTGTGGAACTCGTGTCCCCCAAGTCTTAGCAACAACTACTAT
AAGAACATTATAATTTCTGGGAGTTTTATGGCTTCTCTGGGTTTGGGACAATGCTTGTCTGTTTCCCCCTTGGCAGTGAGCCCCTTG
ACTGAGCAGACTGGCATTTCTTGAACTGTTTTGTTCTGTTGTGCTGTGTACCTTTCAGAGGCTTCCAAGTGTAGCAGCCTCACGACT
ATCATTTCTAGAGCTTCACTGCCCTTGTGGAGAGATCCTCCTGTGCTTTGTGGATGGGCTCACATTTTCTTTTCGTCCCTCCATTCC
TCACCAGACACACCTCTGACAGAGTGGGTGCCTTCCCGGAGGCTTGGTGGGCGCTGCTGGAGCGGCTTGCTCACACTGCTCATTATT
TTTCTTCTTGCTATCAGGTGTCGTGTGGGCTGCCTTTGTCAGTGTGTTGTGTCACTGTGTTCTGTGCAGGTCCTCTCTGTTTCA
TTTTTTCTCAACTCTTGCCACAACAGGAAGAGTTAATGCCAGCTTGAAGGACTTCAGTTCGTTATAAGGAAGAATCTTCTCACAGTCA
CAGTAGTGTAACAGTAGCCAACAAACACGTGTGCCATCACCTTCTTGGGAAATCTGGGATGGTCTGTGACACTGAATAAAGGTGGAA
AATCTTTGACAACTTTCTTGACCCGGACATGACACTTCAAGCCTCTTTTACATTGGGCCAATCATATAGTCTATTTGTTACCTATTT
CTGCAAAACAGATTGCTCTAAATCTTGGCAGCTTGAAACAACAAGCATGTATTATGTCACAGTTTCTGTGAGTCTGGAATCTGGGTG
AAGTTCAGCTGGATACCTCTAATTCAGGAGTTCTGAGGAGGGGAAAGGTACGGCAGAGCAGGAAAGGACAGGCATCAGTCTTT
CTATGACCTAATCTTGGAAGCGATCCCATCACTTTTGCCATTAGAAACAAGTTGCTAGGTCCAGCCTGCACTCAACAGGATGGGGTT
CTGAAAAAGCACAAATATCAGGAGATGGGGGTTGCTGGAAGCTCTTTTCGAGGCGCCTACCTCATCTGGGAAGAGGATAGTGCTGGT
TTTCTGAGACTGAAGAAGTCATTGTTGGTAGAATAAATTTTGAAGGGGCTCAGCTTGCTATTTGCCTCTCTAAAGGTGAGAATTTAT
TTTGTAAAAATATTTCAGAGTCTTTAGTGGACCAAGAATTCTTATAAATCAACTGAGCATATTCTATATATGTAAGCTTAAAAGGGT
ACAAGACAATGCTCCTGCCTTGTGGGGACTTACTGTCTAGCTTAGCTGATAGGACATGAGTAGGTGGAAATATTCACAAATAAAACC
TTGATAAAATACTGAATTAGGCCAGGTGCAGTGGCTCATGCCTGTAATCCCACCCAGCACTTTGGGAGGCCAAGATGAGCAGATCAC
TTGAGGCCAGGAGTTCGAGACCAGCCTGGCCAACATGGCGAAACCCTGTCTCTACTAAAAAAAAAAAAAAAAACTGCAAAAATTACCC
AGGTGTGGTGGTGCATGCCTGTAATCTCACCTACTTGAGAGGCTTGTAGAGATTGCAGTGAGCTGAGATCATGACACTGTGCTCCAG
CCCAGACAACAGAGTGAGACTCCATCTCACACACACACACAAATATATATATATATATATATATATATATATATATATATATATATA
TATATATATATATTCCCCAGTGTATTTACATTCTCCTGTTTGTATCTCGTGTGGAACTCGTGTCCCCCAAGTCTTAGCAACAACTAACAT
TTATTCTTGAATCCCACTTGCCACCAAGTCTTATGTATTCATACAGAAAATTTAGACTCTCATAAAAACCCCACCTCCCTAAATATT
TCTGCATATTTCTAAGAGATGCTACGTATACTCCTGTATCAGTTTGAGTTCCACTTGTTCCAGGCCTCAGTCACTCAGAGACATGGT
TTTCCTTTCACTTCCGACTGATCCACAAGGTCCTCTGCTCCCAGCGAGGGGTGTCCTCTGATGCCAGCAGTCCTCATGGTGCCTCTG
GCAGCATGGAGGTTTCCTGTGAGTGAGAGGAGGACAGTGTCCTCAGTTTCTTGCTCACTTGCTGAGGGGATAGGGCTATAGCAGGGC
TGCTGAGACACCAAAGGACCACAGAATTTAGTAAGACACTCTTTCATCCCAGCACACAGATACTTTTCTTTGCAAGGGCATATGCATG
CCATATGCACTAGAAACATATTCTCTTCTGTCCTATTCATTCCGATGGTCCCTGCATGCAGCCCATGAGGCACATCTGCATGTGTCC
AGCTCCACTGTAGACATACATAGGTACTTTCTTTTTTCCTCCCCCAACCCAAAACCAAAAAACCTCTAAATTTCCATATGCGTCAGTG
GATGCAAACTGTGTCAGCTGATGGGGGAGGGGGTGTAACTCCACAAATGGGATGAATTCAAGTTTGAGCTCCTACGTGGGATTCTTG
CTACATATAAAGAGACCTACAATTCCCACCTCCCAGAGTAGGATGGTCTGGAACCTGTCAGAACTTGCCCAGAGCTGGGTGCAGAAC
CGAGTCATAGCCACTGGTTTGTAGAACTCCCTGGACTGAAACCCTACCAGGAATAAAATACTTAGGACCACAAACTACAGCAGGGTG
GAGAGGATCCTGCCTGGTCCCTGCCCTGTGTTATCTTTGGAGTAGAGTCAGTTTCTGCAATTGTTCCATGAAGTTGCTTAGATGCAA
TCTAGGGTTGGCTTCTCTTTGTTTAGTGATACCATTTGCCATGTGTATTAGCGTCCTAGGGCTACAGCAACAAATCACCACCAATTT
GGTGACTTAAAACAACAGAAATCTTTTCTCTCCTAGACCTGGAGGCTGGAAGTCTGAAATCAAGGTGCTTGCAGGGCAGCGTTCCCT
CCAGAGTCTCAAGGGGAGAATCTTTCCTTGCCTCTTCCGGCTTCTGTTGGCATCTGGTGTTCCCTGGCTACTTGTGGCAGCCTATCT
CCAACTTCTGGCTTCCTCCTCCAGATCATTTCCTCCTCTGTGTGCATCTCTGTGTTTCCCTTCTTATAGGGGCACCTGTCACTGAAT
TTAGGGGCTGCCTCTAAATCCAGGATGATTTCATCTCCAGAGCCTTAACTAGTGACATCTGTGAAGGTCTTTATTTGCAAATGAGTCCC
ATGCTCAGGTTCTGGTAGACATGAAGTTTTGGGAGGGAGTACTAGGTGTTATGGGCTGAATGTGTCCCCGTGCCCCCCAAATTATAT
GTTGAGGTTGTAACCCTCTGTTCTAGGCTACATCTTCCCCCTCCCCCAGATTCATAGGGTGAAGTTCTAACCCCTAGTACCTCGGAA
TGTGACTATATTTGGAGATAAGGTTTTAAAAGAGGTAATGAAAGTAAAATGTCCTTACAGTGGGCCCTAATCCAGTATGACTGGTAT
CCTTATAAGAGGAAATGTGGACATAGGGAGGTTCAGAAGGAAGGTGCTGTGAAGGCACCGGAAGGAAAAGACAGCCACTCACAAGCC
CAGGAGAAGCCTCGGAAGAAAAACCAACCCCAGCAACACCTTGATTGCAGACTTCTGGCCTCCAGAACTGTGAAAAAATAAGTCTCTGT
TGTTTAATCCATGATACTTAATGACAGCCCTGGCTGACAAATACACTATTCAACCCATTACATGATAGGATGAGAAGAACCAAAATA
ACTCTAAGAGCATTTGTCTGCTATTCAAAGTTATTCATGGTGTTTTCTAGGAATGTGCTTATTTTAGTGAAACTATTTTAATAGATT
ATGTGGAGCCTGTAATGGTGGGTTTTCCTTCTTTCCTTCCTCATAGGTAACAGTATCGGGACCCTTACTGCACATGACAGGGGATGAA
GAAAATACTGCCAACAGTTTTCTAAACTACAGGATTGTGGAGCAAACTCCCAAACTTCCCATGGATGGACTCTTCCTAATCCAAACC
```

```
TATGCTGGAATGTTACAGTTAGCTAAACAGTCCTTGAAGAAGCAAGATACTCCTCAGTACAACTTAACGATAGAGGTGTCTGACAAA
GGTTAGTATCATATCCACCGTCTTGAGTGCAAATCATTGTTTTCCTTCTAGCTATGAGTTATTTTCCTGCGCTAATGTCTGGTATGT
TATAGAATGATGCCATTTAAACATTCTTTGCTTCCTTTGTGAAACACACATAACACAGAGATAGCTGGTTGAACACTGTAAAGGCCG
GAGCTGCAGCTCAGGCACCATTGTTGTGTTGGTCTGGGTCCTATGGGAAGCAGATGCCAAAAGGAGATTAAATGTACGAGGATTTTA
TTGGGGGGAGCCCGTGAGAAAAAATTGCGGAAGGGCACAAAGAAGGCTGGGAGAGTCATCAGACCTAAATACAAGCCTGATGCCTGA
CGCTGGGTGAAGGGGAGATGGAGAAGGAAGGCTGGTGGGAATGTCCTAGACTAGGACAGTCTGAGGAAAGCTTGGCACAGCTGTCGG
GGAGTCTAAAGGGGGCTGTCAGAGGAATTCATTGTCTTTGAAAATGAGCTTTACAATTGCTGCTTTTCTCAGTCACTGGCTGTGTAC
AGTCCAGGGGAGTTGAGGTCTCAGTGTGAATGTGGTAATAGATTCCAGAGCACAGCAGTGGAGCTGTTGTCAATTAACAACCCTGTT
GTTGGAGGTCTGCCAGGCACATTCTGCAGAACATAATCACAAACAGGAAAATGAATTGTATTTCCCAATTCTTGCTTTCAGACTGAA
AGGACTCTACTGTAGGAAAGTGGAGGAGAAGAGCATCTTTCGGAAAGTTCTATCTTACCCTAACAGCTTCTAGTTGGCCGTTTGACA
GTATCAGGAGCAATTCTTCCCCATACTCCTTATACAATTGCAGAGCAAGTGGTTCTTAATCTCTATTGGGTCACAGGAACCTTTGAG
AATCTGATAAAAACTATGCATTCTTAATCAACTGCACACATTGTTAAACAAATGTGCACAGACACACAAAATACCTGGATTCAAAGA
CAGACTCATTTCAAGGGCACTCAGACTCCTAGAAGGTCCTTTGAGTTAATGCGTACTAATCTTCGATTACCATATAATTCAGTAATT
GTTCTGCTGGCAGGCACGGATGACACAGGTATGGAGAACAAACAAGAAACGTTTCACACTGTGCTTCATGTTTCTGTCCTTTACAGG
GATATTGAGATACTAGGCAGAAGCCAGGTTTAGTAGGGCCCTAATGCGGAATGTTTGAAACCCAGTAATTTGACAAATATTAAGGCC
CTAATATTTGTCTAGCATTATTGTAAGAGATAGAACAGATGTAAGTATAATAAACCTTCCCTATTCATGACGAGCTAACAATTTCAC
ACTATCAACAGTCCATTCTTTCAGCAGTTATTCTGCATTTACCTATTTGAGGCACCATGCAAGGTACTGGGGAGACATAGATGATTC
TGTCCTCAAGGAGCACTCATTCTGGTAGAAGAGACGGACATGTGAGTCAGTAAATTGCAAGGAACTGCCCAAGGTAGCCCATGAAGG
AAATAAGTGACTCAGTGGAGGGAGAGAACAGGTACTGCTTTCTGGAAGAGGTATTTCTGAGCTGGGCTTTGAAGGATGAATATGACA
AGTTACAAAATCATGTAGGACTTATGGGAGAGTGGGAATCCATGTGGAAATACATGAGGACCTGGAGCAATTTGAAGATAGAGTTTA
AAGTGGGAATCAGATTACAAATTGTTTATTTGCATGAGTACGTCTATAGAAAATATGGACCAGATTATAAAATATATTAAAATACAT
GTGTGTGTTGATATAACTAAACATTCAACTGTGCACTACAGAAAACTAGAGACTAGGCTATAATTAACACAGTTTTACATAAAACCA
GGGAAGGCCTTTTACCCAGAAAGGCCCACTGCTGGTCAAACGAGGTGTGTGCAGAGCAGTGAGCCAGTGGGTTCTGGGTCTGGGACC
ACTCCAGTCTTCAGCCATTCTTGTTGTCTACAAACGTTCTGAGGACCAGCCTTAACTTCAATCAATATCCACTTGGTTGTAATTGGA
TTGTTAATAGATTCTGTCCCTGGGATCATGTCCAGTGCATTGAGCACCAGCTCTAGAGTTAAACTACCTGGGAATATTAGTCAGGAT
CCCAGCAGGAAGTGGAGACTCCCCTCAAAGAGTTGAAAGAAGGGGATTGTTTGCAAAGGTTGTGGGCAGGGTTAAGGGAAACCAACAG
GAGGTGTGAAGCACAGTAAGCAGCATTTTCTGCCCTAAAACCTGCAGGGAGGGGGAGCATCCCCCAAAGGAGCAAGGCCTG
TGCCTGTCACTAGAAGAGGGCTGTTGGCAACGGGGGCGTGACACCCCCAGCTACACTCTTCCTGTCCTCCCTCCCAGTCTCTTGCCAC
GCCTCTCAGTGGCAGAACCCAACTGTTACCCTCAGGGTGAGGATGTACATTGATGCAGTTCATAAGGTCAGCCCCTTGGGCTACAGA
GCACAGACAGAGGAATGGATGGTGGCTGCAGAGGGCAAAGGGAACCTCTGGGTTTGAATCCTGGCTCCATCACTAGTTAGTTGTGTG
ACCCTGGTTAGGTTACCTGAACTTCTCTATTCCCTGGCTTCCTCATCTGTTAATTAAATGGGAATGACAGTAGCACCTACAACATGA
AGTTATCATGAAGATGAATGAGTAACTCGGGTAAACAATACAGTTGCTGCTATATAGAGGCCGCTAACAACTGTATTATGATGAAAA
CATCTGTTCTGTTATTCACAGTTTACAGATAAAATTGAGTGAGGCACCCAAGCTCTCACATACATGCTAAGTGGCAGAGCCAGAGTCC
AAACAGTGAATTCCAAGCCCAGAGTCTTAACCATTATACAGTGCAGTGCACCATGGTTAGAACAACAGCAACCCAACAAAGAACCTG
TCATTTTATCTCTTGTTCTCTCTCCCCCAGGCATCCTGTCCCCACCTGCACTCCACACACATCTGGAATCTAGCTGAAGTGGCTGTG
TGCCTAGGTCTTGGAATTGTGAAACCCATCCCTGAAAGCCTGGGTGATTTCTTCCAGGGGTTCCAGGAGCTACCTCTGTCCCCACCTTG
CTTCCTGCAGGCTGGGTAGCCCAGACTCTTGTGAACAGGCCCAATGGTGGTAGTTCCTCCCCTTATTTAGATAGAAGAGGAGCAGGCCCTT
GGACCTCACCATCCATAGTAGTCAGAGCAGATGTAGGTTGGATGTGTTCAGAGAAGGAGACAGCCACTGGGATGCTCACAGAAGGC
TTTCTGGAAGGACAGTTTTGCTCTGGACTTTGAAGATTACATAAGTTTCACTAGTCAGAAAAAGACTGCTTCACAAAAGGAAGCCAT
CCTGCTCAGGAACAAACCTTACCTAACTTTACTTAGGAGGACAGCAGGTGGACGGCAATGAAAAACCTAGAAAAATCTCTCTTAGAT
CTGTCTTTAGCCACCCTGCCCCACACTTGGTGACCACTTGGGTACAGGTGGGAAACAGAGAGCCCACTCTCTGCACAAATTGCATGT
CAATTGCAAAATTGTGGCTATGGCTGTCTGTGCTGCTGCTAGCTGGTGCCAAGTATTTTCTTGCAGATTTTCTGATTAATGCATGGTTTTCAGTGA
AAATTTCTATGACATATGACTTTGGTTTTTCAGATTTCAAGACCCTTTGTTTTGTGCAAATCAACGTTATTGATATGCAATGATCAGAT
CCCCATCTTTGAAAAATCAGATGTGAGTAGTTGTCATTATTTGTTTTTTCTTTACTTTATTCATTTTACTTCACCCTTTCTTCTGGG
ATAGAGACACAGACAGTAGCTAGGAAAAGTGCCAGACTTAACAGGTAACCTTGGTCAGTGCACAAATAACCCAGATGCCATCTTCAG
TGGAACTTTTTGGGGAACAATAACATTTACTATTACAGGTGTCTGGGAACATTCATTTATAAACTCCTGCCAATAGCTCATTTATTC
CAAGGGCTGCTTAAAAACAACAACCAATCTTTGTCAAATTGAAAAAAGGTGAATGGCGGCTTACAGTTTGTCATTATATATTTTT
TTGTGTTAGGACAATAAATAGATTTACTGCTAGGTCTCAAGCATTTTTAGCCAAAAATTTAATATTTGGAATATTGGCGATTTAATT
TGTAATAATTGGGTAAGAGAAAGGGAGAAGAAAAAAATTAATCTCTGACTTAAGCCAAAGAAAAATGTGCCCAAGACATTTGAGAGA
ATTAGAACTGGACAGAAAGAAAAAAAGTCAATTTAGAACCTCAAGAAAAATGCAAATTACTGTTTTCTTGTCATCGATAGGGCGTTCA
GACTTGGTGATGACTTTATCATTGTTTCCCTAAGGAGGGAAGTCTCTATTTTGAAGGAGCAAGAAGCCTATTTGACTAGACTGTTAT
GTTATTTTTGAAGTCAGTTATGAGGCAGCCCAGAGCCGTGATGATGTTCATAAATCAAAATAGGGACAAGCCTGCAACAAAGAAG
GGAATTGATTTATGAGGGGGCTAGGGGTGGGAAGAGGTGTGAGTGGATATGTCTTGTATAACAGGCACTGAGGTTCAGCAGGCATCA
AATAACCCATTTCTTCTCAGCCTCCATTCTCTTGGGCTTTTGATCCTTTCCTAACTTTGTGACCTCCTCGCCTTTTATCGAGACAGT
GTTTTTTTAGATTGAGGGTAAGAATCCTGCACTTGAGGCTCAGAGCTTAAATACTAATCTGGCTACAGAGAGTGAACCTGCTTGTTT
CAATGCTGTGGTCCTCTCTTCTGAGACCTACTTATAACCTTCTTTAGGATCATGATGCCAAGAGAGATAATCTGGGGGATTTTCAGT
GGAGTGGGAGAGGTCTAGGCCAGTGTTCAAGAGCATAGGCTTTGAAGTTGCACAAACATGGCTTTGAATCCCAATCCCTTTGACCC
ACTAGCTGTGTGACCCTTGGTGGGTTTTACTTCTCCAAGCCTTTGCTCCTCGTAAGTGGAGATGGTAACATAACTACTCCGTAAGGT
GTTTTTGTTGATTTTCTTTATTGAGCACTTACTATGTGCCAAGAATTTAGGAGCTTTACATGCATTGTCTCCCTTAACACCCACAGCA
AACATGAAGTAGGTTCTATTACTATTGTCTCCATTGGACAGATGATGAAACTGACACTTAAAGAAGTGAAGTAACTTGCTTCAAGCC
ACTCAACTAATAAGAAGCAAGCCTACAATTCATATTCAGGCTGTTTAACTCCAAAGTTCAAGCTTTTAATCAACCTCTATTCATTGG
TTGATTTAAGAATTAATTAAATAATGTATATAAAGTGCCTACCACCTGATAGATACTCAATACATGGTGGCTATTTATATCATTACT
ACTACAATAATTAAGTTATTAAACAAATTGGATATGGGAGGGGGAATTTGTTTTAGAATCCTACCAGTAGCTCTCTGAGGTATTGCG
TTCTTTGGTACAATGGATATCAGCTCATGAGTATGACTGATGTTAAAATCAACTGCAATATATTCATCAATAGGAAAAAACATGCCC
CATGAAGATTGATGCAAACTATGACTTGTGTCTATTCTGAAACCAGAGAGATAAGTTTGTCCTTTGGCTTTGATGACAGCAGACCTT
CTCATTGTTTCCTTCTCCATTTTTCTCCTTAGTATGGAAACCTGACTCTTGCTGAAGACACAAACATTGGGTCCACCATCTTAACCAT
CCAGGCCACTGATGCTGATGAGCCATTTACTGGGAGTTCTAAAATTCTGTATCATATCATAAAGGGAGACAGTGAGGGACGCCTGGG
GGTTGACACAGATCCCCATACCAACACCGGATATGTCATAATTAAAAAGGTAAATAGCCCATTTATTTAATAGAATTTGCTTTGTT
TGTGGGTTAAATTTACTAATGACAGTGTGAAGACCTATAAGCAGAGGTGATGTATTAGCAGCCATCTCTCAGGCATGGTGATGGGTTC
CTGGATTCCTGGATTCCTCTTAGTCGTTCCTGTCTTCCTGACAGACGGGAAGTCAGGATTCTAGACCTGGCTCTGTTGCTAGCTAGC
CAAGTAACTTTTGCAGGGAGGTAACGTCATATAAAAATATATAAAATGCAGGATGAACAATTTTTTAGTATAAGTGTGTTCTATGCAA
TATTTGGGATGCACTTATACTAAAAACTTGTTTGTCATTTATCTGAACTTCAAATCGAACTTGGTGTTCTATGTTTTTTATTTGCTAT
TTGCTAAATCTGCAACACTCTTACGGTCTGGGAGTGGCACTCAGGGTGTCGCTTTCAACTTTCCCCCTTTCACGACAAATGTGAGGAGTTGG
GTTGGATGACCCCATGGGCCTTTTAGGCTGTAAACCCTTTATTCTAATGTGCCAGGCAGAGATACCTTTTGAATAATGCACTGCTG
GGTCTCCTTCCAAGGTTTAATAGACAAGTGTCTTTTTAATCCCCCTCTCCGCTGCTTTACTTTCACCTTTTACTGTATTGCTAAGCC
CACGGGCTCTCATTCTGTCTCTCCCTCTGTAAGGACTGGATAATCTGGAGTCAAAGTCTTAATTACCTTGGTTCCTGTCTCTTTGTA
```

```
CAGGATTCTCCCAACCCTCCCTCAATCTACTCCTGTCCCCTGCCATGTATACCTGAATCTTGACTCTTTTCTCTGTGGCTGCCTCAT
CTGTGTTTAGAGCATGTCATGGGTTTACCAGATGACTCAATTGGAAGCAGCACTCAATGCCGTCCAATGTACTTACAGTTAAGATAC
TTTCCTAGGAAAGAGAGTGAAACCTCGCTGTTAAAAAGGGCCAGAGAGAATCTGCAAGGGTGGCTAATTGTTTAAGGTTTGCAGTGC
AGTGTGCTGGAAGCTGCAAAGGAAAGCTCTGGAGTACAGTTTTGAGCCAGAATAAAAGAAAACTGAAAGAGGCTAAAAATGAAGACA
AAATAGAATAATTTCACAGAAAATGGACAGAAGCACAGAGAAAAAAGCACTAGTGCTGAAGCAAGCCCTAGGACAGAGCCTTGGAAT
TGTGATCCTACTGTTACCAAACATTCCAGCTTGAACCAAGGGCCCTGCTGCATTTCAGCAGAGAGGTTGCTGTGGACCAGCTCAAAG
CCTTAGCCTTGTGTGTGAGTCTGACATTTGGAAAAGGTACCATGACAACCTCCCCTTGTGCTCACAGCAGTCTGCATGGAGAGAGAA
TACCACAGAAAGAAAGGATAAAGGAAAGGGCAGTTTTCAGAGCTTGCAGTGGGTTGGTGTGTTCCTTTGCAGGTGACGGGGGCAGCT
TCCTTTTATCCTTTTGCAGGACCAGAGCTCTCTAGGAATCTAAGGGAAGATAGAGGCCCATCCATCCCAGTATGAACAAGGCCCGTA
TGTCATCTTTTTTTTGAACAGCAGTTTAACTTGGCCATTTGTGGTTAATGGGCCCTTTTAGGGAGATGTTGGCACCTATCTCAATGAT
TAGCTGTGGGTGAGAGCTGTAAGTATCCTCCCTCACAACTTGACACGAGAGGTATGCTACAGTAGAGTAGGCCTCTGACCAAACTCT
TTGTCACATCCTTACTTTATATGTGGTTCTATTTCAAGTGTCTATTATGTCTCCTGGCATATATTAGAGGTTCAATAAATATTGCTT
GACTTAATGAATAAATGAATTCTGTCCCATATACAATTATGAACAAGCAGACACATAAGTGATCATCGAACCTGTGCTTTAGGCCTG
TGACATCCCAGCTCTCTGTCCCTTGGGCACTGTGATAACTGTCCTTGAGCAAGTGGATTTGTTCAACCATCCAGAGGCAGGTGATAA
CTGGCAGAACAGAACTGAGCCCTGGAACTCTGCAGGGTACAATCTGCTGGCCAGGCCAGATATCTATACTCCTGTTCACAATGACTA
AGGGATACACTATACTCGTAACCAAACAGCACATCTTTTTTTTGAGACAGATGCAGTGCCGCAGTCATAGCTCATTGCAGCCTCAACC
TCTTGGGCTAGAGCAATCCTCCCACCTCAGCCTCCTAAGTAGCTGGGACCACAGGTGTGTGCCACCACACCCAGCTAATTTTTTATT
TTTGTAGAGATGGGGGTCTCCTTATGTTGCCCAGGCTGGTCTTGAACTCCTGGGCTCAAGTGATCCACCTGCCTCAGCCTCCCAAAT
TGCTGGGATCACAGGTGTTTGAGCCACTGTGCATGGCCCAAAGTGCATATCTTGGACACCACTCATATCACCAAGAAAATTTATTTG
TAAGATCACTTTGCTAATTAACAAACTTTCTAGTTCTAATTGAGATAGTGTTGGCTGAAGGACTATCATAGCTACCTCTGGGATATA
GATCTCGGATTAGGTTCTTTCAGATGCTAAGCAACAACCAACCCTGGGCTGTGGATTCTTGATTAGGACACACCAAATGTTAGCCAA
TTCCCCACTAGGATATAGAATAAAAATATAACAAATGTATGTAGCCATACTATTTGTTATTGAAGTGTTTTATAGACTATATAATTT
GGTTTTTTATGTATGGCTTTGTCTTCTAAGTCCTTGCCTATTAAGCCATCAAGGGACTACCACTGTTCTGAACCACTGTGTTAATAA
TTTTTTAAAAGTTTAAGTTTGTTAATCACAATTATATCATTAACAGTTCATCTGAATTACAAATGTGCTAATCACAGGTAAAATTCAC
TGAGGGACTCATACGTGGCAACTATAGTTTGTTTGCATAAACTCTCTTGCTTCTCCCACTGATTCTGAGGGAGGCAGAAATTCAGGC
CCACAGCAAGGTACAGTCAGGCCTTCTGGTGATGGCAGAGCCGGGAACTTGACTCCAGGCCTCCACCTCCTCTCAGGAAATAATTTC
TGTGAGTCCTGGTGTTGCCACTTACTAGCTTGTGGCCTTGACCAAATGATGTAACCTCTCAGGCCCCAGTTTTCCAATCTGTAAAAA
TGGGTAAAATGGTAGCACCTTTCTAGTAAGGATGTTGAAGAATTAAATGCAAAAGTATGTGTATAGTTCCTAACACAGCATCCAGCA
GCTTGCAGACTTAAACACGTGGCATCACTTGATTCTAATTAATTAGAGCTAGGATTCTACCTGCGGACAGGTATGCTTTCCCCTGTT
GCCTAAGGGGGCCAAACTTGCCTAATTCAAGAGTCAGATGGTGCATACTGAGGACTGCGGCCTCAGCCTCTCAAGGGGAAAGCCTGG
AAATGAGTAATTTTGGCAAGTGCCCAAAGCAATCCTTCAGATTAAACAACTGTCAGAAATGCTCCCTGCTCTGTATTTACTTCTGCC
CAAGCTCCAGCCTCTCGTGTGCTTGGTTATCATGCTCTTGGGGCCAAACGTGTGTCATGAGCAGGAAAGCTATTTCTTTGAGCCCTG
CCTGGCTCAACAATAACTCTCCTGAGATCCTGTTTCTTGAAATTCAGGGTTCTGCCTTATTTTAACTTGCAAATTTTGTCACTTCTC
TACTATGGCTGATCCTTTTGAATTCTCAGTTCACTAGCTATTTTTAACCAGACCAAATGTCATCTGGAAAACTAATGTGCACACTTT
GATATCATCAGTGATCGCTAGTGAAAATTGTAAATATCCCAGTCTCAACCCCTCTCCTTCAGCATTTTCTTTGCCTCCAGGTTGAAT
CTAAATCTCTAGGTTTGACCTTTCAGGCGGTTTTGTGCACAAAAGGAATCGTGGTATGAGCAGGTTTTTTCAGTTAACTTGAATATG
TCTTGCCAGATGGGACTGACGTGTATTGGACCACGTCACGAGGGCTCACGAGGGCCAGGCCCTGCAGACAGTGGGTGCTTATCACTATGAA
ACAATGCACGTGGCCGGACAAGGCTTGTTCTTGGCTCAGCTCTGCTTATTACCATGTGCTAATTTAGCATCTTCCGTTTATAATGAT
TTCTTCCAGAAGATTTGGTTTGATGTTTTTCCAGGTGTGGAAGTTATGCGGATAGAATCCTCAATGCAGGCTGCTGCTTTTCATTTG
TTCTTCCATCCAGTGTTTAGGGAACACTGACACTGCCTTCTAGCTAGTCTTCCCGCCCCAGTGTGGATCACCTCGAATCCATCCCCC
ATGTAGCTGCTCCTGGAGTTATCTTTCTAAGACACACATCTGACCTTGTTAGTCAAGGATGAAGTTCCAACTCCTCAGCTCGCCGTA
CAGTCTTGTCTTTCCACTGTCACGGTTCCTGTTGGTAATACAGGCTGCTTCTCATTTGCACACACAGTGTCTCATTTCT
TTAAACCATTGCCTCTGGGGCCACAGTGATGGGATGAGGAAAAGCCAGGGAGGGTGCTTAGGACCTGCTTGGTGGTGAGGATGGATG
CATGTGCTCTGTCTGAGCATATGGACAACTGTGCTGCCTGCCTGGGCCCGGACTTTGCTGGACTGGATAGGAACAGAGAGCTGTGTC
CTTTGTCCCCATCACAGGCTCATTCGGGGGCTGCTGCCTTCCTGCTTGGTCTACTAGGCTTTTATCTCAACTGGACAGTTCTGTCA
TCCCCTGAGACTGGGAGTGGGTCCTGTGAGTGAGAAACTCCAGCTTGTTCTCCCCACCTCTACTTCAGGGGGAGAAGCCCTAATTGA
ACCGTGGACCACGTGACGTGTATTGGACCACGTCACGAGGGCTGACGAGGGCCAGGCCCTGCAGACAGTGGGTGCTTATCACTATGAA
CAACCTCTTTTTTCACACTTAGCTGGCTTACATGATTTTATCTCTTATATATTTGAACAAATTTCAATTGATTCTTCTTTCTCAACC
CCGAGTCTTCACACACAGTTCCCACCCTTCTACGTGAAAAATTGTCCCACAGCACTTATGAACTAGAGAGTGAGTCATGATGGTGTG
TGTTGGTGCATGCATGCGGGAAAGGGGATTCTGGAAGGTGGGGATGTTCTCCAGAATCTCCTCTCCATGGAGGCCCTCAAGGACCAG
GCCTTCCTGGTCTTTGTAGTTCCTGACCAGCCCACTGTGGGCACTATGTGTCAGGGAAGAGATGAGAAGCAAAGATGAATATGATGC
AGTTCTTCTTCTCATGGAGCTTAGGGTCAGTGAAGATCTAAAATACATCAACACTTAGATCTATTAGGTGCTATGAGACTGCACAGG
ATGTGCAGCTTGTCAGTTTGAGGATGGAATGTAGGGATCAGGAAGCCGACACGGAGAAGACAGCAGTAAGCCCACTCCTGAAGAGGG
GGAGTACTCTTGGGTAGCCCTGTTAAATTCTGCCTCATATTAAACCAAAATTGGTTTCTTGATGTTCATTCCTGGTTCCATCTGGCA
TGTCCTCAGCAGATCATCTGTCATCTCTGTTTCCCTTTCCCATCCTCCATGAGTTTAAAATCTTTGATTTGGGGCTTGAGTTTTGA
CAATACATTTCTGTTGAATAAATGAAGTGTCCCACAGTTAAATAAATTTGAGGACTTCCGTGTTCCAGTTCCTCCTTCTAAGACATAT
TAGCGTAGTGCAAGCTCTGGTAAGACCTGCAGAAAAGACTCTTTCATGGCTCTGTAATCATTCTGACGAAGGTTTTTGCTGACACTCG
ATTGAGAACCACTGCTATGAAAGTTTGTCTTGTTTATAATTGACAAAAATTGTATAGGAGGACAACATGTTTTGAAATATACATACA
TTGTAGAATGACTAATTAGAGCTAACTAGCATCTGAATTACCTCACTTATCATTTTTTGTGATGAAAACACTTAAGATCTACTCTTC
TGTGATTTTAAAGTATACACTATATTGTTGTTAACTATAGTACCACGTTGTAAAATAGATATTTTAAACTCATTCCTTCTAACTGAA
ATTTTGCATGCTTTTACCAGCACCTCCCCAATCTCCTCATGCCCCAGCTCCTGGTAACCAACATTCTACTCTCCAAGTTCAACACCT
TTAGATTCCACATACAAGTGAGGAACATGTGGTATTTGTCTTTTCATGCCTGGCTTATTTCACTTACCGTAATATCTTCTAGGTTCAT
CCGTGTTATCACAAATGATGGAACTTCCTTCCTTTTTAAAGCTGAACAGTATTTCATTGTGTGTGTGTATATATATGTGTGTGTGCG
TGTGTATGTATGTGTATGTATGCATATATATACTGCATTTTGTTTATCCAGTTGTTTGTTGTTCGACACTTAGGCTGATTCCATGTC
TTGGCTATTGTGAATGATGCTGCAGGGAACATAGGAGTGCAGATATCTCTTTGACATACTGACTTTATATACCCAGTAGTGGGATTT
CTGGATCGTATGGTAGTTCTATTTTTAATTGTTTGAGTTTCCTCCATACTGTTTTCCACAATGGCTATTGTAATTTACATTCTTACC
AACAGTGTACAGGGTTCCCTTTTCTCCACATCCTCACCAATGTTTGTTTTTTGTCTTTTTGATAAGTCATTCTAACAGGTGTGAGGT
AATATCTCATTGGGGTTTTAATTTGCATTTCTCTGATGATTAGTAATGAGCATTTTTTTCCATATACTTGTTGGCTATTTGTATGTC
TTCTTTTGAGAAATGACTATTCAGGTCCTTTGCCTAGGTTTAAACAGGTTGTTTTCTTGCTACTGAGTTGTTTGAGTTCCTTATAA
ATTTTTTATATTAATCCTTTTTCTAATGTGTATTTGCAAATATTTTCTCCTGATCTGTAAGTTGCTGCTTCACTGTTTCCTTTGCTA
TGTAGAAGCTTCTTAGTTTGATGCAATCCCATTTGTCTATTTTTGCTTTTGTAGTCTGTGTTTTTGGGGTCACACCTAAAGATCAT
TGCTAGGCTGGACATAGTGGCTCACGCCTATAATCCCAGCATTTTGGGAGGCTGGGTGGGTGGGTCACTTGAGGTCAGGAATTTGA
GACCAGCCTGGCCAACATGGCAAAACCCCCTTCTCTACTAAAAATAAAAGGGTGAGTTTCCTCTATCTCGTGAGATGGTTTGCCCTT
CTGTCATAGGATAACACAGCACAAAGTCCTCAACAGATGCTGGTGCCATGTTCTTAGGCTTTCCAGCCTCTAGAACTGAGAGTCAGA
TAAACTTCTATTCCAGCCAGGCATGGTGGCTCATACCTGTAATCCCAGCCCTTTGGGAGGCCAAGGTGGGCAGATCACTTGAGGTAA
```

```
GGAATTTGTGACCAGCCTGGCCAACATGGTGAAACCCTGTCTCTATTAAAAATACAAAAATTAGCTGGGTGTGGTGGTGGGTGCCTA
TAATCCCAGCCACTTGGGAGACTGAGGCAGGAGAATCACTTGAACAAACCTGGGAGGCGGAGCTTGCAGTGAGCCAAGATTGCGCCA
CTGCACTCAAGGCTCAAGGCTGGGTGACAGAGCATGAGGCTCCATCTCAAAAATAAAAAATAATAAAAAAAGATCATTGCCAAGACC
AATGTCAGAGTTTTCTTCTATGTTTTATTCTAGTGGTGTTATAGTTTCAGGTCTTATATTTTGTCTGTAATCTATTTTTAACGGATT
TTCATATATGGTATGAAATAGTCTAATTTCATTCTCCCACATGTGGATATCCAGTTTTTCCAATATTATATATCGAATATATGAGAG
TTTAAGAAGGATAGGGAATGAATCTTTTAAGGGAGCTAATCACCCCAAAATTAATTTTCTCTCTTTCTCCTACACAGCCTCTTGATT
TTGAAACAGCAGCTGTTTCCAACATTGTGTTCAAAGCAGAAAATCCTGAGCCTCTAGTGTTTGGTGTGAAGTACAATGCAAGTTCTT
TTGCCAAGTTCACGCTTATTGTGACAGATGTGAATGAAGCACCTCAATTTTCCCAACACGTATTCCAAGCGAAAGTCAGTGAGGATG
TAGCTATAGGCACTAAAGTGGGCAATGTGACTGCCCAAGGATCCAGAAGGTCTGGACATAAGGTAAAGGGCAACAGTGCTGGGCAGGC
CTGGCTGCGTGGTCACCAAAGCCAATGGGGAGGAGGAGGAGCTGGCCTGCACTGACTCCAGGGCTGACCCTCACAGCATACCCAGCA
ATGATGAAATTGGCTTTGGTTTAGGAAGCTGTCAACACCCTGGGGGCATTCCAAGGGATGGTGGTTCATCAATAACCATCTAGGCTC
AGACAAGTGGGAAGCAGCATGAGGTTCCATTCCAGCCCATGAGCTTCAGGTCCCAGCTGAGAGCTGGATGGTTCAGGAAGTGAGGTT
CACCTCTGAGAGACTAGAATAACCAGGCCCTGTGTCTAAGCCTGTTATCTAGGCTAGGGTTTAGGAAAGGGTTCCAGCCCTAAAGGT
GTATTGGAGTTCTAGACAGAGACCAAAGCCCAATTGGACAGACCATGCCAACTTACAGGGCTGAGGGAATCTTCTGGATGTAGGATA
CATAGGTTCCTTGGCTCCAAAGAGCTGATCTAGGGCACCCCTGGAGAAGCTGGTGCTCTGGCTGAGGTGGCTAAACAAAATGTGTAA
ATATTTCCATCTTAGCAATTCCTTTTTTTCAGTTCAGTAAGACCCACACAGATTTGCCTTAAAAAGTACAATGATGATTAACTTAAG
AAATATATGTTTGTATATATCTGTATAATATCTATAAAATCTTTAAGATCATGTAGTTAAGACTTGAACTCAGATCTTTCGCTGCTG
TTGTACATTACACCTCCTGTCTATGGCATAGGAAATATTTCAGCATGACTTAAATCACGCGTGTGGCTATGAATGGCAGACAATTCC
TTAAGTTTCTCTAAACATCTGGAGGGAGTCTTGCCTCCCATTGAGCTGTCCTTCAGGCACTTGGATTCACTTCTAAGACCTTTATCT
GATCATTGAGAAGCAAAGTTACCACCTGCAGTTTTACTCTAACTACTTCTGTAGTTTTACAGAGAAGAAAGAACAGTAGGGGATATA
CTGTATGGTTTAGTGTAGATACTACTGGGTCAAAAATTATGAGAAGACACAAAATGTTTTTAATCTTTATACCATTTATTTAACAAA
AGTTCCTATCTTCTTCATGAAAATGCTATAAAAACCATTGGTCAAAGACGTGAATAAAAAGTTTATCAGTGGGAAAAATAAAGGCAT
GTAAGAAAATTACTGTGATGAAACAAGGAAAGGATACATATACCATTAAGTTGAGGTCAGGAAGAGGAGGTTAGCAAGGGAGACTGA
GAAGAGGTGTTCTGTAAGGTACAATTCCAGAAGACAAATGAAGAAAGTGTTTCAGGAGTAAGGAATAATCAATAGTTCAGACATGAG
TAATAAGTCTGTTAAGAATTAAGGATGACTCCAGTGTTGGTCTGAGTAATTTAATTCTCCAGCTCCACAAACTTAAAATCTGTTAGC
ACCCAATGGATTTGAATCCAATCCTTCAGCAAATATTATAGATTCTTTTATTGAAATATATTCAGAATCCACCTGCTTCTCCCTGCT
TCCAGCCTGCTCCAAGGCACCACCATGTCTGTTCTGGATTCGTCCTTGCCCCTGCCGTCCCCCACCCCACTCTGAATCTACTCACTG
AATTGCAGTCAGTGACCTTTTAAAAAAGTCAATCAGATCATCTGACTCCTCTTTCAAAACCCTCCAAAGCATTCCATCTCTTTCAGG
ATGAAATCCCAAGTATAAAGGGTTTCATACTCATTACCCATGTATATAACCTAACACAGTGTTTGGCATATGGAATATACTAAAAAA
ATACATTGTGGGAAATTAACACATCATACTTTTTTAATGATTTCTTGTCCTTTGATATGTAGGTTGCTTCCAGTATTTTTTTATTAT
AGAAAATGTTTTAAATAGATGTATGCATGTAATTCTTTTTAAACTTTGATTAAATTGGGTTTTTTTAGGTAGCATTTCCAGGAGTGA
AATTATTTGGTCTAAAGATATGGACATCCTCATGACTATGATGTGTTGCTTTTTAAAGGACTACCATTGGTTTTCCTGTGGTTCTAG
TCTTGTCTGTCTCAACTTCATCTTCCAAACACCTACCAATGAGCTCCGTTGAAAGCACAAATATGGCCAGGTCACCTTCTTGCTTAA
ATCATTGGCTGGCTGCCCATTGCTTCTTGTGATCTGGCTCTTCAAATCTCTGTGGCTTCACCTTTTGCCACCTTCTGCTTCCCAAGT
ATACCCCAGCACTGCTGAAATGCCTGACCCCACACTCACCTGTTCCTTGGCCCTTGGTGCTTTATCTCACACTCTTCCTTCTA
CTTGAAGCGTCTTCTTCTTGCCTTAAACATCTACTACTTCAGAAAGACTTTGCTGATTCCTGTAAACTTCTCTTGGATTCCATAGGG
CCCCCTCTGTTGTGTTTACCACATTTTATTGGAATCAACTGTTTATCTGTCTATCTCACTTGCCTAAGAACACCTTTTTGTGATCTC
TTCCCTTGGCCCACTGCCTGATCTTCCTCAGCACTCAATAGATGTTTGTTGGATGAGGAATTCACTGTGTTACCAGGGTCTATGAGT
ATCCACCCATCTCGCCCAACGTTTCCACCACTGGGTGTTCAAATTTTCTAGGTGGGTGGTTTGCTAAGAGGTTTTAAAAGATACTTC
CAGGTTACTTTAATTTGAGTTTTTGATTGCCAGAAAAGTTTAACTACTTTCCCCAGAAGTTGTGAACTATACCCTTCCTAATACAAG
CACAAACACCACATTGTAAGGATGAACAAATCCTTTTGAAGACGATGAAATGCAAATAAAGCATTAATAATATGAGATAATAGATC
CTTTAAATATGTTTTAAATAATATACAAGTAATGCTGATAATGATAATCTCAGCATTATCATTTGTATATTATTTACAACATATTTC
AAGGATGCACAAACATATTTAGTTGACGCACAAACTAGCTTCACATTTTCATGCAAAGTAATGCAGGCTTTCTTTTGCTCTTTTCAT
GGACAGAGAAGCCGGAAGTCCATATCGGGTACAAGTGGTGGCCACAGAAGTAGGTAAGCAAAAAAAAAAAAAAAAAAAAAAAAAGGA
ACACAGATTATCAGAAGATACATGGGTTGAAATGGGAGGGAGGAGGATAAGAAAACTGACAGGGCCAAAACACCAGAATATCACAACCTT
AAAGTGGAAGGATGAGAAGGGTACAAAATATAAGGGAGTGCTCATATCTTCCTTTTTTTTTTTTTTTTTTTTTTTTTTGAGATGG
AGTCTTGCTCTGTTGCCCAGGCTGGAGTGCAGTGGTGCGATCTCCACTCACTGCAACCTCCACCTCCTGGGTTCAAGCAATTCTCCT
ACCTCAGCCTTCCGAGTAGCTGGGATTACAGGCACCCACCACCATGCCTGGCTAATTTTTGTATTTTTAGTAGAGACAGGGTTTCAC
CATGTTGGCCAGCCTGGTCTCAAACTTCCGACCTCAGGTGATCTGCCTGCCTCCACCTCCCAAAGTGCTGGGATTACAGGTGTGAGC
CACCACGCCCAGCCCCTTTGTGGTTTTGTTAAAGGTGAAAAACACCTGTGCCCCCAGCAGGGCTGCTGCTCACTCTGTGGTTTCCTG
TAGGGGCTATGGCCTCTTCTAGAGAGTTCTGTGTCCCTGGCTGAACTACCTCCCTTCTTCCAATGTGGTTGTGGGTTTCCCCCAGGA
GCTCTGAGTGGAATCAAACCACTGACCTGTTCACGTAGACCACCACTTGCTCCTTCAGCCTTAAATTGTAACTCTCATCCTTTTTGA
AGCAGAAAATGTTTTATGAAATGTTCTCTACTTTGATGTGTGTGAGAATCCCCGGGGAGCTTGTTGAATAACACTAGGAGTCCACCC
CTGGAGTTTCTGATTCCATAGGTTTGGGTGAACAAGGGGTATCTGTGTTTTGACGAAATCAGGTGAACTTTTGAAAGGAAAGACAGA
AAAGCCCAGGATGTTTGAGCCATTCCTGTGTAATCTTTGTAATCTTTATTTATTTTAGTTGTGTCTGGATATATATTGCCTGCTGGCTCCA
ACTGTGGAGTTCCAGAGAACTTTTTTAAAATAAAAAATAAACCATAGGAATAGTATGTTTACCCAATAATAAGCCTGGCAACTAAAA
AAGAGTTTCTTTGCATTTTCATGGAATTTTCAGACATACAAGTAGGAAATCTGTTGATCCAATGTTGACCAGTCTCTAATTAGCTAG
TAAATGTATCTGGCAGGTTTACCAAAAAAGAGAATTAAACAAATTACTCAATAAATATGTTTTTAGACTCCCCCACCACAGCCCCGG
AAAAAAACTTTCAAAATGTGAGGGCCAGGGTTGAGAGCTGAATTGGAGAAGAGAGACAGGAATCATAAAAACGCCCCTGCATGTGG
AAAGATTAAGAAAAACAAAATTACTTCAGTCAAGTCAAGTCAAAAACCAAAAAGGGTAGAATGCAACAGGTGTGCCAGCTTGGGAGGGGTGTG
TGTGAATGGACAGGCCCTCTGCTTCTGTCTCTTGCTTGAGTGCAGCACATTCCACTATCACATTGTATACATTTAACTCTTGATTTA
AAACCTCCCAGTGAAGCAGCAGGCAGGTTCTGGCTGAGCCTTTATGCTGTGCACACCCCTTTCTCATACCATGGGGATCAGAACTTC
ACTCTGGATGTGTCCCAAGAGTTTCTCACATTCTAGAAGTTTCAGAAATCAAAACCAGGGTCCAAAACCCTGTTCTGTTTTTAGTTT
CTGGCACTGACATGATATAAATAGATACAATAACTTCGAGAACACAGTGGCCTTTTCTTCTTTGGTTAAGTATAGCCATGGACATAG
ACGATGTTGAGAAGAAAATCAAGAAACACAGGGTTTACTGGAAATCAGCAAGAAAAACATTACTGGAATCAAAGGTCAGACATCAGA
AGTTGTAGAAACAATGTCTGTGATTGGTGTTGAGGAGGACATGATAACAGTCAGTGTTTATGTTGCACTGACAGCCCCATACTGAGC
ACTTAGTGTAATAACTTACTCCTCATACAACACTATAACGTAGGTGCTATTATTGACCACATACTGTGAACAAGGAAACCAAAGCAC
TGAGAAATTAAGTAACTTGCCCAAGATTACATAGCTACCAACCAGTTATGCTGCAGATCCAAGTTTTTTACTCCTATCATATAGTAA
ATAATGTCTGTGGAGCCTTTTACTATTTTCCAGAACATTCACTCATATGTGGGATTCTTGCAAAGCCACACAGCTGAAGAAAATAAG
CTTAGAAAACTCTACTAACATGCCTAAGGCCTGCTAAAGAGCAAGTGAAGGTGTGAGTGCTAGAGTTAGACCTCTTGGATTGAAAAC
CTCTCTGCCTTGGGCAAGTTACACAACTTCTGAGCTCAGTTTTTCACAATTATTTTGAAGTAATATATGTAAAAATATTAGCACATATC
TCTTAGTTATTATTATGATGGTCAGTAAGACTTTTTCACCAGTAATGAAACTAGGTCTTTGATTCTAAAAGCTTGTCGTCTTTCTAG
AATCATTCAATTCCTTAGGGGGGTCTTCCTTGAGCTCTGTGTCAGAGTTCCACCTGATCCTTATGGATGTGAATGACAACCCTCCCA
GGCTAGCCAAGGACTACACGGGCTTGTTCTTCTGCCATCCCCTCAGTGCACCTGGAAGTCTCATTTTCGAGGCTACTGATGATGATC
```

```
AGCACTTATTTCGGGGTCCCCATTTTACATTTTCCCTCGGCAGTGGAAGCTTACAAAACGACTGGGAAGTTTCCAAAATCAATGGTG
AGTTGTCAGAAATACATGGAAAAAAACATAGATGGATGATGAAAGTAGGTTTTATTTAACTTGGTGGGTTTGCTGTACTCAGCAAAG
AGCTTTCTTTCATGCTTCAGGAAGGAAGTTCATGCTTTGGAAGCTTGTACAGGGAAGGACTCCAGAGGGGAAAAAGCCCCAGGATTT
CAAGGGAGGAAAGATTATGACCACACAGCATTCTCTTGGTGTAGATGAACAGTTGGATGACCTAAGCATCATTTGCAAAATTTTCAA
TGACAGATGGAAAGCAGATTTTATAGAAATTTTTAAAAACTTTGAGATAGATATATATATTCAAAGTATACAATTTTATGAGAATTG
ATTACATGTAAATACCACTGAAGCCATCAGCACAATCAAGACAATGAACATTTCCACCACCTCCAGAAGATATTTCATGCCCTATTA
TAATCTCTGCTTCCCACCCATCCCCTCTCCCCAGGCAACCATTGAAGTGCTTTCCATTACTATAGTTTTCATTTTCAATAATTTCAT
CTAAATAGAATAGTATAGTATGTATATTTTTTTGGTCTGGCTTCTTTCATCTCAGTATTTTGAGATGTATCGGTGTTGTTACATGC
ATCAGTACTTCATTCCTGATTATTGAGGGGTAGTATTCCATTGAATGACTATACCTGTTCACCTGTTGATTGTAACTCCAACTTTTG
CTATTACAAAGCTGCTGTGAGGATTTGTGAACAAATATTTTGTAGGGACAAAGATTTGCAGTTTTCTTGGTTAAATACTGAGGAGTG
GAATGGCTGGGTCATATGGGAGTTATATGATTCAGTTGTTAAGGAAAGGTTGAACTACTTTCCAAATTGTTCTTAACATTTTACATT
CCCACCAGTGTGTGAGAGGTCCAGTTGCTCAGCATTCTCCCAGCACTTGCTGTGCTCAGCCTCTTTAATTTTAGCCATTCCAGTGGG
CGTGGAGTAGTATCTCATAATGGTTTTATTTGTATTTCTCTAATGACTAGCGGTGTTGAGCATCTTTTCATATGCAATTTGCCATCC
ATCTTCACTGGTGAAGTGTCTTGTTAGATATTCATTTTTTAAAAATTAGGCTGTTTGGATTCTATTGCTGAGTTGCAAGCATTTTTA
ATATACATAGATGATGTGCTTTGTTAGATATATATCTTTTGTAAATATTTTCTCCCAGGCCATGGCTTGTCTTTTCATTTTGCAAGT
CTTTTAAAGAGCAAATTTAAAAATTTTGGTATCTATTTTTTATTCATGTTTTATTCATGATTTGTATGTTGTGATGCTTAAATTTTTGC
CAAACTTAGGGTCCCTAAAGTTTTCTGTTTTTTTCATTTCCCAAATTTTATAGTTTTTAGCTTTTTGCATTTAGCTCTATGCCTTTTTA
AGGTAATTTTTGTATAGGCATACCTCATCTTATTGCACTTTGCTTTATCGTGCCCAATGGATCTTCCATTTTTTTTATAAAATGAAGG
TTTGTGGCAACAAAGTCTCTTGGTGCCATTTTTCCAACAGCATGTGCTCACTTTGTATGTCTGTCACATTTAGGTAATTCTTAAACT
TTTTCATTATTATATCTGTTATGCTGATCAGTGATCTTTGATGTTATTATTATAATTATTTTGGGACACCCGGAGCCATACTCACAT
AAGACGACAAACTTAATCTGTCAATGCGACGTCGACGTACGTTCTGACGCTCCACAGACTGGCTGTTTCCCCATGTCTCTCTCTC
CTTGGGCCTCCCTATTGCCTGAGCACAATATTGAAATTAGGCCAATTAATAACCTTAAAATGGCATGTAAGTGTTCAGGTGAAAGG
AAGAGTCACACATCTCTCACTTTAAATCAAAAGTTAGACGTGATTTAGCTTAGTGAGGATGTTATGTTGATAGCCAAGATTGGCCAA
AAACTAGGCCTCTTGCATGAAATGCTTAGCCAAGTTGTGAAGGCAAAAGTTATTGAGGGAAATTAAAAGTGAACCTACAGTGAACAC
AAATAATAAAAAAGCAAAATAGCCTTATTGCCATTAAGGGAGAAAGTTTGAGTGGTCTGGATAGAAGATTAAAACAGCAACAATAGT
CCTTTAAACCAAAATCTAACCTAGAACAAGGCCTTAACTCTTCAATTCTGTAAAGGCTGACAGAGGTGAGGAAACTGCAGAATAAGT
TTGAAGCTAGCGAGGTTGATTTATGATGTTTAAGGAAAGAAGCTATTTCCATAACAAAGTGCAAAGTGATGCAGCAAATTGATGCA
GAAGCAAGTAATCCAGAAGATCTGAGATCCTTAATGAAAGTGGCTACACTAAACAAGCTTTTCAATGTAGATGAAACAGCCTTCTTT
TGGGAGACAATGCCATCTTAGACATCCATAGCTAGAGAAGTAAATGCCTGGCTTCAAAGCCTCAAAGGACAGGCTGGCTCTTGTTAG
GGCTAATGCAGCTGGTGACTTTAAGTTAAAGCCAACCCTAATTTAGCATTCCTAAAATACTAGGGTCCTTAAGAATTATGCTAAATC
TACTTTGTCTGTGCTCTGTCAATGGAACAACAAAGCCCAGATGACGACAGCACATCTGTTTACAGCATAGTTTACTGAATACTTTAAGGC
CACTGTTGAGACCTACTGTCCCCCAAAAAAGATTCCTCTCAACATATTACTGCTCATTGACAATGCACCTGGTTACTCAAGAGCTCT
GGTGGAGATGTACAAGGAAATGAATGTTGTTTTCATGCCTGCTAACACAGCATCCATTCTGCAGGCCCCAGATCAAGGAGTAATTCT
GACTTTCAAATTATTTAAGATATACATTTCATCAGGTTATAGCTGCTATTAGATAGTAATTCCTCTGATGGATCTGGGCAAAGTAAA
TTGAAAACCTTCTGGAAATGATTCACCAGTTTAGATATCATCAAGAACATTTGTGATTCATGGGAGGAGGTTGAAATATACACATTA
GTAGGAGTTTGGAAGAAATTGATTCCAACCCTCATGGATGACTTTGAGGGGTTCAAGACTTCAGTGGAGGAATCACTGAAAACGTGG
TGAAAATAACAATAGAGCTCGAATTAGAAGTGGAGCCTTGATGATGTAACTGGATTGCTGCACTCTCATGATCAAACTGGAATGAAT
GAGAGTTGCTTCTTACAACTGAACAATGTGTTTTGTTTGTTTGTTTTGTTTTTGAACAATGGAATCTGCTTCTGGTGAAGATGCCATG
AACATTGTTGAAATAACAAGCAAGGATTTAGAATATTCCATAAACATAGTTGATAAAACAGTGGCAGTGTTTGAGAAGACTTACTCC
AATTTTTTGAGAGATGTTGTCTTGTGGGTAAAATGCCATCAACCAGCATCACATGCTATACAGAGGGCTCTCTCATGAAAGGAAGAGT
CAATAAGTGCAACAAACTTCATTGTTGTCTTATTTTCAGAAATTGCCACAGCCATCCCAGCCTTCAGCAACCACCAACATTGAGACA
GGAAATGATTTGCAGCAAAAAGATTTATGACTCATTGAAGGGTCAGTAATCATTAGCATTTTTTGGCAACAAAGTACTTTTAAATTA
AGGTATGGACATTTAGGGGTTTTTTATTTTTAGGTATAATGCTATTAACAGTCTAGAGTATAGTATAAGCTGTTATTTCAGTAGAAA
AACAAAAAATCTGTGTGACTTGCTTCACTGAGCTATTTGCTTTATTGCAGTGGTCTGGAACCAAACCCATATCGCTGAGATATGCTT
GCATAGGGGGTGTGGTAAAGACCAAAGTTTATTGTTTTTAACATAGCTATCTAACTGTTCTAACACCATTTGTTTAAAACATCCTTT
TTTTACATTGAATTATCTTAACAACTTTGGCAAAAATCAAGTGACCACCTATACATAAATCTATTTCTGAATTTTTCTGTTTCATCA
ATCTGTTTCTATTTTTATACCAATAACACATTGTCTTGATTGCTGTAGCTGTATAAGGCTTGAAATCAGGTAGATTAATTTTTCCGA
CTTTGTTCTTTTTCAAAATTCCTTCATTCTATGTCTTTTTCATTTCTGTATAAATTTTAGAATGTAGCCTGTTCCTTTCTACCAAAA
AAATCCTGTTGAGATTGTATTGAATCTACTGGGGAATCTGGAGGAGAATTGATATATTAAGTCTTCCAATTCATGAACATGGTATAT
GTCTCCATTTATTTAGCTCTTTAATTTCGTTCAGCAATGTGTTTTAGTTTTTCAGTGTATAGTTCTTACAAAATTTTCCCTAAGCATT
TCATATTTTTGATATTTTAAATATTTCAATTTCTAAATATTTCTAGATATAGGAATATAATGATTTCTGTATACTGATCTGTATTCT
AGAGGCTTGCTGACCTTATTTAGATTTCATAGATTTTTCTACATAGACAATCATGTTGTCTGTGAATAATGACTTCTTCCTTTTCAGT
CTGTATGCCTTTTGTTTATTACTGGCTGAAACCTTCAGTGAAATGTCAAATAGAGGTAGTAAGAGCAGAAATACTTGACTTGTTCAT
GATCTTAGGAAGAAAGCATTTAGCCTTTCACCATTAAATATGATGTTAGCTGTAGGTTTTTTATTGTAGGTCGTCTTTATTAGATTT
AGGACTTTGCCTTTTATTCCTATTTGTGAGGGTTTTTTTTTTTCCTAAATTGGAAATGGATATTAAATTTTTCAAGTGCTTTTTCTC
CATGTATTGACTATGTGGCTGATTTGATTTTTTGGGCCTATTAATTGATGCATTATTATCAACTTTTGAATCTAAAAATCACCTTAT
TCCTGGGATAAAGCCTACTTAGTGATGAAATATTACCATGTTTATGTATTTGGATTTGCTAAAACTTAATTTTTGCACTACTATATT
CATGAGGGATGTTGGTCTCTCTCTATATTTTGCCTTGTATAATGAGGAAGCATTCCACCTCTTTATCTTCAATTATCTGGACAAGTT
TGCATAAAATTGGTATTACTTCTTCTTAAAGTTTGAAAGAATTTGCCAGTAAAGTCATCTGGGCCTGGTGTATTTTCTTTTTAAGAA
GTTTTAACTACAAATTCAATTTTATAGATATAGAAAAATTCAATTTATATATTTCTTTTTGGTTGAGCTTGTACCTTTCAAATAATT
CATCTATTTCATTAATCTAATGTATTGATATAGTTCATATTTTTATTAATATCTGTAGAATATGTAGTGATATCACCTCATTCTGATA
TTGGCAAATTATCTTTTTTCCTGATAATCTGGCTGAGAAGTTTTATTGATATTTTCAAAGAACCAGCTTTTGGTTTCATTGATTTTTT
TTCTGTTTTGTTTTATATTTCACTGGCTAGATCTTTATTGTTTCCTTTATTCTTCCTACACTGTGTTTTTGTTCGTGTTCTGTTTT
CATTAAGGTGGAAGCTGATGCCATTAATTTGAGATCTTTTTGTTAAACAGGCATTTAGAGCTATAAATTTATCTTAGGTGTCACATT
AGCTGCATTTTTTTTTTAATAGACAGGGCCTCAGTCACCCAGGCTGCAGTGCAGTGGTGTGATCATGGCTTACTGCAGCCTTGAATT
CCTGGGCTCAGACGATCCTCCTGCTTCAGCCTCCCAAGTAGCTGGGACTACACGTATGAGCTACCACACTTGGCTAATTTTTTATTT
TTAAATTTTTTGTAGAGATGGGGGTCACACTATGTTGCTCTGGCTTGTCTCAAACTCCTGGGTTCAAGTGAGCCTCCCACCTCAGCC
TCCCAAAATGCTGGGATTACAGGTGTGAACCACCATGCCAGGCCCACAAAATTTGATACATTGTGTTTTCCCTTTAGATTTCTTTTG
TGATTATGTGTTATTTAGAAGTGTGTTATTTAGTTTCCAAATATTCTGGGAATATTTCCTATACCTTATACCTTTCTAACTTCTAAT
TTAATTACATCATAACCAGAAAACATGACTAGAATACTTTTAAATTTATTTAGACATTTTTTCTTTCAGTACTTTGAAGGTATTTCA
CCACCCCCCCTTTTTTTTTATTTTTTTTTTATTTTTTGGAGACAGTCTTACTCTGTTGCCCAGGCTGGAGTGCAGTGGCACAATCTC
AGCCTCCCAGGTTCAAATGATTCTTTGTGTCCTCAGTCTCCCTAGTAGCTGGGATTACAGGTGCACACCATGATGCCCAGCTACTTTT
GTATTTTTAGTGAGACAGGGTTTTCCCATGTTGGCCAGACTGGTCTCAAACTCCTGGCTTCAAGTAATCCTGCCTGCCCCCAGCCTCC
CAAAGTGCTGGGATTATAGGCATGAGCCACCGGGGGTGCCCTGCCCACTATGGTTCTTCCTTGTTCCATTGAATGTTTGTATTCTTT
TTTCTCTGGGTGCTTTTAAGATATTCTCTTATTACTTTTAAGTAATTTGATGTTCATATTGCCTTGATGGCATTTTATTCATTTTTC
TTATTCCTGAAGTTTGATGATCATCTTGGATCTGAGGATTTGGAGTTTTCATCTAACATGGAAAAAATATTAGCCATTGTCTTATCA
```

211

```
AAAATGTTTTTCTCTCCACTATCTTTCTTGGCTTTCAGGAATTCGATTACACATGTATTAGGCCACTTGAAGTTGTCTCAAAGCTTA
TTTTTTTTCTATTTCTTTTTGGATAGCTTTATTTCTATATCTTCAAGTTTACTGTTTTTTCCTGCTATATTCAATGGCTATATTATA
GCCATTCATCCAACCTAGTCTATTTTTATATCAAACTTTGTAGTTTTCTTCTATAAATTAACTTGGGTTTTTAAAATGTTCTGTATC
TATCTACACTTCTCAGTCTTGTAGCTTCTTGAACATATAGAATATAATTATAACTGTTTTCATGTCTTTTCCCACTATTTATGTAAT
ATGTATAATTTCTGGGTAGGCGTCAATTGATCTTTTCTCTCATAGGTCATATTTTTCTCTTTTACTGAATGTCTGGCAATTTTTTAT
TAAATGCCAGACATTTAGATGCTGAATATTTTGTATTCCCATAAATATTCTTGAACTTTATTGTGAAATACAGTTAGATCATTTGGA
AAAAGTTTGATGCTTTCATGTGTTGCTTTTAAATTTTCTTAGATAGGGCCAGGGCAGCATTTATTCTAGGGCTAATGTTTTCAAACT
TGTGAGGTGAAGCCTTCTATGTATTCTACCTGGTAGCACATGAATTATGAGATTTTTAGCTCTAGGGACCAGGAATGATTCCTGGCT
CTTTGTGAGCGCTGTGTTCTCTAATCTTTTTAGGTGGTTCTTTCCCCAGCCTTGAGTAGTTCCTTCATATAAATACACTGATCAGTA
CTCAGCTAAAGCCTTAAGAGACCCCTCTATAAATCTCTTTAGTGTGCATTCTCTTTTGCAGCTCTCTCTGGGACTCTGCTCTGTAAAC
TCTAGTTTCTTAACTTTGCCTTACTCCTGTGCTCCATCACCTCAACTCAGGGACATCACTGGGCTCTGTCTGAGCTCCTGCTCCCTG
CAACTGATACCTGGATATTTGCTTTGGGTGGTAAGCTGAGGCAATTATAAAATTTACCTCATTTGCTTTCCTTCTTTCAGAAATCAC
TGTCCATTGCCTAATATATAAATCTTTAAACCCATGGTTTCACATATTTTTTGTTCTTTTAGTTGTTTTTGGTGGAATGGTAAATCT
GGTCCCTGTTACTATATCTTATCTAGGTGGGCCATAATAATTTTTAAATATGCATATTTAAATCTTCCCAAATTTGGGAAAATTTCT
TGGCAGATATGAATAGGGAGGGCATAAGTGTGAATTGTAGCAAAATCAAATTTTTGTTTAGAAATTTGAAGTTTAGTGTTTTTGTAC
TCTCAATTTTCTATTTTAAAAAAGGTGGTAAATGCCCTGAAAACCAGGTATAATATCATGCATACCCTTATTTCTGGACTCAGAGGG
ATTTTGAGGCCTATAAATGTTATGACTTACCCAATTACATAGCTAACAAGCAGCAGATTTGAAACCCAGAATCAGGTCTACAGAAGC
TCAGTCCCAGGTACTCTCCAATCTTCTGACACATACAGTCGATCCCCATTATTTGCAGATTGTGTTTATAAAATAATCTACATGATA
AAATTTGTAACCCAAAAGTCAGTACTTACAGGAGCTTTCGCTTTTGCTTTCATGGACACTGGCAGGGTGGTGAAAAATACTGGACAC
ACATATTCCCAGCTCTCACATTGTTAAGTTAGTTAGGTTTTTTTTTTACTTTTTGCAGTTTATGTAGTGCCCTGTTTTTCACGTTTCT
GTGCTTTTTCATTGGTGATTTCATTGTTTAAAATGGCTCTGAACTGGGTGGGGTGTCGCCTCACCTAGGAAGCACAAGGGTGAGGTGA
TTTCCCTTTCCTAGTCAAGGGAAGCCATGACAGACTGTACCTGGAAAAACAGGACACTCCCACCCAAATACTGCACTTTTTCCAAGA
TCTTAGCAACTGGCAGAGAAGGAGATTCTCTCCCGTGCCTGGCTTGGCAGGTCCCGTGCCCACAGAGCCTTGTTCACTGTTAGCACA
GCAGTCTGAGATCAAATTGCAAGGTGGCAGCCTGGCTAGGAGAGGGGCATCCACCATTGCTGAGGCTTCAGTAGGTAAAGCGGCTGG
GAAGCTCAAATGGGGTGGAGGCTACAGCAGCTCAACAAGGCCTACTGCCTCTATAGACTCCATTTCTATGGACAGGGCATAGCTGAA
CAAAAGGTAGCAGATAACTTCTGCAGACTTAAACATCCCTGTCTGACAGCTCTGGAGAGAGCAGCGGTTCTCCCAGCATGGCATTTC
ACCTCTGAGAACAGACACACTGCCTCCTCAAGTGGGTCCCAAACCCCCATGTAGCCTAATTGGGAGACACCTCCCAGTAGGGGCTGA
CTGATACTTCATATAGGTGGGTAGCACTCTGGGACAAAGCTCCCAGAGGAAGGATCAGGCAGCAATATTTGCTGTTCTGCAATATTT
GCTGTTCTGCAGCCTCCACTGGTGATACCCAGGCAAACAGGGTCTGGAGTGGACCTCCAGCAAACTCCAACAGACCTGCAGCTGAGG
GACCTGTTAGAAGGAAAACTAACAAACAGAAAGGAATAGCATCAACATCAGCAAAAAGGACATCTACACCAAAACCCCAACTGTAGG
TCACCAACATCAAAGACCAAAGGTGGGAGAGAAACCAGAGCAGAAAAGCTGAAAATTCTAAAAACCAGAATGCCTCTTCTCCTCCAAA
GGATTGCAGCTCCTTGCCAGCAACGGAACAAAGCTGGATGGAGAATGACTTTGACGAGTTGACAGAAGCAGGCTTCAGAAGGTTGGT
AATAACAAACTTCTCTGAGCTAAAGGAGCACATTTGAACCCAACGCAAGGAAGCTAAAAACCTTGAAAAAGGGTTAGATGAATGGCT
AACTAGAATAAACAGTGTAGAGAAGACCTTAAATGACCTGATAGAGCTGAAAACCATGGCAGGAGAACTTCGTGATGCATGCACAAG
CTTCAGTAGCTGATTCAATCAAGTGGAAGAAAGTGTATCAGTGATTGAAGATCAAATTAATGAAATAAAGCAAGGAGAAAAAGAAGAG
TAAAAAGAAACAAAGCCTCCAAGAAATATGGGACTATGTGAAAAGACCAAATCTATGTCTAATTGGTGTACCTGAAAGTGACGGGGA
GAATGAAACCAAGTTGGAAAACACTCTTCAGGATATTATCCAGGAGAACTTCCCCAATCTAGCAAGGCAGGCCAACATTCAAATTCA
GGAAATACAGAGACCACCACAAAGATACTCCTCGAGAAGAGCAACCCCAAGACACATAATTGTCAGATTCACCAAGGTTGAAATGAA
GGGAAAAATGTTAAGGGCAGCCAGAGAGAAAGGTCGGGTTACCCACAAAGGGAAGCCCATCAGACTAACAGTGGATCTCTCTACAGA
AACCCTACAAGCCAGAAGAGAGTGGGGGCCAATATTCAACGTTCTTAAAGAAAAGAATTTACAACCCAGAATTTCATATCCAGCCAA
ACTAAGCTTCATAAGTGAAGGAGAAAATAAAATCCTTTACAGACAAGCAAATGCTGAGAGATTTTGTCACCACCAGGCCTGCCTTACA
AGAGCTCTTGAAGGAAGCACTAAACATGGAAAGGAACAACCAGTACCAGCCACTGCAAAAACATGCCAAATTGTAAAGACCATCGAT
GCTAGGAAGAAACTATATCAATTAACAGGCAAAATAACCAGCTAACATCATAATGACAGGATCAAATTCACACATAACAATATTAAC
CTTAAATGTAAATGGGCTAAATGCCCCAATTAAAAGACACAGACTGGCAAATTGGATAAAGAGTCAAGACCCATCAGTGTGCTGTTG
GGAGACCCATCTCACGTGCAGAGAAACACATAGACTCAAAATAAAAGGATGGAGGAAGATCTACCAAGCAAATGGAAAGCAAAAAAA
GCAGGGATCGCAATCCTAGTCTCTGATAAAACAGACTTTAAACCAACAAAGATGAAAAGACAAAGGAGGCCATTACATAATGGTA
AAGGGATCAATTCAACAAGAAGAGCTAACTATCCTAAATATATATGCACCTAATACAGGAGCACCCAGATTCATAAAGCAAGTCCTT
AGAGACCTACAAAGACTTAGACTCCCACACAATAATCATGGGAGACTTTAACACCCCACTGTCAATATTAGACATATCAATGAGACA
GAAGGTTAACAAGGATATCCAGGACTTGAACTCAGCTCTGCACCAATCAGACCTAATAGACATCTACAGAACTCTCCACCCCAAATC
AACAGAATATACATTCTTCTCAGTGCCACATAGCACTTATGCCAAAATTGACCATATAATTGGAAGTTAAGCACTCCTCAGCAAATG
TAAAAGAACAGAAATCACAACAAACTGTCTCTCAGCACCACAGTGCAATCAAATTAGAACTCAGGACAAAAACTCACTCAAAACCACA
CAACTACATGGAAACTGAACAAGCTGCTCCTGAATGACTACTGGATAAATAACGAAATGAAGGCAGAAAATAAAGATGTTTTTTGAA
ACCAATGAGAACAAACACACAACGTACCAGAATCTCTGGGACACATTTAAAGCAGTGTGTAGAGGGAAATTTATAGCATTAAATGCC
CACATGAGAAAGCAGGAAAGATCTAAAATTGACACCCTAACATCACAATGAAAAGAACTAGAGAAGAAAGAGCAAACACATTCAAAA
GCTAGCAGAAGGCAGGAAATAACTAAGATCGAACAGAACTGAAGGGAGATGATAGAGACACAAAAAACCCCTCAAAAAAAACAATGAA
TCCAGGAGCTGGTTTTTTGAAAAGATCAACAAAAATTGATAGACTGCTCAGACTAATAAAGAAGAAAGAGAGAAAGAATCAAATA
GACGCAATAAAAAAATGATAAAGGGAATATCACCACTGGTCCCACAGAAATACAAACTACAATCGAGAATACTATAAACACCTCTA
CACAAATAAACTAGAAAATCTAGAAGAAATGGATAAATTCCTGGACACATACACCCTTCCCAAGACTGAACCAGGAAGAAGTTGAAT
CTCTGAATAGACCAATAACAGGCTCTGAAATTGAGGCAATAATTAATAGCCTACCAACAAAAAAAGTCCAGGATCAGATGGATTCA
CAGCTGAATTCTACCAGAGGTACAAGAAGAGCTGGTATCATTCCTTCTGAAACTATTCCAATCAATAGAAAAAGAGGGAATCCTCCC
TAACTCATTTTATGAGGCCAGCTTCATCCTGATGCCAAAGTCTGGCAGAGACACAACAAAAACAGAGAATTTTAGACCAGTATCCTT
GATGAACATCGATGTGAAAATCCTCAGTAAAATACTGGCAAACTGAATCCAGCAGCACATCAAAAAGCTTATCCAACACAATCAAGT
TGGCTTCATCCCTGGGATGCAAGGCTGGTTCAACATACACAAATCAATAAACATAATCCATCACATAAGAAAAACCAATGACAAAAA
TCACATGATTATCTCAATAGATGCAGAAAAGGCCTTTGACAAAATTCAGCCCTTCATGCTAAAAACTCTCAATAAAGTAGGTATTGA
TTTGAAAACCGGCAGAAGACAAGGTGCCCTCTCTCACCACTCCTATTCAACACAGTGTTGGAAGTTCTGGCCAGGGCAATCAGGCA
AGAGAAAGAAATAAAGGGTATTCAATTAGGAAAAGAGGAAGTCAAATTGTCCCTGTTTGCACATGACATGATTGTATATTTAGAAA
CCCTATTGTCTCAGCCCAAAATCTCCTTAAGCTGATAAGCAACTTCAGCAAAATCTCAGGGTACAAAATCAATGTGCAAAAATTACA
AGCGTTCCTATACACCAAAACAAAGAGAGCCAAAACATGAGTGAAGTCCCATTCACAATTGCTGCAAAGAGAATAAAATACCTAGGA
ATCCAACTTACAAGGGATGTGAAGGACCTCTTCAAGGAGAACTATAAACCACTGCTCAACGAAGTAACGGAGGACACAAACAAATGG
AAGAACATTCCATGCTCGTGGATAGGAAGAATCAATGTCGTGAAAATGGCCATACTGCCCAAGGCTAATTCATAGATTCAATGCCATC
CCCTTCAAGCTACCAATGACCTTCTTCACAGAATTAGAAAAAACTGTAAAGTTCATATGGAACCAAAAAAAGAGCCCGCATTGCCAAG
ACAATCCTAAGCAAAAAGAACAATGCTGGAGGTGTCACACTACCTGACTTCAAACTATACTACAAGGCTACAGTAACCAAAACAGCA
TGGTACTGGTACCAAAACAGATATATAGACCAATGGAACAGAACAGAGACCTCAGAAATAACACCACACATGTACAATCATGTGATC
TTTGACAAACCGGAGAAAAACAAGCAATGGGGAAAGGATTCCCTATTTAATAAATGGTGCTGGGAAAACTGGCTAGGCATATGTAGA
AAGCTGAAACTGGATCCATTCCTTACACCTTACACAAAAATTAATTCAAGATGGATTAAAGACTTAAATGTTAGACCTAAAACCATA
```

EP 2 204 376 A2

```
AAATCCCTAGAAGAAAACCTAGGCAATACCATTCAGAAGACAGGCGTGGGCAAGGACTTCATGACTAAAACACCAAAAGCAATGGCA
ACAAAAGACAAACTTGACAAATGGGATCTAATTAAACTAAAGAGCTTCTGCACAGCAAAAGAAACTACCATCAGAGTGAACAGGCAA
CATACAGAATGGAAGAACATTTCTGCAATCTACCCATCTGACAAAGGGCTAATATCCAGAATCTACAAAGAACTCAAACAAATTTAC
AAGAAAAAAACGACCCCATCAAAAAGTGGGCAACAGATATGAACAGACACTTCTCAAAAGACATTTATGCAGCTAACAGACACATCA
ACATGATCATCACTGGTCATCAGAGAAATGCAAATCAAAACCACAATGAGATACCATCTCACACCAGTTAGAAGGGTGATCATTAAA
AAGTCAGGAAACAACAGTTGCAGGAGAGGATGTGGAGAAATAGGAACACTTTTACACTGTTGGTGGGAGTGTAAATTAGTTCAACCA
TTGTGGAAGCAGTGTGGTGATTCCTCAAGGATACAGAACCTGAAAGACCATTTGACCCAGCAATTCCATTACTGGGTATATACCCA
AAGGATTATAAACCATGCTACTATAAAGACACATGCACACGTATGTTTATTGTGGCACTATTCACAAGAGCAAAGACTTGGAACCAA
CCAAAATGTCCATCAATGATAGACTGGATTAAGAAAATGTGGCACATATACACCATGGAATATTATGCAGCCATAAAAAAGGATGAG
TCCATGTCCTTTGCAGGGACATGGATGAAGCTGGAAACCATCATTCTCAGCAAACTATCACAAGGACATAACACCAAACACTGCATG
TTCTCACTCATAGGTGGGAATTGAACAATGAGAACACTTGGTCACAGGGCGGGGAACATCACACACCAGAGCCTGTCAGGGGGTGGG
GGTCTGGGGGAGGATAGCATTAGGAGAAATACCTAATGTAAATGACGAGTTGATGAGTACAGCAAATCAACATGACACATGTATACC
TACGTATCAAACCTGCACATTAAGTTCTAGGGTACATGTGCACAAAGTATTAAAAAAAAAACCACACAGGGTCAAACCTAAAAGATGAC
CCATGATAAAAAAAAAAAATGGCTTTGAACTATAGTGCTGAAGTGCTATCTAGAATTCCTAAGCACAGGAAGGCTGTGATGTGTGCCT
TACAGGGAAAAATTGTGGTTTGGGACACAAATTAAAAGTAATGTCCAAAACTGCGATTATTTTCCACCAACCTAAATATGTGTGTTT
GATAAGCTTTGTTCAGGAAGTTCTAGTGCTGTTAGATGTGAGTTAAATGTTAGAATGAACAATATATGTTAAATAAGATATCTTTGA
ACAGCAACACACAAAAAGCAAGGTTATGTATTGATCAGTTGGGGAAAAATATGACTAGAAGCTCACAGAAACCTAACCCTGTCTTTCC
TCTAGGAGCAATGGTTCAGCATTCATTAATTCAGTGTTTGGGGCAACTTTATAGAGCATAAGTGCTACAAATGATAATTGACTATAT
TTAGTATAAATTTCTACAGAGAAATCATGAATTAAGAACTTAAAGGATGCTTTAGCCCTTTCTTACCTTATGGGAAGATACATGTGG
AATGATGTGGATACTTTGGGCAGTTTAACTCATATCTGATGTGCATGTTCCAGTCAACCTTAGTATGTGATACAAAATGAGCTTTAT
TCCTGCTAACTCAGGGTCTGAAGTTGTATTTTCATTTTTTTTTCCTGTCCTGGCAGGTACTCATGCCCGACTGTCTACCAGGCACAC
AGACTTTGAGGAGAGGGCGTATGTCGTCTTGATCCGCATCAATGATGGGGGTCGGCCACCCTTGGAAGGCATTGTTTCTTTACCAGG
TAGATATTCTGCTATAGGCAACTCAGGCTAGTATCTGTCACCATGGGCCGTTTGAGGAGATTCTTGTCTTTGATATGGGATTCTGCA
ACTATTCACTTGAGAATTTATCCTACCAAATACTGTCCTGCTTTTCAGTTACATTCTGCAGTTGTGTGGAAGGAAGTTGTTTCCGGC
CAGCAGGTCACCAGACTGGGATACCCACTGTGGGCATGGCAGTTGGTATACTGCTGACCACCCTTCTGGTGATTGGTGAGTGTAATT
TCTTAATTCAAAGAGTCTTATCCTGGGCCTCAGAAATGTGAGCTCATCTCAGGGCCTGTCTTCCTGGACTGGTCTGGTGGCTGCTCA
AATGGCAACTCAGGCAATAGCCCAGAAGGTAGATCACCAGCAAATTACATCAGACTTTCTTGATTGGAGTTACAGCTTTAGTACTAC
CCTCTGTCTCTATTGCCTTATTAGTTTAGAAATATAAGGGGCTTTATGGTCATCAATTGTCATATATTATGCCTAGTTACAAAGCTT
TTGTGATAAAGAGGGAAGAAATGCAGTAGCTGTGCCATGTGTCAGGCACTGTGCTTGATGGGGAAAGAGTGACATACACAGATCCTA
CACCAGAGTTTATGGTCTCACCTGCCATTGTTGTTACAGGCAGAAGTTAGCCTTCCAGTGGCGATGCTGAGATACCTGAAATGTGTA
GTGCAGAACTTCCCTATGCATAGAACCCTGATTCTTTACTACCCAGAATAACCCATTGCATCATGACCACAGCCTCTGCCATAGCCC
CTAGCTTGGCTAGACAGTACAGTCCCCTGATCTAGCTGTCTTTTTCTTTTTTGCAAACAACTCAGTGCCTATTCCAAGTGTTTCCTT
ACCCAAAATGCTTGAGAGCAGAAGTGTTTCAGATTTTGGGTTTTTTCCAGATTTTTGAACATTTGCAAATACACGATGAGATATCTT
GGGGATGAGACCCAAGTCTCAACACAAAATTCACGTGATTCATAAACATTTTATACACGTAGTCTGAAGGTATTTCATACAATATTT
TAAATAATATTGTGTATGAAACAAAGTTTTGCCTGCAACCTGTCACATGAGTCAGGTGTAGAATTTTATGCTTGTGACATCAAGTT
GGTACCCCAAAAGTTTCAGATTTGAGATTTTCAGATTAAGGATACTCAACCTATATAGTAATTGCCCTCTTTCAACTGTTGTCCCCA
CCACAAGTTAAACATTAGGAATAGATACTTCATTATTTCATGTGTACCTGAGTGAAGGGGTTTATGGTTTCATATCCTTTTTTCTTTT
AAAAGAAGGACTAGGATTATTCTAAGTATTATTGATTGTTCTCTCAGCATCTGATCTTTTCTCCCATGATCCAGTGTGTCACACTGG
ACTCTGATTCATGCATCTAAAGGGGGTCTCACTTCTTTATACCCTTCAGTCTGGACTCCCTATGGCATGGGAGGGCCTTCATGTCAT
GGCCACTTCCCATCCTAATGTGTGCATGGTGATCATCTTTCCTCCCTGAGAACCACTATCTACTGCACACTTTTCATGGTAATTGAG
CTGTCTTTTTGAGGTCCCACTCACTTACATAGGTCCCTTCTGCCTCTGTGCCTTTTCTTATTCTATTTGCCCTCCCACCTTGAAAGG
ATTTTTGAATTTACAAATCAGATAGCAATTTTCTTAACGTATCAATTTAATTCCATAATCTTACCACAAGTTAAAGACTGCTTGAGT
TTGGGGACATCCAGAAATAGAAGTAATAGGATTTTACAGTTTCATGACCTTATTTTCAAGATAGGGTTGGAATTATAAGTTCTGAG
CATCCAGACTTTTCTGCCATGATTCCTCCTATAAACTTACTAGCAGGTTAATTTCCTTAAAACAACTCCTTCTTCATAGTGGACTCT
GACAGTACACAATCAGTGCTGAGCTGCACAGTGTAATTTTGATGTATCAGGTGGGAGAAACCAAGAAGGTTAAGTAGTCTACCCAGG
GTCCCACAACAACATAGGCAGGATCCAGGAGGAAGGCGCCCAAGGAAATGCGATAGTTGTGATGGACGATGAAAATCAGCTTCAG
GGAGGTGAACAGGTCAGAGAACAGCTTTCTAGTTGTGCTAATTGGATTGTTTTCTCTAGGAAAGGGGAAGAATAGAATCAATATTAA
GTATATATAAAGTTCAGGAACTTTGTCAGGTGCTTATACACTCTATAGTGTGGGACATATACACTCCATAGTGTGGAACATTAGGTC
CTATATTCTTACAGCGAGATCCAGTGAGAGCCACCAAGTCAGCTATGATTTGGGACTTGATACATACATACAACAGGATAAATGATA
AGCTCATTTCTCCAAGAAGGCCAACGACTAGAGTCCCTTATTCACTAGGACAGTGAAATTTGTTGAATCACATACAAAAATGAAGT
TGAGTCATTTTTTTCCTACCACAATACTACTTTATAACTGAATGTGACAGTACATTTTAAAAGGTCCCATTTATTTGACCCTGGGTT
TTTTCTTTTAGGTATAATTTTAGCAGTTGTGTTTATCCGCATAAAGAAGGATAAAGGCAAAGATAATGTTGAAAGTGCTCAAGCATC
TGAAGTCAAACCTCTGAGAAGCTGAATTTGAAAAGGAATGTTTGAATTTATATAGCAAGTGCTATTTCAGCAACAACCATCTCATCC
TATTACTTTTCATCTAACGTGCATTATAATTTTTTAAACAGATATTCCCTCTTGTCCTTTAATATTTGCTAAATATTTCTTTTTTGA
GGTGGAGTCTTGCTCTGTCGCCCAGGCTGGAGTACAGTGGTGTGATCCCAGCTCACTGCAACCTCCGCCTCCTGGGTTCACATGATT
CTCCTGCCTCAGCTTCCTAAGTAGCTGGGTTTACAGGCACCCACCACCATGCCCAGCTAATTTTTGTATTTTTAATAGAGACGGGGT
TTCGCCATTTGGCCAGGCTGGTCTTGAACTCCTGACGTCAAGTGATCTGCCTGCCTTGGTCTCCCAATACAGGCATGAACCACTGCA
CCCACCTACTTAGATATTTCATGTGCTATAGACATTAGAGAGATTTTTCATTTTTCCATGACATTTTTCCTCTCTGCAAATGGCTTA
GCTACTTGTGTTTTTCCCTTTTGGGGCAAGCAGACTCATTAAATATTCTGTACATTTTTTCTTTATCAAGGAGATATATCAGTGTT
GTCTCATAGAACTGCCTGGATTCCATTTATGTTTTTTCTGATTCCATCCTGTGTCCCCTTCATCCTTGACTCCTTTGGTATTTCACT
GAATTTCAAACATTTGTCAGAGAAGAAAAACGTGAGGACTCAGGAAAATAAATAAATAAAAGAACAGCCTTTTCCTTAGTATTAA
CAGAAATGTTTCTGTGTCATTAACCATCTTTAATCAATGTGACATGTTGCTCTTTGGCTGAAATTCTTCAACTTGGAAATGACACAG
ACCCACAGAAGGTGTTCAAACACAACCTACTCTGCAAACCTTGGTAAAGGAACCAGTCAGCTGGCCAGATTTCCTCACTACCTGCCA
TGCATACATGCTGCGCATGTTTCTTCATTCGTATGTTAGTAAAGTTTTGGTTATTATATATTTAACATGTGGAAGAAAACAAGACA
TGAAAAGAGTGGTGACAAATCAAGAATAAACACTGGTTGTAGTCAGTTTTGTTTGTTGATCCGCTCTGCCTCTGTTTTTATTTGTGG
AGGGGAAGGACAGAATAACAATTGTTATTTTTTGTGTATAAAAATTGCCTCCCCAGCAGCCCTAGTCAGGGGCTTATAGATAAAACT
CCCATCTC
```

HUMAN mRNA SEQUENCE : hR28-010.1 (Seq ID No: 61)

```
GGAAGAGGGGAGTGTTCCCGGGGGAGATACTCCAGTCGTAGCAAGAGTCTCGACCACTGAATGGAAGAAAAGGACTTTTAACCACCAT
TTTGTGACTTACAGAAAGGAATTTGAATAAAGAAAACTATGATACTTCAGGCCCATCTTCACTCCCTGTGTCTTCTTATGCTTTATT
TGGCAACTGGATATGGCCACAGAGGGGAAGTTTAGTGGACCCCTGAAACCCATGACATTTTCTATTTATGAAGGCCAAGAACCGAGTC
AAATTATATTCCAGTTTAAGGCCAATCCTCCTGCTGTGACTTTTGAACTAACTGGGGAGACAGACAACATATTTGTGATAGAACGGG
AGGGACTTCTGTATTACAACAGAGCCTTGGACAGGGAAACAAGATCTACTCACAATCTCCAGGTTGCAGCCCTGGACGCTAATGGAA
TTATAGTGGAGGGTCCAGTCCCTATCACCATAGAAGTGAAGGACATCAACGACAATCGACCCACGTTTCTCCAGTCAAAGTACGAAG
GCTCAGTAAGGCAGAACTCTCGCCCAGGAAAGCCCTTCTTGTATGTCAATGCCACAGACCTGGATGATCCGGCCACTCCCAATGGCC
```

213

EP 2 204 376 A2

```
AGCTTTATTACCAGATTGTCATCCAGCTTCCCATGATCAACAATGTCATGTACTTTCAGATCAACAACAAAACGGGAGCCATCTCTC
TTACCCGAGAGGGATCTCAGGAATTGAATCCTGCTAAGAATCCTTCCTATAATCTGGTGATCTCAGTGAAGGACATGGGAGGCCAGA
GTGAGAATTCCTTCAGTGATACCACATCTGTGGATATCATAGTGACAGAGAATATTTGGAAAGCACCAAAACCTGTGGAGATGGTGG
AAAACTCAACTGATCCTCACCCCATCAAAATCACTCAGGTGCGGTGGAATGATCCCGGTGCACAATATTCCTTAGTTGACAAAGAGA
AGCTGCCAAGATTCCCCATTTTTCAATTGACCAGGAAGGAGATATTTACGTGACTCAGCCCTTGGACCGAGAAGAAAAGGATGCATATG
TTTTTTATGCAGTTGCAAAGGATGAGTACGGAAAACCACTTTCATATCCGCTGGAAATTCATGTAAAAGTTAAAGATATTAATGATA
ATCCACCTACATGTCCGTCACCAGTAACCGTATTTGAGGTCCAGGAGAATGAACGACTGGGTAACAGTATCGGGACCCTTACTGCAC
ATGACAGGGATGAAGAAAATACTGCCAACAGTTTTCTAAACTACAGGATTGTGGAGCAAACTCCCAAACTTCCCATGGATGGACTCT
TCCTAATCCAAACCTATGCTGGAATGTTACAGTTAGCTAAACAGTCCTTGAAGAAGCAAGATACTCCTCAGTACAACTTAACGATAG
AGGTGTCTGACAAAGATTTCAAGACCCTTTGTTTTGTGCAAATCAACGTTATTGATATCAATGATCAGATCCCCATCTTTGAAAAAT
CAGATTATGGAAACCTGACTCTTGCTGAAGACACAAACATTGGGTCCACCATCTTAACCATCCAGGCCACTGATGCTGATGAGCCAT
TTACTGGGAGTTCTAAAATTCTGTATCATATCATAAAGGGAGACAGTGAGGGACGCCTGGGGGTTGACACAGATCCCATACCAACA
CCGGATATGTCATAATTAAAAAGCCTCTTGATTTTGAAACAGCAGCTGTTTCCAACATTGTGTTCAAAGCAGAAAATCCTGAGCCTC
TAGTGTTTGGTGTGAAGTACAATGCAAGTTCTTTTGCCAAGTTCACGCTTATTGTGACAGATGTGAATGAAGCACCTCAATTTTCCC
AACACGTATTCCAAGCGAAAGTCAGTGAGGATGTAGCTATAGGCACTAAAGTGGGCAATGTGACTGCCAAGGATCCAGAAGGTCTGG
ACATAAGCTATTCACTGAGGGGGAGACACAAGAGGTTGGCTTAAAATTGACCACGTGACTGGTGAGATCTTTAGTGTGGCTCCATTGG
ACAGAGAAGCCGGAAGTCCATATCGGGTACAAGTGGTGGCCACAGAAGTAGGGGGGGTCTTCCTTGAGCTCTGTGTCAGAGTTCCACC
TGATCCTTATGGATGTGAATGACAACCCTCCCAGGCTAGCCAAGGACTACACGGGCTTGTTCTTCTGCCATCCCCTCAGTGCACCTG
GAAGTCTCATTTTCGAGGCTACTGATGATGATCAGCACTTATTTCGGGGTCCCCATTTTACATTTTCCCTCGGCAGTGGAAGCTTAC
AAAACGACTGGGAAGTTTCCAAAATCAATGGTACTCATCCTGTGATCCCTCACTGCAACCTCCGCCTCCTGGGTTCACATGATTCTCCTGCCTCA
GCTTCCTAAGTAGCTGGGTTTACAGGCACCCACCACCATGCCCAGCTAATTTTTGTATTTTTAATAGAGACGGGGTTTCGCCATTTG
GCCAGGCTGGTCTTGAACTCCTGACGTCAAGTGATCTGCCTGCCTTGGTCTCCCAATACAGGCATGAACCACTGCACCCACCTACTT
AGATATTTCATGTGCTATAGACATTAGAGAGATTTTTCATTTTTTCCATGACATTTTTCCTCTCTGCAAATGGCTTAGCTACTTGTGT
TTTTCCCTTTTGGGGCAAGACAGACTCATTAAATATTCTGTACATTTTTTCTTTATCAAGGAGATATATCAGTGTTGTCTCATAGAA
CTGCCTGGATTCCATTTATGTTTTTTCTGATTCCATCCTGTGTCCCCTTCATCCTTGACTCCTTTGGTATTTCACTGAATTTCAAAC
ATTTGTCAGAGAAGAAAAACGTGAGGACTCAGGAAAAATAAATAAATAAAAGAACAGCCTTTTCCCTTAGTATTAACAGAAATGTTT
CTGTGTCATTAACCATCTTTAATCAATGTGACATGTTGCTCTTTGGCTGAAATTCTTCAACTTGGAAATGACACAGACCCACAGAAG
GTGTTCAAACACAACCTACTCTGCAAACCTTGGTAAAGGAACCAGTCAGCTGGCCAGATTTCCTCACTACCTGCCATGCATACATGC
TGCGCATGTTTTCTTCATTCGTATGTTAGTAAAGTTTTGGTTATTATATATTTAACATGTGGAAGAAAACAAGACATGAAAGAGTG
GTGACAAATCAAGAATAAACACTGGTTGTAGTCAGTTTTGTTTGTTGATCCGCTCTGCCTCTGTTTTTATTGTGGAGGGGAAGGAC
AGAATAACAATTGTTATTTTTTGTGTATAAAAATTGCCTCCCCAGCAGCCCTAGTCAGGGGCTTATAGATAAAACTCCCATCTC
```

HUMAN PROTEIN SEQUENCE : hP28-010.1 (Seq ID No: 62)

MILQAHLHSLCLLMLYLATGYGQEGKFSGPLKPMTFSIYEGQEPSQIIFQFKANPPAVTFELTGETDNIFVI
EREGLLYYNRALDRETRSTHNLQVAALDANGIIVEGPVPITIEVKDINDNRPTFLQSKYEGSVRQNSRPGKPFLYVNATDLDDPATP
NGQLYYQIVIQLPMINNVMYFQINNKTGAISLTREGSQELNPAKNPSYNLVISVKDMGGQSENSFSDTTSVDIIVTENIWKAPKPVE
MVENSTDPHPIKITQVRWNDPGAQYSLVDKEKLPRFPFSIDQEGDIYVTQPLDREEKDAYVFYAVAKDEYGKPLSYPLEIHVKVKDI
NDNPPTCPSPVTVFEVQENERLGNSIGTLTAHDRDEENTANSFLNYRIVEQTPKLPMDGLFLIQTYAGMLQLAKQSLKKQDTPQYNL
TIEVSDKDFKTLCFVQINVIDINDQIPIFEKSDYGNLTLAEDTNIGSTILTIQATDADEPFTGSSKILYHIIKGDSEGRLGVDTDPH
TNTGYVIIKKPLDFETAAVSNIVFKAENPEPLVFGVKYNASSFAKFTLIVTDVNEAPQFSQHVFQAKVSEDVAIGTKVGNVTAKDPE
GLDISYSLRGDTRGWLKIDHVTGEIFSVAPLDREAGSPYRVQVVATEVGGSSLSSVSEFHLILMDVNDNPPRLAKDYTGLFFCHPLS
APGSLIFEATDDDQHLFRGPHFTFSLGSGSLQNDWEVSKINGTHARLSTRHTDFEERAYVVLIRINDGGRPPLEGIVSLPVTFCSCV
EGSCFRPAGHQTGIPTVGMAVGILLTTLLVIGIILAVVFIRIKKDKGKDNVESAQASEVKPLRS*

**Table 7**

```
MOUSE GENOMIC SEQUENCE : mD28-021 (Seq ID No: 63)
AGGAAGGAGCGGAGGCCCTGGTGCCCTGCCCTCAGTGCTGACTGCCCAGCGAGAGTGACTGGTATAAGCAAGCAGAGACCCGGTGAG
TGTGCCCCAGTGTGCGGCCGGGGCTGCAGGGGTGGCTGTGGGCCGGGCTCAATGACTTGGATTTTGCTGTGAGATTCATATAGAGTT
GTGGATGGGATGTCCTGTGTGCACGTGGCTGAGAGACGCTAGGGTGGGATGAGGGAGAGAAGGGAGCTGAGCCTTGTTTAGGGAGGT
GGGAAAGACAGACCACCACCGTGAAGGGTGGGGGGTGGGACGTGAGGGAGGGGAGCTGTCTGGCTAGCGTGGGGGACAGAGGTGGGT
GGGGAGTGATGAGGGTTCATCAGCTGCCGTAGGTGTGAAATAGGCTTCCAGATGACACCAGAGTCTGCCTGCTTCTCCATTCCCATGT
ACAAGGTTAGGCAGTTAGCTAGGAGTCTACATGGTGGGCTTGGATGTGAGGCCGAAGGCTGTAGATGTCTTCCTAAGGGGGCTTTGG
AAGAAGGAAAGTTTCTAAAGCAACAATGTCCTTACATTCTTCTGTCACTTCCTTCGTGGAACACTGGCAAGTGGCACCTCCTGCAAA
CTAGAGCAGCAGACAGAGCACACCCCGTGGACATGATTAGATCTGCATTTAAAGCTGGGCAGTGGTGGCCCGCACTTTAATCCCAGC
ACTTAGGAAGCAGAGGCAGGTGGCTCTCTGAGTTTGAGTTTAGCCTGGTCTCCAGAGTTCCAGGACAGCCAGGGCTACACAGAGAAA
CCCTGTCTCAAAAAAACAAGCAAGCAAACAAAAATATCATTTAAAAAAGGATTGTTTTTATTTTATGTGTATGTTTGCCTACATGTA
TGTATATATTCCATGTGTATGCCTGGCACTTGTGGCGGCAGAAGAGGGGCGTTGGGTCCATTACAGAGTGTTGGGAGCCACCATGTG
GGTATTGGGAACTGAACCTGGTTCCTCAGCAGGAGCTTAAAACACTCTTAACTGCTAAGCCATATCTCCAGCCCCAGGTTTGCATT
TTTTGTGAGCTAAAGGCACCATTGGCTTGTGTTCAGGATGAATTGGAAGGAAATGATGGAAGGCAGGGTGCCCACTGAGAGGATGGG
```

214

```
AGTTAGTTGGGATAGATGAGGGAGGGGCAAATTCAAACTCCCCACAAGTTGGGTGTTTAGAATGAAGAAAGGGCCTTCAAGGGTTTG
CTGACCTGGATAACAGGGTGTCCTTGAGCTTGGTGGGCCTTTCTCTGTAAGCTTGAAGACAAGACCCATGCCTCCGGCCATCACCCT
GTTCATTGGACAGTTCGAGATATGGTTAAACAGAGGAAGCCATGACAAAGATTGATCTGAGCCAGTGAAGAGGCTCAGCAGGGAAAT
GTGCTTGCTGCCTGACCGTCCGAGTTCCCTTCCCAGAACCCAGTGGTAAATGTGGAAGCAGAGAATTGACTCCACAGAGTTGTCCTC
TGACCCCTGAGTAACCGCACACAATTATAACAAATACTTTTTTTTTTGAAAAAAAGAAATGGATCTGGTCTCGATGGAGAGGAATGT
AGATGCTGTGGGTGGTCACACTTGGTGAAATGAGCAGCAGGGTTATGCGGAAGGACAGAGGGGGCTGCCATGGCAGGAAATGTCAG
CTGGGGAGGACGGGGGTAAGGAAACCCTTCCGTCCGTCCTCAGGTGAGGGGGCTGATCTGCTTAAAAAATGCAGAAGAGGCTGGGAAA
GAAGAGCCCTTTGAGCTCTGGGATGCTGCTTGTTAATGGCTTGTCTGGAGGGTGGAGATGGGGTGTGTATGGATGGGGCCCCGCTCC
TGGAGGCTGAGTTAATTTGGGGGTAGGGAGAGACGCTTTGAGAGTCTACAGACCAGGAAGGTGCTGATGACATCAGGCAAGCTAGGA
TAGGACTGCCTGTGGTTGAAGGTGTAAGTGATTCTTAGGGGACTGGGTTCTAGAGCTGGGTCAGCCTGTAGGCCAAGGGCTTGTCAT
GTGTCAGGGTTTCTCAGTTGTGGTACTGTGACCTTTCTAGACAAGGTGATACTTCTTTGTGTGTCTGGCCTGATGTTGTAAGATGTC
ACAAGTATCCAGGGATTCTTCCCATTAGACCCTAGCTAATACCACCTACCTCTATCCCCAGTCCTAACAATCAGAGATGTCTCCAGA
CATTACCTCACGTCCTGTGGCAGGGTAGAGCCACCCTGGACTGAGAACCACTGGTTTGCATTGCCTCTTGTGATTTCACTAAATCTG
TACAACCTGGAGACCTAGGGGGAGTCACTTGGAAGGTGTGTTTATGGATCACTTGAATCTGGTTCTGAAGTGGGCAAGAGGAGGCAG
GAAAGATGGTGAACACTAATCTAAGCAATCCCTCCCCACACCCCCAAAAGGCTGAGGGGTGGTAGCTGTAGAAAGGAATCCACTTTG
ATTTTTGTTTGTTTCATAACGAGGCCCTTGTTAGGTTCTTGGCTGCCACTCACAGAGATTTTGACACCTGTGGTTTCCCCTTTGACT
TCCCCAGACATACAGAAGCTGAGGGCTACAAAAAGGGCCTCAGTGTAGTTAGAGGATCCCTAACACTGGAGTGGGAGGGGATGTTGT
TCCTGGCTGTTCACATGGAGTCTGGCAGCCCGGCTGGAGGAGGTAAGACTGTCTCAGGCTCCTTCCTGCCAGCCACCTTCTCTGCGC
CGGGGTCAAGGACAGGGTTGTGATTTATTCCCATCACAATCCAGCAGTTGGGTTGGTGAGAGTTGGACCCATGCTGGTTTTCCCTTC
CCAAGTGTGACAAGGCCTCAGCAGCGGCTGCTGCAGTACCACGCGGTGAACTGTCCAGGGCATGACAAAAGGGCTCGGTTAGCTGCC
GCCACAGGTTAGAAGATGAACAGCTCACAGCTCTTCCGCCCCATGTGCAGCTTGTCGCTTAAGCCCTGGCCGAGCACACTGTAGAAC
CCGGGATGCCCCTTCTTCTGCCCATGGCCATGTTTGGTCTCCAGTCTCCTGGGAGCCAGGGTCCTGGCCTGAAGAGGAGGTGAGGGA
GGCTAGAGGAACAGGTGGTATTTGGAGACTAATTCATATTTGTGAATAATAACCCATGCCCACAGGAAACAAATCAACCTGTGTTTG
CATATTCATGAGGGGGGCCTTTAGAATGATCCCAGCTGTTTTCCATCCGGGAATCCCTGGGTCCTGGCGTCACATGGTCTCTTGGGG
GTCCTCTTTGGCCCTGGCAGGGATAGCTAGGGTGGATCTCATGGAAGTGACACGCATTTTCCATGAGACACATACATGACTCCTGTG
GCTTGGTGTTGTGCCTGCTGATCCCTCTTTCCTCAGAGCAGTGGTTCCCAGGATCTTTGGTTTCACACACAGTTCAGTTTCCAAGAG
TGCTTAGGGCCAGACAGGGGTTAGCAGGTTTTTTTGTTTTTTGTTTTTTTTTTTGCCAAGTAAGGACATTATTAA
AAAAAGAAAAAAAAACAACTGCTATCGGCTGTCCCTATCACTTCTTCATACAAGAAAGGATATTTTAGCACCAAGGACAAAATTTGA
GAATAAAGGACATTTCTTCCTAAGAAAGGGCAGTTGGCAGCTTTACAGTGGTGTAGCAAGGAAAAGAGATATATCAAAGCCATTCTG
AATGTAGAAAAATTACACTCAATTATATCCTATAATATCGTATGGCACGTAATTGTTTTCCTGAACAAGTATGTCAGAACCAGCAGT
CCTCGGTGCAATTCACAGATGAGCTTGAGTGTCCAAGGGTTTGGGCCTTGAGTCTATCTGAATTGTCCCGGGCACAAGCTCATCTGT
CCATGCTGGTGGAGTGACTGTCTGTTCCAAGCACCGTCTGTGTTGGGTGGGTGCACGAAGCCATGGCAGTCCAGCAAGGCAGACTCGT
TCTGCAGGGGACCTGGAGACCAGGGTGTAGGCAGGACCAACATCTAGACTTTTAAGCCCAGGCCTGGGACTCAGGGTGCTCACCTCT
CTTCTGATGAGGGAGGAGGCCCTGGAGCTGGGAGCAGCCTTGAACTGCCTGACACCAGCATAGGCTCTGTGCCCAGAGTCCAGGGGT
GGGAGTTGCTGAGAAGGAGAGGCCCTGACTCGGGAGACTGTGTAGTTGCCAAAGCGGTTCCCTGTTCGTCCCTGGGGTCTTCTGGTG
TTTCTAATTGTCTGTGGCCTTGCGTTCAAGGACCACTGGAAGCAGCTCTCTTTGGGGGGAGATGTCTAGATTTTTCAACTGAAAAAA
GAAAAAGGTTTAACTATATCCTGTGTATGAGTGTCTGCCTGCCTGTGTGTCTGTGTACCATGTGTGTGGTTCTGGTGCCCACAGAGGGC
AGAGGAGGGCGTTGCATCCCCTGGAACTGGAGTTGGACAGTTGTGAGCACGTGTGTAGGTGCTGGGAAAACTGATGCAAGTCTTGGG
GCAGCCTGTGCCTTTAACTCTGAGCCAACTCTCTGACCCCTGTTTTCACCTGGTCTGCCCATTTGCTACACCGTGGAAGTGTATACA
GTGTTCTGAACACTCCCCCTGACTTACACAGGGGCTCTTGAATCCCCAAGTAAGAGCCGCCGGCCTTTGCTATGCTGGGAATTGGGC
TCAGATAACGCTTGTGGCCAGATTTTCACTTACCTGTTCTGGGAGTGAGACACTATGGTGGCTTTACTGAGAGGAGCTTTGCTCCCC
TTTAATAGTTAATATCCACATCCCCATCCCGTGCCTGTGACATCCCTGTGATGCCCTTATCAGGCAGCTGTACCC
GCAGATGTCTGTACCCTCTGTGTACCCCACATGATGTGTCTGCACCTGGGAGTTAACCATGCTGTGATTGCTGTACTAAGGCAGCTA
CAGGGGGATTAAGGTTCCGTAATTGTACTGGGGAATTGTGCCCCACGATGACTGTGCTGTGCATTTTTGTACGGCGTAACATCTATT
CTGTCTCATCCCTTTATGTCTGTACCAGGGAGATGTGTCCCCATGACACTGGTACTGAAGATTATGTCCATATGGTGTCTGTGTTGG
GGGTGGGGAGCGTGCCTGGGGTACCTGAGCACACAAGTTAAACTCCAGCTTTTGTGATGTCTCACCCACGAGACCTGCCTCGACCAC
TCTGCCTGACTTGTGTGTGGGGTGTCACACTTCTGAGTTGTACCCGTGGTAATGGTACCAAAGCAAGAGTCCTATCTGAATACAGTG
TTCCCATGGTAACCTGGCCAAGGCAGTTCACCACCTGGTGTCCTCATAGCCACACTAAGATAACCATGGTGCTGATGAACAAGCC
CAGATAAGACTTTAACAGAATTAGAACCCCTATCCTGACATGGCAGTGTGTGGCCTCACTGTGGGCTGACCCACCTCAGCTCTGTGG
TCCCTCAGAGGAACTCCCGGCCTGGAATTAGGAGTTCTGGACACTATGTCCTGTCCCTGTAGCGTTATGGCTCTTGCTGCTTATCCC
TTGCTTACAGGTTGGGTAAGCGTCTAAGACAGGGAACATCTCCCCTTCCCAATGGTCACTAGCTCCTCGCTCACTTCACAGTCCCAC
CTGCCTTACCTTTGCCCATTCACCTCATGATCGTGGCTATCCTAAGGGTTCAGATAAGGGTTCAAGTCCCAGGGAGGGGCAGAGCTATG
CTTGAACTCCATCCCTCTTGTTCACCTGTCCTCTCATTTTGAAAATTCTCTTAGCACTATAGGGGCCCATGGGGTCCCAAGTTGTAG
TGAGAAATTTGTGAGGTTTTTGTTTTTCAAAAATACAGAAATATAATGGGAATGTTTTGTTTAAATCCCAGGTGTGGGGTAGGGAGT
GGCTTCAGACTGTCCACAGCAGCTGACTATGATTTGCCTCGTGCTCTAGCAGGGCGTGATTTTTGCCAGTGGCAGATAGCTTCTGC
AATTGTATGATATTTGGAATTTGGGGGTCTTTTCAGAGAGTATTCATTCGTGGAGAGGTGGGGTGGGTTGTTGGTTGGTGGCTGTTT
GTTAAGTAGTTGTGTGCAAAGAAGGAACGAGAAGAAATTAAGATATCCACGTGAAGATTAAACTTGCCCCAAGAAGCTCAGCACC
CCATTCAGCAGGAAGCTCTCTAGTGATATTTTGCCCCCTTCCTCCTCTACCCTTTTTCTCTCTCCTATGTTTAGAGGATGAAAGGGT
GGTAAGAAGGGCAGAAGAAAAAAACCCCACAAAATAGCAAAGGTCCAACTACATTAAATTGTGTCCGGTATCACTCCTCCATTAGAC
CCTGTTGTATTTTCAGAGCATGGCTGAAGCCTGGCCATGCACCAGTGGCCTGTATGTGGCAGTGTGAGCAGAATTGGTGGGCCCTGC
TGCATGTCACCTAAGCCCCCAGACAGGCAATGTGGCACCGGAACCTTTAGAAGCTCACCCTAGGTACATAGTGTGGCCACTTCTGCA
TGGGTTTGCAGAGTGAGAAAGTCATGTAGACAGGAGGGTGGACAAGGCTTGGGAGGCTTCGGTTAGCCCCAAGCACAGCCCTCCCCC
ATCTGCTCTGGACACCCGGAACAAGGGAAGAGGGAAACATGGATGTTGACACTCAGGCTCTAGAATTCCAATCCACAGCCGCTTTATA
GTTTGTCTTTGGGATAGGAAGGTGCCATGGGAATTAGGTAGGTTGAGAGTGACCAGGCAGGGCAGCTGTAACTTGTAGAGAGCAGTC
CTGGCGAGCCCCAACGGGTTGTGGCTTTGGCTGTGAGCCTCTGACGATGTGGAACCAACTACTGCTAAGTCTGTGTACTGGGGCTTT
TCCTGAGTGTGCTGGGGTCTGGCCACTTGCTTTCTGAGGAAGAGCAACAGGATGCCCCAGGGACTAAGATGGCCCTGGACAGTGTAA
CCTGCCCTCATCCCCTAATGGTGTGGAGCCGGAAACTCACATCTGGAATGGACCCCGCCTCGTGTTAGTTTCTAACTAGCTTTCTTCC
CCCTCCTGGCGCTTAGAGGATGCCCTGAAGGTCTCTGCACTCTTTCCATAGGATGGTTGACCTTCAGTGGTGAGGGAGTTTGGGTAG
CCATTGCATGGTCCAGTGTGGGACTGCTGAACTGTCTGAATGGGCAGTGAGGGTGGGTGGGAGTGGGGTGGGGAAGAGCATGTGCAG
TGGGGACTCAGGGGGACACTGGCTAATTGGCTCTGCCTTTCTCTCCATTACAGGTGGATTGTCTTAGGCTGTGGCTGGCTGTGGAGC
TAGAACCCTGGATGGCTCCCGAGCCAGCCCCAGGGAGGAGGATGGTGCCCCTTGTGCCTGCTGCTCTCGTGATGCTTGGTTTGATGGCAG
GTGCTCATGGCGACAGTAAGTCTGACTCCTCATCATACAGATACATCATGTTGAAAATCGACTTAGCTCCTAGCAAGTCTCTAAAAT
GCAATACCGGACCAGGTCCCAGGAAGAAGAAACAGGATGACCATAGATAACCCACTAGTCCTCCTGCCCTAGAGACCTGCACTGGGA
AAGCCCTCTTCTAAGGAGTACGTGCTCCTACTTCCTCTGAACATCCTGCTGAGCACCTGGGTATCCCTTAGGCTCTGCAGAGTGCTG
CATGTCTAAGCGTGACCCTGTTATATAGAGCTGGGGAGCCGTGTCTTGGATTCATGTGATCTTTGTGTGACGTGCATAGGCATGTAC
```

EP 2 204 376 A2

```
TTTGTCCACATTGCAGATCTCTGTAGAAGGCCCCGAGCAGAAGGAAGCAGGGCACCAGACATGTCTGCAGAGCCAGGGAGGGATTCAG
AAGCAGTTGCCCATATCCTTCCCTGAGGCGGCCTGCAGTCCCTGGAAAATAGGGAAGGACTTGTCACTTGGCTTGCAGGAAGGAGAG
AGGGGGGCAGGGAATGAAACTGAGTCTGTGTGTGTTTGTGTGTTGGGGGGATGATTTGTCCCCTACTCCAATCCAACTCTGAACTTTGGT
CTCTTCCCTGCTGCTGCCCTTCCCCCTGCCTGGCACTATAAGACTGGCCATAGCCCGCCCTGTCACCATGGTTACCACTGCTTGGTT
ACTGGTAGGAGGCGGAGCACAGGACAGGCTTAGCCAATCTGCAGACTGGGGGGAGGGGCAAGGTCGGAGCTGAGGTCCCTTGGGGGG
AAGGGGCTATTCCCAGCCTGTCCTGAACCCAGAGGCAGTCCAGGGCCAACCCAGGGGTCAGACCCCAGGATAGCAGGCCATAGGGAT
CCCTCTATCCCCTCTGGGTCTCTAGACAAGAGCCTTGTCCCACCAGGGCTCTATCTAAATGGCTCATTAATAATTCAGGAGCCAGTC
ATAGAAGAGGGTGGCCCTGGAGCACCCCTCCCTTTGTCCTCAGAGGCTGGGCCCTGGATAAGGGAGAATGGGAGGGAGAGGGCCTTT
CTCTGGAGAGTCTAGCCAGTGGAGTGCAGGGCTAGGGGTCTAGAAGAGAAGTCAGGTGGAAAGCCTTAGAAAGGGGTAGTGTGAAGG
TGATCCTTAGCCCTGGCTGGGGCTGCAGAGGCAGCAGAGGGAAGTGATCTGGGGCCCAGCACCCTAGGTGTGTGACTGGCATTGACA
TGGTGAAGGACTGTTGTCACTCTTCTCAGATTGTCTCAGGGCCCTGACAGCAGGGGAGACATGCCACTGAGTTTTGAGAAACAGTGA
GGCCTAGGAAAATAACTGCCTTCTCTATCTTCCCTCTGACCCAGCACACCCCAGCCTGCGCCCGTCCGTCCCACCATCCTGCCTGCC
CCCTGGTTTTTCTCTGTGACTGGCATTACTGTCGACCTGGTTACCAAGTCAAAAAGCTGGGCCTTCTGGCAGCTCCTCTCCCCCTTCC
CCTGCCCCCTTCTGCCAAGCCTTCTTCAGCTTCTGCTTGAGTTTGAGTGTGCACCAGGGAACCTTCTGATGGTTCCCTGCCTCAGTC
CCATTCCTCTGAACCCATTCTCTAGTCTACAGTGGGAATTGGGTTGCGTCTCTTCGCTCCCCAACTGCCTGGAGAGTGAGCTTTAGT
GTCTGACTCTCTGCTACCCTACCCTCCACCGGCCCCGCTGTGCTCTCTCCTTTCGGCTGCACAGAGCTCCGTGTCCAGGCATCCTTC
ATGCCTTCTGTTTGCACTCTCGCAGTCAGGTGTGATGACGTGGGCCTGTATCCCTAGCATTTGAGAGGTGGAAGCAGGAGGATTAAG
AGTTCTAGGGGATCTTGGCTCTGTAGTAAGTTGAAGACAAGCCTGGGCTACGTGACTGTCTCCAAAAACAAACAAGGTGTGGAGGGG
GGGTGTTTTCCCTCCCTTTACATGACCAGAACTGACTCAACTGTCACTGCTTCCTGTAGCAGCAGTGTGGTATGCATGCCCTGGTCCTCT
GCAGCCCTCATATGCTCTTGTGGCGCTTCGCCCTCAGTCTGTCTGCCCTGGCAGGCTGAGAGTTTAGACATTTCCAGCTCCGGGTCT
AGAGGGGGGTTGGTGAATAAGCGAAGGGGCAGGACAGTCCTGACCACCGTAGGAGTGGGGAGTGAGAGGAGCTGCTTTAGGGCAGAGT
TCCAGGGTCTTCCTGCTGGCACCTTGGTCATTTGGTTACAGAGACCAGGCCGGGACTTGATGGTGGAAACCAGGTGAAGAGGCTGGG
ACCCTGCCTCTGCTAAGGGTGAACCCTCAGGTGCTGTGACTTGGGCACTGTCCCAGGCCAAGAGCCATCAGCTAGGGGCTGTGGTAA
GCCAACCTTAGGAGTCAGGGTCTGCCCTCCTAGCCTACCTTTAAGTTACTCTTTTTTTTTTTTTTGAATCTTCCCCCATTGTGACCAG
TCTTGGACTGAACTTGCAAGACTTCTCACTAGGGGCAAGCAGCCAGACACGGACAGGGCTGCTGCAGAAGGCAGGGCGGGTGCCCCC
AGAGAATTGACGACCAGAGCAGTGAAGCGCAGAGCGGACAAATGTGTTTCTATAGGCCTCTTAACGAGACAAATGAATGGGGGCCCT
GGCCTCAGAGGGGAGTGGAGAGAAGCAGGGGAGAGAAGCAGCTGCCAAGAGCTAGTCTGGAGGCCTAGCAGTGATGCCTAGTCAGCT
CCAGTGGAAACCTGGATACGAAGGGATGCTTGCTTGCATGCACGCACCCTTCTGGAACCTCCCTCACCTCGTGTCCCCAACCCAGGA
TTTTATGCCTTCTTGCGAGGGAAGATATAGATGGATGTGGGCATAGAGGTGGTCTTGGTCCAGCCCTCTCAGGCAGAGGTTTCTGCTGA
CCTCATGTGGTAGGTGGTAGGAGGAGAAAGAATCTGCCCAGACCCATCACAAGACAGAACCCACCGAGCTGGGCACACGTCTGTGAGC
TAACAGATCAGTGGGGGCAGGTTGGGGCAGCGCTGGCACAGGCAGCAGCCTGATTATCTTGAGAGGCTTCCTGGAAAAGAGGGCCCC
TGAAGATGAGTGGGCATTTGCTAACATAGAGGATTGCCCAATGAAGGAATGAGACTGGCAGAGTCATACCCCTGCCCTGGCCTTCTG
AGCTTACGAGAGAGCCAGAGTCCTGAAGACAGCACAGGCCAGCGATGGGATCCCCGCCCCCAAGACCAGGTGTACGTTGGGTCCTGG
TTATGACCAGGTTATTTGCAGGCATTAAAGTTGAGCAAGGTGGTTGGTAGGTGAAGGGGTCGGTGGTATGAAGCAGAGAGGCAGGAG
ACTGAAGAGTGTATGTGTGTGTGTGTGTGTTGCTTCTCCAAAGCCCCTATTTTGGAACATGGCAGGGCCCTCCCCCTCATGTT
TATGGGTGGCCCGTCTAGACCCTGCCCTCTGGAGCTTCCGGCACAAGTGCTTTCTCTGAGCCTGTTTTCCTATTGCACAACAGGGGT
AATCACTCCGACCTGTAAAGGTTAACCGAGGTCACATGGGCAAGGCACCTGGCACCTATTGAGTTCTCCCCCTGTTGCAGTGCCTCC
TGGAGCCAGGATTGCCCTTGAGTGGACAGGGCTGGGGGAGGCTGCCAGGAGCAGTCACAGGAGCTCATTAAGTCCCCAGCTCATTGA
GTCCTCAGCTCAGAGCCACTCAGGGCCTGGGAAATCACCTCTTCCACGAGTGCCTCCCCTGCCCTCGTGGTCCTAAAGTGCTGTGTGTGCT
TCCTCCGTGTGCAGTGCTGTTTGTGTGCCTGTGTATATGTGCTTCTCAGGGGAGGCACAAAATAGCAATCAGTCTCCCCATGGAGGA
GCCTTGGAGGTGGCCCAGTTTAGCCGTCTCTGGAGTTGCATGTCTTCATGTCTCCAGGGGCTGTGCCTCCCTCTAGCCCAAGCCAGG
CCCTCTCTCTGGTAACTTCCTCTCATGTACAGGGTCAGCGGTTGACATGTAAAGCATGGCCCAGGGTATCCTACTGTGAGTAGCTGC
CCACGGGGTTGGGTGACACAGCAGGTAACTGTGACACAAAGCTGCTCCTTGCTTGTTCAGATGACACCTGTTTTCTGGTTCAGAAGT
TTTGCTCATAGACCAACAAGATGGCTCAGCAAGTAAAGGCACTCAACCACCCATGGCCGCTACACACACACACACCACCACCACCAC
CACCACCACCACCACCAGCTGATAAGGCCCAAGGCAGGGTGGCCGCAGTAGCCTGGCTTTGCCTACCTGCCTGCCTGGCACCCCAGC
TGGCAGTGGGGTGTGGGCCAGGCTTCCACAGTCGGAAGAGGGGGGCTAATAAAGGATGCAGGGTACACACAGAGGCAGGCCTGGATT
GTGAGCAAGCTCAAGCTAAGTTCTCAGCAGAGTGCTTGCTTGACCTCTGGCAAGTGCATACAAATCAAGATCCTTGCTGGAATGGAA
AAAGGGGGAATTTAGAGAAGCTAGCAGGAGCAAATTTGGGGAGACAGGGTAGAAACAAGATTTGACTGTGATCTCTGAAAGTCTCCT
GAGGAACAACAGGAAGATCCTAGGCTAGGCCGTGGAAGGAGGAAGAGCCAGGTGGCCCGGCCTCTGCGAGGGTACCCTTAG
TCGGAACCAAGGCATAGAAGGCATAGAAGGAGCCTGGGACTGGAGGAGCAATGCCAGCCTGATCCTGGGCAGCACCCTGGCTCTTCT
CCAGTCTCCAAGGGAGAGGAGTAGTTGTACCTCCCTGATGGCCCCAGGCCTTGGGCACAGGCCAGCCCCCACACAGCTTCCAGGTTT
GTTCCTGCTGGCTGCTCCGGAGCTACCTGACTCCTGCCCAGCCTCGGCTCTTTGTTTGGCTCGGGAGGCCCCACCCCACCCCACCCC
AGCACACTCCTAGTCTTTCTGGTTCTTCAGGTTCCAGCCTCTGGTTTCACTGGAGACACGTTGAGGCAGGACCTGGAGGGGCACTTA
TTCCCTGGGTTTCTCTTGCCTAGAGGGAAGGAGTACGTGAGGCCCCAGCCTTCTACACTCCTCTCACTCCTTCCTCTGTCCTCTT
CTTCCTGTTCTGCACACCTGGGTGTCAGGAACTGGGTCAGACAGTGGGACAGGGCAGAAGACAGGACAGACATGGAATCTGCTCTGC
CCAGGGTCATGGTCTGATTGGGAAGCAACTATTAAACACCAGAAAGGTGGGAAGGCTTTAGCTTCCTGTGCACCCCGAAACTGTGGG
CAAACTGTGGGCGGCAGAGCGAGTTTGGTGGGGGTGGGGGTTAGGGAGGGATAGGGGAGAGTTTTCTGAGACGATGACGTTCAGAGG
TGACTTAGGATGTTAGTGGGTAAGGGGTAGTCGAAGAGGCGCTTAGCAGAAGGTGCAGCTTACGCAAAGGCCCTGAGGCAGCATTGC
TTTGTAAAGAGCAGTGGGAGGCCTTTGGAGGAGATTTGATCAGGAGGAGGCTTGTTCTCTGGCTTCAGAATAGAGAATAGAGGAGGTG
GGGTGGGGGTGACAGAGGCAGGGCCCATCAGCCTGTTCAGCCAGCCAAGCCGTTTGTCCCATGGAGGGGATGCAGAGCTGGAAGCTAC
TGGGGTGGGGGAGGGGTTAAGAGATAACCTCAGTAGACCCTGGAGGAGGGGGCTTCCTCTCAGGCAGGTATTGGCAACAGTGATAGG
CTTTGTAAATTCTACAGACTAGGAAAGATCCACACCTCTCTCAGAGGCGGGGGTCGGGGTGGGGGTGGGAGGGTGGTAGAAGCAGA
AAGGAGTTCAGAACTTTTGGGTCACCTGCATGACTTCCTGGCCCCTGCCTGCACTGGGCCCTCTGAGTTGCTCAGCCTCCCCCCCCT
AGGAACAGATTGTGGTGGGTCCCGAGCTTTCTTCCTGGGGAGGACACAGGGTTTATTTAAAGTGAGTTTAGGATTTAGAATGTAGAGTG
AACCCCAGCTCCACCCCAGAACACTGACTCATTGGTCCTTGTTCCTTCCACTGCTTGGATGTCTTTCCCTATCTCTAAAATGAGGGT
GATTATGGGATCCACTGACCCACAGGGCTGCTGATGTTAACAAGTGACTGCCTGCCCTGGGTGAAGGCCTGCCACTGACACATGTCCT
TAAAAAAAACAAAAAAGACCAAAACAAAACCAAAAAAAAAACCAGAATTGGTGCTGGAGAGCTGGCTCAGTGGTCAAGAGTGCTTGCA
GCAAGTGATCTTGTAGAGACCTGGGTTTGCTTCCCAGCACCCACATCAGGCAGTTCACAGCCACCTCCAGCTCTAGAAGATCTAATG
CCCCCTTCTGCCCTGGCATTGCACACATGTGGTGCACATACAGACAAGCAGGCACGTATACATAATACAGATAAAACTCCAA
GAGTTTCTAAACCTTCCTCCAGTTGGCATCTCTTTCCTAGTCACAGCGTGCTGTGACAGTCACCTGTCCTCTCCCTCACCTTGGCT
ACTCCTCTGCTTTTGTCTGACTTTTGCCACCACTGGCTGTTCTCTTCAACAAGGTCCCCTGCCTCCTGTGAACTTTTTGTGTCAGGT
GTTTTGGACTTTGATGGGCCTACATTTGGGTCCCAAATGTGTCAGTACTCACTGGCTGTGTGACTCTGGGCATGTGACTTGGTCCTT
CAGCCTAGAAAGTGGAGGCACCGGTTGTGCATGAAGTGACATAAAGTACCTGGCGCACAGTGGGCACTTTTCTGCCAGGGCCCAAAC
TGAGCCTGGGTTATGGGCAACAAGCAGGTCCTCTGAAGGGCCAGCTCCCAAGGCACTGGCTTTCCTGCACCGTGCACTGGCTGCACT
```

216

EP 2 204 376 A2

```
AATAGGAAACGGATCCACAGTCAGGATTAACGAGGCATTGCTACGTCTCAATGAGCCAGCTTGCTTGGGGCTGTGACCCTGGGTGGT
GGGACATGGAGGGTATGGCCAGGCCTTGGCTGAGCCCCTGTCCTCCTCCTTTGGAGTAGGAACTCTCGGAGCTGCCCATGCTGTGGAA
AGGCAGTTGCCAAGCCGGTGGGGGCAGGGAGTTACAGGAAAGCATCCCTCTGGGAAGTCATCCAGAGCCTCTCAGGAGCTGGAATGT
CCAGGCCTTTGGGGCTGTTGGAATGCAGGGGTAGGGAAGAGGAGGAGTCCCGGGGTCTGCCTAAGGCTTAGCGTAGATGGAAAGGCT
CTAGGAGAGTCTGGGGCACTTCCCAGAAAGCAGTTCTCCAGTATTGTTTTTTATTTTTTTGTTATTCCCGGAGCTTGGAATAACCAGG
AGCCGGAGACAGACGGAGGTCGAGCCCAACTCCAGACTCTGCTCTTGACCAGCTGTGTCCCAATGAACAAATCTCTTGACCTCCGCA
CACCTTGATTTCTTCTCTGTAAACAGTAATTATAGCACCCACCTCCTTGGCTTCAAAGAGTTACAAGGGTTCCGCTCTTTGAACAGG
GCTCGGCACTTATTAAGCACTATATAAAAGTTAGGTATTATTGTTGTTCTCTCTAAGTCTTTCTTGCCCCCTCGTCCCCAAGGCCTG
CTACCTGTAGCATGGTAGCATTGACCATGCCTGGTGTGATCATGCCACTTGATTTGAGTGTCTCTGTCTCTTCCAGGCAAACCCGTC
TTTGTTAAGGTCCCCGAGGACCAGACTGGGCTGTCGGGAGGGGTGGCCTCCTTCGTGTGCCAAGCCACAGGGGAACCCAAGCCTCGA
ATCACGTGGATGAAGAAGGGGAAGAAAGTCAGCTCCCAGCGCTTTGAGGTTCGTCCTGGACTGGCCGGGAGGGCTGGGCAGGCTTTA
GAGTTGGCCTGCTCTCTGTGTGGTCCTTCTCTTGTTCCTCAAGGGTCTTTTGGGGATGTGAGGAAAACTATAGTAGATCCACACACC
CCTAACAGGCTGTGACCACGTCTGTCCTGTGACAGGTAATTGAGTTTGACGATGGAGCAGGGTCAGTGCTGCGGATCCAGCCATTAC
GAGTGCAGCGAGACGAAGCCATCTATGAGTGCACAGCCACGAACAGTCTCGGGGAGATCAACACAAGTGCCAAGCTGTCAGTGCTTG
AAGGTATGTACCAAGAGGGACAGGCCACCGTGGCCAGGTAGACTGAGATGTGGGTGGGGCCCACCAGGACTGGCTGCCCTGGGGTGG
GTGAGAGTCAGTGCTCAGCAGGCCAAGGTCACTGGTCTGTGTTGAAGCCAGCAGCGAGCTTGCCAGCTGTGTGATCTCCTTCCTGCT
GGGTCATCGCTCCCTTGGGTCTGGGTCAGGCTGGAGGTATGGTGCTGCAGCTCTGTGGCCCTGGGGTTACGTGACGGTGGCAGTGAA
GCCTGGCAGTTGCTGGCAGTGTTCTCTTGGTACCCTGATGAAGGAGCTGGAGCTGGCCTCTCCATGTGAATTTTAGTGTCTCTTCTC
ATCTGGTGGCTCGTTCATTCATTTAGTCATTCACAAACTTTGCTTGAACTGCCGTTAAGCCCAGAATGTCACTTGATACGTGCTACC
CTCAGTCAGTGTCCAGTAAATTCTGTAAGGAACGTGGACACTTTGCTTCTGACCCTTTGCCAAGACCCGGGGGTGTAAAGGGGAGCAG
AGCTTCCAGAACTCTGAGTGGTCAATTTGAGGCTTTTCAAGTGGCGGCTGGAGAGCGGAGGGGAGGGGAGGGGAGGGTAATTCA
GTGAGGCTGCAGGATCAGGCTGAGGAACGGGCCCTTTCTTGAGGACGTAGAAAGCCACAAATAGGGTGGGAAGTGACAGGATTAAAC
CTTGACCACAATGGATACATGAATCTGAATGTGCATTTTCAAAATACCGTATTGATTGTTGCTTTCATTTTTGAATGCAATACCAAT
ATCTGTTCACTGTTAAAGAATTGAGAAGCATAGGATAGTGTGTAGGGAGAAATAATAATCTCTTATTATCCTACCCCTAGTGCTATT
AGTTATTTCTTGGTGGTCCATTTTCTATGTGTATGTTTTATATACATCTATGTATATTTGTATTAATTTATATAAACAGAATAGTGA
TCACACACTGTTCCATGACCTTGGGGGTTTTTTTTTTGTTGTTTGTTTTCCAGCTAGAAGGCAAACCTAGGGCTTTTCAGACAAAC
TCCCTCACTGAACCTGGAGCTCACCTATTTGGCTAGCCTAGTGGCCAGCGAGCTCTAAGAATCCACCTATCTCTGCCTGCCTCCCCA
GCACTAGAGTCCAGGCACTCACTTCTGTACCCAGCTTCCTCCATGGGCTCTAGGAAACACAACTCAGGTCTTCACGCTTGCTCAGCA
GGTCCGTTACTGAGCCACCTCCCCAGCTCCTCCTGCTTAGGAAAAAACATAGTGTGAAGATTTTCCTTGCCATTAAATATTCTTCCA
CACCATTATTTTTAATGGCTGTGGTAAGCTGTCACATGGAAGCACCCTAATTTATTTAGCTGTTCCCTTTTTGTGGGGTGCATATGT
TCTCAGCTTCCAGCCTTTGTAAGTACGGTGTCACACAAGTCTGTGACGATGCCGTTGCTTTGCTGGTCATTTCCTGACTCTAGGTTC
GTGGAGTAGAATTGCTAAGTCAAAGGGCACATGCATTTTAAAGCCTTCAATATTGCTAGAAGATTGTATCTGTTTATATTCTTATCA
GGGAGGGCATACGAGGGCCTCTTCTCTGCACATCTTCACCAGCAGTAGAAATTACCATTAAAAAAAAAAAAATCTTTGCCAAGTTAAT
AGGCAAAAAATGGCATCTCAATTTAATTTGCATTTCTTTGATTACAAGAGCAGTGGAACATCTTTTCACATTGGCCATTTTTATGCT
TTGGCTTTATGTGTTCACACATGCATCCATTCAGTAATTCTTCAGTCTGTGTCTCGTGTGAATCAGTGGGTTCTAATAGAGCTGT
GTGCGGCCCTCCCCTGTAGACAGGAACGCTGGGCCTCATTTGTTCCATCCGGGATCCAGGACATCGTACCCACAGTCCAAGCTAGTG
TGGAGCTTTCCCTGTAGCCCAGGATCTCAGAGTCCATTCTGAAGTGTAGACAACCTGAGCCGCTGCTGCTTGGTGTCACTGGGTTGT
GCTAGCCCTGCTGTCCGTTCCATTTGGGGACAGGGGGCCAGACAACCAGGTCTGAGCTGAAACCACTGGCTTGGAGGTTCCTTCTGG
TCATTTCTGACCTCCAATTTGTGACCCAGGAGAAAGGATTTCACCTGTTGGTGTGAGAGTCATGTACCCTGCCTTGTATTGGAGTGG
AGTCTAAAAGACTAGAACTATAATCCCTATCATGGCGGGCAACCTCTGAGGCTCTCCGGCTAGGGAGTAGTCTCCTTGTGAGACTGG
AGAGCTGATTCCTCTAAGACATCCCACTGCCCTGTCAACTTTACCCTTTAAAGGGTCTCTCAAGGCCCGGGGAAGAGAACTCACACT
TGGCATAGGGTTGCCATTAGAAACGGCCTTCCTGGTCAAGAGTTTCTTTCCATGGATTAGAGGGTGTTCTTTCTTATGTGGTGTTGG
GATGGACCCCGGTACTAGACATGGACTTTTATCCCCAGACCTGGGCTTCAGGCTATATGTTGATGCATCTGGGAACTGGAAGATGTC
TGATGATGTGTGAGACAGACAGGTCCCTACTACACCAGAGTGGTTGGAATTGTGGGCTTCGTGGGCCCCTCCTGCTCCTGAGATATT
GCACCAGCGAGGCAAAAGTGCTGACCTGTCCTGTTGTCTTTGTAACCTTGACCAGCTTAAGGCTTGGCAGAAAGGAATGTCACAGGG
GATAAGACCACGGAGGGCAGCCTAAAGCAACCCTGCCTCATTTCCTCCCTGTTCTTCTGGGGTGGGGGGCAGGAAAGCTGGGGTGC
TTGCCTGCAATAGATGAAAAGATGGCTTTTTTTTTTTTTTTTTTTTTTGGAGCTCTGGGGTTGACACGAGGGGAGAGCCAGGGCC
ATTTTCTATGGAGAGACTACCATAGAATCCTAGTAGTGAGAGATAAACTTGGCTTAGGGGCTGGAAGATAGTGCCTGGTTGCTTTGC
TCTCACGTCTCACACACATGTGCCCGCCTGTGCATCAGGAGACAGGTGCTTTGGGGAGTTCCTGGGTAATGCCTGGGGAATCTGGCC
CAGGTGCAGGACCAGTGTGTCTTCAAAAGGGAACCCCAGTATTATACTTCCTTTGGGGCCTTCTGCCCTTCCTCCTACCACCCCAGG
CCCAGCTCAGTCCACTCTCACTGCAGCCTCTGGGGACACCACCATTGCCAGGTCCCCACGAGTCTCTGTACTCTGCCGCTGCTGGT
GCAGCTTGTGAGACCTAGCCCACTATGCTGGGATGGCCAGCCGGTGTGCCAACATCCCTGTCAGGGTGCAAGGTGGGGTGTCCAGCA
CAAGCTGGCTGGGGCTGGGCCTGTCCTGTCCTGGGCTGTGATGAGTACCAGGGCCCTGCCGCCCAGCGTTCCTGTCTGAGTGGTAAT
TGAAGCCATTAGCGCGCCAGCCTCTCCCCTCGCCGGGTAATGGCAGGAAAAGCTCTTCTCGCTCCGCACTCTTGAGGCGGCGGCTGAA
TCATTCCCCCTCCAACCCGCCCGCTGCCGCCACTGACGAGGGAATCTGACATTTTCCCTCCAAAGGGAGGGGAGGGGGTGGGGGGAAG
GGAGTGAGGCCTTGATTCTGGGCTTTCCCTTCCGAGGTGGAGGGGGATAGTGAGGGTGTTGAGCTCAGTTTGGAGGTCATTAGAGTC
CCCAGGGTTTGCAGAGTCCAGGCCAGGCAGAACCATGGTTGGGGAGAACTCCAGGGAGGAGGCTAGACAGCCAAAGCTGTGGTGGAG
CCCTAGTGACTGGGTAAGTTCTGGAAGGGACACCGAATGCCCAAAGGAAGATGGGGACAAGAGGCAGTGGAGTAGGACTTGTCCTGG
GGGCCGGGCTCAAGGGCTGGATGTTTGTCATTTCGTGCACATAGAAGATGATGTGCTTACAGTGGTGTGAGCATGTGTGAAGGGATG
TGTATACCAGCAGTGTGCCTCAGGCAGCTGGGGAGGGGCATGATGTGTAAGCAGGTGGTATGTACTTTAAGGGCTCTGTGCAGTAGT
GAGATGTGTGCATGTGCACGCATCTGTGCATGGCAGGTAGTATCTAGGGTGAGTGTGTGTGAGTGAGTGTGTGATACGGGTGTAC
AGCAGGTGGTATTATACCTTAGCTTTGTGAACAGCGAGAGAGTCAGCAGTCTGAGTTGGATGTGTAGGGTCAGTCTGGTGCTGGTGG
GACGTCAGGTGCCGTATTCAGATTTGTTGTTTCTGGGGTCCCACAGAACAGCGGACAACAACGAATGCTGGTCAGTGTGGTTGGGGC
TAGAGTGTTGTTGTAATCTGCTGCCATCATACTCAGCAGCTCCTCTGTGACCAAGCCAGGAGGAGGGCACACTAGGAGTCCTGACCA
AACCTGGCCTTAGGATTTAGGACTTCTCCTGTCTATGCCACTTGGCTGGACGCTAGAGTGTCTCACATTGCCTAACCTGGAGAGCAG
TGTCTGCTAAGTGGGCCAGACTGTACCACCTATGCCACTTGGCTGGACGCCCCCCCCCCACCTACACTTGTCCTTCTACCTGCTTCCATCTTGGGCTCTG
GCAAGAGAGGGCGTTTCAGTTAACACTGGATCGTGGGTTCTGCATTTGCTCTCACCCTCGTGGGTTCTGAGGCTTTAGGGTGACAGA
TAACTTCTTGCCACACACGCTCGCTCAAGTGTGCGCGCACACTTACACACACAAACACACACCTCCCCCAACACACACATATGATCT
TTGGAAGCAGCTCCAGAGGAAAAGACTGATGGTGACCCTGTCCTGAGCTGTCCTGCCTGAGCTGGGCAGGATGAGTGCTTTCCTAGG
GGTCATTTTTCCCTGCCCCACCTTTTTTCCTACCATAAGCCTTTCTATTCCTTTCACTCTCAGTGGAAAGATGAGGGTGAAAGAGCT
ACTACAGGAAGTCCCAAACCTAGAATTCTAGGCAACCCCTGTTCTCCCCTGGCAGCAGTGACACTGATGTACCTCTTGTCCAGTGAC
CTTTAGGACGTAACCTCCATTTCCTGTGACCACAACAGATCTCAGGACCATGAAGATATGAAATACTATAGCCAGATTTTGGAGGTT
CGGACAGAGATAGTCTTGGGTAGGAAGGCACATCTGGAATGGGGCAGTAGAGGGTAGGAAGGCACATCTGGAATGGGCCAGTAGAGG
CAGCTGGAAGGTCAGACACGGGGGTTTCTTGGTTCCTGGAGAGGATTCTCATGGTTTCCAGTTCTGAGAGTCTTCGAAGAGCTCAGTG
AACTCCTTTTGAACGGAGTCCCCTTCTGTGAGCTGTGGCCTGTGCCCACCTAGTTCCCCACGCCCGTGCTCACAGGTGTCTCGTCTG
TGCTCTGTGCTTTTTGACACCCCCCCCCCGTCTCCCTGCCCAGCTTATGATCATGGGCTGAACCAGCAGCGCTAGAAATAGCTCTCA
```

217

```
AAGTTGGAGGTCTTAGATACCCCTGGATGTATATTCTCCATCTGTGGTGAGGTATCCTCCTCACCCTGATTGCCAGGGGAGAAGAGG
GCTTACTAAGGGCAGATTCATGCTTCAGAGAAGTCCTTGTTGATGCAAAGTAGGGCGGAAGTGTATCTATGAGAGGTAGGTGTAGTC
AATCCAACTAGCAAAAGCCAGCATCACCCGAACCTTGTGAAAAGAAGAGGAAGGGAGATAGATCAAGAGCATGACAGGTAGCATCCA
GCCCAGCTGCCTGGGACACCCCAGGGGTGGTTGGCTCTGCATTCCTGGTTCAAGGCTGGTAGACAGCGGAACCAATTTTCTATCTAA
GATTGAAATACAGGAAGAGGCCCAGGACTGAAGAGGTGAGTTGATAAGTCTGATTTGAAGTCTGTCAAGTCAGGTGTGTGCAGGCAT
CCAAGGAAAGATGTGAGAAGGCCTCACATGGGTTTGCCTGGCACTCTTGAGGGGCTGGAGATGGAATCGTGAGAGCCAAGAAGCCAA
GCCAAAAGGATCCCCAGGAACTGGCGTTCACTGTTGGGAGGAGGTTCCTGGGAGCTAAGGGGGAGCCAGAGCAGGGGAGGCAGTGGA
AGAGTATGAAGGAGGAGAAGCCAAGCAAGATGAGTATTGGGCAGTGTCCTTTGGATTTAGCTCTGCAGTTGGCACTGACCTTGGCGA
ACAGGCTTCCAGAAGCCTCGGGGGTGGCAGCAGGGCTGCAGGGGGCTGAAGAGCGAGTGGGAGGCTGTCAGTGGTCTGAAGTGACTG
GGAGCAAGGGCTGAACCTAGAGAGGTAAATGGCTAAAAAGAAAATTCCTGAGCAGGCTTGGGGTAGAGGGGCATCAGGAGGGGAGAGC
CCAGCATGAGCAGAATCTGAGTGTGGGGACCAGGCAGTGGCTCTCGGGTCCACTGAGTCCAGTCCGCACGGTGGGGAGGCCTCACCT
GCCAGGTTGAGTAGGACTCCGTGGAGTGCCAGGGAAACCCAAGGGAGGGGGGATTACTGAGTGGGGGAGGCCGTGGCTGCCGACAGG
CCCCAAACAGCTCAGACTCAAAAACAAAACAGGCTTTTAAGATGCCAAGTTTGATGAAATCTGAGTGCTGGAAGAGGTGGGAGTTTT
CTCTTGGAAAAACAGCTGAAAGTCGAGACTGAAAGCTGCGAAGGGAGATGGGCGCTGAGGCCCCTTGGGCCCCCCTTACCGCCTGGG
AGCCTGGGGGTGGGGTGCAGGCTAGGCACTGAGCGGGAGAGCTTACAGGTTTGGGGAGGGCTCTGTCTTGACCCTGTCTCTGCTGAG
GATGGAGCTGTTCTGGGGGGCTGAACTGAGGGTGGCCCTGGGCTTCCTTGGAGTGCCAAAGGGCAGGGTGGGGCCTGGCCACCCAC
AGAGGTTCCTTTGTGGGTTTGGTCTTACTGTGTCTCCAGGTGCGTGTGTCTCTTCATGTGTGTGTCTGACTTTGCATGTCCTCGCAC
CCGGTGTTCCCACACCTCTGCATGCCGCCTCTGGATCTGCCTGCAGGAGTCTGTTGCCACAGGTGCTTGTCACTGCAGATGCCCGCC
ACAGGCACATGGAGGTTTCTATTTTTGCACCTGTCTCTTTCTCCTCGTAGTTCTCTAGGTCCCTGGCTGTGCGTGTTTCTAAGGTGT
CTGTGTGTGTTCAGGGACAGGGTGATACACTCATGCATAGAGGAGGGGTCAGCCTCACAGACTGTGCCACCCCGGGGAGCCAGTCTATG
TGCATGCGCATGGGCCTGTATTCAGAGATGCCATGAAGCTGGGTAAACACGGGTGAAGAGGTCTCTCTGACAGACGGTGCCAGTG
CTCTCCCTTACAGCCACCACCCTCCCCTCTAAGGCATCTCCATCTGTTAGCTTTCCTGGTGCAGCCCCTGCCTCTGGCCCAGACTC
CGCCCCAGGCATCCCTGGCTTCATGGACAAAGAGACCTGTGTGTGTTCTCTAGAGGCCTTCAGCAGCCTCCCTCCCCCAGTCCTTGC
AGCCTGTCGGTCTAAACTTCCCACCCACCCCTGCCCTCTCCTTGTCTATCAAAACTCAATACATACCCCAGACTCACCTTGCTGTT
TAGTATTCCTCCATCCTGGCTATAGTCTTTACCCGGCAGTGTGCAGGAAAGCTTTTTGCTTTGCATCATTCTTGATCCTCCTTCCTC
CAGGAAGTCACCAAGATAGCAGATGAGAGGTGGAGTTATAGTAATTAACCTCTACTGCTGAGGGACATCAACCATGCTCCCTGTCTCT
GTCGTTTGTATCCTCCCCACCCCACCCCCTTAGACACGCCCTCCCACATGCCCTGTCATCTCTGAGCTGTAAGACAACAGGACCTCTC
CAGGGAAGGGTCTCTCAACCGCAGTATCTGAAGGGCCAGTTGGGTACAGGAGCCATTCATGAACCACTTGGATGCTTACTCTAGGTG
CACAGATCTGTGCCGGACGGGCAGACTTTCGATTATGTGTGGTACTTATGTATAGAGGCTATCCAGGTCTAGGCTACTGTGTTGGTC
CCTCTCCAGAGTCCCCCAAAGCTAAGGGACTTCTCTTTCTGCTCTGGGAACAGTGCAGTGGAGGACCTGGCCGACACTGCGGACTGG
ATATGCAGAGGCAACTTAAGAGTCTTCTGCACTGTTCTGGACCCTAGCAGGCGGGAGCTGGGTGCGCTGCGTTGGTGGAGCTGGAAT
GCCTAAGGGGGAGCACCCCCCCAACTGAGGCGCAGGGTCCAGGGAGGGGGCACGGCTGCTCCGCCTAGGTGGGGGCGGGAAGGGCGG
CGCCCAGTAGGACTTGGACGCGGAGGCGCGCACCCGCTCGGACACAAGCGCGCGCAGCCCTTCCACCGTGGCACCGCCTCTTTGCC
GTCGCCATGGTTACCGGGTGGCCTGGAGGGGGGGAGGGGCCCTCCCGCACCAGCAGCCCGTGTTTGGAGGGGCTATAGCGTAACCCGT
GCACCCACACACACACAGGCGCGTGCCAGGGATGTCCACATGTGGGGAGCATTGGGCTTCCTTTACAGCACCTGAAAAAAACGTAGAG
GCGTGTGTGCATGGGGGGTCTGCAGTAGAGTCCGTAGCCGGAAGCCGGTTAAGGGCGGGGAAGGGGGAAGGGGCGTGGCTCTTCCCA
GGGACGCCCCTCCCTTCCCCTGCCCTGCCCACCTTTTCGTGAATGCTAGTCTAAAGCAGCAGGGTCCTACCACCTGCTTTCCTCCAA
GCCACACCTCCAATTTAACTGAGTGACCTTGGGCAGGTTTCCAGATTTCTGCCAAGTCCCCATTTGCTCATATGCCTCATAGGGTTT
TGGTAAAGATTGTCATTTTGTAAACCTGAAACAGAGGGACTGCTTCATGCTTGGAACCTTTTATCCCTGTGTCCAGAGGCCTCAGTT
TTTCTCAGTTGTTGATGGGGTTGGAAGTGACAGTGTGGCATGCATCCTCTTGAGAAATTTGCCTTGGTCATTAACCTGAGTGGTTTGGA
CTTCTATAGACACAGCTCTGGTGGTGGTGGTGGGGGGGGGGAGGCTTAAGGTGGCAAGGGGGCCAGTCACTCCGCAGGCATCTGGGGA
ATGTCTGTTGTCCTTGACTGATAAGAGTGTGCTGAAGGCTCCCAGGTGGGAGAGGAGGACCGCTGCTGCAGCAGCAGGTCAGCCCTCA
GGGGCTGACTTCCCCTCCTGGAGTGGTCTAAGAAGCAACCTTGCCTGTGCTCACTGCTTGGGAACCTAGCTGGCCCCCAGGGACTTGC
TTTTGGGTCCTGCTGTTCAGTAGTCGCTCTTCACACTCCCTGGTTTTAGGCTTTGGCAGTGGCGCCTAAGGAATGATCTGAGCCAAG
GACGGAGCCATTTGAGGGTGTGATAATTGAGGGAGGAAAATTAATTGTCCTTAATTTGGCGTAGGTCCCAAAGACCTTCCCCGTGTC
AAGAATTCAGAGTAGATCCCTGGGACCTACTCTGCTTTCATTCTAGATCAGTGTTTTGCACGTAGGCCATAGAGGCTGGAGAGCTCC
TTGGCTCTGACTCTGCCAGGACCTAGCCGCCCCCTCATCCTGCACCCCAGTTGTTCAGACAGACAGGAGTAAGCCTGGAAGAACACGC
TCGATGCGGTGGTCAGTGATGATAGCTGTAGCTCTAGCAGCCTTGGGATCCCTGATGCTAGATGGGGATGGGGAGGTTGCCCTAGGC
CTCAGGGCTTACTCTTAGCTTCCAAGTGGTGCCAGGCCAAGGAAGCTGTGCATCTTCAGTCCTCACTCCCCTCCTCTTTCTCTTCCT
GCTGCTGTTTAATATGATCCTTTCAAGAATCAGCTGAGCCGGGCAGTGGTGGTGCACGCCTTTAATCCTAGCACTTGGGAGGCAGAG
GCAGGCAGAGTTTGAGGCCAGCCTGGTCTACAGAGTGAATTCCAGGACAGCCAGGGCTACACAGAGAAACCCTGTCTTGAACCCCCC
CCCCCCCAAAATCAGCTAAGAGTCAGAGCTCTGTGGCATCATACTTACAGCCAAAGAGACCCAGGGTTTACATCAGAATTTATAGG
GCTCCAAACAGGGAAGTGTATAGCCAATTCCTGGCCAAACCTGGGGATTCAGGGCCATGATGAGAGATCTCCTATAACCCCAGCCAG
ACACCCTTCTAGGATATCCCTAGAAGTTGCAACTTTGATGGAGGTTCCTCTGCCTGCGGTGGCTCCTCCAGCTTCATCTTGTCTGTG
CCCCGGGTGCTGTCACCCTGCCCTTTACTTTTTCTCCCTCTGAGGCTGAGTCTTCCTGGCATGGGAGCCTGGGTGCAGCAAATGTTCC
TGCACACTGACCTGTGCTGGGCATGTGAGAGGGAAGAGTGGAGTGGTGCCTCTGCCACCACCATACTGCCCAGCTTTCTTGCCGAAA
GGTAAACAAAGGGCAGACATTCTCTAGTCCCTCATTAGGTCACCCAGAGCCAAGCTTGGGAAGAGGAGAGGTACTGTTGAATTCTG
GAAAACACTTGATGGTTTTGTTCCAATGGGAAACCATAATGGGAAAGGGGGCCCCAGGCTATAGTTCTGGTATCGTGTCTGCCCCAGT
GGCTCAGGACTGGTTAATTATAACTTCTAACAGTTACTGGGTGCCTGTTCCGTGTCAGATACTCCCTTAGCACCCTGGATTCTTTTT
TTGGTGGAGTCTTCAAGGTATCTTTACGAGGTGGACACCTAAACCCAGTAGATAAGTCAAGCATCAGTGAGTAACGAACACGCATAG
GCCGTAGAAGTGGTAGTCAGTGGTAGAGACGTCCGTCCCACATGGTCTACACTGTCCCAGGGCTTGTCTTCCCAGCTCCTGGTGAGA
GGTGGGCATCGGGCAGCTTTGTCTCAGCTTGTGGGGACTGCTTCAGGAACCTACGGCAGGAGGAAATGGGTCAAGTGAAAGTGATTT
CCTTTGTGAGTCGCTAACTCCAGGAAATAGTGGGAGGGTTGGTTTTGTTTTTGTTGTTGTTTTTACTGTTTGGTTTTTTTTTTTTTT
TTTTTTTGTCTTCTTAGCCTACACATACTCAGACATGGCCCTGACACCTTATCCTGGTTCTTGGGGCCAGGAATGAGTAAGAAAGAC
CAAGGGTCAAGACTATAGTAGAGGATGGTCAGAGGCAAGACTAGAGTTTGGTCATCGGTAGGGCAGGGCCAGGGTCAAAGACCTGAG
GCTCAGCCACATGGAACAGCTGAAGAGATGCAGGCCAAGGAGAGATAGCATGTGTGCTGACAAGGAGCTGCGGGCTGGACCAGCACA
CCTGGTGGGGTTGTGGCTATTGATGCTTGGGGGACTTTACTTCCCTTGCCGCCCCCACCCCGAATCTATATAGCACTTTGCCACAGC
CAGCAGAGCATCGATCCCTGCTCTTCCCAGGGACCTAGAGAGATAAGTGCTGACGTCTTTAGTCTGTGCTTCCAGACTTGGCAACTCC
CCAGCAGGGATGTGGTGCTATGGGCCCTCCTGCAGCCCCTGCCTTGCCCAAAGGGACCTAGGACTCTTGACTCCTCATTTGGGGTAG
GGCTTGTGTTTAGAAGTCAGGGGTGGGAAAGAGAAGTGTATCATGGGTTCCCTGCTAACCTGGAAGTCAGGAGAAAGCTACACAGAG
AGGCCCTCAGTGGTGCAGAGGCAGTCCTAATGCACGCGTCACTCTTTGTTTGCAGAGGACCAGCTGCCGTCTGGGTTCCCGACTATC
GACATGGGACCTGAAGGTGGTGAAGAAGGGTCGCACTGCCACCATGCTGTGTGCAGCCGGTGGGAACCCAGACCCTGAGATC
TCTTGGTTCAAAGACTTCCTTCCTGTGGACCCTGCTCAAGCAACGGTCGTATCAAACAGCTGCGATCAGGTGAGCTGAGTTAAGGC
AGAGTCCTGAGTACCTTGGGGAAGGCACCTTCCAGCCTGGTCCAGCCAGGTGCTGGAGGATGAGCTTTCTAAGAAAGACTCATTAGA
ATGTCCCTATTGTCATAGGGGACAGGATGTGGTGGCTAGCGCTGTCCTGATCCTTCTGGCATTGTCAGGCACCACACTTTAGGCCAG
TTTTGCTCCTCCAGGCATGCGTGACACGCCAGTGTGGCAGGCCTGAGGAAGGTGCCCTGGCTTTTCATCTACTTTCCTAGAGCCCAG
```

EP 2 204 376 A2

```
TCCACGTCTCTGCTTCTCTAGGCAGCATGGCTTGCTGGGGAAGGGGCCAGTGATACTTAATTCATCTGCCTAAGAGCCTGAAACTAG
GGGCATCTGCCTCTGTGCTGTGTGGCCACCAGAGCCACCTGTTTCCCTCCCGTGGATGTCTCAGTCGAGCCACCTTGGAGCCCTGGGAA
CAAGGATGCCATTGTGACCAGGAGGGAAGTGTCGCCCCGTTGATCGATCTGCCTGTGAGGCTCCCGCCAAGAAAATGGATTTTGATT
TTGTAGGGATGGAGTGGGTGGGAAACGAGGCTCAGAATCCGCTGGGCTGTGTTCTACACCCGCTAGCAGCTCAGGACAGACCCACAG
ACTTGGCTCCAAAAGTCCTGCTGTCTGGGACGAAGGTAGGCCTTTCTCACGTGGCTTGACTTCCTGCTGCCTATCAGCTGTGATGTCA
ACACCATGAAGGATGGTGTGTCTGCTTCTCTTAGGGCTCTTCTGGCTTCAGAGGTACACACCAGTCCCAGCCCCTCCTTAGCCCCTC
CTTTCAGGCCCACACCATTTTGGTACCCAAGAAGAGGAGGGAGTGATGAGACACCCAGCAGGGCAGGATTCCGGCCCCAGCCACGGC
CGCCTGAGACAGCCCACGAAGTGTTAGCTCATTTAATTTAATTAAAAACTCAACAAGATGGAGGCAGCTGTAGTGCAGTTAATTAAAA
CAGCCATAATCAAGGCAGGAAATGGTCCGGCAGTGTTTGTGGCTGCTGCCCAGCACAGCACAGAGGCCGGCATGGCTCCGCTCCCCT
GTATTTGTTCATGGGTGCCCCCAGGCAGGTTCCCCATGCTGCCAGACGGTCCTCCCCGCAGGCCTGACTCCTCCCCGGAATGGCATT
GACTGTCTGTGGCAGGCAGCAGTTCTGGGAAGGCCCGCTGTCTGCAGCATTTGTTCATCAAGTATTTATGTAGCGCTTGCTGTGTGC
CAGACCCCGTGCCACCCCTCCCCTGCCCATGACTCCCCGGGATGCTGCGCAGGCCTCTTCCTTCTTGTCCGCACAGTGACCGACAGC
ACTTCCTCCTCCTTGAGCAAACCTCCTGCCCTCCCTCGGGCTTTGCCACCCCTGATTTCCTCGAGCTGTGCTGTTGTCTCCTTGCCT
GAAGCCCAGGTGGGGTTCTTCTAGCCCAGAGACCTGCCAGCCTCACCTTTGCTCTAACAGTCTGTCCTCCAGCAGGCAAACCCATTGC
TTCTGGTGCTTCAGTCACCGCCTCCTTATCTGTCCTGATACTGGTCAACCCAGCCCAGATATCCTGTGAGCTCCAGGTCCCACATTT
GATGGGTGCTGTCTGGATTTCACCCTATCATGGATGAGCAATGACTTCTTTACAAACAGCCCTTCCCTGCTCCTGTTGACCATTTGT
CCATGAGTAGCAACCTCACCCCCACCCCCAGAGCCCAGCCCCCCATTTAGTAAGGATCCACATCCTCTTCATGCCTCACCAAATGGA
TCTCAGATTGGTGCCTTCTTTCTTTCTGTTGCCTCTGTCCTAGCCTGGGACACCACTCCTGTTCTCCCTGCCTCTAAACTTGCTCTG
AAAACTCTGTCCTCCGGCAGCTGTTGGTGTTGTCCATTCATCTTCCTCCTAGACTCCGAGAGCCAGCGCTGGACCCTGGGCTCCTAA
CCCTTGCCCCTGCCTGCTTGGTCAGATATGTGCTCCTGGTCAGGGACCCTGCTCAATCACATGCTGGCTTTATCTCGTCCTGTCCTA
TCTACATGCACCTTATTAGTTCTGTGGCCTTAGGTAACTTTGTCAGTTTTCATATCTTGCAAAGTAGCTGCATTAAGGACACAGTGA
CACTTTGCGGCCTGGTTTGTGGTAAAGGCTCTGTCCCTGCCTGCTGCCACTTACAGGTAACCACGATGACCCATCCTTGCCCACCTG
GCCCTCCGCTCACTTTAGCTTCTTCCACTGGGCACCTGCACCCAGCTTCTGACTACTCCCTCAGGAGCCACCCTGCTTCATGCTGTC
AGCGTTTGTCTCCAGCCCTACTTACCTAGGCTGGATGTGGGAGGAGCTGGGACAGATTTGCTTGGTAAGGTGCCCCTCCCCACCATA
CCCGGCTCTCTGTAAGTCCTCATTGAGGAATTACTTTATTGACCTTTGGGACTTATGACTGAAGATGATATCTACTCATTTGACCTT
TCCTGCTCCAGGAAGCTGTGACTTAAGGATCATAAATAGGCCAGGGAGGGTGTGTGCCCAGTGCTTGGGATGAAGCCGTGTGAGGAC
ATCCCTTCACAGAGTGGGCTCAGGCCCCTATTATACACTCGCCTGTGTCCAAGACCCCTTGTGGTTTTCATGTCACCCACAGTTCAC
AGGGTGCTTTTCTACCCTTATTACCTCAGAGTCTCTTGCAGCGTCTCTGCAGCAGGACAGGCCGTGTTGATTATGTCTACTTTCCGC
AGGTCAAGGGGTCACACGTGGAGGTCCTGGGAGGTAACCCAGGGAGGTCCTTCTAACAGCATATCCTAACCCTGGAGTCTGAGAAGT
AGTTGTATCCATCAGAGCTCCAGACATTAGCTCTCAGACCTCAGGTCCCCCTGGCGGTCTCTACAGTACCGCCAGCATCTGTGAGC
CATAGCACATCTGTACACATCAGCTCTGTTTGAAGGGCTCTTTTCCCGCTGCAGCCATGGGATCCCTGCCTCGGTACCCCATGGGGC
ACTTGAGCTTGAGACCTCAGGCAAGCCCATGCAGAAAGGACGCCTGCTGTGGCAGTGGCCGAATTGGGGCTGTCAAGTGGAGATGGC
AGCTCTGCCTGCAGGAGGAGGCCTCTGTTCCCTCTGATGAGATGCTCCTGGCTTCTCCCTCTGCTGGCTGGCCCAGCTCTGACTGGA
AGAGCCTATCTACCCTGCCTTGCTCTGTCTTTGACTGGGTCTTCCAGAATGGTGCAGTCAGCATGTGAGTTCACTGGCACGAATCTC
TTGGGGAGGCTGACACTTGGGCCCCTTCAGTGCCATATACGTGTCTAGTGAAACAGAAAAGCCAGATTCTCAGTCAGTGGGTTAAGT
GGAGAGAGAACAGAAGCCACAGGCTTGGCTTCAGTGCCCAGGCTGTGCACACTACAGCCCCCGCCCCCAGCTCCAGCCTAGACTGTAGG
AGGTCAGTGCTTGTCAACTTTCTGGTCTTAGTATTGCTAACTTTAGCTCAGAGGACCGTAAGGTAGTTTTGTTTAGCTGGCTTATAC
CTATTGATCTTTACCGTATTAGACATTTAAGATAGAAGATTTTAAATGTTTACTAATTATTTTAAAATAATTTAAAAACTATCAAGT
GTTACCATAAATAAACATTCTAAAATAAAAAAGATAATTGTGTACTCTAAGACAAAAAATATTTACTTAGGAAAACCTCACTGTTAA
TTTGGAGCTTCAGTGTGGCTGCGTTCAGCCTGTTAACAGCGTTACAGGTTGTGAACCCTTCAGGAAATGACACTGTATGTACATAAG
AGAAAGCATCTAAATAAGGCAGATAGTTCTGGTTTTGAAACAAGACCTTTACTGTATAGCCCTGGGCATGATACCTCTATAGAGCCCA
GCCTCTCCTAGAACTTGCTGCAGTCACCCTGTCTCAGCCTCCCAAGTGTTAGGATTACAGGCAGACTCCACACCCCCTCCCCAGTCT
TAGTATTGTAGGAACTACTAATTGTAGGCTTTATTCTGGTGGACTCCCTAAAAGGTTCTCAGTGATTCCCTCTCCCCCAAACACAC
ACACGCGCACGCATGCATGCACGCATGCACGCATACACACCCCTCTGGAGCACACATGGAGAATCTCTATGTTGGTCTAGTATCACT
TTCCTATATGAACACATCACTCCCTCCCCAGCCCCCACCGGTGCCTTCTAGCTTCCGTATCTGTAGGATGTCCGTCAGGAAGGCTCC
AAGTTAGACTGGTTCGATCCTGTTGCCTCACATAAAAGCCTTTTACTGTTTCCAGGCCTCCTTTTCCTCATTCGTGCATAGAGATGG
GTATTTTTTCCAGATGGGTGTTGCCAAGTTTGATGGGACAGGAACTGATCTCTGGCCCCAGCACTTCCAACCCAACCCCCACTGAAG
GATCGAGAGGGTGGATAGACTCGGATCTGGGTCAGTCTATAGGTTCCTGGGTCAGTCTAGGTCCCACTGAAGGAGCTAGAGGGTGGA
TAGACTCGGATCTGGGTCAGTCTATAGGTTCCTGGGTCAGTCTAGGTCCCACTGAAGGAGCTAGAGGGTGGATAGACTCGGATCTGG
GTCAGTCTATAGGTTCCTACTGAAGGAGCTAGAGGGTGGATAGACTTGGATCTGGGTCAGTCTATAGGTTCCTGGCTCCGGTGGTTGA
ACTGGAAGTCTCGGGACAGGCACCTGTGGAAGGCAAACCTACTAAGGGTTCGTAACAGGCCTGGCTTAGGAAGACAAATGCCTGCCT
CTTTTGGCTGCCGATCCCAGGGGTCTGAAGCACCAGACACACAGGGGTGGGTGCTGACCCTTTGCCTCTCACCAGATAGGCTGAGGA
GCTCTGGCTACTCTATCCTAATAGGGGCAGGTGGGTTTTACAGGCTGTTAGCACCGGGGTTTGAGTTGAGTCCCACGCTTCCCCTG
TTGGATGCCAGGGCTGCTCTGTGCCTTGGGATCCCTGATTCATCATGGTAACCTATGTGAGCTCTCCCCTTAACCCTGCCTGCTTAA
ATGCCCTGGTTTTGTTTTGGTAAGTGCTCTCCTTGGACGCTTACCCTGTGGGATTTGGGCGGTCTTTGGGGTGGTTGCTGCTCCCGT
TTCTCTCCATCAGCCCAGCCAGCAAGGGTCATAGGGAGCCCAGCCCCCACCTCCCTGATTCCTGTCAACGCCTCTGCCAGTTCCAGTTC
TCTGCTTCCCCCGAGCAGTGCTAGTGACCCAGGGGACATGCTTCACCCACACTCAGCCTGGCCCAGCCTAGTGCTTGCTCACACAAC
ACTCGAGTCACCTACACTGAGGCCGCAAGTTCTCCATATATATATAGTTAAAGCCCTTTCTAGTGTTTTGTTTTATCTGATTTGGCT
TTGTGTGGCTTTCCTTCCTTTTGTACTAATGTTTCTCTCTGATCTTATTTGCATTCAAATTACCTCGCCAGGTGGTTCACCAATCAG
AGGTAAGAATGCTGTCCGTGCATTTCGCCACGCCACGCCACGTCACACCACGCCACACCACACCACTACTGTCGCCGTCGGTCACCT
CCACTCCATCCCGTCCAGTCCGTCACCTCCCCATACCCATCCTGCCCTCTCCAGTCTCTTCATCTCCAGCTCCAGCCCCTTCTGCCA
CCAGCCACCACACACCTCCACTCTCAGTCATTCCCTCCTGTGGACTCATCCATTATAAGTCTGCATTGTGGACATAACCAGCTGAGA
CCACCCAGGATGGCAAGCCCTGCTACTCAGGCTTTCTAGGCTAGTCCCACAGAACGGACCCATTTGGTTCCCATAGCGAATCCTAGC
CTGAACCTAAGGCTGGCATAGGCTGGGGAGCATGACAAGTCTGGCCCTTTCCCGTGAGAGTCTCCTTTTTCTTAGGATAGTTACCGC
TTGGCCTTTCCTGTAGTATTTTGGTCCCGTAGTCAGATGAGGATCATCCCTTATATCTCTTCAGCAGCAAGGACTAGGATCTTAGGT
GGCAATTGGGAAATAAACAGACCCATGTATAACTAGGTCAGCTGACCTGTCAGTTGCAGCCCCCAGAACTGCCTTCACACGACAG
GGTCCTGCAACCTACTGGTGAGAAAGCATAGATTCTGGGCTGATAGAGGTCAGGGTTCAAGTCTGCCTCCTCTTAGAACTAGAGCCA
GGACCACCTCCCCTCAGGCTGTGTGACCTACAGGAGACCGAGGACGGGTATGGACAGTTTGTCACATACAAAATGTAGTTTGTGTTT
CTATGCCACTATTTTCTTCCCATATTCGTCAATATTTGTGGGCACATCTTGGCCCTCTCTTGAAGGCTAGAGAGCTCAGGAGAGAAG
CTGGGGCTGGGGAGTAGCTATTTGTCCCTCTGACACCAGGACTCGGTTTCCCTTCCCTCTCTGCCTCTCCCTGCACATCCGTCTCCT
TAGCACACGCCCACCCTGCTCTCCCTGCTACTGGATCTGGGTGTCGGTAACGATTTGGAGATATCTGTGTGCAGATAGATCCAGTG
TCTGTCCTGGGTAATGTCCTAGTTTCCTTTTTATTGCTATGATAAACACCATGACCACAGGTGACCTGGGGAGGACGGGGTTCATTT
GACCAGCATGTCCCAATTACTGTCTATCACTGAGGGAAGTCGGGGCAGGGATTCAAACAAACAGAGAAAGCCAGTGGAGGAGAGCTA
CTTACTGACTTGCTCTCCTTGCCTTGCCCAGCTTGCTTTTTTTCTCTACACCCAGGACTACCTGCCCAGGGGTGTTTCCACCCACAGT
GAGCCTGGCCCTCCTGCATCAATTGCTAATCAAGAAAACATCCCACAGACTTGTCTACCAGCAAATCTAACGGGGGCTTTTTTCTCA
ACTGAGAGTCCCTCTTCTCTCATGACTCTCATGGCATGTGTTAAGTTGATAAAAATAACTAACAAGCACAGGTCTCCTAAGCAGACC
```

```
TAGGCTCTTGTCCCAGGGGAGCTTCCTCCGTCCATCAGCTACAATCCTGGGCACTCTTTCCCTCAGCTTGTGGCACCAGCTGCCTGT
CTATCCCCTTACTTTTCTTGAGCTGACCATGGGCACACTCCACCAGCCAGGGCCCTGATCCTCACCTGTTTGAGTGAACGTCTGCTA
TAACGGTGTCCATTCATTTCATGAATATGACCCACTGGCTGCCAGTCAGCACCGTTGTTGCAGTTGCTGGGCATTTGCCTTGTGCTC
TGATGTCCATGGGGAGGCTGAGGTCAAGGCTACGGACAGCCAGCCCTTTTCCTCTGTGTTAGTGGCTCGCGCACACACTCACACACA
GAGCCACCACCACCACCCTCAGCCTTCTGATGTGTACAGCTCCTGCTGCCATGGTGCTGATGGTGGAAGGGGGGAAGAACGGGGTTTG
AAGAAGCTGAAGCGTGGACCCAGAGTGTCTCCCCGTGGGCCTGGGCTGTGAGGACACAGCTTGAAATTGCCTCTGAGGTTCCTGTCC
AGTCCAGTTAGGTGGGACCTGGCAGGTTCTGTCTGTCCTGTGTGGGCGTGATTGTCAGTCGGGGTGGAAGCAGAAGCTCTTTAGAGC
TCTTCCAGCTGTTCGTGAAGGAGGTGGTTTTCTGTCAGACTGTGTCCTGTGGGGGTTATTAGCTGTCCGTTGGACTGTCCTGTGTGG
GTGTAATTGTCTGTCAAATTGTTCATGGGAGGGGCGTGGTGCTTTGCCCTGTGGGTTTCTCTGTCATAGATAGCTATCCGTTTGCTT
TCACTAGCAGTTTCCAAGTGTTTATTGCTGGGGGGTGGGGTGGCATCAATGCAAATGATCCTTCACCTTGGGGTCACTCACTTGACT
TGACATGGCAGTGTGGGTCCCAAATGCAAACTTCATCCATCCTCTCAGAGGGTCCTTTCAGCTACCCCCACCCCCAGCCTTGGCTCC
TGTCTGTCCGCCTGTGGCCAGGAGCAGCCCCTCAGGGCGCTGCCCCAAGTGGGAGCTACAGTCTCCATTAGCCTTGTGAATTATGCA
GCAGCCTTGGTGGGTCGAAGCACTTTAGCAAGACCAAGCCGAGGAAAGTGGCCGGAAAGGGTGGGGCTAGCAGGGTGGAGGCAGAGT
GGGAGACGATGGTGGGAGTGTGGGGCCGAGGCTGCTGAGCGGCCAGCCCCAAGGCAAGCCCCTTCCTTCCTTCCGAGTAGATGATAG
TGTCTCAGGCTCTTGTTTGATGAGGACATTGCTTCTTTAGGAATTCCAGACCTATGGGGTCTGTAGGCTTTGAGGACAAGAACATTG
ATAAGCATGGTTATTGGCAAATAGCCTTAGGACTCACTCGAACAGTGGTGGACGCTCTATGGACAGCCCCTGGGTGGATCTTGCTTC
TAAGTACAAGTTCTTGACTCTTGCTTAGAGGGACCTGGCTCATCAGAAAGAACTTCCTGAAAGCCACACACAGGCCCTAGGCTGTGG
CCCAGGAGAGCCCCTGTCGCTGGGGCTACACTTGTTGTCGAGGCTGCCGTGTCCCAGGCCACACGCACCATGGACCGTGACCGACGT
CTTCTCTTGTGTCCCCCCCACAGGTGCATTGCAGATAGAGAGCAGCGAGGAGTCTGACCAAGGCAAGTACGAGTGTGTGGCCACCAA
CTCTGCAGGCACACGCTACTCGGCCCCCGCCAACCTGTATGTGCGAGGTAAGAACTTGGCCACACACCCAGCTCTGTCAGCTTGGAGCC
AGGCGTCACCTGCCAAGCATTGCTGCCTAGAGGAGACCTTGGTGCAAATACTGGAGGGACTCCGAAAGTCTTTCCTTCCCATCTCTC
TCCGCAGCAGCACTAACAGCCACCACCTCCATAGCTCCCAGGGGCCATTCTACTAGCAGCCGCCACGACTTGCCTTCTTTCCCTTCA
GGCCCTTGGGGAAGCAGACGCTCCCTCCACCCTCTCACCCTTGGGAGCATTGTGTATCTCTTGTAGGTCTTTGCTGCCCGGTCCCAGA
GCCCTATGGGAGTCTCGGAGCCTTCTGAGCAGACATTGCTGTTCGTGTGTGTGTGTGTGTGTGTGTGTGTGTGTGTGTGTGTGTGTGT
GTGTGTGGTGTGGTGGGGGGTGGGGAGTGGGCACACAGACACATGCAGTTACATGTGAATATGCATTTTTGACCTGACTTTCCCTCCT
GCTACTCCAGCAGGCTGGGCTCTAGGACTCTGGTTCTAAACTTTAAATCTGGCTCAGGAATGGCCCAGATCGGCCTTCCGGGAGGAG
CCTGCTGTGGTCTACGACCAACTCCGCCCCTCACGTTGCGTGAGGCTGAGGTAGCTGAGCCCCTATGGGTATATTGCCTTGAGCATT
CCTAGTTGGGGCGAAATGGACTGGGGGCTATTTACAGTATTTACAGTTCGGTCCACAGCTGAAGGGTCTGGGACTGTTTTGGTGTGT
CCAGCCTCTGTTTATAGCCAGAGGGAATCAAAGTTGGCCGCCTGGGCCTCCCTGAGACCATGAGAGATAGTCGAGAAAGATGGATCT
CTCACTGGGGATGGCTGAAGGAAAGAGACCAAGCTTCCTTGTAGCTCCCTGCAGTAGTCACTCCGTCCCTTAAAAGCTTCCTTCCCT
CTTTCTGAGGATATGTATGACGTCTCTCCAACTCATGGAGCCCAAGTGATGGCAGTGGCCTGTCCTGTCTCCAAGGCAGTTCAGTAGAC
TTGCTTACCCAAGGTAGAGAGGTGCAAGCCACCAGCTGCTTACTGAGGGCCAGCCATGCCCCAGGGCACAGGTTTTGGAGGGGTCAA
AGGTGTCAGCTTGTTAGTTGACCAGAGGGGCTGTTTGTATTGGGCTAATTGTGTTCCTTCATGGGAGTTGGGAAACCCCTCTCTCTG
TCCTTGTTGCTGGGTGCCCTTGTATACCCATCAGTGAGATGACCCACGGCCAGTCTTGAGGAACACCACCAGAACAGAGACCAGGAA
CCCAAAAACTTCCTGAAGGCAGAGAGGAGTAGAACTGGAGAGAGCACACTTAACCCAGGGCTGATCCAGGCTGGGTATCTTAACAAA
GGCCCAGGAGTCTGCCAAGTTCCCTCTGGGAGATTAGCAGCTGAAAAGATTTCACAATGACTGGCCTCCTCCCCGCTCTTCAGCCAA
GGCAGCCCCTATCTACACATGGCTACAGCAGAGAGCGGAGAATCCAGCCAGGGCCTTGGTAAACCCCAAGCCAGCTGGCTGTTTAAGC
ATTGCTTTATGGCCGTTTCCTAGGGTAGAGCTGCCAGAGGCAGTACCAAAGCAGGAAAAGGCCACCCAGATTCAGAAAGGATGACGC
GGGGCATCCTAGCTAGGCACCACACCTGTCCAGCTCGCACCGACATGCTGGCTGTGGCCAACGGCGGGGCCATGGTTATCTCTGGAA
ATGGACTGAGGTGGCTGCTAAGGCACTCCTTGGAGCATCCCTACCTGCAGAGTCCCCGCTGTCCACACATGATTGACTTGGACATGA
CCCTAGCTGGAGCCCTGCCTTCTCCCACCTCGTGGCATAAGATCCACTTGGAAGAGCTTTGGGAGGTCAAGGGCATGGGAGCTTCTTT
ACCCATTTTACGCTCAGCCCTTCCTGCCTAGAGCCTAACGTCGCCAACCTGGCTTCTATAGCTCACCCTCCAGGCCCTGCCTCATAT
CTAGAACCCATCTAAATCTATGAGGGACCTTTGATTCTCGGATCTGTACCGTAAGGAAAGCCCTGCTCACTGAGACCCTGGCCTGTA
GTGTTTGCAGCTTCCTTTTGTGCCTAGCAAAGCCCATCAGCCCCAGGAGTGCTCTGGGACCACCTTGTGTAGTAGTACTAACTACC
AGCTCTGTATTTGTAACTCTGTATGTGGGTGGCTAGGGATGGAAGAACCCCGCCCCACTCCAGTGCCTATCTGCTGGACTCTTTGTC
TCCCAATATAGCAATTTCAGTCCATAATGGTTCCTGTTATCACCCAGAGATGGAAGTCCCCCAACTCTTGTCAGGCCTTAAGCCTCC
TGATTCAGGCTAGTATGAATTATAGCTCAGATGTTTCTTACCCTTCGGACTGTTGACATTTTAGACTGAATACATCTCTGTTGAGAG
CCATTCTACGCATAGTAGGATATTTAGAAGCATCCCTAGCCAGTCAGGAACCAGTAGCAGCACTTAATAAGACCAGTCTAAATGGCC
ACAACTCTCTCCAGACGCTGGCACCCAGAGGCAGTTTGCCCCTGTGGATACCCTTTCTCGGGATTGCTGCCTCCTGAGGGCTTCTGT
CTCCATCTCTAACTTTGTTCTTCTGTCTAGTGACTCATCTTCTCAGACTTAGCCTCCCTCCTCACGTCCTTCCTCTGTTCCCCAGAC
TGTCGGGGCTCTCCCTGCCTCTCTGAGGCCTTAGGCTGCTTCAACCGATGGGGCAGTGCATGGGCATCAGAGCTTCCCGCATGCTGAC
CAGGAGCACAGGAGGCGCGTGAAGGTCAGCTGTCTGGGCTCTGTTGGGCTAGCGCTGGGGCACACACATCGTGCAGTCACAGCTGAC
TTTATTCACAGTTGCCTGCAGTTCCACCCACCGTCTAGCAGGCCCAGGCACGCTGGGTGGACCAGCCTCTTTCTCCATCACCATCTG
ACTCCCTCCCTGAGTGACTCACTCTCTCCTTCATCCACGGCGTCTCTCCCCTCCGGCTCTCAGGCATACAGCTTCCCGGGCCAAAG
GCAGAACTCTGCCCCTTGGCAAAGCTTGCCTTACCTGTACCAAGATCACCAGCCTCTCCGTTCTCTTCAGGAACCCGGCATCTGAGAC
TCAGCTGTATGACTAGAGGACATTTCCTCTTAGCCTTTCTTTCCACAGGCATCCGGGCGTGCTCCTCACACTGGTGTTGGCGAGGCA
TGGGGCTTCCTCACAGTGGTGGGGCTGGAGTGGCTTGTGTGGCCTCAGTGGGTTTGTGAGCTGGGTGGACCCTGACCCCAGAGCCTGG
TAGGGTCTGTCTGAGCGGTGAGGGTGTGTGAGGCATGGTGACTAGCCCGTGCCCCACTTCTACCCCTACCTGCTCTGCCCTCATCGT
CCCTGGCACTGCTGGTGGGCCCTACCCTGTCCACTGTGTCTCTCCATGAGTCCTAAGTCCTCTGTGAGCGGCGTGGTCCGTGTGCAC
CTGTGTGCACGTGTGTGTGCGAGGGGCACAGGAGTCCTGTCTTGTCTCTGTGCTGTGTGGGCAGATGGAGGTTGGCCTGTTTTTACT
CTCTCTGTGTTTCTCCTTGTCTTTTTTTATTCCCTCTCATCCTCATCGCACTCTGCCATCAACCCAAACTCTCATCTCTCAGATCAG
CGAGAAGGTTGGTCGTTTTCACTTCTTATCCATCTACAGTTCGCCCACCGACTGTGCCCATCAGTTGGGCAGCCAGCGGGCTTGGTCGG
CTCCCTCAGGCTCACTCCAGCCGCGCCTGCCCGCCAGTTGGCCTCTTCTCGGTGTTTGGGCTGGCTGGCAGGCAGGACAGGGGATGG
GTGGGCAGGCCTTCTGCCCTGCATGTACCTGTTGCTTGACCCTGATTCGCTGTGTGTCTGCATGTCCCCTTAGCCAGGCTCCGCTGT
TGGAGTGGCCACACATGCACGGTGCACGGCATAAAACTGTACCCTACAGAACTGCACTCAGCTTTGCACCCAGACCAGGCTAGCCCT
CTGTTTCAGGAATGGTCATGGGGATCTCTGTCAGAGAAACTAAACTACATGCTTGGCATGTAGGAGTTCAGGGAAGCTTCTACCATT
TCCCCACCCACCCTTATGTTGTCTGCGGAACTAAGAGAAGGCAGAACTGCAGGAGGCAATAGGAGGAGGCTAATTTAGAGTGAGTTG
TCCCCTTGAGATCTCACAGTACGCGACACAGTCCTGCAGTGTCTGCCCTGCCCTTGAGGCCCCCAGAGCCTTACCAATGCTCCACAA
AGCAGACTGGCTGTACTGAGACTTAAGACCAGCCTTGGTCTGCCTCTCAAAGACCCTGATGGCCGTAGTCCAAGGCAGGTGGCATCA
TTTTCATACACTGAGTAAGTGTCCCGACATTATATCTTTTCTGTCTCCTACCAGGTCCAAACCAAGGTCACCCTGCCTGCCCAGACC
ACTCTACCCGAGCTTCCAAGCCAGCCTCTGTGCCTCTCCGAGGGTACAGTGTATGTGGCTCACTGACCAGCTAACAGCCCTCAGTCC
CATGCTGGGCAGTGGCACTGAGCGTTCAGATCCAGTTTGCTTATGCCTGGAGTGACTGAATTGTCACTGCAGTCTCCTTAGGCCTTG
AGTATCAATCAGTCCAGCTCAGCCTTAGATTGGGCAGGGCTTTTTGAAACTCCCTGGATCCTCTCCCAGAGCTGTCCCCTGGAGCCA
GGCCTCTATTTCCCAGTACTGGACCAACAAGGGGCTGGTGGTCCTTTATATATCGTGTGGTGTAAAGGGTTAATCACTGGGCTTTGG
GGGGTCCAAGGAAGAAGCACAGGGCAGACCCCAGCTCCCAGGTGGCCTCTGCATCATGCTCTGTGGCTTTGAGTTTACTCCGGGAGG
ACAAGGGAGCCAGTCAGGAGGAGGGGCTGCAGCCTGGGCTCAGATATCAACATAAGCCTCCATCTGTATGCACTCCTGAGCTCTGGGG
```

```
CATTTCGGTTACACCATTTCGCTGACCAGGCGGATCTGCCAGGGCGTTCAGAAAATAGTCATCAGCAGACAGCCCAGCCCGCAGCCA
CTCCCCCCACACCCCTTCACCTACCCTCTGCATCCTTTGTTCTCTCCCCACCCACCCTGTGGTCCCACCAAAAATACACACATACAC
AAATATTTAACGGGAAGGAGAAATGAGTTTCTAAATATTCCGGGGGGATTTGCCGGGCTGGTAATTTGTTTGGTGCTGATAATTGCA
TCTTACATTTTTCTAGCTTTACTTAAAACTGTGTGCCGGGTGTGCCAGCATTTAATTACTGCTCTGCGGCAGCCACAAGGTTATTTA
TTAAAGAGTTATTTTATCTTGATACAGTGGATCCTGCCCCTTATCCCCTCCTTAATGTTGTGTTATTTTCATCAGAGAAATTTCCGC
AAGTGAATGCAGATTGCGGGGCTACCTCCCCCTGCGATTAGGCTGTCACTCCACTGAATGCGGATACCTCCAGGCGCCGCACCACCC
TATTATAGGGGGTTGTCAGCGCAAATAATTAGACTTAAAAGTTACAGCTGAAATATAATCCAGAAATGGCAGGGCCCTGTTTTGCAA
ATTGTCTATAAAATGTCAGCAGTAAGGATGCACGGGGAACAGTAATAGACCGGCATTGTTGGAGCCTGAGATTAGACCCTAAGTGCA
TTTTCCCCAGCTCCAGTTTTTCCTTCCCTGCTGTTGCTTCTGTCAATAGACCAAGTCCAGGGAGAGTCCTGTTCCCTTTGGAGGCCC
TGTGCTTGTGTGGCAGCCGGGGGAGGACGGTGGAGATGCTATGTTGGAGCATCAGCCACTTGCAACTGTTGGCACAGGAGTAGCTGTCT
AGGCTGGGCTAGGACACAGGGCCTCTAGCATTTGGGGCACTTTGTTGCCTCTTTCCCCATTTTCAGTAGATATGCTGGCCTACCTGA
GCTCTAGCAGATTGACTTCCAGGAGTCTTTGCATGTGGCTGGACCCCAGTGCCCTTCTATAGGAATGTAGTTATCAGTGGAATACCT
GGGGCCTCTGGCTGAGTTCCTTTCCCAGTGCTGAGGTGACCCTGACCTCACACCTGTCTGTAGCCCCCAAAGTTCTCCCTATAGCTT
GCATCTTGGAGGGGAAAATAAAAGCCATTCTTAGCCGGGCGTGGTGGCACACGCCTTTAATCCCAGCACTCAGGAGGCAAAGGCAGGG
GGATTTCTGAGTTCGAGGCCAGCCTGGTCTACAAAGTGAGTTTCCAGGACAGCGCAGGGCTATACAGAGAAACCCTGTCTCGAAAACC
AAAAAAAAAGAAAAAGCCATTCTTAGTGTCACTTCCCATGGGGGCCCAGTTTCCTGACATCGACTAGAGATGGAGCCTTTGAAAGGA
GCGGCCCAGGGCATTGGCCTGGCTCCGAAGCTGATCCCTGAGGACTCTGCTGGCTTGGAAAACACTGTCCACCATGGACTATCCAGG
GGTCAAAGTTTGGACATGTTAGGTATGGGCCCCAGGTATTTTAGCCAAAGACCTACTTCCTACTGCATAAACCCAGTGGCCCCTCT
TTCATTTGGGTTCCACCATTAACTAGAGCCACCATTAACTAGTGTCACTCTCAAAAGTCTTATCTGTATGCCATCTGGAGCTCGACA
TTATGCCAACGCTAAAGCCCCATGGCCTTCATGGGCTTTCGAAGATAGAGTTTTTACCACACAGACCTGATCTTCCTGAGGATATAA
AGGCAGATGCGTCACTTCCTGTGTCAGCATTGACTCTGGCCCTCATCTGCAATTGAGACATGGTGGCACAGGCACCCTCTGTACAGA
GTACAGAGTGGCTTTCCACTAGGCATGATGTGCATGGCTTTAATCCCAGCACTCAGAAGGCAGAGGCAGGTAGACCTTTGTGAGTTC
AAGGCCACCCTGGTGTATACACTAAGCTTCAGGATAGCCAGGGATATATAAACCCTGTCTAAAAAAAAAAAAAAAGAGTGGCTTCATC
AGTACATGACTGCATGCCCTTCAATGAAAAGGAAATGACAGAGAGGCTTGTTCAGTTCCAGCAACTTCTGTGTTCTGAACTCCTCCA
TTCCTTGCTAGGCCTGGACCTGGCTTACATGCAGCACTGTATACCTGTTCTTCACCTTGGAGTGCTCAGCTATTAGACCTGGTATGA
AGCCCAGGAGTGCCCATGCCCTGGGCTGGCAAAGCTGCCTGTACCATCTGCCAGTTGAAAGTTCTTGCAAGGGAGTCTGTTACCAT
GGGACACTTGGTAGCTATGATCCCTGGGATATACCTGTCCCTTGTCATGAAAACTGTGTCTGTGATACCGTGTCATCAGCCATGTAG
ACTCCTGATAGTAACATACATGATGCCCGGCAGTGGTGGCACACACCTTTAATCCCAGCACTTAGGAGGCAGAGCCAGGCAGATTTC
TGAGTTCAAGGCCAGCCTGGTTAACAGAGTGAGTTCCAGGACAGCCAGGGCTACACAGAGAAACCCTGTCTCAAAAAACCAACAACA
AAAAACAAAAACAAACAAAAACAAAAACAAAAATCCATACATGATAAAAAGGCTAGGCATGGTGTCATACACTTAATCCCAGCACTCAG
GAAGGGGGCTGAGCTGAAGGCCAGCCTGCTCTACAGAGTAAGTTTCAGGACAGCCAAGGGTATACAGAGAAATACTGTTTTGAACAA
CAACAAAAAGCATCAATGATGAGGGGTTTGGAGAGAAGGGTTAAGAGCATTTGCTCTTGAAGAGGTCTATGTTCAGTTTCCAGCACC
TACATAGTGGCTTCCAACCATCTGTAACTTCAGTTACAGAGTCTCTAGCATCCTCTTCTAGCCTTCGTGGGTTCAGCATACATTTA
TGATTGTGCAGGCCTACATACACATACATTAAAAAAAAAATTAGGGTCAGAGATTTGGTTGAATGGTTAAGACTACTTGTTCTTGCAA
AGGGCCCAGGCTCAATTCCCAGTTCCATCCTGAAAGCTCACCACATCCTTGGGTATCAGGCACCTAGAGTAATAATAAAATAAATCT
TTAAGTGAATAATAAAGTAAGTTTTGTTGGACAGTGGTCACCACAAACCTTTAATCCCTTCACTTGGGAGGCAGAGGCAGGTGGATTT
CTGAGCTCAAGGCCAACATGGTCTACAGAGTGAGTTCCAGAACAGCCAGGGCTACACAGAGAAATCCTGTCTCAAAAAACCAAACAAG
AAATAGATACGTTTTTTTTCAAAAGAGACTTAGGATAAACCCAGGTATGCTAGACTAAGCAAAATAAAGAGTCTTATGATACAAAACT
AGGAAGTGTAGAAATAGATTCGGTGTTGGGAAGCCCTGGAGCCAGGTCTTCAGTGTGTTCGGGCCGCTGCTCGCCATCGCCTCGCTC
TGTTCCTCCACTGCTCTTACCTTTGTACTGCAGCCGGTCCTCCCCGGCCTTAGGAAAGGAAACCCTGAGCTCAGCTGCCCCTGTAAA
GTGTGTTGCTGCTCTCACCGGCTGTGCAAACATCCAAAGGTTGCTTCTGGTGCAAAGGTTGTTACTTATTAGCCAGATCTAGATTAC
AAACCCCGTGCCAAGGATGGAGAGATGGCTCCCCTAGAGAAAACAAAGATGGGACTGGAAATGTTGCTCAGTGGTAGAGTACAACAC
CTAGCATATATGAGTCTCTGAGTCTTCTTCCTCTAAAAGAGAGAGGAAGAGAGGCCTTGGGGGGGGCGGGGAGGAAAGAATGAGGG
TGCTGCTCTTTCAGGCCAAAGGTGGAAGTCTAGACATGTACAATGCACTCCAGGTAAATGTCTGCCGTGAGTTTAGGGAGCAGACCC
AGCCTGCCTTTCGCTGTGACTCACTTGGGGCTGCCAGTGTTGTGATCCCACCCAGGGCTTAGGGAACCTGGCATAGAAGGTGTGAA
GCAAGTTCTACCCACGGTCCTGTAGGCTGGCCACGGAGCATCAGGAACCACACAGAAGCTTGTTAATCAGGGGCCCAGTTGTCTG
TGCAGAGCTTGGGAGTTGGTTAGGGTGAGAGGTTAGAGGGAGCGCAGGAGTGGTGTTATTTAGATGTTACTAGTAGGGGTGATGGAT
TTTTTTTTTGTACCCCATTTTTTAATAGGGTTATGTGATTTTTCTGGAGTCCAGCTTCTTTTTTGTTGTTGTTGTTTTGTTTTGTTG
TTTTTCTTTTTTGTTGTTGTTGCTTTTTTTTTTTAAATTGGGTATTTTCTTCATTTACATTTCAAATACTATCCCAAANNNNNNNNN
NNNNNNNNNNNCCCTCCTCCCCTACCCACCCACTCCCACTTCTTGGCCCTGGTGTTCCCCTGTACTGGGGTATATAAAGTTTGCTAG
ACCCAAGGGCCTCTCTTCCCAATGATGGCCGACTAGGCCATCTTCTGCTACATATGCAGCTAGAGACATGAGCTCCGGGGGGTACTG
GTTAGTTCATACTGTTGTTCCACCTATAGGGTTGCAGACCCCTTCAGCTTTTGGGTACTTTCTCTAGCTCCTCCATTGGGGGCCCTG
TGTTCCATCCAATAGATGACTGTGAGCATCCACTTCTGTATTTGCCAGGTACCAGCATAGCCTCACAGGAGACAGCTATATCAGGGT
CCTTTCAGCAAAATCTTGCTGGCATATGCAATAGTGTCTGCGTTTGGTGGCTGATTATGGGATGGACCTCTGGGTGGGGCAGTCTCT
GGATGGTCCGTCCTTAGGGTGATGGATTTTGAAAGCTGTCTTAGGCAGCCCTTAGTACAACCCCGGTGACTTTTACTACATGTCTCA
GCTCTAAGCCTCAGTCTGACCCCTAGGAATTGAGGTGAGATTGAACCTTGGGTTCTTTCTTGGGATGCCACCTCTGCTTCTGTGACC
TGGGACTGCGTGACATGCTTCTTACAATGGGTCCTCTGCATGTCCGGACCCCCCAGCAAGAGTCCTCAGAGACCTGTGCTCTCATAG
TGGCCCTCAGGTCACTTGGGCTTGCTGCCTGTCCTCTCTGGCCCTTTGTGTAGGGTGGCCGTCTAGGATACACATGAGCCTCTGCCA
GCAGCTGCATGACCTGCCTGTGTGCCAAGCATGTCTCTGTGTAGCTAGCTCTTGTCCACCTTACCCTTTCTGCATGCTCTGAGTGTC
TGTGCCAAGCTTGCCACTTCGGGGTTGGTTTCGGGCTTTGCCCTGGACTGAGAAGCCCAGGACAGCTTCTGGCCAGGGGAGTCCAAG
GCTGAGGGTTGCGGCTAGGCACAGCCACCTGCCTTGAGGACTTTACTCACTGGTGCAGAAGCCGTCTGCTGGCGCCCCTCATCATG
TTACTGCCATCCTCATGCCCATCCTGCCGCGGACCACGTCCCTGGCCCGCCCAGCCCCTTGTCCCCGGACTAGCCCTTCCTAAGGGG
CCATCCACATTGTGGGTCCCAGGCTGACCTGCATAGGCAAGGCCCAGCAGGTACTTGTGCCATTTTCTCTTCTCTCCTGGGGGCATG
GCCTAGAGGTGGAGACACTGACTCCAGAGAAGGTTGCCATGGTACTCCCAGACTGATCCTTTGCCCCACCCCCCCACCCCCAGCCATG
CTCTGAGGACCTGGATTGCTTTCATTGATGGTCCCTCTGGAGACACAGGAGGAGGGGAGTCAGGAATAAACAAGGGTGGTCATTGGA
GGACCACCTGGAGTGTGGCCCTAACCAGGCCTGGCTCTTGACTGTGTGACCTTGGCTGAGTTGTCCCATGCCATAAGATG
GGAGCTTGGTTATGCAAGTTGTCATTAAGAGGTGTTCCAGATGGTCCCTGAGTGCAGGGACGACTTCTGGAAAGTGAGGTGGGAAAT
GGTGAGCCAGGCCCCAGGGACGCCACTCCTCCTGTCTCTGAGCATGGGCTTGGCTCTGACAAGAGAGGCTTCCTTGATGAGGGGCCC
CTGCCCTTCTGTCCACCCTTGCATCCCGCCCAGCCTGTGAGTGCCTCTCTCCCTCCTCCCACAGTGCGTCGCGTGGCTCCTCGTTTC
TCCATCCCTCCCAGCAACCAAGAGGTGATGCCGGGCGGCAGCGTGAATCTCACATGTGTGGCAGTGGGCGCGCCCATGCCGTATGTG
AAATGGATGATGGCGCCGCCGAGGAACCTGACCAAAGAGGATGAAGTCACGGTTGGCCGAAATGTTCTGGAGCTCAGCAATGTCATGCGA
TCTGCCAACTATACCTGTGTGGCCATCTCTCTTCATTAGGCATGATAGAAGCCACGGCCCAGGTCACAGTAAAAGGTGAGTGTAGCGGT
GCCTCAGCTAGCTGATGTTCTCCTCTGTCTTGGGCTTTAGATCCTCAGGCCTGTGGCAGATAAGGAGGAGCTGGCCTTGAACTCATA
GAGCTCTTCCTGCCTCTGCTCACCAAGAACTGGGGACACAGCTCAGTTGATAGAGAGCATTTACCATGTTAGAAGCCCTGGATTCAG
TCCCCGGTACTGCCAAGAGGAAATCAGGCTGTAAGCTAGGATTAATCATGTCACTCTTCCTGGTCCTCTTTGGGAAGGAGCCCAGGG
CCTCACACACACTAGAGAAGCGCCTACACTCTGAGCCTTACACTGGTTCCTTTATAAAAGCAAAAGGTAGCTTTCAGGGGCTCACGT
```

EP 2 204 376 A2

```
TGCTCTGGGAGTCACCCAGAAACATTAGCCGGTCTCCCACTCTCAGTTTCCGCCAGCTACACTAAAACACACAGTTCTAGTCCCCTG
CCTGCCCCTCCTCTATCACAGTAACTGGCTCAGCACTGTGAGACTCTTCCCGTCCCCCAAGCCCCACACCCATCCAAGGAGAGGCTG
TGCTTTCCTCCTGTCCTTGTTCACAGTTACCATCAACTCATTTGCTGATTATTTTCCCAGACGATAAATTCCAGAGAGCAGGAATGT
GACCACGAGCCATTGTGGCACCAAATGGGGACTTAGTGGCTATTCATTCTTCATGCAGCGATCGCAGGCCTGGGATTGTGCCACCTT
TGTTTTGAGCCAAGTCATGGGGCCTAGTGGCGGGGCTAGCAAGTTCATGGCTCTTTCTGAACTCTAAAGGCCAATTCCTTGGGAGAT
CGTGCATGCGTGTGTGCGTGTGTTACACTTGGTACAAGTGAGAGTAGATGAGCAACAGGTAGGTCTCTGCCTAGTGTAAGCATGTGA
ACTGTGTGAGACTTGATAGGACCAGGTCTGTTTGTTGGGTGAATCACATGTGCTAATGTGACGATCCAGTGCGGGTGTGTGTGTGTG
TGTGTGTGTGTGTGTGTGTGTAGGTTCTAAGGGTGATGTGTTACACACCAAAGGTCTTAAGACTCCTTGGACCACCATCAGACCT
GGCTGCACTATCTCAGTAGTTGCCCTAAGAAGTCACCACAAGCTTTTCAAGCCTTTTTTGAAGGAGGGTTGTGGGCATCAACCACAC
TTAGGTGGCCATACCCTGTGTTAGGATGATGCCTTGGGTTTGTCCTCAGAATCATGATATCTAACAATTCTTTCTCCTCAGCTCTGC
CAAAGCCTCCAATTGATCTTGTGGTGACAGAGACAACCGCCACCAGTGTTACTCTGACATGGGACTCTGGAAATACCGAGCCTGTGT
CCTTCTACGGCATCCAGTACCGCGCAGCTGGCACAGACGGCCCCTTCCAGGAAGTGGATGGTGTGGCCAGCACCCGATACAGCATTG
GTGGCCTCAGCCCCTTTTCGGAATATGCTTTCCGTGTGTTGGCTGTTAACAGCATTGGGCGAGGACCACCCAGTGAGGCAGTGCGAG
CACGCACGGGGGAGCAGGCACCCTCCAGCCCTCCGCGCCGTGTGCAGGCTCGAATGCTTAGCGCCAGCACCATGCTGGTCCAGTGGG
AACCACCTGAAGAGCCCAATGGCCTGGTACGGGGATATCGCTCTGGCTACTATACCCCAGATTCCCGACGCCCTTTGAGCGCCTGGCACA
AGCATAACACTGACGCAGGACTCCTTACCACCGTGGGCAGCCTGCTACCTGGCATCACCTACAGCCTCCGAGTCCTTGCCTTCACTG
CTGTGGGTGACGGCCCACCCAGCCCCACCATCCAAGTCAAGACACAGCAGGGAGGTAGGTAGGGGCTTTGTTCTAGATGGACAGCTG
ACATCAGAGGCTCCGGCAGAGTCCCACCTGTTTCCTCAGGCTGTGAAGTTTGGAGCTTGGGAGGGGCGAGGGTGTCATACTCCACAG
ATAGGTGGTCACTGGGGATGCAGGCCAGGCTGGTGAGAGCTTGGCACCCTCTCACTGGGCCCTTGGAGGTGTTGCGAGGCTGACCTT
GCCTGGGGTGGACATTGCAGTGCCTGCACAGCCTGCGGAGTTCCAGGCTAACGCTGAGTCGGACACCAGGATCCAGCTCTGTGGCT
GCTGCCCCCGCAGGAGCGGATCGTCAAGTATGAGCTGGTGTACTGGGCAGCCGAGGATGAGGGCCAGCAGGTGAGCCAGGAGGCCAG
GAAGGGTGGAAGGGGGATAGCCCCCAGTTCCTGGTGGTCTGTGATGGGCACACCTAGGCAGGCTGCAGACCCTAGGTTTCCCTACAG
CTTTGTAGCTCTCACAGCCTATGTGGTCTGCACCCACCCCTGTGCTAGAGGCAAAACTTCTAAAGTTTGCCTTTGTTCTCTTGGGTTA
GTGGCCCGTGTGATACTGTCCTAGCCTTGAAGCTTCCCCTGGGGACACAGTTCCCTGGTGGCCTTTGTGATCTTTGAGGTGGCTGTG
GCATGAGTGCACGCCATGCATTTCTTGCAAGGCTGTGTGACTATGTGGGTGGGGCTGTCAGAGAACCACAGTGGGTCCTCTGCCAGC
TGCTTGCAGCCCACGTGGGGAATAACCTCTGAGTGGCCTTTGAGATCTGAGCAGTCAGTAAGTGNNNNNNNNNNNNNNNNNNNNNNNCAC
CGTAGAGGCCCGTACAGCACAGTCCAGTAAGTGTCTGCTAAGGCCACCCTTGTCACATCTGGGCTGGTACACTACACACGGGCACAT
ACCATTCCCTTTCAAGCAGATGGGTGGTCAGGTCTGCTGAGTCCAGTATTGGACTCCAGTCTGGGCTCTTGAGTCTGAACCCCGAGA
AAGGGACAGGTAAGTGTCCTGTTAGTCTGTGAACAGGCAGTGGTTGCCTTCTCTGCTCCCTGCCCAGCCTTCTGACCCTCTCTTACC
CCTGTGACTCTTATGTCCTGCACAGGTAAGTTCTGTGTGGCGTGTGACTCATCTCTATCCTCTGTCCTTCTCCTTCCATACCTAGATC
ATAGCACTGATTTGGTGGGCCTGCCACGTTTGAGACCTGCCTAGATGCCTGTCTTCCTCCTCATGGTCTCACATCTCATCACCCATG
TTCCCCACCTCATACCCCACCACCACAGTTCTTTGGGAGTTGTGTAATCTCTGAGGCCTCTGCACTTGCGGACAGGTCCATGAGAAT
GTGACCGTGTGCCAGTGTGTGTGTGTCCCCACATCTGCCTGTGGGAGCACATGGCTTCTGTGACAACGTGACTGTTTGTAGGACCAC
CTGGAGTTTGTGTGAAGACCCAGCCAATAGCACCTGAGTGTGTCAGTGTGTGTGGGCGTGACTCTGTCGCACCCAGATTGTGATTGG
CACAAGATGGCAGATTGTAAATGTGACCTTGGTGTGATAGACTAAATTCATTCTAGAAGCCTATGCTCTAGGTCTGGTAGCGCAG
CCAGAAGGCAAGGCCAGTATCCATAGGGCTCCTGCAGTGGACTGAGACAAACAGTGGTGCCTGTAATTACCCAAATGAGCTCTTAAT
TACATTTGTGCAGAGTGAGCTGGGAGGGTGGAGAGATGGCAGGCAGTACAGGAGCTACTGTTGTTGGTGCAAGGTCAGAGGCAAACA
CTGTTGGTGCAAGGTCAGAGAGAGTTCAGTCTCAGAGCTATGACACGTGACCTCCACTGAAGCTAATTAGGTAAAAAGAACAGGGAA
TATGGTGGCCCTGGGTAGGGAATATAGTGGCCCTGGGTAGGGACTCTGCAGCCACCCAAGCTGTACCAGTCGGGTAGCTCTGAAAAT
GGGCAGGTGACCCAAGATGCAGAGACCACTTAGAAACCTCTTTTGTGGACTAGGCGAAGTTTGCTGCCTTGGCTGAACTGGTCAGGG
GGTGCGGGAGGGGTTACCTAGGCTACTTCCTGGTCTGGGCTTGGTGATAGATTGCAGATGCCGATGCCGTGGCTTAGAGATATGGAGGT
GCATCATGTTCCTGATAGAAGGCTCCAGCAGTGCGCGCTCCCCCCAAGTCAGGTGATGCATGGAAGCAGGCTCGAGCAGTGCGCCTG
GTTTCAGGCACCCAGATTCAATCGCTGTGTCTGTCAGCGGTAGAAGTGTGGAGGTGTAGGGCCTGAGCTGGAGGAGGCAGTCCTGAG
AAGACTAGAAATGAGATCTAGGGCTGGGGAAGAGGACAGAGAGAAAGACAGAGGTGGGCCTGAAGTTGCTGCTGAGTGGCTGAAAGG
GACTAGAAGTAATCAGAAAGCAGGCTTGGGGGTGGGGTGGTGGTGGCAGGCTGTGGTGGTAGCTTGGCAGGGGCTCGGAGTGGATGAG
GAAGTAAGCCAGCATGCTAAGATGTCATTAGGAGCTAGGTGGGGCGCTGTAGCAATGAGGATGGGGTCTTCTTTGAAGGCGGGAACT
CTTGAAGTGCGTCCGTATGCTTTTGTGAGTTAGGAGGACCACGCTGGAAGCATGGATGGAGAGGGACAGCTGGGAAAGTCTCTGGAG
GGAGAAGGAAGGGAGCAGAATGTAGGTGAGAAAGAGCTCGGCCTTGGACTCCACTCTTGAGATTGTACAGGAAGAGGGGGGCTGGGG
CTGGAAATAGAAGCGGGTACTGTTGGAAGGCAGGCAGGCAGCAAGCTTATATGCCTACAGGTAAAGGGAGCTGGGGGTCTGGAAGCT
GTGCAGATTGACTGCGTTATTTGTAAAGTGGCAGAGTGACAAGATAGATTAGTAGATCTCACGCAGGTAGCCCAGAGAGCCCAGGGGAG
CCCGCTGGAGATGCCTTGTCTGCCTGCCTCAGGGCTGCAGTATCCATTACCTAGTGTGGCTCAAGAGATGGTGGGAGAAGGAGGGGC
TGAGGGGATGGCTCAGCAGGTAAGAGCGCTTGCTGCGTAATCATGAAGAGTTGAGTTCAAATCCTCGGCACCCACATAAACAATAGG
GTATCGTCATATATGCACCTTAGCACTAGGACTGTTGGGAGGCCGAGTCAGGAATCTGTGGGCAGGTGCCCATACTCACATATACA
TACACTACACACACTTACAGAGATGAGAAAGTGAGTTTAGGGCACTGGTGAGAATGGTAAGCCAGGGTGCTTTAAGTCACGTCTGAA
TATTCAGGGCTGTAAGGATGGAAGTAAGTGATGGGATCCCCAGGGAGTAAGAGTAGAATGCTGGGGCCCAGGGAGACCCAACTGGGAC
AAGAGGTGGGCGCACAGGGTAAGGTTAAAGGTCATTCCCAGTACTGGTGTGGTGCTTAGCCACCGGTGAGTCAAAGGCTGTTGAAGT
TACCCAGCCAGTGAGTGGCAAACTTGAGGTAAACGGAAGCCCCTGGATTGGTAGTGGCATGGTGTCACCAGGCACAAGGAGAGCACA
TACTTGCCTCCTAACTCATAAGGAATCTGTTGAATACTGCCATCCCTGTGACTAGCCGCAGACGTTGATAGCTCAAGCACAGAGCTC
TGTGCATGCAGGCTCAGGCTAAGTAGTCTTAAACCAAGGGAGGCCTTTGCATCACGTGTCCCAGAAGAGAATTATATTCTCCAAGTG
TTCTGGTGTGTGTGTGTGTGTGTGTGTGTGTGTGTGTATACTTGATACCATGTTTGTGTGAGACGGCATATGCATGGGAAAGACGCTGTTA
GAACTCCATATGTGCGTGTGAGACACTGCACATACTGTGTGTGTGTGTGTGTTTAGAAGTAGTTCTGAGGGAGTTAGGCTGTC
TCAGCGCCATATGCCTAGAGGCCTCAGGATGGCTGGGTGAGTCTCCAGTCCCTTGTTACTTGGTCACTGTCCTGTGGTCCCCAC
CCTCCATTTGCCTGCTTTCCCCCCATTTGTCTTCCCCAGCCCCATCAGCCCCTCCCCAGAAGGTGACATGTGTGAGCACGGGCTCCA
CCACGGTCCGGGTAAGTTGGGTTCCACCGCCGGCCGACAGCCGCAACGGCATTATCACCCAGTACTCCGTGGCCTATGAGGCAGTGG
ACGGCGAAGACCGTAAGCGACATGTGGTGGATGGCATCAGCCGTGAGCATTCCAGCTGGGACCTGCTGGGCCTGGAGAAGTGGACGG
AGTACCGGGTGTGGGTGCGGGCACACAGATGTGGGCCCTGAGAGCAGCCCGGTCTGGTGCACCGATGAGGACGGTA
GGCTGCAGTGGGTAACATTGTTATGAGGAGGGGTGGGGATGAGAAGCATGCCACCTGAGGCTGTCTGCCAAGCACCCAAGATCCCTC
CACAGGACCTGACAGACTCATTTTCTAAATTTAGGCTTAATGGTCTAAACTCTGGGGCGATGTAGTTGGTGGGGTATCCCGTATCCA
GGTCCTGGCTTCTGAGCCGTCTCCAAGATAACTGAAGCTTTAAAACCGTACAGGGCTGTGGCAGAAGCAGCAGCCCCACCTAATATG
AGAGCCAGGATTGAGAAGATGAGTGCAAAAGACTCAAGTGGGCTTTGTCTGGATACTAATGAAATTTGTTACAGATGGTGTGAGCCT
AGGTAGTTGAAGGTGTGGTACCTCACTTCCAGCTGTTCCCCACATCTATGTTTCCCATGAATAAGTAGCCCTGCTAGAGGGACCCAT
AGGGATGGCCCTCGGCAGGCAAATCAGCCAGCCAAAGAACAACATTGGAACCTTTCCCAAGGGGGCAGGTGCAGAGTCACGGCCCT
AAAGTTAGCTGTACGTGCCTAAGCCAGGCGCTGTTGCTCCATATCTGACTCTCTTCTCTGCCTGGCCCCTAGTGCCTAGCGGGCCAC
CACGGAAGGTAGAGGTTGAGCCTCTGAACTCCACTGCTGTGCATGTCTCCTGGAAGCTGCCCGTCCCCAACAAGCAGCACGGACAGA
TTCGTGGCTACCAGGTCACCTATGTGCGGTTGGAGAATGGTGAGCCCCGAGGCCAACCCATCATCCAGGATGTCATGCTGGCTGAGG
CTCAGGTATGTCATTGACGGTGCTAGGTGGCAGGGTAAACAGTGACCAGTGTGTCACAGTGACCCCCTAGAGCCCAGATTTGTCCCT
```

222

```
CCAATGCCTGCCCTCAGAGGTGGTTATGCCTTTTATGGTCATTGGAGCTGCCTTGGACTGTCTGGGCAGGAGTTATTGAGATCGGGA
GGTGAGGCTCATGTTCCAGAGACTTTGTTCTTCTGTTAGGATTGTTAAGCTGTGCTAACCTAGGTCCTGCTACCCCCTCCACCCAGG
TAGCAGTCTTAGTTGCCAGTGCTCACGGGGCTGCTCCCAAAGACCCCCAGGCTCCTTGCACAGCACACTGGCTTCATCCTTGTGGTT
TGCAGAAAGCACATTTGCTGTGGCTTTGAGCTGAACTGCTGTCCCAGGCAATACTGTTCTTAGCATCACCTGGACTGCACAGCCAGG
GGCAGACAAGACTTTTCTCCATCCTCCATGGCCTCAAGCCAGTGACTGAGGCAAAGCTCTGCAAATAATGACCTGAGTGTCACTCTG
CTCTTGACCTGAGTGTTTCTCTCCAGGGCTGGACCTGCCAGCCCTTTTCTGTTGGAAGCTTCTGTCTTCCTGGGGCATCTGCCCACA
GGCTTACAGTGAGGTCAGTATTCTGACCCCCACCTTGCTAGTCCATCAAGGAGCACCGAATACTGTGAGATTATATGTGTGGCCAGA
ATGAGCCAGCATGTAGGTGGCAGGGATTGGAGAGGAACCAAGTCTCTACCCTGGGAAAGGACTAATCAAGTATAGGAAAAACTGTGT
TGTCTGTCCTTGGTCTCCCTGTCTAGAACCAGGTCCTGATGATTCTTGGGCCAGCTTCAAGTCCCAACAGAAAGATCCTAGATTCCC
TCCCCAACCAAGTGGGAACCCCATATTCTGCAGGCTGTGCTGAGGGATGAACCCCCACACAGACAAACTGCTTTCATACCTTTGGCT
AAGCTCCACCCTCTGAGCCAAAATTCTTTTCTACCCAGAGGGTACCCTCTTTCTTCTCCCAACCTGTCTATGCTGAAGGACCCAAGG
GTGGGGGAGGTGGGTGTGTTCATCAGCCTGCATTCCTGGGGCAGGTTGAGGGCTCCTCACAAAGTCTCAGCAGACACACCCGCTCAG
AAACATCCAGAGAATGACTCCATCCAGCCCCTCCCATCCTAGGAGTCTGGCGCCACAGCTGCAGCCTCTGTGGTTTCAGGAAAGTCA
CTTCGAGTACTTTTGTGTGCTGGTCCCCAAGAGTTTGAAGGGGGTTGGGTAATAAGACCAGGGACATTGTCCCCATATTTGTGTTCT
TTACAAGGAATATGCCCAAGCAGGCTCCAGGGAGGCCACGGAAGACAAGGCCTGCCCTTGTAGCCTTCCACCCCCTGGGACTTTGTC
ATTCTGCTTTGGGCCAGTGGGAGACCAGATGGTGCACTTATCCAGCCTGGCCCCAGGGCCTAGGTATCCCCTGTGTGACTCTTTTCA
AGGACACCCCAGCCTGCCCTTATCCACCCAATAGAACCATCTGTGCAGCCCTGGGCCCCAGTAAGATCAGAAAGAAGCATGTGGTGT
GTATCCCCACCAGATGTGCATAGCCCAGCCTGCCTCCCCTGTGCTCCCTCTAGATGAGACTCGCCACCCTCCGCCCCCTTTTTGTCC
CTTCCTTTTGTCTTTTCTCTCACTGTAGGAGTTTAGTCTAGCTACTCTCCATCCCCTTTTCCTTCTCTCCTCTCCCTACTCCTCTTA
CAGCCTCTTCCTTTCCCACCTACCCCTCTGTCTTTTTCCTCTCACTACCTGACATAGGAGGGGCTACCTGCGATAGCTTCACACCAG
GGCTCCCCTCAAGTAAGGAATCCTAACTAGACCTGGTTCCAGCCCGCTCTGATAATTCCAGGACTCTATAGCCTCTCTATATTCTGT
CGCAAAGACTGTGACAGAACTGTCTCAAATAGCAGGAGCTCAGGCCACTCCAGCACCAGGCTAGTGGACAGGCCAGACCAGCTTATA
TGAGGGTGATCTGGCTAGAGGACTCCAAACAGGGCCTGTCCCTCACTAGCCAACTCCTAGTTTCAAGACCACAAGGCTTCACATTAG
AGTCCTCACTCCATATACCTGTTCACCCAGCATACCAGTGCACGCTGGCATTAGAATTTACCACTGCCAGCCAAGAAACTAGTTTTC
CTATTTTGACTCCAGGCACCTGTAGAATCGAAATCAGGGGTGTGGGGTCTCCTTGTGGCTTTGGGGTAATGCCCAGGCTCTGAGCTG
GCTACTTACCCTTTATTATCTCTCTGGCAAGCCTGACTCTTGGCCCACTCCAGCGGTAAGTCCAGCACTTGTGTTCTAGGACACTCA
GCTCAGGTGACTCTAATTTAGTGTCTCAGCTGAAAGCAGGATGCATGGATGGATGGATAAGTGGATGGATGGATAGGTAGGTAGAAA
GAGAGAGAGAGAGAGAGGTAGGTAGGTAGAAAGATAATATAGATAGACAGAGGAAGAGAGGGAGGTAGATAGATAGATAGATAGA
TAGATAGATAGATAGATAGATAGATAGGGGGAGCAAGCTGAACAACCATAGCAGGACATTGGGACTCTAAGCACATATCTTCCCTCT
GCCTAAGCTGTCAAGTCTATCTCTACATCTGACAGTCCACCATGTCTCCCTGCCCTCGACAGGCCTGGTGCACACAATTGGTGCTCA
ATGATGCAGGAACTTGTGTCTTTGAGTGGGCACTGGTCCTGGGCTATCACCTCAGTGGGTACTTCAGAGCTATCTATCAGTTCTCCA
TGGGCCAGGTCCTCTCCAGGCCAAATCCTTCACCATCCTGTCTCCCTTTTTCTCCTTCTCCCGCTGTCAGTGGAGACCAGAGGAGTC
CGAGGACTATGTAAGTAACAGGTGTGCAAGCACGGGCAAGACTGGGTCAGACTGGGCTCATGGGACACTCCCCCTCTCCCAGCCTGT
GTTGCCGAGATCCACAGGGCACCAGCCACATCCAGAGAAAATTGGCGTGTAGACATGAGCACCAGGCTGAGCAGTGATTGGCGTTTC
CTCTAATCCGTACTCCTCGCCTGTCGAGCAGCAATTTGAGGCCAATGTTCAACATCGACCAGGACCTGGCCCCTGCTCTGGCCAGAC
GGGGATGGAGTTAAATCCAATCCCGATCGTTAGGCCTTATTGATCCCTAGAGTGAAAAATCACCTGCTCAGGCCCAGGCTGGGAGAA
GCACTGGGAGCCAGGTCCAACACAGGCACTGCTGGAGCCCGAGCCCTGGGAGACCCTCCAGCTTGGCCCTGCAGAGGGTTGTTGGCA
TCTGCAGTTGGCCCCACCTGAGGCTTAGAGCTGGAGGGACACCAGGGCTGGTGCTGACAGCCCTATGCCTACTCCATGGTGGTACTC
TCCCCTCCTCCTCCTCTCTGTGTGGCGTGGCTTGGTCTCCTATGGTGTTTCTCCGCATGTCCGGAAACATTGCCTTTGCTGGATGGG
TGTGTGGGTAGGATCCGCCAAGGTAGAAGGAAGTGGACTGGTGGCGACTTCTTGGGCATCGGTGGAAACAAGAGGGGAATGTGTTCCTG
GGTTTGGGGAATTCTGTGTCCTGTCTTTTTTTTCCCTCTTATGGTGGTCTCAGGAGGGATTGGATATCCCAGTCACCGGAGTAGATAG
GGTCACTGTAACTTACAGTGTTGTAGGTTGGGCCCTCTCCTTTACCCGGCTCTTTTATAAGTGGCCTTGCACCAGCTAATTCCAGAG
GAATGGTGGCCTGTATGCATTTGCGTGCCAAGAGGCATGGAAGGTACCTGGTGCATGCAAGGCTCTGTCTGTGTGTAGAGACCTCAT
GAGGGTGTCACTCACAGCACAATGGCATGAAAGTAACAGTGTGGTCCTTTGTTCTTCTCTGAAACAAAGAATGAGCTATCTGCCCCAG
GCCTGGGGTTTTGGGTTGGGTGGCATGCTCCTAGCCCACGTCCCACCTGGCTGTCCTGCCTCCAGCAAAGGAAGCAGAGGAAGGATT
TGTCCAGCTTCACCTCCCAGGACCCTGGTCCTGGTCTGTGTACCTGGGGCACCCTAGGCTCCAGTCTTCAGGTGACTGGTAGTCTAACTTCT
TACGCAGTATGATGGGACCTGCCTTTCCCCTTCTTCAGGAGACCACCATCAGCGGCCTCACCCCCGAGACCACCTACTCCATCACCG
TCGCTGCCTACACCACTAAGGGGGATGGTGCCCGAAGCAAGCCCAAGGTGGTTACTACAACAGGGGCAGGTGAGTGAGGGGTTCATG
GACAAAGCTGGGTGGGCAGCAGGGAGGATGGCACCAGAGCCCAGTGCGTGCTGTTCAGTCCCAGGACGGCCCACCATGATGGTCAGC
ACCACGGCCATGCACACCGCGCTGCTGCAGTGGCCACCCACCCAAGGAGCTGCCTGGAGGCTGCTGGGCTACCGTCTCCAATACCGG
CGGGCCGACGAGGCGCGGCCCAACACCATAGACTTTGGCAAGGATGACCAACATTTTACAGTCACCGGCTGCACAAAGGGGCCACC
TACGTCTTCCGGCTCGCTGCCAAGAACCGGGCTGGCCCAGGAGAAGAGTTTGAGAAAGAGATCACCACCCCCGAGGATGTGCCCAGT
GGCTTTCCTCAAAATCTTCGAGTGACAGGACTGACCACATCTACCACGGAACTGACCTGGGACCCGCCGGTGCTGGCGGAGAGGAAT
GGTCACATCACCAACTATACTGTGGTCTACCGTGACATCAACAGCCAGCTTGAACTACAGAATGTCACCAACGCACACACCTTACA
CTCTTGGGCCTTAAACCGGACACCACTTATGACATCAAGGTCCGTGCACATACCAGCAAAGGCGCCGGCCCTCTCAGCCCCAGCATC
CAGTCCCGGACCATGCCCGTGGAGCAAGGTGTGTGTCATGTATGCCATTGGCTGCCAAGCGTGACTGCTCCTAGGGACCTGTGC
TGTCCTTGACCCACTGACCTCTCGCAGCTGATCCACCAGCTTCTGCTGTGTGACACTTCACGCTCTCATGACCACTAACCTCTAATG
GACAGGCACCACATCCTTGGGTGTGTGGCCCTGACCTCTGCTGTCCTTGACCTACTGACCTATCTGCGTGGCCTTCCAATCTCTTGT
GATCTTGATCCACTGATCTCTTTCTGCTACATCACCTTCTAGGGTGTGCAGCTCTCTCTACTTTGCTCTGTAGCACTAACCTTTTGG
GGCCTAACCTACTGACATCCAGAGTCCGTGTGGCCACGTACATCTTGTGACCACAACCCACTAATCCCTGGCTACTCTGAACCAGTG
TCTGAGCCGAGCTTCCCACCCATGCTCTTTCAGAGGTGTGGCCCCAGGACTGGCCATCCCCAGCCGGTTCAGTGTTCACTGTGGAA
GAACCTGGGTTAGGAGTTGGACTGTGCCATGTTAAGCTGCCTGGGGTACTTGGTTGTCACCCTTAGTCCATAGCCCCTCCCTCCCCT
ACTCCTGTGCTACAGCCATTGTCACATCCTCGGGCCCCTTCACCACAGCCCAGCTAGCCACAGATCTCACTCCCTCCTCGTGCTTCC
ACAGTGTTTGCCAAGAATTTCCGTGTGGCCGCTGCGATGAAGACATCTGTGCTGCTCAGTTGGGAGGTCCCCGACTCTTATAAGTCA
GCTGTGCCCTTCAAGGTGGGTAGGACTTCTGCCAACCTAGACCAGAGCTGCCTCCCATGTCTGCTCTGCATCGCCATTCCGATCCCA
GCTGCTCTTCCCCCTCCCCAGATCCTGTACAATGGGCAGAGGTGAGGGCATTGGGTGGCCATTCGATGCGGGAAGCTGATTGCAGA
CCTGCAACCCAACACGGAGTACTCCTTCGTCCTGATGAATCGTGGCAGTAGCGCCGGGGGCCTACAGCCACCTGGTGTCCATCCGCAC
TGCCCCGGACCTCCTACCCCAGAAGCCACTGCCTGCCTCCGCCTTTATAGAGGATGGCCGCTTCTCCCTCTCCATGCCTCAAGTGCA
GGACCCCTCGCTAGTCAGGTGTACAGAGGAGGCTGGAGGCTGGTGCAGATCGGGAGCGTAGCAAAGATCGATGAAGGTTCTGATGGT
TGCTCTGTCCACACAGGTGGTTCTACATTGTGGTGGTGCCCATTGACCGTGTGGGCGGGAACTTGCTGGCACCAAGATGGAACACAC
CAGAGGAGTTGGAGCTGGACGAGGTGCCTAGGGGAAGATCAGTCTGAGGGCAGCCACGTGGTGGGCAGTGGCCCATCTTTCCCTGAG
TGTTGCCCCGAGGAGTCCAAATCTGGCTGTCAGCCGCTCTCTCTCTCTCTGGGGGAGAGGACCTCTGAGGAGTATCCCCAGGAGAATTT
TTAAAACTCCGGAAGGGCCTACAGGGACTTCCTGGAGGTATTTGGTAAGAATCCCATGGTATAGGAGTCACTAGAGACATTTCTGC
AGCTCTCAGTGTGGACCTCAGAGATGCCTGTGGTGTAGAGCTTTAAGCCTAAGTTCTCCTGAACAGATGAAATCACCTCTTTCCTCC
TCACAGCTCATGTCAAAACTGCTTTCTCTGATTAGGATAAGGTTATTCATGTGGCCCTTCCTTATAGGCTACAAGGAGGGTCTCGCT
GTGATGTCAGAAGTATCCTGTAATGGCTATGGCCAGGCCAGGCCCATGAGGGTCCCCAGGAAGAGGGAACCTCTATATCTGCCTCAG
```

```
TAGCTAATGCCCCAGGGGAGGAGGCTCCCAGGCCTTGTGTGACTCTGTGCTCTCCTCTGCCAGCTTCTGGAGGCCATCGAGCAGGGC
GAGGAGAAACAGCGGAGGCGCCGGCGCCAAGCAGAGCGGCTGAAGCCTTATGTGGCGGCCCAAGTGGATGCGCTCCCTGACACCTTC
ACCCTGGGGGACAAGAAGAGCTACCGCGGCTTCTACAACCGGCCCCTGTCTCCGGATCTGAGTTACCAGTGCTTCGTTCTCGCCTCC
CTCAAGGAACCCATGGACCAGGTTGGCCTGAGCTGGCATGACTTTCAGAACCCCAAGACCCCAGGCTTAATCTAAGACCAGGCCAGC
TTCAGACCTTAGGAGACCCCGGGTCTGCATTATCCTGAGACTCCAGCATCCTAGGACTAGGACACCCCTGAGGACCTATACTGGATA
TCCCATGACACTAGGCCTCAGGGTGTGAAGATCAGACCTAACATGGCTTCAGTAACCCTGCTGACCTGTCACATGTCGGTCTCACTG
GTCTCGGTGCCCTCCCTGGGTTTGATGGATGAGGGTGGGGCTTTCTTGCTGTGCTGTGCTCATAGCCTTTGCTGTTGCTTCCTTCCC
TGGACCCCAGAAGCGCTACGCCTCCAGCCCCTACTCGGACGAGATTGTAGTCCAGGTGACGCCAGCACAGCAGCAGGAGGAGCCCGA
GATGCTGTGGGTGACAGGCCCTGTCCTGGCGGTCATTCTCATCATACTCATTGTCATCGCCATCCTCCTGTTCAAGAGGTGAGTGCC
GCGCCTGGTTCTGGGGGACAGAACCTGCCTCCTCGCCTCGCTGGTACCCTCTGGTGCAGTGTTAAGTGCCCAGATGGACGGAAACAGC
GGGTCTGTGTGTGTGTGTGCACTCGGCTGTGCAGGTCCGCCTTTATGAAGAAGGGCTGAGGTGAACTGTGCGTGTCCCGAGGCGTGT
CTGTCTGTGTCTATATGTGTGAGTCTGTCATCTTCAGTTTGAAGGAGCCACAGCTTCCTGGTGTCCTGCTGTAAGCACTTTTCTTCT
GCCACCGAGTGACAGGCTCGTAACGGCTATCACCAGCCCAGGCCCTCCTCTCCTCCTTAGGCCCACAAGAGGTCCAGGCTGAGTTG
CAGACCTACATCCCCATGAGAATTACACAACAGACATTTACTAAAGACCCATCATAGACGTGGCTGTCAACACTTACCTGTCTTGGG
TAGTTGTCGCCACCTGTCCTTGGAGGTGCCCTGCCTGCTATCCTGTGGCTCAGCTTCCAAGCTGCTTCCAATTATTTTTACTTGGTCTTGGTCATTC
CCAATATATATATATTTTTTTTCCTCCTAATATTTTTAAGATGAGGTCTCACTGTATAGCCTTGGCTTCCCTACCGTGTAGTCCAGGG
TGGCTTCAGACTCAGAGACCTGCCTGCTTCTGTCTCCCAGGCTACTCTAGAAACTGAGGTAGAAGGATCCAGGCCTAACTTATCTAA
AAGCTATCTCAAAAAAAAAAAAAAAGTTTTGTTTTTTGTTTTTGAGTCAGGGTTTCTCTGTGTAGGAACTAACTCTGTAGACCAGGCT
GGCCTCGAACTTAGGAATCTGCCTGCCTCTGCCTCCCAAGTGCTGGGATTAAAGGCATGTGCCACCACTGCCCAGCTTGTTTTGTTT
TTTAAGGTGAGGTTCTTTGGCTCGGTGTGGAGTGCCTGCCTTTGCGTGTACAAAGTTCTGGATCCAATCGCCAGTAGCACAAAAACT
TAAAAAGTTTTTTTTAAAAAAGCCTAACCTTTGAGCCTCGACAGTTGAAATGCCTGGTTAAATGTGTCTTTAGCATTTCCCTTGACATCA
TTACACCAAATAAATACGGTCCCTTGGCCCCTGGCTCCTGCTCTGCAGCAGTGCCCCCCCCACACACACACACACACAGCCCCAG
TGCCCCCTCACCTGGCAGCTGTTCATACCATGTTCTTACTTCTAGTCTCTGGTGCTTCAGAGACCCCTGGCTCCTGGTCCCATCTTT
ATACAGCCCCTCATCTACCCCAGAGTGCCCCACTGGATATGTGCTGAGCCCCCAGCAGCTAGCACATGGCTAGCTGTTGAACATGTC
CTGTGCTATGTGTGATGTGTCACCTCATGGAGGTACCCACTTCCCTGTCGTTATCCTGCCCTCATCTGGACCCCATAATCCATCCTG
GCAAGTTCCCTCCTTCCTCTTGCGGTCACAGACCTATAGGTCGTCACTAAACAGTGTCCCTCCACCCACTTACCCTCACCATCCCCAC
TCAGGTTCTGATGTGTGTACATGGAGGTGAACTCCCTGAGGTTGTCCTATGGGTACCTTATAAGAGTCCCATGGTTCCCTTGTCATG
TGAGGGCTGAGTGCTTAGCAGCAACAATGACCATTCCTTTCTTCTGTCCTTGCCTGTCTCTCGGGACCTAGTAAACAAGAAAGGTAG
GAGTCGTTCCTTCTCTCTGTACACGCCTCCCCCTCTACTTATCCCTGTGCACATCTTGGGCCTGCAGGAAGGGGTGGAGCTCTGACC
AAACCCCTCTTAGCCTGTGGCTTTAATCAGCAACCTGCCTGCTCAGCACAGGAGGCTTCTCCAGTTCTGGATGGATCCTCATGGGCAC
GAGGGGGGCAGTCTCGATCACATTCAATGTCAGTCCAACTCTGTCCCTTCCGCAGAAGATGCTCCCCACTGGAGCTGTGCACGCTGCA
GCCCCCATTGCTTCCCTGAGTCCACAGAGCTCTCCAGAACCCTCCTTAGGTTCCCATTTCTCCATCCTAATTCCTACGAGAGGCCAC
CTCTGCACTCGGCAGAGCAGCAAGGAAAGGTGTCGTGATGCAGACACGTGCGGGCATATTTCCAAGTTCTCCCCGGACACTCCTGTG
CAGGGGCTGCACGTGGTCCCCAGTAGTCTGTCTGACCCTGTCCTTTTTAGATGGGCAAGGTGACACCAGTGAGAGAGGACTCTGTCT
GGCTAGGGCCCTGCTGTTCCTGCCCCTCCCCTGCTACTCTGGACATTGGCCCTGAGAGCCCAGGCAGGCTGGGACCCAGGCCCTCGTGA
TGGCGTTTGTGTGTTCTGTGCTACCAGTGCATGTTTTCTCGTGTGGTTTCTGCTGCATCAGCTGGGGTCGAGTTAAGGACAGCAGCC
ACTGCCCAGCGGTGACCTGCCTCCTGTCCAGAGGAACCACCAGCCTCTCTGGCCTCCCTCCCTCGCTGCACTGCTCCTGGGCCCACT
CCACACCTGCCGTCTCCCTGCCCTGTCCCTACCCTGTGTTCTCTCATGTTGACGCTGCTCTCCCCTGGCTCACACCCAGAGATCCC
AGGATAAGGTGGGGTGGTGGGAAGACATCTGGGGCCGTGGGAGCTGCCTCTGTTCCCAGTTTTCACTAGCCTCTTCTTGTTTCTTTA
GGAAGAGAACACACTCCCCATCATCAAAGGATGAGCAGTCAATCGGGCTGAAGGACTCCCTGTTGGCCCACTCTTCTGACCCTGTGG
AGATGCGAAGGCTTAACTACCAGACCCCAGGTAGGGTTGATCCCTCGGCCTCTTGGCCTCCAGCTCAGTCCCACAGGTTGTCAAAAG
ACCTTAGTGCACGCTCACTTCTGGCTGCTTCTCTTCCTTTTTTGTTCCGTTTGAATTGACATGATGGGACTTCATGATGGATGGGGG
CATCCATATTGAACAGGTGGACAGGCTAGGAGACTTCTAGAGACAACAGAGGTCCCTGTGTGTCTCCAGTCTCTGAATAGATGGACT
GTAAGAGACCTCTTGAAGCCCTCAGCTCTGGCACAAGTGGTCAGTCTCAATATCTGCCCTCTGGCCCTTAACAACAAACACACACAC
ACACACACACCAGGCTGTGTTAGAAGTGAGAAGCTGTAGCAGACAGAATGGTAGGTGAGGGGCTCATACCTGGCCCTTGATGAAGGC
TATTTCAGGTTCACTGTGCAAGGGTGTAGGGTTCCTGCTCCAGGCTCAATACCTGTCCCTTCCCCTGAGCTGCTTCCATGTGACTTC
GAACTGTTCCTTCCACCTGGAATATCACTCCTTCCCTACACCCCAGAGGTGTCTCTCAGAGAGCGACAGTCTCTAGCTGTAGCTCCA
GGACCTGGCTGGCTCAACCCGTCCTTGTGTTTATCGTGTGGTAGATTCTCGCTGGATGCTTGAGCACACAACAAAGGAATGAGTGAA
TGAAGTCATGTGTGCACACTGAGGGCTTATGTTTGTGTGAGGTGGTTATCACTGCGCATCAAATTACCCCTAACCTTCAAGGCTTAA
AACAACAGTAATTATGTCTTCTCTCTCACAGTTGTTATGAGTCAGGAATTGGGACAGGGCACGGTAGGATACCCAGCCAGGGTAGGC
CTTCAGCCGGAAGATTTGAAAGCTGGGAACTAGAATCTTATAAAGTGGGGCTGGCAAATTATGGCCTGGAGCCAAATACAGCTCACT
GTCTGCTTTATAAATAAAATTTGCCCAGCACACCACCACACCCATTCATTTCTGGGGACATTTGCACTACAAAGGTCAGTTGACAAC
TAGAAGGTGGAAGAGAACCCTGGGTGTCTGCAAGCCTTTAGCACTGGTTTTCTGGCCTTTGGTAGTGAACTTTGCCAACCCTAGGGT
GGTTTGTTCCGGTGGGAGGTAGTTGGCAGACGAGCTAGCCTCTTTGTGTAGCCTGGACTTCCTTATAGCATGGCGACTGAGTTCCAA
AGACACGTGTCTTGAGAAAGTGTTCCGTGTGGACCCAGCGGGCTGTTGTAACCAGTGCGTGAATCTGTGGTTAGAGGAGGGCTGCTCT
CAGAGGGTGCCTCACTTTGTGGGCACCAGACAGAAGTTGGGTTGGAGGTGGTTCTAAGTGGAAGCACAGTGTGTATACATCAAGTT
AAACCCCTGAAGGGTATTTCTGCAGCATTGGAGCCAAGAAGTGACCGTGACCGGCAGGCTGAGCAGCTTAGACTTCATCCTGACTGA
CCAAGGGAGTCATTGAAGGTCTTCATCAGAGGAGCAGCAGAGCTGGCTGCTTCTGTGGAGAGCGGGCGGGCCCTGGGGAAGACATCA
GGCAGTTAAGTGGTAGGAAAGGGAGGCAGGAGAAGAACAGAGTCTGGGAAGCTGAAGGTGGTAAGGCAGGTGAGCTGAGCTCACACA
AGGATGCAAAGCCAGTTCTGTGTCTGCTGCCATAGAGGATATCCAAGTGGCAGTAGGCCTTTGTGTCTGGACTTTGTGCTGACCTCA
GCCCGGGAGCCACCAGTGTGAATGGTACCAAGGATCAGGTAGTGGTCAAAGCTCAGAATGGGCAGACTCTCCCGGGACGAGCAGGAA
GGGAGAAGGGAACAGGCCGCAGGATGGTGGTGTTATAGGGGAGCATGCAGGCAGAGGTGGGCAGAAAGGTTCCCTGTCTCAGGTG
CTGGCGTAGTGTGGCACTGAGAGGGTAGGGCCCATAGCTGTGTTGAGGTATAGGACCCAAGCCTTCAGGCAGAAGTATTTCCATGGG
TTGAAAAGCAGCAAAAGGGGAATGCAGAACGCTTTTCCATATCAGTGTGTGAAGGATGTCATGGGCACATGACATCCCTGAGAAGGGA
GGAACTTGAGATTTTAAAAGATTTTTTCATTTCCGGTATGGAGACCATCTGAAAATGGCCAAGGTCAGATTTTGAGGGGGTGAGGGCT
GGGCTGGAGTGACTTTATTATAGGGGTCTCAAGAGGCCACTCAGACCTATGTTGCTTGGAAGCTCTCTTTGGGGGAGGGGGACTGT
CTGTTACTGGAAGGCACGTGAGCTGACCCACTCTTTCTGTCTGTGCCCCTTCCTCTTGCCCTTCACCTACAGTCCCTTCAGTGTCGA
ACCGCAGAACCAGGTGACCAGAAAGAGGGTTCCTGAGCTCTGCTCCTGTCCCATTGTTTTCTTTTGGTTTGTTTTCTCTGCAGGTTC
CAGTGCCCCCAGTTGCCCGAATATCTCAAGTTAGTTTTCAAAGTTCCCACATTGGTTTGGGGACACTTTGGCTTCAGTGTGTCTCCA
CTCTTCTACTTCCTCACGTCTCCCCACTTTCTCCTTGCCCTTTTCCCCTTGCCTTTATCCCTTCCCTCCCCTTCCGGCGTCTCCCTT
CTCTCTGTCCCTCCTGTGTCCTCCGTCCTGATCCCTTGCCTGTCTTTGTATCTTCCCCATCTCTGCTTCTCTCTCTCCCTACTTCTA
TGCCACTCTGCCATTGTCTTTGCGCTCCATGACCATTCCACCCACACACTTGGTGCCCGGGGTCACGGGTCACCAAATCTCTGAGG
TGACTGGCGTCAATGAGGGGTCGGGGTAAGGGGGTGTCTGTCTCAGACAGGTGTGGTAAGAGCCCAGCTTCCACCCTGTTCCTGAGC
TCGAAGCCCCGTCTTAGTGGGAAGAAGCCCCTTGCTCCTCTTAGGCAGGCCTGGCAGGCAGAGAGCCCTGTTGTCCCGTGCAGCACC
AGGTCATCTGGACATGCTTCTGTGTGCTCACAGTACACACACCTCCATCTCTGGCCTCCCCTGATGCCCAGCCACTGTGTGTTCTCC
```

```
ATTCCTGTTGCCGCCGCCGCATGTGCTGTTGTCTCCATGCCACCAGTGCTGAGTACTTCATGATCACACATGTTCACACATGCCTTG
GGCTGTCTCTACCACCCTGACCAGAACTCACACTAATGCCACCCATGTGTCTGCCCCACCTCCCCCAGGTATGCGAGACCACCCGCC
CATCCCCATCACTGACCTGGCAGACAATATTGAGCGCCTCAAAGCCAACGATGGGCTCAAGTTCTCCCAGGAGTATGAGGTGAGACA
CCCACCACTCCCACCGTGTGTCCTCAGTCTCTCCAAGTCTGGTTTTGTTTTGTCCTGGGTCCCACCTGAGGTCGAGGATATTGGGAT
GTAGGCCAGGGAGATCAAAAGTTCAGTTGGGGAACTGGCCGTCATAGGTGTTGGGCTCAGCCCACAGGCTCACCATAGCCCTCTGGT
GGTCACCCTAGGACATGCTTCAGAAGGCTCTGCTAGGTGATGCCTGCAGAAGCAGACACTGATTTAGTAGCAATGGCTATAATATCT
AATCTCCAACAAGAACAGGCCTGCACTTACCAGGTACGGGACAGCATCCTCTTACTCGGTCCCCCGTGGAGTGGTACTGTCACAGTC
TTGTCTTACATACCAGAAACCAGACTCAAACAGAGGTGTGAGTTACCTAGAGTCAAACAGCTGAGGAGTCGTGGAGCCACCCAGACC
TTGCCTGTGCTGGTTTCATGACCATCCGCACTTCAGTCTTGCATAACAATGTTACTGAGTCTCCACATGGTAACCATTGTCCGAGAA
GGGTGGGAGGGTCTGTTCTCAGCCCGGCTTCCATTCCCGCTCCCACATGTGATCACACATGCATGCCGAAACACATAGCTCATAGAT
GGGGAGTCAGGAAGCCCTCCAAGTGTTCGCCTCACTCTGGCCTTCACCTCTGTTTGCTAGCTCAGACTTCAGGGTCAGTGACCACCT
GGTTCACCCACAGCTCTTTGTCAGGGACGTTTGGGTGTTCCCAGGCCATACAGTCAGGGGAGCCTTGTTAGAAAGGAAGTGCTAAGG
AGACCCCCTTTCTTTGTCTCCCTGCTGTACTGGCCGCTCTTTTGTCAGCACTTGGCATACACCTCAGAGGATGACTGATGGACAGAT
GATGGATAGATCTGTTCAGCTGGACCCAGGGGGTTGCTCTCTTTTAGTCAGGTGTCCACTCTTGAGTTATGTTTGGGGGATATATCC
CTGGGTACCGGATTTGCCCTTCCGAGGGCACTGGGCCAGGTAGATCCTAAAGGCGACAGGCGCAGGCCCAGGCTTCCAAAGAAGAAACTGAGC
TTGGCAGGTAGACAGATGAGCATGCCTCTGCACAGCAACAGTGTCACCTCTGCTGTGGAGGCAGAGCCTGTCAGCCGGGTGGCCTGG
ATTTAGCTTAACTTCTGGACAAGGAGGCCTTCCTTGTTTTTTACCAGTATTCGCCCTTGTCCTGCCCCACGCCTTGGCATCCTTCCT
ATAAGCCCCTGCTCACGGCCGTCACCTTACCACCTCCCCTCCATCCCTCCCATCTGAGCGTTCTTTAAAGGCCCTTCTTTGTCCTAG
AAGCCACCAGATCTATTGTGGGCTTTCCCGAAACTTTCTCTTCATGGTCTGTAGGTCATTTGTCAGACTGTTGTTAGCACTTAACTG
TATTCCAGTCCCTATGCTGACATGTAGAAGCAGATGGCCTTGGGACGCAGTCACTGAGAAACACAGACAAGAGAGCAGTAATCGTTGCTG
TGAGTGTGTGGTTCTGACAGCATGCACTAAAATCATATATAGCAAACACAATGGTGGGTGTGTCTGGTGGGTGGGGCCTCACAGAAG
TGATGAGACAGCCTCCTTAGAGAATAAGGTGGCTGGGACCAAAACGGAGGAGACTGTGATACAGGCATCGATTCTTTCCATCCTCAA
AGGAAGGACAATAACTCAAACCTCTCTTGAGTAAAAAGATGGAGAAATGCACGCAGTGGCTTAGGACCGTGCTGGGCACACCGTGAG
CTCACACAGAAATGTAGCTGTTAGCATTTTTGTGTCCTGAGCCAGTCAAGAGCATGGGAGGCCTCTGAGCAGGAATTTGATACTCAG
ATCTGCATTTTTTTTTTTTTTTTGAAAGACTGTTCTTGCTGAAGACAGAGGGTTGGAGCAGGGCAAGAGAAGAGGCCTGGAGGTAC
CAAGCAGAGCTAGCTGCTGGCAGCAGCAGCAGGTTGAGAAAGGCAGAGGAAAGGAGTGTAGGGGGTGTGGTAAGCAATGGGAGGGA
CCTTGGGGAGGTCCCAAGATGGACTTTAAACCTGGCTGGGCTTCCTGGGTCAGTGATTTGTGCTATCCACTGAGGGGTGGGGGGGTC
ACATCAGAACCTGCTTTGACAGGAAACAGATGAGCTGGCCTTTAAAGGGACTGGGGTGGAAAGAGCTGTGAAGCTTGCACATGGAGA
TGTCTAGGGGGCCGCAGGATAGTAGGGGCAGCTCTGAGATGTAAGCAGGAGAGTCCTGTGAATGGGGTGGCGGTGGCTAATGGGAGT
GGTTCAGTCAGTGAAGATTGACTTACAAAAGGTGAAGATTGACAGCTATAGCTGTGTAAAATGGCCACTTGTAATCCAGAGTGGGGAG
ACAGAGACAGAAGCCACCATGGTACTACTGGCTAGCCAGCCTGGCTTACTTGGTGAACCGGGGGCCACTGAGAAGTCCTGGCTCAAA
GGCAGAGGGTGTTGCTAGGGTTGTCTACCACACAAGTGCACAAACACATATGTGTACACACATGCATTTTAAGAGAGAGAGAGAAAA
GAAAAACAAATCTTGTGAATGGGCTATGGATGAGACTAATTTGGTAGAGTGCTTGCCTAGAATTTGCAAAGCCCTGGATTTCCTCCA
GCATCACATAAATGGGGTAGCACACACTTTAATCCCACCACTCACCCACACATAACATGGAAGTAGGAAGATGAGAGAGAACTTTGG
AGCCATCCTTAATTGTATCCTATATAGTAGGTAGTAAATTCAAACCTAGCCAGGTATACATGAGACCCTATCCAAACAAGCAAACAA
ACAAAACATTTCCTGAGCAACGAAGTGAGAAGAGAGCCAGGGAAGAGATGTAGAGGAGCTTAGATCCTGCCTACGGGGAGGGCAGGG
AACAGACAGAATAGGAAAAGCCAGAGATGGAGAAGAGAGCTGGGCAGCATGGTATGTGGACCTGTAGCCTTGGACAGTCAGTCTCCA
GCACAGCCCAGGTGGCCGCATCATCATCATCATCATTATCTCCATCTCATTCCACTGGTTGGATGCAGGGCCTGACCCATGACC
CACCTGGTCTTCATAAGCTCTACAAGGGGCTGCCATGGAAGTTTGAGTCTTCAATTTATCTCTTTCCAGACTTGGGCTGGGGAATGT
AGGTGTTACTTGGCTGCAAAGCACCATGGATAGGCCTCCCACGACTTGGCCCTCACTTTCCCTGTTTCTTCCATTTCTGGCAGTCCA
TTGACCCTGGACAGCAGTTCACATGGGAGAATTCCAACTCGGGATGAACAAGCCCAAGAACCGCTATGCAAATGTCATTGCCCTATG
ACCATTCTCGAGTCCTCCTCACCTCCATTGATGGTGAGCCCGGGGCACAGTGCCCCCTTGCCCTTTGCTGTCCTGTACCCTGCTCTC
CAGAATGCTCTGAGCCCCAGGTTAACACTCTCGAATCCCTTTGCATTCCTGTAGGTGTTCCTGGGAGTGACTACATCAATGCCAACT
ACATTGATGGCTACCGAAAGCAGAATGCCTACATCGCCACACAGGGTCCGCTGCCCGAGACCATGGGCGATTTCTGGAGGATGGTGT
GGGAACAGCGCACAGCCACAGTGGTCATGATGACCAGGCTAGAGGAGAAATCCCGGGTGAGGGGGTGATGCTGGGTACCACCTCCT
GTGAGTGTTTTCAAGGGACTCCCCAAAGGCTGTGGTTTTCCTGGAGTATTGTCTCATCCCCTAGATAGAGGGGAACTGCTGGGATGT
AATGTAATGTGGTCCCCAGGTGTTTATGGGTTCCCGGGATGTTAATATGTTTTACCAAATGCTGTCGTTTTCTGGAATGTTAATATG
TTCCCCAGTTTCTGGGGTGTTCCTGGGGTATTCACATGTTCCCCAGATGCCAGAGTGCTCCTGGGGTGTTCACACATCTAAGTTAAT
TAGTGGAAGGAGCTGCTGATGTCTGCTTTTGCACCGCGAAGCATTGACTGGAACATAAAATTTACAGAGCTTGAAAATGGGGCATAA
ACGTTATGGGTAAATTTGGCAGAAAATGTGCCTGGTACGATCTGGTGTTGAATAAATAATTTTCAATTATGGTCCTCCTTCAGCCAG
TGAGGGCCAGGGTGGTAGCTTAGTGTGAGGAGGTTTGGAGAGGAAGAGGGAAGTGAGAATCGAGGGAGCTGGGGCCTTCCCCTTGCG
TCTGCCTGTTTGAGCCTTTGGGCATCCTATAACTTTGTCCCCTGTCCTGCCCATCCCCTCCAGGTGAAGTGTGATCAGTATTGGCC
AGTCCGTGGCACTGAGACCTATGGCCTCATTCAGGTGACCCTGGTGGACACTGTGGAGTTGGCCACATACACCATGCGCACCTTTGC
CCTCCATAAGGTATGTCCCTGAGTTCCCAAGCACTGGCCCATCCCTTAGGTTGTAGCCTGAGTGTGATGGGCTGGCGGAGGGCAGCT
GTGGGGTCAGGGCCTGGGAGGTGTATGAGGGGTTCTCACGGCTTGTGTTAGGTGACTGTCTGCTGTGGGCTTCTGAGTGTCACCTGAG
AGTGGCTGGTCTGGCCCCTTCCCTGGGTGTGTGGCAAGCAAACCTCAAACCTCATCTCCGTTTCCCAGAGTGGCTCCAGTG
AGAAGCGTGAGCTGCGTCAGTTCCAGTTCATGGCCTGGCCAGACCACGGCGTTCCTGAGTACCCCACTCCCATCTTGGCCTTCCTGA
GACGGGTCAAGGCCTGTAACCCACTAGATGCGGGGCCCATGGTGGTGCATTGCAGGTGAGAGTCCAGAGCCACCAGAGGGGAGGGGT
GGGAGGTGAGGGGACCCCTGCCCCAAGCTCTGCTCTTCTCCTCAGTGCGGGTGTGGGGCGCACAGGCTGCTTCATCGTCATCGACGC
AATGCTGGAGCGTATGAAGCACGAGAAGACGGTTGACATCTATGGCCACGTGACGTGCATGCGCTCACAAAGGAACTACATGGTGCA
GACCGAGGACCAGTATGTGTTCATCCACGAGGCCCTGCTCTAGCTACGGCTGCCATGTGCGGACACACCGAGGTGCTCGGCGGACCTCTA
TGCCCACATCCAGAAGCTAGGCCAAGTGCCTCCCGGGGAGAGCGTCACGGCCATGGAACTTGAGTTCAAGGTGGGCATCCAGAGGCC
TGACTTGGTCGGTCGTGGGAAGCCTGTTTGCAGCAGGCCAGGCCTGACAGAACCCTTGCTCTTCACAGTTGCTGGCCAACTCCAAGG
CCCACACGTCGCGCTTTGTCAGTGCCAACCTGCCCTGCAACAAGTTCAAGAACCGCCTAGTGAACATCATGCCCTATGAGCTGACCC
GAGTGTGCTTGCAACCCATCCGTGGTGTGGAGGGCTCAGACTACATCAATGCCAGCTTTCTAGATGGCTACAGGTCGGCATGCTTCT
CCCTGCTGCCCCACTGTGCCCCTTCTGGTACCTTGGTCTGCTTCTCGGTTGGGGAGTTAAGAGCTAATCCAAAAGATCAGATGAGT
TGGGGACAGAAGTGAGGGCTTCCTGGAGGAGGGCTGCTCTCTGAGGAACTAGACAATGTCAGGATAGGTGCGGGCAGCAGCACCTCC
ATCATGTGAAGTCTTATGTCACCCAGACAGCAGAAGGCCTACATAGCTACACAGGGGCCTCTGGCAGAGAGCACCGAGGACTTCTGG
CGCATGTTATGGGAGCACAATTCCACCATCATCGTCATGCTGACCAAGCTTCGGGAGATGGGCAGGGTAAGCCCACCCCTTCTCCTG
GGGCCCCTCTCATACCAGGGGGGACACCAGCTACCCTTGGGAAAGGGGAACCTCCTCCACCCTCTGCTCTCATGCCCCAGCTCCCCA
TCAGCATGGCCTACGACCTCTTTACCAACACCTGAGGCCACCCCTTCCCAAAGTCCTCTGAAAGGTGGCATTCTCCTTCTGGCCTCCCA
ACCTGATCTTGGGCGGTGCCTGGTGTTCTGTCCACAGGAGAAATGTCACCAGTACTGGCCAGCAGAGCGCTCCGTCGCTATCAGTA
CTTCGTTGTTGACCCGATGGCTGAGTACAACATGCCCCAGTATATTCTGCGTGAATTCAAAGTCACAGACGCCCGGGTGAGTGTCAG
CCAAAGAATATGCACACAGATGTGTCCCTCGAGCTACCCGGGCACACGTGGCTCCATCTGCCCCAAGACACTGCAGGGATCTGAGTC
GTGGGCGAGTTGTTTGCGTTACCTCCTAGAGTGTGTCCTTGAGAGTGCGTCCCTGGGTGGTTGTGCTCCTGGTTTAGAGGGCCTAGTAC
CTAACATCCAAACCTTCATTGTGACCCCTGACCCTCTCTCCCAGGATGGGCAGTCAAGGACAATCCGACAGTTCCAGTTTACAGACT
```

```
GGCCAGAGCAAGGAGTACCCAAAACAGGTGAAGGCTTCATCGGACTTCATCGGGCAGGTGCACAAGACAAAGGAGCAGTTTGGCCAGG
ATGGGCCCATCACGGTGCACTGCAGGTTGGGACGGCCCCCGTGGAGGGGATATGGGAGGGGGGACGGTGGTCCCGAAGGCCAGGGAAG
TTGATTGTGTGAAGGGAGCAGGACTCCTGACACAGCCATTCTCTGAGCAGTGCTGGTGTGGGCCGCACCGGTGTGTTCATCACCCTG
AGCATTGTCCTGGAGCGCATGCGGTATGAGGGTGTGGTTGACATGTTCCAGACCGTGAAGACCCTCCGCACACAGCGCCCTGCAATG
GTGCAGACAGAGGTATGCAGGCCAGAGGGAGGGGACCCACGTGTCTTTGCTACAACCCACAGCTGCACCTGCAGCTGGCCCACGTGTA
TTTGTTCTCCTTATAGCTCTGTGGTATTGCTTGTCCCACATGGCGTGCAGGGAAGAGGGAAGAGGGAGCTGTGTTCATTCCGTAGCCCC
TTCCTGAGCGCCAGTGTGTCATGTTGCCTTTGGGGTCAGAAGTAGACGTAGCAGTCTGTGTAAGACGGTGGGTGAGTCTGGGTACGA
ACAAGTGGTTTGAGTAGCAGAGGTGAACACAGGCCTGGGAGAGGCAAAAAGGAGAAATGGCCTTTCGAGACTTCAATTGGAGAGTAC
CTGGGGTAGTTCTCGGTGTTCAGCATCTGCTCTTAGCCAACAGAGGCCATTTCTGTGGCCACTGGCTGCAGCACATGCAAACCCTTGT
GTTTGGAATAGCTATTGGCTCTGTTCACACTGACCAGTCCCCCTCTTTCCTCAAAGGACCAATACCAGCTGTGCTACCGTGCGGCCC
TGGAATACCTCGGCAGCTTTGATCACTATGCAACGTAACTACTGCTCCCCTCTCCTTCCGACGGTCCCCCGCGGGGCTCCGGAGGGGA
CCCAGCTCCTCTGAGCCATACCAACCATCGTCCAGCCCTCCTGCACGGATGCTGTTGCCGGCAGAGCACAGCCCACTGGGATCACGA
CATTTCGGGGAACATTGCCACACCAGTCAGAGAGCCCAGAACACCTGGGCAAGTAGGCGGACTGGCAGCCTGGCTCTGGGCCCTCGT
CCACCGGGCCAAGTGGAGCCCCGCTTCAAGCTCTCTGTTCAGCTCCGCGTTCTCATGCTTCTCATGGGGTGGGAAAAGGGGGCAAAG
CCCCCACTTTTTATACACTAGGCGGGGTAGACTGCGGGGTCCTAGCCTCTTCCTCCGACTTTGCTTTTGCAGGTCTTTCACTGCAGA
TGGGGCTGCTGTGGGAGTTGGGACTTGTTTGTTTTCGTTTTTGACGTTGGATCCTTTCTTGTACAACTTCTGCGGAAGGACA
CAGTAGTAACTCGCCTTCCTTGTGCAGAGCTAGGGCCCTACCTGAGCAAGTCGGCTGTGGCCCGGGAGGCAGCGTGACTCCTGCTGT
CCTCCAGCCTTTCAGATGAGATCCTATTCCAGCCAAATGCAGGGAAACACTTTATTTTGTTTGTTTTAGGTTTTGTTTTTCCTTGAG
AGCCTTTTTTTAGGCCCCACAGACAGTGGTGGGTGGGGAGGCGATAGGAAACACATTCCCCAGTGTGCATTTAACATTCATAGCCTA
CAACCACAGACGTGTCTGGGGGAGCCTGGCAAGGCGTTCCTCGTCACCATCGTGTTTGCAAAGGTTCAAAAAACAAAAATCAAAAAA
AAAAAAAACATAAAAATATTTTTTTTTAAGAAAAGAAATAAAGATTCATCCCCTGGCCTCTACTTCAGATTCGAAGTGGGAGGCAA
CTCAATGTGCCTGGGGCTCGCCATGGGACCCACAGAGTCTCGCTATCATCCCCAGCGTCGCAGTGTGGCAGGTGTTTGTAGGCTGTGG
GTTCTGGCCACCTCGGGAAATGAATGGCACAGGTGAGGGCCTGTGCCACGCCCCACACACCTCAGGGCCAAGCGGGGGCGTGGCTGG
CCCTTCAGGTCAGGCCAGTGGGCCTGGTAGCACATGTCTGTCCTCAGCCGGACAGATGCTTTCTCTCCTGGTTTGCAGCTGTCTTCA
AATCCCCCCATGACCCGCTCTTCCCACTCCTTCAAGTTGCCCTCACAAACCAATGTGGCAAGACTACTGGACTTGTATCCATGGTAC
TATACTCAGTCCTCTTATCTCAGCTTGCTGAGGGCAGGGAGAGGGTCTCTTCCTCTGGGCAGCATCATCTAGATAGGTAAGTGGGG
GCGGGGAAGGGTGCATAGCTGTTTTAGCCGAGGGACTCGATACCGACGTCCCAGATATAGGCTAGGCTAAGTCAAGATCAACAGTCC
GGGGTTGGTGGTGTTGGATGAAACATTCATTTTTACCTTGTGGATGCTAGTGCTGTAGAGTTCACTGTTGTACACAGTCTGTTTTCT
ATTTGTTAAGAAAAACTACAGCATCATTGCATACTTCTTGATGGTAATAAATTTGAATAATCAAGATTTCTTACACACTAGGCCTCT
GTCTCAGCTCCTGTATCTAGAGTGTGGTATCTTAAGTCTATGGCTGGGGTGGGTTCTGAGGAGTC
```

MOUSE mRNA SEQUENCE : mR28-021.1 (Seq ID No: 64)
```
AGGAAGGAGCGGAGGCCCTGGTGCCCTGCCCTCAGTGCTGACTGCCCAGCGAGAGTGACTGGTATAAGCAAGCAGAGACCCGGTGGA
TTGTCTTAGGCTGTGGCTGCTGTGTGGAGCTAGAACCCTGGATGGCTCCCGAGCCAGCCCCAGGGAGGAGGATGGTGCCCCTTGTGCC
TGCTCTCGTGATGCTTGGTTTGATGGCAGGTGCTCATGGCGACAGCAAACCCGTCTTTGTTAAGGTCCCCGAGGACCAGACTGGGCT
GTCGGGAGGGGTGGCCTCCTTCGTGTGCCAAGCCACAGGGAAACCCAAGCCTCGAATCACGTGGATGAAGAAGGGGAAGAAAGTCAG
CTCCCAGCGCTTTGAGGTAATTGAGTTTGACGATGGCAGGGTCAGTGCTGCGGATCCAGCCATTACGAGTGCAGCGAGACGAAGC
CATCTATGAGTGCACAGCCACGAACAGTCTCGGGGAGATCAACACAAGTGCCAAGCTGTCAGTGCTTGAAGAGGACCAGCTGCCGTC
TGGGTTCCCGACTATCGACATGGGACCTCAGCTGAAGGTGGTGGAGAAGGGTCGCACTGCCACCATGCTGTGTGCAGCCGGTGGGAA
CCCAGACCCTGAGATCTCTTGGTTCAAAGACTTCCTTCCTGTGGACCCTGCTGCAAGCAACGGTCGTATCAAACAGCTGCGATCAGG
TGCATTGCAGATAGAGAGCAGCAGCGAGGAGTCTGACCAAGGCAAGTACGAGTGTGTGGCCACCAACTCTGCAGGCACACGCTACTCGGC
CCCCGCCAACCTGTATGTGCGAGTGCGTCGCGTGGCCTCCTCGTTTCTCCATCCCTCCCAACCAACCAAGAGGTGATGCCGGGCGGCA
CGTGAATCTCACATGTGTGTGGCAGTGGGCCGCGCCCATGCCGTATGTGAAATGGATGATGGGCGCCGAAGGGAACTGACCAAAGAGGATGA
GATGCCAGTTGGCCGAAATGTTCTGGAGCTCAGCAATGTCATGCGATCTGCCAACTATACCTGTGTGGCCATCTCTTCATTAGGCAT
GATAGAAGCCACGGCCCAGGTCACAGTAAAAGCTCTGCCAAAGCCTCCAATTGATCTTGTGGTGACAGAGACAACCGCACCAGTGT
TACTCTGACATGGGACTCTGGAAATACCGAGCCTGTGTCCTTCTACGGCATCCAGTACCGCGCAGCTGGCACAGACGGCCCCTTCCA
GGAAGTGGGATGGTGTGGCCCAGCACCCGATACAGCATTGGTGGCCTCAGCCCCTTTTCCAGGAATATGCTTTCCGTGTGTTGGCTGTTAA
CAGCATTGGGCGAGGACCCACCCAGTGAGGCAGTGCGAGCACGCACGGGGGAGCAGGCACCCTCCAGCCTCCGCGCCGTGTGCAGGC
TCGAATGCTTAGCGCCAGCACCATGCTGGTCCAGTGGGAACCACCTGAAGAGCCCAATGGCCTGGTACGGGGATATGCGCGTCTACTA
TACCCCAGATTCCCGACGCCCTTTGAGCGCCTGGCACAAGCATAACACTGACGCAGGACTCCTTACCACCGTGGGCAGCCTGCTACC
TGGCATCACCTACAGCCTCCGAGTCCTTGCCTTCACTGCTGTGGGTGACGGCCCACCCAGCCCCACCATCCAAGTCAAGACACAGCA
GGGAGTGCCTGCACAGCCTGCGGACTTCCAGGCTAACGCTGAGTCGGCACCAGGATCCAGCTCTCGTGGCTGCTGCCCCCGCAGGA
GCGGATCGTCAAGTATGAGCTGGTGTACTGGGCAGCCGAGGATGAGGGCCAGCAGCCCCATCAGCCCCTCCCCAGAAGGTGACATGT
GTGAGCACGGGCTCCACCACGGTCCGGGTAAGTTGGGGTTCCACCGCCGGCCGACAGCCGCAACGGCATTATCACCCAGTACTCCGTG
GCCTATGAGGCAGTGGACGGCGAAGACCGTAAGCGACATGTGGTGGATGGCATCAGCCGTGAGCATTCCAGCTGGGACCTGCTGGGC
ACCGATGAGGACGTGCCCTAGCGGGCCACCACGGAAGGTAGGGTTGCAGCCTCTGAACTCCACTGCTGTGCATGTCTCCTGGAAGCTG
CCCGTCCCCAACAAGCAGCACGGACAGATTCGTGGCTACCAGGTCACCTATGTGCGGTTGGAGAATGGTGAGCCCCGAGGCCAACCC
ATCATCCAGGATGTCATGCTGGCTGAGGCTCAGGAGACCACCATCAGCGGCCTCACCCCCGAGACCACCTACTCCATCACCGTCGCT
GCCTACACCACTAAGGGGGATGGTGCCCGAAGCAAGCCCAAGGTGGTTACTACAACAGGGGCAGGACGGCCCACCATGATGGTCAGC
ACCACGGCCATGCACACCGCGCTGCTGCAGTGGCACCCACCCAAGGAGCTGCCTGGAGAGCTGCTGGGCTACCGTCTCCAATACCGG
CGGGCCGACGAGGCGCGGCCCAACACCATAGACTTTGGCAAGGATGACCAACATTTTACAGTCACCGGCCTGCACAAAGGGGCCACC
TACGTCTTCCGGCTCGCTGCCAAGAACCGGGCTGGCCCAGGAGAAGAGTTTGAGAAAGAGATCACCACCCCCGAGGATGTGCCCAGT
GGCTTTCCTCAAAATCTTCGAGTGACAGGACTGACCCACATCTACCACGGAACTGACCTGGGACCCGCCGGTGCTGGCGGAGAGGAAT
GGTCACATCACCAACTATACTGTGGTCTACCGTGACATCAACAGCCAGCTTGAACTACAGAATGTCACCAACGACACACACCTTACA
CTCTTGGGCCTTAAACCGGACACCACTTATGACATCAAGGTCCGTGCACATACCAGCAAAGGCGCCGGCCCTCTCAGCCCCAGCATC
CAGTCCCGGACCATGCCCGTGGGACAAGTGTTTGCCAAGAAATTTCCGTGTGGCTGCGATGAAAGACATCTGTGCTGCTCAGTTGG
GAGGTCCCCGACTCTTATAAGTCAGCTGTGCCCTTCAAGATCCTGTACAATGGGCAGAGCGTGGAGGTGGATGGGCACTCGATGCGG
AAGCTGATTGCAGACCTGCAACCCAACACGGAGTACTCCTTCGTCCTGATGAATCGTGGCAGTAGCGCCGGGGGCCTACAGCACCTG
GTGTCCATCCGCACTGCCCCGGACCTCCTACCCCAGAAGCCACTGCCTGCCTCCGCCTTTATAGAGGATGGCCGCTTCTCCCTCTCC
ATGCCTCAAGTGCAGGACCCCTCGCTAGTCAGGTGGTTCTACATTGTGGTGGTGCCCATTGACCGTGTGGGCGGGAACTTGCTGGCA
CCAAGATGGAACACACCAGAGGAGTTGGAGCTGGACGGGCTTCTGGAGGCATCGAGCACGGGCGAGGAGAAACAGCGGAGGCGCCTGG
CGCCAAGCAGAGCGGCTGAAGCCTTATGTGGCGGCCCAAGTGGATGCGCTCCCTGACACCTTCACCCTGGGGGACAAGAAGAGCTAC
CGCGGCTTCTACAACCGGCCCCTGTCTCCGGATCTGAGTTACCAGTGCTTCGTTCTCGCCTCCCTCAAGGAACCCATGGACCAGAAG
CGCTACGCCTCCAGCCCCTACTCGGACGAGATTGTAGTCCAGGTGACGCCAGCACAGCAGCAGGAGGAGCCCGAGATGCTGTGGGTG
ACAGGCCCTGTCCTGGCGGTCATTCTCATCATACTCATTGTCATCGCCATCCTCCTGTTCAAGAGGAAGAGAACACACTCCCCCATCA
```

```
TCAAAGGATGAGCAGTCAATCGGGCTGAAGGACTCCCTGTTGGCCCACTCTTCTGACCCTGTGGAGATGCGAAGGCTTAACTACCAG
ACCCCAGGTATGCGAGACCACCCGCCCATCCCCATCACTGACCTGGCAGACAATATTGAGCGCCTCAAAGCCAACGATGGGCTCAAG
TTCTCCCAGGAGTATGAGTCCATTGACCCTGGACAGCAGTTCACATGGGAGAATTCCAACTCGGAGGTGAACAAGCCCAAGAACCGC
TATGCAAATGTCATTGCCTATGACCATTCTCGAGTCCTCCTCACCTCCATTGATGGTGTTCCTGGGAGTGACTACATCAATGCCAAC
TACATTGATGGCTACCGAAAGCAGAATGCCTACATCGCCACACAGGGTCCGCTGCCCGAGACCATGGGCGATTTCTGGAGGATGGTG
TGGGAACAGCGCACAGCCACAGTGGTCATGATGACCAGGCTAGAGGAGAAATCCCGGGTGAAGTGTGATCAGTATTGGCCAGTCCGT
GGCACTGAGACCTATGGCCTCATTCAGGTGACCCTGGTGGCACACTGTGGAGTTGGCCACATACACCATGCGCACCTTTGCCCTCCAT
AAGAGTGGCTCCAGTGAGAAGCGTGAGCTGCGTCAGTTCCAGTTCATGGCCTGGCCAGACCACGGCGTTCCTGAGTACCCCACTCCC
ATCTTGGCCTTCCTGAGACGGGTCAAGGCCTGTAACCCACTAGATGCGGGGCCCATGGTGGTGCATTGCAGTGCGGGTGTGGGGCGC
ACAGGCTGCTTCATCGTCATCGACGCAATGCTGGAGCGTATGAAGCACGAGAAGACGGTTGACATCTATGGCCACGTGACGTGCATG
CGCTCACAAAAGGAACTACATGGTGCAGACCGAGGACCAGTATGTGTTCATCCACGAGGCCCTGCTAGAGGCTGCCATGTGCGGACAC
ACCGAGGTGCTCGCTCGGAACCTCTATGCCCACATCCAGAAGCTAGGCCAAGTGCCTCCCGGGGAGAGCGTCACGGCCATGGAACTT
GAGTTCAAGTTGCTGGCCAACTCCAAGGCCCACACGTCGCGCTTTGTCAGTGCCAACCTGCCCTGCAACAAGTTCAAGAACCGCCTA
GTGAACATCATGCCCTATGAGCTGACCCGAGTGTGCTTGCAACCCATCCGTGGTGTGGAGGGCTCAGACTACATCAATGCCAGCTTT
CTAGATGGCTACAGACAGCAGAAGGCCTACATAGCTACACAGGAGCCCTCTGGCAGAGAGCACCGAGGACTTCTGGCGCATGTTATGG
GAGCACAATTCCACCATCATCGTCATGCTGACCAAGCTTCGGGGAGATGGGCAGGGAGAAATGTCACCAGTACTGGCAGCAGAGCGC
TCCGCTCGCTATCAGTACTTCGTTGTTGACCCGATGGCTGAGTACAACATGCCCCAGTATATTCTGCGTGAATTCAAAGTCACAGAC
GCCCGGGATGGGCAGTCAAGGACAATCCGACAGTTCCAGTTTACAGACTGGCCAGAGCAAGGAGTACCCAAAACAGGTGAAGGCTTC
ATCGACTTCATCGGGCAGGTGCACAAGACAAAGGAGCAGTTTGGCCAGGATGGGCCCATCACGGTGCACTGCAGTGCTGGTGTGGGC
CGCACCGGTGTGTTCATCACCCTGAGCATTGTCCTGGAGCGCATGCGGTATGAGGGTGTGGTTGACATGTTCCAGACCGTGAAGACC
CTCCGCACACAGCGCCCTGCAATGGTGCAGACAGAGGACCAATACCAGCTGTGCTACCGTGCGGCCCTGGAATACCTCGGCAGCTTT
GATCACTATGCAACGTAACTACTGCTCCCCTCTCCTCCGACGCTCCCCCGCGGGGCTCCGGAGGGGACCCAGCTCCTCTGAGCCATA
CCAACCATCGTCCAGCCCTCCTGCACGGATGCTGTTGCCGGCAGAGCACAGCCCACTGGGATCACAGCATTTCGGGGAACATTGCCA
CACCAGTCAGAGAGCCCAGAACACCTGGGCAAGTAGGCGGACTGGCAGCCTGGCTCTGGGCCCTCGTCCACCGGGCCAAGTGGAGCC
CCGCTTCAAGCTCTCTGTTCAGCTCCCGCGTTCTCATGCTTTCTCATGGGTGGGAAAAGGGGGCAAAGCCCCCACTTTTTATACACTA
GGCGGGGTAGACTGCGGGGTCCTAGCCTCTTCCTCCGACTTTGCTTTTGCAGGTCTTTCACTGCAGATGGGGCTGCTGTGGGAGTTG
GGACTTGTTTGTTTTCCTTTTTGAGTTCACGTTGGATCCTTTCTTGTACAACTTCTGCGGAAGGACACAGTAGTAACTCGCCTTCCT
TGTGCAGAGCTAGGGCCCTACCTGAGCAAGTCGGCTGTGGCCCGGGAGGCAGCGTGACTCCTGCTGTCCTCCAGCCTTTCAGATGAG
ATCCTATTCCAGCCAAATGCAGGGAAACACTTTATTTTGTTTGTTTTAGGTTTTGTTTTTTCCTTGAGAGCCTTTTTTTAGGCCCCAC
AGACAGTGGTGGGTGGGGAGGCGATAGGAAACACATTCCCCAGTGTGCATTTAACATTCATAGCCTACAACCACAGACGTGTCTGGG
GGAGCCTGGCAAGGCGTTCCTCGTCACCATCGTGTTTGCAAAGGTTCAAAAAACAAAAATCAAAAAAAAAAAAAAAACATAAAAATAT
TTTTTTTTAAGAAAAGAAATAAAGATTCATCCCCTGGCCTCTACTTCAGATTCGAAGTGGGAGGCAACTCAATGTGCCTGGGGCTCG
CCATGGGCCCACAGAGTCTCGCTATCATCCCCAGCGTCGCAGTGTGGCAGGTGTTTGTAGGCTGTGGGTTCTGGCCACCTCGGGAAA
TGAATGGCACAGGTGAGGGCCTGTGCCACGCCCCACACACCTCAGGGCCAAGCGGGGGCGTGGCTGGCCCTTCAGGTCAGGCCAGTG
GGCCTGGTGACACATGTCTGTCCTCAGCCGGACAGATGCTTTCTCTCCTGGTTTGCAGCTGTCTTCAAATCCCCCCATGACCCGCTC
TTCCCACTCCTTCAAGTTGCCCTCACAAACCAATGTGGCAAGACTACTGGACTTGTATCCATGGTACTATACTCAGTCCTCTTATCT
CAGCTTGCTGAGGGGCAGGAGAGGGTCTCTTCCTCTGGGCAGCACTATCTAGATAGGTAAGTGGGGGCGGGGAAGGGTGCATAGCT
GTTTTAGCCGAGGGACTCGATACCGACGTCCCAGATATAGCTAGGCTAAGTCAAGATCAACAGTCCGGGGTTGGTGGTGTTGGATG
AAACATTCATTTTTACCTTGTGGATGCTAGTGCTGTAGAGTTCACTGTTGTACACAGTCTGTTTTCTATTTGTTAAGAAAAACTACA
GCATCATTGCATACTTCTTGATGGTAATAAATTTGAATAATCAAGATTTCTTACACACTAGGCCTCTGTCTCAGCTCCTGTATCTAG
AGTGTGGTATCTTAAGTCTATGGCTGGGGTGGGTTCTGAGGAGTC
```

MOUSE PROTEIN SEQUENCE : mP28-021.1 (Seq ID No: 65)
MAPEPAPGRRMVPLVPALVMLGLMAGAHGDSKPVFVKVPEDQTGLSGGVASFVCQATGEPKPRITWMKKGKKVSSQRFEVIEFDDGA
GSVLRIQPLRVQRDEAIYECTATNSLGEINTSAKLSVLEEDQLPSGFPTIDMGPQLKVVEKGRTATMLCAAGGNPDPEISWFKDFLP
VDPAASNGRIKQLRSGALQIESSEESDQGKYECVATNSAGTRYSAPANLYVRVRRVAPRFSIPPSNQEVMPGGSVNLTCVAVGAPMP
YVKWMMGAEELTKEDEMPVGRNVLELSNVMRSANYTCVAISSLGMIEATAQVTVKALPKPPIDLVVTETTATSVTLTWDSGNTEPVS
FYGIQYRAAGTDGPFQEVDGVASTRYSIGGLSPFSEYAFRVLAVNSIGRGPPSEAVRARTGEQAPSSPPRRVQARMLSASTMLVQWE
PPEEPNGLVRGYRVYYTPDSRRPLSAWHKHNTDAGLLTTVGSLLPGITYSLRVLAFTAVGDGPPSPTIQVKTQQGVPAQPADFQANA
ESDTRIQLSWLLPPQERIVKYELVYWAAEDEGQQPHQPLPRR*

MOUSE PANTHER CLASSIFICATIONS
        FAMILY (SUBFAMILY)
                Unclassified

MOUSE GENE ONTOLOGY
        BIOLOGICAL PROCESS
                protein modification > protein dephosphorylation
                enzyme linked receptor protein signaling pathway > transmembrane
receptor protein tyrosine phosphatase signaling pathway
                isoprenoid catabolism > phosphate metabolism
                axon guidance > motor axon guidance
                defasciculation of neuron > defasciculation of motor neuron
        MOLECULAR FUNCTION
                protein tyrosine phosphatase > prenylated protein tyrosine phosphatase
                enzyme > protein phosphatase
                protein phosphatase > protein tyrosine phosphatase
                protein tyrosine phosphatase > transmembrane receptor protein tyrosine
phosphatase
                transmembrane receptor > transmembrane receptor protein tyrosine
phosphatase
                protein tyrosine phosphatase > non-membrane spanning protein tyrosine
phosphatase
        CELL COMPONENT
                plasma membrane > integral plasma membrane protein
                cell > plasma membrane

```
                    cell > membrane fraction
                    cell > soluble fraction
                    cytoplasm > cytoskeleton
MOUSE PROTEIN DOMAINS (INTERPRO SIGNATURES)
             IPR001777 (FNTYPEIII)
             IPR001777 (FN3)
             IPR003598 (IGc2)
             IPR003599 (IG)
             IPR001777 (fn3)
             IPR003006 (ig)


HUMAN GENOMIC SEQUENCE : hD28-021 (Seq ID No: 66)
GCGTGTGTGGACTGAAACCCAAAAGCCAGAAACTCTCTGCATGTGTCCCTCATGGAGCCCATATGGTCTCTAGTCTCTGTTTTTCAAA
ATTTGCAGGACTGATGACTGATTGGACAGTGGTGCAGGAGGGGAAGGTCTCTGCATGTCAGTGATTAAATTGCTGAAGATGCCACTGC
CTGAGACGGGCAGTATTGAAGGTAAGCAGATCTGGGGTGGGCGGGACAGAGGAGGCTCTTAGTTATATGGATCTGGAGCTCACAGGA
GCGGCCCAGACTGGAGATAGAGACTTGGAAGTCATCAGCATATGGTTGGTAGTGGTCAAGATGATCCAGAGAAAAGGGGCAGAGCAG
GAGAAAGAGGAGGCCTAGGACAGAGTCCCGGAAGAGCAGAAGACGAGGTGGTAAAGGAATCAGAGACAGTAGCCAGTGAGGAGAGG
GTGGGCCAATGAAGCCAAGGAAAAGAACTGTTTTAATAACTCAGCAATGGGCAGGAATAATTGGCTGAAAAGTATACTTTTAAAATT
TGGGGGCCGAGAGGGAATCTCTAAATCCTCAAAGTTCTGGAAAAAAAGGCAAAAATGCTTAAAGATGCATAAAATTTGCAATACCTC
CCCCGTGGTGCACCAAAATTACCAATGGTGAAAAACCACTCACTGGAAATATTCATTTTTGATCCGTTTGTCATCCAAACCAAATTG
TCCAGAATCACTGGAGCCAGTCCCATCCCCTCCTCATACGCCAGGGGGCGCACACGCACTGTTTGTGAATTCAAGTTCAGGACAAAA
ACAGGCAGTGAATCAGGTGGTTACCAGAACCTGATTCCTCAGCCCAGATCCCTGAACACCACAGCAACCATGCCCTGCACATTACCA
CGTGATCCCTGAATCCAGGCACCTCAACGATCTCCAAATTTTATTTCTCACCTGACTCCAAACTTACAGGGTTATCAGAAAGACCCC
ACAGAAGAAACCCCACAGAGGAGAAACCTCATCTCTTTTTTCTGCTCAGTTACCTGACCCAGGGCAGTGGGGGTAGGTCCCCCATCCT
TCATGGTGAAGCCTAGCAAGCGGCGGGGGGAGGAGGGTGGGGTAGATAGGAAAGAGCTGGTGTGGCTTTGGCTGACTCTTAAGTGG
TTTTAACACGGATTGCGTGGGGGCGGGGTCTGGAGGCAGGGGCGCTTGGATTGAAGCTCATCAAATCCCTCTGATCAGTAGGTCTTG
GAATAAATCTTCAGGTAGGTTATTGGTTTCACCCAGAAATTTCTAGGGTCATTATTTGAGTTTGGTATGAGAAGTCAGAAACTCAGT
ACTGACATTTAAATTACATTTAGAGGGCATTATCATGTCTTTAATTCCGAGGCCAATGTAAGGTTCACAGCTGGAATTGGTATAATA
TTGGGGTATTAGTCAGTCTCTGCTTGGGGGTTGACACTAGGTCAGAGAAGTGGAGGGTGAACATGAGGATGAAGGGGCTTTGTGTGGA
GTTTCTGCAAAGCCAGTCTTTATCTTGGGTTCCAAGATTATTATTATTAAAGCTGGGTTGGTTTAGGGTCCTAAATGGTCTCAAGTTTGGG
TGGGGTTTCAGGTCAGTATCTGTGTTTGGGGGTCCTTTTATGGTGACCCAATCCCAACTCTGGAATTGAGAATCGAATTCAGAGTGC
ATTGGTGTTGATCTGGGAATGGGAGAGCAGTACTGGAGTTTTATGTAAGGCTTGGGGTTCAGAAGAGAGATAACTGGGGTCCATAGC
AAGGTAGCTTTTGGACTTTAGGGTTCATTTAGGGACTGGGGGCTGAGCATTAGTACAGGGTTCCATTTAAGGTCCAGCGTTGGTGCT
AGCGTCACTATCAAGGTTAGGGCAAGTAGAAGTAATAAAGAGATTTAGAGTCAGAATTGGTATTTGGGATCAGTATAGAGGTTCACA
ACAAAATTACTACTTGTCTAATTTGGGGGCAGATTAAGGGCTTTGGACTCAATATTGGGTTTAGGGCAAGGTGTGGGCTCAGTATC
AGGTTCAGATCTGTATGGGGAGGAAATGGGGTCCTAGAGTGGTTATTAGGTTCATGCTAGGGCTGGGAGCCACTAGAAGGATCTGGAT
GTAGTATCAGAGTTTGGTCTGTCTATGTTCATTATTGGGGTTCGGGTTCAATATTGAGGTTCCGGTCGGTTTCGACTTGGCGTTGGG
TTGTGGCCTGTCTGGGCCTCAGCGGCTCCGCGGCTCTACGAGCGAGAGTGCACGAGGGGAGGGGCGCCGCCGGGGGCGCGCACGGCA
GGGGCAGGGGCGCGGGCGCGAGCGCGAGGGGGAGCGCGCGGCTGGAGCTGGCGCGGGAGCGGCGGGAGCGGTGGCGGCGGCAGAGGCG
GCGGCTCCAGCTTCGGCTCCGGCTCGGGCTCGGGCTCCGGCTCCGGCTCCGGCTCCGGCTCCAGCTCGGGTGGCGGTGGCGGGAGCG
GGACCAGGTGGAGGCGGCGGCGGCAGAGGAGTGGGGAGCAGCGGCCCTAGCGGCTTGCGGGGGGACATGCGGACGCGACGGCCCTGGA
TAGGCGGTGAGTGACCCCCCGGCCCCCCACCAGCCCCCTCCGCTCCCGTCCCTTCCCGCTCTCCTTGCCCTCCCCGCTAGTCCACC
CGGCGGAGCTGGGGGCGGTCCCGGGGGGGACGAGGGCGCCGCTGAGGCCGGGCCGCGCCCCCACCTGCCGCGCCCGCAGCCTCGGCG
AAGNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNNNNNNNNNNN
NNNNNNNNNNNNNNNNNNNNNNNNNNNNNNNNNNNNNNNNNNNNNNNNNNNNNNNNNNNNNNNNNNNNNNNNNNNNNNNCCCTCCCAG
CCGGCGGGGGATCCGCAGCCGGCGCGCCCGGGTCTCGCTCGCGGCCCCGGCGCACGTGCGGTCCCGGGCTGCGCCGGGCGCGAGTTT
GAAGCCCCCCCACCCCCTTCGTTCGCGAGCACGGGATGGGGAGGGGCCGAACGGGCTGTACCCGCCCCCAACCGGGTGCAACGCCCT
GGAGGCCCTGGAGCGACCTGTCCCGTGAGGACGTGGCCACTCGGACCCTCTCCGAAAGTTCACTCAGTGGCCGCCCCGCGTCCCGTC
CCGTCCCGTCCCGGCTCCCCATCGCCGTGCCGGCGTCTCTGTCTCGGCTGCTTTCTGTTTTCCTCGGCGTCTCTGCCTCCCCGTGTC
AGTGCCTCCCAATCTCACGCCCCTGCAATCCCAGGGTCTCTCCAGCTGTCTCCAGCTGTCCATTCTTTTTCCCCAGGTCACTCTGTTCTTTCTCC
CCAGGTCTCTCGATTCTGTCTCCCTGGGTCGTCTTGTTCCCTGTCCCTGAGTCTGTTCTCTCCGCTTGGGTCTCTCTATTTCCTCTC
CCTGGGTCTCTGTCTCCCTCCTTAAGTCTCTGCCTCTTGTCTCTCACCCATCTCTTGGTGTCTCTGCACTCTCTGTGCTAGGCGCCC
CCCCATTTCCCTGGAATGCTGCCCCTCTGGTTCTTCCACCCCGGAGGGGGAGGGGCTAGACTTTCTCCCCATTTCAAGCCCCCCTGC
CCCCCAGTGCACGGCCCCTGAGTTGGAGCAGGTTGGGGGTGGGGAGCGCTACCTGCCTGAGCTGTGAAATGGGAGAGGGAGGCTGCC
CCAGCCTGGGGGTTGTCTGGATCTCCCTGAGGGGCCACAGGGCAGGGCCGGGAGGGCTGGATTAGGTTGAGGAAGGCTCCACTTTTGA
AGGAACAGGGGCAGGGATCCAGAGCGATCTGGTGTCGGATCTGGCCTGGCAGGAAGCAGGGAAGTGCACTGGGGTGAGAGGCGTAGAAAA
GGATGTGGGGTGGTGGTTCCAGGCGGGTGCTCATGGGGAGGAATCTGCTGAGAAGGGAAGATCTGGTGGGGAATCCTTTGGGAGGCT
TCGGGGATCTGAGGGATCCAGAAGAGAGTTCTGACAGGCTATCTAAGGAAATTTCTGTGCTTAGCTCCTTATTAACTCTTGCTTCAG
GGAGCATGAAAATCCCTGTTTTTTTACTGATTATATGAATGAGCCTAGGGCTTCAAGAAGGAGCAGGTGTAACTCCCCAAGTACAACC
CCCTACATTCTCAGCCCCCAGGCACAGGCCACACTTTCTCGTACCTTCTTGCTCCTTCATTTCCACACTCAGCTCACCCCCTCCACCCCC
ACACACACTCCCTATTCAGCTGCTTTCTTGCCCCCTCTCGCATACCTGGGCCCGCATGCCGGCCAGGGCTCAAGAAATCTGTGCCTG
CACACTCTCGCAGCATACCTGTGCACATGCACAGTCATCCTTGTGTATTTTTGTATATGCACTTTCTTGCACATTTTACTTGCTCAG
TCTCTTATTTGAAATGTCTTTATAGTGTTTTTTTTTAGTTTCCTTTATAGTTTCCTTTTCCAGCTGCTCTTCCTCTTCCCTCTTTCTT
TCCTTTAACAAGAAAATTGTTTTAACATAATTCCTTCCTTCCTTCCTCCCTTTCTTCCTTCTTCTTGCAACTACTGATACCTCCCCT
TTATCCAGCTTTGTGTGCCCTGGGCGGGTTGGCTTGGTGTACAAGGGCTGCCAAGAGAGGGAGGGGGTGGTTGGGTGTTGGTGAAGGAA
TTTGGCGGGGCAGAGTTAGACTGCAGCAAACGGAAACTAGCTACACATCTTTTACTGGGAAGTGTATGGATACGCAGCAAAATACTA
GCCCTGGCAGTTTGGGCACAGTCCACCCTTTTTTCCATCAGCCTGAACTATGGGAGTCTAGCTTTGAGGGTCCTCCCTGGGGGCGGG
GAGGAGGGGACCCAGACCTCCTGCAGTAGAGGGGTTGTGATACACACCACGGCTCCTCCACTTTCCAGCTTCAGGCCTCAAAGAGGC
CCCTTTCTCCCTGAGTCTCAATTTCACTCCCTGTAAAATGGGGTAGCTAATCCAACCCGGACTGGGTCCCAGGGCTGTTGTAAGAAT
CCAAGGGAACTGTGTGGCAGTGCATCGTCAGCAGTGGCAGTCACTTCCCTTGTGACTGACACTGCTGTGTGCTTGGTTCCAGCTTGC
AGTCAGGGCCCTACCCTCGAGAAGCTCGTGGTCCTTGGGGGAGCAGGTTGCACCATGGGATTGCAAAAGCAATTCAGTTACCTGTCA
TTGATTGCTGACTGTGTGCCAGACCCCGGGGTAGGCGCTTTCGGCCCCGGCTGCCTTTCTTCCTTTGGGACTAACAGCTATGTAGAA
GAGGCAGCCCACCCCTAAGGTTCAGGCATCTGCTCAACTAAAGCAGCTGCGGTTCTGAGTTGGGTTAGAACATTGCAAGAGTTCATC
AAGAGACGGGGGAGAGTTCGGAGGTCAGGGTGAGGTGAGTCCCAGAGGAGACCAAGAAGAAGTGATGTGAACTAGGTTTGGAGGGAT
GAGTAGGAGTTTGAAGAAGAGAGAAAGGACTTGAGATAGATAAAAACCAGTATGAATAAAGACTGGGAGGTGTTGTCACTTGTGAAG
```

228

```
TATTCAGAGATGTGGCAGATCACAGAGGGCATTGAATGCCCTGTTAAGTACCTGTAATTTTACTCTGAGAACCCAGAGAGAAAACAC
CAAAGGTTTAAAGCTTGGATAGGGAGGGTGGAGTGTGCTGTGGTCAGGTTTATGTTTCCAGAAAATGCTCTGTGGCTATGGGAAAAG
GATGGAGACAGGATGCCCAGTGGGGCAAGAGGGTGCAGGTAAGTAAGGTGAGAGAAGATGCTGGCTTTGATAGGTGGTAGCATGGTG
CCATCTTGAAGGTACACTTGTCAGGACAGAATGATTTATTGAGTACTGTGGGAGTGAAGGAGAGGAAGAGGAAGGAGTCCAGGTTCCTCG
GAGTGGTTGAGCGGGCCTTCACTGAAGGCCCTGAAGCTTCACTGTGTTAGGGACTTGGTGAGGAGGGGCAGAGAATAAGCTCTTAGT
TGGACATGTGGCCCTTGAGGTACTTGGGACATTGTGGAGGCTCAGTGGGGGGAGGCACATCAAGGAGGGGCTTTGGTGTGAGGGTATGGG
AGAGAAATGTCGGTCGGACCAAGAGAAGCGAGAGCTGTATGAGAGACCATAGAGCTGTGCACCTCGAGAGAAAGTAACCTCCGACAG
TGGTGAAGAGCTCGAGTTTTATACTGCTAGGATTGGACTCTGTTTACTAGCAGCGTGACTTTAAGCAAGTGGCTTAACCTCTCTGAA
TCCGCTTCCTCATTTGTCAAATGATTATAATAAAAACCCGTGCCTTATAAGGGTTGTTATGAGGATTAAAGATAGTATGCATAAAGT
GTGTGTAGCACAGGGCTTGGCATCTAGTGAGTACTGTGTCTGTGTTTGCAGTTACTGTAATCATCCTCAACTGGGATGGTGTAAAC
CCACCCTCCCATCAGGGGTCACATGAGGTTGCCTTTAATGAGAAGATAGACTGCTAAGGTTAGGGTCCCTGTCTGCCTAAGGTTGCT
GCAATTCTGAGCTGAGATTAGCAGGTCAAGGAGCTCAACAAAGGGGGTGCCTGCCCCTATTGTTATCGATGGGATTGTTTGTGTTGC
TCAGGTGTTTGACCTTCAGGTGGGTGGGCTTGAGAAGACTAGAGGTGAGGCCTGCAGAAGAGACTCCATGTTTGGGGTAGGTGGAAG
GGAATTTTGAGAAGGGGCAGCCTTGGGGTATTGAGTTCTCCATTTCCTCCCACCCTCCCTGGGCCCTGGGCTTCTACTGAGGCTCTT
TCTGCCACCATCTGCCTCTGTGGTCCAGATGCACCATGTCTCTGTGTGACCGTGACTTCTAGGTCTGAGAACTCTGGTTTTAGCTCC
TTAGCAGTATTTTAGCTGCTGTTTCTGGGGACTGTTCCTGGTGCAGCCACAGGGGCATTCTGGGGGTTGCTCCAGAGGGCAGCTGTG
CCTGCATTCCCCTAGGTATCATCGACTTGCTCCTGACCCACTGCCGGGCCAGGTTTCAGGCCTGGCAGATGATGGGGAACCCTACTG
GGGCCAGCCCTGCGTTGGCCCCAAGGGCTTTCTTTCTTGATATTGGACATGACTGTGGACTTCACCCCTTCTGAGGCCTTCCTGGGT
GCCAAGCAGTGTGATTTCTTTCTTTTTCTCTTTGGTTTGAAACAGAATCTCACTCTGTTGCCCAGGCTGGAGTGTAGTGGTGCAGTC
GTGGCTCACTGCAGCCTCCACCTCCTGGGCTCAAGTGATCCTCCCACCTCAGCCTCCTGAGTAACTGAGACTACAGGTGTAGTCCCA
CCTGGCTAATTTTTGTATTTTTTTAGAGACAGGGTTTTGTCATGTTGTCCAGGCTGCTCTTGAACTCCTGGCTCAAGTGATCCACCT
GCCTTGGCCTCCCAAAGTGCCGGGATTACAGGGGTGAGCCACTGTGACTGGCCAGCAGTGTGATTTCTAACTTGGGTCCTGAACCAT
TCAGGCCCTTTCTGAGTAGCTTTCTAGTCAACCTTCCAGATGTCAGCTCTGGCTCTGTTCTGGGGGTGTATCTGGTTGTCCTGGTTG
TTCTGGGCTCTGTCCCAGTGTGAGTGTAGAGTGGGTTCTGGGAGCTGCAGTTGGTGAATCTGAGTCTTTATTTGGTTGCCTGATAGG
TTGTTTTGAGGTCAGTTTGATCCCAGACTGACGTGGTTCAAAGTTACTGTTTGGCTTTTCTAGGATCCTGCCCTCATGCTGTTCTGT
GTGCCTCAGGGTCTTTGCAAATGTTTAGTCTTCTAAAATGGCCCTGGCCCACTTTGGGTACACACCTCTTTCCAGGTGTGGCTTCTG
CTCCATGTAGGGCCTGTCTCAGTCTCCAAGCACGAGACCCTGCTGTGGCTGGGTGTGGCAGCAGCGCCTGGCCAGCTTGGGGGGCCT
TTCCCATTACCCAGTTCCCTGCCTTGGACTCTGCCCCTCTGCCCAGGGTCTCTGCACAATGGTGCAGCCAAAAACCTCACTTGAATA
CTGTGAGGCCCTGCGGGCATCTCTAGACAAATGGGCCTTTAAGTTTGGGGCCTGGAAGCTGGTTTGTCTGGCCAGACCTGCTGGGCA
GCCTGTATGCTGGGAGCAGCAGCTGCTGGAAACTCAGCTTGGGGAGCGGGAGGGAAGCATGCAGAGAGGGCTGCGGGTTCTGGTGC
AGGTAGGTGGGGCAGTGGAGGCTGGGTCGCCAAGCTGGCGAGGGCCATCTTGGGTCAGTTGGGATGGCCTTCAGTCGGTGAGAGTAC
CCCCATATTGGCAGAGGTGGTTAGTGCAACTTGAGGGTAATTCCCAGGTGCAGAGGTGTGTCCTTGATTTCATGATGAGGGTCAGTC
TTGGGCCGGTGAGGACATTCCCACGCACCACCCTCCCACTGTGGTTTCTTTGTGTCCGTTATAGGAGGAGCCAAGGTGGCAGAGTGG
TGTGGGGTGGGGATCACTGTGCATGTGGGGGCTTGAGGTGAGAAGCCTGCTGCAAGGTGCAGCTTGCGTCGAGTAGGCCAAACAGCT
GAGCTTCCGTGAAGTGGTGATGAGGTGGTATGGGTGTGTCTGACCCCACCCCCACCCAGGATGGTACCCACGCTGGTGGGGACTCA
ATAATGGCTCTAGAAGGAAGGGGCTGGGCTCTGTCGCTGCATGTGTGCCAGCAGAGGAGGCATCCAGGGGCACGCCTTCATTTTAAA
AATTTATAGAGTAGAGAAAGTCAAAAATAAATATTGCTTCTGAAGCTTCTTTGAAGACTGGCATGTATGTTGGGGTGTGTTCTTCCAG
GCTGGGCTGTTTTCTTTGAACTCATTCATGCGTTCATCCACTCATTCAGTGTATGCTGACTGAGCACCTACTATGTGCCAGCTGTCA
GCCAGGCGCTGGGGATACGGAAATGAGCTGGGCAGCCACAGTCCATGCTCCTATCAAGCTTATGGTAGTGACTCAGTAATTGTAAAA
ATATATAAATAGTCATGTTTGTAGTCACGGCTCCAAGCCAAGGTCCAGGCAGGTAGTCTGGGACATTGTGGAAGGCTTCCCTGGAGAA
AGTGCCACTGGACTTGAGATCCGAGGGTAAGTTGGGCCAGAAGAGCATGGGTAGCCTGTGCCAGGCAGTGGCATTCAGCACTGGCAAG
GCTGGGAGACAGGAAGAAATTTGGCACATTTGAGGATCAGAAGAGGAAGTTCCCGTTCTGGCAGGAGGAGAGCATGGGCAGGTAGGG
CTGCAGAGGTGGGAGGGGCCGACTCTGCTTGGCTCTGGGGTCATGCTGAGGAGCTGGGCCACAGAGTCTACTGCTGAGGTCAGGGAC
TTGGTCTGTGTGGCTCACAGCTATATGGCTGGTGCCTGGAAGGACGCCCAGCACATAGTAGGTGCTTAGGGAGTATCAAAATGAATG
AGCGAGGGAGTGAAGGGAAAAGATGGGCATTTTTACCAGATGGGGATGGTTCTACATGAACATTTCCATAACCTGCTTTTTTTTTCTG
CATAAACAGCAAATTTTTAATGTCTTTCTGTGTCAATACATATGCTGTTGTGATGAAATTCTTGATGTTTTCTCATGCTTTCCTTTC
TGTCCAGGAAGGAGTGGAGGCCCTGGTGCCCGGCCCTTGGTGCTGAGTATCCAGCAAGAGTGACCGGGGTGAAGAAGCAAAGACTCG
GTGAGTGTGCCCCACAGAGTGGCCAGGAGCAGGGGTGCACAGGGGTCCTATGGACCAGGCTGAGCAGCTTGGATTTGATGCAGGGTC
AGTGGAGAGCTATAGAGGGGGTGTCAGCAGGGGGCATGACATGATCCAGTGTGCACTGGTGAGAGATTGTGGAGGCCTGGACCAGGG
AGGCAGTGACAGAAGTGGAGAGAAAGGGATGTGAGTGCTATTTAGGGAGGTAGAGTAGCAGGACGTGGTGAAGGATTGAATGTGGGG
CATGAAGGAGGGAGGTGTCAAGGATGGTGCCTGAGTTTCTGGCTGGAGTGGATGATAGAGGTGGATGGGGAATGACATACAGCTCG
TCAGCTACTGTAGGTGTGAAATAGGCTTCCGGGTGATGTCCTGGGACTTACACCAGGATCTTTCTTCTGCTTCTCCATTCACCACGT
TTCTGTAGAAAAGACTGTTCCCATCGACTGTAGGTTGTCGTAGAAAGAGCTTGGTGCCTGCAGGATCTGGGCATGTCCTCTGCATCT
CTTTGATGTCCCCTTGTTCTCCTCTCTGTCTGTTAGAAGCACAGAATGGGTGGGCTGGAGTTGCAGGGAATGAGGTTGGTAAGTTGG
TTTAGAGTCAGCATCATGGGCCTTGGATGTCAGGCTAAAGACTTTAGACTTTTTCCTGAGGGCAATACAGCAGCATTGAAGGTTCTT
AAGCAGAGAAGCAATATCCATGTGTTGATCTGTCCATTGCCCATCCGTTCATTCTTTCACGGAACACTGTCTGAATGCCTGCTGTGA
AATAAAGTGGCAAACAAAAGTGCAGATTTGCATTTTTATGTGCTGAAAGGTGGCACTGGTCTGTGTGCGGGATGGGTTGGGAGTGAT
TGAAGGCAGGAAGCCCACCAAAGAGGCAGGAAGTGATGAGGTCTTACTAACACAGTGGCAGTGAGGATGGAGGCAGGTCAGATTCAA
ACTTATTGATGGGTTAGATGTGTGGGATGAGGACAAGGGAGCATTTAAGGAGGGTTTGCGGGCTTGGTGACTGACAACATGGTGGTG
CTGTCCCCAAGACTGGACACCCTGGAGGAGGAGTGGAATTGGGGCAGATGAGTTCTGGTTGGGCCATCCTGAGCCAAGAGCTCGGTG
GGTCTGTTCACCCTCTGTAAGCTCCTCGAAGGCAGAGCCTGTGACTCTCTTGTTCATCACCCTGCCCACTGAACATCTGACGGGGTG
GCTGAACTAACAAGGCTGTGGAAGAGATGGGTCTGGCCTGGGTAGGGGAAGGAAATGTGTATGTCCTGGGTGTCTCCTGGGGTCCAG
ACCCTACGTTTGGTGAGATGGGCACTGGGGTTATGTAGAGGGTGCAGCAAGAGGGGCTGTGTCACAGGCTGGAAAGGTCAGATGGGG
AGGGTGGGTGTAGGGACACTATGTGTCCCTTTGCCTCTCCATCCTCAGATGATGGTCTGACCTAACTCGAAGTCCAGTCTTGCCAGA
ATAGGTACCTGAAGGTGGAGCCCTCTGTCCTCTCGGGAGGCCACTAGCTGATGGCATGTCTGGTGGGGTGCAGATGGGGTGTGCTAT
AGGACCTGACTTCTGGAGGCTGGGTAGGGCTGATGTGGGGTACAGGGGAAGATACTCTGAGATCCTAGGGCCAGGCGCCAGCAAAT
ACAGGAGTTAAGCCAGGTTGGAGCTTCCTTGGGTACAAGGCCCAGGGTGCCCACAGGGGATTGGGTTCTGGGGCAGGGGCCAGGTCA
GGCTCTAGGCTCAATTGCAAAGGATCTTGTTGTCTGTGTTGGGGTTTCTCAGCCTCAGCACCATTGACATTTTGGATCAGATAACC
CTTATTGTGGGGCAGGGCTTGTTATTGTAGGGTGTCTAGCAGCATCCCTGGTTTCTTCCCCCTAGATGCCAGTAGCATCTCCCCGC
TTTCCCCAGTCTCGACAATCAAAAATGTCTCCAGTCGTTGCGTAATGTCCCATGGGGGCAAAATTACCCTGAATTGAGAACCTCTGG
TCTACAGCCTCTTGCTGCTTTCACTATACTGTACAAACCTGGAAAATTGGGGAGGGTCAGTGGGGAAGATGGATAGTGATGGGCCAC
TTGAGCAGAGTTCTGAGGGGTGAGTGAGTGCTGTGGGGCAGGAGGAAGGACATTTTTTTCCAATAGACAGAACAGGCAGGTACGAGGCTT
GGAGGCACAAAGTTGGGAAACAGTAATCCAAGGGGGACCCCAGCCCCAGAGGAAGGGGCCTGGAGGCTTAGGGGTTACAGCCGCAGG
AAGATACCTCTGCTGGTCCCCTTTGGTCATTTGCCTCCAGAGTTAGTCTCCTGCTGACCCTTTGCCGCCTCCAAGTCTCTGCGACAC
TCCTCCTAATCTCCCCCTCCACTCCTCAGTGAGATGGAGAAGCTGAGAGCTGCAACAGGGTGGGACTCAGTCCAGCTGGTGGGAGCC
CCGATACCAGCCGTGGGAGGGAGGGCTGTTCCTGGCTCTGCTCACGGATTCTCGGCCAACTGGCTGGAGGAGGGAAGGCGGCCTCACC
```

```
CTCTTCCCCACAGGCCCCTCCTTCCCTGGGTCAGGGGTCCTGGCTGAGACTATAATTTATTCCCGTCATAATCCAGTGGTTGGTTTG
GTGAGAGCTGGAAACATGTTGGTTTTCCCTTCCCAAGTATAACAAGGCCTGTTTCCGCCTCCGCGGCCGCTGCTGCAGTGCCACGCG
GTGAACTGTCCAGGACATGACAAAAGGGCTCGGTTAGCTGCCCGCTGGTTAGAAGATGAACGGCTCAGAGCTCTCCCTGCCGACAGG
CTCTTCCCTTCCTGTTGCCCAAGTGCTGGCCTTTCCTCTTGGCCGTCTGCTCTGTAGGGCCCTGGATACCCCTCCTTCTGCTCATGT
CCATTTCTGGGCCCCAGGGTCCTGGCCTGGGGAGGTGAGATGGGGGAGGCTACAGAAACAGGTGGTATTTGGAGACTAATTAATATT
TGTGAATAATAATGCATGCCCAAGGAAAACAAATCAATCTGTGTTTGCGTATTTATGATGAGGACTCAGGAATGATCCCAGCTGTTC
TCCAGCCAGGGAAGCCCCCATCACATGGTCCCTTGGGCCCCCACAGAGGCTGGCAGGGTGGTTAGGGTGGGCTGCATGGAGGTGACA
TGGCTTCTGTGTTTTGCAACGTGAATCTTCTGTGACGTTCATATGCGAATGCTGTGGCTTGGTCTAGTGCCTGCTTCTTCCTTTTCC
CTCAAAGCAGTGATTCCCAGGCTCTTTGGTTTCACGAACCAATACAATTTCCAAAAGTACTCAAGAGCCAGACATGGGGTTGGCAAT
TTTTTATTTTGCCAAGTAAAGGCATTATAAAAAAAACAACTGCTATCTGCTGTCCCCATCATTTCATACAGGAAAGAACATTTTAACA
CCAAGGACAATATTTGAGAATAAAGCAAGAAAGGGCAGTTGGCAGCTTTACACTGACGTAGCAAAGGAAAAGGATATATTAAAACCA
TTCTGAATGTGGAAAAATTGTGTCCAATTATATTCTATAATATCATACCGTATGTAATTGTGTTCCTGCCCAAGTATGTCAGCTTGG
CAGAACGGATGGTCCACAGTGCAATTCAGAGACGAGCTTGAGTGCCCCAGTGCTCCGGCCTTGTTTTTATCTCGGTTGTTGAAAGCC
CAGGCTCACCCAGCCATGCTGGTGAAGTGGGTGCTCTGCTGTGAACACCGTTGCATGGATTCACAAGATGCTCAACCTCGCAACCGC
AGTCAGGCTAGCAGCAGCCCTGCCCCTTGTTCCTCTCCCACCTTCTCCCCACAGGTGACCCTGGGACCCTGGGACTCTGGGGGCCAA
GGTCTACTCTTGAGGCCAGGCCTGGGGATCCAGGGCTGCTCTGGCTGTGAACACCGTTGCATGGAGGGAGGCCCTGGAGCTGGGAGTGGCCTGAACCGCC
TGACACCAGGGCAGGCTCTGTGCCCGGAGTCTCAGGGCGGGAGGCAGCTTGCTCTCGCCAGGAGCTGGTGAGGGAGAGGCCCTGGCT
CGTACAGCTGTGTGGCTGCCAGAGTAGTTGCCTGAGAATGTGTTTGTGTGTGTCCCTTGTGTTTCCCCATCTCCCTGGGCATCTGTG
TCCTTGTGTCCATCATGGACCACTGGAAGAAGCTCTCTGTTCTACTTTCTGGATCTGAACAAAGTGTCCTGAGCACCCCAACCCAGA
TACACAGGGGGTTTCTGGAGGCCCCACGTTGGGGGTAGGGTTACCTGAGGCCCAAGTTCTCCAACACCCATGGCCAGACTCTCATCC
AGGCTTGTACCACATGGGTGTCTATTCTGGGGAGTTTTACCCCCATGATGGCTCTCCTGGGAGCTCTGCCCCCACCATGAAGTCTGTA
ACAGCACGTTACACCTCTGCTAATGTATGGGGAGTTGCACCCGTGTAATGCCTTATCAGGCAGCTGCACTCCATGATGTCTGTACC
CTCTGTGTCCGCCCACGTGATGTCTAGACCACACCATTACACCTCCATGATGTCTGCACCTGGGCGTTATACTGCTATGATTACTGC
ACCAAGGCGGCTACAGGGGATTAAGCCTCTGCTCATTGTACTGGGGAGCTATATCCCATGATGACGGTGCTGTGCATTTTATTTTCA
TAGCAGGGAGATATGCTCATGCTGGGGGCCTATGTCCCTATGAAGCCTGCCTGGCAGGGGTTGCATGCTTGGAATGCTTGTTCCTGG
AAGTTAACCTCTGTGACTATTATATTGTTATACCCGATGATGCCGGTACGAGGCCACTGTGCCCTGTCCTTTGGTGCAACACAGCTG
TTGAGTTATAAACTCATGTGTCTCATTCAGGGGCTCCAGGATGGTCATCAGGGGACCATACCCCCATGATGACCTGGCCTGGCATA
GGATACCAGATGTGTCCACAGGCCCAGGGAGGAAGAGCCACTCATAGTCCACCACCTGATGGCCGCTGGGAAGGTGCTCCATCGTTG
GCTGCATGTGGCACCATAGCGCAAGCTCAGGCAGGGCCTTGGTGATGTGTGACCTCCATCCCACTGTGGCCTTGCAGGCGTCATTGT
GGTCTCAGCCACCTCAGCTCTGGGATTGTGGAGCAGATCCATCCCCAGCTTGGTGTAAATGGGGCTGGACACCTATCAACGTTCTTA
CTTGAAGAACTGGTGTAAATGAAGGCTGGACACCCTTCACCGTTCTTACTTTGTTGTTCAGCTCCTGTCTCAGATTTTTGGTGGGAT
CCTGGGACAGGGACAACTCTCACCCCTTTTCCTTTGTGTTTGGCCCCATCCCACTTTGCATTCCCCACCTGCTTTGTCCAACACCTT
TGCTCAACTGCTGCCTTCTGACCTCATGAACTTTGATTATCTCAGTTGAGACAAGGGTTCAAATTTCAGAAACGACTGGAGCACCTT
TGAGCCCTGCCCATTCCTGGTCACCTGCCCTGTCACCTTGCAAATTCTCTTACTATTGTGGGTCAGCGGGGACCTGGGTCTGATCAC
AACTCCCCTATCAAAGTCCATGCGTATCACTGTGGGGCCCCAAGGCGTGTTCACCGTCATGCACTCTGCTCTGTTGCGTCTTCACAG
CATTCATGAGGGATCGACCACGTCTCACCCCTCTGCCCAGAGCATGGTGTGATCAGAGCTTGTGGGCCCTTCCATACATCCCCTGGT
GCCCCCCAGGTGAGGGGTATGGGAGAGACCCTGGGGAGCTCACCAGTGGGAGCTGGATAGCGAGCGTATCTCCCATGTATA
GTGCGGCCATGCTAGAGGCTTCACCCAGGTGCCTGCAGGCACTTCAGGCCAGGAGCCTTGGAAGTAAAGGCCTAGGGGCATTCAGCC
AGTCCCAGACGCTACTCAGTCTTTTTGTGCATAGCTTCCTGTCTCAAACATCAGCCCCTGAGGTTCACACCCCATCATCCCTACACT
TGTTTGGCTCCGGGCTTCTGGGGCAAGGGCAGAAGAGATGTGAGGTTTGAATTCTAGGGTCTAACACCCCATTCCTGGCCACTGAGG
CCTCCCTGTGGTTTCTCTGTGAAGTAGGAAGGTGCCATGGGAATGGGTAGGACGAGGGTGGCCAGGCAGGGCAGCAGCAGCTTGTCA
AGAGCACGTCCTGGTGAGCACTGAGGGGTTGTGGCTTTGGCTGGAAGCCTCTGATAACCTGGAGCCTGTCTTTCTGCTAACAAGCCTGT
GTTCAGGGGCCTGTGCTCAGCGTGCTCGTGGAGTCTGGCCTCCCACCTTCTCCCTCCCTGCTGGGGAGGAGGGGCAACAGAATTC
TACCAGGGAGACTCCAGAGTCAGTTGGCTTTGCCCCTCACTGCTCCTTGTCACTGTCTTGTCTCTCTGTCCTCGTGTCCCACGTAAGC
GTCTCTCTGACCCTGTTTCCCTGTCCTCTTATGGGGGTGAGTTAGAAGCTCAAGGTTTGGACTGGAACACACTGTACATCCTCTGGT
CTCACTCACTCCCTGTACCTCCTACTCAGGTTTAGAGAATGGCCCGAAGGCCTCCTGCGCTCTTTCTCCCTCCACCGTGACTGTTGG
GCTGTCCTTGTGGGTGCTGGGGAATAAGTGAGGACGCTTGGGTGGTCACTGGGGTCAGAAGGTCTAGGTGGTCATTGCACAGCCCAAG
TGGGGGGCTCTGTTGAACTAGGCTCTGGATGGTCAGTGAGGATGACTGGGGGTCAGAAAGCGTCAGGGGTGGGCATTAGGGACAGCA
GTAAATACCAGCTAACTGGCTCTGCCCTTCTCTCCATTACAGGTTGATTGTCCTGGGCTGTGGCTGGCTGTGGAGCTAGAGCCCTGG
ATGGCCCCTGAGCCAGCCCCAGGGAGGACGATGGTGCCCCTTGTGCCTGCACTGGTGATGCTTGGTTTGGTGGCAGGCGCCCATGGT
GACAGTAAGTCTGACCCCTCAAGGTACAGATCTCCCAGGTTGAAAGGTGGCATCCTTCCCAGCAGGACTCTGGGATGGGGACAGACC
GGCTACTAGGAGAGACAGGGTGGCCAGAAACCCTTTAGACCTTCCTAGGAGAGCCCCCACTGCTTGGAGAGCCTCCTTCTAAGG
AATACCTGGGTACTTAGGAGCATCTTTAGGCACCAGAGCGGGGCAGTCGAATCGTGACCCCTGTCTTATGGGGCTGGGGTGAGACCTG
TCCTGGATTCATGTGGTCATTGTGTGATGTGCGCGTGTGTGTGCTGTGTGCACTGTTGCAGATCTGTGCTGAAGGCACAGTGTGAGC
TACAGCCAGGGGTCGGCATGGGAATGGGGGGTGGCACGCAGGGCATGCGTATGGATGTGTGCCCAGGACGCGGGTGTGTCCATAGGC
CCAAGGAGGGTCAAAAGCGGGAGCCACCCATAGCTCTTCCCCTACGCTGGACCCTCAGGATCCAAGTATGCAGATCTGAGCCCCTCC
TTCTCCTTTCATGGGGACCTTCTAGGCAAAGGAGCATCCCCACCCCAGCTCCTCAGACCTGGAAATGGGGGGAAGATTGTGTTTG
GCCGGCAGGAAGCTGAGCAACGCCCCTCTGTGTGTGTTTGTGTTGGAGGGAGGTGGAATTGTCCCCATCCAGATCCAACTCCGAACT
TTGGTCCCTTTCCTGCTGCCCTTCCCCCTGCCTGGCACTATGAGACTGGCCTCAGCCCGCCCCATCGCCATGGTTACCACTCCTTGG
TTACTGGTGGGAGGCGGGGCACAGGGCAGATTTAGCCAATCTGCAGGCTGAGGGGGAGGGGCGAGGTCGGAGCCAAGGTCCCTGGGG
GAAGGGGCCGTTCCCAGCCTGTCCAGAGCCCAGAGGTGATCCAGGGCCAACCCTGGGGTCAGCCCACGGGTCACCAGGCCATAGGGC
TCCCCCACCTGCCTCCGTATCTCTGGGTACAGATCTCTCCCCTTGCCCCACCAGGGCTCTTTCTCCTAATGGCTCATTAATTATTCA
GGAACTGATTCTGGAGTGGGGTGGGACTGGTGTGGTGGTCCCGCCCTTTGTCCTCAAGTGCTGGGTCTGGGTGGAGCTAGAAGGGAAAG
GAGAAGGGGCAGGCATTCCCAAGGGGTGGGGCAAAGGGTCATGGGAGCACCAGGTACCAAAGAGAGGGCTGGGCAGGTGAGAGCAAG
GAAAAAAGACAGGTGGGGAATCACATGGGGAAGTGGGGCAGGGGATCTCCAGGCCTGGCTGGAACCTGCAGGGGCAGGTTGGAGTAG
AAAGCCTTATCAGGTGTGACCCACATGGTTGGCAGGAAGGTGAAGGCTTGTCCACGTGTGGTCAGTAGCTCTTGGACATTGAAGTAC
TGTCCTTGCCCTCCCCGCCGACCCAACCCCAGGCACTTTCAGGGCCTTCAGACAGGACTCTAGGGCAGGGGAAGGATGGCCCAGTT
GGGCTTCAACAGATAACACATCCTGGGAGAAGAGCTGCCTCCTCCTCCTGCCTCACGACCCAACGCGCCCAACCTGTACCCCTCAC
TCTCATCCCCCAGCCTGCTGTCTTCCCGTGTTCTCTCCCGTGAAAGGCATTACCACCCACCCGGTCACCCAAGTCAGAACCTGGGCC
TCCTCCAGCCTCCTCCCTCCGTGATATCCCACATCTAATCCATCACCAAGCTCCGTTGCTTCTACCTTCTGAATATCTTTGGAATGC
ATCCACTTCTCTCTCTACCGCCTTCATCCAAGCCACCCTCGGCTCTTGGGCTTTTGCACGCAACAGCCTCCTGACGGTTTCCCTGCC
TCCAGTCTTTTCCTTCCTAATCCATTCTGCATTCCAAAGGTAGAGTCGTGTCACAAGGTGAAAACATCATCCTGTCACTCTTCAGTT
TAGAATCCTTCATTCCCTCCCACCCCACCCCCACTCACCCACGCTGACTTCTTTCAGTGGTTCACCAATTCCCAGTAGTAATTGGTAGGG
GCAAGGGGGAACAGTTTTTAAAGCAGAGAACCTCCATGCTGGTTCCTTAGGAACCAAAGCCCAAACACCAGGTCTTGACGGTAGAAA
GCAGCTGAAGAAGCTGAAATCCTGCCTTCTTCAGGTAGGGGGTTCAGGGGACTCAGGCCTTCTCCCAGGCAGAGAGCCATGGGGCAG
```

```
GAGCCTGGGCTGGGGGTCAGAGTGACCGAGCCGCAGGAGCCAGGGTCTGTCTTCCAGGCCTGTCTTACACTGCACACAGTTTTGGAT
CTCTCCCTGCACTGAGGCAGGGCTCAGGCTGAGCTTGGGCAACTACTAGCCAGGGGCAAGTGGCCATACACAGACAGGGCCACTGCA
GAAGATAGGGTAGGCACCCCAGAGAATTGACAGCTGGGGCAGTGAAGCGCGGAGTGGACAAATGTGTTTCAATAGGCCTCTTAACAA
GACAAATGAATGGGGGCCCTGGCCTCTGAGGGGAGTGGAGGGAAGCGGGTGGAGAAGCAGCTGCCAAGAGTTAGCCCAGAGGCCCCA
GTGTCAGTGCCGCACGCGCAGCTCCAGTGGAGATTTGGGCACACATTGGGGGTAGGATCTGCTGCAGCGCAGCTTCCCCAGCCAGGCT
TTGTGGCTTCTCAGGAGGGGAGGTTGTGGGGCCAGAGTGTCCTGAGCCAGGGCAGGAGAGGTTTTTGCTGATCTCAAGTGCGTGTCGCG
TGCCTGTCTTCAGGCAAGACCTGCAGTGTGGAGGCACAGGCTTGTGAGCAGTGACCACAGGGTGTGCTGGGTAGGTGGCACTGACAC
AGGCCATGGCAGAAGCATCTTGGGAGAGGAGTTGGGAGGCTTCCTGGAAGAGGGGAGCCCTGAAGGGTGAGTGGACATTTGCTAATG
GGGGGGTATTGCATAATGAGGTGGGGTTGGGGGGAAGAAGCACGAATGTGGCTGGGCTTCTCTGTGGAATTCATGGGAGAGCCACAG
TGAGAACCCGACAGCATGGGACAGAGGTGGGGTCCGAAGACCAGGTGGGAGTGGGAGGTGAGACCCTGGTTATGCCCATTTACCCAGAATCCT
AGGAGCTCAGAGCAGGATGCGTGCTGGCTGGAGGGGTGGGTGGCGGGTGGGTTGACAGAGGTGGGCAGAGGCTGAGGAGCTGGGAGT
GTGTCTGTTGTGTCATTTCCCTCCTCCCCAGAGCCTGTGGAGCACACAGGGTCTGTTGTCTGTCGTCATGCTCTCCCCCTCGTTCTA
TGGGTGGCCTGTCTAGACTCTGCTCCCCTGTGGGACTTCCCGCAGATTCCGTTGCTTTCTCTCTCTGGGCCTGTTTTCCTATTGCACA
ATGGGGATAATCACTCCTACCTGGAAAGGTTAACTGAGGTCACATGGATGAGGCGCCTGGAACCTAGTATGTATTCCCCTTATCTGA
GGCCATGACCTGGGGCTCTTGCTCTGTCCCTGGGAACCAGGCTGTCTCTGAGTGGGCTCCAGGGGGTACCAGGAACAGTCACAGG
AGCTCACTGAGTCCCAGCTTAAGCTGCTCAGACCCAGGGATATCTGTCTCTCCAGAAGCTCCCTGCCCTGCCTTGCCGGCCCTCAT
GGCCCTGCCTCCGTGTGTACATGTGTATGTGTATTCCATGGGAAAGGCACAAAATAGCAGTCAGTCTCTCCATAGAAGAGCCTTGTT
GGTGGCCCAGTTTGACTCTCCCTGGGGCTGGACCCCTACAGCCTCCCTGGGAGGTGGTTGCAGCCCCCTTCCTCCAGCCAGTTCCAC
TTACTCCTTTCTTAGGCCACTTCCTCCCACCCTGCATGGGCTTGGTGGCTCGAGAATGTTGCCGTCCATACCCTGGGAGCTGTGCTG
AAAGGGCTGTGCGGCCCCCGACCACTGTGTGTGTCAGGGAGGGGGCACGCTCTCGTGGGGTGTCAGGCCAGGTGGCGGTGGGTAACT
GGCAGAAAGGCCCTCCTGGTGTGCTCTGGTGGCACCCTGTTGACCCAGTCTCAGAAGTTGTGTTCGACCCTCACTGAACACCAGCT
GTGGGTCAGGCACGGGGCAGAGTAGTCCAAGTAGCTTGGTTTGCTGCCTGCCTGGGGACCTGACACTGTGGGATCTGGTCAGTGCTG
GGATGGGAAGCTCTGGGCACCTCAGGCCATGGGACACAGAGCAGGCTCCTACAGCAGCTTGGCTGGGTGGGACATGAGAGAGGGGCT
GGGCTGGGCACACTCAAAGGCAGGGAGGAGTCTGAGGGCCTGGCCTGTCAGGGTGGCCTAGGTGGTGGGTCCAAGCTGTGTGCTCTG
CACAGTGCTAGGCCTGTACTATAGTAGGTGCTCAAAAAAATACTTGTTGAAAGAGTAAAGAAGCCGGGTGTGGTGGCTCATACCCGTA
ATCCCAACACTTTGGGAGGCCAAGGCAGGTGGATCGCCAGAGCTCAGGAGTTTGAGACCAGCCTGGCAATGTGGTGAAACCCTGTCT
TTACCAAAAATACAAAAAATTAGCCAGGCATGGTGGTGTGCACCTGTGGTCCCAGCTACTCGGGAGGCTGAGGTGGGAGGATTGCTTT
GAGCCTAGGAGGTGGAGGCGGAGGTTACAGTGAGCTGAGATTGTGCCACTTGTACTCCAACCTGGGTGACAAAGTGAGACCCCCCTC
TCAAAAAAAAAAAAGACTAAAGAAAAGTGAGCCTGAGAGCTTAGGAGGAGCACATTTCAGAGGGGGAACGGAGAGAGGAACATCAGGC
CCGTTGGTAGCTGAGGAGAGGTGCGGTTAGATCTGTGCTCCCCAAAGATCCTCTGCTGAACATAAGGGGCAACGCCTTGTCTCCTGT
GCTGTGTCCTGCGGGTGGAGGTGGATTGGAGGGAAGCGGAGGGCGAGGCCTGGTTGAGGGGGCGGGGCCTGCCTGTCTGGTCCCCCGG
.GCTGCCTTGGGCCAGCCTGGCCTAGTCTGTTGGGTGGGCGGGCAGGGTGCAGGCTCCTCTCCAGCCTCCAAGGGGAGGGGAGTTGTTC
TGCCTCCTCGATAGCCCCAGGCCTTGGGCACAGCCCAGCCTCCCACGGCTCTTGGGCCCTCCTCCTTCCAGGCCGCCGGTGACCCAC
ACCTGGCTCTCCTCCCCGGCGTCTCCTCTCCGCTTCTTTGTTTGGAGCGGAGGCCCCGCCCCACCCCGCCCCCAGGCGCACTCGCCC
GGCCATTCCGGTTCAGCCGGTTCCAGCCCCCAGTTTCTGCCGCTGCAGGTCCCGGCAGGAGCTGGAGGGCACTTTCTCCCTGGGTT
TCTCTTCCCTGGTGCAGCAGGGGCCGCCGGTCCTCATCCTCCTGGTTCCTCAGTTCGGTCCTTCTTTCATTCTCCACCCCTGGGTGCC
AGGAACTGGGTCAGACACTGGGACAGGAATCCAGACAGGCATGCTATCTGCCCTGCCCAGGGTTATGTTCTAGGAGGGGAAGCAGCC
ATTAATCAAACACCAAAAATGTGGAAAAGTAATAATCTCACACGTGTGCATAATAAACTGTGAGTGAAAGTTATAAGCTCGGCAGGT
AGGTAATAAGCTAGGAGCAGTGCTGTGGGAGGCAAGGGAGTTACCCGGGAGTTTCAAACTAGGAACTGAGCTCATAGGTTGGGGGCA
GGGGGACTGGAGAAGGCAGTGATACTTAAATGGAGAGCAGAAGGATGAATGGAAGTTAGAGTGTATGGCGGAGGTTGGCAGAAGCAG
CAGCTTATGCAAAGGCCCTGTGGCTGCAGGGAACATGACGTTGCTCTTTAGAGGAGCCAAAGCTGGGGCTCTGGGGAGAGCAGCTGG
GTCAGACCCCGCGGCTTTGTCTGCCATAACAGGTGTTTTGAGAGTGATTCGGCAGGTCTTTGGAGGGTTTTGATAGGGCGGGGTGTC
GGGGGAGGCTGTCAGCTCACTCTGGACACTGAGTAGAGAACAGACGGGAGGCGTGGGGCAGGCCTGGAGGCAGGGGCTTCCGCGTGT
TAGGCCAGTGGAGTGCCAGTCAAGGGAAGGTGGTATCTGGACTAGGGTGTGGCAGCGCAGGTGGAGAACCCTGAGCTGCTTTGTGGA
GGGCTTCAAGTGTGGGGGAAGAGTGCACGGTATGGGGGTGGGGGACAGGAGACACCCCCAGTGGAGCCTGAGCAGGAGAGTCGTGTC
TGAGAGGGTCTGTCTGGAAGGCGCAACAGAGGCCAACTTTGCAGACAGCTGCAGTCAGTGGGAGGCTCCTTCAAAGGGCAT
TCAGGGGAAGGGCAAGGCACGCTGGGGGGTTCTGGACCTTCTGTGGTGTCTTCTTGTCTTTCTGGTCCCTACAGCCTCCCTGAGCTG
GCTGCCCGAGCCTGCCCTAGGCACTCTAAGAACATAGTCAGTCCCAAGGTCTCCCTCCAGGGAAGGCCGTAGGTGAGCTTAGGAGTG
AGAAGGCTGGATCAAAGCCTGGCTCCATGCCTGCATCCCTCTGACTTGCCAGTCATTTCACCCTCCGAGCCTCTATTTCCCCACCTC
TTAAATGGGGATAATAATACTACCTACCTTATGGGATTGCGGTAAGACTGATAATGCTGGTACGAGTGACAGCTTCCCTCATGGAAG
GCCCACCACGTAAAGCAGTTTACAGCCATCTCATTCCATCTATGACAGAATCCTGTGTCACTGTTTTGGAGATGGGAAAAAAGAGGC
TCAGAGATGGTGAGTGACTTGCATAATAATTACATAAAACCCCCAGAGCCCCTGGCCCCTGGAGTTCTCAAAAGTTCCTTCTCTGTT
GGTACCTGCAGCTGCCACTCCCTACCCCGCTCCCATAGACCCTCTCCTCCTTGGAGACTCTGCCCCATCTGCCGTCCCTTCTCTGCT
GGATCAACACCTTTTCCCTCTGCTGGCTCCCATCAGTATTTAAACATTGCCTTTCATATCTGTCTTCAAGAAAAAAAGAAAAAAA
AATTCACAAACCTCCCTTCCCGCCTCATCCTTTCCAGCTGCTGGCTGTATAGTCACCTGTACTTCCCTCTCTCCCTCATCGCCTCCC
AGTCATTCTTTGGCCTTCTTTGGTCTGGCTTTGGCCCCCACCCACCACTCCACTGACTCTGTTCTTGTCAAGGTCCCTGACAATCTT
GTGTGAACTGTTTTGTACCAGGTGTTTGACAGTCAAACATGCCTGAATTCAGGTCCCAGATGTGCCCCTCACTGCCATGTGATCTTG
GACAAGTGACTTGACCCCTCTGAGCCTGTAAACTGAGGATAATAGCAATGAAGGACTAAAGATAAAGAACCTGGTGCAGAGTGGGTG
CTTGGCAAAGGATTGTCATCATCGCACACGTTTCTGTGCCAGGGACCAGGCTGGGCCTGGGCTCCTGGGGACCAAACAGGTGGTCTG
AAAGGTCATTTCTCACAGCACTAGCCCTTTTTGGAGCTGTTCATTGGTCTGATTAATAGGAAATGGATCAGCTGTCAAGATTAACGA
GCTATTGCTACAAGATTGTAGCAAATGGGTTGGGCTTCTCTGGGTTCATGACCCTAGGTGGTTGAATTCTTAGGGATAGGGGCTGTG
GACTGGCCCTGGTATGTGTACTGAGGTGATGAGGGTGTGGCAGTGCCATGTCTGAGCCCCTACCTTTCTTCCTCCCTCTGCCTCC
CTGTGGACACCTTGAGGAGACTGTCAGAAGGCAATAACTAAGTCGGGGGGGAGGGATGGGAGAGGCAGATTTACAGGAAAGCATTCA
CCTGGGAAGATATCCAGAGAGACTTATGAACTGGACTGTCTAGGCCTTTGGGACTGCTGCTGGTATGTGGGGGCTGGGAGAGAGGGA
GGAGTCTCGGTTCCTGGCCGGAGCCCCGGGGTGGATGGTGGTGCCATCACTGAGATGGAGAGCAGGGGGAGGGACAACTCTCAGGGA
GAGCTGGAGCTCTTCCCAGCAGCTCTCCAGCACGCCTTTTTCCTGGAGCTTGGAATTGATGTGGGGCGGGCAACAGAAGGGGTGCAGT
GGTAGTGTGAACTCCAGACTTGGAACACCTGGGTTCAGATCTTAGCTCTACCACTTACCAGCTGTGTCATATGGGACAAATCCCTTA
ACCTCTCTGGGCCTCTAGAAACAGATACAGTTATAGCACTCACCTCATATGCTTAACAGAGTTAAAAAATGTTAAACTCTCTGAACA
GTGCCTGGCACATACTAAGCGCTACATAAAGGTGAGGTGTCCTTGTTTTCTTTGTAGGTCTTTCTCTCTGCCCCCATGACTGCCACC
TTCCTCACTGGCCATTCCTATAGTGACTGTGCTGTGGTGACTTGGTGTCTCCATCTCTTCCAGGCAAACCTGTCTTCATTAAAGTCC
CTGAGGACCAGACCTGGGCTGTCAGGGATGGTAGCCTCCTTCGTGTGCCCAAGCTACAGGAGAACCCAAGCCGCGCATCACATGGATGA
AGAAGGGGAAGAAAGTCAGCTCCCAGCGCTTCGAGGTGCGTCTGTGGTGGAAGGGGTCGGCAGGGCTCAGGGTCTGCCCACACTCT
CTCCTTTTCAGTGTCCCTCCTCATGGACCTTTTGGAGGTGGGAGGACAACTGACCCTGAGCAGGCTCCTGTGTCCTGAGTAGGCTGTG
ACCCCATGTCTGTCCTCTGACAGGTCATTGAGTTTGATGATGGGCAGGGTCAGTGCTTCGGATCCAGCCATTGCGGGTGCAGCGAG
ATGAAGCCATCTATGAGTGTACAGCTACTAACAGCCTGGGTGAGATCAACACTAGTGCCAAGCTCTCAGTGCTCGAAGGTACGTGCT
AGGGAGACGTGGCACGGTGGGCTGCCGGGCTGAGGCGTGGGAAGAGCCAGCCAGCCCTGATCCTGTCCTGGGCCCATGTGCATTTGG
```

```
CAGAAAGGAGGACTGGCCACCTTGGGGTCAGTGAAAGTCAGTGGTGGACAGGGATAGTCATTGGATCTGGCCTGGATTGTGCGGCTT
ATGCTGAGGCCAGCCATGTGGGGCATGATGCCTTTGTATTCTCCTGCTGAGCCGGGTCGTTGGTTGGGTGGGGTCTGGGGTCTGACT
TGAGGTGTGGAGCTGCAGCTGTGTATCCCTTGGGTTACGTGGTTATGGCTGTGGCTGTTTGGCAGTGAACCGGATTTCCATGTGGAG
CCTGGCCGTAGGTGTCAGGCAGGTGTGTTCCTTGTTGCCCCTGTGAGCTGAGGGCTGGGGCTCTGTCCGTGGATTTTAGTGTCTTCT
CTCACTTGGTGGCTTCTCCATTCATTCACAAACACTCCCTGGACCACCTTGAAGTCCTCTGAGCACCGAGGAGGAGGAAGCTGTGTC
TAAGCCCAAGTCTTGAGGACAGGTGGGAGTTGGGGGTGGCAGTTGGCAGCTAGGCAGGTGCCCAGGCCCAGAAGCAAGAGAGGATGGA
GCTTTCAGAGAGCTCTGAGTAGTTCAATTTGGGTTTTCTGGAGGGCAGAGGGGGAGCTAGAGAGCACAGGAAGAAGGAGAAAGCAAT
TCAGCATGAGTCTGGAGAGGTTTGGAGGGCAGATTACACAGGATCTGGCTGAGAAATGAACACTCTCCTAGGGACATAGGAAGCCAC
AAACAAGGCGGGGGTGACATGATCAGACCCCAGCCACAACTGATTCATCAGTCTGAATGTGTTTATGTTTTCAAAATATAGCATCGA
TTGTTGCTTGCTTTTTTTTCTTTTCTTTCTTTCTTTTTTTTTTTTTGAGATGCAGTCTCACTCTTGTGCCCAGGCTGGAGTG
CAATGGTGTGATCTCAGCTCACTGCAACCTCTGCCTCCCGGGTTCAAGCGATTCTCCTGCCCGGCCTCCCAAGTAGCTGGGATTACA
GGCATGCGCCACCATGCCTGGCTAATTTTGTATTATTAGTAGAGATGGAGTTTTACCATGTTGGTCAGGCTGGTCTCAAACTCCTGA
CCTCAGGTGATCCGCCTGCCTCAGCCTCCCAAACTGCTGGGATTACAGGTGTGAGCCACCGCACCCGGGCCGATTGTTGCTTTCTTT
TTAAGAATGTGATGTCGATACCTGTTCCTTTTTGAAAAATTGGAAAGTATAGAATAGCACAGAGGAAAAAATTAAAATGTCTCAGTT
TACCTCTAGTAATAACATTTGGTTACTTACTCGTGGCCCATTTTCTGTGCATACATATATATATGTGTGTGTGTGAACAGAAATGGGAC
CACATACTGTCCGATGATTTGTAGACTGCTTTAAAAACAAACAAAAAAAAAAATATGGCTAACATCTTCCTTGCCACTAAATATTCTTC
TGTATCATTATTCTTTTTTTTTTTTTTTTTTTTTGAGACGGAGTCTAGCTCTGTCACCCAGCCTGGAGTCCCGTGGTGCCATCTTGG
CTCACTGCAGCCTCTGCCTCCTGGGTTCAAGCGATTCTCCTGGCTCAGCCTCCCGAGTAGCTGGGACTACAGGTGCGCACCACCACT
CGTGACTAATTTTTGTATTTTTAGTAAAGACGGGGTTTCACCATATTGAACAGGCTGGTCTGGAACTCCTGACCTCGTGATCCGCCC
ACCTTGGCCTCCCAAAGTGCTGGGATTACAGGCGTGAGCCACCACGTCCAGCCTGTATCATTATCCTTAATGGCTATGGGTAAGCTG
TCACATGCAAGTACCCTAATTTATTTAGCCATTCCCTTATTGTTGGACACATATGTTCTCAGTTTTTTCGTTTCTATAAATAAGGTG
CCATACACGTCGTTGCAGATGGATGTGTTCACCTTCCTGATTATTCCCTTATTCTAGGTTCATGGAAGTGGAATTGCTGAGTCAAAG
GGCACATGCATTTTTAAGCCTTTTGATATTTCCAGAAGATTGTGTCAATTCATACTCCTGCCAAGCAGGGCAGAAGAGGGCCTCTTT
CCTGCACATCTTCCCCACTGTTGGGAAATATCTTCAAAAAAATATTTTTTGCCAAGTTAATAGGCAAAAAATGGCATCTCAATTTAA
TTTGCATTTCTTTGATTACAAGAACAGCTGCACATGTTTTCACATTGGCCATTTTTACGCTGTGGCTTTATCTGTTCACACACACAT
CTCCATTCAGTTACTCTTTTTGTTGTTGTTGTTGTTGTTTGGTTTTGGGGGGTTTTTTAGTTTTGTTTGAGACAGAGTCTCACTCTG
TTGCCAGGCTGGAGTGCAGTGGTGAGATCTCGGCTCACTGCAACCTCCACCTCCCGGGTTCAAGTGATTGTCCTGCCTCAGCCTCCC
AAGTAGCTGGGACTACTGGCAGGTGCCACCACGCCCAGCTAATGTTTGTATTTTTAGTAGAGACGGGGTTTCACCATGTTGGCCAGG
CTGGTCTCGAACTCCTGACCTCGCCCGCCTTGGCCTCCCAAAGTGCTGGGATTACAGGCCTGAGGCACTGCGTCCAGTGTCGGTTAC
TGTTTTGCATCTGTGTCTTGTATGAGTCCGTGGGCTCTACAAGCTTAGAGGAGCCATAGAGGACAGTGGTTAAGACTCTGGACCACT
CACTTCTCGTGTGTCTTTGGGTTAGTAACTATGCCTCTGACCCTCATTTGCTCCATCTGTAAATGGATATAATGCCTACCACCCAAG
CTGGTTTGGAAGATTGAATGCTGTGTATGAAGCGCTTCCCAGGAGTGTGGTGGTGGTTGCTGTGGGGCTGGAAGCTGTACATCTTGA
GGCCTCACCACGTGAGCTGAAGGTGGTTGGCCTCCGTGCTGCGTGGCATCATCCGGGTGATGCAGCTCCACTGCCTGCTCCCACGGG
GGGACATACCTGTGCAATAGGAACTCAGAGAACAGGCCCTGGGCCAGCATCACTGATTGCTGAGTGTTCTCAGGCTCCTCCCTGCCA
CTTCTGACCTGTTTCCCCACTCGTGACCTGGGGCAAGTATTTCAATGCTGCCCAGTCCTCCTATTGGCAGCTGTCCTAGCAGGGAG
CCATCTGCCCTGCCCTGGCATTTGGGAGGTGGCTGAAGGACCAGAGGCCCCAGAGGGCTTCTTTTTCAGGCTCTCAGATTGGGGAACA
GGGCCTCCTTGTTAGTATTGAAAGAGGAGTCCTTCAAAAGCGCCTAAGCCCCAGGTCCTGGGTCAGGAGCCCTCTTACCTCTGCCAC
TGCCCTGGGCAGCCTTACCTTTCAAAGGGTTGAGGAGGATGGAGGTGGTCTTTCAGGCCTGGGGTGAGGAGGACGGACTTCAGACTG
CGAGACCAGCCTGGCCAACATGGCAAAACCCATCTCTACCAAAAAATACAAAATTAACTGGGCGTGGTGGCGCACGCCTGTAGTCC
CAGCTACTTGGGAGGCTGAGGCAGGAGAATTGCTTGAACCCAGGAGGAGGAGGTTGCAGTGAGCTGAGATTGCGCCACTGCACTCCA
GCCTGGGCGACAGGAGACTCCATCTCAAAAAAAAAAAAAAAGAGGACCTGCAGGAGGTGCCTCCGTTTGGTTTCTCTACCAGGGGCC
AGCAGTATATGTTTGCGCTTCTGGGGTGTGAGAAGTGTCTGATGAGGCTGGGGAGAGTGTGGCGCAAGTACCCAGACCCTGTCCATG
CTCCGGGGAAGGGTGTGGAGCTGTGGGCCTGAGTGAGCCCCTCCTGCTTCTGAAACATAGCACCAGGAAGACAAAATGCTGACTTAT
CCTGCTGGTCTGTATAACCTTGGCCAGCATTGGGCTTGACAGAAAGGCGTGCCTGCAGGAGGGCCAGGAGGGAAGCAACCCAGAGGGTG
AACACCCGCCCCCTGCCCCGCCTCACCCCACCCCTCCCCTCCCTCCCTCCTAGGAGGATCAAGAAAGCTGGGTGCCTGCAGGAACTGGGCTG
AAAGGATGGTTGGACTTGGACTGGAGCTCTGGGGTGGGGCCGGCAGGAGCCAGGGCCATTCTTGGGAGGCCACCACAGGGTACGAGG
AGGGCTGGGGAGGGGATGGAGCTAGTTGGGGTCGGGGAAGTAAGAGTCCTTCCTGAGTTGCACACAGCTCTCCCTGTGAGCTGGTCT
CCTTGGAGAATGGCTGAGGGCTGTCTGGGGTGAACTGGCTCAGGTGAAGATCAGTGTGCCCCCAAAAAGGAGTCCAGGCCTGCAGTC
TGTTCTGTGCCCTCTGCCTTTGCCTCATGTCACACCAGGCCCAACTGCTGTGGCTCACTCCCACCACAGCCCGTGGGAGT
GCCCCCCAAATCCCCCACAGCCGTGCCCTTTGCACCTCGCATCTGAGCAGGATTGTTATTCCCAGTGTGGCCTCCCCTTGACACCC
CCGCCAGGACTGCATACGAGGTGGGGGTGCCCAGCACAAGCTGGCCGGGGTGAGCCTGTCCTGGCTGTGATGAGCGTGGGGCCCGCC
GCCCAGCCGTTCCTGTCTGAGTGGTAATTGAAGCCATTAGCGCGCCAGCCTCTCCCTGCCGGGTAATGGCAGGAAAAGCTCTTCTCG
CTCCGCACTCTTGAGGCGGCGGCTGAATCACTCCCCCTCCAACCCGCCCGCTGCCGCCACTGAGACAGGGAATCTGACATTTTCCCT
CACCAGGGAGGGGGAGCCCTGGGGAGGGGAGGGGAGGCAGGCCTGGATTCCTGGCCTTTCCTCCAGGAGGTTGAGGGGCAGTGAAG
GTCTTGGAGCTCAGTCTGTAAGTCATGGATTCACCTGGGGCCGCAGATTTCAGGCCTGGAAGGACTGGCCGGGAAAGCCCAGGAGGC
CGCCAGACATTCAGTGGTGTGGGGAGGGCACTGATGACTTTGGGTAGGTTCTGGGAGGGACAAGGAGGCGGGGAGAAGAAAGAAGCT
GTGGAGCAGAGATGAGCTGTTTTGGACTTCTCCTGGGGGGGCTTAGCTCCAAGGGTTCTGAGTACAGGTAGCATTACTTGTCTTGGGG
TCATTGATAGGGCAGATAACGTGCACTGTTCGTGTGGTGTGGGTGCCCTGTGTGCCATGTTTAAGGCAGTGTTTGTGTGAAGTGTGT
GGTGCAGTGTGTGCAGCAGGGGTGCTGCCTTGCAAGCTGTGCGCACAGACAGGGGTGTGCAGCGGTCAGCGCACACGTTACCATCAG
GCAGTGGGGAAGGGGCAGTGTGTGCAGGAGGCAGTGCATGCTGTATGGGCTGTATGCAGCGCGCAGTGTTTGTGTGCACGTGTGT
GCGTCAGGCAGTGCGTGGTGGGTGGTAAGCAGGTAGTGAGTACCTTATGGGCTATGTATACAGCAAGGCATATGCATCAGGCAGTG
TGTGGGCAGTAGGCAGTGTGTGTGTGTGTTATCAGGCAGTATATGTGTGCGGTGTGCCGTGCGGCAGGCTACCTGTGTTTATATCGG
GTAGTGTGTGTGTGTTTATGTGCAGCAAGGGTGTGCAGTAGGTGTGCAGTAGACTGTGTGCAGCAGGCGTGTGTGTGTGTACAGCAG
GTGGTGTGCAGCAGGCAGTGTGTGTATGTATCAGGCGGGGTGTGGTGTGTGTTTATGTGCAGCATGCGGCGAGTACTTTGTGTGGTG
TACAGCAGGTGGTGTGCGCAGGCTGTGTGTATGTATCAGCTGTGTGTGTGTGTGCGCGCGTGTGTACAGCAGGTGGTGTGTAGCAAGCTGTGTGTATGTGTCA
GGCAGTGTGTTTATGTGTGTGTGTCCAGCAGGTGGTGTGTAGCAGGCTGTGTATGTATCAGGCAGTGTGTGTGTGCGCAGCGGGTAG
TTTGCAGGGGTGTACAGCAAGCAGTGTGCATGGTATGTACAGCAGATGGTGTGCAGCAGGCAGTGTGTGTGTGTGTGTGTACAAT
AGGTGGCATGCAGTGCAGTGCATTTGTGTGCATGCAGCAGGCTGTGGTGTTGTACAGCAGGTGGTGTGCAGACAGACTGTGTGTTTG
TATGCATCAGGCCGTGTGTGTGTGTGCAGCAGGCAGTGTGTGTGTGTGTCATGCGCACACGCAACAGGCACTATATTTGTGCAGCAGG
CCATGTGTGTGGTGCATACAGCAGGTGGTGTGCAGCAGGCTGTGTGCTTGTACATGCAGCACATAGTGTATGTGTGTTGATGTCAGA
CAATGCATGTGCATGTGTGTGGAGTGTACAGCAGGTGATGTGCAGCAGGCTGTGTGTGTAGGTGTGCCTCGGGAGTGTGTGGGGGTG
GGGTCAGTCTGGCACTGGCAGGAGGTCGGAGATGCTGTGTCTGGGCTCATTGTCTCTGGGACCCCGCAGGATCCCTGTTTGGAGCTG
```

EP 2 204 376 A2

```
TTGGTCCGCCAGGAAGTTCGACAGAGTTGGATGTGGCCAGAATGGTCTGGCTGAGCTGTTCTCAGCACCTGTTGTGGTCGACCTCAG
CAGCTCCTCTGTAGCCAGGCCAGGAGGTGGGGTGCACCAGATAGGGATCCTGCCCAGACCTGCCTGCAGGAGGTCTTGGGACCCTTT
CCTCTCCCTCCCCTACCCCACCAAGCCCACAGTCCCTTCTGTCTGTTCCCACCTGGCCTCAGTGTCCAGCCAGCACCATACCACCTA
TGCCATGCGGGCCAGGGAATCCAGGTGTCTCGATGGAGTGCCTGGTGTTGCCAGCCTTGGAGCAGGCTCTGGGGAAGGGCTGTCCGG
CACATGAAGTAGTGACCAGGGTGGGGGCTAATCAGTGCTGTACTGGGCTTGACCTCTGGGTTCTGCAGGAGCTCTGACCCTGGCTG
GCTTTGGGGCTCGTGGGATGACGAAGGACCTCTTGATATACCCCCTGACCACTCCACAACACAGTCCTTTTGGAAATAGCCCTAGAG
ACAGAGGGTCAATGATAGACATATGACCCTGTCCTGAGGTATCCTGGGCTGGGCTGGAACTGGAGCAGAGCTGGTGAGGGAGCTTAC
ATTTGCAAGATGGGTACATGTGTGTTTTCTCAAGGAGTCATCTCTCCCTGCTCCACCTCCTTCCCTGCTCCAAACCTGTATATTCCT
TCCACCCATGGTGGAATGACTTGGCCCAGGCCTGACTTTGATGGCTGGGGAGTTGGGTAAAGGGCTATTGCAGGCACAAGATGGCTT
CCCAAGTGGCAAGATGTGATCCCTTTCTATTGTCTACATGTGTGTCTGGGGTAACAGTCTGCTTTGGCAGAAGCTGGGGCTGTCAAC
TACCTGATTTGCAGCACAGAGGATGTAGAAGTGGCCCCAGAACCTGGAACCCAAGCCAGCTTCTACCTCTCCCTTAGCAACTGAGGC
ATGACCTGTATACCCCTGGTCCAGCTGCTGTAGTCCTCTGTGACCTGTCCTTACACCCCCATGACTGCAGTAGATTTCAGATCCACG
CAGACATGAGGTTCTACAATCATTTCCTGGAGGTTCTAGGCCTCAGGGATGGGGATGGGAGAGGTATTAAGTAAGTTCTCCTGTTTT
TATTAGCTGTGATCTGCCTTGAGGCGCCCAAGTGGATTCCCTTCCGAATGGGTGCTCCTGTTTAGTAGGTGGTACTCCTGCCTCTTC
TGTTAGCCATGGACACTGGGCACTTCCCCTGTGCCCAGCCTGCCCCCCTGCCCCTATACTCACAGGTACTTGTAAGGCCCACTGTAC
GTGTTCCCTAGCCTCATGACCACGGTGGAGCCAATGGCTCTGGGAACAGGCTCACCAGGATGGCGGTTTAAATGCCCAAATGCCCTG
TGCATGCACATTCTCCATCCTTGGTAAGGTGCTCCCTCCTACTGGTTTGCTTGCTGGGGAGAGAGGACTTGTTTTTTTTTTTGAGAT
GGTGTCTGACTCTGTGGCCCAGACTGGAGTGCGGTGGCACAATCTCAGCTCACTGCAACTTCCGCCTCCCGGGTTCAGATGATTCTC
CTGCCTCAGCCTTCCGAGCAGCTGGGACTACAGGCACGCGCCACCACACCTGACTAATTTTTTGTATTTTTAGTAGAGATGGGGTTT
CACCGTGTTAGCCAGGATGGTCTTGACCTCCTGACCTCATGATCCACCCGCCTCAGCCTCCCAAGTGCTGGGATTATAGGCGTGAG
CCACCGCGCCTGGCCCTGAGAGGACTTTTTTGAAACGGAGTCTCACTCTGCCACCCAGGCTGGAGTGCAGTGGCGTGATCTCGGCTC
ACTGCAACCTCTGCCTCCTGGGTTGAAGCAATTCTCATGTCTCAGCCTCCCGAATAGCTTGGGATTACAGGCATGGACCACCACGCC
CGGCTAATTTTTTGTATTTTTAGTAGAGATGGGGTTTCACGAAACATGTTGGCCAGGCTGGTCTTGAACTCCTGACCTCAAGTGATT
TGCCCGTTTTGGCCTCCCAGAGTGCTGGGATTACAGGCATGAGCCACTGCTCCTGGCCAAATTTTAATCAGAGCAATGACGTGATCA
AACTTAAGCTTCAGAAAGGTCCGTGTGGGTGCATAGTGGGGGGTGGAAGGAAACCAATGACAGGTTGTTGCAGTCCAGGTGAGTGAT
GGCATTGGCGCAAATCTGGGTATGGGTAGGGGAAGATAGGGGAGCAGATCACTAGAAGGTTAGAAAGAGGCCGGCACGGTGGATCA
TGCCTATAATCCCAGCGCTTTGGGAGGCCAAGGTGGGCAGATCACTTGAGGTCGGGAGTTCGAGACCAGCCTGGCCAACATAGTGAA
ACCCCGTCTCTACTAAAAACTGTAAAAATTAGCTGGGTGTGGTGGCAGGAGCCTGTAATCCCAGCTACTCTGGAGGCTGAGGCATAAG
AATTGCTTGAACCCGGGAGGCAGAGGTTGCAGTGATATGAGATGGCGCCACTGCACTTCAGCCTAGGCGACCAAGCGAGACTCCATC
TCAAAAAAAAAAAAAAAAATAAGATTAGAAAGAGTAGAGGCCTGCCTGGCCTACCTGTAGGATTGGGGAGGTGCAAGGCTCCAGGTTC
CTGGCTCTGGGGCTGGGTAGGTGGGGGTGCCATTTCTAAGATTCATGTACTAGAGGAGGACCAGGTTTGAGGGAGGCGAGTTGGTGAG
TCCAGTTAGAAATCTGTTGAGCCAGGTGTGCGCAGGCATCCAGGGAGGTGTCAAAGAGGCCTCGTACACATTTGCCTGGCACTCCGG
GGGACATCTGAAGTTGGAATCTTACAGGCTGAGAAATAGCAAATCTCAGGAGGCCAGAGCCAGGACAGAAGGAGCCCCAGGAACCGA
CTCTCCATGTTGGGAGGAGGTTCCTGAGAGACTGGAGGGAAACTGGGAGTGTGTGAGGTCAAGAAATGCCAAGAGAAAAAAGTGTGC
GAAGGAGGAGGAGTGGTCAGCAGGGCGGGGTGCTGCCGAGAGGCCCGGCATGTGTCCTTAACAGTTAGTTCCGTGGAGCCATTGTTG
GCTTTGGTGTTGGGAGACAGCAGGGCTGAGAAGTGAGAGGGAGGCTATCAGTGTCTGTAGTGACTGGAAGGCCCGAACCTAGGGAG
TGAATGGATGACAAGAGTCTCCCTGGAGTAGGACTGAGGTTGGGGAGCCACTCGAAGGAGAGTCCAGTGTGGGCCCGAGTCTGGAACT
CCAGTGTGGGTCGGGTGTATGCGCAGGGCATGGCCCCTGGGTTCTTGTGGTGGGGAGGCCTCAACTGGGTTGAGTAGGGCGATGTGG
GGTGCCAGGGAAACCCGAGGAAGGAGGGGATGACTGGGAGGGGGGACGCCGTGCCTGCCAACAGGCCCCAAACAGCTCAGATTGAAAA
ACAAAACAGGCTTTTAAGATGCCAAGTTTGATGAAATCTGAGTGCTGGAAGAGGTGGGAGTTTGCTCTTGGAAAAACAGCTGAAAAT
CGAGACTCAAAGCTGCGAAGGGAGATGGGCCCAAGGCTCCCCAGGCCCCCCTTACTGCCTGGAAGCCTGGGGGTGGGGCTGGGCTCT
CTGGGAGAGTGTGGGAGCGTGCAGATCTGGCAGCCTTCGACTTTTGGAAGGCGTCTGGCTCTGGCCCTGCTGAGGATGGAGCTGCTG
GAGGTGGCCTTGGCTTCTGTGGAGCGGCTGAAGGGCAGGGGGAGCCAGCAGCCCTGCCACCTACGAGGTTCCTTCATGTGTCTCGTC
CCTGCATGTGTCTCCAGGCGCACGTGTTTTGGCATAGGTGTGTCTGGCTGTGCATGTCTCCACATGTGTGTTTGCGTGTCTCTGTGT
GCACCGCCTCTGGATCTGCTTGCATGCGCCTGTTTCCACAGGTGCTAGTCACTGCAGGTGCCCCTCCACATGCACATGTAGGTTTCT
ATTTTTACACCTGTCTGTTTCTCCATGTATATTTCTGTGGGTTCCTGGCTGTGCATGTTTCTATGGTGTCTGTGTTGAGAGGCAG
GGTGAGGCCAGGCCCAACACTCAAGCTTAGGGGAGGCGGTCAGGCTCACAGACGGTGCCACCCCAGGGGGCCTGTGTCTGTGTGTGC
GGGTGCGTGCCTGCGTTTGCACGGAGACGCCACGAAGCTGGGTAAACATGGGTGAAAAGGTCATACTGACAGACGGTGCTGCCTGCC
CCAGGACCCTGCAAGGCGTCTCCATCTGTCAGCTTCCCTGGTGCAGCCCCCTCCCTCTGGCCCAGAGACTCACCCTCAGGCGTCCAA
CAACACATGCCCAGCTCCCACCAGACTCGCCTTGGTACTGTCATCCCCACCACCTTCCCCCACAACAGCCTTTACAAAGGCAGTTTT
CCCCTCCTCCCTGGAAAGCTTTCTGCCTCCCATGCTCATGTGTTTCCTGTTCTTGAATCTCCCTCCTCCAGGAAGCCACCAAGATAG
CAAGTGAGCTGTGGAGTCAAACCGATCAGCTCCAACACTGCTGTGGGAGGTTAGCCAAGCGCTCATCCACTTCTGAACCTTGGATTC
CCACCATCGACCCCCGACCCTCCCCTCGCTAGGGCTTGTCATCGTCTTCTGCCCATGGGGCAACCAAACCTCTCCACGGAAGGGGAC
AGGTCTCCTTGCTGCAGTGGGTAAAAGGCCAGCGCAGTTAGGTGCAGGAGGCATTCACACACACGTGCACACTCCCCACCTTGCACAC
ATATCTGCGTGAGCCGGGGAGACCCTAGGGAATGTGTGCATGTTGTCTGCTGCATGCGGGTAGAATCCGCAAACGGTGTGGAGACT
CGGGCTCTTGGGTACCTCTGAAGGCCCCTGAAGTCCCCATGGGCTTCTCCTTCCGTCCAGGGCACCCTCTTATCAGGCCATGGCCCT
GAGACGCGTAGTGCAGACGCCCCCGGCGCTGAGGCTGAGGAGGCAGATGGCCCCTCCCCGCACTGTGCAGGGCACCCGGTTGGGGGT
GGAGGGGAGGGCCGCGTCGGTGAAAGCGGGAAAGCCTAGTGGGAGGATTCCCTGGAGCTGAGGAGCCGGGGCCTGGGAAGGGGCGCAG
AGGCTCCACCCAGGCGGGGGCGGGAAGGGCGGTGCCAGGGCGGACAGCGGACGCGCGCCTGCACGGACTCGGGCACACGCAGCCC
TTCCGCGGCAGCGCCTCCACCGTCGCCATGGCTACCGGCTGGCTGGACGGGGAGGGGGGCCCTTCCTCCCCTTCGGCGCCA
ACAGGAGGCGATTTGAGGGGACTCAGTGTGACTGGTGCATCCCCGGGGTTGGAAAATGGGTGGGTGCTTGCGACTGTCCACGTGTGGG
GGACCCTGGGGTTCGCTTTGCGGTAGATGCAAACGCCGCGGCGCGTGTGCGGGGCTCTGCAGTGGAGCCTGAGCCGTGCCGGCCGAG
GCGTGGTGTGGGGGAGGCTGCCGGCCCTCTCGCGCGCGGGGTGTTCACGCCTAGAGCGCTGGGGCTGGGGGCCTACCACCCGGTCTC
CTCCCAGCCCCACCTCCGATTTAGCTGTGTGACCTTGGGCAGGTGTCCAGATGTCTAGATCTCTCTCAGCCCCTGGTTGCCCATGTA
CCTCATAAGGGTTTGGTAAAGATTTAAGTCATTTTGTAAAGCATTTTAACATAGTACCTGGAACCTAGTTAAATGCTTCATAAATCTT
ACTGTCCCTCATACCTGGGTCTCAGTTTTCCCAGTTGTTGATGGGTTTGGAGTGATCATGTGACATCATCTGAGGAGTTGCCCAGGT
CCTCAGCCTGAGTGTTGAGGCTGCGGTAGACCCAGCTTCCGCGGGTGCCCGTGGGGGAAGGTGGTAAGTGTGCCAGTCTGGCTGATA
GATCAGTTTACACCAGGATGCCCAGTGCTCAGCCAGGCCAGGCGCATGGTGGGCGTCAGGAAAGGGCTGCTGTACTTGGCTGAGTTG
AATGTTCAGAGGCGCCTGGATGGGAGAGAAGGAAGAGGCAGCAGCAAGTCGCTCCTGAGGGGCTGGAGCCCTCCTGTAAGACCCACT
TCCCTTCCCGGGTGGCAGAGTGGCAGACTTCCGAGTGCTGCCTAGAAGCCTAGTTGGCACAGGGGACTGGCTTTTGGGTCCCGCTGT
TTTATGGACAGCTCTCCACACATTCTGGTTTTAGGCTCTGGCGGCAGTGCCTGAGGGATGATCTGAGCCAAGGACAGAGCCACTGAG
GGCGTGATAATTGAGGGGAGGAAAATTAATTGTCCTTAATTTGGCGTAAATCCCAAAGACCTTCCTCGTGTAAGGAATTCAGAGTAGA
TTCCGAGACACAGGGCTGCACACATTTGTACTTCCCTTCCCTTCCCTATCTGCGGGTGGAGATGAAGGCCACTTGACTCCTGGGCCC
TGACTCTGGCAGGCCATGGCCACGTCTTCCCCATGAGCTGGGCAGGTAGGAATGAGGTCTTGAAAGAGTTAGACTGGTGCTCTGGAG
GGCACCCAGGATGGCCCTAGCAGCCCCGAGTGTCCCCAGGTGTTGGGGAGGTGAGCCCTGCACCTCTGGTCCCCCTCAGGCCTTCCT
```

233

```
ATGGAAGCAAGCAGCAGCTGGGCCAAAGGAGGCTGATCCCCTGCCTGGTGCATCTCAGTCCTCTTCATTTCCGTCCTCCTTCCCTTC
TCTTGCCACTGTTGAACATTTTACTTTTAAAAATCTGAAAGGGCACTGTGGGATCATATTTACAGCCAAGGAGACACTGGGTTTATA
TCCAGAACTTCTAGGGCTGCAGGTGGGGAAACGTACAGCCAGGTCCCAGGAAAGGCTGGGGCGGCAAGGCCGTGCTGGGAATCCTAT
TGCTCTCTAGCCTGAGACCTCTGCTCCTCATGGGCCACAGTCCCTTTGGGATGTCCCTGGCAGCTAGAGCCTTGGGGAGCATCCCCT
GGGACTGGCAGCAGATAGATAGGTATCTTGCTCCTGCCTGTTGGGGCTCGTCCAACTCCCTCTTCTACCCGCCCCCCAGTCGCTCTC
CCTGGGTCTCCAAGAGGCTCCAGGGAGGGCTAGTTTCTGCCAGCCTTTACCTTCTTCATGTCTGAGGATGCCATGTGCCTTTACTCT
GGCATAGAAGCCTGACTTCCCTTGGCACATGTTCCCATACACCCATCTTGTGCTGGGCTTGTGGAAAGGAGGTAGAGTGGTGCTGGT
CTCCCCCACCATGAGCCCAGCTCCCGCCTTCCCCAGGAGACAGACAAAGAACACACATTCCCCTTGCCCCACATTGGGTGTGTCTG
GCATCCACACTGGGAGAGACACTCTGCTGAGGCCTTGAAAATTGGTGGTTTGGGATGGGGCCTGGTGGCTCACAGCTGTAATCCCAG
CACTTTGGGAGGCCGAGGCGGGTAGATCACCAGAGGTCAGAAGTTCGAGCCCAGCCTGGCCAACATGGTGAAACCCCGTCTCTACTA
AAAATACAAAATTTGCCGGGCGTGGTGGTGGGCGCTTGTAATCCCAGCTACTCGGGAGGCTGAGACAGGAGAATTGCTTGAACCCAG
GAGGCGGAGGTTGCGGTGAGCTGAGATCGCACCACTGCTACTCCAGCCTGGGCAGCAGAGTGAGACTCCATCTCAAAAAAAAAAAAA
AGAAAAAGAAAATTGGTGGTTTGGTCCTAGTGGGAAGGGCCTCTCACCAGCCTAGAGTGGAAAAGGGAGTTCCCGACTCTAGTCTCA
ATACCTGTCTGCCCCAGTGGCTCAGCCCTTACTAGTTACACCAGCTAATATTCACTGGGAGTGAATTCCGCATCAGATGCTCCGCAA
CACCTTGCATGCATGATCAGATACAGACCTCACAGTAGCCTTACCATCAAGGTGGGCACTTGAACCCTGTGTACAGATAAAGGAGAC
AAAGAATGAGTAACATGCCAGGCCCTAAAGCTAGTTCGTGGTGAACGTGGGAGTCCCATTTGATCTATACTAGTCCTGAGCCTGTCC
TGGACTTGTGCTTCCAAGGGGTGGAGAGTAGATAGCCTTGCCCTGCAGCCCCTCGGGGCTGATATGGGAGCCCATGTACAGTGGGAG
TGGGTTTGCTGCTGACATCTGTTCCTCTTACTCTATGCTAGTGACTCCTCTGTGTGCCGCCAACCCCTAGCAAGCTGGGAGAAGGCA
GCCAGGAGGGAGTTTTTTCTCCCCCTACCAACTTTTTGTGTCTTTAGAGCTTTTTTATCTCCTTTGCCTCCACACACTCAGGCATGG
TCTGCAGCCCTGACCGTGACTCCCAGGGTCAGGACTAAGTGAGGGAGAAAGCTCAAGGTCAAGGCTGCAGTAACGAATAGGTCAAGG
TCAGGATCGGAGTTAGAAGGGGATCATTGGTAGGGCTGGGGGTGCCCGGGTCAGGGCTAGAGACCAGGCGGTGACCTGGGGCTCTG
CCATGTGATAGAGCTGAAGGCTGGATGAAGGGAACACTGTGTGTGCGGACAGGGGAGAGGGGGCTGGACAGCACAGAGGCCTTCAGG
CTGAGCTGTGGCTGTTGGATGCTGCGCGAGCTCCCTGCTAGCCCCCCCCCCACCCCCTGCAGCCCCAGCATTCATGCAGTGCTTTC
TGCTGTGACCAGCAGAGCATTGATTCTCGTTCTTCTCAGGGCCTGGAGAGATAAGTGCTGACGCCTTCAGTCTGAGGCGTTGCCTCT
CAGACCTGGAAACTCCCTGACAGGGCAGGGGTGGGCCCCACTGCAGCCTCTGCCCTGCCAAAGAGACTTGGGACCCTGGTTCCTCAA
ATCGGGGTATGCTTCATGCTTAGAAGTCAAGGAAAAGGGGAGGGGAGTCTTGAGGGCCCTGGCCAACCTGCAGTTGGGGAGGTTACCC
CCAGAGGGGTCATAGGGGGCAGGCAGAGCCAGCCCTAATACACACATTGCTGTTTGTCTGCAGAGGAACAGCTGCCCCCTGGGTTCC
CTTCCATCGACATGGGGCCTCAGCTGAAGGTGGTGGAGAAGGCACGCACAGCCACCATGCTATGTGCCGCAGGCGGGAAATCCAGACC
CTGAGATTTCTTGGTTCAAGGACTTCCTTCCTGTAGACCCTGCCACGAGCAACGGCCGCATCAAGCAGCTGCGTTCAGGTGAGCAGA
GGGCAGGGGTCAAGGGGCCATGCAGACCTCAGAACAAGCGTCTTGTCAGATCCCAGCACAGCCTACTCCCTTGGGCCTGGGCACCTC
CAGGGCTGAGCGGAGGGTACCTGGTGGGGTGGGCTGGGTCTTACTGCAGGTGTGCCTGGCTCAGGGAAGAGAGCTCGTGGTTGGCTG
TGCCGTTACCTTCTTCGGATTGTCAGACTCCAGACTTTGGGCCAGTTCTGCCCCTCCCAGCACATGTGATGTGCCAGTGTGGTGGAC
TCTTCAAGGGTGCTCTATGGATGTTCACCCTCCTCCTTCCCTGTAGCCTGGCCTGAGACAGGGCCTGGATGATGCTTCTCTTTGCTT
CCTCAGATGGCAGGGCTTAGCTGGGAAAAGAGGCTAAAGGTGCCTGATTCATCAGGCTTCAAAAGGCTGGATCTCAGGGGCCTGGAA
CTAAGGGGACTTGCTGTTGTCCCTCGACCACCAGAGCCACCTGTCTCCTCTGGATGTCTCCGTCGAGCCAGCTCGGAGCCCTGGGAG
CAAGGATGCCATCGTGCAGGAGGGAGGTGTCACCCCATTGATCTGCCTGTGTAGGCTCCTGCCAGGATAATTGATTCAGTTTTT
GTGGGAACAGAGCAGGCGGGAAAAGAGGCTCAGAATCAGCTTGGCTGCATTCTGCATCTGCTGCCAGCACGGCCTGGACCAATAGTC
TTTGCTTCAGAAGCCCTCCTGCTAGCTATGGGATGGTTGGCCTCGGGCAGGATGGCCAGTGCCGGCCAGAGCCCCTTCTGCCTGTCA
GTTGTGATGTCAATGATGAAAAGGAGGACATGACTCTTGCCCCTTTCAGGGGCCTGCAGGCTTCAGAGGTGCCCTCCCACTCCCTGG
GATGCCAGCCCCTCCCCATCAATTCCCACCAGCCTCACAGCCCCTTGGTGCCCAGCAGGAGGAGGGAGAGACAAGCTGCCCAGCAGG
GCAAGATTCTGGCCCCAGCCACGGCCCGCCTGAGACAGCCCACGAAGTGTTAGCTCATTTAATTTAATTAAAACTCAACAAGATGGAG
GCAGCTGTAGCGCAGTTAATTAAAACAGCCATAATCAAGGCAGCAAACGGCCGGCAGTGTTTGTGGCCGCTGCCCAGCGCAGCACAG
CGGCCAGCACGGTTCGGCTCCTCTGCATTTTCTCATAGTTCCTCCAGGCAGGCTCCCCAAGCAGCCAGACGCTCCTCCCTGCTGGCC
TGGGCCCCTCCACAGAACCACATGGACTTGTCTGGCAGCAGCTCTGGGAAGGCTCGCTCACACATTGGTTCATCTAGTATTTATATA
GTGCTTGGGGTGCCCGGCCCTGGGCCACCCCTTCCTTGCCTATCACTCCACTGGATGCCACTCAGGCCCCATCCTCCTTGTCCTCTC
GGCAGCAGCTAACATTCCTCCTCCTTCCCAACCACACTCCCATTTTTCCCTAGCCTTTCAGGGCTCTGTCTCTTGCGACTTCCCTC
TTCCTAGTGCCCCTTCCCTGCAGCCAGCATCTTCCCTTCCCTCCACCAAGCCCTGCCTTTGCCCTTATGGACACGACCCTTGCCCTGGACGAG
TCACCCCCTCCTAAAGCTCCAACTGCCATCTCCTCACCTGCAACTCTAGCTTTAGTCTTTCCAGCCCAAACCTCTTACCTGAGCTCT
AGATCCAAAATTCGACCTGCCTTCTGGTCACCCTGACCAGTGGTGATCAGTGATGAGTGATGACCTTCTTTTCAGACAGCTGTACCC
TCTTTCCATAAGTGGCACCCTCCAACCTGGAACATGGGTCTCAAGTTGGTTTGAGAGCTCTCAAATGTACCACCCTCGTGGCTCTCA
AATTGGCCCCTTCTCCCCTCCTGCTGCGTCAGTCCTATCCCAGGCACCAGACACTGCTTTCCCTGCCTCCGGGCCCTCCCGAGAAT
CCATCATCCTGTAGGTCAGGTTTGCTGCTCATACCTTCCTGTGCCTCAGTGGTGCTCTCCTTGGTCAAGTGACGCTCCCA
AGCAAGGCTTAGAGGGCCCTTCTTGGTCTTCCCCTGCCCGTGTCTCATCGGGTCCTGGCTACACCACTTACCAGCTCTCTGGCATTG
GTTAACTTTTTCGTGTGTCAGTTTTCTCATGTTTGAAAAGGAGTTACAGTAAGCAGTGAGTGAGTCAATGGCAGTCAAACCTTGAGT
TGATGGCCTGGCCTCTGGTAAGGGCTTCATGGAGCTCTTTCTACTTTCTGTACCCTTGACCCTCCTAACACTGAGCTGTGCTGACCC
GTCCTTGGTCCTTGCCCACCTCCGGCCCTCTGCTCACCTTCCGGCTGCTCCACTTGAAAAACTCCACCCTGCTCAGCCTCTGGCTGC
TGCCTCAGGCCTCACCTTGCTTTACATTGTCACCATCTGACCTCTCCTGTGCCTCAGCTAGGCCTTGCAGGGAGGGCCTGGGTCTGAT
CACACTTGGTGAGGTCAGCTGTAGGACAGGTCTTCTCTGAGCCCTTGTCAAGTGAATGATTTCATGAACTTGACCTTTGGCACTTGT
CCCTGTAGGCTAATATCTGCTCTAATGTTCACTCCTCCTTCTGCTTTCCAGGTCCAAGGAAGACTTCCTTATTTGTCTTCCTGCCAC
CTGGAAGTTGTGACCTCAGGGTCGTGGGCCCAGGGTCCAGCTCCTGGGATGGAGCCAGATGGCACCTAAGGGGCCTCAACCATCCCA
CCTCTGCAGTAATAACTGGGCTCTCTCCCCTCCCTGCCTGACCTGGCCTGGGACCGTTGGCCTCAGTTGTTGCTGGCCTTATCCCAT
TACCATTTAGAAGGGTGCTAAGGCTATTCCGTGCACATTTTTCAGGGACAGCCCTTCATGGAGTGGAACTCAGGCCCCTGAGCACTCA
GCTGTTTACCGGGACCTTTACGGTTTACGCATCACCGACAGTTTACAACAGAGCTTTCCCCGCTTTTGTTGCAGCTGATTCTCTTGC
AGCCCTGTGAAGTAGGACAGGCTGTGATTACCATGCCCCCTTCACAGCTGAGTTAAGGGAGTCACTTGGGGTCACACATCGAGACTC
GGCCTAGGAGCTCCCCTGTGAGATCACCTCAGGACCTAGTATCACAATAGCAAACCTGGGGACCTGAGGAGCAGCTGGACCCTTCTG
GGGCTTCAGAGCTGCACATTCCCAGCTTCTCCAGACCCCAGGCCCCCACTGACCAGTACCCAGAAGTCCTCCACCATCTGCAACCTG
AGCCACAGCACATCTAACCAGGGCATGACCCCCCAAGTAGGGACGTGGACAGGAGGTAGCTGACGGCATGCGCTGCCCAGATGTGAGC
TCTGCTCAGCAGGCCTTTTCTTCTCTGTAGTGATGTGACATGCTGCCAAAACCACCTCCTGGAGAATTGGAACTTGAGACCGGGGTA
GGCCCAGGAGGAACAGGAACAAGCTTATAGAGTGGAAATGGAGTTGTGAGCAGGGGCTCAGAGCCCCTGCTGGGTCCTGAGAGGAGC
TGTTGGCCTGCAGGCTGTGCCGAGCCTGGACAGGGCTTGAGGAGATTCCCGCACTCCTGCTGTGGCCTGAACATATGAGCTGCCATC
CTTTGTCGTAGAGGACAGCCTAACTCACTAAGTCCATGTGCTCATCCAGAGAGCAGTCTCTTCCCCACCCCCAGCACCCTGAGGCGA
AACCTGGGGGTCTTAAGAAGAGATGCAGCATCTGGCTGCAGGAGGGAGCCCGTGGGTGGGACGCAGAGGGCTTGCAGCCCCTCACCC
TGCTGGCTGGCCCCAGCTCTGGCTGGAAGAGCCTGTCCCCACCCCACTCTGCTCTGCCATCTGCGGGCCTGCCAGGAAGGCACACT
GCCAGTGCATGCTCACAATTTCCCTTTGGCCCAGAGCTCCCTGGCACCTCTTGGACACGAATACACCCCTAAGGATGCTGACTTCTG
GGCCCCTTCAGTCCCCCACACCCATCTTGTGAAATGGAAAAGTCAGATTCTCTGTTTGGTGGGGAAATTACTGTTAGATTCTTTCAG
AATAGGTTAGGTTCTGGAAGAGCTGAGGCCAGGAGCGAGGGATGCCAGCCCTGGACCATATCCACTGCTCCCACCCCCACCAAGTCC
```

```
TGGCGTGGATGACAGGAGATCAGCAATGTCAACTTTTTGGTCTCAGGACCTCTACTTGTAAATATCAGAGAACCTCAAAGAGGGTTT
GCTTGTGTGGGTTCTTTTTTTTTTTTTTTTTTTTTTTTTTTTTTTTTTTTTTGAGACAGAGTTTCGCTCTTGGTACCTAGGCTGGAGTG
CAATGGCGCGATCTCGGCTCATCGCAACCTCTGCCTCCCAGGTTCAAGCAATTCTCCTGCCTCATACTCCTGAGTAGCTGGGATTAC
AAGCATGAGTCACCACGCCTGGGTAATTTTGTATTTTTAGTAGAGACAGGGTTTCACCATGTTGGTCAGACTGGTCTTGAACTCCGA
ACCTCGGGTGATCCGCCCGCCTCAGCCTCCCAAAGTGCTGGGATTACAGGGGTGAGCCACCGTGCCCAGCCATTGTGTGGGTTATTT
CTATTGATGTTTACCATATTAGATATTAAGACTGAGATTATTTTAAAATATTTATTAATGCATTTTAAAATAATAAAAAACCTATTAT
ATATTAGCATAAATAACATTTTTAAGGAAAAATAGTTATTTTTTTAAAACAAAAATATTTACCAAGAGGGACAACATTGGTTTACCC
TTATTACAAATCTCTTTAATTTGGGACTTATATAGAAGGCAGCTGGATTTTCTAGCTACTTTTGCATTAGGGCAATTGTGATAGCAC
ATGTCATGTAGCCTCTGAAAAACTCTACGTTCATGAGAGAAAGTGTATAAAGCAAATAATTTCTTAGTATTATGAAAACAGTTTTGA
CCTTGCAGACCTCCTTATGGACCTCCTGAAAGGGTCTCTTGGAGAGAGAGAATCATTGTGCGGATTCTTCCCACATAACCCTCACTC
CCAGGTCCCAGCCTTTGCCCTCTCCCCTGCCTCTGCATCTGCAGGACTGGCAGCCTCATCCTGTGGGCAGGCTATGAGTTAGATGGA
CCTTGTCAAGTCCTTCCACTTCACTCACAAGCTGGTGTGACCTTGGGCTTTGAGTAATTCCCTGAATGTCTCTTGGCCTCTGTTTTC
TCATTTGAACTTAGAGATGAAGAATTCCTCTAGGTGCTGTTGTCAAGTTCCATGAAACGGATTGGTGTTGATTTTTCTGCACACTTC
CCAATCCCCAGACGTGGGCAGGAAGCAGGCAGCAGGGCAGGGTAGACGTGGGCTGGACCTGGGCCCATCTGTGGGCTCCTCACTCCT
CTGGGGAAGGCAGTGGTTGCACAGGGGGCTTGAGCCTCAGAGAAGGTACCTGTTGGGGATGAAACCCGCTCAAATCTCCTAATAGGC
CTGGATATGGAGGGTGAACGCCTGGCCCTCTGGGTTACCAACCCTCAGGGTTATGAGGTGCTAGACAGGAAAAAGGGTGGGCCCTGAT
CTCTGCCTCTCGCCAGCCAGGCCAAATTGGGGAAGTTCAGTAGTCCCCCAGTTTTTAGCAGGGTTGAGTGGGCTTTCTAGGGACTCT
GTCTCTGGGCTGTGAGTTTGCTGAGTCCTGCTGCCCGTTCCTCTCGGCTGGGCTGCGTGTTGTGGGAGGCCCAGTGTGCTAGTAGCC
CATGGCAAGCCCTCCCTTTAGCCTGTGTCCTGTCTGCCTTAACACCAGGCTTTGTTTTGGTAAGTGCTTTGTTTTGGAATCTTACCC
ACCGGTGGGATTTGGGTGGCCCTGTGGGGTGGGGTTACTACCTCTTTTCTTCACCTTCCCCCTCCATTGGCCCCAGCCAGTGGGGTC
TGCAGGAGTCCTGACTCACCTCCCTGATTCTCGTCACCCCCAGCCCCACTGAGCCCCCTCCTCCTCCCCAGAGCAGAGCCAGTGACC
TGGGACACAGCTTCACTGACACTCAGTCTGGCTCTGCCTGCTTGCTCACACTGTCCGTGTCGGCCTAACTCAGGCCTCAATTTCT
CCATGTATTTAAGGCCCTTTCTTGTGTTTTATTTTACCTGATTTGACTTTGTGGCTTTCCTTCCTTTTATACTAATGCTTCTCTC
TGATCTTATTTGCATTCAAATTACCTCGCCAGGTGGTTCACCAATCAGAGGTAAGAATGCTGTCCGTGCCTCTCGCCACACCACGCC
TTGCCACTGCCGTCACCTCCACTCCATCCCGTCCAGTCTGTCACCTCCCCATCCCCATCCCAACCTCTTCAGCCTCCTCATCTCCAG
CTCCAGCACCCCCAACACCACCGGCCACCACACACATCCACTCTCAGTCATTCCCTCCTGTGGACTCATCCATTGCAAGTCTGAATT
GTGAAGATACTCGCCGGGCCCCGCCTAGGACAGCAAGCCCTGCTGCCCAGGCCTTCCAGGCCATCCCCACAGAAGGGACCCCATCAG
CTCCTACTGTGAAACTTAGCCTGTCCCCCGGCTTCCCTAGACAGAAGAGCTTCCCTTGAGGTTGAGGAGTGTGACCAAGCCTTGCCT
TTTCTCTTGACAGTCTCTTTTGGGGGGACCTCAATTATTACTTGACTTTCCCTTTAGTATCCCGGCTCTGTACCGTGGTAGAATGAG
GATCATCCCTTCAGCCTGGCAAGGATTAGGAGGAATGTTCACTTAAGAGTTCTGTGTAGGTGCTAGGATGACAACAGGGAGGTAAAC
AGGCTCGTGCGTAACTCCATCACCTGACCTGTCAGCTCGGAGTCCCCGCTAGGCTGCCTCCACGCAGCAGGGCCCTGCAGCCTAAGA
GTTAAAAGCACAGATTCTGGACTCAGGAAGATCCAGGTTCAAATCTGACTTACCACCTATGTGATCTTCAGAAACTCAGTTCATCTC
TCAGAAGTGTATTTCCTCTTTAAATTGGGTCACGGCCACCTGCCGCACAGGGTCATGAGAATTAGAGGAGAGGAGGATGTGTAGTGT
CTGGCGCAGAGCAGACACCTGTAAAATGGTGGCTTGTCTATTCACATCATTTTCTCTCCAGTTCTCTCAGTGTCTGGGCACATCTAG
ACCTTCAGAGCTCAGGACCAGGATGGTCTCAGGCAAGAGCAGCTGCCTTCCTGGGTGCTATCTTGCCCATCACCCGGAGGCTGGCTC
TTGCTTCACCTGGGAGCCCCCAATCCCCCTGCCCATCTACTCAGCCTGTGGTGACTCTTTGTTGTCCCTGCTCCTGGGTCCTGGTG
TTGGTTCCAGATTTGGGGATTTCTGTATGCAAATAGGCACCCAGTCTCTGTTAGGCCCCCTGACCACTCTTAGGCTCTTGTTGCAGA
GGAACCTCCTATTTTCTGGGTCAGAAATTTCACCCAGGAACCACTTTCTGCCCCAAGCCTGTGGCACCAGCCACCAGGTCTCCCTCA
CCCCACCCACACCCCCTGCCTCACTCAGGTGACCTTAGGGCAGCCTCGACCCCTCAGCGGCCTGAACCCGACCTGCTTGAAGGAAC
ATTTTCTGTACTAGTGTCCATCCATCCCATCAGCATGACCTGTTGGGTGCCCATCAGGGCTGCTCTTAAAGGAGATGGTATTTGGCA
TGTGCACTGGGCCCTGGTGCTCCCTGGCATCTGGTGGGGAGGGCTGAGGCCAGCACCAAAGAGGCAGGCTGGTCCTGCGTCCTGCAT
TGGTGGCCTCCCCTCTTCCTTCACCTCCAGCTGCCAGCCCCCTGGGGGTTCCCAGTCCCTCCTGCTGCCCTGGGTTAGATGGTGGAA
GAGGGACATCAGGTAGTGAGGTGGCTATAGGTCAGACCCAGGGTGGTACCCCTTTGGGCCCCAACCGTGAGAGCATGACTTGGAACT
CTTCCTCTGGCTCCTGTCCAGTCCAGGCAGGGGGGGCCCGGCAGCTTCTGTCCATGTCCTGTGTGGGTGTGATTGTCCATCAGCCTG
GGCCCTGGGAAGGTGGGGGCTGTCAGGTTAGGCTGGCTGTTCTGTGCAGGAGGTGGTTTTGTCAGACTGTGTCCTGTGGGGGTTCTT
AACTGTTGGACCGTCCTGTGTGGGTGTAATTGTCTGTTGGATTGTTCCAGGGAGGGGTGTGCTGCCTGTGCCCGGGGGTGTGTCTGT
CACAGACAGCTTGCCCAGTGTGCTTTCACAAGCAGTTTTTCAAGTGTTTATTGGTGGGGCCAGGGGACGGCCAAGTCCAGTGGATC
CACCCCCACCTTGCCGCTCTGCCTTGGCCTCCCCCGCCAGGACCTGTCAGAACTGGCCTCAAGCCCAAGATTCCTCCACCCTTTCTG
AGGGTCCTTTTGCTACCCACCCCGATCCTGGCTCCTGTCTGTCCGTCTGCGGCCAGAGCAGCCCCTCAGGGCACTGGCCCGAGTGAG
AGCTCCAACCTCCATTAGCCTCTTGAATTATGCAGGAGCCGTGGTGGGTCGAAGCACTTTAGCAGGACCGGGCTGAGGAGTGGAGCT
GGAATGGGTGGGGCTGGCGGGGAGGGGGTGGAGTAGGAGGGGAAGATGGTGCTGGGAGCAGAGGCTGCTGGGCACCAGGCCCCCAGG
GCAAACCCATTCCTTCCTGGTGGGCGGCAAATTCTCAAGCTCCAGCCTGATGAGGGCATGGCGTGCTTGTGAATCCCAGGCCCGCCA
GTCTGCAGGCTGCTCCCACACCTGGCTCTGACTGGCTTAGTGGGGCTGCGGGGCCAGGAACACTAACAGGGATGTTGATTGGGCAAAT
AGGCTCCAGTACTGGCAAGAGTGTGGCAGAAACTGAATGGAGCTGGGGGTTGGGGTACAGTCCCTGGCAGAGCAAAGGTGGGTCTTG
CTGGACTGTGGGGAGCATGAGATTGTGCTCCAAAGCACAACCGTTGGTTCTAGACAGGAGGCCACTGGGCTCTTCAGGGAGAGCTTC
CTGGAAGCAGCAGGGGTGCCAGGGTGTGGCCTGGATGAGCTTCGACATCTGGTCAACATCAGCCACAGATGCTGTCGAGACCTCTCA
CCGTGTTCCAGGCCACACTCGACATGGGCGTGCCTGCCTCCTCTGCTTCGTGTACCCACAGGTGCCTTGCAGATAGAGAGCAGTGAG
GAATCCGACCAAGGCAAGTACGAGTGTGTGGCGACCAACTCGGCAGGCACACGTTACTCAGCCCCTGCGAACCTGTATGTGGAGGT
AAGGACTCAGGCAGTGCCTGGCCCCTGTCACCACAGAGCTGTGCTGCACCTGCCGGGCTCTCTGCCCAGAGCCCTTGGTGCAGACAC
GCAAGGGACTGCCATGGGCCCAGTCTCTTCTCCTTCCTGCTTCTTTCTGCAGCAGCAGCAACAGCTCCCACTGGGCAAGTTCCTGGC
GTCTGCCACTACTTCGCCTTCCTTCCTTGCAGGCCCATGGGGAAGCAGCCACTCTTGGGAGCATTTGTATCTTTTGTAGGTCTTGCC
GCATGGGCCCGGAGCCCCATGGGAATTTGGAGCCATCCAATCCGACTTCTTGGTGTATGTGCATGTGTGTGGTGCACACACGGGCACA
CTTATCTGTGTGTAAATGCATGCATACCTGGCCTGGCTTTCCTTCAGTACATCCTCCTGCCTGCCCGCAGCATGGTGGCCTCCAGAAC
CACCATGGCTCTGGGCTTTGGGGTAGTAAGGCCTCAGGAACGGACCAGGCCAGGTGAGCCTATCCTCTGGCCAGGTCTTCCTGGAGC
CTTCCTGGAGGAACCCTTGTGTTCAGCTGTGAGCCCCTGTGGTTATGTTGCCTCGGGTGAGCACCTGGTGTGTTCCAGATGTTCTTT
GCTGGGAGCGAGTGGACAGTGTGCTTTCTGGAAGTCCAAGTTCCCAGCAGAGAGGAACCTGGGGACTGTTTTGGGGTTTCCAATCTC
TGCTATGCTCACAGCCTAGGGGTGACTAAAAGCTGGCCACCTTGCCCTTTCCTGAGAACACACTGGGTCTGGTATACATGTGCCCAGA
AGGGGCCCTGAGAGCCCCTTCTACCCTCATACTGTCCCTCACCTAGCCTTCTTGTACAGATGGATGTCATTAAAAGTTAAACTGTT
GACCGGGTGTGGTGGCTCACGCCTGTAATCCCAGCACTTTGGGAGGCTGAGGCAGGCAGATCACGAGGTCAGGAGATCGAGACCATC
CTGGCCAACATGGTGAAACCCCGTCTCTACTAAAACTACAAAAAATTAGCTGGGCGTGGTGGCGCGTGCCTGTAATCCCAGCTACTCA
GGAGGCTGAGGTAGGAAAATCGCTTGAACCAGAGGTTGCAGTGAGCCAAGGTCGCGCCACTGCACTCCAGCTTGGTGAAAGAGCAAG
ACTCCATCTCAAAAAAAAAAAAAAAAGTACAACTGTCTCCACCCAGGGGATTGTAAAGGAAAGCGGACATGGCTCCTTGCAGTGATGA
TTCCATCACGCCAAGTGCTTAGCGCGACTTCCTGAGGGTGTCGTGCACTCTCCACCCAGTGCACGTGGTGGGTGATGGCGGTGGC
CTGTCCGACTCTTTGGGGCCCATGTGGTCAGACTTGCTTACTCAGAGCAGAGAGAATTAGGCTGTCAAGGCTGTCAACCAAAATACAC
TTATCAAGGAGTTGCTGTCTGTCATGGGAGAGGGTGGTTCTGGAGAGGCCACAGGCTGCCAGCCCATTCGGTGACCAGTTGAGCTGC
AGAGGAACTATTTGTGGTGGGCAAATTGTGCTTCAGAGACTATTAAAGATGCTTCATGAGAGTTGGGGGCATGGGATGGGAGGTCCC
```

235

```
CTAGCTTACTGTCAGGTGTCTTACTCCTCAAGGCTCAGGCCTCAGGTCAGCTTGTCTGTCTTCAGGAACAAGAGCCTGAAAACTTCC
CAAATGGCCTAGAGGAGTGGAGTTGTAAGCGGCACAGCCTTGGCCCCACCCTGCCCCAGGCAGAGTGTCTGGCAGGCATTGGGGCTG
GCGAGTCCACTCCAACAGAGGAGCAGCTGGAATGACCCTTCTCAAAGATCTGCCTCCTCCCACCCTGACTATCGCAAATGCTGTGCT
CTCTAGAGCTCTGCTCACAAGACTGTAGGGTCGAGAGTGGCAGTTCCAGGCCTCAGCAAGAAAGCCTTTGCATGTGGCTGCACCAGG
GAGAGGAGAGGCCCAGTAGAGCCACTGTAAACCCAAGCCAGCTGCAAGGGCGTGGCCTATGGGCACATTTCCCAGGGCACAGCTACA
GGAGGCAGTGCTGAGGCAGAAGAAGGCCCAGAGGTGGATGGACCCCCGCATTGTCCTCTTGACTAGGCAGCATTCTCTGAAGTCACT
GTTACACTGGCCCCAGCCCTGGAGGCAGAGCAGTGGGTCTCTTTGGAGATCGACTGAGCTCAGCTGCTGTAGCTGGCAGCTCAGTCA
CTGCCCAGGGAGCTCTTATGTGCAGCCCCTTGGAGCATCCCTGTCTGTGGGGCTCCCTGTTTACACATGGTAGACTCAGGCAGGGCC
CTGACTGGCTCTTGCCATGGCCCAACCCCTAACTCTACCAGCTCTTCTTGAATCTACCCTTCTGGCTCCATCCCCACATGCCTTTTG
GTAGGAAATACGGGGGTTCCTTTAGTTCCAGCCCCACTTGCCAGGGACTCCAAAACAGCCAAACCTGACTTCTGTTGTCTCTCTCAC
ACACACATACGACGTTCCTCTAGGCCCTGATGTCTCAGGACGCGTTTCTAAGCTCTGTCCCCAGGACCTGCCTCATGTAGACCCTAAT
TGAATCTACACCACCCTGCTTTGCCAAGAGCAACCTGCCCCATAGGCCCTACTGTCCTACTGACAGGACTTAGGCACACCTGAAAGG
CCCTGCTCCCTGGGTGCCTTGCCTCTGGTGCCCCCACTGCTCGTCATCTCATGGTTGGCAAAACCCCTGCCTTCTGCCCTCAGCAGT
ACTAGGGGCCCTTCAGCCCCAGCGCCTGGGGACAGCCTGTACTGGGCTGACCACCAGCTCTGCATCCTGAGCCCTGCTTGTGGGTGG
ATGGCCGTGGAGGAGTCCCCAGTGCCAGCCTGCTTTCTGGCCTGCACCCCGTCCCATCTCCCAGCAGAGAGAGGCTTCGAGGTTTCCTC
CCACAGCAGGCTCCTGAGCCTCAGACGGCTTGATGTGGGCTAGCCCTGAACTTCTCAGCCGCAGCACTGTTGACATTTTGGGCTG
GACGATTCTTAGTTGTGAGGGGCCATCCTGTGCATTGGAGGGTGTTTAGCAGCATCTCTAGCTCACTAGATGCCAGTGGCACCCCTC
AGTCAAAACAAACGTTGCAACGTGTCACCTGGGGGAAATTACTGATGGCTTCCCACTGCCATTGTCTCCACCCATCTGACTCTCTTC
ACCTGTGTCCACTGGCCGCAAGGCTTCGTCTCCTCATCAGACTTGGCTTCGGTGTCTACCTCACATCTCCCTTCTGTCCCCCAAATT
CTCAGGGCTCCCAGTGTCGCTCTGGAGGCCACGCGACTGCTACATTTAATGGGACAGTACACGTGAAGCACCAGGCACATTGCCAG
GCACACAGGAAGCACTTGCTAGTCAGTAGCCTCGCAGCTAGCACTCGGCTACTAGTCAGTAGCCTCTCGGATAGCACTGTGGGGGG
ATGTGTCATCCAGTTACATCTGACTTTGTTCACAGTTGCCTGCAGCTCCACCCACAGTCTAGCAGGCCCAGGCATGCTGGGTGGGCC
AGAGCCTTTCTCCTTCACCACCTGACTCTCCCTGAGTGACTCATTCTCTCCTTCCATCTACAGCTCTCTGGGTGTACAGCTGCTGGG
GCCAGGGTGGAGCCCTGCCCTCCTCAGAGCCTGGCCTACCTGTGCCAGGACTACCAGCCTTCCCCCTTTCTCTAGGGACCTGGCTGC
GGGGCCCACAGCTGTCTAAAACAGGGACAGTGCCTTTTTCCCCACAGGTGCCCAGACATGCTCCTTACACCGGTGGTGTGTGTGGGG
TGGCTTCTAGTGGCTCCTGTACCTTGGCAGGTTTGTGGGCTGGGTGGGCCTTGACCCCAGAGCCCGGTCCACAGGGTCTGTCTGAGC
TGTGGGGTGCGTGTGAGGCATGGGGGCCTGCCTGTGCCCCATTTTCACCTGCCCGGCCCCACCCTCGGCCTCCCTGGCGCCTGCTG
GCGGGCCTCAGCCCTGTCCACCATGTCCTCCATGAGTCCTGAGTCTTTTGTGAGTGATGTGGTTCGTGTGCACCTGTGTGCATGTGT
GTGTGCGAGGGGGCACAGGAGTCTCGTCTCGTCTCCGTGCTGTGTGGGCAGATGAAGGTTGGCCTGTTTTTACTCTCTCTGTGTTTC
TCCTTGTCTTTTTTTTATTCCCTCCTCATCTTCATCGCACTCTGCCATCAACCCAAACTCTCATCTCTCAGATCAGCGAGAAGGTTG
GTCCTTTTCACTTCTTATCCATCTACAGTTCGCCCATCGATGGGACACGCCTGTCAGTAGGGCCCAGCGGGCTCGGTCAGCTCCCTC
AGGCTCACTGCGCCGTGCCTGCCTGCCAGTCTCTGTTGTTTGGGCCGGCGGGCAGGCAGGACCAGGGATGGGTGGGCAGACCCCTGA
CCTGCATGTTCCTGCTGCTTGGGTCCCTGGTGCACCACGTGTCTGCATGTCCCCTTAGCCTGGTTCCCCTGCAAGGGTGGTGGGGTG
GGCATGCTGCATGGCATGAAACTGTACCCCACCTTTGCACCCAGGCCGGGCCAGCTGTCTGATCCAGGCGGTGCTCAGGAATGGTTG
TGGGGGGCTGTGGTCAGAGAGAGGTGTAAGACCTGTAGCGCTTTGTGTGCACCTGTGGGACCTTTCACCGCCCTCCTCGCCTAGAGA
CCCAGGCTCAGCCGCGAAACTACTGGAACATTAAGGCGAGAGGCTAGAGCAGGCTGTCCTCCCAGTATCCTGCAGCACAGGGCAAAAGT
CCTGCAGTGTGTACCCTGTCCTTGAGGCCCTCAGGTCCCCCACCAGGGCTTAATGGGGCAGTCAGCCCTCGGTCTGCCTCAGGGAGCT
TGGTGTCCATGGCGCAGGGTGAGTAGCGTCATCTTGGCACCCTGAGGAAGTCTCCCATAAGGTTGTCTTTTCCTTCTCCAGCCAGAT
CCAAATTAAGATCCTCCTACCTGCCCCAGGCCCCTCTGCCCAAGCTTCCCAGCTAGCTCCCGTGTCTGTTTGAGGGTGGAATTTAGC
CCACCAGCCAGCTAACTGACATTCCTTAGCTTGATGCTTTGCATTCAGACTCAAGTTGCTTCTACCTGGAGTAACTGAACGGTCAGC
TGCAACTTAGCAGGATCCATATGGGAGCCTGGAAGTTGGCCCCTGACTCAGCCACAGGTTAGGAGGGTTTCCTGGAGACTGTCCTTG
AGCCCATTCCAGAGCAGGTCTGTTTGGGGGCTTGGTTTTCCGCCCCACCCTTGGAGCCAGACCCCCACCACTGATGCTGGGGGTTGGCA
GCAGGGGCTAGTGGTCATTCATGTATCACATGGTCTGTGTGGTCAGAGGAGACTCACCTGTCCATTGGGTTTTGGGGGGTTCAGGATA
GCAGTGTAGGACAGGCTGAGCCTCAGGGAGCAAACCCAGGCCATCCTCGTTCCCTCTGCCACATCCTCCGTGGCTTGAGTTTCCTCT
GTGAGGGTGAGGGAGCCGGTCGAGAGGAGGGCTATAGCCTAGGCTCAGATATCAGCCCTGAGCATCCATCCATATGCACAGCTGAGC
ACTGGGGCATTTCTGTTACACCATTTTGCTGACCGGGCAGCTGCCGAGGGCGTCCAGAAAACAGTCATCAGCAGAGACAGCCCCAGC
CCAGAGTGGCTCCTTCCATGCCCCTTCCTCTACCCTCCTGGTCCCTCGTCCCCTCCCTTCCCCCACCCCACCCTATGGTCCCACCAA
AAATAAGCACATGGACAAATATTTAGCGGGAAGGCAGACATGAGTTTCTAAATATTCCAGGGGGATTTGCCGGGCTGGTAATTTGTT
TGGTGCTGATAATTGCATCTTACATTTTTCCAGCTTTACTTAAAACTGTGTGCCGGGTTTGCCGGCATTTAATTACTGCTCTGCGGC
AGCCACAAGGTTATTTATTAAAGAGTTATTTTATCTTGATACAGTGGATCCTGCCCCTCATCCCCTCCTTAATGTTGTGTTATTTTC
ATCAGAGAAATTTCTCTGAGTGAATGCAGATTGCGGGGCTGCCTCCCCCTGCGATTAGGCTGTCACTCTACTGAATGCTGATGCCTC
TGGGCGCCGCACCGCCCTATTATAGGGGGTTGTCAGCGCAAATAATTAGACTTAAAAGTTACAGCTGAAATATAATCCAGAAATGGC
AGGGGCCCTGTTTTGGAAATTGTCTATAAAATGTCAGCACTAAGGATGCACAGGGAACGGTAATATACTGGCATTGTTGGAGCCTAAG
ATTAGACCCTGAGTGCATTTTTCCCAGCTCCAGTTTTTCTTTCCCTGCTGTTGCTTCTGTCAATAGACTGTCCAGGGTGAGGCCTGT
TCCTTTTTGGAGGCCCTGTGCCTGCAGCAGTGGGTGGAGGATGGTTGGGGCTGCTGCAGTGAGATGCCAGCCACTCCAGCTGTTGGC
ATGGGGGTGGCTGTCTGGGCAGGTCTAGGATGCGGGTCCCTGGTTGGCAGCTTGGAATACAAGGAGGTTGTCCCTTGCCTGTTTCCCCC
ACCCTTCCTCTGTAGACATACCCTGACCTCCCTCACATGGAAAATGTCTCAGTCTCAGTAACGTTTCTGCGTGGCTGAAGCCCAGTG
TCCTTGTGAGAGTGAAGGTGGTGTGCCCAGGAGCCTCTGACCTGAGGCCCTTGCTTGGTGTTGCAGGTGACCCTAAAGGCCACTGT
TGACCTCTCACACTTATGCCTAGCCCCTAGAGCTCTCCCTGCCCCCTGCATCTAGGAGGGAAAAAATAAACACAACTCTTCTGTGAA
GCGCCTCAATTTCCTGACAGCCGCAGGAGTTGGAGGCTTTAGAAGACGTGGCTCAGGGCGTTGGCCTGACCTCCAGGGCTGATCCAT
GAAGTCCGCCTTTGGCCTGAAGACCCTGTCCTCCAGGGACAATTTGGGGGTAAAGTCCAGTCAAGTTTCTAGGTTGGTGCCCAAGG
TTTATTGGCCAAGGACCTACTTCCTTCTGCTGGGGCTCTTTGTCCTGGTATCCAACGACGTGATTAGGCCCCATGATTCCACCACCG
ACTGGGGTCAAGCCTGCAATCATGACCTCTCCCTGGCACAGGCTGTCTAAACCCATCCTTACGCCCGCTGGAGCAGCGTCCTCTCCT
GCCAGCCTCTGCTAATTCTGATGCTTCTCCCCAGAGCCTTCTGGGTCTTTTTTCAGGCCTGTCAGCTAGGGCTCGAAAGGGTGACAG
TTTTTCATCACACAGACCTACTCCTACTACGTATATAAACTTGGTCATGTGGCTTGGTTTTAAAACAGTTCCCTTGACTGTAATTGG
CAGGTGATAGCATCTACAGCATGGAAACCTGCACCTAGTAGGTGCTTAAGCATAGCTTCCCTTCCCATGCAGGAGGAGGAGACAGGGC
ACCCACTGGCCGAGAGGACAGGAGAGATTTAGTTCATTAAGGCTGCTCCTCTGTGCTGCCCCCACCCCGCTTCGTGTGGCAGGCCTGG
ACCTGATCTGCAAACAGACTTGCCTGCCTGTGCCTGTCCCTGAGGCCCATCTCCAGACAGAGGGAGGGCATGCACCGCTGGGCTGG
GTGGTGGTCCTGGCTGAGCCTCCTGCTCTCACCTTGGACCATTTGAGGTGCGTGCTCAGAGAGTCCTTTGTTGCCATGAGACACTTG
CCAGCCATGCCCCGGGATCCCACCTGTTCTGCCACAAGAACTGTGTCTGTGACATGCTGTCATCATTGGTAGAGACTCCTGGCACTAG
AACAGATGACAGAAACCCACTTATACCAGCGTAAGCAAAACAAGGAAGTCCTGTGTTACAAATCTGAAAAGTGTAGTAGATTTGGTG
CTGGGCAGGGCTAGATCCAGGTACTTAAACAGGTTGGCCAGTCATTTGTCTGTCTGTTTCTGAGCTCTCCTCTCCTCGTCGACATC
TTTGTTCTCAAGCAGCTTTTCCTGTGTGTTGGGAGAAGCCATCCCCAGCTGCTCTAAGCTTAGCTACCTCAGTAGAGAGAGAAGGTC
GCTTTTCTAATAGTTTCTGCGGAAGTCCAAAGGCTGTTTCTCCATAGTCAGATCTGGGTCACACACCCCATGTATGAAGAATGCAGG
TAGAAGTGGTTTCCTTAAAGACAATGGGAATGCTCATTGATTTGAGGAGAAAGCAGAACCCTGAATGTCTACAAGATACTCTCATGC
CCTAGGTGAACCTGGGGACACAGATCCAGCCCACCAGCACCTGTCAGGCCTGGTCTCTGGGGCTGCCAGGGTTTGGGTTCTGTCTGG
```

```
AGCACCAGAGTAAGGCAGGCTGTGGAAGGAGTTGAAGCAAGTGCTAAATTAGGTAGGGTCTCTTGGGCTGGGCAGAGGAGTCACTGG
GAGCCATGCAGGAGCTTGTTAAACAGGGAGTGGCATCAGATTTGTGCCAGCTGTTTGTGGAGGGCCCGTGAGCAGGAATTGGGGCAG
TGGACAACAAGGCAGGAGGACTGACGCAGAACACATTGCAGCAACTCAGGGAAGGATGATGATGGCTTGGACTTTGGTGGTGGCAGT
TGCCTGTGTGTATGGAGAGAAATACATGGATTTGGAAGATATGTTTATGCCACGCTGTTGACAGGACCTGGTGACTTGCTGAATGTCT
GATCTCTGGGTCTAAGTTTTGACCCCTGTATGGTGAGGTCAGACGTTGGACCGGGAGGTCTCTGAAGTTGCTTCTAGCTCTAGTTCT
GTGATTTGGGGCTGTGTGAGCAGCATAGTCGGTGCTTATAGTGGAGATGCTCTTTATGTGGTGCTGTGGGGTCTTTGCATGCCTGG
GCCCCCCAGCGAGGGTCCTCAGAGACCAGTGCTCTCAGCAGTGCTCTTGGGTACTGACTAGCCTGCTGCCTGCCGCCTTTGGCCCTG
CGTGTGAGAGTGCCCGTGTAGGACACACCCTGGCCTCTGCTCACCAGCTGCATGGCCCATCTGCATGCCAGACATGCCTGCCCACGG
CTGGGTTTCATCACCTCCCTTCCCTCTGCATGCTCCTGTGTCTGTGTCAAGCTTCCAACTTCGGGGGCGGGTGGTTCTAGGCTGTAC
CCTGGACTGAGAAGCTCAGAACAGCTTCTGGTCAGGGGAGTCCAAGGCTGGAGGTCGTGGCCAGGCACAGCCACAGCCACCCCTCCC
CTTGAGACCCTTGTTTGTTGGCGCAGGAAGCCGTCTGTTCGGCGCCCTCATCATGTTACTGCCATCGTCATGCCCATCCTGCCCGCA
GACCATGTCCCTGGCCTGCCCTGACCCCTGCCCCTCCCCAGTCCTCCTGAGGGCAGCCCTTTTGCTTGGGTTCCCAGGTTGTGCCCT
GAGCATGGCTGAGACCCTGGCAGGCGGCCCTGCTGCTTGCCTTATTTCTTCCCCTCAGGCCATGGCCTGGAGGTGGAGGCAGTGACT
CCACGGCAGGTGGCTGTGTCCCTCTGGACTGGCCTTCTGCTCTGCCCAGCCATGCTCTGAGGACCCTCCACCCTAGGGCCTGCTTTC
TCTCCTTGGTGCTCCAGCCAGGAGCAGGCAGGCAGAGATAGGCCCTGGAGAACACCCTGGGTTGTGGTCCTGACCAGGCCTGGACCTTGTA
CTGAGTCCCTGTGTGACCCTGGGCAGACAGGACCTTCCTGACAGGCCTCAGTTTCCTAGGCTATAAAATGGGAGCTTGGTTGCAAGA
TCTCTCATTGAGTAAGTCATCGTGCTCCAGACAGTCCCTGAGTGTGGGGGCCACTTCTAGGCTGGAAGTGGGTGGGGTGGGAGTGGA
TGATGAGCTGGGGTCTGGCGGTGCCACCCCTCCTGTCTCTGAGCATGGGTTTGGCTCTGACCAGAGGGGCTTCCTGAATGAGGGGCC
CCTGCCCCTTCCATGCAGTCGTGTGTCCTGCCCGGCCTGTGAGTGCCTCTCTCCCTCCTCCTGCAGTGCGCCGCGTGGCTCCTCGTTT
CTCCATCCCTCCCAGCAGCCAGGAGGTGATGCCAGGCGGCAGCGTGAACCTGACATGCGTGGCAGTGGGTGCACCCATGCCCTACGT
GAAGTGGATGATGGGGGGCCGAGGAGCTCACCAAGGAGGATGAGATGCCAGTTGGCCGCAACGTCCTGGAGCTCAGCAATGTCGTACG
CTCTGCCAACTACACCTGTGTGGCCATCTCCTCGCTGGGCATGATCGAGGCCACAGCCCAGGTCACAGTGAAAGGTGAGTGTGGCAG
GTGCTGTAACCAGTGCCCTCCCTGTCATCTGGGAGGTCCTGGTGGTGGGCGAATGTGAGCTGGCTGCCATGGGCACAGGCATGGCTG
AGGGATTCTTGCCCTTTCCTGGGTGTCCCTGCCCTGGGGTCCTCCAGCCCTTAGAGGGAGGGAGGGATTTCTGTTATTAGCCGGCTT
AATGATAATAGTTAAGGCATATTGAGTTTCTTGGTGGGAGCATCATGCTAGCAAGCACAGTTGATTTTGTTTTTTTCTGTTTTACC
CTGGGAGATAGGTGCTCTTATTATTTCCATTTTTGAAACGAGGGAACCGAGGCACAGAGAGGGTGTATCACTTGCCCCAGGGTCACA
TAAAAATAAATGACAGAGCAGGGACTTAAACCCAGTGTGGTCTGAATCCATATCTCACCCTCACCACTACATAGTACCAGACCTTCA
GGTTTAATAGCCTCCCAGCAAGAAGGGTGTGGTAAAATAGTAGGGGAAAGTGTGTTCTCTCTGCCCTGATACCTGGGGACAGGCACA
CATGTATTACACATATGTCACATATATGTAACCGATTCTGGCCAGGCACGGTGGCTCATTCCTGTAAGCCTAGCGCTTTGGGAGGCC
AAGTTGGAAGGATTGCTTGAGCTCAGGAGTTTGAGACCAGCCCAGTTAACGTAGTGAGACCCCCATCTCTACGAAAAAAAAAAAAAA
AAAAAAACTGGGCATGGTGGCACACACCTGTGGCCCAGCTACTTGGGAGGGCTGAGACAGGAGGATCACTTGAGCCCAGGAGGGTGA
GGCTACAGTGACGCAGTGAGCCATGATCACACCACTGCACTCCAGCCTGAGTGACAGAGCGAGACCCTATGAAAAAAAAAAAAAACCA
GATTCCTTCCTGGTCTCCCCGCTGCAATTCTCAACAGCCTCTGATCCATTATCTGTTTTGCAGCTTGAGTGATCTTTAAAAAATGTA
ATTCAGGATCAAGTGACTCCCTTTTAGGAAAGGGAGAAACACCTTCCAGTGGCTTCCCGTTGTTTTAGGATACAGATCAGGATCCTC
ACTGTGGTCTCCCAGGGCACTGGCTTCCCACCCTGTCACGGCCCAGCTGTCCAGACACATGCTGCTTTCCGTTTCCGTTTGCCGTGCTCCTT
GCCTGGCCCCTTCTGTCACTGGCTCCATGTACCCTTCCAGGCCTCAGCTTGGTGATGCTTTCCTTCCCTCATCACCGCATCCAAGGA
GATTCCCTGGATCTTACTCCACCTCAGCACCCTGATTCCTGCTGTCCTTGCACATGGCAGAATCATGAGTACGATCGACTTGTTTAT
TGATTCTCTCTTGTACTGAGGTGTAAACTCCATGAGAGCAGGGATGTGACTGTGTGGCATTGTAGCCCCAGCACAATGTCTGGCATT
GAGTGGGTGCTTGTTAATTATTTGTTGAATGACTGTGGACTCAGGACCTTTCCACTTCAATTTGAGCCAAGCTGCAGGGTCTGTGGG
GCCAGCAGCCCCATCACTCTTTCTATCCGGGCCAGTCCCTAAGGAAATATCTCCCCTTCCCCTGCCTATTACACATACTTTCCACCA
GGTGGGCTCAGGTGACCTGCAGAGGCACATAGCTGCTACCCAGTCTGGGTGTCTTTCATCTACATGGGACTGCAAGGGTCACATCACC
CTCCAGGTCTGTTTGTTAGATGAGTCTGGTGTGCTGATGGGAGGGTCCAGTGAATCCCACAGTTGATATGTGTACATACTAAATGAC
CCAAGGCCCTGGGTGGGGCACTGACAGGCCTGGCCACTGTCCCTCACTTGTGCCTTCAGAGGTCACCATAAGCTTTTCCAGCCATTT
TTCAAGGTAGGCTGTGGGTATCAGCCACACTCAGGTGACCCCACCAGATATCCTGGATAATCCCCAAGTCTAGGGTTGGTTCCTAAG
GATCTTGACCTCGGGCAGCTTTGAGCCTTCCACTTTGTCTCCAGCTCTTCCAAAGCCTCCGATTGATCTTGTGGTGACAGAGACAAC
TGCCACCAGTGTCACCCTCACCTGGGACTCTGGGAACTCGGAGCCTGTAACCTACTATGGCATCCAGTACCGCGCAGCGGGCACGGA
GGGCCCCTTTCAGGAGGTGGATGGTGTGGCCACCACCCGCTACAGCATTGGCGGCTCAGCCCTTTCTCGGAATATGCCTTCCGCGT
GCTGGCGGTGAACAGCATCGGGCGAGGGCCGCCCAGCGAGGCAGTGCGGGCACGCACGGGAGAACAGGCGCCCTCCAGCCCACCGCG
CCGCGTGCAGGCACGCATGCTGAGCGCCAGCACCATGCTGGTGCAGTGGGAGCCTCCCGAGGAGCCCAACGGCCTGGTGCGGGGATA
CCGCGTCTACTATACTCCGGACTCCCGCCGCCCCCGAACGCCTGGCACAAGCACAACACCGACGCGGGGCTCCTCACGACCGTGGG
CAGCCTGCTGCCTGGCATCACCTCAGCCTGCGCGTGCTTGCCTTCACCGCCGTGGCCGATGGCCCTCCCAGCCCCACCATCCAGGT
CAAGACGCAGCAGGGAGGTAGGTGGGGGCATGCCGGCTGGGCAGCCAACAGCAGAGAAGGGGAGGCTGAGGTTGTGGCGGTGCCTTT
CCCCCTCCCTCGGCTGTGAGGCTGGGGGCTCTTGGGAGGATCAAGGTGCCGTATTCCATAGATGTGTGGTCAGTTGGGATGTAGGAT
AAGGGTGTGAGGTTAGGACCTGACTTCCTCGGCTCCCTCCTCCCTGGGCACCCCTGACCTCACGCAGATGAGGCTGACCTGCCTGGT
GTGGGGTGTTGCAGTGCCTGCCCGCGGACTTCCAGGCCGAGGTGGAGTCGGACACCAGGATCCAGCTCTCGTGGCTGCTGCC
CCCTCAGGAGCGGATCATCATGTATGAACTGGTGTACTGGCGGCAGAGGACAGAAGCAGAAGCAACAGGTGTGCAGCGGGCAGAGAAGCAC
TGAGGGGGTCTCCTGGTCCCTGAGGGTCTGTGATGGGCTTAACCCAGGAGGGTATTTTCTGGATTCTGTGGCTTATGTGGGCACAGC
CTCTAAGGTTTCTGCTGGAGGCTTAGTGGTGCATGCGTGTCATGTGCCCGCACAGCCTGTATGAATCTGGAGTCATTGTCACCCTG
TGCTAGGGGCAAAGTCCCCAGGGTCTGCCTTGGGTTTCCTAGGCTCTGTGGCTCATATGACTGGCAGAGCCCTGAAGTTTCCCCAGA
AGCCATGCGTCCCAGATGGCTCCTGTGGTCCATGTGGTCTGTGAGGTGTCTGTGGCATGGGCTAAGCCCTGAGTTCCTTTTGTGCTC
CATGTGGCCTTATGGTGTGGACGCCAGTCCTGGCAGCAGCTGGTGGGTCACATTGCACCCATGTTGGGGAACAACCTGT
GAGTGACTTTGAGCTCAGAGCTGTCAGTGAGTGCTCCCTGCTCTTCCCAGCACAAGGTGACCTTCGACCCAACCTCCTCCTACACAC
TAGAGGACCTGAAGCCTGACACACTCTACCGCTTCCAGCTGGCTGCACGCTCGGATATGGGGGTGGGCGTCTTCACCCCCACCATTG
AGGCCCGCACAGCCCAGTCCAGTAAGTGTCTCCCAAGTCCGCTGCCTGTTACACCTGGGCTGGACACACACACACATGCACACA
CATCCTTCCCCCTCGACCAGGCAGGTGGGGTGGTCAGGTCTGCTGGCCAAACCGAACTCTGGCCTGGGCTCTGAGTCTGGGCCTCGG
GAGAGGGCCAGGTAAGTGCCTCCCAGTCTGTCCAGTCCAGGCGGCTGCTGCCCTCCCTGCTCCCTGCCCAGCCTCCTGCCCCTCCTC
CCGCCCATGCCCCACACACTGCCCCAGGTAAGTTCTGTGTCTGTGCCTCATCCTCCGCCCCCACTCTGTGCTGGTCACACTAGCTTAGC
TGATGGGCCATCCTCGGTGGGTCTGCCATGTCCCAGACCTGCTTTGGCGGCTGTTTCTCCTCCCTATGGCCCGCATCTCGTCTCCC
CATCACCCACATTCCCTTCCTCATACTCCCCATGAAAGTTACCTGAGAGCCATGTGATCACCCTGAGCTGACTGCACTCGGGGACAG
GTCTGAGAATGCGGCCATGTGACCCAGCCTGCCCACGTGTGCCTTGAATTGTGACCACGTGGCCGTGCAGGGGACTGTCTGCAAGGC
TGTCTGTGTGACGATCTGGTGTTGTGTGTGAGAGACTCCGATGCTGCAGCTGTGGGAGTGGCACTGCCCCATGAGTGTGAGTGTGTGA
CCACACCTGACCAGCTGGCTGTGCAGCACCTGTGTATGTCATCAGTGGATCTTGGGCATGACCCCTTGTCTTTGTGGGCGACTCTGG
CCTGATCCTGTAACCGTATTTGTGGGTCTCGGTAGCCCCCAGCAGTAGACTGTGTGTGTGTGTGACTCTGTGGTTGCTGCTGGGC
GTCCTGTGTGTGGTCACACAGCCACAGTCAGCTGCTGCGTGAGACTCTGTGTGATAGGGTGTCATGGATGTGACTGGCTGGGTAGCA
TTGTGGGATTGAGATGGTGACCACATCTGATGTGGCCATGTGATGACCAGAAACATGGCGGTGTCTGAAGTGGTCCTTATGAAGTGG
GTTATGGTCACAAGGTAAGCCACCGTGCCTGCTCTTCCATCCTACTTTACACAAATGTCATTAAAAGCTCATTTGGAGGCCAGGCA
```

EP 2 204 376 A2

```
CGGTGGCTCACACCTGTAATCCCAGCACTTTGGGGAGGCCAAGGTGGGCGGATCACCTGAGGTCAGGAGTTAGAGACCAGCCTGGCC
AACATGGTGAAACTTTGTCTTTACTAAAAATACAAAAATTAGCCAGGCGTGGTGGTGGTTGCCTGCAATCCCACATACTCGGGAGGC
TGAGGCAGGAGAATCACTTGACCCCAGCAGGCAGAGGTTGCAGTGAGCCGAGATGGTGCCATTGTACTGCAGCCTGGGTGACGAGCA
AAACTCCGTCTTGAAAAATAAATAAAATAAAAAAGCTCATTTGGGTAATTACAGGTGCAATGTCTGTCTGCCCTGCTGGAATGCAGG
ATCCCCATGGGCAGACACCATGTGTCTGGTCATGCTGCCAGCCCTGTACCGTGCATGGGGCCTGGCCCCCAGCTGCAGCACAGCAGG
TGCTTCTGGAATGAATCCATGAATACAAGGTCACACACACAGCCTCTTGACATCACTGGTACATGCACATGTACCCTGCTCCTGTTCAT
TTACCAGTTGGCTCCCACCCTTCCTTCAGGCCTTGGGAAAGTGTCACCTCCCCAAGGCCTGAAGGAAGGGTGGGAGCCAACTGGTAA
ATGAACAGGAGCAGGGAGCACACAGGCCTCCGGGAGAGTCTGCAGGCCTGAAGGCCTTCCAGCTGCAAGGCACATGGTTATGGTCAC
AAGGTAAGCAGCAGGCCAGTGTGGCTGCAGTAGCTGCCGGATCTATGGCAGTCAGGGCCTTGGGGACACAGGGTAGGCAGGAGAGCT
GACTCTGGCTGCTCTGTGAGAATGGACAGGGCAGCCTGTGAGGAAGCAGAGACCGGTTAGGAGGCTTTGTAGCGGTCCAGGGAAAAG
ATGGTGGCGCAGGTGAGGTGATGCGGTGGATCTGAGAACTGTCAGGGATGCTGACTGGTCAGACTTGGTAATGGGTTATGAATAGG
GGCCCAGGGGGAGGGAAGTGGAGGTGAATCTTGTTTCTGGTGTATTAACCTGGGAGGAGAGTGGTGCTGGCGTAGAATGGGAAATGG
GGCAGAGGCAGGTGTGAAAACTACAGCTGGTCTCAGACACCCAAGTTCAAGATGCCGTGTGCCATCTACATGGCAGTGCTCGGCAGG
CTGTGGGAATCCAGGGCTGGGCTGGAGCTAAAGGACCCTGAGAAGAAAGACTGCAGATGAGAGGAAATCAGGGGAGGGGAGCGACTT
GGAGGTTGGAAGGAGAGAAAGTTTCAAGAACTAGACGAGATGCTGCTGAGCGGTCAGGAGTACCTGTTGGACCGAGCAACATGGACG
AGTGGCCCTGGAGATGAAGCTTGGTGCATGGTAGGACCAAAGCTGGTGGATGTTTCATGAGGCCTGTGTGGGTGCTGAGAATGTGGA
GGCAGCAAGCCTAGACGTTGCCAGAAAGTGTAGCTCTGAACAAGGGGACCACTATGGCTAGAGAGGGCCGTGGAGCTGAGGGTGGGA
TTTTGTTTTGTTTTGTTTTGTTTTTGTTTTTGTTTTTTTGAGACAAAGTGTTGCTCTGTCTCCCAGGCTGGAGTGCAGTGGCATGATC
TTGGCTCACTGCAACCTCCGTCCACCTCCTGGGTTCAGGTGATTCTCCTGCCTCAGCCTCCCGAGTAGCTGGGTTTATAGGCGTGCC
CGCCACCACACCCAGCTAATTTTTTTTTTTGTAATTTTAGTAGAGACAGTGTTTCACCGTGTTAGCCAGGGTGGTCTGGATCTCCTGA
CCTCGTGATCCGCCCGCCTCGACCTCCCAAGGTGCTGGGATTACAGGCGTGAGCCACCGCGCCCGGCCTGGGATTTTTCTTTTTAAGA
TGGGAAAATTTCAAGCATATTTAAATGCTATTGGAAACAGTCAATAGGAAAAAAAGAAACCGTGGAAAAGAGGGTAACTGGTGGAGC
CTTGGGAGGTGGAGAGAAGGGGAACAGTATGGGTGGTGAGAGAGCTTAGCCTTGGACAGGGTGGCTCAACTCTCCATTCTCATGGGT
GGAAAGGAGAGAATTGCTGCAGATGCAAGTACGGTGGGGGGGTTGTAGCAAGAGATGGAGGGAGCTGCTGTGAAAGCCTGGTCTTCT
CTGCAGTGTGGGAGACATGGTCATGCGCCAAGAGGAGGCAGGCAAAGGGAGCTGGAGGTCTGGGGAGCATGGAGGAGGGGGACTGTT
TCATGTGAGGTGGCAGAGCGAGCCAGGCAGGGGGCAGAAACAGGCAGCATGGAGAGTCCTGGAGGATGAGGCCTCTAGGGACGTGTG
CATAGGACGCCAGCCTGCCTCAGTGCCGCTGTAGAGAAGGAGGGGGAGAGCTGGATTCATCTAGGGCTTGGCAGCGGCCAGGAAGGA
GCATGGAAAGATCAGGAGGCGAGGGACCTTGCGATATTGGTGAGAGTGTGGTAGGTGGTAGACTGTGGAAGCTGAACATGGAGGGTG
GGAAGGACAAGGTTGATGGACAGGCATCCTGAGGAGCTGGCGAGCAGGCTCAGTGCCCGGGGCCTGGAGTGACTGAGCAAGAACAAG
GGGGTGGGGTCTGCAAGGGGATGTGAGAGCCCGTGATTCTGGAGGGGAGGGCCATCCCCAGTACTGGCAGCCTCCAGGGCATGACCAC
AGCTGTGCAGGGGAGGAGAAAGTCCTGGAGATGAGGGGCTCTGGGGCCCAGTGGGTCAAGGGGCTGCTGAAGTCCCCATGGCAGGGGG
TGGTGACAGAACTTGAAGAGGAAACCTGTGCATTGCAGCAGAGTCCTCGGTGGGCGTGGAGTCTGGACACATCACTGGACACAAGGG
GACACGTGTCTGCCTCCTAATTCCCAAAGAAGCTGTTGAGTGAGGGCACCTCTGTTGGTGGGTACTTTTTCTCCACGCCAGGGGCTG
TTGGCCTTCATGTTCGGCCTCCTGTTGGTGATTACCTGTGGCACTGGCAAAGAGACTGTTTCCAGGCAGGCACAGGTGAAGGCTGCC
TTATGCCAAAAGGGCCACTGGGGCCACTCTTCCTCGGAAGAGCAGAACCGTGGGCACAGCAGATGTGAAGCAGTTCTCCATATGTGG
CTGTGTGTGCGGGTGGCAGTGGGATGGCCACGTGCTTGTGTGTGTATGTGATATCGTGTATGTTTTGTGTGAGACAGCATATGTGTGGG
AGAGACCTCGTGTGAGAGATGCTGTGTCAGAACTCTAAGACATTGTGTGTGAGAGTGTGTGTCAGATGCCATGTGTGAGACACCAAG
ACACGGGTATGTGATACTCTGTGTATATGTGAGTCTGTGTGAGAGACACTGATACTCCAAGACATTGTGTGTGTGTGACACTGTGAG
ACACCAAGACAGTATATGTGCGAGACAGCCTATGTATGTGACAGTGTGTGTGTGAGACACCATGAGAGACTGTGTATTTACTGTGAG
AGACTGTGTGAGAGACACGTGTGAGACACTGTGTATATGCACTATGTATACGTGAGACTGTGAGACACTATGTGTATGTGACACTG
TATATGTGTGAGACCGTATGAGACAGTGTATATGACACTGTATATGTGTGAGACTGTGTGTGTGAAACACTATATGACACTGTATAT
GTGTGACACTGTATATGACACTATATGTGGACTATGTGTGAGACACTATGTATATGTGACACTATGTATGTGTGACACTGTGA
GAGACACTGTGAGACACCAAGACAGTATATGTATGAGACACCCTGTGTGTGTGACACAGCGTGTGACTGTGTGAGACGTGTGTGAGG
CACTGAGACTGACACTGTGTGTGAGGCTGTGTGAGAGTCAGTGTGTGTGATAACTGTGTGTGTATCACCGTGTGTGTGTGTGAGAGA
GGGAAGGAGAGAGAGGGAGGCAGGTCAGAGGGAGTCAGGCGTCCTTGGCGAGAGTGGCAGCAGCCTGCCTGGAGGGACCCTGGGATG
GCCATTTCAGCACCTAAGGGGTAGCCTGCCCGGGTGAGTCTCGACTCAGTCATTGTGCCTGTGATCCCCACCCTCC
ATCTGCTTGCTTCCCCCCCATTTGTCTTCCCCAGCCCCCTCCGCCCCTCCCCAGAAGGTGATGTGTGTGAGCATGGGCTCCACCACG
GTCCGGGTAAGTTGGGTCCCGCCGCCTGCCGACAGCCGCAACGGCGTTATCACCCAGTACTCCGTGGCCTACGAGGCGGTGGACGGC
GAGGACCGCGGGCGGCATGTGGTGGATGGCATCAGCCGTGAGCACTCCAGCTGGGACCTGGTGGGCCTGGAGAAGTGGACGGAGTAC
CGGGTGTGGGTGCGGGCACACACAGACGTGGGCCCCGGCCCCGAGAGCAGCCCGGTGCTGGTGCGCACCGATGAGGACGGTAGGCAG
TGCCACCGGGGCGGAGGGGGAGGCGTTCTGCCTCAGACACCACCACCAAGCTCCCCAGGGCCTTCCTTTCCTGAACACAGGCCCAG
GTCAACTCATCTTTCTGGTTCAGGTGTAATGGCCTAAAGTGGGGGGATGTCACTTACGGGATAACTGAGGCCCAAATCCCAGCCTTT
GGGGCTGTCTCCAAAGCAGCTGAAACCTTCACAGGCTAAGATGGGAGAAGCAGCCCTGTCTAAGATTTGAAAGGTCAAGATTGGGCA
GATTGGTGTAAAAGATACTCAAAGTAGAATCAGCAAGACTCCACGTTGGCTGGACATTGGCATGATTGGGGCCTAGGAGGAGTCAGG
ACAGGTTGTGCTGACTAGGGGTGGTCAAAGACCTGGTGCCCCACCTCCACCTGTTCCCCCATGTCGACCCTCCCCACCAAGTGAGAG
GCCTGGGCCAGAGGGGTGGGCAGGGCCAGTCCTGGGCTCTTACCCGTGGCGGCAGAGGGAGCCCTTCCGTGTGCCTCACCAGGGACA
GATGATCTAAGGAAACTGTATCCAGGGCCTTTCCTGGGGGAGTGGGGTGCTGAGGCAGGACCCTCAAGTTTGCTGTGCCCACCTGAGC
TAGGGTTGATACCTCCAGGCCTGACTTCCTTCTCTACCTGACCCCCCAGTGCCCAGCGGGCCTCCGCGGAAGGTGGAGGTGGAGCCA
CTGAACTCCACTGCTGTGCATGTCTACTGGAAGCTGCCTGTCCCCAGCAAGCAGCATGGCCAGATCCGCGGCTACCAGGTCACCTAC
GTGCGGCTGGAGAATGGCGAGCCCGTGGACTCCCCATCATCCAAGACGTCATGCTAGCCGAGGCCCAGGTGCAGCATTGGGTGGTG
GTGGGGTGGCAGGTGAGCACAGACCAGCCATGCACAAGCTCCCTTTTGGGGCCCAGATATGTCCCTCTTCCCCTGCCTGCCCTCAGC
AGTGCTGTGACTGCCTTTCCTTGGTTGTGAGACCCGAGATGCTTTGCAGCATCAGGGGTTAGGCTGGGGTTTTTTGGGTGTGGGTTT
TTTGTTTGTTTGTTTGTTTTGAGATAGAGCTTCACTCTTGTCGCCTAGGCTGGAGTGCCTCCTGGGTTCAAGCAATTCTCCTTCCTC
AGCCTCCCGAGTAGCTGGGATTACAGGCGTCTGCCACTGCGCCTAGCCAATTTTCGTATTTTTAGTAGAGACAGGGTTTCACCATAT
TGCCCCACCAGTCGGGGTTTTGAGATGTGCCTTTCCCCTAGACAGTGCTGGGCTGGCATCCACTCTTCCCACAGAAAGGGGTAGAGA
GAGTGCTCCAGGGCTGTCTTACCCCACTGGCGGCCATGGGTCCGTGGTTGCTGCAAAGCTCTGTGAGTAGCCAAGTAGAGTGTTGCC
CTGCTCCTGGCCCTGCAGGGAACGATTCAGCCCCTGATGTTCACCCTCAAGAGCTAGGCCTGCTGGCCAGCCTCACACCTCCCTCTG
TGCACATCTGTCTTCCTGGGAGGATGGTCCTGCCCTTGAGCTTGGGGTGAGGTCCCTGGGGTATTCTGAACACTGGTTGCTATTCAG
ATGAAGAACTTGGAATGCTGGGGGGGTTATGAGAGTGGTATGGAATTATTCAGCAAGTAGGTGGCTGCTGCGCTTGGATGAGAAGCCA
TGTCTGTGGACCCCTAGGAAAGGGCCACAGTTGCTGTCATGAGCCCCCTCCCAAAAGACCCTGCTGGAGAGTCACAACACCTGGTGT
GGTGCTCTCAAGGTCTCCCTATCCAGGAGCAGGGCCTCCCCATTGAGCCTCTCACCTCTGCCTGGGTGGGAGAGCAGGGGTGCGTGTA
CCACTCAATGCTGTACACACTGTGCAGAGGGGTGGGGTCACCCACACAGACAGGAGCCTTATTCCTCCAGCTGGGCTCAGCCATCTG
AAGCAAAATTCTTTCTGCCCAGAGGGTGCTCTCTTCCCCCTCTCAGCCTGCCCAGTGCTAAGGCATCCGGGGTGGAGGAGGCAGGCA
TGTGCACCCACCTGCATTCCTGGGGCAGGTTGAGGGGCTCCTTGTGGAGCCTCAGTGAACACACCTACCCAGAAACATCCCAGGAATG
GGTTCCCCTAGCCCCTCCTCTCTGAGGGGGGCCAGTGGCCACAGCTGTGGCCAGTGGGGTTCCAGAGAAGCCACTTCAAGTGCCCCCT
```

238

```
TCTGGTCCCAAGAGGTTCGGAAGGGAGCTGGGCCAGAGGCTCAGGGACGCTGCCCATTTAGTCTCAGACATCCCATCATGGGGGCGG
TAACTCGAGTCTGGGCTCCCGGAGGACACTGAAGATGGAGGCCTGCTCCGTAGCCCTCTCAGGACTGGGTCATTCTGTTCCTCGCCA
GTGGGAGAGCAGTGGGGCCTGGTCCCGAGCGTGGCCACAGGGCCCAGCTGTCCCTGTGCTTCTGTCAAGGGCGGCACATTCTGTCCT
TCACGCAACAGCACCATCCCCTCAGCTCATCCCCCATCATGCCTGAAGGAACTATGTGGTGCAGGTTCCCACCCCCAGCCTGGAAGT
GTGGAGCCAGACCTGTCTCCTTTTACGTCAGATTCCATCTCCCTGCCTCCCTTTTTCCCCTCTCGTGGCCTGCATTTCTCTCTCCTT
CTTGGAGTCTCTGTTTTTGTCTTGCTCTTTCTCTTCTCTCTCCTCCTTCCTTCCCTCCTCCCCCTGCCATTCCTCTGACCCCTTTCC
TTCATCTCCATTTGGTTTCATCCTCCGTCCTTCCCTTCTTCTTGCTCTGCCTCTAGCGCAGGACAGGGTCATGTGATGTGAGAAC
CTCCACGCCAAGGCTTGGTTGGGACAGCCCAGGTCTCCCCGCAGGCAAGGAGACTGGAAGGGACGTGGGCCCAGCCACACCCTATAA
AGTGGCCATGCACTAAGGACCTGTCCAGAGTCTCCTCTCATATTCTGTTCCATGCTTCTTGCAAGGACTTTCCAACAGAGTGTCCCA
GGACAGGAGGAACTTAGGCCACCCAGCATGGAGGCCAGTGGACAGAGGCCAGACCGGCACTGTGGGGGTGCTTGGGCTGGAGGACCC
CAGGTACTTCCGGCTTGGAGACATCCTGGACCATCTCCCTGTGATGTTTCATGGGGCCTCAGAATGGAGACCTCACAGTCCCTCCAC
CTGTCCATCTAGAATATCTACATGCACCAAAGGCCCGCAACACTGCCAGCCCCGAAACATTCCTTCCTACCTTAATCCTCAGACACC
CCGAGGAAGGAACGGGTATAGGTGATTTCCCGTGCCTGTGGGCTACAGCCCAGGCTCTGAGCTTGGCACTCACAGCCTTCATAGTCT
TGCTGACTGGCTTGCCCTTGGGCCCACCCTGGCCATGTGCCCTGTTCACCCCCAACTCTTACTGTTGTGGGACACTTCTCATGCCTG
GGGTGATTGTGTAATGTATGATCACATCTGGGATGCTTTGGCGGGTGGAAGGAGACACTGAATAGTCAATTCCATGGCAACAAGCAC
AGGCCAGGACTGTCCGAGGCAAACCAGGGCATATGGGCACCCCATTCACCCCTCAGTGTCTTCCTTTCCCTTGGCCTGAGCTGTTGA
ACCCCACCCTTCTTGTCTGGCAGCCTCTCCCTCCCAGCAAGCCCAAAATTGTCAGAGCATCTGTAGCTGCTTTGTGTTCTTAGGGCCT
TCTGTCAGCGGGTCCTCTCCCTGCAGGCTGCCTGCCCTCCCTGTCTCCCTACCCTCCTCAGCCCTGGCACACCCAGTTGGTGCTCAG
TGAGGCAGGGATTCAAATCTTTGAACCGGGAGTTGTTCTGGGGTGCTACCCCCATGGGTACTTTGAGGCCCAAAAGCCCTCCCTCTG
TCCTCCCTGGGCAAGGTCCCTTCCAGGCCAGGCCCTCTCCAGGTCAGATGCCTTCACCATCCTGTCTCCCTTTCTCTCCCTCTCCCG
CGGTCAGTGGCGGCCAGAGGAGTCCGAGGACTATGTAAGTAACAGGTGTGCGAACGCGGACAAGACATGGGTCAAGCTGGGCTCGTG
GGACGCTCCTCCTCTCCCTCCTTTCCTGCTAGCCTGCACTGCCAAGATCCACAGGGCTCTAGCCACATCAGGAGAAAATTGGCGTTT
AGACACAAGCACTGACTGAGCAGCGATTGGCATTTCCTCTAATCCTTACTTCTTGCCTGTCGAGCAGCAATTTGAGGCCAGTGTTC
ATGATCGACCAGGGCCTGGCCCCTGCCCCGGCCAGACAGGGATGGAGTTAAATCCAATCCTGATCGTTAGGCCTTATTGATCCCTGG
AGTGAAAAATCACCTGCTTTAGGGCCCAGGCTGGGAGGGCTGTCTGGAGAGTCGGATTCTGGCATTGGTGCATTCTGGACCCCAGC
CCTGGGAGACCCTCCAGCTGTGGCAGGAGGGGTCCATGAGGGGGTGGTGGCAGCTGCAGGGGCCCCACTCAAGGCCAGAGCTGGAGG
GATACCAGGGTTGATGACGGCTCTGTTCCCACTGCACGGTGGTCCCTGCCCTGCTTCCCACTCTTCTCTCTGTGTGGTGTGGCTTGA
CCTCCATGGTGTTTCTCCGCATGTCCAGAGATGATGCCTTTGCTGCAGATGGGTATATGGGCAGGGTCTGCCAAGCGGGGAGGACA
TTGCCCTGGCTGCTGTCTCAGGCATCACTGAGAACAGATGTGGAAGCCAGTTCCCCAGGTGTGGAGGTTTTCTTATTCTCCTAGCCC
CTCCCCTGCCTTTCTCAAGAGGTATTGCAGGGTATAGACATTCACAGAGTTAGTGGCCTATATGGGGGCAGCGGAGTCCTGACTGGG
TCCTATAGGATGGCACCTTAGCCCATCCTGCCACCTTGCTCTGTCTGTGCATGCATGCACTGGTGTCCACAGGCAGCCTTACGCCTG
CTTATGCAGGAGCTGTAGGCTGTGTGCGTGTGGCTGCCAAGAGCCACGCAAGGTGCTGGGTGCGTGCGAGGCTGTGCCCTGTTGATA
TCCTCAGTCTCCGTTCACAGCACAGTGGAGTGAGGGAAACAGTCTCGGCCCTTTGTTCTTGTCTGAAAAGAATAATGAGCTGTCTGCC
CCAGGCGTGCGGCTCCTGGGATGGGCGGGGTCCTCCCAGGCCTGCATCCTACCTGCCTGCTTCCTCTCCAGCAGAGGCCACCATTGT
ATAGCCCCACCTTCCACAACCCCTGGCCTTGTGTGCCCCGGGGCTCCCCTCAGGCTAGGGTCCTGAGGTCCCTGACAAGGTCTGGCC
TCTCCCTGCATTCTTGTGATGGGAACGAACCCCTCCTCCTCCCTCCCTCAGGAAACCACTATCAGCGGCCTGACCCCGGAGACCACTACT
CCGTTACTGTTGCTGCCTATACCACCAAGGGGGATGGTGCCCGCAGCAAGCCCAAAATTGTCACTACAACAGGTGCAGGTGAGTGAG
GGGTCAGGACGGACCTGAGGGTGGGGCAGCAGGAGGGCAGCGCCAGAGCCCAGCCCGTGGTCCTTCAGTCCCAGGCCGGCCCACCAT
GATGATCAGCACCACGGCCATGAACACTGCGCTGCTCCAGTGGCACCCACCCAAGGAACTGCCTGGCGAGCTGCTGGGCTACCGGCT
GCAGTACTGCCGGGCCGACGAGGCGCGGCCCAACACCATAGATTTCGGCAAGGATGACCAGCACTTCACAGTCACCGGCCTGCACAA
GGGGACCACCTACATCTTCCGGCTTGCTGCCAAGAACCGGGCTGGCTTGGGTGAGGAGTTCGAGAAGGAGATCAGGACCCCCGAGGA
CCTGCCCAGCGGCTTCCCCCAAAAACCTGCATGTGACAGGACTGACCACGTCTACCACAGAACTGGCCTGGGACCCGCCAGTGCTGGC
GGAGAGGAACGGGCGCATCATCAGCTACACCGTGGTGTTCCGAGACATCAACAGCCAACAGGAGCTGCAGAACATCACGACAGACAC
CCGCTTTACCCTTACTGGCCTCAAGCCAGACACCACTTACGACATCAAGGTCCGCGCATGGACCAGCAAAGGCTCTGGCCCACTCAG
CCCCAGCATCCAGTCCCGGACCATGCCGGTGGAGCAAGGTGTGTGCTGTGGACATGGCATCCCTTCCCGAGTGTGGCTGCATCTGGG
GGTCTCTGCTCTCCTTGAGCCCACTGACCTCTGGCGACTGTGATCCACCAGCCTCTGGTGTGCACCTCCAATCTCTCATGACTGTGA
CCACTAACCTCTAGTGAATGGGCACCACATTCTTGATGCCTGACCTCTGCTGTCCTTAACCTACTGACCTCTGCTGTATGACCTTCT
GATCTCTTGTGACCTTGACCCACTGATCTCTTTTGACTGTGTCACTATTCTTGGGTGTGCAACCTCCTGATCTTTGGTGTGTGACAC
TAATCTCTTGGGGCCATGACCCACCGACCTCTAGTGAACATGCTCCACCACGCTCTGGTGTGTGGCCACATGCTTCTCATGACCGAA
ACCCACTGACCCTCTGATCACTCTGGCCTGGTGTCCATCGGCTCAAGCTTTTACACTCGCGTTTCTGGAGATTCTGACCCTGGTTGC
TGTGGCATCCCCCGTGTTTGGTGCTCACTGTGGAAGAGCTTGGAGGTGGCAGGGACGTGCCGTTTGAAGCTGGCTGGGGAGTG
GGGCGCTTGGTACTCTGCAGCCATCCTTCAACCCCCTGTTCCCCAACCAGTGTCTCATCCTGGCCATTCACCTTCCACCCTTCCAGC
CCATTCACCCCACCTCATATACCCAGCAGAGCTGACTCTCTCTATGCCTTTGCAGTGTTTGCCAAGAACTTCCGGGTGGCGGCTGCA
ATGAAGACGTCTGTGCTGCTCAGCTGGGAGGTTCCCGACTCCTATAAGTCAGCTGTGCCCTTTAAGGTGAGTAAGGGCCACGGCCAG
CTGAGCCTGGCACACACACAGGCCTGCTGGGTGCTGTCTTTCCAGTCCTAACCCATGTGCATCCGGCTGTGGAGCAGGAATGTGGTT
GTGTATCCGTGCACTGTGCCTTGCAGCCCGTGGCTGGAGAGCACGTCACCCAAAGGCATTGATTGCCCCTCCGTCCCCCACAGATTCTG
TACAATGGGCAGAGTGTGGAGGTGGACGGGCACTCGATGCGGAAGCTGATCGCAGACCTGCAGCCCAACACAGAGTACTCGTTTGTG
CTGATGAACCGTGGCAGCAGCGCAGGGGGCCTGCAGCACCTGGTGTCCATCCGCACAGCCCCCGACCTCCTGCCTCACAAGCCGCTG
CCTGCCTCTGCCTACATAGAGGACGGCCGCTTCGATCTCTCCATGCCCCATGTGCAAGACCCCTCGCTTGTCAGGTGTGCACACGAG
GTATCGGGGGAGGCGGGGCAGGGCTGGAGGTAACCAGCAGTGACAGTCCTGATTCCTGCCCTGCCCACCCAGGTGGTTCTACATTGT
TGTGGTACCCATTGACCGTGTGGGCGGGAGCATGCTGACGCCAAGGTGGAGCACACCCGAGGAACTGGAGCTGGACGAGGTACCTGG
GGAGGGGATGGGGACACTGACAGCCCCATTGCAGTGGTCAGCTGTGGCCTTCCTGCCCTGAGCACTGTCCCAGTGACTCTCAGATTC
ACTCCCCAAATTGAAATCTCTCTTCTGGCTGGCAGCCCGCCCCTCTCTGGAGAGAGGGACTCTGAGGGAAACCATCTGGGAGTATTCA
CAGAACTCCCGGAGGGCTTACGAAGAATCCCTGGGGTGGGATGTCGTGGAGATGCCTCTGCAGGTCTAGAGAATGCCAAGCCCTGTA
GACACTGCAGAGCCTCGCAGATCTAGAAGCTATGGAGGGCTTAGTGAGCCTTATAGCCAGGAGTCCCTGAATGTTTGAAATCCTTCA
GTCCTTTCTCATAGCCAGTGTGGCAACAGCCTGGTTAGGGTGGGGCAGCTTACACACAGGGCTGCTTCTCATGGGCTGGTGGGGGAGG
AGTGGCTTCACGGTGTCTGTTACTCTGTAGGGGCAGTGGGTGGGCAGGTGTGGGCTCTTACACGGAAGGTGAGCCTTGATCTCGGC
CCAGGGAGCTGACATCCCAGGCCACAGCCCCAGGGCTGGCCGGCATGCTCCAAGGCCCCTCATGACCCCCATGCTCTGCTCTGCCAG
CTTCTAGAAGCCATCGAGCAAGGCGGAGAGGAGCAGCGCGGCGGCGGCGGCAGGCAGAACGTCTGAAGCCATATGTGGCTGCTCAA
CTGGATGTGCTCCCGGAGACCTTTACCTTGGGGGACAAGAAGAACTACCGGGGGCTTCTACAACCGGCCCCTGTCTCCGGACTTGAGC
TACCAGTGCTTTGTGCTTGCCTCCTTGAAGGAACCCATGGACCAGGTCTGCCTGAGCCGGCTTGGCTGTCAGCACCCTGATTCCCTG
GGCCTGGCCTGAGACGATGCCAGTCTCAAACACCACAAGACCCCAGGTCTTTATCAGTTTGGGGGCTTCAGGATCCTGGGCAGCAC
TAAAGACCCAAGATCTGTCCCGGGGATCCTAAGACGCGGCCCTGGGACCCAGAGGCCAGACCTAATGTGGCTCCAGGGACCCAGTCC
TGCCAGGTCCACCTTGTAGGGTCTGGGAGACCAGGCCCAGGGTAACCCAGACCCAGAGCCCTTTCTCCAGGATTGATAGGCAGAGGG
TGGGGGGTTCTCACGCTGAGCTCACAGCCTGCTGTTCTCCACCGGGCCACAGAAGCGCTATGCCTCCAGCCCCTACTCGGATGAGAT
CGTGGTCCAGGTGACACCAGCCCAGCAGCAGGAGGAGCCGGAGATGCTGTGGGTGACGGGTCCCGTGCTGGCAGTCATCCTCATCAT
```

```
CCTCATTGTCATCGCCATCCTCTTGTTCAAAAGGTGAGCACTGCCCTCAGAGCTCCGGGAACGGCCACCTGCCCCTCGCCTTTCAGG
CCCTCTCCGGGTGTGGTGCCTGTGGAGAGCGTGCAGCCTTGCATCCTGGACCCTGAGCCTCAGGCTCAGCAGGAAGGCAGGAAGGGC
AGGAGCAGTGTGTGTGCCTACCTGTGTCTGCAGGCCTGCATCTGTCAGGTGAGGGAGCCTCTGCAGCAGCCTGGGCGTGAGGAACTA
AAGCCTCAGAGGGTTGTCTCATGTGCATCCCTGGCACGTCTGTCTGTCTCTGTGTTTCTGTGGATAAGAGTGCGTCTGTCTCCTCTT
CAGTTTTAAGACACCGCCATCTCCTGAGCTCCTACCTCGAGTCCTTTCCTTCTGCCACCATTCCTGGATGATTCCCCAGCCCTACTC
TCCTGCTTAGGCCTAGAGAGGTCGAGCTGGAGGCCCACAGGCCATGGATGCTCACTAACAGACATGACTGATGGCCTGCTGTGCCCG
TGCCATCCGCAGCGGCCTCAGCCTCAGTGTGTCTTTCCCACTCAGTTCTCTATCGGCAGTGGCCACCACCTGCCCTAGGAGTCTCCC
TTCCTATCCGTGCACCTTCCAGCCTGAAAGGGATTTCAAAGATTTAACTTTGTCACTCTCAGTTTAAAACCTTTTGATGGCTGCCC
ACGTAAAGCCCACCCTCTTTAGCCTGACAGCCAAACACCTGGCTAATCTGACCCCTGCCACACTCCTGACATTGCGACACTGAACTC
ATTCAGTGTTCAGGCCCACACAGGGTGTGCCCTCTGCGGATCCTCCCCCTGCCCACTTGGCAACAGCAATCTCATCGTTCAGGA
CTCAAGTCCATGCTCTGGTGCCTGCAAGGCCTTGCCTGGCCCCTACCCCTTCCTTGCACAGCTGTCCCCTGTGATGCTCCACTGCAA
ACGCAGCTGGGCCTGTGTGCCAAGCCTTCAGCCCCCAGCACTGGGCTGAGCCCACAGGCATGTCAGTGAGCACCTGGCAGATGAAGA
TATGCTAGTCTGTGTGTCTGAGCACATCTCTGCACCATGCAGGAGAGCCTGTATCCTGTGTGCCTACCCTGGACCCACTGTTCCTTC
CTAAAGAGTCCACTCCTTCCTCCCAGGGTGGATGGACCTGTGGGCGGGTCCCTAAGGAGTGTCCCACAGATGCACTCATCCTTCCCA
CTTCCACCTGCATTCTGGGTCTGTCCAGAGAGGTAGACCCCCTCCCTGTGAATGTCATGTGGGTGCCATATAAGGGGCCTGCAGGGT
CCCCATGTCATTAGAGGGCTGAGTGCTTGGTGGAAACTGTGACCGTTCCTTTCTTCTGTCCTTGCTTGTCTTTCGGGACCTAGTAAA
CAAGAAAGGTAGGAGTTGTTCCTTCTCTCTTCACACACCTCCCCCTCTACTTCTAATCCTTGGGCCAGCAGGGAGGGATGACGCTTT
GACCAGAAGTCCCCTGGCCTATGGCTTTAAGCAGCAAACTGTCCAGTCAGCTCAGCAGGCTCCCTGGTTCAGGACTGAGTCCTCATT
GGTGTGAGGAGGCAGTTTCTGTCTCACTCTTGCCAGTTCAACTCTGCAGAGGATGCTTCTTCTCTGGGGCACATACATAATACAGAC
TGCATTGCTTTCCCTGAGCGTACAGAGCTCACCAGAACCATCACCCGGCTCCCAGTTCTCTGCGAAGTTGCCTAAGTGCTGCGAGAG
ACAGTCTCGCACACAGACAAAGAGCAGTCACTGTGTGGAACAGCAGAGAGGCATGTCCTTGTACCCACCTGAGCACACTCACTCAAAT
TCTTCTTCCCGAAGCACATGCGTCTGCAGACCACAAGTAGCCTCTGGCTATTTGTTGGCAGCTGAATCCTATGGTCTGTCTCTGACC
CTGTCCTTGTAGATGGGAAAACTGAGGCCAGTGAGAGAGGACTCTGCCCAGCTAGGGTGACGCCTTTCCCGTCCCTCCCCTGCTGCC
CTGGGCATTTACCCTGATACTCTGAGGGCCAAAAAGCTGGGGCCCTGTCTCCTGTGCTGTTTGTGTATTTTATGTGTCATTTGCATG
TTTGTTCCCGGTGGTTCCTGCTACTCAGCTGGGTGGCCTTGGAGATAGCAGTCAGTCACTGTCCTGCAGTGACCTGCCTTCTATCCAAAGG
AGCTGCTCGCCCCTGCAACCCCTTCCTCCCTGCTGCACTGCCCCTCTGCTCACGCCACACTCCTGCTCTCCCTACCCCCTCCCTGCC
CTGTGTTCTCTCATGTCAAGGCTGTTCTGGCCCTGGCTCCTCCGCGCTCAGAGATCCTGGGAAGAGGTGGGGCAGTAGGAGGACAGA
GCCGTGGACATGGGGGGCTCCTTGGCAGCCAGCCATGCCATGTGTCACTGTCTCAGCCTGGACCTCAGGTTCTCACCAGCCTCCCTTC
TGTCTCTCTAGGAAAAGGACCCACTCTCCGTCCTCTAAGGATGAGCAGTCGATCGGACTGAAGGACTCCTTGCTGGCCCACTCCTCT
GACCCTGTGGAGATGCGGAGGCTCAACTACCAGACCCCAGGTAGGGCACTCCTATGGCCTGTGTGCCCCCAGCCCAGACCTAGCAGG
CCTGTGGGTCCGTTCTGCAGGTCTCAGGGTCGCCACTGAAGTTCCTGGGCCGCATGCACTTGTTCCTGCTCCTTTTCTTCCTTTCTG
CCCTTCTCCCTGTGCTCATCCACTTTGGTTGGAATGACTGGGGAGCCCCATATCCTGCAGCTGGACAGGTGCCCCAGGCCAGGACC
CTCTTAGGTGGCAAAGCCACTTAGCCACTGCCTTGTCTTCAGTCAAGGCCTCTGAATATGGGCTGAGGACCTCCCAGAGCCCACGTC
TCTGCCACAGGGTGGCCAGTGTCTGGCCCACGCTCTGACGCCTCCACATGAAAGAGGGGCATGTTAGCAGTGGGAGATGCCTCACCA
GACATCTGTGGGGAGATGAAGTGCCTGCACCTGACCCTTCCTGGAGGCTGCACCCCAGGTTTACCATACCACGTACCTGCCCCTTCTC
CTAGCCACTCTCATGGGGCTGTCCACCTCTTCCTTCTACCTGAAATGTTACTCCTCCCAACACCCAGCAGACACAGCTCAGACCTCT
CCTCTTGCCCACACCCCCACAGGGTTAGGTGTCTCTTCTTGGCTCCAGGAACCTCCCTCCTTCTTCCTTTTGACGCCTCTCACAGTG
AGCTGGGATTACCTCAGTGACAGTCTCTGAGCTCTGTGGTGCCAGGAACTGTGTCTTGTCATCCCCCATCCCTTGTATTCAGCGCAC
GATGGGGTTCTTGTTGAATGCCTGTGAAATGAACACACAGACAAATGAAGGAATGAGTGAACTAATGCATACACACAGGGTTTGTGTA
TGTATGAGCTGGTTATTCTGCATGTCAAATTACCCCAAAACTTAGTGGCTTAAAACAACAATAATTATGTATTATCTGTCACAGTTT
TTGTGAGGCAGGAATTCAGACAGGGGCACAGCAGGAACAGCTTGTCTTTATTCCAGAAAGTCTGGGCCCTCAGCCGGGAAGACTTGAA
GGCTGGGAGCTAGAGTCATTTAAAGCAGGGGTTGGCAGACTATGGCCCGTGGCCAAGTACAGCCCACCACCTGTTTTTGTAAATAAA
TTTTTACTGGCTGGGCGTGGTGGCTCATGCCTGTGATCCCAGCACTTTGGGAGGCTGAGCCGGGTGGATCACCTGAGGTCAGGAGTT
CGAGACCAGCCTAATGAAATCCCATCTCTACTAAAAGTACAAAAATTAGCCAGGTGTGTGGCCCCTGTCATCCCAGCTACTCAGGAGG
CTGAGGCAGGAGAATCACTTGAACCCAGGAGGTAAAGGTTGCGTGAGCCAAGATCGTACCATCGCATTCCAGCCTGGGTGACAAGAG
CAAAACCCCATCTCAAAAATAAACAAATAAATAAAATTCTACTGGAACAGACACACCCATTTATTTATATGCTACCTACCTATGGTT
GCTTTTGCACTGTAAAGGCACCATTAAATAGATGCAGTAGATACCAGATGTCCAAAAGCCTAAACTATTTACGCTCTGGCCTGTTA
TGGATACCAAGTTTGCCAACCCCTGGACTCTAAAGCCTCTTTCATCCATGTGACTGGTGGTTGATGAGCCAGCTCTGAGCCTTGCTG
TGGCTGTCAGCTGGGACACCCAACATGAGGCTTCTTCAGGTGGCCTGGGCTTCCTCACAACATGGTGACTGAGTTTCATGAGCATGT
GTCTGGAGAGTGTGCCAGGCAGACCTAGCAGGCTGTTGCAACCCAGCATGATTAGATCTCTGAAGAATCAAGCACCAGGATGCTGGG
ACCTGGAGTGCAGAGAAGCCTCTTCGGGGATGGGATGCTGAGGCCATGTGGTTATTGGTCTGGAAAAGAAAGGATGGGAGAGAGCTCCT
CAGTGGAAGCATGTGCAGAAGCTTGGGCAGATTGCATGTGCGTCAAATTATGAGCACCTGAGGGTTTCTGCAGCATGAGAGTTAGGG
GCTTGGGGAATACTGAGCCAAGAGAAGACTGGAGGGCTAGGCAGAGGTCCAGTCAAAAGGACCTTGAATGTCAGGCTAAGGGGCTTA
GACTTCCTCTTCAGGATGCCAGGGAGGTAGGTATTAAAGGTTTTCAGCGGGGGAGCCAGGTGGTCAGAACCATGCTTTAGAAATGTC
ACTCTGGCTCCTTGAGGAGACTGGATTAGAGAGGAGAAATTGAGGCAGGGAGACCATTTAAAAAGGCCAGCAACCAAGTGAAAGGTGG
CAGTGGAACAGAGGGGACAGACTTGGGAACGTGAAGGACGCAGAGTCCGGGCTTAAGCCCACTTGGGAGAGGTAA
GGGTGGCTCTGCAGTTGCTGGCTGAATGAGCAGGGGGTAGGGGTGTCCTGGGGCTGGTGGGTCTGTGGGTCTGAGGACTTCTGAGCT
GACATCAGGGCTGAAGCCCTGTGGGAGGGGCCAAGGAGCATGTGGTAGTCAAAGCCTGAGAATAGATGGGCTCTCCCAGGGTGAGGG
CCAGAAGGAGACCGGGACCAAAAAGCCCAGGGCAGGGAAGGTGGTGCAGCATTAAGGAAAGATGGATGGAGAGCAGCTAGGGAGATT
GAGAAGGAGCAGCCACAGCCAGACGAGAGCAGGAGCGCAGTGTCCCTGATGCCAGGGTTGGGGGAGTTGTGCAAGGGACGAGAGGAC
TTAATGAGGTAGGGCAGTGGGGACACCCAGTGGGAGGACCGTTGGCTTTGGCAGATGGGGCCTTGAAGAGAGGGTGGTGGCCACGTC
CTGTGGGCAGAAGTGTTGCAGTGAGCAGCGTGGCAAGTGGGGGCTGAGAAAGTGGAGAAACCCCTTTCAAAGATCTGGGAAGATAC
CTGGGAAGGAGGCAATGAGCAGGGCACAGTGGGAGAGTAGAGGGTGTGGGATCTGAGAAGGAGGGCTCTTTTTTCCAACGTGGAAAT
CATCTGAAAATCGCCAAGGGCAAATTTTGGTATCAAAAGGGGCAGGGCTGGTTTGGACTTAAGTATTTAGTCATAGAGCCCCCCAAG
GCTGCCGAGCCACCAGGGTTTAGAAGTGCTCGCCTCTGGGGGTGGGACACCCAGTCTGTATTAACTGGGAGACAGAAGGAGCCTTGA
CGGAACTTGTCCGCAGCCCCTCACCGCCCCCTCCTCTTCCTCCGCCAGCCCCTCGCAAGCCCGCTCGGCACCTCCACTGTGT
GTGATGGTGCTGTAAGCAGAAAAAGTTAACGGGCTCTCTTTTCTTGCCCGTTGTTTTTTTCGTTTGTTTGTTTGTTTTTTCTCTG
CAGGTTCCAGTGTCCCCAGTTGCCCGAATACCTCAAGTTAGTTTTCAAAGTTCCCGTGTTTGGGGGATACTTTGGCTTCTGTGTCTG
TTTCCACTCCTTACTTTTGTTTACCCCATCGCCTCCATCCTTCCTTGAATTCTTCTCTCCCCTTCCCTTTCTTCTCCCCAATCCCA
CTGTCTCCTAACCTTTTTCCTTCCCTACCCTCCCCTCTGCCCACCTTGCTTCCTCCAGGCCTGTTTTCTCTCCCACCGGCCCCGTCT
CTGTTCTGCCTCTCTGGCCTCCAGTCCCGGCCTGACACCCTTCCTTCTGCGCTCACCTCTACCTCTCATCTCCTCTCCTCTGTCTCACA
CCCCCCCTCCTGGTCTCTGCTTCTCTCTCTATTGTGTCGTACTTCATGACCACTCCATCTACACATTGGTGCCCGGGATGACGATG
GCTGTGAGTCTCCCATGGTGACTGCCACCGGTGAGGGGCAGGAGGGCTGTCTGCAGGCAGATGCGATGGAGCCCAGCTCCTGTCACG
TCTGCTGCCACCGACCTGGGCGTCCCACCCCCTCCTGGGAGGAAGGAAGCCTCTCTTCCATCTTGAGAGACCTGCCAGGCAGGGCCTA
GTGCCCCCACTCAGCACCCCGCCACCAAAACAGGCTCCACATGCTCATGGCACAACACCGCCCTCTGTCCTCTCCCACCCTCCGCCA
TCCCTGTCGCCGCATGTGCTGCTGTCTCCATGCCACCAGTTCCAAGTGCTCCATGGTCACACATGTTCACATGTGCACATACATGCG
```

EP 2 204 376 A2

```
TTGGGGCTTTCTCTGCCACACTGCTCAAGCCTCACACTAATGCTGCCTGTGTATGCCCTACCTCCCCTAGGTATGCGAGACCACCCA
CCCATCCCCATCACCGACCTGGCGGACAACATCGAGCGCCTCAAAGCCAACGATGGCCTCAAGTTCTCCCAGGAGTATGAGGTGAGA
TGTTCCCGCCCCCTACCATGTGCCTGGCCCAGGCCTACCCAAACCAGCTCCTGTCCTGTCCTAGGTCCCAGCTGTGGTGGGTGAGGA
AGCAGGGGTCCAGCTTTTTCAGGAGCACAGAGAGGAGGGGTTGGCAGTGGTAAGGGTCAGCTGGGAACCGGGTGCCTCAGATGCTGGG
TCTGGCCATAGCCTGGCCCAGCACCTTCTTGGGGTCACCCTTAGGAGATGGTTTCAAAAGGCTGTGAGTGACACCAGGGTCTGGACA
CTCAGACACGTGCTCAAGTGCTCACAGGCAGACACAAGGCCACAGGCATACAGACATAGATCAGTGATAACAGCCACAGTAGCTGGC
ATCTATCAAGAGCATCCTGCAGGCCAGCACCGGTCTGGGCACTTGGCATGCATTATCTCTTTTGGTTCCCCCACAGTCTTCAGAAAT
GGGGACTGTTATTATCCCATCTTCCAGGTGAGGAAGCGGGGGCTCAGAGAGGGGAAGTGACTTGCCCAAGTCAAACAGCTGATGAGT
AGTGGAGCCAGGATTCAAACCCAGGCCTGCCTGCTGCCCTGTGCTCTGCACATGCATGTGCTCACGTGTGTACTCCGATGCCACAGC
TCACGGGGAGTCGGGGCCTCGGAGACTGGCTGCTCAGGCTGTACAAGTCCCGCTTGGAGCCCTCCACACGTTCATCTTGTTCTGGACT
TAACTCCTAGGAGCCTGCTGGGGGCTGAGCCTTCAAGAGTCTGAGGGTTTCCCACCCACAGATGGTGTCGGGGTGACTCTGAGCATC
CCCAGGCTGCCCATCTAGGAAGGGGGATTTGTTAGAGAAGGAGGTGATTTAAAGACAAGACTCCTGGCCAGGCGCAGTCGCTCACGCC
TGTAATCCCAGCACTTTGGGAGGCCGAGGCGGGTGGATCACCTGAGGTCGAGAGTTTGAGACCAGCCTGGCCACCGTGGCGAAACTG
CATCTCTACTATAAATACAAAAATTAGCCAGCTGTGGTGGCACATGCCTGTAGTCCCAGCTATTTGTGAGGGCTGAGGCAGGAGAAT
CGTTTGAACCCGGGAGGCGGAGGTTGCGTTGAGCCAAGGTCATGCCATTGCGCTCCAGCCTGGGTGACAGAGTGAGACTCCCTCTCA
AAAAATATAAAATAAAAAAAAACTCTTAAAAAAGAGTATTTTTACTTAAAAAAGAGAGAGAGAGACCTCCTCCTCTTCCACCTCCTCC
AAGCAGCAGCCTGTGCTTGTCGTTCTGCCTTGTCCACAGCTGTTTCCTCAGCACCTGGCACTGGCCTCAGTAGATGGTTGGTGGACA
GGCAATTGAGGGGTTGGCTGAGCCTAACCTGTGAGTTTGCGCCCCTTCTGATGTCCACCCTCAGCTGTGTTTGGGGGATGCATCCTA
GGGCTCAAAGATTGCCTTTCCCAAGGGCTGTGGGCCAGGTTTCTGAAGAGAAAGCTGGGCTTGGCAGGCAAATGGATGAGTATGTCT
GCGGCACAGCAACCGTGCTGCCTCTGCCTATAGGGCCCCCCTGGGGCCCTGCTCCACACAAGGCTGGGCTTTGGGTCACGGAGCCCG
TAAGGTGGGCTCCCTGCCTCCCATGGCCTCCACCCACACTCATCTGTACCATGTCCTACACCTGCCCTTCCTTCCAGCGCAGCCCTG
CTTCCCCATCTGGGCTCGTGGGGCCTCTGTTCACAGTAGTCCCCTTCCATCTTCTACCTGCTTCCCTCCTCTGATCAGAGCTTTCCT
TACAAGACCCTCCTCCTCCAGGAAGCCTTCTCAGGCTCCCCAAGGCTGCCGTGGGCTCTCCCTCAGCCAGGACCCCATAGCTCTGTT
GTCTTTTTTTTTTTTTTTTTTTTTTTTTTTTTTTTTCTTTTTTGGAAGTCTCGCTCCGTCATCCAGGCTGGAGTGCAGTGGCGTGATCTC
GGCTCAGTGCAAGCTCTGCCTCCTGGGTTCACGCCATTCTCCTGCCTCAGCCTCCCGAGTAGCTGGGACTACAGGTGCCCGCCACCA
CACCCGGCTAATTTTTTTTATTTTTTAGTAGAGATGGGGTTTCACCGTGTTAGCCAGGATGGTCTCGATCTCCTGACCTCGTGATCCG
CCCACCTTGGCCTCCCAAGTGCTGGGATTACAGGCGTGAGCCACTGCACCAGGCCTGCTCTGTTGTCTTTAACGTTCAGTCAGTCAT
TGATCACACATTTTCCATGTGCCAGTCCCCGTGCTGGGGATGTGGAGACACATGCCCTCAGGGAACCCAGTCGTGGGGAGCATGTGC
AGACAGATAATCAGCATTGAATATGTGACTCTGACAGCATGCCCTGGTGTTGAATAGGACCGACAGGCGTGCAAAAGGTGGGCTGAC
AGCACCAGAGCAGCCTGAGGGGTGGGGTGTGCTGCGGGGTCTCACGGAAGCAGTGACGCGGTCCTGTGGAGAGTAATGAGGCTGGGG
ACAACATATGGAGGACATTGCGTGCTATGCTGAGAACTTCAGATCGTATCCATCTGCGAAAATGTGAAGATAATAATGGAACCTATCT
CGTATGAGAATTTGAGGAGGGAATACATGCAAAGTGCTCACAGCGGTGCCAGGCACACTGTGGGTCCTCACTAACGCTTCGCTATTA
TCATTATTGCTCTCCGAGGTGAAGAAGAGCCACAGAAGGCCTTTAAGCAGGAAATTGATATTCACATCTGCATTTTGGAAGGATTAT
TCTGGCTGTGGGGCTGAGTGGGTCAGAGTGGGACAAGAGGAAAGAGGCAGGAAGAGGCTGAGCCTCAATTGGGGTGGCCTGGCCCAG
GGCAGTGGCAGCAGGTTGGAGGAGAGCTGAGGGGGTGGGGAAGCACCAGAACCTGGTGACTGGCAGGGCGGGGTTGGTGGAGGCCGGA
GGAATTTTGAGCTTGACTTCTAGGTTCTGGCCTGGCACTGAGGGTGAGGGTGCCTTTCGCTAAGGTGTAGTCCCAGCAGCAGCGCC
AGGTTTGGAGGGGAAGCTGATGAACTCAGCTTTGAAAGGACTGCGGTTGAAGGACCTCCCGGTGGAGGTGTCAGAGAGGCTGCTGGG
TACTCGGGGAAGAGCTCTGTGCTGCTGCTGAGGTTATGGGCAGCTCCAGCATGTGGTGTAAGAATCAGGTGAGCAAATGAGGTCACC
CAGGAAAGGTTGTAGAGAGAGAAGAGGGCCTAGAAGGAACCCTGGAGGAGCTCTGCGTCCTGCCCACGGGAAGGGCAGGGGAGAGAG
AGGAAACAGAGAAGGAGCAGCTGAAAGGAAGAGAGCCGAGCAGGGAGGGATCCTGGACTACCTGAGAGGGGCCACCATCCCTTCCAT
CTGTCCTGGCTCATTTCATTAGGTGAATGCAGGGCTTCGGGAGGGACTCTGGGTGTTCAGAGCCCTCAGCAGTTTCGGGACTGCCTG
AGAGGGGGCCACCACCTGTCCTAGCTCATTTCATTCATTAGGTGAGTGCAGGGCTTCGAGAGACCCTTCACCTGCCCCATCCCAGCTCAGA
CCACTCTACCAGGCAAGGGGATTTGGTACCATGGTCAGAGGAGTCCCCAGTCCATCACTTTTTCTGATAGGTGATGTGGCAACCTGTG
AGCTCCATGGCTGGCACCACGAGATAGAGGGCCTGGCTGTGGCCTGTGGAGTGAAGGCAGGATGTGAGCATCACCGGGAAGGCTGGG
TCCCCTGCAGGAGAAGCAGGTCAACCTTGGCTCTTACCCCACCCCACCCGCTTTCTCCATTCTCTGCAGTCCATCGACCCTGGACAG
CAGTTCACGTGGGAGAATTCAAACCTGGAGGTGAACAAGCCCAAGACCTATGCGAATGTCATCGCCTACGACCACTCTGCAGTC
ATCCTTACCTCTATCGATGGTGAGCCAAGGGGGTGCCCCTCCCATCCCCTTGCTCTCCCCCTTGCTAGCTAGGGCAACATGTCATTC
TACAGAGGATGTCCACGAGTCTCAGGGGTGCACTGAGGCATGGTGGGCTGGGCTGGGGACCCTGTAGTAATGCCCTCCCACCTCCTT
TCTTATCCATAGGCGTCCCCGGGAGTGACTACATCAATGCCAACTACATCGATGGCTACCGCAAGCAGAATGCCTACATCGCCACGC
AGGGGCCCCCTGCCCGAGACCATGGGCGATTTCTGGAGAATGGTGTGGGAACAGCGCACGGCCACTGTGGTCATGATGACACGGCTGG
AGGAGAAGTCCCGGGTGAGGGCTGCAGGGCCCTGCCCAGGAGGCGGGTGGGAAATGCCCAGCCACAAGGTGATACAGGGCACCTTCTTC
TGTGCCGCTTTCTTCTGTGGAGGAAGTCGCTCAAGTGATCCCCAGATGCTATTGTTACTGGGGGTATTATGCTCCCCAAATACTGGG
TGTTTCTGGGATACAGCATGTTCCCCACATGCTAGTGGGTTCCTTAAGATGTTAATATGTTTCACCAAATGCTGTCATTTTCGGAGA
ATGTTAATGTGTTCCCCAGTTGCTGGCGTGTTCCCGGGGTATTCGTGTGTTCCCCAGATGCTGGAGTGTTCCTGGGGCATTAACACA
TCTCCTAAGTTAATTAGTGGAAAGAGCTGCTGTTATCTGTTTTTGGACTGCAAAGCATTGACTGGAACGTAAAATTTACAGAGCTTG
AAAATGGCACATAAAACGTTATGGGTAAATTTGGCAGAAATGTGCCTGGTGCGATCTGGCGTTGAATAAATAATTTTCAATTATGAC
CCTTATTCCACTAGCTAGGGCAGGGCGGTGACATAGCTTGAGGACCTGATGTGGCTTGTGGAGGACAGGGGGCAATGGATCTGAGAG
CTCAGGGCTGGGGGGCTTTGTAGCCAGAAAGGCTACAGCCAGGGAGTTGACCAGCCTCCACCCTGCTTCTGCCCGTCTGAGCCTGTG
GGCTTCCTTCAGCCTGCCCTGCTCATCCTCCTGCAGGTAAAATGTGATCAGTACTGGCCAGCCCGTGGCACCGAGACCTGTGGCCTT
ATTCAGGTGACCCTGTTGGACACAGTGGAGCTGGCCACATACACTGTGCGCACCTTCGCACTCCACAAGGTATAGCCTTTCCCCAGT
GCATATCTCTTACCCAGACACTGTAAGGACAGTGGCCTGGGTGTGGTGTGGCTGGGGTCGGGGGGAAGCTGGAGCCTGGGTGTTGGAGG
GTCGGAGGCTCAGGTGTGTGAGTGATGATGATCCATGTTATGGGAACAGTGCTAGGAGCTTCAGGCTACTCTGTGTGGCTTCTGA
GTCCCATGGGGAAGTGGCGGGTATGGCCTCAGCATCAGGTCATTCAGTCCTGAGTCTATGGCAGGTAGGCTCCTAGTCGCCAGTATG
TCCCCACTTTGTCCCCCAGAGTGGCTCCAGTGAGAAGCGTGAGCTGCGTCAGTTTCAGTTCATGGCCTGGCCAGACCATGGAGTTCC
TGAGTACCCAACTCCCATCCTGGCCTTCCTACGACGGGTCAAGGCCTGCAACCCCCTAGACGCAGGGCCCATGGTGGTGCACTGCAG
GTGAGAGAGGTACAGTGCCACCCAGGAGGTGGGTGGGGTGGGAGTGGCGCCTGGTCCTCAAGCTGAGCCCGTGTCCTGCAGCG
CGGGCGTGGGCCGCACCGGCTGCTTCATCGTCGATTGATGCCATGTTGGAGCGGATGAAGCACGAAGACGGTGGACATCTATGGCC
ACGTGACCTGCATGCGATCACAGAGGAACTACATGGTGCAGACGGAGGACCAGTACGTGTTCATCCATGAGGCGCTGCTGGAGGCTG
CCACGTGCCGGCCACACAGAGGTGCCTGCCCGCAACCTGTATGCCCACATCCAGAAGCTGGGCAAGTGCCTCCAGGGGAGAGTGTGA
CCGCCATGGAGCTCGAGTTCAAGGTGGGGCTCGGGTGGGCCTGCTTGGCTCCAGGGCCTAGACTGGGTCATGCAGATGACCCCCACC
CCCACAGGAGCCTGGCCTGCCAATCCCTGCCTCTCAATAGTTGCTGGCCAGCTCCAAGGCCCACACGTCCCGCTTCATCAGCGCC
AACCTGCCCTGCAACAAGTTCAAGACACCGGCTGGTGAACATCATGCCCTACGAATTGACCCGTGTGTGTCTGCAGCCCATCCGTGGT
GTGGAGGGCTCTGACTACATCAATGCCAGCTTCCTGGATGGTTATAGGTCAGCATGCATGTCACTGCCCACCATGCCCTACAGGGG
CCTAGGCCTGTGCCTGCTGGTGGGGGTGGGCAGCAGAGTAGGGCCAGCCTAGAAGACCAGAGAGGGCTGGGTAGAGCAGTGAGGAC
TTCCTGGAGGAGGGGTGATCTGAGCAGGGCCCCAAGGGGCTAGGCAGCCTAAGGGGAGACTCTAGGGGCAGCAGCACCTCCAGCATG
TCCAGTCTTATGTCCACCCCAGACAGCAGAAGGCCTACATAGCTACACAGGGGCCTCTGGCAGAGAGCACCGAGGACTTCTGGCGCA
```

241

EP 2 204 376 A2

```
TGCTATGGGAGCACAATTCCACCATCATCGTCATGCTGACCAAGCTTCGGGAGATGGGCAGGGTGAGCCCACCCTTTCCCCCAGGGC
CCCTGTCATACCTGGGAGAACACCAGCCACCCTTGGGGGAGCTGCCGCCTATGTTACTGTCTCCTTTGACACCCCAGCTGCTTGTCA
GCATGGCCTCAGGCGCCCGTTATTACTACCTGAGGCATCTGTCCCAGAATCCTGTGAAGCCTGGCACCCCTCCCCCATTCCTTCTCA
CCTGATTATGGGGGCCCGACCCTCTGTCCACAGGAGAAATGCCACCAGTACTGGCCACAGAGCGCTCTGCTCGCTACCAGTACTTT
GTTGTTGACCCGATGGCTGAGTACAACATGCCCCAGTATATCCTGCGTGAGTTCAAGGTCACGGATGCCCGGGTGAGTGAGTGCATT
GAGTGTGTCCATAACGCTGCCTGTCCACACGCTGGGTGGATGGCTGCCTGCATGGTACCTTAGCTCAAGCTTCAGAAATCTGAGGCG
GTGGGTGGGTATTAGGGTGTGAGCACATCTCCCCCTGTGGCCTCGGGTGCAGTGACACAGATGCATGCCTGTATCATGGTACTACCC
TGGTCTAGTCCAGAGGGGTGGCTGCCTAAGGCACGAATTCTAATCATGTACCCCACCCACCTTTCCCAGGATGGGCAGTCAAGGACA
ATCCGGCAGTTCCAGTTCACAGACTGGCCAGAGCAGGGCGTGCCCAAGACAGGCGAGGGATTCATTGACTTCATCGGGCAGGTGCAT
AAGACCAAGGAGCAGTTTGGACAGGATGGGCCTATCACGGTGCACTGCAGGTGGGACTGGCCCCCTGGAGGGCTGGGGTGGGTGGGC
CTGAAGGCCTGGCAGACCCACTGCATGAGGCAAGCAGGACTCCTGACCCAACTGTGTTTCTGAGCAGTGCTGGCGTGGGCCGCACCG
GGGTGTTCATCACTCTGAGCATCGTCCTGGAGCGCATGCGCTACGAGGGCGTGGTCGACATGTTTCAGACCGTGAAGACCCTGCGTA
CACAGCGTCCTGCCATGGTGCAGACAGAGGTAACGCAGACCAGGCTGCAGGGCCAGGGCCTTGGCAGCAGCGCTGCTGGGAACCCTA
GGCTTTAGCAACAGTTTGATGCCCACAGGCATGTGCATTCATTCATGCTGCCAACCTTTCACGTGGCCTGCGATAGGCATGGTGGTG
TGTGCTTATGGTCCTACCTACTTGGGAGGTTGATGTGATGGCTAGCACAGCACTTGAGGTCAGAAGTTCGAGGCTGCAGTGAGCTATGATTAT
ACCACGGCACTCCAGCCTGGGTGGCAGAGCAAGACCCTGTTGCTGAAAACAAAACAAAACAAAACAAAAATGCTGCAACATTGCCTG
TGCTGCCTGGGGGCTCAGGGGATTGATGAGTAAGATGTGTTCTTTCATTCGGCAGCTCTCTGCTGAGCCCCAGTGGTGTGCCTGGCT
CTGGGCAAGGTTGCACAGAGCAATCCTTATGGGGTGCCCAGTCAGGAGCAGAGAAACATGATTGGGATGGCAGATGGAAGGCAGGTA
GATGTGGGGGGCCTGAGAGAATGACAGGAGAGAGATGAGCCTTCAGAGGCTCTTTCAGGCTCCCCCGCACACTGCCTGATGTAGCTGC
CAGGGTCCAGCACCTGCTCTTGGCCAGCAGAGGCTAACTCCATGGCTGCAGTGTGAGTGTCAGCTGTGTAGTGGGGGTGTCCACTGG
CGCGACCCACACTGACCAGCCCCCTATCCTGGCAGGACCAGTATCAGCTGTGTGTACCGTGCGGCCCTGGAGTACCTCGGCAGCTTTG
ACCACTATGCAACGTAACTACCGCTCCCCTCTCCTCCGCCACCCCCGCCGTGGGGCTCCGGAGGGGACCCAGCTCCTCTGAGCCATA
CCGACCATCGTCCAGCCCTCCTACGCAGATGCTGTCACTGGCAGAGCACAGCCCACGGGGATCACAGCGTTTCAGGAACGTTGCCAC
ACCAATCAGAGAGCCTAGAACATCCCTGGGCAAGTGGATGGCCCAGCAGGCAGGCACTGTGGCCCTTCTGTCCACCAGACCCACCTG
GAGCCCGCTTCAAGCTCTCTGTTGCCGCTCCCGCATTTCTCATGCTTCTTCTCATGGGGTGGGGTTGGGGCAAAGCCTCCTTTTTAAT
ACATTAAGTGGGGTAGACTGAGGGGATTTTAGCCTCTTCCCTCTGATTTTTCCTTTCGCGAATCCGTATCTGCAGAATGGGCCACTGT
AGGGGTTGGGGTTTATTTTGTTTTGTTTTTTTTTTCTTGAGTTCACTTTGGATCCTTATTTTGTATGACTTCTGCTGAAGGACAGA
ACATTGCCTTCCTCGTGCAGAGCTGGGGCTGCCAGCCTGAGCGGAGGCTCGGCCGTGGGCCGGGAGGCAGTGCTGATCCGGCTGCTC
CTCCAGCCCTTCAGACGAGATCCTGTTTCAGCTAAATGCAGGGAAACTCAATGTTTTTTTAAGTTTTGTTTTCCCTTTAAAGCCTTT
TTTTAGGCCACATTGACAGTGGTGGGCGGGGAGAAGATAGGGAACACTCATCCCTGGTCGTCTATCCCAGTGTGTGTGTTTAACATTCA
CAGCCCAGAACCACAGATGTGTCTGGGAGAGCCTGGCAAGGCATTCCTCATCACCATCGTGTTTGCAAAGGTTAAAACAAAAACAAA
AAACCACAAAAATAAAAAACAAAAAAAAACAAAAAACCCAAGAAAAAAAAAAAAGAGTCAGCCCTTGGCTTCTGCTTCAAACCCTCAAG
AGGGGAAGCAACTCCGTGTGCCTGGGGTTCCCGAGGGAGCTGCTGGCTGACCTGGGCCCACAGAGCCTGGCTTTGGTCCCCAGCATT
GCAGTATGGTGTGGTGTTTGTAGGCTGTGGGGTCTGGCTGTGTGGCCAAGGTGAATAGCACAGGTTAGGGTGTGTGCCACACCCCAT
GCACCTCAGGGCAAGCGGGGGCGTGGCTGGCCTTTCAGGTCCAGGCCAGTGGGCCTGGTAGCACATGTCTGTCCTCAGAGCAGGGG
CCAGATGATTTTCCTCCCTGGTTGCAGCTGTTTTCAAAGCCCCCGATAATCGCTCTTTTCCACTCCAAGATGCCCTCATAAACCAA
TGTGGCAAGACTACTGGACTTCTATCAATGGTACTCTAATCAGTCCTTATTATCCCAGCTTGCTGAGGGGCAGGGAGAGCGCCTCTT
CCTCTGGGCAGCGCTATCTAGATAGGTAAGTGGGGGCGGGGAAGGGTGCATAGCTGTTTTAGCTGAGGGACGTGGTGCCGACGTCCC
CAAACCTAGCTAGGCTAAGTCAAGATCAACATTCCAGGGTTGGTAATGTTGGATGATGAAACATTCATTTTTACCTTGTGGATGCTA
GTGCTGTAGAGTTCACTGTTGTACACAGTCTGTTTTCTATTTGTTAAGAAAAACTACAGCATCATTGCATAATTCTTGATGGTAATA
AATTTGAATAATCAGATTTCTTAC
```

HUMAN mRNA SEQUENCE : hR28-021.1 (Seq ID No: 67)
```
GGCGCCGCCGGGGGCGCGCACGGCAGGGGCAGGGGCGCGGGCGCGAGCGCGAGGGGGAGCGCGCGGCTGGAGCTGGCGCGGGAGCGGC
GGGAGCGGTGGCGGCGGCAGAGGCGGCGGCTCCAGCTTCGGGCTCCGGCTCGGGCTCGGGCTCCGGCTCCGGCTCCGGCTCGGCTCC
AGCTCGGGTGGCGGTGGCCGGGAGCGGGGACCAGGTGGAGGCGGCGGCGGGCAGAGGAGTGGGAGCAGCGCCCTAGCGGCTTGCGGGGG
GACATGCGGACCGACGGCCCCTGGATAGGCGGGAAGGAGTGGAGGCCCTGGTGCCCGGCCCTTGGTGCTGAGTATCCAGCAAGAGTGA
CCGGGGTGAAGAAGCAAAGACTCGGTTGATTGTCCTGGGCTGTGGCTGGCTGTGGAGCTAGAGCCCTGGATGGCCCCTGAGCCAGCC
CCAGGGAGGACGATGGTGCCCCTTGTGCCTGCACTGGTGATGCTTGGTTTGGTGGCAGGCGCCCATGGTGACAGCAAACCTGTCTTC
ATTAAAGTCCCTGAGGACCAGACTGGGCTGTCAGGAGGGGTAGCCTCCTTCGTGTGCCAAGCTACAGGAGAACCCAAGCCGCGCATC
ACATGGATGAAGAAGGGAAGAAAGTCAGCTCCCAGCCGCTTCGAGGTCATTGAGTTTGATGATGGGCAGGGTCAGTGCTTCAGGATC
CAGCCATTGCGGGTGCAGCGAGATGAAGCCATCTATGAGTGTACAGCTACTAACAGCCTGGGTGAGATCAACACTAGTGCCAAGCTC
TCAGTGCTCGAAGAGGAACAGCTGCCCCCTGGGTTCCCTTCCATCGACATGGGGCCTCAGCTGAAGGTGGTGGAGAAGGCACGCACA
GCCACCATGCTATGTGCCGCAGGCGGAAATCCAGACCCTGAGATTTCTTGGTTCAAGGACTTCCTTCCTGTAGACCCTGCCACGAGC
AACGGCCGCATCAAGCAGCTGCGTTCAGGTGCCTTGCAGATAGAGAGCAGTGAGGAATCCGACCAAGGCAAGTACGAGTGTGTGGCG
ACCAACTCGGCAGGCACACGTTACTCAGCCCCTGCGAACCTGTATGTGCGAGTGCGCCGCGTGGCCTCCTCGTTTCTCCATCCCTCCC
AGCAGCCAGGAGGTGATGCCAGGCGGCAGCGTGAACCTGACATGCGTGGCAGTGGGTCGCACCCATGCCCTACGTGAAGTGGATGATG
GGGGCCGAGGAGCTCACCAAGGAGGATGAGATGCCAGTTGGCCGCAACGTCCTGGAGCTCAGCAATGTCGTACGCTCTGCCAACTAC
ACCTGTGTGGCCATCTCCTCGCTGGGCATGATCGAGGCCACAGCCCAGGTCACAGTGAAAGCTCTTCCAAAGCCTCCGATTGATCTT
GTGGTGACAGAGACAACTGCCACCAGTGTCACCCTCACCTGGGACTCTGGGAACTCGGAGCCTGTAACCTACTATGGCATCCAGTAC
CGCGCAGCGGGCACGGAGGGCCCCTTTCAGGAGGTGGATGGTGGTGGCCACCACCCGCTACAGGCCATTGGCGGCCTCAGCCCTTTCTCG
GAATATGCCTTCCGCGTGCTGGCGGTGAACAGCATCGGGCGAGGGCCGCCCAGCCGAGGCAGTGCGGGCACGCAGGGACAGGCG
CCCTCCAGCCCACCGCGCCGCGTGCAGGCACGCATGCTGAGCGCCAGCACCATGCTGGTGCAGTGGGAGCCTCCCGAGGAGCCCAAC
GGCCTGGTGCGGGGATACCGCGTCTACTATACTCCGGACTCCCGCCGCCCCCCGAACGCCTGGCACAAGCACAACACCGACGCGGGG
CTCCTCACGACCGTGGGCAGCCTGCTGCCTGGCATCACCTACAGCCTGCGCGTGCTTGCCTTCACCGCCGTGGGCGATGGCCCTCCC
AGCCCCACCATCCAGGTCAAGACGCAGCAGGGAGTGCCCTGCCCAGCCCGCGGAGCTTCCAGGGAGCTGCTTCCAGGGAGCTGCTTCCAGGGAGCTG
```

242

EP 2 204 376 A2

```
GCAGTCCCAGGCCGGCCCACCATGATGATCAGCACCACGGCCATGAACACTGCGCTGCTCCAGTGGCACCCACCCAAGGAACTGCCT
GGCGAGCTGCTGGGCTACCGGCTGCAGTACTGCCGGGCCGACGAGGCGCGGCCCAACACCATAGATTTCGGCAAGGATGACCAGCAC
TTCACAGTCACCGGCCTGCACAAGGGGACCACCTACATCTTCCGGCTTGCTGCCAAGAACCGGGCTGGCTTGGGTGAGGAGTTCGAG
AAGGAGATCAGGACCCCCGAGGACCTGCCCAGCGGCTTCCCCCAAAACCTGCATGTGACAGGACTGACCACGTCTACCACAGAACTG
.GCCTGGGACCCGCCAGTGCTGGCGGAGAGGAACGGGCGCATCATCAGCTACACCGTGGTGTTCCGAGACATCAACAGCCAACAGGAG
CTGCAGAACATCACGACAGACACCCGCTTTACCCCTTACTGGCCTCAAGCCAGACACCACTTACGACATCAAGGTCCGCGCATGGACC
AGCAAAGGCTCTGGCCCACTCAGCCCCAGCATCCAGTCCCGGACCATGCCGGTGGAGCAAGTGTTTGCCAAGAACTTCCGGGTGGCG
GCTGCAATGAAGACGTCTGTGCTGCTCAGCTGGGAGGTTCCCGACTCCTATAAGTCAGCTGTGCCCTTTAAGATTCGTACAATGGGG
CAGAGTGTGGAGGTGGACGGGCATCTGATGCGGAAGCTGATCGCAGACCTGCAGCCCAACACAGAGTACTCGTTTGTGCTGATGAAC
CGTGGCAGCAGCGCAGGGGGCCTGCAGCACCTGGTGTCCATCCGCACAGCCCCCGACCTCCTGCCTCACAAGCCGCTGCCTGCCTCT
GCCTACATAGAGGACGGCCGCTTCGATCTCTCCATGCCCCATGTGCAAGACCCCTCGCTTGTCAGGTGGTTCTACATTGTTGTGGTA
CCCATTGACCGTGTGGGCGGGAGCATGCTGACGCCAAGGTGGAGCACACCCGAGGAACTGGAGCTGGACGAGCTTCTAGAAGCCATC
GAGCAAGGCGGAGAGGAGCAGCGGCGGCGGCGGCGGCAGGCAGAACGTCTGAAGCCATATGTGGCTGCTCAACTGGATGTGCTCCCG
GAGACCTTTACCTTGGGGGACAAGAAGAACTACCGGGGCTTCTACAACCGGCCCCTGTCTCCGGACTTGAGCTACCAGTGCTTTGTG
CTTGCCTCCTTGAAGGAACCCATGGACCAGAAGCGCTATGCCTCCAGCCCCTACTCGGATGAGATCGTGGTCCAGGTGACACCAGCC
CAGCAGCAGGAGGAGCCGGAGATGCTGTGGGTGACGGGTCCCGTGCTGGCAGTCATCCTCATCATCCTCATTGTCATCGCCATCCTC
TTGTTCAAAAGGAAAAGGACCCACTCTCCGTCCTCTAAGGATGAGCAGTCGATCGGACTGAAGGACTCCTTGCTGGCCCACTCCTCT
GACCCTGTGGAGATGCGGAGGCTCAACTACCAGACCCCAGGTATGCGAGACCACCCCACCCATCCCCATCACCGACCTGGCGGACAAC
ATCGAGCGCCTCAAAGCCAACGATGGCCTCAAGTTCTCCCAGGAGTATGAGTCCATCGACCCTGGACAGCAGTTCACGTGGGAGAAT
TCAAACCTGGAGGTGAACAAGCCCAAGAACCGCTATGCGAATATGTCATCGCCTACGACCACTCTCGAGTCATCCTTACCTCTATCGAT
GGCGTCCCCGGGAGTGACTACATCAATGCCAACTACATCGATGGCTACCGCAAGCAGAATGCCTACATCGCCACGCAGGGCCCCTG
CCCGAGACCATGGGCGATTTCTGGAGAATGGTGTGGGAACAGCGCACGGCCACTGTGGTCATGATGACACGGCTGGAGGAGAAGTCC
CGGGTAAAATGTGATCAGTACTGGCCAGCCCGTGGCACCGAGACCTGTGGCCTTATTCAGGTGACCCTGTTGGACACAGTGGAGCTG
GCCACATACACTGTGCGCACCTTCGCACTCCACAAGAGTGGCTCCAGTGAGAAGCGTGAGCTGCGTCAGTTTCAGTTCATGGCCTGG
CCAGACCATGGAGTTCCTGAGTACCCAACTCCCATCCTGCCTTCCTACGACGGGTCAAGGCCTGCAACCCCCTAGACGCAGGGCCC
ATGGTGGTGCACTGCAGCGCGGGCGTGGGCCGCACCGGCTGCTTCATCGTGATTGATGCCATGTTGGAGCGGATGAAGCACGAGAAG
ACGGTGGACATCTATGGCCACGTGACCTGCATGCGATCACAGAGGAACTACATGGTGCAGACGGAGGACCAGTACGTGTTCATCCAT
GAGGCGCTGCTGGAGGCTGCCACGTGCGGCCACACAGAGGTGCCTGCCCGCAACCTGTATGCCCACATCCAGAAGCTGGGCCAAGTG
CCTCCAGGGGAGAGTGTGACCGCCATGGAGCTCGAGTTCAAGTTGCTGGCCAGCTCCAAGGCCCACACGTCCCGCTTCATCAGCGCC
AACCTGCCCTGCAACAAGTTCAAGAACCGGCTGGTGAACATCATGCCCTACGAATTGACCCGTGTGTGTCTGCAGCCCATCCGTGGT
GTGGAGGGCTCTGACTACATCAATGCCAGCTTCCTGGATGGTTATAGACAGCAGAAGGCCTACATAGCTACACAGGGGCCTCTGGCA
GAGAGCACCGAGGACTTCTGGCGCATGCTATGGGAGCACAATTCCACCATCATCGTCATGCTGACCAAGCTTCGGGAGATGGGCAGG
GAGAAATGCCACCAGTACTGGCCAGCAGAGCGCTCTGCTCGCTACCAGTACTTTGTTGTTGACCCGATGGCTGAGTACAACATGCCC
CAGTATATCCTGCGTGAGTTCAAGGTCACGGATGCCCGGGATGGGCAGTCAAGGACAATCCGGCAGTTCCAGTTCACAGACTGGCCA
GAGCAGGGCGTGCCCAAGACAGCGGAGGGATTCATTGACTTCATCGGGCAGGTGCATAAGACCAAGGAGCAGTTTGGACAGGATGGG
CCTATCACGGTGCACTGCAGTGCTGGCGTGGGCCGCACCGGGGTGTTCATCACTCTGAGCATCGTCCTGGAGCGGATGCGCTACGAG
GGCGTGGTCGACATGTTTCAGACCGTGAAGACCCTGCGTACACAGCGTCCTGCCATGGTGCAGACAGAGGACCAGTATCAGCTGTGC
TACCGTGCGGCCCTGGAGTACCTCGGCAGCTTTGACCACTATGCAACGTAACTACCGCTCCCCTCTCCTCCGCCACCCCCGCCGTGG
GGCTCCGGAGGGGACCCAGCTCCTCTGAGCCATACCGACCATCGTCCAGCCCTCCTACGCAGATGCTGTCACTGGCAGAGCACAGCC
CACGGGGATCACAGCGTTTCAGGAACGTTGCCACACCAATCAGAGAGCCTAGAACATCCCTGGGCAAGTGGATGGCCCAGCAGGCAG
GCACTGTGGCCCTTCTGTCCACCAGACCCACCTGGAGCCCGCTTCAAGCCTCTGTTGCGCTCCCGCATTTCTCATGCTCTTCTCA
TGGGGTGGGGTTGGGGCAAAGCCTCCTTTTTAATACATTAAGTGGGGTAGACTGAGGGATTTTAGCCTCTTCCCTCTGATTTTTCCT
TTCGCGAATCCGTATCTGCAGAATGGGCCACTGTAGGGGTTGGGGGTTTATTTTGTTTTGTTTTTTTTTTTCTTGATTTGTATGACTT
CTGCTGAAGGACAGAACATTGCCTTCCTCGTGCAGAGCTGGGGCTGCCAGCCTGAGCGGAGGCTCGGCCGTGGGCCGGGAGGCAGTG
CTGATCCGGCTGCTCCTCCAGCCCTTCAGACGGAGATCCTGTTTCAGCTAAATGCAGGGAAACTCAATGTTTTTTTAAGTTTTGTTTT
CCCTTTAAAGCCTTTTTTTAGGCCACATTGACAGTGGTGGGCGGGGAGAAGATAGGGAACACTCATCCCTGGTCGTCTATCCCAGTG
TGTGTTTAACATTCACAGCCCAGAACCACAGATGTGTCTGGGAGAGCCTGGCAAGGCATTCCTCATCACCATCGTGTTTGCAAAGGT
TAAAACAAAACAAAAAACCACAAAAATAAAAAACAAAAAAAACAAAAAACCCAAGAAAAAAAAAAAGAGTCAGCCCTTGGCTTCTG
CTTCAAACCCTCAAGAGGGGAAGCAACTCCGTGTGCCTGGGGTTCCCGAGGGAGCTGCTGGCTGACCTGGGCCCACAGAGCCTGGCT
TTGGTCCCCAGCATTGCAGTATGGTGGTGGGTTGTTTGGGGCTGTGGGGTCTGGCTGTGTGGCCAAGGTGAATAGCACAGGTTAGGGTG
TGTGCCACACCCCATGCACCTCAGGGCCAAGCGGGGGCGTGGCCTTTCAGGTCCAGGCCAGTGGGCCTGGTAGCACATGTCTG
TCCTCAGAGCAGGGGCCAGATGATTTTCCTCCCTGGTTTGCAGCTGTTTTCAAAGCCCCCGATAATCGCTCTTTTCCACTCCAAGAT
GCCCTCATAAACCAATGTGGCAAGACTACTGGACTTCTATCAATGGTACTCTAATCAGTCCTTATTATCCCAGCTTGCTGAGGGGCA
GGGAGAGCGCCTCTTCCTCTGGGCAGCGCTATCTAGATAGGTAAGTGGGGGCGGGGAAGGGTGCATAGCTGTTTTAGCTGAGGGACG
TGGTGCCGACGTCCCCAAACCTAGCTAGGCTAAGTCAAGATCAACATTCCAGGGTTGGTAATGTTGGATGATGAAACATTCATTTTT
ACCTTGTGGATGCTAGTGCTGTAGAGTTCACTGTTGTACACAGTCTGTTTTTCTATTTGTTAAGAAAAACTACAGCATCATTGCATAA
TTCTTGATGGTAATAAATTTGAATAATCAGATTTCTTAC


HUMAN PROTEIN SEQUENCE : hP28-021.1 (Seq ID No: 68)
MAPEPAPGRTMVPLVPALVMLGLVGAHGDSKPVFIKVPEDQTGLSGGVASFVCQATGEPKRITWMKKGKKVSSQRFEVIEFDDGA
GSVLRIQPLRVQRDEAIYECTATNSLGEINTSAKLSVLEEEQLPPGFPSIDMGPQLKVVEKARTATMLCAAGGNPDPEISWFKDFLP
VDPATSNGRIKQLRSGALQIESSEESDQGKYECVATNSAGTRYSAPANLYVRVRRVAPRFSIPPSSQEVMPGGSVNLTCVAVGAPMP
YVKWMMGAEELTKEDEMPVGRNVLELSNVVRSANYTCVAISSLGMIEATAQVTVKALPKPPIDLVVTETTATSVTLTWDSGNSEPVT
YYGIQYRAAGTEGPFQEVDGVATTRYSIGGLSPFSEYAFRVLAVNSIGRGPPSEAVRARTGEQAPSSPPRRVQARMLSASTMLVQWE
PPEEPNGLVRGYRVYYTPDSRRPPNAWHKHNTDAGLLTTVGSLLPGITYSLRVLAFTAVGDGPPSPTIQVKTQQGVPAQPADFQAEV
ESDTRIQLSWLLPPQERIIMYELVYWAAEDEDQQHKVTFDPTSSYTLEDLKPDTLYRFQLAARSDMGVGVFTPTIEARTAQSTPSAP
PQKVMCVSMGSTTVRVSWVPPPADSRNGVITQYSVAYEAVDGEDRGRHVVDGISREHSSWDLVGLEKWTEYRVWVRAHTDVGPGPES
SPVLVRTDEDVPSGPPRKVEVEPLNSTAVHVYWKLPVPSKQHGQIRGYQVTYVRLENGEPRGLPIIQDVMLAEAQWRPEESEDYETT
ISGLTPETTYSVTVAAYTTKGDGARSKPKIVTTTGAVPGRPTMMISTTAMNTALLQWHPPKELPGELLGYRLQYCRADEARPNTIDF
GKDDQHFTVTGLHKGTTYIFRLAAKNRAGLGEEFEKEIRTPEDLPSGFPQNLHVTGLTTSTTELAWDPPVLAERNGRIISYTVVFRD
INSQQELQNITTDTRFTLTGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIEDGRFDLSMPHVQDPSLVRW
FYIVVVPIDRVGGSMLTPRWSTPEELELDELLEAIEQGGEEQRRRRRQAERLKPYVAAQLDVLPETFTLGDKKNYRGFYNRPLSPDL
SYQCFVLASLKEPMDQKRYASSPYSDEIVVQVTPAQQQEEPEMLWVTGPVLAVILIILIVIAILLFKRKRTHSPSSKDEQSIGLKDS
LLAHSSDPVEMRRLNYQTPGMRDHPPIPITDLADNIERLKANDGLKFSQEYESIDPGQQFTWENSNLEVNKPKNRYANVIAYDHSRV
ILTSIDGVPGSDYINANYIDGYRKQNAYIATQGPLPETMGDFWRMVWEQRTATVVMMTRLEEKSRVKCDQYWPARGTETCGLIQVTL
```

243

```
LDTVELATYTVRTFALHKSGSSEKRELRQFQFMAWPDHGVPEYPTPILAPLRRVKACNPLDAGPMVVHCSAGVGRTGCFIVIDAMLE
RMKHEKTVDIYGHVTCMRSQRNYMVQTEDQYVFIHEALLEAATCGHTEVPARNLYAHIQKLGQVPPGESVTAMELEFKLLASSKAHT
SRFISANLPCNKFKNRLVNIMPYELTRVCLQPIRGVEGSDYINASFLDGYRQQKAYIATQGPLAESTEDFWRMLWEHNSTIIVMLTK
LREMGREKCHQYWPAERSARYQYFVVDPMAEYNMPQYILREFKVTDARDGQSRTIRQFQFTDWPEQGVPKTGEGFIDFIGQVHKTKE
QFGQDGPITVHCSAGVGRTGVFITLSIVLERMRYEGVVDMFQTVKTLRTQRPAMVQTEDQYQLCYRAALEYLGSFDHYAT*


HUMAN mRNA SEQUENCE : hR28-021.2 (Seq ID No: 69)
GGCGCCGCCGGGGGCGCGCACGGCAGGGGCAGGGGCGCGGGCGCGAGCGCGAGGGGAGCGCGCGGCTGGAGCTGGCGCGGGAGCGGC
GGGAGCGGTGGCGGCGGCAGAGGCGGCGGCTCCAGCTTCGGCTCCGGCTCGGGCTCGGGCTCCGGCTCCGGCTCCGGCTCCGGCTCC
AGCTCGGGTGGCGGTGGCGGGAGCGGGACCAGGTGGAGGCGGCGGCGGCAGAGGAGTGGGAGCAGCGGCCCTAGCGGCTTGCGGGGG
GACATGCGGACCGACGGCCCCTGGATAGGCGGGAAGGAGTGGAGGCCCTGGTGCCCGGCCCTTGGTGCTGAGTATCCAGCAAGAGTGA
CCGGGGTGAAGAAGCAAAGACTCGGTTGATTGTCCTGGGCTGTGGCTGTGGACTAGAGCCCTGGATGGCCCCTGAGCCAGCC
CCAGGGAGGACGATGGTGCCCCTTGTGCCTGCACTGGTGATGCTTGGTTTGGTGGCAGGCGCCCATGGTGACAGCAAACCTGTCTTC
ATTAAAGTCCCTGAGGACCAGACTGGGCTGTCAGGAGGGGTAGCCTCCTTCGTGTGCCAAGCTACAGGAGAACCCAAGCCGCGCATC
ACATGGATGAAGAAGGGGAAGAAAGTCAGCTCCCAGCGCTTCGAGGTCATTGAGTTTGATGATGGGCAGGGTCAGTGCTTCGGATC
CAGCCATTGCGGGGTGCAGCGAGATGAAGCCATCTATGAGTGTACAGCTACTAACAGCCTGGGTGAGATCAACACTAGTGCCAAGCTC
TCAGTGCTCGAAGAGGAACAGCTGCCCCCTGGGTTCCCTTCCATCGACATGGGGCCTCAGCTGAAGGTGGTGGAGAAGGCACGCACA
GCCACCATGCTATGTGCCGCAGGCGGAAATCCAGACCCTGAGATTTCTTGGTTCAAGGACTTCCTTCCTGTAGACCCTGCCACGAGC
AACGGCCGCATCAAGCAGCTGCGTTCAGGTGCCTTGCAGATAGAGAGCAGTGAGGAATCCGACCAAGGCAAGTACGAGTGTGTGGCG
ACCAACTCGGCAGGCACACGTTACTCAGCCCCTGCGAACCTGTATGTGCGAGTGCGCCGCGTGGCTCCTCGTTTCTCCATCCCTCCC
AGCAGCCAGGAGGTGATGCCAGGCGGCAGCGTGAACCTGACATGCGTGGCAGTGGGTGCACCCATGCCCTACGTGAAGTGGATGATG
GGGGCCGAGGAGCTCACCAAGGAGGATGAGATGCCAGTTGGCCGCAACGTCCTGGAGCTCAGCAATGTCGTACGCTCTGCCAACTAC
ACCTGTGTGGCCATCTCCTCGCTGGGCATGATCGAGGCCACAGCCCAGGTCACAGTGAAAGCTCTTCCAAAGCCTCCGATTGATCTT
GTGGTGACAGAGACAACTGCCACCAGTGTCACCCTCACCTGGGACTCTGGGAACTCGGAGCCTGTAACCTACTATGGCATCCAGTAC
CGCGCAGCGGGCACGGAGGGCCCCTTTCAGGAGGTGGATGGTGTGGCCACCACCCGCTACAGCATTGGCGGCCTCAGCCCTTTCTCG
GAATATGCCTTCCGCGTGCTGGCGGTGAACAGCATCGGGCGAGGGCCGCCCAGCGAGGCAGTGCGGGCACGCACGGGAGAACAGGCG
CCCTCCAGCCCACCGCCGCCGTGCAGGCACGCATGCTGAGCGCCACAGCCATGCTGGTGCAGTGGCAGCCTCCCGAGGAGCCCAAC
GGCCTGGTGCGGGGATACCGCGTCTACTATACTCCGGACTCCCGCCGCCCCCCGAACGCCTGGCACAAGCAACACCGACGCGGGG
CTCCTCACGACCGTGGGCAGCCTGCTGCCTGGCATCACCTACAGCCTGCGCGTGCTTGCCTTCACCGCCGTGGGCGATGGCCCTCCC
AGCCCCACCATCCAGGTCAAGACGCAGCAGGGAGTGCCTGCCCAGCCCGCGGACTTCCAGGCCGAGGTGGAGTCGGACACCAGGATC
CAGCTCTCGTGGCTGCTGCCCCCTCAGGAGCGGATCATCATGTATGAACTGGTGTACTGGGCGGCAGAGGACGAAGACCAACAGCAC
AAGGTGACCTTCGACCCAACCTCCTCCTACACACTAGAGGACCTGAAGCCTGACACACTCTACCGGTTCCAGCTGGCTGCACGCTCG
GATATGGGGGTGGGCGTCTTCACCCCCACCATTGAGGCCCGCACAGCCCAGTCCACCCCCTCGCCCCTCCCCAGAAGGTGATGTGT
GTGAGCATGGGCTCCACCACGGTCCGGGTAAGTTGGGTCCCGCCGCCTGCCGACAGCCGCAACGGCGTTATCACCCAGTACTCCGTG
GCCTACGAGGCGGTGGACGGCGAGGACCGCGGGCGGCATGTGGTGGATGGCATCAGCCGTGAGCACTCCAGCTGGGACCTGGTGGGC
CTGGAGAAGTGGACGGAGTACCGGGTGTGGGTGCGGGCACACACAGACGTGGGCCCCGGCCCCGAGAGCAGCCCGGTGCTGGTGCGC
ACCGATGAGGACGTGCCCAGCGGGCCTTCCGCGGCAGGTGGAGGCCACTGAACTCCACTGCTGTGCATGTCTACTGGAAGCTG
CCTGTCCCCAGCAAGCAGCATGCCCAGATCCGCGGCTACCAGGTCACCTACGTGCGGCTGGGAATGGCGAGCCCGTGGACTCCCC
ATCATCCAAGACGTCATGCTAGCCGAGGCCCAGGAAACCACTATCAGCGGCCTGACCCCGGAGACCACCTACTCCGTTACTGTTGCT
GCCTATACCACCAAGGGGGATGGTGCCCGCAGCAAGCCCAAAATTGTCACTACAACAGGTGCAGTCCCAGGCCGGCCCACCATGATG
ATCAGCACCACGGCCATGAACACTGCGCTGCTCCAGTGGCACCCACCCAAGGAACTGCCTGGCGAGCTGCTGGGCTACCGGCTGCAG
TACTGCCGGGCCGACGAGGCCGGCCCAACACCATAGATTTCGGCAAGGATGACCAGCACTTCACAGTCACCGGCCTGCACAAGGAG
ACCACCTACATCTTCCGGCTTGCTGCCAAGAACCGGGCTGGCTTGGGTGAGGAGTTCGAGAAGGAGATCAGGACCCCCGAGGACCTG
CCCAGCCGGCTTCCCCCAAAACCTGCATGTGACAGGACTGACCACGTCTACCACAGAACTGGCCTGGGACCCGCCAGTGCTGGCGGAG
AGGAACGGGCGCATCATCAGCTACACCGTGGTGTTCCGAGACATCAACAGCCAACAGGAGCTGCAGAACATCACGACAGACACCCGC
TTTACCCTTACTGGCCTCAAGCCAGACACCACTTACGACATCAAGGTCCGCGCATGGACCAGCAAAGGCTCTGGCCCACTCAGCCCC
AGCATCCAGTCCCAGACCATGCCGGTGGCAAGTGTTTGCCAAGAACTTCCGGGTGGCGGCTGCAATGAAGACGTCTGTGCTGCTC
AGCTGGGAGGTTCCCGACTCCTATAAGTCAGCTGTGCCCTTTAAGATTCTGTACAATGACGCAGAGTGTGGAGGTGGACGGGCACTCG
ATGCGGAAGCTGATCGCAGACCTGCAGCCCAACACAGAGTACTCGTTTGTGCTGATGAACCGTGGCAGCAGCGCAGGGGGCCTGCAG
CACCTGGTGTCCATCCGCACAGCCCCCGACCTCCTGCCTCACAAGCCGCTGCCTGCCTCTGCCTACATAGAGGACGGCCGCTTCGAT
CTCTCCATGCCCCATGTGCAAGACCCCTCGCTTGTCAGGTGGTTCTACATTGTTGTGGTACCCATTGACCGTGTGGGCGGGAGCATG
CTGACGCCAAGGTGGAGACCACCCGAGGAACTGGAGCTGGACGGCTTCTAGAAGCCATCGAGCAAGGCGGAGAGGAGCAGCGGCGG
CGGCGGCGGCAGGCAGAACGTCTGAAGCCCATATGTGGCTGCTCAACTGGATGTGCTCCCGGAGACCTTTACCTTGGGGGACAAGAAG
AACTACCGGGGCTTCTACAACCGGCCCCTGTCTCCGGACTTGAGCTACCAGTGCTTTGTGCTTGCCTCCTTGAAGGAACCCATGGAC
CAGAAGCGCTATGCCTCCAGCCCCTACTCGGATGAGATCGTGGTCCAGGTGACACCAGCCCAGCAGCAGGAGGAGCCGGAGATGCTG
TGGGTGACGGGTCCCGTGCTGGCAGTCATCCTCATCATCCTCATTGTCATCGCCATCCTCTTGTTCAAAAAGGAAAAGGACCCACTCT
CCGTCCTCTAAGGATGAGCAGTCGATCGGACTGAAGGACTCCTTGCTGGCCCACTCCTCTGACCCTGTGGAGATGCGGAGCGTCAAC
TACCAGACCCCCAGGTATGCGAGACCACCCACCCATCCCCATCACCGACCTGGCGGACAACAACTGAGCGCCTCAAAGCCAACGATGGC
CTCAAGTTCTCCCAGGAGTATGAGTCCATCGACCCTGGACAGCAGTTCACGTGGGAGAATTCAAACCTGGAGGTGAACAAGCCCAAG
AACCGCTATGCGAATGTCATCGCCTACGACCACTCTCGAGTCATCCTTACCTCTATCGATGGCGTCCCCGGGAGTGACTACATCAAT
GCCAACTACATCGATGGCTACCGCAAGCAGAATGCCTACATCGCCACGCAGGGCCCCCTGCCCGAGACCATGGGCGATTTCTGGAGA
GCCCGTGGCACCGAGACCGTGTGGCCTTATTCAGGTGACCCTGTTGGACACAGTGGAGCTGGCCACATACACTGTGCGCACCTTCGCA
CTCCACAAGAGTGGCTCCAGTGAGAAGCGTGAGCTGCGTCAGTTTCAGTTCATGGCCTGGCCAGACCATGGAGTTCCTGAGTACCCA
ACTCCCATCCTGGCCTTCCTACGACGGGTCAAGGCCTGCAACCCCCTAGACGCAGGGCCCATGGTGGTCACTGCAGCGCGGGCGTG
GGCCGCACCGGCTGCTTCATCGTGATTGATGCCATGTTGGAGCGGATGAAGCACGAGAAGACGGTGGACATCTATGGCCACGTGACC
TGCATGCGATCACAGAGGAACTACATGGTGCAGACGGAGGACCAGTACGTGTTCATCCATGAGGCGCTGCTGGAGGCTGCCACGTGC
GGCCACACAGAGGTGCCTGCCCGCAACCTGTATGCCCACATCCAGAAGCTGGGCCAAGTGCCTCCAGGGGAGAGTGTGACCGCCATG
GAGCTCGAGTTCAAGTTGCTGGCCAGCTCCAAGGCCACACGTCCCGCTTCATCAGCGCCAACCTGCCCTGCAACAAGTTCAAGAAC
CGGCTGGTGAACATCATGCCCTACGAATTGACCCGTGTGTGTCTGCAGCCCATCCGTGGTGTGGAGGGCTCTGACTACATCAATGCC
AGCTTCCTGGATGGTTATAGACAGCAGAAGGCCTACATAGCTACACAGGGGCCTCTGGCAGAGAGCACCGAGGACTTCTGGCGCATG
CTATGGGAGCACAATTCCACCATCATCGTCATGCTGACCAAGCTTCGGGAGATGGGCAGGGAGAAATGCCACCAGTACTGGCCAGCA
GAGCGCTCTGCCTACCAGTACTTTGTTGTGGACCCGATGGCTGAGTACAACATGCCCCAGTATATCCTGCGTGAGTTCAAGGTC
ACGGATGCCCGGGATGGGCAGTCAAGGACAATCCGGCAGTTCCAGTTCACAGACTGGCCAGAGCAGGGCGTGCCCAAGACAGGCGAG
GGATTCATTGACTTCATCGGGCAGGTGCATAAGACCAAGGAGCAGTTTGGACAGGATGGGCCTATCACGGTGCACTGCAGTGCTGGC
GTGGGCCGCACCGGGGTGTTCATCACTCTCTGAGCATCGTCCTGGAGCGCATGCGCTACGAGGGCGTGGTCGACATGTTTCAGACCGTG
```

244

```
AAGACCCTGCGTACACAGCGTCCTGCCATGGTGCAGACAGAGGACCAGTATCAGCTGTGCTACCGTGCGGCCCTGGAGTACCTCGGC
AGCTTTGACCACTATGCAACGTAACTACCGCTCCCCTCTCCTCCGCCACCCCCGCCGTGGGGCTCCGGAGGGGACCCAGCTCCTCTG
AGCCATACCGACCATCGTCCAGCCCTCCTACGCAGATGCTGTCACTGGCAGAGCACAGCCCACGGGGATCACAGCGTTTCAGGAACG
TTGCCACACCAATCAGAGAGCCTAGAACATCCCTGGGCAAGTGGATGGCCCAGCAGGCAGGCACTGTGGCCCTTCGTCCACCAGAC
CCACCTGGAGCCCGCTTCAAGCTCTCTGTTGCGCTCCCGCATTTCTCATGCTTCTTCTCATGGGGTGGGGTTGGGGCAAAGCCTCCT
TTTTAATACATTAAGTGGGGTAGACTGAGGGATTTTAGCCTCTTCCCTCTGATTTTTCCTTTCGCGAATCCGTATCTGCAGAATGGG
CCACTGTAGGGGTTGGGGTTTATTTTGTTTTGTTTTTTTTTTTTCTTGATTTGTATGACTTCTGCTGAAGGACAGAACATTGCCTTCC
TCGTGCAGAGCTGGGGCTGCCAGCCTGAGCGGAGGCTCGGCCGTGGGCCGGGAGGCAGTGCTGATCCGGCTGCTCCTCCAGCCCTTC
AGACGAGATCCTGTTTCAGCTAAATGCAGGGAAACTCAATGTTTTTTTAAGTTTTGTTTTCCCTTTAAAGCCTTTTTTTAGGCCACA
TTGACAGTGGTGGGCGGGGAGAAGATAGGGAACACTCATCCCTGGTCGTCTATCCCAGTGTGTGTTTAACATTCACAGCCCAGAACC
ACAGATGTGTCTGGGAGAGCCTGGCAAGGCATTCCTCATCACCATCGTGTTTGCAAAGGTTAAAACAAAAACAAAAAACCACAAAAA
TAAAAAAACAAAAAAAAACAAAAAAACCCAAGAAAAAAAAAAAAAGAGTCAGCCCTTGGCTTCTGCTTCAAACCCTCAAGAGGGGAAGCAAC
TCCGTGTGCCTGGGGTTCCCGAGGGAGCTGCTGGCTGACCTGGGCCCACAGAGCCTGGCTTTGGTCCCCAGCATTGCAGTATGGTGT
GGTGTTTGTAGGCTGTGGGGTCTGGCTGTGTGGCCAAGGTGAATAGCACAGGTTAGGGTGTGTGCCACACCCCATGCACCTCAGGGC
CAAGCGGGGGCGTGGCTGGCCTTTCAGGTCCAGGCCAGTGGGCCTGGTAGCACATGTCTGTCCTCAGAGCAGGGGCCAGATGATTTT
CCTCCCTGGTTTGCAGCTGTTTTCAAAGCCCCCGATAATCGCTCTTTTCCCACTCCAAGATGCCCTCATAAACCAATGTGGCCAAGACT
ACTGGACTTCTATCAATGGTACTCTAATCAGTCCTTATTATCCCAGCTTGCTGAGGGGCAGGGAGAGCGCCCTCTTCCTCTGGGCAGC
GCTATCTAGATAGGTAAGTGGGGGCGGGGAAGGGTGCATAGCTGTTTTAGCTGAGGGACGTGGTGCCGACGTCCCCAAACCTAGCTA
GGCTAAGTCAAGATCAACATTCCAGGGTTGGTAATGTTGGATGATGAAACATTCATTTTTACCTTGTGGATGCTAGTGCTGTAGAGT
TCACTGTTGTACACAGTCGTGTTTTCTATTTGTTAAGAAAAACTACAGCATCATTGCATAATTCTTGATGGTAATAAATTTGAATAAT
CAGATTTCTTAC
```

HUMAN PROTEIN SEQUENCE : hP28-021.2 (Seq ID No: 70)

```
MAPEPAPGRTMVPLVPALVMLGLVAGAHGDSKPVFIKVPEDQTGLSGGVASFVCQATGEPKPRITWMKKGKKVSSQRFEVIEFDDGA
GSVLRIQPLRVQRDEAIYECTATNSLGEINTSAKLSVLEEEQLPPGFPSIDMGPQLKVVEKARTATMLCAAGGNPDPEISWFKDFLP
VDPATSNGRIKQLRSGALQIESSEESDQGKYECVATNSAGTRYSAPANLYVRVRRVAPRFSIPPSSQEVMPGGSVNLTCVAVGAPMP
YVKWMMGAEELTKEDEMPVGRNVLELSNVVRSANYTCVAISSLGMIEATAQVTVKALPKPPIDLVVTETTATSVTLTWDSGNSEPVT
YYGIQYRAAGTEGPFQEVDGVATTRYSIGGLSPFSEYAFRVLAVNSIGRGPPSEAVRARTGEQAPSSPPRRVQARMLSASTMLVQWE
PPEEPNGLVRGYRVYYTPDSRRPPNAWHKHNTDAGLLTTVGSLLPGITYSLRVLAFTAVGDGPPSPTIQVKTQQGVPAQPADFQAEV
ESDTRIQLSWLLPPQERIIMYELVYWAAEDEDQQHKVTFDPTSSYTLEDLKPDTLYRFQLAARSDMGVGVFTPTIEARTAQSTPSAP
PQKVMCVSMGSTTVRVSWVPPPADSRNGVITQYSVAYEAVDGEDRGRHVVDGISREHSSWDLVGLEKWTEYRVWVRAHTDVGPGPES
SPVLVRTDEDVPSGPPRKVEVEPLNSTAVHVYWKLPVPSKQHGQIRGYQVTYVRLENGEPRGLPIIQDVMLAEAQETTISGLTPETT
YSVTVAAYTTKGDGARSKPKIVTTTGAVPGRPTMMISTTAMNTALLQWHPPKELPGELLGYRLQYCRADEARPNTIDFGKDDQHFTV
TGLHKGTTYIFRLAAKNRAGLGEEFEKEIRTPEDLPSGFPQNLHVTGLTTSTTELAWDPPVLAERNGRIISYTVVFRDINSQQELQN
ITTDTRFTLTGLKPDTTYDIKVRAWTSKGSGPLSPSIQSRTMPVEQVFAKNFRVAAAMKTSVLLSWEVPDSYKSAVPFKILYNGQSV
EVDGHSMRKLIADLQPNTEYSFVLMNRGSSAGGLQHLVSIRTAPDLLPHKPLPASAYIEDGRFDLSMPHVQDPSLVRWFYIVVVPID
RVGGSMLTPRWSTPEELELDELLEAIEQGGEEQRRRRQAERLKPVAAQLDVLPETFTLGDKKNYRGFYNRPLSPDLSYQCFVLAS
LKEPMDQKRYASSPYSDEIVVQVTPAQQQEEPEMLWVTGPVLAVILIILIVIAILLFKRKRTHSPSSKDEQSIGLKDSLLAHSSDPV
EMRRLNYQTPGMRDHPPIPITDLADNIERLKANDGLKFSQEYESIDPGQQFTWENSNLEVNKPKNRYANVIAYDHSRVILTSIDGVP
GSDYINANYIDGYRKQNAYIATQGPLPETMGDFWRMVWEQRTATVVMMTRLEEKSRVKCDQYWPARGTETCGLIQVTLLDTVELATY
TVRTFALHKSGSSEKRELRQFQFMAWPDHGVPEYPTPILAFLRRVKACNPLDAGPMVVHCSAGVGRTGCFIVIDAMLERMKHEKTVD
IYGHVTCMRSQRNYMVQTEDQYVFIHEALLEAATCGHTEVPARNLYAHIQKLGQVPPGESVTAMELEFKLLASSKAHTSRFISANLP
CNKFKNRLVNIMPYELTRVCLQPIRGVEGSDYINASFLDGYRQQKAYIATQGPLAESTEDFWRMLWEHNSTIIVMLTKLREMGREKC
HQYWPAERSARYQYFVVDPMAEYNMPQYILREFKVTDARDGQSRTIRQFQFTDWPEQGVPKTGEGFIDFIGQVHKTKEQFGQDGPIT
VHCSAGVGRTGVFITLSIVLERMRYEGVVDMFQTVKTLRTQRPAMVQTEDQYQLCYRAALEYLGSFDHYAT*
```

HUMAN mRNA SEQUENCE : hR28-021.3 (Seq ID No: 71)

```
GCGTGTGTGGACTGAAACCCAAAAGCCAGAAACTCTCTGCATGTGTCCCTCATGGAGCCATATGGTCTCTAGTCTCTGTTTTTCAAA
ATTTGCAGGACTGATGACTGATTGGACAGTGGTGCAGGAGGGAAGGTCTCTGCATGTCAGTGATTAAATTGCTGAAGATGCCACTGC
CTGAGACGGGCAGTATTGAAGGAAGGAGTGGAGGCCCTGGTGCCCGGCCCTTGGTGCTGAGTATCCAGCAAGAGTGACCGGGGTGAA
GAAGCAAAGACTCGGTTGATTGTCCTGGGCTGTGGCTGGCTGTGGAGCTAGAGCCCTGGATGGCCCCTGAGCCAGCCCCAGGGAGGA
CGATGGTGCCCCTTGTGCCTGCACTGGTGATGCTTGGTTTGGTGGCAGGCGCCCATGGTGACAGCAAACCTGTCTTCATTAAAGTCC
CTGAGGACCAGACTGGGCTGTCAGGAGGGGTAGCCTCCTTCGTGTGCCAAGCTACAGGAGAACCCAAGCCGCGCATCACATGGATGA
AGAAGGGGAAGAAAGTCAGCTCCCAGCGCTTCGAGGTCATTGAGTTTGATGATGGGGCAGGGTCAGTGCTTCGGATCCAGCCATTGC
GGGGTGCAGCGAGATGAAGCCATCTATGAGTGTACAGCTACTAACAGCCTGGGTGAGATCAACACTAGTGCCAAGCTCTCAGTGCTCG
AAGAGGAACAGCTGCCCCCTGGGTTCCCTTCCATCGACATGGGGCCTCAGCTGAAGGTGGTGGAGAAGGCACGCACAGCCACCATGC
TATGTGCCGCAGGCGGAAATCCAGACCCTGAGATTTCTTGGTTCAAGGACTTCCTTCCTGTAGACCCGTGCCACGAGCAAGGCCGCCA
TCAAGCAGCTGCGTTCAGGTGCCTTGCAGATAGAGAGCAGTGAGGAATCCGACCAAGGCAAGTACGAGTGTGTGGCGACCAACTCGG
CAGGCACACGTTACTCAGCCCCTGCGAACCTGTATGTGCGAGTGCGCCGCGTGGCTCCTCGTTTCTCCATCCCTCCCAGCAGCCAGG
AGGTGATGCCAGGCGGCAGCGTGAACCTGACATGCGTGGCAGTGGGTGCACCCATGCCCTACGTGAAGTGGATGATGGGGGCCGAGG
AGCTCACCAAGGAGGATGAGATGCCAGTTGGCCGCAACGTCCTGGAGCTCAGCAATGTCGTACGCTCTGCCAACTACACCTGTGTGG
CCATCTCCTCGCTGGGCATGATCGAGGCCACAGCCCAGGTCACAGTGAAAGCTCTTCCAAAGCCTCCGATTGATCTTGTGGTGACAG
AGACAACTGCCACCAGTGTCACCCTCACCTGGGACTCTGGGAACTCGGAGCCTGTAACCTACTATGGCATCCAGTACCGCGCAGCGG
GCACGGAGGGCCCCTTTCAGGAGGTGGATGGTGTGGCCACCACCCGCTACAGCATTGGCGGCCTCAGCCCTTTCTCGGAATATGCCT
TCCGCGTGCTGGCCGTGAACAGCATCGGGCGAGGGCCGCCCAGCGAGGCAGTGCGGGCACGCACGGGAGAACAGGCGCCCTCCAGCC
CACCGCGCCGCGTGCAGGCACGCATGCTGAGCGCCAGCACCATGCTGGTGCAGTGGGAGCCTCCCGAGGAGCCCAACGGCCTGGTGC
GGGGATACCGCGTCTACTATACTCCGGACTCCCGCCGCCCCCCGAACGCCTGGCACAAGCACAACACCGACGCGGGGCTCCTCACGA
CCGTGGGCAGCCTGCTGCCTGGCATCACCTACAGCCTGCGCGTGCTGGCCTTCACCGCGTGGGCGATGGCCCTCCCAGCCCCACCA
TCCAGGTCAAGACGCAGCAGGGAGTGCCTGCCCAGCCCGCGGACTTCCAGGCCGAGGTGGAGTCGGACACCAGGATCCAGCTCTCGT
GGCTGCTGCCCCCTCAGGAGCGGATCATCATGTATGAACTGGTGTACTGGGCGGCAGAGGACGAAGACCAACAGCACAAGGTGACCT
TCGACCCAACCTCCTCCTACACACTAGAGGACCTGAAGCCTGACACACTCTACCGCTTCCAGCTGGCTGCACGCTCGGATATGGGGG
TGGGCGTCTTCACCCCCACCATTGAGGCCCGACAGCCCAGTCCACCCCTCCGCCCCTCCCCAGAAGGTGATGTGTGTGAGCATGG
GCTCCACCACGGTCCGGGTAAGTTGGGTCCCGCCGCCGTCCGCGACAGCCGCAACGGCGTTATCACCCAGTACTCCGTGGCCTACGAGG
CGGTGGACGGCGAGGACCGCGGGCGGCATGTGGTGGATGGCATCAGCCGTGAGCACTCCAGCTGGGACCTGGTGGGCCTGGAGAAGT
GGACGGAGTACCGGGTGTGGGTGCGGGCACACACAGACGTGGGCCCCGGCCCCGAGAGCAGCCCGGTGCTGGTGCGCACCGATGAGG
ACGTGCCCAGCGGGCCTCCGCGGAAGGTGGAGGTGGAGCCACTGAACTCCACTGCTGTGCATGTCTACTGGAAGCTGCCTGTCCCCA
```

```
GCAAGCAGCATGGCCAGATCCGCGGCTACCAGGTCACCTACGTGCGGCTGGAGAATGGCGAGCCCCGTGGACTCCCCATCATCCAAG
ACGTCATGCTAGCCGAGGCCCAGGAAACCACTATCAGCGGCCTGACCCCGGAGACCACCTACTCCGTTACTGTTGCTGCCTATACCA
CCAAGGGGGATGGTGCCCGCAGCAAGCCCAAAATTGTCACTACAACAGGTGCAGTCCCAGGCCGGCCCACCATGATGATCAGCACCA
CGGCCATGAACACTGCGCTGCTCCAGTGGCACCCACCCAAGGAACTGCCTGGCGAGCTGCTGGGCTACCGGCTGCAGTACTGCCGGG
CCGACGAGGCGCGGCCCAACACCATAGATTTCGGCAAGGATGACCAGCACTTCACAGTCACCGGCCTGCACAAGGGCACCACCTACA
TCTTCCGGCTTGCTGCCAAGAACCGGGCTGGCTTGGGTGAGGAGTTCGAGAAGGAGATCAGGACCCCCGAGGACCTGCCCACGCGGCT
TCCCCCAAAACCTGCATGTGACAGGACTGACCACGTCTACCACAGAACTGGCCTGGGACCCGCCAGTGCTGGCGGAGAGGAACGGGC
GCATCATCAGCTACACCGTGGTGTTCCGAGACATCAACAGCCAACAGGAGCTGCAGAACATCACGACAGACACCCGCTTTACCCTTA
CTGGCCTCAAGCCAGACACCACTTACGACATCAAGGTCCGCGCATGGACCAGCAAAGGCTCTGGCCCACTCAGCCCCAGCATCCAGT
CCCGGACCATGCCGGTGGAGCAAGTGTTTGCCAAGAACTTCCGGGTGGCGGCTGCAATGAAGACGTCTGTGCTGCTCAGCTGGGAGG
TTCCCGACTCCTATAAGTCAGCTGTGCCCTTTAAGATTCTGTACAATGGGCAGAGTGTGGAGGTGGACGGGCACTCGATGCGGAAGC
TGATCGCAGACCTGCAGCCCAACACAGAGTACTCGTTTGTGCTGATGAACCGTGGCAGCAGCGCAGGGGGCCTGCAGCACCTGGTGT
CCATCCGCACAGCCCCCGACCTCCTGCCTCACAAGCCGCTGCCTGCCTCTGCCTACATAGAGGACGGCCGCTTCGATCTCTCCATGC
CCCATGTGCAAGACCCCTCGCTTGTCAGGTGGTTCTACATTGTTGTGGTACCCATTGACCGTGTGGGCGGGAGCATGCTGACGCCAA
GGTGGAGCACACCGAGGAACTGGAGCTGGACAGGTTCTAGAAGCCATCGAGCAAGGTGGCAGGGGAGCAGCGGCGGCGGCGGCGGC
AGGCAGAACGTCTGAAGCCATATGTGGCTGCTCAACTGGATGTGCTCCCGGGAGACCTTTACCTTGGGGGACAAGAAGAACTACCGGG
GCTTCTACAACCGGCCCCTGTCTCCGGACTTGAGCTACCAGTGCTTTGTGCTTGCCTCCTTGAAGGAACCCATGGACCAGAAGCGCT
ATGCCTCCAGCCCCTACTGGATGAGATCGTGGTCCAGGTGACACCAGCCCAGCAGCAGGAGGAGCCGGAGATGCTGTGGGTGACGG
GTCCCGTGCTGGCAGTCATCCTCATCATCCTCATTGTCATCGCCATCCTCTTGTTCAAAAGGAAAAGGACCCACTCTCCGTCCTCTA
AGGATGAGCAGTCGATCGGACTGAAGGACTCCTTGCTGGCCCACTCCTCTGACCCTGTGGGAGATCGGGAGGCTCAACTACCAGACCC
CAGGTATGCGAGACCACCCACCCCATCCCCATCACCGACCTGGCGGACAACATCGAGCGCCTCAAAGCCAACGATGGCCTCAAGTTCT
CCCAGGAGTATGAGTCCATCGACCCTGGACAGCAGTTCACGTGGGAGAATTCAAACCTGGAGGTGAACAAGCCCAAGAACCGCTATG
CGAATGTCATCGCCTACGACCACTCTCGAGTCATCCTTACCTCTATCGATGGCGTCCCCGGGAGTGACTACATCAATGCCAACTACA
TCGATGGCTACCGCAAGCAGAATGCCTACATCGCCACGCAGGGCCCCCTGCCCGAGACCATGGGCGATTTCTGGAGAATGGTGTGGG
AACAGCGCACGGCCACTGTGGTCATGATGACCACGGCTGGAGGAGAAGTCCCGGGTAAAATGTGATCAGTACTGGCCAGCCCGTGGCA
CCGAGACCTGTGGCCTTATTCAGGTGACCCTGTTGGACACAGTGGAGCTGGCCACATACACTGTGCGCACCTTCGCACTCCACAAGA
GTGGCTCCAGTGAGAAGCGTGAGCTGCGTCAGTTTCAGTTCATGGCCTGGCCAGACCATGGAGTTCCTGAGTACCCAACTCCCATCC
TGGCCTTCCTACGACGGGTCAAGGCCTGCAACCCCCTAGACGCAGGGCCCATGGTGGTGCACTGCAGCGCGGGCGTGGGCCGCACCG
GCTGCTTCATCGTGATTGATGCCATGTTGGAGCGGATGAAGCACGAGAAGACGGTGGACATCTATGGCCACGTGACCTGCATGCGAT
CACAGAGGAACTACATGGTGCAGACGGAGGACCAGTACGTGTTCATCCATGAGGCGCTGCTGGAGGCTGCCACGTGCGGCCACACAG
AGGTGCCTGCCCGCAACCTGTATGCCCACATCCAGAAGCTGGGCCAAGTGCCTCCAGGGGAGAGTGTGACCGCCATGGAGCTGCAGT
TCAAGTTGCTGGCCAGCTCCAAGGCCCACACGTCCCGCTTCATCAGCGCCAACCTGCCCTGCAACAAGTTCAAGAACCGGCTGGTGA
ACATCATGCCCTACGAATTGACCCGTGTGTGTCTGCAGCCCATCCGTGGTGTGGAGGGCTCTGACTACATCAATGCCAGCTTCCTGG
ATGGTTATAGACAGCAGAAGGCCTACATAGCTACACAGGGGCCTCTGGCAGAGAGCACCGAGGACTTCTGGCGCATGCTATGGGAGC
ACAATTCCACCATCATCGTCATGCTGACCAAGCTTCGGGAGATGGGCAGGGAGAAATGCCACCAGTACTGGCCAGCAGAGCGCTCTG
CTCGCTACCAGTACTTTGTTGTTGACCCGATGGCTGAGTACAACATGCCCCAGTATATCCTGCGTGAGTTCAAGGTCACGATGCCC
GGGATGGGCAGTCAAGGACAATCCGGCAGTTCCAGTTCACAGACTGGCCAGACAGGGCGTGCCCAAGACAGGCGAGGGATTCATTG
ACTTCATCGGGCAGGTGCATAAGACCAAGGAGCAGTTTGGACAGGATGGGCCTATCACGGTGCACTGCAGTGCTGGCGTGGGCCGCA
CCGGGGTGTTCATCACTCTGAGCATCGTCCTGGAGCGCATGCGCTACGAGGGCGTGGTCGACATGTTTCAGACCGTGAAGACCCTGC
GTACACAGCGTCCTGCCATGGTGCAGACAGAGGACCAGTATCAGCTGTGCTACCGTGCGGCCCTGGAGTACCTCGGCAGCTTTGACC
ACTATGCAACGTAACTACCGCTCCCCTCTCCTCCGCCAACCCGCCGTGGGGCTCCGGAGGGGACCCAGCTCCTCTGAGCCATACCG
ACCATCGTCCAGCCCTCCTACGACGATGCTGTCACTGGCAGAGCACAGCCCACGGGGATCACAGCGTTTCAGGAACGTTGCCACACC
AATCAGAGAGCCTAGAACATCCCTGGGCAAGTGGATGGCCCAGCAGGCAGGCACTGTGGCCCTTCTGTCCACCAGACCCACCTGGAG
CCCGCTTCAAGCTCTCTGTTGCGCTCCGCATTTCTCATGCTTCTTCTCATGGGGTGGGGTTGGGGCAAAGCCTCCTTTTTAATACA
TTAAGTGGGGTAGACTGAGGGATTTTAGCCTCTTCCCTCTGATTTTTCCTTTCGCGAATCCGTATCTGCAGAATGGGCCACTGTAGG
GGTTGGGGTTTATTTTGTTTTGTTTTTTTTTTCTTGATTTGTATGACTTCTGCTGAAGGACAGAACATTGCCTTCCTCGTGCAGAG
CTGGGGCTGCCAGCCTGAGCGGAGGCTCGGCCGTGGGCCGGGAGGCAGTGCTGATCCGGCTGCTCCTCCAGCCCTTCAGACGAGATC
CTGTTTCAGCTAAATGCAGGGAAACTCAATGTTTTTTTAAGTTTTGTTTTCCCTTTAAAGCCTTTTTTTTAGGCCACATTGACAGTGG
TGGGCGGGGAGAAGATAGGGAACACTCATCCCTGGTCGTCTATCCCAGTGTGTGTTTAACATTCACAGCCCAGAACCACAGATGTGT
CTGGGAGAGCCTGGCAAGGCATTCCTCATCACCATCGTGTTTGCAAAGGTTAAAACAAAAACAAAAAAACCACAAAAATAAAAAACAA
AAAAAACAAAAAACCCAAGAAAAAAAAAAAAGAGTCAGCCCTTGGCTTCTGCTTCAAACCCTCAAGAGGGGAAGCAACTCCGTGTGCC
TGGGGTTCCCGAGGGAGCTGCTGGCTGACCTGGGCCCACAGAGCCTGGCTTTGGTCCCCAGCATTGCAGTATGGTGTGGTGTTTGTA
GGCTGTGGGGTCTGGCTGTGTGGCCAAGGTGAATAGCACAGGTTAGGGTGTGTGCCACACCCCATGCACCTCAGGGCCAAGCGGGGG
CGTGGCTGGCCTTTCAGGTCCAGGCCAGTGGGCCTGGTAGCACATGTCTGTCCTCAGAGCAGGGGCCAGATGATTTTCCTCCCTGGT
TTGCAGCTGTTTTCAAAGCCCCCGATAATCGCTCTTTTCCACTCCAAGATGCCCTCATAAACCAATGTGGCAAGACTACTGGACTTC
TATCAATGGTACTCTAATCAGTCCTTATTATCCCAGCTTGCTGAGGGGCAGGGAGAGCGCCTCTTCCTCTGGGCAGCGCTATCTAGA
TAGGTAAGTGGGGGCGGGGAAGGGGTGCATAGCTGTTTTAGCTGAGGGACGTGGTGCCGACGTCCCCAAACCTAGCTAGGCTAAGTCA
AGATCAACATTCCAGGGTTGGTAATGTTGGATGATGAAACATTCATTTTTACCTTGTGGATGCTAGTGCTGTAGAGTTCACTGTTGT
ACACAGTCTGTTTTCTATTTGTTAAGAAAAACTACAGCATCATTGCATAATTCTTGATGGTAATAAATTTGAATAATCAGATTTCTT
AC
```

HUMAN PROTEIN SEQUENCE : hP28-021.3 (Seq ID No: 72)

```
MAPEPAPGRTMVPLVPALVMLGLVAGAHGDSKPVFIKVPEDQTGLSGGVASFVCQATGEPKRITWMKKGKKVSSQRFEVIEFDDGA
GSVLRIQPLRVQRDEAIYECTATNSLGEINTSAKLSVLEEEQLPPGFPSIDMGPQLKVVEKARTATMLCAAGGNPDPEISWFKDFLP
VDPATSNGRIKQLRSGALQIESSEESDQGKYECVATNSAGTRYSAPANLYVRVRRVAPRFSIPPSSQEVMPGGSVNLTCVAVGAPMP
YVKWMMGAEELTKEDEMPVGRNVLELSNVVRSANYTCVAISSLGMIEATAQVTVKALPKPPIDLVVTETTATSVTLTWDSGNSEPVT
YYGIQYRAAGTEGPFQEVDGVATTRYSIGGLSPFSEYAFRVLAVNSIGRGPPSEAVRARTGEQAPSSPPRRVQARMLSASTMLVQWE
PPEEPNGLVRGYRVYYTPDSRRPPNAWHKHNTDAGLLTTVGSLLPGITYSLRVLAFTAVGDGPPSPTIQVKTQQGVPAQPADFQAEV
ESDTRIQLSWLLPPQERIIMYELVYWAAEDEDQQHKVTFDPTSSYTLEDLKPDTLYRFQLAARSDMGVGVFTPTIEARTAQSTPSAP
PQKVMCVSMGSTTVRVSWVPPPADSRNGVITQYSVAYEAVDGEDRGRHVVDGISREHSSWDLVGLEKWTEYRVWVRAHTDVGPGPES
SPVLVRTDEDVPSGPPRKVEVEPLNSTAVHVYWKLPVPSKQHGQIRGYQVTYVRLENGEPRGLPIIQDVMLAEAQETTISGLTPETT
YSVTVAAYTTKGDGARSKPKIVTTTGAVPGRPTMMISTTAMNTALLQWHPPKELPGELLGYRLQYCRADEARPNTIDFGKDDQHFTV
TGLHKGTTYIFRLAAKNRAGLGEEFEKEIRTPEDLPSGFPQNLHVTGLTTSTTELAWDPPVLAERNGRIISYTVVVFRDINSQQELQN
ITTDTRFTLTGLKPDTTYDIKVRAWTSKGSGPLSPSIQSRTMPVEQVFAKNFRVAAAMKTSVLLSWEVPDSYKSAVPFKILYNGQSV
EVDGHSMRKLIADLQPNTEYSFVLMNRGSSAGGLQHLVSIRTAPDLLPHKPLPASAYIEDGRFDLSMPHVQDPSLVRWFYIVVVPID
RVGGSMLTPRWSTPEELELDELLEAIEQGGEEQRRRRRQAERLKPYVAAQLDVLPETFTLGDKKNYRGFYNRPLSPDLSYQCFVLAS
```

```
LKEPMDQKRYASSPYSDEIVVQVTPAQQQEEPEMLWVTGPVLAVILIILIVIAILLFKRKRTHSPSSKDEQSIGLKDSLLAHSSDPV
EMRRLNYQTPGMRDHPPIPITDLADNIERLKANDGLKFSQEYESIDPGQQFTWENSNLEVNKPKNRYANVIAYDHSRVILTSIDGVP
GSDYINANYIDGYRKQNAYIATQGGPLPETMGDFWRMVWEQRTATVVMMTRLEEKSRVKCDQYWPARGTETCGLIQVTLLDTVELATY
TVRTFALHKSGSSEKRELRQFQFMAWPDHGVPEYPTPILAFLRRVKACNPLDAGPMVVHCSAGVGRTGCFIVIDAMLERMKHEKTVD
IYGHVTCMRSQRNYMVQTEDQYVFIHEALLEAATCGHTEVPARNLYAHIQKLGQVPPGESVTAMELEFKLLASSKAHTSRFISANLP
CNKFKNRLVNIMPYELTRVCLQPIRGVEGSDYINASFLDGYRQQKAYIATQGPLAESTEDFWRMLWEHNSTIIVMLTKLREMGREKC
HQYWPAERSARYQYFVVDPMAEYNMPQYILREFKVTDARDGQSRTIRQFQFTDWPEQGVPKTGEGFIDFIGQVHKTKEQFGQDGPIT
VHCSAGVGRTGVFITLSIVLERMRYEGVVDMFQTVKTLRTQRPAMVQTEDQYQLCYRAALEYLGSFDHYAT*


HUMAN mRNA SEQUENCE : hR28-021.4 (Seq ID No: 73)
GCGTGTGTGGACTGAAACCCAAAAGCCAGAAACTCTCTGCATGTGTCCCTCATGGAGCCATATGGTCTCTAGTCTCTGTTTTTCAAA
ATTTGCAGGACTGATGACTGATTGGACAGTGGTGCAGGAGGGAAGGTCTCTGCATGTCAGTGATTAAATTGCTGAAGATGCCACTGC
CTGAGACGGGCAGTATTGAAGGAAGGAGTGGAGGCCCTGGTGCCCGGCCCTTGGTGCTGAGTATCCAGCAAGAGTGACCGGGGTGAA
GAAGCAAAGACTCGGTTGATTGTCCTGGGCTGTGGCTGGCTGTGGAGCTAGAGCCCTGGATGGCCCCTGAGCCAGCCCCAGGGAGGA
CGATGGTGCCCCTTGTGCCTGCACTGGTGATGCTTGGTTTGGTGGCAGGCGCCCATGGTGACAGCAAACCTGTCTTCATTAAAGTCC
CTGCAGGACCAGACTGGGCTGTCAGGAGGGGTAGCCTCCTTCGTGTGCCAAGCTACAGGAGAACCCAAGCCGCGCATCACATGGATGA
AGAAGGGGAAGAAAGTCAGCTCCCAGCGCTTCGAGGTCATTGAGTTTGATGATGGGGCAGGGTCAGTGCTTCGGATCCAGCCATTGC
GGGTGCAGCGAGATGAAGCCATCTATGAGTGTACAGCTACTAACAGCCTGGGTGAGATCAACACTAGTGCCAAGCTCTCAGTGCTCG
AAGAGGAACAGCTGCCCCCTGGGTTCCCTTCCATCGACATGGGGCCTCAGCTGAAGGTGGTGGAGAAGGCACGCACAGCCACCATGC
TATGTGCCGCAGGCGGAAATCCAGACCCTGAGATTTCTTGGTTCAAGGACTTCCTTCCTGTAGACCCTGCCACGAGCAACGGCCGCA
TCAAGCAGCTGGTTCAGGTGCCTTGCAGATAGAGAGCAAGGAATCCGACCAAGGCAAGTACGAGTGGTGTGGCGACCAACTCGG
CAGGCACACGTTACTCAGCCCCTGCGAACCTGTATGTGCGAGTGCGCCGCGTGGCTCCTCGTTTCTCCATCCCTCCCAGCAGCCAGG
AGGTGATGCCAGGCGGCAGCGTGAACCTGACATGCGTGGCAGTGGGTGCACCCATGCCCTACGTGAAGTGGATGATGGGGGCCGAGG
AGCTCACCAAGGAGGATGAGATGCCAGTTGGCCGCAACGTCCTGGAGCTCAGCAATGTCGTACGCTCTGCCAACTACACCTGTGTGG
CCATCTCCTCGCTGGGCATGATCGAGGCCACAGCCCAGGTCACAGTGAAAGCTCTTCCAAAGCCTCCGATTGATCTTGTGGTGACAG
AGACAACTGCCACCAGTGTCACCCTCACCTGGGATCTCTGGGAACCTGTAACCTACTACTATGGCATCCAGTACCGCGCGCAGCGG
GCACGGAGGGCCCCTTTCAGGAGGTGGATGGTGTGGCCACCACCCGCTACAGCATTGGCGGCCTCAGCCCTTTCTGGGAATATGCCT
TCCGCGTGCTGGCCGGTGAACAGCATCGGGCGAGGGCCGCCCAGCGAGGCAGTGCGGGCACGCACGGGAGAACAGGCGCCCTCCAGCC
CACCGCGCCGCGTGCAGGCACGCATGCTGAGCGCCAGCACCATGCTGGTGCAGTGGGAGCCTCCCGAGGAGCCCAACGGCCTGGTGC
GGGGATACCGCGTCTACTATACTCCGGACTCCCGCCGCCCCCGAACGCCTGGCACAAGCACAACACCGACGCGGGGCTCCTCACGA
CCGTGGGCAGCCTGCTGCCCTGGCATCACCTACAGCCTGCGCGTTGCCTTCACCGCCGTGGGCGATGGCCCTCCCAGCCCCACCA
TCCAGGTCAAGACGCAGCAGGGAGTGCCTGCCCAGCCCGCGGACTTCCAGGCCGAGGTGGAGTCGGACACCAGGATCCAGCTCTCGT
GGCTGCTGCCCCCTCAGGAGCGGATCATCATGTATGAACTGGTGTACTGGGCGGCAGAGGACGAAGACCAACAGCACAAGGTGACCT
TCGACCCAACCTCCTCCTACACACTAGAGGACCTGAAGCCTGACACACTCTACCGCTTCCAGCTGGCTGCACGCTCGGATATGGGGG
TGGGCGTCTTCACCCCCACCATTGAGGCCCGCACAGCCCAGTCCACCCCCTCCGCCCCTCCCCAGAAGGTGATGTGTGTGAGCATGG
GCTCCACCACGGTCCGGGTAAGTTGGGTCCCGCCGCCGTTATCACCCGCGTTATCACCCGATTACTCCGTGGCCTACGAGG
CGGTGGACGGCGAGGACCGCGGGCGGCATGTGGTGGATGGCATCAGCCGTGAGCACTCCAGCTGGGACTGGTGGGCCTGGAGAAGT
GGACGGAGTACCGGGTGTGGGTGCGGGCACACACAGACGTGGGCCCCGGCCCCGAGAGCAGCCCGGTGCTGGTGCGCACCGATGAGG
ACGTGCCCAGCGGGCCTCCGCGGAAGGTGGAGGTGGAGCCACTGAACTCCACTGCTGTGCATGTCTACTGGAAGCTGCCTGTCCCCA
GCAAGCAGCATGGCCAGATCCGCGGCTACCAGGTCACCTACGTGCGGCTGGAGAATGGCGAGCCCCGTGGACTCCCCATCATCCAAG
ACGTCATGCTAGCCGAGGCCCAGTGGCGGCCAGAGGAGCTATGAAACCACTATCAGCGGCCTGACCCCGGAGACCACCT
ACTCCGTTACTGTTGCTGCCTATACCACCAAGGGGATGGTGCCCAGCAAGCCCAAAATTGTCACTACAACAGGTGCAGTCCCAG
GCCGGCCCACCATGATGATCAGCACCACGGCCATGAACACTGCGCTGCTCCAGTGGCACCCACCCAAGGAACTGCTGGGCGAGCTGC
TGGGCTACCGGCTGCAGTACTGCCGGGCCGACGAGGCGCGGCCCAACACCATAGATTTCGGCAAGGATGACCAGCACTTCACAGTCA
CCGGCCTGCACAAGGGGACCACCTACATCTTCCGGCTTGCTGCCAAGAACCGGGCTGGCTTGGGTGAGGAGTTCGAGAAGGAGATCA
GGACCCCGCAGGACCTGCCCAGCGGCTTCCCCCAAAACCTGCATGTGACAGGACTGACCACGTCTACCACAGGACTGGCCTGGGACC
CGCCAGTGCTGGCGGAGAGGAACGGGCGCATCATCAGCTACACCGTGGTGTTCCGAGACATCAACAGCCAACAGGAGCTGCAGAACA
TCACGACAGACACCCGCTTTACCCTTACTGGCCTCAAGCCAGACACCACTTACGACATCAAGGTCCGCGCATGGACCAGCAAAGGCT
CTGGCCCACTCAGCCCCAGCATCCAGTCCCGGACCATGCCGGTGGAGCAAGTGTTTGCCAAGAACTTCCGGGTGGCGGCTGCAATGA
AGACGTCTGTGCTGCTCAGCTGGGAGGTTCCCGACTCCTATAAGTCAGCTGTGCCCTTTAAGATTCTGTACAATGGGCAGAGTGTGG
AGGTGGACGGGCACTCGATGCGGAAGCTGATCGCAGACCTGCAGCCCAACACAGAGTACTCGTTTGTGCTGATGAACCGTGGCAGCA
GCGCAGGGGGCCTGCAGCACCTGGTGTCCATCCGCACAGCCCCCGACCTCCTGCCTCACAAGCCGCTGCCTGCCTGCCTACATAG
AGGACGGCCGCTTCGATCTCTCCATGCCCCATGTGCAAGACCCCTCGCTTGTCAGGTGGTTCTACATTGTTGTGGTACCCATTGACC
GTGTGGGCGGGAGCATGCTGACGCCAAGGTGGAGCACACCCGAGGAACTGGAGCTGGACGAGCTTCTAGAAGCCATCGAGCAAGGCG
GAGAGGAGCAGCGGCGGCGGCGGCGGCAGGCAGAACGTCTGAAGCCATATGTGGCTGCTCAACTGGATGTGCTCCCGGAGACCTTTA
CCTTGGGGGACAAGAAGAACTACCGGGGCTTCTACAACGGCCCCTGTCTCCGGACTTGGACTACCAGTGCTTTGTGCTTTGCCTCCT
TGAAGGAACCCATGGACCAGAAGCGCTATGCCTCCAGCCCCTACTCGGATGAGATCGTGGTCCAGGTGACACCAGCCCAGCAGCAGG
AGGAGCCGGAGATGCTGTGGGTGACGGGTCCCGTGCTGGCAGTCATCCTCATCATCCTCATTGTCATCGCCATCCTCTTGTTCAAAA
GGAAAAGGACCCACTCTCCGTCCTCTAAGGATGAGCAGTCGATCGGACTGAAGGACTCCTTGCTGGCCCACTCCTCTGACCCTGTGG
AGATGCGGAGGCTCAACTACCAGACCCCAGGTATGCGAGACCACCCACCCATCCCCATCACCGACCTGGCGGACAACATCGAGCGCC
TCAAAGCCAACGATGGCCTCAAGTTCTCCCAGGAGTATGAGTCCATCGACCCTGGACAGCAGTTCACGTGGGAGAATTCAAACCTGG
AGGTGAACAAGCCCAAGAACCGCTATGCGAATGTCATCGCCTACGACCACTCTCGAGTCATCCTTACCTCTATCGATGGCGTCCCCG
GGAGTGACTACATCAATGCCAACTACATCGATGGCTACCGCAAGCAGAATGCCTACATCGCCACGCAGGGCCCCCTGCCCGAGACCA
TGGGCGATTTCTGGAGAATGGTGTGGGAACAGCGCACGGCCACTGTGGTCATGATGACACGGCTGGAGGAGAAGTCCCGGGTAAAT
GTGATCAGTACTGGCCAGCCCGTGGCACCGAGACCTGTGGCCTTATTCAGGTGACCCTGTTGGACACAGTGGAGCTGGCCACATACA
CTGTGCGCACCTTCGCACTCCACAAGAGTGGCTCCAGTGAGAAGCGTGAGCTGCGTCAGTTTCAGTTCATGGCCTGGCCAGACCATG
GAGTTCCTGAGTACCCAACTCCCATCCTGGCCTTCCTACGACGGGTCAAGGCCTGCAACCCCCTAGACGCAGGGCCCATGGTGGTGCA
CTGCAGCGCGGGCGTGGGCCGCACCGGCTGCTTCATCGTGATTGATGCCATGTTGGAGCGGATGAAGCACGAGAAGACGGTGGACA
TCTATGGCCACGTGACCTGCATGCGATCACAGAGGAACTACATGGTGCAGACGGAGGACCAGTACGTGTTCATCCATGAGGCGCTGC
TGGAGGCTGCCACGTGCGGCCACACAGAGGTGCCTGCCCGCAACCTGTATGCCCACATCCAGAAGCTGGGCCAAGTGCCTCCAGGGG
GCAACAAGTTCAAGAACCGCTGGTGAACATCATGCCCTACGAATTGACCCGTGTGTGCTCGCAGCCCATCCGTGGTGTGGGAGGGCT
CTGACTACATCAATGCCAGCTTCCTGGATGGTTATAGACAGCAGAAGGCCTACATAGCTCACAGGGGCCCTCTGGCAGAGAGCACCG
AGGACTTCTGGCGCATGCTATGGGAGCACAATTCCACCATCATCGTCATGCTGACCAAGCTTCGGGAGATGGGCAGGGAGAAATGCC
ACCAGTACTGGCCAGCAGAGCGCTCTGCTCGCTACCAGTACTTTGTTGTTGACCCGATGGCTGAGTACAACATGCCCCAGTATATCC
TGCGTGAGTTCAAGGTCACGGATGCCCGGGATGGGCAGTCAAGGACAATCCGGCAGTTCCAGTTCACAGACTGGCCAGAGCAGGGCG
```

```
TGCCCAAGACAGGCGAGGGATTCATTGACTTCATCGGGCAGGTGCATAAGACCAAGGAGCAGTTTGGACAGGATGGGCCTATCACGG
TGCACTGCAGTGCTGGCGTGGGCCGCACCGGGGTGTTCATCACTCTGAGCATCGTCCTGGAGCGCATGCGCTACGAGGGCGTGGTCG
ACATGTTTCAGACCGTGAAGACCCTGCGTACACAGCGTCCTGCCATGGTGCAGACAGAGGACCAGTATCAGCTGTGCTACCGTGCGG
CCCTGGAGTACCTCGGCAGCTTTGACCACTATGCAACGTAACTACCGCTCCCCTCTCCTCCGCCACCCCCGCCGTGGGGCTCCGGAG
GGGACCCAGCTCCTCTGAGCCATACCGACCATCGTCCAGCCCTCCTACGCAGATGCTGTCACTGGCAGAGCACAGCCCACGGGGATC
ACAGCGTTTCAGGAACGTTGCCACACCAATCAGAGAGCCTAGAACATCCCTGGGCAAGTGGATGGCCCAGCAGGCAGGCACTGTGGC
CCTTCTGTCCACCAGACCCACCTGGAGCCCGCTTCAAGCTCTCTGTTGCGCTCCCGCATTTCTCATGCTTCTTCTCATGGGGTGGGG
TTGGGGCAAAGCCTCCTTTTTAATACATTAAGTGGGGTAGACTGAGGGATTTTAGCCTCTTCCCTCTGATTTTTCCTTTCGCGAATC
CGTATCTGCAGAATGGGCCACTGTAGGGGTTGGGGTTTATTTTGTTTGTTTTTTTTTTTCTTGATTTGTATGACTTCTGCTGAAGG
ACAGAACATTGCCTTCCTCGTGCAGAGCTGGGGCTGCCAGCCTGAGCGGAGGCTCGGCCGTGGGCCGGGAGGCAGTGCTGATCCGGC
TGCTCCTCCAGCCCTTCAGACGAGATCCTGTTTCAGCTAAATGCAGGGAAACTCAATGTTTTTTTAAGTTTTGTTTTCCCTTTAAAG
CCTTTTTTTAGGCCACATTGACAGTGGTGGGCGGGGAGAAGATAGGGAACACTCATCCCTGGTCGTCTATCCCAGTGTGTGTTTAAC
ATTCACAGCCCAGAACCACAGATGTGTCTGGGGAGAGCCTGGCAAGGCATTCCTCATCACCATCGTGTTTGCAAAGGTTAAAAACAAAA
ACAAAAAACCACAAAAATAAAAAACAAAAAAAAACAAAAAACCCAAGAAAAAAAAAAAAGAGTCAGCCCTTGGCTTCTGCTTCAAACCC
TCAAGAGGGGAAGCAACTCCGTGTGCCTGGGGTTCCCGAGGGGAGCTGCTGGCTGACCTGGGCCCACAGAGCCTGGCTTTGGTCCCCA
GCATTGCAGTATGGTGTGGTGTTTGTAGGCTGTGGGGTCTGGCTGTGTGGCCAAGGTGAATAGCACAGGTTAGGGTGTGTGCCCACAC
CCCATGCACCTCAGGGCCAAGCGGGGGCGTGGCTGGCCTTTCAGGTCCAGGCCCAGTGGGCCTGGTAGCACATGTCTGTCCTCAGAGC
AGGGGCCAGATGATTTTCCTCCCTGGTTTGCAGCTGTTTTCAAAGCCCCCGATAATCGCTCTTTTCCACTCCAAGATGCCCTCATAA
ACCAATGTGGCAAGACTACTGGACTTCTATCAATGGTACTCTAATCAGTCCTTATTATCCCAGCTTGCTGAGGGGCAGGGAGAGCGC
CTCTTCCTCTGGGCAGCGCTATCTAGATAGGTAAGTGGGGGCGGGGAAGGGTGCATAGCTGTTTTAGCTGAGGGACGTGGTGCCGAC
GTCCCCAAACCTAGCTAGGCTAAGTCAAGATCAACATTCCAGGGTTGGTAATGTTGGATGATGAAACATTCATTTTTACCTTGTGGA
TGCTAGTGCTGTAGAGTTCACTGTTGTACACAGTCTGTTTTCTATTTGTTAAGAAAAACTACAGCATCATTGCATAATTCTTGATGG
TAATAAATTTGAATAATCAGATTTCTTAC

HUMAN PROTEIN SEQUENCE : hP28-021.4 (Seq ID No: 74)
MAPEPAPGRTMVPLVPALVMLGLVAGAHGDSKPVFIKVPEDQTGLSGGVASFVCQATGEPKPRITWMKKGKKVSSQRFEVIEFDDGA
GSVLRIQPLRVQRDEAIYECTATNSLGEINTSAKLSVLEEEQLPPGFPSIDMGPQLKVVEKARTATMLCAAGGNPDPEISWFKDFLP
VDPATSNGRIKQLRSGALQIESSEESDQGKYECVATNSAGTRYSAPANLYVRVRRVAPRFSIPPSSQEVMPGGSVNLTCVAVGAPMP
YVKWMMGAEELTKEDEMPVGRNVLELSNVVRSANYTCVAISSLGMIEATAQVTVKALPKPPIDLVVTETTATSVTLTWDSGNSEPVT
YYGIQYRAAGTEGPFQEVDGVATTRYSIGGLSPFSEYAFRVLAVNSIGRGPPSEAVRARTGEQAPSSPPRRVQARMLSASTMLVQWE
PPEEPNGLVRGYRVYYTPDSRRPPNAWHKHNTDAGLLTTVGSLLPGITYSLRVLAFTAVGDGPPSPTIQVKTQQGVPAQPADFQAEV
ESDTRIQLSWLLPPQERIIMYELVYWAAEDEDQQHKVTFDPTSSYTLEDLKPDTLYRFQLAARSDMGVGVFTPTIEARTAQSTPSAP
PQKVMCVSMGSTTVRVSWVPPPADSRNGVITQYSVAYEAVDGEDRGRHVVDGISREHSSWDLVGLEKWTEYRVWVRAHTDVGPGPES
SPVLVRTDEDVPSGPPRRKVEVEPLNSTAVHVYWKLPNSTAVHVYWKLPVDPATSNGRIKQLRGYQVTYVRLENGEPRGLPIIQDVMLAEAQWRPEESEDYETT
ISGLTPETTYSVTVAAYTTKGDGARSKPKIVTTTGAVPGRPTMMISTTAMNTALLQWHPPKELPGELLGYRLQYCRADEARPNTIDF
GKDDQHFTVTGLHKGTTYIFRLAAKNRAGLGEEFEKEIRTPEDLPSGFPQNLHVTGLTTSTTELAWDPPVLAERNGRIISYTVVFRD
INSQQELQNITTDTRFTLTGLKPDTTYDIKVRAWTSKGSGPLSPSIQSRTMPVEQVFAKNFRVAAAMKTSVLLSWEVPDSYKSAVPF
KILYNGQSVEVDGHSMRKLIADLQPNTEYSFVLMNRGSSAGGLQHLVSIRTAPDLLPHKPLPASAYIEDGRFDLSMPHVQDPSLVRW
FYIVVVPIDRVGGSMLTPRWSTPEELELDELLEAIEQGGEEQRRRRRQAERLKPYVAAQLDVLPETFTLGDKKNYRGFYNRPLSPDL
SYQCFVLASLKEPMDQKRYASSPYSDEIVVQVTPAQQQEEPEMLWVTGPVLAVILIILIVIAILLFKRKRTHSPSSKDEQSIGLKDS
LLAHSSDPVEMRRLNYQTPGMRDHPPIPITDLADNIERLKANDGLKFSQEYESIDPGQQFTWENSNLEVNKPKNRYANVIAYDHSRV
ILTSIDGVPGSDYINANYIDGYRKQNAYIATQGGLPETMGDFWRMVWEQRTATVVMMTRLEEKSRVKCDQYWPARGTETCGLIQVTL
LDTVELATYTVRTFALHKSGSSEKRELRQFQFMAWPDHGVPEYPTPILAFLRRVKACNPLDAGPMVVHCSAGVGRTGCFIVIDAMLE
RMKHEKTVDIYGHVTCMRSQRNYMVQTEDQYVFIHEALLEAATCGHTEVPARNLYAHIQKLGQVPPGESVTAMELEFKLLASSKAHT
SRFISANLPCNKFKNRLVNIMPYELTRVCLQPIRGVEGSDYINASFLDGYRQQKAYIATQGPLAESTEDFWRMLWEHNSTIIVMLTK
LREMGREKCHQYWPAERSARYQYFVVDPMAEYNMPQYILREFKVTDARDGQSRTIRQFQFTDWPEQGVPKTGEGFIDFIGQVHKTKE
QFGQDGPITVHCSAGVGRTGVFITLSIVLERMRYEGVVDMFQTVKTLRTQRPAMVQTEDQYQLCYRAALEYLGSFDHYAT*
```

The mouse and human genomic DNA sequence, sequence corresponding to the mRNA(s) generated therefrom and amino acid sequences of the proteins as shown in Tables 1-7 are described according to SEQ ID NOS as follows in Table 8.

**Table 8**

| DESIGNATION | SEQ ID NO | TYPE OF SEQUENCE |
|---|---|---|
| mD22-022 | SEQ ID NO: 1 | MOUSE GENOMIC SEQUENCE |
| mR22-022.1 | SEQ ID NO: 2 | MOUSE mRNA SEQUENCE |
| mP22-022.1 | SEQ ID NO: 3 | MOUSE PROTEIN SEQUENCE |
| mR22-022.2 | SEQ ID NO: 4 | MOUSE mRNA SEQUENCE |
| mP22-022.2 | SEQ ID NO: 5 | MOUSE PROTEIN SEQUENCE |
| hD22-022 | SEQ ID NO: 6 | HUMAN GENOMIC SEQUENCE |
| hR22-022.1 | SEQ ID NO: 7 | HUMAN mRNA SEQUENCE |
| hP22-022.1 | SEQ ID NO: 8 | HUMAN PROTEIN SEQUENCE |
| hR22-022.2 | SEQ ID NO: 9 | HUMAN mRNA SEQUENCE |
| hP22-022.2 | SEQ ID NO: 10 | HUMAN PROTEIN SEQUENCE |

(continued)

| DESIGNATION | SEQ ID NO | TYPE OF SEQUENCE |
|---|---|---|
| mD27-007 | SEQ ID NO: 11 | MOUSE GENOMIC SEQUENCE |
| mR27-007.1 | SEQ ID NO: 12 | MOUSE mRNA SEQUENCE |
| mP27-007.1 | SEQ ID NO: 13 | MOUSE PROTEIN SEQUENCE |
| mR27-007.2 | SEQ ID NO: 14 | MOUSE mRNA SEQUENCE |
| mP27-007.2 | SEQ ID NO: 15 | MOUSE PROTEIN SEQUENCE |
| hD27-007 | SEQ ID NO: 16 | HUMAN GENOMIC SEQUENCE |
| hR27-007.1 | SEQ ID NO: 17 | HUMAN mRNA SEQUENCE |
| hP27-007.1 | SEQ ID NO: 18 | HUMAN PROTEIN SEQUENCE |
| hR27-007.2 | SEQ ID NO: 19 | HUMAN mRNA SEQUENCE |
| hP27-007.2 | SEQ ID NO: 20 | HUMAN PROTEIN SEQUENCE |
| hR27-007.3 | SEQ ID NO: 21 | HUMAN mRNA SEQUENCE |
| hP27-007.3 | SEQ ID NO: 22 | HUMAN PROTEIN SEQUENCE |
| mD27-009 | SEQ ID NO: 23 | MOUSE GENOMIC SEQUENCE |
| mR27-009.1 | SEQ ID NO: 24 | MOUSE mRNA SEQUENCE |
| mP27-009.1 | SEQ ID NO: 25 | MOUSE PROTEIN SEQUENCE |
| hD27-009 | SEQ ID NO: 26 | HUMAN GENOMIC SEQUENCE |
| hR27-009.1 | SEQ ID NO: 27 | HUMAN mRNA SEQUENCE |
| hP27-009.1 | SEQ ID NO: 28 | HUMAN PROTEIN SEQUENCE |
| hR27-009.2 | SEQ ID NO: 29 | HUMAN mRNA SEQUENCE |
| hP27-009.2 | SEQ ID NO: 30 | HUMAN PROTEIN SEQUENCE |
| mD27-014 | SEQ ID NO: 31 | MOUSE GENOMIC SEQUENCE |
| mR27-014.1 | SEQ ID NO: 32 | MOUSE mRNA SEQUENCE |
| mP27-014.1 | SEQ ID NO: 33 | MOUSE PROTEIN SEQUENCE |
| mR27-014.2 | SEQ ID NO: 34 | MOUSE mRNA SEQUENCE |
| mP27-014.2 | SEQ ID NO: 35 | MOUSE PROTEIN SEQUENCE |
| mR27-014.3 | SEQ ID NO: 36 | MOUSE mRNA SEQUENCE |
| mP27-014.3 | SEQ ID NO: 37 | MOUSE PROTEIN SEQUENCE |
| mR27-014.4 | SEQ ID NO: 38 | MOUSE mRNA SEQUENCE |
| mP27-014.4 | SEQ ID NO: 39 | MOUSE PROTEIN SEQUENCE |
| hD27-014 | SEQ ID NO: 40 | HUMAN GENOMIC SEQUENCE |
| hR27-014.1 | SEQ ID NO: 41 | HUMAN mRNA SEQUENCE |
| hP27-014.1 | SEQ ID NO: 42 | HUMAN PROTEIN SEQUENCE |
| hR27-014.2 | SEQ ID NO: 43 | HUMAN mRNA SEQUENCE |
| hP27-014.2 | SEQ ID NO: 44 | HUMAN PROTEIN SEQUENCE |
| hR27-014.3 | SEQ ID NO: 45 | HUMAN mRNA SEQUENCE |
| hP27-014.3 | SEQ ID NO: 46 | HUMAN PROTEIN SEQUENCE |
| hR27-014.4 | SEQ ID NO: 47 | HUMAN mRNA SEQUENCE |
| hP27-014.4 | SEQ ID NO: 48 | HUMAN PROTEIN SEQUENCE |

(continued)

| DESIGNATION | SEQ ID NO | TYPE OF SEQUENCE |
|---|---|---|
| mD28-002 | SEQ ID NO: 49 | MOUSE GENOMIC SEQUENCE |
| mR28-002.1 | SEQ ID NO: 50 | MOUSE mRNA SEQUENCE |
| mP28-002.1 | SEQ ID NO: 51 | MOUSE PROTEIN SEQUENCE |
| hD28-002 | SEQ ID NO: 52 | HUMAN GENOMIC SEQUENCE |
| hR28-002.1 | SEQ ID NO: 53 | HUMAN mRNA SEQUENCE |
| hP28-002.1 | SEQ ID NO: 54 | HUMAN PROTEIN SEQUENCE |
| mD28-010 | SEQ ID NO: 55 | MOUSE GENOMIC SEQUENCE |
| mR28-010.1 | SEQ ID NO: 56 | MOUSE mRNA SEQUENCE |
| mP28-010.1 | SEQ ID NO: 57 | MOUSE PROTEIN SEQUENCE |
| mR28-010.2 | SEQ ID NO: 58 | MOUSE mRNA SEQUENCE |
| mP28-010.2 | SEQ ID NO: 59 | MOUSE PROTEIN SEQUENCE |
| hD28-010 | SEQ ID NO: 60 | HUMAN GENOMIC SEQUENCE |
| hR28-010.1 | SEQ ID NO: 61 | HUMAN mRNA SEQUENCE |
| hP28-010.1 | SEQ ID NO: 62 | HUMAN PROTEIN SEQUENCE |
| mD28-021 | SEQ ID NO: 63 | MOUSE GENOMIC SEQUENCE |
| mR28-021.1 | SEQ ID NO: 64 | MOUSE mRNA SEQUENCE |
| mP28-021.1 | SEQ ID NO: 65 | MOUSE PROTEIN SEQUENCE |
| hD28-021 | SEQ ID NO: 66 | HUMAN GENOMIC SEQUENCE |
| hR28-021.1 | SEQ ID NO: 67 | HUMAN mRNA SEQUENCE |
| hP28-021.1 | SEQ ID NO: 68 | HUMAN PROTEIN SEQUENCE |
| hR28-021.2 | SEQ ID NO: 69 | HUMAN mRNA SEQUENCE |
| hP28-021.2 | SEQ ID NO: 70 | HUMAN PROTEIN SEQUENCE |
| hR28-021.3 | SEQ ID NO: 71 | HUMAN mRNA SEQUENCE |
| hP28-021.3 | SEQ ID NO: 72 | HUMAN PROTEIN SEQUENCE |
| hR28-021.4 | SEQ ID NO: 73 | HUMAN mRNA SEQUENCE |
| hP28-021.4 | SEQ ID NO: 74 | HUMAN PROTEIN SEQUENCE |

The CA sequences were analyzed by Panther™ (Molecular Diagnostics, Palo Alto, CA) software designed to detect homologs and enable prediction of molecular function through a system for protein functional classification. Human Gene Ontlogy annotations were prepared in accordance with the Gene Ontology Consortium (Gene Ontology: tool for the unification of biology. The Gene Ontology Consortium Nature Genet. 25: 25-29 (2000)). Similar analysis was carried out by determining IPR information regarding the CA polypeptides from InterPro, which is an integrated documentation resource for protein families, domains and functional sites (Apweiler at al. Bioinformatics 16(12):1145-1150 (2000)).

The CA sequences may be classified according to the following predicted general classifications of function by Panther™ analysis, human gene ontology and IPR domain information for polypeptides having SEQ ID NOS: 8, 10, 18, 20, 22, 28, 30, 42, 44, 46, 48, 54, 62, 68, 70, 72 and 74 as shown in Tables 1-7. The classifications are shown in Table 9 below. The biological function of a CA protein comprises a function shown in Table 9 below.

Table 9

| Human Protein | SEQ ID NO: | FUNCTION |
|---|---|---|
| hP22-022.1 | SEQ ID NO: 8 | HUMAN PANTHER CLASSIFICATIONS FAMILY (SUBFAMILY) |

(continued)

| Human Protein | SEQ ID NO: | FUNCTION |
|---|---|---|
| | | SHORT-CHAIN DEHYDROGENASE-RELATED(FOLLICULAR VARIANT TRANSLOCATION PROTEIN 1)<br>    BIOLOGICAL PROCESS<br>    Biological process unclassified(2.99.00.00.00)<br>    MOLECULAR FUNCTIONS<br>    Oxidoreductase(1.19.00.00.00) > Reductase(1.19.06.00.00)<br>    Oxidoreductase(1.19.00.00.00) > Dehydrogenase(1.19.05.00.00)<br><br>HUMAN GENE ONTOLOGY<br>    No Gene Ontology<br>HUMAN PROTEIN DOMAINS (INTERPRO SIGNATURES)<br>    IPR002198 (SDRFAMILY)<br>    IPR002347 (GDHRDH)<br>    IPR002198 (adh short) |
| hP22-022.2 | SEQ ID NO: 10 | HUMAN PANTHER CLASSIFICATIONS<br>    FAMILY (SUBFAMILY)<br>    SHORT-CHAIN DEHYDROGENASE-RELATED(FOLLICULAR VARIANT TRANSLOCATION PROTEIN 1)<br>    BIOLOGICAL PROCESS<br>    Biological process unclassified(2.99.00.00.00)<br>    MOLECULAR FUNCTIONS<br>    Oxidoreductase(1.19.00.00.00) > Reductase(1.19.06.00.00)<br>    Oxidoreductase(1.19.00.00.00) > Dehydrogenase(1.19.05.00.00)<br><br>HUMAN GENE ONTOLOGY<br>    No Gene Ontology<br>HUMAN PROTEIN DOMAINS (INTERPRO SIGNATURES)<br>    IPR002198 (SDRFAMILY)<br>    IPR002347 (GDHRDH)<br>    IPR002198 (adh short) |
| hP27-007.1 | SEQ ID NO: 18 | HUMAN PANTHER CLASSIFICATIONS<br>    FAMILY (SUBFAMILY)<br>    PLATELET ENDOTHELIAL CELL ADHESION MOLECULE (PECAM-1)(SH2 DOMAIN-CONTAINING PHOSPHATASE ANCHOR PROTEIN 1-RELATED)<br>    BIOLOGICAL PROCESS<br>    Biological process unclassified(2.99.00.00.00)<br>    MOLECULAR FUNCTIONS<br>    Molecular function unclassified(1.97.00.00.00)<br>HUMAN GENE ONTOLOGY<br>    BIOLOGICAL PROCESS<br>    phagocytosis > phagocytosis, engulfment<br>    defence response > immune response<br>    MOLECULAR FUNCTION<br>    GO molecular function > cell adhesion<br>    defense/immunity protein > immunoglobulin<br>    B cell receptor > immunoglobulin<br>    CELL COMPONENT<br>    cell > membrane fraction |

(continued)

| Human Protein | SEQ ID NO: | FUNCTION |
|---|---|---|
| | | cell > plasma membrane<br>HUMAN PROTEIN DOMAINS (INTERPRO SIGNATURES)<br>    IPR003598 (IGc2)<br>    IPR003599 (IG)<br>    IPR003006 (ig) |
| hP27-007.2 | SEQ ID NO: 20 | HUMAN PANTHER CLASSIFICATIONS<br>    FAMILY (SUBFAMILY)<br>    PLATELET ENDOTHELIAL CELL ADHESION MOLECULE (PECAM-1)(SH2 DOMAIN-CONTAINING PHOSPHATASE ANCHOR PROTEIN 1-RELATED)<br>    BIOLOGICAL PROCESS<br>    Biological process unclassified(2.99.00.00.00)<br>    MOLECULAR FUNCTIONS<br>    Molecular function unclassified(1.97.00.00.00)<br><br>HUMAN GENE ONTOLOGY<br>    BIOLOGICAL PROCESS<br>    defence response > immune response<br>    phagocytosis > phagocytosis, engulfment<br>    MOLECULAR FUNCTION<br>    defense/immunity protein > immunoglobulin<br>    B cell receptor > immunoglobulin<br>    GO molecular function > cell adhesion<br>    CELL COMPONENT<br>    cell > membrane fraction<br>    cell > plasma membrane<br>HUMAN PROTEIN DOMAINS (INTERPRO SIGNATURES)<br>    IPR003598 (IGc2)<br>    IPR003599 (IG)<br>    IPR003006 (ig) |
| hP27-007.3 | SEQ ID NO: 22 | HUMAN PANTHER CLASSIFICATIONS<br>    FAMILY (SUBFAMILY)<br>    PLATELET ENDOTHELIAL CELL<br>ADHESION MOLECULE (PECAM-1)(SH2 DOMAIN-CONTAINING PHOSPHATASE ANCHOR PROTEIN 1-RELATED)<br>    BIOLOGICAL PROCESS<br>    Biological process unclassified(2.99.00.00.00)<br>    MOLECULAR FUNCTIONS<br>    Molecular function unclassified(1.97.00.00.00)<br><br>HUMAN GENE ONTOLOGY<br>    BIOLOGICAL PROCESS<br>    phagocytosis > phagocytosis, engulfment<br>    defence response > immune response<br>    MOLECULAR FUNCTION<br>    GO molecular function > cell adhesion<br>    defense/immunity protein > immunoglobulin<br>    B cell receptor > immunoglobulin<br>    CELL COMPONENT |

(continued)

| Human Protein | SEQ ID NO: | FUNCTION |
|---|---|---|
| | | cell > membrane fraction<br>cell > plasma membrane<br>HUMAN PROTEIN DOMAINS (INTERPRO SIGNATURES)<br>    IPR003598 (IGc2)<br>    IPR003599 (IG)<br>    IPR003006 (ig) |
| hP27-009.1 | SEQ ID NO: 28 | HUMAN PANTHER CLASSIFICATIONS<br>    FAMILY (SUBFAMILY)<br>    CF10153(POTASSIUM INTERMEDIATE/SMALL CONDUCTANCE CALCIUM-ACTIVATED CHANNEL)<br>    BIOLOGICAL PROCESS<br>    Transport(2.15.00.00.00) > Ion transport(2.15.01.00.00) > Cation transport(2.15.01.01.00)<br>    Neuronal activities(2.18.00.00.00) > Synaptic transmission (2.18.01.00.00)<br>    MOLECULAR FUNCTIONS<br>    Ion channel(1.03.00.00.00) > Voltage-gated ion channel(1.03.03.00.00) > Voltage-gated potassium channel(1.03.03.03.00)<br><br>HUMAN GENE ONTOLOGY<br>    No Gene Ontology<br>HUMAN PROTEIN DOMAINS (INTERPRO SIGNATURES)<br>    IPR003931 (SK channel)<br>    IPR000694 (PRO RICH)<br>    NULL (GLN RICH) |
| hP27-009.2 | SEQ ID NO: 30 | HUMAN PANTHER CLASSIFICATIONS<br>    FAMILY (SUBFAMILY)<br>    CF10153(POTASSIUM INTERMEDIATE/SMALL CONDUCTANCE CALCIUM-ACTIVATED CHANNEL)<br>    BIOLOGICAL PROCESS<br>    Transport(2.15.00.00.00) > Ion transport(2.15.01.00.00) > Cation transport(2.15.01.01.00)<br>    Neuronal activities(2.18.00.00.00) > Synaptic transmission (2.18.01.00.00)<br>    MOLECULAR FUNCTIONS<br>    Ion channel(1.03.00.00.00) > Voltage-gated ion channel(1.03.03.00.00) > Voltage-gated potassium channel(1.03.03.03.00)<br><br>HUMAN GENE ONTOLOGY<br>    No Gene Ontology<br>HUMAN PROTEIN DOMAINS (INTERPRO SIGNATURES)<br>    IPR003931 (SKCHANNEL)<br>    IPR004178 (CaMBD)<br>    IPR003931 (SK channel)<br>    IPR000694 (PRO RICH)<br>    NULL (GLN RICH)<br>    IPR001622 (CHANNEL PORE K) |
| hP27-014. | SEQ ID NO: 42 | HUMAN PANTHER CLASSIFICATIONS<br>    FAMILY (SUBFAMILY) |

(continued)

| Human Protein | SEQ ID NO: | FUNCTION |
|---|---|---|
| | | MACROPHAGE COLONY STIMULATING FACTOR-1(MACROPHAGE COLONY STIMULATING FACTOR-1)<br>BIOLOGICAL PROCESS<br>Immunity and defense(2.16.00.00.00) > Macrophage-mediated immunity(2.16.05.00.00)<br>Immunity and defense(2.16.00.00.00) > Granulocyte-mediated immunity(2.16.04.00.00)<br>Developmental processes(2.23.00.00.00) > Mesoderm development (2.23.10.00.00) > Hematopoesis(2.23.10.06.00)<br>MOLECULAR FUNCTIONS<br>Signaling molecule(1.02.00.00.00) > Cytokine(1.02.01.00.00) > Other cytokine(1.02.01.99.00)<br><br>HUMAN GENE ONTOLOGY<br>No Gene Ontology<br>HUMAN PROTEIN DOMAINS (INTERPRO SIGNATURES)<br>No Domain Hit |
| hP27-014.2 | SEQ ID NO: 44 | HUMAN PANTHER CLASSIFICATIONS<br>FAMILY (SUBFAMILY)<br>MACROPHAGE COLONY STIMULATING FACTOR-1(MACROPHAGE COLONY STIMULATING FACTOR-1)<br>BIOLOGICAL PROCESS<br>Immunity and defense(2.16.00.00.00) > Macrophage-mediated immunity(2.16.05.00.00)<br>Immunity and defense(2.16.00.00.00) > Granulocyte-mediated immunity(2.16.04.00.00)<br>Developmental processes(2.23.00.00.00) > Mesoderm development (2.23.10.00.00) > Hematopoesis(2.23.10.06.00)<br>MOLECULAR FUNCTIONS<br>Signaling molecule(1.02.00.00.00) > Cytokine(1.02.01.00.00) > Other cytokine(1.02.01.99.00)<br><br>HUMAN GENE ONTOLOGY<br>No Gene Ontology<br>HUMAN PROTEIN DOMAINS (INTERPRO SIGNATURES)<br>No Domain Hit |
| hP27-014.3 | SEQ ID NO: 46 | HUMAN PANTHER CLASSIFICATIONS<br>FAMILY (SUBFAMILY)<br>MACROPHAGE COLONY STIMULATING FACTOR-1(Unassigned)<br>BIOLOGICAL PROCESS<br>Immunity and defense(2.16.00.00.00) > Macrophage-mediated immunity(2.16.05.00.00)<br>Immunity and defense(2.16.00.00.00) > Granulocyte-mediated immunity(2.16.04.00.00)<br>Developmental processes(2.23.00.00.00) > Mesoderm development (2.23.10.00.00) > Hematopoesis(2.23.10.06.00)<br>MOLECULAR FUNCTIONS |

(continued)

| Human Protein | SEQ ID NO: | FUNCTION |
|---|---|---|
| | | Signaling molecule(1.02.00.00.00) > Cytokine(1.02.01.00.00) > Other cytokine(1.02.01.99.00)<br><br>HUMAN GENE ONTOLOGY<br>    No Gene Ontology<br>HUMAN PROTEIN DOMAINS (INTERPRO SIGNATURES)<br>    No Domain Hit |
| hP27-014.4 | SEQ ID NO: 48 | HUMAN PANTHER CLASSIFICATIONS<br>    FAMILY (SUBFAMILY)<br>    MACROPHAGE COLONY<br>    STIMULATING FACTOR-1(MACROPHAGE COLONY STIMULATING FACTOR-1)<br>    BIOLOGICAL PROCESS<br>    Immunity and defense(2.16.00.00.00) > Macrophage-mediated immunity(2.16.05.00.00)<br>    Immunity and defense(2.16.00.00.00) > Granulocyte-mediated immunity(2.16.04.00.00)<br>    Developmental processes(2.23.00.00.00) > Mesoderm development (2.23.10.00.00) > Hematopoesis(2.23.10.06.00)<br>    MOLECULAR FUNCTIONS<br>    Signaling molecule(1.02.00.00.00) > Cytokine(1.02.01.00.00) > Other cytokine(1.02.01.99.00)<br><br>HUMAN GENE ONTOLOGY<br>    No Gene Ontology<br>HUMAN PROTEIN DOMAINS (INTERPRO SIGNATURES)<br>    No Domain Hit |
| hP28-002.1 | SEQ ID NO: 54 | HUMAN PANTHER CLASSIFICATIONS<br>    FAMILY (SUBFAMILY)<br>    TETRASPANIN-RELATED(TETRASPANIN TSPAN-5)<br>    BIOLOGICAL PROCESS<br>    Cell adhesion(2.29.00.00.00)<br>    MOLECULAR FUNCTIONS<br>    Cell adhesion molecule(1.05.00.00.00) > Other cell adhesion molecule (1.05.99.00.00)<br><br>HUMAN GENE ONTOLOGY<br>    No Gene Ontology<br>HUMAN PROTEIN DOMAINS (INTERPRO SIGNATURES)<br>    IPR000301 (TMFOUR)<br>    IPR000301 (transmembrane4)<br>    IPR000301 (TM4 2) |
| hP28-010.1 | SEQ ID NO: 62 | HUMAN PANTHER CLASSIFICATIONS<br>    FAMILY (SUBFAMILY)<br>    CADHERIN(LIVER-INTESTINE-CADHERIN)<br>    BIOLOGICAL PROCESS<br>    Signal transduction(2.11.00.00.00) > Cell communication (2.11.03.00.00) > Cell adhesion-mediated signaling(2.11.03.01.00)<br>    Cell adhesion(2.29.00.00.00) |

(continued)

| Human Protein | SEQ ID NO: | FUNCTION |
|---|---|---|
| | | MOLECULAR FUNCTIONS<br>Cell adhesion molecule(1.05.00.00.00) > Cadherin(1.05.02.00.00)<br><br>HUMAN GENE ONTOLOGY<br>    BIOLOGICAL PROCESS<br>    cell adhesion > homophilic cell adhesion<br>    neurogenesis > central nervous system development<br>    transcription, DNA-dependent > transcription regulation<br>    cell death > apoptosis<br>    peptidoglycan catabolism > microtubule-based movement<br>    microtubule-based process > microtubule-based movement<br>    nuclear congression > microtubule-based movement<br>    MOLECULAR FUNCTION<br>    cell adhesion > calcium-dependent cell adhesion<br>    ligand binding or carrier > calcium binding<br>    enzyme > nitric oxide synthase<br>    GO molecular function > cell cycle regulator<br>    CELL COMPONENT<br>    cell > membrane fraction<br>    mitochondrial membrane > mitochondrial inner membrane<br>    GO cellular component > extracellular<br>    extracellular > extracellular space HUMAN PROTEIN DOMAINS<br>(INTERPRO SIGNATURES)<br>    IPR002126 (CADHERIN)<br>    IPR002126 (CA)<br>    IPR002126 (cadherin)<br>    IPR002126 (CADHERIN 2 6) |
| hP28-021.1 | SEQ ID NO: 68 | HUMAN PANTHER CLASSIFICATIONS<br>    FAMILY (SUBFAMILY)<br>    CELL ADHESION MOLECULE-RELATED(RECEPTOR PROTEIN<br>TYROSINE PHOSPHATASE)<br>    BIOLOGICAL PROCESS<br>    Protein metabolism and modification(2.05.00.00.00) > Protein<br>modification(2.05.03.00.00) > Protein phosphorylation(2.05.03.01.00)<br>vSignal transduction(2.11.00.00.00) > Cell surface receptor mediated signal<br>    transduction(2.11.01.00.00) > Other receptor mediated signaling<br>pathway(2.11.01.99.00)<br>    MOLECULAR FUNCTIONS<br>    Receptor(1.01.00.00.00) > Other receptor(1.01.99.00.00)<br>    Phosphatase(1.16.00.00.00) > Protein phosphatase(1.16.01.00.00)<br>HUMAN GENE ONTOLOGY<br>    BIOLOGICAL PROCESS<br>    protein modification > protein dephosphorylation<br>enzyme linked receptor protein signaling pathway > transmembrane receptor<br>protein tyrosine phosphatase signaling pathway catabolism ><br>    isoprenoid phosphate metabolism<br>    defasciculation of neuron > defasciculation of motor neuron<br>    enzyme linked receptor protein signaling pathway > transmembrane<br>receptor protein tyrosine kinase signaling pathway<br>    MOLECULAR FUNCTION |

(continued)

| Human Protein | SEQ ID NO: | FUNCTION |
|---|---|---|
| | | enzyme > protein phosphatase |
| | | protein phosphatase > protein tyrosine phosphatase |
| | | protein tyrosine phosphatase > prenylated protein tyrosine phosphatase |
| | | protein tyrosine phosphatase > transmembrane receptor protein tyrosine phosphatase |
| | | transmembrane receptor > transmembrane receptor protein tyrosine phosphatase |
| | | protein tyrosine phosphatase > non-membrane spanning protein tyrosine phosphatase |
| | | CELL COMPONENT |
| | | cell > membrane fraction |
| | | cytoplasm > cytoskeleton |
| | | cell > plasma membrane |
| | | plasma membrane > integral plasma membrane protein |
| | | cell > soluble fraction |
| | | HUMAN PROTEIN DOMAINS (INTERPRO SIGNATURES) |
| | | IPR003962 (FNTYPEIII) |
| | | IPR000242 (PRTYPHPHTASE) |
| | | IPR000242 (PTPc) |
| | | IPR003961 (FN3) |
| | | IPR003598 (IGc2) |
| | | IPR003599 (IG) |
| | | IPR003595 (PTPc motif) |
| | | IPR003961 (fn3) |
| | | IPR000242 (Y phosphatase) |
| | | IPR003006 (ig) |
| | | IPR000387 (TYR PHOSPHATASE 2 2) |
| | | IPR000242 (TYR PHOSPHATASE PTP2) |
| hP28-021.2 | SEQ ID NO: 70 | HUMAN PANTHER CLASSIFICATIONS FAMILY (SUBFAMILY) |
| | | CELL ADHESION MOLECULE-RELATED(RECEPTOR PROTEIN TYROSINE PHOSPHATASE) |
| | | BIOLOGICAL PROCESS |
| | | Protein metabolism and modification(2.05.00.00.00) > Protein modification(2.05.03.00.00) > Protein phosphorylation(2.05.03.01.00) |
| | | Signal transduction(2.11.00.00.00) > Cell surface receptor mediated signal transduction(2.11.01.00.00) > Other receptor mediated signaling pathway(2.11.01.99.00) |
| | | MOLECULAR FUNCTIONS |
| | | Receptor(1.01.00.00.00) > Other receptor(1.01.99.00.00) |
| | | Phosphatase(1.16.00.00.00) > Protein phosphatase(1.16.01.00.00) |
| | | HUMAN GENE ONTOLOGY |
| | | BIOLOGICAL PROCESS |
| | | protein modification > protein dephosphorylation |
| | | enzyme linked receptor protein signaling pathway > transmembrane receptor protein tyrosine phosphatase signaling pathway |
| | | isoprenoid catabolism > phosphate metabolism |
| | | defasciculation of neuron > defasciculation of motor neuron |
| | | enzyme linked receptor protein signaling pathway > transmembrane receptor protein tyrosine kinase signaling pathway |

(continued)

| Human Protein | SEQ ID NO: | FUNCTION |
|---|---|---|
| | | MOLECULAR FUNCTION |
| | | enzyme > protein phosphatase |
| | | protein phosphatase > protein tyrosine phosphatase |
| | | protein tyrosine phosphatase > prenylated protein tyrosine phosphatase |
| | | protein tyrosine phosphatase > transmembrane receptor protein tyrosine phosphatase |
| | | transmembrane receptor > transmembrane receptor protein tyrosine phosphatase |
| | | protein tyrosine phosphatase > non-membrane spanning protein tyrosine phosphatase |
| | | CELL COMPONENT |
| | | cell > membrane fraction |
| | | cytoplasm > cytoskeleton |
| | | cell > plasma membrane |
| | | plasma membrane > integral plasma |
| | | membrane protein |
| | | cell > soluble fraction |
| | | HUMAN PROTEIN DOMAINS (INTERPRO SIGNATURES) |
| | | IPR003962 (FNTYPEIII) |
| | | IPR000242 (PRTYPHPHTASE) |
| | | IPR000242 (PTPc) |
| | | IPR003961 (FN3) |
| | | IPR003598 (IGc2) |
| | | IPR003599 (IG) |
| | | IPR003595 (PTPc motif) |
| | | IPR003961 (fn3) |
| | | IPR000242 (Y phosphatase) |
| | | IPR003006 (ig) |
| | | IPR000387 (TYR PHOSPHATASE 2 2) |
| | | IPR000242 (TYR PHOSPHATASE PTP2) |
| hP28-021.3 | SEQ ID NO: 72 | HUMAN PANTHER CLASSIFICATIONS |
| | | FAMILY (SUBFAMILY) |
| | | CELL ADHESION MOLECULE-RELATED(RECEPTOR PROTEIN TYROSINE PHOSPHATASE) |
| | | BIOLOGICAL PROCESS |
| | | Protein metabolism and modification(2.05.00.00.00) > Protein modification(2.05.03.00.00) > Protein phosphorylation(2.05.03.01.00) |
| | | Signal transduction(2.11.00.00.00) > Cell surface receptor mediated signal transduction(2.11.01.00.00) > Other receptor mediated signaling pathway(2.11.01.99.00) |
| | | MOLECULAR FUNCTIONS |
| | | Receptor(1.01.00.00.00) > Other receptor(1.01.99.00.00) |
| | | Phosphatase(1.16.00.00.00) > Protein phosphatase(1.16.01.00.00) |
| | | HUMAN GENE ONTOLOGY BIOLOGICAL PROCESS |
| | | protein modification > protein dephosphorylation |
| | | enzyme linked receptor protein signaling pathway > transmembrane receptor protein tyrosine phosphatase signaling pathway |
| | | isoprenoid catabolism > phosphate metabolism |
| | | defasciculation of neuron > defasciculation of motor neuron |

(continued)

| Human Protein | SEQ ID NO: | FUNCTION |
|---|---|---|
| | | enzyme linked receptor protein signaling pathway > transmembrane receptor protein tyrosine kinase signaling pathway<br>    MOLECULAR FUNCTION<br>    enzyme > protein phosphatase<br>    protein phosphatase > protein tyrosine phosphatase<br>    protein tyrosine phosphatase > prenylated protein tyrosine phosphatase<br>    protein tyrosine phosphatase > transmembrane receptor protein tyrosine phosphatase<br>    transmembrane receptor > transmembrane receptor protein tyrosine phosphatase<br>    protein tyrosine phosphatase > non-membrane spanning protein tyrosine phosphatase<br>    CELL COMPONENT<br>    cell > membrane fraction<br>    cytoplasm > cytoskeleton<br>    cell > plasma membrane<br>    plasma membrane > integral plasma membrane protein<br>    cell > soluble fraction<br>HUMAN PROTEIN DOMAINS (INTERPRO SIGNATURES)<br>    IPR003962 (FNTYPEIII)<br>    IPR000242 (PRTYPHPHTASE)<br>    IPR000242 (PTPc)<br>    IPR003961 (FN3)<br>    IPR003598 (IGc2)<br>    IPR003599 (IG)<br>    IPR003595 (PTPc motif)<br>    IPR003961 (fn3)<br>    IPR000242 (Y phosphatase)<br>    IPR003006 (ig)<br>    IPR000387 (TYR PHOSPHATASE 2 2)<br>    IPR000242 (TYR PHOSPHATASE PTP2) |
| hP28-021.4 | SEQ ID NO: 74 | HUMAN PANTHER CLASSIFICATIONS<br>    FAMILY (SUBFAMILY)<br>    CELL ADHESION MOLECULE-RELATED(RECEPTOR PROTEIN TYROSINE PHOSPHATASE)<br>    BIOLOGICAL PROCESS<br>    Protein metabolism and modification(2.05.00.00.00) > Protein modification(2.05.03.00.00) > Protein phosphorylation(2.05.03.01.00)<br>    Signal transduction(2.11.00.00.00) > Cell surface receptor mediated signal transduction(2.11.01.00.00) > Other receptor mediated signaling pathway(2.11.01.99.00)<br>    MOLECULAR FUNCTIONS<br>    Receptor(1.01.00.00.00) > Other receptor(1.01.99.00.00)<br>    Phosphatase(1.16.00.00.00) > Protein phosphatase(1.16.01.00.00)<br><br>HUMAN GENE ONTOLOGY<br>    BIOLOGICAL PROCESS<br>    protein modification > protein dephosphorylation<br>    enzyme linked receptor protein signaling pathway > transmembrane receptor protein tyrosine phosphatase signaling pathway |

(continued)

| Human Protein | SEQ ID NO: | FUNCTION |
|---|---|---|
| | | isoprenoid catabolism > phosphate metabolism |
| | | defasciculation of neuron > defasciculation of motor neuron |
| | | enzyme linked receptor protein signaling pathway > transmembrane receptor protein tyrosine kinase signaling pathway |
| | | MOLECULAR FUNCTION |
| | | enzyme > protein phosphatase |
| | | protein phosphatase > protein tyrosine phosphatase |
| | | protein tyrosine phosphatase > prenylated protein tyrosine phosphatase |
| | | protein tyrosine phosphatase > transmembrane receptor protein tyrosine phosphatase |
| | | transmembrane receptor > transmembrane receptor protein tyrosine phosphatase |
| | | protein tyrosine phosphatase > non-membrane spanning protein tyrosine phosphatase |
| | | CELL COMPONENT |
| | | cell > membrane fraction |
| | | cytoplasm > cytoskeleton |
| | | cell > plasma membrane |
| | | plasma membrane > integral plasma membrane protein |
| | | cell > soluble fraction |
| | | HUMAN PROTEIN DOMAINS (INTERPRO SIGNATURES) IPR003962 (FNTYPEIH) |
| | | IPR000242 (PRTYPHPHTASE) |
| | | IPR000242 (PTPc) |
| | | IPR003961 (FN3) |
| | | IPR003598 (IGc2) |
| | | IPR003599 (IG) |
| | | IPR003595 (PTPc motif) |
| | | IPR003961 (fn3) |
| | | IPR000242 (Y phosphatase) |
| | | IPR003006 (ig) |
| | | IPR000387 (TYR PHOSPHATASE 2 2) |
| | | IPR000242 (TYR PHOSPHATASE PTP2) |

A CA protein (CAP) is expressed on a cell surface, wherein the CA protein is selected from the group consisting of SEQ ID NOS: 8, 10, 18, 20, 22, 28, 30, 42, 44, 46, 48, 54, 62, 68, 70, 72 and 74 as shown in Tables 1-7.

Certain aspects of the present invention are described in greater detail in the non-limiting examples that follow.

EXAMPLES

The following examples are put forth so as to provide those of ordinary skill in the art with a complete disclosure and description of how to make and use the present invention, and are not intended to limit the scope of what the inventors regard as their invention nor are they intended to represent that the experiments below are all and only experiments performed. Efforts have been made to ensure accuracy with respect to numbers used (e.g. amounts, temperature, etc.) but some experimental errors and deviations should be accounted for. Unless indicated otherwise, parts are parts by weight, molecular weight is weight average molecular weight, temperature is in degrees Celsius, and pressure is at or near atmospheric.

**Example 1: Insertion site analysis following tumor induction in mice**

Tumors are induced in mice using either mouse mammary tumor virus (MMTV) or murine leukemia virus (MLV). MMTV causes mammary adenocarcinomas and MLV causes a variety of different hematopoetic malignancies (primarily T- or B-cell lymphomas). Three routes of infection are used: (1) injection of neonates with purified virus preparations, (2) infection by milk-borne virus during nursing, and (3) genetic transmission of pathogenic proviruses via the germ-line (*Akvr1* and/or *Mtv2*). The type of malignancy present in each affected mouse is determined by histological analysis of H&E-stained thin sections of formalin-fixed, paraffin-embedded biopsy samples. Host DNA sequences flanking all clonally-integrated proviruses in each tumor are recovered by nested anchored-PCR using two virus-specific primers and two primers specific for a 40 bp double stranded DNA anchor ligated to restriction enzyme digested tumor DNA. Amplified bands representing host/virus junction fragments are cloned and sequenced. Then the host sequences (called "tags") are used to BLAST analyze the Celera mouse genomic sequence. For each individual tag, three parameters are recorded: (1) the mouse chromosome assignment, (2) base pair coordinates at which the integration occurred, and (3) provirus orientation. Using this information, all available tags from all analyzed tumors are mapped to the mouse genome. To identify the protooncogene targets of provirus insertion mutation, the provirus integration pattern at each cluster of integrants is analyzed relative to the locations of all known genes in the transcriptome. The presence of provirus at the same locus in two or more independent tumors is *prima facie* evidence that a protooncogene is present at or very near the proviral integration sites. This is because the genome is too large for random integrations to result in observable clustering. Any clustering that is detected is unequivocal evidence for biological selection during tumorigenesis. In order to identify the human orthologs of the protooncogene targets of provirus insertion mutation, a comparative analysis of syntenic regions of the mouse and human genomes is performed.

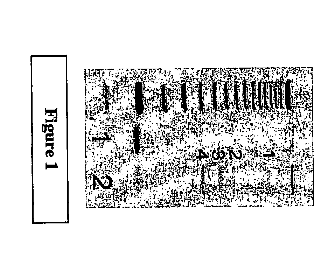

An example of PCR amplification of host/virus junction fragments is presented in Fig 1. Lane 1 contains the amplification products from normal control DNA and lane 2 contains the amplification products from tumor DNA. The bands result from 5' host/virus junction fragments present in the DNA samples. Lane 1 has bands from the *envl3'* LTR junctions from all proviruses (upper) and the host / 5'LTR from the pathogenic endogenous *Mtv2* provirus present in this particular mouse strain. This endogenous provirus is detected because its sequence is identical to the new clonally integrated proviruses in the tumor. All four new clonally integrated proviruses known to be in this tumor are readily detected.

## Example 2: Analysis of Quantitative RT-PCR: Comparative $C_T$ Method.

The expression level of target genes is quantified using the ABI PRISM 7900HT Sequence Detection System (Applied Biosystems, California). The method is based on the quantitation of the initial copy number of target template in comparison to that of a reference (normalizer) housekeeper gene (Pre-Developed TaqMan® Assay Reagents Gene Expression Quantification Protocol, Applied Biosystems, 2001). Accumulation of DNA product with each PCR cycle is related to amplicon efficiency and the initial template concentration. Therefore the amplification efficiency of both the target and the normalizer must be approximately equal. The threshold cycle ($C_T$), which is dependent on the starting template copy number and the DNA amplification efficiency, is a PCR cycle during which PCR product growth is exponential. With a similar dynamic range for the target and normalizer, the comparative $C_T$ method is applicable.

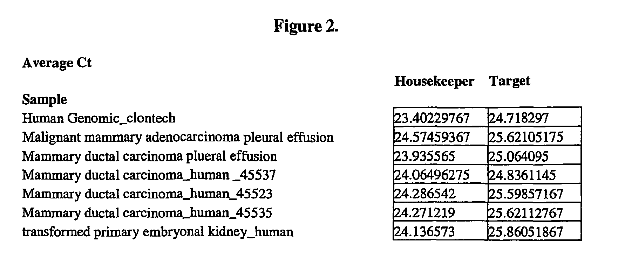

An example of the comparative $C_T$ method of gene expression for quantitative RT-PCR is shown in Figure 2. In the first step, assays are performed in quadruplicate on a normal tissue and several sample tissues. In these tissues, the means and standard deviations of $C_T$ values are determined for housekeeper genes (chosen as controls if shown to be biologically stable among various samples, irrespective of disease state) and for the target gene. Figure 2 shows an example of average $C_T$ values for a housekeeper gene and target gene. These values can fall within a range from upper teens to 40 depending on the intrinsic expression level of the gene in the particular tissue. The coefficient of variance of all replicate sets cannot exceed 1.5%.

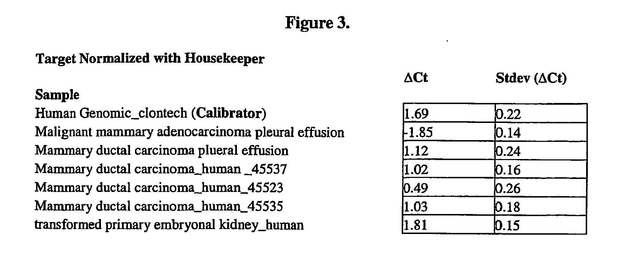

An assessment of how the $\Delta C_T$ changes with template dilution verifies that the efficiencies of the target and housekeeper amplicons are approximately equal if the log input amount of template RNA versus $\Delta C_T$ plot has a slope < 0.10. With the relative efficiencies verified for target and housekeeper, the $\Delta\Delta C_T$ comparative calculation becomes valid, as mentioned above. An example of the calculated difference between the $C_T$ values of target and housekeeper genes ($\Delta C_T$) for various samples is shown in Figure 3. The $\Delta\Delta C_T$ is calculated for each sample by subtracting its $\Delta C_T$ value from the $\Delta C_T$ value of the baseline (calibrator) sample. If the expression is increased in some samples and decreased in others, $\Delta\Delta C_T$ will be a mixture of negative and positive values. The final step in the calculation is to transform these values to absolute values. The formula for this is:

$$\text{Comparative expression level} = 2^{-\Delta\Delta CT}$$

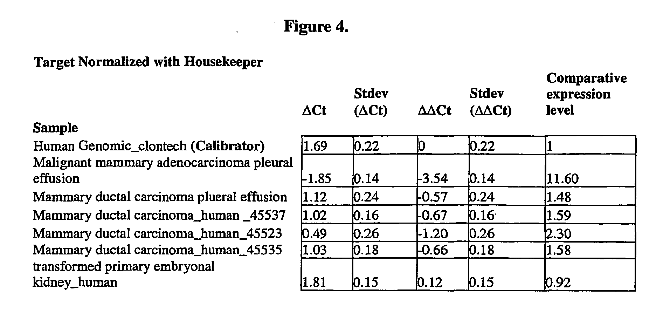

The final value for the calibrator should always be one. Figure 4 shows the $\Delta\Delta C_T$ and comparative expression level for each sample from Figure 3.

**Example 3: Detection of elevated levels of cDNA associated with cancer using arrays.**

cDNA sequences representing a variety of candidate CA genes to be screened for differential expression in cancer are assayed by hybridization on polynucleotide arrays. The cDNA sequences include cDNA clones isolated from cell lines or tissues of interest. The cDNA sequences analyzed also include polynucleotides comprising sequence overlap with sequences in the Unigene database, and which encode a variety of gene products of various origins, functionality, and levels of characterization. cDNAs are spotted onto reflective slides (Amersham) according to methods well known in the art at a density of 9,216 spots per slide representing 4,068 sequences (including controls) spotted in duplicate, with approximately 0.8 µl of an approximately 200ng/µl solution of cDNA.

PCR products of selected cDNA clones corresponding to the gene products of interest are prepared in a 50% DMSO solution. These PCR products are spotted onto Amersham aluminum microarray slides at a density of 9216 clones per array using a Molecular Dynamics Generation III spotting robot. Clones are spotted in duplicate, for a total of 4608 different sequences per chip.

cDNA probes are prepared from total RNA obtained by laser capture microdissection (LCM, Arcturus Enginering Inc., Mountain View, CA) of tumor tissue samples and normal tissue samples isolated from patients.

Total RNA is first reverse transcribed into cDNA using a primer containing a T7 RNA polymerase promoter, followed by second strand DNA synthesis. cDNA is then transcribed *in vitro* to produce antisense RNA using the T7 promoter-mediated expression (see, *e.g.,* Luo et al. (1999) Nature Med 5:117-122), and the antisense RNA is then converted into cDNA. The second set of cDNAs are again transcribed i*n vitro,* using the T7 promoter, to provide antisense RNA. This antisense RNA is then fluorescently labeled, or the RNA is again converted into cDNA, allowing for a third round of T7-mediated amplification to produce more antisense RNA. Thus the procedure provides for two or three rounds of *in vitro* transcription to produce the final RNA used for fluorescent labeling. Probes are labeled by making fluorescently labeled cDNA from the RNA starting material. Fluorescently labeled cDNAs prepared from the tumor RNA sample are compared to fluorescently labeled cDNAs prepared from normal cell RNA sample. For example, the cDNA probes from the normal cells are labeled with Cy3 fluorescent dye (green) and the cDNA probes prepared from suspected cancer cells are labeled with Cy5 fluorescent dye (red).

The differential expression assay is performed by mixing equal amounts of probes from tumor cells and normal cells of the same patient. The arrays are prehybridized by incubation for about 2 hrs at 60°C in 5x SSC, 0.2% SDS, 1 mM EDTA, and then washing three times in water and twice in isopropanol. Following prehybridization of the array, the probe mixture is then hybridized to the array under conditions of high stringency (overnight at 42°C in 50% formamide, 5X SSC, and 0.2% SDS. After hybridization, the array is washed at 55°C three times as follows: 1) first wash in 1X SSC/ 0.2% SDS; 2) second wash in 0.1X SSC/0.2% SDS; and 3) third wash in 0.1X SSC.

The arrays are then scanned for green and red fluorescence using a Molecular Dynamics Generation III dual color laser-scanner/detector. The images are processed using BioDiscovery Autogene software, and the data from each scan set normalized. The experiment is repeated, this time labeling the two probes with the opposite color in order to perform the assay in both "color directions." Each experiment is sometimes repeated with two more slides (one in each color direction). The data from each scan is normalized, and the level of fluorescence for each sequence on the array expressed as a ratio of the geometric mean of 8 replicate spots/genes from the four arrays or 4 replicate spots/gene from 2 arrays or some other permutation.

Normalization: The objective of normalization is to generate a cDNA library in which all transcripts expressed in a particular cell type or tissue are equally represented (S.M. Weissman, Mol Biol. Med. 4(3):133-143 (1987); Patanjali, et al., Proc. Natl. Acad. Sci. USA 88(5):1943-1947 (1991)), and therefore isolation of as few as 30,000 recombinant clones in an optimally normalized library may represent the entire gene expression repertoire of a cell, estimated to number 10,000 per cell.

Total RNA is extracted from harvested cells using RNeasy™ Protect Kit (Qiagen, Valencia, CA), following manufacturer's recommended procedures. RNA is quantified using RiboGreen™ RNA quantification kit (Molecular Probes, Inc. Eugene, OR). One µg of total RNA is reverse transcribed and PCR amplified using SMART™ PCR cDNA synthesis kit (ClonTech, Palo Alto, CA). The cDNA products are size-selected by agarose gel electrophoresis using standard procedures (Sambrook, J.T., et al. Molecular Cloning: A Laboratory Manual, 2d ed., Cold Spring Harbor Laboratory Press, NY). The cDNA is extracted using Bio 101 Geneclean® II kit (Qbiogene, Carlsbad, CA). Normalization of the cDNA is carried out using kinetics of hybridization principles: 1.0 µg of cDNA is denatured by heat at 100° C for 10 minutes, then incubated at 42° C for 42 hours in the presence of 120 mM Nad, 10 mM Tris.HCl (pH=8.0), 5 mM EDTA.Na+ and 50% formamide. Single-stranded cDNA ("normalized") is purified by hydroxyapatite chromatography (#130-0520, BioRad, Hercules, CA) following the manufacturer's recommended procedures, amplified and converted to double-stranded cDNA by three cycles of PCR amplification, and cloned into plasmid vectors using standard procedures (Sambrook, J.T., et al. Molecular Cloning: A Laboratory Manual, 2d ed., Cold Spring Harbor Laboratory Press, NY). All primers/adaptors used in the normalization and cloning process are provided by the manufacturer in the SMART™ PCR cDNA synthesis kit (ClonTech, Palo Alto, CA). Supercompetent cells (XL-2 Blue Ultracompetent Cells, Stratagene, California) are transfected with the normalized cDNA libraries, plated on solid media and grown overnight at 36° C.

The sequences of 10,000 recombinants per normalized library are analyzed by capillary sequencing using the ABI PRISM 3700 DNA Analyzer (Applied Biosystems, California). To determine the representation of transcripts in a library, BLAST analysis is performed on the clone sequences to assign transcript identity to each isolated clone, i.e., the sequences of the isolated polynucleotides are first masked to eliminate low complexity sequences using the XBLAST masking program (Claverie "Effective Large-Scale Sequence Similarity Searches," Computer Methods for Macromolecular Sequence Analysis, Doolittle, ed., Meth. Enzymol. 266:212-227 Academic Press, NY, NY (1996); see particularly Claverie, in "Automated DNA Sequencing and Analysis Techniques" Adams et al., eds., Chap. 36, p. 267 Academic Press, San Diego, 1994 and Claverie et al. Comput. Chem. (1993) 17:191). Generally, masking does not influence the final search results, except to eliminate sequences of relative little interest due to their low complexity, and to eliminate multiple "hits" based on similarity to repetitive regions common to multiple sequences, e.g., Alu repeats. The remaining sequences are then used in a BLASTN vs. GenBank search. The sequences are also used as query sequence in a BLASTX vs. NRP (non-redundant proteins) database search.

Automated sequencing reactions are performed using a Perkin-Elmer PRISM Dye Terminator Cycle Sequencing Ready Reaction Kit containing AmpliTaq DNA Polymerase, FS, according to the manufacturer's directions. The reactions are cycled on a GeneAmp PCR System 9600 as per manufacturer's instructions, except that they are annealed at 20° C. or 30° C. for one minute. Sequencing reactions are ethanol precipitated, pellets are resuspended in 8 microliters of loading buffer, 1.5 microliters is loaded on a sequencing gel, and the data is collected by an ABI PRISM 3700 DNA Sequencer. (Applied Biosystems, Foster City, CA).

The number of times a sequence is represented in a library is determined by performing sequence identity analysis on the cloned cDNA sequences and assigning transcript identity to each isolated clone. First, each sequence is checked to determine if it is a bacterial, ribosomal, or mitochondrial contaminant. Such sequences are excluded from the subsequent analysis. Second, sequence artifacts, such as vector and repetitive elements, are masked and/or removed from each sequence.

The remaining sequences are compared via BLAST (Altschul et. al, J. Mol. Biol., 215:40, 1990) to GenBank and EST databases for gene identification and are compared with each other via FastA (Pearson & Lipman, PNAS, 85: 2444, 1988) to calculate the frequency of cDNA appearance in the normalized cDNA library. The sequences are also searched against the GenBank and GeneSeq nucleotide databases using the BLASTN program (BLASTN 1.3MP: Altschul et al., J. Mol. Bio. 215:403, 1990). Fourth, the sequences are analyzed against a non-redundant protein (NRP) database with the BLASTX program (BLASTX 1.3MP: Altschul et al., supra). This protein database is a combination of the Swiss-Prot, PIR, and NCBI GenPept protein databases. The BLASTX program is run using the default BLOSUM-62 substitution matrix with the filter parameter: "xnu+seg". The score cutoff utilized is 75. Assembly of overlapping clones into contigs is done using the program Sequencher (Gene Codes Corp.; Ann Arbor, Mich.). The assembled contigs are analyzed using the programs in the GCG package (Genetic Computer Group, University Research Park, 575 Science Drive, Madison, Wis. 53711) Suite Version 10.1.

## Example 4: Detection of CA -Sequences in Human Cancer Cells and Tissues.

DNA from prostate and breast cancer tissues and other human cancer tissues, human colon, normal human tissues including non-cancerous prostate, and from other human cell lines are extracted following the procedure of Delli Bovi et al. (1986, Cancer Res. 46:6333-6338). The DNA is resuspended in a solution containing 0.05 M Tris HC1 buffer, pH 7.8, and 0.1 mM EDTA, and the amount of DNA recovered is determined by microfluorometry using Hoechst 33258 dye. Cesarone, C. et al., Anal Biochem 100:188-197 (1979).

Polymerase chain reaction (PCR) is performed using Taq polymerase following the conditions recommended by the manufacturer (Perkin Elmer Cetus) with regard to buffer, $Mg^{2+}$, and nucleotide concentrations. Thermocycling is performed in a DNA cycler by denaturation at 94° C. for 3 min. followed by either 35 or 50 cycles of 94° C. for 1.5 min., 50° C. for 2 min. and 72° C. for 3 min. The ability of the PCR to amplify the selected regions of the CA gene is tested by using a cloned CA polynucleotide(s) as a positive template(s). Optimal $Mg^{2+}$, primer concentrations and requirements for the different cycling temperatures are determined with these templates. The master mix recommended by the manufacturer is used. To detect possible contamination of the master mix components, reactions without template are routinely tested.

Southern blotting and hybridization are performed as described by Southern, E. M., (J. Mol. Biol. 98:503-517, 1975), using the cloned sequences labeled by the random primer procedure (Feinberg, A. P., et al., 1983, Anal. Biochem. 132:6-13). Prehybridization and hybridization are performed in a solution containing 6xSSPE, 5% Denhardt's, 0.5% SDS, 50% formamide, 100 μg/ml denatured salmon testis DNA, incubated for 18 hrs at 42° C., followed by washings with 2xSSC and 0.5% SDS at room temperature and at 37° C. and finally in 0.1xSSC with 0.5% SDS at 68° C. for 30 min (Sambrook et al., 1989, in "Molecular Cloning: A Laboratory Manual", Cold Spring Harbor Lab. Press). For paraffin-embedded tissue sections the conditions described by Wright and Manos (1990, in "PCR Protocols", Innis et al., eds.,

Academic Press, pp. 153-158) are followed using primers designed to detect a 250 bp sequence.

**Example 5: Expression of cloned polynucleotides in host cells.**

To study the protein products of CA genes, restriction fragments from CA DNA are cloned into the expression vector pMT2 (Sambrook, et al., Molecular Cloning: A Laboratory Manual, Cold Spring Harbor Laboratory Press pp 16.17-16.22 (1989)) and transfected into COS cells grown in DMEM supplemented with 10% FCS. Transfections are performed employing calcium phosphate techniques (Sambrook, et al (1989) pp. 16.32-16.40, supra) and cell lysates are prepared forty-eight hours after transfection from both transfected and untransfected COS cells. Lysates are subjected to analysis by immunoblotting using anti-peptide antibody.

In immunoblotting experiments, preparation of cell lysates and electrophoresis are performed according to standard procedures. Protein concentration is determined using BioRad protein assay solutions. After semi-dry electrophoretic transfer to nitrocellulose, the membranes are blocked in 500 mM NaCl, 20 mM Tris, pH 7.5, 0.05% Tween-20 (TTBS) with 5% dry milk. After washing in TTBS and incubation with secondary antibodies (Amersham), enhanced chemiluminescence (ECL) protocols (Amersham) are performed as described by the manufacturer to facilitate detection.

**Example 6: Generation of antibodies against polypeptides.**

Polypeptides, unique to CA genes are synthesized or isolated from bacterial or other (e.g., yeast, baculovirus) expression systems and conjugated to rabbit serum albumin (RSA) with m-maleimido benzoic acid N-hydroxysuccinimide ester (MBS) (Pierce, Rockford, Ill.). Immunization protocols with these peptides are performed according to standard methods. Initially, a pre-bleed of the rabbits is performed prior to immunization. The first immunization includes Freund's complete adjuvant and 500 $\mu$g conjugated peptide or 100 $\mu$g purified peptide. All subsequent immunizations, performed four weeks after the previous injection, include Freund's incomplete adjuvant with the same amount of protein. Bleeds are conducted seven to ten days after the immunizations.

For affinity purification of the antibodies, the corresponding CA polypeptide is conjugated to RSA with MBS, and coupled to CNBr-activated Sepharose (Pharmacia, Uppsala, Sweden). Antiserum is diluted 10-fold in 10 mM Tris-HCl, pH 7.5, and incubated overnight with the affinity matrix. After washing, bound antibodies are eluted from the resin with 100 mM glycine, pH 2.5.

**Example 7: Generation of monoclonal antibodies against a CA polypeptide**

A non-denaturing adjuvant (Ribi, R730, Corixa, Hamilton MT) is rehydrated to 4ml in phosphate buffered saline. 100$\mu$l of this rehydrated adjuvant is then diluted with 400$\mu$l of Hank's Balanced Salt Solution and this is then gently mixed with the cell pellet used for immunization. Approximately 500 $\mu$g conjugated peptide or 100 $\mu$g purified peptide and Freund's complete are injected into Balb/c mice via foot-pad, once a week. After 6 weeks of weekly injection, a drop of blood is drawn from the tail of each immunized animal to test the titer of antibodies against CA polypeptides using FACS analysis. When the titer reaches at least 1:2000, the mice are sacrificed in a $CO_2$ chamber followed by cervical dislocation. Lymph nodes are harvested for hybridoma preparation. Lymphocytes from mice with the highest titer are fused with the mouse myeloma line X63-Ag8.653 using 35% polyethylene glycol 4000. On day 10 following the fusion, the hybridoma supernatants are screened for the presence of CAP-specific monoclonal antibodies by fluorescence activated cell sorting (FACS). Conditioned medium from each hybridoma is incubated for 30 minutes with a combined aliquot of PC3, Colo-205, LnCap, or Panc-1 cells. After incubation, the cell samples are washed, resuspended in 0.1 ml diluent and incubated with 1 $\mu$g/ml of FITC conjugated F(ab')2 fragment of goat anti-mouse IgG for 30 min at $4^0$C. The cells are washed, resuspended in 0.5 ml FACS diluent and analyzed using a FACScan cell analyzer (Becton Dickinson; San Jose, CA). Hybridoma clones are selected for further expansion, cloning, and characterization based on their binding to the surface of one or more of cell lines which express the CA polypeptide as assessed by FACS. A hybridoma making a monoclonal antibody designated mAbCA which binds an antigen designated Ag-CA.x and an epitope on that antigen designated Ag-CA.x.1 is selected.

**Example 8: ELISA assay for Detecting CA related antigens.**

To test blood samples for antibodies that bind specifically to recombinantly produced CA antigens, the following procedure is employed. After a recombinant CA related protein is purified, the recombinant protein is diluted in PBS to a concentration of 5 $\mu$g/ml (500 ng/100 $\mu$l). 100 microliters of the diluted antigen solution is added to each well of a 96-well Immulon 1 plate (Dynatech Laboratories, Chantilly, Va.), and the plate is then incubated for 1 hour at room temperature, or overnight at 4° C., and washed 3 times with 0.05% Tween 20 in PBS. Blocking to reduce nonspecific binding of antibodies is accomplished by adding to each well 200 $\mu$l of a 1% solution of bovine serum albumin in PBS/Tween 20 and incubation for 1 hour. After aspiration of the blocking solution, 100 μl of the primary antibody solution (anticoagulated whole blood, plasma, or serum), diluted in the range of 1/16 to 1/2048 in blocking solution, is added and incubated for 1 hour at room temperature or overnight at 4° C. The wells are then washed 3 times, and 100 μl of goat anti-human IgG antibody conjugated to horseradish peroxidase (Organon Teknika, Durham, N.C.), diluted 1/500 or 1/1000 in PBS/Tween 20, 100 μl of o-phenylenediamine dihydrochloride (OPD, Sigma) solution is added to each well and incubated for 5-15 minutes. The OPD solution is prepared by dissolving a 5 mg OPD tablet in 50 ml 1% methanol in $H_2O$ and adding 50 μl 30% $H_2O_2$ immediately before use. The reaction is stopped by adding 251 of 4M $H_2SO_4$. Absorbances are read at 490 nm in a microplate reader (Bio-Rad).

## Example 9: Identification and characterization of CA antigen on cancer cell surface

A cell pellet of proximately 25 ul packed cell volume of a cancer cell preparation is lysed by first diluting the cells to 0.5 ml in water followed by freezing and thawing three times. The solution is centrifuged at 14,000 rpm. The resulting pellet, containing the cell membrane fragments, is resuspended in 50 μl of SDS sample buffer (Invitrogen, Carlsbad, CA). The sample is heated at 80°C for 5 minutes and then centrifuged for 2 minutes at 14,000 rpm to remove any insoluble materials.

The samples are analyzed by Western blot using a 4 to 20% polyacrylamide gradient gel in Tris-Glycine SDS (Invitrogen; Carlsbad CA) following the manufacturer's directions. Ten microliters of membrane sample are applied to one lane on the polyacrylamide gel. A separate 10 μL sample is reduced first by the addition of 2 μL of dithiothreitol (100 mM) with heating at 80°C for 2 minutes and then loaded into another lane. Pre-stained molecular weight markers SeeBlue Plus2 (Invitrogen; Carlsbad, CA) are used to assess molecular weight on the gel. The gel proteins are transferred to a nitrocellulose membrane using a transfer buffer of 14.4 g/l glycine, 3 g/l of Tris Base, 10% methanol, and 0.05% SDS. The membranes are blocked, probed with a CAP-specific monoclonal antibody (at a concentration of 0.5 ug/ml), and developed using the Invitrogen WesternBreeze Chromogenic Kit-AntiMouse according to the manufacturer's directions. In the reduced sample of the tumor cell membrane samples, a prominent band is observed migrating at a molecular weight within about 10% of the predicted molecular weight of the corresponding CA protein.

## Example 10: Preparation of vaccines.

The present invention also relates to a method of stimulating an immune response against cells that express CA polypeptides in a patient using CA polypeptides of the invention that act as an antigen produced by or associated with a malignant cell. This aspect of the invention provides a method of stimulating an immune response in a human against cancer cells or cells that express CA polynucleotides and polypeptides. The method comprises the step of administering to a human an immunogenic amount of a polypeptide comprising: (a) the amino acid sequence of a huma CA protein or (b) a mutein or variant of a polypeptide comprising the amino acid sequence of a human endogenous retrovirus CA protein.

## Example 11: Generation of transgenic animals expressing polypeptides as a means for testing therapeutics.

CA nucleic acids are used to generate genetically modified non-human animals, or site specific gene modifications thereof, in cell lines, for the study of function or regulation of prostate tumor-related genes, or to create animal models of diseases, including prostate cancer. The term "transgenic" is intended to encompass genetically modified animals having an exogenous CA gene(s) that is stably transmitted in the host cells where the gene(s) may be altered in sequence to produce a modified protein, or having an exogenous CA LTR promoter operably linked to a reporter gene. Transgenic animals may be made through a nucleic acid construct randomly integrated into the genome. Vectors for stable integration include plasmids, retroviruses and other animal viruses, YACs, and the like. Of interest are transgenic mammals, e.g. cows, pigs, goats, horses, etc., and particularly rodents, e.g. rats, mice, etc.

The modified cells or animals are useful in the study of CA gene function and regulation. For example, a series of small deletions and/or substitutions may be made in the CA genes to determine the role of different genes in tumorigenesis. Specific constructs of interest include, but are not limited to, antisense constructs to block CA gene expression, expression of dominant negative CA gene mutations, and over-expression of a CA gene. Expression of a CA gene or variants thereof in cells or tissues where it is not normally expressed or at abnormal times of development is provided. In addition, by providing expression of proteins derived from CA in cells in which it is otherwise not normally produced, changes in cellular behavior can be induced.

DNA constructs for random integration need not include regions of homology to mediate recombination. Conveniently, markers for positive and negative selection are included. For various techniques for transfecting mammalian cells, see Keown et al., Methods in Enzymology 185:527-537 (1990).

For embryonic stem (ES) cells, an ES cell line is employed, or embryonic cells are obtained freshly from a host, e.g. mouse, rat, guinea pig, etc. Such cells are grown on an appropriate fibroblast-feeder layer or grown in the presence of appropriate growth factors, such as leukemia inhibiting factor (LIF). When ES cells are transformed, they may be used to produce transgenic animals. After transformation, the cells are plated onto a feeder layer in an appropriate medium. Cells containing the construct may be detected by employing a selective medium. After sufficient time for colonies to grow, they are picked and analyzed for the occurrence of integration of the construct. Those colonies that are positive may then be used for embryo manipulation and blastocyst injection. Blastocysts are obtained from 4 to 6 week old superovulated females. The ES cells are trypsinized, and the modified cells are injected into the blastocoel of the blastocyst. After injection, the blastocysts are returned to each uterine horn of pseudopregnant females. Females are then allowed to go to term and the resulting chimeric animals screened for cells bearing the construct. By providing for a different phenotype of the blastocyst and the ES cells, chimeric progeny can be readily detected.

The chimeric animals are screened for the presence of the modified gene and males and females having the modification are mated to produce homozygous progeny. If the gene alterations cause lethality at some point in development, tissues or organs are maintained as allogeneic or congenic grafts or transplants, or in in vitro culture. The transgenic animals may be any non-human mammal, such as laboratory animals, domestic animals, etc. The transgenic animals are used in functional studies, drug screening, etc., e.g. to determine the effect of a candidate drug on prostate cancer, to test potential therapeutics or treatment regimens, etc.

## Example 12: Diagnostic Imaging Using CA Specific Antibodies

The present invention encompasses the use of antibodies to CA polypeptides to accurately stage cancer patients at initial presentation and for early detection of metastatic spread of cancer. Radioimmunoscintigraphy using monoclonal antibodies specific for CA polypeptides can provide an additional cancer-specific diagnostic test. The monoclonal antibodies of the instant invention are used for histopathological diagnosis of carcinomas.

Subcutaneous human xenografts of cancer cells in nude mice is used to test whether a technetium-99m ($^{99m}$Tc)-labeled monoclonal antibody of the invention can successfully image the xenografted cancer by external gamma scintography as described for seminoma cells by Marks, et al., Brit. J. Urol. 75:225 (1995). Each monoclonal antibody specific for a CA polypeptide is purified from ascitic fluid of BALB/c mice bearing hybridoma tumors by affinity chromatography on protein A-Sepharose. Purified antibodies, including control monoclonal antibodies such as an avidin-specific monoclonal antibody (Skea, et al., J. Immunol. 151:3557 (1993)) are labeled with $^{99m}$Tc following reduction, using the methods of Mather, et al., J. Nucl. Med. 31:692 (1990) and Zhang et al., Nucl. Med. Biol. 19:607 (1992). Nude mice bearing human cancer cells are injected intraperitoneally with 200-500 $\mu$Ci of $^{99m}$Tc-labeled antibody. Twenty-four hours after injection, images of the mice are obtained using a Siemens ZLC3700 gamma camera equipped with a 6 mm pinhole collimator set approximately 8 cm from the animal. To determine monoclonal antibody biodistribution following imaging, the normal organs and tumors are removed, weighed, and the radioactivity of the tissues and a sample of the injectate are measured. Additionally, CA-specific antibodies conjugated to antitumor compounds are used for cancer-specific chemotherapy.

## Example 13: Immunohistochemical methods

Frozen tissue samples from cancer patients are embedded in an optimum cutting temperature (OCT) compound and quick-frozen in isopentane with dry ice. Cryosections are cut with a Leica 3050 CM mictrotome at thickness of 5 $\mu$m and thaw-mounted on vectabound-coated slides. The sections are fixed with ethanol at - 20°C and allowed to air dry overnight at room temperature. The fixed sections are stored at -80°C until use. For immunohistochemistry, the tissue sections are retrieved and first incubated in blocking buffer (PBS, 5% normal goat serum, 0.1% Tween 20) for 30 minutes at room temperature, and then incubated with the CA protein-specific monoclonal antibody and control monoclonal antibodies diluted in blocking buffer (1 $\mu$g/ml) for 120 minutes. The sections are then washed three times with the blocking buffer. The bound monoclonal antibodies are detected with a goat anti-mouse IgG + IgM (H+L) F(ab')2-peroxidase conjugates and the peroxidase substrate diaminobenzidine (1 mg/ml, Sigma Catalog No. D 5637) in 0.1 M sodium acetate buffer pH 5.05 and 0.003% hydrogen peroxide (Sigma cat. No. H1009). The stained slides are counter-stained with hematoxylin and examined under Nikon microscope.

Monoclonal antibody against a CA protein (antigen) is used to test reactivity with various cell lines from different types of tissues. Cells from different established cell lines are removed from the growth surface without using proteases, packed and embedded in OCT compound. The cells are frozen and sectioned, then stained using a standard IHC protocol. The CellArray ™ technology is described in WO 01/43869. Normal tissue (human) obtained by surgical resection are frozen and mounted. Cryosections are cut with a Leica 3050 CM mictrotome at thickness of 5 $\mu$m and thaw-mounted on vectabound-coated slides. The sections are fixed with ethanol at -20°C and allowed to air dry overnight at room temperature. PolyMICA™ Detection kit is used to determine binding of a CA-specific monoclonal antibody to normal tissue. Primary monoclonal antibody is used at a final concentration of 1 $\mu$g/ml.

All publications and patent applications cited in this specification are herein incorporated by reference as if each individual publication or patent application were specifically and individually indicated to be incorporated by reference.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the spirit or scope of the appended claims.

PREFERRED EMBODIMENTS:

1. A nucleic acid array for detecting a cancer associated (CA) nucleic acid comprising: at least two nucleic acid probes each comprising at least 10 contiguous nucleotides of at least two sequences selected from the group consisting of the polynucleotide sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, or its complement.

2. The nucleic acid array according to embodiment 1, the probes comprising at least 15 contiguous nucleotides.

3. The nucleic acid array according to embodiment 1, the probes comprising at least 20 contiguous nucleotides.

4. A peptide array comprising at least two isolated polypeptides, each encoded within an open reading frame of a CA sequence selected from the group consisting of the polynucleotide sequences of SEQ ID NOS: 7, 15, 25, 43, 59, 68, 71, 79, 85, 99, 111, 121, 140, 148, 156, 164, 176, 186, 202, 219, 227, 235, 245, 259, 277, 289, 297, 307, 313, 319, 331, 339, 347, 361, 367, 375, 385, 399, and 405 shown in Tables 1-46, or its complement.

5. The peptide array of embodiment 4, wherein each said polypeptide comprises the amino acid sequence encoded by a polynucleotide selected from the group consisting of SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46.

6. The peptide array of embodiment 4, wherein each said polypeptide comprises the amino acid sequence encoded by a polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46.

7. The peptide array of embodiment 4, wherein each said polypeptide comprises the amino acid sequence of an epitope of the amino acid sequence of a CA polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46.

8. The peptide array of embodiment 4, wherein said polypeptide is expressed on a cell surface, wherein the CA protein selected from the group consisting of SEQ ID NOS: 8, 10, 18, 20, 22, 28, 30, 42, 44, 46, 48, 54, 62, 68, 70, 72 and 74.

9. A compound that binds to a polypeptide of the peptide array of embodiment 4.

10. The compound according to embodiment 9, wherein the compound is chemically synthesized.

11. The compound according to embodiment 9, wherein the compound is naturally occurring.

12. An isolated antibody or antigen binding fragment thereof, that binds to a polypeptide comprising the amino acid sequence encoded by a polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46.

13. The isolated antibody or antigen binding fragment thereof according the embodiment 12, wherein said antibody or fragment thereof is attached to a solid support.

14. The isolated antibody or antigen binding fragment thereof according the embodiment 12, wherein said antibody is a monoclonal antibody.

15. The isolated antibody or antigen binding fragment thereof according the embodiment 12, wherein said antibody is a polyclonal antibody.

16. The isolated antibody or antigen binding fragment thereof according the embodiment 12, wherein said antibody or fragment thereof further comprises a detectable label.

17. An isolated antibody that binds to a polypeptide, or antigen binding fragment thereof, according to to embodiment 8, prepared by a method comprising the steps of: (i) immunizing a host animal with a composition comprising said polypeptide, or antigen binding fragment thereof, and (ii) collecting cells from said host expressing antibodies against the antigen or antigen binding fragment thereof.

18. The monoclonal antibody according to embodiment 14, wherein the monoclonal antibody binds to the extracellular domain of the CA protein.

19. The monoclonal antibody according to embodiment 14, wherein the monoclonal antibody binds to at least one human cancer cell line.

20. The monoclonal antibody according to embodiment 14, wherein the monoclonal antibody is prepared by a process comprising:

(a) providing a hybridoma capable of producing the monoclonal antibody; and
(b) culturing the hybridoma under conditions that provide for the production of the monoclonal antibody by the hybridoma.

21. A hybridoma that produces the monoclonal antibody according to embodiment 14.

22. The antibody according to embodiment 12, wherein the antibody is a humanized antibody.

23. The antibody according to embodiment 12, wherein the CAP is expressed on a cancer cell surface but not on a normal cell surface.

24. The antibody according to embodiment 12, wherein the CAP is differentially expressed on a cancer cell surface relative to a normal cell surface.

25. The antibody according to embodiment 12, wherein the antibody is linked to a therapeutic agent.

26. The antibody according to embodiment 14, wherein the antibody is linked to a therapeutic agent.

27. A pharmaceutical composition comprising the antibody according to embodiment 12 and a pharmaceutically acceptable excipient.

28. A pharmaceutical composition comprising the antibody according to embodiment 14 and a pharmaceutically acceptable excipient.

29. A pharmaceutical composition comprising the antibody according to embodiment 15 and a pharmaceutically acceptable excipient.

30. A kit for detecting cancer cells comprising the antibody according to embodiment 12.

31. A kit for detecting cancer cells comprising the monoclonal antibody according to embodiment 14.

32. A method for detecting a presence or an absence of cancer cells in an individual, the method comprising: contacting cells from the individual with the antibody according to embodiment 12; and detecting a complex of a CAP from the cancer cells and the antibody, wherein detection of the complex correlates with the presence of cancer cells in the individual.

33. A kit for diagnosing the presence of cancer in a test sample, said kit comprising at least two polynucleotides that selectively hybridize to at least two CA polynucleotide sequences selected from the group consisting of the polynucleotide sequences SEQ ID NOS: 7, 15, 25, 43, 59, 68, 71, 79, 85, 99, 111, 121, 140, 148, 156, 164, 176, 186, 202, 219, 227, 235, 245, 259, 277, 289, 297, 307, 313, 319, 331, 339, 347, 361, 367, 375, 385, 399, and 405 shown in Tables 1-46, or its complement.

34. A kit for diagnosing the presence of cancer in a test sample, said kit comprising at least two polynucleotides that selectively hybridize to the sequence of at least two polynucleotide sequences selected from the group consisting of the polynucleotide sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, a fragment thereof, or their complement.

35. An electronic library comprising a polynucleotide, or fragment thereof, comprising at least two CA polynucleotide sequences selected from the group consisting of the polynucleotide sequences of SEQ ID NOS: 7, 15, 25, 43, 59, 68, 71, 79, 85, 99, 111, 121, 140, 148, 156, 164, 176, 186, 202, 219, 227, 235, 245, 259, 277, 289, 297, 307, 313, 319, 331, 339, 347, 361, 367, 375, 385, 399, and 405 shown in Tables 1-46, or its complement.

36. An electronic library comprising a polynucleotide, or fragment thereof, comprising at least two CA polynucleotide sequences selected from the group consisting of the polynucleotide sequences of SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46.

37. An electronic library comprising a polypeptide, or fragment thereof, comprising at least two CA polypeptide sequences selected from the group consisting of the polypeptide sequences of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1- 46.

38. A method of screening for anticancer activity comprising:

(a) providing a cell that expresses a cancer associated (CA) gene encoded by a nucleic acid sequence selected from the group consisting of the sequences SEQ ID NOS: 7, 15, 25, 43, 59, 68, 71, 79, 85, 99, 111, 121, 140, 148, 156, 164, 176, 186, 202, 219, 227, 235, 245, 259, 277, 289, 297, 307, 313, 319, 331, 339, 347, 361, 367, 375, 385, 399, and 405 shown in Tables 1-46, or fragment thereof;
(b) contacting a tissue sample derived from a cancer cell with an anticancer drug candidate; and
(c) monitoring an effect of the anticancer drug candidate on an expression of the CA polynucleotide in the tissue sample.

39. The method of screening for anticancer activity according to embodiment 38, wherein the CA gene comprises at least one nucleic acid sequence selected from the group consisting of the sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1- 46.

40. The method of screening for anticancer activity according to embodiment 38, further comprising:

1. (d) comparing the level of expression in the absence of said drug candidate to the level of expression in the presence of the drug candidate.

41. The method of screening for anticancer activity according to embodiment 39, wherein the drug candidate is an inhibitor of transcription and further wherein the nucleic acid sequence is selected from the group consisting of SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46.

42. A method for detecting cancer associated with expression of a polypeptide in a test cell sample, comprising the steps of:

(i) detecting a level of expression of at least one polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46, or a fragment thereof; and
(ii) comparing the level of expression of the polypeptide in the test sample with a level of expression of polypeptide in a normal cell sample, wherein an altered level of expression of the polypeptide in the test cell sample relative to the level of polypeptide expression in the normal cell sample is indicative of the presence of cancer in the test cell sample.

43. A method for detecting cancer associated with expression of a polypeptide in a test cell sample, comprising the steps of:

(i) detecting a level of activity of at least one polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46, or a fragment thereof, wherein said activity corresponds to at least one activity for the polypeptide listed in Tables 1-46; and
(iii) comparing the level of activity of the polypeptide in the test sample with a level of activity of polypeptide in a normal cell sample, wherein an altered level of activity of the polypeptide in the test cell sample relative to the level of polypeptide activity in the normal cell sample is indicative of the presence of cancer in the test cell sample.

44. A method for detecting cancer associated with the presence of an antibody in a test serum sample, comprising the steps of: (i) detecting a level of an antibody against an antigenic polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46, or antigenic fragment thereof; and 2. (ii) comparing said level of said antibody in the test sample with a level of said antibody in the control sample, wherein an altered level of antibody in said test sample relative to the level of antibody in the control sample is indicative of the presence of cancer in the test serum sample.

45. A method for screening for a bioactive agent capable of modulating the activity of a CA protein (CAP), wherein said CAP is encoded by a nucleic acid comprising a nucleic acid sequence selected from the group consisting of the polynucleotide sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, said method comprising: a) combining said CAP and a candidate bioactive agent; and b) determining the effect of the candidate agent on the bioactivity of said CAP.

46. The method of screening for the bioactive agent according to embodiment 45, wherein the bioactive agent affects the expression of the CA protein (CAP).

47. The method of screening for the bioactive agent according to embodiment 45, wherein the bioactive agent affects the activity of the CA protein (CAP), wherein such activity is selected from the activities listed in Tables 1-46.

48. The method of screening for the bioactive agent according to embodiment 45, wherein the bioactive agent is a modulator of an activity selected from the group consisting of: G-protein coupled receptor, nucleotide binding, nucleic acid binding, calcium binding protein, apoptosis, cell cycle regulator, chromatin protein, transcription factor, transport protein, signal transduction, nuclear receptor, growth factor, protein kinase, protein phosphorylase, peptidase, electron transport, thioredoxin, semaphorin, signaling, and motility as shown in Tables 1-46.

49. A method for diagnosing cancer comprising: a) determining the expression of one or more genes comprising a nucleic acid sequence selected from the group consisting of the human genomic and mRNA sequences outlined in Tables 1-46, in a first tissue type of a first individual; and b) comparing said expression of said gene(s) from a second normal tissue type from said first individual or a second unaffected individual; wherein a difference in said expression indicates that the first individual has cancer.

50. A method for treating cancers comprising administering to a patient an inhibitor of a CA protein (CAP), wherein said CAP is encoded by a nucleic acid comprising a nucleic acid sequence selected from the group consisting of the human nucleic acid sequences in Tables 1-46.

51. The method for treating cancers according to embodiment 50, wherein the inhibitor of a CA protein (CAP) binds to the CA protein.

52. A method for inhibiting expression of a cancer associated (CA) gene in a cell comprising: contacting a cell expressing a CA gene with a double stranded RNA comprising a sequence capable of hybridizing to a cancer associated (CA) mRNA corresponding to the polynucleotide sequences of SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, in an amount sufficient to elicit RNA interference; and inhibiting expression of the CA gene in the cell.

53. The method of embodiment 52, wherein the double stranded RNA is provided by introducing a short interfering RNA (siRNA) into the cell by a method selected from the group consisting of transfection, electroporation, and microinjection.

54. The method of embodiment 52, wherein the double stranded RNA is provided by introducing a short interfering RNA (siRNA) into the cell by an expression vector.

## Claims

1. A nucleic acid array for detecting a cancer associated (CA) nucleic acid comprising: at least two nucleic acid probes each comprising at least 10 contiguous nucleotides of at least two sequences selected from the group consisting of the polynucleotide sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, or its complement.

2. A peptide array comprising at least two isolated polypeptides, each encoded within an open reading frame of a CA sequence selected from the group consisting of the polynucleotide sequences of SEQ ID NOS: 7, 15, 25, 43, 59, 68, 71, 79, 85, 99, 111, 121, 140, 148, 156, 164, 176, 186, 202, 219, 227, 235, 245, 259, 277, 289, 297, 307, 313, 319, 331, 339, 347, 361, 367, 375, 385, 399, and 405 shown in Tables 1-46, or its complement.

3. The peptide array of claim 4, wherein each said polypeptide:

(a) comprises the amino acid sequence encoded by a polynucleotide selected from the group consisting of SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46; or
(b) comprises the amino acid sequence encoded by a polynucleotide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46; or
(c) comprises the amino acid sequence of an epitope of the amino acid sequence of a CA polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46; or
(d) is expressed on a cell surface, wherein the CA protein selected from the group consisting of SEQ ID NOS: 8, 10, 18, 20, 22, 28, 30, 42, 44, 46, 48, 54, 62, 68, 70, 72 and 74.

4. An isolated antibody or antigen binding fragment thereof, that binds to a polypeptide comprising the amino acid sequence encoded by a polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46.

5. The isolated antibody or antigen binding fragment thereof according the claim 4, wherein said antibody is a monoclonal antibody.

6. The monoclonal antibody according to claim 5, wherein the monoclonal antibody binds to the extracellular domain of the CA protein; or binds to at least one human cancer cell line.

7. A pharmaceutical composition comprising the antibody according to claim 5 or claim 6 and a pharmaceutically acceptable excipient.

8. A kit for detecting cancer cells comprising the antibody according to claim 5 or claim 6.

9. A method for detecting a presence or an absence of cancer cells in an individual, the method comprising: contacting cells from the individual with the antibody according to claim 5 or claim 6; and detecting a complex of a CAP from the cancer cells and the antibody, wherein detection of the complex correlates with the presence of cancer cells in the individual.

10. A kit for diagnosing the presence of cancer in a test sample, said kit comprising at least two polynucleotides that selectively hybridize to:

(a) at least two CA polynucleotide sequences selected from the group consisting of the polynucleotide sequences SEQ ID NOS: 7, 15, 25, 43, 59, 68, 71, 79, 85, 99, 111, 121, 140, 148, 156, 164, 176, 186, 202, 219, 227, 235, 245, 259, 277, 289, 297, 307, 313, 319, 331, 339, 347, 361, 367, 375, 385, 399, and 405 shown in Tables 1-46, or its complement; or
(b) the sequence of at least two polynucleotide sequences selected from the group consisting of the polynucleotide sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, a fragment thereof, or their complement.

11. A method of screening for anticancer activity comprising:

(a) providing a cell that expresses a cancer associated (CA) gene encoded by a nucleic acid sequence selected from the group consisting of the sequences SEQ ID NOS: 7, 15, 25, 43, 59, 68, 71, 79, 85, 99, 111, 121, 140, 148, 156, 164, 176, 186, 202, 219, 227, 235, 245, 259, 277, 289, 297, 307, 313, 319, 331, 339, 347, 361, 367, 375, 385, 399, and 405 shown in Tables 1-46, or fragment thereof;
(b) contacting a tissue sample derived from a cancer cell with an anticancer drug candidate; and
(c) monitoring an effect of the anticancer drug candidate on an expression of the CA polynucleotide in the tissue sample.

12. The method of screening for anticancer activity according to claim 11, wherein the CA gene comprises at least one nucleic acid sequence selected from the group consisting of the sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1- 46.

13. A method for detecting cancer associated with expression of a polypeptide or the presence of an antibody in a test cell sample, comprising the steps of:

(i) detecting a level of expression of at least one polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46, or a fragment thereof; or detecting a level of an antibody against an antigenic polypeptide selected from the group consisting of SEQ ID NOS: 9, 11, 17, 19, 21, 27, 45, 47, 61, 70, 73, 81, 87, 89, 91, 93, 95, 101, 113, 115, 123, 142, 144, 150, 152, 158, 160, 166, 168, 170, 172, 178, 180, 182, 188, 190, 192, 194, 196, 198, 204, 206, 208, 210, 221, 223, 229, 231, 237, 239, 247, 249, 261, 263, 265, 267, 269, 279, 281, 291, 299, 309, 315, 321, 323, 325, 333, 335, 341, 343, 349, 351, 353, 355, 357, 363, 369, 371, 377, 379, 381, 387, 389, 391, 393, 401, 407, 409, and 411 shown in Tables 1-46, or antigenic fragment thereof; and
(ii) comparing the level of expression of the polypeptide in the test sample or the level of the antibody with a level of expression of polypeptide or antibody in a normal cell sample, wherein an altered level of expression of the polypeptide or altered level of antibody in the test cell sample relative to the level of polypeptide expression or antibody level in the normal cell sample is indicative of the presence of cancer in the test cell sample.

14. A method for screening for a bioactive agent capable of modulating the activity of a CA protein (CAP), wherein said CAP is encoded by a nucleic acid comprising a nucleic acid sequence selected from the group consisting of the polynucleotide sequences SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, said method comprising: a) combining said CAP and a candidate bioactive agent; and b) determining the effect of the candidate agent on the bioactivity of said CAP.

15. A method for diagnosing cancer comprising:

(a) determining the expression of one or more genes comprising a nucleic acid sequence selected from the group consisting of the human genomic and mRNA sequences outlined in Tables 1-46, in a first tissue type of a first individual; and
(b) comparing said expression of said gene(s) from a second normal tissue type from said first individual or a second unaffected individual; wherein a difference in said expression indicates that the first individual has cancer.

273

16. A method for treating cancers comprising administering to a patient an inhibitor of a CA protein (CAP), wherein said CAP is encoded by a nucleic acid comprising a nucleic acid sequence selected from the group consisting of the human nucleic acid sequences in Tables 1-46.

17. A method for inhibiting expression of a cancer associated (CA) gene in a cell comprising: contacting a cell expressing a CA gene with a double stranded RNA comprising a sequence capable of hybridizing to a cancer associated (CA) mRNA corresponding to the polynucleotide sequences of SEQ ID NOS: 8, 10, 16, 18, 20, 26, 44, 46, 60, 69, 72, 80, 86, 88, 90, 92, 94, 100, 112, 114, 122, 141, 143, 149, 151, 157, 159, 165, 167, 169, 171, 177, 179, 181, 187, 189, 191, 193, 195, 197, 203, 205, 207, 209, 220, 222, 228, 230, 236, 238, 246, 248, 260, 262, 264, 266, 268, 278, 280, 290, 298, 308, 314, 320, 322, 324, 332, 334, 340, 342, 348, 350, 352, 354, 356, 362, 368, 370, 376, 378, 380, 386, 388, 390, 392, 400, 406, 408, and 410 shown in Tables 1-46, in an amount sufficient to elicit RNA interference; and inhibiting expression of the CA gene in the cell.

EP 2 204 376 A2

Figure 1

275

# Figure 2.

**Average Ct**

**Sample**

| | Housekeeper | Target |
|---|---|---|
| Human Genomic_clontech | 23.40229767 | 24.718297 |
| Malignant mammary adenocarcinoma pleural effusion | 24.57459367 | 25.62105175 |
| Mammary ductal carcinoma plueral effusion | 23.935565 | 25.064095 |
| Mammary ductal carcinoma_human _45537 | 24.06496275 | 24.8361145 |
| Mammary ductal carcinoma_human_45523 | 24.286542 | 25.59857167 |
| Mammary ductal carcinoma_human_45535 | 24.271219 | 25.62112767 |
| transformed primary embryonal kidney_human | 24.136573 | 25.86051867 |

# Figure 3.

**Target Normalized with Housekeeper**

| Sample | ΔCt | Stdev (ΔCt) |
|---|---|---|
| Human Genomic_clontech (**Calibrator**) | 1.69 | 0.22 |
| Malignant mammary adenocarcinoma pleural effusion | -1.85 | 0.14 |
| Mammary ductal carcinoma plueral effusion | 1.12 | 0.24 |
| Mammary ductal carcinoma_human _45537 | 1.02 | 0.16 |
| Mammary ductal carcinoma_human_45523 | 0.49 | 0.26 |
| Mammary ductal carcinoma_human_45535 | 1.03 | 0.18 |
| transformed primary embryonal kidney_human | 1.81 | 0.15 |

## Figure 4.

**Target Normalized with Housekeeper**

| Sample | ΔCt | Stdev (ΔCt) | ΔΔCt | Stdev (ΔΔCt) | Comparative expression level |
|---|---|---|---|---|---|
| Human Genomic_clontech (Calibrator) | 1.69 | 0.22 | 0 | 0.22 | 1 |
| Malignant mammary adenocarcinoma pleural effusion | -1.85 | 0.14 | -3.54 | 0.14 | 11.60 |
| Mammary ductal carcinoma plueral effusion | 1.12 | 0.24 | -0.57 | 0.24 | 1.48 |
| Mammary ductal carcinoma_human _45537 | 1.02 | 0.16 | -0.67 | 0.16 | 1.59 |
| Mammary ductal carcinoma_human_45523 | 0.49 | 0.26 | -1.20 | 0.26 | 2.30 |
| Mammary ductal carcinoma_human_45535 | 1.03 | 0.18 | -0.66 | 0.18 | 1.58 |
| transformed primary embryonal kidney_human | 1.81 | 0.15 | 0.12 | 0.15 | 0.92 |

# EP 2 204 376 A2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 10034650 B
- US 10035832 B
- US 10004113 B
- US 09997722 B
- US 10085117 B
- US 10087192 B
- US 10322281 B
- US 10322696 B
- US 10331053 B
- US 10330773 B
- US 10367094 B
- US 10388838 B
- US 10417375 B
- US 10461862 B
- US 10663431 B
- US 10669920 B
- US 10670914 B
- US 10674575 B
- US 10676684 B
- US 10692382 B
- US 10833833 B
- US 10836956 B
- US 5644048 A
- US 5386023 A
- US 5637684 A
- US 5602240 A
- US 5216141 A
- US 4469863 A
- US 5235033 A
- US 5034506 A
- US 5837832 A, Chee
- US 6410229 B, Lockhart
- US 5470967 A
- US 5714331 A
- EP 0225807 A
- US 5419966 A
- WO 9525116 A
- WO 9535505 A
- US 5445934 A
- US 5384261 A
- US 5700637 A
- US 9701019 W
- US 9701048 W
- US PN4959314 A
- WO 8705330 A
- US 4640835 A
- US 4496689 A
- US 4301144 A
- US 4670417 A
- US 4791192 A
- US 4179337 A
- US 4753894 A, Frankel
- US 4938948 A, Ring
- US 4956453 A, Bjorn
- EP 519596 A
- US 4816567 A
- US 5545807 A
- US 5545806 A
- US 5569825 A
- US 5625126 A
- US 5633425 A
- US 5661016 A
- US 5530101 A
- US 5585089 A
- WO 9824893 A
- WO 9110741 A
- WO 9630498 A
- WO 9402602 A
- US 5939598 A
- WO 9633735 A
- US 5681702 A
- US 5597909 A
- US 5545730 A
- US 5594117 A
- US 5591584 A
- US 5571670 A
- US 5580731 A
- US 5624802 A
- US 5635352 A
- US 5594118 A
- US 5359100 A
- US 5124246 A
- US 5681697 A
- WO 9104753 A
- WO 9010448 A
- WO 0044895 A, Kreutzer
- WO 0136646 A, Zernicka-Goetz
- WO 9932619 A, Fire
- WO 0129058 A, Mello and Fire
- WO 9314778 A
- WO 9007936 A
- WO 9403622 A
- WO 9325698 A
- WO 9325234 A
- US PN5219740 A
- WO 9311230 A
- WO 9310218 A
- US PN4777127 A

279

- GB 2200651 A
- EP 0345242 A
- WO 9102805 A
- WO 9412649 A
- WO 9303769 A
- WO 9319191 A
- WO 9428938 A
- WO 9511984 A
- WO 9500655 A
- US PN5814482 A
- WO 9507994 A
- WO 9617072 A
- WO 9530763 A
- WO 9742338 A
- WO 9011092 A
- US PN5580859 A
- US PN5422120 A
- WO 9513796 A
- WO 9423697 A
- WO 9114445 A
- EP 0524968 A
- US PN5206152 A
- WO 9211033 A
- US PN5149655 A
- US 9303868 W
- WO 9202526 A
- US PN5124246 A
- US PN4683195 A
- US PN4683202 A
- WO 0143869 A

**Non-patent literature cited in the description**

- **Sorensen et al.** *J. of Virology,* 2000, vol. 74, 2161
- **Hansen et al.** *Genome Res.,* 2000, vol. 10 (2), 237-43
- **Sorensen et al.** *J. Virology,* 1996, vol. 70, 4063
- **Sorensen et al.** *J. Virology,* 1993, vol. 67, 7118
- **Joosten et al.** *Virology,* 2000, vol. 268, 308
- **Li et al.** *Nature Genetics,* 1999, vol. 23, 348
- Mammary Tumors in the Mouse. Elsevier/North-Holland Biomedical Press
- **Marshall.** *Cell,* 1991, vol. 64, 313-326
- **Weinberg.** *Science,* 1991, vol. 254, 1138-1146
- Cancer: Principles and Practice of Oncology. 2001, 495-508
- **Beaucage et al.** *Tetrahedron,* 1993, vol. 49 (10), 1925
- **Letsinger.** *J. Org. Chem.,* 1970, vol. 35, 3800
- **Sprinzl et al.** *Eur. J. Biochem.,* 1977, vol. 81, 579
- **Letsinger et al.** *Nucl. Acids Res.,* 1986, vol. 14, 3487
- **Sawai et al.** *Chem. Lett.,* 1984, vol. 805
- **Letsinger et al.** *J. Am. Chem. Soc.,* 1988, vol. 110, 4470
- **Pauwels et al.** *Chemica Scripta,* 1986, vol. 26, 141
- **Mag et al.** *Nucleic Acids Res.,* 1991, vol. 19, 1437
- **Briu et al.** *J. Am. Chem. Soc.,* 1989, vol. 111, 2321
- **Eckstein.** Oligonucleotides and Analogues: A Practical Approach. Oxford University Press
- **Egholm.** *J. Am. Chem. Soc.,* 1992, vol. 114, 1895
- **Meier et al.** *Chem. Int. Ed. Engl.,* 1992, vol. 31, 1008
- **Nielsen.** *Nature,* 1993, vol. 365, 566
- **Carlsson et al.** *Nature,* 1996, vol. 380, 207
- **Denpcy et al.** *Proc. Natl. Acad. Sci. USA,* 1995, vol. 92, 6097
- **Kiedrowshi et al.** *Angew. Chem. Intl. Ed. English,* 1991, vol. 30, 423
- **Letsinger et al.** *Nucleoside & Nucleotide,* 1994, vol. 13, 1597
- Carbohydrate Modifications in Antisense Research. ASC Symposium Series, vol. 580
- **Mesmaeker et al.** *Bioorganic & Medicinal Chem. Lett.,* 1994, vol. 4, 395
- **Jeffs et al.** *J. Biomolecular NMR,* 1994, vol. 34, 17
- *Tetrahedron Lett.,* 1996, vol. 37, 743
- **Jenkins et al.** *Chem. Soc. Rev.,* 1995, 169-176
- **Rawls, C.** *E News,* 02 June 1997, 35
- **Smith ; Waterman.** *Adv. Appl. Math.,* 1981, vol. 2, 482
- **Needleman ; Wunsch.** *J. Mol. Biol.,* 1970, vol. 48, 443
- **Pearson ; Lipman.** *PNAS USA,* 1988, vol. 85, 2444
- **Devereux et al.** *Nucl. Acid Res.,* 1984, vol. 12, 387-395
- **Feng ; Doolittle.** *J. Mol. Evol.,* 1987, vol. 35, 351-360
- **Higgins ; Sharp.** *CABIOS,* 1989, vol. 5, 151-153
- **Altschul et al.** *J. Mol. Biol.,* 1990, vol. 215, 403-410
- **Karlin et al.** *PNAS USA,* 1993, vol. 90, 5873-5787
- **Altschul et al.** *Methods in Enzymology,* 1996, vol. 266, 460-480, http://blast.wustl.edu
- **Maniatis et al.** Molecular Cloning: A Laboratory Manual. 1989
- Short Protocols in Molecular Biology

- **Tijssen.** Overview of principles of hybridization and the strategy of nucleic acid assays. *Techniques in Biochemistry and Molecular Biology--Hybridization with Nucleic Acid Probes,* 1993
- **Schena et al.** *Science,* 1995, vol. 270, 467-470
- **DeRisi et al.** *Nature Genetics,* 1996, vol. 14, 457-460
- **Nielsen et al.** *Science,* 1991, vol. 254, 1497-1500
- **Nielsen.** *Curr. Opin. Biotechnol.,* 1999, vol. 10, 71-75
- **Urdea et al.** *Nucleic Acids Symp. Ser.,* 1991, vol. 24, 197-200
- **Pevzner et al.** *J. Biomol. Struc. & Dyn.,* 1991, vol. 9, 399-410
- **Maskos ; Southern.** *Nuc. Acids Res.,* 1992, vol. 20, 1679-84
- **R. Drmanac et al.** *J. Biomol. Struc. & Dyn.,* 1991, vol. 5, 1085-1102
- **P. A. Pevzner.** *J. Biomol. Struc. & Dyn.,* 1989, vol. 7, 63-73
- **T. Sano ; C. R. Cantor.** *Bio/Technology,* 1991, vol. 9, 1378-81
- *Pierce Chemical Company catalog,* 1994, 155-200
- **Smith et al.** Direct Mechanical Measurements of the Elasticity of Single DNA Molecules by Using Magnetic Beads. *Science,* 1992, vol. 258, 1122-1126
- **Pease et al.** *Proc. Natl. Acad. Sci. USA,* 1994, vol. 91 (11), 5022-5026
- **Guo et al.** *Nucleic Acids Res.,* 1994, vol. 22, 5456-5465
- **Maskos ; Southern.** *Nucleic Acids Research,* 1992, vol. 20, 1679-1684
- **Germer, S. et al.** *Genome Res.,* 2000, vol. 10, 258-266
- **Heid, C. A. et al.** *Genome Res.,* 1996, vol. 6, 986-994
- **Hunter et al.** *Nature,* 1962, vol. 144, 945
- **David et al.** *Biochemistry,* 1974, vol. 13, 1014
- **Pain et al.** *J. Immunol. Meth.,* 1981, vol. 40, 219
- **Nygren.** *J. Histochem. and Cytochem.,* 1982, vol. 30, 407
- **Smith ; Waterman.** *Adv. Appl. Math.,* 1981, vol. 2, 482-489
- **Go et al.** *Int. J. Peptide Protein Res.,* 1980, vol. 15, 211
- **Querol et al.** *Prot. Eng.,* 1996, vol. 9, 265
- **Olsen ; Thomsen.** *J. Gen. Microbiol.,* 1991, vol. 137, 579
- **Clarke et al.** *Biochemistry,* 1993, vol. 32, 4322
- **Wakarchuk et al.** *Protein Eng.,* 1994, vol. 7, 1379
- **Toma et al.** *Biochemistry,* 1991, vol. 30, 97
- **Haezerbrouck et al.** *Protein Eng.,* 1993, vol. 6, 643
- **Masul et al.** *Appl. Env. Microbiol.,* 1994, vol. 60, 3579
- **T.E. Creighton.** Proteins: Structure and Molecular Properties. W.H. Freeman & Co, 1983, 79-86
- **Aplin ; Wriston.** *LA Crit. Rev. Biochem.,* 1981, 259-306
- **Hakimuddin et al.** *Arch. Biochem. Biophys.,* 1987, vol. 259, 52
- **Edge et al.** *Anal. Biochem.,* 1981, vol. 118, 131
- **Thotakura et al.** *Meth. Enzymol.,* 1987, vol. 138, 350
- **Field et al.** *Mol. Cell. Biol.,* 1988, vol. 8, 2159-2165
- **Evan et al.** *Molecular and Cellular Biology,* 1985, vol. 5, 3610-3616
- **Paborsky et al.** *Protein Engineering,* 1990, vol. 3 (6), 547-553
- **Hopp et al.** *BioTechnology,* 1988, vol. 6, 1204-1210
- **Martin et al.** *Science,* 1992, vol. 255, 192-194
- **Skinner et al.** *J. Biol. Chem.,* 1991, vol. 266, 15163-15166
- **Lutz-Freyermuth et al.** *Proc. Natl. Acad. Sci. USA,* 1990, vol. 87, 6393-6397
- **Garnier et al.** *J. Mol. Biol.,* 1978, vol. 120, 97
- *Adv. in Enzymol.,* 1978, vol. 47, 45-148
- *J. Mol. Biol.,* 1982, vol. 157, 105-132
- *J. Virol.,* 1985, vol. 55 (3), 836-839
- **Jameson-Wolf.** *CABIOS,* 1988, vol. 4 (1), 181-186
- **Welling, G.W. et al.** *FEBS Lett.,* 1985, vol. 188, 215-218
- **Kohler ; Milstein.** *Nature,* 1975, vol. 256, 495
- **Goding.** Monoclonal Antibodies: Principles and Practice. Academic Press, 1986, 59-103
- **Waldmann, T. A.** *Science,* 1991, vol. 252, 1657-1662
- **Winter et al.** *Nature,* 1991, vol. 349, 293-299
- **Lobuglio et al.** *Proc. Nat. Acad. Sci. USA,* 1989, vol. 86, 4220-4224
- **Shaw et al.** *J Immunol,* 1987, vol. 138, 4534-4538
- **Brown et al.** *Cancer Res.,* 1987, vol. 47, 3577-3583
- **Riechmann et al.** *Nature,* 1988, vol. 332, 323-327
- **Verhoeyen et al.** *Science,* 1988, vol. 239, 1534-1536
- **Jones et al.** *Nature,* 1986, vol. 321, 522-525
- **Riechmann et al.** *Nature,* 1988, vol. 332, 323-329
- **Presta.** *Curr. Op. Struct. Biol.,* 1992, vol. 2, 593-596

- **Hoogenboom ; Winter.** *J. Mol. Biol.,* 1991, vol. 227, 381
- **Marks et al.** *J. Mol. Biol.,* 1991, vol. 222, 581
- **Cole et al.** Monoclonal Antibodies and Cancer Therapy. Alan R. Liss, 1985, 77
- **Boerner et al.** *J. Immunol.,* 1991, vol. 147 (1), 86-95
- **Marks et al.** *Bio/Technology,* 1992, vol. 10, 779-783
- **Lonberg et al.** *Nature,* 1994, vol. 368, 856-859
- **Morrison.** *Nature,* 1994, vol. 368, 812-13
- **Fishwild et al.** *Nature Biotechnology,* 1996, vol. 14, 845-51
- **Neuberger.** *Nature Biotechnology,* 1996, vol. 14, 826
- **Lonberg ; Huszar.** *Intern. Rev. Immunol.,* 1995, vol. 13, 65-93
- **Morrison et al.** *Proc. Natl. Acad. Sci, US.A.,* 1984, vol. 81, 6851-6855
- **Morrison.** *Oi, Adv. Immunol.,* 1988, vol. 44, 65-92
- **Verhoeyer et al.** *Science,* 1988, vol. 239, 1534-1536
- **Padlan.** *Molec. Immun.,* 1991, vol. 28, 489-498
- **Padlan.** *Molec. Immunol.,* 1994, vol. 31 (3), 169-217
- **Kettleborough, C.A. et al.** *Protein Eng.,* 1991, vol. 4 (7), 773-83
- **Chothia et al.** *J. Mol. Biol.,* 1987, vol. 196, 901-917
- **Kabat et al.** *U.S. Dept. of Health and Human Services NIH,* 1991
- **Merrifeld.** *J. Am. Chem. Soc.,* 1963, vol. 85, 2149
- **Caprino ; Han.** *J. Org. Chem.,* 1972, vol. 37, 3404
- **Milstein ; Kohler.** *Nature,* 1975, vol. 256, 495-497
- **Gulfre ; Milstein.** Methods in Enzymology: Immunochemical Techniques. Academic Press, 1981, vol. 73, 1-46
- **Nevelle et al.** *Immunol Rev,* 1982, vol. 62, 75-91
- **Ross et al.** *Eur. J Biochem,* 1980, vol. 104
- **Vitteta et al.** *Immunol Rev,* 1982, vol. 62, 158-183
- **Raso et al.** *Cancer Res,* 1982, vol. 42, 457-464
- **Trowbridge et al.** *Nature,* 1981, vol. 294, 171-173
- **Scopes, R.** Protein Purification. Springer-Verlag, 1982
- **Lockhart.** *Nature Biotechnology,* 1996, vol. 14, 1675-1680
- **Zlokarnik et al.** *Science,* 1998, vol. 279, 84-8
- **Heid et al.** *Genome Res.,* 1996, vol. 6, 986-994
- **Stein ; Cohen.** *Cancer Res.,* 1988, vol. 48, 2659
- **van der Krol et al.** *BioTechniques,* 1988, vol. 6, 958
- **Wu-Pong.** *BioPharm,* November 1994, 20-33
- **Mukhopadhyay ; Roth.** *Crit. Rev. in Oncogenesis,* 1996, vol. 7, 151-190
- **Fire et al.** *Nature,* 1998, vol. 391, 806
- **Sharp, P.A.** *Genes & Development,* 2001, vol. 15, 485-490
- **Elbashir, S.M. et al.** *Nature,* 2001, vol. 411, 494-498
- **Caplen, N.J. et al.** *Proc. Natl. Acad. Sci. USA,* 2001, vol. 98, 9742-9747
- **Harborth, J. et al.** *J. Cell Sci.,* 2001, vol. 114, 4557-4565
- **Lee, N.S. et al.** *Nat. Biotechnol.,* 2002, vol. 20, 500-505
- **Miyagishi, M. ; Taira, K.** *Nat. Biotechnol.,* 2002, vol. 20, 497-500
- **Paul, C.P. et al.** *Nat. Biotechnol.,* 2002, vol. 20, 505-508
- **Paule, M.R. ; White, R.J.** *Nucleic Acids Res.,* 2000, vol. 28, 1283-1298
- **Ziauddin, J. ; Sabatini, D.M.** *Nature,* 2001, vol. 411, 107-110
- **Berstein et al.** *Nature,* 2001, vol. 409, 363
- **Hutvagner et al.** *Science,* 2001, vol. 293, 834
- **Elbashir et al.** *Genes Dev.,* 2001, vol. 15, 188
- **Alfonso Gennaro.** Remington: The Science and Practice of Pharmacy. Lippincott, Williams, & Wilkins, 1995
- **Findeis et al.** *Trends Biotechnol.,* 1993, vol. 11, 202
- **Chiou et al.** Gene Therapeutics: Methods And Applications Of Direct Gene Transfer. 1994
- **Wu et al.** *J. Biol. Chem.,* 1988, vol. 263, 621
- **Wu et al.** *J. Biol. Chem.,* 1994, vol. 269, 542
- **Zenke et al.** *Proc. Natl. Acad Sci. (USA),* 1990, vol. 87, 3655
- **Wu et al.** *J. Biol. Chem.,* 1991, vol. 266, 338
- **Jolly.** *Cancer Gene Therapy,* 1994, vol. 1, 51
- **Kimura.** *Human Gene Therapy,* 1994, vol. 5, 845
- **Connelly.** *Human Gene Therapy,* 1995, vol. 1, 185
- **Kaplitt.** *Nature Genetics,* 1994, vol. 6, 148
- **Curiel.** *Hum. Gene Ther.,* 1992, vol. 3, 147
- **Wu.** *J. Biol. Chem.,* 1989, vol. 264, 16985
- **Philip.** *Mol. Cell Biol.,* 1994, vol. 14, 2411
- **Woffendin.** *Proc. Natl. Acad. Sci.,* 1994, vol. 91, 1581
- **Woffendin et al.** *Proc. Natl. Acad. Sci. USA,* 1994, vol. 91 (24), 11581

- **Brower.** *Nature Biotechnology,* 1998, vol. 16, 1304-1305
- **Mullis et al.** *Meth. Enzymol.,* 1987, vol. 155, 335
- **Sambrook et al.** Molecular Cloning: A Laboratory Manual. Cold Spring Harbor Laboratory, 1989
- **Saiki et al.** *Science,* 1985, vol. 239, 487
- **Sambrook et al.** Molecular Cloning: A Laboratory Manual. CSH Press, 1989, 14.2-14.33
- **Hanahan et al.** *Cell,* 2000, vol. 100, 57-70
- Gene Ontology: tool for the unification of biology. *The Gene Ontology Consortium Nature Genet.,* 2000, vol. 25, 25-29
- **Apweiler.** *Bioinformatics,* 2000, vol. 16 (12), 1145-1150
- **Luo et al.** *Nature Med,* 1999, vol. 5, 117-122
- **S.M. Weissman.** *Mol Biol. Med.,* 1987, vol. 4 (3), 133-143
- **Patanjali et al.** *Proc. Natl. Acad. Sci. USA,* 1991, vol. 88 (5), 1943-1947
- **Sambrook, J.T. et al.** Molecular Cloning: A Laboratory Manual. Cold Spring Harbor Laboratory Press
- Effective Large-Scale Sequence Similarity Searches,'' Computer Methods for Macromolecular Sequence Analysis. **Claverie.** Meth. Enzymol. Academic Press, 1996, vol. 266, 212-227
- **Claverie et al.** Automated DNA Sequencing and Analysis Techniques. Academic Press, 1994, 267
- **Claverie et al.** *Comput. Chem.,* 1993, vol. 17, 191
- **Altschul.** *J. Mol. Biol.,* 1990, vol. 215, 40
- **Pearson ; Lipman.** *PNAS,* 1988, vol. 85, 2444
- **Altschul et al.** *J. Mol. Bio.,* 1990, vol. 215, 403
- **Delli Bovi et al.** *Cancer Res.,* 1986, vol. 46, 6333-6338
- **Cesarone, C. et al.** *Anal Biochem,* 1979, vol. 100, 188-197
- **Southern, E. M.** *J. Mol. Biol.,* 1975, vol. 98, 503-517
- **Feinberg, A. P. et al.** *Anal. Biochem.,* 1983, vol. 132, 6-13
- **Sambrook et al.** Molecular Cloning: A Laboratory Manual. Cold Spring Harbor Lab. Press, 1989
- **Wright ; Manos et al.** PCR Protocols. Academic Press, 1990, 153-158
- **Sambrook et al.** Molecular Cloning: A Laboratory Manual. Cold Spring Harbor Laboratory Press, 1989, 16.17-16.22
- **Keown et al.** *Methods in Enzymology,* 1990, vol. 185, 527-537
- **Marks et al.** *Brit. J. Urol.,* 1995, vol. 75, 225
- **Skea et al.** *J. Immunol.,* 1993, vol. 151, 3557
- **Mather et al.** *J. Nucl. Med.,* 1990, vol. 31, 692
- **Zhang et al.** *Nucl. Med. Biol.,* 1992, vol. 19, 607